# EUROPEAN PATENT APPLICATION

(11) **EP 1 816 213 A2**
(43) Date of publication of application: **08.08.2007**
(21) Application number: 06024949.7
(22) Date of filing: 16.07.2001
(51) Int. Cl.: C12Q 1/68, C12Q 1/48

(54) **Novel DNA polymerase III holoenzyme delta subunit nucleic acid molecules and proteins**

(30) Priority: 14.07.2000 US 218246 P; 28.03.2001 US 818780
(62) Divisional of application: 01957164.5
(71) Applicant: Replidyne, Inc., Louisville, CO 80027 (US)
(72) Inventor: Bullard, James J., Longmont, CO 80504 (US); Janjic, Nebojsa, Boulder, CO 80301 (US); McHenry, Charles S., Denver, CO 80206 (US)
(74) Representative: UEXKÜLL & STOLBERG

(57) **Abstract**

Gene and amino acid sequences encoding DNA polymerase III holoenzyme δ subunits and structural genes from bacteria are provided. Also provided are antibodies and other reagents useful to identify DNA polymerase III δ subunit molecules.

Also provided are methods to identify DNA polymerase III δ subunit molecules. The use of DNA polymerase III δ subunit molecules in assays to identify candidate antibiotics are also provided.

### Annex to the application documents - subsequently filed sequences listing

SEQUENCE LISTING

<110> Bullard, James M.
      Janjic, Nebojsa
      McHenry, Charles S.
      Replidyne, Inc.

<120> NOVEL DNA POLYMERASE III HOLOENZYME DELTA SUBUNIT
      NUCLEIC ACID MOLECULES AND PROTEINS

<130> RDYN03PCT

<140>
<141>

<150> 60/218,246
<151> 2000-07-14

<160> 230

<170> PatentIn Ver. 2.0

<210> 1
<211> 1053
<212> DNA
<213> Aquifex aeolicus

<400> 1
   gtggaaacca caatattcca gttccagaaa acttttttca caaaacctcc gaaggagagg 60
   gtcttcgtcc ttcatggaga agagcagtat ctcataagaa cctttttgtc taagctgaag 120
   gaaaagtacg gggagaatta cacggttctg tggggggatg agataagcga ggaggaattc 180
   tacactgccc tttccgagac cagtatattc ggcggttcaa aggaaaaagc ggtggtcatt 240
   tacaacttcg gggatttcct gaagaagctc ggaaggaaga aaaaggaaaa agaaaggctt 300
   ataaaagtcc tcagaaacgt aaagagtaac tacgtattta tagtgtacga tgcgaaactc 360
   cagaaacagg aactttcttc ggaacctctg aaatccgtag cgtctttcgg cggtatagtg 420
   gtagcaaaca ggctgagcaa ggagaggata aaacagctcg tccttaagaa gttcaaagaa 480
   aaagggataa acgtagaaaa cgatgccctt gaataccttc tccagctcac gggttacaac 540
   ttgatggagc tcaaacttga ggttgaaaaa ctgatagatt acgcaagtga aaagaaaatt 600
   ttaacactcg atgaggtaaa gagagtagcc ttctcagtct cagaaaacgt aaacgtattt 660
   gagttcgttg atttactcct cttaaaagat tacgaaaagg ctcttaaagt tttggactcc 720
   ctcatttcct tcggaataca ccccctccag attatgaaaa tcctgtcctc ctatgctcta 780
   aaactttaca ccctcaagag gcttgaagag aagggagagg acctgaataa ggcgatggaa 840
   agcgtgggaa taaagaacaa ctttctcaag atgaagttca aatcttactt aaaggcaaac 900
   tctaaagagg acttgaagaa cctaatcctc tccctccaga ggatagacgc tttttctaaa 960
   ctttactttc aggacacagt gcagttgctg agggatttct tgacctcaag actggagagg 1020
   gaagttgtga aaaatacttc tcatggtgga taa 1053

<210> 2
<211> 1053
<212> DNA
<213> Aquifex aeolicus

<220>
<221> CDS
<222> (1)..(1053)

<400> 2
gtg gaa acc aca ata ttc cag ttc cag aaa act ttt ttc aca aaa cct 48
Val Glu Thr Thr Ile Phe Gln Phe Gln Lys Thr Phe Phe Thr Lys Pro
1               5                   10                  15

ccg aag gag agg gtc ttc gtc ctt cat gga gaa gag cag tat ctc ata 96
Pro Lys Glu Arg Val Phe Val Leu His Gly Glu Glu Gln Tyr Leu Ile
            20                  25                  30

aga acc ttt ttg tct aag ctg aag gaa aag tac ggg gag aat tac acg 144
Arg Thr Phe Leu Ser Lys Leu Lys Glu Lys Tyr Gly Glu Asn Tyr Thr
        35                  40                  45

gtt ctg tgg ggg gat gag ata agc gag gag gaa ttc tac act gcc ctt 192
Val Leu Trp Gly Asp Glu Ile Ser Glu Glu Glu Phe Tyr Thr Ala Leu
    50                  55                  60

tcc gag acc agt ata ttc ggc ggt tca aag gaa aaa gcg gtg gtc att 240
Ser Glu Thr Ser Ile Phe Gly Gly Ser Lys Glu Lys Ala Val Val Ile
65                  70                  75                  80

tac aac ttc ggg gat ttc ctg aag aag ctc gga agg aag aaa aag gaa 288

Tyr Asn Phe Gly Asp Phe Leu Lys Lys Leu Gly Arg Lys Lys Lys Glu
                85                  90                  95

aaa gaa agg ctt ata aaa gtc ctc aga aac gta aag agt aac tac gta 336
Lys Glu Arg Leu Ile Lys Val Leu Arg Asn Val Lys Ser Asn Tyr Val
            100                 105                 110

ttt ata gtg tac gat gcg aaa ctc cag aaa cag gaa ctt tct tcg gaa 384
Phe Ile Val Tyr Asp Ala Lys Leu Gln Lys Gln Glu Leu Ser Ser Glu
        115                 120                 125

cct ctg aaa tcc gta gcg tct ttc ggc ggt ata gtg gta gca aac agg 432
Pro Leu Lys Ser Val Ala Ser Phe Gly Gly Ile Val Val Ala Asn Arg
    130                 135                 140

ctg agc aag gag agg ata aaa cag ctc gtc ctt aag aag ttc aaa gaa 480
Leu Ser Lys Glu Arg Ile Lys Gln Leu Val Leu Lys Lys Phe Lys Glu
145                 150                 155                 160

aaa ggg ata aac gta gaa aac gat gcc ctt gaa tac ctt ctc cag ctc 528
Lys Gly Ile Asn Val Glu Asn Asp Ala Leu Glu Tyr Leu Leu Gln Leu
                165                 170                 175

acg ggt tac aac ttg atg gag ctc aaa ctt gag gtt gaa aaa ctg ata 576
Thr Gly Tyr Asn Leu Met Glu Leu Lys Leu Glu Val Glu Lys Leu Ile
            180                 185                 190

gat tac gca agt gaa aag aaa att tta aca ctc gat gag gta aag aga 624
Asp Tyr Ala Ser Glu Lys Lys Ile Leu Thr Leu Asp Glu Val Lys Arg
        195                 200                 205

gta gcc ttc tca gtc tca gaa aac gta aac gta ttt gag ttc gtt gat 672
Val Ala Phe Ser Val Ser Glu Asn Val Asn Val Phe Glu Phe Val Asp
    210                 215                 220

tta ctc ctc tta aaa gat tac gaa aag gct ctt aaa gtt ttg gac tcc 720
Leu Leu Leu Leu Lys Asp Tyr Glu Lys Ala Leu Lys Val Leu Asp Ser
225                 230                 235                 240

ctc att tcc ttc gga ata cac ccc ctc cag att atg aaa atc ctg tcc 768
Leu Ile Ser Phe Gly Ile His Pro Leu Gln Ile Met Lys Ile Leu Ser
                245                 250                 255

tcc tat gct cta aaa ctt tac acc ctc aag agg ctt gaa gag aag gga 816
Ser Tyr Ala Leu Lys Leu Tyr Thr Leu Lys Arg Leu Glu Glu Lys Gly
            260                 265                 270

gag gac ctg aat aag gcg atg gaa agc gtg gga ata aag aac aac ttt 864

Glu Asp Leu Asn Lys Ala Met Glu Ser Val Gly Ile Lys Asn Asn Phe
        275                 280                 285

ctc aag atg aag ttc aaa tct tac tta aag gca aac tct aaa gag gac    912
Leu Lys Met Lys Phe Lys Ser Tyr Leu Lys Ala Asn Ser Lys Glu Asp
    290                 295                 300

ttg aag aac cta atc ctc tcc ctc cag agg ata gac gct ttt tct aaa    960
Leu Lys Asn Leu Ile Leu Ser Leu Gln Arg Ile Asp Ala Phe Ser Lys
305                 310                 315                 320

ctt tac ttt cag gac aca gtg cag ttg ctg agg gat ttc ttg acc tca    1008
Leu Tyr Phe Gln Asp Thr Val Gln Leu Leu Arg Asp Phe Leu Thr Ser
                325                 330                 335

aga ctg gag agg gaa gtt gtg aaa aat act tct cat ggt gga taa        1053
Arg Leu Glu Arg Glu Val Val Lys Asn Thr Ser His Gly Gly
            340                 345                 350

<210> 3
<211> 350
<212> PRT
<213> Aquifex aeolicus

<400> 3
Val Glu Thr Thr Ile Phe Gln Phe Gln Lys Thr Phe Phe Thr Lys Pro
 1               5                  10                  15

Pro Lys Glu Arg Val Phe Val Leu His Gly Glu Glu Gln Tyr Leu Ile
            20                  25                  30

Arg Thr Phe Leu Ser Lys Leu Lys Glu Lys Tyr Gly Glu Asn Tyr Thr
        35                  40                  45

Val Leu Trp Gly Asp Glu Ile Ser Glu Glu Glu Phe Tyr Thr Ala Leu
    50                  55                  60

Ser Glu Thr Ser Ile Phe Gly Gly Ser Lys Glu Lys Ala Val Val Ile
65                  70                  75                  80

Tyr Asn Phe Gly Asp Phe Leu Lys Lys Leu Gly Arg Lys Lys Lys Glu
                85                  90                  95

Lys Glu Arg Leu Ile Lys Val Leu Arg Asn Val Lys Ser Asn Tyr Val
            100                 105                 110

Phe Ile Val Tyr Asp Ala Lys Leu Gln Lys Gln Glu Leu Ser Ser Glu

115                 120                 125

Pro Leu Lys Ser Val Ala Ser Phe Gly Gly Ile Val Val Ala Asn Arg
    130                 135                 140

Leu Ser Lys Glu Arg Ile Lys Gln Leu Val Leu Lys Lys Phe Lys Glu
145                 150                 155                 160

Lys Gly Ile Asn Val Glu Asn Asp Ala Leu Glu Tyr Leu Leu Gln Leu
                165                 170                 175

Thr Gly Tyr Asn Leu Met Glu Leu Lys Leu Glu Val Glu Lys Leu Ile
            180                 185                 190

Asp Tyr Ala Ser Glu Lys Lys Ile Leu Thr Leu Asp Glu Val Lys Arg
        195                 200                 205

Val Ala Phe Ser Val Ser Glu Asn Val Asn Val Phe Glu Phe Val Asp
    210                 215                 220

Leu Leu Leu Leu Lys Asp Tyr Glu Lys Ala Leu Lys Val Leu Asp Ser
225                 230                 235                 240

Leu Ile Ser Phe Gly Ile His Pro Leu Gln Ile Met Lys Ile Leu Ser
                245                 250                 255

Ser Tyr Ala Leu Lys Leu Tyr Thr Leu Lys Arg Leu Glu Glu Lys Gly
            260                 265                 270

Glu Asp Leu Asn Lys Ala Met Glu Ser Val Gly Ile Lys Asn Asn Phe
        275                 280                 285

Leu Lys Met Lys Phe Lys Ser Tyr Leu Lys Ala Asn Ser Lys Glu Asp
    290                 295                 300

Leu Lys Asn Leu Ile Leu Ser Leu Gln Arg Ile Asp Ala Phe Ser Lys
305                 310                 315                 320

Leu Tyr Phe Gln Asp Thr Val Gln Leu Leu Arg Asp Phe Leu Thr Ser
                325                 330                 335

Arg Leu Glu Arg Glu Val Val Lys Asn Thr Ser His Gly Gly
            340                 345                 350

<210> 4
<211> 1029
<212> DNA

<213> Bacillus halodurans

<400> 4
atgaactatt taaaattaaa gcaagatgtc caagcgggac gagtggcccc tgtatatttt 60

atgtatggga ccgagttgtt tttaatggaa gacctcatac aggacatttt gtccgtaacg 120

ttatcggatg aggaaaggga tatgaatgtt tcatcttatt cccttactga tgttccgatt 180

gaggcggcgc tcgaagaagc agaaacggtt cctttttttg gctcgaagcg ggtcgtgatc 240

ttaaaagatg ctgccctatt tacaagccaa aagcttgatg ttgagcacga tgtgaaacga 300

ttggagcaat acatattgaa cccagttcca gagacggttt tgctgatcat ggccccttat 360

gaaaagctcg atgaacgaaa aaaaatcacg aagcttatca aaaaagagtc gcttgttctt 420

gaggcaaagc ctttggacga aggagaggca aaagcatggc tatcctcact ggcaagcgag 480

cttcaggtgg agatggacga gaaagctatc gagacgttgt tagggatgac gggtcttcgc 540

ttgacccaat tagcatctga aatgaataaa ttagcgttgt atgtagggga aggaggcatc 600

atccgatcag aggacgtaac cttgctcgtt gctaaaacgc tcgatcaaaa catatttgat 660

ctgattgatt ttgccatcaa tcagaggact catcaagcgc tctctcttta ccatgagctc 720

ctgaaacaaa aagaggagcc attgaagctg ttggccttat taacacggca gtttcgcatc 780

atgtaccaag tcaaagagct tggacgaaga ggatacaccc cgaatcagat ggcgaagccg 840

ttaaaaattc atccctatgt tgccaaactt gcgggaaaaa aagcagccag catgtcagac 900

caactgttat attccctaat cgaaaaagct gcggatacag agtttgcgat taaaagtgga 960

aaggttgata aagtgctggc attagaattg tttttactca caatgggttc tagggagagc 1020

gtaggttag 1029

<210> 5
<211> 1029
<212> DNA
<213> Bacillus halodurans

<220>
<221> CDS
<222> (1)..(1029)

<400> 5
atg aac tat tta aaa tta aag caa gat gtc caa gcg gga cga gtg gcc     48
Met Asn Tyr Leu Lys Leu Lys Gln Asp Val Gln Ala Gly Arg Val Ala
1               5                   10                  15

cct gta tat ttt atg tat ggg acc gag ttg ttt tta atg gaa gac ctc     96
Pro Val Tyr Phe Met Tyr Gly Thr Glu Leu Phe Leu Met Glu Asp Leu
            20                  25                  30

ata cag gac att ttg tcc gta acg tta tcg gat gag gaa agg gat atg     144
Ile Gln Asp Ile Leu Ser Val Thr Leu Ser Asp Glu Glu Arg Asp Met
        35                  40                  45

aat gtt tca tct tat tcc ctt act gat gtt ccg att gag gcg gcg ctc     192
Asn Val Ser Ser Tyr Ser Leu Thr Asp Val Pro Ile Glu Ala Ala Leu
    50                  55                  60

gaa gaa gca gaa acg gtt cct ttt ttt ggc tcg aag cgg gtc gtg atc     240
Glu Glu Ala Glu Thr Val Pro Phe Phe Gly Ser Lys Arg Val Val Ile
65                  70                  75                  80

tta aaa gat gct gcc cta ttt aca agc caa aag ctt gat gtt gag cac     288
Leu Lys Asp Ala Ala Leu Phe Thr Ser Gln Lys Leu Asp Val Glu His
                85                  90                  95

gat gtg aaa cga ttg gag caa tac ata ttg aac cca gtt cca gag acg     336
Asp Val Lys Arg Leu Glu Gln Tyr Ile Leu Asn Pro Val Pro Glu Thr
            100                 105                 110

gtt ttg ctg atc atg gcc cct tat gaa aag ctc gat gaa cga aaa aaa     384
Val Leu Leu Ile Met Ala Pro Tyr Glu Lys Leu Asp Glu Arg Lys Lys
        115                 120                 125

atc acg aag ctt atc aaa aaa gag tcg ctt gtt ctt gag gca aag cct     432
Ile Thr Lys Leu Ile Lys Lys Glu Ser Leu Val Leu Glu Ala Lys Pro
    130                 135                 140

ttg gac gaa gga gag gca aaa gca tgg cta tcc tca ctg gca agc gag     480
Leu Asp Glu Gly Glu Ala Lys Ala Trp Leu Ser Ser Leu Ala Ser Glu
145                 150                 155                 160

ctt cag gtg gag atg gac gag aaa gct atc gag acg ttg tta ggg atg     528
Leu Gln Val Glu Met Asp Glu Lys Ala Ile Glu Thr Leu Leu Gly Met
                165                 170                 175

acg ggt ctt cgc ttg acc caa tta gca tct gaa atg aat aaa tta gcg     576
Thr Gly Leu Arg Leu Thr Gln Leu Ala Ser Glu Met Asn Lys Leu Ala

            180                 185                 190

ttg tat gta ggg gaa gga ggc atc atc cga tca gag gac gta acc ttg 624
Leu Tyr Val Gly Glu Gly Gly Ile Ile Arg Ser Glu Asp Val Thr Leu
        195                 200                 205

ctc gtt gct aaa acg ctc gat caa aac ata ttt gat ctg att gat ttt 672
Leu Val Ala Lys Thr Leu Asp Gln Asn Ile Phe Asp Leu Ile Asp Phe
    210                 215                 220

gcc atc aat cag agg act cat caa gcg ctc tct ctt tac cat gag ctc 720
Ala Ile Asn Gln Arg Thr His Gln Ala Leu Ser Leu Tyr His Glu Leu
225                 230                 235                 240

ctg aaa caa aaa gag gag cca ttg aag ctg ttg gcc tta tta aca cgg 768
Leu Lys Gln Lys Glu Glu Pro Leu Lys Leu Leu Ala Leu Leu Thr Arg
                245                 250                 255

cag ttt cgc atc atg tac caa gtc aaa gag ctt gga cga aga gga tac 816
Gln Phe Arg Ile Met Tyr Gln Val Lys Glu Leu Gly Arg Arg Gly Tyr
            260                 265                 270

acc ccg aat cag atg gcg aag ccg tta aaa att cat ccc tat gtt gcc 864
Thr Pro Asn Gln Met Ala Lys Pro Leu Lys Ile His Pro Tyr Val Ala
        275                 280                 285

aaa ctt gcg gga aaa aaa gca gcc agc atg tca gac caa ctg tta tat 912
Lys Leu Ala Gly Lys Lys Ala Ala Ser Met Ser Asp Gln Leu Leu Tyr
    290                 295                 300

tcc cta atc gaa aaa gct gcg gat aca gag ttt gcg att aaa agt gga 960
Ser Leu Ile Glu Lys Ala Ala Asp Thr Glu Phe Ala Ile Lys Ser Gly
305                 310                 315                 320

aag gtt gat aaa gtg ctg gca tta gaa ttg ttt tta ctc aca atg ggt 1008
Lys Val Asp Lys Val Leu Ala Leu Glu Leu Phe Leu Leu Thr Met Gly
                325                 330                 335

tct agg gag agc gta ggt tag 1029
Ser Arg Glu Ser Val Gly
            340

<210> 6
<211> 342
<212> PRT
<213> Bacillus halodurans

<400> 6
Met Asn Tyr Leu Lys Leu Lys Gln Asp Val Gln Ala Gly Arg Val Ala
1               5                   10                  15

Pro Val Tyr Phe Met Tyr Gly Thr Glu Leu Phe Leu Met Glu Asp Leu
            20                  25                  30

Ile Gln Asp Ile Leu Ser Val Thr Leu Ser Asp Glu Glu Arg Asp Met
        35                  40                  45

Asn Val Ser Ser Tyr Ser Leu Thr Asp Val Pro Ile Glu Ala Ala Leu
    50                  55                  60

Glu Glu Ala Glu Thr Val Pro Phe Phe Gly Ser Lys Arg Val Val Ile
65                  70                  75                  80

Leu Lys Asp Ala Ala Leu Phe Thr Ser Gln Lys Leu Asp Val Glu His
                85                  90                  95

Asp Val Lys Arg Leu Glu Gln Tyr Ile Leu Asn Pro Val Pro Glu Thr
            100                 105                 110

Val Leu Leu Ile Met Ala Pro Tyr Glu Lys Leu Asp Glu Arg Lys Lys
        115                 120                 125

Ile Thr Lys Leu Ile Lys Lys Glu Ser Leu Val Leu Glu Ala Lys Pro
    130                 135                 140

Leu Asp Glu Gly Glu Ala Lys Ala Trp Leu Ser Ser Leu Ala Ser Glu
145                 150                 155                 160

Leu Gln Val Glu Met Asp Glu Lys Ala Ile Glu Thr Leu Leu Gly Met
                165                 170                 175

Thr Gly Leu Arg Leu Thr Gln Leu Ala Ser Glu Met Asn Lys Leu Ala
            180                 185                 190

Leu Tyr Val Gly Glu Gly Gly Ile Ile Arg Ser Glu Asp Val Thr Leu
        195                 200                 205

Leu Val Ala Lys Thr Leu Asp Gln Asn Ile Phe Asp Leu Ile Asp Phe
    210                 215                 220

Ala Ile Asn Gln Arg Thr His Gln Ala Leu Ser Leu Tyr His Glu Leu
225                 230                 235                 240

Leu Lys Gln Lys Glu Glu Pro Leu Lys Leu Leu Ala Leu Leu Thr Arg
                245                 250                 255

Gln Phe Arg Ile Met Tyr Gln Val Lys Glu Leu Gly Arg Arg Gly Tyr
            260                 265                 270

Thr Pro Asn Gln Met Ala Lys Pro Leu Lys Ile His Pro Tyr Val Ala
        275                 280                 285

Lys Leu Ala Gly Lys Lys Ala Ala Ser Met Ser Asp Gln Leu Leu Tyr
    290                 295                 300

Ser Leu Ile Glu Lys Ala Ala Asp Thr Glu Phe Ala Ile Lys Ser Gly
305                 310                 315                 320

Lys Val Asp Lys Val Leu Ala Leu Glu Leu Phe Leu Leu Thr Met Gly
                325                 330                 335

Ser Arg Glu Ser Val Gly
            340

<210> 7
<211> 1044
<212> DNA
<213> Bacillus subtilis

<400> 7
atggtatttg atgtgtggaa aagcctgaaa aaaggagagg tccatccggt ttattgttta 60

tacggaaaag agacatatct gctgcaagaa accgtcagca ggatcagaca gacggtcgtt 120

gatcaggaga caaaggattt caatctgtcc gtttttgatc tggaagagga cccgctggat 180

caagcgattg cagatgctga aacgtttccg tttatggggg agcggcgtct tgtcattgtg 240

aaaaatccat attttttaac aggtgaaaag aaaaaagaaa aaattgagca caacgtcagt 300

gcgcttgaat catatataca atcacctgcg ccttacacag tctttgtcct gcttgctccg 360

tatgaaaagc ttgatgagcg aaaaaagctg acgaaagcgt taaagaagca cgcgtttatg 420

atggaggcga aggaattaaa tgcaaaggaa acgacagact ttactgtcaa ccttgcaaaa 480

acagagcaga aaacaatcgg cacggaagcg gcggagcatt tggttctgct tgtgaacggt 540

catctgtcat cgatttttca ggagattcaa aagctctgca cgtttattgg agatcgtgaa 600

gaaattacgc tggatgatgt aaaaatgctt gttgctagaa gccttgaaca aaatattttt 660

gagctgatca ataaaatcgt caaccgaaaa cgaacagaga gtctgcaaat tttttatgat 720

ttgctaaaac aaaatgaaga accgatcaaa attatggcgc ttatttcgaa tcagttccgg 780

ctgattctgc agacgaagta cttcgcggaa cagggatacg gacaaaaaca aatcgcttct 840

aatctaaaag ttcacccatt tcgggtaaag ctggcgatgg atcaagcaag gcttttttca 900

gaggaggagc tccgtttaat tattgaacag cttgccgtca tggactatga gatgaaaacc 960

gggaaaaagg acaagcagct cctgctcgaa ctgttccttt tacagctgtt aaaaagaaat 1020

gaaaaaaacg atccccatta ttga 1044

<210> 8
<211> 1044
<212> DNA
<213> Bacillus subtilis

<220>
<221> CDS
<222> (1)..(1044)

<400> 8
atg gta ttt gat gtg tgg aaa agc ctg aaa aaa gga gag gtc cat ccg 48
Met Val Phe Asp Val Trp Lys Ser Leu Lys Lys Gly Glu Val His Pro
1               5                   10                  15

gtt tat tgt tta tac gga aaa gag aca tat ctg ctg caa gaa acc gtc 96
Val Tyr Cys Leu Tyr Gly Lys Glu Thr Tyr Leu Leu Gln Glu Thr Val
            20                  25                  30

agc agg atc aga cag acg gtc gtt gat cag gag aca aag gat ttc aat 144
Ser Arg Ile Arg Gln Thr Val Val Asp Gln Glu Thr Lys Asp Phe Asn
        35                  40                  45

ctg tcc gtt ttt gat ctg gaa gag gac ccg ctg gat caa gcg att gca 192
Leu Ser Val Phe Asp Leu Glu Glu Asp Pro Leu Asp Gln Ala Ile Ala
    50                  55                  60

gat gct gaa acg ttt ccg ttt atg ggg gag cgg cgt ctt gtc att gtg 240
Asp Ala Glu Thr Phe Pro Phe Met Gly Glu Arg Arg Leu Val Ile Val
65                  70                  75                  80

aaa aat cca tat ttt tta aca ggt gaa aag aaa aaa gaa aaa att gag 288
Lys Asn Pro Tyr Phe Leu Thr Gly Glu Lys Lys Lys Glu Lys Ile Glu
                85                  90                  95

cac aac gtc agt gcg ctt gaa tca tat ata caa tca cct gcg cct tac 336
His Asn Val Ser Ala Leu Glu Ser Tyr Ile Gln Ser Pro Ala Pro Tyr
            100                 105                 110

aca gtc ttt gtc ctg ctt gct ccg tat gaa aag ctt gat gag cga aaa 384
Thr Val Phe Val Leu Leu Ala Pro Tyr Glu Lys Leu Asp Glu Arg Lys
        115                 120                 125

aag ctg acg aaa gcg tta aag aag cac gcg ttt atg atg gag gcg aag 432
Lys Leu Thr Lys Ala Leu Lys Lys His Ala Phe Met Met Glu Ala Lys
    130                 135                 140

gaa tta aat gca aag gaa acg aca gac ttt act gtc aac ctt gca aaa 480
Glu Leu Asn Ala Lys Glu Thr Thr Asp Phe Thr Val Asn Leu Ala Lys
145                 150                 155                 160

aca gag cag aaa aca atc ggc acg gaa gcg gcg gag cat ttg gtt ctg 528
Thr Glu Gln Lys Thr Ile Gly Thr Glu Ala Ala Glu His Leu Val Leu
                165                 170                 175

ctt gtg aac ggt cat ctg tca tcg att ttt cag gag att caa aag ctc 576
Leu Val Asn Gly His Leu Ser Ser Ile Phe Gln Glu Ile Gln Lys Leu
            180                 185                 190

tgc acg ttt att gga gat cgt gaa gaa att acg ctg gat gat gta aaa 624
Cys Thr Phe Ile Gly Asp Arg Glu Glu Ile Thr Leu Asp Asp Val Lys
        195                 200                 205

atg ctt gtt gct aga agc ctt gaa caa aat att ttt gag ctg atc aat 672
Met Leu Val Ala Arg Ser Leu Glu Gln Asn Ile Phe Glu Leu Ile Asn
    210                 215                 220

aaa atc gtc aac cga aaa cga aca gag agt ctg caa att ttt tat gat 720
Lys Ile Val Asn Arg Lys Arg Thr Glu Ser Leu Gln Ile Phe Tyr Asp
225                 230                 235                 240

ttg cta aaa caa aat gaa gaa ccg atc aaa att atg gcg ctt att tcg 768
Leu Leu Lys Gln Asn Glu Glu Pro Ile Lys Ile Met Ala Leu Ile Ser
                245                 250                 255

aat cag ttc cgg ctg att ctg cag acg aag tac ttc gcg gaa cag gga 816
Asn Gln Phe Arg Leu Ile Leu Gln Thr Lys Tyr Phe Ala Glu Gln Gly
            260                 265                 270

tac gga caa aaa caa atc gct tct aat cta aaa gtt cac cca ttt cgg 864
Tyr Gly Gln Lys Gln Ile Ala Ser Asn Leu Lys Val His Pro Phe Arg
        275                 280                 285

gta aag ctg gcg atg gat caa gca agg ctt ttt tca gag gag gag ctc    912
Val Lys Leu Ala Met Asp Gln Ala Arg Leu Phe Ser Glu Glu Glu Leu
    290                 295                 300

cgt tta att att gaa cag ctt gcc gtc atg gac tat gag atg aaa acc    960
Arg Leu Ile Ile Glu Gln Leu Ala Val Met Asp Tyr Glu Met Lys Thr
305                 310                 315                 320

ggg aaa aag gac aag cag ctc ctg ctc gaa ctg ttc ctt tta cag ctg    1008
Gly Lys Lys Asp Lys Gln Leu Leu Leu Glu Leu Phe Leu Leu Gln Leu
                325                 330                 335

tta aaa aga aat gaa aaa aac gat ccc cat tat tga                    1044
Leu Lys Arg Asn Glu Lys Asn Asp Pro His Tyr
            340                 345

<210> 9
<211> 347
<212> PRT
<213> Bacillus subtilis

<400> 9
Met Val Phe Asp Val Trp Lys Ser Leu Lys Lys Gly Glu Val His Pro
  1               5                  10                  15

Val Tyr Cys Leu Tyr Gly Lys Glu Thr Tyr Leu Leu Gln Glu Thr Val
             20                  25                  30

Ser Arg Ile Arg Gln Thr Val Val Asp Gln Glu Thr Lys Asp Phe Asn
         35                  40                  45

Leu Ser Val Phe Asp Leu Glu Glu Asp Pro Leu Asp Gln Ala Ile Ala
     50                  55                  60

Asp Ala Glu Thr Phe Pro Phe Met Gly Glu Arg Arg Leu Val Ile Val
 65                  70                  75                  80

Lys Asn Pro Tyr Phe Leu Thr Gly Glu Lys Lys Lys Glu Lys Ile Glu
                 85                  90                  95

His Asn Val Ser Ala Leu Glu Ser Tyr Ile Gln Ser Pro Ala Pro Tyr
            100                 105                 110

Thr Val Phe Val Leu Leu Ala Pro Tyr Glu Lys Leu Asp Glu Arg Lys
        115                 120                 125

Lys Leu Thr Lys Ala Leu Lys Lys His Ala Phe Met Met Glu Ala Lys
    130                 135                 140

Glu Leu Asn Ala Lys Glu Thr Thr Asp Phe Thr Val Asn Leu Ala Lys
145                 150                 155                 160

Thr Glu Gln Lys Thr Ile Gly Thr Glu Ala Ala Glu His Leu Val Leu
                165                 170                 175

Leu Val Asn Gly His Leu Ser Ser Ile Phe Gln Glu Ile Gln Lys Leu
            180                 185                 190

Cys Thr Phe Ile Gly Asp Arg Glu Glu Ile Thr Leu Asp Asp Val Lys
        195                 200                 205

Met Leu Val Ala Arg Ser Leu Glu Gln Asn Ile Phe Glu Leu Ile Asn
    210                 215                 220

Lys Ile Val Asn Arg Lys Arg Thr Glu Ser Leu Gln Ile Phe Tyr Asp
225                 230                 235                 240

Leu Leu Lys Gln Asn Glu Glu Pro Ile Lys Ile Met Ala Leu Ile Ser
                245                 250                 255

Asn Gln Phe Arg Leu Ile Leu Gln Thr Lys Tyr Phe Ala Glu Gln Gly
            260                 265                 270

Tyr Gly Gln Lys Gln Ile Ala Ser Asn Leu Lys Val His Pro Phe Arg
        275                 280                 285

Val Lys Leu Ala Met Asp Gln Ala Arg Leu Phe Ser Glu Glu Glu Leu
    290                 295                 300

Arg Leu Ile Ile Glu Gln Leu Ala Val Met Asp Tyr Glu Met Lys Thr
305                 310                 315                 320

Gly Lys Lys Asp Lys Gln Leu Leu Leu Glu Leu Phe Leu Leu Gln Leu
                325                 330                 335

Leu Lys Arg Asn Glu Lys Asn Asp Pro His Tyr
            340                 345

<210> 10
<211> 996
<212> DNA
<213> Buchnera

<400> 10
atgaaaaata tatatccaaa tgaactaaaa aaaagtttaa tgcaaaaatt gcattatttt 60

tatatttttt taggagaaga ttttttttta ttagaaaaaa atcaagatat gatattgaat 120

tttgcatata aaaaaggatt cctagaaaaa attataattg atgtagaaaa aaatcaagat 180

tggaaaaaaa ttattttatt ttataaaact aataatttat tttttaaaaa aactacttta 240

gtaatcaact ttcttataaa aaagttaaat gttattttaa ttcaaaatct taataaaatg 300

ttttctttat tgcatccaga tatattaata atattaaaat tcaatcattt atcacgtttt 360

attcaaaaaa ataaatcatt aaaagaattt aaaaactaca atatagtttc ttgttttacg 420

ccgtataatt taaattttat taattggatt aagtatgaaa tacaagaaaa aaaaataaat 480

attgaggaaa aagcattttt tttattatgt aaatattatg aaggtaatac tttatttata 540

tataaaattt tagatatgct atttataata tggcccgata catgtattac agaaaaaaaa 600

ataaaaaaaa ttatcattga gttttttgat gtttctccat catattggat caattctata 660

tttcaaggta aaacagaaaa atccttttat atattaaata ttttttttaa aaaaaaatat 720

aatcctctta ttttagtacg ttctttgcaa aaagatttgt tacaattgat tcatatgaag 780

cgtgaaaaaa aaataagtat ttatgtaatg ttagaaaaat ataatatttt tgtaacaaga 840

cgtaagtttt ttatcaaggc atttaataaa attaacaacg atagtttatt aaaagctatt 900

caaattcttg taaaaataga agtaaatatc aaaaaaaaat ataataatta tgtttggaat 960

caactacaag aattaatttt aatattatgt aactaa 996


<210> 11
<211> 996
<212> DNA
<213> Buchnera

<220>
<221> CDS
<222> (1)..(996)

<400> 11
atg aaa aat ata tat cca aat gaa cta aaa aaa agt tta atg caa aaa 48
Met Lys Asn Ile Tyr Pro Asn Glu Leu Lys Lys Ser Leu Met Gln Lys

1                 5                   10                  15

ttg cat tat ttt tat att ttt tta gga gaa gat ttt ttt tta tta gaa    96
Leu His Tyr Phe Tyr Ile Phe Leu Gly Glu Asp Phe Phe Leu Leu Glu
            20                  25                  30

aaa aat caa gat atg ata ttg aat ttt gca tat aaa aaa gga ttc cta    144
Lys Asn Gln Asp Met Ile Leu Asn Phe Ala Tyr Lys Lys Gly Phe Leu
        35                  40                  45

gaa aaa att ata att gat gta gaa aaa aat caa gat tgg aaa aaa att    192
Glu Lys Ile Ile Ile Asp Val Glu Lys Asn Gln Asp Trp Lys Lys Ile
    50                  55                  60

att tta ttt tat aaa act aat aat tta ttt ttt aaa aaa act act tta    240
Ile Leu Phe Tyr Lys Thr Asn Asn Leu Phe Phe Lys Lys Thr Thr Leu
65                  70                  75                  80

gta atc aac ttt ctt ata aaa aag tta aat gtt att tta att caa aat    288
Val Ile Asn Phe Leu Ile Lys Lys Leu Asn Val Ile Leu Ile Gln Asn
                85                  90                  95

ctt aat aaa atg ttt tct tta ttg cat cca gat ata tta ata ata tta    336
Leu Asn Lys Met Phe Ser Leu Leu His Pro Asp Ile Leu Ile Ile Leu
            100                 105                 110

aaa ttc aat cat tta tca cgt ttt att caa aaa aat aaa tca tta aaa    384
Lys Phe Asn His Leu Ser Arg Phe Ile Gln Lys Asn Lys Ser Leu Lys
        115                 120                 125

gaa ttt aaa aac tac aat ata gtt tct tgt ttt acg ccg tat aat tta    432
Glu Phe Lys Asn Tyr Asn Ile Val Ser Cys Phe Thr Pro Tyr Asn Leu
    130                 135                 140

aat ttt att aat tgg att aag tat gaa ata caa gaa aaa aaa ata aat    480
Asn Phe Ile Asn Trp Ile Lys Tyr Glu Ile Gln Glu Lys Lys Ile Asn
145                 150                 155                 160

att gag gaa aaa gca ttt ttt tta tta tgt aaa tat tat gaa ggt aat    528
Ile Glu Glu Lys Ala Phe Phe Leu Leu Cys Lys Tyr Tyr Glu Gly Asn
                165                 170                 175

act tta ttt ata tat aaa att tta gat atg cta ttt ata ata tgg ccc    576
Thr Leu Phe Ile Tyr Lys Ile Leu Asp Met Leu Phe Ile Ile Trp Pro
            180                 185                 190

gat aca tgt att aca gaa aaa aaa ata aaa aaa att atc att gag ttt    624
Asp Thr Cys Ile Thr Glu Lys Lys Ile Lys Lys Ile Ile Ile Glu Phe

195                 200                 205

ttt gat gtt tct cca tca tat tgg atc aat tct ata ttt caa ggt aaa     672
Phe Asp Val Ser Pro Ser Tyr Trp Ile Asn Ser Ile Phe Gln Gly Lys
    210                 215                 220

aca gaa aaa tcc ttt tat ata tta aat att ttt ttt aaa aaa aaa tat     720
Thr Glu Lys Ser Phe Tyr Ile Leu Asn Ile Phe Phe Lys Lys Lys Tyr
225                 230                 235                 240

aat cct ctt att tta gta cgt tct ttg caa aaa gat ttg tta caa ttg     768
Asn Pro Leu Ile Leu Val Arg Ser Leu Gln Lys Asp Leu Leu Gln Leu
                245                 250                 255

att cat atg aag cgt gaa aaa aaa ata agt att tat gta atg tta gaa     816
Ile His Met Lys Arg Glu Lys Lys Ile Ser Ile Tyr Val Met Leu Glu
            260                 265                 270

aaa tat aat att ttt gta aca aga cgt aag ttt ttt atc aag gca ttt     864
Lys Tyr Asn Ile Phe Val Thr Arg Arg Lys Phe Phe Ile Lys Ala Phe
        275                 280                 285

aat aaa att aac aac gat agt tta tta aaa gct att caa att ctt gta     912
Asn Lys Ile Asn Asn Asp Ser Leu Leu Lys Ala Ile Gln Ile Leu Val
    290                 295                 300

aaa ata gaa gta aat atc aaa aaa aaa tat aat aat tat gtt tgg aat     960
Lys Ile Glu Val Asn Ile Lys Lys Lys Tyr Asn Asn Tyr Val Trp Asn
305                 310                 315                 320

caa cta caa gaa tta att tta ata tta tgt aac taa                     996
Gln Leu Gln Glu Leu Ile Leu Ile Leu Cys Asn
                325                 330

<210> 12
<211> 331
<212> PRT
<213> Buchnera

<400> 12
Met Lys Asn Ile Tyr Pro Asn Glu Leu Lys Lys Ser Leu Met Gln Lys
1               5                   10                  15

Leu His Tyr Phe Tyr Ile Phe Leu Gly Glu Asp Phe Phe Leu Leu Glu
            20                  25                  30

Lys Asn Gln Asp Met Ile Leu Asn Phe Ala Tyr Lys Lys Gly Phe Leu

        35                  40                  45

Glu Lys Ile Ile Ile Asp Val Glu Lys Asn Gln Asp Trp Lys Lys Ile
    50                  55                  60

Ile Leu Phe Tyr Lys Thr Asn Asn Leu Phe Phe Lys Lys Thr Thr Leu
65                  70                  75                  80

Val Ile Asn Phe Leu Ile Lys Lys Leu Asn Val Ile Leu Ile Gln Asn
                85                  90                  95

Leu Asn Lys Met Phe Ser Leu Leu His Pro Asp Ile Leu Ile Ile Leu
            100                 105                 110

Lys Phe Asn His Leu Ser Arg Phe Ile Gln Lys Asn Lys Ser Leu Lys
        115                 120                 125

Glu Phe Lys Asn Tyr Asn Ile Val Ser Cys Phe Thr Pro Tyr Asn Leu
    130                 135                 140

Asn Phe Ile Asn Trp Ile Lys Tyr Glu Ile Gln Glu Lys Lys Ile Asn
145                 150                 155                 160

Ile Glu Glu Lys Ala Phe Phe Leu Leu Cys Lys Tyr Tyr Glu Gly Asn
                165                 170                 175

Thr Leu Phe Ile Tyr Lys Ile Leu Asp Met Leu Phe Ile Ile Trp Pro
            180                 185                 190

Asp Thr Cys Ile Thr Glu Lys Lys Ile Lys Lys Ile Ile Ile Glu Phe
        195                 200                 205

Phe Asp Val Ser Pro Ser Tyr Trp Ile Asn Ser Ile Phe Gln Gly Lys
    210                 215                 220

Thr Glu Lys Ser Phe Tyr Ile Leu Asn Ile Phe Phe Lys Lys Lys Tyr
225                 230                 235                 240

Asn Pro Leu Ile Leu Val Arg Ser Leu Gln Lys Asp Leu Leu Gln Leu
                245                 250                 255

Ile His Met Lys Arg Glu Lys Lys Ile Ser Ile Tyr Val Met Leu Glu
            260                 265                 270

Lys Tyr Asn Ile Phe Val Thr Arg Arg Lys Phe Phe Ile Lys Ala Phe
        275                 280                 285

Asn Lys Ile Asn Asn Asp Ser Leu Leu Lys Ala Ile Gln Ile Leu Val

290                 295                 300

Lys Ile Glu Val Asn Ile Lys Lys Lys Tyr Asn Asn Tyr Val Trp Asn
305                 310                 315                 320

Gln Leu Gln Glu Leu Ile Leu Ile Leu Cys Asn
                325                 330

<210> 13
<211> 990
<212> DNA
<213> Borrelia burgdorferi

<400> 13
atgcaagcgg tttatttatt gttgggtaat gagcaaggtt taaaagaagc ctatttaaaa 60

gagcttttaa ttaaaatgga tgcttttaaa tccgaagttt cagttactaa aatttttttg 120

tcagaactct cagctgtagg atttgctgag aaattatttt ccaattcttt tttttcaaaa 180

aaagaaattt ttattgttta tgagtctgaa cttttaaaag caggaaaaga tttagagcta 240

gtgtgtaatt caatcttaaa gtctaacaat aaaactgtta tttttgtttc caatagcaat 300

acatgtaaca ttgattttaa gaataagctt aagtttataa aaaaagtttt ttatgagatt 360

cctgatgatg ataaatttac atttgtaaaa agaaattttt ttaatcttaa tattaaaatt 420

acagattctg caataaattt aatgctttta atgttaaatt cagatactaa aattttgaaa 480

ttttatatag attcttttgc gctttttgcc aagaataata ccattgagga ggaagatata 540

gcttcttgga ttagttttat tcgctttgag aacacctttt ccttatttaa ttcaattttg 600

agaaaagata tgactcagtc tttgatcaag attaagtcta ttttggatca gggagaagat 660

ttgcttaata ttttaatgag tcttatttgg caatttaaaa gattattaaa ggtgcaaata 720

gattataatg catatgggag cttgcagagt gcattgaata aaaataaaat ctttttttca 780

ttaaataaaa tttatagagt aggaattaaa aattattcaa ttccagaaat taaaattgtt 840

ttgaaaattt tatataagtt tgatttatat ttgagaattt attctaaaaa tattcatcaa 900

aatttgtcat atttttaat attctcaatt ttaaagctta ataataattt tttaatgcat 960

tattctacag aatcaaaatt taatttttaa                                  990

<210> 14
<211> 990
<212> DNA
<213> Borrelia burgdorferi

<220>
<221> CDS
<222> (1)..(990)

<400> 14
atg caa gcg gtt tat tta ttg ttg ggt aat gag caa ggt tta aaa gaa     48
Met Gln Ala Val Tyr Leu Leu Leu Gly Asn Glu Gln Gly Leu Lys Glu
1               5                   10                  15

gcc tat tta aaa gag ctt tta att aaa atg gat gct ttt aaa tcc gaa     96
Ala Tyr Leu Lys Glu Leu Leu Ile Lys Met Asp Ala Phe Lys Ser Glu
            20                  25                  30

gtt tca gtt act aaa att ttt ttg tca gaa ctc tca gct gta gga ttt     144
Val Ser Val Thr Lys Ile Phe Leu Ser Glu Leu Ser Ala Val Gly Phe
        35                  40                  45

gct gag aaa tta ttt tcc aat tct ttt ttt tca aaa aaa gaa att ttt     192
Ala Glu Lys Leu Phe Ser Asn Ser Phe Phe Ser Lys Lys Glu Ile Phe
    50                  55                  60

att gtt tat gag tct gaa ctt tta aaa gca gga aaa gat tta gag cta     240
Ile Val Tyr Glu Ser Glu Leu Leu Lys Ala Gly Lys Asp Leu Glu Leu
65                  70                  75                  80

gtg tgt aat tca atc tta aag tct aac aat aaa act gtt att ttt gtt     288
Val Cys Asn Ser Ile Leu Lys Ser Asn Asn Lys Thr Val Ile Phe Val
                85                  90                  95

tcc aat agc aat aca tgt aac att gat ttt aag aat aag ctt aag ttt     336
Ser Asn Ser Asn Thr Cys Asn Ile Asp Phe Lys Asn Lys Leu Lys Phe
            100                 105                 110

ata aaa aaa gtt ttt tat gag att cct gat gat gat aaa ttt aca ttt     384
Ile Lys Lys Val Phe Tyr Glu Ile Pro Asp Asp Asp Lys Phe Thr Phe
        115                 120                 125

gta aaa aga aat ttt ttt aat ctt aat att aaa att aca gat tct gca     432
Val Lys Arg Asn Phe Phe Asn Leu Asn Ile Lys Ile Thr Asp Ser Ala
    130                 135                 140

ata aat tta atg ctt tta atg tta aat tca gat act aaa att ttg aaa 480
Ile Asn Leu Met Leu Leu Met Leu Asn Ser Asp Thr Lys Ile Leu Lys
145                 150                 155                 160

ttt tat ata gat tct ttt gcg ctt ttt gcc aag aat aat acc att gag 528
Phe Tyr Ile Asp Ser Phe Ala Leu Phe Ala Lys Asn Asn Thr Ile Glu
                165                 170                 175

gag gaa gat ata gct tct tgg att agt ttt att cgc ttt gag aac acc 576
Glu Glu Asp Ile Ala Ser Trp Ile Ser Phe Ile Arg Phe Glu Asn Thr
            180                 185                 190

ttt tcc tta ttt aat tca att ttg aga aaa gat atg act cag tct ttg 624
Phe Ser Leu Phe Asn Ser Ile Leu Arg Lys Asp Met Thr Gln Ser Leu
        195                 200                 205

atc aag att aag tct att ttg gat cag gga gaa gat ttg ctt aat att 672
Ile Lys Ile Lys Ser Ile Leu Asp Gln Gly Glu Asp Leu Leu Asn Ile
    210                 215                 220

tta atg agt ctt att tgg caa ttt aaa aga tta tta aag gtg caa ata 720
Leu Met Ser Leu Ile Trp Gln Phe Lys Arg Leu Leu Lys Val Gln Ile
225                 230                 235                 240

gat tat aat gca tat ggg agc ttg cag agt gca ttg aat aaa aat aaa 768
Asp Tyr Asn Ala Tyr Gly Ser Leu Gln Ser Ala Leu Asn Lys Asn Lys
                245                 250                 255

atc ttt ttt tca tta aat aaa att tat aga gta gga att aaa aat tat 816
Ile Phe Phe Ser Leu Asn Lys Ile Tyr Arg Val Gly Ile Lys Asn Tyr
            260                 265                 270

tca att cca gaa att aaa att gtt ttg aaa att tta tat aag ttt gat 864
Ser Ile Pro Glu Ile Lys Ile Val Leu Lys Ile Leu Tyr Lys Phe Asp
        275                 280                 285

tta tat ttg aga att tat tct aaa aat att cat caa aat ttg tca tat 912
Leu Tyr Leu Arg Ile Tyr Ser Lys Asn Ile His Gln Asn Leu Ser Tyr
    290                 295                 300

ttt tta ata ttc tca att tta aag ctt aat aat aat ttt tta atg cat 960
Phe Leu Ile Phe Ser Ile Leu Lys Leu Asn Asn Asn Phe Leu Met His
305                 310                 315                 320

tat tct aca gaa tca aaa ttt aat ttt taa 990
Tyr Ser Thr Glu Ser Lys Phe Asn Phe
325 330

<210> 15
<211> 329
<212> PRT
<213> Borrelia burgdorferi

<400> 15
Met Gln Ala Val Tyr Leu Leu Leu Gly Asn Glu Gln Gly Leu Lys Glu
1               5                   10                  15

Ala Tyr Leu Lys Glu Leu Leu Ile Lys Met Asp Ala Phe Lys Ser Glu
            20                  25                  30

Val Ser Val Thr Lys Ile Phe Leu Ser Glu Leu Ser Ala Val Gly Phe
        35                  40                  45

Ala Glu Lys Leu Phe Ser Asn Ser Phe Phe Ser Lys Lys Glu Ile Phe
    50                  55                  60

Ile Val Tyr Glu Ser Glu Leu Leu Lys Ala Gly Lys Asp Leu Glu Leu
65                  70                  75                  80

Val Cys Asn Ser Ile Leu Lys Ser Asn Asn Lys Thr Val Ile Phe Val
                85                  90                  95

Ser Asn Ser Asn Thr Cys Asn Ile Asp Phe Lys Asn Lys Leu Lys Phe
            100                 105                 110

Ile Lys Lys Val Phe Tyr Glu Ile Pro Asp Asp Asp Lys Phe Thr Phe
        115                 120                 125

Val Lys Arg Asn Phe Phe Asn Leu Asn Ile Lys Ile Thr Asp Ser Ala
    130                 135                 140

Ile Asn Leu Met Leu Leu Met Leu Asn Ser Asp Thr Lys Ile Leu Lys
145                 150                 155                 160

Phe Tyr Ile Asp Ser Phe Ala Leu Phe Ala Lys Asn Asn Thr Ile Glu
                165                 170                 175

Glu Glu Asp Ile Ala Ser Trp Ile Ser Phe Ile Arg Phe Glu Asn Thr
            180                 185                 190

Phe Ser Leu Phe Asn Ser Ile Leu Arg Lys Asp Met Thr Gln Ser Leu
        195                 200                 205

Ile Lys Ile Lys Ser Ile Leu Asp Gln Gly Glu Asp Leu Leu Asn Ile
    210                 215                 220

Leu Met Ser Leu Ile Trp Gln Phe Lys Arg Leu Leu Lys Val Gln Ile
225                 230                 235                 240

Asp Tyr Asn Ala Tyr Gly Ser Leu Gln Ser Ala Leu Asn Lys Asn Lys
                245                 250                 255

Ile Phe Phe Ser Leu Asn Lys Ile Tyr Arg Val Gly Ile Lys Asn Tyr
            260                 265                 270

Ser Ile Pro Glu Ile Lys Ile Val Leu Lys Ile Leu Tyr Lys Phe Asp
        275                 280                 285

Leu Tyr Leu Arg Ile Tyr Ser Lys Asn Ile His Gln Asn Leu Ser Tyr
    290                 295                 300

Phe Leu Ile Phe Ser Ile Leu Lys Leu Asn Asn Asn Phe Leu Met His
305                 310                 315                 320

Tyr Ser Thr Glu Ser Lys Phe Asn Phe
                325

<210> 16
<211> 966
<212> DNA
<213> Campylobacter jejuni

<400> 16
atgtatagaa aagagcttca aatcttactt tctaaagatt caatacctaa tttttctttt 60

ctttatggag cagataattt tcaaagcgaa ctttatgctg aatttataaa agaaaaatat 120

aagcccgatg aaactttgaa actttttttt gaagaataca acttcactcg tgcgagtgat 180

tttttaagcg caggatcgct ttttagtgaa aaaaaattac tcgaaattaa aacttctaaa 240

aaaattccaa caaaagatct taaagtttta gtcgaacttt gtaaaaacaa tacggacaat 300

ttctttcttt tagagcttta tgatgaaagt tctaaacaaa gcgatataga aaaaattttc 360

tctcctcatt ttgtaagatt ttttaaagct aacggagcta aagaaggcgt ggagctttta 420

agcataaaag caaaacaact aggggttgaa atcactcaaa atgctttatt tactcttttt 480

acaagttttg atgaaaactt atatcttgct gcgagtgagc ttaataaatt tagtggttta 540

agagtagatg aaaaaaccat agaacaatac tgctatagtt taaatacagg aagttttgaa 600

agcttttttg ataaaatttt aaaaaaacaa gactttaaaa gcgaacttga aaaaatttta 660

gataatttta atgaaattgc tttaatcaac tctttatata actcttttta tcgtttattt 720

aaaattgcac tttatgctaa aatcaatggt aaaattgatt ttaaagagct tttaggctac 780

actccacccc ctcaagtggg acaaaattta agctctcaag cctttagttt aaaaatcgaa 840

caatataaag aaattttcac tcttttgctt aaaagcgaat acgaacttaa aaccaatcct 900

aaactcgtaa aaaaagaatt tttgatttca aatttactta aacttgcaag gattttaaaa 960

aattaa 966

<210> 17
<211> 966
<212> DNA
<213> Campylobacter jejuni

<220>
<221> CDS
<222> (1)..(966)

<400> 17
atg tat aga aaa gag ctt caa atc tta ctt tct aaa gat tca ata cct 48
Met Tyr Arg Lys Glu Leu Gln Ile Leu Leu Ser Lys Asp Ser Ile Pro
1               5                   10                  15

aat ttt ttc ttt ctt tat gga gca gat aat ttt caa agc gaa ctt tat 96
Asn Phe Phe Phe Leu Tyr Gly Ala Asp Asn Phe Gln Ser Glu Leu Tyr
            20                  25                  30

gct gaa ttt ata aaa gaa aaa tat aag ccc gat gaa act ttg aaa ctt 144
Ala Glu Phe Ile Lys Glu Lys Tyr Lys Pro Asp Glu Thr Leu Lys Leu
        35                  40                  45

ttt ttt gaa gaa tac aac ttc act cgt gcg agt gat ttt tta agc gca 192
Phe Phe Glu Glu Tyr Asn Phe Thr Arg Ala Ser Asp Phe Leu Ser Ala
    50                  55                  60

gga tcg ctt ttt agt gaa aaa aaa tta ctc gaa att aaa act tct aaa 240
Gly Ser Leu Phe Ser Glu Lys Lys Leu Leu Glu Ile Lys Thr Ser Lys
65                  70                  75                  80

aaa att cca aca aaa gat ctt aaa gtt tta gtc gaa ctt tgt aaa aac 288
Lys Ile Pro Thr Lys Asp Leu Lys Val Leu Val Glu Leu Cys Lys Asn

                85                  90                  95

aat acg gac aat ttc ttt ctt tta gag ctt tat gat gaa agt tct aaa 336
Asn Thr Asp Asn Phe Phe Leu Leu Glu Leu Tyr Asp Glu Ser Ser Lys
            100                 105                 110

caa agc gat ata gaa aaa att ttc tct cct cat ttt gta aga ttt ttt 384
Gln Ser Asp Ile Glu Lys Ile Phe Ser Pro His Phe Val Arg Phe Phe
        115                 120                 125

aaa gct aac gga gct aaa gaa ggc gtg gag ctt tta agc ata aaa gca 432
Lys Ala Asn Gly Ala Lys Glu Gly Val Glu Leu Leu Ser Ile Lys Ala
    130                 135                 140

aaa caa cta ggg gtt gaa atc act caa aat gct tta ttt act ctt ttt 480
Lys Gln Leu Gly Val Glu Ile Thr Gln Asn Ala Leu Phe Thr Leu Phe
145                 150                 155                 160

aca agt ttt gat gaa aac tta tat ctt gct gcg agt gag ctt aat aaa 528
Thr Ser Phe Asp Glu Asn Leu Tyr Leu Ala Ala Ser Glu Leu Asn Lys
                165                 170                 175

ttt agt ggt tta aga gta gat gaa aaa acc ata gaa caa tac tgc tat 576
Phe Ser Gly Leu Arg Val Asp Glu Lys Thr Ile Glu Gln Tyr Cys Tyr
            180                 185                 190

agt tta aat aca gga agt ttt gaa agc ttt ttt gat aaa att tta aaa 624
Ser Leu Asn Thr Gly Ser Phe Glu Ser Phe Phe Asp Lys Ile Leu Lys
        195                 200                 205

aaa caa gac ttt aaa agc gaa ctt gaa aaa att tta gat aat ttt aat 672
Lys Gln Asp Phe Lys Ser Glu Leu Glu Lys Ile Leu Asp Asn Phe Asn
    210                 215                 220

gaa att gct tta atc aac tct tta tat aac tct ttt tat cgt tta ttt 720
Glu Ile Ala Leu Ile Asn Ser Leu Tyr Asn Ser Phe Tyr Arg Leu Phe
225                 230                 235                 240

aaa att gca ctt tat gct aaa atc aat ggt aaa att gat ttt aaa gag 768
Lys Ile Ala Leu Tyr Ala Lys Ile Asn Gly Lys Ile Asp Phe Lys Glu
                245                 250                 255

ctt tta ggc tac act cca ccc cct caa gtg gga caa aat tta agc tct 816
Leu Leu Gly Tyr Thr Pro Pro Pro Gln Val Gly Gln Asn Leu Ser Ser
            260                 265                 270

caa gcc ttt agt tta aaa atc gaa caa tat aaa gaa att ttc act ctt 864
Gln Ala Phe Ser Leu Lys Ile Glu Gln Tyr Lys Glu Ile Phe Thr Leu

        275                 280                 285

ttg ctt aaa agc gaa tac gaa ctt aaa acc aat cct aaa ctc gta aaa 912
Leu Leu Lys Ser Glu Tyr Glu Leu Lys Thr Asn Pro Lys Leu Val Lys
    290                 295                 300

aaa gaa ttt ttg att tca aat tta ctt aaa ctt gca agg att tta aaa 960
Lys Glu Phe Leu Ile Ser Asn Leu Leu Lys Leu Ala Arg Ile Leu Lys
305                 310                 315                 320

aat taa 966
Asn

<210> 18
<211> 321
<212> PRT
<213> Campylobacter jejuni

<400> 18
Met Tyr Arg Lys Glu Leu Gln Ile Leu Leu Ser Lys Asp Ser Ile Pro
1               5                   10                  15

Asn Phe Phe Phe Leu Tyr Gly Ala Asp Asn Phe Gln Ser Glu Leu Tyr
            20                  25                  30

Ala Glu Phe Ile Lys Glu Lys Tyr Lys Pro Asp Glu Thr Leu Lys Leu
        35                  40                  45

Phe Phe Glu Glu Tyr Asn Phe Thr Arg Ala Ser Asp Phe Leu Ser Ala
    50                  55                  60

Gly Ser Leu Phe Ser Glu Lys Lys Leu Leu Glu Ile Lys Thr Ser Lys
65                  70                  75                  80

Lys Ile Pro Thr Lys Asp Leu Lys Val Leu Val Glu Leu Cys Lys Asn
                85                  90                  95

Asn Thr Asp Asn Phe Phe Leu Leu Glu Leu Tyr Asp Glu Ser Ser Lys
            100                 105                 110

Gln Ser Asp Ile Glu Lys Ile Phe Ser Pro His Phe Val Arg Phe Phe
        115                 120                 125

Lys Ala Asn Gly Ala Lys Glu Gly Val Glu Leu Leu Ser Ile Lys Ala
    130                 135                 140

Lys Gln Leu Gly Val Glu Ile Thr Gln Asn Ala Leu Phe Thr Leu Phe

145                 150                 155                 160

Thr Ser Phe Asp Glu Asn Leu Tyr Leu Ala Ala Ser Glu Leu Asn Lys
                165                 170                 175

Phe Ser Gly Leu Arg Val Asp Glu Lys Thr Ile Glu Gln Tyr Cys Tyr
            180                 185                 190

Ser Leu Asn Thr Gly Ser Phe Glu Ser Phe Phe Asp Lys Ile Leu Lys
        195                 200                 205

Lys Gln Asp Phe Lys Ser Glu Leu Glu Lys Ile Leu Asp Asn Phe Asn
    210                 215                 220

Glu Ile Ala Leu Ile Asn Ser Leu Tyr Asn Ser Phe Tyr Arg Leu Phe
225                 230                 235                 240

Lys Ile Ala Leu Tyr Ala Lys Ile Asn Gly Lys Ile Asp Phe Lys Glu
                245                 250                 255

Leu Leu Gly Tyr Thr Pro Pro Pro Gln Val Gly Gln Asn Leu Ser Ser
            260                 265                 270

Gln Ala Phe Ser Leu Lys Ile Glu Gln Tyr Lys Glu Ile Phe Thr Leu
        275                 280                 285

Leu Leu Lys Ser Glu Tyr Glu Leu Lys Thr Asn Pro Lys Leu Val Lys
    290                 295                 300

Lys Glu Phe Leu Ile Ser Asn Leu Leu Lys Leu Ala Arg Ile Leu Lys
305                 310                 315                 320

Asn

<210> 19
<211> 1053
<212> DNA
<213> Caulobacter crescentus

<400> 19
atgatcctgt ccaagcgtcc ggacgtcgaa cgcttcctca aggagccgac cccggacatc 60

cgggccgtag tgatctacgg caaggatcgg ggcgtcgtgc gggaccgggc caatgcgctg 120

gcccggcgcg tggtcgagcg cccggatgat cccttcgata cggcgcaact gaccgacggc 180

gatctcgacg ccgacccggc caagcttgag gacgaactgt cggcgatgtc gctgatgggg 240

gggcggcgcc tggtgcgcct gcgactgtcc agcgacaagg ctggccccga caagcaggcc 300

gccgaagccc tgacccgcca tgtcgagggt caactcaatc cggacgcctt ctttctggtc 360

gaggccggcg cgctgggcag agactcacag cttcgcaagg tcggcgagaa ggccaacggc 420

tgcgccgtca tcccctgtta cgaggacgag gccggggacc tagcccgcct gacgcgcgac 480

accctggcca acgacaaggt cagcgtgaac agcgaggcgc tggaactctt cgtttcgcgc 540

ctacccaagg aacggggcgt cgcccgcgcc gagatcgaac ggctggccct ctacctcggc 600

ccgggcagcg gtatcaacgc caccgccgca gacctgaccg acttcctggg cgtcgaaccc 660

gaggcctccc ttagtgacgc cgccgccgat gcgttcggcg gaaaggtcgg cgcggcccag 720

caggcgcttc gccgcgccgc cgccgagggg gaaggcggac cggccgccgt cagggcgatg 780

ggctactatc tgggtcgcct gcgtcgcgtc ctgaccctgc acaagaacgg cgtcgacctg 840

caagccgccg ccaaggccgc cggcgtgttc tggaagcagg agcgagaatt cctccgtcag 900

gcgcgagcct ggaacctgga ggccattctc gagatccagg gcgagatcct gaccgccgac 960

cgagcctgca agacgtcggg ctcccccgat caactgatcg ccgagcgtct ggccctgatg 1020

atcgccggcc gcgcccgccg actgggcctg tag 1053

<210> 20
<211> 1053
<212> DNA
<213> Caulobacter crescentus

<220>
<221> CDS
<222> (1)..(1053)

<400> 20
atg atc ctg tcc aag cgt ccg gac gtc gaa cgc ttc ctc aag gag ccg 48
Met Ile Leu Ser Lys Arg Pro Asp Val Glu Arg Phe Leu Lys Glu Pro
1               5                   10                  15

acc ccg gac atc cgg gcc gta gtg atc tac ggc aag gat cgg ggc gtc 96
Thr Pro Asp Ile Arg Ala Val Val Ile Tyr Gly Lys Asp Arg Gly Val
            20                  25                  30

gtg cgg gac cgg gcc aat gcg ctg gcc cgg cgc gtg gtc gag cgc ccg    144
Val Arg Asp Arg Ala Asn Ala Leu Ala Arg Arg Val Val Glu Arg Pro
            35                  40                  45

gat gat ccc ttc gat acg gcg caa ctg acc gac ggc gat ctc gac gcc    192
Asp Asp Pro Phe Asp Thr Ala Gln Leu Thr Asp Gly Asp Leu Asp Ala
        50                  55                  60

gac ccg gcc aag ctt gag gac gaa ctg tcg gcg atg tcg ctg atg ggg    240
Asp Pro Ala Lys Leu Glu Asp Glu Leu Ser Ala Met Ser Leu Met Gly
 65                  70                  75                  80

ggg cgg cgc ctg gtg cgc ctg cga ctg tcc agc gac aag gct ggc ccc    288
Gly Arg Arg Leu Val Arg Leu Arg Leu Ser Ser Asp Lys Ala Gly Pro
                 85                  90                  95

gac aag cag gcc gcc gaa gcc ctg acc cgc cat gtc gag ggt caa ctc    336
Asp Lys Gln Ala Ala Glu Ala Leu Thr Arg His Val Glu Gly Gln Leu
                100                 105                 110

aat ccg gac gcc ttc ttt ctg gtc gag gcc ggc gcg ctg ggc aga gac    384
Asn Pro Asp Ala Phe Phe Leu Val Glu Ala Gly Ala Leu Gly Arg Asp
            115                 120                 125

tca cag ctt cgc aag gtc ggc gag aag gcc aac ggc tgc gcc gtc atc    432
Ser Gln Leu Arg Lys Val Gly Glu Lys Ala Asn Gly Cys Ala Val Ile
    130                 135                 140

ccc tgt tac gag gac gag gcc ggg gac cta gcc cgc ctg acg cgc gac    480
Pro Cys Tyr Glu Asp Glu Ala Gly Asp Leu Ala Arg Leu Thr Arg Asp
145                 150                 155                 160

acc ctg gcc aac gac aag gtc agc gtg aac agc gag gcg ctg gaa ctc    528
Thr Leu Ala Asn Asp Lys Val Ser Val Asn Ser Glu Ala Leu Glu Leu
                165                 170                 175

ttc gtt tcg cgc cta ccc aag gaa cgg ggc gtc gcc cgc gcc gag atc    576
Phe Val Ser Arg Leu Pro Lys Glu Arg Gly Val Ala Arg Ala Glu Ile
            180                 185                 190

gaa cgg ctg gcc ctc tac ctc ggc ccg ggc agc ggt atc aac gcc acc    624
Glu Arg Leu Ala Leu Tyr Leu Gly Pro Gly Ser Gly Ile Asn Ala Thr
        195                 200                 205

gcc gca gac ctg acc gac ttc ctg ggc gtc gaa ccc gag gcc tcc ctt    672
Ala Ala Asp Leu Thr Asp Phe Leu Gly Val Glu Pro Glu Ala Ser Leu
    210                 215                 220

agt gac gcc gcc gcc gat gcg ttc ggc gga aag gtc ggc gcg gcc cag 720
Ser Asp Ala Ala Ala Asp Ala Phe Gly Gly Lys Val Gly Ala Ala Gln
225                 230                 235                 240

cag gcg ctt cgc cgc gcc gcc gcc gag ggg gaa ggc gga ccg gcc gcc 768
Gln Ala Leu Arg Arg Ala Ala Ala Glu Gly Glu Gly Gly Pro Ala Ala
                245                 250                 255

gtc agg gcg atg ggc tac tat ctg ggt cgc ctg cgt cgc gtc ctg acc 816
Val Arg Ala Met Gly Tyr Tyr Leu Gly Arg Leu Arg Arg Val Leu Thr
            260                 265                 270

ctg cac aag aac ggc gtc gac ctg caa gcc gcc gcc aag gcc gcc ggc 864
Leu His Lys Asn Gly Val Asp Leu Gln Ala Ala Ala Lys Ala Ala Gly
        275                 280                 285

gtg ttc tgg aag cag gag cga gaa ttc ctc cgt cag gcg cga gcc tgg 912
Val Phe Trp Lys Gln Glu Arg Glu Phe Leu Arg Gln Ala Arg Ala Trp
    290                 295                 300

aac ctg gag gcc att ctc gag atc cag ggc gag atc ctg acc gcc gac 960
Asn Leu Glu Ala Ile Leu Glu Ile Gln Gly Glu Ile Leu Thr Ala Asp
305                 310                 315                 320

cga gcc tgc aag acg tcg ggc tcc ccc gat caa ctg atc gcc gag cgt 1008
Arg Ala Cys Lys Thr Ser Gly Ser Pro Asp Gln Leu Ile Ala Glu Arg
                325                 330                 335

ctg gcc ctg atg atc gcc ggc cgc gcc cgc cga ctg ggc ctg tag 1053
Leu Ala Leu Met Ile Ala Gly Arg Ala Arg Arg Leu Gly Leu
            340                 345                 350

<210> 21
<211> 350
<212> PRT
<213> Caulobacter crescentus

<400> 21
Met Ile Leu Ser Lys Arg Pro Asp Val Glu Arg Phe Leu Lys Glu Pro
1               5                   10                  15

Thr Pro Asp Ile Arg Ala Val Val Ile Tyr Gly Lys Asp Arg Gly Val
            20                  25                  30

Val Arg Asp Arg Ala Asn Ala Leu Ala Arg Arg Val Val Glu Arg Pro
        35                  40                  45

Asp Asp Pro Phe Asp Thr Ala Gln Leu Thr Asp Gly Asp Leu Asp Ala
    50                  55                  60

Asp Pro Ala Lys Leu Glu Asp Glu Leu Ser Ala Met Ser Leu Met Gly
65                  70                  75                  80

Gly Arg Arg Leu Val Arg Leu Arg Leu Ser Ser Asp Lys Ala Gly Pro
                85                  90                  95

Asp Lys Gln Ala Ala Glu Ala Leu Thr Arg His Val Glu Gly Gln Leu
            100                 105                 110

Asn Pro Asp Ala Phe Phe Leu Val Glu Ala Gly Ala Leu Gly Arg Asp
        115                 120                 125

Ser Gln Leu Arg Lys Val Gly Glu Lys Ala Asn Gly Cys Ala Val Ile
    130                 135                 140

Pro Cys Tyr Glu Asp Glu Ala Gly Asp Leu Ala Arg Leu Thr Arg Asp
145                 150                 155                 160

Thr Leu Ala Asn Asp Lys Val Ser Val Asn Ser Glu Ala Leu Glu Leu
                165                 170                 175

Phe Val Ser Arg Leu Pro Lys Glu Arg Gly Val Ala Arg Ala Glu Ile
            180                 185                 190

Glu Arg Leu Ala Leu Tyr Leu Gly Pro Gly Ser Gly Ile Asn Ala Thr
        195                 200                 205

Ala Ala Asp Leu Thr Asp Phe Leu Gly Val Glu Pro Glu Ala Ser Leu
    210                 215                 220

Ser Asp Ala Ala Ala Asp Ala Phe Gly Gly Lys Val Gly Ala Ala Gln
225                 230                 235                 240

Gln Ala Leu Arg Arg Ala Ala Ala Glu Gly Glu Gly Gly Pro Ala Ala
                245                 250                 255

Val Arg Ala Met Gly Tyr Tyr Leu Gly Arg Leu Arg Arg Val Leu Thr
            260                 265                 270

Leu His Lys Asn Gly Val Asp Leu Gln Ala Ala Ala Lys Ala Ala Gly
        275                 280                 285

Val Phe Trp Lys Gln Glu Arg Glu Phe Leu Arg Gln Ala Arg Ala Trp
    290                 295                 300

Asn Leu Glu Ala Ile Leu Glu Ile Gln Gly Glu Ile Leu Thr Ala Asp
305                 310                 315                 320

Arg Ala Cys Lys Thr Ser Gly Ser Pro Asp Gln Leu Ile Ala Glu Arg
                325                 330                 335

Leu Ala Leu Met Ile Ala Gly Arg Ala Arg Arg Leu Gly Leu
            340                 345                 350

<210> 22
<211> 945
<212> DNA
<213> Chlamydia muridarum

<400> 22
atggaaacct gccaaaatag tgtccgcata gagtctataa aagactttat tcatcatttt 60

gaaaatcaac gctttaaagt gtttataatt ggctcttcat ttcccgaaga caaggatatt 120

tttctggaac tttatgtttc cggaagaggg cgactatttg atggacaacg tttagttaaa 180

caagagcttt tatcttggac tgataatttt gggttgttca cttcaaagga aactattggt 240

atttatcaag ttgagaagat gagctcctct ttacaagagt ttatcataag ctatgctcat 300

catcctaatc cgcatctcac attatttctt ttcacaagta aagcagagtt cttctcagct 360

ttttcatcga agctccctaa tgccctttgt ttatctctct ttggtgagta ttttgcagaa 420

cgcgactctc gtatagcaca ggtattgatc aagcgttcaa tggatgttca actgtcttgt 480

tctttagggg tcgctaagct tttcgtaagt aagttcccgc aggttggagt ttttgaaatt 540

tttagtgaat ttcaaaagtt gatttgtcaa attgggggca agagttgttt agaagcttca 600

gacgttcaat cgtttgtaga aaaaagagaa tctgcttctt tatggaaatt acgggatgct 660

cttcttcgta gagatctctc aacagggcaa agtttgatgc aatctctggt atctgatttg 720

ggagaagatc ctcttgctat tctcaatttt ttacgtagcc aatatttatc tgggcttcga 780

gcaatagcgg aacagtcgaa agatcgcaaa acacaaatct ttttcgcagc aggagagcag 840

gctcttttga atggattaaa cttatttttt catgcggaga gtttgattaa gaataacgtg 900

caagatccca ttctttcttt agaaaccatg attgcaagac tgtaa                945

<210> 23
<211> 945
<212> DNA
<213> Chlamydia muridarum

<220>
<221> CDS
<222> (1)..(945)

<400> 23
atg gaa acc tgc caa aat agt gtc cgc ata gag tct ata aaa gac ttt     48
Met Glu Thr Cys Gln Asn Ser Val Arg Ile Glu Ser Ile Lys Asp Phe
1               5                   10                  15

att cat cat ttt gaa aat caa cgc ttt aaa gtg ttt ata att ggc tct     96
Ile His His Phe Glu Asn Gln Arg Phe Lys Val Phe Ile Ile Gly Ser
            20                  25                  30

tca ttt ccc gaa gac aag gat att ttt ctg gaa ctt tat gtt tcc gga     144
Ser Phe Pro Glu Asp Lys Asp Ile Phe Leu Glu Leu Tyr Val Ser Gly
        35                  40                  45

aga ggg cga cta ttt gat gga caa cgt tta gtt aaa caa gag ctt tta     192
Arg Gly Arg Leu Phe Asp Gly Gln Arg Leu Val Lys Gln Glu Leu Leu
    50                  55                  60

tct tgg act gat aat ttt ggg ttg ttc act tca aag gaa act att ggt     240
Ser Trp Thr Asp Asn Phe Gly Leu Phe Thr Ser Lys Glu Thr Ile Gly
65                  70                  75                  80

att tat caa gtt gag aag atg agc tcc tct tta caa gag ttt atc ata     288
Ile Tyr Gln Val Glu Lys Met Ser Ser Ser Leu Gln Glu Phe Ile Ile
                85                  90                  95

agc tat gct cat cat cct aat ccg cat ctc aca tta ttt ctt ttc aca     336
Ser Tyr Ala His His Pro Asn Pro His Leu Thr Leu Phe Leu Phe Thr
            100                 105                 110

agt aaa gca gag ttc ttc tca gct ttt tca tcg aag ctc cct aat gcc     384
Ser Lys Ala Glu Phe Phe Ser Ala Phe Ser Ser Lys Leu Pro Asn Ala
        115                 120                 125

ctt tgt tta tct ctc ttt ggt gag tat ttt gca gaa cgc gac tct cgt     432
Leu Cys Leu Ser Leu Phe Gly Glu Tyr Phe Ala Glu Arg Asp Ser Arg
    130                 135                 140

ata gca cag gta ttg atc aag cgt tca atg gat gtt caa ctg tct tgt     480

Ile Ala Gln Val Leu Ile Lys Arg Ser Met Asp Val Gln Leu Ser Cys
145                 150                 155                 160

tct tta ggg gtc gct aag ctt ttc gta agt aag ttc ccg cag gtt gga     528
Ser Leu Gly Val Ala Lys Leu Phe Val Ser Lys Phe Pro Gln Val Gly
                165                 170                 175

gtt ttt gaa att ttt agt gaa ttt caa aag ttg att tgt caa att ggg     576
Val Phe Glu Ile Phe Ser Glu Phe Gln Lys Leu Ile Cys Gln Ile Gly
            180                 185                 190

ggc aag agt tgt tta gaa gct tca gac gtt caa tcg ttt gta gaa aaa     624
Gly Lys Ser Cys Leu Glu Ala Ser Asp Val Gln Ser Phe Val Glu Lys
        195                 200                 205

aga gaa tct gct tct tta tgg aaa tta cgg gat gct ctt ctt cgt aga     672
Arg Glu Ser Ala Ser Leu Trp Lys Leu Arg Asp Ala Leu Leu Arg Arg
    210                 215                 220

gat ctc tca aca ggg caa agt ttg atg caa tct ctg gta tct gat ttg     720
Asp Leu Ser Thr Gly Gln Ser Leu Met Gln Ser Leu Val Ser Asp Leu
225                 230                 235                 240

gga gaa gat cct ctt gct att ctc aat ttt tta cgt agc caa tat tta     768
Gly Glu Asp Pro Leu Ala Ile Leu Asn Phe Leu Arg Ser Gln Tyr Leu
                245                 250                 255

tct ggg ctt cga gca ata gcg gaa cag tcg aaa gat cgc aaa aca caa     816
Ser Gly Leu Arg Ala Ile Ala Glu Gln Ser Lys Asp Arg Lys Thr Gln
            260                 265                 270

atc ttt ttc gca gca gga gag cag gct ctt ttg aat gga tta aac tta     864
Ile Phe Phe Ala Ala Gly Glu Gln Ala Leu Leu Asn Gly Leu Asn Leu
        275                 280                 285

ttt ttt cat gcg gag agt ttg att aag aat aac gtg caa gat ccc att     912
Phe Phe His Ala Glu Ser Leu Ile Lys Asn Asn Val Gln Asp Pro Ile
    290                 295                 300

ctt tct tta gaa acc atg att gca aga ctg taa                         945
Leu Ser Leu Glu Thr Met Ile Ala Arg Leu
305                 310                 315

<210> 24
<211> 314
<212> PRT
<213> Chlamydia muridarum

<400> 24
Met Glu Thr Cys Gln Asn Ser Val Arg Ile Glu Ser Ile Lys Asp Phe
1               5                   10                  15

Ile His His Phe Glu Asn Gln Arg Phe Lys Val Phe Ile Ile Gly Ser
            20                  25                  30

Ser Phe Pro Glu Asp Lys Asp Ile Phe Leu Glu Leu Tyr Val Ser Gly
        35                  40                  45

Arg Gly Arg Leu Phe Asp Gly Gln Arg Leu Val Lys Gln Glu Leu Leu
    50                  55                  60

Ser Trp Thr Asp Asn Phe Gly Leu Phe Thr Ser Lys Glu Thr Ile Gly
65                  70                  75                  80

Ile Tyr Gln Val Glu Lys Met Ser Ser Ser Leu Gln Glu Phe Ile Ile
                85                  90                  95

Ser Tyr Ala His His Pro Asn Pro His Leu Thr Leu Phe Leu Phe Thr
            100                 105                 110

Ser Lys Ala Glu Phe Phe Ser Ala Phe Ser Ser Lys Leu Pro Asn Ala
        115                 120                 125

Leu Cys Leu Ser Leu Phe Gly Glu Tyr Phe Ala Glu Arg Asp Ser Arg
    130                 135                 140

Ile Ala Gln Val Leu Ile Lys Arg Ser Met Asp Val Gln Leu Ser Cys
145                 150                 155                 160

Ser Leu Gly Val Ala Lys Leu Phe Val Ser Lys Phe Pro Gln Val Gly
                165                 170                 175

Val Phe Glu Ile Phe Ser Glu Phe Gln Lys Leu Ile Cys Gln Ile Gly
            180                 185                 190

Gly Lys Ser Cys Leu Glu Ala Ser Asp Val Gln Ser Phe Val Glu Lys
        195                 200                 205

Arg Glu Ser Ala Ser Leu Trp Lys Leu Arg Asp Ala Leu Leu Arg Arg
    210                 215                 220

Asp Leu Ser Thr Gly Gln Ser Leu Met Gln Ser Leu Val Ser Asp Leu
225                 230                 235                 240

Gly Glu Asp Pro Leu Ala Ile Leu Asn Phe Leu Arg Ser Gln Tyr Leu

                245                 250                 255

Ser Gly Leu Arg Ala Ile Ala Glu Gln Ser Lys Asp Arg Lys Thr Gln
            260                 265                 270

Ile Phe Phe Ala Ala Gly Glu Gln Ala Leu Leu Asn Gly Leu Asn Leu
        275                 280                 285

Phe Phe His Ala Glu Ser Leu Ile Lys Asn Asn Val Gln Asp Pro Ile
    290                 295                 300

Leu Ser Leu Glu Thr Met Ile Ala Arg Leu
305                 310

<210> 25
<211> 945
<212> DNA
<213> Chlamydia trachomatis

<400> 25
atgggaaaca gccagaatag tattcacata acgtctacta aagattttgt tcagtatatt 60

gaacgtgaac gatttagagt gattgtgatt ggctcttcat ctcttgagga caaagatatc 120

ttttcggaac tttatatttc tggaaggaag agcttttttg acggacagcg tttgttgcaa 180

caagagctct tatcttggac ggatcatttt ggtttgttcg cttcccaaga gactataggc 240

atttatcaag cagagaaaat gagctcttct ctacaagagt ttattataga ttacactcgg 300

catcctaacc cgaatcttac gttgttcctt ttcacaaata aagcagagct gttctcttct 360

ttatcttcga agctttctaa tgctctttgt ttatctttat ttggagaata ttttgctgag 420

cgagatgctc gcatagctca ggttttgata aaacgttcta aagagtcgca gctttcttgc 480

tctttaggag ttgcaaagat ttttgtgagt aagtttcctc agacaggttt atttgaaatt 540

cttagtgaat ttcaaaagtt aatttgtcag atgggagaca aggaatctat agaagcttcg 600

gatattcaat catttgtaga gaaaaaagag gcgatctctc tgtggaagtt gcgagatgct 660

cttcttcgta aagaccgtgt cgctgcacac agtctgatga gatctttggt gtctgatatg 720

ggagaggagc ctctggcgat tctcaacttt ttacgtagtc agtacctctt aggactgagg 780

gcagtggcag agcagtcgaa agagcgtaaa acacaaatat ttattgctgc aggagagccg 840

gctcttttaa atggtttgaa cctgtttttc catacggaaa gtttaattaa gaataacttc 900

caagatccca tgctttcatt agaaatgcta gtttcacgac tttag 945

<210> 26
<211> 945
<212> DNA
<213> Chlamydia trachomatis

<220>
<221> CDS
<222> (1)..(945)

<400> 26
atg gga aac agc cag aat agt att cac ata acg tct act aaa gat ttt 48
Met Gly Asn Ser Gln Asn Ser Ile His Ile Thr Ser Thr Lys Asp Phe
1               5                   10                  15

gtt cag tat att gaa cgt gaa cga ttt aga gtg att gtg att ggc tct 96
Val Gln Tyr Ile Glu Arg Glu Arg Phe Arg Val Ile Val Ile Gly Ser
            20                  25                  30

tca tct ctt gag gac aaa gat atc ttt tcg gaa ctt tat att tct gga 144
Ser Ser Leu Glu Asp Lys Asp Ile Phe Ser Glu Leu Tyr Ile Ser Gly
        35                  40                  45

agg aag agc ttt ttt gac gga cag cgt ttg ttg caa caa gag ctc tta 192
Arg Lys Ser Phe Phe Asp Gly Gln Arg Leu Leu Gln Gln Glu Leu Leu
    50                  55                  60

tct tgg acg gat cat ttt ggt ttg ttc gct tcc caa gag act ata ggc 240
Ser Trp Thr Asp His Phe Gly Leu Phe Ala Ser Gln Glu Thr Ile Gly
65                  70                  75                  80

att tat caa gca gag aaa atg agc tct tct cta caa gag ttt att ata 288
Ile Tyr Gln Ala Glu Lys Met Ser Ser Ser Leu Gln Glu Phe Ile Ile
                85                  90                  95

gat tac act cgg cat cct aac ccg aat ctt acg ttg ttc ctt ttc aca 336
Asp Tyr Thr Arg His Pro Asn Pro Asn Leu Thr Leu Phe Leu Phe Thr
            100                 105                 110

aat aaa gca gag ctg ttc tct tct tta tct tcg aag ctt tct aat gct 384
Asn Lys Ala Glu Leu Phe Ser Ser Leu Ser Ser Lys Leu Ser Asn Ala
        115                 120                 125

ctt tgt tta tct tta ttt gga gaa tat ttt gct gag cga gat gct cgc     432
Leu Cys Leu Ser Leu Phe Gly Glu Tyr Phe Ala Glu Arg Asp Ala Arg
    130                 135                 140

ata gct cag gtt ttg ata aaa cgt tct aaa gag tcg cag ctt tct tgc     480
Ile Ala Gln Val Leu Ile Lys Arg Ser Lys Glu Ser Gln Leu Ser Cys
145                 150                 155                 160

tct tta gga gtt gca aag att ttt gtg agt aag ttt cct cag aca ggt     528
Ser Leu Gly Val Ala Lys Ile Phe Val Ser Lys Phe Pro Gln Thr Gly
                165                 170                 175

tta ttt gaa att ctt agt gaa ttt caa aag tta att tgt cag atg gga     576
Leu Phe Glu Ile Leu Ser Glu Phe Gln Lys Leu Ile Cys Gln Met Gly
            180                 185                 190

gac aag gaa tct ata gaa gct tcg gat att caa tca ttt gta gag aaa     624
Asp Lys Glu Ser Ile Glu Ala Ser Asp Ile Gln Ser Phe Val Glu Lys
        195                 200                 205

aaa gag gcg atc tct ctg tgg aag ttg cga gat gct ctt ctt cgt aaa     672
Lys Glu Ala Ile Ser Leu Trp Lys Leu Arg Asp Ala Leu Leu Arg Lys
    210                 215                 220

gac cgt gtc gct gca cac agt ctg atg aga tct ttg gtg tct gat atg     720
Asp Arg Val Ala Ala His Ser Leu Met Arg Ser Leu Val Ser Asp Met
225                 230                 235                 240

gga gag gag cct ctg gcg att ctc aac ttt tta cgt agt cag tac ctc     768
Gly Glu Glu Pro Leu Ala Ile Leu Asn Phe Leu Arg Ser Gln Tyr Leu
                245                 250                 255

tta gga ctg agg gca gtg gca gag cag tcg aaa gag cgt aaa aca caa     816
Leu Gly Leu Arg Ala Val Ala Glu Gln Ser Lys Glu Arg Lys Thr Gln
            260                 265                 270

ata ttt att gct gca gga gag ccg gct ctt tta aat ggt ttg aac ctg     864
Ile Phe Ile Ala Ala Gly Glu Pro Ala Leu Leu Asn Gly Leu Asn Leu
        275                 280                 285

ttt ttc cat acg gaa agt tta att aag aat aac ttc caa gat ccc atg     912
Phe Phe His Thr Glu Ser Leu Ile Lys Asn Asn Phe Gln Asp Pro Met
    290                 295                 300

ctt tca tta gaa atg cta gtt tca cga ctt tag                         945
Leu Ser Leu Glu Met Leu Val Ser Arg Leu
305                 310                 315

<210> 27
<211> 314
<212> PRT
<213> Chlamydia trachomatis

<400> 27
Met Gly Asn Ser Gln Asn Ser Ile His Ile Thr Ser Thr Lys Asp Phe
1               5                   10                  15

Val Gln Tyr Ile Glu Arg Glu Arg Phe Arg Val Ile Val Ile Gly Ser
            20                  25                  30

Ser Ser Leu Glu Asp Lys Asp Ile Phe Ser Glu Leu Tyr Ile Ser Gly
        35                  40                  45

Arg Lys Ser Phe Phe Asp Gly Gln Arg Leu Leu Gln Gln Glu Leu Leu
    50                  55                  60

Ser Trp Thr Asp His Phe Gly Leu Phe Ala Ser Gln Glu Thr Ile Gly
65                  70                  75                  80

Ile Tyr Gln Ala Glu Lys Met Ser Ser Ser Leu Gln Glu Phe Ile Ile
                85                  90                  95

Asp Tyr Thr Arg His Pro Asn Pro Asn Leu Thr Leu Phe Leu Phe Thr
            100                 105                 110

Asn Lys Ala Glu Leu Phe Ser Ser Leu Ser Ser Lys Leu Ser Asn Ala
        115                 120                 125

Leu Cys Leu Ser Leu Phe Gly Glu Tyr Phe Ala Glu Arg Asp Ala Arg
    130                 135                 140

Ile Ala Gln Val Leu Ile Lys Arg Ser Lys Glu Ser Gln Leu Ser Cys
145                 150                 155                 160

Ser Leu Gly Val Ala Lys Ile Phe Val Ser Lys Phe Pro Gln Thr Gly
                165                 170                 175

Leu Phe Glu Ile Leu Ser Glu Phe Gln Lys Leu Ile Cys Gln Met Gly
            180                 185                 190

Asp Lys Glu Ser Ile Glu Ala Ser Asp Ile Gln Ser Phe Val Glu Lys
        195                 200                 205

Lys Glu Ala Ile Ser Leu Trp Lys Leu Arg Asp Ala Leu Leu Arg Lys
    210                 215                 220

Asp Arg Val Ala Ala His Ser Leu Met Arg Ser Leu Val Ser Asp Met
225                 230                 235                 240

Gly Glu Glu Pro Leu Ala Ile Leu Asn Phe Leu Arg Ser Gln Tyr Leu
                245                 250                 255

Leu Gly Leu Arg Ala Val Ala Glu Gln Ser Lys Glu Arg Lys Thr Gln
            260                 265                 270

Ile Phe Ile Ala Ala Gly Glu Pro Ala Leu Leu Asn Gly Leu Asn Leu
        275                 280                 285

Phe Phe His Thr Glu Ser Leu Ile Lys Asn Asn Phe Gln Asp Pro Met
    290                 295                 300

Leu Ser Leu Glu Met Leu Val Ser Arg Leu
305                 310

<210> 28
<211> 948
<212> DNA
<213> Chlamydophila pneumonia

<400> 28
atgactctac ctatgcaaaa atccttaacg agttttgatg acttttccca ggcgtatgca 60

gagaaagtgc ccgctatagc tcttataggg agtgctttgg aagacgataa agatgcgctg 120

attgaattat tagtctctga gagcttcaaa gagctcggtg gtcagggact catgccagca 180

accctcatgt cttggaccga gacgtttgca ctctttcaag agcatgaaac tttggggatt 240

attcatgcag agaaattccc tctagcaact aaggaatttc taagccgcta tgctcggaat 300

cctcaacctc accttacgat tttgatcttc accacaaaac aagaatgctt tcgagaactg 360

tcaaaagcct tgccatcggc tctttctttg agtttatttg gtgagtggcc cgcagatcgt 420

cagaaaagga tcatacgcct cctgttgcaa agagctgagc gtgtggggat ttcttgctct 480

caatcattgg catctttgtt tttgcgtgca cttgcttcaa cctctcttcc tgatattctc 540

agtgaattcg ataagctact gtgctctgtt ggcaagaaaa cgtccttgga tcactctgat 600

attaaagagc tcgttgtcaa aaaagaaaag gcttccctat ggaaatttcg agactctcta 660

ttgaagaggg atccggtaga aggtcaccag cagttgcatt ttctactcga ggatggtgaa 720

gatcccttgg ggattattac tttccttcgt acccaatgtc tctatggttt acgtagtatt 780

gaagagggat cgaaagaaaa taaacaccga atgttcgtcc tttatggaaa ggagagacta 840

caccaagcct taaattcttt attttatgca gaaaccttaa ttaaaaataa tgtccaagat 900

cctatagtag ctgtggaaac tttagtgatt agaatggtta acctgtga 948

<210> 29
<211> 948
<212> DNA
<213> Chlamydophila pneumonia

<220>
<221> CDS
<222> (1)..(948)

<400> 29
atg act cta cct atg caa aaa tcc tta acg agt ttt gat gac ttt tcc 48
Met Thr Leu Pro Met Gln Lys Ser Leu Thr Ser Phe Asp Asp Phe Ser
1               5                   10                  15

cag gcg tat gca gag aaa gtg ccc gct ata gct ctt ata ggg agt gct 96
Gln Ala Tyr Ala Glu Lys Val Pro Ala Ile Ala Leu Ile Gly Ser Ala
            20                  25                  30

ttg gaa gac gat aaa gat gcg ctg att gaa tta tta gtc tct gag agc 144
Leu Glu Asp Asp Lys Asp Ala Leu Ile Glu Leu Leu Val Ser Glu Ser
        35                  40                  45

ttc aaa gag ctc ggt ggt cag gga ctc atg cca gca acc ctc atg tct 192
Phe Lys Glu Leu Gly Gly Gln Gly Leu Met Pro Ala Thr Leu Met Ser
    50                  55                  60

tgg acc gag acg ttt gca ctc ttt caa gag cat gaa act ttg ggg att 240
Trp Thr Glu Thr Phe Ala Leu Phe Gln Glu His Glu Thr Leu Gly Ile
65                  70                  75                  80

att cat gca gag aaa ttc cct cta gca act aag gaa ttt cta agc cgc 288
Ile His Ala Glu Lys Phe Pro Leu Ala Thr Lys Glu Phe Leu Ser Arg
                85                  90                  95

tat gct cgg aat cct caa cct cac ctt acg att ttg atc ttc acc aca 336
Tyr Ala Arg Asn Pro Gln Pro His Leu Thr Ile Leu Ile Phe Thr Thr
            100                 105                 110

aaa caa gaa tgc ttt cga gaa ctg tca aaa gcc ttg cca tcg gct ctt 384
Lys Gln Glu Cys Phe Arg Glu Leu Ser Lys Ala Leu Pro Ser Ala Leu
        115                 120                 125

tct ttg agt tta ttt ggt gag tgg ccc gca gat cgt cag aaa agg atc 432
Ser Leu Ser Leu Phe Gly Glu Trp Pro Ala Asp Arg Gln Lys Arg Ile
    130                 135                 140

ata cgc ctc ctg ttg caa aga gct gag cgt gtg ggg att tct tgc tct 480
Ile Arg Leu Leu Leu Gln Arg Ala Glu Arg Val Gly Ile Ser Cys Ser
145                 150                 155                 160

caa tca ttg gca tct ttg ttt ttg cgt gca ctt gct tca acc tct ctt 528
Gln Ser Leu Ala Ser Leu Phe Leu Arg Ala Leu Ala Ser Thr Ser Leu
                165                 170                 175

cct gat att ctc agt gaa ttc gat aag cta ctg tgc tct gtt ggc aag 576
Pro Asp Ile Leu Ser Glu Phe Asp Lys Leu Leu Cys Ser Val Gly Lys
            180                 185                 190

aaa acg tcc ttg gat cac tct gat att aaa gag ctc gtt gtc aaa aaa 624
Lys Thr Ser Leu Asp His Ser Asp Ile Lys Glu Leu Val Val Lys Lys
        195                 200                 205

gaa aag gct tcc cta tgg aaa ttt cga gac tct cta ttg aag agg gat 672
Glu Lys Ala Ser Leu Trp Lys Phe Arg Asp Ser Leu Leu Lys Arg Asp
    210                 215                 220

ccg gta gaa ggt cac cag cag ttg cat ttt cta ctc gag gat ggt gaa 720
Pro Val Glu Gly His Gln Gln Leu His Phe Leu Leu Glu Asp Gly Glu
225                 230                 235                 240

gat ccc ttg ggg att att act ttc ctt cgt acc caa tgt ctc tat ggt 768
Asp Pro Leu Gly Ile Ile Thr Phe Leu Arg Thr Gln Cys Leu Tyr Gly
                245                 250                 255

tta cgt agt att gaa gag gga tcg aaa gaa aat aaa cac cga atg ttc 816
Leu Arg Ser Ile Glu Glu Gly Ser Lys Glu Asn Lys His Arg Met Phe
            260                 265                 270

gtc ctt tat gga aag gag aga cta cac caa gcc tta aat tct tta ttt 864
Val Leu Tyr Gly Lys Glu Arg Leu His Gln Ala Leu Asn Ser Leu Phe
        275                 280                 285

tat gca gaa acc tta att aaa aat aat gtc caa gat cct ata gta gct 912
Tyr Ala Glu Thr Leu Ile Lys Asn Asn Val Gln Asp Pro Ile Val Ala
    290                 295                 300

gtg gaa act tta gtg att aga atg gtt aac ctg tga                    948
Val Glu Thr Leu Val Ile Arg Met Val Asn Leu
305                 310                 315

<210> 30
<211> 315
<212> PRT
<213> Chlamydophila pneumonia

<400> 30
Met Thr Leu Pro Met Gln Lys Ser Leu Thr Ser Phe Asp Asp Phe Ser
1               5                   10                  15

Gln Ala Tyr Ala Glu Lys Val Pro Ala Ile Ala Leu Ile Gly Ser Ala
            20                  25                  30

Leu Glu Asp Asp Lys Asp Ala Leu Ile Glu Leu Leu Val Ser Glu Ser
        35                  40                  45

Phe Lys Glu Leu Gly Gly Gln Gly Leu Met Pro Ala Thr Leu Met Ser
    50                  55                  60

Trp Thr Glu Thr Phe Ala Leu Phe Gln Glu His Glu Thr Leu Gly Ile
65                  70                  75                  80

Ile His Ala Glu Lys Phe Pro Leu Ala Thr Lys Glu Phe Leu Ser Arg
                85                  90                  95

Tyr Ala Arg Asn Pro Gln Pro His Leu Thr Ile Leu Ile Phe Thr Thr
            100                 105                 110

Lys Gln Glu Cys Phe Arg Glu Leu Ser Lys Ala Leu Pro Ser Ala Leu
        115                 120                 125

Ser Leu Ser Leu Phe Gly Glu Trp Pro Ala Asp Arg Gln Lys Arg Ile
    130                 135                 140

Ile Arg Leu Leu Leu Gln Arg Ala Glu Arg Val Gly Ile Ser Cys Ser
145                 150                 155                 160

Gln Ser Leu Ala Ser Leu Phe Leu Arg Ala Leu Ala Ser Thr Ser Leu
                165                 170                 175

Pro Asp Ile Leu Ser Glu Phe Asp Lys Leu Leu Cys Ser Val Gly Lys
            180                 185                 190

Lys Thr Ser Leu Asp His Ser Asp Ile Lys Glu Leu Val Val Lys Lys
        195                 200                 205

Glu Lys Ala Ser Leu Trp Lys Phe Arg Asp Ser Leu Leu Lys Arg Asp
    210                 215                 220

Pro Val Glu Gly His Gln Gln Leu His Phe Leu Leu Glu Asp Gly Glu
225                 230                 235                 240

Asp Pro Leu Gly Ile Ile Thr Phe Leu Arg Thr Gln Cys Leu Tyr Gly
                245                 250                 255

Leu Arg Ser Ile Glu Glu Gly Ser Lys Glu Asn Lys His Arg Met Phe
            260                 265                 270

Val Leu Tyr Gly Lys Glu Arg Leu His Gln Ala Leu Asn Ser Leu Phe
        275                 280                 285

Tyr Ala Glu Thr Leu Ile Lys Asn Asn Val Gln Asp Pro Ile Val Ala
    290                 295                 300

Val Glu Thr Leu Val Ile Arg Met Val Asn Leu
305                 310                 315

<210> 31
<211> 903
<212> DNA
<213> Deinococcus radiodurans

<400> 31
atgccagtcc tcgcctttac cggcaaccgt tttctggctg acgaaaccct gcgcgacacg 60

ctgagtgcgc gtgggctcaa cgcccgtgac ctgccgcgct tttccggcga ggacgtgagc 120

gccgagacgc tggggccgca cctcgcgccg agtctctttg gcgacggcgg cgtagtcgtc 180

gatttcgagg gcctcaagcc cgacaaggcc ctgctggagc tgctttcctc ggcgcccgtc 240

acggtggccg tgctcgacga ggcgccgccc gcaacccggc tcaagctcta tcagaaggcg 300

ggcgaggtta tcccctcggc ggccccgagt aagcccggcg acgtgaccgg ctgggtggtc 360

acgcgggcca aaaaaatggg cctgcggttg gagcgcgacg cggcgagcta tctggccgag 420

gtgttcggtg ccgacctggc gggcatcgcg ggcgagttga acaagctgga gctgctcggc 480

ggcgcgctga atcgagaacg ggtgcagggc atcgtgggcc gcgatccgcc cggcgactcg 540

tttgccatgc tcggcgcggc gacggcgggc cgccccggtg aagcggtgct gcaactgcgc 600

cgcctgctcg gctcgggcga agaccccttc cggctgctcg gcgcggtggt gtggcagtac 660

agcctgattg cccgcacggt cggcctgctc caggaggaag gccgcgtgaa tgacgcggtg 720

gccgctcagc gcctgggcgt caagcccttt ccggcgaaaa aggcgctgga agtcgcgcgg 780

cgcctgagtg aaagccggat tcgcagccac ctgggcgcca ttctcgatgc cgacctcgcc 840

atgaagcgcg ggctggaccc gcaagtcgcc cttgagcggc tgctggtcaa gctgagcgta 900

tag 903

<210> 32
<211> 903
<212> DNA
<213> Deinococcus radiodurans

<220>
<221> CDS
<222> (1)..(903)

<400> 32
atg cca gtc ctc gcc ttt acc ggc aac cgt ttt ctg gct gac gaa acc 48
Met Pro Val Leu Ala Phe Thr Gly Asn Arg Phe Leu Ala Asp Glu Thr
1               5                   10                  15

ctg cgc gac acg ctg agt gcg cgt ggg ctc aac gcc cgt gac ctg ccg 96
Leu Arg Asp Thr Leu Ser Ala Arg Gly Leu Asn Ala Arg Asp Leu Pro
            20                  25                  30

cgc ttt tcc ggc gag gac gtg agc gcc gag acg ctg ggg ccg cac ctc 144
Arg Phe Ser Gly Glu Asp Val Ser Ala Glu Thr Leu Gly Pro His Leu
        35                  40                  45

gcg ccg agt ctc ttt ggc gac ggc ggc gta gtc gtc gat ttc gag ggc 192
Ala Pro Ser Leu Phe Gly Asp Gly Gly Val Val Val Asp Phe Glu Gly
    50                  55                  60

ctc aag ccc gac aag gcc ctg ctg gag ctg ctt tcc tcg gcg ccc gtc 240
Leu Lys Pro Asp Lys Ala Leu Leu Glu Leu Leu Ser Ser Ala Pro Val
65                  70                  75                  80

acg gtg gcc gtg ctc gac gag gcg ccg ccc gca acc cgg ctc aag ctc 288
Thr Val Ala Val Leu Asp Glu Ala Pro Pro Ala Thr Arg Leu Lys Leu

                85                  90                  95

tat cag aag gcg ggc gag gtt atc ccc tcg gcg gcc ccg agt aag ccc 336
Tyr Gln Lys Ala Gly Glu Val Ile Pro Ser Ala Ala Pro Ser Lys Pro
            100                 105                 110

ggc gac gtg acc ggc tgg gtg gtc acg cgg gcc aaa aaa atg ggc ctg 384
Gly Asp Val Thr Gly Trp Val Val Thr Arg Ala Lys Lys Met Gly Leu
        115                 120                 125

cgg ttg gag cgc gac gcg gcg agc tat ctg gcc gag gtg ttc ggt gcc 432
Arg Leu Glu Arg Asp Ala Ala Ser Tyr Leu Ala Glu Val Phe Gly Ala
    130                 135                 140

gac ctg gcg ggc atc gcg ggc gag ttg aac aag ctg gag ctg ctc ggc 480
Asp Leu Ala Gly Ile Ala Gly Glu Leu Asn Lys Leu Glu Leu Leu Gly
145                 150                 155                 160

ggc gcg ctg aat cga gaa cgg gtg cag ggc atc gtg ggc cgc gat ccg 528
Gly Ala Leu Asn Arg Glu Arg Val Gln Gly Ile Val Gly Arg Asp Pro
                165                 170                 175

ccc ggc gac tcg ttt gcc atg ctc ggc gcg gcg acg gcg ggc cgc ccc 576
Pro Gly Asp Ser Phe Ala Met Leu Gly Ala Ala Thr Ala Gly Arg Pro
            180                 185                 190

ggt gaa gcg gtg ctg caa ctg cgc cgc ctg ctc ggc tcg ggc gaa gac 624
Gly Glu Ala Val Leu Gln Leu Arg Arg Leu Leu Gly Ser Gly Glu Asp
        195                 200                 205

ccc ttc cgg ctg ctc ggc gcg gtg gtg tgg cag tac agc ctg att gcc 672
Pro Phe Arg Leu Leu Gly Ala Val Val Trp Gln Tyr Ser Leu Ile Ala
    210                 215                 220

cgc acg gtc ggc ctg ctc cag gag gaa ggc cgc gtg aat gac gcg gtg 720
Arg Thr Val Gly Leu Leu Gln Glu Glu Gly Arg Val Asn Asp Ala Val
225                 230                 235                 240

gcc gct cag cgc ctg ggc gtc aag ccc ttt ccg gcg aaa aag gcg ctg 768
Ala Ala Gln Arg Leu Gly Val Lys Pro Phe Pro Ala Lys Lys Ala Leu
                245                 250                 255

gaa gtc gcg cgg cgc ctg agt gaa agc cgg att cgc agc cac ctg ggc 816
Glu Val Ala Arg Arg Leu Ser Glu Ser Arg Ile Arg Ser His Leu Gly
            260                 265                 270

gcc att ctc gat gcc gac ctc gcc atg aag cgc ggg ctg gac ccg caa 864
Ala Ile Leu Asp Ala Asp Leu Ala Met Lys Arg Gly Leu Asp Pro Gln

275                 280                 285

gtc gcc ctt gag cgg ctg ctg gtc aag ctg agc gta tag                 903
Val Ala Leu Glu Arg Leu Leu Val Lys Leu Ser Val
    290                 295                 300

<210> 33
<211> 300
<212> PRT
<213> Deinococcus radiodurans

<400> 33
Met Pro Val Leu Ala Phe Thr Gly Asn Arg Phe Leu Ala Asp Glu Thr
1               5                   10                  15

Leu Arg Asp Thr Leu Ser Ala Arg Gly Leu Asn Ala Arg Asp Leu Pro
            20                  25                  30

Arg Phe Ser Gly Glu Asp Val Ser Ala Glu Thr Leu Gly Pro His Leu
        35                  40                  45

Ala Pro Ser Leu Phe Gly Asp Gly Gly Val Val Val Asp Phe Glu Gly
    50                  55                  60

Leu Lys Pro Asp Lys Ala Leu Leu Glu Leu Leu Ser Ser Ala Pro Val
65                  70                  75                  80

Thr Val Ala Val Leu Asp Glu Ala Pro Pro Ala Thr Arg Leu Lys Leu
                85                  90                  95

Tyr Gln Lys Ala Gly Glu Val Ile Pro Ser Ala Ala Pro Ser Lys Pro
            100                 105                 110

Gly Asp Val Thr Gly Trp Val Val Thr Arg Ala Lys Lys Met Gly Leu
        115                 120                 125

Arg Leu Glu Arg Asp Ala Ala Ser Tyr Leu Ala Glu Val Phe Gly Ala
    130                 135                 140

Asp Leu Ala Gly Ile Ala Gly Glu Leu Asn Lys Leu Glu Leu Leu Gly
145                 150                 155                 160

Gly Ala Leu Asn Arg Glu Arg Val Gln Gly Ile Val Gly Arg Asp Pro
                165                 170                 175

Pro Gly Asp Ser Phe Ala Met Leu Gly Ala Ala Thr Ala Gly Arg Pro
            180                 185                 190

Gly Glu Ala Val Leu Gln Leu Arg Arg Leu Leu Gly Ser Gly Glu Asp
        195                 200                 205

Pro Phe Arg Leu Leu Gly Ala Val Val Trp Gln Tyr Ser Leu Ile Ala
    210                 215                 220

Arg Thr Val Gly Leu Leu Gln Glu Glu Gly Arg Val Asn Asp Ala Val
225                 230                 235                 240

Ala Ala Gln Arg Leu Gly Val Lys Pro Phe Pro Ala Lys Lys Ala Leu
                245                 250                 255

Glu Val Ala Arg Arg Leu Ser Glu Ser Arg Ile Arg Ser His Leu Gly
            260                 265                 270

Ala Ile Leu Asp Ala Asp Leu Ala Met Lys Arg Gly Leu Asp Pro Gln
        275                 280                 285

Val Ala Leu Glu Arg Leu Leu Val Lys Leu Ser Val
    290                 295                 300

<210> 34
<211> 1035
<212> DNA
<213> Haemophilus influenzae

<400> 34
atgaaccgca tttttccaga gcaactaaat catcatcttg cgcaaggctt ggctagagtt 60

tatctcttgc aagggcaaga tcctttatta cttagtgaaa ctgaggatac tatttgtcaa 120

gtggcaaacc tgcaaggttt tgatgaaaaa aatactattc aggttgatag ccaaactgat 180

tgggcgcaac ttatagaatc ttgtcaatct ataggattgt tttttagtaa acaaatacta 240

agcttgaatt tacctgaaaa tttcaccgca cttttgcaga aaaatctgca agaacttata 300

tccgtattgc ataaagatgt cttgcttatt ttacaggtag caaaattggc taaagggatt 360

gaaaagcaaa cgtggtttat tacacttaat caatatgaac caaatacaat actgataaat 420

tgtcaaacac cgacggtaga aaacttacca cgttgggtaa aaaatcgtac caaagcaatg 480

ggattagacg ctgacaacga agcaatccaa caactttgtt atagttatga aaataacctg 540

ctcgcactca aacaagctct gcagctttta gatttgctct atcctgacca taaacttaac 600

tacaatcgtg ttattagcgt ggtggaacaa tcctcaattt ttacaccatt tcaatggatt 660

gatgcgttgc ttgttggaaa ggcaaatcgt gctaaacgta ttttaaaagg tttacaagct 720

gaagatgtgc aacctgttat tttattacgc acactacagc gagaattatt tacgttgctt 780

gaactcacaa aaccacagca acgtattgtg acaacggaaa aacttccaat tcaacaaatt 840

aagactgaat ttgaccgtct aaaaatctgg caaaatcgcc gaccgctctt tttaagtgcg 900

attcaacgtt taacttacca gactctctac gaaattatcc aagaactcgc taatattgaa 960

cgccttgcta aacaagaatt tagtgatgaa gtgtggatca aactcgctga tttatctgta 1020

aaaatttgtt tgtag 1035

<210> 35
<211> 1035
<212> DNA
<213> Haemophilus influenzae

<220>
<221> CDS
<222> (1)..(1035)

<400> 35
atg aac cgc att ttt cca gag caa cta aat cat cat ctt gcg caa ggc 48
Met Asn Arg Ile Phe Pro Glu Gln Leu Asn His His Leu Ala Gln Gly
1               5                   10                  15

ttg gct aga gtt tat ctc ttg caa ggg caa gat cct tta tta ctt agt 96
Leu Ala Arg Val Tyr Leu Leu Gln Gly Gln Asp Pro Leu Leu Leu Ser
            20                  25                  30

gaa act gag gat act att tgt caa gtg gca aac ctg caa ggt ttt gat 144
Glu Thr Glu Asp Thr Ile Cys Gln Val Ala Asn Leu Gln Gly Phe Asp
        35                  40                  45

gaa aaa aat act att cag gtt gat agc caa act gat tgg gcg caa ctt 192
Glu Lys Asn Thr Ile Gln Val Asp Ser Gln Thr Asp Trp Ala Gln Leu
    50                  55                  60

ata gaa tct tgt caa tct ata gga ttg ttt ttt agt aaa caa ata cta 240
Ile Glu Ser Cys Gln Ser Ile Gly Leu Phe Phe Ser Lys Gln Ile Leu
65                  70                  75                  80

agc ttg aat tta cct gaa aat ttc acc gca ctt ttg cag aaa aat ctg 288
Ser Leu Asn Leu Pro Glu Asn Phe Thr Ala Leu Leu Gln Lys Asn Leu
                85                  90                  95

caa gaa ctt ata tcc gta ttg cat aaa gat gtc ttg ctt att tta cag 336
Gln Glu Leu Ile Ser Val Leu His Lys Asp Val Leu Leu Ile Leu Gln
            100                 105                 110

gta gca aaa ttg gct aaa ggg att gaa aag caa acg tgg ttt att aca 384
Val Ala Lys Leu Ala Lys Gly Ile Glu Lys Gln Thr Trp Phe Ile Thr
        115                 120                 125

ctt aat caa tat gaa cca aat aca ata ctg ata aat tgt caa aca ccg 432
Leu Asn Gln Tyr Glu Pro Asn Thr Ile Leu Ile Asn Cys Gln Thr Pro
    130                 135                 140

acg gta gaa aac tta cca cgt tgg gta aaa aat cgt acc aaa gca atg 480
Thr Val Glu Asn Leu Pro Arg Trp Val Lys Asn Arg Thr Lys Ala Met
145                 150                 155                 160

gga tta gac gct gac aac gaa gca atc caa caa ctt tgt tat agt tat 528
Gly Leu Asp Ala Asp Asn Glu Ala Ile Gln Gln Leu Cys Tyr Ser Tyr
                165                 170                 175

gaa aat aac ctg ctc gca ctc aaa caa gct ctg cag ctt tta gat ttg 576
Glu Asn Asn Leu Leu Ala Leu Lys Gln Ala Leu Gln Leu Leu Asp Leu
            180                 185                 190

ctc tat cct gac cat aaa ctt aac tac aat cgt gtt att agc gtg gtg 624
Leu Tyr Pro Asp His Lys Leu Asn Tyr Asn Arg Val Ile Ser Val Val
        195                 200                 205

gaa caa tcc tca att ttt aca cca ttt caa tgg att gat gcg ttg ctt 672
Glu Gln Ser Ser Ile Phe Thr Pro Phe Gln Trp Ile Asp Ala Leu Leu
    210                 215                 220

gtt gga aag gca aat cgt gct aaa cgt att tta aaa ggt tta caa gct 720
Val Gly Lys Ala Asn Arg Ala Lys Arg Ile Leu Lys Gly Leu Gln Ala
225                 230                 235                 240

gaa gat gtg caa cct gtt att tta tta cgc aca cta cag cga gaa tta 768
Glu Asp Val Gln Pro Val Ile Leu Leu Arg Thr Leu Gln Arg Glu Leu
                245                 250                 255

ttt acg ttg ctt gaa ctc aca aaa cca cag caa cgt att gtg aca acg 816
Phe Thr Leu Leu Glu Leu Thr Lys Pro Gln Gln Arg Ile Val Thr Thr
            260                 265                 270

gaa aaa ctt cca att caa caa att aag act gaa ttt gac cgt cta aaa    864
Glu Lys Leu Pro Ile Gln Gln Ile Lys Thr Glu Phe Asp Arg Leu Lys
        275                 280                 285

atc tgg caa aat cgc cga ccg ctc ttt tta agt gcg att caa cgt tta    912
Ile Trp Gln Asn Arg Arg Pro Leu Phe Leu Ser Ala Ile Gln Arg Leu
    290                 295                 300

act tac cag act ctc tac gaa att atc caa gaa ctc gct aat att gaa    960
Thr Tyr Gln Thr Leu Tyr Glu Ile Ile Gln Glu Leu Ala Asn Ile Glu
305                 310                 315                 320

cgc ctt gct aaa caa gaa ttt agt gat gaa gtg tgg atc aaa ctc gct    1008
Arg Leu Ala Lys Gln Glu Phe Ser Asp Glu Val Trp Ile Lys Leu Ala
                325                 330                 335

gat tta tct gta aaa att tgt ttg tag                                1035
Asp Leu Ser Val Lys Ile Cys Leu
            340                 345

<210> 36
<211> 344
<212> PRT
<213> Haemophilus influenzae

<400> 36
Met Asn Arg Ile Phe Pro Glu Gln Leu Asn His His Leu Ala Gln Gly
  1               5                  10                  15

Leu Ala Arg Val Tyr Leu Leu Gln Gly Gln Asp Pro Leu Leu Leu Ser
             20                  25                  30

Glu Thr Glu Asp Thr Ile Cys Gln Val Ala Asn Leu Gln Gly Phe Asp
         35                  40                  45

Glu Lys Asn Thr Ile Gln Val Asp Ser Gln Thr Asp Trp Ala Gln Leu
     50                  55                  60

Ile Glu Ser Cys Gln Ser Ile Gly Leu Phe Phe Ser Lys Gln Ile Leu
 65                  70                  75                  80

Ser Leu Asn Leu Pro Glu Asn Phe Thr Ala Leu Leu Gln Lys Asn Leu
                 85                  90                  95

Gln Glu Leu Ile Ser Val Leu His Lys Asp Val Leu Leu Ile Leu Gln
            100                 105                 110

Val Ala Lys Leu Ala Lys Gly Ile Glu Lys Gln Thr Trp Phe Ile Thr
        115                 120                 125

Leu Asn Gln Tyr Glu Pro Asn Thr Ile Leu Ile Asn Cys Gln Thr Pro
    130                 135                 140

Thr Val Glu Asn Leu Pro Arg Trp Val Lys Asn Arg Thr Lys Ala Met
145                 150                 155                 160

Gly Leu Asp Ala Asp Asn Glu Ala Ile Gln Gln Leu Cys Tyr Ser Tyr
                165                 170                 175

Glu Asn Asn Leu Leu Ala Leu Lys Gln Ala Leu Gln Leu Leu Asp Leu
            180                 185                 190

Leu Tyr Pro Asp His Lys Leu Asn Tyr Asn Arg Val Ile Ser Val Val
        195                 200                 205

Glu Gln Ser Ser Ile Phe Thr Pro Phe Gln Trp Ile Asp Ala Leu Leu
    210                 215                 220

Val Gly Lys Ala Asn Arg Ala Lys Arg Ile Leu Lys Gly Leu Gln Ala
225                 230                 235                 240

Glu Asp Val Gln Pro Val Ile Leu Leu Arg Thr Leu Gln Arg Glu Leu
                245                 250                 255

Phe Thr Leu Leu Glu Leu Thr Lys Pro Gln Gln Arg Ile Val Thr Thr
            260                 265                 270

Glu Lys Leu Pro Ile Gln Gln Ile Lys Thr Glu Phe Asp Arg Leu Lys
        275                 280                 285

Ile Trp Gln Asn Arg Arg Pro Leu Phe Leu Ser Ala Ile Gln Arg Leu
    290                 295                 300

Thr Tyr Gln Thr Leu Tyr Glu Ile Ile Gln Glu Leu Ala Asn Ile Glu
305                 310                 315                 320

Arg Leu Ala Lys Gln Glu Phe Ser Asp Glu Val Trp Ile Lys Leu Ala
                325                 330                 335

Asp Leu Ser Val Lys Ile Cys Leu
            340

<210> 37
<211> 1023

<212> DNA
<213> Helicobacter pylori 26695

<400> 37
atgtatcgta aagatttgga ccattattta aaacaacgcc tccctaaagc ggtgtttttg 60

tatggggagt ttgatttttt tatccattat tatattcaaa cgattagcgc gctttttaaa 120

tgcgataacc ctgacataga aacttcgctt ttttatgcga gcgattatga aaaaagccag 180

attgcgaccc ttttagagca ggattcttta tttggaggga gcagtttagt cgttttaaaa 240

ctggattttg ccctgcataa gaaatttaag gaaaatgata tcaatctttt tttaaaagct 300

ttagagcggc ctagccataa caggcttatc atagggcttt ataatgctaa aagcgacacc 360

acaaaataca aatacactag cgatgctatc gttaaatttt tccaaaaaag ccccttgaaa 420

gatgaagcca tttgcgcgcg cttttttatc cctaaaactt gggagagttt gaaattcttg 480

caagaaaggg ctaatttttt gcatttagac atcagcggcc atcttttaaa cgctcttttt 540

gaaattaata acgaagattt aggcgtttcg tttaacgatt tagacaagct agcggtttta 600

aacgcaccca tcactttaga agacattcaa gaattaagct ccaatgcggg ggatatggat 660

ttgcaaaagc tcattttagg gcttttttta aaaaaaagtg cgcttgatat ttatgattat 720

ttgttaaaag agggcaaaaa agatgcggat attttaaggg ggttagagcg ctatttttac 780

caactttttt tatttttcgc tcatattaaa acgaccggtt taatggacgc taaagaggtt 840

ttaggctacg ctccccctaa agaaattgcc gaaaattacg ctaaaaacgc cttgcgtttg 900

aaagaagccg gctataagag ggtttttgaa atttttaggt tatggcacat tcaaagcatg 960

caagggcaaa aggaattggg ctttttgtat ttgacctcca ttcaaaaaat cattaacccc 1020

taa 1023


<210> 38
<211> 1023
<212> DNA
<213> Helicobacter pylori 26695

<220>
<221> CDS

<222> (1)..(1023)

<400> 38
atg tat cgt aaa gat ttg gac cat tat tta aaa caa cgc ctc cct aaa    48
Met Tyr Arg Lys Asp Leu Asp His Tyr Leu Lys Gln Arg Leu Pro Lys
1               5                   10                  15

gcg gtg ttt ttg tat ggg gag ttt gat ttt ttt atc cat tat tat att    96
Ala Val Phe Leu Tyr Gly Glu Phe Asp Phe Phe Ile His Tyr Tyr Ile
            20                  25                  30

caa acg att agc gcg ctt ttt aaa tgc gat aac cct gac ata gaa act    144
Gln Thr Ile Ser Ala Leu Phe Lys Cys Asp Asn Pro Asp Ile Glu Thr
        35                  40                  45

tcg ctt ttt tat gcg agc gat tat gaa aaa agc cag att gcg acc ctt    192
Ser Leu Phe Tyr Ala Ser Asp Tyr Glu Lys Ser Gln Ile Ala Thr Leu
    50                  55                  60

tta gag cag gat tct tta ttt gga ggg agc agt tta gtc gtt tta aaa    240
Leu Glu Gln Asp Ser Leu Phe Gly Gly Ser Ser Leu Val Val Leu Lys
65                  70                  75                  80

ctg gat ttt gcc ctg cat aag aaa ttt aag gaa aat gat atc aat ctt    288
Leu Asp Phe Ala Leu His Lys Lys Phe Lys Glu Asn Asp Ile Asn Leu
                85                  90                  95

ttt tta aaa gct tta gag cgg cct agc cat aac agg ctt atc ata ggg    336
Phe Leu Lys Ala Leu Glu Arg Pro Ser His Asn Arg Leu Ile Ile Gly
            100                 105                 110

ctt tat aat gct aaa agc gac acc aca aaa tac aaa tac act agc gat    384
Leu Tyr Asn Ala Lys Ser Asp Thr Thr Lys Tyr Lys Tyr Thr Ser Asp
        115                 120                 125

gct atc gtt aaa ttt ttc caa aaa agc ccc ttg aaa gat gaa gcc att    432
Ala Ile Val Lys Phe Phe Gln Lys Ser Pro Leu Lys Asp Glu Ala Ile
    130                 135                 140

tgc gcg cgc ttt ttt atc cct aaa act tgg gag agt ttg aaa ttc ttg    480
Cys Ala Arg Phe Phe Ile Pro Lys Thr Trp Glu Ser Leu Lys Phe Leu
145                 150                 155                 160

caa gaa agg gct aat ttt ttg cat tta gac atc agc ggc cat ctt tta    528
Gln Glu Arg Ala Asn Phe Leu His Leu Asp Ile Ser Gly His Leu Leu
                165                 170                 175

aac gct ctt ttt gaa att aat aac gaa gat tta ggc gtt tcg ttt aac    576

Asn Ala Leu Phe Glu Ile Asn Asn Glu Asp Leu Gly Val Ser Phe Asn
            180                 185                 190

gat tta gac aag cta gcg gtt tta aac gca ccc atc act tta gaa gac     624
Asp Leu Asp Lys Leu Ala Val Leu Asn Ala Pro Ile Thr Leu Glu Asp
        195                 200                 205

att caa gaa tta agc tcc aat gcg ggg gat atg gat ttg caa aag ctc     672
Ile Gln Glu Leu Ser Ser Asn Ala Gly Asp Met Asp Leu Gln Lys Leu
    210                 215                 220

att tta ggg ctt ttt tta aaa aaa agt gcg ctt gat att tat gat tat     720
Ile Leu Gly Leu Phe Leu Lys Lys Ser Ala Leu Asp Ile Tyr Asp Tyr
225                 230                 235                 240

ttg tta aaa gag ggc aaa aaa gat gcg gat att tta agg ggg tta gag     768
Leu Leu Lys Glu Gly Lys Lys Asp Ala Asp Ile Leu Arg Gly Leu Glu
                245                 250                 255

cgc tat ttt tac caa ctt ttt tta ttt ttc gct cat att aaa acg acc     816
Arg Tyr Phe Tyr Gln Leu Phe Leu Phe Phe Ala His Ile Lys Thr Thr
            260                 265                 270

ggt tta atg gac gct aaa gag gtt tta ggc tac gct ccc cct aaa gaa     864
Gly Leu Met Asp Ala Lys Glu Val Leu Gly Tyr Ala Pro Pro Lys Glu
        275                 280                 285

att gcc gaa aat tac gct aaa aac gcc ttg cgt ttg aaa gaa gcc ggc     912
Ile Ala Glu Asn Tyr Ala Lys Asn Ala Leu Arg Leu Lys Glu Ala Gly
    290                 295                 300

tat aag agg gtt ttt gaa att ttt agg tta tgg cac att caa agc atg     960
Tyr Lys Arg Val Phe Glu Ile Phe Arg Leu Trp His Ile Gln Ser Met
305                 310                 315                 320

caa ggg caa aag gaa ttg ggc ttt ttg tat ttg acc tcc att caa aaa     1008
Gln Gly Gln Lys Glu Leu Gly Phe Leu Tyr Leu Thr Ser Ile Gln Lys
                325                 330                 335

atc att aac ccc taa                                                 1023
Ile Ile Asn Pro
            340

<210> 39
<211> 340
<212> PRT
<213> Helicobacter pylori 26695

<400> 39
Met Tyr Arg Lys Asp Leu Asp His Tyr Leu Lys Gln Arg Leu Pro Lys
1               5                   10                  15

Ala Val Phe Leu Tyr Gly Glu Phe Asp Phe Phe Ile His Tyr Tyr Ile
            20                  25                  30

Gln Thr Ile Ser Ala Leu Phe Lys Cys Asp Asn Pro Asp Ile Glu Thr
        35                  40                  45

Ser Leu Phe Tyr Ala Ser Asp Tyr Glu Lys Ser Gln Ile Ala Thr Leu
    50                  55                  60

Leu Glu Gln Asp Ser Leu Phe Gly Gly Ser Ser Leu Val Val Leu Lys
65                  70                  75                  80

Leu Asp Phe Ala Leu His Lys Lys Phe Lys Glu Asn Asp Ile Asn Leu
                85                  90                  95

Phe Leu Lys Ala Leu Glu Arg Pro Ser His Asn Arg Leu Ile Ile Gly
            100                 105                 110

Leu Tyr Asn Ala Lys Ser Asp Thr Thr Lys Tyr Lys Tyr Thr Ser Asp
        115                 120                 125

Ala Ile Val Lys Phe Phe Gln Lys Ser Pro Leu Lys Asp Glu Ala Ile
    130                 135                 140

Cys Ala Arg Phe Phe Ile Pro Lys Thr Trp Glu Ser Leu Lys Phe Leu
145                 150                 155                 160

Gln Glu Arg Ala Asn Phe Leu His Leu Asp Ile Ser Gly His Leu Leu
                165                 170                 175

Asn Ala Leu Phe Glu Ile Asn Asn Glu Asp Leu Gly Val Ser Phe Asn
            180                 185                 190

Asp Leu Asp Lys Leu Ala Val Leu Asn Ala Pro Ile Thr Leu Glu Asp
        195                 200                 205

Ile Gln Glu Leu Ser Ser Asn Ala Gly Asp Met Asp Leu Gln Lys Leu
    210                 215                 220

Ile Leu Gly Leu Phe Leu Lys Lys Ser Ala Leu Asp Ile Tyr Asp Tyr
225                 230                 235                 240

Leu Leu Lys Glu Gly Lys Lys Asp Ala Asp Ile Leu Arg Gly Leu Glu

                245                 250                 255

Arg Tyr Phe Tyr Gln Leu Phe Leu Phe Phe Ala His Ile Lys Thr Thr
            260                 265                 270

Gly Leu Met Asp Ala Lys Glu Val Leu Gly Tyr Ala Pro Pro Lys Glu
        275                 280                 285

Ile Ala Glu Asn Tyr Ala Lys Asn Ala Leu Arg Leu Lys Glu Ala Gly
    290                 295                 300

Tyr Lys Arg Val Phe Glu Ile Phe Arg Leu Trp His Ile Gln Ser Met
305                 310                 315                 320

Gln Gly Gln Lys Glu Leu Gly Phe Leu Tyr Leu Thr Ser Ile Gln Lys
                325                 330                 335

Ile Ile Asn Pro
            340

<210> 40
<211> 1023
<212> DNA
<213> Helicobacter pylori J99

<400> 40
atgtatcgta aagatttgga taattactta aaacagcgcc tccctaaagc ggtgtttttg 60

tatggggagt ttgatttttt catccattat tatattcaaa cgattagcgc gctttttaaa 120

ggcaataacc ctgacacaga aacttcgctt ttttatgcga gcgattatga aaaaagccag 180

attgcgaccc ttttagagca ggattcttta tttggaggga gcagtttagt tattttaaaa 240

ctggattttg cattgcataa gaaatttaag gaaaatgata tcaatccttt tttaaaagct 300

ttagagcggc ctagccataa taggcttatc atagggcttt ataatgctaa aagcgacacc 360

acaaaataca aatacactag cgaaattatc gttaaatttt tccaaaaaag ccccttgaaa 420

gatgaagcca tttgcgtgcg ctttttaccc cctaaagcgt gggagagttt gaaattcttg 480

caagaaaggg ctaatttttt gcatttagac atcagcggcc atcttttaaa cgctcttttt 540

gaaattaata acgaagattt aagcgtttcg tttaacgatt tagacaagct agcggtttta 600

aacgcgccca tcactttaga agacattcaa gaattaagct ccaatgcggg ggatatggat 660

ttgcaaaagc tcattttagg gctttttttg aaaaaaagcg tccttgatat ttatgattat 720

ttgttaaaag agggcaaaaa ggatgcggat attttaaggg ggttagagcg ctatttttac 780

cagctttttt tatttttcgc ccacattaaa acgaccggtt taatggacgc taaagaggtc 840

ttaggctacg ctcctcctaa agagattgta gaaaattacg ctaaaaacgc cctgcgtttg 900

aaagaagccg gctataagag ggtttttgaa atttttaggt tatggcacct tcaaagcatg 960

caagggcaaa aggaattggg ctttttgtat ttgaccccca ttcaaaaaat cattaaccct 1020

tga 1023

<210> 41
<211> 1023
<212> DNA
<213> Helicobacter pylori J99

<220>
<221> CDS
<222> (1)..(1023)

<400> 41
atg tat cgt aaa gat ttg gat aat tac tta aaa cag cgc ctc cct aaa 48
Met Tyr Arg Lys Asp Leu Asp Asn Tyr Leu Lys Gln Arg Leu Pro Lys
1               5                   10                  15

gcg gtg ttt ttg tat ggg gag ttt gat ttt ttc atc cat tat tat att 96
Ala Val Phe Leu Tyr Gly Glu Phe Asp Phe Phe Ile His Tyr Tyr Ile
            20                  25                  30

caa acg att agc gcg ctt ttt aaa ggc aat aac cct gac aca gaa act 144
Gln Thr Ile Ser Ala Leu Phe Lys Gly Asn Asn Pro Asp Thr Glu Thr
        35                  40                  45

tcg ctt ttt tat gcg agc gat tat gaa aaa agc cag att gcg acc ctt 192
Ser Leu Phe Tyr Ala Ser Asp Tyr Glu Lys Ser Gln Ile Ala Thr Leu
    50                  55                  60

tta gag cag gat tct tta ttt gga ggg agc agt tta gtt att tta aaa 240
Leu Glu Gln Asp Ser Leu Phe Gly Gly Ser Ser Leu Val Ile Leu Lys
65                  70                  75                  80

ctg gat ttt gca ttg cat aag aaa ttt aag gaa aat gat atc aat cct 288
Leu Asp Phe Ala Leu His Lys Lys Phe Lys Glu Asn Asp Ile Asn Pro

                85                  90                  95

ttt tta aaa gct tta gag cgg cct agc cat aat agg ctt atc ata ggg 336
Phe Leu Lys Ala Leu Glu Arg Pro Ser His Asn Arg Leu Ile Ile Gly
            100                 105                 110

ctt tat aat gct aaa agc gac acc aca aaa tac aaa tac act agc gaa 384
Leu Tyr Asn Ala Lys Ser Asp Thr Thr Lys Tyr Lys Tyr Thr Ser Glu
        115                 120                 125

att atc gtt aaa ttt ttc caa aaa agc ccc ttg aaa gat gaa gcc att 432
Ile Ile Val Lys Phe Phe Gln Lys Ser Pro Leu Lys Asp Glu Ala Ile
    130                 135                 140

tgc gtg cgc ttt ttt acc cct aaa gcg tgg gag agt ttg aaa ttc ttg 480
Cys Val Arg Phe Phe Thr Pro Lys Ala Trp Glu Ser Leu Lys Phe Leu
145                 150                 155                 160

caa gaa agg gct aat ttt ttg cat tta gac atc agc ggc cat ctt tta 528
Gln Glu Arg Ala Asn Phe Leu His Leu Asp Ile Ser Gly His Leu Leu
                165                 170                 175

aac gct ctt ttt gaa att aat aac gaa gat tta agc gtt tcg ttt aac 576
Asn Ala Leu Phe Glu Ile Asn Asn Glu Asp Leu Ser Val Ser Phe Asn
            180                 185                 190

gat tta gac aag cta gcg gtt tta aac gcg ccc atc act tta gaa gac 624
Asp Leu Asp Lys Leu Ala Val Leu Asn Ala Pro Ile Thr Leu Glu Asp
        195                 200                 205

att caa gaa tta agc tcc aat gcg ggg gat atg gat ttg caa aag ctc 672
Ile Gln Glu Leu Ser Ser Asn Ala Gly Asp Met Asp Leu Gln Lys Leu
    210                 215                 220

att tta ggg ctt ttt ttg aaa aaa agc gtc ctt gat att tat gat tat 720
Ile Leu Gly Leu Phe Leu Lys Lys Ser Val Leu Asp Ile Tyr Asp Tyr
225                 230                 235                 240

ttg tta aaa gag ggc aaa aag gat gcg gat att tta agg ggg tta gag 768
Leu Leu Lys Glu Gly Lys Lys Asp Ala Asp Ile Leu Arg Gly Leu Glu
                245                 250                 255

cgc tat ttt tac cag ctt ttt tta ttt ttc gcc cac att aaa acg acc 816
Arg Tyr Phe Tyr Gln Leu Phe Leu Phe Phe Ala His Ile Lys Thr Thr
            260                 265                 270

ggt tta atg gac gct aaa gag gtc tta ggc tac gct cct cct aaa gag 864
Gly Leu Met Asp Ala Lys Glu Val Leu Gly Tyr Ala Pro Pro Lys Glu

        275                 280                 285

att gta gaa aat tac gct aaa aac gcc ctg cgt ttg aaa gaa gcc ggc 912
Ile Val Glu Asn Tyr Ala Lys Asn Ala Leu Arg Leu Lys Glu Ala Gly
    290                 295                 300

tat aag agg gtt ttt gaa att ttt agg tta tgg cac ctt caa agc atg 960
Tyr Lys Arg Val Phe Glu Ile Phe Arg Leu Trp His Leu Gln Ser Met
305                 310                 315                 320

caa ggg caa aag gaa ttg ggc ttt ttg tat ttg acc ccc att caa aaa 1008
Gln Gly Gln Lys Glu Leu Gly Phe Leu Tyr Leu Thr Pro Ile Gln Lys
                325                 330                 335

atc att aac cct tga 1023
Ile Ile Asn Pro
            340

<210> 42
<211> 340
<212> PRT
<213> Helicobacter pylori J99

<400> 42
Met Tyr Arg Lys Asp Leu Asp Asn Tyr Leu Lys Gln Arg Leu Pro Lys
1               5                   10                  15

Ala Val Phe Leu Tyr Gly Glu Phe Asp Phe Phe Ile His Tyr Tyr Ile
            20                  25                  30

Gln Thr Ile Ser Ala Leu Phe Lys Gly Asn Asn Pro Asp Thr Glu Thr
        35                  40                  45

Ser Leu Phe Tyr Ala Ser Asp Tyr Glu Lys Ser Gln Ile Ala Thr Leu
    50                  55                  60

Leu Glu Gln Asp Ser Leu Phe Gly Gly Ser Ser Leu Val Ile Leu Lys
65                  70                  75                  80

Leu Asp Phe Ala Leu His Lys Lys Phe Lys Glu Asn Asp Ile Asn Pro
                85                  90                  95

Phe Leu Lys Ala Leu Glu Arg Pro Ser His Asn Arg Leu Ile Ile Gly
            100                 105                 110

Leu Tyr Asn Ala Lys Ser Asp Thr Thr Lys Tyr Lys Tyr Thr Ser Glu
        115                 120                 125

Ile Ile Val Lys Phe Phe Gln Lys Ser Pro Leu Lys Asp Glu Ala Ile
    130                 135                 140

Cys Val Arg Phe Phe Thr Pro Lys Ala Trp Glu Ser Leu Lys Phe Leu
145                 150                 155                 160

Gln Glu Arg Ala Asn Phe Leu His Leu Asp Ile Ser Gly His Leu Leu
                165                 170                 175

Asn Ala Leu Phe Glu Ile Asn Asn Glu Asp Leu Ser Val Ser Phe Asn
            180                 185                 190

Asp Leu Asp Lys Leu Ala Val Leu Asn Ala Pro Ile Thr Leu Glu Asp
        195                 200                 205

Ile Gln Glu Leu Ser Ser Asn Ala Gly Asp Met Asp Leu Gln Lys Leu
    210                 215                 220

Ile Leu Gly Leu Phe Leu Lys Lys Ser Val Leu Asp Ile Tyr Asp Tyr
225                 230                 235                 240

Leu Leu Lys Glu Gly Lys Lys Asp Ala Asp Ile Leu Arg Gly Leu Glu
                245                 250                 255

Arg Tyr Phe Tyr Gln Leu Phe Leu Phe Phe Ala His Ile Lys Thr Thr
            260                 265                 270

Gly Leu Met Asp Ala Lys Glu Val Leu Gly Tyr Ala Pro Pro Lys Glu
        275                 280                 285

Ile Val Glu Asn Tyr Ala Lys Asn Ala Leu Arg Leu Lys Glu Ala Gly
    290                 295                 300

Tyr Lys Arg Val Phe Glu Ile Phe Arg Leu Trp His Leu Gln Ser Met
305                 310                 315                 320

Gln Gly Gln Lys Glu Leu Gly Phe Leu Tyr Leu Thr Pro Ile Gln Lys
                325                 330                 335

Ile Ile Asn Pro
            340

<210> 43
<211> 1005
<212> DNA
<213> Lactococcus lactis

<400> 43
atgacagtat ttgatgaatt taatgaaatc aaaagaaaag ggctccctca aattttagta 60

atttttggtg agtcaaggga actagttgga gaattgaaaa atcaactctt aacagaggtt 120

aatttcgagc caacggattt gagccaggct tattatgact taactcctag taatagcgat 180

ttagcccttg aagatttgga atctctccct tttttctcag attctcggtt ggtaatcctg 240

gaaaacttgg ttaatttaac aacggctaaa aaaagtgttt tagatgaaaa acagttgaag 300

cgctttgaaa attttttaga tgatccgccg gaaactacgc aacttgtttt aatcctttat 360

ggaaaattgg acagtcgtct taaagttgtt aaaaaattaa aagctaaagc aactttactt 420

gaagcacaag aattaaagag tcaagaatta attcaatatt ttagtcgaat aagtccttta 480

ccaaaggtag ttttgagttt aattgccgct aaatcaaatg ataatttttc agtaatgaag 540

caaaatattg acctcgttca aacttatgca ctagggcgag aagtgacact tgatgatgtg 600

gaaaaagtcg tcccaaaatc gctacaagat aatatttttg ccttaactga tttaattttt 660

aaaggaaaaa ttgatgaggc gcgtgattta gtccatgatt tgaccttgca aggggaagat 720

ttaattaaaa tattagctat tttgacaaat tcttatcgtt tgtatttaca agtcaaactt 780

ttccaagcca agggttggca agaaaatcaa caagttgcct ttttaaaaat gcacccttat 840

cctgtaaaat tggccaatca attggttaga aaattaaacg ttaaatcctt aaaaaatgga 900

ctttctgatt taattaaact tgattttgat ataaaaacaa gtgcagctga taaatcctat 960

ctttttgata tcactttaat caggttgacc ttgaaaaaaa actga 1005

<210> 44
<211> 1005
<212> DNA
<213> Lactococcus lactis

<220>
<221> CDS
<222> (1)..(1005)

<400> 44
atg aca gta ttt gat gaa ttt aat gaa atc aaa aga aaa ggg ctc cct 48

Met Thr Val Phe Asp Glu Phe Asn Glu Ile Lys Arg Lys Gly Leu Pro
1               5                   10                  15

caa att tta gta att ttt ggt gag tca agg gaa cta gtt gga gaa ttg    96
Gln Ile Leu Val Ile Phe Gly Glu Ser Arg Glu Leu Val Gly Glu Leu
            20                  25                  30

aaa aat caa ctc tta aca gag gtt aat ttc gag cca acg gat ttg agc    144
Lys Asn Gln Leu Leu Thr Glu Val Asn Phe Glu Pro Thr Asp Leu Ser
        35                  40                  45

cag gct tat tat gac tta act cct agt aat agc gat tta gcc ctt gaa    192
Gln Ala Tyr Tyr Asp Leu Thr Pro Ser Asn Ser Asp Leu Ala Leu Glu
    50                  55                  60

gat ttg gaa tct ctc cct ttt ttc tca gat tct cgg ttg gta atc ctg    240
Asp Leu Glu Ser Leu Pro Phe Phe Ser Asp Ser Arg Leu Val Ile Leu
65                  70                  75                  80

gaa aac ttg gtt aat tta aca acg gct aaa aaa agt gtt tta gat gaa    288
Glu Asn Leu Val Asn Leu Thr Thr Ala Lys Lys Ser Val Leu Asp Glu
                85                  90                  95

aaa cag ttg aag cgc ttt gaa aat ttt tta gat gat ccg ccg gaa act    336
Lys Gln Leu Lys Arg Phe Glu Asn Phe Leu Asp Asp Pro Pro Glu Thr
            100                 105                 110

acg caa ctt gtt tta atc ctt tat gga aaa ttg gac agt cgt ctt aaa    384
Thr Gln Leu Val Leu Ile Leu Tyr Gly Lys Leu Asp Ser Arg Leu Lys
        115                 120                 125

gtt gtt aaa aaa tta aaa gct aaa gca act tta ctt gaa gca caa gaa    432
Val Val Lys Lys Leu Lys Ala Lys Ala Thr Leu Leu Glu Ala Gln Glu
    130                 135                 140

tta aag agt caa gaa tta att caa tat ttt agt cga ata agt cct tta    480
Leu Lys Ser Gln Glu Leu Ile Gln Tyr Phe Ser Arg Ile Ser Pro Leu
145                 150                 155                 160

cca aag gta gtt ttg agt tta att gcc gct aaa tca aat gat aat ttt    528
Pro Lys Val Val Leu Ser Leu Ile Ala Ala Lys Ser Asn Asp Asn Phe
                165                 170                 175

tca gta atg aag caa aat att gac ctc gtt caa act tat gca cta ggg    576
Ser Val Met Lys Gln Asn Ile Asp Leu Val Gln Thr Tyr Ala Leu Gly
            180                 185                 190

cga gaa gtg aca ctt gat gat gtg gaa aaa gtc gtc cca aaa tcg cta    624

Arg Glu Val Thr Leu Asp Asp Val Glu Lys Val Val Pro Lys Ser Leu
        195                 200                 205

caa gat aat att ttt gcc tta act gat tta att ttt aaa gga aaa att     672
Gln Asp Asn Ile Phe Ala Leu Thr Asp Leu Ile Phe Lys Gly Lys Ile
    210                 215                 220

gat gag gcg cgt gat tta gtc cat gat ttg acc ttg caa ggg gaa gat     720
Asp Glu Ala Arg Asp Leu Val His Asp Leu Thr Leu Gln Gly Glu Asp
225                 230                 235                 240

tta att aaa ata tta gct att ttg aca aat tct tat cgt ttg tat tta     768
Leu Ile Lys Ile Leu Ala Ile Leu Thr Asn Ser Tyr Arg Leu Tyr Leu
                245                 250                 255

caa gtc aaa ctt ttc caa gcc aag ggt tgg caa gaa aat caa caa gtt     816
Gln Val Lys Leu Phe Gln Ala Lys Gly Trp Gln Glu Asn Gln Gln Val
            260                 265                 270

gcc ttt tta aaa atg cac cct tat cct gta aaa ttg gcc aat caa ttg     864
Ala Phe Leu Lys Met His Pro Tyr Pro Val Lys Leu Ala Asn Gln Leu
        275                 280                 285

gtt aga aaa tta aac gtt aaa tcc tta aaa aat gga ctt tct gat tta     912
Val Arg Lys Leu Asn Val Lys Ser Leu Lys Asn Gly Leu Ser Asp Leu
    290                 295                 300

att aaa ctt gat ttt gat ata aaa aca agt gca gct gat aaa tcc tat     960
Ile Lys Leu Asp Phe Asp Ile Lys Thr Ser Ala Ala Asp Lys Ser Tyr
305                 310                 315                 320

ctt ttt gat atc act tta atc agg ttg acc ttg aaa aaa aac tga         1005
Leu Phe Asp Ile Thr Leu Ile Arg Leu Thr Leu Lys Lys Asn
                325                 330                 335

<210> 45
<211> 334
<212> PRT
<213> Lactococcus lactis

<400> 45
Met Thr Val Phe Asp Glu Phe Asn Glu Ile Lys Arg Lys Gly Leu Pro
  1               5                  10                  15

Gln Ile Leu Val Ile Phe Gly Glu Ser Arg Glu Leu Val Gly Glu Leu
             20                  25                  30

Lys Asn Gln Leu Leu Thr Glu Val Asn Phe Glu Pro Thr Asp Leu Ser
        35                  40                  45

Gln Ala Tyr Tyr Asp Leu Thr Pro Ser Asn Ser Asp Leu Ala Leu Glu
    50                  55                  60

Asp Leu Glu Ser Leu Pro Phe Phe Ser Asp Ser Arg Leu Val Ile Leu
65                  70                  75                  80

Glu Asn Leu Val Asn Leu Thr Thr Ala Lys Lys Ser Val Leu Asp Glu
                85                  90                  95

Lys Gln Leu Lys Arg Phe Glu Asn Phe Leu Asp Asp Pro Pro Glu Thr
            100                 105                 110

Thr Gln Leu Val Leu Ile Leu Tyr Gly Lys Leu Asp Ser Arg Leu Lys
        115                 120                 125

Val Val Lys Lys Leu Lys Ala Lys Ala Thr Leu Leu Glu Ala Gln Glu
    130                 135                 140

Leu Lys Ser Gln Glu Leu Ile Gln Tyr Phe Ser Arg Ile Ser Pro Leu
145                 150                 155                 160

Pro Lys Val Val Leu Ser Leu Ile Ala Ala Lys Ser Asn Asp Asn Phe
                165                 170                 175

Ser Val Met Lys Gln Asn Ile Asp Leu Val Gln Thr Tyr Ala Leu Gly
            180                 185                 190

Arg Glu Val Thr Leu Asp Asp Val Glu Lys Val Val Pro Lys Ser Leu
        195                 200                 205

Gln Asp Asn Ile Phe Ala Leu Thr Asp Leu Ile Phe Lys Gly Lys Ile
    210                 215                 220

Asp Glu Ala Arg Asp Leu Val His Asp Leu Thr Leu Gln Gly Glu Asp
225                 230                 235                 240

Leu Ile Lys Ile Leu Ala Ile Leu Thr Asn Ser Tyr Arg Leu Tyr Leu
                245                 250                 255

Gln Val Lys Leu Phe Gln Ala Lys Gly Trp Gln Glu Asn Gln Gln Val
            260                 265                 270

Ala Phe Leu Lys Met His Pro Tyr Pro Val Lys Leu Ala Asn Gln Leu
        275                 280                 285

Val Arg Lys Leu Asn Val Lys Ser Leu Lys Asn Gly Leu Ser Asp Leu
    290                 295                 300

Ile Lys Leu Asp Phe Asp Ile Lys Thr Ser Ala Ala Asp Lys Ser Tyr
305                 310                 315                 320

Leu Phe Asp Ile Thr Leu Ile Arg Leu Thr Leu Lys Lys Asn
                325                 330

<210> 46
<211> 1170
<212> DNA
<213> Mycobacterium leprae

<400> 46
gtgcctgcgg caatcgttcc cggtccggct agtgccctcg gcgtgctgct gctggtgttg 60

gtcctgtggc ggtgctttcg ggtggcgatg atttcagcac tgatggtcgc cgttgcgtgc 120

ttgcctgatt ggttgtcagg gttcctgacc ggcgggctta tcgcgggctc gtcggctcgt 180

cgtgccacca tctacggagt gagtaataag ttttcgtcgc tgcacttggt cctaggaaat 240

gaggaactgc tggttgagcg ggccgtgggg gaggtgttgc ggtcggcgcg gcagcgggca 300

ggcacccagg acgtccccgt tagccgaatg cgagccggtg acgtcggcac ctatgagctc 360

accgaactgc tgagcccgtc attgttcgcc gacgagcgga ttgtcgtgct agaggccgcc 420

gctgaagcgg gtaaggaggc cgctgcgctg atcgtgtcgg ctgccgctga cataccgcaa 480

ggcacggtgc tggtggtggt gcattcaggt ggtggtcgag ccaaagcgct ggctaacgag 540

ctgcagtcgt tgggtgctac ggtgcatccc tgcgcgcgga tcactaagct tagcgagcgt 600

actgattttg ttcgaaaaga attgcgttcg ttgcgggtca aggtcgacga gggggcagtg 660

accgccctgc tgaacgctgt tggttctgac gtgcgcgaac tcgcttcggc ctgttcgcaa 720

ttggtagccg acaccgccgg tgatgtcgac gccgacgcgg ttcagcgtta tcacagcggc 780

aaagcggaag tgaagggctt tgacattgcc gacaaggcgg tcggtggcga tgtctcagga 840

gcagtggagg cgttgcggtg ggcgatgatg cgcggcgagc cactggtggt gttggctgat 900

gcgctggccg aagcagtgca cacgatcggc cgggttgggc cgctgtccgg tgattcctac 960

cgcctggcgt cgcggttggg aatgccgccc tggcgggtgc agaaggcaca acaacaggct 1020

cggcgttggt cgcgtgacac agtggcagcc gcgatgagag tggtggctgc gctcaacgcc 1080

gacgtgaaag gggccgccgc ggacgcctac tacgctctgg aatccgcggt caggaaagtt 1140

gctgagctgg ccgccgatgg gagccgatga 1170

<210> 47
<211> 1170
<212> DNA
<213> Mycobacterium leprae

<220>
<221> CDS
<222> (1)..(1170)

<400> 47
gtg cct gcg gca atc gtt ccc ggt ccg gct agt gcc ctc ggc gtg ctg 48
Val Pro Ala Ala Ile Val Pro Gly Pro Ala Ser Ala Leu Gly Val Leu
1               5                   10                  15

ctg ctg gtg ttg gtc ctg tgg cgg tgc ttt cgg gtg gcg atg att tca 96
Leu Leu Val Leu Val Leu Trp Arg Cys Phe Arg Val Ala Met Ile Ser
            20                  25                  30

gca ctg atg gtc gcc gtt gcg tgc ttg cct gat tgg ttg tca ggg ttc 144
Ala Leu Met Val Ala Val Ala Cys Leu Pro Asp Trp Leu Ser Gly Phe
        35                  40                  45

ctg acc ggc ggg ctt atc gcg ggc tcg tcg gct cgt cgt gcc acc atc 192
Leu Thr Gly Gly Leu Ile Ala Gly Ser Ser Ala Arg Arg Ala Thr Ile
    50                  55                  60

tac gga gtg agt aat aag ttt tcg tcg ctg cac ttg gtc cta gga aat 240
Tyr Gly Val Ser Asn Lys Phe Ser Ser Leu His Leu Val Leu Gly Asn
65                  70                  75                  80

gag gaa ctg ctg gtt gag cgg gcc gtg ggg gag gtg ttg cgg tcg gcg 288
Glu Glu Leu Leu Val Glu Arg Ala Val Gly Glu Val Leu Arg Ser Ala
                85                  90                  95

cgg cag cgg gca ggc acc cag gac gtc ccc gtt agc cga atg cga gcc 336
Arg Gln Arg Ala Gly Thr Gln Asp Val Pro Val Ser Arg Met Arg Ala
            100                 105                 110

ggt gac gtc ggc acc tat gag ctc acc gaa ctg ctg agc ccg tca ttg 384

Gly Asp Val Gly Thr Tyr Glu Leu Thr Glu Leu Leu Ser Pro Ser Leu
        115                 120                 125

ttc gcc gac gag cgg att gtc gtg cta gag gcc gcc gct gaa gcg ggt     432
Phe Ala Asp Glu Arg Ile Val Val Leu Glu Ala Ala Ala Glu Ala Gly
    130                 135                 140

aag gag gcc gct gcg ctg atc gtg tcg gct gcc gct gac ata ccg caa     480
Lys Glu Ala Ala Ala Leu Ile Val Ser Ala Ala Ala Asp Ile Pro Gln
145                 150                 155                 160

ggc acg gtg ctg gtg gtg gtg cat tca ggt ggt ggt cga gcc aaa gcg     528
Gly Thr Val Leu Val Val Val His Ser Gly Gly Gly Arg Ala Lys Ala
                165                 170                 175

ctg gct aac gag ctg cag tcg ttg ggt gct acg gtg cat ccc tgc gcg     576
Leu Ala Asn Glu Leu Gln Ser Leu Gly Ala Thr Val His Pro Cys Ala
            180                 185                 190

cgg atc act aag ctt agc gag cgt act gat ttt gtt cga aaa gaa ttg     624
Arg Ile Thr Lys Leu Ser Glu Arg Thr Asp Phe Val Arg Lys Glu Leu
        195                 200                 205

cgt tcg ttg cgg gtc aag gtc gac gag ggg gca gtg acc gcc ctg ctg     672
Arg Ser Leu Arg Val Lys Val Asp Glu Gly Ala Val Thr Ala Leu Leu
    210                 215                 220

aac gct gtt ggt tct gac gtg cgc gaa ctc gct tcg gcc tgt tcg caa     720
Asn Ala Val Gly Ser Asp Val Arg Glu Leu Ala Ser Ala Cys Ser Gln
225                 230                 235                 240

ttg gta gcc gac acc gcc ggt gat gtc gac gcc gac gcg gtt cag cgt     768
Leu Val Ala Asp Thr Ala Gly Asp Val Asp Ala Asp Ala Val Gln Arg
                245                 250                 255

tat cac agc ggc aaa gcg gaa gtg aag ggc ttt gac att gcc gac aag     816
Tyr His Ser Gly Lys Ala Glu Val Lys Gly Phe Asp Ile Ala Asp Lys
            260                 265                 270

gcg gtc ggt ggc gat gtc tca gga gca gtg gag gcg ttg cgg tgg gcg     864
Ala Val Gly Gly Asp Val Ser Gly Ala Val Glu Ala Leu Arg Trp Ala
        275                 280                 285

atg atg cgc ggc gag cca ctg gtg gtg ttg gct gat gcg ctg gcc gaa     912
Met Met Arg Gly Glu Pro Leu Val Val Leu Ala Asp Ala Leu Ala Glu
    290                 295                 300

gca gtg cac acg atc ggc cgg gtt ggg ccg ctg tcc ggt gat tcc tac     960

Ala Val His Thr Ile Gly Arg Val Gly Pro Leu Ser Gly Asp Ser Tyr
305                 310                 315                 320

cgc ctg gcg tcg cgg ttg gga atg ccg ccc tgg cgg gtg cag aag gca     1008
Arg Leu Ala Ser Arg Leu Gly Met Pro Pro Trp Arg Val Gln Lys Ala
                325                 330                 335

caa caa cag gct cgg cgt tgg tcg cgt gac aca gtg gca gcc gcg atg     1056
Gln Gln Gln Ala Arg Arg Trp Ser Arg Asp Thr Val Ala Ala Ala Met
            340                 345                 350

aga gtg gtg gct gcg ctc aac gcc gac gtg aaa ggg gcc gcc gcg gac     1104
Arg Val Val Ala Ala Leu Asn Ala Asp Val Lys Gly Ala Ala Ala Asp
        355                 360                 365

gcc tac tac gct ctg gaa tcc gcg gtc agg aaa gtt gct gag ctg gcc     1152
Ala Tyr Tyr Ala Leu Glu Ser Ala Val Arg Lys Val Ala Glu Leu Ala
    370                 375                 380

gcc gat ggg agc cga tga                                             1170
Ala Asp Gly Ser Arg
385                 390

<210> 48
<211> 389
<212> PRT
<213> Mycobacterium leprae

<400> 48
Val Pro Ala Ala Ile Val Pro Gly Pro Ala Ser Ala Leu Gly Val Leu
  1               5                  10                  15

Leu Leu Val Leu Val Leu Trp Arg Cys Phe Arg Val Ala Met Ile Ser
                 20                  25                  30

Ala Leu Met Val Ala Val Ala Cys Leu Pro Asp Trp Leu Ser Gly Phe
         35                  40                  45

Leu Thr Gly Gly Leu Ile Ala Gly Ser Ser Ala Arg Arg Ala Thr Ile
     50                  55                  60

Tyr Gly Val Ser Asn Lys Phe Ser Ser Leu His Leu Val Leu Gly Asn
 65                  70                  75                  80

Glu Glu Leu Leu Val Glu Arg Ala Val Gly Glu Val Leu Arg Ser Ala
                 85                  90                  95

Arg Gln Arg Ala Gly Thr Gln Asp Val Pro Val Ser Arg Met Arg Ala
            100                 105                 110

Gly Asp Val Gly Thr Tyr Glu Leu Thr Glu Leu Leu Ser Pro Ser Leu
        115                 120                 125

Phe Ala Asp Glu Arg Ile Val Val Leu Glu Ala Ala Ala Glu Ala Gly
    130                 135                 140

Lys Glu Ala Ala Ala Leu Ile Val Ser Ala Ala Ala Asp Ile Pro Gln
145                 150                 155                 160

Gly Thr Val Leu Val Val Val His Ser Gly Gly Gly Arg Ala Lys Ala
                165                 170                 175

Leu Ala Asn Glu Leu Gln Ser Leu Gly Ala Thr Val His Pro Cys Ala
            180                 185                 190

Arg Ile Thr Lys Leu Ser Glu Arg Thr Asp Phe Val Arg Lys Glu Leu
        195                 200                 205

Arg Ser Leu Arg Val Lys Val Asp Glu Gly Ala Val Thr Ala Leu Leu
    210                 215                 220

Asn Ala Val Gly Ser Asp Val Arg Glu Leu Ala Ser Ala Cys Ser Gln
225                 230                 235                 240

Leu Val Ala Asp Thr Ala Gly Asp Val Asp Ala Asp Ala Val Gln Arg
                245                 250                 255

Tyr His Ser Gly Lys Ala Glu Val Lys Gly Phe Asp Ile Ala Asp Lys
            260                 265                 270

Ala Val Gly Gly Asp Val Ser Gly Ala Val Glu Ala Leu Arg Trp Ala
        275                 280                 285

Met Met Arg Gly Glu Pro Leu Val Val Leu Ala Asp Ala Leu Ala Glu
    290                 295                 300

Ala Val His Thr Ile Gly Arg Val Gly Pro Leu Ser Gly Asp Ser Tyr
305                 310                 315                 320

Arg Leu Ala Ser Arg Leu Gly Met Pro Pro Trp Arg Val Gln Lys Ala
                325                 330                 335

Gln Gln Gln Ala Arg Arg Trp Ser Arg Asp Thr Val Ala Ala Ala Met
            340                 345                 350

Arg Val Val Ala Ala Leu Asn Ala Asp Val Lys Gly Ala Ala Ala Asp
        355                 360                 365

Ala Tyr Tyr Ala Leu Glu Ser Ala Val Arg Lys Val Ala Glu Leu Ala
    370                 375                 380

Ala Asp Gly Ser Arg
385

<210> 49
<211> 969
<212> DNA
<213> Mycobacterium tuberculosis

<400> 49
gtgagcgagg ctaagccgtt gcacctggtc ctgggagacg aagaactgct ggtcgaaagg 60

gcggtggccg acgtgttgcg ctcggctcgg cagcgggcag gtacagccga cgtcccggtg 120

agccgaatgc gcgcgggtga cgtcggtgcc tatgagctcg ccgaactgct gagcccgtca 180

ctgttcgccg aggagcggat cgttgtgctg ggggccgctg cggaggcggg caaggacgct 240

gccgcggtaa tcgagtcggc cgccgccgat cttccggccg gcaccgtgct ggtagtggtc 300

cactcgggtg gcgggcgcgc caaatcgctg gccaaccagc tgcggtcgat gggtgcgcag 360

gttcatccgt gcgcgcggat caccaaggtc agtgagcgcg ccgacttcat ccgtagcgag 420

ttcgcgtcgc tgcgggtcaa ggtcgacgac gagaccgtga ccgccctgct ggacgccgtc 480

ggctccgacg tgcgcgaact cgcctcggcc tgttcacagc tggtcgccga taccggagga 540

gccgtcgacg ccgccgctgt acggcgctat cacagcggca aagccgaggt gaggggcttc 600

gacatcgccg acaaggcggt agccggcgac gtggcgggag ctgccgaagc gttgcggtgg 660

gcgatgatgc gcggtgagcc gctagtggtg ttggccgatg cgctcgccga agccgtgcac 720

accatcggcc gggtcgggcc gcagtccggc gacccgtacc gcctggccgc acaactgggg 780

atgccgccct ggcgggtgca gaaagcccag aagcaggctc ggcggtggtc gcgtgacacg 840

gtggcgaccg cgatgaggtt ggtggccgaa ctcaatgcta acgtcaaggg cgccgtcgcg 900

gatgcggact acgcgctgga atccgcggtc cggcaggtcg ccgagttggt ggccgaccgc 960

ggccgatga                                                          969

<210> 50
<211> 969
<212> DNA
<213> Mycobacterium tuberculosis

<220>
<221> CDS
<222> (1)..(969)

<400> 50
gtg agc gag gct aag ccg ttg cac ctg gtc ctg gga gac gaa gaa ctg      48
Val Ser Glu Ala Lys Pro Leu His Leu Val Leu Gly Asp Glu Glu Leu
1               5                   10                  15

ctg gtc gaa agg gcg gtg gcc gac gtg ttg cgc tcg gct cgg cag cgg      96
Leu Val Glu Arg Ala Val Ala Asp Val Leu Arg Ser Ala Arg Gln Arg
            20                  25                  30

gca ggt aca gcc gac gtc ccg gtg agc cga atg cgc gcg ggt gac gtc      144
Ala Gly Thr Ala Asp Val Pro Val Ser Arg Met Arg Ala Gly Asp Val
        35                  40                  45

ggt gcc tat gag ctc gcc gaa ctg ctg agc ccg tca ctg ttc gcc gag      192
Gly Ala Tyr Glu Leu Ala Glu Leu Leu Ser Pro Ser Leu Phe Ala Glu
    50                  55                  60

gag cgg atc gtt gtg ctg ggg gcc gct gcg gag gcg ggc aag gac gct      240
Glu Arg Ile Val Val Leu Gly Ala Ala Ala Glu Ala Gly Lys Asp Ala
65                  70                  75                  80

gcc gcg gta atc gag tcg gcc gcc gcc gat ctt ccg gcc ggc acc gtg      288
Ala Ala Val Ile Glu Ser Ala Ala Ala Asp Leu Pro Ala Gly Thr Val
                85                  90                  95

ctg gta gtg gtc cac tcg ggt ggc ggg cgc gcc aaa tcg ctg gcc aac      336
Leu Val Val Val His Ser Gly Gly Gly Arg Ala Lys Ser Leu Ala Asn
            100                 105                 110

cag ctg cgg tcg atg ggt gcg cag gtt cat ccg tgc gcg cgg atc acc      384
Gln Leu Arg Ser Met Gly Ala Gln Val His Pro Cys Ala Arg Ile Thr
        115                 120                 125

aag gtc agt gag cgc gcc gac ttc atc cgt agc gag ttc gcg tcg ctg      432
Lys Val Ser Glu Arg Ala Asp Phe Ile Arg Ser Glu Phe Ala Ser Leu
    130                 135                 140

cgg gtc aag gtc gac gac gag acc gtg acc gcc ctg ctg gac gcc gtc 480
Arg Val Lys Val Asp Asp Glu Thr Val Thr Ala Leu Leu Asp Ala Val
145                 150                 155                 160

ggc tcc gac gtg cgc gaa ctc gcc tcg gcc tgt tca cag ctg gtc gcc 528
Gly Ser Asp Val Arg Glu Leu Ala Ser Ala Cys Ser Gln Leu Val Ala
                165                 170                 175

gat acc gga gga gcc gtc gac gcc gcc gct gta cgg cgc tat cac agc 576
Asp Thr Gly Gly Ala Val Asp Ala Ala Ala Val Arg Arg Tyr His Ser
            180                 185                 190

ggc aaa gcc gag gtg agg ggc ttc gac atc gcc gac aag gcg gta gcc 624
Gly Lys Ala Glu Val Arg Gly Phe Asp Ile Ala Asp Lys Ala Val Ala
        195                 200                 205

ggc gac gtg gcg gga gct gcc gaa gcg ttg cgg tgg gcg atg atg cgc 672
Gly Asp Val Ala Gly Ala Ala Glu Ala Leu Arg Trp Ala Met Met Arg
    210                 215                 220

ggt gag ccg cta gtg gtg ttg gcc gat gcg ctc gcc gaa gcc gtg cac 720
Gly Glu Pro Leu Val Val Leu Ala Asp Ala Leu Ala Glu Ala Val His
225                 230                 235                 240

acc atc ggc cgg gtc ggg ccg cag tcc ggc gac ccg tac cgc ctg gcc 768
Thr Ile Gly Arg Val Gly Pro Gln Ser Gly Asp Pro Tyr Arg Leu Ala
                245                 250                 255

gca caa ctg ggg atg ccg ccc tgg cgg gtg cag aaa gcc cag aag cag 816
Ala Gln Leu Gly Met Pro Pro Trp Arg Val Gln Lys Ala Gln Lys Gln
            260                 265                 270

gct cgg cgg tgg tcg cgt gac acg gtg gcg acc gcg atg agg ttg gtg 864
Ala Arg Arg Trp Ser Arg Asp Thr Val Ala Thr Ala Met Arg Leu Val
        275                 280                 285

gcc gaa ctc aat gct aac gtc aag ggc gcc gtc gcg gat gcg gac tac 912
Ala Glu Leu Asn Ala Asn Val Lys Gly Ala Val Ala Asp Ala Asp Tyr
    290                 295                 300

gcg ctg gaa tcc gcg gtc cgg cag gtc gcc gag ttg gtg gcc gac cgc 960
Ala Leu Glu Ser Ala Val Arg Gln Val Ala Glu Leu Val Ala Asp Arg
305                 310                 315                 320

ggc cga tga 969
Gly Arg

<210> 51
<211> 322
<212> PRT
<213> Mycobacterium tuberculosis

<400> 51
Val Ser Glu Ala Lys Pro Leu His Leu Val Leu Gly Asp Glu Glu Leu
1               5                   10                  15

Leu Val Glu Arg Ala Val Ala Asp Val Leu Arg Ser Ala Arg Gln Arg
            20                  25                  30

Ala Gly Thr Ala Asp Val Pro Val Ser Arg Met Arg Ala Gly Asp Val
        35                  40                  45

Gly Ala Tyr Glu Leu Ala Glu Leu Leu Ser Pro Ser Leu Phe Ala Glu
    50                  55                  60

Glu Arg Ile Val Val Leu Gly Ala Ala Ala Glu Ala Gly Lys Asp Ala
65                  70                  75                  80

Ala Ala Val Ile Glu Ser Ala Ala Ala Asp Leu Pro Ala Gly Thr Val
                85                  90                  95

Leu Val Val Val His Ser Gly Gly Gly Arg Ala Lys Ser Leu Ala Asn
            100                 105                 110

Gln Leu Arg Ser Met Gly Ala Gln Val His Pro Cys Ala Arg Ile Thr
        115                 120                 125

Lys Val Ser Glu Arg Ala Asp Phe Ile Arg Ser Glu Phe Ala Ser Leu
    130                 135                 140

Arg Val Lys Val Asp Asp Glu Thr Val Thr Ala Leu Leu Asp Ala Val
145                 150                 155                 160

Gly Ser Asp Val Arg Glu Leu Ala Ser Ala Cys Ser Gln Leu Val Ala
                165                 170                 175

Asp Thr Gly Gly Ala Val Asp Ala Ala Ala Val Arg Arg Tyr His Ser
            180                 185                 190

Gly Lys Ala Glu Val Arg Gly Phe Asp Ile Ala Asp Lys Ala Val Ala
        195                 200                 205

Gly Asp Val Ala Gly Ala Ala Glu Ala Leu Arg Trp Ala Met Met Arg
    210                 215                 220

Gly Glu Pro Leu Val Val Leu Ala Asp Ala Leu Ala Glu Ala Val His
225                 230                 235                 240

Thr Ile Gly Arg Val Gly Pro Gln Ser Gly Asp Pro Tyr Arg Leu Ala
                245                 250                 255

Ala Gln Leu Gly Met Pro Pro Trp Arg Val Gln Lys Ala Gln Lys Gln
            260                 265                 270

Ala Arg Arg Trp Ser Arg Asp Thr Val Ala Thr Ala Met Arg Leu Val
        275                 280                 285

Ala Glu Leu Asn Ala Asn Val Lys Gly Ala Val Ala Asp Ala Asp Tyr
    290                 295                 300

Ala Leu Glu Ser Ala Val Arg Gln Val Ala Glu Leu Val Ala Asp Arg
305                 310                 315                 320

Gly Arg


<210> 52
<211> 1044
<212> DNA
<213> Mesorhizobium loti

<400> 52
atggcacaga agaaagcttt cgaggtcgat tcctggctcg cgcggcctga cgcgcgcatc 60

tccatcgtcc tgctctacgg ccccgatcgc ggcctcgtct ccgagcgtgc aaaggccttt 120

gccgaaaaga ccggcctgcc gctcgacgat ccgttttccg tggtccggct cgaggggtcg 180

gaggtcgacc gcgacgaagg ccggctgctc gacgaagccc gcaccgtgcc gatgttttcc 240

gaccgccgcc tgctctgggt gcgcaatgcc accggccaga aggcgctggc cgaggacgtc 300

aaggcgctga cagcggagcc cgcgcgcgat gccatcatcc tcatcgaggc cggcgacctc 360

aagaagggcg tcggcttgcg cgccgtggtc gagggcgccg acaatgccat ggcgctgcct 420

tgctacgccg acgaggcccg cgacatcgac acggtcatcg acgatgaatt gcgcaaggcc 480

ggcatgtcga tgacgctgga ggcgaggcag gcgctgcgcc gcaatctcgg cggcgacagg 540

ctcgcttcgc gcggcgagat cgaaaaactc atcctctacg cccacggcca gaaggagatc 600

ggcctggagg atgtcagggc gatgtcgggc gacgtgtccg gcacctcgtt cgacgacgcc 660

gtcgacgcgc tgctggaagg caaggtcggc gacttcgaca ccgccttcac ccgccactgc 720

cagtccggcg gcccaccctt cctggtgctg tcctcggcga tgcgccagct gcagcagatc 780

caaatcatgc gcggccagat ggactctggc gggcgcaatg ccgcgtcggt ggtcgccgcc 840

gcgcgcccgc cggttttctt ttcgcggcgc aagctggtgg agaaggcgct cgagcgctgg 900

agcagcgatg cgctgagccg cgcgctaacc cggctgcaga ccgcggtgct gcagacccgc 960

aaacggcccg atctgtcaga ggcgctggcg cgccaggcgc tgctcggcat tgcggtggag 1020

agcgcgaggc tggggcagcg ttag 1044

<210> 53
<211> 1044
<212> DNA
<213> Mesorhizobium loti

<220>
<221> CDS
<222> (1)..(1044)

<400> 53
atg gca cag aag aaa gct ttc gag gtc gat tcc tgg ctc gcg cgg cct 48
Met Ala Gln Lys Lys Ala Phe Glu Val Asp Ser Trp Leu Ala Arg Pro
1               5                   10                  15

gac gcg cgc atc tcc atc gtc ctg ctc tac ggc ccc gat cgc ggc ctc 96
Asp Ala Arg Ile Ser Ile Val Leu Leu Tyr Gly Pro Asp Arg Gly Leu
            20                  25                  30

gtc tcc gag cgt gca aag gcc ttt gcc gaa aag acc ggc ctg ccg ctc 144
Val Ser Glu Arg Ala Lys Ala Phe Ala Glu Lys Thr Gly Leu Pro Leu
        35                  40                  45

gac gat ccg ttt tcc gtg gtc cgg ctc gag ggg tcg gag gtc gac cgc 192
Asp Asp Pro Phe Ser Val Val Arg Leu Glu Gly Ser Glu Val Asp Arg
    50                  55                  60

gac gaa ggc cgg ctg ctc gac gaa gcc cgc acc gtg ccg atg ttt tcc 240
Asp Glu Gly Arg Leu Leu Asp Glu Ala Arg Thr Val Pro Met Phe Ser
65                  70                  75                  80

gac cgc cgc ctg ctc tgg gtg cgc aat gcc acc ggc cag aag gcg ctg 288

Asp Arg Arg Leu Leu Trp Val Arg Asn Ala Thr Gly Gln Lys Ala Leu
                85                  90                  95

gcc gag gac gtc aag gcg ctg aca gcg gag ccc gcg cgc gat gcc atc 336
Ala Glu Asp Val Lys Ala Leu Thr Ala Glu Pro Ala Arg Asp Ala Ile
            100                 105                 110

atc ctc atc gag gcc ggc gac ctc aag aag ggc gtc ggc ttg cgc gcc 384
Ile Leu Ile Glu Ala Gly Asp Leu Lys Lys Gly Val Gly Leu Arg Ala
        115                 120                 125

gtg gtc gag ggc gcc gac aat gcc atg gcg ctg cct tgc tac gcc gac 432
Val Val Glu Gly Ala Asp Asn Ala Met Ala Leu Pro Cys Tyr Ala Asp
    130                 135                 140

gag gcc cgc gac atc gac acg gtc atc gac gat gaa ttg cgc aag gcc 480
Glu Ala Arg Asp Ile Asp Thr Val Ile Asp Asp Glu Leu Arg Lys Ala
145                 150                 155                 160

ggc atg tcg atg acg ctg gag gcg agg cag gcg ctg cgc cgc aat ctc 528
Gly Met Ser Met Thr Leu Glu Ala Arg Gln Ala Leu Arg Arg Asn Leu
                165                 170                 175

ggc ggc gac agg ctc gct tcg cgc ggc gag atc gaa aaa ctc atc ctc 576
Gly Gly Asp Arg Leu Ala Ser Arg Gly Glu Ile Glu Lys Leu Ile Leu
            180                 185                 190

tac gcc cac ggc cag aag gag atc ggc ctg gag gat gtc agg gcg atg 624
Tyr Ala His Gly Gln Lys Glu Ile Gly Leu Glu Asp Val Arg Ala Met
        195                 200                 205

tcg ggc gac gtg tcc ggc acc tcg ttc gac gac gcc gtc gac gcg ctg 672
Ser Gly Asp Val Ser Gly Thr Ser Phe Asp Asp Ala Val Asp Ala Leu
    210                 215                 220

ctg gaa ggc aag gtc ggc gac ttc gac acc gcc ttc acc cgc cac tgc 720
Leu Glu Gly Lys Val Gly Asp Phe Asp Thr Ala Phe Thr Arg His Cys
225                 230                 235                 240

cag tcc ggc ggc cca ccc ttc ctg gtg ctg tcc tcg gcg atg cgc cag 768
Gln Ser Gly Gly Pro Pro Phe Leu Val Leu Ser Ser Ala Met Arg Gln
                245                 250                 255

ctg cag cag atc caa atc atg cgc ggc cag atg gac tct ggc ggg cgc 816
Leu Gln Gln Ile Gln Ile Met Arg Gly Gln Met Asp Ser Gly Gly Arg
            260                 265                 270

aat gcc gcg tcg gtg gtc gcc gcc gcg cgc ccg ccg gtt ttc ttt tcg 864

Asn Ala Ala Ser Val Val Ala Ala Ala Arg Pro Pro Val Phe Phe Ser
        275                 280                 285

cgg cgc aag ctg gtg gag aag gcg ctc gag cgc tgg agc agc gat gcg     912
Arg Arg Lys Leu Val Glu Lys Ala Leu Glu Arg Trp Ser Ser Asp Ala
    290                 295                 300

ctg agc cgc gcg cta acc cgg ctg cag acc gcg gtg ctg cag acc cgc     960
Leu Ser Arg Ala Leu Thr Arg Leu Gln Thr Ala Val Leu Gln Thr Arg
305                 310                 315                 320

aaa cgg ccc gat ctg tca gag gcg ctg gcg cgc cag gcg ctg ctc ggc     1008
Lys Arg Pro Asp Leu Ser Glu Ala Leu Ala Arg Gln Ala Leu Leu Gly
                325                 330                 335

att gcg gtg gag agc gcg agg ctg ggg cag cgt tag                     1044
Ile Ala Val Glu Ser Ala Arg Leu Gly Gln Arg
            340                 345

<210> 54
<211> 347
<212> PRT
<213> Mesorhizobium loti

<400> 54
Met Ala Gln Lys Lys Ala Phe Glu Val Asp Ser Trp Leu Ala Arg Pro
  1               5                  10                  15

Asp Ala Arg Ile Ser Ile Val Leu Leu Tyr Gly Pro Asp Arg Gly Leu
            20                  25                  30

Val Ser Glu Arg Ala Lys Ala Phe Ala Glu Lys Thr Gly Leu Pro Leu
        35                  40                  45

Asp Asp Pro Phe Ser Val Val Arg Leu Glu Gly Ser Glu Val Asp Arg
    50                  55                  60

Asp Glu Gly Arg Leu Leu Asp Glu Ala Arg Thr Val Pro Met Phe Ser
 65                  70                  75                  80

Asp Arg Arg Leu Leu Trp Val Arg Asn Ala Thr Gly Gln Lys Ala Leu
                 85                  90                  95

Ala Glu Asp Val Lys Ala Leu Thr Ala Glu Pro Ala Arg Asp Ala Ile
            100                 105                 110

Ile Leu Ile Glu Ala Gly Asp Leu Lys Lys Gly Val Gly Leu Arg Ala

115                 120                 125

Val Val Glu Gly Ala Asp Asn Ala Met Ala Leu Pro Cys Tyr Ala Asp
    130                 135                 140

Glu Ala Arg Asp Ile Asp Thr Val Ile Asp Asp Glu Leu Arg Lys Ala
145                 150                 155                 160

Gly Met Ser Met Thr Leu Glu Ala Arg Gln Ala Leu Arg Arg Asn Leu
                165                 170                 175

Gly Gly Asp Arg Leu Ala Ser Arg Gly Glu Ile Glu Lys Leu Ile Leu
            180                 185                 190

Tyr Ala His Gly Gln Lys Glu Ile Gly Leu Glu Asp Val Arg Ala Met
        195                 200                 205

Ser Gly Asp Val Ser Gly Thr Ser Phe Asp Asp Ala Val Asp Ala Leu
    210                 215                 220

Leu Glu Gly Lys Val Gly Asp Phe Asp Thr Ala Phe Thr Arg His Cys
225                 230                 235                 240

Gln Ser Gly Gly Pro Pro Phe Leu Val Leu Ser Ser Ala Met Arg Gln
                245                 250                 255

Leu Gln Gln Ile Gln Ile Met Arg Gly Gln Met Asp Ser Gly Gly Arg
            260                 265                 270

Asn Ala Ala Ser Val Val Ala Ala Ala Arg Pro Pro Val Phe Phe Ser
        275                 280                 285

Arg Arg Lys Leu Val Glu Lys Ala Leu Glu Arg Trp Ser Ser Asp Ala
    290                 295                 300

Leu Ser Arg Ala Leu Thr Arg Leu Gln Thr Ala Val Leu Gln Thr Arg
305                 310                 315                 320

Lys Arg Pro Asp Leu Ser Glu Ala Leu Ala Arg Gln Ala Leu Leu Gly
                325                 330                 335

Ile Ala Val Glu Ser Ala Arg Leu Gly Gln Arg
            340                 345

<210> 55
<211> 894
<212> DNA

<213> Mycoplasma genitalium

<400> 55
atgacaataa tttatggtca agatattggg ctaattcacc aaaaattaag tcaaatcaaa 60

agcaattctc catataaaac tatttggttt aaagatctta agcaacttta tgatctcttt 120

tcacaaccat tatttggttc taataatgaa aagtttattg tcaataattg cagttttttg 180

gaaaaaacaa gcttaactag acaagaaaac ttgtgtttag aaaaactaaa aacaactgat 240

gtagttttaa ctgtttatac tgacaatcct tttagtggga ttaaaaccat taaatcaatc 300

acaactgttt tttgtgataa attggattgg aaatcaatgc ataaagcaat tggtgaagtt 360

tgtagagaac ttaacttaaa acttgatttg gaaataattg attggttagc gaatgcttta 420

cctttaaata tgggggtaat ttaccaagaa attaacaaac ttagcttgtt aggtaaaaat 480

gaaatcaaag ataacaaatt agttgaaaca gttatttgtg attatcaacc tgttcaaatt 540

tacaaattaa ccaaagctct aacaaattca caaattgtaa aggcatttaa atatattgat 600

gaattggcaa gtacaaaacc aaattttgcc acccaatttt tagaattttt tagtggggag 660

ttgttattag cattgatggt taaaagttgt aatccaaagc aactaactaa cattaatcta 720

aatgttaatc agtttcgctt aattgctatt caaagtcagt accataactt tactagcaaa 780

gttttagtaa atataattaa tgcaattcaa aaattggata ttaaattaaa gcacaatgat 840

ggctttgcca ttccgttgtt aaaaaacttt gctttaagct tttttactaa ctaa 894


<210> 56
<211> 894
<212> DNA
<213> Mycoplasma genitalium

<220>
<221> CDS
<222> (1)..(894)

<400> 56
atg aca ata att tat ggt caa gat att ggg cta att cac caa aaa tta 48
Met Thr Ile Ile Tyr Gly Gln Asp Ile Gly Leu Ile His Gln Lys Leu
1               5                   10                  15

agt caa atc aaa agc aat tct cca tat aaa act att tgg ttt aaa gat    96
Ser Gln Ile Lys Ser Asn Ser Pro Tyr Lys Thr Ile Trp Phe Lys Asp
            20                  25                  30

ctt aag caa ctt tat gat ctc ttt tca caa cca tta ttt ggt tct aat    144
Leu Lys Gln Leu Tyr Asp Leu Phe Ser Gln Pro Leu Phe Gly Ser Asn
        35                  40                  45

aat gaa aag ttt att gtc aat aat tgc agt ttt ttg gaa aaa aca agc    192
Asn Glu Lys Phe Ile Val Asn Asn Cys Ser Phe Leu Glu Lys Thr Ser
    50                  55                  60

tta act aga caa gaa aac ttg tgt tta gaa aaa cta aaa aca act gat    240
Leu Thr Arg Gln Glu Asn Leu Cys Leu Glu Lys Leu Lys Thr Thr Asp
65                  70                  75                  80

gta gtt tta act gtt tat act gac aat cct ttt agt ggg att aaa acc    288
Val Val Leu Thr Val Tyr Thr Asp Asn Pro Phe Ser Gly Ile Lys Thr
                85                  90                  95

att aaa tca atc aca act gtt ttt tgt gat aaa ttg gat tgg aaa tca    336
Ile Lys Ser Ile Thr Thr Val Phe Cys Asp Lys Leu Asp Trp Lys Ser
            100                 105                 110

atg cat aaa gca att ggt gaa gtt tgt aga gaa ctt aac tta aaa ctt    384
Met His Lys Ala Ile Gly Glu Val Cys Arg Glu Leu Asn Leu Lys Leu
        115                 120                 125

gat ttg gaa ata att gat tgg tta gcg aat gct tta cct tta aat atg    432
Asp Leu Glu Ile Ile Asp Trp Leu Ala Asn Ala Leu Pro Leu Asn Met
    130                 135                 140

ggg gta att tac caa gaa att aac aaa ctt agc ttg tta ggt aaa aat    480
Gly Val Ile Tyr Gln Glu Ile Asn Lys Leu Ser Leu Leu Gly Lys Asn
145                 150                 155                 160

gaa atc aaa gat aac aaa tta gtt gaa aca gtt att tgt gat tat caa    528
Glu Ile Lys Asp Asn Lys Leu Val Glu Thr Val Ile Cys Asp Tyr Gln
                165                 170                 175

cct gtt caa att tac aaa tta acc aaa gct cta aca aat tca caa att    576
Pro Val Gln Ile Tyr Lys Leu Thr Lys Ala Leu Thr Asn Ser Gln Ile
            180                 185                 190

gta aag gca ttt aaa tat att gat gaa ttg gca agt aca aaa cca aat    624
Val Lys Ala Phe Lys Tyr Ile Asp Glu Leu Ala Ser Thr Lys Pro Asn
        195                 200                 205

ttt gcc acc caa ttt tta gaa ttt ttt agt ggg gag ttg tta tta gca 672
Phe Ala Thr Gln Phe Leu Glu Phe Phe Ser Gly Glu Leu Leu Leu Ala
    210                 215                 220

ttg atg gtt aaa agt tgt aat cca aag caa cta act aac att aat cta 720
Leu Met Val Lys Ser Cys Asn Pro Lys Gln Leu Thr Asn Ile Asn Leu
225                 230                 235                 240

aat gtt aat cag ttt cgc tta att gct att caa agt cag tac cat aac 768
Asn Val Asn Gln Phe Arg Leu Ile Ala Ile Gln Ser Gln Tyr His Asn
                245                 250                 255

ttt act agc aaa gtt tta gta aat ata att aat gca att caa aaa ttg 816
Phe Thr Ser Lys Val Leu Val Asn Ile Ile Asn Ala Ile Gln Lys Leu
            260                 265                 270

gat att aaa tta aag cac aat gat ggc ttt gcc att ccg ttg tta aaa 864
Asp Ile Lys Leu Lys His Asn Asp Gly Phe Ala Ile Pro Leu Leu Lys
        275                 280                 285

aac ttt gct tta agc ttt ttt act aac taa 894
Asn Phe Ala Leu Ser Phe Phe Thr Asn
    290                 295

<210> 57
<211> 297
<212> PRT
<213> Mycoplasma genitalium

<400> 57
Met Thr Ile Ile Tyr Gly Gln Asp Ile Gly Leu Ile His Gln Lys Leu
1               5                   10                  15

Ser Gln Ile Lys Ser Asn Ser Pro Tyr Lys Thr Ile Trp Phe Lys Asp
            20                  25                  30

Leu Lys Gln Leu Tyr Asp Leu Phe Ser Gln Pro Leu Phe Gly Ser Asn
        35                  40                  45

Asn Glu Lys Phe Ile Val Asn Asn Cys Ser Phe Leu Glu Lys Thr Ser
    50                  55                  60

Leu Thr Arg Gln Glu Asn Leu Cys Leu Glu Lys Leu Lys Thr Thr Asp
65                  70                  75                  80

Val Val Leu Thr Val Tyr Thr Asp Asn Pro Phe Ser Gly Ile Lys Thr
                85                  90                  95

Ile Lys Ser Ile Thr Thr Val Phe Cys Asp Lys Leu Asp Trp Lys Ser
            100                 105                 110

Met His Lys Ala Ile Gly Glu Val Cys Arg Glu Leu Asn Leu Lys Leu
        115                 120                 125

Asp Leu Glu Ile Ile Asp Trp Leu Ala Asn Ala Leu Pro Leu Asn Met
    130                 135                 140

Gly Val Ile Tyr Gln Glu Ile Asn Lys Leu Ser Leu Leu Gly Lys Asn
145                 150                 155                 160

Glu Ile Lys Asp Asn Lys Leu Val Glu Thr Val Ile Cys Asp Tyr Gln
                165                 170                 175

Pro Val Gln Ile Tyr Lys Leu Thr Lys Ala Leu Thr Asn Ser Gln Ile
            180                 185                 190

Val Lys Ala Phe Lys Tyr Ile Asp Glu Leu Ala Ser Thr Lys Pro Asn
        195                 200                 205

Phe Ala Thr Gln Phe Leu Glu Phe Phe Ser Gly Glu Leu Leu Leu Ala
    210                 215                 220

Leu Met Val Lys Ser Cys Asn Pro Lys Gln Leu Thr Asn Ile Asn Leu
225                 230                 235                 240

Asn Val Asn Gln Phe Arg Leu Ile Ala Ile Gln Ser Gln Tyr His Asn
                245                 250                 255

Phe Thr Ser Lys Val Leu Val Asn Ile Ile Asn Ala Ile Gln Lys Leu
            260                 265                 270

Asp Ile Lys Leu Lys His Asn Asp Gly Phe Ala Ile Pro Leu Leu Lys
        275                 280                 285

Asn Phe Ala Leu Ser Phe Phe Thr Asn
    290                 295

<210> 58
<211> 945
<212> DNA
<213> Mycoplasma pneumoniae

<400> 58
atgggctgtg gggcttgttg tatactgtgg gtacagtctt ttactatgac cgtagtttat 60

ggtgccgata ttgggttaat tcaccagcag ttaaaccaac tgctcaaccc agccgcttgt 120

aaacaggtct ggtttcaaga tgttaataag ctgtatgatg tggtgttaaa ccagaatctg 180

tttgctgagg acactaaacc gatcctcatt cacaactgca gttttttgga aaagaacaac 240

ttaactaagg ctgaattaca ctgcttaaaa acacttaaag atactgatgt ggtggtcacc 300

atttacagcg acagtcccgc taatgccctc attaatgacc gggcaattac caagtatgct 360

tgtaaaccag tcacagctaa aaccatccac caggtgatca gtaaggccgc taagacactc 420

aaactcaact taaaccctga tttaattgac cacttagcca cgattctacc ctttaactta 480

ggggtcattg aacaggagtt gcgtaagtta actctactta gcccggcaga attacaggac 540

aaaaaaatgt tagaagccgt tttgtgtgat taccaaacca gtcaaatctt acaattaact 600

gacgccatgg tgcgtttgca aacagctaaa gccttaaagt tgattgaacg cttgttttta 660

ccaaagcagc tcacaccacc gcaattccta gaatttttag caaatgaact acttttggcc 720

ttgatggtca aaggcgttaa accacaagct gttttccaac tacagtggaa tgtcaatccc 780

tttcgtttaa aggcgattca aagccagtac cgcttttggt ccaccaacca attaacggcc 840

ttaattaatg cgatttggca gctggatatt aaaattaagc gcaatgatgg cttagcaatc 900

catttgctaa agcacttcgt gttgcgattc tttgcgcaaa agtaa 945

<210> 59
<211> 945
<212> DNA
<213> Mycoplasma pneumoniae

<220>
<221> CDS
<222> (1)..(945)

<400> 59
atg ggc tgt ggg gct tgt tgt ata ctg tgg gta cag tct ttt act atg 48
Met Gly Cys Gly Ala Cys Cys Ile Leu Trp Val Gln Ser Phe Thr Met
1               5                   10                  15

acc gta gtt tat ggt gcc gat att ggg tta att cac cag cag tta aac 96
Thr Val Val Tyr Gly Ala Asp Ile Gly Leu Ile His Gln Gln Leu Asn

20                  25                  30

caa ctg ctc aac cca gcc gct tgt aaa cag gtc tgg ttt caa gat gtt     144
Gln Leu Leu Asn Pro Ala Ala Cys Lys Gln Val Trp Phe Gln Asp Val
        35                  40                  45

aat aag ctg tat gat gtg gtg tta aac cag aat ctg ttt gct gag gac     192
Asn Lys Leu Tyr Asp Val Val Leu Asn Gln Asn Leu Phe Ala Glu Asp
    50                  55                  60

act aaa ccg atc ctc att cac aac tgc agt ttt ttg gaa aag aac aac     240
Thr Lys Pro Ile Leu Ile His Asn Cys Ser Phe Leu Glu Lys Asn Asn
65                  70                  75                  80

tta act aag gct gaa tta cac tgc tta aaa aca ctt aaa gat act gat     288
Leu Thr Lys Ala Glu Leu His Cys Leu Lys Thr Leu Lys Asp Thr Asp
                85                  90                  95

gtg gtg gtc acc att tac agc gac agt ccc gct aat gcc ctc att aat     336
Val Val Val Thr Ile Tyr Ser Asp Ser Pro Ala Asn Ala Leu Ile Asn
            100                 105                 110

gac cgg gca att acc aag tat gct tgt aaa cca gtc aca gct aaa acc     384
Asp Arg Ala Ile Thr Lys Tyr Ala Cys Lys Pro Val Thr Ala Lys Thr
        115                 120                 125

atc cac cag gtg atc agt aag gcc gct aag aca ctc aaa ctc aac tta     432
Ile His Gln Val Ile Ser Lys Ala Ala Lys Thr Leu Lys Leu Asn Leu
    130                 135                 140

aac cct gat tta att gac cac tta gcc acg att cta ccc ttt aac tta     480
Asn Pro Asp Leu Ile Asp His Leu Ala Thr Ile Leu Pro Phe Asn Leu
145                 150                 155                 160

ggg gtc att gaa cag gag ttg cgt aag tta act cta ctt agc ccg gca     528
Gly Val Ile Glu Gln Glu Leu Arg Lys Leu Thr Leu Leu Ser Pro Ala
                165                 170                 175

gaa tta cag gac aaa aaa atg tta gaa gcc gtt ttg tgt gat tac caa     576
Glu Leu Gln Asp Lys Lys Met Leu Glu Ala Val Leu Cys Asp Tyr Gln
            180                 185                 190

acc agt caa atc tta caa tta act gac gcc atg gtg cgt ttg caa aca     624
Thr Ser Gln Ile Leu Gln Leu Thr Asp Ala Met Val Arg Leu Gln Thr
        195                 200                 205

gct aaa gcc tta aag ttg att gaa cgc ttg ttt tta cca aag cag ctc     672
Ala Lys Ala Leu Lys Leu Ile Glu Arg Leu Phe Leu Pro Lys Gln Leu

210                 215                 220

aca cca ccg caa ttc cta gaa ttt tta gca aat gaa cta ctt ttg gcc     720
Thr Pro Pro Gln Phe Leu Glu Phe Leu Ala Asn Glu Leu Leu Leu Ala
225                 230                 235                 240

ttg atg gtc aaa ggc gtt aaa cca caa gct gtt ttc caa cta cag tgg     768
Leu Met Val Lys Gly Val Lys Pro Gln Ala Val Phe Gln Leu Gln Trp
                245                 250                 255

aat gtc aat ccc ttt cgt tta aag gcg att caa agc cag tac cgc ttt     816
Asn Val Asn Pro Phe Arg Leu Lys Ala Ile Gln Ser Gln Tyr Arg Phe
            260                 265                 270

tgg tcc acc aac caa tta acg gcc tta att aat gcg att tgg cag ctg     864
Trp Ser Thr Asn Gln Leu Thr Ala Leu Ile Asn Ala Ile Trp Gln Leu
        275                 280                 285

gat att aaa att aag cgc aat gat ggc tta gca atc cat ttg cta aag     912
Asp Ile Lys Ile Lys Arg Asn Asp Gly Leu Ala Ile His Leu Leu Lys
    290                 295                 300

cac ttc gtg ttg cga ttc ttt gcg caa aag taa                         945
His Phe Val Leu Arg Phe Phe Ala Gln Lys
305                 310                 315

<210> 60
<211> 314
<212> PRT
<213> Mycoplasma pneumoniae

<400> 60
Met Gly Cys Gly Ala Cys Cys Ile Leu Trp Val Gln Ser Phe Thr Met
  1               5                  10                  15

Thr Val Val Tyr Gly Ala Asp Ile Gly Leu Ile His Gln Gln Leu Asn
             20                  25                  30

Gln Leu Leu Asn Pro Ala Ala Cys Lys Gln Val Trp Phe Gln Asp Val
         35                  40                  45

Asn Lys Leu Tyr Asp Val Val Leu Asn Gln Asn Leu Phe Ala Glu Asp
     50                  55                  60

Thr Lys Pro Ile Leu Ile His Asn Cys Ser Phe Leu Glu Lys Asn Asn
 65                  70                  75                  80

Leu Thr Lys Ala Glu Leu His Cys Leu Lys Thr Leu Lys Asp Thr Asp
                85                  90                  95

Val Val Val Thr Ile Tyr Ser Asp Ser Pro Ala Asn Ala Leu Ile Asn
            100                 105                 110

Asp Arg Ala Ile Thr Lys Tyr Ala Cys Lys Pro Val Thr Ala Lys Thr
        115                 120                 125

Ile His Gln Val Ile Ser Lys Ala Ala Lys Thr Leu Lys Leu Asn Leu
    130                 135                 140

Asn Pro Asp Leu Ile Asp His Leu Ala Thr Ile Leu Pro Phe Asn Leu
145                 150                 155                 160

Gly Val Ile Glu Gln Glu Leu Arg Lys Leu Thr Leu Leu Ser Pro Ala
                165                 170                 175

Glu Leu Gln Asp Lys Lys Met Leu Glu Ala Val Leu Cys Asp Tyr Gln
            180                 185                 190

Thr Ser Gln Ile Leu Gln Leu Thr Asp Ala Met Val Arg Leu Gln Thr
        195                 200                 205

Ala Lys Ala Leu Lys Leu Ile Glu Arg Leu Phe Leu Pro Lys Gln Leu
    210                 215                 220

Thr Pro Pro Gln Phe Leu Glu Phe Leu Ala Asn Glu Leu Leu Leu Ala
225                 230                 235                 240

Leu Met Val Lys Gly Val Lys Pro Gln Ala Val Phe Gln Leu Gln Trp
                245                 250                 255

Asn Val Asn Pro Phe Arg Leu Lys Ala Ile Gln Ser Gln Tyr Arg Phe
            260                 265                 270

Trp Ser Thr Asn Gln Leu Thr Ala Leu Ile Asn Ala Ile Trp Gln Leu
        275                 280                 285

Asp Ile Lys Ile Lys Arg Asn Asp Gly Leu Ala Ile His Leu Leu Lys
    290                 295                 300

His Phe Val Leu Arg Phe Phe Ala Gln Lys
305                 310

<210> 61
<211> 999

<212> DNA
<213> Neisseria meningitidis MC58

<400> 61
gtggcggcac atatcggacg cattgatacg gacgcgcctt tgaaacccct gtacgtcatc 60

cacggcgagg aagaactgtt gcgtatcgag gcattggacg cattgagggc ggcggcgaag 120

aaacaaggtt accttaatcg ggaagtttat acggcagaca atgccttcga ttggaacgag 180

ctgctgcaaa ccgcaggcag tgcgggtctg tttgccgatt tgaagctgtt ggaactgcat 240

atccctaacg gcaagcccgg caaaaccggc ggcgaggcgt tgcaggattt tgccgcccga 300

ttgccggaag atacggtaac gctggttttg ctgcccaaac tggagaaaac ccagctccag 360

tccaaatggt ttgccgcatt ggcggcaaag ggggaagtgt gggaagccaa accggtcggc 420

gcggcggctt tgccccaatg gatacgcgga cggctggaca aaatcggttt gggtatcgag 480

gcagacgcat tggcactgtt tgctgagcgc gtggaaggca atctgttggc ggcgcgtcag 540

gaaatcgaca agctcgggct gctgtatccg aaagggcata ccgtcaatat cgatgaggcg 600

caaaccgccg ttgccaacgt cgcccgcttc gacgcgttcc aactggcagg cgcgtggatg 660

aagggcgatg tcctgcgcgt atgcaggctt ttggacggat tgcgggaaga gggcgaagaa 720

ccggtgctgt tgctgtgggc ggttgccgaa gacgtgcgga cgctgatccg gcttgctgcc 780

gccctgaagc aggggcagag catccaatcc gtccgcaaca gcctcaggct ttggggcgac 840

aagcagacgc tcgcaccgct tgcggtcaag cggatttccg tcgtccgcct gcttgacgcg 900

ctcaaaacct gcgcccaaat cgaccgaatc atcaaaggtg cggaagacgg cgacgcatgg 960

acggtattca aacggcttgt cgtgtcgctg gcggaataa 999

<210> 62
<211> 999
<212> DNA
<213> Neisseria meningitidis MC58

<220>
<221> CDS
<222> (1)..(999)

<400> 62
gtg gcg gca cat atc gga cgc att gat acg gac gcg cct ttg aaa ccc     48
Val Ala Ala His Ile Gly Arg Ile Asp Thr Asp Ala Pro Leu Lys Pro
1               5                   10                  15

ctg tac gtc atc cac ggc gag gaa gaa ctg ttg cgt atc gag gca ttg     96
Leu Tyr Val Ile His Gly Glu Glu Glu Leu Leu Arg Ile Glu Ala Leu
            20                  25                  30

gac gca ttg agg gcg gcg gcg aag aaa caa ggt tac ctt aat cgg gaa     144
Asp Ala Leu Arg Ala Ala Ala Lys Lys Gln Gly Tyr Leu Asn Arg Glu
        35                  40                  45

gtt tat acg gca gac aat gcc ttc gat tgg aac gag ctg ctg caa acc     192
Val Tyr Thr Ala Asp Asn Ala Phe Asp Trp Asn Glu Leu Leu Gln Thr
    50                  55                  60

gca ggc agt gcg ggt ctg ttt gcc gat ttg aag ctg ttg gaa ctg cat     240
Ala Gly Ser Ala Gly Leu Phe Ala Asp Leu Lys Leu Leu Glu Leu His
65                  70                  75                  80

atc cct aac ggc aag ccc ggc aaa acc ggc ggc gag gcg ttg cag gat     288
Ile Pro Asn Gly Lys Pro Gly Lys Thr Gly Gly Glu Ala Leu Gln Asp
                85                  90                  95

ttt gcc gcc cga ttg ccg gaa gat acg gta acg ctg gtt ttg ctg ccc     336
Phe Ala Ala Arg Leu Pro Glu Asp Thr Val Thr Leu Val Leu Leu Pro
            100                 105                 110

aaa ctg gag aaa acc cag ctc cag tcc aaa tgg ttt gcc gca ttg gcg     384
Lys Leu Glu Lys Thr Gln Leu Gln Ser Lys Trp Phe Ala Ala Leu Ala
        115                 120                 125

gca aag ggg gaa gtg tgg gaa gcc aaa ccg gtc ggc gcg gcg gct ttg     432
Ala Lys Gly Glu Val Trp Glu Ala Lys Pro Val Gly Ala Ala Ala Leu
    130                 135                 140

ccc caa tgg ata cgc gga cgg ctg gac aaa atc ggt ttg ggt atc gag     480
Pro Gln Trp Ile Arg Gly Arg Leu Asp Lys Ile Gly Leu Gly Ile Glu
145                 150                 155                 160

gca gac gca ttg gca ctg ttt gct gag cgc gtg gaa ggc aat ctg ttg     528
Ala Asp Ala Leu Ala Leu Phe Ala Glu Arg Val Glu Gly Asn Leu Leu
                165                 170                 175

gcg gcg cgt cag gaa atc gac aag ctc ggg ctg ctg tat ccg aaa ggg     576
Ala Ala Arg Gln Glu Ile Asp Lys Leu Gly Leu Leu Tyr Pro Lys Gly
            180                 185                 190

cat acc gtc aat atc gat gag gcg caa acc gcc gtt gcc aac gtc gcc     624
His Thr Val Asn Ile Asp Glu Ala Gln Thr Ala Val Ala Asn Val Ala
        195                 200                 205

cgc ttc gac gcg ttc caa ctg gca ggc gcg tgg atg aag ggc gat gtc     672
Arg Phe Asp Ala Phe Gln Leu Ala Gly Ala Trp Met Lys Gly Asp Val
    210                 215                 220

ctg cgc gta tgc agg ctt ttg gac gga ttg cgg gaa gag ggc gaa gaa     720
Leu Arg Val Cys Arg Leu Leu Asp Gly Leu Arg Glu Glu Gly Glu Glu
225                 230                 235                 240

ccg gtg ctg ttg ctg tgg gcg gtt gcc gaa gac gtg cgg acg ctg atc     768
Pro Val Leu Leu Leu Trp Ala Val Ala Glu Asp Val Arg Thr Leu Ile
                245                 250                 255

cgg ctt gct gcc gcc ctg aag cag ggg cag agc atc caa tcc gtc cgc     816
Arg Leu Ala Ala Ala Leu Lys Gln Gly Gln Ser Ile Gln Ser Val Arg
            260                 265                 270

aac agc ctc agg ctt tgg ggc gac aag cag acg ctc gca ccg ctt gcg     864
Asn Ser Leu Arg Leu Trp Gly Asp Lys Gln Thr Leu Ala Pro Leu Ala
        275                 280                 285

gtc aag cgg att tcc gtc gtc cgc ctg ctt gac gcg ctc aaa acc tgc     912
Val Lys Arg Ile Ser Val Val Arg Leu Leu Asp Ala Leu Lys Thr Cys
    290                 295                 300

gcc caa atc gac cga atc atc aaa ggt gcg gaa gac ggc gac gca tgg     960
Ala Gln Ile Asp Arg Ile Ile Lys Gly Ala Glu Asp Gly Asp Ala Trp
305                 310                 315                 320

acg gta ttc aaa cgg ctt gtc gtg tcg ctg gcg gaa taa                 999
Thr Val Phe Lys Arg Leu Val Val Ser Leu Ala Glu
                325                 330

<210> 63
<211> 332
<212> PRT
<213> Neisseria meningitidis MC58

<400> 63
Val Ala Ala His Ile Gly Arg Ile Asp Thr Asp Ala Pro Leu Lys Pro
1               5                   10                  15

Leu Tyr Val Ile His Gly Glu Glu Leu Leu Arg Ile Glu Ala Leu

            20                  25                  30

Asp Ala Leu Arg Ala Ala Ala Lys Lys Gln Gly Tyr Leu Asn Arg Glu
            35                  40                  45

Val Tyr Thr Ala Asp Asn Ala Phe Asp Trp Asn Glu Leu Leu Gln Thr
        50                  55                  60

Ala Gly Ser Ala Gly Leu Phe Ala Asp Leu Lys Leu Leu Glu Leu His
65 70 75 80

Ile Pro Asn Gly Lys Pro Gly Lys Thr Gly Gly Glu Ala Leu Gln Asp
85 90 95

Phe Ala Ala Arg Leu Pro Glu Asp Thr Val Thr Leu Val Leu Leu Pro
                100                 105                 110

Lys Leu Glu Lys Thr Gln Leu Gln Ser Lys Trp Phe Ala Ala Leu Ala
            115                 120                 125

Ala Lys Gly Glu Val Trp Glu Ala Lys Pro Val Gly Ala Ala Ala Leu
        130                 135                 140

Pro Gln Trp Ile Arg Gly Arg Leu Asp Lys Ile Gly Leu Gly Ile Glu
145 150 155 160

Ala Asp Ala Leu Ala Leu Phe Ala Glu Arg Val Glu Gly Asn Leu Leu
165 170 175

Ala Ala Arg Gln Glu Ile Asp Lys Leu Gly Leu Leu Tyr Pro Lys Gly
                180                 185                 190

His Thr Val Asn Ile Asp Glu Ala Gln Thr Ala Val Ala Asn Val Ala
            195                 200                 205

Arg Phe Asp Ala Phe Gln Leu Ala Gly Ala Trp Met Lys Gly Asp Val
        210                 215                 220

Leu Arg Val Cys Arg Leu Leu Asp Gly Leu Arg Glu Glu Gly Glu Glu
225 230 235 240

Pro Val Leu Leu Leu Trp Ala Val Ala Glu Asp Val Arg Thr Leu Ile
245 250 255

Arg Leu Ala Ala Ala Leu Lys Gln Gly Gln Ser Ile Gln Ser Val Arg
                260                 265                 270

Asn Ser Leu Arg Leu Trp Gly Asp Lys Gln Thr Leu Ala Pro Leu Ala

            275                 280                 285

Val Lys Arg Ile Ser Val Val Arg Leu Leu Asp Ala Leu Lys Thr Cys
        290                 295                 300

Ala Gln Ile Asp Arg Ile Ile Lys Gly Ala Glu Asp Gly Asp Ala Trp
305 310 315 320

Thr Val Phe Lys Arg Leu Val Val Ser Leu Ala Glu
325 330

<210> 64
<211> 999
<212> DNA
<213> Neisseria meningitidis Z2491

<400> 64
gtggcggcac atatcggacg cattgatacg gacgcgcctt tgaaacctct gtacgtcatc 60

cacggcgagg aagaactgtt gcgtatcgag gcattggacg cattgagggc ggcggcgaag 120

aaacaaggtt accttaatcg ggaagtttat acggcagaca atgccttcga ttggaacgaa 180

ctgttgcaaa ccgcaggcag tgcgggtctg tttgccgatt tgaagctgtt ggaactgcat 240

atccccaacg gaaaacccgg caaaaccggc ggcgaggcgt tgcaggattt tgccgcccga 300

ttgccggaag atacggtaac gctggttttg ctgcccaaac tggagaaaac ccagctccag 360

tccaaatggt ttgccgcatt ggcggcaaag ggggaagtgt gggaagccaa accggtcggc 420

gcggcggctt tgccccaatg gatacgcgga cggctggaca aaatcggttt gggtatcgag 480

gcagacgcat tggcactgtt tgctgagcgc gtggaaggca atctgttggc ggcgcgtcag 540

gaaatcgaca agctcgggct gctgtatccg aaagggcata ccgtcaatat cgatgaggcg 600

caaaccgccg ttgccaacgt cgcccgcttc gacgcgttcc aactggcagg cgcgtggatg 660

aagggcgatg tcctgcgcgt atgcaggctt ttggacggat tgcgggaaga gggcgaagaa 720

ccggtgctgt tgctgtgggc ggttgccgaa gacgtgcgga cgctgatccg gcttgctgcc 780

gccctgaagc aggggcagag catccaatcc gtccgcaaca gcctcaggct ttggggcgac 840

aagcagacgc tcgcaccgct tgcggtcaag cggatttccg tcgtccgcct gcttgacgcg 900

ctcaaaacct gcgcccaaat cgaccgaatc atcaaaggcg cggaagaggg cgacgcatgg 960

acggtattca aacggcttgt cgtgtcgctg gcggaataa 999

<210> 65
<211> 999
<212> DNA
<213> Neisseria meningitidis Z2491

<220>
<221> CDS
<222> (1)..(999)

<400> 65
gtg gcg gca cat atc gga cgc att gat acg gac gcg cct ttg aaa cct 48
Val Ala Ala His Ile Gly Arg Ile Asp Thr Asp Ala Pro Leu Lys Pro
1               5                   10                  15

ctg tac gtc atc cac ggc gag gaa gaa ctg ttg cgt atc gag gca ttg 96
Leu Tyr Val Ile His Gly Glu Glu Glu Leu Leu Arg Ile Glu Ala Leu
            20                  25                  30

gac gca ttg agg gcg gcg gcg aag aaa caa ggt tac ctt aat cgg gaa 144
Asp Ala Leu Arg Ala Ala Ala Lys Lys Gln Gly Tyr Leu Asn Arg Glu
        35                  40                  45

gtt tat acg gca gac aat gcc ttc gat tgg aac gaa ctg ttg caa acc 192
Val Tyr Thr Ala Asp Asn Ala Phe Asp Trp Asn Glu Leu Leu Gln Thr
    50                  55                  60

gca ggc agt gcg ggt ctg ttt gcc gat ttg aag ctg ttg gaa ctg cat 240
Ala Gly Ser Ala Gly Leu Phe Ala Asp Leu Lys Leu Leu Glu Leu His
65                  70                  75                  80

atc ccc aac gga aaa ccc ggc aaa acc ggc ggc gag gcg ttg cag gat 288
Ile Pro Asn Gly Lys Pro Gly Lys Thr Gly Gly Glu Ala Leu Gln Asp
                85                  90                  95

ttt gcc gcc cga ttg ccg gaa gat acg gta acg ctg gtt ttg ctg ccc 336
Phe Ala Ala Arg Leu Pro Glu Asp Thr Val Thr Leu Val Leu Leu Pro
            100                 105                 110

aaa ctg gag aaa acc cag ctc cag tcc aaa tgg ttt gcc gca ttg gcg 384
Lys Leu Glu Lys Thr Gln Leu Gln Ser Lys Trp Phe Ala Ala Leu Ala
        115                 120                 125

gca aag ggg gaa gtg tgg gaa gcc aaa ccg gtc ggc gcg gcg gct ttg 432

Ala Lys Gly Glu Val Trp Glu Ala Lys Pro Val Gly Ala Ala Ala Leu
    130                 135                 140

ccc caa tgg ata cgc gga cgg ctg gac aaa atc ggt ttg ggt atc gag     480
Pro Gln Trp Ile Arg Gly Arg Leu Asp Lys Ile Gly Leu Gly Ile Glu
145                 150                 155                 160

gca gac gca ttg gca ctg ttt gct gag cgc gtg gaa ggc aat ctg ttg     528
Ala Asp Ala Leu Ala Leu Phe Ala Glu Arg Val Glu Gly Asn Leu Leu
                165                 170                 175

gcg gcg cgt cag gaa atc gac aag ctc ggg ctg ctg tat ccg aaa ggg     576
Ala Ala Arg Gln Glu Ile Asp Lys Leu Gly Leu Leu Tyr Pro Lys Gly
            180                 185                 190

cat acc gtc aat atc gat gag gcg caa acc gcc gtt gcc aac gtc gcc     624
His Thr Val Asn Ile Asp Glu Ala Gln Thr Ala Val Ala Asn Val Ala
        195                 200                 205

cgc ttc gac gcg ttc caa ctg gca ggc gcg tgg atg aag ggc gat gtc     672
Arg Phe Asp Ala Phe Gln Leu Ala Gly Ala Trp Met Lys Gly Asp Val
    210                 215                 220

ctg cgc gta tgc agg ctt ttg gac gga ttg cgg gaa gag ggc gaa gaa     720
Leu Arg Val Cys Arg Leu Leu Asp Gly Leu Arg Glu Glu Gly Glu Glu
225                 230                 235                 240

ccg gtg ctg ttg ctg tgg gcg gtt gcc gaa gac gtg cgg acg ctg atc     768
Pro Val Leu Leu Leu Trp Ala Val Ala Glu Asp Val Arg Thr Leu Ile
                245                 250                 255

cgg ctt gct gcc gcc ctg aag cag ggg cag agc atc caa tcc gtc cgc     816
Arg Leu Ala Ala Ala Leu Lys Gln Gly Gln Ser Ile Gln Ser Val Arg
            260                 265                 270

aac agc ctc agg ctt tgg ggc gac aag cag acg ctc gca ccg ctt gcg     864
Asn Ser Leu Arg Leu Trp Gly Asp Lys Gln Thr Leu Ala Pro Leu Ala
        275                 280                 285

gtc aag cgg att tcc gtc gtc cgc ctg ctt gac gcg ctc aaa acc tgc     912
Val Lys Arg Ile Ser Val Val Arg Leu Leu Asp Ala Leu Lys Thr Cys
    290                 295                 300

gcc caa atc gac cga atc atc aaa ggc gcg gaa gag ggc gac gca tgg     960
Ala Gln Ile Asp Arg Ile Ile Lys Gly Ala Glu Glu Gly Asp Ala Trp
305                 310                 315                 320

acg gta ttc aaa cgg ctt gtc gtg tcg ctg gcg gaa taa                 999

Thr Val Phe Lys Arg Leu Val Val Ser Leu Ala Glu
                325                 330

<210> 66
<211> 332
<212> PRT
<213> Neisseria meningitidis Z2491

<400> 66
Val Ala Ala His Ile Gly Arg Ile Asp Thr Asp Ala Pro Leu Lys Pro
1               5                   10                  15

Leu Tyr Val Ile His Gly Glu Glu Glu Leu Leu Arg Ile Glu Ala Leu
            20                  25                  30

Asp Ala Leu Arg Ala Ala Ala Lys Lys Gln Gly Tyr Leu Asn Arg Glu
        35                  40                  45

Val Tyr Thr Ala Asp Asn Ala Phe Asp Trp Asn Glu Leu Leu Gln Thr
    50                  55                  60

Ala Gly Ser Ala Gly Leu Phe Ala Asp Leu Lys Leu Leu Glu Leu His
65                  70                  75                  80

Ile Pro Asn Gly Lys Pro Gly Lys Thr Gly Gly Glu Ala Leu Gln Asp
                85                  90                  95

Phe Ala Ala Arg Leu Pro Glu Asp Thr Val Thr Leu Val Leu Leu Pro
            100                 105                 110

Lys Leu Glu Lys Thr Gln Leu Gln Ser Lys Trp Phe Ala Ala Leu Ala
        115                 120                 125

Ala Lys Gly Glu Val Trp Glu Ala Lys Pro Val Gly Ala Ala Ala Leu
    130                 135                 140

Pro Gln Trp Ile Arg Gly Arg Leu Asp Lys Ile Gly Leu Gly Ile Glu
145                 150                 155                 160

Ala Asp Ala Leu Ala Leu Phe Ala Glu Arg Val Glu Gly Asn Leu Leu
                165                 170                 175

Ala Ala Arg Gln Glu Ile Asp Lys Leu Gly Leu Leu Tyr Pro Lys Gly
            180                 185                 190

His Thr Val Asn Ile Asp Glu Ala Gln Thr Ala Val Ala Asn Val Ala
        195                 200                 205

Arg Phe Asp Ala Phe Gln Leu Ala Gly Ala Trp Met Lys Gly Asp Val
    210                 215                 220

Leu Arg Val Cys Arg Leu Leu Asp Gly Leu Arg Glu Glu Gly Glu Glu
225                 230                 235                 240

Pro Val Leu Leu Leu Trp Ala Val Ala Glu Asp Val Arg Thr Leu Ile
                245                 250                 255

Arg Leu Ala Ala Ala Leu Lys Gln Gly Gln Ser Ile Gln Ser Val Arg
            260                 265                 270

Asn Ser Leu Arg Leu Trp Gly Asp Lys Gln Thr Leu Ala Pro Leu Ala
        275                 280                 285

Val Lys Arg Ile Ser Val Val Arg Leu Leu Asp Ala Leu Lys Thr Cys
    290                 295                 300

Ala Gln Ile Asp Arg Ile Ile Lys Gly Ala Glu Glu Gly Asp Ala Trp
305                 310                 315                 320

Thr Val Phe Lys Arg Leu Val Val Ser Leu Ala Glu
                325                 330

<210> 67
<211> 1080
<212> DNA
<213> Pasteurella multocida

<400> 67
atgcaacgga ttttcacaga acaattagga tctgctctac aaaacaaact agccaagctc 60

tattatttcg tagggcaaga tccgttactg ttaagtgaaa atcaagatcg cataaatcaa 120

tatgcattca agcaaggttt tgacgaaaaa aatgtcatca caattgatca gaatactaat 180

tggtctgatc tctttgaacg ctgccaatcc attggcttgt ttttcaataa acagattttg 240

tttcttaatt taccggaaaa cctcagtgca agtttacaaa ctcgtttaca agaactgatc 300

tcactcctca acgatgacat cctgttaata ctacaattgc ctaaactgaa caaagcgact 360

gaaaaacaaa catggtttat ccaagctaat cagtatgaac cggaagccgt actcgtgcat 420

tgccaaaccc caaccattga acaattgcct cgttggatcg cgttgcgtgc gaaaagtatg 480

ggactacaga ttgaagagca agcaatccaa cttctttgtt atagccatga aaataactta 540

ttagccttaa aacaaagttt gcagctcctt gctttactct atcctgacaa caaaatcagt 600

tatgcacgag tacaatccac tatcgctcag acctcagtgt tcacaccatt tcaatggctc 660

gatgcgctat tagaaggcaa aagtaaacgc gcccaacgta ttttagaagg attaaaagca 720

gaggaaatgg agcccattat tttattgcgg acgttacaac gagatttgct caccttatta 780

ggattggcga agccatcaca tcctgtcacc cttaccacgc ccctcccagc tcatcaactg 840

cgtgaacaat ttgatcaatt aaaagtgtgg caaaatcgac gccctttctt tactcaagcc 900

tttaaacgct tgaattacaa acagctttat ttgattattc aagcgctagc agagatcgaa 960

cgtttagcga aacacacatt tagccaagat atttgggatc gtttagccga actttctgct 1020

ttcatttgtt taccagatga tcccaatcac ctactatctc taccaacttt ctctcgttga 1080

<210> 68
<211> 1080
<212> DNA
<213> Pasteurella multocida

<220>
<221> CDS
<222> (1)..(1080)

<400> 68
atg caa cgg att ttc aca gaa caa tta gga tct gct cta caa aac aaa 48
Met Gln Arg Ile Phe Thr Glu Gln Leu Gly Ser Ala Leu Gln Asn Lys
1               5                   10                  15

cta gcc aag ctc tat tat ttc gta ggg caa gat ccg tta ctg tta agt 96
Leu Ala Lys Leu Tyr Tyr Phe Val Gly Gln Asp Pro Leu Leu Leu Ser
            20                  25                  30

gaa aat caa gat cgc ata aat caa tat gca ttc aag caa ggt ttt gac 144
Glu Asn Gln Asp Arg Ile Asn Gln Tyr Ala Phe Lys Gln Gly Phe Asp
        35                  40                  45

gaa aaa aat gtc atc aca att gat cag aat act aat tgg tct gat ctc 192
Glu Lys Asn Val Ile Thr Ile Asp Gln Asn Thr Asn Trp Ser Asp Leu
    50                  55                  60

ttt gaa cgc tgc caa tcc att ggc ttg ttt ttc aat aaa cag att ttg 240

Phe Glu Arg Cys Gln Ser Ile Gly Leu Phe Phe Asn Lys Gln Ile Leu
65                  70                  75                  80

ttt ctt aat tta ccg gaa aac ctc agt gca agt tta caa act cgt tta    288
Phe Leu Asn Leu Pro Glu Asn Leu Ser Ala Ser Leu Gln Thr Arg Leu
                85                  90                  95

caa gaa ctg atc tca ctc ctc aac gat gac atc ctg tta ata cta caa    336
Gln Glu Leu Ile Ser Leu Leu Asn Asp Asp Ile Leu Leu Ile Leu Gln
            100                 105                 110

ttg cct aaa ctg aac aaa gcg act gaa aaa caa aca tgg ttt atc caa    384
Leu Pro Lys Leu Asn Lys Ala Thr Glu Lys Gln Thr Trp Phe Ile Gln
        115                 120                 125

gct aat cag tat gaa ccg gaa gcc gta ctc gtg cat tgc caa acc cca    432
Ala Asn Gln Tyr Glu Pro Glu Ala Val Leu Val His Cys Gln Thr Pro
    130                 135                 140

acc att gaa caa ttg cct cgt tgg atc gcg ttg cgt gcg aaa agt atg    480
Thr Ile Glu Gln Leu Pro Arg Trp Ile Ala Leu Arg Ala Lys Ser Met
145                 150                 155                 160

gga cta cag att gaa gag caa gca atc caa ctt ctt tgt tat agc cat    528
Gly Leu Gln Ile Glu Glu Gln Ala Ile Gln Leu Leu Cys Tyr Ser His
                165                 170                 175

gaa aat aac tta tta gcc tta aaa caa agt ttg cag ctc ctt gct tta    576
Glu Asn Asn Leu Leu Ala Leu Lys Gln Ser Leu Gln Leu Leu Ala Leu
            180                 185                 190

ctc tat cct gac aac aaa atc agt tat gca cga gta caa tcc act atc    624
Leu Tyr Pro Asp Asn Lys Ile Ser Tyr Ala Arg Val Gln Ser Thr Ile
        195                 200                 205

gct cag acc tca gtg ttc aca cca ttt caa tgg ctc gat gcg cta tta    672
Ala Gln Thr Ser Val Phe Thr Pro Phe Gln Trp Leu Asp Ala Leu Leu
    210                 215                 220

gaa ggc aaa agt aaa cgc gcc caa cgt att tta gaa gga tta aaa gca    720
Glu Gly Lys Ser Lys Arg Ala Gln Arg Ile Leu Glu Gly Leu Lys Ala
225                 230                 235                 240

gag gaa atg gag ccc att att tta ttg cgg acg tta caa cga gat ttg    768
Glu Glu Met Glu Pro Ile Ile Leu Leu Arg Thr Leu Gln Arg Asp Leu
                245                 250                 255

ctc acc tta tta gga ttg gcg aag cca tca cat cct gtc acc ctt acc    816

Leu Thr Leu Leu Gly Leu Ala Lys Pro Ser His Pro Val Thr Leu Thr
            260                 265                 270

acg ccc ctc cca gct cat caa ctg cgt gaa caa ttt gat caa tta aaa 864
Thr Pro Leu Pro Ala His Gln Leu Arg Glu Gln Phe Asp Gln Leu Lys
        275                 280                 285

gtg tgg caa aat cga cgc cct ttc ttt act caa gcc ttt aaa cgc ttg 912
Val Trp Gln Asn Arg Arg Pro Phe Phe Thr Gln Ala Phe Lys Arg Leu
    290                 295                 300

aat tac aaa cag ctt tat ttg att att caa gcg cta gca gag atc gaa 960
Asn Tyr Lys Gln Leu Tyr Leu Ile Ile Gln Ala Leu Ala Glu Ile Glu
305                 310                 315                 320

cgt tta gcg aaa cac aca ttt agc caa gat att tgg gat cgt tta gcc 1008
Arg Leu Ala Lys His Thr Phe Ser Gln Asp Ile Trp Asp Arg Leu Ala
                325                 330                 335

gaa ctt tct gct ttc att tgt tta cca gat gat ccc aat cac cta cta 1056
Glu Leu Ser Ala Phe Ile Cys Leu Pro Asp Asp Pro Asn His Leu Leu
            340                 345                 350

tct cta cca act ttc tct cgt tga 1080
Ser Leu Pro Thr Phe Ser Arg
355 360

<210> 69
<211> 359
<212> PRT
<213> Pasteurella multocida

<400> 69
Met Gln Arg Ile Phe Thr Glu Gln Leu Gly Ser Ala Leu Gln Asn Lys
1               5                   10                  15

Leu Ala Lys Leu Tyr Tyr Phe Val Gly Gln Asp Pro Leu Leu Leu Ser
            20                  25                  30

Glu Asn Gln Asp Arg Ile Asn Gln Tyr Ala Phe Lys Gln Gly Phe Asp
        35                  40                  45

Glu Lys Asn Val Ile Thr Ile Asp Gln Asn Thr Asn Trp Ser Asp Leu
    50                  55                  60

Phe Glu Arg Cys Gln Ser Ile Gly Leu Phe Phe Asn Lys Gln Ile Leu
65                  70                  75                  80

Phe Leu Asn Leu Pro Glu Asn Leu Ser Ala Ser Leu Gln Thr Arg Leu
                85                  90                  95

Gln Glu Leu Ile Ser Leu Leu Asn Asp Asp Ile Leu Leu Ile Leu Gln
            100                 105                 110

Leu Pro Lys Leu Asn Lys Ala Thr Glu Lys Gln Thr Trp Phe Ile Gln
        115                 120                 125

Ala Asn Gln Tyr Glu Pro Glu Ala Val Leu Val His Cys Gln Thr Pro
    130                 135                 140

Thr Ile Glu Gln Leu Pro Arg Trp Ile Ala Leu Arg Ala Lys Ser Met
145                 150                 155                 160

Gly Leu Gln Ile Glu Glu Gln Ala Ile Gln Leu Leu Cys Tyr Ser His
                165                 170                 175

Glu Asn Asn Leu Leu Ala Leu Lys Gln Ser Leu Gln Leu Leu Ala Leu
            180                 185                 190

Leu Tyr Pro Asp Asn Lys Ile Ser Tyr Ala Arg Val Gln Ser Thr Ile
        195                 200                 205

Ala Gln Thr Ser Val Phe Thr Pro Phe Gln Trp Leu Asp Ala Leu Leu
    210                 215                 220

Glu Gly Lys Ser Lys Arg Ala Gln Arg Ile Leu Glu Gly Leu Lys Ala
225                 230                 235                 240

Glu Glu Met Glu Pro Ile Ile Leu Leu Arg Thr Leu Gln Arg Asp Leu
                245                 250                 255

Leu Thr Leu Leu Gly Leu Ala Lys Pro Ser His Pro Val Thr Leu Thr
            260                 265                 270

Thr Pro Leu Pro Ala His Gln Leu Arg Glu Gln Phe Asp Gln Leu Lys
        275                 280                 285

Val Trp Gln Asn Arg Arg Pro Phe Phe Thr Gln Ala Phe Lys Arg Leu
    290                 295                 300

Asn Tyr Lys Gln Leu Tyr Leu Ile Ile Gln Ala Leu Ala Glu Ile Glu
305                 310                 315                 320

Arg Leu Ala Lys His Thr Phe Ser Gln Asp Ile Trp Asp Arg Leu Ala
                325                 330                 335

Glu Leu Ser Ala Phe Ile Cys Leu Pro Asp Asp Pro Asn His Leu Leu
            340                 345                 350

Ser Leu Pro Thr Phe Ser Arg
        355

<210> 70
<211> 1038
<212> DNA
<213> Pseudomonas aeruginosa

<400> 70
atgaagctga ctcccgcgca actcgccaag cacctccagg gccccctggc gcccgtctat 60

gtcgtcagcg gcgatgaacc gctgctctgc caggaagcct gcgatgccat tcgccaggcc 120

tgccgcgagc gcgacttcgg cgagcgccag gtattcaatg ccgaagccaa cttcgattgg 180

ggcttgctgc tggaggccgg cgccagcctg tcgctgttcg ccgagaagcg cctgatcgaa 240

ctgcgcctgc cctccggcaa gcccggcgac aaaggcgcgg cgattctcca ggaatacctg 300

caacggccgc cggaagacac cgtgctcctg ctcggtctgc ccaagctcga tggcagcacg 360

cagaagacca agtgggccaa ggccctgatc gatggcaacg ccgcgcagtt catccaggtc 420

tggccggtcg acgtccacca gttgccgcag tggattcgcc agcgcctgtc ccaggctggc 480

ctgtccgcca gccccgaagc cctggagctg atcgccgcgc gcgtggaagg caacctgctc 540

gccgccgccc aggaaatcga aaagctcaag ctgctcgccg agggcaacca gatcgacgcc 600

gccaccgtac aggccgcggt cgccgacagc gcacgcttcg atgtcttcgg cctgatcgac 660

gctgcccttg gcggtgaagc ggcccacgcc ctgcgcatcc tggaaggact gcgcggcgaa 720

ggcatcgagc cgccggtgat cctctggggc ctggcccgcg agatccgcct gctggctggc 780

ctgtcccagc aatacggcca gggcatcccg ctggagaaag ccttcgccca ggcgcgtccg 840

ccggtctggg acaagcgccg tccgctgctg acccgtgccc tgcaacgcca ctccagcagc 900

cgctggaacc agatgctgag ggatgcccag ttgatcgacg cgcagatcaa gggacaggca 960

cccggcagcc cctggagcgg tctctcgctt ctcgccctga gcctcgccgg ccagcgcctc 1020

ggcctgccgg cagcctga                                                1038

<210> 71
<211> 1038
<212> DNA
<213> Pseudomonas aeruginosa

<220>
<221> CDS
<222> (1)..(1038)

<400> 71
atg aag ctg act ccc gcg caa ctc gcc aag cac ctc cag ggc ccc ctg      48
Met Lys Leu Thr Pro Ala Gln Leu Ala Lys His Leu Gln Gly Pro Leu
1               5                   10                  15

gcg ccc gtc tat gtc gtc agc ggc gat gaa ccg ctg ctc tgc cag gaa      96
Ala Pro Val Tyr Val Val Ser Gly Asp Glu Pro Leu Leu Cys Gln Glu
            20                  25                  30

gcc tgc gat gcc att cgc cag gcc tgc cgc gag cgc gac ttc ggc gag     144
Ala Cys Asp Ala Ile Arg Gln Ala Cys Arg Glu Arg Asp Phe Gly Glu
        35                  40                  45

cgc cag gta ttc aat gcc gaa gcc aac ttc gat tgg ggc ttg ctg ctg     192
Arg Gln Val Phe Asn Ala Glu Ala Asn Phe Asp Trp Gly Leu Leu Leu
    50                  55                  60

gag gcc ggc gcc agc ctg tcg ctg ttc gcc gag aag cgc ctg atc gaa     240
Glu Ala Gly Ala Ser Leu Ser Leu Phe Ala Glu Lys Arg Leu Ile Glu
65                  70                  75                  80

ctg cgc ctg ccc tcc ggc aag ccc ggc gac aaa ggc gcg gcg att ctc     288
Leu Arg Leu Pro Ser Gly Lys Pro Gly Asp Lys Gly Ala Ala Ile Leu
                85                  90                  95

cag gaa tac ctg caa cgg ccg ccg gaa gac acc gtg ctc ctg ctc ggt     336
Gln Glu Tyr Leu Gln Arg Pro Pro Glu Asp Thr Val Leu Leu Leu Gly
            100                 105                 110

ctg ccc aag ctc gat ggc agc acg cag aag acc aag tgg gcc aag gcc     384
Leu Pro Lys Leu Asp Gly Ser Thr Gln Lys Thr Lys Trp Ala Lys Ala
        115                 120                 125

ctg atc gat ggc aac gcc gcg cag ttc atc cag gtc tgg ccg gtc gac     432
Leu Ile Asp Gly Asn Ala Ala Gln Phe Ile Gln Val Trp Pro Val Asp
    130                 135                 140

gtc cac cag ttg ccg cag tgg att cgc cag cgc ctg tcc cag gct ggc     480
Val His Gln Leu Pro Gln Trp Ile Arg Gln Arg Leu Ser Gln Ala Gly
145                 150                 155                 160

ctg tcc gcc agc ccc gaa gcc ctg gag ctg atc gcc gcg cgc gtg gaa     528
Leu Ser Ala Ser Pro Glu Ala Leu Glu Leu Ile Ala Ala Arg Val Glu
                165                 170                 175

ggc aac ctg ctc gcc gcc gcc cag gaa atc gaa aag ctc aag ctg ctc     576
Gly Asn Leu Leu Ala Ala Ala Gln Glu Ile Glu Lys Leu Lys Leu Leu
            180                 185                 190

gcc gag ggc aac cag atc gac gcc gcc acc gta cag gcc gcg gtc gcc     624
Ala Glu Gly Asn Gln Ile Asp Ala Ala Thr Val Gln Ala Ala Val Ala
        195                 200                 205

gac agc gca cgc ttc gat gtc ttc ggc ctg atc gac gct gcc ctt ggc     672
Asp Ser Ala Arg Phe Asp Val Phe Gly Leu Ile Asp Ala Ala Leu Gly
    210                 215                 220

ggt gaa gcg gcc cac gcc ctg cgc atc ctg gaa gga ctg cgc ggc gaa     720
Gly Glu Ala Ala His Ala Leu Arg Ile Leu Glu Gly Leu Arg Gly Glu
225                 230                 235                 240

ggc atc gag ccg ccg gtg atc ctc tgg ggc ctg gcc cgc gag atc cgc     768
Gly Ile Glu Pro Pro Val Ile Leu Trp Gly Leu Ala Arg Glu Ile Arg
                245                 250                 255

ctg ctg gct ggc ctg tcc cag caa tac ggc cag ggc atc ccg ctg gag     816
Leu Leu Ala Gly Leu Ser Gln Gln Tyr Gly Gln Gly Ile Pro Leu Glu
            260                 265                 270

aaa gcc ttc gcc cag gcg cgt ccg ccg gtc tgg gac aag cgc cgt ccg     864
Lys Ala Phe Ala Gln Ala Arg Pro Pro Val Trp Asp Lys Arg Arg Pro
        275                 280                 285

ctg ctg acc cgt gcc ctg caa cgc cac tcc agc agc cgc tgg aac cag     912
Leu Leu Thr Arg Ala Leu Gln Arg His Ser Ser Ser Arg Trp Asn Gln
    290                 295                 300

atg ctg agg gat gcc cag ttg atc gac gcg cag atc aag gga cag gca     960
Met Leu Arg Asp Ala Gln Leu Ile Asp Ala Gln Ile Lys Gly Gln Ala
305                 310                 315                 320

ccc ggc agc ccc tgg agc ggt ctc tcg ctt ctc gcc ctg agc ctc gcc     1008
Pro Gly Ser Pro Trp Ser Gly Leu Ser Leu Leu Ala Leu Ser Leu Ala
                325                 330                 335

ggc cag cgc ctc ggc ctg ccg gca gcc tga                                    1038
Gly Gln Arg Leu Gly Leu Pro Ala Ala
            340                 345

<210> 72
<211> 345
<212> PRT
<213> Pseudomonas aeruginosa

<400> 72
Met Lys Leu Thr Pro Ala Gln Leu Ala Lys His Leu Gln Gly Pro Leu
  1               5                  10                  15

Ala Pro Val Tyr Val Val Ser Gly Asp Glu Pro Leu Leu Cys Gln Glu
             20                  25                  30

Ala Cys Asp Ala Ile Arg Gln Ala Cys Arg Glu Arg Asp Phe Gly Glu
         35                  40                  45

Arg Gln Val Phe Asn Ala Glu Ala Asn Phe Asp Trp Gly Leu Leu Leu
     50                  55                  60

Glu Ala Gly Ala Ser Leu Ser Leu Phe Ala Glu Lys Arg Leu Ile Glu
 65                  70                  75                  80

Leu Arg Leu Pro Ser Gly Lys Pro Gly Asp Lys Gly Ala Ala Ile Leu
                 85                  90                  95

Gln Glu Tyr Leu Gln Arg Pro Pro Glu Asp Thr Val Leu Leu Leu Gly
            100                 105                 110

Leu Pro Lys Leu Asp Gly Ser Thr Gln Lys Thr Lys Trp Ala Lys Ala
        115                 120                 125

Leu Ile Asp Gly Asn Ala Ala Gln Phe Ile Gln Val Trp Pro Val Asp
    130                 135                 140

Val His Gln Leu Pro Gln Trp Ile Arg Gln Arg Leu Ser Gln Ala Gly
145                 150                 155                 160

Leu Ser Ala Ser Pro Glu Ala Leu Glu Leu Ile Ala Ala Arg Val Glu
                165                 170                 175

Gly Asn Leu Leu Ala Ala Ala Gln Glu Ile Glu Lys Leu Lys Leu Leu
            180                 185                 190

Ala Glu Gly Asn Gln Ile Asp Ala Ala Thr Val Gln Ala Ala Val Ala
        195                 200                 205

Asp Ser Ala Arg Phe Asp Val Phe Gly Leu Ile Asp Ala Ala Leu Gly
    210                 215                 220

Gly Glu Ala Ala His Ala Leu Arg Ile Leu Glu Gly Leu Arg Gly Glu
225                 230                 235                 240

Gly Ile Glu Pro Pro Val Ile Leu Trp Gly Leu Ala Arg Glu Ile Arg
                245                 250                 255

Leu Leu Ala Gly Leu Ser Gln Gln Tyr Gly Gln Gly Ile Pro Leu Glu
            260                 265                 270

Lys Ala Phe Ala Gln Ala Arg Pro Pro Val Trp Asp Lys Arg Arg Pro
        275                 280                 285

Leu Leu Thr Arg Ala Leu Gln Arg His Ser Ser Ser Arg Trp Asn Gln
    290                 295                 300

Met Leu Arg Asp Ala Gln Leu Ile Asp Ala Gln Ile Lys Gly Gln Ala
305                 310                 315                 320

Pro Gly Ser Pro Trp Ser Gly Leu Ser Leu Leu Ala Leu Ser Leu Ala
                325                 330                 335

Gly Gln Arg Leu Gly Leu Pro Ala Ala
            340                 345

<210> 73
<211> 966
<212> DNA
<213> Rickettsia prowazekii

<400> 73
atgcaatttt atttttcgca aataggtaga ttatttgaat taatagcagc cgggaagatt 60

agggctcttt tactatatgg tcctgataaa ggctatatag aaaagatttg tacatattta 120

atcaaaaact tgaatatgct acaatcttct atagaatatg aagatcttaa tattttatct 180

ttagatatat tacttaattc acctaatttt tttggtcaaa aagaattaat taaagtgaga 240

tcaattggaa attcactcga taagaattta aaaacaattc taagcagtga ttatataaat 300

ttccctgtat ttatagggga agatatgaat tctagtggga gtgttaaaaa gttttttgaa 360

acagaagaat atttagcggt agttgcttgt tatcatgacg atgaagcaaa aattgaacga 420

attattttag gtaaattagc aaaaactaat aaagttataa gtaaagaagc tatcacttac 480

ttaaaaactc atttaaaagg tgatcatgct ttaatttgta gcgaaatcaa taaattgata 540

ttttttgctc atgatgtaca tgaaattact ttaaaccatg ttttagaagt tataagtagt 600

gaaattacag caaatggtag taatttggca atatattttt caaaaaaaga ttatagtaat 660

tttctgcaag agcttgatat tttaaaaaaa caaaatatta atgaagtttt aattattaga 720

gctttaataa ggcattattt aaatttatat atagttttat cgaaagtaaa aaatggtgaa 780

tgtttggaaa ttgcaataaa atcattatca ccgccgattt tctatcataa tattaatgat 840

tttacaaaga tagtacatgc tctcagccta actgaatgta taaagacttt gaaactacta 900

caacaggcag aagtggatta taagttaaat cctgctagtt tcgatttatt tcaaagtata 960

ctctaa 966

<210> 74
<211> 966
<212> DNA
<213> Rickettsia prowazekii

<220>
<221> CDS
<222> (1)..(966)

<400> 74
atg caa ttt tat ttt tcg caa ata ggt aga tta ttt gaa tta ata gca 48
Met Gln Phe Tyr Phe Ser Gln Ile Gly Arg Leu Phe Glu Leu Ile Ala
1               5                   10                  15

gcc ggg aag att agg gct ctt tta cta tat ggt cct gat aaa ggc tat 96
Ala Gly Lys Ile Arg Ala Leu Leu Leu Tyr Gly Pro Asp Lys Gly Tyr
            20                  25                  30

ata gaa aag att tgt aca tat tta atc aaa aac ttg aat atg cta caa 144
Ile Glu Lys Ile Cys Thr Tyr Leu Ile Lys Asn Leu Asn Met Leu Gln
        35                  40                  45

tct tct ata gaa tat gaa gat ctt aat att tta tct tta gat ata tta 192
Ser Ser Ile Glu Tyr Glu Asp Leu Asn Ile Leu Ser Leu Asp Ile Leu

50                  55                  60

ctt aat tca cct aat ttt ttt ggt caa aaa gaa tta att aaa gtg aga    240
Leu Asn Ser Pro Asn Phe Phe Gly Gln Lys Glu Leu Ile Lys Val Arg
65                  70                  75                  80

tca att gga aat tca ctc gat aag aat tta aaa aca att cta agc agt    288
Ser Ile Gly Asn Ser Leu Asp Lys Asn Leu Lys Thr Ile Leu Ser Ser
                85                  90                  95

gat tat ata aat ttc cct gta ttt ata ggg gaa gat atg aat tct agt    336
Asp Tyr Ile Asn Phe Pro Val Phe Ile Gly Glu Asp Met Asn Ser Ser
            100                 105                 110

ggg agt gtt aaa aag ttt ttt gaa aca gaa gaa tat tta gcg gta gtt    384
Gly Ser Val Lys Lys Phe Phe Glu Thr Glu Glu Tyr Leu Ala Val Val
        115                 120                 125

gct tgt tat cat gac gat gaa gca aaa att gaa cga att att tta ggt    432
Ala Cys Tyr His Asp Asp Glu Ala Lys Ile Glu Arg Ile Ile Leu Gly
    130                 135                 140

aaa tta gca aaa act aat aaa gtt ata agt aaa gaa gct atc act tac    480
Lys Leu Ala Lys Thr Asn Lys Val Ile Ser Lys Glu Ala Ile Thr Tyr
145                 150                 155                 160

tta aaa act cat tta aaa ggt gat cat gct tta att tgt agc gaa atc    528
Leu Lys Thr His Leu Lys Gly Asp His Ala Leu Ile Cys Ser Glu Ile
                165                 170                 175

aat aaa ttg ata ttt ttt gct cat gat gta cat gaa att act tta aac    576
Asn Lys Leu Ile Phe Phe Ala His Asp Val His Glu Ile Thr Leu Asn
            180                 185                 190

cat gtt tta gaa gtt ata agt agt gaa att aca gca aat ggt agt aat    624
His Val Leu Glu Val Ile Ser Ser Glu Ile Thr Ala Asn Gly Ser Asn
        195                 200                 205

ttg gca ata tat ttt tca aaa aaa gat tat agt aat ttt ctg caa gag    672
Leu Ala Ile Tyr Phe Ser Lys Lys Asp Tyr Ser Asn Phe Leu Gln Glu
    210                 215                 220

ctt gat att tta aaa aaa caa aat att aat gaa gtt tta att att aga    720
Leu Asp Ile Leu Lys Lys Gln Asn Ile Asn Glu Val Leu Ile Ile Arg
225                 230                 235                 240

gct tta ata agg cat tat tta aat tta tat ata gtt tta tcg aaa gta    768
Ala Leu Ile Arg His Tyr Leu Asn Leu Tyr Ile Val Leu Ser Lys Val

                245                 250                 255

aaa aat ggt gaa tgt ttg gaa att gca ata aaa tca tta tca ccg ccg 816
Lys Asn Gly Glu Cys Leu Glu Ile Ala Ile Lys Ser Leu Ser Pro Pro
            260                 265                 270

att ttc tat cat aat att aat gat ttt aca aag ata gta cat gct ctc 864
Ile Phe Tyr His Asn Ile Asn Asp Phe Thr Lys Ile Val His Ala Leu
        275                 280                 285

agc cta act gaa tgt ata aag act ttg aaa cta cta caa cag gca gaa 912
Ser Leu Thr Glu Cys Ile Lys Thr Leu Lys Leu Leu Gln Gln Ala Glu
    290                 295                 300

gtg gat tat aag tta aat cct gct agt ttc gat tta ttt caa agt ata 960
Val Asp Tyr Lys Leu Asn Pro Ala Ser Phe Asp Leu Phe Gln Ser Ile
305                 310                 315                 320

ctc taa 966
Leu

<210> 75
<211> 321
<212> PRT
<213> Rickettsia prowazekii

<400> 75
Met Gln Phe Tyr Phe Ser Gln Ile Gly Arg Leu Phe Glu Leu Ile Ala
1               5                   10                  15

Ala Gly Lys Ile Arg Ala Leu Leu Leu Tyr Gly Pro Asp Lys Gly Tyr
            20                  25                  30

Ile Glu Lys Ile Cys Thr Tyr Leu Ile Lys Asn Leu Asn Met Leu Gln
        35                  40                  45

Ser Ser Ile Glu Tyr Glu Asp Leu Asn Ile Leu Ser Leu Asp Ile Leu
    50                  55                  60

Leu Asn Ser Pro Asn Phe Phe Gly Gln Lys Glu Leu Ile Lys Val Arg
65                  70                  75                  80

Ser Ile Gly Asn Ser Leu Asp Lys Asn Leu Lys Thr Ile Leu Ser Ser
                85                  90                  95

Asp Tyr Ile Asn Phe Pro Val Phe Ile Gly Glu Asp Met Asn Ser Ser
            100                 105                 110

Gly Ser Val Lys Lys Phe Phe Glu Thr Glu Glu Tyr Leu Ala Val Val
        115                 120                 125

Ala Cys Tyr His Asp Asp Glu Ala Lys Ile Glu Arg Ile Ile Leu Gly
    130                 135                 140

Lys Leu Ala Lys Thr Asn Lys Val Ile Ser Lys Glu Ala Ile Thr Tyr
145                 150                 155                 160

Leu Lys Thr His Leu Lys Gly Asp His Ala Leu Ile Cys Ser Glu Ile
                165                 170                 175

Asn Lys Leu Ile Phe Phe Ala His Asp Val His Glu Ile Thr Leu Asn
            180                 185                 190

His Val Leu Glu Val Ile Ser Ser Glu Ile Thr Ala Asn Gly Ser Asn
        195                 200                 205

Leu Ala Ile Tyr Phe Ser Lys Lys Asp Tyr Ser Asn Phe Leu Gln Glu
    210                 215                 220

Leu Asp Ile Leu Lys Lys Gln Asn Ile Asn Glu Val Leu Ile Ile Arg
225                 230                 235                 240

Ala Leu Ile Arg His Tyr Leu Asn Leu Tyr Ile Val Leu Ser Lys Val
                245                 250                 255

Lys Asn Gly Glu Cys Leu Glu Ile Ala Ile Lys Ser Leu Ser Pro Pro
            260                 265                 270

Ile Phe Tyr His Asn Ile Asn Asp Phe Thr Lys Ile Val His Ala Leu
        275                 280                 285

Ser Leu Thr Glu Cys Ile Lys Thr Leu Lys Leu Leu Gln Gln Ala Glu
    290                 295                 300

Val Asp Tyr Lys Leu Asn Pro Ala Ser Phe Asp Leu Phe Gln Ser Ile
305                 310                 315                 320

Leu

<210> 76
<211> 1002
<212> DNA
<213> Synechocystis

<400> 76
atgcccgtat atttctattg gggcgaagac caatttaccc tgcaccaagc agtcaaacaa 60

ctgcagaaac gttgtttaga tccccaatgg gaggccttta attttgaaaa aattccgggg 120

gaacaagctg atgctaccca acggggtctg gagcaagcct tgacaccccc ttttggtagt 180

ggcgatcgcc tggtgtgggt ggtggactcc accttaggac aaagttgcga cgatggtttg 240

ctggctaggt tgcaaaaaag tttgcccgcc attcccaccg actgccattt actattcaca 300

tccagtaaaa agctcgatcg ccgactgaaa tcgaccaaat atttggaagg taatgccact 360

atccgggaat ttgccctcat ttcaccctgg aatgtggacg ctctcatcca tcaaattcag 420

gcgatcgccc aggatctgca acttccctta gcaacggaaa cggaaggttt tttggcagaa 480

gcgttgggta atgacacgag gctaatttgg aatgagttgg gcaagctcaa actctatagc 540

gagagtcaaa cgggcccctt aacggtggcc caggtagaac agttggtcaa taccagcacc 600

caaaatagtt tgcaattagc ccaggcgatc cgggatggag aaacccccca ggcattgcaa 660

ttggtggcag acttattgct gacccacaac gaaccggctt taaaaatatt ggctaccctc 720

accggccagt ttcgtacttg gacattgatc aagcttgctt tggaggcagg ggaaaaagat 780

gacaaagcga tcgccgccca ggccgatctc agtaatccca aacgtttgta ttttttaaag 840

aaggaattgc ggggcattac cggcgggcaa ttgctcaaaa ccttacccct gttgttggaa 900

ttggaatatc aactaaagcg aggagcggac ccctgttcga gcctacaaac taaggtgatt 960

gaactatcat cattattcca gcccatacac cgtcgccgtt ga 1002

<210> 77
<211> 1002
<212> DNA
<213> Synechocystis

<220>
<221> CDS
<222> (1)..(1002)

<400> 77
atg ccc gta tat ttc tat tgg ggc gaa gac caa ttt acc ctg cac caa 48
Met Pro Val Tyr Phe Tyr Trp Gly Glu Asp Gln Phe Thr Leu His Gln

1                   5                   10                  15

gca gtc aaa caa ctg cag aaa cgt tgt tta gat ccc caa tgg gag gcc    96
Ala Val Lys Gln Leu Gln Lys Arg Cys Leu Asp Pro Gln Trp Glu Ala
            20                  25                  30

ttt aat ttt gaa aaa att ccg ggg gaa caa gct gat gct acc caa cgg    144
Phe Asn Phe Glu Lys Ile Pro Gly Glu Gln Ala Asp Ala Thr Gln Arg
        35                  40                  45

ggt ctg gag caa gcc ttg aca ccc cct ttt ggt agt ggc gat cgc ctg    192
Gly Leu Glu Gln Ala Leu Thr Pro Pro Phe Gly Ser Gly Asp Arg Leu
    50                  55                  60

gtg tgg gtg gtg gac tcc acc tta gga caa agt tgc gac gat ggt ttg    240
Val Trp Val Val Asp Ser Thr Leu Gly Gln Ser Cys Asp Asp Gly Leu
65                  70                  75                  80

ctg gct agg ttg caa aaa agt ttg ccc gcc att ccc acc gac tgc cat    288
Leu Ala Arg Leu Gln Lys Ser Leu Pro Ala Ile Pro Thr Asp Cys His
                85                  90                  95

tta cta ttc aca tcc agt aaa aag ctc gat cgc cga ctg aaa tcg acc    336
Leu Leu Phe Thr Ser Ser Lys Lys Leu Asp Arg Arg Leu Lys Ser Thr
            100                 105                 110

aaa tat ttg gaa ggt aat gcc act atc cgg gaa ttt gcc ctc att tca    384
Lys Tyr Leu Glu Gly Asn Ala Thr Ile Arg Glu Phe Ala Leu Ile Ser
        115                 120                 125

ccc tgg aat gtg gac gct ctc atc cat caa att cag gcg atc gcc cag    432
Pro Trp Asn Val Asp Ala Leu Ile His Gln Ile Gln Ala Ile Ala Gln
    130                 135                 140

gat ctg caa ctt ccc tta gca acg gaa acg gaa ggt ttt ttg gca gaa    480
Asp Leu Gln Leu Pro Leu Ala Thr Glu Thr Glu Gly Phe Leu Ala Glu
145                 150                 155                 160

gcg ttg ggt aat gac acg agg cta att tgg aat gag ttg ggc aag ctc    528
Ala Leu Gly Asn Asp Thr Arg Leu Ile Trp Asn Glu Leu Gly Lys Leu
                165                 170                 175

aaa ctc tat agc gag agt caa acg ggc ccc tta acg gtg gcc cag gta    576
Lys Leu Tyr Ser Glu Ser Gln Thr Gly Pro Leu Thr Val Ala Gln Val
            180                 185                 190

gaa cag ttg gtc aat acc agc acc caa aat agt ttg caa tta gcc cag    624
Glu Gln Leu Val Asn Thr Ser Thr Gln Asn Ser Leu Gln Leu Ala Gln

        195                 200                 205

gcg atc cgg gat gga gaa acc ccc cag gca ttg caa ttg gtg gca gac 672
Ala Ile Arg Asp Gly Glu Thr Pro Gln Ala Leu Gln Leu Val Ala Asp
    210                 215                 220

tta ttg ctg acc cac aac gaa ccg gct tta aaa ata ttg gct acc ctc 720
Leu Leu Leu Thr His Asn Glu Pro Ala Leu Lys Ile Leu Ala Thr Leu
225                 230                 235                 240

acc ggc cag ttt cgt act tgg aca ttg atc aag ctt gct ttg gag gca 768
Thr Gly Gln Phe Arg Thr Trp Thr Leu Ile Lys Leu Ala Leu Glu Ala
                245                 250                 255

ggg gaa aaa gat gac aaa gcg atc gcc gcc cag gcc gat ctc agt aat 816
Gly Glu Lys Asp Asp Lys Ala Ile Ala Ala Gln Ala Asp Leu Ser Asn
            260                 265                 270

ccc aaa cgt ttg tat ttt tta aag aag gaa ttg cgg ggc att acc ggc 864
Pro Lys Arg Leu Tyr Phe Leu Lys Lys Glu Leu Arg Gly Ile Thr Gly
        275                 280                 285

ggg caa ttg ctc aaa acc tta ccc ctg ttg ttg gaa ttg gaa tat caa 912
Gly Gln Leu Leu Lys Thr Leu Pro Leu Leu Leu Glu Leu Glu Tyr Gln
    290                 295                 300

cta aag cga gga gcg gac ccc tgt tcg agc cta caa act aag gtg att 960
Leu Lys Arg Gly Ala Asp Pro Cys Ser Ser Leu Gln Thr Lys Val Ile
305                 310                 315                 320

gaa cta tca tca tta ttc cag ccc ata cac cgt cgc cgt tga 1002
Glu Leu Ser Ser Leu Phe Gln Pro Ile His Arg Arg Arg
                325                 330

<210> 78
<211> 333
<212> PRT
<213> Synechocystis

<400> 78
Met Pro Val Tyr Phe Tyr Trp Gly Glu Asp Gln Phe Thr Leu His Gln
1               5                   10                  15

Ala Val Lys Gln Leu Gln Lys Arg Cys Leu Asp Pro Gln Trp Glu Ala
            20                  25                  30

Phe Asn Phe Glu Lys Ile Pro Gly Glu Gln Ala Asp Ala Thr Gln Arg

35                  40                  45

Gly Leu Glu Gln Ala Leu Thr Pro Pro Phe Gly Ser Gly Asp Arg Leu
    50                  55                  60

Val Trp Val Val Asp Ser Thr Leu Gly Gln Ser Cys Asp Asp Gly Leu
65                  70                  75                  80

Leu Ala Arg Leu Gln Lys Ser Leu Pro Ala Ile Pro Thr Asp Cys His
                85                  90                  95

Leu Leu Phe Thr Ser Ser Lys Lys Leu Asp Arg Arg Leu Lys Ser Thr
            100                 105                 110

Lys Tyr Leu Glu Gly Asn Ala Thr Ile Arg Glu Phe Ala Leu Ile Ser
        115                 120                 125

Pro Trp Asn Val Asp Ala Leu Ile His Gln Ile Gln Ala Ile Ala Gln
    130                 135                 140

Asp Leu Gln Leu Pro Leu Ala Thr Glu Thr Glu Gly Phe Leu Ala Glu
145                 150                 155                 160

Ala Leu Gly Asn Asp Thr Arg Leu Ile Trp Asn Glu Leu Gly Lys Leu
                165                 170                 175

Lys Leu Tyr Ser Glu Ser Gln Thr Gly Pro Leu Thr Val Ala Gln Val
            180                 185                 190

Glu Gln Leu Val Asn Thr Ser Thr Gln Asn Ser Leu Gln Leu Ala Gln
        195                 200                 205

Ala Ile Arg Asp Gly Glu Thr Pro Gln Ala Leu Gln Leu Val Ala Asp
    210                 215                 220

Leu Leu Leu Thr His Asn Glu Pro Ala Leu Lys Ile Leu Ala Thr Leu
225                 230                 235                 240

Thr Gly Gln Phe Arg Thr Trp Thr Leu Ile Lys Leu Ala Leu Glu Ala
                245                 250                 255

Gly Glu Lys Asp Asp Lys Ala Ile Ala Ala Gln Ala Asp Leu Ser Asn
            260                 265                 270

Pro Lys Arg Leu Tyr Phe Leu Lys Lys Glu Leu Arg Gly Ile Thr Gly
        275                 280                 285

Gly Gln Leu Leu Lys Thr Leu Pro Leu Leu Leu Glu Leu Glu Tyr Gln

290                 295                 300

Leu Lys Arg Gly Ala Asp Pro Cys Ser Ser Leu Gln Thr Lys Val Ile
305                 310                 315                 320

Glu Leu Ser Ser Leu Phe Gln Pro Ile His Arg Arg Arg
                325                 330

<210> 79
<211> 990
<212> DNA
<213> Thermotoga maritima

<400> 79
atgagaggtg attgtatgcc agtcacgttt ctcacaggta ctgcagaaac tcagaaggaa 60

gaattgataa agaaactcct gaaggatggt aacgtggagt acataaggat ccatccggag 120

gatcccgaca agatcgattt cataaggtct ttactcagga caaagacgat cttttccaac 180

aagacgatca ttgacatcgt caatttcgat gagtggaaag cacaggagca gaagcgtctc 240

gttgaacttt tgaaaaacgt accggaagac gttcatatct tcatccgttc tcaaaaaaca 300

ggtggaaagg gagtagcgct ggagcttccg aagccatggg aaacggacaa gtggcttgag 360

tggatagaaa agcgcttcag ggagaatggt ttgctcatcg ataaagatgc ccttcagctg 420

tttttctcca aggttggaac gaacgacctg atcatagaaa gggagattga aaaactgaaa 480

gcttattccg aggacagaaa gataacggta gaagacgtgg aagaggtcgt ttttacctat 540

cagactccgg gatacgatga tttttgcttt gctgtttccg aaggaaaaag gaagctcgct 600

cactctcttc tgtcgcagct gtggaaaacc acagagtccg tggtgattgc cactgtcctt 660

gcgaatcact tcttggatct cttcaaaatc ctcgttcttg tgacaaagaa aagatactac 720

acctggcctg atgtgtccag ggtgtccaaa gagctgggaa ttcccgttcc tcgtgtggct 780

cgtttcctcg gtttctcctt taagacctgg aaattcaagg tgatgaacca cctcctctac 840

tacgatgtga agaaggttag aaagatactg agggatctct acgatctgga cagagccgtg 900

aaaagcgaag aagatccaaa accgttcttc cacgagttca tagaagaggt ggcactggat 960

gtatattctc ttcagagaga tgaagaataa                                   990

<210> 80
<211> 990
<212> DNA
<213> Thermotoga maritima

<220>
<221> CDS
<222> (1)..(990)

<400> 80
atg aga ggt gat tgt atg cca gtc acg ttt ctc aca ggt act gca gaa     48
Met Arg Gly Asp Cys Met Pro Val Thr Phe Leu Thr Gly Thr Ala Glu
1               5                   10                  15

act cag aag gaa gaa ttg ata aag aaa ctc ctg aag gat ggt aac gtg     96
Thr Gln Lys Glu Glu Leu Ile Lys Lys Leu Leu Lys Asp Gly Asn Val
            20                  25                  30

gag tac ata agg atc cat ccg gag gat ccc gac aag atc gat ttc ata     144
Glu Tyr Ile Arg Ile His Pro Glu Asp Pro Asp Lys Ile Asp Phe Ile
        35                  40                  45

agg tct tta ctc agg aca aag acg atc ttt tcc aac aag acg atc att     192
Arg Ser Leu Leu Arg Thr Lys Thr Ile Phe Ser Asn Lys Thr Ile Ile
    50                  55                  60

gac atc gtc aat ttc gat gag tgg aaa gca cag gag cag aag cgt ctc     240
Asp Ile Val Asn Phe Asp Glu Trp Lys Ala Gln Glu Gln Lys Arg Leu
65                  70                  75                  80

gtt gaa ctt ttg aaa aac gta ccg gaa gac gtt cat atc ttc atc cgt     288
Val Glu Leu Leu Lys Asn Val Pro Glu Asp Val His Ile Phe Ile Arg
                85                  90                  95

tct caa aaa aca ggt gga aag gga gta gcg ctg gag ctt ccg aag cca     336
Ser Gln Lys Thr Gly Gly Lys Gly Val Ala Leu Glu Leu Pro Lys Pro
            100                 105                 110

tgg gaa acg gac aag tgg ctt gag tgg ata gaa aag cgc ttc agg gag     384
Trp Glu Thr Asp Lys Trp Leu Glu Trp Ile Glu Lys Arg Phe Arg Glu
        115                 120                 125

aat ggt ttg ctc atc gat aaa gat gcc ctt cag ctg ttt ttc tcc aag     432
Asn Gly Leu Leu Ile Asp Lys Asp Ala Leu Gln Leu Phe Phe Ser Lys
    130                 135                 140

gtt gga acg aac gac ctg atc ata gaa agg gag att gaa aaa ctg aaa     480
Val Gly Thr Asn Asp Leu Ile Ile Glu Arg Glu Ile Glu Lys Leu Lys
145                 150                 155                 160

gct tat tcc gag gac aga aag ata acg gta gaa gac gtg gaa gag gtc     528
Ala Tyr Ser Glu Asp Arg Lys Ile Thr Val Glu Asp Val Glu Glu Val
                165                 170                 175

gtt ttt acc tat cag act ccg gga tac gat gat ttt tgc ttt gct gtt     576
Val Phe Thr Tyr Gln Thr Pro Gly Tyr Asp Asp Phe Cys Phe Ala Val
            180                 185                 190

tcc gaa gga aaa agg aag ctc gct cac tct ctt ctg tcg cag ctg tgg     624
Ser Glu Gly Lys Arg Lys Leu Ala His Ser Leu Leu Ser Gln Leu Trp
        195                 200                 205

aaa acc aca gag tcc gtg gtg att gcc act gtc ctt gcg aat cac ttc     672
Lys Thr Thr Glu Ser Val Val Ile Ala Thr Val Leu Ala Asn His Phe
    210                 215                 220

ttg gat ctc ttc aaa atc ctc gtt ctt gtg aca aag aaa aga tac tac     720
Leu Asp Leu Phe Lys Ile Leu Val Leu Val Thr Lys Lys Arg Tyr Tyr
225                 230                 235                 240

acc tgg cct gat gtg tcc agg gtg tcc aaa gag ctg gga att ccc gtt     768
Thr Trp Pro Asp Val Ser Arg Val Ser Lys Glu Leu Gly Ile Pro Val
                245                 250                 255

cct cgt gtg gct cgt ttc ctc ggt ttc tcc ttt aag acc tgg aaa ttc     816
Pro Arg Val Ala Arg Phe Leu Gly Phe Ser Phe Lys Thr Trp Lys Phe
            260                 265                 270

aag gtg atg aac cac ctc ctc tac tac gat gtg aag aag gtt aga aag     864
Lys Val Met Asn His Leu Leu Tyr Tyr Asp Val Lys Lys Val Arg Lys
        275                 280                 285

ata ctg agg gat ctc tac gat ctg gac aga gcc gtg aaa agc gaa gaa     912
Ile Leu Arg Asp Leu Tyr Asp Leu Asp Arg Ala Val Lys Ser Glu Glu
    290                 295                 300

gat cca aaa ccg ttc ttc cac gag ttc ata gaa gag gtg gca ctg gat     960
Asp Pro Lys Pro Phe Phe His Glu Phe Ile Glu Glu Val Ala Leu Asp
305                 310                 315                 320

gta tat tct ctt cag aga gat gaa gaa taa                             990
Val Tyr Ser Leu Gln Arg Asp Glu Glu
                325                 330

<210> 81
<211> 329
<212> PRT
<213> Thermotoga maritima

<400> 81
Met Arg Gly Asp Cys Met Pro Val Thr Phe Leu Thr Gly Thr Ala Glu
1               5                   10                  15

Thr Gln Lys Glu Glu Leu Ile Lys Lys Leu Leu Lys Asp Gly Asn Val
            20                  25                  30

Glu Tyr Ile Arg Ile His Pro Glu Asp Pro Asp Lys Ile Asp Phe Ile
        35                  40                  45

Arg Ser Leu Leu Arg Thr Lys Thr Ile Phe Ser Asn Lys Thr Ile Ile
    50                  55                  60

Asp Ile Val Asn Phe Asp Glu Trp Lys Ala Gln Glu Gln Lys Arg Leu
65                  70                  75                  80

Val Glu Leu Leu Lys Asn Val Pro Glu Asp Val His Ile Phe Ile Arg
                85                  90                  95

Ser Gln Lys Thr Gly Gly Lys Gly Val Ala Leu Glu Leu Pro Lys Pro
            100                 105                 110

Trp Glu Thr Asp Lys Trp Leu Glu Trp Ile Glu Lys Arg Phe Arg Glu
        115                 120                 125

Asn Gly Leu Leu Ile Asp Lys Asp Ala Leu Gln Leu Phe Phe Ser Lys
    130                 135                 140

Val Gly Thr Asn Asp Leu Ile Ile Glu Arg Glu Ile Glu Lys Leu Lys
145                 150                 155                 160

Ala Tyr Ser Glu Asp Arg Lys Ile Thr Val Glu Asp Val Glu Glu Val
                165                 170                 175

Val Phe Thr Tyr Gln Thr Pro Gly Tyr Asp Asp Phe Cys Phe Ala Val
            180                 185                 190

Ser Glu Gly Lys Arg Lys Leu Ala His Ser Leu Leu Ser Gln Leu Trp
        195                 200                 205

Lys Thr Thr Glu Ser Val Val Ile Ala Thr Val Leu Ala Asn His Phe
    210                 215                 220

Leu Asp Leu Phe Lys Ile Leu Val Leu Val Thr Lys Lys Arg Tyr Tyr
225                 230                 235                 240

Thr Trp Pro Asp Val Ser Arg Val Ser Lys Glu Leu Gly Ile Pro Val
                245                 250                 255

Pro Arg Val Ala Arg Phe Leu Gly Phe Ser Phe Lys Thr Trp Lys Phe
            260                 265                 270

Lys Val Met Asn His Leu Leu Tyr Tyr Asp Val Lys Lys Val Arg Lys
        275                 280                 285

Ile Leu Arg Asp Leu Tyr Asp Leu Asp Arg Ala Val Lys Ser Glu Glu
    290                 295                 300

Asp Pro Lys Pro Phe Phe His Glu Phe Ile Glu Glu Val Ala Leu Asp
305                 310                 315                 320

Val Tyr Ser Leu Gln Arg Asp Glu Glu
                325

<210> 82
<211> 765
<212> DNA
<213> Treponema pallidum

<400> 82
atgtccgtct ggctttttac cggacctgaa ataggggagc gagatagtgc agttcaggag 60

gtgtgcgcgc gtgcacaagc gcaagggacg gtggacgtac atcggctcta tgcacacgaa 120

acgcctgtgg cagatcttgt agatctcctg agaactcgtg cgctgtttgc agatgcagtc 180

tgcgtggtac tgtacaacgc agaggttatt aagaagtgtg acgaagtgca cgtgcttaca 240

gaatggataa aggatggcgg ctcgcgcgct gacgtgtttt tggtactgat ttccgatagt 300

gtcagtatac ataagcgtat agaacagaat atttctcccg tgcataagcg tgttttttgg 360

gagttgtttg aaaataaaaa acacgcgtgg gtgcagcgat ttttttttca gcatgaaatg 420

cgcattgaac aggaggctat cgaatctctt cttgagttgg tggagaacaa cactcgtgcg 480

ctcaaaactg tttgtacgca gctttctctt ttttttgaaa aaggacgccg catcactgcg 540

cacgacatta gttcgttgct ggtgcacaca aaagaagaga catcgtttac cttattcgat 600

gcgctgtcca aacgagatct tgagcattca cttatgattc tcaacacgct gctttgttcg 660

aaagacgttg cccctgtaca aatccttgca ggactgctta cgcgttccgt cgccttgcgc 720

attggcacca tataccggag gacaaaacgc accctgcaac gctaa 765

<210> 83
<211> 765
<212> DNA
<213> Treponema pallidum

<220>
<221> CDS
<222> (1)..(765)

<400> 83
atg tcc gtc tgg ctt ttt acc gga cct gaa ata ggg gag cga gat agt 48
Met Ser Val Trp Leu Phe Thr Gly Pro Glu Ile Gly Glu Arg Asp Ser
1               5                   10                  15

gca gtt cag gag gtg tgc gcg cgt gca caa gcg caa ggg acg gtg gac 96
Ala Val Gln Glu Val Cys Ala Arg Ala Gln Ala Gln Gly Thr Val Asp
            20                  25                  30

gta cat cgg ctc tat gca cac gaa acg cct gtg gca gat ctt gta gat 144
Val His Arg Leu Tyr Ala His Glu Thr Pro Val Ala Asp Leu Val Asp
        35                  40                  45

ctc ctg aga act cgt gcg ctg ttt gca gat gca gtc tgc gtg gta ctg 192
Leu Leu Arg Thr Arg Ala Leu Phe Ala Asp Ala Val Cys Val Val Leu
    50                  55                  60

tac aac gca gag gtt att aag aag tgt gac gaa gtg cac gtg ctt aca 240
Tyr Asn Ala Glu Val Ile Lys Lys Cys Asp Glu Val His Val Leu Thr
65                  70                  75                  80

gaa tgg ata aag gat ggc ggc tcg cgc gct gac gtg ttt ttg gta ctg 288
Glu Trp Ile Lys Asp Gly Gly Ser Arg Ala Asp Val Phe Leu Val Leu
                85                  90                  95

att tcc gat agt gtc agt ata cat aag cgt ata gaa cag aat att tct 336
Ile Ser Asp Ser Val Ser Ile His Lys Arg Ile Glu Gln Asn Ile Ser
            100                 105                 110

ccc gtg cat aag cgt gtt ttt tgg gag ttg ttt gaa aat aaa aaa cac 384
Pro Val His Lys Arg Val Phe Trp Glu Leu Phe Glu Asn Lys Lys His

115                 120                 125

gcg tgg gtg cag cga ttt ttt ttt cag cat gaa atg cgc att gaa cag     432
Ala Trp Val Gln Arg Phe Phe Phe Gln His Glu Met Arg Ile Glu Gln
    130                 135                 140

gag gct atc gaa tct ctt ctt gag ttg gtg gag aac aac act cgt gcg     480
Glu Ala Ile Glu Ser Leu Leu Glu Leu Val Glu Asn Asn Thr Arg Ala
145                 150                 155                 160

ctc aaa act gtt tgt acg cag ctt tct ctt ttt ttt gaa aaa gga cgc     528
Leu Lys Thr Val Cys Thr Gln Leu Ser Leu Phe Phe Glu Lys Gly Arg
                165                 170                 175

cgc atc act gcg cac gac att agt tcg ttg ctg gtg cac aca aaa gaa     576
Arg Ile Thr Ala His Asp Ile Ser Ser Leu Leu Val His Thr Lys Glu
            180                 185                 190

gag aca tcg ttt acc tta ttc gat gcg ctg tcc aaa cga gat ctt gag     624
Glu Thr Ser Phe Thr Leu Phe Asp Ala Leu Ser Lys Arg Asp Leu Glu
        195                 200                 205

cat tca ctt atg att ctc aac acg ctg ctt tgt tcg aaa gac gtt gcc     672
His Ser Leu Met Ile Leu Asn Thr Leu Leu Cys Ser Lys Asp Val Ala
    210                 215                 220

cct gta caa atc ctt gca gga ctg ctt acg cgt tcc gtc gcc ttg cgc     720
Pro Val Gln Ile Leu Ala Gly Leu Leu Thr Arg Ser Val Ala Leu Arg
225                 230                 235                 240

att ggc acc ata tac cgg agg aca aaa cgc acc ctg caa cgc taa         765
Ile Gly Thr Ile Tyr Arg Arg Thr Lys Arg Thr Leu Gln Arg
                245                 250                 255

<210> 84
<211> 254
<212> PRT
<213> Treponema pallidum

<400> 84
Met Ser Val Trp Leu Phe Thr Gly Pro Glu Ile Gly Glu Arg Asp Ser
1               5                   10                  15

Ala Val Gln Glu Val Cys Ala Arg Ala Gln Ala Gln Gly Thr Val Asp
            20                  25                  30

Val His Arg Leu Tyr Ala His Glu Thr Pro Val Ala Asp Leu Val Asp

35                  40                  45

Leu Leu Arg Thr Arg Ala Leu Phe Ala Asp Ala Val Cys Val Val Leu
    50                  55                  60

Tyr Asn Ala Glu Val Ile Lys Lys Cys Asp Glu Val His Val Leu Thr
65                  70                  75                  80

Glu Trp Ile Lys Asp Gly Gly Ser Arg Ala Asp Val Phe Leu Val Leu
                85                  90                  95

Ile Ser Asp Ser Val Ser Ile His Lys Arg Ile Glu Gln Asn Ile Ser
            100                 105                 110

Pro Val His Lys Arg Val Phe Trp Glu Leu Phe Glu Asn Lys Lys His
        115                 120                 125

Ala Trp Val Gln Arg Phe Phe Phe Gln His Glu Met Arg Ile Glu Gln
    130                 135                 140

Glu Ala Ile Glu Ser Leu Leu Glu Leu Val Glu Asn Asn Thr Arg Ala
145                 150                 155                 160

Leu Lys Thr Val Cys Thr Gln Leu Ser Leu Phe Phe Glu Lys Gly Arg
                165                 170                 175

Arg Ile Thr Ala His Asp Ile Ser Ser Leu Leu Val His Thr Lys Glu
            180                 185                 190

Glu Thr Ser Phe Thr Leu Phe Asp Ala Leu Ser Lys Arg Asp Leu Glu
        195                 200                 205

His Ser Leu Met Ile Leu Asn Thr Leu Leu Cys Ser Lys Asp Val Ala
    210                 215                 220

Pro Val Gln Ile Leu Ala Gly Leu Leu Thr Arg Ser Val Ala Leu Arg
225                 230                 235                 240

Ile Gly Thr Ile Tyr Arg Arg Thr Lys Arg Thr Leu Gln Arg
                245                 250

<210> 85
<211> 945
<212> DNA
<213> Ureaplasma urealyticum

<400> 85

atgaacttaa taaataaaga acttttttta attactggtg acaatcgttt aaaaatggat 60

caaaaaatta atgaaattgc gacctctttt gatgaattag ttaaaattaa tgatcaagaa 120

atttcattaa tttcttttaa aaatcttatt gaacaagatg atttattcaa tagtaataag 180

atctacttat ttaaaaacgt taactggttc gaaaatttag aacatttaaa aaatgttagt 240

gatttaattg attatttttt taataatcat gttgctatta ttattacaat tgaatctaca 300

aaaatttcta cagcaaaaaa aattcaagaa acaattgcaa aatttaatca tcaaattact 360

tttatgacat atacaaatga aaatgctatg gctttcttaa aagaagagtt aaatcagcgt 420

aatttatcat taaataaatc tataatgcaa acgattattc aaaaaacaaa ttttaatatt 480

aattttctta ataatgaatt agataaaata gaattaatta atgatttttt aaaaaatcat 540

ggtatgtttg atattaataa ttttatatgt gattatggtg aatatcaaat ttttagttta 600

ttaaatcttt tgtatcaaca taaaataaat gaatcaataa atttaattaa taaaatgctc 660

attgacaaaa ttgatgaact cacaataatt aatatgttag cgacaataat gagtacacat 720

tacttaatta aattattaca tgaaaaaaaa tataacaaaa acgttatatc tagctcttta 780

aatcaaaaac cttatattat tgatttaaat ttaaaattaa ttagaaatta ttcagctaaa 840

tttttactta aaaaaatgta tgatttatta caattagaaa ttaaagtcaa ggaaaataaa 900

attgataaat attttggatt aattaattgg gttttgaatt tttaa 945

<210> 86
<211> 945
<212> DNA
<213> Ureaplasma urealyticum

<220>
<221> CDS
<222> (1)..(945)

<400> 86
atg aac tta ata aat aaa gaa ctt ttt tta att act ggt gac aat cgt 48
Met Asn Leu Ile Asn Lys Glu Leu Phe Leu Ile Thr Gly Asp Asn Arg
1               5                   10                  15

tta aaa atg gat caa aaa att aat gaa att gcg acc tct ttt gat gaa 96

Leu Lys Met Asp Gln Lys Ile Asn Glu Ile Ala Thr Ser Phe Asp Glu
            20                  25                  30

tta gtt aaa att aat gat caa gaa att tca tta att tct ttt aaa aat 144
Leu Val Lys Ile Asn Asp Gln Glu Ile Ser Leu Ile Ser Phe Lys Asn
        35                  40                  45

ctt att gaa caa gat gat tta ttc aat agt aat aag atc tac tta ttt 192
Leu Ile Glu Gln Asp Asp Leu Phe Asn Ser Asn Lys Ile Tyr Leu Phe
    50                  55                  60

aaa aac gtt aac tgg ttc gaa aat tta gaa cat tta aaa aat gtt agt 240
Lys Asn Val Asn Trp Phe Glu Asn Leu Glu His Leu Lys Asn Val Ser
65                  70                  75                  80

gat tta att gat tat ttt ttt aat aat cat gtt gct att att att aca 288
Asp Leu Ile Asp Tyr Phe Phe Asn Asn His Val Ala Ile Ile Ile Thr
                85                  90                  95

att gaa tct aca aaa att tct aca gca aaa aaa att caa gaa aca att 336
Ile Glu Ser Thr Lys Ile Ser Thr Ala Lys Lys Ile Gln Glu Thr Ile
            100                 105                 110

gca aaa ttt aat cat caa att act ttt atg aca tat aca aat gaa aat 384
Ala Lys Phe Asn His Gln Ile Thr Phe Met Thr Tyr Thr Asn Glu Asn
        115                 120                 125

gct atg gct ttc tta aaa gaa gag tta aat cag cgt aat tta tca tta 432
Ala Met Ala Phe Leu Lys Glu Glu Leu Asn Gln Arg Asn Leu Ser Leu
    130                 135                 140

aat aaa tct ata atg caa acg att att caa aaa aca aat ttt aat att 480
Asn Lys Ser Ile Met Gln Thr Ile Ile Gln Lys Thr Asn Phe Asn Ile
145                 150                 155                 160

aat ttt ctt aat aat gaa tta gat aaa ata gaa tta att aat gat ttt 528
Asn Phe Leu Asn Asn Glu Leu Asp Lys Ile Glu Leu Ile Asn Asp Phe
                165                 170                 175

tta aaa aat cat ggt atg ttt gat att aat aat ttt ata tgt gat tat 576
Leu Lys Asn His Gly Met Phe Asp Ile Asn Asn Phe Ile Cys Asp Tyr
            180                 185                 190

ggt gaa tat caa att ttt agt tta tta aat ctt ttg tat caa cat aaa 624
Gly Glu Tyr Gln Ile Phe Ser Leu Leu Asn Leu Leu Tyr Gln His Lys
        195                 200                 205

ata aat gaa tca ata aat tta att aat aaa atg ctc att gac aaa att 672

Ile Asn Glu Ser Ile Asn Leu Ile Asn Lys Met Leu Ile Asp Lys Ile
    210                 215                 220

gat gaa ctc aca ata att aat atg tta gcg aca ata atg agt aca cat     720
Asp Glu Leu Thr Ile Ile Asn Met Leu Ala Thr Ile Met Ser Thr His
225                 230                 235                 240

tac tta att aaa tta tta cat gaa aaa aaa tat aac aaa aac gtt ata     768
Tyr Leu Ile Lys Leu Leu His Glu Lys Lys Tyr Asn Lys Asn Val Ile
                245                 250                 255

tct agc tct tta aat caa aaa cct tat att att gat tta aat tta aaa     816
Ser Ser Ser Leu Asn Gln Lys Pro Tyr Ile Ile Asp Leu Asn Leu Lys
            260                 265                 270

tta att aga aat tat tca gct aaa ttt tta ctt aaa aaa atg tat gat     864
Leu Ile Arg Asn Tyr Ser Ala Lys Phe Leu Leu Lys Lys Met Tyr Asp
        275                 280                 285

tta tta caa tta gaa att aaa gtc aag gaa aat aaa att gat aaa tat     912
Leu Leu Gln Leu Glu Ile Lys Val Lys Glu Asn Lys Ile Asp Lys Tyr
    290                 295                 300

ttt gga tta att aat tgg gtt ttg aat ttt taa                         945
Phe Gly Leu Ile Asn Trp Val Leu Asn Phe
305                 310                 315

<210> 87
<211> 314
<212> PRT
<213> Ureaplasma urealyticum

<400> 87
Met Asn Leu Ile Asn Lys Glu Leu Phe Leu Ile Thr Gly Asp Asn Arg
  1               5                  10                  15

Leu Lys Met Asp Gln Lys Ile Asn Glu Ile Ala Thr Ser Phe Asp Glu
             20                  25                  30

Leu Val Lys Ile Asn Asp Gln Glu Ile Ser Leu Ile Ser Phe Lys Asn
         35                  40                  45

Leu Ile Glu Gln Asp Asp Leu Phe Asn Ser Asn Lys Ile Tyr Leu Phe
     50                  55                  60

Lys Asn Val Asn Trp Phe Glu Asn Leu Glu His Leu Lys Asn Val Ser
 65                  70                  75                  80

Asp Leu Ile Asp Tyr Phe Phe Asn Asn His Val Ala Ile Ile Ile Thr
                85                  90                  95

Ile Glu Ser Thr Lys Ile Ser Thr Ala Lys Lys Ile Gln Glu Thr Ile
            100                 105                 110

Ala Lys Phe Asn His Gln Ile Thr Phe Met Thr Tyr Thr Asn Glu Asn
        115                 120                 125

Ala Met Ala Phe Leu Lys Glu Glu Leu Asn Gln Arg Asn Leu Ser Leu
    130                 135                 140

Asn Lys Ser Ile Met Gln Thr Ile Ile Gln Lys Thr Asn Phe Asn Ile
145                 150                 155                 160

Asn Phe Leu Asn Asn Glu Leu Asp Lys Ile Glu Leu Ile Asn Asp Phe
                165                 170                 175

Leu Lys Asn His Gly Met Phe Asp Ile Asn Asn Phe Ile Cys Asp Tyr
            180                 185                 190

Gly Glu Tyr Gln Ile Phe Ser Leu Leu Asn Leu Leu Tyr Gln His Lys
        195                 200                 205

Ile Asn Glu Ser Ile Asn Leu Ile Asn Lys Met Leu Ile Asp Lys Ile
    210                 215                 220

Asp Glu Leu Thr Ile Ile Asn Met Leu Ala Thr Ile Met Ser Thr His
225                 230                 235                 240

Tyr Leu Ile Lys Leu Leu His Glu Lys Lys Tyr Asn Lys Asn Val Ile
                245                 250                 255

Ser Ser Ser Leu Asn Gln Lys Pro Tyr Ile Ile Asp Leu Asn Leu Lys
            260                 265                 270

Leu Ile Arg Asn Tyr Ser Ala Lys Phe Leu Leu Lys Lys Met Tyr Asp
        275                 280                 285

Leu Leu Gln Leu Glu Ile Lys Val Lys Glu Asn Lys Ile Asp Lys Tyr
    290                 295                 300

Phe Gly Leu Ile Asn Trp Val Leu Asn Phe
305                 310

<210> 88

<211> 1029
<212> DNA
<213> Vibrio cholerae

<400> 88
atgcgcattt acgctgaaaa actggctgaa tcactgcaca aaacgctcta tccgatttac 60

ctcgtattcg gtaatgagcc actgctgctc caagaagcca aaaccgccat cgaaaaaacg 120

gcacaagcgc aaggtttttt ggaaaaacac cgatttagcg cggatgccgg gctcgattgg 180

aacgcggtgt atgactgttg ccaagcactg agtctgtttt catctcgcca acttatcgaa 240

attgagatcc cagaatctgg ggtcaatgct caaacggcaa aggaacttag cgcactggta 300

ggccaacttc accaagacat tctcttgctg gtgattggcc ccaaactgac taaggcgcaa 360

gagaacgcag cttggttcaa aacgcttgcg cagcaagcct gttgggttaa ctgcctcacg 420

ccagaattga gccgtttacc tcagtttgtt cagcagcgct gttttgccct cggtttaaaa 480

ccggatgccg aagcggtaca aatgttggcg cagtggcatg aaggtaatct ctttgcatta 540

gcccagagtc tcgaaaaatt agcgttactc taccccgatg gtctactgac tttagtgcgc 600

ttggaagagt cgctgagccg ccataaccat ttcacgcctt atcactggat ggatgcgcta 660

ttagaaggca aagccaaccg tgctcagcgt attttgcgcc agcttatgct cgaagagagt 720

gagccgatta ttctgatccg caccgcgcaa aaagagctga cccaactgct taaatggcaa 780

caagagcgcc aacagctggg taatttgggg agtctttttg atcgctatcg tgtctggcag 840

aatcgaagac cactctactc tgccgcttta cagcgcttac caagtcgcgc cctacttcgg 900

ttggttggaa tattaactca ggctgaatta ctggcaaaaa cgcaatacga acagccagta 960

tggccaatct tgcagcaact aagccttgaa tgctgcaatc ctcaggcaaa cctacctttg 1020

cctcactaa 1029

<210> 89
<211> 1029
<212> DNA
<213> Vibrio cholerae

<220>

<221> CDS
<222> (1)..(1029)

<400> 89
atg cgc att tac gct gaa aaa ctg gct gaa tca ctg cac aaa acg ctc     48
Met Arg Ile Tyr Ala Glu Lys Leu Ala Glu Ser Leu His Lys Thr Leu
1               5                   10                  15

tat ccg att tac ctc gta ttc ggt aat gag cca ctg ctg ctc caa gaa     96
Tyr Pro Ile Tyr Leu Val Phe Gly Asn Glu Pro Leu Leu Leu Gln Glu
            20                  25                  30

gcc aaa acc gcc atc gaa aaa acg gca caa gcg caa ggt ttt ttg gaa     144
Ala Lys Thr Ala Ile Glu Lys Thr Ala Gln Ala Gln Gly Phe Leu Glu
        35                  40                  45

aaa cac cga ttt agc gcg gat gcc ggg ctc gat tgg aac gcg gtg tat     192
Lys His Arg Phe Ser Ala Asp Ala Gly Leu Asp Trp Asn Ala Val Tyr
    50                  55                  60

gac tgt tgc caa gca ctg agt ctg ttt tca tct cgc caa ctt atc gaa     240
Asp Cys Cys Gln Ala Leu Ser Leu Phe Ser Ser Arg Gln Leu Ile Glu
65                  70                  75                  80

att gag atc cca gaa tct ggg gtc aat gct caa acg gca aag gaa ctt     288
Ile Glu Ile Pro Glu Ser Gly Val Asn Ala Gln Thr Ala Lys Glu Leu
                85                  90                  95

agc gca ctg gta ggc caa ctt cac caa gac att ctc ttg ctg gtg att     336
Ser Ala Leu Val Gly Gln Leu His Gln Asp Ile Leu Leu Leu Val Ile
            100                 105                 110

ggc ccc aaa ctg act aag gcg caa gag aac gca gct tgg ttc aaa acg     384
Gly Pro Lys Leu Thr Lys Ala Gln Glu Asn Ala Ala Trp Phe Lys Thr
        115                 120                 125

ctt gcg cag caa gcc tgt tgg gtt aac tgc ctc acg cca gaa ttg agc     432
Leu Ala Gln Gln Ala Cys Trp Val Asn Cys Leu Thr Pro Glu Leu Ser
    130                 135                 140

cgt tta cct cag ttt gtt cag cag cgc tgt ttt gcc ctc ggt tta aaa     480
Arg Leu Pro Gln Phe Val Gln Gln Arg Cys Phe Ala Leu Gly Leu Lys
145                 150                 155                 160

ccg gat gcc gaa gcg gta caa atg ttg gcg cag tgg cat gaa ggt aat     528
Pro Asp Ala Glu Ala Val Gln Met Leu Ala Gln Trp His Glu Gly Asn
                165                 170                 175

ctc ttt gca tta gcc cag agt ctc gaa aaa tta gcg tta ctc tac ccc 576
Leu Phe Ala Leu Ala Gln Ser Leu Glu Lys Leu Ala Leu Leu Tyr Pro
            180                 185                 190

gat ggt cta ctg act tta gtg cgc ttg gaa gag tcg ctg agc cgc cat 624
Asp Gly Leu Leu Thr Leu Val Arg Leu Glu Glu Ser Leu Ser Arg His
        195                 200                 205

aac cat ttc acg cct tat cac tgg atg gat gcg cta tta gaa ggc aaa 672
Asn His Phe Thr Pro Tyr His Trp Met Asp Ala Leu Leu Glu Gly Lys
    210                 215                 220

gcc aac cgt gct cag cgt att ttg cgc cag ctt atg ctc gaa gag agt 720
Ala Asn Arg Ala Gln Arg Ile Leu Arg Gln Leu Met Leu Glu Glu Ser
225                 230                 235                 240

gag ccg att att ctg atc cgc acc gcg caa aaa gag ctg acc caa ctg 768
Glu Pro Ile Ile Leu Ile Arg Thr Ala Gln Lys Glu Leu Thr Gln Leu
                245                 250                 255

ctt aaa tgg caa caa gag cgc caa cag ctg ggt aat ttg ggg agt ctt 816
Leu Lys Trp Gln Gln Glu Arg Gln Gln Leu Gly Asn Leu Gly Ser Leu
            260                 265                 270

ttt gat cgc tat cgt gtc tgg cag aat cga aga cca ctc tac tct gcc 864
Phe Asp Arg Tyr Arg Val Trp Gln Asn Arg Arg Pro Leu Tyr Ser Ala
        275                 280                 285

gct tta cag cgc tta cca agt cgc gcc cta ctt cgg ttg gtt gga ata 912
Ala Leu Gln Arg Leu Pro Ser Arg Ala Leu Leu Arg Leu Val Gly Ile
    290                 295                 300

tta act cag gct gaa tta ctg gca aaa acg caa tac gaa cag cca gta 960
Leu Thr Gln Ala Glu Leu Leu Ala Lys Thr Gln Tyr Glu Gln Pro Val
305                 310                 315                 320

tgg cca atc ttg cag caa cta agc ctt gaa tgc tgc aat cct cag gca 1008
Trp Pro Ile Leu Gln Gln Leu Ser Leu Glu Cys Cys Asn Pro Gln Ala
                325                 330                 335

aac cta cct ttg cct cac taa 1029
Asn Leu Pro Leu Pro His
            340

<210> 90
<211> 342
<212> PRT

<213> Vibrio cholerae

<400> 90
Met Arg Ile Tyr Ala Glu Lys Leu Ala Glu Ser Leu His Lys Thr Leu
1               5                   10                  15

Tyr Pro Ile Tyr Leu Val Phe Gly Asn Glu Pro Leu Leu Leu Gln Glu
            20                  25                  30

Ala Lys Thr Ala Ile Glu Lys Thr Ala Gln Ala Gln Gly Phe Leu Glu
        35                  40                  45

Lys His Arg Phe Ser Ala Asp Ala Gly Leu Asp Trp Asn Ala Val Tyr
    50                  55                  60

Asp Cys Cys Gln Ala Leu Ser Leu Phe Ser Ser Arg Gln Leu Ile Glu
65                  70                  75                  80

Ile Glu Ile Pro Glu Ser Gly Val Asn Ala Gln Thr Ala Lys Glu Leu
                85                  90                  95

Ser Ala Leu Val Gly Gln Leu His Gln Asp Ile Leu Leu Leu Val Ile
            100                 105                 110

Gly Pro Lys Leu Thr Lys Ala Gln Glu Asn Ala Ala Trp Phe Lys Thr
        115                 120                 125

Leu Ala Gln Gln Ala Cys Trp Val Asn Cys Leu Thr Pro Glu Leu Ser
    130                 135                 140

Arg Leu Pro Gln Phe Val Gln Gln Arg Cys Phe Ala Leu Gly Leu Lys
145                 150                 155                 160

Pro Asp Ala Glu Ala Val Gln Met Leu Ala Gln Trp His Glu Gly Asn
                165                 170                 175

Leu Phe Ala Leu Ala Gln Ser Leu Glu Lys Leu Ala Leu Leu Tyr Pro
            180                 185                 190

Asp Gly Leu Leu Thr Leu Val Arg Leu Glu Glu Ser Leu Ser Arg His
        195                 200                 205

Asn His Phe Thr Pro Tyr His Trp Met Asp Ala Leu Leu Glu Gly Lys
    210                 215                 220

Ala Asn Arg Ala Gln Arg Ile Leu Arg Gln Leu Met Leu Glu Glu Ser
225                 230                 235                 240

Glu Pro Ile Ile Leu Ile Arg Thr Ala Gln Lys Glu Leu Thr Gln Leu
                245                 250                 255

Leu Lys Trp Gln Gln Glu Arg Gln Gln Leu Gly Asn Leu Gly Ser Leu
            260                 265                 270

Phe Asp Arg Tyr Arg Val Trp Gln Asn Arg Arg Pro Leu Tyr Ser Ala
        275                 280                 285

Ala Leu Gln Arg Leu Pro Ser Arg Ala Leu Leu Arg Leu Val Gly Ile
    290                 295                 300

Leu Thr Gln Ala Glu Leu Leu Ala Lys Thr Gln Tyr Glu Gln Pro Val
305                 310                 315                 320

Trp Pro Ile Leu Gln Gln Leu Ser Leu Glu Cys Cys Asn Pro Gln Ala
                325                 330                 335

Asn Leu Pro Leu Pro His
            340

<210> 91
<211> 1032
<212> DNA
<213> Xylella fastidiosa

<400> 91
atggaacttc gcccagagca actcgccaac ctgcctgcca atcaaccctt agagcctgcc 60

tacctcattg ccggccccga aaccctacgc ttagtcgaag ctgccgatgc gatacgtacc 120

catgctcgtg ccgacggcat caccgaacgc gaaatattcg acgtcgaaag ccgtgacttc 180

gactggcaac aacttgcctc cagcttcaac gcccccagcc tcttcagctc acgtcgctta 240

atcgaaatac gcctcccagg aggtaaacct ggtaaagaag gcagtgaagt catcacaagg 300

ttctgcgcca ccccctcatc agatgttatc ttgctgatca cctgcaacga atggagcaaa 360

gcacaccatg gcaagtggac cgatgccatc gctcgcatcg gtaaaatcgc aatcgcctgg 420

accatcaagc ctcacgaact acccgactgg attacacgcc gcctacatag caaaggctta 480

cgtgccaaca cagatgtgat acaccaatta agcacacgcc tagaaggcaa cctactcgcc 540

gccgcccaag aaattgacaa attggcgtta ttgaaccaag gccctctcct cgacctagag 600

accatggaat cctttgttgc cgacacagcg cgttacgacg tattccgact gaccgaagcg 660

atgctctccg gccaacccac cgcaacgata cgcatgctcg gcaaactgcg ggccgaaggt 720

gaagcagtgg cagcactgct gcccattctg accaaagaac tcatccgtac cacgaccctg 780

tcatgggtca aacatgacaa cggcaatcta gctgccgaaa tgaaagccca gggcatctgg 840

gaaacacgtc aagcaccgtt caagcgcgca ttacaacgtc atacctcacc gtacaaatgg 900

gaacacttcg ttgccgaagt cgggcgcata gaccgcatca ccaaaggacg caacgacggc 960

aatgcatggc tatcactcga acgcctcatg gtggcagtgg cagaagcaga tgcaacacgt 1020

ctgctagcat aa 1032

<210> 92
<211> 1032
<212> DNA
<213> Xylella fastidiosa

<220>
<221> CDS
<222> (1)..(1032)

<400> 92
atg gaa ctt cgc cca gag caa ctc gcc aac ctg cct gcc aat caa ccc 48
Met Glu Leu Arg Pro Glu Gln Leu Ala Asn Leu Pro Ala Asn Gln Pro
1               5                   10                  15

tta gag cct gcc tac ctc att gcc ggc ccc gaa acc cta cgc tta gtc 96
Leu Glu Pro Ala Tyr Leu Ile Ala Gly Pro Glu Thr Leu Arg Leu Val
            20                  25                  30

gaa gct gcc gat gcg ata cgt acc cat gct cgt gcc gac ggc atc acc 144
Glu Ala Ala Asp Ala Ile Arg Thr His Ala Arg Ala Asp Gly Ile Thr
        35                  40                  45

gaa cgc gaa ata ttc gac gtc gaa agc cgt gac ttc gac tgg caa caa 192
Glu Arg Glu Ile Phe Asp Val Glu Ser Arg Asp Phe Asp Trp Gln Gln
    50                  55                  60

ctt gcc tcc agc ttc aac gcc ccc agc ctc ttc agc tca cgt cgc tta 240
Leu Ala Ser Ser Phe Asn Ala Pro Ser Leu Phe Ser Ser Arg Arg Leu
65                  70                  75                  80

atc gaa ata cgc ctc cca gga ggt aaa cct ggt aaa gaa ggc agt gaa 288

Ile Glu Ile Arg Leu Pro Gly Gly Lys Pro Gly Lys Glu Gly Ser Glu
                85                  90                  95

gtc atc aca agg ttc tgc gcc acc ccc tca tca gat gtt atc ttg ctg 336
Val Ile Thr Arg Phe Cys Ala Thr Pro Ser Ser Asp Val Ile Leu Leu
            100                 105                 110

atc acc tgc aac gaa tgg agc aaa gca cac cat ggc aag tgg acc gat 384
Ile Thr Cys Asn Glu Trp Ser Lys Ala His His Gly Lys Trp Thr Asp
        115                 120                 125

gcc atc gct cgc atc ggt aaa atc gca atc gcc tgg acc atc aag cct 432
Ala Ile Ala Arg Ile Gly Lys Ile Ala Ile Ala Trp Thr Ile Lys Pro
    130                 135                 140

cac gaa cta ccc gac tgg att aca cgc cgc cta cat agc aaa ggc tta 480
His Glu Leu Pro Asp Trp Ile Thr Arg Arg Leu His Ser Lys Gly Leu
145                 150                 155                 160

cgt gcc aac aca gat gtg ata cac caa tta agc aca cgc cta gaa ggc 528
Arg Ala Asn Thr Asp Val Ile His Gln Leu Ser Thr Arg Leu Glu Gly
                165                 170                 175

aac cta ctc gcc gcc gcc caa gaa att gac aaa ttg gcg tta ttg aac 576
Asn Leu Leu Ala Ala Ala Gln Glu Ile Asp Lys Leu Ala Leu Leu Asn
            180                 185                 190

caa ggc cct ctc ctc gac cta gag acc atg gaa tcc ttt gtt gcc gac 624
Gln Gly Pro Leu Leu Asp Leu Glu Thr Met Glu Ser Phe Val Ala Asp
        195                 200                 205

aca gcg cgt tac gac gta ttc cga ctg acc gaa gcg atg ctc tcc ggc 672
Thr Ala Arg Tyr Asp Val Phe Arg Leu Thr Glu Ala Met Leu Ser Gly
    210                 215                 220

caa ccc acc gca acg ata cgc atg ctc ggc aaa ctg cgg gcc gaa ggt 720
Gln Pro Thr Ala Thr Ile Arg Met Leu Gly Lys Leu Arg Ala Glu Gly
225                 230                 235                 240

gaa gca gtg gca gca ctg ctg ccc att ctg acc aaa gaa ctc atc cgt 768
Glu Ala Val Ala Ala Leu Leu Pro Ile Leu Thr Lys Glu Leu Ile Arg
                245                 250                 255

acc acg acc ctg tca tgg gtc aaa cat gac aac ggc aat cta gct gcc 816
Thr Thr Thr Leu Ser Trp Val Lys His Asp Asn Gly Asn Leu Ala Ala
            260                 265                 270

gaa atg aaa gcc cag ggc atc tgg gaa aca cgt caa gca ccg ttc aag 864

Glu Met Lys Ala Gln Gly Ile Trp Glu Thr Arg Gln Ala Pro Phe Lys
        275                 280                 285

cgc gca tta caa cgt cat acc tca ccg tac aaa tgg gaa cac ttc gtt     912
Arg Ala Leu Gln Arg His Thr Ser Pro Tyr Lys Trp Glu His Phe Val
    290                 295                 300

gcc gaa gtc ggg cgc ata gac cgc atc acc aaa gga cgc aac gac ggc     960
Ala Glu Val Gly Arg Ile Asp Arg Ile Thr Lys Gly Arg Asn Asp Gly
305                 310                 315                 320

aat gca tgg cta tca ctc gaa cgc ctc atg gtg gca gtg gca gaa gca     1008
Asn Ala Trp Leu Ser Leu Glu Arg Leu Met Val Ala Val Ala Glu Ala
                325                 330                 335

gat gca aca cgt ctg cta gca taa                                     1032
Asp Ala Thr Arg Leu Leu Ala
            340

<210> 93
<211> 343
<212> PRT
<213> Xylella fastidiosa

<400> 93
Met Glu Leu Arg Pro Glu Gln Leu Ala Asn Leu Pro Ala Asn Gln Pro
  1               5                  10                  15

Leu Glu Pro Ala Tyr Leu Ile Ala Gly Pro Glu Thr Leu Arg Leu Val
             20                  25                  30

Glu Ala Ala Asp Ala Ile Arg Thr His Ala Arg Ala Asp Gly Ile Thr
         35                  40                  45

Glu Arg Glu Ile Phe Asp Val Glu Ser Arg Asp Phe Asp Trp Gln Gln
     50                  55                  60

Leu Ala Ser Ser Phe Asn Ala Pro Ser Leu Phe Ser Ser Arg Arg Leu
 65                  70                  75                  80

Ile Glu Ile Arg Leu Pro Gly Gly Lys Pro Gly Lys Glu Gly Ser Glu
                 85                  90                  95

Val Ile Thr Arg Phe Cys Ala Thr Pro Ser Ser Asp Val Ile Leu Leu
            100                 105                 110

Ile Thr Cys Asn Glu Trp Ser Lys Ala His His Gly Lys Trp Thr Asp

115                 120                 125

Ala Ile Ala Arg Ile Gly Lys Ile Ala Ile Ala Trp Thr Ile Lys Pro
    130                 135                 140

His Glu Leu Pro Asp Trp Ile Thr Arg Arg Leu His Ser Lys Gly Leu
145                 150                 155                 160

Arg Ala Asn Thr Asp Val Ile His Gln Leu Ser Thr Arg Leu Glu Gly
                165                 170                 175

Asn Leu Leu Ala Ala Ala Gln Glu Ile Asp Lys Leu Ala Leu Leu Asn
            180                 185                 190

Gln Gly Pro Leu Leu Asp Leu Glu Thr Met Glu Ser Phe Val Ala Asp
        195                 200                 205

Thr Ala Arg Tyr Asp Val Phe Arg Leu Thr Glu Ala Met Leu Ser Gly
    210                 215                 220

Gln Pro Thr Ala Thr Ile Arg Met Leu Gly Lys Leu Arg Ala Glu Gly
225                 230                 235                 240

Glu Ala Val Ala Ala Leu Leu Pro Ile Leu Thr Lys Glu Leu Ile Arg
                245                 250                 255

Thr Thr Thr Leu Ser Trp Val Lys His Asp Asn Gly Asn Leu Ala Ala
            260                 265                 270

Glu Met Lys Ala Gln Gly Ile Trp Glu Thr Arg Gln Ala Pro Phe Lys
        275                 280                 285

Arg Ala Leu Gln Arg His Thr Ser Pro Tyr Lys Trp Glu His Phe Val
    290                 295                 300

Ala Glu Val Gly Arg Ile Asp Arg Ile Thr Lys Gly Arg Asn Asp Gly
305                 310                 315                 320

Asn Ala Trp Leu Ser Leu Glu Arg Leu Met Val Ala Val Ala Glu Ala
                325                 330                 335

Asp Ala Thr Arg Leu Leu Ala
            340

<210> 94
<211> 945
<212> DNA

<213> Mycoplasma pulmonis

<400> 94
atgcatttaa tttatggaca agaagaattt ttaattgaat caaaaacaaa aaacataatt 60

gagaatcaaa aaaaagattc gatagttttt acattttatg aaaactttga aattgatgat 120

cttttaaatg atattgttca aaaagatctt tttagtccta ataaaataat tcatattaaa 180

aactggaaat tacttgaaaa tccaagacct tcaaaagtaa ttttaaacaa actaaaattg 240

cttagcgaaa taatagaaaa tgataaaagt ttaattttta ttttttctca cattggtgaa 300

attgaaaaaa attttttaag tgatttttta ttaaaaaaag cttcagtaaa acactataaa 360

aaacttgagt taaatgaaaa aattaaattc attgaaagct atgttactaa aaacaaaatt 420

aaaatttcaa cagtgcttat ttc[?]tattta ttaatcaatg tttcatcatc tatttcaatt 480

gttataaatg aaattaataa actattttta gaaaaccaaa acatcactag agaaatgatt 540

gaaaagtcag ttggttttta tactaaagaa cctaactttg aatttatcaa ttcaatcaaa 600

tctagaaatt caaattcaat ttggaaaaca tttttagaac aaaaaaattc aggaattgaa 660

cctttagatc ttttaaaaca aatttcatca gcttttattt taggaaataa aattaacaac 720

atcatcaaat tagatcactc acttgatcaa gttgctaaaa ttttaaatgt gcacatttat 780

agagcaaaag aaattttttc tttaattaaa ggttttagtc aaaactcaat taatgaaatt 840

atcgaattat tagagttaac tgataaaaga atcaaaacta catatatgga tcctgagatg 900

gctttagatt tttttattct aaaattaatt agacactata attaa 945

<210> 95
<211> 945
<212> DNA
<213> Mycoplasma pulmonis

<220>
<221> CDS
<222> (1)..(945)

<400> 95
atg cat tta att tat gga caa gaa gaa ttt tta att gaa tca aaa aca 48
Met His Leu Ile Tyr Gly Gln Glu Glu Phe Leu Ile Glu Ser Lys Thr

1                   5                   10                  15

aaa aac ata att gag aat caa aaa aaa gat tcg ata gtt ttt aca ttt    96
Lys Asn Ile Ile Glu Asn Gln Lys Lys Asp Ser Ile Val Phe Thr Phe
                20                  25                  30

tat gaa aac ttt gaa att gat gat ctt tta aat gat att gtt caa aaa    144
Tyr Glu Asn Phe Glu Ile Asp Asp Leu Leu Asn Asp Ile Val Gln Lys
            35                  40                  45

gat ctt ttt agt cct aat aaa ata att cat att aaa aac tgg aaa tta    192
Asp Leu Phe Ser Pro Asn Lys Ile Ile His Ile Lys Asn Trp Lys Leu
        50                  55                  60

ctt gaa aat cca aga cct tca aaa gta att tta aac aaa cta aaa ttg    240
Leu Glu Asn Pro Arg Pro Ser Lys Val Ile Leu Asn Lys Leu Lys Leu
65                  70                  75                  80

ctt agc gaa ata ata gaa aat gat aaa agt tta att ttt att ttt tct    288
Leu Ser Glu Ile Ile Glu Asn Asp Lys Ser Leu Ile Phe Ile Phe Ser
                    85                  90                  95

cac att ggt gaa att gaa aaa aat ttt tta agt gat ttt tta tta aaa    336
His Ile Gly Glu Ile Glu Lys Asn Phe Leu Ser Asp Phe Leu Leu Lys
                100                 105                 110

aaa gct tca gta aaa cac tat aaa aaa ctt gag tta aat gaa aaa att    384
Lys Ala Ser Val Lys His Tyr Lys Lys Leu Glu Leu Asn Glu Lys Ile
            115                 120                 125

aaa ttc att gaa agc tat gtt act aaa aac aaa att aaa att tca aca    432
Lys Phe Ile Glu Ser Tyr Val Thr Lys Asn Lys Ile Lys Ile Ser Thr
    130                 135                 140

gtg ctt att tct tat tta tta atc aat gtt tca tca tct att tca att    480
Val Leu Ile Ser Tyr Leu Leu Ile Asn Val Ser Ser Ser Ile Ser Ile
145                 150                 155                 160

gtt ata aat gaa att aat aaa cta ttt tta gaa aac caa aac atc act    528
Val Ile Asn Glu Ile Asn Lys Leu Phe Leu Glu Asn Gln Asn Ile Thr
                165                 170                 175

aga gaa atg att gaa aag tca gtt ggt ttt tat act aaa gaa cct aac    576
Arg Glu Met Ile Glu Lys Ser Val Gly Phe Tyr Thr Lys Glu Pro Asn
            180                 185                 190

ttt gaa ttt atc aat tca atc aaa tct aga aat tca aat tca att tgg    624
Phe Glu Phe Ile Asn Ser Ile Lys Ser Arg Asn Ser Asn Ser Ile Trp

        195                 200                 205

aaa aca ttt tta gaa caa aaa aat tca gga att gaa cct tta gat ctt 672
Lys Thr Phe Leu Glu Gln Lys Asn Ser Gly Ile Glu Pro Leu Asp Leu
    210                 215                 220

tta aaa caa att tca tca gct ttt att tta gga aat aaa att aac aac 720
Leu Lys Gln Ile Ser Ser Ala Phe Ile Leu Gly Asn Lys Ile Asn Asn
225                 230                 235                 240

atc atc aaa tta gat cac tca ctt gat caa gtt gct aaa att tta aat 768
Ile Ile Lys Leu Asp His Ser Leu Asp Gln Val Ala Lys Ile Leu Asn
                245                 250                 255

gtg cac att tat aga gca aaa gaa att ttt tct tta att aaa ggt ttt 816
Val His Ile Tyr Arg Ala Lys Glu Ile Phe Ser Leu Ile Lys Gly Phe
            260                 265                 270

agt caa aac tca att aat gaa att atc gaa tta tta gag tta act gat 864
Ser Gln Asn Ser Ile Asn Glu Ile Ile Glu Leu Leu Glu Leu Thr Asp
        275                 280                 285

aaa aga atc aaa act aca tat atg gat cct gag atg gct tta gat ttt 912
Lys Arg Ile Lys Thr Thr Tyr Met Asp Pro Glu Met Ala Leu Asp Phe
    290                 295                 300

ttt att cta aaa tta att aga cac tat aat taa 945
Phe Ile Leu Lys Leu Ile Arg His Tyr Asn
305 310 315

<210> 96
<211> 314
<212> PRT
<213> Mycoplasma pulmonis

<400> 96
Met His Leu Ile Tyr Gly Gln Glu Glu Phe Leu Ile Glu Ser Lys Thr
1               5                   10                  15

Lys Asn Ile Ile Glu Asn Gln Lys Lys Asp Ser Ile Val Phe Thr Phe
            20                  25                  30

Tyr Glu Asn Phe Glu Ile Asp Asp Leu Leu Asn Asp Ile Val Gln Lys
        35                  40                  45

Asp Leu Phe Ser Pro Asn Lys Ile Ile His Ile Lys Asn Trp Lys Leu
    50                  55                  60

Leu Glu Asn Pro Arg Pro Ser Lys Val Ile Leu Asn Lys Leu Lys Leu
65                  70                  75                  80

Leu Ser Glu Ile Ile Glu Asn Asp Lys Ser Leu Ile Phe Ile Phe Ser
                85                  90                  95

His Ile Gly Glu Ile Glu Lys Asn Phe Leu Ser Asp Phe Leu Leu Lys
            100                 105                 110

Lys Ala Ser Val Lys His Tyr Lys Lys Leu Glu Leu Asn Glu Lys Ile
        115                 120                 125

Lys Phe Ile Glu Ser Tyr Val Thr Lys Asn Lys Ile Lys Ile Ser Thr
    130                 135                 140

Val Leu Ile Ser Tyr Leu Leu Ile Asn Val Ser Ser Ser Ile Ser Ile
145                 150                 155                 160

Val Ile Asn Glu Ile Asn Lys Leu Phe Leu Glu Asn Gln Asn Ile Thr
                165                 170                 175

Arg Glu Met Ile Glu Lys Ser Val Gly Phe Tyr Thr Lys Glu Pro Asn
            180                 185                 190

Phe Glu Phe Ile Asn Ser Ile Lys Ser Arg Asn Ser Asn Ser Ile Trp
        195                 200                 205

Lys Thr Phe Leu Glu Gln Lys Asn Ser Gly Ile Glu Pro Leu Asp Leu
    210                 215                 220

Leu Lys Gln Ile Ser Ser Ala Phe Ile Leu Gly Asn Lys Ile Asn Asn
225                 230                 235                 240

Ile Ile Lys Leu Asp His Ser Leu Asp Gln Val Ala Lys Ile Leu Asn
                245                 250                 255

Val His Ile Tyr Arg Ala Lys Glu Ile Phe Ser Leu Ile Lys Gly Phe
            260                 265                 270

Ser Gln Asn Ser Ile Asn Glu Ile Ile Glu Leu Leu Glu Leu Thr Asp
        275                 280                 285

Lys Arg Ile Lys Thr Thr Tyr Met Asp Pro Glu Met Ala Leu Asp Phe
    290                 295                 300

Phe Ile Leu Lys Leu Ile Arg His Tyr Asn
305                 310

<210> 97
<211> 975
<212> DNA
<213> Staphylococcus aureus

<400> 97
atgagcgaca atattgtagc tatttatgga gatgtgcctg aattggttga aaaacaaagt 60

gcagaaatca tatcacaatt tttgaaaagt gatagagatg actttaactt tgtgaaatat 120

aatttatacg aaaaagagat tgcaccaatt gttgaagaaa cattaacatt gcctttcttt 180

tcagataaaa aagcaatttt ggttaaaaat gcatatatat ttacaggtga aaaagcgcca 240

aaagatatgg ctcataatgt agatcaatta atagaattta ttgaaaaata tgatggcgaa 300

aatttgattg tctttgagat atatcaaaat aaacttgatg aaagaaaaaa gttaactaaa 360

actctaaaaa agcatgcaag gcttaaaaaa atagagcaaa tgtctgaaga agaaataaaa 420

aaatggattc aaagtaaatt aaatgagaat ttcaaagata tcaaaagaga tgcattagat 480

ttatttattg agttgacagg tattaacttt aatattgtct cacaagagat agaaaagttg 540

attttatttt taggcgatag accaacaatt aataagcagg atgttaacca aattattaat 600

agaagtttag agcaaaatgt atttttactg actgaataca ttcagaaaag aaagaaagaa 660

caagcaattc atttagtaaa agatttaata actatgaaag aagaaccaat taaattactt 720

gcactaatta caagtaatta ccgattattt tatcaatgta agattctgag tcaaaaagga 780

tatagtggac agcaaattgc taaaacaata ggagtgcatc catacagagt aaaattagcg 840

ttaggacaag taagacatta tcaacttgat gaattattga atattgtaga tgcttgtgcg 900

gaaactgatt ataaacttaa atcatcatat atggataaac agttaatact ggaattattc 960

attctatctt tataa 975

<210> 98
<211> 975
<212> DNA
<213> Staphylococcus aureus

<220>
<221> CDS
<222> (1)..(975)

<400> 98
atg agc gac aat att gta gct att tat gga gat gtg cct gaa ttg gtt 48
Met Ser Asp Asn Ile Val Ala Ile Tyr Gly Asp Val Pro Glu Leu Val
1               5                   10                  15

gaa aaa caa agt gca gaa atc ata tca caa ttt ttg aaa agt gat aga 96
Glu Lys Gln Ser Ala Glu Ile Ile Ser Gln Phe Leu Lys Ser Asp Arg
            20                  25                  30

gat gac ttt aac ttt gtg aaa tat aat tta tac gaa aaa gag att gca 144
Asp Asp Phe Asn Phe Val Lys Tyr Asn Leu Tyr Glu Lys Glu Ile Ala
        35                  40                  45

cca att gtt gaa gaa aca tta aca ttg cct ttc ttt tca gat aaa aaa 192
Pro Ile Val Glu Glu Thr Leu Thr Leu Pro Phe Phe Ser Asp Lys Lys
    50                  55                  60

gca att ttg gtt aaa aat gca tat ata ttt aca ggt gaa aaa gcg cca 240
Ala Ile Leu Val Lys Asn Ala Tyr Ile Phe Thr Gly Glu Lys Ala Pro
65                  70                  75                  80

aaa gat atg gct cat aat gta gat caa tta ata gaa ttt att gaa aaa 288
Lys Asp Met Ala His Asn Val Asp Gln Leu Ile Glu Phe Ile Glu Lys
                85                  90                  95

tat gat ggc gaa aat ttg att gtc ttt gag ata tat caa aat aaa ctt 336
Tyr Asp Gly Glu Asn Leu Ile Val Phe Glu Ile Tyr Gln Asn Lys Leu
            100                 105                 110

gat gaa aga aaa aag tta act aaa act cta aaa aag cat gca agg ctt 384
Asp Glu Arg Lys Lys Leu Thr Lys Thr Leu Lys Lys His Ala Arg Leu
        115                 120                 125

aaa aaa ata gag caa atg tct gaa gaa gaa ata aaa aaa tgg att caa 432
Lys Lys Ile Glu Gln Met Ser Glu Glu Glu Ile Lys Lys Trp Ile Gln
    130                 135                 140

agt aaa tta aat gag aat ttc aaa gat atc aaa aga gat gca tta gat 480
Ser Lys Leu Asn Glu Asn Phe Lys Asp Ile Lys Arg Asp Ala Leu Asp
145                 150                 155                 160

tta ttt att gag ttg aca ggt att aac ttt aat att gtc tca caa gag 528
Leu Phe Ile Glu Leu Thr Gly Ile Asn Phe Asn Ile Val Ser Gln Glu
                165                 170                 175

ata gaa aag ttg att tta ttt tta ggc gat aga cca aca att aat aag 576
Ile Glu Lys Leu Ile Leu Phe Leu Gly Asp Arg Pro Thr Ile Asn Lys
            180                 185                 190

cag gat gtt aac caa att att aat aga agt tta gag caa aat gta ttt 624
Gln Asp Val Asn Gln Ile Ile Asn Arg Ser Leu Glu Gln Asn Val Phe
        195                 200                 205

tta ctg act gaa tac att cag aaa aga aag aaa gaa caa gca att cat 672
Leu Leu Thr Glu Tyr Ile Gln Lys Arg Lys Lys Glu Gln Ala Ile His
    210                 215                 220

tta gta aaa gat tta ata act atg aaa gaa gaa cca att aaa tta ctt 720
Leu Val Lys Asp Leu Ile Thr Met Lys Glu Glu Pro Ile Lys Leu Leu
225                 230                 235                 240

gca cta att aca agt aat tac cga tta ttt tat caa tgt aag att ctg 768
Ala Leu Ile Thr Ser Asn Tyr Arg Leu Phe Tyr Gln Cys Lys Ile Leu
                245                 250                 255

agt caa aaa gga tat agt gga cag caa att gct aaa aca ata gga gtg 816
Ser Gln Lys Gly Tyr Ser Gly Gln Gln Ile Ala Lys Thr Ile Gly Val
            260                 265                 270

cat cca tac aga gta aaa tta gcg tta gga caa gta aga cat tat caa 864
His Pro Tyr Arg Val Lys Leu Ala Leu Gly Gln Val Arg His Tyr Gln
        275                 280                 285

ctt gat gaa tta ttg aat att gta gat gct tgt gcg gaa act gat tat 912
Leu Asp Glu Leu Leu Asn Ile Val Asp Ala Cys Ala Glu Thr Asp Tyr
    290                 295                 300

aaa ctt aaa tca tca tat atg gat aaa cag tta ata ctg gaa tta ttc 960
Lys Leu Lys Ser Ser Tyr Met Asp Lys Gln Leu Ile Leu Glu Leu Phe
305                 310                 315                 320

att cta tct tta taa 975
Ile Leu Ser Leu
325

<210> 99
<211> 324
<212> PRT
<213> Staphylococcus aureus

<400> 99

Met Ser Asp Asn Ile Val Ala Ile Tyr Gly Asp Val Pro Glu Leu Val
1               5                   10                  15

Glu Lys Gln Ser Ala Glu Ile Ile Ser Gln Phe Leu Lys Ser Asp Arg
            20                  25                  30

Asp Asp Phe Asn Phe Val Lys Tyr Asn Leu Tyr Glu Lys Glu Ile Ala
        35                  40                  45

Pro Ile Val Glu Glu Thr Leu Thr Leu Pro Phe Phe Ser Asp Lys Lys
    50                  55                  60

Ala Ile Leu Val Lys Asn Ala Tyr Ile Phe Thr Gly Glu Lys Ala Pro
65                  70                  75                  80

Lys Asp Met Ala His Asn Val Asp Gln Leu Ile Glu Phe Ile Glu Lys
                85                  90                  95

Tyr Asp Gly Glu Asn Leu Ile Val Phe Glu Ile Tyr Gln Asn Lys Leu
            100                 105                 110

Asp Glu Arg Lys Lys Leu Thr Lys Thr Leu Lys Lys His Ala Arg Leu
        115                 120                 125

Lys Lys Ile Glu Gln Met Ser Glu Glu Glu Ile Lys Lys Trp Ile Gln
    130                 135                 140

Ser Lys Leu Asn Glu Asn Phe Lys Asp Ile Lys Arg Asp Ala Leu Asp
145                 150                 155                 160

Leu Phe Ile Glu Leu Thr Gly Ile Asn Phe Asn Ile Val Ser Gln Glu
                165                 170                 175

Ile Glu Lys Leu Ile Leu Phe Leu Gly Asp Arg Pro Thr Ile Asn Lys
            180                 185                 190

Gln Asp Val Asn Gln Ile Ile Asn Arg Ser Leu Glu Gln Asn Val Phe
        195                 200                 205

Leu Leu Thr Glu Tyr Ile Gln Lys Arg Lys Lys Glu Gln Ala Ile His
    210                 215                 220

Leu Val Lys Asp Leu Ile Thr Met Lys Glu Glu Pro Ile Lys Leu Leu
225                 230                 235                 240

Ala Leu Ile Thr Ser Asn Tyr Arg Leu Phe Tyr Gln Cys Lys Ile Leu
                245                 250                 255

Ser Gln Lys Gly Tyr Ser Gly Gln Gln Ile Ala Lys Thr Ile Gly Val
            260                 265                 270

His Pro Tyr Arg Val Lys Leu Ala Leu Gly Gln Val Arg His Tyr Gln
        275                 280                 285

Leu Asp Glu Leu Leu Asn Ile Val Asp Ala Cys Ala Glu Thr Asp Tyr
    290                 295                 300

Lys Leu Lys Ser Ser Tyr Met Asp Lys Gln Leu Ile Leu Glu Leu Phe
305                 310                 315                 320

Ile Leu Ser Leu

<210> 100
<211> 1011
<212> DNA
<213> Streptomyces coelicolor

<400> 100
gtgcgtgcga tgcttgtcgc gatggccagg aagactgcga acgacgaccc tctcgccccg 60

gtgacccttg ccgtgggcca ggaggacctc ctgctcgacc gtgccgtgca ggaggtggtg 120

gccgccgcga aggccgccga cgccgacacg gacgtacgcg acctcacccc cgaccagctg 180

cagcccggca ccctcgccga gctgaccagc ccctcgctct tcgccgagcg caaagtcgtg 240

gtcgtacgca atgcgcagga cctgtccgcc gacacggtca aggacgtcaa ggcctacctc 300

ggcgcccccg ccgaggagat caccctcgtc ctgctgcacg cgggcggcgc caagggcaag 360

ggcgtcctcg acgccggccg caaggcgggg gcgcgcgagg tggcctgccc gaagatgacc 420

aagcccgccg accggctggc cttcgtgcgc gcggagttcc gcacggccgg gcggtcggcc 480

acacccgagg cctgccaggc cctggtcgac gcgatcggca gcgacctcag ggagctggcc 540

tcggcggtgt cccagctgac cgccgacgtc gaggggaccg tcgacgaggc ggtcgtcggg 600

cggtactaca ccgggcgggc ggaggcctcc agcttcacgg tcgccgaccg ggcggtcgag 660

ggacgcgcgg cggaggcgct ggaggcgctg cgctggtcgc tggcgaccgg agtggcgccg 720

gtgctgatca ccagcgcgct cgcccagggc gtgcgggcca tcggcaagct gtcctccgcc 780

cgcggcggac gccccgccga cctcgcccgc gagctgggga tgccgccctg gaagatcgac 840

cgcgtccggc agcagatgcg cggctggacc ccggacggcg tctccgtggc cctgcgcgcg 900

gtcgccgagg ccgacgcggg ggtgaagggc ggcggggacg atcccgagta cgccctggag 960

aaggccgtgg tgatcatcgc gcgggcggcg cgttcacggg gccggacctg a 1011

<210> 101
<211> 1011
<212> DNA
<213> Streptomyces coelicolor

<220>
<221> CDS
<222> (1)..(1011)

<400> 101
gtg cgt gcg atg ctt gtc gcg atg gcc agg aag act gcg aac gac gac 48
Val Arg Ala Met Leu Val Ala Met Ala Arg Lys Thr Ala Asn Asp Asp
1               5                   10                  15

cct ctc gcc ccg gtg acc ctt gcc gtg ggc cag gag gac ctc ctg ctc 96
Pro Leu Ala Pro Val Thr Leu Ala Val Gly Gln Glu Asp Leu Leu Leu
            20                  25                  30

gac cgt gcc gtg cag gag gtg gtg gcc gcc gcg aag gcc gcc gac gcc 144
Asp Arg Ala Val Gln Glu Val Val Ala Ala Ala Lys Ala Ala Asp Ala
        35                  40                  45

gac acg gac gta cgc gac ctc acc ccc gac cag ctg cag ccc ggc acc 192
Asp Thr Asp Val Arg Asp Leu Thr Pro Asp Gln Leu Gln Pro Gly Thr
    50                  55                  60

ctc gcc gag ctg acc agc ccc tcg ctc ttc gcc gag cgc aaa gtc gtg 240
Leu Ala Glu Leu Thr Ser Pro Ser Leu Phe Ala Glu Arg Lys Val Val
65                  70                  75                  80

gtc gta cgc aat gcg cag gac ctg tcc gcc gac acg gtc aag gac gtc 288
Val Val Arg Asn Ala Gln Asp Leu Ser Ala Asp Thr Val Lys Asp Val
                85                  90                  95

aag gcc tac ctc ggc gcc ccc gcc gag gag atc acc ctc gtc ctg ctg 336
Lys Ala Tyr Leu Gly Ala Pro Ala Glu Glu Ile Thr Leu Val Leu Leu
            100                 105                 110

cac gcg ggc ggc gcc aag ggc aag ggc gtc ctc gac gcc ggc cgc aag 384
His Ala Gly Gly Ala Lys Gly Lys Gly Val Leu Asp Ala Gly Arg Lys

        115                 120                 125

gcg ggg gcg cgc gag gtg gcc tgc ccg aag atg acc aag ccc gcc gac 432
Ala Gly Ala Arg Glu Val Ala Cys Pro Lys Met Thr Lys Pro Ala Asp
    130                 135                 140

cgg ctg gcc ttc gtg cgc gcg gag ttc cgc acg gcc ggg cgg tcg gcc 480
Arg Leu Ala Phe Val Arg Ala Glu Phe Arg Thr Ala Gly Arg Ser Ala
145                 150                 155                 160

aca ccc gag gcc tgc cag gcc ctg gtc gac gcg atc ggc agc gac ctc 528
Thr Pro Glu Ala Cys Gln Ala Leu Val Asp Ala Ile Gly Ser Asp Leu
                165                 170                 175

agg gag ctg gcc tcg gcg gtg tcc cag ctg acc gcc gac gtc gag ggg 576
Arg Glu Leu Ala Ser Ala Val Ser Gln Leu Thr Ala Asp Val Glu Gly
            180                 185                 190

acc gtc gac gag gcg gtc gtc ggg cgg tac tac acc ggg cgg gcg gag 624
Thr Val Asp Glu Ala Val Val Gly Arg Tyr Tyr Thr Gly Arg Ala Glu
        195                 200                 205

gcc tcc agc ttc acg gtc gcc gac cgg gcg gtc gag gga cgc gcg gcg 672
Ala Ser Ser Phe Thr Val Ala Asp Arg Ala Val Glu Gly Arg Ala Ala
    210                 215                 220

gag gcg ctg gag gcg ctg cgc tgg tcg ctg gcg acc gga gtg gcg ccg 720
Glu Ala Leu Glu Ala Leu Arg Trp Ser Leu Ala Thr Gly Val Ala Pro
225                 230                 235                 240

gtg ctg atc acc agc gcg ctc gcc cag ggc gtg cgg gcc atc ggc aag 768
Val Leu Ile Thr Ser Ala Leu Ala Gln Gly Val Arg Ala Ile Gly Lys
                245                 250                 255

ctg tcc tcc gcc cgc ggc gga cgc ccc gcc gac ctc gcc cgc gag ctg 816
Leu Ser Ser Ala Arg Gly Gly Arg Pro Ala Asp Leu Ala Arg Glu Leu
            260                 265                 270

ggg atg ccg ccc tgg aag atc gac cgc gtc cgg cag cag atg cgc ggc 864
Gly Met Pro Pro Trp Lys Ile Asp Arg Val Arg Gln Gln Met Arg Gly
        275                 280                 285

tgg acc ccg gac ggc gtc tcc gtg gcc ctg cgc gcg gtc gcc gag gcc 912
Trp Thr Pro Asp Gly Val Ser Val Ala Leu Arg Ala Val Ala Glu Ala
    290                 295                 300

gac gcg ggg gtg aag ggc ggc ggg gac gat ccc gag tac gcc ctg gag 960
Asp Ala Gly Val Lys Gly Gly Gly Asp Asp Pro Glu Tyr Ala Leu Glu

305                 310                 315                 320

aag gcc gtg gtg atc atc gcg cgg gcg gcg cgt tca cgg ggc cgg acc     1008
Lys Ala Val Val Ile Ile Ala Arg Ala Ala Arg Ser Arg Gly Arg Thr
                325                 330                 335

tga                                                                 1011

<210> 102
<211> 336
<212> PRT
<213> Streptomyces coelicolor

<400> 102
Val Arg Ala Met Leu Val Ala Met Ala Arg Lys Thr Ala Asn Asp Asp
1               5                   10                  15

Pro Leu Ala Pro Val Thr Leu Ala Val Gly Gln Glu Asp Leu Leu Leu
            20                  25                  30

Asp Arg Ala Val Gln Glu Val Val Ala Ala Ala Lys Ala Ala Asp Ala
        35                  40                  45

Asp Thr Asp Val Arg Asp Leu Thr Pro Asp Gln Leu Gln Pro Gly Thr
    50                  55                  60

Leu Ala Glu Leu Thr Ser Pro Ser Leu Phe Ala Glu Arg Lys Val Val
65                  70                  75                  80

Val Val Arg Asn Ala Gln Asp Leu Ser Ala Asp Thr Val Lys Asp Val
                85                  90                  95

Lys Ala Tyr Leu Gly Ala Pro Ala Glu Glu Ile Thr Leu Val Leu Leu
            100                 105                 110

His Ala Gly Gly Ala Lys Gly Lys Gly Val Leu Asp Ala Gly Arg Lys
        115                 120                 125

Ala Gly Ala Arg Glu Val Ala Cys Pro Lys Met Thr Lys Pro Ala Asp
    130                 135                 140

Arg Leu Ala Phe Val Arg Ala Glu Phe Arg Thr Ala Gly Arg Ser Ala
145                 150                 155                 160

Thr Pro Glu Ala Cys Gln Ala Leu Val Asp Ala Ile Gly Ser Asp Leu
                165                 170                 175

Arg Glu Leu Ala Ser Ala Val Ser Gln Leu Thr Ala Asp Val Glu Gly
            180                 185                 190

Thr Val Asp Glu Ala Val Val Gly Arg Tyr Tyr Thr Gly Arg Ala Glu
        195                 200                 205

Ala Ser Ser Phe Thr Val Ala Asp Arg Ala Val Glu Gly Arg Ala Ala
    210                 215                 220

Glu Ala Leu Glu Ala Leu Arg Trp Ser Leu Ala Thr Gly Val Ala Pro
225                 230                 235                 240

Val Leu Ile Thr Ser Ala Leu Ala Gln Gly Val Arg Ala Ile Gly Lys
                245                 250                 255

Leu Ser Ser Ala Arg Gly Gly Arg Pro Ala Asp Leu Ala Arg Glu Leu
            260                 265                 270

Gly Met Pro Pro Trp Lys Ile Asp Arg Val Arg Gln Gln Met Arg Gly
        275                 280                 285

Trp Thr Pro Asp Gly Val Ser Val Ala Leu Arg Ala Val Ala Glu Ala
    290                 295                 300

Asp Ala Gly Val Lys Gly Gly Gly Asp Asp Pro Glu Tyr Ala Leu Glu
305                 310                 315                 320

Lys Ala Val Val Ile Ile Ala Arg Ala Ala Arg Ser Arg Gly Arg Thr
                325                 330                 335

<210> 103
<211> 1041
<212> DNA
<213> Streptococcus pyogenes

<400> 103
atgattgcga tagaaaagat tgaaaaactg agtaaagaaa atttgggtct tataaccctt 60

gtcacaggag atgacattgg tcagtatagc cagttgaaat cccgcttaat ggagcagatt 120

gcttttgata aggatgattt ggcctattct tactttgata tgtctgaggc cgcttatcag 180

gatgcagaaa tggatctagt gagcctaccc ttctttgctg agcagaaggt ggttattttt 240

gaccatttgt tagatatcac gaccaataaa aaaagtttct taaaagaaaa agacctaaag 300

gcctttgaag cctatttaga aaatccctta gagactactc gactaattat ctttgctcca 360

ggtaaattgg atagtaagag acggcttgtt aagcttttga aacgtgatgc ccttgtttta 420

gaagccaacc ctctgaaaga agcagagcta agaacttatt ttcaaaaata cagtcatcaa 480

ctgggtttag gtttcgagag tggtgccttt gaccaattac ttttgaaatc aaacgatgat 540

tttagtcaaa tcatgaaaaa catggccttt ttaaaagcct ataaaaaaac gggaaatatt 600

agcctaactg atattgagca agccattcct aaaagtttac aagataatat tttcgatctg 660

actagacttg tcctaggagg taaaattgat gcggctagag atttgattca tgatttacgg 720

ttatctggag aagatgacat taaattaatc gctatcatgc taggccaatt tcgcttattt 780

ttgcagctga ctattcttgc tagagatgta aaaaacgagc aacaactagt gattagttta 840

tcagatattc ttgggcggcg ggttaatcct taccaggtca agtatgcgtt aaaggattct 900

aggaccttat ctcttgcctt tctaacagga gcggtgaaaa ccttgattga gacagattac 960

cagataaaaa caggacttta tgagaagagt tatctagttg atattgctct cttaaaaatc 1020

atgactcact ctcaaaaata g 1041

<210> 104
<211> 1041
<212> DNA
<213> Streptococcus pyogenes

<220>
<221> CDS
<222> (1)..(1041)

<400> 104
atg att gcg ata gaa aag att gaa aaa ctg agt aaa gaa aat ttg ggt 48
Met Ile Ala Ile Glu Lys Ile Glu Lys Leu Ser Lys Glu Asn Leu Gly
1               5                   10                  15

ctt ata acc ctt gtc aca gga gat gac att ggt cag tat agc cag ttg 96
Leu Ile Thr Leu Val Thr Gly Asp Asp Ile Gly Gln Tyr Ser Gln Leu
            20                  25                  30

aaa tcc cgc tta atg gag cag att gct ttt gat aag gat gat ttg gcc 144
Lys Ser Arg Leu Met Glu Gln Ile Ala Phe Asp Lys Asp Asp Leu Ala
        35                  40                  45

tat tct tac ttt gat atg tct gag gcc gct tat cag gat gca gaa atg 192
Tyr Ser Tyr Phe Asp Met Ser Glu Ala Ala Tyr Gln Asp Ala Glu Met
    50                  55                  60

gat cta gtg agc cta ccc ttc ttt gct gag cag aag gtg gtt att ttt 240
Asp Leu Val Ser Leu Pro Phe Phe Ala Glu Gln Lys Val Val Ile Phe
65                  70                  75                  80

gac cat ttg tta gat atc acg acc aat aaa aaa agt ttc tta aaa gaa 288
Asp His Leu Leu Asp Ile Thr Thr Asn Lys Lys Ser Phe Leu Lys Glu
                85                  90                  95

aaa gac cta aag gcc ttt gaa gcc tat tta gaa aat ccc tta gag act 336
Lys Asp Leu Lys Ala Phe Glu Ala Tyr Leu Glu Asn Pro Leu Glu Thr
            100                 105                 110

act cga cta att atc ttt gct cca ggt aaa ttg gat agt aag aga cgg 384
Thr Arg Leu Ile Ile Phe Ala Pro Gly Lys Leu Asp Ser Lys Arg Arg
        115                 120                 125

ctt gtt aag ctt ttg aaa cgt gat gcc ctt gtt tta gaa gcc aac cct 432
Leu Val Lys Leu Leu Lys Arg Asp Ala Leu Val Leu Glu Ala Asn Pro
    130                 135                 140

ctg aaa gaa gca gag cta aga act tat ttt caa aaa tac agt cat caa 480
Leu Lys Glu Ala Glu Leu Arg Thr Tyr Phe Gln Lys Tyr Ser His Gln
145                 150                 155                 160

ctg ggt tta ggt ttc gag agt ggt gcc ttt gac caa tta ctt ttg aaa 528
Leu Gly Leu Gly Phe Glu Ser Gly Ala Phe Asp Gln Leu Leu Leu Lys
                165                 170                 175

tca aac gat gat ttt agt caa atc atg aaa aac atg gcc ttt tta aaa 576
Ser Asn Asp Asp Phe Ser Gln Ile Met Lys Asn Met Ala Phe Leu Lys
            180                 185                 190

gcc tat aaa aaa acg gga aat att agc cta act gat att gag caa gcc 624
Ala Tyr Lys Lys Thr Gly Asn Ile Ser Leu Thr Asp Ile Glu Gln Ala
        195                 200                 205

att cct aaa agt tta caa gat aat att ttc gat ctg act aga ctt gtc 672
Ile Pro Lys Ser Leu Gln Asp Asn Ile Phe Asp Leu Thr Arg Leu Val
    210                 215                 220

cta gga ggt aaa att gat gcg gct aga gat ttg att cat gat tta cgg 720
Leu Gly Gly Lys Ile Asp Ala Ala Arg Asp Leu Ile His Asp Leu Arg
225                 230                 235                 240

tta tct gga gaa gat gac att aaa tta atc gct atc atg cta ggc caa     768
Leu Ser Gly Glu Asp Asp Ile Lys Leu Ile Ala Ile Met Leu Gly Gln
                245                 250                 255

ttt cgc tta ttt ttg cag ctg act att ctt gct aga gat gta aaa aac     816
Phe Arg Leu Phe Leu Gln Leu Thr Ile Leu Ala Arg Asp Val Lys Asn
            260                 265                 270

gag caa caa cta gtg att agt tta tca gat att ctt ggg cgg cgg gtt     864
Glu Gln Gln Leu Val Ile Ser Leu Ser Asp Ile Leu Gly Arg Arg Val
        275                 280                 285

aat cct tac cag gtc aag tat gcg tta aag gat tct agg acc tta tct     912
Asn Pro Tyr Gln Val Lys Tyr Ala Leu Lys Asp Ser Arg Thr Leu Ser
    290                 295                 300

ctt gcc ttt cta aca gga gcg gtg aaa acc ttg att gag aca gat tac     960
Leu Ala Phe Leu Thr Gly Ala Val Lys Thr Leu Ile Glu Thr Asp Tyr
305                 310                 315                 320

cag ata aaa aca gga ctt tat gag aag agt tat cta gtt gat att gct     1008
Gln Ile Lys Thr Gly Leu Tyr Glu Lys Ser Tyr Leu Val Asp Ile Ala
                325                 330                 335

ctc tta aaa atc atg act cac tct caa aaa tag                         1041
Leu Leu Lys Ile Met Thr His Ser Gln Lys
            340                 345

<210> 105
<211> 346
<212> PRT
<213> Streptococcus pyogenes

<400> 105
Met Ile Ala Ile Glu Lys Ile Glu Lys Leu Ser Lys Glu Asn Leu Gly
1               5                   10                  15

Leu Ile Thr Leu Val Thr Gly Asp Asp Ile Gly Gln Tyr Ser Gln Leu
            20                  25                  30

Lys Ser Arg Leu Met Glu Gln Ile Ala Phe Asp Lys Asp Asp Leu Ala
        35                  40                  45

Tyr Ser Tyr Phe Asp Met Ser Glu Ala Ala Tyr Gln Asp Ala Glu Met
    50                  55                  60

Asp Leu Val Ser Leu Pro Phe Phe Ala Glu Gln Lys Val Val Ile Phe
65                  70                  75                  80

Asp His Leu Leu Asp Ile Thr Thr Asn Lys Lys Ser Phe Leu Lys Glu
                85                  90                  95

Lys Asp Leu Lys Ala Phe Glu Ala Tyr Leu Glu Asn Pro Leu Glu Thr
            100                 105                 110

Thr Arg Leu Ile Ile Phe Ala Pro Gly Lys Leu Asp Ser Lys Arg Arg
        115                 120                 125

Leu Val Lys Leu Leu Lys Arg Asp Ala Leu Val Leu Glu Ala Asn Pro
    130                 135                 140

Leu Lys Glu Ala Glu Leu Arg Thr Tyr Phe Gln Lys Tyr Ser His Gln
145                 150                 155                 160

Leu Gly Leu Gly Phe Glu Ser Gly Ala Phe Asp Gln Leu Leu Leu Lys
                165                 170                 175

Ser Asn Asp Asp Phe Ser Gln Ile Met Lys Asn Met Ala Phe Leu Lys
            180                 185                 190

Ala Tyr Lys Lys Thr Gly Asn Ile Ser Leu Thr Asp Ile Glu Gln Ala
        195                 200                 205

Ile Pro Lys Ser Leu Gln Asp Asn Ile Phe Asp Leu Thr Arg Leu Val
    210                 215                 220

Leu Gly Gly Lys Ile Asp Ala Ala Arg Asp Leu Ile His Asp Leu Arg
225                 230                 235                 240

Leu Ser Gly Glu Asp Asp Ile Lys Leu Ile Ala Ile Met Leu Gly Gln
                245                 250                 255

Phe Arg Leu Phe Leu Gln Leu Thr Ile Leu Ala Arg Asp Val Lys Asn
            260                 265                 270

Glu Gln Gln Leu Val Ile Ser Leu Ser Asp Ile Leu Gly Arg Arg Val
        275                 280                 285

Asn Pro Tyr Gln Val Lys Tyr Ala Leu Lys Asp Ser Arg Thr Leu Ser
    290                 295                 300

Leu Ala Phe Leu Thr Gly Ala Val Lys Thr Leu Ile Glu Thr Asp Tyr
305                 310                 315                 320

Gln Ile Lys Thr Gly Leu Tyr Glu Lys Ser Tyr Leu Val Asp Ile Ala
                325                 330                 335

Leu Leu Lys Ile Met Thr His Ser Gln Lys
            340                 345

<210> 106
<211> 879
<212> DNA
<213> Thermus thermophilus

<400> 106
atggtcatcg ccttcaccgg ggatcccttc ctggcgcggg aggccctctt agaggaggca 60

aggcttaggg gcctttcccg cttcaccgag cccaccccgg aggccctggc ccaggccctc 120

gccccggggc ttttcggggg cgggggggcg atgctggacc tgagggaggt gggggaggcg 180

gagtggaagg ccctaaagcc cctcctggaa agcgtgcccg agggcgtccc cgtcctcctc 240

ctggacccta agccaagccc ctcccgggcg gccttctacc ggaaccggga aaggcgggac 300

ttccccaccc ccaagggaaa ggacctggtg cggcacctgg aaaaccgggc caagcgcctg 360

gggctcaggc tcccgggcgg ggtggcccag tacctggcct ccctggaggg ggacctcgag 420

gccctggaac gggagctgga gaagcttgcc ctcctctccc ctcccctcac cctggagaag 480

gtggagaagg tggtggccct gaggcccccc ctcacgggct ttgacctggt gcgctccgtc 540

ctggagaagg accccaagga ggccctcctg cgcctcaggc gcctcaagga ggagggggag 600

gagcccctca ggctcctcgg ggccctctcc tggcagttcg ccctcctcgc ccgggccttc 660

ttcctcctcc gggaaaaccc caggcccaag gaggaggacc tcgcccgcct cgaggcccac 720

ccctacgccg ccaaaaaggc cctggaggcg gcgaggcgcc ttacggaaga agccctcaag 780

gaggccctgg acgccctcat ggaggcggaa aagagggcca agggggggaa agacccatgg 840

cttgccctgg aggcggcggt cctccgcctc gcccgttga 879

<210> 107
<211> 879
<212> DNA
<213> Thermus thermophilus

<220>
<221> CDS
<222> (1)..(879)

<400> 107
atg gtc atc gcc ttc acc ggg gat ccc ttc ctg gcg cgg gag gcc ctc 48
Met Val Ile Ala Phe Thr Gly Asp Pro Phe Leu Ala Arg Glu Ala Leu
1               5                   10                  15

tta gag gag gca agg ctt agg ggc ctt tcc cgc ttc acc gag ccc acc 96
Leu Glu Glu Ala Arg Leu Arg Gly Leu Ser Arg Phe Thr Glu Pro Thr
            20                  25                  30

ccg gag gcc ctg gcc cag gcc ctc gcc ccg ggg ctt ttc ggg ggc ggg 144
Pro Glu Ala Leu Ala Gln Ala Leu Ala Pro Gly Leu Phe Gly Gly Gly
        35                  40                  45

ggg gcg atg ctg gac ctg agg gag gtg ggg gag gcg gag tgg aag gcc 192
Gly Ala Met Leu Asp Leu Arg Glu Val Gly Glu Ala Glu Trp Lys Ala
    50                  55                  60

cta aag ccc ctc ctg gaa agc gtg ccc gag ggc gtc ccc gtc ctc ctc 240
Leu Lys Pro Leu Leu Glu Ser Val Pro Glu Gly Val Pro Val Leu Leu
65                  70                  75                  80

ctg gac cct aag cca agc ccc tcc cgg gcg gcc ttc tac cgg aac cgg 288
Leu Asp Pro Lys Pro Ser Pro Ser Arg Ala Ala Phe Tyr Arg Asn Arg
                85                  90                  95

gaa agg cgg gac ttc ccc acc ccc aag gga aag gac ctg gtg cgg cac 336
Glu Arg Arg Asp Phe Pro Thr Pro Lys Gly Lys Asp Leu Val Arg His
            100                 105                 110

ctg gaa aac cgg gcc aag cgc ctg ggg ctc agg ctc ccg ggc ggg gtg 384
Leu Glu Asn Arg Ala Lys Arg Leu Gly Leu Arg Leu Pro Gly Gly Val
        115                 120                 125

gcc cag tac ctg gcc tcc ctg gag ggg gac ctc gag gcc ctg gaa cgg 432
Ala Gln Tyr Leu Ala Ser Leu Glu Gly Asp Leu Glu Ala Leu Glu Arg
    130                 135                 140

gag ctg gag aag ctt gcc ctc ctc tcc cct ccc ctc acc ctg gag aag 480
Glu Leu Glu Lys Leu Ala Leu Leu Ser Pro Pro Leu Thr Leu Glu Lys
145                 150                 155                 160

gtg gag aag gtg gtg gcc ctg agg ccc ccc ctc acg ggc ttt gac ctg 528
Val Glu Lys Val Val Ala Leu Arg Pro Pro Leu Thr Gly Phe Asp Leu

                165                 170                 175

gtg cgc tcc gtc ctg gag aag gac ccc aag gag gcc ctc ctg cgc ctc     576
Val Arg Ser Val Leu Glu Lys Asp Pro Lys Glu Ala Leu Leu Arg Leu
            180                 185                 190

agg cgc ctc aag gag gag ggg gag gag ccc ctc agg ctc ctc ggg gcc     624
Arg Arg Leu Lys Glu Glu Gly Glu Glu Pro Leu Arg Leu Leu Gly Ala
        195                 200                 205

ctc tcc tgg cag ttc gcc ctc ctc gcc cgg gcc ttc ttc ctc ctc cgg     672
Leu Ser Trp Gln Phe Ala Leu Leu Ala Arg Ala Phe Phe Leu Leu Arg
    210                 215                 220

gaa aac ccc agg ccc aag gag gag gac ctc gcc cgc ctc gag gcc cac     720
Glu Asn Pro Arg Pro Lys Glu Glu Asp Leu Ala Arg Leu Glu Ala His
225                 230                 235                 240

ccc tac gcc gcc aaa aag gcc ctg gag gcg gcg agg cgc ctt acg gaa     768
Pro Tyr Ala Ala Lys Lys Ala Leu Glu Ala Ala Arg Arg Leu Thr Glu
                245                 250                 255

gaa gcc ctc aag gag gcc ctg gac gcc ctc atg gag gcg gaa aag agg     816
Glu Ala Leu Lys Glu Ala Leu Asp Ala Leu Met Glu Ala Glu Lys Arg
            260                 265                 270

gcc aag ggg ggg aaa gac cca tgg ctt gcc ctg gag gcg gcg gtc ctc     864
Ala Lys Gly Gly Lys Asp Pro Trp Leu Ala Leu Glu Ala Ala Val Leu
        275                 280                 285

cgc ctc gcc cgt tga                                                 879
Arg Leu Ala Arg
    290

<210> 108
<211> 292
<212> PRT
<213> Thermus thermophilus

<400> 108
Met Val Ile Ala Phe Thr Gly Asp Pro Phe Leu Ala Arg Glu Ala Leu
1               5                   10                  15

Leu Glu Glu Ala Arg Leu Arg Gly Leu Ser Arg Phe Thr Glu Pro Thr
            20                  25                  30

Pro Glu Ala Leu Ala Gln Ala Leu Ala Pro Gly Leu Phe Gly Gly Gly

35                  40                  45

Gly Ala Met Leu Asp Leu Arg Glu Val Gly Glu Ala Glu Trp Lys Ala
    50                  55                  60

Leu Lys Pro Leu Leu Glu Ser Val Pro Glu Gly Val Pro Val Leu Leu
65                  70                  75                  80

Leu Asp Pro Lys Pro Ser Pro Ser Arg Ala Ala Phe Tyr Arg Asn Arg
                85                  90                  95

Glu Arg Arg Asp Phe Pro Thr Pro Lys Gly Lys Asp Leu Val Arg His
            100                 105                 110

Leu Glu Asn Arg Ala Lys Arg Leu Gly Leu Arg Leu Pro Gly Gly Val
        115                 120                 125

Ala Gln Tyr Leu Ala Ser Leu Glu Gly Asp Leu Glu Ala Leu Glu Arg
    130                 135                 140

Glu Leu Glu Lys Leu Ala Leu Leu Ser Pro Pro Leu Thr Leu Glu Lys
145                 150                 155                 160

Val Glu Lys Val Val Ala Leu Arg Pro Pro Leu Thr Gly Phe Asp Leu
                165                 170                 175

Val Arg Ser Val Leu Glu Lys Asp Pro Lys Glu Ala Leu Leu Arg Leu
            180                 185                 190

Arg Arg Leu Lys Glu Glu Gly Glu Glu Pro Leu Arg Leu Leu Gly Ala
        195                 200                 205

Leu Ser Trp Gln Phe Ala Leu Leu Ala Arg Ala Phe Phe Leu Leu Arg
    210                 215                 220

Glu Asn Pro Arg Pro Lys Glu Glu Asp Leu Ala Arg Leu Glu Ala His
225                 230                 235                 240

Pro Tyr Ala Ala Lys Lys Ala Leu Glu Ala Ala Arg Arg Leu Thr Glu
                245                 250                 255

Glu Ala Leu Lys Glu Ala Leu Asp Ala Leu Met Glu Ala Glu Lys Arg
            260                 265                 270

Ala Lys Gly Gly Lys Asp Pro Trp Leu Ala Leu Glu Ala Ala Val Leu
        275                 280                 285

Arg Leu Ala Arg

    290

<210> 109
<211> 1035
<212> DNA
<213> Yersinia pestis

<400> 109
atgatccgga tttaccctga acaacttgtc gcgcagctcc atgaggggct acgcgcttgt 60

tatctattgt gtggcaatga acccttgttg ctacaggaaa gtcaggacca cattcgtcgg 120

gtggcatcgc agcacgattt cactgagcat ttcagctttg cgttggacgc gcacaccgaa 180

tgggagcata tttttagcct ttgccaggca ctgagtttgt tcgccagccg ccagacactg 240

ctgctgagtt tcccagacag tgggctgacc gcgccaatta gcgagcagtt ggttaaatta 300

tctggcctgt tacacccaga cattttattg atattgcggg cgaataagtt gaccaaagcg 360

caagagaaca gcgcctggtt taaagctctc agtaaaaatg gggtatttgt cagttgccag 420

acaccagaac aggcccagct tccccgctgg gtgagcgctc gcgccaagag cctcaacttg 480

aacgtagacg atgccgccat tcaattactt tgctactgct atgaaggtaa cttgctggcc 540

ttatctcagg cgttagaacg gctctcgctg ctctaccccg acggtaagtt aaccctgcct 600

aaagtagaac aagccgtcaa cgacgctgcg catttcacac cataccattg gcttgatgcc 660

ttgttgatgg ggaagagcaa gcgggcttgg catattttgc agcaattaca gcaagaagac 720

agtgaacccg ttattttact gcgtaccgta caacgtgaac tgctcctgct gttagcgctc 780

aaacgtcaga tggaacaagt tccattgcgc gcattattcg atcaacacaa aatttggcag 840

aaccggcggc ccatgatgac tcaggcactg caacggctct cccttcagca actgcaacag 900

gcggttcatc tgctcacaca gatggaaatc cggctgaaac aggattatgg tcaatcaatc 960

tggcctgagt tagaaacgct atctatgctg atgtgtggaa aaacattacc tgagagcttt 1020

tttgatgccc attaa 1035

<210> 110
<211> 1035

<212> DNA
<213> Yersinia pestis

<220>
<221> CDS
<222> (1)..(1035)

<400> 110
atg atc cgg att tac cct gaa caa ctt gtc gcg cag ctc cat gag ggg     48
Met Ile Arg Ile Tyr Pro Glu Gln Leu Val Ala Gln Leu His Glu Gly
1               5                   10                  15

cta cgc gct tgt tat cta ttg tgt ggc aat gaa ccc ttg ttg cta cag     96
Leu Arg Ala Cys Tyr Leu Leu Cys Gly Asn Glu Pro Leu Leu Leu Gln
            20                  25                  30

gaa agt cag gac cac att cgt cgg gtg gca tcg cag cac gat ttc act     144
Glu Ser Gln Asp His Ile Arg Arg Val Ala Ser Gln His Asp Phe Thr
        35                  40                  45

gag cat ttc agc ttt gcg ttg gac gcg cac acc gaa tgg gag cat att     192
Glu His Phe Ser Phe Ala Leu Asp Ala His Thr Glu Trp Glu His Ile
    50                  55                  60

ttt agc ctt tgc cag gca ctg agt ttg ttc gcc agc cgc cag aca ctg     240
Phe Ser Leu Cys Gln Ala Leu Ser Leu Phe Ala Ser Arg Gln Thr Leu
65                  70                  75                  80

ctg ctg agt ttc cca gac agt ggg ctg acc gcg cca att agc gag cag     288
Leu Leu Ser Phe Pro Asp Ser Gly Leu Thr Ala Pro Ile Ser Glu Gln
                85                  90                  95

ttg gtt aaa tta tct ggc ctg tta cac cca gac att tta ttg ata ttg     336
Leu Val Lys Leu Ser Gly Leu Leu His Pro Asp Ile Leu Leu Ile Leu
            100                 105                 110

cgg gcg aat aag ttg acc aaa gcg caa gag aac agc gcc tgg ttt aaa     384
Arg Ala Asn Lys Leu Thr Lys Ala Gln Glu Asn Ser Ala Trp Phe Lys
        115                 120                 125

gct ctc agt aaa aat ggg gta ttt gtc agt tgc cag aca cca gaa cag     432
Ala Leu Ser Lys Asn Gly Val Phe Val Ser Cys Gln Thr Pro Glu Gln
    130                 135                 140

gcc cag ctt ccc cgc tgg gtg agc gct cgc gcc aag agc ctc aac ttg     480
Ala Gln Leu Pro Arg Trp Val Ser Ala Arg Ala Lys Ser Leu Asn Leu
145                 150                 155                 160

aac gta gac gat gcc gcc att caa tta ctt tgc tac tgc tat gaa ggt 528
Asn Val Asp Asp Ala Ala Ile Gln Leu Leu Cys Tyr Cys Tyr Glu Gly
                165                 170                 175

aac ttg ctg gcc tta tct cag gcg tta gaa cgg ctc tcg ctg ctc tac 576
Asn Leu Leu Ala Leu Ser Gln Ala Leu Glu Arg Leu Ser Leu Leu Tyr
            180                 185                 190

ccc gac ggt aag tta acc ctg cct aaa gta gaa caa gcc gtc aac gac 624
Pro Asp Gly Lys Leu Thr Leu Pro Lys Val Glu Gln Ala Val Asn Asp
        195                 200                 205

gct gcg cat ttc aca cca tac cat tgg ctt gat gcc ttg ttg atg ggg 672
Ala Ala His Phe Thr Pro Tyr His Trp Leu Asp Ala Leu Leu Met Gly
    210                 215                 220

aag agc aag cgg gct tgg cat att ttg cag caa tta cag caa gaa gac 720
Lys Ser Lys Arg Ala Trp His Ile Leu Gln Gln Leu Gln Gln Glu Asp
225                 230                 235                 240

agt gaa ccc gtt att tta ctg cgt acc gta caa cgt gaa ctg ctc ctg 768
Ser Glu Pro Val Ile Leu Leu Arg Thr Val Gln Arg Glu Leu Leu Leu
                245                 250                 255

ctg tta gcg ctc aaa cgt cag atg gaa caa gtt cca ttg cgc gca tta 816
Leu Leu Ala Leu Lys Arg Gln Met Glu Gln Val Pro Leu Arg Ala Leu
            260                 265                 270

ttc gat caa cac aaa att tgg cag aac cgg cgg ccc atg atg act cag 864
Phe Asp Gln His Lys Ile Trp Gln Asn Arg Arg Pro Met Met Thr Gln
        275                 280                 285

gca ctg caa cgg ctc tcc ctt cag caa ctg caa cag gcg gtt cat ctg 912
Ala Leu Gln Arg Leu Ser Leu Gln Gln Leu Gln Gln Ala Val His Leu
    290                 295                 300

ctc aca cag atg gaa atc cgg ctg aaa cag gat tat ggt caa tca atc 960
Leu Thr Gln Met Glu Ile Arg Leu Lys Gln Asp Tyr Gly Gln Ser Ile
305                 310                 315                 320

tgg cct gag tta gaa acg cta tct atg ctg atg tgt gga aaa aca tta 1008
Trp Pro Glu Leu Glu Thr Leu Ser Met Leu Met Cys Gly Lys Thr Leu
                325                 330                 335

cct gag agc ttt ttt gat gcc cat taa 1035
Pro Glu Ser Phe Phe Asp Ala His
340 345

<210> 111
<211> 344
<212> PRT
<213> Yersinia pestis

<400> 111
Met Ile Arg Ile Tyr Pro Glu Gln Leu Val Ala Gln Leu His Glu Gly
1               5                   10                  15

Leu Arg Ala Cys Tyr Leu Leu Cys Gly Asn Glu Pro Leu Leu Leu Gln
            20                  25                  30

Glu Ser Gln Asp His Ile Arg Arg Val Ala Ser Gln His Asp Phe Thr
        35                  40                  45

Glu His Phe Ser Phe Ala Leu Asp Ala His Thr Glu Trp Glu His Ile
    50                  55                  60

Phe Ser Leu Cys Gln Ala Leu Ser Leu Phe Ala Ser Arg Gln Thr Leu
65                  70                  75                  80

Leu Leu Ser Phe Pro Asp Ser Gly Leu Thr Ala Pro Ile Ser Glu Gln
                85                  90                  95

Leu Val Lys Leu Ser Gly Leu Leu His Pro Asp Ile Leu Leu Ile Leu
            100                 105                 110

Arg Ala Asn Lys Leu Thr Lys Ala Gln Glu Asn Ser Ala Trp Phe Lys
        115                 120                 125

Ala Leu Ser Lys Asn Gly Val Phe Val Ser Cys Gln Thr Pro Glu Gln
    130                 135                 140

Ala Gln Leu Pro Arg Trp Val Ser Ala Arg Ala Lys Ser Leu Asn Leu
145                 150                 155                 160

Asn Val Asp Asp Ala Ala Ile Gln Leu Leu Cys Tyr Cys Tyr Glu Gly
                165                 170                 175

Asn Leu Leu Ala Leu Ser Gln Ala Leu Glu Arg Leu Ser Leu Leu Tyr
            180                 185                 190

Pro Asp Gly Lys Leu Thr Leu Pro Lys Val Glu Gln Ala Val Asn Asp
        195                 200                 205

Ala Ala His Phe Thr Pro Tyr His Trp Leu Asp Ala Leu Leu Met Gly
    210                 215                 220

Lys Ser Lys Arg Ala Trp His Ile Leu Gln Gln Leu Gln Gln Glu Asp
225                 230                 235                 240

Ser Glu Pro Val Ile Leu Leu Arg Thr Val Gln Arg Glu Leu Leu Leu
                245                 250                 255

Leu Leu Ala Leu Lys Arg Gln Met Glu Gln Val Pro Leu Arg Ala Leu
            260                 265                 270

Phe Asp Gln His Lys Ile Trp Gln Asn Arg Arg Pro Met Met Thr Gln
        275                 280                 285

Ala Leu Gln Arg Leu Ser Leu Gln Gln Leu Gln Gln Ala Val His Leu
    290                 295                 300

Leu Thr Gln Met Glu Ile Arg Leu Lys Gln Asp Tyr Gly Gln Ser Ile
305                 310                 315                 320

Trp Pro Glu Leu Glu Thr Leu Ser Met Leu Met Cys Gly Lys Thr Leu
                325                 330                 335

Pro Glu Ser Phe Phe Asp Ala His
            340

<210> 112
<211> 1038
<212> DNA
<213> Streptococcus agalactiae

<400> 112
atgattgcga ttgaagaaat tgggagaatt accccagata atttagggtt agtaactgtt 60

ttagcaggag aagatttagg gcaatatgct cagatgaaag agaagctatt tcaggtaatt 120

ggattcaata aagacgattt agcctattct tattttgatt taagtgaaga agactatcaa 180

aatgcggaat tagacttaga aagtttacct tttttatcag actacaaggt tgttattttt 240

gatcagtttc aagatattac aactgataaa aaaacttatt tagatgaaca agcaatgaag 300

cgcttggaag cctacttgca gaatcctgta gatactacga gacttgttat ttgtgctcca 360

ggaaaacttg atggaaaacg tcgacttgta aagttattga aacgtgatgc tcgtgtttta 420

gaggctaata ctttaaaaga atcagattta aagacgtatt ttcaaaaata tgctcaccaa 480

gaaggattag tttttgaagc aggtgttttt gatgagttat tgattaagtc taactatgat 540

tttagtgata ctttaacgaa tatcgctttc ttaaagtcat ataaaacaga cggtcatatt 600

tcgtccaatg atgttagaga ggctatccct aaaagcctgc aagataatat atttgatttg 660

actcaagatg tcttacttgg caggatagat ttaccgcgtg atttagtaag agatttacgt 720

ctacaaggcg aagacgaaat caaattgatt gcaatcatgt taggacagtt tcggatgttt 780

ttgcaagtta aaatattggc tagtaaaggg aagtcagaga gtcaaattgt ttctgaacta 840

tcccattaca taggacgtaa aatcaaccct tatcaagtaa aatttgctgt cagagattct 900

agaaaccttc cccttgcttt tttaaaagaa gcgattagga ttttaatcga gactgactat 960

gctattaaaa gagggactta tgataaggat tatctctttg atttagcctt attaaaaatt 1020

gctcataaaa agccctaa 1038

<210> 113
<211> 1038
<212> DNA
<213> Streptococcus agalactiae

<220>
<221> CDS
<222> (1)..(1038)

<400> 113
atg att gcg att gaa gaa att ggg aga att acc cca gat aat tta ggg 48
Met Ile Ala Ile Glu Glu Ile Gly Arg Ile Thr Pro Asp Asn Leu Gly
1               5                   10                  15

tta gta act gtt tta gca gga gaa gat tta ggg caa tat gct cag atg 96
Leu Val Thr Val Leu Ala Gly Glu Asp Leu Gly Gln Tyr Ala Gln Met
            20                  25                  30

aaa gag aag cta ttt cag gta att gga ttc aat aaa gac gat tta gcc 144
Lys Glu Lys Leu Phe Gln Val Ile Gly Phe Asn Lys Asp Asp Leu Ala
        35                  40                  45

tat tct tat ttt gat tta agt gaa gaa gac tat caa aat gcg gaa tta 192
Tyr Ser Tyr Phe Asp Leu Ser Glu Glu Asp Tyr Gln Asn Ala Glu Leu
    50                  55                  60

gac tta gaa agt tta cct ttt tta tca gac tac aag gtt gtt att ttt 240

Asp Leu Glu Ser Leu Pro Phe Leu Ser Asp Tyr Lys Val Val Ile Phe
65                  70                  75                  80

gat cag ttt caa gat att aca act gat aaa aaa act tat tta gat gaa     288
Asp Gln Phe Gln Asp Ile Thr Thr Asp Lys Lys Thr Tyr Leu Asp Glu
                85                  90                  95

caa gca atg aag cgc ttg gaa gcc tac ttg cag aat cct gta gat act     336
Gln Ala Met Lys Arg Leu Glu Ala Tyr Leu Gln Asn Pro Val Asp Thr
            100                 105                 110

acg aga ctt gtt att tgt gct cca gga aaa ctt gat gga aaa cgt cga     384
Thr Arg Leu Val Ile Cys Ala Pro Gly Lys Leu Asp Gly Lys Arg Arg
        115                 120                 125

ctt gta aag tta ttg aaa cgt gat gct cgt gtt tta gag gct aat act     432
Leu Val Lys Leu Leu Lys Arg Asp Ala Arg Val Leu Glu Ala Asn Thr
    130                 135                 140

tta aaa gaa tca gat tta aag acg tat ttt caa aaa tat gct cac caa     480
Leu Lys Glu Ser Asp Leu Lys Thr Tyr Phe Gln Lys Tyr Ala His Gln
145                 150                 155                 160

gaa gga tta gtt ttt gaa gca ggt gtt ttt gat gag tta ttg att aag     528
Glu Gly Leu Val Phe Glu Ala Gly Val Phe Asp Glu Leu Leu Ile Lys
                165                 170                 175

tct aac tat gat ttt agt gat act tta acg aat atc gct ttc tta aag     576
Ser Asn Tyr Asp Phe Ser Asp Thr Leu Thr Asn Ile Ala Phe Leu Lys
            180                 185                 190

tca tat aaa aca gac ggt cat att tcg tcc aat gat gtt aga gag gct     624
Ser Tyr Lys Thr Asp Gly His Ile Ser Ser Asn Asp Val Arg Glu Ala
        195                 200                 205

atc cct aaa agc ctg caa gat aat ata ttt gat ttg act caa gat gtc     672
Ile Pro Lys Ser Leu Gln Asp Asn Ile Phe Asp Leu Thr Gln Asp Val
    210                 215                 220

tta ctt ggc agg ata gat tta ccg cgt gat tta gta aga gat tta cgt     720
Leu Leu Gly Arg Ile Asp Leu Pro Arg Asp Leu Val Arg Asp Leu Arg
225                 230                 235                 240

cta caa ggc gaa gac gaa atc aaa ttg att gca atc atg tta gga cag     768
Leu Gln Gly Glu Asp Glu Ile Lys Leu Ile Ala Ile Met Leu Gly Gln
                245                 250                 255

ttt cgg atg ttt ttg caa gtt aaa ata ttg gct agt aaa ggg aag tca     816

Phe Arg Met Phe Leu Gln Val Lys Ile Leu Ala Ser Lys Gly Lys Ser
                260                 265                 270

gag agt caa att gtt tct gaa cta tcc cat tac ata gga cgt aaa atc     864
Glu Ser Gln Ile Val Ser Glu Leu Ser His Tyr Ile Gly Arg Lys Ile
            275                 280                 285

aac cct tat caa gta aaa ttt gct gtc aga gat tct aga aac ctt ccc     912
Asn Pro Tyr Gln Val Lys Phe Ala Val Arg Asp Ser Arg Asn Leu Pro
    290                 295                 300

ctt gct ttt tta aaa gaa gcg att agg att tta atc gag act gac tat     960
Leu Ala Phe Leu Lys Glu Ala Ile Arg Ile Leu Ile Glu Thr Asp Tyr
305                 310                 315                 320

gct att aaa aga ggg act tat gat aag gat tat ctc ttt gat tta gcc     1008
Ala Ile Lys Arg Gly Thr Tyr Asp Lys Asp Tyr Leu Phe Asp Leu Ala
                325                 330                 335

tta tta aaa att gct cat aaa aag ccc taa                             1038
Leu Leu Lys Ile Ala His Lys Lys Pro
                340                 345

<210> 114
<211> 345
<212> PRT
<213> Streptococcus agalactiae

<400> 114
Met Ile Ala Ile Glu Glu Ile Gly Arg Ile Thr Pro Asp Asn Leu Gly
  1               5                  10                  15

Leu Val Thr Val Leu Ala Gly Glu Asp Leu Gly Gln Tyr Ala Gln Met
             20                  25                  30

Lys Glu Lys Leu Phe Gln Val Ile Gly Phe Asn Lys Asp Asp Leu Ala
         35                  40                  45

Tyr Ser Tyr Phe Asp Leu Ser Glu Glu Asp Tyr Gln Asn Ala Glu Leu
     50                  55                  60

Asp Leu Glu Ser Leu Pro Phe Leu Ser Asp Tyr Lys Val Val Ile Phe
 65                  70                  75                  80

Asp Gln Phe Gln Asp Ile Thr Thr Asp Lys Lys Thr Tyr Leu Asp Glu
                 85                  90                  95

Gln Ala Met Lys Arg Leu Glu Ala Tyr Leu Gln Asn Pro Val Asp Thr
            100                 105                 110

Thr Arg Leu Val Ile Cys Ala Pro Gly Lys Leu Asp Gly Lys Arg Arg
        115                 120                 125

Leu Val Lys Leu Leu Lys Arg Asp Ala Arg Val Leu Glu Ala Asn Thr
    130                 135                 140

Leu Lys Glu Ser Asp Leu Lys Thr Tyr Phe Gln Lys Tyr Ala His Gln
145                 150                 155                 160

Glu Gly Leu Val Phe Glu Ala Gly Val Phe Asp Glu Leu Leu Ile Lys
                165                 170                 175

Ser Asn Tyr Asp Phe Ser Asp Thr Leu Thr Asn Ile Ala Phe Leu Lys
            180                 185                 190

Ser Tyr Lys Thr Asp Gly His Ile Ser Ser Asn Asp Val Arg Glu Ala
        195                 200                 205

Ile Pro Lys Ser Leu Gln Asp Asn Ile Phe Asp Leu Thr Gln Asp Val
    210                 215                 220

Leu Leu Gly Arg Ile Asp Leu Pro Arg Asp Leu Val Arg Asp Leu Arg
225                 230                 235                 240

Leu Gln Gly Glu Asp Glu Ile Lys Leu Ile Ala Ile Met Leu Gly Gln
                245                 250                 255

Phe Arg Met Phe Leu Gln Val Lys Ile Leu Ala Ser Lys Gly Lys Ser
            260                 265                 270

Glu Ser Gln Ile Val Ser Glu Leu Ser His Tyr Ile Gly Arg Lys Ile
        275                 280                 285

Asn Pro Tyr Gln Val Lys Phe Ala Val Arg Asp Ser Arg Asn Leu Pro
    290                 295                 300

Leu Ala Phe Leu Lys Glu Ala Ile Arg Ile Leu Ile Glu Thr Asp Tyr
305                 310                 315                 320

Ala Ile Lys Arg Gly Thr Tyr Asp Lys Asp Tyr Leu Phe Asp Leu Ala
                325                 330                 335

Leu Leu Lys Ile Ala His Lys Lys Pro
            340                 345

<210> 115
<211> 1038
<212> DNA
<213> Streptococcus pneumonia

<400> 115
atgctagcca ttgaagaaag tcagaagttg actttatcaa atttaccgag cctgagccta 60

tttacaggga cagatcaggg tcagtttgaa gtgatgaaga gtcaaatgtt gaaacagatt 120

gggtatgatt ctgctgacct caactttgcc tactttgata tgaaagaagt agtttacaag 180

gatgtggaac tggagttggt cagccttcct ttctttgcgg atgagaaaat cgtgatatta 240

gattatttta tggatatcac gactgctaag aaacgctttt tgacagatga tgagcttaag 300

tcatttgagg aataccttga caatccttct ccaacaacca agttgataat ctttgcagaa 360

ggaaagctgg atagcaaaag acggttagtc aaattactta agcgtgatgc caaggccttc 420

gatgcagtag aagtaaaaga acaagaattg cgccagtact tccaaaagtg gagtcagaaa 480

caaggtctgc agtttaccaa tcattctttt gaaaatctcc tcatcaagtc ggggtttcaa 540

tttagcgaaa tccagaaaaa tcttctcttt ttacagtcct ataaggcgaa ttctgttatt 600

gaggaagagg atattgttaa cgcaattccc aagaccttgc aggacaatat ttttgattta 660

actcagttta ttctgactaa aaagatggat caggcgcgcg atttggtgag agacttgacc 720

ttgcaagggg aagatgaaat caaactgatt gcagtcatgc tgggacaatt tcggactttt 780

actcaggtga agattttggc ggagtctggc caaacagaat cgcagattgc aagtagttta 840

ggtagttatc tgggacgtaa cccaaatcct tatcaaatca agtttgcatt aagagattcg 900

agaggacttt ctttgagctt tttgaagcaa gctatttcct atttgattga gacagactat 960

cagattaaga caggtcttta tgaaaaaggt ttcctttttg aaaaggcact cttacagatt 1020

gctagtcagg tcaattga 1038

<210> 116
<211> 1038
<212> DNA
<213> Streptococcus pneumonia

<220>
<221> CDS
<222> (1)..(1038)

<400> 116
atg cta gcc att gaa gaa agt cag aag ttg act tta tca aat tta ccg     48
Met Leu Ala Ile Glu Glu Ser Gln Lys Leu Thr Leu Ser Asn Leu Pro
1               5                   10                  15

agc ctg agc cta ttt aca ggg aca gat cag ggt cag ttt gaa gtg atg     96
Ser Leu Ser Leu Phe Thr Gly Thr Asp Gln Gly Gln Phe Glu Val Met
            20                  25                  30

aag agt caa atg ttg aaa cag att ggg tat gat tct gct gac ctc aac     144
Lys Ser Gln Met Leu Lys Gln Ile Gly Tyr Asp Ser Ala Asp Leu Asn
        35                  40                  45

ttt gcc tac ttt gat atg aaa gaa gta gtt tac aag gat gtg gaa ctg     192
Phe Ala Tyr Phe Asp Met Lys Glu Val Val Tyr Lys Asp Val Glu Leu
    50                  55                  60

gag ttg gtc agc ctt cct ttc ttt gcg gat gag aaa atc gtg ata tta     240
Glu Leu Val Ser Leu Pro Phe Phe Ala Asp Glu Lys Ile Val Ile Leu
65                  70                  75                  80

gat tat ttt atg gat atc acg act gct aag aaa cgc ttt ttg aca gat     288
Asp Tyr Phe Met Asp Ile Thr Thr Ala Lys Lys Arg Phe Leu Thr Asp
                85                  90                  95

gat gag ctt aag tca ttt gag gaa tac ctt gac aat cct tct cca aca     336
Asp Glu Leu Lys Ser Phe Glu Glu Tyr Leu Asp Asn Pro Ser Pro Thr
            100                 105                 110

acc aag ttg ata atc ttt gca gaa gga aag ctg gat agc aaa aga cgg     384
Thr Lys Leu Ile Ile Phe Ala Glu Gly Lys Leu Asp Ser Lys Arg Arg
        115                 120                 125

tta gtc aaa tta ctt aag cgt gat gcc aag gcc ttc gat gca gta gaa     432
Leu Val Lys Leu Leu Lys Arg Asp Ala Lys Ala Phe Asp Ala Val Glu
    130                 135                 140

gta aaa gaa caa gaa ttg cgc cag tac ttc caa aag tgg agt cag aaa     480
Val Lys Glu Gln Glu Leu Arg Gln Tyr Phe Gln Lys Trp Ser Gln Lys
145                 150                 155                 160

caa ggt ctg cag ttt acc aat cat tct ttt gaa aat ctc ctc atc aag     528
Gln Gly Leu Gln Phe Thr Asn His Ser Phe Glu Asn Leu Leu Ile Lys

                165                 170                 175

tcg ggg ttt caa ttt agc gaa atc cag aaa aat ctt ctc ttt tta cag 576
Ser Gly Phe Gln Phe Ser Glu Ile Gln Lys Asn Leu Leu Phe Leu Gln
            180                 185                 190

tcc tat aag gcg aat tct gtt att gag gaa gag gat att gtt aac gca 624
Ser Tyr Lys Ala Asn Ser Val Ile Glu Glu Glu Asp Ile Val Asn Ala
        195                 200                 205

att ccc aag acc ttg cag gac aat att ttt gat tta act cag ttt att 672
Ile Pro Lys Thr Leu Gln Asp Asn Ile Phe Asp Leu Thr Gln Phe Ile
    210                 215                 220

ctg act aaa aag atg gat cag gcg cgc gat ttg gtg aga gac ttg acc 720
Leu Thr Lys Lys Met Asp Gln Ala Arg Asp Leu Val Arg Asp Leu Thr
225                 230                 235                 240

ttg caa ggg gaa gat gaa atc aaa ctg att gca gtc atg ctg gga caa 768
Leu Gln Gly Glu Asp Glu Ile Lys Leu Ile Ala Val Met Leu Gly Gln
                245                 250                 255

ttt cgg act ttt act cag gtg aag att ttg gcg gag tct ggc caa aca 816
Phe Arg Thr Phe Thr Gln Val Lys Ile Leu Ala Glu Ser Gly Gln Thr
            260                 265                 270

gaa tcg cag att gca agt agt tta ggt agt tat ctg gga cgt aac cca 864
Glu Ser Gln Ile Ala Ser Ser Leu Gly Ser Tyr Leu Gly Arg Asn Pro
        275                 280                 285

aat cct tat caa atc aag ttt gca tta aga gat tcg aga gga ctt tct 912
Asn Pro Tyr Gln Ile Lys Phe Ala Leu Arg Asp Ser Arg Gly Leu Ser
    290                 295                 300

ttg agc ttt ttg aag caa gct att tcc tat ttg att gag aca gac tat 960
Leu Ser Phe Leu Lys Gln Ala Ile Ser Tyr Leu Ile Glu Thr Asp Tyr
305                 310                 315                 320

cag att aag aca ggt ctt tat gaa aaa ggt ttc ctt ttt gaa aag gca 1008
Gln Ile Lys Thr Gly Leu Tyr Glu Lys Gly Phe Leu Phe Glu Lys Ala
                325                 330                 335

ctc tta cag att gct agt cag gtc aat tga 1038
Leu Leu Gln Ile Ala Ser Gln Val Asn
            340                 345

<210> 117

<211> 345
<212> PRT
<213> Streptococcus pneumonia

<400> 117
Met Leu Ala Ile Glu Glu Ser Gln Lys Leu Thr Leu Ser Asn Leu Pro
1               5                   10                  15

Ser Leu Ser Leu Phe Thr Gly Thr Asp Gln Gly Gln Phe Glu Val Met
            20                  25                  30

Lys Ser Gln Met Leu Lys Gln Ile Gly Tyr Asp Ser Ala Asp Leu Asn
        35                  40                  45

Phe Ala Tyr Phe Asp Met Lys Glu Val Val Tyr Lys Asp Val Glu Leu
    50                  55                  60

Glu Leu Val Ser Leu Pro Phe Phe Ala Asp Glu Lys Ile Val Ile Leu
65                  70                  75                  80

Asp Tyr Phe Met Asp Ile Thr Thr Ala Lys Lys Arg Phe Leu Thr Asp
                85                  90                  95

Asp Glu Leu Lys Ser Phe Glu Glu Tyr Leu Asp Asn Pro Ser Pro Thr
            100                 105                 110

Thr Lys Leu Ile Ile Phe Ala Glu Gly Lys Leu Asp Ser Lys Arg Arg
        115                 120                 125

Leu Val Lys Leu Leu Lys Arg Asp Ala Lys Ala Phe Asp Ala Val Glu
    130                 135                 140

Val Lys Glu Gln Glu Leu Arg Gln Tyr Phe Gln Lys Trp Ser Gln Lys
145                 150                 155                 160

Gln Gly Leu Gln Phe Thr Asn His Ser Phe Glu Asn Leu Leu Ile Lys
                165                 170                 175

Ser Gly Phe Gln Phe Ser Glu Ile Gln Lys Asn Leu Leu Phe Leu Gln
            180                 185                 190

Ser Tyr Lys Ala Asn Ser Val Ile Glu Glu Glu Asp Ile Val Asn Ala
        195                 200                 205

Ile Pro Lys Thr Leu Gln Asp Asn Ile Phe Asp Leu Thr Gln Phe Ile
    210                 215                 220

Leu Thr Lys Lys Met Asp Gln Ala Arg Asp Leu Val Arg Asp Leu Thr

225                 230                 235                 240

Leu Gln Gly Glu Asp Glu Ile Lys Leu Ile Ala Val Met Leu Gly Gln
                245                 250                 255

Phe Arg Thr Phe Thr Gln Val Lys Ile Leu Ala Glu Ser Gly Gln Thr
            260                 265                 270

Glu Ser Gln Ile Ala Ser Ser Leu Gly Ser Tyr Leu Gly Arg Asn Pro
        275                 280                 285

Asn Pro Tyr Gln Ile Lys Phe Ala Leu Arg Asp Ser Arg Gly Leu Ser
    290                 295                 300

Leu Ser Phe Leu Lys Gln Ala Ile Ser Tyr Leu Ile Glu Thr Asp Tyr
305                 310                 315                 320

Gln Ile Lys Thr Gly Leu Tyr Glu Lys Gly Phe Leu Phe Glu Lys Ala
                325                 330                 335

Leu Leu Gln Ile Ala Ser Gln Val Asn
            340                 345

<210> 118
<211> 1035
<212> DNA
<213> Actinobacillus actinomycetemcomitans

<400> 118
atgaatcggc tatttcctga acagcttgct tcgtcactgg aacggcattt agcccatgtg 60

tattttctgg tgggcgagga tccgttgttg ctcaatgaaa gcgccaatgg tatttaccaa 120

acggcgcttc agcggggatt cgatgaaaaa gttgaactgg acattaatgc ctccaccgac 180

tggaatgatt tattcgagcg ggtgcaatcc atgggcttat ttttcaataa acagctcatt 240

attttagatt tacctgaaaa tgcgaccgca cttttacaga aaaacttatc cgaattcatt 300

tcgttgcttc agcccgatgt gctaccgatt ttccgtttag ccaaactcac taaagccgct 360

gaaaagcagg cgtggtttat ggcggcgaat cagtatgaac cgcaggcggt attggtaaac 420

tgtcaaaccc ccaatgccga acaattgccc cgctgggtgg cgaatcgggc gaagatgttg 480

gggttaagca ttgaacaaga agcggtgcag ttgttgtgtt acagctacga aaacaacctg 540

ttggcactca agcaaacact gcaattactg gatttgcttt atcctgatcg taaactcact 600

tttgcccgcg tgaatagcgt ggtggaacag tcctcggtgt ttacgccgtt ccaatgggtt 660

gatgcgattt taggcggaaa agggaatcgc gcccgccgaa ttctcaccgg tttaaaagat 720

gaagatgttc agccgattat tctcctgcgc acgttgcagc gcgatttaat gactttactg 780

gagatcagta aaccggaaca accccaatcc cttgactcgc ctttacccac cgatcaactg 840

cgtgaacagt ttgatcgcct gaaagtatgg caaaaccgcc gttcattgtt tactcaagcg 900

gtgcaacgtt taacctaccg taaactctat cttttttttc agcaactcgc cgatgtggag 960

cgctgtgcga aacaggaatt tagcgatgat atttggcaac aactggaaga tttatcggta 1020

agatttgccg gttag 1035

<210> 119
<211> 1035
<212> DNA
<213> Actinobacillus actinomycetemcomitans

<220>
<221> CDS
<222> (1)..(1035)

<400> 119
atg aat cgg cta ttt cct gaa cag ctt gct tcg tca ctg gaa cgg cat 48
Met Asn Arg Leu Phe Pro Glu Gln Leu Ala Ser Ser Leu Glu Arg His
1               5                   10                  15

tta gcc cat gtg tat ttt ctg gtg ggc gag gat ccg ttg ttg ctc aat 96
Leu Ala His Val Tyr Phe Leu Val Gly Glu Asp Pro Leu Leu Leu Asn
            20                  25                  30

gaa agc gcc aat ggt att tac caa acg gcg ctt cag cgg gga ttc gat 144
Glu Ser Ala Asn Gly Ile Tyr Gln Thr Ala Leu Gln Arg Gly Phe Asp
        35                  40                  45

gaa aaa gtt gaa ctg gac att aat gcc tcc acc gac tgg aat gat tta 192
Glu Lys Val Glu Leu Asp Ile Asn Ala Ser Thr Asp Trp Asn Asp Leu
    50                  55                  60

ttc gag cgg gtg caa tcc atg ggc tta ttt ttc aat aaa cag ctc att 240
Phe Glu Arg Val Gln Ser Met Gly Leu Phe Phe Asn Lys Gln Leu Ile
65                  70                  75                  80

att tta gat tta cct gaa aat gcg acc gca ctt tta cag aaa aac tta 288
Ile Leu Asp Leu Pro Glu Asn Ala Thr Ala Leu Leu Gln Lys Asn Leu
                85                  90                  95

tcc gaa ttc att tcg ttg ctt cag ccc gat gtg cta ccg att ttc cgt 336
Ser Glu Phe Ile Ser Leu Leu Gln Pro Asp Val Leu Pro Ile Phe Arg
            100                 105                 110

tta gcc aaa ctc act aaa gcc gct gaa aag cag gcg tgg ttt atg gcg 384
Leu Ala Lys Leu Thr Lys Ala Ala Glu Lys Gln Ala Trp Phe Met Ala
        115                 120                 125

gcg aat cag tat gaa ccg cag gcg gta ttg gta aac tgt caa acc ccc 432
Ala Asn Gln Tyr Glu Pro Gln Ala Val Leu Val Asn Cys Gln Thr Pro
    130                 135                 140

aat gcc gaa caa ttg ccc cgc tgg gtg gcg aat cgg gcg aag atg ttg 480
Asn Ala Glu Gln Leu Pro Arg Trp Val Ala Asn Arg Ala Lys Met Leu
145                 150                 155                 160

ggg tta agc att gaa caa gaa gcg gtg cag ttg ttg tgt tac agc tac 528
Gly Leu Ser Ile Glu Gln Glu Ala Val Gln Leu Leu Cys Tyr Ser Tyr
                165                 170                 175

gaa aac aac ctg ttg gca ctc aag caa aca ctg caa tta ctg gat ttg 576
Glu Asn Asn Leu Leu Ala Leu Lys Gln Thr Leu Gln Leu Leu Asp Leu
            180                 185                 190

ctt tat cct gat cgt aaa ctc act ttt gcc cgc gtg aat agc gtg gtg 624
Leu Tyr Pro Asp Arg Lys Leu Thr Phe Ala Arg Val Asn Ser Val Val
        195                 200                 205

gaa cag tcc tcg gtg ttt acg ccg ttc caa tgg gtt gat gcg att tta 672
Glu Gln Ser Ser Val Phe Thr Pro Phe Gln Trp Val Asp Ala Ile Leu
    210                 215                 220

ggc gga aaa ggg aat cgc gcc cgc cga att ctc acc ggt tta aaa gat 720
Gly Gly Lys Gly Asn Arg Ala Arg Arg Ile Leu Thr Gly Leu Lys Asp
225                 230                 235                 240

gaa gat gtt cag ccg att att ctc ctg cgc acg ttg cag cgc gat tta 768
Glu Asp Val Gln Pro Ile Ile Leu Leu Arg Thr Leu Gln Arg Asp Leu
                245                 250                 255

atg act tta ctg gag atc agt aaa ccg gaa caa ccc caa tcc ctt gac 816
Met Thr Leu Leu Glu Ile Ser Lys Pro Glu Gln Pro Gln Ser Leu Asp
            260                 265                 270

tcg cct tta ccc acc gat caa ctg cgt gaa cag ttt gat cgc ctg aaa    864
Ser Pro Leu Pro Thr Asp Gln Leu Arg Glu Gln Phe Asp Arg Leu Lys
        275                 280                 285

gta tgg caa aac cgc cgt tca ttg ttt act caa gcg gtg caa cgt tta    912
Val Trp Gln Asn Arg Arg Ser Leu Phe Thr Gln Ala Val Gln Arg Leu
    290                 295                 300

acc tac cgt aaa ctc tat ctt ttt ttt cag caa ctc gcc gat gtg gag    960
Thr Tyr Arg Lys Leu Tyr Leu Phe Phe Gln Gln Leu Ala Asp Val Glu
305                 310                 315                 320

cgc tgt gcg aaa cag gaa ttt agc gat gat att tgg caa caa ctg gaa    1008
Arg Cys Ala Lys Gln Glu Phe Ser Asp Asp Ile Trp Gln Gln Leu Glu
                325                 330                 335

gat tta tcg gta aga ttt gcc ggt tag                                1035
Asp Leu Ser Val Arg Phe Ala Gly
            340                 345

<210> 120
<211> 344
<212> PRT
<213> Actinobacillus actinomycetemcomitans

<400> 120
Met Asn Arg Leu Phe Pro Glu Gln Leu Ala Ser Ser Leu Glu Arg His
1               5                   10                  15

Leu Ala His Val Tyr Phe Leu Val Gly Glu Asp Pro Leu Leu Leu Asn
            20                  25                  30

Glu Ser Ala Asn Gly Ile Tyr Gln Thr Ala Leu Gln Arg Gly Phe Asp
        35                  40                  45

Glu Lys Val Glu Leu Asp Ile Asn Ala Ser Thr Asp Trp Asn Asp Leu
    50                  55                  60

Phe Glu Arg Val Gln Ser Met Gly Leu Phe Phe Asn Lys Gln Leu Ile
65                  70                  75                  80

Ile Leu Asp Leu Pro Glu Asn Ala Thr Ala Leu Leu Gln Lys Asn Leu
                85                  90                  95

Ser Glu Phe Ile Ser Leu Leu Gln Pro Asp Val Leu Pro Ile Phe Arg
            100                 105                 110

Leu Ala Lys Leu Thr Lys Ala Ala Glu Lys Gln Ala Trp Phe Met Ala
        115                 120                 125

Ala Asn Gln Tyr Glu Pro Gln Ala Val Leu Val Asn Cys Gln Thr Pro
    130                 135                 140

Asn Ala Glu Gln Leu Pro Arg Trp Val Ala Asn Arg Ala Lys Met Leu
145                 150                 155                 160

Gly Leu Ser Ile Glu Gln Glu Ala Val Gln Leu Leu Cys Tyr Ser Tyr
                165                 170                 175

Glu Asn Asn Leu Leu Ala Leu Lys Gln Thr Leu Gln Leu Leu Asp Leu
            180                 185                 190

Leu Tyr Pro Asp Arg Lys Leu Thr Phe Ala Arg Val Asn Ser Val Val
        195                 200                 205

Glu Gln Ser Ser Val Phe Thr Pro Phe Gln Trp Val Asp Ala Ile Leu
    210                 215                 220

Gly Gly Lys Gly Asn Arg Ala Arg Arg Ile Leu Thr Gly Leu Lys Asp
225                 230                 235                 240

Glu Asp Val Gln Pro Ile Ile Leu Leu Arg Thr Leu Gln Arg Asp Leu
                245                 250                 255

Met Thr Leu Leu Glu Ile Ser Lys Pro Glu Gln Pro Gln Ser Leu Asp
            260                 265                 270

Ser Pro Leu Pro Thr Asp Gln Leu Arg Glu Gln Phe Asp Arg Leu Lys
        275                 280                 285

Val Trp Gln Asn Arg Arg Ser Leu Phe Thr Gln Ala Val Gln Arg Leu
    290                 295                 300

Thr Tyr Arg Lys Leu Tyr Leu Phe Phe Gln Gln Leu Ala Asp Val Glu
305                 310                 315                 320

Arg Cys Ala Lys Gln Glu Phe Ser Asp Asp Ile Trp Gln Gln Leu Glu
                325                 330                 335

Asp Leu Ser Val Arg Phe Ala Gly
            340

<210> 121

<211> 1056
<212> DNA
<213> Bordetella pertussis

<400> 121
atggctgcgc ccctggacag cgacgccctg gccagccacc tcgcacgcgc ggggggccgg 60

ctggcgccgc tgtacacggt atcgggcgac gagccgctgc tggtcaccga ggccgccgac 120

gccatccgcg cggcggcgcg cgcggccggc tacaccgacc gcacctccat ggtcatggac 180

gcgcgcagcg actggagcgc ggtggcggcc gccacgcaaa gcgtctcgct gttcggcgac 240

cggcgcctgc tggaactgaa aatcccgacc ggcaagcccg gcaagagcgg cggcgagatg 300

ctggcccggc tggccgacca ggcgcgcgac caggccgacg ccgacacgct cgtggtcgtg 360

gccctgcccc gcctggacaa ggccacgcgc gaaagcaaat gggcccaggc gctggcgcgc 420

ggcggcgtca tggccgacat cgccaacgtc gagcgcggcc gcctgccggc atggattggc 480

atgcgcctgg ggcgccagaa ccagcgcgcc gacaccgcca cgctgcaatg gatggccgac 540

aaggtcgagg gcaacctgct ggcggcccac caggaaatcc agaagctggg gctgctttat 600

ccggaaggcc agctggccgc cgaggacgtc gagcgcgccg tgctcaacgt ggcgcgctac 660

gacgtcttcg ggctgcgcga cgccatgctg gccggcgaca cggcgcgcac ggtgcgcatg 720

ctcgacggct tgcgcgccga gggcgaagcc ctgccgctgg tgctgtgggc ggtcggcgag 780

gaaatccgcc tgctggcgcg cgtagcgcag gcgcgccagc aaggccagga cgccggcgca 840

ctgatgcgcc gtctgcgcat tttcggcgcc cacgaacggc ttgcgctgca agcgctgggc 900

cgggtcgcac ccggcgcatg gccggcagcc gtgcagcacg cccatgaagt cgatcgcctg 960

atcaaaggcc tgagcgtacc gggccggctg gccgatccct gggaagaaat gacccggctc 1020

gcgctgcgcg tggccgcggc cggcgcccga ccgtga 1056

<210> 122
<211> 1056
<212> DNA
<213> Bordetella pertussis

<220>

<221> CDS
<222> (1)..(1056)

<400> 122
atg gct gcg ccc ctg gac agc gac gcc ctg gcc agc cac ctc gca cgc 48
Met Ala Ala Pro Leu Asp Ser Asp Ala Leu Ala Ser His Leu Ala Arg
1               5                   10                  15

gcg ggg ggc cgg ctg gcg ccg ctg tac acg gta tcg ggc gac gag ccg 96
Ala Gly Gly Arg Leu Ala Pro Leu Tyr Thr Val Ser Gly Asp Glu Pro
            20                  25                  30

ctg ctg gtc acc gag gcc gcc gac gcc atc cgc gcg gcg gcg cgc gcg 144
Leu Leu Val Thr Glu Ala Ala Asp Ala Ile Arg Ala Ala Ala Arg Ala
        35                  40                  45

gcc ggc tac acc gac cgc acc tcc atg gtc atg gac gcg cgc agc gac 192
Ala Gly Tyr Thr Asp Arg Thr Ser Met Val Met Asp Ala Arg Ser Asp
    50                  55                  60

tgg agc gcg gtg gcg gcc gcc acg caa agc gtc tcg ctg ttc ggc gac 240
Trp Ser Ala Val Ala Ala Ala Thr Gln Ser Val Ser Leu Phe Gly Asp
65                  70                  75                  80

cgg cgc ctg ctg gaa ctg aaa atc ccg acc ggc aag ccc ggc aag agc 288
Arg Arg Leu Leu Glu Leu Lys Ile Pro Thr Gly Lys Pro Gly Lys Ser
                85                  90                  95

ggc ggc gag atg ctg gcc cgg ctg gcc gac cag gcg cgc gac cag gcc 336
Gly Gly Glu Met Leu Ala Arg Leu Ala Asp Gln Ala Arg Asp Gln Ala
            100                 105                 110

gac gcc gac acg ctc gtg gtc gtg gcc ctg ccc cgc ctg gac aag gcc 384
Asp Ala Asp Thr Leu Val Val Val Ala Leu Pro Arg Leu Asp Lys Ala
        115                 120                 125

acg cgc gaa agc aaa tgg gcc cag gcg ctg gcg cgc ggc ggc gtc atg 432
Thr Arg Glu Ser Lys Trp Ala Gln Ala Leu Ala Arg Gly Gly Val Met
    130                 135                 140

gcc gac atc gcc aac gtc gag cgc ggc cgc ctg ccg gca tgg att ggc 480
Ala Asp Ile Ala Asn Val Glu Arg Gly Arg Leu Pro Ala Trp Ile Gly
145                 150                 155                 160

atg cgc ctg ggg cgc cag aac cag cgc gcc gac acc gcc acg ctg caa 528
Met Arg Leu Gly Arg Gln Asn Gln Arg Ala Asp Thr Ala Thr Leu Gln
                165                 170                 175

tgg atg gcc gac aag gtc gag ggc aac ctg ctg gcg gcc cac cag gaa     576
Trp Met Ala Asp Lys Val Glu Gly Asn Leu Leu Ala Ala His Gln Glu
            180                 185                 190

atc cag aag ctg ggg ctg ctt tat ccg gaa ggc cag ctg gcc gcc gag     624
Ile Gln Lys Leu Gly Leu Leu Tyr Pro Glu Gly Gln Leu Ala Ala Glu
        195                 200                 205

gac gtc gag cgc gcc gtg ctc aac gtg gcg cgc tac gac gtc ttc ggg     672
Asp Val Glu Arg Ala Val Leu Asn Val Ala Arg Tyr Asp Val Phe Gly
    210                 215                 220

ctg cgc gac gcc atg ctg gcc ggc gac acg gcg cgc acg gtg cgc atg     720
Leu Arg Asp Ala Met Leu Ala Gly Asp Thr Ala Arg Thr Val Arg Met
225                 230                 235                 240

ctc gac ggc ttg cgc gcc gag ggc gaa gcc ctg ccg ctg gtg ctg tgg     768
Leu Asp Gly Leu Arg Ala Glu Gly Glu Ala Leu Pro Leu Val Leu Trp
                245                 250                 255

gcg gtc ggc gag gaa atc cgc ctg ctg gcg cgc gta gcg cag gcg cgc     816
Ala Val Gly Glu Glu Ile Arg Leu Leu Ala Arg Val Ala Gln Ala Arg
            260                 265                 270

cag caa ggc cag gac gcc ggc gca ctg atg cgc cgt ctg cgc att ttc     864
Gln Gln Gly Gln Asp Ala Gly Ala Leu Met Arg Arg Leu Arg Ile Phe
        275                 280                 285

ggc gcc cac gaa cgg ctt gcg ctg caa gcg ctg ggc cgg gtc gca ccc     912
Gly Ala His Glu Arg Leu Ala Leu Gln Ala Leu Gly Arg Val Ala Pro
    290                 295                 300

ggc gca tgg ccg gca gcc gtg cag cac gcc cat gaa gtc gat cgc ctg     960
Gly Ala Trp Pro Ala Ala Val Gln His Ala His Glu Val Asp Arg Leu
305                 310                 315                 320

atc aaa ggc ctg agc gta ccg ggc cgg ctg gcc gat ccc tgg gaa gaa     1008
Ile Lys Gly Leu Ser Val Pro Gly Arg Leu Ala Asp Pro Trp Glu Glu
                325                 330                 335

atg acc cgg ctc gcg ctg cgc gtg gcc gcg gcc ggc gcc cga ccg tga     1056
Met Thr Arg Leu Ala Leu Arg Val Ala Ala Ala Gly Ala Arg Pro
            340                 345                 350

<210> 123
<211> 351
<212> PRT

<213> Bordetella pertussis

<400> 123
Met Ala Ala Pro Leu Asp Ser Asp Ala Leu Ala Ser His Leu Ala Arg
1               5                   10                  15

Ala Gly Gly Arg Leu Ala Pro Leu Tyr Thr Val Ser Gly Asp Glu Pro
            20                  25                  30

Leu Leu Val Thr Glu Ala Ala Asp Ala Ile Arg Ala Ala Ala Arg Ala
        35                  40                  45

Ala Gly Tyr Thr Asp Arg Thr Ser Met Val Met Asp Ala Arg Ser Asp
    50                  55                  60

Trp Ser Ala Val Ala Ala Ala Thr Gln Ser Val Ser Leu Phe Gly Asp
65                  70                  75                  80

Arg Arg Leu Leu Glu Leu Lys Ile Pro Thr Gly Lys Pro Gly Lys Ser
                85                  90                  95

Gly Gly Glu Met Leu Ala Arg Leu Ala Asp Gln Ala Arg Asp Gln Ala
            100                 105                 110

Asp Ala Asp Thr Leu Val Val Val Ala Leu Pro Arg Leu Asp Lys Ala
        115                 120                 125

Thr Arg Glu Ser Lys Trp Ala Gln Ala Leu Ala Arg Gly Gly Val Met
    130                 135                 140

Ala Asp Ile Ala Asn Val Glu Arg Gly Arg Leu Pro Ala Trp Ile Gly
145                 150                 155                 160

Met Arg Leu Gly Arg Gln Asn Gln Arg Ala Asp Thr Ala Thr Leu Gln
                165                 170                 175

Trp Met Ala Asp Lys Val Glu Gly Asn Leu Leu Ala Ala His Gln Glu
            180                 185                 190

Ile Gln Lys Leu Gly Leu Leu Tyr Pro Glu Gly Gln Leu Ala Ala Glu
        195                 200                 205

Asp Val Glu Arg Ala Val Leu Asn Val Ala Arg Tyr Asp Val Phe Gly
    210                 215                 220

Leu Arg Asp Ala Met Leu Ala Gly Asp Thr Ala Arg Thr Val Arg Met
225                 230                 235                 240

Leu Asp Gly Leu Arg Ala Glu Gly Glu Ala Leu Pro Leu Val Leu Trp
                245                 250                 255

Ala Val Gly Glu Glu Ile Arg Leu Leu Ala Arg Val Ala Gln Ala Arg
            260                 265                 270

Gln Gln Gly Gln Asp Ala Gly Ala Leu Met Arg Arg Leu Arg Ile Phe
        275                 280                 285

Gly Ala His Glu Arg Leu Ala Leu Gln Ala Leu Gly Arg Val Ala Pro
    290                 295                 300

Gly Ala Trp Pro Ala Ala Val Gln His Ala His Glu Val Asp Arg Leu
305                 310                 315                 320

Ile Lys Gly Leu Ser Val Pro Gly Arg Leu Ala Asp Pro Trp Glu Glu
                325                 330                 335

Met Thr Arg Leu Ala Leu Arg Val Ala Ala Ala Gly Ala Arg Pro
            340                 345                 350

<210> 124
<211> 1011
<212> DNA
<213> Bacillus anthracis

<400> 124
atgagtgata tacataaaaa gattaaaaaa aagcagtttg ctccgttgta tttactgtat 60

ggaacggaag cgttttttat aaatgaaacg ataaagctta ttacaacaga agcgcttgaa 120

gaggaagatc gcgagtttaa tgttgtgaca tatgatttag aagaagcgta tttagaagat 180

gtggttgagg atgcacgtac acttcctttt ttcggagaac gtaaagtgtt attaataaaa 240

tcaccattat ttttaacgtc acaaaaagaa aagttagaac aaaatataaa aattttagaa 300

gaatatatcg gggaaccttc tccattttct attcttgttt ttgttgcgcc ttacgaaaag 360

cttgatgaac gaaaaaaaat tacaaaacta ttaaagaaaa cagcagatat agtagaagcg 420

aatgcgatgc aagtgcagga tgttcaaaag tggattgtag ctcgtgcaga ggaagggcat 480

gtacatattg ataatgcagc tgttagttta ttgttagagc ttgtgggaag taatgtgacg 540

atgttggcga aggaaatgga caagttaacg ttatacgtcg gtatgggcgg agagattacg 600

ccgaaacttg ttgcagagct tgtgccaaaa tcagttgaac aaaatgtatt tgctttaaca 660

gaaaaagtgg taaaaaaaga tatcgcaggt gcgatgcaaa ttttggatgg attatttacg 720

cagcaggagg aaccgattaa attgcttgcg ttgttagtaa gtcaattccg cttgctgcat 780

caagtaaaag agttacagca gcgtggttat ggacaaaatc aaattgcttc tcatattggt 840

gtacatccat atcgtgtaaa gttagcgatg aatcaaacga agtttttctc ttttgaagaa 900

ctaaaaaaag tgattataga attggcggaa gctgattata gtatgaagac tggaaaaatg 960

gataagaaac ttgtgcttga gtttttctta atgcgattaa atcatatgtg a 1011

<210> 125
<211> 1011
<212> DNA
<213> Bacillus anthracis

<220>
<221> CDS
<222> (1)..(1011)

<400> 125
atg agt gat ata cat aaa aag att aaa aaa aag cag ttt gct ccg ttg 48
Met Ser Asp Ile His Lys Lys Ile Lys Lys Lys Gln Phe Ala Pro Leu
1               5                   10                  15

tat tta ctg tat gga acg gaa gcg ttt ttt ata aat gaa acg ata aag 96
Tyr Leu Leu Tyr Gly Thr Glu Ala Phe Phe Ile Asn Glu Thr Ile Lys
            20                  25                  30

ctt att aca aca gaa gcg ctt gaa gag gaa gat cgc gag ttt aat gtt 144
Leu Ile Thr Thr Glu Ala Leu Glu Glu Glu Asp Arg Glu Phe Asn Val
        35                  40                  45

gtg aca tat gat tta gaa gaa gcg tat tta gaa gat gtg gtt gag gat 192
Val Thr Tyr Asp Leu Glu Glu Ala Tyr Leu Glu Asp Val Val Glu Asp
    50                  55                  60

gca cgt aca ctt cct ttt ttc gga gaa cgt aaa gtg tta tta ata aaa 240
Ala Arg Thr Leu Pro Phe Phe Gly Glu Arg Lys Val Leu Leu Ile Lys
65                  70                  75                  80

tca cca tta ttt tta acg tca caa aaa gaa aag tta gaa caa aat ata 288
Ser Pro Leu Phe Leu Thr Ser Gln Lys Glu Lys Leu Glu Gln Asn Ile
                85                  90                  95

aaa att tta gaa gaa tat atc ggg gaa cct tct cca ttt tct att ctt 336
Lys Ile Leu Glu Glu Tyr Ile Gly Glu Pro Ser Pro Phe Ser Ile Leu
            100                 105                 110

gtt ttt gtt gcg cct tac gaa aag ctt gat gaa cga aaa aaa att aca 384
Val Phe Val Ala Pro Tyr Glu Lys Leu Asp Glu Arg Lys Lys Ile Thr
        115                 120                 125

aaa cta tta aag aaa aca gca gat ata gta gaa gcg aat gcg atg caa 432
Lys Leu Leu Lys Lys Thr Ala Asp Ile Val Glu Ala Asn Ala Met Gln
    130                 135                 140

gtg cag gat gtt caa aag tgg att gta gct cgt gca gag gaa ggg cat 480
Val Gln Asp Val Gln Lys Trp Ile Val Ala Arg Ala Glu Glu Gly His
145                 150                 155                 160

gta cat att gat aat gca gct gtt agt tta ttg tta gag ctt gtg gga 528
Val His Ile Asp Asn Ala Ala Val Ser Leu Leu Leu Glu Leu Val Gly
                165                 170                 175

agt aat gtg acg atg ttg gcg aag gaa atg gac aag tta acg tta tac 576
Ser Asn Val Thr Met Leu Ala Lys Glu Met Asp Lys Leu Thr Leu Tyr
            180                 185                 190

gtc ggt atg ggc gga gag att acg ccg aaa ctt gtt gca gag ctt gtg 624
Val Gly Met Gly Gly Glu Ile Thr Pro Lys Leu Val Ala Glu Leu Val
        195                 200                 205

cca aaa tca gtt gaa caa aat gta ttt gct tta aca gaa aaa gtg gta 672
Pro Lys Ser Val Glu Gln Asn Val Phe Ala Leu Thr Glu Lys Val Val
    210                 215                 220

aaa aaa gat atc gca ggt gcg atg caa att ttg gat gga tta ttt acg 720
Lys Lys Asp Ile Ala Gly Ala Met Gln Ile Leu Asp Gly Leu Phe Thr
225                 230                 235                 240

cag cag gag gaa ccg att aaa ttg ctt gcg ttg tta gta agt caa ttc 768
Gln Gln Glu Glu Pro Ile Lys Leu Leu Ala Leu Leu Val Ser Gln Phe
                245                 250                 255

cgc ttg ctg cat caa gta aaa gag tta cag cag cgt ggt tat gga caa 816
Arg Leu Leu His Gln Val Lys Glu Leu Gln Gln Arg Gly Tyr Gly Gln
            260                 265                 270

aat caa att gct tct cat att ggt gta cat cca tat cgt gta aag tta 864
Asn Gln Ile Ala Ser His Ile Gly Val His Pro Tyr Arg Val Lys Leu
        275                 280                 285

gcg atg aat caa acg aag ttt ttc tct ttt gaa gaa cta aaa aaa gtg    912
Ala Met Asn Gln Thr Lys Phe Phe Ser Phe Glu Glu Leu Lys Lys Val
    290                 295                 300

att ata gaa ttg gcg gaa gct gat tat agt atg aag act gga aaa atg    960
Ile Ile Glu Leu Ala Glu Ala Asp Tyr Ser Met Lys Thr Gly Lys Met
305                 310                 315                 320

gat aag aaa ctt gtg ctt gag ttt ttc tta atg cga tta aat cat atg    1008
Asp Lys Lys Leu Val Leu Glu Phe Phe Leu Met Arg Leu Asn His Met
                325                 330                 335

tga                                                                1011

<210> 126
<211> 336
<212> PRT
<213> Bacillus anthracis

<400> 126
Met Ser Asp Ile His Lys Lys Ile Lys Lys Lys Gln Phe Ala Pro Leu
1               5                   10                  15

Tyr Leu Leu Tyr Gly Thr Glu Ala Phe Phe Ile Asn Glu Thr Ile Lys
            20                  25                  30

Leu Ile Thr Thr Glu Ala Leu Glu Glu Glu Asp Arg Glu Phe Asn Val
        35                  40                  45

Val Thr Tyr Asp Leu Glu Glu Ala Tyr Leu Glu Asp Val Val Glu Asp
    50                  55                  60

Ala Arg Thr Leu Pro Phe Phe Gly Glu Arg Lys Val Leu Leu Ile Lys
65                  70                  75                  80

Ser Pro Leu Phe Leu Thr Ser Gln Lys Glu Lys Leu Glu Gln Asn Ile
                85                  90                  95

Lys Ile Leu Glu Glu Tyr Ile Gly Glu Pro Ser Pro Phe Ser Ile Leu
            100                 105                 110

Val Phe Val Ala Pro Tyr Glu Lys Leu Asp Glu Arg Lys Lys Ile Thr
        115                 120                 125

Lys Leu Leu Lys Lys Thr Ala Asp Ile Val Glu Ala Asn Ala Met Gln

130                 135                 140

Val Gln Asp Val Gln Lys Trp Ile Val Ala Arg Ala Glu Glu Gly His
145                 150                 155                 160

Val His Ile Asp Asn Ala Ala Val Ser Leu Leu Leu Glu Leu Val Gly
                165                 170                 175

Ser Asn Val Thr Met Leu Ala Lys Glu Met Asp Lys Leu Thr Leu Tyr
            180                 185                 190

Val Gly Met Gly Gly Glu Ile Thr Pro Lys Leu Val Ala Glu Leu Val
        195                 200                 205

Pro Lys Ser Val Glu Gln Asn Val Phe Ala Leu Thr Glu Lys Val Val
    210                 215                 220

Lys Lys Asp Ile Ala Gly Ala Met Gln Ile Leu Asp Gly Leu Phe Thr
225                 230                 235                 240

Gln Gln Glu Glu Pro Ile Lys Leu Leu Ala Leu Leu Val Ser Gln Phe
                245                 250                 255

Arg Leu Leu His Gln Val Lys Glu Leu Gln Gln Arg Gly Tyr Gly Gln
            260                 265                 270

Asn Gln Ile Ala Ser His Ile Gly Val His Pro Tyr Arg Val Lys Leu
        275                 280                 285

Ala Met Asn Gln Thr Lys Phe Phe Ser Phe Glu Glu Leu Lys Lys Val
    290                 295                 300

Ile Ile Glu Leu Ala Glu Ala Asp Tyr Ser Met Lys Thr Gly Lys Met
305                 310                 315                 320

Asp Lys Lys Leu Val Leu Glu Phe Phe Leu Met Arg Leu Asn His Met
                325                 330                 335

<210> 127
<211> 1089
<212> DNA
<213> Burkholderia pseudomallei

<400> 127
atgcaactgc gacttgacgc gctggagccg catctctcca agggcctggc cggactgtac 60

gtcgtctacg gcgacgaacc gctgctcgcg caggaggcgt gcgacaagat ccgcgccgcc 120

gcgcgcgcgg cgggcttcac cgagcgttcg gtgcacaccg tcgagcgcgg cttcgactgg 180

agcacgctga tcggcgcgag ccaggcgatg tcgctgttcg gcgagcggca gctcgtcgag 240

ctgcggattc cgtccggcaa gcccggcaag gacggcgccg acgcgctgaa gacgctcgcc 300

ggcgcggcca acccggacgt gctgatgctc gtcacgctgc cgcggctcga tgcggcgacg 360

cagaagtccg cgtggttcac ggcgctcgcg aacggcggcg tcgcgctgaa gatcgacccc 420

gtcgagcgcg cgcagttgcc gaactggatc ggtcagcggc tcgcgctgca ggggcagcgg 480

gtcgccccgg gcgacgacgg acggcgcgcg ctcgcgttcg tcgccgagcg ggtcgaaggc 540

aatctgctcg ccgcgcatca ggaaatccag aagctcggcc tgctgtatcc ggccggcgcg 600

ctgacgttcg agcagatcca cgacgcggtg ctgaacgtcg cgcgctacga cgtgttcaag 660

ctcaacgagg cgatgctcgc gggcgacgcc gcgcggcttt cgcggatgat cgacggcctg 720

aagggcgagg gcgaggcgct cgtgctcgtg ctgtgggcgg tcgtcgagga gttgcgcacg 780

ctgttgcgga tcaagcgcgg cgtcgcggcc ggcaagccgc ttgccgtgct cgtgcgcgag 840

aaccgcgtgt gggggccgcg cgagcggctc gtcgggcccg cgctttcgcg cgtatcggaa 900

gcggcgctcg agcacgcgct cgcgttcgcc gcgcggctcg accggcaggt caaggggttg 960

tcggccgtgt cgcgcggcgc ggcgcgcggc gagccgccgc ccgatccgtg ggacggcctg 1020

ttccagctcg cgatgaccgt cgcgcgcgcg gccgggccag gcggcgacgc gccgcgccgg 1080

cccgcctga 1089

<210> 128
<211> 1089
<212> DNA
<213> Burkholderia pseudomallei

<220>
<221> CDS
<222> (1)..(1089)

<400> 128
atg caa ctg cga ctt gac gcg ctg gag ccg cat ctc tcc aag ggc ctg 48
Met Gln Leu Arg Leu Asp Ala Leu Glu Pro His Leu Ser Lys Gly Leu

1                 5                   10                  15

gcc gga ctg tac gtc gtc tac ggc gac gaa ccg ctg ctc gcg cag gag     96
Ala Gly Leu Tyr Val Val Tyr Gly Asp Glu Pro Leu Leu Ala Gln Glu
            20                  25                  30

gcg tgc gac aag atc cgc gcc gcc gcg cgc gcg gcg ggc ttc acc gag     144
Ala Cys Asp Lys Ile Arg Ala Ala Ala Arg Ala Ala Gly Phe Thr Glu
        35                  40                  45

cgt tcg gtg cac acc gtc gag cgc ggc ttc gac tgg agc acg ctg atc     192
Arg Ser Val His Thr Val Glu Arg Gly Phe Asp Trp Ser Thr Leu Ile
    50                  55                  60

ggc gcg agc cag gcg atg tcg ctg ttc ggc gag cgg cag ctc gtc gag     240
Gly Ala Ser Gln Ala Met Ser Leu Phe Gly Glu Arg Gln Leu Val Glu
65                  70                  75                  80

ctg cgg att ccg tcc ggc aag ccc ggc aag gac ggc gcc gac gcg ctg     288
Leu Arg Ile Pro Ser Gly Lys Pro Gly Lys Asp Gly Ala Asp Ala Leu
                85                  90                  95

aag acg ctc gcc ggc gcg gcc aac ccg gac gtg ctg atg ctc gtc acg     336
Lys Thr Leu Ala Gly Ala Ala Asn Pro Asp Val Leu Met Leu Val Thr
            100                 105                 110

ctg ccg cgg ctc gat gcg gcg acg cag aag tcc gcg tgg ttc acg gcg     384
Leu Pro Arg Leu Asp Ala Ala Thr Gln Lys Ser Ala Trp Phe Thr Ala
        115                 120                 125

ctc gcg aac ggc ggc gtc gcg ctg aag atc gac ccc gtc gag cgc gcg     432
Leu Ala Asn Gly Gly Val Ala Leu Lys Ile Asp Pro Val Glu Arg Ala
    130                 135                 140

cag ttg ccg aac tgg atc ggt cag cgg ctc gcg ctg cag ggg cag cgg     480
Gln Leu Pro Asn Trp Ile Gly Gln Arg Leu Ala Leu Gln Gly Gln Arg
145                 150                 155                 160

gtc gcc ccg ggc gac gac gga cgg cgc gcg ctc gcg ttc gtc gcc gag     528
Val Ala Pro Gly Asp Asp Gly Arg Arg Ala Leu Ala Phe Val Ala Glu
                165                 170                 175

cgg gtc gaa ggc aat ctg ctc gcc gcg cat cag gaa atc cag aag ctc     576
Arg Val Glu Gly Asn Leu Leu Ala Ala His Gln Glu Ile Gln Lys Leu
            180                 185                 190

ggc ctg ctg tat ccg gcc ggc gcg ctg acg ttc gag cag atc cac gac     624
Gly Leu Leu Tyr Pro Ala Gly Ala Leu Thr Phe Glu Gln Ile His Asp

195                 200                 205

gcg gtg ctg aac gtc gcg cgc tac gac gtg ttc aag ctc aac gag gcg     672
Ala Val Leu Asn Val Ala Arg Tyr Asp Val Phe Lys Leu Asn Glu Ala
    210                 215                 220

atg ctc gcg ggc gac gcc gcg cgg ctt tcg cgg atg atc gac ggc ctg     720
Met Leu Ala Gly Asp Ala Ala Arg Leu Ser Arg Met Ile Asp Gly Leu
225                 230                 235                 240

aag ggc gag ggc gag gcg ctc gtg ctc gtg ctg tgg gcg gtc gtc gag     768
Lys Gly Glu Gly Glu Ala Leu Val Leu Val Leu Trp Ala Val Val Glu
                245                 250                 255

gag ttg cgc acg ctg ttg cgg atc aag cgc ggc gtc gcg gcc ggc aag     816
Glu Leu Arg Thr Leu Leu Arg Ile Lys Arg Gly Val Ala Ala Gly Lys
            260                 265                 270

ccg ctt gcc gtg ctc gtg cgc gag aac cgc gtg tgg ggg ccg cgc gag     864
Pro Leu Ala Val Leu Val Arg Glu Asn Arg Val Trp Gly Pro Arg Glu
        275                 280                 285

cgg ctc gtc ggg ccc gcg ctt tcg cgc gta tcg gaa gcg gcg ctc gag     912
Arg Leu Val Gly Pro Ala Leu Ser Arg Val Ser Glu Ala Ala Leu Glu
    290                 295                 300

cac gcg ctc gcg ttc gcc gcg cgg ctc gac cgg cag gtc aag ggg ttg     960
His Ala Leu Ala Phe Ala Ala Arg Leu Asp Arg Gln Val Lys Gly Leu
305                 310                 315                 320

tcg gcc gtg tcg cgc ggc gcg gcg cgc ggc gag ccg ccg ccc gat ccg     1008
Ser Ala Val Ser Arg Gly Ala Ala Arg Gly Glu Pro Pro Pro Asp Pro
                325                 330                 335

tgg gac ggc ctg ttc cag ctc gcg atg acc gtc gcg cgc gcg gcc ggg     1056
Trp Asp Gly Leu Phe Gln Leu Ala Met Thr Val Ala Arg Ala Ala Gly
            340                 345                 350

cca ggc ggc gac gcg ccg cgc cgg ccc gcc tga                         1089
Pro Gly Gly Asp Ala Pro Arg Arg Pro Ala
        355                 360

<210> 129
<211> 362
<212> PRT
<213> Burkholderia pseudomallei

<400> 129
Met Gln Leu Arg Leu Asp Ala Leu Glu Pro His Leu Ser Lys Gly Leu
1               5                   10                  15

Ala Gly Leu Tyr Val Val Tyr Gly Asp Glu Pro Leu Leu Ala Gln Glu
            20                  25                  30

Ala Cys Asp Lys Ile Arg Ala Ala Ala Arg Ala Ala Gly Phe Thr Glu
        35                  40                  45

Arg Ser Val His Thr Val Glu Arg Gly Phe Asp Trp Ser Thr Leu Ile
    50                  55                  60

Gly Ala Ser Gln Ala Met Ser Leu Phe Gly Glu Arg Gln Leu Val Glu
65                  70                  75                  80

Leu Arg Ile Pro Ser Gly Lys Pro Gly Lys Asp Gly Ala Asp Ala Leu
                85                  90                  95

Lys Thr Leu Ala Gly Ala Ala Asn Pro Asp Val Leu Met Leu Val Thr
            100                 105                 110

Leu Pro Arg Leu Asp Ala Ala Thr Gln Lys Ser Ala Trp Phe Thr Ala
        115                 120                 125

Leu Ala Asn Gly Gly Val Ala Leu Lys Ile Asp Pro Val Glu Arg Ala
    130                 135                 140

Gln Leu Pro Asn Trp Ile Gly Gln Arg Leu Ala Leu Gln Gly Gln Arg
145                 150                 155                 160

Val Ala Pro Gly Asp Asp Gly Arg Arg Ala Leu Ala Phe Val Ala Glu
                165                 170                 175

Arg Val Glu Gly Asn Leu Leu Ala Ala His Gln Glu Ile Gln Lys Leu
            180                 185                 190

Gly Leu Leu Tyr Pro Ala Gly Ala Leu Thr Phe Glu Gln Ile His Asp
        195                 200                 205

Ala Val Leu Asn Val Ala Arg Tyr Asp Val Phe Lys Leu Asn Glu Ala
    210                 215                 220

Met Leu Ala Gly Asp Ala Ala Arg Leu Ser Arg Met Ile Asp Gly Leu
225                 230                 235                 240

Lys Gly Glu Gly Glu Ala Leu Val Leu Val Leu Trp Ala Val Val Glu
                245                 250                 255

Glu Leu Arg Thr Leu Leu Arg Ile Lys Arg Gly Val Ala Ala Gly Lys
            260                 265                 270

Pro Leu Ala Val Leu Val Arg Glu Asn Arg Val Trp Gly Pro Arg Glu
        275                 280                 285

Arg Leu Val Gly Pro Ala Leu Ser Arg Val Ser Glu Ala Ala Leu Glu
    290                 295                 300

His Ala Leu Ala Phe Ala Ala Arg Leu Asp Arg Gln Val Lys Gly Leu
305                 310                 315                 320

Ser Ala Val Ser Arg Gly Ala Ala Arg Gly Glu Pro Pro Pro Asp Pro
                325                 330                 335

Trp Asp Gly Leu Phe Gln Leu Ala Met Thr Val Ala Arg Ala Ala Gly
            340                 345                 350

Pro Gly Gly Asp Ala Pro Arg Arg Pro Ala
        355                 360

<210> 130
<211> 1041
<212> DNA
<213> Chlorobium tepidum

<400> 130
atggctgacc tgaaaaaaca gattgcctca ggcaaaatcg cccctgtcta tttttttcag 60

ggtccggaaa gctggctcaa ggaggagatt gaaacgcaac tgaaggcggc cattttccct 120

tcggaaaacg aggctgccct caacacgctt gtcctgtacg ggccagacct caccctcggc 180

cagatcgttt cagcggcctc agaatacccg atgttcaccg aaaagaagct ggtcgtcgtc 240

cgccagttcg acaagctgaa aaaggtgacc gacaaaacgg aaagccagcg gcaggagaag 300

tcgctcctga gctatctttc cgatccgccg tcatttaccg tgctggtgct cgatgcagac 360

gagatcgaaa aaacggagct tgaaaaagcc ccgtacaaac atctcaaagc gtttcgccat 420

gacttcgggg cgatcaaaaa tgcggagatc ttcgccgctg aaagagcccg ggaagcggga 480

tgggagttcg agccggaagc cctgaaaaca ttctcgggat acatcgagcc atcatcgagg 540

ctgatctgcc aggagatcga gaagctcacg ctctacgcct cgaacaaacg ccccgaacgg 600

cgcatcaccc tcgacgacgt ctgcgactgc gtcggcatct ctcgaaaata taatgtcttc 660

gagcttgaaa aagcactggt cagcaagaac ctccggcagt gtagcggcat cgctttgatg 720

atcatggagc aggaggggca gaaagagggg ctcatgaaca tcgtgcgcta cctgaccacc 780

tttttcctgc ggatatggaa gctccagaca ccgggcgcac agcaactctc ccttcaggaa 840

acagcagcga tgctcggcat gtacggacgc caggagtatt tcgccaaaaa cttcatcggc 900

tacgcccgcc agttcagcgc cgccgaaacc gaaaatgcca ttctcgccct gcgtgacgcc 960

gatgccgccc tcaaaggcat tattccctcg cccgacgaca gattcaccct gctcaaactg 1020

atgcagcaac tcttcaattg a 1041

<210> 131
<211> 1041
<212> DNA
<213> Chlorobium tepidum

<220>
<221> CDS
<222> (1)..(1041)

<400> 131
atg gct gac ctg aaa aaa cag att gcc tca ggc aaa atc gcc cct gtc 48
Met Ala Asp Leu Lys Lys Gln Ile Ala Ser Gly Lys Ile Ala Pro Val
1               5                   10                  15

tat ttt ttt cag ggt ccg gaa agc tgg ctc aag gag gag att gaa acg 96
Tyr Phe Phe Gln Gly Pro Glu Ser Trp Leu Lys Glu Glu Ile Glu Thr
            20                  25                  30

caa ctg aag gcg gcc att ttc cct tcg gaa aac gag gct gcc ctc aac 144
Gln Leu Lys Ala Ala Ile Phe Pro Ser Glu Asn Glu Ala Ala Leu Asn
        35                  40                  45

acg ctt gtc ctg tac ggg cca gac ctc acc ctc ggc cag atc gtt tca 192
Thr Leu Val Leu Tyr Gly Pro Asp Leu Thr Leu Gly Gln Ile Val Ser
    50                  55                  60

gcg gcc tca gaa tac ccg atg ttc acc gaa aag aag ctg gtc gtc gtc 240
Ala Ala Ser Glu Tyr Pro Met Phe Thr Glu Lys Lys Leu Val Val Val
65                  70                  75                  80

cgc cag ttc gac aag ctg aaa aag gtg acc gac aaa acg gaa agc cag 288
Arg Gln Phe Asp Lys Leu Lys Lys Val Thr Asp Lys Thr Glu Ser Gln
                85                  90                  95

cgg cag gag aag tcg ctc ctg agc tat ctt tcc gat ccg ccg tca ttt 336
Arg Gln Glu Lys Ser Leu Leu Ser Tyr Leu Ser Asp Pro Pro Ser Phe
            100                 105                 110

acc gtg ctg gtg ctc gat gca gac gag atc gaa aaa acg gag ctt gaa 384
Thr Val Leu Val Leu Asp Ala Asp Glu Ile Glu Lys Thr Glu Leu Glu
        115                 120                 125

aaa gcc ccg tac aaa cat ctc aaa gcg ttt cgc cat gac ttc ggg gcg 432
Lys Ala Pro Tyr Lys His Leu Lys Ala Phe Arg His Asp Phe Gly Ala
    130                 135                 140

atc aaa aat gcg gag atc ttc gcc gct gaa aga gcc cgg gaa gcg gga 480
Ile Lys Asn Ala Glu Ile Phe Ala Ala Glu Arg Ala Arg Glu Ala Gly
145                 150                 155                 160

tgg gag ttc gag ccg gaa gcc ctg aaa aca ttc tcg gga tac atc gag 528
Trp Glu Phe Glu Pro Glu Ala Leu Lys Thr Phe Ser Gly Tyr Ile Glu
                165                 170                 175

cca tca tcg agg ctg atc tgc cag gag atc gag aag ctc acg ctc tac 576
Pro Ser Ser Arg Leu Ile Cys Gln Glu Ile Glu Lys Leu Thr Leu Tyr
            180                 185                 190

gcc tcg aac aaa cgc ccc gaa cgg cgc atc acc ctc gac gac gtc tgc 624
Ala Ser Asn Lys Arg Pro Glu Arg Arg Ile Thr Leu Asp Asp Val Cys
        195                 200                 205

gac tgc gtc ggc atc tct cga aaa tat aat gtc ttc gag ctt gaa aaa 672
Asp Cys Val Gly Ile Ser Arg Lys Tyr Asn Val Phe Glu Leu Glu Lys
    210                 215                 220

gca ctg gtc agc aag aac ctc cgg cag tgt agc ggc atc gct ttg atg 720
Ala Leu Val Ser Lys Asn Leu Arg Gln Cys Ser Gly Ile Ala Leu Met
225                 230                 235                 240

atc atg gag cag gag ggg cag aaa gag ggg ctc atg aac atc gtg cgc 768
Ile Met Glu Gln Glu Gly Gln Lys Glu Gly Leu Met Asn Ile Val Arg
                245                 250                 255

tac ctg acc acc ttt ttc ctg cgg ata tgg aag ctc cag aca ccg ggc 816
Tyr Leu Thr Thr Phe Phe Leu Arg Ile Trp Lys Leu Gln Thr Pro Gly
            260                 265                 270

gca cag caa ctc tcc ctt cag gaa aca gca gcg atg ctc ggc atg tac     864
Ala Gln Gln Leu Ser Leu Gln Glu Thr Ala Ala Met Leu Gly Met Tyr
        275                 280                 285

gga cgc cag gag tat ttc gcc aaa aac ttc atc ggc tac gcc cgc cag     912
Gly Arg Gln Glu Tyr Phe Ala Lys Asn Phe Ile Gly Tyr Ala Arg Gln
    290                 295                 300

ttc agc gcc gcc gaa acc gaa aat gcc att ctc gcc ctg cgt gac gcc     960
Phe Ser Ala Ala Glu Thr Glu Asn Ala Ile Leu Ala Leu Arg Asp Ala
305                 310                 315                 320

gat gcc gcc ctc aaa ggc att att ccc tcg ccc gac gac aga ttc acc     1008
Asp Ala Ala Leu Lys Gly Ile Ile Pro Ser Pro Asp Asp Arg Phe Thr
                325                 330                 335

ctg ctc aaa ctg atg cag caa ctc ttc aat tga                         1041
Leu Leu Lys Leu Met Gln Gln Leu Phe Asn
            340                 345

<210> 132
<211> 346
<212> PRT
<213> Chlorobium tepidum

<400> 132
Met Ala Asp Leu Lys Lys Gln Ile Ala Ser Gly Lys Ile Ala Pro Val
1               5                   10                  15

Tyr Phe Phe Gln Gly Pro Glu Ser Trp Leu Lys Glu Glu Ile Glu Thr
            20                  25                  30

Gln Leu Lys Ala Ala Ile Phe Pro Ser Glu Asn Glu Ala Ala Leu Asn
        35                  40                  45

Thr Leu Val Leu Tyr Gly Pro Asp Leu Thr Leu Gly Gln Ile Val Ser
    50                  55                  60

Ala Ala Ser Glu Tyr Pro Met Phe Thr Glu Lys Lys Leu Val Val Val
65                  70                  75                  80

Arg Gln Phe Asp Lys Leu Lys Lys Val Thr Asp Lys Thr Glu Ser Gln
                85                  90                  95

Arg Gln Glu Lys Ser Leu Leu Ser Tyr Leu Ser Asp Pro Pro Ser Phe
            100                 105                 110

Thr Val Leu Val Leu Asp Ala Asp Glu Ile Glu Lys Thr Glu Leu Glu
        115                 120                 125

Lys Ala Pro Tyr Lys His Leu Lys Ala Phe Arg His Asp Phe Gly Ala
    130                 135                 140

Ile Lys Asn Ala Glu Ile Phe Ala Ala Glu Arg Ala Arg Glu Ala Gly
145                 150                 155                 160

Trp Glu Phe Glu Pro Glu Ala Leu Lys Thr Phe Ser Gly Tyr Ile Glu
                165                 170                 175

Pro Ser Ser Arg Leu Ile Cys Gln Glu Ile Glu Lys Leu Thr Leu Tyr
            180                 185                 190

Ala Ser Asn Lys Arg Pro Glu Arg Arg Ile Thr Leu Asp Asp Val Cys
        195                 200                 205

Asp Cys Val Gly Ile Ser Arg Lys Tyr Asn Val Phe Glu Leu Glu Lys
    210                 215                 220

Ala Leu Val Ser Lys Asn Leu Arg Gln Cys Ser Gly Ile Ala Leu Met
225                 230                 235                 240

Ile Met Glu Gln Glu Gly Gln Lys Glu Gly Leu Met Asn Ile Val Arg
                245                 250                 255

Tyr Leu Thr Thr Phe Phe Leu Arg Ile Trp Lys Leu Gln Thr Pro Gly
            260                 265                 270

Ala Gln Gln Leu Ser Leu Gln Glu Thr Ala Ala Met Leu Gly Met Tyr
        275                 280                 285

Gly Arg Gln Glu Tyr Phe Ala Lys Asn Phe Ile Gly Tyr Ala Arg Gln
    290                 295                 300

Phe Ser Ala Ala Glu Thr Glu Asn Ala Ile Leu Ala Leu Arg Asp Ala
305                 310                 315                 320

Asp Ala Ala Leu Lys Gly Ile Ile Pro Ser Pro Asp Asp Arg Phe Thr
                325                 330                 335

Leu Leu Lys Leu Met Gln Gln Leu Phe Asn
            340                 345

<210> 133
<211> 840

<212> DNA
<213> Chloroflexus aurantiacus

<400> 133
caactggttg cggcatgtga ggcacacccg tttcttgccg agcggcgact ggtgatcgtc 60

tacgatgcgt tgaagcatag taaggcaggg aaggggcgcg aagaattacg tagttattgc 120

gagcgtgtgc cggcaacctg tgatctggtg tttgtggagc aagaagaggt tgatcgccga 180

catctgcttt tcacatacct cagcaagcat ggtgtcgttc gtgaattccc gttgctccag 240

ggagcagagt tactccgctg gattgaacag cgggcgaaga tgctgcatgc gacgattgaa 300

tctgccgccg cccaacgtct ggtcgaactg gcagggaatg atagccggtt actggctaac 360

gagctggcca aactggccag ttatgtgggt cgcaatggga agatcgatct gcgggctgta 420

gatcgcctgg ttaccgatca acaagagcaa aatctgtttg cctttctcga tgaattaagt 480

tcgcgacggt tagggccggc attacgcggc gcacgagcac tggttgaaga tggccaggca 540

ccgccctacg tgctctttat gctggctcgt cagatcagaa ttctcttgca ggtcaggcaa 600

ctgctcagtc aacggcgtcg cgctgatgag attgctgccg agctgaacct gaaaccattc 660

gtggcccgaa aggcaactgc acaggcgcgt aacttcagcc ttcccgaact gatacacgcc 720

catgatcgtc tgctcgaact tgatcacgcg atcaagaccg gacgtatcca ggccgaaaca 780

gcacttgaac tatttgttgt ggaagtgtgt cagatgccgc aagaggtggg gaagcgttag 840


<210> 134
<211> 840
<212> DNA
<213> Chloroflexus aurantiacus

<220>
<221> CDS
<222> (1)..(840)

<400> 134
caa ctg gtt gcg gca tgt gag gca cac ccg ttt ctt gcc gag cgg cga 48
Gln Leu Val Ala Ala Cys Glu Ala His Pro Phe Leu Ala Glu Arg Arg
1               5                   10                  15

ctg gtg atc gtc tac gat gcg ttg aag cat agt aag gca ggg aag ggg 96

Leu Val Ile Val Tyr Asp Ala Leu Lys His Ser Lys Ala Gly Lys Gly
            20                  25                  30

cgc gaa gaa tta cgt agt tat tgc gag cgt gtg ccg gca acc tgt gat 144
Arg Glu Glu Leu Arg Ser Tyr Cys Glu Arg Val Pro Ala Thr Cys Asp
        35                  40                  45

ctg gtg ttt gtg gag caa gaa gag gtt gat cgc cga cat ctg ctt ttc 192
Leu Val Phe Val Glu Gln Glu Glu Val Asp Arg Arg His Leu Leu Phe
    50                  55                  60

aca tac ctc agc aag cat ggt gtc gtt cgt gaa ttc ccg ttg ctc cag 240
Thr Tyr Leu Ser Lys His Gly Val Val Arg Glu Phe Pro Leu Leu Gln
65                  70                  75                  80

gga gca gag tta ctc cgc tgg att gaa cag cgg gcg aag atg ctg cat 288
Gly Ala Glu Leu Leu Arg Trp Ile Glu Gln Arg Ala Lys Met Leu His
                85                  90                  95

gcg acg att gaa tct gcc gcc gcc caa cgt ctg gtc gaa ctg gca ggg 336
Ala Thr Ile Glu Ser Ala Ala Ala Gln Arg Leu Val Glu Leu Ala Gly
            100                 105                 110

aat gat agc cgg tta ctg gct aac gag ctg gcc aaa ctg gcc agt tat 384
Asn Asp Ser Arg Leu Leu Ala Asn Glu Leu Ala Lys Leu Ala Ser Tyr
        115                 120                 125

gtg ggt cgc aat ggg aag atc gat ctg cgg gct gta gat cgc ctg gtt 432
Val Gly Arg Asn Gly Lys Ile Asp Leu Arg Ala Val Asp Arg Leu Val
    130                 135                 140

acc gat caa caa gag caa aat ctg ttt gcc ttt ctc gat gaa tta agt 480
Thr Asp Gln Gln Glu Gln Asn Leu Phe Ala Phe Leu Asp Glu Leu Ser
145                 150                 155                 160

tcg cga cgg tta ggg ccg gca tta cgc ggc gca cga gca ctg gtt gaa 528
Ser Arg Arg Leu Gly Pro Ala Leu Arg Gly Ala Arg Ala Leu Val Glu
                165                 170                 175

gat ggc cag gca ccg ccc tac gtg ctc ttt atg ctg gct cgt cag atc 576
Asp Gly Gln Ala Pro Pro Tyr Val Leu Phe Met Leu Ala Arg Gln Ile
            180                 185                 190

aga att ctc ttg cag gtc agg caa ctg ctc agt caa cgg cgt cgc gct 624
Arg Ile Leu Leu Gln Val Arg Gln Leu Leu Ser Gln Arg Arg Arg Ala
        195                 200                 205

gat gag att gct gcc gag ctg aac ctg aaa cca ttc gtg gcc cga aag 672

Asp Glu Ile Ala Ala Glu Leu Asn Leu Lys Pro Phe Val Ala Arg Lys
    210                 215                 220

gca act gca cag gcg cgt aac ttc agc ctt ccc gaa ctg ata cac gcc     720
Ala Thr Ala Gln Ala Arg Asn Phe Ser Leu Pro Glu Leu Ile His Ala
225                 230                 235                 240

cat gat cgt ctg ctc gaa ctt gat cac gcg atc aag acc gga cgt atc     768
His Asp Arg Leu Leu Glu Leu Asp His Ala Ile Lys Thr Gly Arg Ile
                245                 250                 255

cag gcc gaa aca gca ctt gaa cta ttt gtt gtg gaa gtg tgt cag atg     816
Gln Ala Glu Thr Ala Leu Glu Leu Phe Val Val Glu Val Cys Gln Met
            260                 265                 270

ccg caa gag gtg ggg aag cgt tag                                     840
Pro Gln Glu Val Gly Lys Arg
        275                 280

<210> 135
<211> 279
<212> PRT
<213> Chloroflexus aurantiacus

<400> 135
Gln Leu Val Ala Ala Cys Glu Ala His Pro Phe Leu Ala Glu Arg Arg
  1               5                  10                  15

Leu Val Ile Val Tyr Asp Ala Leu Lys His Ser Lys Ala Gly Lys Gly
             20                  25                  30

Arg Glu Glu Leu Arg Ser Tyr Cys Glu Arg Val Pro Ala Thr Cys Asp
         35                  40                  45

Leu Val Phe Val Glu Gln Glu Glu Val Asp Arg Arg His Leu Leu Phe
     50                  55                  60

Thr Tyr Leu Ser Lys His Gly Val Val Arg Glu Phe Pro Leu Leu Gln
 65                  70                  75                  80

Gly Ala Glu Leu Leu Arg Trp Ile Glu Gln Arg Ala Lys Met Leu His
                 85                  90                  95

Ala Thr Ile Glu Ser Ala Ala Ala Gln Arg Leu Val Glu Leu Ala Gly
            100                 105                 110

Asn Asp Ser Arg Leu Leu Ala Asn Glu Leu Ala Lys Leu Ala Ser Tyr

115                 120                 125

Val Gly Arg Asn Gly Lys Ile Asp Leu Arg Ala Val Asp Arg Leu Val
    130                 135                 140

Thr Asp Gln Gln Glu Gln Asn Leu Phe Ala Phe Leu Asp Glu Leu Ser
145                 150                 155                 160

Ser Arg Arg Leu Gly Pro Ala Leu Arg Gly Ala Arg Ala Leu Val Glu
                165                 170                 175

Asp Gly Gln Ala Pro Pro Tyr Val Leu Phe Met Leu Ala Arg Gln Ile
            180                 185                 190

Arg Ile Leu Leu Gln Val Arg Gln Leu Leu Ser Gln Arg Arg Arg Ala
        195                 200                 205

Asp Glu Ile Ala Ala Glu Leu Asn Leu Lys Pro Phe Val Ala Arg Lys
    210                 215                 220

Ala Thr Ala Gln Ala Arg Asn Phe Ser Leu Pro Glu Leu Ile His Ala
225                 230                 235                 240

His Asp Arg Leu Leu Glu Leu Asp His Ala Ile Lys Thr Gly Arg Ile
                245                 250                 255

Gln Ala Glu Thr Ala Leu Glu Leu Phe Val Val Glu Val Cys Gln Met
            260                 265                 270

Pro Gln Glu Val Gly Lys Arg
        275

<210> 136
<211> 1044
<212> DNA
<213> Clostridium difficile

<400> 136
atgaatcaca aggatattat taggaatgta aaagagaaaa attttgaaaa agtgtatctt 60

ttttatggaa gagagtatta tttaattgag aatgcaataa aagcatttaa agatagtcta 120

aacgagggaa tgcttgattt taatttagat ataatagatg ggaaggaaat agtcttaaat 180

cagcttataa gctctataga aacacttcca tttatggatg ataggaagat agtaataata 240

aaagactttg aactattaaa aggaaagaaa aagaatttta cagatagtga tgagaaatac 300

ttaatagagc atttagacaa tattccagac accacaacta tagtttttgt tgtatatgga 360

gatgtagaca aaagaaaatc attagttaag aaaataggaa ataatggaat tgtttttgat 420

tgtgataagc tttctgatat ggatttgttt aaatggataa aaaaatcttt tgctttaaat 480

gatgttataa tagataattc tcaaatcatg tattttattg aacaagaagg atacagagat 540

aagagtagtg aaaagacatt atctgattta gaaaatgaaa taaataaaat aagttcattt 600

gttggtaaag ggaataatgt aaccaatgat gtaattgata aattatctca aaaaaaagtt 660

gagaatgata tattcaaatt aatcgattat attggagaac aaaatgcttc taatgcaatg 720

aaaatattaa atgatatgat acaagaaggc gaatctgtat taggtatatt ttctatgata 780

gcaagacaat ttaaaataat tatgcaagta agacagttac aacttgatgg atattcaaca 840

aaacttatag ctgataaatt aaaaatgcat caatttgttg taggaaaagc attaaagcaa 900

actaaaaatt tttctgatga tataattgtg gaaatattaa actatatatt agaaagtgat 960

tataaaataa agacaggtct tataagagat actttagcag ttgaaatgtt ggttagtaga 1020

tactgtaaaa gagaagcaat ttaa 1044

<210> 137
<211> 1044
<212> DNA
<213> Clostridium difficile

<220>
<221> CDS
<222> (1)..(1044)

<400> 137
atg aat cac aag gat att att agg aat gta aaa gag aaa aat ttt gaa 48
Met Asn His Lys Asp Ile Ile Arg Asn Val Lys Glu Lys Asn Phe Glu
1               5                   10                  15

aaa gtg tat ctt ttt tat gga aga gag tat tat tta att gag aat gca 96
Lys Val Tyr Leu Phe Tyr Gly Arg Glu Tyr Tyr Leu Ile Glu Asn Ala
            20                  25                  30

ata aaa gca ttt aaa gat agt cta aac gag gga atg ctt gat ttt aat 144
Ile Lys Ala Phe Lys Asp Ser Leu Asn Glu Gly Met Leu Asp Phe Asn

35                  40                  45

tta gat ata ata gat ggg aag gaa ata gtc tta aat cag ctt ata agc    192
Leu Asp Ile Ile Asp Gly Lys Glu Ile Val Leu Asn Gln Leu Ile Ser
    50                  55                  60

tct ata gaa aca ctt cca ttt atg gat gat agg aag ata gta ata ata    240
Ser Ile Glu Thr Leu Pro Phe Met Asp Asp Arg Lys Ile Val Ile Ile
65                  70                  75                  80

aaa gac ttt gaa cta tta aaa gga aag aaa aag aat ttt aca gat agt    288
Lys Asp Phe Glu Leu Leu Lys Gly Lys Lys Lys Asn Phe Thr Asp Ser
                85                  90                  95

gat gag aaa tac tta ata gag cat tta gac aat att cca gac acc aca    336
Asp Glu Lys Tyr Leu Ile Glu His Leu Asp Asn Ile Pro Asp Thr Thr
            100                 105                 110

act ata gtt ttt gtt gta tat gga gat gta gac aaa aga aaa tca tta    384
Thr Ile Val Phe Val Val Tyr Gly Asp Val Asp Lys Arg Lys Ser Leu
        115                 120                 125

gtt aag aaa ata gga aat aat gga att gtt ttt gat tgt gat aag ctt    432
Val Lys Lys Ile Gly Asn Asn Gly Ile Val Phe Asp Cys Asp Lys Leu
    130                 135                 140

tct gat atg gat ttg ttt aaa tgg ata aaa aaa tct ttt gct tta aat    480
Ser Asp Met Asp Leu Phe Lys Trp Ile Lys Lys Ser Phe Ala Leu Asn
145                 150                 155                 160

gat gtt ata ata gat aat tct caa atc atg tat ttt att gaa caa gaa    528
Asp Val Ile Ile Asp Asn Ser Gln Ile Met Tyr Phe Ile Glu Gln Glu
                165                 170                 175

gga tac aga gat aag agt agt gaa aag aca tta tct gat tta gaa aat    576
Gly Tyr Arg Asp Lys Ser Ser Glu Lys Thr Leu Ser Asp Leu Glu Asn
            180                 185                 190

gaa ata aat aaa ata agt tca ttt gtt ggt aaa ggg aat aat gta acc    624
Glu Ile Asn Lys Ile Ser Ser Phe Val Gly Lys Gly Asn Asn Val Thr
        195                 200                 205

aat gat gta att gat aaa tta tct caa aaa aaa gtt gag aat gat ata    672
Asn Asp Val Ile Asp Lys Leu Ser Gln Lys Lys Val Glu Asn Asp Ile
    210                 215                 220

ttc aaa tta atc gat tat att gga gaa caa aat gct tct aat gca atg    720
Phe Lys Leu Ile Asp Tyr Ile Gly Glu Gln Asn Ala Ser Asn Ala Met

225                 230                 235                 240

aaa ata tta aat gat atg ata caa gaa ggc gaa tct gta tta ggt ata     768
Lys Ile Leu Asn Asp Met Ile Gln Glu Gly Glu Ser Val Leu Gly Ile
                245                 250                 255

ttt tct atg ata gca aga caa ttt aaa ata att atg caa gta aga cag     816
Phe Ser Met Ile Ala Arg Gln Phe Lys Ile Ile Met Gln Val Arg Gln
            260                 265                 270

tta caa ctt gat gga tat tca aca aaa ctt ata gct gat aaa tta aaa     864
Leu Gln Leu Asp Gly Tyr Ser Thr Lys Leu Ile Ala Asp Lys Leu Lys
        275                 280                 285

atg cat caa ttt gtt gta gga aaa gca tta aag caa act aaa aat ttt     912
Met His Gln Phe Val Val Gly Lys Ala Leu Lys Gln Thr Lys Asn Phe
    290                 295                 300

tct gat gat ata att gtg gaa ata tta aac tat ata tta gaa agt gat     960
Ser Asp Asp Ile Ile Val Glu Ile Leu Asn Tyr Ile Leu Glu Ser Asp
305                 310                 315                 320

tat aaa ata aag aca ggt ctt ata aga gat act tta gca gtt gaa atg     1008
Tyr Lys Ile Lys Thr Gly Leu Ile Arg Asp Thr Leu Ala Val Glu Met
                325                 330                 335

ttg gtt agt aga tac tgt aaa aga gaa gca att taa                     1044
Leu Val Ser Arg Tyr Cys Lys Arg Glu Ala Ile
            340                 345

<210> 138
<211> 347
<212> PRT
<213> Clostridium difficile

<400> 138
Met Asn His Lys Asp Ile Ile Arg Asn Val Lys Glu Lys Asn Phe Glu
1               5                   10                  15

Lys Val Tyr Leu Phe Tyr Gly Arg Glu Tyr Tyr Leu Ile Glu Asn Ala
            20                  25                  30

Ile Lys Ala Phe Lys Asp Ser Leu Asn Glu Gly Met Leu Asp Phe Asn
        35                  40                  45

Leu Asp Ile Ile Asp Gly Lys Glu Ile Val Leu Asn Gln Leu Ile Ser
    50                  55                  60

Ser Ile Glu Thr Leu Pro Phe Met Asp Asp Arg Lys Ile Val Ile Ile
65                  70                  75                  80

Lys Asp Phe Glu Leu Leu Lys Gly Lys Lys Lys Asn Phe Thr Asp Ser
                85                  90                  95

Asp Glu Lys Tyr Leu Ile Glu His Leu Asp Asn Ile Pro Asp Thr Thr
            100                 105                 110

Thr Ile Val Phe Val Val Tyr Gly Asp Val Asp Lys Arg Lys Ser Leu
        115                 120                 125

Val Lys Lys Ile Gly Asn Asn Gly Ile Val Phe Asp Cys Asp Lys Leu
    130                 135                 140

Ser Asp Met Asp Leu Phe Lys Trp Ile Lys Lys Ser Phe Ala Leu Asn
145                 150                 155                 160

Asp Val Ile Ile Asp Asn Ser Gln Ile Met Tyr Phe Ile Glu Gln Glu
                165                 170                 175

Gly Tyr Arg Asp Lys Ser Ser Glu Lys Thr Leu Ser Asp Leu Glu Asn
            180                 185                 190

Glu Ile Asn Lys Ile Ser Ser Phe Val Gly Lys Gly Asn Asn Val Thr
        195                 200                 205

Asn Asp Val Ile Asp Lys Leu Ser Gln Lys Lys Val Glu Asn Asp Ile
    210                 215                 220

Phe Lys Leu Ile Asp Tyr Ile Gly Glu Gln Asn Ala Ser Asn Ala Met
225                 230                 235                 240

Lys Ile Leu Asn Asp Met Ile Gln Glu Gly Glu Ser Val Leu Gly Ile
                245                 250                 255

Phe Ser Met Ile Ala Arg Gln Phe Lys Ile Ile Met Gln Val Arg Gln
            260                 265                 270

Leu Gln Leu Asp Gly Tyr Ser Thr Lys Leu Ile Ala Asp Lys Leu Lys
        275                 280                 285

Met His Gln Phe Val Val Gly Lys Ala Leu Lys Gln Thr Lys Asn Phe
    290                 295                 300

Ser Asp Asp Ile Ile Val Glu Ile Leu Asn Tyr Ile Leu Glu Ser Asp
305                 310                 315                 320

Tyr Lys Ile Lys Thr Gly Leu Ile Arg Asp Thr Leu Ala Val Glu Met
                325                 330                 335

Leu Val Ser Arg Tyr Cys Lys Arg Glu Ala Ile
            340                 345

<210> 139
<211> 975
<212> DNA
<213> Corynebacterium diphtheriae

<400> 139
atgggtcagc aacttctacc agtgcacctc gtagttggcg acgatgaatt cctagccgaa 60

cgcgcacgac acagcatcat cgcagccatc aacaaaacca ttggcagcga cgtccccgtg 120

accacccttc gggcaggcga agtcaacgcc tccgaactca tccaactcac cagcccgtcg 180

ctcttcggcg aagaccgcat cattgtgctg accaacatgg aagatgctgg caaagaaccc 240

gcggaactcg tcctcaacgt tgccgtcgac ccagcaccag gaatctacct catcatcgtg 300

cactccggcg gcggacggca aaaagcacta gtaccaaaac tcagcaaaat cagccaagtc 360

cacgaagcca acaaactcaa acctaaagac cgcgtgggct gggtcaccaa cgaattcaaa 420

cgccacggag tacgccccac cccagacgtg gtccacgccc tgctcgaagg cgtaggatcc 480

gacctccgcg aactcgcctc cgccatcggc cagcttgttg ctgacaacaa tggcgacgtc 540

accctcgcca ccgtccgcga ctactacgtg ggagtagcag aagtctccgg tttcgacgtg 600

gcagatcttg cctgctcagg acaaacccaa cgtgccgtcg ccagcgcgcg ccgggcactg 660

caactaggag tctcaccggt ggcacttgcc gccgcattga gcatgaaagt tagcgctatc 720

gcgcggttgt attccacacg agggcgtatt gactcccgta aactcgcagg agagctgggc 780

atgcacccct tcgttgttga aaaaaccgcc aaagttgcgc gcatgtggac gggtaatgct 840

gtgagccaag cggtgatctt gatggcggat gtggattcca gtcttaaggg ccacggcctc 900

gatggcgaac tcggcggtga ccccgactac gaaatcgagg atgctatccg caggatctct 960

gagctcgcag gctaa                                                  975

<210> 140
<211> 975
<212> DNA
<213> Corynebacterium diphtheriae

<220>
<221> CDS
<222> (1)..(975)

<400> 140
atg ggt cag caa ctt cta cca gtg cac ctc gta gtt ggc gac gat gaa     48
Met Gly Gln Gln Leu Leu Pro Val His Leu Val Val Gly Asp Asp Glu
1               5                   10                  15

ttc cta gcc gaa cgc gca cga cac agc atc atc gca gcc atc aac aaa     96
Phe Leu Ala Glu Arg Ala Arg His Ser Ile Ile Ala Ala Ile Asn Lys
            20                  25                  30

acc att ggc agc gac gtc ccc gtg acc acc ctt cgg gca ggc gaa gtc     144
Thr Ile Gly Ser Asp Val Pro Val Thr Thr Leu Arg Ala Gly Glu Val
        35                  40                  45

aac gcc tcc gaa ctc atc caa ctc acc agc ccg tcg ctc ttc ggc gaa     192
Asn Ala Ser Glu Leu Ile Gln Leu Thr Ser Pro Ser Leu Phe Gly Glu
    50                  55                  60

gac cgc atc att gtg ctg acc aac atg gaa gat gct ggc aaa gaa ccc     240
Asp Arg Ile Ile Val Leu Thr Asn Met Glu Asp Ala Gly Lys Glu Pro
65                  70                  75                  80

gcg gaa ctc gtc ctc aac gtt gcc gtc gac cca gca cca gga atc tac     288
Ala Glu Leu Val Leu Asn Val Ala Val Asp Pro Ala Pro Gly Ile Tyr
                85                  90                  95

ctc atc atc gtg cac tcc ggc ggc gga cgg caa aaa gca cta gta cca     336
Leu Ile Ile Val His Ser Gly Gly Gly Arg Gln Lys Ala Leu Val Pro
            100                 105                 110

aaa ctc agc aaa atc agc caa gtc cac gaa gcc aac aaa ctc aaa cct     384
Lys Leu Ser Lys Ile Ser Gln Val His Glu Ala Asn Lys Leu Lys Pro
        115                 120                 125

aaa gac cgc gtg ggc tgg gtc acc aac gaa ttc aaa cgc cac gga gta     432
Lys Asp Arg Val Gly Trp Val Thr Asn Glu Phe Lys Arg His Gly Val
    130                 135                 140

cgc ccc acc cca gac gtg gtc cac gcc ctg ctc gaa ggc gta gga tcc     480

Arg Pro Thr Pro Asp Val Val His Ala Leu Leu Glu Gly Val Gly Ser
145                 150                 155                 160

gac ctc cgc gaa ctc gcc tcc gcc atc ggc cag ctt gtt gct gac aac     528
Asp Leu Arg Glu Leu Ala Ser Ala Ile Gly Gln Leu Val Ala Asp Asn
                165                 170                 175

aat ggc gac gtc acc ctc gcc acc gtc cgc gac tac tac gtg gga gta     576
Asn Gly Asp Val Thr Leu Ala Thr Val Arg Asp Tyr Tyr Val Gly Val
            180                 185                 190

gca gaa gtc tcc ggt ttc gac gtg gca gat ctt gcc tgc tca gga caa     624
Ala Glu Val Ser Gly Phe Asp Val Ala Asp Leu Ala Cys Ser Gly Gln
        195                 200                 205

acc caa cgt gcc gtc gcc agc gcg cgc cgg gca ctg caa cta gga gtc     672
Thr Gln Arg Ala Val Ala Ser Ala Arg Arg Ala Leu Gln Leu Gly Val
    210                 215                 220

tca ccg gtg gca ctt gcc gcc gca ttg agc atg aaa gtt agc gct atc     720
Ser Pro Val Ala Leu Ala Ala Ala Leu Ser Met Lys Val Ser Ala Ile
225                 230                 235                 240

gcg cgg ttg tat tcc aca cga ggg cgt att gac tcc cgt aaa ctc gca     768
Ala Arg Leu Tyr Ser Thr Arg Gly Arg Ile Asp Ser Arg Lys Leu Ala
                245                 250                 255

gga gag ctg ggc atg cac ccc ttc gtt gtt gaa aaa acc gcc aaa gtt     816
Gly Glu Leu Gly Met His Pro Phe Val Val Glu Lys Thr Ala Lys Val
            260                 265                 270

gcg cgc atg tgg acg ggt aat gct gtg agc caa gcg gtg atc ttg atg     864
Ala Arg Met Trp Thr Gly Asn Ala Val Ser Gln Ala Val Ile Leu Met
        275                 280                 285

gcg gat gtg gat tcc agt ctt aag ggc cac ggc ctc gat ggc gaa ctc     912
Ala Asp Val Asp Ser Ser Leu Lys Gly His Gly Leu Asp Gly Glu Leu
    290                 295                 300

ggc ggt gac ccc gac tac gaa atc gag gat gct atc cgc agg atc tct     960
Gly Gly Asp Pro Asp Tyr Glu Ile Glu Asp Ala Ile Arg Arg Ile Ser
305                 310                 315                 320

gag ctc gca ggc taa                                                 975
Glu Leu Ala Gly
                325

<210> 141
<211> 324
<212> PRT
<213> Corynebacterium diphtheriae

<400> 141
Met Gly Gln Gln Leu Leu Pro Val His Leu Val Val Gly Asp Asp Glu
1               5                   10                  15

Phe Leu Ala Glu Arg Ala Arg His Ser Ile Ile Ala Ala Ile Asn Lys
            20                  25                  30

Thr Ile Gly Ser Asp Val Pro Val Thr Thr Leu Arg Ala Gly Glu Val
        35                  40                  45

Asn Ala Ser Glu Leu Ile Gln Leu Thr Ser Pro Ser Leu Phe Gly Glu
    50                  55                  60

Asp Arg Ile Ile Val Leu Thr Asn Met Glu Asp Ala Gly Lys Glu Pro
65                  70                  75                  80

Ala Glu Leu Val Leu Asn Val Ala Val Asp Pro Ala Pro Gly Ile Tyr
                85                  90                  95

Leu Ile Ile Val His Ser Gly Gly Gly Arg Gln Lys Ala Leu Val Pro
            100                 105                 110

Lys Leu Ser Lys Ile Ser Gln Val His Glu Ala Asn Lys Leu Lys Pro
        115                 120                 125

Lys Asp Arg Val Gly Trp Val Thr Asn Glu Phe Lys Arg His Gly Val
    130                 135                 140

Arg Pro Thr Pro Asp Val Val His Ala Leu Leu Glu Gly Val Gly Ser
145                 150                 155                 160

Asp Leu Arg Glu Leu Ala Ser Ala Ile Gly Gln Leu Val Ala Asp Asn
                165                 170                 175

Asn Gly Asp Val Thr Leu Ala Thr Val Arg Asp Tyr Tyr Val Gly Val
            180                 185                 190

Ala Glu Val Ser Gly Phe Asp Val Ala Asp Leu Ala Cys Ser Gly Gln
        195                 200                 205

Thr Gln Arg Ala Val Ala Ser Ala Arg Arg Ala Leu Gln Leu Gly Val
    210                 215                 220

Ser Pro Val Ala Leu Ala Ala Ala Leu Ser Met Lys Val Ser Ala Ile
225                 230                 235                 240

Ala Arg Leu Tyr Ser Thr Arg Gly Arg Ile Asp Ser Arg Lys Leu Ala
                245                 250                 255

Gly Glu Leu Gly Met His Pro Phe Val Val Glu Lys Thr Ala Lys Val
            260                 265                 270

Ala Arg Met Trp Thr Gly Asn Ala Val Ser Gln Ala Val Ile Leu Met
        275                 280                 285

Ala Asp Val Asp Ser Ser Leu Lys Gly His Gly Leu Asp Gly Glu Leu
    290                 295                 300

Gly Gly Asp Pro Asp Tyr Glu Ile Glu Asp Ala Ile Arg Arg Ile Ser
305                 310                 315                 320

Glu Leu Ala Gly

<210> 142
<211> 1035
<212> DNA
<213> Cytophaga hutchinsonii

<400> 142
atggctgaaa ccagttctgc tccggataaa gtattaaaag accttaagga aggtaaatac 60

gctcctgtat attttttgca gggagaggaa ccctatttta tagatctaat atccagttat 120

atagaaaata actgtttgcc ggaatcggag agaggattta atcagacgat cctgtatggc 180

aaagatacca atgtttctac cattcttcag aatgcccgca agtatccgat gttttcggaa 240

aggcaggtag taatggttaa ggaagcacag gatgtttcgg atctgaccaa agattcaaca 300

gagaaatttt taatggcata tctcgaaaat ccattgccta caaccattct ggtattttgc 360

cacaagtata aaaaactgga tgcccgtaaa aaaataacca agcacctgca gaagtattcg 420

gtgtttgttg aatctgccaa actttacgat aataaattgc ctgaatggat taatggctat 480

ttcagggaaa agaattacag gattgatccg aaggcggtcg ttctattggc cgaatctatt 540

ggtacggatc tttcccgaat ggcaaatgaa attgataagc tgctcatcaa cgttaaagat 600

cctgctataa ccatcaccga agatttaatc gaaaaatatg ttggcgtgag caaagaatac 660

aatgtctttg aattacaaaa agctatcggt gtacgggata taataaaagc gaataaaatc 720

gttaatcatt ttgaatccaa tccaaaagcg aatccgattg taatggtgct gtcaagtttg 780

tttacttact atcttaaaat actggcgatt catgccgcca cagataagag tgaagcaagc 840

ctggcaaagg ttgttggtgt acatccattc tttgtaaaag aatacatcaa tgcgtcgcgc 900

atcaatgata ttaataaatg catctcttgt gtgcgcgcca ttcgtgaagc ggacaaaatg 960

gtgaaagggt atacggttgt taatgttacc gatggcttta ttttaaaaga attattgttt 1020

aaattattac attga 1035

<210> 143
<211> 1035
<212> DNA
<213> Cytophaga hutchinsonii

<220>
<221> CDS
<222> (1)..(1035)

<400> 143
atg gct gaa acc agt tct gct ccg gat aaa gta tta aaa gac ctt aag 48
Met Ala Glu Thr Ser Ser Ala Pro Asp Lys Val Leu Lys Asp Leu Lys
1               5                   10                  15

gaa ggt aaa tac gct cct gta tat ttt ttg cag gga gag gaa ccc tat 96
Glu Gly Lys Tyr Ala Pro Val Tyr Phe Leu Gln Gly Glu Glu Pro Tyr
            20                  25                  30

ttt ata gat cta ata tcc agt tat ata gaa aat aac tgt ttg ccg gaa 144
Phe Ile Asp Leu Ile Ser Ser Tyr Ile Glu Asn Asn Cys Leu Pro Glu
        35                  40                  45

tcg gag aga gga ttt aat cag acg atc ctg tat ggc aaa gat acc aat 192
Ser Glu Arg Gly Phe Asn Gln Thr Ile Leu Tyr Gly Lys Asp Thr Asn
    50                  55                  60

gtt tct acc att ctt cag aat gcc cgc aag tat ccg atg ttt tcg gaa 240
Val Ser Thr Ile Leu Gln Asn Ala Arg Lys Tyr Pro Met Phe Ser Glu
65                  70                  75                  80

agg cag gta gta atg gtt aag gaa gca cag gat gtt tcg gat ctg acc 288
Arg Gln Val Val Met Val Lys Glu Ala Gln Asp Val Ser Asp Leu Thr

                85                  90                  95

aaa gat tca aca gag aaa ttt tta atg gca tat ctc gaa aat cca ttg 336
Lys Asp Ser Thr Glu Lys Phe Leu Met Ala Tyr Leu Glu Asn Pro Leu
            100                 105                 110

cct aca acc att ctg gta ttt tgc cac aag tat aaa aaa ctg gat gcc 384
Pro Thr Thr Ile Leu Val Phe Cys His Lys Tyr Lys Lys Leu Asp Ala
        115                 120                 125

cgt aaa aaa ata acc aag cac ctg cag aag tat tcg gtg ttt gtt gaa 432
Arg Lys Lys Ile Thr Lys His Leu Gln Lys Tyr Ser Val Phe Val Glu
    130                 135                 140

tct gcc aaa ctt tac gat aat aaa ttg cct gaa tgg att aat ggc tat 480
Ser Ala Lys Leu Tyr Asp Asn Lys Leu Pro Glu Trp Ile Asn Gly Tyr
145                 150                 155                 160

ttc agg gaa aag aat tac agg att gat ccg aag gcg gtc gtt cta ttg 528
Phe Arg Glu Lys Asn Tyr Arg Ile Asp Pro Lys Ala Val Val Leu Leu
                165                 170                 175

gcc gaa tct att ggt acg gat ctt tcc cga atg gca aat gaa att gat 576
Ala Glu Ser Ile Gly Thr Asp Leu Ser Arg Met Ala Asn Glu Ile Asp
            180                 185                 190

aag ctg ctc atc aac gtt aaa gat cct gct ata acc atc acc gaa gat 624
Lys Leu Leu Ile Asn Val Lys Asp Pro Ala Ile Thr Ile Thr Glu Asp
        195                 200                 205

tta atc gaa aaa tat gtt ggc gtg agc aaa gaa tac aat gtc ttt gaa 672
Leu Ile Glu Lys Tyr Val Gly Val Ser Lys Glu Tyr Asn Val Phe Glu
    210                 215                 220

tta caa aaa gct atc ggt gta cgg gat ata ata aaa gcg aat aaa atc 720
Leu Gln Lys Ala Ile Gly Val Arg Asp Ile Ile Lys Ala Asn Lys Ile
225                 230                 235                 240

gtt aat cat ttt gaa tcc aat cca aaa gcg aat ccg att gta atg gtg 768
Val Asn His Phe Glu Ser Asn Pro Lys Ala Asn Pro Ile Val Met Val
                245                 250                 255

ctg tca agt ttg ttt act tac tat ctt aaa ata ctg gcg att cat gcc 816
Leu Ser Ser Leu Phe Thr Tyr Tyr Leu Lys Ile Leu Ala Ile His Ala
            260                 265                 270

gcc aca gat aag agt gaa gca agc ctg gca aag gtt gtt ggt gta cat 864
Ala Thr Asp Lys Ser Glu Ala Ser Leu Ala Lys Val Val Gly Val His

        275                 280                 285

cca ttc ttt gta aaa gaa tac atc aat gcg tcg cgc atc aat gat att 912
Pro Phe Phe Val Lys Glu Tyr Ile Asn Ala Ser Arg Ile Asn Asp Ile
    290                 295                 300

aat aaa tgc atc tct tgt gtg cgc gcc att cgt gaa gcg gac aaa atg 960
Asn Lys Cys Ile Ser Cys Val Arg Ala Ile Arg Glu Ala Asp Lys Met
305                 310                 315                 320

gtg aaa ggg tat acg gtt gtt aat gtt acc gat ggc ttt att tta aaa 1008
Val Lys Gly Tyr Thr Val Val Asn Val Thr Asp Gly Phe Ile Leu Lys
                325                 330                 335

gaa tta ttg ttt aaa tta tta cat tga 1035
Glu Leu Leu Phe Lys Leu Leu His
340 345

<210> 144
<211> 344
<212> PRT
<213> Cytophaga hutchinsonii

<400> 144
Met Ala Glu Thr Ser Ser Ala Pro Asp Lys Val Leu Lys Asp Leu Lys
1               5                   10                  15

Glu Gly Lys Tyr Ala Pro Val Tyr Phe Leu Gln Gly Glu Glu Pro Tyr
            20                  25                  30

Phe Ile Asp Leu Ile Ser Ser Tyr Ile Glu Asn Asn Cys Leu Pro Glu
        35                  40                  45

Ser Glu Arg Gly Phe Asn Gln Thr Ile Leu Tyr Gly Lys Asp Thr Asn
    50                  55                  60

Val Ser Thr Ile Leu Gln Asn Ala Arg Lys Tyr Pro Met Phe Ser Glu
65                  70                  75                  80

Arg Gln Val Val Met Val Lys Glu Ala Gln Asp Val Ser Asp Leu Thr
                85                  90                  95

Lys Asp Ser Thr Glu Lys Phe Leu Met Ala Tyr Leu Glu Asn Pro Leu
            100                 105                 110

Pro Thr Thr Ile Leu Val Phe Cys His Lys Tyr Lys Lys Leu Asp Ala
        115                 120                 125

Arg Lys Lys Ile Thr Lys His Leu Gln Lys Tyr Ser Val Phe Val Glu
    130                 135                 140

Ser Ala Lys Leu Tyr Asp Asn Lys Leu Pro Glu Trp Ile Asn Gly Tyr
145                 150                 155                 160

Phe Arg Glu Lys Asn Tyr Arg Ile Asp Pro Lys Ala Val Val Leu Leu
                165                 170                 175

Ala Glu Ser Ile Gly Thr Asp Leu Ser Arg Met Ala Asn Glu Ile Asp
            180                 185                 190

Lys Leu Leu Ile Asn Val Lys Asp Pro Ala Ile Thr Ile Thr Glu Asp
        195                 200                 205

Leu Ile Glu Lys Tyr Val Gly Val Ser Lys Glu Tyr Asn Val Phe Glu
    210                 215                 220

Leu Gln Lys Ala Ile Gly Val Arg Asp Ile Ile Lys Ala Asn Lys Ile
225                 230                 235                 240

Val Asn His Phe Glu Ser Asn Pro Lys Ala Asn Pro Ile Val Met Val
                245                 250                 255

Leu Ser Ser Leu Phe Thr Tyr Tyr Leu Lys Ile Leu Ala Ile His Ala
            260                 265                 270

Ala Thr Asp Lys Ser Glu Ala Ser Leu Ala Lys Val Val Gly Val His
        275                 280                 285

Pro Phe Phe Val Lys Glu Tyr Ile Asn Ala Ser Arg Ile Asn Asp Ile
    290                 295                 300

Asn Lys Cys Ile Ser Cys Val Arg Ala Ile Arg Glu Ala Asp Lys Met
305                 310                 315                 320

Val Lys Gly Tyr Thr Val Val Asn Val Thr Asp Gly Phe Ile Leu Lys
                325                 330                 335

Glu Leu Leu Phe Lys Leu Leu His
            340

<210> 145
<211> 1200
<212> DNA
<213> Dehalococcoides ethenogenes

<400> 145
atgtttgcgg agttcgtagg gcattacaac ttcttcgggc attgtttcaa atcctccaag 60

tgcagcttac gggcagattg taacatactg ccaggcattt ttgcactaaa cttcaaaggt 120

tacgcgcccg tttcgttatg ctacaatatc agccgaaggt ggctgttaaa tttgctgtat 180

atttttaccg gcagtgatga tttttccctg aaagaacgcc tgaagcagat aaaactggct 240

ttgggtgatg agtctttgtc ctctgccaat accaccatgc tggagggcaa aaacctgacg 300

gctgccgaac ttcaaaacta tgtccagact attccctttt tagcccctgc ccggctggtt 360

atggtaaacg gcctgctgga gaggtttgaa ggctcaaaag gtaaagccgc cgccaaagat 420

aaggcagaca aaaacagccc ggatgaattt gcccgggcgc tgtctaatct gcctcccaca 480

accctggcgg tgcttctgga ctataccacc gctaaagacg gtgattaccg tgaaaaagaa 540

aaagtatccg ctttggcctc aaaccccctt tttaagctgg taagccccgg tgctgaggta 600

aagcactttg cacccctttc tgcccagaac ctgcccggct gggtaatgca gcgtgccaaa 660

cagtcttcta tccagatgtc tgttccggca gcccgtctgc tggcccgttt tgtgggggcg 720

gacttgtggg cgctttcctc cgagattgag aaactgggct tgtatgccca ggggcgggcg 780

gtggaggaag ccgatgttga aaatatggtg ggttattccc aggaagtaaa tatattcggt 840

ttggtggatg ccgtactgga ccgcaaggtt aaagacgccc agcagtctct ggcggctttt 900

wctgycggcg gcgggtyacc cggttttttg ctgtttatgc tggtacgcca gctgcggctg 960

atggtgcgtt ataccgccct taaaaagcgg gggctgaaag aggccgagat ttttaaagct 1020

atggkcggct ctgagtacgc ccttaggaaa acagcctctc aggcggcctg ccagaacatg 1080

gacagcctta aagctattta ttccaaactg ctggaaacag attttaatat aaaaaccggc 1140

cgctttgaac cggagctggc catgggtttg ctggttgccg aactttgcgg gagaaaatag 1200


<210> 146
<211> 1200
<212> DNA
<213> Dehalococcoides ethenogenes

<220>
<221> CDS
<222> (1)..(1200)

<400> 146
atg ttt gcg gag ttc gta ggg cat tac aac ttc ttc ggg cat tgt ttc     48
Met Phe Ala Glu Phe Val Gly His Tyr Asn Phe Phe Gly His Cys Phe
1               5                   10                  15

aaa tcc tcc aag tgc agc tta cgg gca gat tgt aac ata ctg cca ggc     96
Lys Ser Ser Lys Cys Ser Leu Arg Ala Asp Cys Asn Ile Leu Pro Gly
            20                  25                  30

att ttt gca cta aac ttc aaa ggt tac gcg ccc gtt tcg tta tgc tac     144
Ile Phe Ala Leu Asn Phe Lys Gly Tyr Ala Pro Val Ser Leu Cys Tyr
        35                  40                  45

aat atc agc cga agg tgg ctg tta aat ttg ctg tat att ttt acc ggc     192
Asn Ile Ser Arg Arg Trp Leu Leu Asn Leu Leu Tyr Ile Phe Thr Gly
    50                  55                  60

agt gat gat ttt tcc ctg aaa gaa cgc ctg aag cag ata aaa ctg gct     240
Ser Asp Asp Phe Ser Leu Lys Glu Arg Leu Lys Gln Ile Lys Leu Ala
65                  70                  75                  80

ttg ggt gat gag tct ttg tcc tct gcc aat acc acc atg ctg gag ggc     288
Leu Gly Asp Glu Ser Leu Ser Ser Ala Asn Thr Thr Met Leu Glu Gly
                85                  90                  95

aaa aac ctg acg gct gcc gaa ctt caa aac tat gtc cag act att ccc     336
Lys Asn Leu Thr Ala Ala Glu Leu Gln Asn Tyr Val Gln Thr Ile Pro
            100                 105                 110

ttt tta gcc cct gcc cgg ctg gtt atg gta aac ggc ctg ctg gag agg     384
Phe Leu Ala Pro Ala Arg Leu Val Met Val Asn Gly Leu Leu Glu Arg
        115                 120                 125

ttt gaa ggc tca aaa ggt aaa gcc gcc gcc aaa gat aag gca gac aaa     432
Phe Glu Gly Ser Lys Gly Lys Ala Ala Ala Lys Asp Lys Ala Asp Lys
    130                 135                 140

aac agc ccg gat gaa ttt gcc cgg gcg ctg tct aat ctg cct ccc aca     480
Asn Ser Pro Asp Glu Phe Ala Arg Ala Leu Ser Asn Leu Pro Pro Thr
145                 150                 155                 160

acc ctg gcg gtg ctt ctg gac tat acc acc gct aaa gac ggt gat tac     528
Thr Leu Ala Val Leu Leu Asp Tyr Thr Thr Ala Lys Asp Gly Asp Tyr
                165                 170                 175

cgt gaa aaa gaa aaa gta tcc gct ttg gcc tca aac ccc ctt ttt aag 576
Arg Glu Lys Glu Lys Val Ser Ala Leu Ala Ser Asn Pro Leu Phe Lys
            180                 185                 190

ctg gta agc ccc ggt gct gag gta aag cac ttt gca ccc ctt tct gcc 624
Leu Val Ser Pro Gly Ala Glu Val Lys His Phe Ala Pro Leu Ser Ala
        195                 200                 205

cag aac ctg ccc ggc tgg gta atg cag cgt gcc aaa cag tct tct atc 672
Gln Asn Leu Pro Gly Trp Val Met Gln Arg Ala Lys Gln Ser Ser Ile
    210                 215                 220

cag atg tct gtt ccg gca gcc cgt ctg ctg gcc cgt ttt gtg ggg gcg 720
Gln Met Ser Val Pro Ala Ala Arg Leu Leu Ala Arg Phe Val Gly Ala
225                 230                 235                 240

gac ttg tgg gcg ctt tcc tcc gag att gag aaa ctg ggc ttg tat gcc 768
Asp Leu Trp Ala Leu Ser Ser Glu Ile Glu Lys Leu Gly Leu Tyr Ala
                245                 250                 255

cag ggg cgg gcg gtg gag gaa gcc gat gtt gaa aat atg gtg ggt tat 816
Gln Gly Arg Ala Val Glu Glu Ala Asp Val Glu Asn Met Val Gly Tyr
            260                 265                 270

tcc cag gaa gta aat ata ttc ggt ttg gtg gat gcc gta ctg gac cgc 864
Ser Gln Glu Val Asn Ile Phe Gly Leu Val Asp Ala Val Leu Asp Arg
        275                 280                 285

aag gtt aaa gac gcc cag cag tct ctg gcg gct ttt wct gyc ggc ggc 912
Lys Val Lys Asp Ala Gln Gln Ser Leu Ala Ala Phe Xaa Xaa Gly Gly
    290                 295                 300

ggg tya ccc ggt ttt ttg ctg ttt atg ctg gta cgc cag ctg cgg ctg 960
Gly Xaa Pro Gly Phe Leu Leu Phe Met Leu Val Arg Gln Leu Arg Leu
305                 310                 315                 320

atg gtg cgt tat acc gcc ctt aaa aag cgg ggg ctg aaa gag gcc gag 1008
Met Val Arg Tyr Thr Ala Leu Lys Lys Arg Gly Leu Lys Glu Ala Glu
                325                 330                 335

att ttt aaa gct atg gkc ggc tct gag tac gcc ctt agg aaa aca gcc 1056
Ile Phe Lys Ala Met Xaa Gly Ser Glu Tyr Ala Leu Arg Lys Thr Ala
            340                 345                 350

tct cag gcg gcc tgc cag aac atg gac agc ctt aaa gct att tat tcc 1104
Ser Gln Ala Ala Cys Gln Asn Met Asp Ser Leu Lys Ala Ile Tyr Ser
        355                 360                 365

aaa ctg ctg gaa aca gat ttt aat ata aaa acc ggc cgc ttt gaa ccg 1152
Lys Leu Leu Glu Thr Asp Phe Asn Ile Lys Thr Gly Arg Phe Glu Pro
    370                 375                 380

gag ctg gcc atg ggt ttg ctg gtt gcc gaa ctt tgc ggg aga aaa tag 1200
Glu Leu Ala Met Gly Leu Leu Val Ala Glu Leu Cys Gly Arg Lys
385 390 395 400

<210> 147
<211> 399
<212> PRT
<213> Dehalococcoides ethenogenes

<400> 147
Met Phe Ala Glu Phe Val Gly His Tyr Asn Phe Phe Gly His Cys Phe
1               5                   10                  15

Lys Ser Ser Lys Cys Ser Leu Arg Ala Asp Cys Asn Ile Leu Pro Gly
            20                  25                  30

Ile Phe Ala Leu Asn Phe Lys Gly Tyr Ala Pro Val Ser Leu Cys Tyr
        35                  40                  45

Asn Ile Ser Arg Arg Trp Leu Leu Asn Leu Leu Tyr Ile Phe Thr Gly
    50                  55                  60

Ser Asp Asp Phe Ser Leu Lys Glu Arg Leu Lys Gln Ile Lys Leu Ala
65                  70                  75                  80

Leu Gly Asp Glu Ser Leu Ser Ser Ala Asn Thr Thr Met Leu Glu Gly
                85                  90                  95

Lys Asn Leu Thr Ala Ala Glu Leu Gln Asn Tyr Val Gln Thr Ile Pro
            100                 105                 110

Phe Leu Ala Pro Ala Arg Leu Val Met Val Asn Gly Leu Leu Glu Arg
        115                 120                 125

Phe Glu Gly Ser Lys Gly Lys Ala Ala Ala Lys Asp Lys Ala Asp Lys
    130                 135                 140

Asn Ser Pro Asp Glu Phe Ala Arg Ala Leu Ser Asn Leu Pro Pro Thr
145                 150                 155                 160

Thr Leu Ala Val Leu Leu Asp Tyr Thr Thr Ala Lys Asp Gly Asp Tyr
                165                 170                 175

Arg Glu Lys Glu Lys Val Ser Ala Leu Ala Ser Asn Pro Leu Phe Lys
            180                 185                 190

Leu Val Ser Pro Gly Ala Glu Val Lys His Phe Ala Pro Leu Ser Ala
        195                 200                 205

Gln Asn Leu Pro Gly Trp Val Met Gln Arg Ala Lys Gln Ser Ser Ile
    210                 215                 220

Gln Met Ser Val Pro Ala Ala Arg Leu Leu Ala Arg Phe Val Gly Ala
225                 230                 235                 240

Asp Leu Trp Ala Leu Ser Ser Glu Ile Glu Lys Leu Gly Leu Tyr Ala
                245                 250                 255

Gln Gly Arg Ala Val Glu Glu Ala Asp Val Glu Asn Met Val Gly Tyr
            260                 265                 270

Ser Gln Glu Val Asn Ile Phe Gly Leu Val Asp Ala Val Leu Asp Arg
        275                 280                 285

Lys Val Lys Asp Ala Gln Gln Ser Leu Ala Ala Phe Xaa Xaa Gly Gly
    290                 295                 300

Gly Xaa Pro Gly Phe Leu Leu Phe Met Leu Val Arg Gln Leu Arg Leu
305                 310                 315                 320

Met Val Arg Tyr Thr Ala Leu Lys Lys Arg Gly Leu Lys Glu Ala Glu
                325                 330                 335

Ile Phe Lys Ala Met Xaa Gly Ser Glu Tyr Ala Leu Arg Lys Thr Ala
            340                 345                 350

Ser Gln Ala Ala Cys Gln Asn Met Asp Ser Leu Lys Ala Ile Tyr Ser
        355                 360                 365

Lys Leu Leu Glu Thr Asp Phe Asn Ile Lys Thr Gly Arg Phe Glu Pro
    370                 375                 380

Glu Leu Ala Met Gly Leu Leu Val Ala Glu Leu Cys Gly Arg Lys
385                 390                 395

<210> 148
<211> 1008
<212> DNA
<213> Porphyromonas gingivalis

<400> 148
atggcctcct attccgatat tgtcgattct gtacgtaagt gccaattctc gccactctat 60

ctgctggccg gcgaagaacc cttctatata gacgagttgg ccactctgct ggagacgcac 120

gtggtgccgg tagatgaatg ggacttcaac agggttatcc tctacggcga caaaacctcc 180

gtagccgata tagcgaacga agcgcgccgt ttcccgatga tgggccggag gcagttgatc 240

gtcgttcggg aggcgcagtt agtggacaat atcgacttgc tcgaagcaca ttatggcact 300

ttccccgata ctacgattct ggtcattgcc tataagaaga aacccgataa gcgaaaggct 360

ttctatacga aggctgaaaa gttcgggaaa gtattcgtgt ccgagaccat acccgactat 420

aagatgccgg acttcatcct gtcggctgct gccggcaaga aactgagcgt atcgcccgaa 480

gtggcatata tgctggccga ttacctcggc aatgatctgg agaagttgat gaatgagctg 540

gacaagctca ttctcataac acaagatagt aggggagtgg tcacgtccga gatcgtggag 600

cagcatatcg gagtaagcaa atccttcaat aatttcgaac tccttcgtgc catagtcaat 660

cgcgaaacgg gcaaaacctt tcgcatcgct cactactttg ccagaaatga gaaggagcac 720

cccatacagg ccactctacc cgtactcttc aactatttca gcaacctgat gatcgtttgc 780

tacttgccac agaaaaaacc cgatgctatc atgaaagcat tgtcgatacg caatttccaa 840

gtgcgcgact atatgacagg gctgaagatg tattcgacga ggaaggtttt cgacattata 900

cacgagatcc ggatgacgga tgcccgcagt aagggggtgg atacgaccgg cacatttgcc 960

ggcagtgggg atttgcttcg ggagctactc cattttatat tccattga 1008

<210> 149
<211> 1008
<212> DNA
<213> Porphyromonas gingivalis

<220>
<221> CDS
<222> (1)..(1008)

<400> 149
atg gcc tcc tat tcc gat att gtc gat tct gta cgt aag tgc caa ttc 48

Met Ala Ser Tyr Ser Asp Ile Val Asp Ser Val Arg Lys Cys Gln Phe
1               5                   10                  15

tcg cca ctc tat ctg ctg gcc ggc gaa gaa ccc ttc tat ata gac gag     96
Ser Pro Leu Tyr Leu Leu Ala Gly Glu Glu Pro Phe Tyr Ile Asp Glu
            20                  25                  30

ttg gcc act ctg ctg gag acg cac gtg gtg ccg gta gat gaa tgg gac     144
Leu Ala Thr Leu Leu Glu Thr His Val Val Pro Val Asp Glu Trp Asp
        35                  40                  45

ttc aac agg gtt atc ctc tac ggc gac aaa acc tcc gta gcc gat ata     192
Phe Asn Arg Val Ile Leu Tyr Gly Asp Lys Thr Ser Val Ala Asp Ile
    50                  55                  60

gcg aac gaa gcg cgc cgt ttc ccg atg atg ggc cgg agg cag ttg atc     240
Ala Asn Glu Ala Arg Arg Phe Pro Met Met Gly Arg Arg Gln Leu Ile
65                  70                  75                  80

gtc gtt cgg gag gcg cag tta gtg gac aat atc gac ttg ctc gaa gca     288
Val Val Arg Glu Ala Gln Leu Val Asp Asn Ile Asp Leu Leu Glu Ala
                85                  90                  95

cat tat ggc act ttc ccc gat act acg att ctg gtc att gcc tat aag     336
His Tyr Gly Thr Phe Pro Asp Thr Thr Ile Leu Val Ile Ala Tyr Lys
            100                 105                 110

aag aaa ccc gat aag cga aag gct ttc tat acg aag gct gaa aag ttc     384
Lys Lys Pro Asp Lys Arg Lys Ala Phe Tyr Thr Lys Ala Glu Lys Phe
        115                 120                 125

ggg aaa gta ttc gtg tcc gag acc ata ccc gac tat aag atg ccg gac     432
Gly Lys Val Phe Val Ser Glu Thr Ile Pro Asp Tyr Lys Met Pro Asp
    130                 135                 140

ttc atc ctg tcg gct gct gcc ggc aag aaa ctg agc gta tcg ccc gaa     480
Phe Ile Leu Ser Ala Ala Ala Gly Lys Lys Leu Ser Val Ser Pro Glu
145                 150                 155                 160

gtg gca tat atg ctg gcc gat tac ctc ggc aat gat ctg gag aag ttg     528
Val Ala Tyr Met Leu Ala Asp Tyr Leu Gly Asn Asp Leu Glu Lys Leu
                165                 170                 175

atg aat gag ctg gac aag ctc att ctc ata aca caa gat agt agg gga     576
Met Asn Glu Leu Asp Lys Leu Ile Leu Ile Thr Gln Asp Ser Arg Gly
            180                 185                 190

gtg gtc acg tcc gag atc gtg gag cag cat atc gga gta agc aaa tcc     624

Val Val Thr Ser Glu Ile Val Glu Gln His Ile Gly Val Ser Lys Ser
        195                 200                 205

ttc aat aat ttc gaa ctc ctt cgt gcc ata gtc aat cgc gaa acg ggc 672
Phe Asn Asn Phe Glu Leu Leu Arg Ala Ile Val Asn Arg Glu Thr Gly
    210                 215                 220

aaa acc ttt cgc atc gct cac tac ttt gcc aga aat gag aag gag cac 720
Lys Thr Phe Arg Ile Ala His Tyr Phe Ala Arg Asn Glu Lys Glu His
225                 230                 235                 240

ccc ata cag gcc act cta ccc gta ctc ttc aac tat ttc agc aac ctg 768
Pro Ile Gln Ala Thr Leu Pro Val Leu Phe Asn Tyr Phe Ser Asn Leu
                245                 250                 255

atg atc gtt tgc tac ttg cca cag aaa aaa ccc gat gct atc atg aaa 816
Met Ile Val Cys Tyr Leu Pro Gln Lys Lys Pro Asp Ala Ile Met Lys
            260                 265                 270

gca ttg tcg ata cgc aat ttc caa gtg cgc gac tat atg aca ggg ctg 864
Ala Leu Ser Ile Arg Asn Phe Gln Val Arg Asp Tyr Met Thr Gly Leu
        275                 280                 285

aag atg tat tcg acg agg aag gtt ttc gac att ata cac gag atc cgg 912
Lys Met Tyr Ser Thr Arg Lys Val Phe Asp Ile Ile His Glu Ile Arg
    290                 295                 300

atg acg gat gcc cgc agt aag ggg gtg gat acg acc ggc aca ttt gcc 960
Met Thr Asp Ala Arg Ser Lys Gly Val Asp Thr Thr Gly Thr Phe Ala
305                 310                 315                 320

ggc agt ggg gat ttg ctt cgg gag cta ctc cat ttt ata ttc cat tga 1008
Gly Ser Gly Asp Leu Leu Arg Glu Leu Leu His Phe Ile Phe His
                325                 330                 335

<210> 150
<211> 335
<212> PRT
<213> Porphyromonas gingivalis

<400> 150
Met Ala Ser Tyr Ser Asp Ile Val Asp Ser Val Arg Lys Cys Gln Phe
1               5                   10                  15

Ser Pro Leu Tyr Leu Leu Ala Gly Glu Glu Pro Phe Tyr Ile Asp Glu
            20                  25                  30

Leu Ala Thr Leu Leu Glu Thr His Val Val Pro Val Asp Glu Trp Asp
        35                  40                  45

Phe Asn Arg Val Ile Leu Tyr Gly Asp Lys Thr Ser Val Ala Asp Ile
    50                  55                  60

Ala Asn Glu Ala Arg Arg Phe Pro Met Met Gly Arg Arg Gln Leu Ile
65                  70                  75                  80

Val Val Arg Glu Ala Gln Leu Val Asp Asn Ile Asp Leu Leu Glu Ala
                85                  90                  95

His Tyr Gly Thr Phe Pro Asp Thr Thr Ile Leu Val Ile Ala Tyr Lys
            100                 105                 110

Lys Lys Pro Asp Lys Arg Lys Ala Phe Tyr Thr Lys Ala Glu Lys Phe
        115                 120                 125

Gly Lys Val Phe Val Ser Glu Thr Ile Pro Asp Tyr Lys Met Pro Asp
    130                 135                 140

Phe Ile Leu Ser Ala Ala Ala Gly Lys Lys Leu Ser Val Ser Pro Glu
145                 150                 155                 160

Val Ala Tyr Met Leu Ala Asp Tyr Leu Gly Asn Asp Leu Glu Lys Leu
                165                 170                 175

Met Asn Glu Leu Asp Lys Leu Ile Leu Ile Thr Gln Asp Ser Arg Gly
            180                 185                 190

Val Val Thr Ser Glu Ile Val Glu Gln His Ile Gly Val Ser Lys Ser
        195                 200                 205

Phe Asn Asn Phe Glu Leu Leu Arg Ala Ile Val Asn Arg Glu Thr Gly
    210                 215                 220

Lys Thr Phe Arg Ile Ala His Tyr Phe Ala Arg Asn Glu Lys Glu His
225                 230                 235                 240

Pro Ile Gln Ala Thr Leu Pro Val Leu Phe Asn Tyr Phe Ser Asn Leu
                245                 250                 255

Met Ile Val Cys Tyr Leu Pro Gln Lys Lys Pro Asp Ala Ile Met Lys
            260                 265                 270

Ala Leu Ser Ile Arg Asn Phe Gln Val Arg Asp Tyr Met Thr Gly Leu
        275                 280                 285

Lys Met Tyr Ser Thr Arg Lys Val Phe Asp Ile Ile His Glu Ile Arg
    290                 295                 300

Met Thr Asp Ala Arg Ser Lys Gly Val Asp Thr Thr Gly Thr Phe Ala
305                 310                 315                 320

Gly Ser Gly Asp Leu Leu Arg Glu Leu Leu His Phe Ile Phe His
                325                 330                 335

<210> 151
<211> 1005
<212> DNA
<213> Prochlorococcus marinus

<400> 151
atgccaatac aaataatatg gggaaatgat ttaaatgcaa gtaataagtt tataaaacaa 60

ataattgatg aaaaagtatc caaaacatgg ggagaaataa atattagtta cttaaatggt 120

gatgatgata atcaaataaa acaagcattt gatgaaatac taacacctcc tttgggggat 180

ggatctcgag tagttgtatt aaaaaataac ccattattca caaataaaaa tgaggaaata 240

aggctcaaat ttgaaaaaat ttatcaaaac attccaagta acacttactt tcttttacaa 300

aatacaaaaa aaccagattc aagactaaag agtacaaaat ttattcaaaa tttaataaaa 360

aaagatttag caattgaaac ctcattttca ttgcccgata tctgggattt tgaaggccag 420

aaaagatact tagaattcac agcaaattcg atgaatattc atttaggtaa agggaccgct 480

gatttaatta ttgattcagt aggaaatgat agttttaact tagctagcga attatctaaa 540

gcaaagatat atctatctgc aataaataat aacgaaaaca aaggacttat tctagaaagt 600

gatgttgtaa aaaaaatatt caatgatcaa cagtcaaata tctttaaaat aatcgatcat 660

ctattacaaa atagtattag tgatggtctt atagaaatct attatttatt aaaaaaaggg 720

gaacctgctt taaggctcaa tgcgggatta ataagtcaaa tcagaatgca taccatagta 780

aaattacttc ataaatctgg tgagcaagat ttatcaaaaa tatgcaaact tgcaaatata 840

tctaacccaa aaagaatttt ttttatccgt aaaaaagtaa acaatatatc ctcagatttc 900

ttaattaaat tgatgagtaa tttattagat attgaatcat cactaaaaaa aggcaataat 960

cctattaatg tatttgcgga aaatctcgtt aacttaagtt aataa 1005

<210> 152
<211> 1005
<212> DNA
<213> Prochlorococcus marinus

<220>
<221> CDS
<222> (1)..(1005)

<400> 152
atg cca ata caa ata ata tgg gga aat gat tta aat gca agt aat aag 48
Met Pro Ile Gln Ile Ile Trp Gly Asn Asp Leu Asn Ala Ser Asn Lys
1               5                   10                  15

ttt ata aaa caa ata att gat gaa aaa gta tcc aaa aca tgg gga gaa 96
Phe Ile Lys Gln Ile Ile Asp Glu Lys Val Ser Lys Thr Trp Gly Glu
            20                  25                  30

ata aat att agt tac tta aat ggt gat gat gat aat caa ata aaa caa 144
Ile Asn Ile Ser Tyr Leu Asn Gly Asp Asp Asp Asn Gln Ile Lys Gln
        35                  40                  45

gca ttt gat gaa ata cta aca cct cct ttg ggg gat gga tct cga gta 192
Ala Phe Asp Glu Ile Leu Thr Pro Pro Leu Gly Asp Gly Ser Arg Val
    50                  55                  60

gtt gta tta aaa aat aac cca tta ttc aca aat aaa aat gag gaa ata 240
Val Val Leu Lys Asn Asn Pro Leu Phe Thr Asn Lys Asn Glu Glu Ile
65                  70                  75                  80

agg ctc aaa ttt gaa aaa att tat caa aac att cca agt aac act tac 288
Arg Leu Lys Phe Glu Lys Ile Tyr Gln Asn Ile Pro Ser Asn Thr Tyr
                85                  90                  95

ttt ctt tta caa aat aca aaa aaa cca gat tca aga cta aag agt aca 336
Phe Leu Leu Gln Asn Thr Lys Lys Pro Asp Ser Arg Leu Lys Ser Thr
            100                 105                 110

aaa ttt att caa aat tta ata aaa aaa gat tta gca att gaa acc tca 384
Lys Phe Ile Gln Asn Leu Ile Lys Lys Asp Leu Ala Ile Glu Thr Ser
        115                 120                 125

ttt tca ttg ccc gat atc tgg gat ttt gaa ggc cag aaa aga tac tta 432
Phe Ser Leu Pro Asp Ile Trp Asp Phe Glu Gly Gln Lys Arg Tyr Leu
    130                 135                 140

gaa ttc aca gca aat tcg atg aat att cat tta ggt aaa ggg acc gct 480
Glu Phe Thr Ala Asn Ser Met Asn Ile His Leu Gly Lys Gly Thr Ala
145                 150                 155                 160

gat tta att att gat tca gta gga aat gat agt ttt aac tta gct agc 528
Asp Leu Ile Ile Asp Ser Val Gly Asn Asp Ser Phe Asn Leu Ala Ser
                165                 170                 175

gaa tta tct aaa gca aag ata tat cta tct gca ata aat aat aac gaa 576
Glu Leu Ser Lys Ala Lys Ile Tyr Leu Ser Ala Ile Asn Asn Asn Glu
            180                 185                 190

aac aaa gga ctt att cta gaa agt gat gtt gta aaa aaa ata ttc aat 624
Asn Lys Gly Leu Ile Leu Glu Ser Asp Val Val Lys Lys Ile Phe Asn
        195                 200                 205

gat caa cag tca aat atc ttt aaa ata atc gat cat cta tta caa aat 672
Asp Gln Gln Ser Asn Ile Phe Lys Ile Ile Asp His Leu Leu Gln Asn
    210                 215                 220

agt att agt gat ggt ctt ata gaa atc tat tat tta tta aaa aaa ggg 720
Ser Ile Ser Asp Gly Leu Ile Glu Ile Tyr Tyr Leu Leu Lys Lys Gly
225                 230                 235                 240

gaa cct gct tta agg ctc aat gcg gga tta ata agt caa atc aga atg 768
Glu Pro Ala Leu Arg Leu Asn Ala Gly Leu Ile Ser Gln Ile Arg Met
                245                 250                 255

cat acc ata gta aaa tta ctt cat aaa tct ggt gag caa gat tta tca 816
His Thr Ile Val Lys Leu Leu His Lys Ser Gly Glu Gln Asp Leu Ser
            260                 265                 270

aaa ata tgc aaa ctt gca aat ata tct aac cca aaa aga att ttt ttt 864
Lys Ile Cys Lys Leu Ala Asn Ile Ser Asn Pro Lys Arg Ile Phe Phe
        275                 280                 285

atc cgt aaa aaa gta aac aat ata tcc tca gat ttc tta att aaa ttg 912
Ile Arg Lys Lys Val Asn Asn Ile Ser Ser Asp Phe Leu Ile Lys Leu
    290                 295                 300

atg agt aat tta tta gat att gaa tca tca cta aaa aaa ggc aat aat 960
Met Ser Asn Leu Leu Asp Ile Glu Ser Ser Leu Lys Lys Gly Asn Asn
305                 310                 315                 320

cct att aat gta ttt gcg gaa aat ctc gtt aac tta agt taa taa 1005
Pro Ile Asn Val Phe Ala Glu Asn Leu Val Asn Leu Ser
325 330 335

<210> 153
<211> 333
<212> PRT
<213> Prochlorococcus marinus

<400> 153
Met Pro Ile Gln Ile Ile Trp Gly Asn Asp Leu Asn Ala Ser Asn Lys
1               5                   10                  15

Phe Ile Lys Gln Ile Ile Asp Glu Lys Val Ser Lys Thr Trp Gly Glu
            20                  25                  30

Ile Asn Ile Ser Tyr Leu Asn Gly Asp Asp Asp Asn Gln Ile Lys Gln
        35                  40                  45

Ala Phe Asp Glu Ile Leu Thr Pro Pro Leu Gly Asp Gly Ser Arg Val
    50                  55                  60

Val Val Leu Lys Asn Asn Pro Leu Phe Thr Asn Lys Asn Glu Glu Ile
65                  70                  75                  80

Arg Leu Lys Phe Glu Lys Ile Tyr Gln Asn Ile Pro Ser Asn Thr Tyr
                85                  90                  95

Phe Leu Leu Gln Asn Thr Lys Lys Pro Asp Ser Arg Leu Lys Ser Thr
            100                 105                 110

Lys Phe Ile Gln Asn Leu Ile Lys Lys Asp Leu Ala Ile Glu Thr Ser
        115                 120                 125

Phe Ser Leu Pro Asp Ile Trp Asp Phe Glu Gly Gln Lys Arg Tyr Leu
    130                 135                 140

Glu Phe Thr Ala Asn Ser Met Asn Ile His Leu Gly Lys Gly Thr Ala
145                 150                 155                 160

Asp Leu Ile Ile Asp Ser Val Gly Asn Asp Ser Phe Asn Leu Ala Ser
                165                 170                 175

Glu Leu Ser Lys Ala Lys Ile Tyr Leu Ser Ala Ile Asn Asn Asn Glu
            180                 185                 190

Asn Lys Gly Leu Ile Leu Glu Ser Asp Val Val Lys Lys Ile Phe Asn
        195                 200                 205

Asp Gln Gln Ser Asn Ile Phe Lys Ile Ile Asp His Leu Leu Gln Asn

210                 215                 220

Ser Ile Ser Asp Gly Leu Ile Glu Ile Tyr Tyr Leu Leu Lys Lys Gly
225                 230                 235                 240

Glu Pro Ala Leu Arg Leu Asn Ala Gly Leu Ile Ser Gln Ile Arg Met
                245                 250                 255

His Thr Ile Val Lys Leu Leu His Lys Ser Gly Glu Gln Asp Leu Ser
            260                 265                 270

Lys Ile Cys Lys Leu Ala Asn Ile Ser Asn Pro Lys Arg Ile Phe Phe
        275                 280                 285

Ile Arg Lys Lys Val Asn Asn Ile Ser Ser Asp Phe Leu Ile Lys Leu
    290                 295                 300

Met Ser Asn Leu Leu Asp Ile Glu Ser Ser Leu Lys Lys Gly Asn Asn
305                 310                 315                 320

Pro Ile Asn Val Phe Ala Glu Asn Leu Val Asn Leu Ser
                325                 330

<210> 154
<211> 1032
<212> DNA
<213> Salmonella typhi

<400> 154
atgatcaggt tgtatcctga acaactccgc gcgcagctca acgaagggct gcgtgcggca 60

tatctattat tgggcaacga tccgctgctg ctgcaggaaa gtcaggacgc tattcgtctg 120

gcggctgcgt cgcaaggctt cgaagagcac cacgctttta ctctcgatcc cagtaccgac 180

tggggatcgc ttttctcact ctgccaggcg atgagcctgt tcgccagccg ccaaacgctg 240

gtgctgcaac tgccggaaaa cgggcctaac gcggcaatga atgagcaact cgccaccctg 300

agcgagctac tgcatgacga cctgttatta atagtacgcg gaaacaagct caccaaagcg 360

caggaaaacg ccgcctggta taccgcgctg gcagatcgta gcgttcaggt cagttgtcag 420

acgccggagc aggcgcagct tccccgctgg gtggccgcca gagcgaaagc acagaatttg 480

caactggatg acgccgcgaa ccaactgctg tgctattgct atgagggcaa tctgttggcg 540

ctggcgcagg cgctggaacg gctgtcgctg ctttggcctg acggtaagtt aacgcttcct 600

cgcgtggagc aggccgtcaa tgacgccgcc cactttaccc ctttccactg ggtggatgcg 660

ttactgatgg ggaaaagcaa acgcgcgctg catatcctgc aacagctacg ccttgaaggc 720

agcgaaccgg ttattctgct gcggaccctc cagcgagagc tgctgctact ggtaaatctc 780

aaacgtcagt ccgcgcatac gccgttacgc gcgctgtttg ataaacaccg ggtctggcaa 840

aaccgtcgcc ccatgatcgg cgacgcgttg cagcgtctcc atcctgcaca gcttcgccag 900

gccgtgcagt tactgacgcg tacagaaatt acgctcaaac aggattatgg tcagtcggta 960

tgggcagatc ttgaagggct gtcgctcctg ctctgccata aggcgctggc agacgtattt 1020

attgatgggt ga 1032

<210> 155
<211> 1032
<212> DNA
<213> Salmonella typhi

<220>
<221> CDS
<222> (1)..(1032)

<400> 155
atg atc agg ttg tat cct gaa caa ctc cgc gcg cag ctc aac gaa ggg 48
Met Ile Arg Leu Tyr Pro Glu Gln Leu Arg Ala Gln Leu Asn Glu Gly
1               5                   10                  15

ctg cgt gcg gca tat cta tta ttg ggc aac gat ccg ctg ctg ctg cag 96
Leu Arg Ala Ala Tyr Leu Leu Leu Gly Asn Asp Pro Leu Leu Leu Gln
            20                  25                  30

gaa agt cag gac gct att cgt ctg gcg gct gcg tcg caa ggc ttc gaa 144
Glu Ser Gln Asp Ala Ile Arg Leu Ala Ala Ala Ser Gln Gly Phe Glu
        35                  40                  45

gag cac cac gct ttt act ctc gat ccc agt acc gac tgg gga tcg ctt 192
Glu His His Ala Phe Thr Leu Asp Pro Ser Thr Asp Trp Gly Ser Leu
    50                  55                  60

ttc tca ctc tgc cag gcg atg agc ctg ttc gcc agc cgc caa acg ctg 240
Phe Ser Leu Cys Gln Ala Met Ser Leu Phe Ala Ser Arg Gln Thr Leu
65                  70                  75                  80

gtg ctg caa ctg ccg gaa aac ggg cct aac gcg gca atg aat gag caa 288
Val Leu Gln Leu Pro Glu Asn Gly Pro Asn Ala Ala Met Asn Glu Gln
                85                  90                  95

ctc gcc acc ctg agc gag cta ctg cat gac gac ctg tta tta ata gta 336
Leu Ala Thr Leu Ser Glu Leu Leu His Asp Asp Leu Leu Leu Ile Val
            100                 105                 110

cgc gga aac aag ctc acc aaa gcg cag gaa aac gcc gcc tgg tat acc 384
Arg Gly Asn Lys Leu Thr Lys Ala Gln Glu Asn Ala Ala Trp Tyr Thr
        115                 120                 125

gcg ctg gca gat cgt agc gtt cag gtc agt tgt cag acg ccg gag cag 432
Ala Leu Ala Asp Arg Ser Val Gln Val Ser Cys Gln Thr Pro Glu Gln
    130                 135                 140

gcg cag ctt ccc cgc tgg gtg gcc gcc aga gcg aaa gca cag aat ttg 480
Ala Gln Leu Pro Arg Trp Val Ala Ala Arg Ala Lys Ala Gln Asn Leu
145                 150                 155                 160

caa ctg gat gac gcc gcg aac caa ctg ctg tgc tat tgc tat gag ggc 528
Gln Leu Asp Asp Ala Ala Asn Gln Leu Leu Cys Tyr Cys Tyr Glu Gly
                165                 170                 175

aat ctg ttg gcg ctg gcg cag gcg ctg gaa cgg ctg tcg ctg ctt tgg 576
Asn Leu Leu Ala Leu Ala Gln Ala Leu Glu Arg Leu Ser Leu Leu Trp
            180                 185                 190

cct gac ggt aag tta acg ctt cct cgc gtg gag cag gcc gtc aat gac 624
Pro Asp Gly Lys Leu Thr Leu Pro Arg Val Glu Gln Ala Val Asn Asp
        195                 200                 205

gcc gcc cac ttt acc cct ttc cac tgg gtg gat gcg tta ctg atg ggg 672
Ala Ala His Phe Thr Pro Phe His Trp Val Asp Ala Leu Leu Met Gly
    210                 215                 220

aaa agc aaa cgc gcg ctg cat atc ctg caa cag cta cgc ctt gaa ggc 720
Lys Ser Lys Arg Ala Leu His Ile Leu Gln Gln Leu Arg Leu Glu Gly
225                 230                 235                 240

agc gaa ccg gtt att ctg ctg cgg acc ctc cag cga gag ctg ctg cta 768
Ser Glu Pro Val Ile Leu Leu Arg Thr Leu Gln Arg Glu Leu Leu Leu
                245                 250                 255

ctg gta aat ctc aaa cgt cag tcc gcg cat acg ccg tta cgc gcg ctg 816
Leu Val Asn Leu Lys Arg Gln Ser Ala His Thr Pro Leu Arg Ala Leu
            260                 265                 270

ttt gat aaa cac cgg gtc tgg caa aac cgt cgc ccc atg atc ggc gac 864
Phe Asp Lys His Arg Val Trp Gln Asn Arg Arg Pro Met Ile Gly Asp
        275                 280                 285

gcg ttg cag cgt ctc cat cct gca cag ctt cgc cag gcc gtg cag tta 912
Ala Leu Gln Arg Leu His Pro Ala Gln Leu Arg Gln Ala Val Gln Leu
    290                 295                 300

ctg acg cgt aca gaa att acg ctc aaa cag gat tat ggt cag tcg gta 960
Leu Thr Arg Thr Glu Ile Thr Leu Lys Gln Asp Tyr Gly Gln Ser Val
305                 310                 315                 320

tgg gca gat ctt gaa ggg ctg tcg ctc ctg ctc tgc cat aag gcg ctg 1008
Trp Ala Asp Leu Glu Gly Leu Ser Leu Leu Leu Cys His Lys Ala Leu
                325                 330                 335

gca gac gta ttt att gat ggg tga 1032
Ala Asp Val Phe Ile Asp Gly
            340

<210> 156
<211> 343
<212> PRT
<213> Salmonella typhi

<400> 156
Met Ile Arg Leu Tyr Pro Glu Gln Leu Arg Ala Gln Leu Asn Glu Gly
1               5                   10                  15

Leu Arg Ala Ala Tyr Leu Leu Leu Gly Asn Asp Pro Leu Leu Leu Gln
            20                  25                  30

Glu Ser Gln Asp Ala Ile Arg Leu Ala Ala Ala Ser Gln Gly Phe Glu
        35                  40                  45

Glu His His Ala Phe Thr Leu Asp Pro Ser Thr Asp Trp Gly Ser Leu
    50                  55                  60

Phe Ser Leu Cys Gln Ala Met Ser Leu Phe Ala Ser Arg Gln Thr Leu
65                  70                  75                  80

Val Leu Gln Leu Pro Glu Asn Gly Pro Asn Ala Ala Met Asn Glu Gln
                85                  90                  95

Leu Ala Thr Leu Ser Glu Leu Leu His Asp Asp Leu Leu Leu Ile Val
            100                 105                 110

Arg Gly Asn Lys Leu Thr Lys Ala Gln Glu Asn Ala Ala Trp Tyr Thr
        115                 120                 125

Ala Leu Ala Asp Arg Ser Val Gln Val Ser Cys Gln Thr Pro Glu Gln
    130                 135                 140

Ala Gln Leu Pro Arg Trp Val Ala Ala Arg Ala Lys Ala Gln Asn Leu
145                 150                 155                 160

Gln Leu Asp Asp Ala Ala Asn Gln Leu Leu Cys Tyr Cys Tyr Glu Gly
                165                 170                 175

Asn Leu Leu Ala Leu Ala Gln Ala Leu Glu Arg Leu Ser Leu Leu Trp
            180                 185                 190

Pro Asp Gly Lys Leu Thr Leu Pro Arg Val Glu Gln Ala Val Asn Asp
        195                 200                 205

Ala Ala His Phe Thr Pro Phe His Trp Val Asp Ala Leu Leu Met Gly
    210                 215                 220

Lys Ser Lys Arg Ala Leu His Ile Leu Gln Gln Leu Arg Leu Glu Gly
225                 230                 235                 240

Ser Glu Pro Val Ile Leu Leu Arg Thr Leu Gln Arg Glu Leu Leu Leu
                245                 250                 255

Leu Val Asn Leu Lys Arg Gln Ser Ala His Thr Pro Leu Arg Ala Leu
            260                 265                 270

Phe Asp Lys His Arg Val Trp Gln Asn Arg Arg Pro Met Ile Gly Asp
        275                 280                 285

Ala Leu Gln Arg Leu His Pro Ala Gln Leu Arg Gln Ala Val Gln Leu
    290                 295                 300

Leu Thr Arg Thr Glu Ile Thr Leu Lys Gln Asp Tyr Gly Gln Ser Val
305                 310                 315                 320

Trp Ala Asp Leu Glu Gly Leu Ser Leu Leu Leu Cys His Lys Ala Leu
                325                 330                 335

Ala Asp Val Phe Ile Asp Gly
            340

<210> 157

<211> 32
<212> DNA
<213> Artificial Sequence

<220>
<223> Description of Artificial Sequence: Primer

<400> 157
gatcctgcag gaaaccacaa tattccagtt cc                                32


<210> 158
<211> 40
<212> DNA
<213> Artificial Sequence

<220>
<223> Description of Artificial Sequence: Primer

<400> 158
gatcaagctt ttattatcca ccatgagaag tatttttcac                        40


<210> 159
<211> 32
<212> DNA
<213> Artificial Sequence

<220>
<223> Description of Artificial Sequence: Primer

<400> 159
gatccatatg gaaaccacaa tattccagtt cc                                32


<210> 160
<211> 40
<212> DNA
<213> Artificial Sequence

<220>
<223> Description of Artificial Sequence: Primer

<400> 160
gatcggtacc ttattatcca ccatgagaag tatttttcac                        40


<210> 161

<211> 32
<212> DNA
<213> Artificial Sequence

<220>
<223> Description of Artificial Sequence: Primer

<400> 161
gatcggtacc cagaggtgat tgtatgccag tc                                32


<210> 162
<211> 34
<212> DNA
<213> Artificial Sequence

<220>
<223> Description of Artificial Sequence: Primer

<400> 162
gatcactagt ttattattct tcatctctct gaag                              34


<210> 163
<211> 29
<212> DNA
<213> Artificial Sequence

<220>
<223> Description of Artificial Sequence: Primer

<400> 163
gatccatatg agaggtgatt gtatgccag                                    29


<210> 164
<211> 35
<212> DNA
<213> Artificial Sequence

<220>
<223> Description of Artificial Sequence: Primer

<400> 164
gatcctgcag gaaaaagttt ttttggaaaa actcc                             35


<210> 165

<211> 36
<212> DNA
<213> Artificial Sequence

<220>
<223> Description of Artificial Sequence: Primer

<400> 165
gatcggtacc ttattaatcc gcctgaacgg ctaacg 36


<210> 166
<211> 35
<212> DNA
<213> Artificial Sequence

<220>
<223> Description of Artificial Sequence: Primer

<400> 166
gatccatatg gaaaaagttt ttttggaaaa actcc 35


<210> 167
<211> 31
<212> DNA
<213> Artificial Sequence

<220>
<223> Description of Artificial Sequence: Primer

<400> 167
gatcctgcag agtatttcag aaaaaccctt g 31


<210> 168
<211> 35
<212> DNA
<213> Artificial Sequence

<220>
<223> Description of Artificial Sequence: Primer

<400> 168
gatcggtacc taatcaaacg ctgaactttt cttcg 35


<210> 169

<211> 27
<212> DNA
<213> Artificial Sequence

<220>
<223> Description of Artificial Sequence: Primer

<400> 169
gatctcatga gtatttcaga aaaaccc                                          27


<210> 170
<211> 33
<212> DNA
<213> Artificial Sequence

<220>
<223> Description of Artificial Sequence: Primer

<400> 170
gatcctgcag aacgatttga tcagaaagta cgc                                   33


<210> 171
<211> 34
<212> DNA
<213> Artificial Sequence

<220>
<223> Description of Artificial Sequence: Primer

<400> 171
gatcggtacc ttatcagctc caagcgttga cacc                                  34


<210> 172
<211> 33
<212> DNA
<213> Artificial Sequence

<220>
<223> Description of Artificial Sequence: Primer

<400> 172
gatccatatg aacgatttga tcagaaagta cgc                                   33


<210> 173

<211> 1161
<212> PRT
<213> Aquifex aeolicus

<400> 173
Met Ser Lys Asp Phe Val His Leu His Leu His Thr Gln Phe Ser Leu
1               5                   10                  15

Leu Asp Gly Ala Ile Lys Ile Asp Glu Leu Val Lys Lys Ala Lys Glu
            20                  25                  30

Tyr Gly Tyr Lys Ala Val Gly Met Ser Asp His Gly Asn Leu Phe Gly
        35                  40                  45

Ser Tyr Lys Phe Tyr Lys Ala Leu Lys Ala Glu Gly Ile Lys Pro Ile
    50                  55                  60

Ile Gly Met Glu Ala Tyr Phe Thr Thr Gly Ser Arg Phe Asp Arg Lys
65                  70                  75                  80

Thr Lys Thr Ser Glu Asp Asn Ile Thr Asp Lys Tyr Asn His His Leu
                85                  90                  95

Ile Leu Ile Ala Lys Asp Asp Lys Gly Leu Lys Asn Leu Met Lys Leu
            100                 105                 110

Ser Thr Leu Ala Tyr Lys Glu Gly Phe Tyr Tyr Lys Pro Arg Ile Asp
        115                 120                 125

Tyr Glu Leu Leu Glu Lys Tyr Gly Glu Gly Leu Ile Ala Leu Thr Ala
    130                 135                 140

Cys Leu Lys Gly Val Pro Thr Tyr Tyr Ala Ser Ile Asn Glu Val Lys
145                 150                 155                 160

Lys Ala Glu Glu Trp Val Lys Lys Phe Lys Asp Ile Phe Gly Asp Asp
                165                 170                 175

Leu Tyr Leu Glu Leu Gln Ala Asn Asn Ile Pro Glu Gln Glu Val Ala
            180                 185                 190

Asn Arg Asn Leu Ile Glu Ile Ala Lys Lys Tyr Asp Val Lys Leu Ile
        195                 200                 205

Ala Thr Gln Asp Ala His Tyr Leu Asn Pro Glu Asp Arg Tyr Ala His
    210                 215                 220

Thr Val Leu Met Ala Leu Gln Met Lys Lys Thr Ile His Glu Leu Ser

225                 230                 235                 240

Ser Gly Asn Phe Lys Cys Ser Asn Glu Asp Leu His Phe Ala Pro Pro
                245                 250                 255

Glu Tyr Met Trp Lys Lys Phe Glu Gly Lys Phe Glu Gly Trp Glu Lys
            260                 265                 270

Ala Leu Leu Asn Thr Leu Glu Val Met Glu Lys Thr Ala Asp Ser Phe
        275                 280                 285

Glu Ile Phe Glu Asn Ser Thr Tyr Leu Leu Pro Lys Tyr Asp Val Pro
    290                 295                 300

Pro Asp Lys Thr Leu Glu Glu Tyr Leu Arg Glu Leu Ala Tyr Lys Gly
305                 310                 315                 320

Leu Arg Gln Arg Ile Glu Arg Gly Gln Ala Lys Asp Thr Lys Glu Tyr
                325                 330                 335

Trp Glu Arg Leu Glu Tyr Glu Leu Glu Val Ile Asn Lys Met Gly Phe
            340                 345                 350

Ala Gly Tyr Phe Leu Ile Val Gln Asp Phe Ile Asn Trp Ala Lys Lys
        355                 360                 365

Asn Asp Ile Pro Val Gly Pro Gly Arg Gly Ser Ala Gly Gly Ser Leu
    370                 375                 380

Val Ala Tyr Ala Ile Gly Ile Thr Asp Val Asp Pro Ile Lys His Gly
385                 390                 395                 400

Phe Leu Phe Glu Arg Phe Leu Asn Pro Glu Arg Val Ser Met Pro Asp
                405                 410                 415

Ile Asp Val Asp Phe Cys Gln Asp Asn Arg Glu Lys Val Ile Glu Tyr
            420                 425                 430

Val Arg Asn Lys Tyr Gly His Asp Asn Val Ala Gln Ile Ile Thr Tyr
        435                 440                 445

Asn Val Met Lys Ala Lys Gln Thr Leu Arg Asp Val Ala Arg Ala Met
    450                 455                 460

Gly Leu Pro Tyr Ser Thr Ala Asp Lys Leu Ala Lys Leu Ile Pro Gln
465                 470                 475                 480

Gly Asp Val Gln Gly Thr Trp Leu Ser Leu Glu Glu Met Tyr Lys Thr

                485                 490                 495

Pro Val Glu Glu Leu Leu Gln Lys Tyr Gly Glu His Arg Thr Asp Ile
            500                 505                 510

Glu Asp Asn Val Lys Lys Phe Arg Gln Ile Cys Glu Glu Ser Pro Glu
        515                 520                 525

Ile Lys Gln Leu Val Glu Thr Ala Leu Lys Leu Glu Gly Leu Thr Arg
    530                 535                 540

His Thr Ser Leu His Ala Ala Gly Val Val Ile Ala Pro Lys Pro Leu
545                 550                 555                 560

Ser Glu Leu Val Pro Leu Tyr Tyr Asp Lys Glu Gly Glu Val Ala Thr
                565                 570                 575

Gln Tyr Asp Met Val Gln Leu Glu Glu Leu Gly Leu Leu Lys Met Asp
            580                 585                 590

Phe Leu Gly Leu Lys Thr Leu Thr Glu Leu Lys Leu Met Lys Glu Leu
        595                 600                 605

Ile Lys Glu Arg His Gly Val Asp Ile Asn Phe Leu Glu Leu Pro Leu
    610                 615                 620

Asp Asp Pro Lys Val Tyr Lys Leu Leu Gln Glu Gly Lys Thr Thr Gly
625                 630                 635                 640

Val Phe Gln Leu Glu Ser Arg Gly Met Lys Glu Leu Leu Lys Lys Leu
                645                 650                 655

Lys Pro Asp Ser Phe Asp Asp Ile Val Ala Val Leu Ala Leu Tyr Arg
            660                 665                 670

Pro Gly Pro Leu Lys Ser Gly Leu Val Asp Thr Tyr Ile Lys Arg Lys
        675                 680                 685

His Gly Lys Glu Pro Val Glu Tyr Pro Phe Pro Glu Leu Glu Pro Val
    690                 695                 700

Leu Lys Glu Thr Tyr Gly Val Ile Val Tyr Gln Glu Gln Val Met Lys
705                 710                 715                 720

Met Ser Gln Ile Leu Ser Gly Phe Thr Pro Gly Glu Ala Asp Thr Leu
                725                 730                 735

Arg Lys Ala Ile Gly Lys Lys Lys Ala Asp Leu Met Ala Gln Met Lys

            740                 745                 750

Asp Lys Phe Ile Gln Gly Ala Val Glu Arg Gly Tyr Pro Glu Glu Lys
        755                 760                 765

Ile Arg Lys Leu Trp Glu Asp Ile Glu Lys Phe Ala Ser Tyr Ser Phe
    770                 775                 780

Asn Lys Ser His Ser Val Ala Tyr Gly Tyr Ile Ser Tyr Trp Thr Ala
785                 790                 795                 800

Tyr Val Lys Ala His Tyr Pro Ala Glu Phe Phe Ala Val Lys Leu Thr
                805                 810                 815

Thr Glu Lys Asn Asp Asn Lys Phe Leu Asn Leu Ile Lys Asp Ala Lys
            820                 825                 830

Leu Phe Gly Phe Glu Ile Leu Pro Pro Asp Ile Asn Lys Ser Asp Val
        835                 840                 845

Gly Phe Thr Ile Glu Gly Glu Asn Arg Ile Arg Phe Gly Leu Ala Arg
    850                 855                 860

Ile Lys Gly Val Gly Glu Glu Thr Ala Lys Ile Ile Val Glu Ala Arg
865                 870                 875                 880

Lys Lys Tyr Lys Gln Phe Lys Gly Leu Ala Asp Phe Ile Asn Lys Thr
                885                 890                 895

Lys Asn Arg Lys Ile Asn Lys Lys Val Val Glu Ala Leu Val Lys Ala
            900                 905                 910

Gly Ala Phe Asp Phe Thr Lys Lys Lys Arg Lys Glu Leu Leu Ala Lys
        915                 920                 925

Val Ala Asn Ser Glu Lys Ala Leu Met Ala Thr Gln Asn Ser Leu Phe
    930                 935                 940

Gly Ala Pro Lys Glu Glu Val Glu Glu Leu Asp Pro Leu Lys Leu Glu
945                 950                 955                 960

Lys Glu Val Leu Gly Phe Tyr Ile Ser Gly His Pro Leu Asp Asn Tyr
                965                 970                 975

Glu Lys Leu Leu Lys Asn Arg Tyr Thr Pro Ile Glu Asp Leu Glu Glu
            980                 985                 990

Trp Asp Lys Glu Ser Glu Ala Val Leu Thr Gly Val Ile Thr Glu Leu

995                 1000                1005

Lys Val Lys Lys Thr Lys Asn Gly Asp Tyr Met Ala Val Phe Asn Leu
    1010                1015                1020

Val Asp Lys Thr Gly Leu Ile Glu Cys Val Val Phe Pro Gly Val Tyr
1025                1030                1035                1040

Glu Glu Ala Lys Glu Leu Ile Glu Glu Asp Arg Val Val Val Val Lys
                1045                1050                1055

Gly Phe Leu Asp Glu Asp Leu Glu Thr Glu Asn Val Lys Phe Val Val
            1060                1065                1070

Lys Glu Val Phe Ser Pro Glu Glu Phe Ala Lys Glu Met Arg Asn Thr
        1075                1080                1085

Leu Tyr Ile Phe Leu Lys Arg Glu Gln Ala Leu Asn Gly Val Ala Glu
    1090                1095                1100

Lys Leu Lys Gly Ile Ile Glu Asn Asn Arg Thr Glu Asp Gly Tyr Asn
1105                1110                1115                1120

Leu Val Leu Thr Val Asp Leu Gly Asp Tyr Phe Val Asp Leu Ala Leu
                1125                1130                1135

Pro Gln Asp Met Lys Leu Lys Ala Asp Arg Lys Val Val Glu Glu Ile
            1140                1145                1150

Glu Lys Leu Gly Val Lys Val Ile Ile
        1155                1160

<210> 174
<211> 363
<212> PRT
<213> Aquifex aeolicus

<400> 174
Met Arg Val Lys Val Asp Arg Glu Glu Leu Glu Glu Val Leu Lys Lys
1               5                   10                  15

Ala Arg Glu Ser Thr Glu Lys Lys Ala Ala Leu Pro Ile Leu Ala Asn
            20                  25                  30

Phe Leu Leu Ser Ala Lys Glu Glu Asn Leu Ile Val Arg Ala Thr Asp
        35                  40                  45

Leu Glu Asn Tyr Leu Val Val Ser Val Lys Gly Glu Val Glu Glu Glu
    50                  55                  60

Gly Glu Val Cys Val His Ser Gln Lys Leu Tyr Asp Ile Val Lys Asn
65                  70                  75                  80

Leu Asn Ser Ala Tyr Val Tyr Leu His Thr Glu Gly Glu Lys Leu Val
                85                  90                  95

Ile Thr Gly Gly Lys Ser Thr Tyr Lys Leu Pro Thr Ala Pro Ala Glu
            100                 105                 110

Asp Phe Pro Glu Phe Pro Glu Ile Val Glu Gly Gly Glu Thr Leu Ser
        115                 120                 125

Gly Asn Leu Leu Val Asn Gly Ile Glu Lys Val Glu Tyr Ala Ile Ala
    130                 135                 140

Lys Glu Glu Ala Asn Ile Ala Leu Gln Gly Met Tyr Leu Arg Gly Tyr
145                 150                 155                 160

Glu Asp Arg Ile His Phe Val Gly Ser Asp Gly His Arg Leu Ala Leu
                165                 170                 175

Tyr Glu Pro Leu Gly Glu Phe Ser Lys Glu Leu Leu Ile Pro Arg Lys
            180                 185                 190

Ser Leu Lys Val Leu Lys Lys Leu Ile Thr Gly Ile Glu Asp Val Asn
        195                 200                 205

Ile Glu Lys Ser Glu Asp Glu Ser Phe Ala Tyr Phe Ser Thr Pro Glu
    210                 215                 220

Trp Lys Leu Ala Val Arg Leu Leu Glu Gly Glu Phe Pro Asp Tyr Met
225                 230                 235                 240

Ser Val Ile Pro Glu Glu Phe Ser Ala Glu Val Leu Phe Glu Thr Glu
                245                 250                 255

Glu Val Leu Lys Val Leu Lys Arg Leu Lys Ala Leu Ser Glu Gly Lys
            260                 265                 270

Val Phe Pro Val Lys Ile Thr Leu Ser Glu Asn Leu Ala Ile Phe Glu
        275                 280                 285

Phe Ala Asp Pro Glu Phe Gly Glu Ala Arg Glu Glu Ile Glu Val Glu
    290                 295                 300

Tyr Thr Gly Glu Pro Phe Glu Ile Gly Phe Asn Gly Lys Tyr Leu Met
305                 310                 315                 320

Glu Ala Leu Asp Ala Tyr Asp Ser Glu Arg Val Trp Phe Lys Phe Thr
                325                 330                 335

Thr Pro Asp Thr Ala Thr Leu Leu Glu Ala Glu Asp Tyr Glu Lys Glu
            340                 345                 350

Pro Tyr Lys Cys Ile Ile Met Pro Met Arg Val
        355                 360

<210> 175
<211> 202
<212> PRT
<213> Aquifex aeolicus

<400> 175
Met Lys Ser Ser Pro Lys Ser Leu Lys Met Arg Asp Asn Leu Leu Asp
  1               5                  10                  15

Gly Thr Phe Val Val Ile Asp Leu Glu Ala Thr Gly Phe Asp Val Glu
             20                  25                  30

Lys Ser Glu Val Ile Asp Leu Ala Ala Val Arg Val Glu Gly Gly Ile
         35                  40                  45

Ile Thr Glu Lys Phe Ser Thr Leu Val Tyr Pro Gly Tyr Phe Ile Pro
     50                  55                  60

Glu Arg Ile Lys Lys Leu Thr Gly Ile Thr Asn Ala Met Leu Val Gly
 65                  70                  75                  80

Gln Pro Thr Ile Glu Glu Val Leu Pro Glu Phe Leu Glu Phe Val Gly
                 85                  90                  95

Asp Asn Ile Val Val Gly His Phe Val Glu Gln Asp Ile Lys Phe Ile
            100                 105                 110

Asn Lys Tyr Thr Lys Gln Tyr Arg Gly Lys Lys Phe Arg Asn Pro Ser
        115                 120                 125

Leu Cys Thr Leu Lys Leu Ala Arg Lys Val Phe Pro Gly Leu Lys Lys
    130                 135                 140

Tyr Ser Leu Lys Glu Ile Ala Glu Asn Phe Gly Phe Glu Thr Asn Gly
145                 150                 155                 160

Val His Arg Ala Leu Lys Asp Ala Thr Leu Thr Ala Glu Ile Phe Ile
                165                 170                 175

Lys Ile Leu Glu Glu Leu Trp Phe Lys Tyr Gly Ile Gly Asp Tyr Tyr
            180                 185                 190

Ser Leu Lys Arg Leu Glu Lys Gly Lys Phe
        195                 200

<210> 176
<211> 473
<212> PRT
<213> Aquifex aeolicus

<400> 176
Met Asn Tyr Val Pro Phe Ala Arg Lys Tyr Arg Pro Lys Phe Phe Arg
  1               5                  10                  15

Glu Val Ile Gly Gln Glu Ala Pro Val Arg Ile Leu Lys Asn Ala Ile
             20                  25                  30

Lys Asn Asp Arg Val Ala His Ala Tyr Leu Phe Ala Gly Pro Arg Gly
         35                  40                  45

Val Gly Lys Thr Thr Ile Ala Arg Ile Leu Ala Lys Ala Leu Asn Cys
     50                  55                  60

Lys Asn Pro Ser Lys Gly Glu Pro Cys Gly Glu Cys Glu Asn Cys Arg
 65                  70                  75                  80

Glu Ile Asp Arg Gly Val Phe Pro Asp Leu Ile Glu Met Asp Ala Ala
                 85                  90                  95

Ser Asn Arg Gly Ile Asp Asp Val Arg Ala Leu Lys Glu Ala Val Asn
            100                 105                 110

Tyr Lys Pro Ile Lys Gly Lys Tyr Lys Val Tyr Ile Ile Asp Glu Ala
        115                 120                 125

His Met Leu Thr Lys Glu Ala Phe Asn Ala Leu Leu Lys Thr Leu Glu
    130                 135                 140

Glu Pro Pro Pro Arg Thr Val Phe Val Leu Cys Thr Thr Glu Tyr Asp
145                 150                 155                 160

Lys Ile Leu Pro Thr Ile Leu Ser Arg Cys Gln Arg Ile Ile Phe Ser

                165                 170                 175

Lys Val Arg Lys Glu Lys Val Ile Glu Tyr Leu Lys Lys Ile Cys Glu
            180                 185                 190

Lys Glu Gly Ile Glu Cys Glu Glu Gly Ala Leu Glu Val Leu Ala His
        195                 200                 205

Ala Ser Glu Gly Cys Met Arg Asp Ala Ala Ser Leu Leu Asp Gln Ala
    210                 215                 220

Ser Val Tyr Gly Glu Gly Arg Val Thr Lys Glu Val Val Glu Asn Phe
225                 230                 235                 240

Leu Gly Ile Leu Ser Gln Glu Ser Val Arg Ser Phe Leu Lys Leu Leu
                245                 250                 255

Leu Asn Ser Glu Val Asp Glu Ala Ile Lys Phe Leu Arg Glu Leu Ser
            260                 265                 270

Glu Lys Gly Tyr Asn Leu Thr Lys Phe Trp Glu Met Leu Glu Glu Glu
        275                 280                 285

Val Arg Asn Ala Ile Leu Val Lys Ser Leu Lys Asn Pro Glu Ser Val
    290                 295                 300

Val Gln Asn Trp Gln Asp Tyr Glu Asp Phe Lys Asp Tyr Pro Leu Glu
305                 310                 315                 320

Ala Leu Leu Tyr Val Glu Asn Leu Ile Asn Arg Gly Lys Val Glu Ala
                325                 330                 335

Arg Thr Arg Glu Pro Leu Arg Ala Phe Glu Leu Ala Val Ile Lys Ser
            340                 345                 350

Leu Ile Val Lys Asp Ile Ile Pro Val Ser Gln Leu Gly Ser Val Val
        355                 360                 365

Lys Glu Thr Lys Lys Glu Glu Lys Lys Val Glu Val Lys Glu Glu Pro
    370                 375                 380

Lys Val Lys Glu Glu Lys Pro Lys Glu Gln Glu Glu Asp Arg Phe Gln
385                 390                 395                 400

Lys Val Leu Asn Ala Val Asp Gly Lys Ile Leu Lys Arg Ile Leu Glu
                405                 410                 415

Gly Ala Lys Arg Glu Glu Arg Asp Gly Lys Ile Val Leu Lys Ile Glu

            420                 425                 430

Ala Ser Tyr Leu Arg Thr Met Lys Lys Glu Phe Asp Ser Leu Lys Glu
        435                 440                 445

Thr Phe Pro Phe Leu Glu Phe Glu Pro Val Glu Asp Lys Lys Lys Pro
    450                 455                 460

Gln Lys Ser Ser Gly Thr Arg Leu Phe
465                 470

<210> 177
<211> 147
<212> PRT
<213> Aquifex aeolicus

<400> 177
Met Leu Asn Lys Val Phe Ile Ile Gly Arg Leu Thr Gly Asp Pro Val
1               5                   10                  15

Ile Thr Tyr Leu Pro Ser Gly Thr Pro Val Val Glu Phe Thr Leu Ala
            20                  25                  30

Tyr Asn Arg Arg Tyr Lys Asn Gln Asn Gly Glu Phe Gln Glu Glu Ser
        35                  40                  45

His Phe Phe Asp Val Lys Ala Tyr Gly Lys Met Ala Glu Asp Trp Ala
    50                  55                  60

Thr Arg Phe Ser Lys Gly Tyr Leu Val Leu Val Glu Gly Arg Leu Ser
65                  70                  75                  80

Gln Glu Lys Trp Glu Lys Glu Gly Lys Lys Phe Ser Lys Val Arg Ile
                85                  90                  95

Ile Ala Glu Asn Val Arg Leu Ile Asn Arg Pro Lys Gly Ala Glu Leu
            100                 105                 110

Gln Ala Glu Glu Glu Glu Glu Val Pro Pro Ile Glu Glu Glu Ile Glu
        115                 120                 125

Lys Leu Gly Lys Glu Glu Glu Lys Pro Phe Thr Asp Glu Glu Asp Glu
    130                 135                 140

Ile Pro Phe
145

<210> 178
<211> 478
<212> PRT
<213> Thermotoga maritima

<400> 178
Met Glu Val Leu Tyr Arg Lys Tyr Arg Pro Lys Thr Phe Ser Glu Val
1               5                   10                  15

Val Asn Gln Asp His Val Lys Lys Ala Ile Ile Gly Ala Ile Gln Lys
            20                  25                  30

Asn Ser Val Ala His Gly Tyr Ile Phe Ala Gly Pro Arg Gly Thr Gly
        35                  40                  45

Lys Thr Thr Leu Ala Arg Ile Leu Ala Lys Ser Leu Asn Cys Glu Asn
    50                  55                  60

Arg Lys Gly Val Glu Pro Cys Asn Ser Cys Arg Ala Cys Arg Glu Ile
65                  70                  75                  80

Asp Glu Gly Thr Phe Met Asp Val Ile Glu Leu Asp Ala Ala Ser Asn
                85                  90                  95

Arg Gly Ile Asp Glu Ile Arg Arg Ile Arg Asp Ala Val Gly Tyr Arg
            100                 105                 110

Pro Met Glu Gly Lys Tyr Lys Val Tyr Ile Ile Asp Glu Val His Met
        115                 120                 125

Leu Thr Lys Glu Ala Phe Asn Ala Leu Leu Lys Thr Leu Glu Glu Pro
    130                 135                 140

Pro Ser His Val Val Phe Val Leu Ala Thr Thr Asn Leu Glu Lys Val
145                 150                 155                 160

Pro Pro Thr Ile Ile Ser Arg Cys Gln Val Phe Glu Phe Arg Asn Ile
                165                 170                 175

Pro Asp Glu Leu Ile Glu Lys Arg Leu Gln Glu Val Ala Glu Ala Glu
            180                 185                 190

Gly Ile Glu Ile Asp Arg Glu Ala Leu Ser Phe Ile Ala Lys Arg Ala
        195                 200                 205

Ser Gly Gly Leu Arg Asp Ala Leu Thr Met Leu Glu Gln Val Trp Lys
    210                 215                 220

Phe Ser Glu Gly Lys Ile Asp Leu Glu Thr Val His Arg Ala Leu Gly
225                 230                 235                 240

Leu Ile Pro Ile Gln Val Val Arg Asp Tyr Val Asn Ala Ile Phe Ser
                245                 250                 255

Gly Asp Val Lys Arg Val Phe Thr Val Leu Asp Asp Val Tyr Tyr Ser
            260                 265                 270

Gly Lys Asp Tyr Glu Val Leu Ile Gln Glu Ala Val Glu Asp Leu Val
        275                 280                 285

Glu Asp Leu Glu Arg Glu Arg Gly Val Tyr Gln Val Ser Ala Asn Asp
    290                 295                 300

Ile Val Gln Val Ser Arg Gln Leu Leu Asn Leu Leu Arg Glu Ile Lys
305                 310                 315                 320

Phe Ala Glu Glu Lys Arg Leu Val Cys Lys Val Gly Ser Ala Tyr Ile
                325                 330                 335

Ala Thr Arg Phe Ser Thr Thr Asn Val Gln Glu Asn Asp Val Arg Glu
            340                 345                 350

Lys Asn Asp Asn Ser Asn Val Gln Gln Lys Glu Glu Lys Lys Glu Thr
        355                 360                 365

Val Lys Ala Lys Glu Glu Lys Gln Glu Asp Ser Glu Phe Glu Lys Arg
    370                 375                 380

Phe Lys Glu Leu Met Glu Glu Leu Lys Glu Lys Gly Asp Leu Ser Ile
385                 390                 395                 400

Phe Val Ala Leu Ser Leu Ser Glu Val Gln Phe Asp Gly Glu Lys Val
                405                 410                 415

Ile Ile Ser Phe Asp Ser Ser Lys Ala Met His Tyr Glu Leu Met Lys
            420                 425                 430

Lys Lys Leu Pro Glu Leu Glu Asn Ile Phe Ser Arg Lys Leu Gly Lys
        435                 440                 445

Lys Val Glu Val Glu Leu Arg Leu Met Gly Lys Glu Glu Thr Ile Glu
    450                 455                 460

Lys Val Ser Gln Lys Ile Leu Arg Leu Phe Glu Gln Glu Gly
465                 470                 475

<210> 179
<211> 366
<212> PRT
<213> Thermotoga maritima

<400> 179
Met Lys Val Thr Val Thr Thr Leu Glu Leu Lys Asp Lys Ile Thr Ile
1               5                   10                  15

Ala Ser Lys Ala Leu Ala Lys Lys Ser Val Lys Pro Ile Leu Ala Gly
            20                  25                  30

Phe Leu Phe Glu Val Lys Asp Gly Asn Phe Tyr Ile Cys Ala Thr Asp
        35                  40                  45

Leu Glu Thr Gly Val Lys Ala Thr Val Asn Ala Ala Glu Ile Ser Gly
    50                  55                  60

Glu Ala Arg Phe Val Val Pro Gly Asp Val Ile Gln Lys Met Val Lys
65                  70                  75                  80

Val Leu Pro Asp Glu Ile Thr Glu Leu Ser Leu Glu Gly Asp Ala Leu
                85                  90                  95

Val Ile Ser Ser Gly Ser Thr Val Phe Arg Ile Thr Thr Met Pro Ala
            100                 105                 110

Asp Glu Phe Pro Glu Ile Thr Pro Ala Glu Ser Gly Ile Thr Phe Glu
        115                 120                 125

Val Asp Thr Ser Leu Leu Glu Glu Met Val Glu Lys Val Ile Phe Ala
    130                 135                 140

Ala Ala Lys Asp Glu Phe Met Arg Asn Leu Asn Gly Val Phe Trp Glu
145                 150                 155                 160

Leu His Lys Asn Leu Leu Arg Leu Val Ala Ser Asp Gly Phe Arg Leu
                165                 170                 175

Ala Leu Ala Glu Glu Gln Ile Glu Asn Glu Glu Glu Ala Ser Phe Leu
            180                 185                 190

Leu Ser Leu Lys Ser Met Lys Glu Val Gln Asn Val Leu Asp Asn Thr
        195                 200                 205

Thr Glu Pro Thr Ile Thr Val Arg Tyr Asp Gly Arg Arg Val Ser Leu

210                 215                 220

Ser Thr Asn Asp Val Glu Thr Val Met Arg Val Val Asp Ala Glu Phe
225                 230                 235                 240

Pro Asp Tyr Lys Arg Val Ile Pro Glu Thr Phe Lys Thr Lys Val Val
                245                 250                 255

Val Ser Arg Lys Glu Leu Arg Glu Ser Leu Lys Arg Val Met Val Ile
            260                 265                 270

Ala Ser Lys Gly Ser Glu Ser Val Lys Phe Glu Ile Glu Glu Asn Val
        275                 280                 285

Met Arg Leu Val Ser Lys Ser Pro Asp Tyr Gly Glu Val Val Asp Glu
    290                 295                 300

Val Glu Val Gln Lys Glu Gly Glu Asp Leu Val Ile Ala Phe Asn Pro
305                 310                 315                 320

Lys Phe Ile Glu Asp Val Leu Lys His Ile Glu Thr Glu Glu Ile Glu
                325                 330                 335

Met Asn Phe Val Asp Ser Thr Ser Pro Cys Gln Ile Asn Pro Leu Asp
            340                 345                 350

Ile Ser Gly Tyr Leu Tyr Ile Val Met Pro Ile Arg Leu Ala
        355                 360                 365

<210> 180
<211> 189
<212> PRT
<213> Thermotoga maritima

<400> 180
Met Leu Ala Met Ile Trp Asn Asp Thr Val Phe Cys Val Val Asp Thr
1               5                   10                  15

Glu Thr Thr Gly Thr Asp Pro Phe Ala Gly Asp Arg Ile Val Glu Ile
            20                  25                  30

Ala Ala Val Pro Val Phe Lys Gly Lys Ile Tyr Arg Asn Lys Ala Phe
        35                  40                  45

His Ser Leu Val Asn Pro Arg Ile Arg Ile Pro Ala Leu Ile Gln Lys
    50                  55                  60

Val His Gly Ile Ser Asn Met Asp Ile Val Glu Ala Pro Asp Met Asp
65                  70                  75                  80

Thr Val Tyr Asp Leu Phe Arg Asp Tyr Val Lys Gly Thr Val Leu Val
                85                  90                  95

Phe His Asn Ala Asn Phe Asp Leu Thr Phe Leu Asp Met Met Ala Lys
            100                 105                 110

Glu Thr Gly Asn Phe Pro Ile Thr Asn Pro Tyr Ile Asp Thr Leu Asp
        115                 120                 125

Leu Ser Glu Glu Ile Phe Gly Arg Pro His Ser Leu Lys Trp Leu Ser
    130                 135                 140

Glu Arg Leu Gly Ile Lys Thr Thr Ile Arg His Arg Ala Leu Pro Asp
145                 150                 155                 160

Ala Leu Val Thr Ala Arg Val Phe Val Lys Leu Val Glu Phe Leu Gly
                165                 170                 175

Glu Asn Arg Val Asn Glu Phe Ile Arg Gly Lys Arg Gly
            180                 185

<210> 181
<211> 1367
<212> PRT
<213> Thermotoga maritima

<400> 181
Met Lys Lys Ile Glu Asn Leu Lys Trp Lys Asn Val Ser Phe Lys Ser
1               5                   10                  15

Leu Glu Ile Asp Pro Asp Ala Gly Val Val Leu Val Ser Val Glu Lys
            20                  25                  30

Phe Ser Glu Glu Ile Glu Asp Leu Val Arg Leu Leu Glu Lys Lys Thr
        35                  40                  45

Arg Phe Arg Val Ile Val Asn Gly Val Gln Lys Ser Asn Gly Asp Leu
    50                  55                  60

Arg Gly Lys Ile Leu Ser Leu Leu Asn Gly Asn Val Pro Tyr Ile Lys
65                  70                  75                  80

Asp Val Val Phe Glu Gly Asn Arg Leu Ile Leu Lys Val Leu Gly Asp
                85                  90                  95

Phe Ala Arg Asp Arg Ile Ala Ser Lys Leu Arg Ser Thr Lys Lys Gln
            100                 105                 110

Leu Asp Glu Leu Leu Pro Pro Gly Thr Glu Ile Met Leu Glu Val Val
        115                 120                 125

Glu Pro Pro Glu Asp Leu Leu Lys Lys Glu Val Pro Gln Pro Glu Lys
    130                 135                 140

Arg Glu Glu Pro Lys Gly Glu Glu Leu Lys Ile Glu Asp Glu Asn His
145                 150                 155                 160

Ile Phe Gly Gln Lys Pro Arg Lys Ile Val Phe Thr Pro Ser Lys Ile
                165                 170                 175

Phe Glu Tyr Asn Lys Lys Thr Ser Val Lys Gly Lys Ile Phe Lys Ile
            180                 185                 190

Glu Lys Ile Glu Gly Lys Arg Thr Val Leu Leu Ile Tyr Leu Thr Asp
        195                 200                 205

Gly Glu Asp Ser Leu Ile Cys Lys Val Phe Asn Asp Val Glu Lys Val
    210                 215                 220

Glu Gly Lys Val Ser Val Gly Asp Val Ile Val Ala Thr Gly Asp Leu
225                 230                 235                 240

Leu Leu Glu Asn Gly Glu Pro Thr Leu Tyr Val Lys Gly Ile Thr Lys
                245                 250                 255

Leu Pro Glu Ala Lys Arg Met Asp Lys Ser Pro Val Lys Arg Val Glu
            260                 265                 270

Leu His Ala His Thr Lys Phe Ser Asp Gln Asp Ala Ile Thr Asp Val
        275                 280                 285

Asn Glu Tyr Val Lys Arg Ala Lys Glu Trp Gly Phe Pro Ala Ile Ala
    290                 295                 300

Leu Thr Asp His Gly Asn Val Gln Ala Ile Pro Tyr Phe Tyr Asp Ala
305                 310                 315                 320

Ala Lys Glu Ala Gly Ile Lys Pro Ile Phe Gly Ile Glu Ala Tyr Leu
                325                 330                 335

Val Ser Asp Val Glu Pro Val Ile Arg Asn Leu Ser Asp Asp Ser Thr
            340                 345                 350

Phe Gly Asp Ala Thr Phe Val Val Leu Asp Phe Glu Thr Thr Gly Leu
        355                 360                 365

Asp Pro Gln Val Asp Glu Ile Ile Glu Ile Gly Ala Val Lys Ile Gln
    370                 375                 380

Gly Gly Gln Ile Val Asp Glu Tyr His Thr Leu Ile Lys Pro Ser Arg
385                 390                 395                 400

Glu Ile Ser Arg Lys Ser Ser Glu Ile Thr Gly Ile Thr Gln Glu Met
                405                 410                 415

Leu Glu Asn Lys Arg Ser Ile Glu Glu Val Leu Pro Glu Phe Leu Gly
            420                 425                 430

Phe Leu Glu Asp Ser Ile Ile Val Ala His Asn Ala Asn Phe Asp Tyr
        435                 440                 445

Arg Phe Leu Arg Leu Trp Ile Lys Lys Val Met Gly Leu Asp Trp Glu
    450                 455                 460

Arg Pro Tyr Ile Asp Thr Leu Ala Leu Ala Lys Ser Leu Leu Lys Leu
465                 470                 475                 480

Arg Ser Tyr Ser Leu Asp Ser Val Val Glu Lys Leu Gly Leu Gly Pro
                485                 490                 495

Phe Arg His His Arg Ala Leu Asp Asp Ala Arg Val Thr Ala Gln Val
            500                 505                 510

Phe Leu Arg Phe Val Glu Met Met Lys Lys Ile Gly Ile Thr Lys Leu
        515                 520                 525

Ser Glu Met Glu Lys Leu Lys Asp Thr Ile Asp Tyr Thr Ala Leu Lys
    530                 535                 540

Pro Phe His Cys Thr Ile Leu Val Gln Asn Lys Lys Gly Leu Lys Asn
545                 550                 555                 560

Leu Tyr Lys Leu Val Ser Asp Ser Tyr Ile Lys Tyr Phe Tyr Gly Val
                565                 570                 575

Pro Arg Ile Leu Lys Ser Glu Leu Ile Glu Asn Arg Glu Gly Leu Leu
            580                 585                 590

Val Gly Ser Ala Cys Ile Ser Gly Glu Leu Gly Arg Ala Ala Leu Glu
        595                 600                 605

Gly Ala Ser Asp Ser Glu Leu Glu Glu Ile Ala Lys Phe Tyr Asp Tyr
    610                 615                 620

Ile Glu Val Met Pro Leu Asp Val Ile Ala Glu Asp Glu Glu Asp Leu
625                 630                 635                 640

Asp Arg Glu Arg Leu Lys Glu Val Tyr Arg Lys Leu Tyr Arg Ile Ala
                645                 650                 655

Lys Lys Leu Asn Lys Phe Val Val Met Thr Gly Asp Val His Phe Leu
            660                 665                 670

Asp Pro Glu Asp Ala Arg Gly Arg Ala Ala Leu Leu Ala Pro Gln Gly
        675                 680                 685

Asn Arg Asn Phe Glu Asn Gln Pro Ala Leu Tyr Leu Arg Thr Thr Glu
    690                 695                 700

Glu Met Leu Glu Lys Ala Ile Glu Ile Phe Glu Asp Glu Glu Ile Ala
705                 710                 715                 720

Arg Glu Val Val Ile Glu Asn Pro Asn Arg Ile Ala Asp Met Ile Glu
                725                 730                 735

Glu Val Gln Pro Leu Glu Lys Lys Leu His Pro Pro Ile Ile Glu Asn
            740                 745                 750

Ala Asp Glu Ile Val Arg Asn Leu Thr Met Lys Arg Ala Tyr Glu Ile
        755                 760                 765

Tyr Gly Asp Pro Leu Pro Glu Ile Val Gln Lys Arg Val Glu Lys Glu
    770                 775                 780

Leu Asn Ala Ile Ile Asn His Gly Tyr Ala Val Leu Tyr Leu Ile Ala
785                 790                 795                 800

Gln Glu Leu Val Gln Lys Ser Met Ser Asp Gly Tyr Val Val Gly Ser
                805                 810                 815

Arg Gly Ser Val Gly Ser Ser Leu Val Ala Asn Leu Leu Gly Ile Thr
            820                 825                 830

Glu Val Asn Pro Leu Pro Pro His Tyr Arg Cys Pro Glu Cys Lys Tyr
        835                 840                 845

Phe Glu Val Val Glu Asp Asp Arg Tyr Gly Ala Gly Tyr Asp Leu Pro
    850                 855                 860

Asn Lys Asn Cys Pro Arg Cys Gly Ala Pro Leu Arg Lys Asp Gly His
865                 870                 875                 880

Gly Ile Pro Phe Glu Thr Phe Met Gly Phe Glu Gly Asp Lys Val Pro
                885                 890                 895

Asp Ile Asp Leu Asn Phe Ser Gly Glu Tyr Gln Glu Arg Ala His Arg
            900                 905                 910

Phe Val Glu Glu Leu Phe Gly Lys Asp His Val Tyr Arg Ala Gly Thr
        915                 920                 925

Ile Asn Thr Ile Ala Glu Arg Ser Ala Val Gly Tyr Val Arg Ser Tyr
    930                 935                 940

Glu Glu Lys Thr Gly Lys Lys Leu Arg Lys Ala Glu Met Glu Arg Leu
945                 950                 955                 960

Val Ser Met Ile Thr Gly Val Lys Arg Thr Thr Gly Gln His Pro Gly
                965                 970                 975

Gly Leu Met Ile Ile Pro Lys Asp Lys Glu Val Tyr Asp Phe Thr Pro
            980                 985                 990

Ile Gln Tyr Pro Ala Asn Asp Arg Asn Ala Gly Val Phe Thr Thr His
        995                 1000                1005

Phe Ala Tyr Glu Thr Ile His Asp Asp Leu Val Lys Ile Asp Ala Leu
    1010                1015                1020

Gly His Asp Asp Pro Thr Phe Ile Lys Met Leu Lys Asp Leu Thr Gly
1025                1030                1035                1040

Ile Asp Pro Met Thr Ile Pro Met Asp Asp Pro Asp Thr Leu Ala Ile
                1045                1050                1055

Phe Ser Ser Val Lys Pro Leu Gly Val Asp Pro Val Glu Leu Glu Ser
            1060                1065                1070

Asp Val Gly Thr Tyr Gly Ile Pro Glu Phe Gly Thr Glu Phe Val Arg
        1075                1080                1085

Gly Met Leu Val Glu Thr Arg Pro Lys Ser Phe Ala Glu Leu Val Arg
    1090                1095                1100

Ile Ser Gly Leu Ser His Gly Thr Asp Val Trp Leu Asn Asn Ala Arg
1105                1110                1115                1120

Asp Trp Ile Asn Leu Gly Tyr Ala Lys Leu Ser Glu Val Ile Ser Cys
                1125                1130                1135

Arg Asp Asp Ile Met Asn Phe Leu Ile His Lys Gly Met Glu Pro Ser
            1140                1145                1150

Leu Ala Phe Lys Ile Met Glu Asn Val Arg Lys Gly Lys Gly Ile Thr
        1155                1160                1165

Glu Glu Met Glu Ser Glu Met Arg Arg Leu Lys Val Pro Glu Trp Phe
    1170                1175                1180

Ile Glu Ser Cys Lys Arg Ile Lys Tyr Leu Phe Pro Lys Ala His Ala
1185                1190                1195                1200

Val Ala Tyr Val Ser Met Ala Phe Arg Ile Ala Tyr Phe Lys Val His
                1205                1210                1215

Tyr Pro Leu Gln Phe Tyr Ala Ala Tyr Phe Thr Ile Lys Gly Asp Gln
            1220                1225                1230

Phe Asp Pro Val Leu Val Leu Arg Gly Lys Glu Ala Ile Lys Arg Arg
        1235                1240                1245

Leu Arg Glu Leu Lys Ala Met Pro Ala Lys Asp Ala Gln Lys Lys Asn
    1250                1255                1260

Glu Val Ser Val Leu Glu Val Ala Leu Glu Met Ile Leu Arg Gly Phe
1265                1270                1275                1280

Ser Phe Leu Pro Pro Asp Ile Phe Lys Ser Asp Ala Lys Lys Phe Leu
                1285                1290                1295

Ile Glu Gly Asn Ser Leu Arg Ile Pro Phe Asn Lys Leu Pro Gly Leu
            1300                1305                1310

Gly Asp Ser Val Ala Glu Ser Ile Ile Arg Ala Arg Glu Glu Lys Pro
        1315                1320                1325

Phe Thr Ser Val Glu Asp Leu Met Lys Arg Thr Lys Val Asn Lys Asn
    1330                1335                1340

His Ile Glu Leu Met Lys Ser Leu Gly Val Leu Gly Asp Leu Pro Glu
1345                1350                1355                1360

Thr Glu Gln Phe Thr Leu Phe
                1365

<210> 182
<211> 842
<212> PRT
<213> Thermotoga maritima

<400> 182
Met Ile Pro Trp Val Ile Ser Pro Tyr Ser Phe Asp Gly Ser Val Val
1               5                   10                  15

Arg Phe Glu Lys Leu Ala Leu Leu Leu Lys Arg Lys Gly Leu Lys Ser
            20                  25                  30

Val Ile Leu Ala Asp Arg Asn Phe His Ala Ala Val Lys Phe Asn Thr
        35                  40                  45

Ile Met Arg Lys His Gly Leu Ile Pro Val His Gly Leu Trp Lys Asp
    50                  55                  60

Gly Arg Ile Phe Val Ala Arg Asn Arg Glu Glu Phe Asp Ser Leu Val
65                  70                  75                  80

Arg Tyr Tyr Asn Gly Glu Thr His Glu Ile Glu Asp Ile Pro Val Phe
                85                  90                  95

Gln Glu Ser Glu Leu Thr Pro Val Arg Tyr Leu Asp Ala Ser Glu Lys
            100                 105                 110

Lys Ala Ser Ile Phe Met Arg Lys Ile Phe Gly Leu Asp Glu Asp Val
        115                 120                 125

Gln Gly Phe Pro Glu Lys Cys Glu Asp Val Ala Asp Ile Leu Asn Ala
    130                 135                 140

Glu Ala Tyr Asp Leu Arg Val Asn His Arg Phe Pro Thr Pro Pro Lys
145                 150                 155                 160

Asn Trp Asn Glu Leu Leu Ile Lys Lys Ala Glu Pro Leu Gly Glu Glu
                165                 170                 175

Tyr Ile Ser Arg Leu Lys Arg Glu Leu Glu Val Ile Lys Arg Lys Gly
            180                 185                 190

Phe Thr Pro Tyr Ile Tyr Thr Val Glu Lys Val Val Glu Ile Ala Lys
        195                 200                 205

Lys Met Gly Ile Lys Val Gly Pro Gly Arg Gly Ser Ala Val Gly Ser

210                 215                 220

Leu Val Ala Tyr Leu Cys Gly Ile Thr Glu Val Asp Pro Ile Lys Tyr
225                 230                 235                 240

Asp Leu Leu Phe Glu Arg Phe Leu Asn Glu Glu Arg Gln Glu Pro Pro
                245                 250                 255

Asp Ile Asp Val Asp Val Glu Asp Arg Arg Arg Lys Asp Leu Ile Lys
            260                 265                 270

Glu Leu Ser Lys Ser Phe Gln Val Tyr Gln Val Ser Thr Phe Gly Asn
        275                 280                 285

Leu Thr Glu Lys Ser Leu Lys Asn Leu Ile Asn Ser Val Leu Pro Asp
    290                 295                 300

Ala Ser Leu Glu Glu Lys Asn Glu Ile Tyr Lys Thr Val Tyr Gly Leu
305                 310                 315                 320

Pro His His Pro Ser Val His Ala Ala Gly Val Val Ile Ser Glu Asn
                325                 330                 335

Pro Leu Pro Leu Pro Thr Arg Thr Glu Glu Asp Ile Pro Ile Thr Asp
            340                 345                 350

Tyr Asp Met Tyr Asp Leu Gln Glu Ile Gly Val Val Lys Ile Asp Ile
        355                 360                 365

Leu Gly Leu Lys Thr Leu Ser Phe Ile Lys Asp Phe Lys Lys Glu Ile
    370                 375                 380

Phe Asp Tyr Ser Asp Glu Lys Thr Tyr His Leu Ile Ser Lys Gly Lys
385                 390                 395                 400

Thr Leu Gly Val Phe Gln Leu Glu Gly Leu Gln Ala Arg Lys Leu Cys
                405                 410                 415

Arg Arg Ile Ser Pro Arg Asn Met Asp Glu Leu Ser Ile Leu Leu Ala
            420                 425                 430

Leu Asn Arg Pro Gly Pro Leu Arg Ser Gly Leu Asp Val Met Phe Ser
        435                 440                 445

Asn Ser Lys Asn Val Pro Ala Phe Phe Arg Lys Met Phe Pro Glu Thr
    450                 455                 460

Arg Gly Val Leu Ile Tyr Gln Glu Gln Ile Met Arg Leu Ala Met Phe

465                 470                 475                 480

Ala Gly Leu Ser Gly Thr Glu Ala Asp Ile Leu Arg Arg Ala Ile Ala
                485                 490                 495

Lys Lys Glu Arg Glu Lys Met Glu Pro Leu Leu Glu Lys Met Lys Lys
            500                 505                 510

Gly Leu Leu Glu Lys Gly Met Glu Asn Ala Glu Gln Ile Leu Glu Ile
        515                 520                 525

Leu Leu Asn Phe Ser Ser Tyr Ala Phe Asn Lys Ser His Ser Val Ala
    530                 535                 540

Tyr Ala His Ile Thr Tyr Gln Thr Ala Tyr Leu Lys Ala His His Leu
545                 550                 555                 560

Glu Glu Phe Phe Lys Leu Tyr Phe Ala Tyr Asn Ser Ser Asp Ala Gly
                565                 570                 575

Lys Ile Phe Leu Ala Val Gln Glu Leu Arg Asn Glu Gly Tyr Arg Val
            580                 585                 590

His Pro Pro Asp Ile Asn Ile Ser Gly Lys Asp Leu Val Phe His Gly
        595                 600                 605

Lys Asp Val Tyr Leu Pro Leu Thr Val Val Lys Gly Val Gly Val Thr
    610                 615                 620

Leu Val Glu Gln Ile Glu Lys Ile Arg Pro Val Ser Ser Val Arg Glu
625                 630                 635                 640

Leu Gln Glu Arg Val Thr Gly Val Pro Arg Asn Val Val Glu Ser Leu
                645                 650                 655

Ile Thr Ala Gly Ala Phe Asp Lys Leu Tyr Glu Asn Arg Lys Leu Ala
            660                 665                 670

Leu Glu Glu Leu Asn Lys Arg Val Glu Lys Asp Ile Leu Glu Ile Arg
        675                 680                 685

Ser Leu Phe Gly Glu Lys Val Glu Gln Glu Ser Ser Asn Ile Lys Ile
    690                 695                 700

Gly Asp Ile Thr Glu Leu Glu Glu Lys Ser Met Gly Phe Pro Leu Thr
705                 710                 715                 720

Pro Val His Glu Val Pro Thr Gly Leu Phe Ala Arg Ile Asp Asp Val

725                 730                 735

Phe Thr Tyr Gly Arg Ile Leu Pro Val Leu Val Lys Arg Val Ser Arg
            740                 745                 750

Asn Ile Val Thr Asp Gly Leu Ser Val Cys Arg Val Arg Thr Asp Val
        755                 760                 765

Pro Asp Gly Val His Leu Val Leu Leu Ser Pro Leu Gln Lys Ile Ile
    770                 775                 780

Lys Ile Trp Pro Phe Asn Glu Asn Thr Arg Phe Val Tyr Arg Val Asp
785                 790                 795                 800

Phe Thr Ala Thr Leu Glu Lys Ala Gly Gln Asn Glu Ile Thr Glu Val
                805                 810                 815

Leu Lys Asn Gly Ala Val Val Arg Tyr Glu Gly Tyr Arg Pro Leu Thr
            820                 825                 830

Asp Glu Tyr Arg Tyr Arg Val Val Pro Arg
        835                 840


<210> 183
<211> 141
<212> PRT
<213> Thermotoga maritima

<400> 183
Met Ser Phe Phe Asn Lys Ile Ile Leu Ile Gly Arg Leu Val Arg Asp
1               5                   10                  15

Pro Glu Glu Arg Tyr Thr Leu Ser Gly Thr Pro Val Thr Thr Phe Thr
            20                  25                  30

Ile Ala Val Asp Arg Val Pro Arg Lys Asn Ala Pro Asp Asp Ala Gln
        35                  40                  45

Thr Thr Asp Phe Phe Arg Ile Val Thr Phe Gly Arg Leu Ala Glu Phe
    50                  55                  60

Ala Arg Thr Tyr Leu Thr Lys Gly Arg Leu Val Leu Val Glu Gly Glu
65                  70                  75                  80

Met Arg Met Arg Arg Trp Glu Thr Pro Thr Gly Glu Lys Arg Val Ser
                85                  90                  95

Pro Glu Val Val Ala Asn Val Val Arg Phe Met Asp Arg Lys Pro Ala
            100                 105                 110

Glu Thr Val Ser Glu Thr Glu Glu Glu Leu Glu Ile Pro Glu Glu Asp
        115                 120                 125

Phe Ser Ser Asp Thr Phe Ser Glu Asp Glu Pro Pro Phe
    130                 135                 140

<210> 184
<211> 250
<212> DNA
<213> Artificial Sequence

<220>
<223> Description of Artificial Sequence: PCR product

<400> 184
gaattcggat cccgggtcta gaggaggtta attaacatat gaaaaaaaaa accaggttgc 60

tagcggtacc actagtggtg gtggtggtct ggttccgcgt ggttcccacc accaccacct 120

gcaccacatc ctggacgctc agaaaatggt ttggaaccac tgccgttgat gagcagttag 180

agaaagaacc catagtagga gcagaaactt tctaggtcga cagcttggct gttttggcgg 240

atgagagaag                                                        250

<210> 185
<211> 250
<212> DNA
<213> Artificial Sequence

<220>
<223> Description of Artificial Sequence: PCR product

<220>
<221> misc_feature
<222> Complement((1)..(250))

<400> 185
cttctctcat ccgccaaaac agccaagctg tcgacctaga aagtttctgc tcctactatg 60

ggttctttct ctaactgctc atcaacggca gtggttccaa accattttct gagcgtccag 120

gatgtggtgc aggtggtggt ggtgggaacc acgcggaacc agaccaccac caccactagt 180

ggtaccgcta gcaacctggt tttttttttc atatgttaat taacctcctc tagacccggg 240

atccgaattc 250

<210> 186
<211> 200
<212> DNA
<213> Artificial Sequence

<220>
<223> Description of Artificial Sequence: PCR product

<400> 186
gaattcggat cccgggtcta gaggaggtaa taacatatgg ctggttgcct gaacgacatc 60

ttcgaagctc agaaaatcga atggcaccat caccatcacc atctggttcc gcgtggttcc 120

ggcggcggcg gcctgcaggt acccaaaaat gcatgagctc gctagcaagc ttaaaaaaaa 180

aactagtgtc gacagcttgg 200

<210> 187
<211> 200
<212> DNA
<213> Artificial Sequence

<220>
<223> Description of Artificial Sequence: PCR product

<220>
<221> misc_feature
<222> Complement((1)..(200))

<400> 187
ccaagctgtc gacactagtt ttttttttaa gcttgctagc gagctcatgc atttttgggt 60

acctgcaggc cgccgccgcc ggaaccacgc ggaaccagat ggtgatggtg atggtgccat 120

tcgattttct gagcttcgaa gatgtcgttc aggcaaccag ccatatgtta ttacctcctc 180

tagacccggg atccgaattc 200

<210> 188
<211> 200

<212> DNA
<213> Artificial Sequence

<220>
<223> Description of Artificial Sequence: PCR product

<400> 188
gaattcggat cccgggtcta gaggaggtaa taacatatgg ctggttgcct gaacgacatc 60

ttcgaagctc agaaaatcga atggcaccat caccatcacc atctggttcc gcgtggttcc 120

ggcggcggcg gcctgcagaa cctaggaaaa aaaaaggtac caaaaaaaaa ggccggccac 180

tagtgtcgac agcttggctg 200


<210> 189
<211> 200
<212> DNA
<213> Artificial Sequence

<220>
<223> Description of Artificial Sequence: PCR product

<220>
<221> misc_feature
<222> Complement((1)..(200))

<400> 189
cagccaagct gtcgacacta gtggccggcc tttttttttg gtaccttttt ttttcctagg 60

ttctgcaggc cgccgccgcc ggaaccacgc ggaaccagat ggtgatggtg atggtgccat 120

tcgattttct gagcttcgaa gatgtcgttc aggcaaccag ccatatgtta ttacctcctc 180

tagacccggg atccgaattc 200


<210> 190
<211> 250
<212> DNA
<213> Artificial Sequence

<220>
<223> Description of Artificial Sequence: PCR product

<400> 190
acacagaatt cggatcccgg gtctagagga ggttaattaa ccatggaaaa aaaaaggtac 60

caaaaaaaaa ggccggccac tagtggtggt ggtggtctgg ttccgcgtgg ttcccaccac 120

caccacctgc accacatcct ggacgctcag aaaatggttt ggaaccactg ccgttgatga 180

gcagttagag aaagaaccca tagtaggagc agaaactttc taggtcgaca gcttggctgt 240

tttggcggat 250

<210> 191
<211> 250
<212> DNA
<213> Artificial Sequence

<220>
<223> Description of Artificial Sequence: PCR product

<220>
<221> misc_feature
<222> Complement((1)..(250))

<400> 191
atccgccaaa acagccaagc tgtcgaccta gaaagtttct gctcctacta tgggttcttt 60

ctctaactgc tcatcaacgg cagtggttcc aaaccatttt ctgagcgtcc aggatgtggt 120

gcaggtggtg gtggtgggaa ccacgcggaa ccagaccacc accaccacta gtggccggcc 180

tttttttttg gtaccttttt ttttccatgg ttaattaacc tcctctagac ccgggatccg 240

aattctgtgt 250

<210> 192
<211> 150
<212> DNA
<213> Artificial Sequence

<220>
<223> Description of Artificial Sequence: PCR product

<400> 192
acacagaatt cggatcccgg gtctagagga ggttaattaa ccatgagtat ttcagaaaaa 60

cccttgagtg aactgctcag gccgaaagac ttcgaggatt tcgtcggtca ggatcatata 120

ttcggtaata aagggattct ccggcgaact 150

<210> 193
<211> 150
<212> DNA
<213> Artificial Sequence

<220>
<223> Description of Artificial Sequence: PCR product

<220>
<221> misc_feature
<222> Complement((1)..(150))

<400> 193
agttcgccgg agaatccctt tattaccgaa tatatgatcc tgaccgacga aatcctcgaa 60

gtctttcggc ctgagcagtt cactcaaggg tttttctgaa atactcatgg ttaattaacc 120

tcctctagac ccgggatccg aattctgtgt 150


<210> 194
<211> 22
<212> PRT
<213> Artificial Sequence

<220>
<223> Description of Artificial Sequence: Consensus
      sequence

<220>
<221> UNSURE
<222> (1)..(22)
<223> Xaa at positions 1, 4, 6, 7, 14, 15 and 22 is Leu,
      Ile or Val

<220>
<221> UNSURE
<222> (1)..(22)
<223> Xaa at position 11 is Asp or Glu

<400> 194
Xaa Xaa Xaa Xaa Tyr Xaa Xaa Xaa Gly Xaa Xaa Xaa Xaa Xaa Xaa Xaa
  1               5                  10                  15

Xaa Xaa Xaa Xaa Xaa Xaa
            20

<210> 195
<211> 19
<212> PRT
<213> Artificial Sequence

<220>
<223> Description of Artificial Sequence: Consensus
      sequence

<220>
<221> UNSURE
<222> (1)..(19)
<223> Xaa at positions 1, 2, 10, and 16-19 is Leu, Ile
      or Val

<220>
<221> UNSURE
<222> (1)..(19)
<223> Xaa at positions 7 and 12 is Ala or Ser

<220>
<221> UNSURE
<222> (1)..(19)
<223> Xaa at position 14 is Lys or Arg

<400> 195
Xaa Xaa Xaa Xaa Xaa Xaa Xaa Xaa Xaa Xaa Phe Xaa Xaa Xaa Xaa Xaa
  1               5                  10                  15

Xaa Xaa Xaa

<210> 196
<211> 21
<212> PRT
<213> Artificial Sequence

<220>
<223> Description of Artificial Sequence: Consensus
      sequence

<220>
<221> UNSURE
<222> (1)..(21)
<223> Xaa at positions 1, 4- 5, 13-16 and 21 is Leu, Ile

or Val

<220>
<221> UNSURE
<222> (1)..(21)
<223> Xaa at positions 11 is Asp or Glu

<400> 196
Xaa Xaa Xaa Xaa Xaa Xaa Xaa Xaa Xaa Xaa Xaa Xaa Xaa Xaa Xaa Xaa
1               5                   10                  15

Xaa Xaa Xaa Lys Xaa
            20

<210> 197
<211> 38
<212> PRT
<213> Artificial Sequence

<220>
<223> Description of Artificial Sequence: Consensus
      sequence

<220>
<221> UNSURE
<222> (1)..(38)
<223> Xaa at positions 1 and 34 is Arg or Lys

<220>
<221> UNSURE
<222> (1)..(38)
<223> Xaa at positions 5, 7, 9, 14, 17, 25, 28, 32, 35
      and 37-38 is Leu, Ile or Val

<220>
<221> UNSURE
<222> (1)..(38)
<223> Xaa at positions 10, 12 and 31 is Asp or Glu

<400> 197
Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Asn Xaa Xaa Xaa Xaa Xaa Xaa Xaa Xaa
            20                  25                  30

Xaa Xaa Xaa Xaa Xaa Xaa

        35

<210> 198
<211> 46
<212> PRT
<213> Artificial Sequence

<220>
<223> Description of Artificial Sequence: Consensus
      sequence

<220>
<221> UNSURE
<222> (1)..(46)
<223> Xaa at positions 3, 7-8, 16, 18-19, 22, 30, 32-33,
      36, 40, 43 and 46 is Leu, Ile or Val

<220>
<221> UNSURE
<222> (1)..(46)
<223> Xaa at positions 5 and 28 is Asp or Glu

<220>
<221> UNSURE
<222> (1)..(46)
<223> Xaa at positions at 6 and 15 is Ala or Ser

<220>
<221> UNSURE
<222> (1)..(46)
<223> Xaa at position 11 is Arg or Lys

<400> 198
Phe Xaa Xaa Xaa Xaa Xaa Xaa Xaa Xaa Gly Xaa Xaa Xaa Xaa Xaa Xaa
  1               5                  10                  15

Xaa Xaa Xaa Xaa Xaa Xaa Xaa Xaa Xaa Gly Xaa Xaa Pro Xaa Xaa Xaa
              20                  25                  30

Xaa Xaa Xaa Xaa Xaa Xaa Xaa Xaa Xaa Xaa Xaa Xaa Xaa Xaa
          35                  40                  45


<210> 199
<211> 34
<212> PRT
<213> Artificial Sequence

<220>
<223> Description of Artificial Sequence: Consensus
      sequence

<220>
<221> UNSURE
<222> (1)..(34)
<223> Xaa at positions 1, 5, 8, 15, 27, 31- 32, 34 is
      Leu, Ile or Val

<220>
<221> UNSURE
<222> (1)..(34)
<223> Xaa at positions 12, 22 and 28 is Asp or Glu

<220>
<221> UNSURE
<222> (1)..(34)
<223> Xaa at position 16 is Arg or Lys

<400> 199
Xaa Xaa Xaa Xaa Xaa Xaa Xaa Xaa Xaa Xaa Xaa Xaa Xaa Xaa Xaa Xaa
  1               5                  10                  15

Xaa Xaa Xaa Xaa Xaa Xaa Xaa Xaa Xaa Xaa Xaa Xaa Xaa Xaa Xaa Xaa
             20                  25                  30

Xaa Xaa

<210> 200
<211> 643
<212> PRT
<213> Escherichia coli

<400> 200
Met Ser Tyr Gln Val Leu Ala Arg Lys Trp Arg Pro Gln Thr Phe Ala
  1               5                  10                  15

Asp Val Val Gly Gln Glu His Val Leu Thr Ala Leu Ala Asn Gly Leu
             20                  25                  30

Ser Leu Gly Arg Ile His His Ala Tyr Leu Phe Ser Gly Thr Arg Gly
         35                  40                  45

Val Gly Lys Thr Ser Ile Ala Arg Leu Leu Ala Lys Gly Leu Asn Cys

    50                  55                  60

Glu Thr Gly Ile Thr Ala Thr Pro Cys Gly Val Cys Asp Asn Cys Arg
65                  70                  75                  80

Glu Ile Glu Gln Gly Arg Phe Val Asp Leu Ile Glu Ile Asp Ala Ala
                85                  90                  95

Ser Arg Thr Lys Val Glu Asp Thr Arg Asp Leu Leu Asp Asn Val Gln
            100                 105                 110

Tyr Ala Pro Ala Arg Gly Arg Phe Lys Val Tyr Leu Ile Asp Glu Val
        115                 120                 125

His Met Leu Ser Arg His Ser Phe Asn Ala Leu Leu Lys Thr Leu Glu
    130                 135                 140

Glu Pro Pro Glu His Val Lys Phe Leu Leu Ala Thr Thr Asp Pro Gln
145                 150                 155                 160

Lys Leu Pro Val Thr Ile Leu Ser Arg Cys Leu Gln Phe His Leu Lys
                165                 170                 175

Ala Leu Asp Val Glu Gln Ile Arg His Gln Leu Glu His Ile Leu Asn
            180                 185                 190

Glu Glu His Ile Ala His Glu Pro Arg Ala Leu Gln Leu Leu Ala Arg
        195                 200                 205

Ala Ala Glu Gly Ser Leu Arg Asp Ala Leu Ser Leu Thr Asp Gln Ala
    210                 215                 220

Ile Ala Ser Gly Asp Gly Gln Val Ser Thr Gln Ala Val Ser Ala Met
225                 230                 235                 240

Leu Gly Thr Leu Asp Asp Asp Gln Ala Leu Ser Leu Val Glu Ala Met
                245                 250                 255

Val Glu Ala Asn Gly Glu Arg Val Met Ala Leu Ile Asn Glu Ala Ala
            260                 265                 270

Ala Arg Gly Ile Glu Trp Glu Ala Leu Leu Val Glu Met Leu Gly Leu
        275                 280                 285

Leu His Arg Ile Ala Met Val Gln Leu Ser Pro Ala Ala Leu Gly Asn
    290                 295                 300

Asp Met Ala Ala Ile Glu Leu Arg Met Arg Glu Leu Ala Arg Thr Ile

305                 310                 315                 320

Pro Pro Thr Asp Ile Gln Leu Tyr Tyr Gln Thr Leu Leu Ile Gly Arg
                325                 330                 335

Lys Glu Leu Pro Tyr Ala Pro Asp Arg Arg Met Gly Val Glu Met Thr
            340                 345                 350

Leu Leu Arg Ala Leu Ala Phe His Pro Arg Met Pro Leu Pro Glu Pro
        355                 360                 365

Glu Val Pro Arg Gln Ser Phe Ala Pro Val Ala Pro Thr Ala Val Met
    370                 375                 380

Thr Pro Thr Gln Val Pro Pro Gln Pro Gln Ser Ala Pro Gln Gln Ala
385                 390                 395                 400

Pro Thr Val Pro Leu Pro Glu Thr Thr Ser Gln Val Leu Ala Ala Arg
                405                 410                 415

Gln Gln Leu Gln Arg Val Gln Gly Ala Thr Lys Ala Lys Lys Ser Glu
            420                 425                 430

Pro Ala Ala Ala Thr Arg Ala Arg Pro Val Asn Asn Ala Ala Leu Glu
        435                 440                 445

Arg Leu Ala Ser Val Thr Asp Arg Val Gln Ala Arg Pro Val Pro Ser
    450                 455                 460

Ala Leu Glu Lys Ala Pro Ala Lys Lys Glu Ala Tyr Arg Trp Lys Ala
465                 470                 475                 480

Thr Thr Pro Val Met Gln Gln Lys Glu Val Val Ala Thr Pro Lys Ala
                485                 490                 495

Leu Lys Lys Ala Leu Glu His Glu Lys Thr Pro Glu Leu Ala Ala Lys
            500                 505                 510

Leu Ala Ala Glu Ala Ile Glu Arg Asp Pro Trp Ala Ala Gln Val Ser
        515                 520                 525

Gln Leu Ser Leu Pro Lys Leu Val Glu Gln Val Ala Leu Asn Ala Trp
    530                 535                 540

Lys Glu Glu Ser Asp Asn Ala Val Cys Leu His Leu Arg Ser Ser Gln
545                 550                 555                 560

Arg His Leu Asn Asn Arg Gly Ala Gln Gln Lys Leu Ala Glu Ala Leu

565                    570                    575

Ser Met Leu Lys Gly Ser Thr Val Glu Leu Thr Ile Val Glu Asp Asp
            580                    585                    590

Asn Pro Ala Val Arg Thr Pro Leu Glu Trp Arg Gln Ala Ile Tyr Glu
        595                    600                    605

Glu Lys Leu Ala Gln Ala Arg Glu Ser Ile Ile Ala Asp Asn Asn Ile
    610                    615                    620

Gln Thr Leu Arg Arg Phe Phe Asp Ala Glu Leu Asp Glu Glu Ser Ile
625                    630                    635                    640

Arg Pro Ile

<210> 201
<211> 15
<212> PRT
<213> Artificial Sequence

<220>
<223> Description of Artificial Sequence: Consensus
      sequence

<220>
<221> UNSURE
<222> (1)..(15)
<223> Xaa at position 8 is Thr or Ser

<220>
<221> UNSURE
<222> (1)..(15)
<223> Xaa at position 15 is Ile or Val

<400> 201
Gly Xaa Xaa Xaa Xaa Gly Lys Xaa Xaa Xaa Xaa Xaa Xaa Xaa Xaa
  1               5                  10                  15

<210> 202
<211> 14
<212> PRT
<213> Artificial Sequence

<220>

<223> Description of Artificial Sequence: Consensus
      sequence

<220>
<221> UNSURE
<222> (1)..(14)
<223> Xaa at positions 1 and 13 is Arg or Lys

<220>
<221> UNSURE
<222> (1)..(14)
<223> Xaa at positions 10-13 is hydrophobic residue

<400> 202
Xaa Xaa Xaa Xaa Gly Xaa Xaa Xaa Leu Xaa Xaa Xaa Xaa Asp
  1               5                   10

<210> 203
<211> 14
<212> PRT
<213> Artificial Sequence

<220>
<223> Description of Artificial Sequence: Consensus
      sequence

<220>
<221> UNSURE
<222> (1)..(14)
<223> Xaa at position 14 is Thr or Ser

<400> 203
His Ala Tyr Leu Phe Ser Gly Xaa Xaa Gly Xaa Gly Lys Xaa
  1               5                   10

<210> 204
<211> 10
<212> PRT
<213> Artificial Sequence

<220>
<223> Description of Artificial Sequence: Consensus
      sequence

<220>
<221> UNSURE

<222> (1)..(10)
<223> Xaa at positions 1, 3-4 and 6 is Leu, Ile or Val

<220>
<221> UNSURE
<222> (1)..(10)
<223> Xaa at position 8 is Ala or Gly

<400> 204
Xaa Asp Xaa Xaa Glu Xaa Asp Xaa Ala Ser
1               5                   10

<210> 205
<211> 12
<212> PRT
<213> Artificial Sequence

<220>
<223> Description of Artificial Sequence: Consensus
      sequence

<220>
<221> UNSURE
<222> (1)..(12)
<223> Xaa at position 1 is Lys or Arg

<220>
<221> UNSURE
<222> (1)..(12)
<223> Xaa at position 2, 4-5 and 11 is Leu, Ile or Val

<220>
<221> UNSURE
<222> (1)..(12)
<223> Xaa at position 12 is Ser or Thr

<220>
<221> UNSURE
<222> (1)..(12)
<223> Xaa at position 3 is Tyr or Phe

<400> 205
Xaa Xaa Xaa Xaa Xaa Asp Glu Xaa His Met Xaa Xaa
1               5                   10

<210> 206

<211> 334
<212> PRT
<213> Artificial Sequence

<220>
<223> Description of Artificial Sequence: Consensus
      sequence

<400> 206
Met Arg Trp Tyr Pro Trp Leu Arg Pro Asp Phe Glu Lys Leu Val Ala
  1               5                  10                  15

Ser Tyr Gln Ala Gly Arg Gly His His Ala Leu Leu Ile Gln Ala Leu
             20                  25                  30

Pro Gly Met Gly Asp Asp Ala Leu Ile Tyr Ala Leu Ser Arg Tyr Leu
         35                  40                  45

Leu Cys Gln Gln Pro Gln Gly His Lys Ser Cys Gly His Cys Arg Gly
     50                  55                  60

Cys Gln Leu Met Gln Ala Gly Thr His Pro Asp Tyr Tyr Thr Leu Ala
 65                  70                  75                  80

Pro Glu Lys Gly Lys Asn Thr Leu Gly Val Asp Ala Val Arg Glu Val
                 85                  90                  95

Thr Glu Lys Leu Asn Glu His Ala Arg Leu Gly Gly Ala Lys Val Val
            100                 105                 110

Trp Val Thr Asp Ala Ala Leu Leu Thr Asp Ala Ala Ala Asn Ala Leu
        115                 120                 125

Leu Lys Thr Leu Glu Glu Pro Pro Ala Glu Thr Trp Phe Phe Leu Ala
    130                 135                 140

Thr Arg Glu Pro Glu Arg Leu Leu Ala Thr Leu Arg Ser Arg Cys Arg
145                 150                 155                 160

Leu His Tyr Leu Ala Pro Pro Pro Glu Gln Tyr Ala Val Thr Trp Leu
                165                 170                 175

Ser Arg Glu Val Thr Met Ser Gln Asp Ala Leu Leu Ala Ala Leu Arg
            180                 185                 190

Leu Ser Ala Gly Ser Pro Gly Ala Ala Leu Ala Leu Phe Gln Gly Asp
        195                 200                 205

Asn Trp Gln Ala Arg Glu Thr Leu Cys Gln Ala Leu Ala Tyr Ser Val
    210                 215                 220

Pro Ser Gly Asp Trp Tyr Ser Leu Leu Ala Ala Leu Asn His Glu Gln
225                 230                 235                 240

Ala Pro Ala Arg Leu His Trp Leu Ala Thr Leu Leu Met Asp Ala Leu
                245                 250                 255

Lys Arg His His Gly Ala Ala Gln Val Thr Asn Val Asp Val Pro Gly
            260                 265                 270

Leu Val Ala Glu Leu Ala Asn His Leu Ser Pro Ser Arg Leu Gln Ala
        275                 280                 285

Ile Leu Gly Asp Val Cys His Ile Arg Glu Gln Leu Met Ser Val Thr
    290                 295                 300

Gly Ile Asn Arg Glu Leu Leu Ile Thr Asp Leu Leu Leu Arg Ile Glu
305                 310                 315                 320

His Tyr Leu Gln Pro Gly Val Val Leu Pro Val Pro His Leu
                325                 330

<210> 207
<211> 13
<212> PRT
<213> Artificial Sequence

<220>
<223> Description of Artificial Sequence: Consensus
      sequence

<220>
<221> UNSURE
<222> (1)..(13)
<223> Xaa at position 1 and 4  is Ala or Ser

<220>
<221> UNSURE
<222> (1)..(13)
<223> Xaa at position 5-6 and 9 is Leu, Ile or Val

<220>
<221> UNSURE
<222> (1)..(13)
<223> Xaa at position 7 is Lys or Arg

<220>
<221> UNSURE
<222> (1)..(13)
<223> Xaa at position 11 is Glu and Asp

<400> 207
Xaa Ala Asn Xaa Xaa Xaa Xaa Xaa Xaa Glu Xaa Pro Pro
1               5                   10

<210> 208
<211> 8
<212> PRT
<213> Artificial Sequence

<220>
<223> Description of Artificial Sequence: Consensus
      sequence

<220>
<221> UNSURE
<222> (1)..(8)
<223> Xaa at positions 1-2 and 5 is Leu, Ile or Val

<400> 208
Xaa Xaa Xaa Thr Xaa Xaa Ser Arg
1               5

<210> 209
<211> 343
<212> PRT
<213> Artificial Sequence

<220>
<223> Description of Artificial Sequence: Consensus
      sequence

<400> 209
Met Ile Arg Leu Tyr Pro Glu Gln Leu Arg Ala Gln Leu Asn Glu Gly
1               5                   10                  15

Leu Arg Ala Ala Tyr Leu Leu Leu Gly Asn Asp Pro Leu Leu Leu Gln
            20                  25                  30

Glu Ser Gln Asp Ala Val Arg Gln Val Ala Ala Ala Gln Gly Phe Glu
        35                  40                  45

Glu His His Thr Phe Ser Ile Asp Pro Asn Thr Asp Trp Asn Ala Ile
    50                  55                  60

Phe Ser Leu Cys Gln Ala Met Ser Leu Phe Ala Ser Arg Gln Thr Leu
65                  70                  75                  80

Leu Leu Leu Leu Pro Glu Asn Gly Pro Asn Ala Ala Ile Asn Glu Gln
                85                  90                  95

Leu Leu Thr Leu Thr Gly Leu Leu His Asp Asp Leu Leu Leu Ile Val
            100                 105                 110

Arg Gly Asn Lys Leu Ser Lys Ala Gln Glu Asn Ala Ala Trp Phe Thr
        115                 120                 125

Ala Leu Ala Asn Arg Ser Val Gln Val Thr Cys Gln Thr Pro Glu Gln
    130                 135                 140

Ala Gln Leu Pro Arg Trp Val Ala Ala Arg Ala Lys Gln Leu Asn Leu
145                 150                 155                 160

Glu Leu Asp Asp Ala Ala Asn Gln Val Leu Cys Tyr Cys Tyr Glu Gly
                165                 170                 175

Asn Leu Leu Ala Leu Ala Gln Ala Leu Glu Arg Leu Ser Leu Leu Trp
            180                 185                 190

Pro Asp Gly Lys Leu Thr Leu Pro Arg Val Glu Gln Ala Val Asn Asp
.195 200 205

Ala Ala His Phe Thr Pro Phe His Trp Val Asp Ala Leu Leu Met Gly
210 215 220

Lys Ser Lys Arg Ala Leu His Ile Leu Gln Gln Leu Arg Leu Glu Gly
225 230 235 240

Ser Glu Pro Val Ile Leu Leu Arg Thr Leu Gln Arg Glu Leu Leu Leu
245 250 255

Leu Val Asn Leu Lys Arg Gln Ser Ala His Thr Pro Leu Arg Ala Leu
260 265 270

Phe Asp Lys His Arg Val Trp Gln Asn Arg Arg Gly Met Met Gly Glu
275 280 285

Ala Leu Asn Arg Leu Ser Gln Thr Gln Leu Arg Gln Ala Val Gln Leu
290 295 300

Leu Thr Arg Thr Glu Leu Thr Leu Lys Gln Asp Tyr Gly Gln Ser Val
305                 310                 315                 320

Trp Ala Glu Leu Glu Gly Leu Ser Leu Leu Leu Cys His Lys Pro Leu
                325                 330                 335

Ala Asp Val Phe Ile Asp Gly
            340

<210> 210
<211> 324
<212> PRT
<213> Thermotoga maritima

<400> 210
Met Pro Val Thr Phe Leu Thr Gly Thr Ala Glu Thr Gln Lys Glu Glu
  1               5                  10                  15

Leu Ile Lys Lys Leu Leu Lys Asp Gly Asn Val Glu Tyr Ile Arg Ile
             20                  25                  30

His Pro Glu Asp Pro Asp Lys Ile Asp Phe Ile Arg Ser Leu Leu Arg
         35                  40                  45

Thr Lys Thr Ile Phe Ser Asn Lys Thr Ile Ile Asp Ile Val Asn Phe
     50                  55                  60

Asp Glu Trp Lys Ala Gln Glu Gln Lys Arg Leu Val Glu Leu Leu Lys
 65                  70                  75                  80

Asn Val Pro Glu Asp Val His Ile Phe Ile Arg Ser Gln Lys Thr Gly
                 85                  90                  95

Gly Lys Gly Val Ala Leu Glu Leu Pro Lys Pro Trp Glu Thr Asp Lys
            100                 105                 110

Trp Leu Glu Trp Ile Glu Lys Arg Phe Arg Glu Asn Gly Leu Leu Ile
        115                 120                 125

Asp Lys Asp Ala Leu Gln Leu Phe Phe Ser Lys Val Gly Thr Asn Asp
    130                 135                 140

Leu Ile Ile Glu Arg Glu Ile Glu Lys Leu Lys Ala Tyr Ser Glu Asp
145                 150                 155                 160

Arg Lys Ile Thr Val Glu Asp Val Glu Glu Val Val Phe Thr Tyr Gln

                165                 170                 175

Thr Pro Gly Tyr Asp Asp Phe Cys Phe Ala Val Ser Glu Gly Lys Arg
            180                 185                 190

Lys Leu Ala His Ser Leu Leu Ser Gln Leu Trp Lys Thr Thr Glu Ser
        195                 200                 205

Val Val Ile Ala Thr Val Leu Ala Asn His Phe Leu Asp Leu Phe Lys
    210                 215                 220

Ile Leu Val Leu Val Thr Lys Lys Arg Tyr Tyr Thr Trp Pro Asp Val
225                 230                 235                 240

Ser Arg Val Ser Lys Glu Leu Gly Ile Pro Val Pro Arg Val Ala Arg
                245                 250                 255

Phe Leu Gly Phe Ser Phe Lys Thr Trp Lys Phe Lys Val Met Asn His
            260                 265                 270

Leu Leu Tyr Tyr Asp Val Lys Lys Val Arg Lys Ile Leu Arg Asp Leu
        275                 280                 285

Tyr Asp Leu Asp Arg Ala Val Lys Ser Glu Glu Asp Pro Lys Pro Phe
    290                 295                 300

Phe His Glu Phe Ile Glu Glu Val Ala Leu Asp Val Tyr Ser Leu Gln
305                 310                 315                 320

Arg Asp Glu Glu

<210> 211
<211> 1115
<212> DNA
<213> Thermotoga maritima

<400> 211
cactttgaaa ttgtttctca aagcgtgtta aaatcaccat gagaggtgat tgtatgccag 60

tcacgtttct cacaggtact gcagaaactc agaaggaaga attgataaag aaactcctga 120

aggatggtaa cgtggagtac ataaggatcc atccggagga tcccgacaag atcgatttca 180

taaggtcttt actcaggaca aagacgatct tttccaacaa gacgatcatt gacatcgtca 240

atttcgatga gtggaaagca caggagcaga agcgtctcgt tgaacttttg aaaaacgtac 300

cggaagacgt tcatatcttc atccgttctc aaaaaacagg tggaaaggga gtagcgctgg 360

agcttccgaa gccatgggaa acggacaagt ggcttgagtg gatagaaaag cgcttcaggg 420

agaatggttt gctcatcgat aaagatgccc ttcagctgtt tttctccaag gttggaacga 480

acgacctgat catagaaagg gagattgaaa aactgaaagc ttattccgag gacagaaaga 540

taacggtaga agacgtggaa gaggtcgttt ttacctatca gactccggga tacgatgatt 600

tttgctttgc tgtttccgaa ggaaaaagga agctcgctca ctctcttctg tcgcagctgt 660

ggaaaaccac agagtccgtg gtgattgcca ctgtccttgc gaatcacttc ttggatctct 720

tcaaaatcct cgttcttgtg acaaagaaaa gatactacac ctggcctgat gtgtccaggg 780

tgtccaaaga gctgggaatt cccgttcctc gtgtggctcg tttcctcggt ttctccttta 840

agacctggaa attcaaggtg atgaaccacc tcctctacta cgatgtgaag aaggttagaa 900

agatactgag ggatctctac gatctggaca gagccgtgaa aagcgaagaa gatccaaaac 960

cgttcttcca cgagttcata gaagaggtgg cactggatgt atattctctt cagagagatg 1020

aagaataact ggtacagcct cgcggccctt ctctcaacca tatacagcag gcacctcgat 1080

gtggaagcaa gacctgtgaa gtttgaggaa ataaa 1115

<210> 212
<211> 1200
<212> DNA
<213> Aquifex aeolicus

<400> 212
tggcttttac ctctttgtag agttaagtat taaatttaat ttaactgtgg aaaccacaat 60

attccagttc cagaaaactt ttttcacaaa acctccgaag gagagggtct tcgtccttca 120

tggagaagag cagtatctca taagaacctt tttgtctaag ctgaaggaaa agtacgggga 180

gaattacacg gttctgtggg gggatgagat aagcgaggag gaattctaca ctgccctttc 240

cgagaccagt atattcggcg gttcaaagga aaaagcggtg gtcatttaca acttcgggga 300

tttcctgaag aagctcggaa ggaagaaaaa ggaaaaagaa aggcttataa aagtcctcag 360

aaacgtaaag agtaactacg tatttatagt gtacgatgcg aaactccaga aacaggaact 420

ttcttcggaa cctctgaaat ccgtagcgtc tttcggcggt atagtggtag caaacaggct 480

gagcaaggag aggataaaac agctcgtcct taagaagttc aaagaaaaag ggataaacgt 540

agaaaacgat gcccttgaat accttctcca gctcacgggt tacaacttga tggagctcaa 600

acttgaggtt gaaaaactga tagattacgc aagtgaaaag aaaattttaa cactcgatga 660

ggtaaagaga gtagccttct cagtctcaga aaacgtaaac gtatttgagt tcgttgattt 720

actcctctta aaagattacg aaaaggctct taaagttttg gactccctca tttccttcgg 780

aatacacccc ctccagatta tgaaaatcct gtcctcctat gctctaaaac tttacaccct 840

caagaggctt gaagagaagg gagaggacct gaataaggcg atggaaagcg tgggaataaa 900

gaacaacttt ctcaagatga agttcaaatc ttacttaaag gcaaactcta aagaggactt 960

gaagaaccta atcctctccc tccagaggat agacgctttt tctaaacttt actttcagga 1020

cacagtgcag ttgctgaggg atttcttgac ctcaagactg gagagggaag ttgtgaaaaa 1080

tacttctcat ggtggataat cttttttatg aagtttgcgg tttgcgtttt tcccggttct 1140

aactgcgatt acgacaccta ctacgttata agggacattc ttgaaaagga cgtggaattc 1200

<210> 213
<211> 350
<212> PRT
<213> Aquifex aeolicus

<400> 213
Met Glu Thr Thr Ile Phe Gln Phe Gln Lys Thr Phe Phe Thr Lys Pro
1               5                   10                  15

Pro Lys Glu Arg Val Phe Val Leu His Gly Glu Glu Gln Tyr Leu Ile
            20                  25                  30

Arg Thr Phe Leu Ser Lys Leu Lys Glu Lys Tyr Gly Glu Asn Tyr Thr
        35                  40                  45

Val Leu Trp Gly Asp Glu Ile Ser Glu Glu Glu Phe Tyr Thr Ala Leu
    50                  55                  60

Ser Glu Thr Ser Ile Phe Gly Gly Ser Lys Glu Lys Ala Val Val Ile

65                  70                  75                  80

Tyr Asn Phe Gly Asp Phe Leu Lys Lys Leu Gly Arg Lys Lys Lys Glu
                85                  90                  95

Lys Glu Arg Leu Ile Lys Val Leu Arg Asn Val Lys Ser Asn Tyr Val
            100                 105                 110

Phe Ile Val Tyr Asp Ala Lys Leu Gln Lys Gln Glu Leu Ser Ser Glu
        115                 120                 125

Pro Leu Lys Ser Val Ala Ser Phe Gly Gly Ile Val Val Ala Asn Arg
    130                 135                 140

Leu Ser Lys Glu Arg Ile Lys Gln Leu Val Leu Lys Lys Phe Lys Glu
145                 150                 155                 160

Lys Gly Ile Asn Val Glu Asn Asp Ala Leu Glu Tyr Leu Leu Gln Leu
                165                 170                 175

Thr Gly Tyr Asn Leu Met Glu Leu Lys Leu Glu Val Glu Lys Leu Ile
            180                 185                 190

Asp Tyr Ala Ser Glu Lys Lys Ile Leu Thr Leu Asp Glu Val Lys Arg
        195                 200                 205

Val Ala Phe Ser Val Ser Glu Asn Val Asn Val Phe Glu Phe Val Asp
    210                 215                 220

Leu Leu Leu Leu Lys Asp Tyr Glu Lys Ala Leu Lys Val Leu Asp Ser
225                 230                 235                 240

Leu Ile Ser Phe Gly Ile His Pro Leu Gln Ile Met Lys Ile Leu Ser
                245                 250                 255

Ser Tyr Ala Leu Lys Leu Tyr Thr Leu Lys Arg Leu Glu Glu Lys Gly
            260                 265                 270

Glu Asp Leu Asn Lys Ala Met Glu Ser Val Gly Ile Lys Asn Asn Phe
        275                 280                 285

Leu Lys Met Lys Phe Lys Ser Tyr Leu Lys Ala Asn Ser Lys Glu Asp
    290                 295                 300

Leu Lys Asn Leu Ile Leu Ser Leu Gln Arg Ile Asp Ala Phe Ser Lys
305                 310                 315                 320

Leu Tyr Phe Gln Asp Thr Val Gln Leu Leu Arg Asp Phe Leu Thr Ser

                325                 330                 335

Arg Leu Glu Arg Glu Val Val Lys Asn Thr Ser His Gly Gly
            340                 345                 350

<210> 214
<211> 32
<212> PRT
<213> Escherichia coli

<400> 214
Met Ala Gly Cys Leu Asn Asp Ile Phe Glu Ala Gln Lys Ile Glu Trp
1               5                   10                  15

His His His His His His Leu Val Pro Arg Gly Ser Gly Gly Gly Gly
            20                  25                  30

<210> 215
<211> 305
<212> PRT
<213> Aquifex aeolicus

<400> 215
Met Glu Lys Val Phe Leu Glu Lys Leu Gln Lys Thr Leu His Ile Pro
1               5                   10                  15

Gly Gly Leu Leu Phe Tyr Gly Lys Glu Gly Ser Gly Lys Thr Lys Thr
            20                  25                  30

Ala Phe Glu Phe Ala Lys Gly Ile Leu Cys Lys Glu Asn Val Pro Trp
        35                  40                  45

Gly Cys Gly Ser Cys Pro Ser Cys Lys His Val Asn Glu Leu Glu Glu
    50                  55                  60

Ala Phe Phe Lys Gly Glu Ile Glu Asp Phe Lys Val Tyr Lys Asp Lys
65                  70                  75                  80

Asp Gly Lys Lys His Phe Val Tyr Leu Met Gly Glu His Pro Asp Phe
                85                  90                  95

Val Val Ile Ile Pro Ser Gly His Tyr Ile Lys Ile Glu Gln Ile Arg
            100                 105                 110

Glu Val Lys Asn Phe Ala Tyr Val Lys Pro Ala Leu Ser Arg Arg Lys
        115                 120                 125

Val Ile Ile Ile Asp Asp Ala His Ala Met Thr Ser Gln Ala Ala Asn
    130                 135                 140

Ala Leu Leu Lys Val Leu Glu Glu Pro Pro Ala Asp Thr Thr Phe Ile
145                 150                 155                 160

Leu Thr Thr Asn Arg Arg Ser Ala Ile Leu Pro Thr Ile Leu Ser Arg
                165                 170                 175

Thr Phe Gln Val Glu Phe Lys Gly Phe Ser Val Lys Glu Val Met Glu
            180                 185                 190

Ile Ala Lys Val Asp Glu Glu Ile Ala Lys Leu Ser Gly Gly Ser Leu
        195                 200                 205

Lys Arg Ala Ile Leu Leu Lys Glu Asn Lys Asp Ile Leu Asn Lys Val
    210                 215                 220

Lys Glu Phe Leu Glu Asn Glu Pro Leu Lys Val Tyr Lys Leu Ala Ser
225                 230                 235                 240

Glu Phe Glu Lys Trp Glu Pro Glu Lys Gln Lys Leu Phe Leu Glu Ile
                245                 250                 255

Met Glu Glu Leu Val Ser Gln Lys Leu Thr Glu Glu Lys Lys Asp Asn
            260                 265                 270

Tyr Thr Tyr Leu Leu Asp Thr Ile Arg Leu Phe Lys Asp Gly Leu Ala
        275                 280                 285

Arg Gly Val Asn Glu Pro Leu Trp Leu Phe Thr Leu Ala Val Gln Ala
    290                 295                 300

Asp
305

<210> 216
<211> 1540
<212> DNA
<213> Aquifex aeolicus

<400> 216
actctctttc agacttaaag aaaagatacg ggaaattctc ggcgagaaat ttgagcattt 60

aataaaagtt ttaggggata taatttccga taatacactt gaaacacctt taataacact 120

cggtgaaggt aaaaacctca agaaaaagtt taacgaggaa ttcatagaaa cccttgagaa 180

tgcttcctat tcaaacgagg aggccggaaa attaagggaa tttttaaaga cactttacgc 240

tttggcactc tgtaatagta gggaagaaat cactctggac ctttgtgtaa tattactcca 300

ctccgaacag cacgcaactt tagaaaaaca actcctttct ctcgtacaac cgctactaaa 360

aattgataaa aaaccgaaga aagaactgaa cctatagtcc gcgaagttgc aaaggttata 420

aacgaaacct gtttaaatta aatttgtatg gaaaaagttt ttttggaaaa actccagaaa 480

accttgcaca tacccggagg actccttttt tacggcaaag aaggaagcgg aaagacgaaa 540

acagcttttg aatttgcaaa aggtatttta tgtaaggaaa acgtaccgtg gggatgcgga 600

agttgtccct cctgcaaaca cgtaaacgag ctggaggaag ccttctttaa aggagaaata 660

gaagacttta aagtttataa agacaaggac ggtaaaaagc acttcgttta ccttatgggc 720

gaacatcccg actttgtggt aataatcccg agcggacatt acataaagat agaacagata 780

agggaagtta agaactttgc ctatgtgaag cccgcactaa gcaggagaaa agtaattata 840

atagacgacg cccacgcgat gacctctcag gcggcaaacg ctcttttaaa ggtattggaa 900

gagccacctg cggacaccac ctttatcttg accacgaaca ggcgttctgc aatcctgccg 960

actatcctct ccagaacttt tcaagtggag ttcaagggct tttcagtaaa agaggttatg 1020

gaaatagcga aagtagacga ggaaatagcg aaactctctg gaggcagtct aaaaagggct 1080

atcttactaa aggaaaacaa agatatccta aacaaagtaa aggaattctt ggaaaacgag 1140

ccgttaaaag tttacaagct tgcaagtgaa ttcgaaaagt gggaacctga aaagcaaaaa 1200

ctcttccttg aaattatgga agaattggta tctcaaaaat tgaccgaaga gaaaaaagac 1260

aattacacct accttcttga tacgatcaga ctctttaaag acggactcgc aaggggtgta 1320

aacgaacctc tgtggctgtt tacgttagcc gttcaggcgg attaataaac cgttattgat 1380

tccgtaacat ttaaacctta atctaaatta tgagagcctt tgaaggaggt ctggtatgga 1440

aaatttgaag attagatata tagatacgag gaagatagga accgtgagcg gtgtaaaagt 1500

tccggtaaaa aagggctaca aaatagtcgt ggaagacgag                    1540

<210> 217
<211> 599
<212> PRT
<213> Thermotoga maritima

<400> 217
Met Ser Ile Ser Glu Lys Pro Leu Ser Glu Leu Leu Arg Pro Lys Asp
1               5                   10                  15

Phe Glu Asp Phe Val Gly Gln Asp His Ile Phe Gly Asn Lys Gly Ile
            20                  25                  30

Leu Arg Arg Thr Leu Lys Thr Gly Asn Met Phe Ser Ser Ile Leu Tyr
        35                  40                  45

Gly Pro Pro Gly Ser Gly Lys Thr Ser Val Phe Ser Leu Leu Lys Arg
    50                  55                  60

Tyr Phe Asn Gly Glu Val Val Tyr Leu Ser Ser Thr Val His Gly Val
65                  70                  75                  80

Ser Glu Ile Lys Asn Val Leu Lys Arg Gly Glu Gln Leu Arg Lys Tyr
                85                  90                  95

Gly Lys Lys Leu Leu Leu Phe Leu Asp Glu Ile His Arg Leu Asn Lys
            100                 105                 110

Asn Gln Gln Met Val Leu Val Ser His Val Glu Arg Gly Asp Ile Val
        115                 120                 125

Leu Val Ala Thr Thr Thr Glu Asn Pro Ser Phe Val Ile Val Pro Ala
    130                 135                 140

Leu Leu Ser Arg Cys Arg Ile Leu Tyr Phe Lys Lys Leu Ser Asp Glu
145                 150                 155                 160

Asp Leu Met Lys Ile Leu Lys Lys Ala Thr Glu Val Leu Asn Ile Asp
                165                 170                 175

Leu Glu Glu Ser Val Glu Lys Ala Ile Val Arg His Ser Glu Gly Asp
            180                 185                 190

Ala Arg Lys Leu Leu Asn Thr Leu Glu Ile Val His Gln Ala Phe Lys
        195                 200                 205

Asn Lys Arg Val Thr Leu Glu Asp Leu Glu Thr Leu Leu Gly Asn Val
    210                 215                 220

Ser Gly Tyr Thr Lys Glu Ser His Tyr Asp Phe Ala Ser Ala Phe Ile
225                 230                 235                 240

Lys Ser Met Arg Gly Ser Asp Pro Asn Ala Ala Val Tyr Tyr Leu Val
                245                 250                 255

Lys Met Ile Glu Met Gly Glu Asp Pro Arg Phe Ile Ala Arg Arg Met
            260                 265                 270

Ile Ile Phe Ala Ser Glu Asp Val Gly Leu Ala Asp Pro Asn Ala Leu
        275                 280                 285

His Ile Ala Val Ser Thr Ser Ile Ala Val Glu His Val Gly Leu Pro
    290                 295                 300

Glu Cys Leu Met Asn Leu Val Glu Cys Ala Val Tyr Leu Ser Leu Ala
305                 310                 315                 320

Pro Lys Ser Asn Ser Val Tyr Leu Ala Met Lys Lys Val Gln Glu Leu
                325                 330                 335

Pro Val Glu Asp Val Pro Leu Phe Leu Arg Asn Pro Val Thr Glu Glu
            340                 345                 350

Met Lys Lys Arg Gly Tyr Gly Glu Gly Tyr Leu Tyr Pro His Asp Phe
        355                 360                 365

Gly Gly Phe Val Lys Thr Asn Tyr Leu Pro Glu Lys Leu Lys Asn Glu
    370                 375                 380

Val Ile Phe Gln Pro Lys Arg Val Gly Phe Glu Glu Glu Leu Phe Glu
385                 390                 395                 400

Arg Leu Arg Lys Leu Trp Pro Glu Lys Tyr Gly Gly Glu Ser Met Ala
                405                 410                 415

Glu Val Arg Lys Glu Leu Glu Tyr Lys Gly Lys Lys Ile Arg Ile Val
            420                 425                 430

Lys Gly Asp Ile Thr Arg Glu Glu Val Asp Ala Ile Val Asn Ala Ala
        435                 440                 445

Asn Glu Tyr Leu Lys His Gly Gly Gly Val Ala Gly Ala Ile Val Arg
    450                 455                 460

Ala Gly Gly Ser Val Ile Gln Glu Glu Ser Asp Arg Ile Val Gln Glu
465                 470                 475                 480

Arg Gly Arg Val Pro Thr Gly Glu Ala Val Val Thr Ser Ala Gly Lys
                485                 490                 495

Leu Lys Ala Lys Tyr Val Ile His Thr Val Gly Pro Val Trp Arg Gly
            500                 505                 510

Gly Ser His Gly Glu Asp Glu Leu Leu Tyr Lys Ala Val Tyr Asn Ala
        515                 520                 525

Leu Leu Arg Ala His Glu Leu Lys Leu Lys Ser Ile Ser Met Pro Ala
    530                 535                 540

Ile Ser Thr Gly Ile Phe Gly Phe Pro Lys Glu Arg Ala Val Gly Ile
545                 550                 555                 560

Phe Ser Lys Ala Ile Arg Asp Phe Ile Asp Gln His Pro Asp Thr Thr
                565                 570                 575

Leu Glu Glu Ile Arg Ile Cys Asn Ile Asp Glu Glu Thr Thr Lys Ile
            580                 585                 590

Phe Glu Glu Lys Phe Ser Val
        595

<210> 218
<211> 2640
<212> DNA
<213> Thermotoga maritima

<400> 218
caagacagga tccgtacggt gaagcgggcg tggtggccat cttcactgaa aggatgctca 60

ggggagagga agttcacata ttcggtgatg gagagtacgt cagagactac gtttacgttg 120

atgacgtggt cagagcgaat cttctcgcca tggagaaagg cgacaacgaa gtcttcaaca 180

taggaacggg aaggggcaca accgtgaacc agctcttcaa actgctgaaa gagatcactg 240

gctacgataa agagcctgtc tataaaccac cgcgtaaggg cgacgtgaga aagagcattc 300

tcgattacac gaaggcgaag gaaaagctcg gctgggaacc caaagtttcc cttgaggagg 360

gattgaagct caccgttgag tatttcagaa aaacccttga gtgaactgct caggccgaaa 420

gacttcgagg atttcgtcgg tcaggatcat atattcggta ataaagggat tctccggcga 480

actttaaaga caggcaacat gttctcttct atcctctatg gaccaccggg gtctggtaag 540

acctcggttt tttcactgct gaaaaggtat ttcaacggcg aggtagttta tctgagctcc 600

accgttcacg gggtttctga aataaagaac gttctcaaaa gaggagaaca gttgagaaaa 660

tatggaaaaa aattgcttct ctttctcgat gaaatacacc gcctgaacaa aaaccagcag 720

atggttctgg tttcccatgt tgaacgagga gacatcgtac tggttgcgac aacaactgaa 780

aatccgagtt ttgtcatcgt acctgcactt ctctcaaggt gcaggattct ttatttcaaa 840

aaactctctg acgaggacct gatgaagatc ttgaaaaaag caacagaggt tctcaacatc 900

gatctggaag aatcggtaga gaaagccata gtaaggcatt ccgaaggaga cgccagaaaa 960

ctcttgaaca ccctggagat cgtccaccag gcgttcaaaa acaagagagt aactcttgag 1020

gacctggaaa ctctgctggg aaacgtgagc gggtacacga aggaatcaca ctacgatttc 1080

gcttcggcct tcataaagag tatgagaggt agcgatccaa acgccgctgt ttactacctc 1140

gtcaaaatga tagagatggg agaagacccg cgattcatag cacgaagaat gatcatattc 1200

gccagcgaag acgttggact cgccgaccca aacgctctac atatcgccgt ttcgacctcc 1260

atcgctgtcg aacacgtggg acttcccgaa tgtttgatga accttgtaga gtgtgccgtc 1320

tatctttccc tcgctcccaa gagcaactct gtttatctcg caatgaaaaa agtccaggaa 1380

cttcctgtgg aggacgtacc gctttttctg agaaatcccg tcaccgaaga gatgaaaaag 1440

cgtggatacg gagaagggta tctttatccc catgatttcg gtggtttcgt gaaaacgaac 1500

tatcttccag aaaagttgaa aaatgaggtc atcttccagc caaagagagt aggttttgag 1560

gaagaactct ttgaaaggct cagaaaactc tggcccgaga agtacggggg tgaaagtatg 1620

gccgaagtga gaaaagaact ggaatacaaa gggaaaaaga tcagaatcgt aaagggagac 1680

atcacaagag aagaagtgga cgccatagtg aacgcggcga acgaatatct gaaacacgga 1740

ggaggagtag cgggagcgat cgtgagagca ggtggaagcg ttatccagga agaaagcgac 1800

aggatcgttc aagaacgagg aagagtcccg acaggtgaag cggtggtaac cagcgctgga 1860

aagctgaagg caaaatacgt gatccacacc gtggggcccg tttggagagg aggcagtcat 1920

ggagaggacg aacttctcta caaagcagtt tacaacgctc ttcttcgagc tcacgaactg 1980

aaattgaaga gcatctcaat gcctgctatc agtacaggaa tattcggatt tccgaaagaa 2040

agagcggtgg gaatcttttc aaaagcaata agagatttca tcgatcaaca tcctgatacc 2100

actctggaag agatccgcat atgcaacata gatgaggaaa caacgaagat tttcgaagaa 2160

aagttcagcg tttgatgaaa ccaaaagaag gggcaaacgc cccttctttt ttaagacctc 2220

tgaggaagta ttgaatcaac tcaaacgagt tcttggacct cttccgcaac agatttccat 2280

acctgaaagg aactattgat attttcatca taccacaaaa actaaaaaag taaagggggc 2340

caagggcccc ctttatttcc atcagaattc ttcaggcatg ctcggtgttt ctttcttctc 2400

ctcgggtttt tcaacgatga gcacttccgt tgtcaggagc attccagcga tggatgcagc 2460

attctgcagg gcgctccttg taacctttgc tgggtcaatg attcctcttt caaacatgtt 2520

gcagtattct cctctgagtg cgtcgaatcc ataggctgga tcgtcattcg aaagtatctt 2580

ctcaatgatc accgctccgt cgtatcccgc gttttcagca atctgcttaa taggggctga 2640

<210> 219
<211> 312
<212> PRT
<213> Thermotoga maritima

<400> 219
Met Asn Asp Leu Ile Arg Lys Tyr Ala Lys Asp Gln Leu Glu Thr Leu
1               5                   10                  15

Lys Arg Ile Ile Glu Lys Ser Glu Gly Ile Ser Ile Leu Ile Asn Gly
            20                  25                  30

Glu Asp Leu Ser Tyr Pro Arg Glu Val Ser Leu Glu Leu Pro Glu Tyr
        35                  40                  45

Val Glu Lys Phe Pro Pro Lys Ala Ser Asp Val Leu Glu Ile Asp Pro
    50                  55                  60

Glu Gly Glu Asn Ile Gly Ile Asp Asp Ile Arg Thr Ile Lys Asp Phe
65                  70                  75                  80

Leu Asn Tyr Ser Pro Glu Leu Tyr Thr Arg Lys Tyr Val Ile Val His
                85                  90                  95

Asp Cys Glu Arg Met Thr Gln Gln Ala Ala Asn Ala Phe Leu Lys Ala
            100                 105                 110

Leu Glu Glu Pro Pro Glu Tyr Ala Val Ile Val Leu Asn Thr Arg Arg
        115                 120                 125

Trp His Tyr Leu Leu Pro Thr Ile Lys Ser Arg Val Phe Arg Val Val
    130                 135                 140

Val Asn Val Pro Lys Glu Phe Arg Asp Leu Val Lys Glu Lys Ile Gly
145                 150                 155                 160

Asp Leu Trp Glu Glu Leu Pro Leu Leu Glu Arg Asp Phe Lys Thr Ala
                165                 170                 175

Leu Glu Ala Tyr Lys Leu Gly Ala Glu Lys Leu Ser Gly Leu Met Glu
            180                 185                 190

Ser Leu Lys Val Leu Glu Thr Glu Lys Leu Leu Lys Lys Val Leu Ser
        195                 200                 205

Lys Gly Leu Glu Gly Tyr Leu Ala Cys Arg Glu Leu Leu Glu Arg Phe
    210                 215                 220

Ser Lys Val Glu Ser Lys Glu Phe Phe Ala Leu Phe Asp Gln Val Thr
225                 230                 235                 240

Asn Thr Ile Thr Gly Lys Asp Ala Phe Leu Leu Ile Gln Arg Leu Thr
                245                 250                 255

Arg Ile Ile Leu His Glu Asn Thr Trp Glu Ser Val Glu Asp Gln Lys
            260                 265                 270

Ser Val Ser Phe Leu Asp Ser Ile Leu Arg Val Lys Ile Ala Asn Leu
        275                 280                 285

Asn Asn Lys Leu Thr Leu Met Asn Ile Leu Ala Ile His Arg Glu Arg
    290                 295                 300

Lys Arg Gly Val Asn Ala Trp Ser
305                 310

<210> 220

<211> 1980
<212> DNA
<213> Thermotoga maritima

<400> 220
ggaagatagc tccgctccgc ccaaacgtcc ggttgctcgt tcagaatgtg gtacagtatc 60

tcaagaccgt ggtttgaagt acctacctcg taagtgtcgg gaaaaacgag ggctatccgg 120

agggaaattt cctctggatc tttcacaacg ctgttgattt ctcttcctat gtatctactt 180

ggttttctaa cccagggaag tgtttcattc aagaatttta gaatcatcct ttcacctcag 240

ttctgatttt atcattggtt gttataatta gaaggcgagg tgagatcatg aacgatttga 300

tcagaaagta cgctaaagat caactggaaa ctttgaaaag gatcatagaa aagtctgaag 360

gaatatccat cctcataaat ggagaagatc tctcgtatcc gagagaagta tcccttgaac 420

ttcccgagta cgtggagaaa tttcccccga aggcctcgga tgttctggag atagatcccg 480

agggggagaa cataggcata gacgacatca gaacgataaa ggacttcctg aactacagcc 540

ccgagctcta cacgagaaag tacgtgatag tccacgactg tgaaagaatg acccagcagg 600

cggcgaacgc gtttctgaag gcccttgaag aaccaccaga atacgctgtg atcgttctga 660

acactcgccg ctggcattat ctactgccga cgataaagag ccgagtgttc agagtggttg 720

tgaacgttcc aaaggagttc agagatctcg tgaaagagaa aataggagat ctctgggagg 780

aacttccact tcttgagaga gacttcaaaa cggctctcga agcctacaaa cttggtgcgg 840

aaaaactttc tggattgatg gaaagtctca aagttttgga gacggaaaaa ctcttgaaaa 900

aggtcctttc aaaaggcctc gaaggttatc tcgcatgtag ggagctcctg gagagatttt 960

caaaggtgga atcgaaggaa ttctttgcgc tttttgatca ggtgactaac acgataacag 1020

gaaaagacgc gtttcttttg atccagagac tgacaagaat cattctccac gaaaacacat 1080

gggaaagcgt tgaagatcaa aaaagcgtgt ctttcctcga ttcaattctc agggtgaaga 1140

tagcgaatct gaacaacaaa ctcactctga tgaacatcct cgcgatacac agagagagaa 1200

agagaggtgt caacgcttgg agctgaaggc aagggcagtg ggagtggaga taataccaaa 1260

gggtaagata atttactact ctgtacccaa cggtgatgag tacgagagag gagacctcgt 1320

tctggtactg ggagattttg gacttgaagt gggaaaggtt ctgatacctc ccagagaggt 1380

ggtcatagac gaggtggggt acgaactcaa ggccgtgatc agaaagttga cggatgaaga 1440

tctggaacaa tacaggaaga atgtagagga tgcgttgaag gccttccaga tatgtaagca 1500

gaaaataaaa gaacacggtc ttcccatgaa gcttctctac gccaagtaca ccttcgatag 1560

aactaggctc atctttttct tcagtgcgga aggaagggtt gatttcagag agcttgtaag 1620

agacctcgca aagatcttca aaacaaggat agagttgagg caagttggtg tgagagatga 1680

gatgaagttc tttggaggcc ttggattgtg cggacttccg acatgctgtt ccacatttct 1740

cagagagttc accagcgtca cactgaagca cgcaaagaag cagcagatga tgatcaatcc 1800

agcgaagata tccggaccat gcagaaggct tctgtgctgt cttacctacg aatacgattt 1860

ctatgaaaag gagctggagg gaattcctga cgaagggagc acgatcacct acgaaggaaa 1920

gcgctacaaa gtcgtgaacg tgaacgtgtt tctcagaacg gtgactctct tctcggaaca 1980

<210> 221
<211> 6
<212> PRT
<213> Artificial Sequence

<220>
<223> Description of Artificial Sequence: Consensus
      sequence

<220>
<221> UNSURE
<222> (1)..(6)
<223> Xaa at position 6 is Thr or Ser

<400> 221
Gly Xaa Gly Lys Thr Xaa
  1               5

<210> 222
<211> 11
<212> PRT
<213> Artificial Sequence

<220>
<223> Description of Artificial Sequence: Consensus
      sequence

<400> 222
Asn Ala Leu Leu Lys Thr Leu Glu Glu Pro Pro
  1               5                  10


<210> 223
<211> 996
<212> DNA
<213> Mycobacterium tuberculosis

<400> 223
gtgagcgagg ctaagccgtt gcacctggtg agcgaggcta agccgttgca cctggtcctg 60

ggagacgaag aactgctggt cgaaagggcg gtggccgacg tgttgcgctc ggctcggcag 120

cgggcaggta cagccgacgt cccggtgagc cgaatgcgcg cgggtgacgt cggtgcctat 180

gagctcgccg aactgctgag cccgtcactg ttcgccgagg agcggatcgt tgtgctgggg 240

gccgctgcgg aggcgggcaa ggacgctgcc gcggtaatcg agtcggccgc cgccgatctt 300

ccggccggca ccgtgctggt agtggtccac tcgggtggcg ggcgcgccaa atcgctggcc 360

aaccagctgc ggtcgatggg tgcgcaggtt catccgtgcg cgcggatcac caaggtcagt 420

gagcgcgccg acttcatccg tagcgagttc gcgtcgctgc gggtcaaggt cgacgacgag 480

accgtgaccg ccctgctgga cgccgtcggc tccgacgtgc gcgaactcgc ctcggcctgt 540

tcacagctgg tcgccgatac cggaggagcc gtcgacgccg ccgctgtacg gcgctatcac 600

agcggcaaag ccgaggtgag gggcttcgac atcgccgaca aggcggtagc cggcgacgtg 660

gcgggagctg ccgaagcgtt gcggtgggcg atgatgcgcg gtgagccgct agtggtgttg 720

gccgatgcgc tcgccgaagc cgtgcacacc atcggccggg tcgggccgca gtccggcgac 780

ccgtaccgcc tggccgcaca actggggatg ccgccctggc gggtgcagaa agcccagaag 840

caggctcggc ggtggtcgcg tgacacggtg gcgaccgcga tgaggttggt ggccgaactc 900

aatgctaacg tcaagggcgc cgtcgcggat gcggactacg cgctggaatc cgcggtccgg 960

caggtcgccg agttggtggc cgaccgcggc cgatga                            996

<210> 224
<211> 996
<212> DNA
<213> Mycobacterium tuberculosis

<220>
<221> CDS
<222> (1)..(996)

<400> 224
gtg agc gag gct aag ccg ttg cac ctg gtg agc gag gct aag ccg ttg     48
Val Ser Glu Ala Lys Pro Leu His Leu Val Ser Glu Ala Lys Pro Leu
1               5                   10                  15

cac ctg gtc ctg gga gac gaa gaa ctg ctg gtc gaa agg gcg gtg gcc     96
His Leu Val Leu Gly Asp Glu Glu Leu Leu Val Glu Arg Ala Val Ala
            20                  25                  30

gac gtg ttg cgc tcg gct cgg cag cgg gca ggt aca gcc gac gtc ccg     144
Asp Val Leu Arg Ser Ala Arg Gln Arg Ala Gly Thr Ala Asp Val Pro
        35                  40                  45

gtg agc cga atg cgc gcg ggt gac gtc ggt gcc tat gag ctc gcc gaa     192
Val Ser Arg Met Arg Ala Gly Asp Val Gly Ala Tyr Glu Leu Ala Glu
    50                  55                  60

ctg ctg agc ccg tca ctg ttc gcc gag gag cgg atc gtt gtg ctg ggg     240
Leu Leu Ser Pro Ser Leu Phe Ala Glu Glu Arg Ile Val Val Leu Gly
65                  70                  75                  80

gcc gct gcg gag gcg ggc aag gac gct gcc gcg gta atc gag tcg gcc     288
Ala Ala Ala Glu Ala Gly Lys Asp Ala Ala Ala Val Ile Glu Ser Ala
                85                  90                  95

gcc gcc gat ctt ccg gcc ggc acc gtg ctg gta gtg gtc cac tcg ggt     336
Ala Ala Asp Leu Pro Ala Gly Thr Val Leu Val Val Val His Ser Gly
            100                 105                 110

ggc ggg cgc gcc aaa tcg ctg gcc aac cag ctg cgg tcg atg ggt gcg     384
Gly Gly Arg Ala Lys Ser Leu Ala Asn Gln Leu Arg Ser Met Gly Ala
        115                 120                 125

cag gtt cat ccg tgc gcg cgg atc acc aag gtc agt gag cgc gcc gac     432
Gln Val His Pro Cys Ala Arg Ile Thr Lys Val Ser Glu Arg Ala Asp
    130                 135                 140

ttc atc cgt agc gag ttc gcg tcg ctg cgg gtc aag gtc gac gac gag 480
Phe Ile Arg Ser Glu Phe Ala Ser Leu Arg Val Lys Val Asp Asp Glu
145                 150                 155                 160

acc gtg acc gcc ctg ctg gac gcc gtc ggc tcc gac gtg cgc gaa ctc 528
Thr Val Thr Ala Leu Leu Asp Ala Val Gly Ser Asp Val Arg Glu Leu
                165                 170                 175

gcc tcg gcc tgt tca cag ctg gtc gcc gat acc gga gga gcc gtc gac 576
Ala Ser Ala Cys Ser Gln Leu Val Ala Asp Thr Gly Gly Ala Val Asp
            180                 185                 190

gcc gcc gct gta cgg cgc tat cac agc ggc aaa gcc gag gtg agg ggc 624
Ala Ala Ala Val Arg Arg Tyr His Ser Gly Lys Ala Glu Val Arg Gly
        195                 200                 205

ttc gac atc gcc gac aag gcg gta gcc ggc gac gtg gcg gga gct gcc 672
Phe Asp Ile Ala Asp Lys Ala Val Ala Gly Asp Val Ala Gly Ala Ala
    210                 215                 220

gaa gcg ttg cgg tgg gcg atg atg cgc ggt gag ccg cta gtg gtg ttg 720
Glu Ala Leu Arg Trp Ala Met Met Arg Gly Glu Pro Leu Val Val Leu
225                 230                 235                 240

gcc gat gcg ctc gcc gaa gcc gtg cac acc atc ggc cgg gtc ggg ccg 768
Ala Asp Ala Leu Ala Glu Ala Val His Thr Ile Gly Arg Val Gly Pro
                245                 250                 255

cag tcc ggc gac ccg tac cgc ctg gcc gca caa ctg ggg atg ccg ccc 816
Gln Ser Gly Asp Pro Tyr Arg Leu Ala Ala Gln Leu Gly Met Pro Pro
            260                 265                 270

tgg cgg gtg cag aaa gcc cag aag cag gct cgg cgg tgg tcg cgt gac 864
Trp Arg Val Gln Lys Ala Gln Lys Gln Ala Arg Arg Trp Ser Arg Asp
        275                 280                 285

acg gtg gcg acc gcg atg agg ttg gtg gcc gaa ctc aat gct aac gtc 912
Thr Val Ala Thr Ala Met Arg Leu Val Ala Glu Leu Asn Ala Asn Val
    290                 295                 300

aag ggc gcc gtc gcg gat gcg gac tac gcg ctg gaa tcc gcg gtc cgg 960
Lys Gly Ala Val Ala Asp Ala Asp Tyr Ala Leu Glu Ser Ala Val Arg
305                 310                 315                 320

cag gtc gcc gag ttg gtg gcc gac cgc ggc cga tga 996
Gln Val Ala Glu Leu Val Ala Asp Arg Gly Arg
                325                 330

<210> 225
<211> 331
<212> PRT
<213> Mycobacterium tuberculosis

<400> 225
Val Ser Glu Ala Lys Pro Leu His Leu Val Ser Glu Ala Lys Pro Leu
1               5                   10                  15

His Leu Val Leu Gly Asp Glu Glu Leu Leu Val Glu Arg Ala Val Ala
            20                  25                  30

Asp Val Leu Arg Ser Ala Arg Gln Arg Ala Gly Thr Ala Asp Val Pro
        35                  40                  45

Val Ser Arg Met Arg Ala Gly Asp Val Gly Ala Tyr Glu Leu Ala Glu
    50                  55                  60

Leu Leu Ser Pro Ser Leu Phe Ala Glu Glu Arg Ile Val Val Leu Gly
65                  70                  75                  80

Ala Ala Ala Glu Ala Gly Lys Asp Ala Ala Ala Val Ile Glu Ser Ala
                85                  90                  95

Ala Ala Asp Leu Pro Ala Gly Thr Val Leu Val Val Val His Ser Gly
            100                 105                 110

Gly Gly Arg Ala Lys Ser Leu Ala Asn Gln Leu Arg Ser Met Gly Ala
        115                 120                 125

Gln Val His Pro Cys Ala Arg Ile Thr Lys Val Ser Glu Arg Ala Asp
    130                 135                 140

Phe Ile Arg Ser Glu Phe Ala Ser Leu Arg Val Lys Val Asp Asp Glu
145                 150                 155                 160

Thr Val Thr Ala Leu Leu Asp Ala Val Gly Ser Asp Val Arg Glu Leu
                165                 170                 175

Ala Ser Ala Cys Ser Gln Leu Val Ala Asp Thr Gly Gly Ala Val Asp
            180                 185                 190

Ala Ala Ala Val Arg Arg Tyr His Ser Gly Lys Ala Glu Val Arg Gly
        195                 200                 205

Phe Asp Ile Ala Asp Lys Ala Val Ala Gly Asp Val Ala Gly Ala Ala
    210                 215                 220

Glu Ala Leu Arg Trp Ala Met Met Arg Gly Glu Pro Leu Val Val Leu
225                 230                 235                 240

Ala Asp Ala Leu Ala Glu Ala Val His Thr Ile Gly Arg Val Gly Pro
                245                 250                 255

Gln Ser Gly Asp Pro Tyr Arg Leu Ala Ala Gln Leu Gly Met Pro Pro
            260                 265                 270

Trp Arg Val Gln Lys Ala Gln Lys Gln Ala Arg Arg Trp Ser Arg Asp
        275                 280                 285

Thr Val Ala Thr Ala Met Arg Leu Val Ala Glu Leu Asn Ala Asn Val
    290                 295                 300

Lys Gly Ala Val Ala Asp Ala Asp Tyr Ala Leu Glu Ser Ala Val Arg
305                 310                 315                 320

Gln Val Ala Glu Leu Val Ala Asp Arg Gly Arg
                325                 330

<210> 226
<211> 350
<212> PRT
<213> Aquifex aeolicus

<400> 226
Met Glu Thr Thr Ile Phe Gln Phe Gln Lys Thr Phe Phe Thr Lys Pro
  1               5                  10                  15

Pro Lys Glu Arg Val Phe Val Leu His Gly Glu Glu Gln Tyr Leu Ile
             20                  25                  30

Arg Thr Phe Leu Ser Lys Leu Lys Glu Lys Tyr Gly Glu Asn Tyr Thr
         35                  40                  45

Val Leu Trp Gly Asp Glu Ile Ser Glu Glu Glu Phe Tyr Thr Ala Leu
     50                  55                  60

Ser Glu Thr Ser Ile Phe Gly Gly Ser Lys Glu Lys Ala Val Val Ile
 65                  70                  75                  80

Tyr Asn Phe Gly Asp Phe Leu Lys Lys Leu Gly Arg Lys Lys Lys Glu
                 85                  90                  95

Lys Glu Arg Leu Ile Lys Val Leu Arg Asn Val Lys Ser Asn Tyr Val

            100                 105                 110

Phe Ile Val Tyr Asp Ala Lys Leu Gln Lys Gln Glu Leu Ser Ser Glu
        115                 120                 125

Pro Leu Lys Ser Val Ala Ser Phe Gly Gly Ile Val Val Ala Asn Arg
    130                 135                 140

Leu Ser Lys Glu Arg Ile Lys Gln Leu Val Leu Lys Lys Phe Lys Glu
145                 150                 155                 160

Lys Gly Ile Asn Val Glu Asn Asp Ala Leu Glu Tyr Leu Leu Gln Leu
                165                 170                 175

Thr Gly Tyr Asn Leu Met Glu Leu Lys Leu Glu Val Glu Lys Leu Ile
            180                 185                 190

Asp Tyr Ala Ser Glu Lys Lys Ile Leu Thr Leu Asp Glu Val Lys Arg
        195                 200                 205

Val Ala Phe Ser Val Ser Glu Asn Val Asn Val Phe Glu Phe Val Asp
    210                 215                 220

Leu Leu Leu Leu Lys Asp Tyr Glu Lys Ala Leu Lys Val Leu Asp Ser
225                 230                 235                 240

Leu Ile Ser Phe Gly Ile His Pro Leu Gln Ile Met Lys Ile Leu Ser
                245                 250                 255

Ser Tyr Ala Leu Lys Leu Tyr Thr Leu Lys Arg Leu Glu Glu Lys Gly
            260                 265                 270

Glu Asp Leu Asn Lys Ala Met Glu Ser Val Gly Ile Lys Asn Asn Phe
        275                 280                 285

Leu Lys Met Lys Phe Lys Ser Tyr Leu Lys Ala Asn Ser Lys Glu Asp
    290                 295                 300

Leu Lys Asn Leu Ile Leu Ser Leu Gln Arg Ile Asp Ala Phe Ser Lys
305                 310                 315                 320

Leu Tyr Phe Gln Asp Thr Val Gln Leu Leu Arg Asp Phe Leu Thr Ser
                325                 330                 335

Arg Leu Glu Arg Glu Val Val Lys Asn Thr Ser His Gly Gly
            340                 345                 350

<210> 227
<211> 316
<212> PRT
<213> Mycobacterium tuberculosis

<400> 227
Met His Leu Val Leu Gly Asp Glu Glu Leu Leu Val Glu Arg Ala Val
1               5                   10                  15

Ala Asp Val Leu Arg Ser Ala Arg Gln Arg Ala Gly Thr Ala Asp Val
            20                  25                  30

Pro Val Ser Arg Met Arg Ala Gly Asp Val Gly Ala Tyr Glu Leu Ala
        35                  40                  45

Glu Leu Leu Ser Pro Ser Leu Phe Ala Glu Glu Arg Ile Val Val Leu
    50                  55                  60

Gly Ala Ala Ala Glu Ala Gly Lys Asp Ala Ala Ala Val Ile Glu Ser
65                  70                  75                  80

Ala Ala Ala Asp Leu Pro Ala Gly Thr Val Leu Val Val Val His Ser
                85                  90                  95

Gly Gly Gly Arg Ala Lys Ser Leu Ala Asn Gln Leu Arg Ser Met Gly
            100                 105                 110

Ala Gln Val His Pro Cys Ala Arg Ile Thr Lys Val Ser Glu Arg Ala
        115                 120                 125

Asp Phe Ile Arg Ser Glu Phe Ala Ser Leu Arg Val Lys Val Asp Asp
    130                 135                 140

Glu Thr Val Thr Ala Leu Leu Asp Ala Val Gly Ser Asp Val Arg Glu
145                 150                 155                 160

Leu Ala Ser Ala Cys Ser Gln Leu Val Ala Asp Thr Gly Gly Ala Val
                165                 170                 175

Asp Ala Ala Ala Val Arg Arg Tyr His Ser Gly Lys Ala Glu Val Arg
            180                 185                 190

Gly Phe Asp Ile Ala Asp Lys Ala Val Ala Gly Asp Val Ala Gly Ala
        195                 200                 205

Ala Glu Ala Leu Arg Trp Ala Met Met Arg Gly Glu Pro Leu Val Val
    210                 215                 220

Leu Ala Asp Ala Leu Ala Glu Ala Val His Thr Ile Gly Arg Val Gly
225                 230                 235                 240

Pro Gln Ser Gly Asp Pro Tyr Arg Leu Ala Ala Gln Leu Gly Met Pro
                245                 250                 255

Pro Trp Arg Val Gln Lys Ala Gln Lys Gln Ala Arg Arg Trp Ser Arg
            260                 265                 270

Asp Thr Val Ala Thr Ala Met Arg Leu Val Ala Glu Leu Asn Ala Asn
        275                 280                 285

Val Lys Gly Ala Val Ala Asp Ala Asp Tyr Ala Leu Glu Ser Ala Val
    290                 295                 300

Arg Gln Val Ala Glu Leu Val Ala Asp Arg Gly Arg
305                 310                 315

<210> 228
<211> 322
<212> PRT
<213> Mycobacterium tuberculosis

<400> 228
Met Ser Glu Ala Lys Pro Leu His Leu Val Leu Gly Asp Glu Glu Leu
  1               5                  10                  15

Leu Val Glu Arg Ala Val Ala Asp Val Leu Arg Ser Ala Arg Gln Arg
             20                  25                  30

Ala Gly Thr Ala Asp Val Pro Val Ser Arg Met Arg Ala Gly Asp Val
         35                  40                  45

Gly Ala Tyr Glu Leu Ala Glu Leu Leu Ser Pro Ser Leu Phe Ala Glu
     50                  55                  60

Glu Arg Ile Val Val Leu Gly Ala Ala Ala Glu Ala Gly Lys Asp Ala
 65                  70                  75                  80

Ala Ala Val Ile Glu Ser Ala Ala Ala Asp Leu Pro Ala Gly Thr Val
                 85                  90                  95

Leu Val Val Val His Ser Gly Gly Gly Arg Ala Lys Ser Leu Ala Asn
            100                 105                 110

Gln Leu Arg Ser Met Gly Ala Gln Val His Pro Cys Ala Arg Ile Thr
        115                 120                 125

Lys Val Ser Glu Arg Ala Asp Phe Ile Arg Ser Glu Phe Ala Ser Leu
    130                 135                 140

Arg Val Lys Val Asp Asp Glu Thr Val Thr Ala Leu Leu Asp Ala Val
145                 150                 155                 160

Gly Ser Asp Val Arg Glu Leu Ala Ser Ala Cys Ser Gln Leu Val Ala
                165                 170                 175

Asp Thr Gly Gly Ala Val Asp Ala Ala Ala Val Arg Arg Tyr His Ser
            180                 185                 190

Gly Lys Ala Glu Val Arg Gly Phe Asp Ile Ala Asp Lys Ala Val Ala
        195                 200                 205

Gly Asp Val Ala Gly Ala Ala Glu Ala Leu Arg Trp Ala Met Met Arg
    210                 215                 220

Gly Glu Pro Leu Val Val Leu Ala Asp Ala Leu Ala Glu Ala Val His
225                 230                 235                 240

Thr Ile Gly Arg Val Gly Pro Gln Ser Gly Asp Pro Tyr Arg Leu Ala
                245                 250                 255

Ala Gln Leu Gly Met Pro Pro Trp Arg Val Gln Lys Ala Gln Lys Gln
            260                 265                 270

Ala Arg Arg Trp Ser Arg Asp Thr Val Ala Thr Ala Met Arg Leu Val
        275                 280                 285

Ala Glu Leu Asn Ala Asn Val Lys Gly Ala Val Ala Asp Ala Asp Tyr
    290                 295                 300

Ala Leu Glu Ser Ala Val Arg Gln Val Ala Glu Leu Val Ala Asp Arg
305                 310                 315                 320

Gly Arg

<210> 229
<211> 336
<212> PRT
<213> Streptomyces coelicolor

<400> 229
Met Arg Ala Met Leu Val Ala Met Ala Arg Lys Thr Ala Asn Asp Asp

1               5                   10                  15

Pro Leu Ala Pro Val Thr Leu Ala Val Gly Gln Glu Asp Leu Leu Leu
                20                  25                  30

Asp Arg Ala Val Gln Glu Val Val Ala Ala Ala Lys Ala Ala Asp Ala
            35                  40                  45

Asp Thr Asp Val Arg Asp Leu Thr Pro Asp Gln Leu Gln Pro Gly Thr
        50                  55                  60

Leu Ala Glu Leu Thr Ser Pro Ser Leu Phe Ala Glu Arg Lys Val Val
65                  70                  75                  80

Val Val Arg Asn Ala Gln Asp Leu Ser Ala Asp Thr Val Lys Asp Val
                85                  90                  95

Lys Ala Tyr Leu Gly Ala Pro Ala Glu Glu Ile Thr Leu Val Leu Leu
            100                 105                 110

His Ala Gly Gly Ala Lys Gly Lys Gly Val Leu Asp Ala Gly Arg Lys
        115                 120                 125

Ala Gly Ala Arg Glu Val Ala Cys Pro Lys Met Thr Lys Pro Ala Asp
    130                 135                 140

Arg Leu Ala Phe Val Arg Ala Glu Phe Arg Thr Ala Gly Arg Ser Ala
145                 150                 155                 160

Thr Pro Glu Ala Cys Gln Ala Leu Val Asp Ala Ile Gly Ser Asp Leu
                165                 170                 175

Arg Glu Leu Ala Ser Ala Val Ser Gln Leu Thr Ala Asp Val Glu Gly
            180                 185                 190

Thr Val Asp Glu Ala Val Val Gly Arg Tyr Tyr Thr Gly Arg Ala Glu
        195                 200                 205

Ala Ser Ser Phe Thr Val Ala Asp Arg Ala Val Glu Gly Arg Ala Ala
    210                 215                 220

Glu Ala Leu Glu Ala Leu Arg Trp Ser Leu Ala Thr Gly Val Ala Pro
225                 230                 235                 240

Val Leu Ile Thr Ser Ala Leu Ala Gln Gly Val Arg Ala Ile Gly Lys
                245                 250                 255

Leu Ser Ser Ala Arg Gly Gly Arg Pro Ala Asp Leu Ala Arg Glu Leu

260                 265                 270

Gly Met Pro Pro Trp Lys Ile Asp Arg Val Arg Gln Gln Met Arg Gly
        275                 280                 285

Trp Thr Pro Asp Gly Val Ser Val Ala Leu Arg Ala Val Ala Glu Ala
    290                 295                 300

Asp Ala Gly Val Lys Gly Gly Gly Asp Asp Pro Glu Tyr Ala Leu Glu
305                 310                 315                 320

Lys Ala Val Val Ile Ile Ala Arg Ala Ala Arg Ser Arg Gly Arg Thr
                325                 330                 335

<210> 230
<211> 478
<212> PRT
<213> Thermotoga maritima

<400> 230
Met Glu Val Leu Tyr Arg Lys Tyr Arg Pro Lys Thr Phe Ser Glu Val
  1               5                  10                  15

Val Asn Gln Asp His Val Lys Lys Ala Ile Ile Gly Ala Ile Gln Lys
             20                  25                  30

Asn Ser Val Ala His Gly Tyr Ile Phe Ala Gly Pro Arg Gly Thr Gly
         35                  40                  45

Lys Thr Thr Leu Ala Arg Ile Leu Ala Lys Ser Leu Asn Cys Glu Asn
     50                  55                  60

Arg Lys Gly Val Glu Pro Cys Asn Ser Cys Arg Ala Cys Arg Glu Ile
 65                  70                  75                  80

Asp Glu Gly Thr Phe Met Asp Val Ile Glu Leu Asp Ala Ala Ser Asn
                 85                  90                  95

Arg Gly Ile Asp Glu Ile Arg Arg Ile Arg Asp Ala Val Gly Tyr Arg
            100                 105                 110

Pro Met Glu Gly Lys Tyr Lys Val Tyr Ile Ile Asp Glu Val His Met
        115                 120                 125

Leu Thr Lys Glu Ala Phe Asn Ala Leu Leu Lys Thr Leu Glu Glu Pro
    130                 135                 140

Pro Ser His Val Val Phe Val Leu Ala Thr Thr Asn Leu Glu Lys Val
145                 150                 155                 160

Pro Pro Thr Ile Ile Ser Arg Cys Gln Val Phe Glu Phe Arg Asn Ile
                165                 170                 175

Pro Asp Glu Leu Ile Glu Lys Arg Leu Gln Glu Val Ala Glu Ala Glu
            180                 185                 190

Gly Ile Glu Ile Asp Arg Glu Ala Leu Ser Phe Ile Ala Lys Arg Ala
        195                 200                 205

Ser Gly Gly Leu Arg Asp Ala Leu Thr Met Leu Glu Gln Val Trp Lys
    210                 215                 220

Phe Ser Glu Gly Lys Ile Asp Leu Glu Thr Val His Arg Ala Leu Gly
225                 230                 235                 240

Leu Ile Pro Ile Gln Val Val Arg Asp Tyr Val Asn Ala Ile Phe Ser
                245                 250                 255

Gly Asp Val Lys Arg Val Phe Thr Val Leu Asp Asp Val Tyr Tyr Ser
            260                 265                 270

Gly Lys Asp Tyr Glu Val Leu Ile Gln Glu Ala Val Glu Asp Leu Val
        275                 280                 285

Glu Asp Leu Glu Arg Glu Arg Gly Val Tyr Gln Val Ser Ala Asn Asp
    290                 295                 300

Ile Val Gln Val Ser Arg Gln Leu Leu Asn Leu Leu Arg Glu Ile Lys
305                 310                 315                 320

Phe Ala Glu Glu Lys Arg Leu Val Cys Lys Val Gly Ser Ala Tyr Ile
                325                 330                 335

Ala Thr Arg Phe Ser Thr Thr Asn Val Gln Glu Asn Asp Val Arg Glu
            340                 345                 350

Lys Asn Asp Asn Ser Asn Val Gln Gln Lys Glu Glu Lys Lys Glu Thr
        355                 360                 365

Val Lys Ala Lys Glu Glu Lys Gln Glu Asp Ser Glu Phe Glu Lys Arg
    370                 375                 380

Phe Lys Glu Leu Met Glu Glu Leu Lys Glu Lys Gly Asp Leu Ser Ile
385                 390                 395                 400

Phe Val Ala Leu Ser Leu Ser Glu Val Gln Phe Asp Gly Glu Lys Val
                405                 410                 415

Ile Ile Ser Phe Asp Ser Ser Lys Ala Met His Tyr Glu Leu Met Lys
            420                 425                 430

Lys Lys Leu Pro Glu Leu Glu Asn Ile Phe Ser Arg Lys Leu Gly Lys
        435                 440                 445

Lys Val Glu Val Glu Leu Arg Leu Met Gly Lys Glu Glu Thr Ile Glu
    450                 455                 460

Lys Val Ser Gln Lys Ile Leu Arg Leu Phe Glu Gln Glu Gly
465                 470                 475

SEQUENCE LISTING

<110> Bullard, James M.
      Janjic, Nebojsa
      McHenry, Charles S.
      Replidyne, Inc.

<120> NOVEL DNA POLYMERASE III HOLOENZYME DELTA SUBUNIT
      NUCLEIC ACID MOLECULES AND PROTEINS

<130> RDYN03PCT

<140> EP 06024949.7
<141> 2006/12/01

<150> 06024949.7
<151> 2006-01-12

<160> 230

<170> PatentIn Ver. 2.0

<210> 1
<211> 1053
<212> DNA
<213> Aquifex aeolicus

<400> 1
gtggaaacca caatattcca gttccagaaa acttttttca caaaacctcc gaaggagagg 60

gtcttcgtcc ttcatggaga agagcagtat ctcataagaa cctttttgtc taagctgaag 120

gaaaagtacg gggagaatta cacggttctg tggggggatg agataagcga ggaggaattc 180

tacactgccc tttccgagac cagtatattc ggcggttcaa aggaaaaagc ggtggtcatt 240

tacaacttcg gggatttcct gaagaagctc ggaaggaaga aaaaggaaaa agaaaggctt 300

ataaaagtcc tcagaaacgt aaagagtaac tacgtattta tagtgtacga tgcgaaactc 360

cagaaacagg aactttcttc ggaacctctg aaatccgtag cgtctttcgg cggtatagtg 420

gtagcaaaca ggctgagcaa ggagaggata aaacagctcg tccttaagaa gttcaaagaa 480

aaagggataa acgtagaaaa cgatgccctt gaataccttc tccagctcac gggttacaac 540

ttgatggagc tcaaacttga ggttgaaaaa ctgatagatt acgcaagtga aaagaaaatt 600

ttaacactcg atgaggtaaa gagagtagcc ttctcagtct cagaaaacgt aaacgtattt 660

gagttcgttg atttactcct cttaaaagat tacgaaaagg ctcttaaagt tttggactcc 720

ctcatttcct tcggaataca ccccctccag attatgaaaa tcctgtcctc ctatgctcta 780

aaactttaca ccctcaagag gcttgaagag aagggagagg acctgaataa ggcgatggaa 840

agcgtgggaa taaagaacaa ctttctcaag atgaagttca aatcttactt aaaggcaaac 900

tctaaagagg acttgaagaa cctaatcctc tccctccaga ggatagacgc tttttctaaa 960

ctttactttc aggacacagt gcagttgctg agggatttct tgacctcaag actggagagg 1020

gaagttgtga aaaatacttc tcatggtgga taa                              1053

<210> 2
<211> 1053
<212> DNA

<213> Aquifex aeolicus

<220>
<221> CDS
<222> (1)..(1053)

<400> 2
gtg gaa acc aca ata ttc cag ttc cag aaa act ttt ttc aca aaa cct     48
Val Glu Thr Thr Ile Phe Gln Phe Gln Lys Thr Phe Phe Thr Lys Pro
1               5                   10                  15

ccg aag gag agg gtc ttc gtc ctt cat gga gaa gag cag tat ctc ata     96
Pro Lys Glu Arg Val Phe Val Leu His Gly Glu Glu Gln Tyr Leu Ile
            20                  25                  30

aga acc ttt ttg tct aag ctg aag gaa aag tac ggg gag aat tac acg     144
Arg Thr Phe Leu Ser Lys Leu Lys Glu Lys Tyr Gly Glu Asn Tyr Thr
        35                  40                  45

gtt ctg tgg ggg gat gag ata agc gag gag gaa ttc tac act gcc ctt     192
Val Leu Trp Gly Asp Glu Ile Ser Glu Glu Glu Phe Tyr Thr Ala Leu
    50                  55                  60

tcc gag acc agt ata ttc ggc ggt tca aag gaa aaa gcg gtg gtc att     240
Ser Glu Thr Ser Ile Phe Gly Gly Ser Lys Glu Lys Ala Val Val Ile
65                  70                  75                  80

tac aac ttc ggg gat ttc ctg aag aag ctc gga agg aag aaa aag gaa     288
Tyr Asn Phe Gly Asp Phe Leu Lys Lys Leu Gly Arg Lys Lys Lys Glu
                85                  90                  95

aaa gaa agg ctt ata aaa gtc ctc aga aac gta aag agt aac tac gta     336
Lys Glu Arg Leu Ile Lys Val Leu Arg Asn Val Lys Ser Asn Tyr Val
            100                 105                 110

ttt ata gtg tac gat gcg aaa ctc cag aaa cag gaa ctt tct tcg gaa     384
Phe Ile Val Tyr Asp Ala Lys Leu Gln Lys Gln Glu Leu Ser Ser Glu
        115                 120                 125

cct ctg aaa tcc gta gcg tct ttc ggc ggt ata gtg gta gca aac agg     432
Pro Leu Lys Ser Val Ala Ser Phe Gly Gly Ile Val Val Ala Asn Arg
    130                 135                 140

ctg agc aag gag agg ata aaa cag ctc gtc ctt aag aag ttc aaa gaa     480
Leu Ser Lys Glu Arg Ile Lys Gln Leu Val Leu Lys Lys Phe Lys Glu
145                 150                 155                 160

aaa ggg ata aac gta gaa aac gat gcc ctt gaa tac ctt ctc cag ctc     528
Lys Gly Ile Asn Val Glu Asn Asp Ala Leu Glu Tyr Leu Leu Gln Leu
                165                 170                 175

acg ggt tac aac ttg atg gag ctc aaa ctt gag gtt gaa aaa ctg ata     576
Thr Gly Tyr Asn Leu Met Glu Leu Lys Leu Glu Val Glu Lys Leu Ile
            180                 185                 190

gat tac gca agt gaa aag aaa att tta aca ctc gat gag gta aag aga     624
Asp Tyr Ala Ser Glu Lys Lys Ile Leu Thr Leu Asp Glu Val Lys Arg
        195                 200                 205

gta gcc ttc tca gtc tca gaa aac gta aac gta ttt gag ttc gtt gat     672
Val Ala Phe Ser Val Ser Glu Asn Val Asn Val Phe Glu Phe Val Asp
    210                 215                 220

tta ctc ctc tta aaa gat tac gaa aag gct ctt aaa gtt ttg gac tcc     720
Leu Leu Leu Leu Lys Asp Tyr Glu Lys Ala Leu Lys Val Leu Asp Ser
225                 230                 235                 240

ctc att tcc ttc gga ata cac ccc ctc cag att atg aaa atc ctg tcc     768

Leu Ile Ser Phe Gly Ile His Pro Leu Gln Ile Met Lys Ile Leu Ser
                245                 250                 255

tcc tat gct cta aaa ctt tac acc ctc aag agg ctt gaa gag aag gga     816
Ser Tyr Ala Leu Lys Leu Tyr Thr Leu Lys Arg Leu Glu Glu Lys Gly
            260                 265                 270

gag gac ctg aat aag gcg atg gaa agc gtg gga ata aag aac aac ttt     864
Glu Asp Leu Asn Lys Ala Met Glu Ser Val Gly Ile Lys Asn Asn Phe
        275                 280                 285

ctc aag atg aag ttc aaa tct tac tta aag gca aac tct aaa gag gac     912
Leu Lys Met Lys Phe Lys Ser Tyr Leu Lys Ala Asn Ser Lys Glu Asp
    290                 295                 300

ttg aag aac cta atc ctc tcc ctc cag agg ata gac gct ttt tct aaa     960
Leu Lys Asn Leu Ile Leu Ser Leu Gln Arg Ile Asp Ala Phe Ser Lys
305                 310                 315                 320

ctt tac ttt cag gac aca gtg cag ttg ctg agg gat ttc ttg acc tca    1008
Leu Tyr Phe Gln Asp Thr Val Gln Leu Leu Arg Asp Phe Leu Thr Ser
                325                 330                 335

aga ctg gag agg gaa gtt gtg aaa aat act tct cat ggt gga taa        1053
Arg Leu Glu Arg Glu Val Val Lys Asn Thr Ser His Gly Gly
            340                 345                 350


<210> 3
<211> 350
<212> PRT
<213> Aquifex aeolicus

<400> 3
Val Glu Thr Thr Ile Phe Gln Phe Gln Lys Thr Phe Phe Thr Lys Pro
1               5                   10                  15

Pro Lys Glu Arg Val Phe Val Leu His Gly Glu Glu Gln Tyr Leu Ile
            20                  25                  30

Arg Thr Phe Leu Ser Lys Leu Lys Glu Lys Tyr Gly Glu Asn Tyr Thr
        35                  40                  45

Val Leu Trp Gly Asp Glu Ile Ser Glu Glu Glu Phe Tyr Thr Ala Leu
    50                  55                  60

Ser Glu Thr Ser Ile Phe Gly Gly Ser Lys Glu Lys Ala Val Val Ile
65                  70                  75                  80

Tyr Asn Phe Gly Asp Phe Leu Lys Lys Leu Gly Arg Lys Lys Lys Glu
                85                  90                  95

Lys Glu Arg Leu Ile Lys Val Leu Arg Asn Val Lys Ser Asn Tyr Val
            100                 105                 110

Phe Ile Val Tyr Asp Ala Lys Leu Gln Lys Gln Glu Leu Ser Ser Glu
        115                 120                 125

Pro Leu Lys Ser Val Ala Ser Phe Gly Gly Ile Val Val Ala Asn Arg
    130                 135                 140

Leu Ser Lys Glu Arg Ile Lys Gln Leu Val Leu Lys Lys Phe Lys Glu
145                 150                 155                 160

Lys Gly Ile Asn Val Glu Asn Asp Ala Leu Glu Tyr Leu Leu Gln Leu
                165                 170                 175

Thr Gly Tyr Asn Leu Met Glu Leu Lys Leu Glu Val Glu Lys Leu Ile

            180                 185                 190

Asp Tyr Ala Ser Glu Lys Lys Ile Leu Thr Leu Asp Glu Val Lys Arg
        195                 200                 205

Val Ala Phe Ser Val Ser Glu Asn Val Asn Val Phe Glu Phe Val Asp
    210                 215                 220

Leu Leu Leu Leu Lys Asp Tyr Glu Lys Ala Leu Lys Val Leu Asp Ser
225                 230                 235                 240

Leu Ile Ser Phe Gly Ile His Pro Leu Gln Ile Met Lys Ile Leu Ser
                245                 250                 255

Ser Tyr Ala Leu Lys Leu Tyr Thr Leu Lys Arg Leu Glu Glu Lys Gly
            260                 265                 270

Glu Asp Leu Asn Lys Ala Met Glu Ser Val Gly Ile Lys Asn Asn Phe
        275                 280                 285

Leu Lys Met Lys Phe Lys Ser Tyr Leu Lys Ala Asn Ser Lys Glu Asp
    290                 295                 300

Leu Lys Asn Leu Ile Leu Ser Leu Gln Arg Ile Asp Ala Phe Ser Lys
305                 310                 315                 320

Leu Tyr Phe Gln Asp Thr Val Gln Leu Leu Arg Asp Phe Leu Thr Ser
                325                 330                 335

Arg Leu Glu Arg Glu Val Val Lys Asn Thr Ser His Gly Gly
            340                 345                 350

<210> 4
<211> 1029
<212> DNA
<213> Bacillus halodurans

<400> 4
atgaactatt taaaattaaa gcaagatgtc caagcgggac gagtggcccc tgtatatttt 60

atgtatggga ccgagttgtt tttaatggaa gacctcatac aggacatttt gtccgtaacg 120

ttatcggatg aggaaaggga tatgaatgtt tcatcttatt cccttactga tgttccgatt 180

gaggcggcgc tcgaagaagc agaaacggtt cctttttttg gctcgaagcg ggtcgtgatc 240

ttaaaagatg ctgccctatt tacaagccaa aagcttgatg ttgagcacga tgtgaaacga 300

ttggagcaat acatattgaa cccagttcca gagacggttt tgctgatcat ggccccttat 360

gaaaagctcg atgaacgaaa aaaaatcacg aagcttatca aaaaagagtc gcttgttctt 420

gaggcaaagc ctttggacga aggagaggca aaagcatggc tatcctcact ggcaagcgag 480

cttcaggtgg agatggacga gaaagctatc gagacgttgt tagggatgac gggtcttcgc 540

ttgacccaat tagcatctga aatgaataaa ttagcgttgt atgtagggga aggaggcatc 600

atccgatcag aggacgtaac cttgctcgtt gctaaaacgc tcgatcaaaa catatttgat 660

ctgattgatt ttgccatcaa tcagaggact catcaagcgc tctctcttta ccatgagctc 720

ctgaaacaaa aagaggagcc attgaagctg ttggccttat taacacggca gtttcgcatc 780

atgtaccaag tcaaagagct tggacgaaga ggatacaccc cgaatcagat ggcgaagccg 840

ttaaaaattc atccctatgt tgccaaactt gcgggaaaaa aagcagccag catgtcagac 900

caactgttat attccctaat cgaaaaagct gcggatacag agtttgcgat taaaagtgga 960

aaggttgata aagtgctggc attagaattg tttttactca caatgggttc tagggagagc 1020

gtaggttag 1029

<210> 5
<211> 1029
<212> DNA
<213> Bacillus halodurans

<220>
<221> CDS
<222> (1)..(1029)

<400> 5
atg aac tat tta aaa tta aag caa gat gtc caa gcg gga cga gtg gcc 48
Met Asn Tyr Leu Lys Leu Lys Gln Asp Val Gln Ala Gly Arg Val Ala
1               5                   10                  15

cct gta tat ttt atg tat ggg acc gag ttg ttt tta atg gaa gac ctc 96
Pro Val Tyr Phe Met Tyr Gly Thr Glu Leu Phe Leu Met Glu Asp Leu
            20                  25                  30

ata cag gac att ttg tcc gta acg tta tcg gat gag gaa agg gat atg 144
Ile Gln Asp Ile Leu Ser Val Thr Leu Ser Asp Glu Glu Arg Asp Met
        35                  40                  45

aat gtt tca tct tat tcc ctt act gat gtt ccg att gag gcg gcg ctc 192
Asn Val Ser Ser Tyr Ser Leu Thr Asp Val Pro Ile Glu Ala Ala Leu
    50                  55                  60

gaa gaa gca gaa acg gtt cct ttt ttt ggc tcg aag cgg gtc gtg atc 240
Glu Glu Ala Glu Thr Val Pro Phe Phe Gly Ser Lys Arg Val Val Ile
65                  70                  75                  80

tta aaa gat gct gcc cta ttt aca agc caa aag ctt gat gtt gag cac 288
Leu Lys Asp Ala Ala Leu Phe Thr Ser Gln Lys Leu Asp Val Glu His
                85                  90                  95

gat gtg aaa cga ttg gag caa tac ata ttg aac cca gtt cca gag acg 336
Asp Val Lys Arg Leu Glu Gln Tyr Ile Leu Asn Pro Val Pro Glu Thr
            100                 105                 110

gtt ttg ctg atc atg gcc cct tat gaa aag ctc gat gaa cga aaa aaa 384
Val Leu Leu Ile Met Ala Pro Tyr Glu Lys Leu Asp Glu Arg Lys Lys
        115                 120                 125

atc acg aag ctt atc aaa aaa gag tcg ctt gtt ctt gag gca aag cct 432
Ile Thr Lys Leu Ile Lys Lys Glu Ser Leu Val Leu Glu Ala Lys Pro
    130                 135                 140

ttg gac gaa gga gag gca aaa gca tgg cta tcc tca ctg gca agc gag 480
Leu Asp Glu Gly Glu Ala Lys Ala Trp Leu Ser Ser Leu Ala Ser Glu
145                 150                 155                 160

ctt cag gtg gag atg gac gag aaa gct atc gag acg ttg tta ggg atg 528
Leu Gln Val Glu Met Asp Glu Lys Ala Ile Glu Thr Leu Leu Gly Met
                165                 170                 175

acg ggt ctt cgc ttg acc caa tta gca tct gaa atg aat aaa tta gcg 576
Thr Gly Leu Arg Leu Thr Gln Leu Ala Ser Glu Met Asn Lys Leu Ala
            180                 185                 190

ttg tat gta ggg gaa gga ggc atc atc cga tca gag gac gta acc ttg 624
Leu Tyr Val Gly Glu Gly Gly Ile Ile Arg Ser Glu Asp Val Thr Leu

195                 200                 205

ctc gtt gct aaa acg ctc gat caa aac ata ttt gat ctg att gat ttt     672
Leu Val Ala Lys Thr Leu Asp Gln Asn Ile Phe Asp Leu Ile Asp Phe
    210                 215                 220

gcc atc aat cag agg act cat caa gcg ctc tct ctt tac cat gag ctc     720
Ala Ile Asn Gln Arg Thr His Gln Ala Leu Ser Leu Tyr His Glu Leu
225                 230                 235                 240

ctg aaa caa aaa gag gag cca ttg aag ctg ttg gcc tta tta aca cgg     768
Leu Lys Gln Lys Glu Glu Pro Leu Lys Leu Leu Ala Leu Leu Thr Arg
                245                 250                 255

cag ttt cgc atc atg tac caa gtc aaa gag ctt gga cga aga gga tac     816
Gln Phe Arg Ile Met Tyr Gln Val Lys Glu Leu Gly Arg Arg Gly Tyr
            260                 265                 270

acc ccg aat cag atg gcg aag ccg tta aaa att cat ccc tat gtt gcc     864
Thr Pro Asn Gln Met Ala Lys Pro Leu Lys Ile His Pro Tyr Val Ala
        275                 280                 285

aaa ctt gcg gga aaa aaa gca gcc agc atg tca gac caa ctg tta tat     912
Lys Leu Ala Gly Lys Lys Ala Ala Ser Met Ser Asp Gln Leu Leu Tyr
    290                 295                 300

tcc cta atc gaa aaa gct gcg gat aca gag ttt gcg att aaa agt gga     960
Ser Leu Ile Glu Lys Ala Ala Asp Thr Glu Phe Ala Ile Lys Ser Gly
305                 310                 315                 320

aag gtt gat aaa gtg ctg gca tta gaa ttg ttt tta ctc aca atg ggt     1008
Lys Val Asp Lys Val Leu Ala Leu Glu Leu Phe Leu Leu Thr Met Gly
                325                 330                 335

tct agg gag agc gta ggt tag                                         1029
Ser Arg Glu Ser Val Gly
            340

<210> 6
<211> 342
<212> PRT
<213> Bacillus halodurans

<400> 6
Met Asn Tyr Leu Lys Leu Lys Gln Asp Val Gln Ala Gly Arg Val Ala
1               5                   10                  15

Pro Val Tyr Phe Met Tyr Gly Thr Glu Leu Phe Leu Met Glu Asp Leu
            20                  25                  30

Ile Gln Asp Ile Leu Ser Val Thr Leu Ser Asp Glu Glu Arg Asp Met
        35                  40                  45

Asn Val Ser Ser Tyr Ser Leu Thr Asp Val Pro Ile Glu Ala Ala Leu
    50                  55                  60

Glu Glu Ala Glu Thr Val Pro Phe Phe Gly Ser Lys Arg Val Val Ile
65                  70                  75                  80

Leu Lys Asp Ala Ala Leu Phe Thr Ser Gln Lys Leu Asp Val Glu His
                85                  90                  95

Asp Val Lys Arg Leu Glu Gln Tyr Ile Leu Asn Pro Val Pro Glu Thr
            100                 105                 110

Val Leu Leu Ile Met Ala Pro Tyr Glu Lys Leu Asp Glu Arg Lys Lys
        115                 120                 125

Ile Thr Lys Leu Ile Lys Lys Glu Ser Leu Val Leu Glu Ala Lys Pro
    130                 135                 140

Leu Asp Glu Gly Glu Ala Lys Ala Trp Leu Ser Ser Leu Ala Ser Glu
145                 150                 155                 160

Leu Gln Val Glu Met Asp Glu Lys Ala Ile Glu Thr Leu Leu Gly Met
                165                 170                 175

Thr Gly Leu Arg Leu Thr Gln Leu Ala Ser Glu Met Asn Lys Leu Ala
            180                 185                 190

Leu Tyr Val Gly Glu Gly Gly Ile Ile Arg Ser Glu Asp Val Thr Leu
        195                 200                 205

Leu Val Ala Lys Thr Leu Asp Gln Asn Ile Phe Asp Leu Ile Asp Phe
    210                 215                 220

Ala Ile Asn Gln Arg Thr His Gln Ala Leu Ser Leu Tyr His Glu Leu
225                 230                 235                 240

Leu Lys Gln Lys Glu Glu Pro Leu Lys Leu Leu Ala Leu Leu Thr Arg
                245                 250                 255

Gln Phe Arg Ile Met Tyr Gln Val Lys Glu Leu Gly Arg Arg Gly Tyr
            260                 265                 270

Thr Pro Asn Gln Met Ala Lys Pro Leu Lys Ile His Pro Tyr Val Ala
        275                 280                 285

Lys Leu Ala Gly Lys Lys Ala Ala Ser Met Ser Asp Gln Leu Leu Tyr
    290                 295                 300

Ser Leu Ile Glu Lys Ala Ala Asp Thr Glu Phe Ala Ile Lys Ser Gly
305                 310                 315                 320

Lys Val Asp Lys Val Leu Ala Leu Glu Leu Phe Leu Leu Thr Met Gly
                325                 330                 335

Ser Arg Glu Ser Val Gly
            340

<210> 7
<211> 1044
<212> DNA
<213> Bacillus subtilis

<400> 7
atggtatttg atgtgtggaa aagcctgaaa aaaggagagg tccatccggt ttattgttta 60

tacggaaaag agacatatct gctgcaagaa accgtcagca ggatcagaca gacggtcgtt 120

gatcaggaga caaaggattt caatctgtcc gtttttgatc tggaagagga cccgctggat 180

caagcgattg cagatgctga aacgtttccg tttatggggg agcggcgtct tgtcattgtg 240

aaaaatccat attttttaac aggtgaaaag aaaaaagaaa aaattgagca caacgtcagt 300

gcgcttgaat catatataca atcacctgcg ccttacacag tctttgtcct gcttgctccg 360

tatgaaaagc ttgatgagcg aaaaaagctg acgaaagcgt taaagaagca cgcgtttatg 420

atggaggcga aggaattaaa tgcaaaggaa acgacagact ttactgtcaa ccttgcaaaa 480

acagagcaga aaacaatcgg cacggaagcg gcggagcatt tggttctgct tgtgaacggt 540

catctgtcat cgatttttca ggagattcaa aagctctgca cgtttattgg agatcgtgaa 600

gaaattacgc tggatgatgt aaaaatgctt gttgctagaa gccttgaaca aaatattttt 660

gagctgatca ataaaatcgt caaccgaaaa cgaacagaga gtctgcaaat tttttatgat 720

ttgctaaaac aaaatgaaga accgatcaaa attatggcgc ttatttcgaa tcagttccgg 780

ctgattctgc agacgaagta cttcgcggaa cagggatacg gacaaaaaca aatcgcttct 840

aatctaaaag ttcacccatt tcgggtaaag ctggcgatgg atcaagcaag gcttttttca 900

gaggaggagc tccgtttaat tattgaacag cttgccgtca tggactatga gatgaaaacc 960

gggaaaaagg acaagcagct cctgctcgaa ctgttccttt tacagctgtt aaaaagaaat 1020

gaaaaaaacg atccccatta ttga 1044

<210> 8
<211> 1044
<212> DNA
<213> Bacillus subtilis

<220>
<221> CDS
<222> (1)..(1044)

<400> 8
atg gta ttt gat gtg tgg aaa agc ctg aaa aaa gga gag gtc cat ccg 48
Met Val Phe Asp Val Trp Lys Ser Leu Lys Lys Gly Glu Val His Pro
1               5                   10                  15

gtt tat tgt tta tac gga aaa gag aca tat ctg ctg caa gaa acc gtc 96
Val Tyr Cys Leu Tyr Gly Lys Glu Thr Tyr Leu Leu Gln Glu Thr Val
            20                  25                  30

agc agg atc aga cag acg gtc gtt gat cag gag aca aag gat ttc aat 144
Ser Arg Ile Arg Gln Thr Val Val Asp Gln Glu Thr Lys Asp Phe Asn
        35                  40                  45

ctg tcc gtt ttt gat ctg gaa gag gac ccg ctg gat caa gcg att gca 192
Leu Ser Val Phe Asp Leu Glu Glu Asp Pro Leu Asp Gln Ala Ile Ala
    50                  55                  60

gat gct gaa acg ttt ccg ttt atg ggg gag cgg cgt ctt gtc att gtg 240
Asp Ala Glu Thr Phe Pro Phe Met Gly Glu Arg Arg Leu Val Ile Val
65                  70                  75                  80

aaa aat cca tat ttt tta aca ggt gaa aag aaa aaa gaa aaa att gag 288
Lys Asn Pro Tyr Phe Leu Thr Gly Glu Lys Lys Lys Glu Lys Ile Glu
                85                  90                  95

cac aac gtc agt gcg ctt gaa tca tat ata caa tca cct gcg cct tac 336
His Asn Val Ser Ala Leu Glu Ser Tyr Ile Gln Ser Pro Ala Pro Tyr
            100                 105                 110

aca gtc ttt gtc ctg ctt gct ccg tat gaa aag ctt gat gag cga aaa 384
Thr Val Phe Val Leu Leu Ala Pro Tyr Glu Lys Leu Asp Glu Arg Lys
        115                 120                 125

aag ctg acg aaa gcg tta aag aag cac gcg ttt atg atg gag gcg aag 432
Lys Leu Thr Lys Ala Leu Lys Lys His Ala Phe Met Met Glu Ala Lys
    130                 135                 140

gaa tta aat gca aag gaa acg aca gac ttt act gtc aac ctt gca aaa 480
Glu Leu Asn Ala Lys Glu Thr Thr Asp Phe Thr Val Asn Leu Ala Lys
145                 150                 155                 160

aca gag cag aaa aca atc ggc acg gaa gcg gcg gag cat ttg gtt ctg     528
Thr Glu Gln Lys Thr Ile Gly Thr Glu Ala Ala Glu His Leu Val Leu
                165                 170                 175

ctt gtg aac ggt cat ctg tca tcg att ttt cag gag att caa aag ctc     576
Leu Val Asn Gly His Leu Ser Ser Ile Phe Gln Glu Ile Gln Lys Leu
            180                 185                 190

tgc acg ttt att gga gat cgt gaa gaa att acg ctg gat gat gta aaa     624
Cys Thr Phe Ile Gly Asp Arg Glu Glu Ile Thr Leu Asp Asp Val Lys
        195                 200                 205

atg ctt gtt gct aga agc ctt gaa caa aat att ttt gag ctg atc aat     672
Met Leu Val Ala Arg Ser Leu Glu Gln Asn Ile Phe Glu Leu Ile Asn
    210                 215                 220

aaa atc gtc aac cga aaa cga aca gag agt ctg caa att ttt tat gat     720
Lys Ile Val Asn Arg Lys Arg Thr Glu Ser Leu Gln Ile Phe Tyr Asp
225                 230                 235                 240

ttg cta aaa caa aat gaa gaa ccg atc aaa att atg gcg ctt att tcg     768
Leu Leu Lys Gln Asn Glu Glu Pro Ile Lys Ile Met Ala Leu Ile Ser
                245                 250                 255

aat cag ttc cgg ctg att ctg cag acg aag tac ttc gcg gaa cag gga     816
Asn Gln Phe Arg Leu Ile Leu Gln Thr Lys Tyr Phe Ala Glu Gln Gly
            260                 265                 270

tac gga caa aaa caa atc gct tct aat cta aaa gtt cac cca ttt cgg     864
Tyr Gly Gln Lys Gln Ile Ala Ser Asn Leu Lys Val His Pro Phe Arg
        275                 280                 285

gta aag ctg gcg atg gat caa gca agg ctt ttt tca gag gag gag ctc     912
Val Lys Leu Ala Met Asp Gln Ala Arg Leu Phe Ser Glu Glu Glu Leu
    290                 295                 300

cgt tta att att gaa cag ctt gcc gtc atg gac tat gag atg aaa acc     960
Arg Leu Ile Ile Glu Gln Leu Ala Val Met Asp Tyr Glu Met Lys Thr
305                 310                 315                 320

ggg aaa aag gac aag cag ctc ctg ctc gaa ctg ttc ctt tta cag ctg     1008
Gly Lys Lys Asp Lys Gln Leu Leu Leu Glu Leu Phe Leu Leu Gln Leu
                325                 330                 335

tta aaa aga aat gaa aaa aac gat ccc cat tat tga                    1044
Leu Lys Arg Asn Glu Lys Asn Asp Pro His Tyr
            340                 345

<210> 9
<211> 347
<212> PRT
<213> Bacillus subtilis

<400> 9
Met Val Phe Asp Val Trp Lys Ser Leu Lys Lys Gly Glu Val His Pro
1               5                   10                  15

Val Tyr Cys Leu Tyr Gly Lys Glu Thr Tyr Leu Leu Gln Glu Thr Val
            20                  25                  30

Ser Arg Ile Arg Gln Thr Val Val Asp Gln Glu Thr Lys Asp Phe Asn
        35                  40                  45

Leu Ser Val Phe Asp Leu Glu Glu Asp Pro Leu Asp Gln Ala Ile Ala
    50                  55                  60

Asp Ala Glu Thr Phe Pro Phe Met Gly Glu Arg Arg Leu Val Ile Val
65                  70                  75                  80

Lys Asn Pro Tyr Phe Leu Thr Gly Glu Lys Lys Lys Glu Lys Ile Glu
                85                  90                  95

His Asn Val Ser Ala Leu Glu Ser Tyr Ile Gln Ser Pro Ala Pro Tyr
            100                 105                 110

Thr Val Phe Val Leu Leu Ala Pro Tyr Glu Lys Leu Asp Glu Arg Lys
        115                 120                 125

Lys Leu Thr Lys Ala Leu Lys Lys His Ala Phe Met Met Glu Ala Lys
    130                 135                 140

Glu Leu Asn Ala Lys Glu Thr Thr Asp Phe Thr Val Asn Leu Ala Lys
145                 150                 155                 160

Thr Glu Gln Lys Thr Ile Gly Thr Glu Ala Ala Glu His Leu Val Leu
                165                 170                 175

Leu Val Asn Gly His Leu Ser Ser Ile Phe Gln Glu Ile Gln Lys Leu
            180                 185                 190

Cys Thr Phe Ile Gly Asp Arg Glu Glu Ile Thr Leu Asp Asp Val Lys
        195                 200                 205

Met Leu Val Ala Arg Ser Leu Glu Gln Asn Ile Phe Glu Leu Ile Asn
    210                 215                 220

Lys Ile Val Asn Arg Lys Arg Thr Glu Ser Leu Gln Ile Phe Tyr Asp
225                 230                 235                 240

Leu Leu Lys Gln Asn Glu Glu Pro Ile Lys Ile Met Ala Leu Ile Ser
                245                 250                 255

Asn Gln Phe Arg Leu Ile Leu Gln Thr Lys Tyr Phe Ala Glu Gln Gly
            260                 265                 270

Tyr Gly Gln Lys Gln Ile Ala Ser Asn Leu Lys Val His Pro Phe Arg
        275                 280                 285

Val Lys Leu Ala Met Asp Gln Ala Arg Leu Phe Ser Glu Glu Glu Leu
    290                 295                 300

Arg Leu Ile Ile Glu Gln Leu Ala Val Met Asp Tyr Glu Met Lys Thr
305                 310                 315                 320

Gly Lys Lys Asp Lys Gln Leu Leu Leu Glu Leu Phe Leu Leu Gln Leu
                325                 330                 335

Leu Lys Arg Asn Glu Lys Asn Asp Pro His Tyr
            340                 345


<210> 10
<211> 996
<212> DNA
<213> Buchnera sp.

<400> 10
atgaaaaata tatatccaaa tgaactaaaa aaaagtttaa tgcaaaaatt gcattatttt 60

tatatttttt taggagaaga ttttttttta ttagaaaaaa atcaagatat gatattgaat 120

tttgcatata aaaaaggatt cctagaaaaa attataattg atgtagaaaa aaatcaagat 180

tggaaaaaaa ttattttatt ttataaaact aataatttat tttttaaaaa aactacttta 240

gtaatcaact ttcttataaa aaagttaaat gttattttaa ttcaaaatct taataaaatg 300
ttttctttat tgcatccaga tatattaata atattaaaat tcaatcattt atcacgtttt 360
attcaaaaaa ataaatcatt aaaagaattt aaaaactaca atatagtttc ttgttttacg 420
ccgtataatt taaattttat taattggatt aagtatgaaa tacaagaaaa aaaaataaat 480
attgaggaaa aagcattttt tttattatgt aaatattatg aaggtaatac tttatttata 540
tataaaattt tagatatgct atttataata tggcccgata catgtattac agaaaaaaaa 600
ataaaaaaaa ttatcattga gttttttgat gtttctccat catattggat caattctata 660
tttcaaggta aaacagaaaa atccttttat atattaaata ttttttttaa aaaaaaatat 720
aatcctctta ttttagtacg ttctttgcaa aaagatttgt tacaattgat tcatatgaag 780
cgtgaaaaaa aaataagtat ttatgtaatg ttagaaaaat ataatatttt tgtaacaaga 840
cgtaagtttt ttatcaaggc atttaataaa attaacaacg atagtttatt aaaagctatt 900
caaattcttg taaaaataga agtaaatatc aaaaaaaaat ataataatta tgtttggaat 960
caactacaag aattaatttt aatattatgt aactaa 996

<210> 11
<211> 996
<212> DNA
<213> Buchnera sp.

<220>
<221> CDS
<222> (1)..(996)

<400> 11
atg aaa aat ata tat cca aat gaa cta aaa aaa agt tta atg caa aaa 48
Met Lys Asn Ile Tyr Pro Asn Glu Leu Lys Lys Ser Leu Met Gln Lys
1               5                   10                  15

ttg cat tat ttt tat att ttt tta gga gaa gat ttt ttt tta tta gaa 96
Leu His Tyr Phe Tyr Ile Phe Leu Gly Glu Asp Phe Phe Leu Leu Glu
            20                  25                  30

aaa aat caa gat atg ata ttg aat ttt gca tat aaa aaa gga ttc cta 144
Lys Asn Gln Asp Met Ile Leu Asn Phe Ala Tyr Lys Lys Gly Phe Leu
        35                  40                  45

gaa aaa att ata att gat gta gaa aaa aat caa gat tgg aaa aaa att 192
Glu Lys Ile Ile Ile Asp Val Glu Lys Asn Gln Asp Trp Lys Lys Ile
    50                  55                  60

att tta ttt tat aaa act aat aat tta ttt ttt aaa aaa act act tta 240
Ile Leu Phe Tyr Lys Thr Asn Asn Leu Phe Phe Lys Lys Thr Thr Leu
65                  70                  75                  80

gta atc aac ttt ctt ata aaa aag tta aat gtt att tta att caa aat 288
Val Ile Asn Phe Leu Ile Lys Lys Leu Asn Val Ile Leu Ile Gln Asn
                85                  90                  95

ctt aat aaa atg ttt tct tta ttg cat cca gat ata tta ata ata tta 336
Leu Asn Lys Met Phe Ser Leu Leu His Pro Asp Ile Leu Ile Ile Leu
            100                 105                 110

aaa ttc aat cat tta tca cgt ttt att caa aaa aat aaa tca tta aaa 384
Lys Phe Asn His Leu Ser Arg Phe Ile Gln Lys Asn Lys Ser Leu Lys

        115                 120                 125

gaa ttt aaa aac tac aat ata gtt tct tgt ttt acg ccg tat aat tta 432
Glu Phe Lys Asn Tyr Asn Ile Val Ser Cys Phe Thr Pro Tyr Asn Leu
    130                 135                 140

aat ttt att aat tgg att aag tat gaa ata caa gaa aaa aaa ata aat 480
Asn Phe Ile Asn Trp Ile Lys Tyr Glu Ile Gln Glu Lys Lys Ile Asn
145                 150                 155                 160

att gag gaa aaa gca ttt ttt tta tta tgt aaa tat tat gaa ggt aat 528
Ile Glu Glu Lys Ala Phe Phe Leu Leu Cys Lys Tyr Tyr Glu Gly Asn
                165                 170                 175

act tta ttt ata tat aaa att tta gat atg cta ttt ata ata tgg ccc 576
Thr Leu Phe Ile Tyr Lys Ile Leu Asp Met Leu Phe Ile Ile Trp Pro
            180                 185                 190

gat aca tgt att aca gaa aaa aaa ata aaa aaa att atc att gag ttt 624
Asp Thr Cys Ile Thr Glu Lys Lys Ile Lys Lys Ile Ile Ile Glu Phe
        195                 200                 205

ttt gat gtt tct cca tca tat tgg atc aat tct ata ttt caa ggt aaa 672
Phe Asp Val Ser Pro Ser Tyr Trp Ile Asn Ser Ile Phe Gln Gly Lys
    210                 215                 220

aca gaa aaa tcc ttt tat ata tta aat att ttt ttt aaa aaa aaa tat 720
Thr Glu Lys Ser Phe Tyr Ile Leu Asn Ile Phe Phe Lys Lys Lys Tyr
225                 230                 235                 240

aat cct ctt att tta gta cgt tct ttg caa aaa gat ttg tta caa ttg 768
Asn Pro Leu Ile Leu Val Arg Ser Leu Gln Lys Asp Leu Leu Gln Leu
                245                 250                 255

att cat atg aag cgt gaa aaa aaa ata agt att tat gta atg tta gaa 816
Ile His Met Lys Arg Glu Lys Lys Ile Ser Ile Tyr Val Met Leu Glu
            260                 265                 270

aaa tat aat att ttt gta aca aga cgt aag ttt ttt atc aag gca ttt 864
Lys Tyr Asn Ile Phe Val Thr Arg Arg Lys Phe Phe Ile Lys Ala Phe
        275                 280                 285

aat aaa att aac aac gat agt tta tta aaa gct att caa att ctt gta 912
Asn Lys Ile Asn Asn Asp Ser Leu Leu Lys Ala Ile Gln Ile Leu Val
    290                 295                 300

aaa ata gaa gta aat atc aaa aaa aaa tat aat aat tat gtt tgg aat 960
Lys Ile Glu Val Asn Ile Lys Lys Lys Tyr Asn Asn Tyr Val Trp Asn
305                 310                 315                 320

caa cta caa gaa tta att tta ata tta tgt aac taa 996
Gln Leu Gln Glu Leu Ile Leu Ile Leu Cys Asn
                325                 330

<210> 12
<211> 331
<212> PRT
<213> Buchnera sp.

<400> 12
Met Lys Asn Ile Tyr Pro Asn Glu Leu Lys Lys Ser Leu Met Gln Lys
1               5                   10                  15

Leu His Tyr Phe Tyr Ile Phe Leu Gly Glu Asp Phe Phe Leu Leu Glu
            20                  25                  30

Lys Asn Gln Asp Met Ile Leu Asn Phe Ala Tyr Lys Lys Gly Phe Leu

35                  40                  45

Glu Lys Ile Ile Ile Asp Val Glu Lys Asn Gln Asp Trp Lys Lys Ile
    50                  55                  60

Ile Leu Phe Tyr Lys Thr Asn Asn Leu Phe Phe Lys Lys Thr Thr Leu
65                  70                  75                  80

Val Ile Asn Phe Leu Ile Lys Lys Leu Asn Val Ile Leu Ile Gln Asn
                85                  90                  95

Leu Asn Lys Met Phe Ser Leu Leu His Pro Asp Ile Leu Ile Ile Leu
            100                 105                 110

Lys Phe Asn His Leu Ser Arg Phe Ile Gln Lys Asn Lys Ser Leu Lys
        115                 120                 125

Glu Phe Lys Asn Tyr Asn Ile Val Ser Cys Phe Thr Pro Tyr Asn Leu
    130                 135                 140

Asn Phe Ile Asn Trp Ile Lys Tyr Glu Ile Gln Glu Lys Lys Ile Asn
145                 150                 155                 160

Ile Glu Glu Lys Ala Phe Phe Leu Leu Cys Lys Tyr Tyr Glu Gly Asn
                165                 170                 175

Thr Leu Phe Ile Tyr Lys Ile Leu Asp Met Leu Phe Ile Ile Trp Pro
            180                 185                 190

Asp Thr Cys Ile Thr Glu Lys Lys Ile Lys Lys Ile Ile Ile Glu Phe
        195                 200                 205

Phe Asp Val Ser Pro Ser Tyr Trp Ile Asn Ser Ile Phe Gln Gly Lys
    210                 215                 220

Thr Glu Lys Ser Phe Tyr Ile Leu Asn Ile Phe Phe Lys Lys Lys Tyr
225                 230                 235                 240

Asn Pro Leu Ile Leu Val Arg Ser Leu Gln Lys Asp Leu Leu Gln Leu
                245                 250                 255

Ile His Met Lys Arg Glu Lys Lys Ile Ser Ile Tyr Val Met Leu Glu
            260                 265                 270

Lys Tyr Asn Ile Phe Val Thr Arg Arg Lys Phe Phe Ile Lys Ala Phe
        275                 280                 285

Asn Lys Ile Asn Asn Asp Ser Leu Leu Lys Ala Ile Gln Ile Leu Val
    290                 295                 300

Lys Ile Glu Val Asn Ile Lys Lys Lys Tyr Asn Asn Tyr Val Trp Asn
305                 310                 315                 320

Gln Leu Gln Glu Leu Ile Leu Ile Leu Cys Asn
                325                 330

<210> 13
<211> 990
<212> DNA
<213> Borrelia burgdorferi

<400> 13
atgcaagcgg tttatttatt gttgggtaat gagcaaggtt taaaagaagc ctatttaaaa 60

gagcttttaa ttaaaatgga tgcttttaaa tccgaagttt cagttactaa aatttttttg 120

tcagaactct cagctgtagg atttgctgag aaattatttt ccaattcttt tttttcaaaa 180

aaagaaattt ttattgttta tgagtctgaa cttttaaaag caggaaaaga tttagagcta 240

gtgtgtaatt caatcttaaa gtctaacaat aaaactgtta tttttgtttc caatagcaat 300

acatgtaaca ttgattttaa gaataagctt aagtttataa aaaaagtttt ttatgagatt 360

cctgatgatg ataaatttac atttgtaaaa agaaattttt ttaatcttaa tattaaaatt 420

acagattctg caataaattt aatgctttta atgttaaatt cagatactaa aattttgaaa 480

ttttatatag attcttttgc gctttttgcc aagaataata ccattgagga ggaagatata 540

gcttcttgga ttagttttat tcgctttgag aacacctttt ccttatttaa ttcaattttg 600

agaaaagata tgactcagtc tttgatcaag attaagtcta ttttggatca gggagaagat 660

ttgcttaata ttttaatgag tcttatttgg caatttaaaa gattattaaa ggtgcaaata 720

gattataatg catatgggag cttgcagagt gcattgaata aaaataaaat ctttttttca 780

ttaaataaaa tttatagagt aggaattaaa aattattcaa ttccagaaat taaaattgtt 840

ttgaaaattt tatataagtt tgatttatat ttgagaattt attctaaaaa tattcatcaa 900

aatttgtcat attttttaat attctcaatt ttaaagctta ataataattt tttaatgcat 960

tattctacag aatcaaaatt taatttttaa 990

<210> 14
<211> 990
<212> DNA
<213> Borrelia burgdorferi

<220>
<221> CDS
<222> (1)..(990)

<400> 14
atg caa gcg gtt tat tta ttg ttg ggt aat gag caa ggt tta aaa gaa 48
Met Gln Ala Val Tyr Leu Leu Leu Gly Asn Glu Gln Gly Leu Lys Glu
1               5                   10                  15

gcc tat tta aaa gag ctt tta att aaa atg gat gct ttt aaa tcc gaa 96
Ala Tyr Leu Lys Glu Leu Leu Ile Lys Met Asp Ala Phe Lys Ser Glu
            20                  25                  30

gtt tca gtt act aaa att ttt ttg tca gaa ctc tca gct gta gga ttt 144
Val Ser Val Thr Lys Ile Phe Leu Ser Glu Leu Ser Ala Val Gly Phe
        35                  40                  45

gct gag aaa tta ttt tcc aat tct ttt ttt tca aaa aaa gaa att ttt 192
Ala Glu Lys Leu Phe Ser Asn Ser Phe Phe Ser Lys Lys Glu Ile Phe
    50                  55                  60

att gtt tat gag tct gaa ctt tta aaa gca gga aaa gat tta gag cta 240
Ile Val Tyr Glu Ser Glu Leu Leu Lys Ala Gly Lys Asp Leu Glu Leu
65                  70                  75                  80

gtg tgt aat tca atc tta aag tct aac aat aaa act gtt att ttt gtt 288
Val Cys Asn Ser Ile Leu Lys Ser Asn Asn Lys Thr Val Ile Phe Val
                85                  90                  95

tcc aat agc aat aca tgt aac att gat ttt aag aat aag ctt aag ttt 336
Ser Asn Ser Asn Thr Cys Asn Ile Asp Phe Lys Asn Lys Leu Lys Phe
            100                 105                 110

ata aaa aaa gtt ttt tat gag att cct gat gat gat aaa ttt aca ttt     384
Ile Lys Lys Val Phe Tyr Glu Ile Pro Asp Asp Asp Lys Phe Thr Phe
        115                 120                 125

gta aaa aga aat ttt ttt aat ctt aat att aaa att aca gat tct gca     432
Val Lys Arg Asn Phe Phe Asn Leu Asn Ile Lys Ile Thr Asp Ser Ala
    130                 135                 140

ata aat tta atg ctt tta atg tta aat tca gat act aaa att ttg aaa     480
Ile Asn Leu Met Leu Leu Met Leu Asn Ser Asp Thr Lys Ile Leu Lys
145                 150                 155                 160

ttt tat ata gat tct ttt gcg ctt ttt gcc aag aat aat acc att gag     528
Phe Tyr Ile Asp Ser Phe Ala Leu Phe Ala Lys Asn Asn Thr Ile Glu
                165                 170                 175

gag gaa gat ata gct tct tgg att agt ttt att cgc ttt gag aac acc     576
Glu Glu Asp Ile Ala Ser Trp Ile Ser Phe Ile Arg Phe Glu Asn Thr
            180                 185                 190

ttt tcc tta ttt aat tca att ttg aga aaa gat atg act cag tct ttg     624
Phe Ser Leu Phe Asn Ser Ile Leu Arg Lys Asp Met Thr Gln Ser Leu
        195                 200                 205

atc aag att aag tct att ttg gat cag gga gaa gat ttg ctt aat att     672
Ile Lys Ile Lys Ser Ile Leu Asp Gln Gly Glu Asp Leu Leu Asn Ile
    210                 215                 220

tta atg agt ctt att tgg caa ttt aaa aga tta tta aag gtg caa ata     720
Leu Met Ser Leu Ile Trp Gln Phe Lys Arg Leu Leu Lys Val Gln Ile
225                 230                 235                 240

gat tat aat gca tat ggg agc ttg cag agt gca ttg aat aaa aat aaa     768
Asp Tyr Asn Ala Tyr Gly Ser Leu Gln Ser Ala Leu Asn Lys Asn Lys
                245                 250                 255

atc ttt ttt tca tta aat aaa att tat aga gta gga att aaa aat tat     816
Ile Phe Phe Ser Leu Asn Lys Ile Tyr Arg Val Gly Ile Lys Asn Tyr
            260                 265                 270

tca att cca gaa att aaa att gtt ttg aaa att tta tat aag ttt gat     864
Ser Ile Pro Glu Ile Lys Ile Val Leu Lys Ile Leu Tyr Lys Phe Asp
        275                 280                 285

tta tat ttg aga att tat tct aaa aat att cat caa aat ttg tca tat     912
Leu Tyr Leu Arg Ile Tyr Ser Lys Asn Ile His Gln Asn Leu Ser Tyr
    290                 295                 300

ttt tta ata ttc tca att tta aag ctt aat aat aat ttt tta atg cat     960
Phe Leu Ile Phe Ser Ile Leu Lys Leu Asn Asn Asn Phe Leu Met His
305                 310                 315                 320

tat tct aca gaa tca aaa ttt aat ttt taa                             990
Tyr Ser Thr Glu Ser Lys Phe Asn Phe
                325                 330

<210> 15
<211> 329
<212> PRT
<213> Borrelia burgdorferi

<400> 15
   Met Gln Ala Val Tyr Leu Leu Leu Gly Asn Glu Gln Gly Leu Lys Glu
   1               5                   10                  15
   Ala Tyr Leu Lys Glu Leu Leu Ile Lys Met Asp Ala Phe Lys Ser Glu
               20                  25                  30
   Val Ser Val Thr Lys Ile Phe Leu Ser Glu Leu Ser Ala Val Gly Phe
               35                  40                  45
   Ala Glu Lys Leu Phe Ser Asn Ser Phe Phe Ser Lys Lys Glu Ile Phe
           50                  55                  60
   Ile Val Tyr Glu Ser Glu Leu Leu Lys Ala Gly Lys Asp Leu Glu Leu
   65                  70                  75                  80
   Val Cys Asn Ser Ile Leu Lys Ser Asn Asn Lys Thr Val Ile Phe Val
                   85                  90                  95
   Ser Asn Ser Asn Thr Cys Asn Ile Asp Phe Lys Asn Lys Leu Lys Phe
               100                 105                 110
   Ile Lys Lys Val Phe Tyr Glu Ile Pro Asp Asp Asp Lys Phe Thr Phe
           115                 120                 125
   Val Lys Arg Asn Phe Phe Asn Leu Asn Ile Lys Ile Thr Asp Ser Ala
       130                 135                 140
   Ile Asn Leu Met Leu Leu Met Leu Asn Ser Asp Thr Lys Ile Leu Lys
   145                 150                 155                 160
   Phe Tyr Ile Asp Ser Phe Ala Leu Phe Ala Lys Asn Asn Thr Ile Glu
                   165                 170                 175
   Glu Glu Asp Ile Ala Ser Trp Ile Ser Phe Ile Arg Phe Glu Asn Thr
               180                 185                 190
   Phe Ser Leu Phe Asn Ser Ile Leu Arg Lys Asp Met Thr Gln Ser Leu
           195                 200                 205
   Ile Lys Ile Lys Ser Ile Leu Asp Gln Gly Glu Asp Leu Leu Asn Ile
       210                 215                 220
   Leu Met Ser Leu Ile Trp Gln Phe Lys Arg Leu Leu Lys Val Gln Ile
   225                 230                 235                 240
   Asp Tyr Asn Ala Tyr Gly Ser Leu Gln Ser Ala Leu Asn Lys Asn Lys
                   245                 250                 255
   Ile Phe Phe Ser Leu Asn Lys Ile Tyr Arg Val Gly Ile Lys Asn Tyr
               260                 265                 270
   Ser Ile Pro Glu Ile Lys Ile Val Leu Lys Ile Leu Tyr Lys Phe Asp
           275                 280                 285
   Leu Tyr Leu Arg Ile Tyr Ser Lys Asn Ile His Gln Asn Leu Ser Tyr
       290                 295                 300
   Phe Leu Ile Phe Ser Ile Leu Lys Leu Asn Asn Asn Phe Leu Met His
   305                 310                 315                 320
   Tyr Ser Thr Glu Ser Lys Phe Asn Phe
                   325

<210> 16
<211> 966
<212> DNA
<213> Campylobacter jejuni

<400> 16
atgtatagaa aagagcttca aatcttactt tctaaagatt caatacctaa tttttctttt 60

ctttatggag cagataattt tcaaagcgaa ctttatgctg aatttataaa agaaaaatat 120

aagcccgatg aaactttgaa actttttttt gaagaataca acttcactcg tgcgagtgat 180

tttttaagcg caggatcgct ttttagtgaa aaaaaattac tcgaaattaa aacttctaaa 240

aaaattccaa caaaagatct taaagtttta gtcgaacttt gtaaaaacaa tacggacaat 300

ttctttcttt tagagcttta tgatgaaagt tctaaacaaa gcgatataga aaaaattttc 360

tctcctcatt ttgtaagatt ttttaaagct aacggagcta aagaaggcgt ggagctttta 420

agcataaaag caaaacaact aggggttgaa atcactcaaa atgctttatt tactcttttt 480

acaagttttg atgaaaactt atatcttgct gcgagtgagc ttaataaatt tagtggttta 540

agagtagatg aaaaaaccat agaacaatac tgctatagtt taaatacagg aagttttgaa 600

agcttttttg ataaaatttt aaaaaaacaa gactttaaaa gcgaacttga aaaaatttta 660

gataatttta atgaaattgc tttaatcaac tctttatata actcttttta tcgtttattt 720

aaaattgcac tttatgctaa aatcaatggt aaaattgatt ttaaagagct tttaggctac 780

actccacccc ctcaagtggg acaaaattta agctctcaag cctttagttt aaaaatcgaa 840

caatataaag aaattttcac tcttttgctt aaaagcgaat acgaacttaa aaccaatcct 900

aaactcgtaa aaaaagaatt tttgatttca aatttactta aacttgcaag gattttaaaa 960

aattaa 966

<210> 17
<211> 966
<212> DNA
<213> Campylobacter jejuni

<220>
<221> CDS
<222> (1)..(966)

<400> 17
atg tat aga aaa gag ctt caa atc tta ctt tct aaa gat tca ata cct 48
Met Tyr Arg Lys Glu Leu Gln Ile Leu Leu Ser Lys Asp Ser Ile Pro
1               5                   10                  15

aat ttt ttc ttt ctt tat gga gca gat aat ttt caa agc gaa ctt tat 96
Asn Phe Phe Phe Leu Tyr Gly Ala Asp Asn Phe Gln Ser Glu Leu Tyr
            20                  25                  30

gct gaa ttt ata aaa gaa aaa tat aag ccc gat gaa act ttg aaa ctt 144
Ala Glu Phe Ile Lys Glu Lys Tyr Lys Pro Asp Glu Thr Leu Lys Leu
        35                  40                  45

ttt ttt gaa gaa tac aac ttc act cgt gcg agt gat ttt tta agc gca 192
Phe Phe Glu Glu Tyr Asn Phe Thr Arg Ala Ser Asp Phe Leu Ser Ala
    50                  55                  60

gga tcg ctt ttt agt gaa aaa aaa tta ctc gaa att aaa act tct aaa 240
Gly Ser Leu Phe Ser Glu Lys Lys Leu Leu Glu Ile Lys Thr Ser Lys
65                  70                  75                  80

aaa att cca aca aaa gat ctt aaa gtt tta gtc gaa ctt tgt aaa aac 288
Lys Ile Pro Thr Lys Asp Leu Lys Val Leu Val Glu Leu Cys Lys Asn
                85                  90                  95

aat acg gac aat ttc ttt ctt tta gag ctt tat gat gaa agt tct aaa 336
Asn Thr Asp Asn Phe Phe Leu Leu Glu Leu Tyr Asp Glu Ser Ser Lys

100                 105                 110

caa agc gat ata gaa aaa att ttc tct cct cat ttt gta aga ttt ttt     384
Gln Ser Asp Ile Glu Lys Ile Phe Ser Pro His Phe Val Arg Phe Phe
        115                 120                 125

aaa gct aac gga gct aaa gaa ggc gtg gag ctt tta agc ata aaa gca     432
Lys Ala Asn Gly Ala Lys Glu Gly Val Glu Leu Leu Ser Ile Lys Ala
    130                 135                 140

aaa caa cta ggg gtt gaa atc act caa aat gct tta ttt act ctt ttt     480
Lys Gln Leu Gly Val Glu Ile Thr Gln Asn Ala Leu Phe Thr Leu Phe
145                 150                 155                 160

aca agt ttt gat gaa aac tta tat ctt gct gcg agt gag ctt aat aaa     528
Thr Ser Phe Asp Glu Asn Leu Tyr Leu Ala Ala Ser Glu Leu Asn Lys
                165                 170                 175

ttt agt ggt tta aga gta gat gaa aaa acc ata gaa caa tac tgc tat     576
Phe Ser Gly Leu Arg Val Asp Glu Lys Thr Ile Glu Gln Tyr Cys Tyr
            180                 185                 190

agt tta aat aca gga agt ttt gaa agc ttt ttt gat aaa att tta aaa     624
Ser Leu Asn Thr Gly Ser Phe Glu Ser Phe Phe Asp Lys Ile Leu Lys
        195                 200                 205

aaa caa gac ttt aaa agc gaa ctt gaa aaa att tta gat aat ttt aat     672
Lys Gln Asp Phe Lys Ser Glu Leu Glu Lys Ile Leu Asp Asn Phe Asn
    210                 215                 220

gaa att gct tta atc aac tct tta tat aac tct ttt tat cgt tta ttt     720
Glu Ile Ala Leu Ile Asn Ser Leu Tyr Asn Ser Phe Tyr Arg Leu Phe
225                 230                 235                 240

aaa att gca ctt tat gct aaa atc aat ggt aaa att gat ttt aaa gag     768
Lys Ile Ala Leu Tyr Ala Lys Ile Asn Gly Lys Ile Asp Phe Lys Glu
                245                 250                 255

ctt tta ggc tac act cca ccc cct caa gtg gga caa aat tta agc tct     816
Leu Leu Gly Tyr Thr Pro Pro Pro Gln Val Gly Gln Asn Leu Ser Ser
            260                 265                 270

caa gcc ttt agt tta aaa atc gaa caa tat aaa gaa att ttc act ctt     864
Gln Ala Phe Ser Leu Lys Ile Glu Gln Tyr Lys Glu Ile Phe Thr Leu
        275                 280                 285

ttg ctt aaa agc gaa tac gaa ctt aaa acc aat cct aaa ctc gta aaa     912
Leu Leu Lys Ser Glu Tyr Glu Leu Lys Thr Asn Pro Lys Leu Val Lys
    290                 295                 300

aaa gaa ttt ttg att tca aat tta ctt aaa ctt gca agg att tta aaa     960
Lys Glu Phe Leu Ile Ser Asn Leu Leu Lys Leu Ala Arg Ile Leu Lys
305                 310                 315                 320

aat taa                                                             966
Asn

<210> 18
<211> 321
<212> PRT
<213> Campylobacter jejuni

<400> 18
Met Tyr Arg Lys Glu Leu Gln Ile Leu Leu Ser Lys Asp Ser Ile Pro
  1               5                  10                  15

Asn Phe Phe Phe Leu Tyr Gly Ala Asp Asn Phe Gln Ser Glu Leu Tyr

            20                  25                  30

Ala Glu Phe Ile Lys Glu Lys Tyr Lys Pro Asp Glu Thr Leu Lys Leu
        35                  40                  45

Phe Phe Glu Glu Tyr Asn Phe Thr Arg Ala Ser Asp Phe Leu Ser Ala
    50                  55                  60

Gly Ser Leu Phe Ser Glu Lys Lys Leu Leu Glu Ile Lys Thr Ser Lys
65                  70                  75                  80

Lys Ile Pro Thr Lys Asp Leu Lys Val Leu Val Glu Leu Cys Lys Asn
                85                  90                  95

Asn Thr Asp Asn Phe Phe Leu Leu Glu Leu Tyr Asp Glu Ser Ser Lys
            100                 105                 110

Gln Ser Asp Ile Glu Lys Ile Phe Ser Pro His Phe Val Arg Phe Phe
        115                 120                 125

Lys Ala Asn Gly Ala Lys Glu Gly Val Glu Leu Leu Ser Ile Lys Ala
    130                 135                 140

Lys Gln Leu Gly Val Glu Ile Thr Gln Asn Ala Leu Phe Thr Leu Phe
145                 150                 155                 160

Thr Ser Phe Asp Glu Asn Leu Tyr Leu Ala Ala Ser Glu Leu Asn Lys
                165                 170                 175

Phe Ser Gly Leu Arg Val Asp Glu Lys Thr Ile Glu Gln Tyr Cys Tyr
            180                 185                 190

Ser Leu Asn Thr Gly Ser Phe Glu Ser Phe Phe Asp Lys Ile Leu Lys
        195                 200                 205

Lys Gln Asp Phe Lys Ser Glu Leu Glu Lys Ile Leu Asp Asn Phe Asn
    210                 215                 220

Glu Ile Ala Leu Ile Asn Ser Leu Tyr Asn Ser Phe Tyr Arg Leu Phe
225                 230                 235                 240

Lys Ile Ala Leu Tyr Ala Lys Ile Asn Gly Lys Ile Asp Phe Lys Glu
                245                 250                 255

Leu Leu Gly Tyr Thr Pro Pro Pro Gln Val Gly Gln Asn Leu Ser Ser
            260                 265                 270

Gln Ala Phe Ser Leu Lys Ile Glu Gln Tyr Lys Glu Ile Phe Thr Leu
        275                 280                 285

Leu Leu Lys Ser Glu Tyr Glu Leu Lys Thr Asn Pro Lys Leu Val Lys
    290                 295                 300

Lys Glu Phe Leu Ile Ser Asn Leu Leu Lys Leu Ala Arg Ile Leu Lys
305                 310                 315                 320

Asn

<210> 19
<211> 1053
<212> DNA
<213> Caulobacter crescentus

<400> 19
atgatcctgt ccaagcgtcc ggacgtcgaa cgcttcctca aggagccgac cccggacatc 60

cgggccgtag tgatctacgg caaggatcgg ggcgtcgtgc gggaccgggc caatgcgctg 120

gcccggcgcg tggtcgagcg cccggatgat cccttcgata cggcgcaact gaccgacggc 180

gatctcgacg ccgacccggc caagcttgag gacgaactgt cggcgatgtc gctgatgggg 240

gggcggcgcc tggtgcgcct gcgactgtcc agcgacaagg ctggccccga caagcaggcc 300

gccgaagccc tgacccgcca tgtcgagggt caactcaatc cggacgcctt ctttctggtc 360

gaggccggcg cgctgggcag agactcacag cttcgcaagg tcggcgagaa ggccaacggc 420

tgcgccgtca tcccctgtta cgaggacgag gccggggacc tagcccgcct gacgcgcgac 480

accctggcca acgacaaggt cagcgtgaac agcgaggcgc tggaactctt cgtttcgcgc 540

ctacccaagg aacggggcgt cgcccgcgcc gagatcgaac ggctggccct ctacctcggc 600

ccgggcagcg gtatcaacgc caccgccgca gacctgaccg acttcctggg cgtcgaaccc 660

gaggcctccc ttagtgacgc cgccgccgat gcgttcggcg gaaaggtcgg cgcggcccag 720

caggcgcttc gccgcgccgc cgccgagggg gaaggcggac cggccgccgt cagggcgatg 780

ggctactatc tgggtcgcct gcgtcgcgtc ctgaccctgc acaagaacgg cgtcgacctg 840

caagccgccg ccaaggccgc cggcgtgttc tggaagcagg agcgagaatt cctccgtcag 900

gcgcgagcct ggaacctgga ggccattctc gagatccagg gcgagatcct gaccgccgac 960

cgagcctgca agacgtcggg ctcccccgat caactgatcg ccgagcgtct ggccctgatg 1020

atcgccggcc gcgcccgccg actgggcctg tag 1053

<210> 20
<211> 1053
<212> DNA
<213> Caulobacter crescentus

<220>
<221> CDS
<222> (1)..(1053)

<400> 20
atg atc ctg tcc aag cgt ccg gac gtc gaa cgc ttc ctc aag gag ccg 48
Met Ile Leu Ser Lys Arg Pro Asp Val Glu Arg Phe Leu Lys Glu Pro
1               5                   10                  15

acc ccg gac atc cgg gcc gta gtg atc tac ggc aag gat cgg ggc gtc 96
Thr Pro Asp Ile Arg Ala Val Val Ile Tyr Gly Lys Asp Arg Gly Val
            20                  25                  30

gtg cgg gac cgg gcc aat gcg ctg gcc cgg cgc gtg gtc gag cgc ccg 144
Val Arg Asp Arg Ala Asn Ala Leu Ala Arg Arg Val Val Glu Arg Pro
        35                  40                  45

gat gat ccc ttc gat acg gcg caa ctg acc gac ggc gat ctc gac gcc 192
Asp Asp Pro Phe Asp Thr Ala Gln Leu Thr Asp Gly Asp Leu Asp Ala
    50                  55                  60

gac ccg gcc aag ctt gag gac gaa ctg tcg gcg atg tcg ctg atg ggg 240
Asp Pro Ala Lys Leu Glu Asp Glu Leu Ser Ala Met Ser Leu Met Gly
65                  70                  75                  80

ggg cgg cgc ctg gtg cgc ctg cga ctg tcc agc gac aag gct ggc ccc 288
Gly Arg Arg Leu Val Arg Leu Arg Leu Ser Ser Asp Lys Ala Gly Pro
                85                  90                  95

gac aag cag gcc gcc gaa gcc ctg acc cgc cat gtc gag ggt caa ctc     336
Asp Lys Gln Ala Ala Glu Ala Leu Thr Arg His Val Glu Gly Gln Leu
            100                 105                 110

aat ccg gac gcc ttc ttt ctg gtc gag gcc ggc gcg ctg ggc aga gac     384
Asn Pro Asp Ala Phe Phe Leu Val Glu Ala Gly Ala Leu Gly Arg Asp
        115                 120                 125

tca cag ctt cgc aag gtc ggc gag aag gcc aac ggc tgc gcc gtc atc     432
Ser Gln Leu Arg Lys Val Gly Glu Lys Ala Asn Gly Cys Ala Val Ile
    130                 135                 140

ccc tgt tac gag gac gag gcc ggg gac cta gcc cgc ctg acg cgc gac     480
Pro Cys Tyr Glu Asp Glu Ala Gly Asp Leu Ala Arg Leu Thr Arg Asp
145                 150                 155                 160

acc ctg gcc aac gac aag gtc agc gtg aac agc gag gcg ctg gaa ctc     528
Thr Leu Ala Asn Asp Lys Val Ser Val Asn Ser Glu Ala Leu Glu Leu
                165                 170                 175

ttc gtt tcg cgc cta ccc aag gaa cgg ggc gtc gcc cgc gcc gag atc     576
Phe Val Ser Arg Leu Pro Lys Glu Arg Gly Val Ala Arg Ala Glu Ile
            180                 185                 190

gaa cgg ctg gcc ctc tac ctc ggc ccg ggc agc ggt atc aac gcc acc     624
Glu Arg Leu Ala Leu Tyr Leu Gly Pro Gly Ser Gly Ile Asn Ala Thr
        195                 200                 205

gcc gca gac ctg acc gac ttc ctg ggc gtc gaa ccc gag gcc tcc ctt     672
Ala Ala Asp Leu Thr Asp Phe Leu Gly Val Glu Pro Glu Ala Ser Leu
    210                 215                 220

agt gac gcc gcc gcc gat gcg ttc ggc gga aag gtc ggc gcg gcc cag     720
Ser Asp Ala Ala Ala Asp Ala Phe Gly Gly Lys Val Gly Ala Ala Gln
225                 230                 235                 240

cag gcg ctt cgc cgc gcc gcc gcc gag ggg gaa ggc gga ccg gcc gcc     768
Gln Ala Leu Arg Arg Ala Ala Ala Glu Gly Glu Gly Gly Pro Ala Ala
                245                 250                 255

gtc agg gcg atg ggc tac tat ctg ggt cgc ctg cgt cgc gtc ctg acc     816
Val Arg Ala Met Gly Tyr Tyr Leu Gly Arg Leu Arg Arg Val Leu Thr
            260                 265                 270

ctg cac aag aac ggc gtc gac ctg caa gcc gcc gcc aag gcc gcc ggc     864
Leu His Lys Asn Gly Val Asp Leu Gln Ala Ala Ala Lys Ala Ala Gly
        275                 280                 285

gtg ttc tgg aag cag gag cga gaa ttc ctc cgt cag gcg cga gcc tgg     912
Val Phe Trp Lys Gln Glu Arg Glu Phe Leu Arg Gln Ala Arg Ala Trp
    290                 295                 300

aac ctg gag gcc att ctc gag atc cag ggc gag atc ctg acc gcc gac     960
Asn Leu Glu Ala Ile Leu Glu Ile Gln Gly Glu Ile Leu Thr Ala Asp
305                 310                 315                 320

cga gcc tgc aag acg tcg ggc tcc ccc gat caa ctg atc gcc gag cgt     1008
Arg Ala Cys Lys Thr Ser Gly Ser Pro Asp Gln Leu Ile Ala Glu Arg
                325                 330                 335

ctg gcc ctg atg atc gcc ggc cgc gcc cgc cga ctg ggc ctg tag         1053
Leu Ala Leu Met Ile Ala Gly Arg Ala Arg Arg Leu Gly Leu
            340                 345                 350

<210> 21
<211> 350
<212> PRT

<213> Caulobacter crescentus

<400> 21
Met Ile Leu Ser Lys Arg Pro Asp Val Glu Arg Phe Leu Lys Glu Pro
1               5                   10                  15

Thr Pro Asp Ile Arg Ala Val Val Ile Tyr Gly Lys Asp Arg Gly Val
            20                  25                  30

Val Arg Asp Arg Ala Asn Ala Leu Ala Arg Arg Val Val Glu Arg Pro
        35                  40                  45

Asp Asp Pro Phe Asp Thr Ala Gln Leu Thr Asp Gly Asp Leu Asp Ala
    50                  55                  60

Asp Pro Ala Lys Leu Glu Asp Glu Leu Ser Ala Met Ser Leu Met Gly
65                  70                  75                  80

Gly Arg Arg Leu Val Arg Leu Arg Leu Ser Ser Asp Lys Ala Gly Pro
                85                  90                  95

Asp Lys Gln Ala Ala Glu Ala Leu Thr Arg His Val Glu Gly Gln Leu
            100                 105                 110

Asn Pro Asp Ala Phe Phe Leu Val Glu Ala Gly Ala Leu Gly Arg Asp
        115                 120                 125

Ser Gln Leu Arg Lys Val Gly Glu Lys Ala Asn Gly Cys Ala Val Ile
    130                 135                 140

Pro Cys Tyr Glu Asp Glu Ala Gly Asp Leu Ala Arg Leu Thr Arg Asp
145                 150                 155                 160

Thr Leu Ala Asn Asp Lys Val Ser Val Asn Ser Glu Ala Leu Glu Leu
                165                 170                 175

Phe Val Ser Arg Leu Pro Lys Glu Arg Gly Val Ala Arg Ala Glu Ile
            180                 185                 190

Glu Arg Leu Ala Leu Tyr Leu Gly Pro Gly Ser Gly Ile Asn Ala Thr
        195                 200                 205

Ala Ala Asp Leu Thr Asp Phe Leu Gly Val Glu Pro Glu Ala Ser Leu
    210                 215                 220

Ser Asp Ala Ala Ala Asp Ala Phe Gly Gly Lys Val Gly Ala Ala Gln
225                 230                 235                 240

Gln Ala Leu Arg Arg Ala Ala Ala Glu Gly Glu Gly Gly Pro Ala Ala
                245                 250                 255

Val Arg Ala Met Gly Tyr Tyr Leu Gly Arg Leu Arg Arg Val Leu Thr
            260                 265                 270

Leu His Lys Asn Gly Val Asp Leu Gln Ala Ala Ala Lys Ala Ala Gly
        275                 280                 285

Val Phe Trp Lys Gln Glu Arg Glu Phe Leu Arg Gln Ala Arg Ala Trp
    290                 295                 300

Asn Leu Glu Ala Ile Leu Glu Ile Gln Gly Glu Ile Leu Thr Ala Asp
305                 310                 315                 320

Arg Ala Cys Lys Thr Ser Gly Ser Pro Asp Gln Leu Ile Ala Glu Arg
                325                 330                 335

Leu Ala Leu Met Ile Ala Gly Arg Ala Arg Arg Leu Gly Leu
            340                 345                 350

<210> 22
<211> 945
<212> DNA
<213> Chlamydia muridarum

<400> 22
atggaaacct gccaaaatag tgtccgcata gagtctataa aagactttat tcatcatttt 60

gaaaatcaac gctttaaagt gtttataatt ggctcttcat ttcccgaaga caaggatatt 120

tttctggaac tttatgtttc cggaagaggg cgactatttg atggacaacg tttagttaaa 180

caagagcttt tatcttggac tgataatttt gggttgttca cttcaaagga aactattggt 240

atttatcaag ttgagaagat gagctcctct ttacaagagt ttatcataag ctatgctcat 300

catcctaatc cgcatctcac attatttctt ttcacaagta aagcagagtt cttctcagct 360

ttttcatcga agctccctaa tgccctttgt ttatctctct ttggtgagta ttttgcagaa 420

cgcgactctc gtatagcaca ggtattgatc aagcgttcaa tggatgttca actgtcttgt 480

tctttagggg tcgctaagct tttcgtaagt aagttcccgc aggttggagt ttttgaaatt 540

tttagtgaat ttcaaaagtt gatttgtcaa attgggggca agagttgttt agaagcttca 600

gacgttcaat cgtttgtaga aaaaagagaa tctgcttctt tatggaaatt acgggatgct 660

cttcttcgta gagatctctc aacagggcaa agtttgatgc aatctctggt atctgatttg 720

ggagaagatc ctcttgctat tctcaatttt ttacgtagcc aatatttatc tgggcttcga 780

gcaatagcgg aacagtcgaa agatcgcaaa acacaaatct ttttcgcagc aggagagcag 840

gctcttttga atggattaaa cttatttttt catgcggaga gtttgattaa gaataacgtg 900

caagatccca ttctttcttt agaaaccatg attgcaagac tgtaa 945

<210> 23
<211> 945
<212> DNA
<213> Chlamydia muridarum

<220>
<221> CDS
<222> (1)..(945)

<400> 23
atg gaa acc tgc caa aat agt gtc cgc ata gag tct ata aaa gac ttt 48
Met Glu Thr Cys Gln Asn Ser Val Arg Ile Glu Ser Ile Lys Asp Phe
1               5                   10                  15

att cat cat ttt gaa aat caa cgc ttt aaa gtg ttt ata att ggc tct 96
Ile His His Phe Glu Asn Gln Arg Phe Lys Val Phe Ile Ile Gly Ser
            20                  25                  30

tca ttt ccc gaa gac aag gat att ttt ctg gaa ctt tat gtt tcc gga 144
Ser Phe Pro Glu Asp Lys Asp Ile Phe Leu Glu Leu Tyr Val Ser Gly
        35                  40                  45

aga ggg cga cta ttt gat gga caa cgt tta gtt aaa caa gag ctt tta 192
Arg Gly Arg Leu Phe Asp Gly Gln Arg Leu Val Lys Gln Glu Leu Leu
    50                  55                  60

tct tgg act gat aat ttt ggg ttg ttc act tca aag gaa act att ggt 240

Ser Trp Thr Asp Asn Phe Gly Leu Phe Thr Ser Lys Glu Thr Ile Gly
65                  70                  75                  80

att tat caa gtt gag aag atg agc tcc tct tta caa gag ttt atc ata     288
Ile Tyr Gln Val Glu Lys Met Ser Ser Ser Leu Gln Glu Phe Ile Ile
                85                  90                  95

agc tat gct cat cat cct aat ccg cat ctc aca tta ttt ctt ttc aca     336
Ser Tyr Ala His His Pro Asn Pro His Leu Thr Leu Phe Leu Phe Thr
            100                 105                 110

agt aaa gca gag ttc ttc tca gct ttt tca tcg aag ctc cct aat gcc     384
Ser Lys Ala Glu Phe Phe Ser Ala Phe Ser Ser Lys Leu Pro Asn Ala
        115                 120                 125

ctt tgt tta tct ctc ttt ggt gag tat ttt gca gaa cgc gac tct cgt     432
Leu Cys Leu Ser Leu Phe Gly Glu Tyr Phe Ala Glu Arg Asp Ser Arg
    130                 135                 140

ata gca cag gta ttg atc aag cgt tca atg gat gtt caa ctg tct tgt     480
Ile Ala Gln Val Leu Ile Lys Arg Ser Met Asp Val Gln Leu Ser Cys
145                 150                 155                 160

tct tta ggg gtc gct aag ctt ttc gta agt aag ttc ccg cag gtt gga     528
Ser Leu Gly Val Ala Lys Leu Phe Val Ser Lys Phe Pro Gln Val Gly
                165                 170                 175

gtt ttt gaa att ttt agt gaa ttt caa aag ttg att tgt caa att ggg     576
Val Phe Glu Ile Phe Ser Glu Phe Gln Lys Leu Ile Cys Gln Ile Gly
            180                 185                 190

ggc aag agt tgt tta gaa gct tca gac gtt caa tcg ttt gta gaa aaa     624
Gly Lys Ser Cys Leu Glu Ala Ser Asp Val Gln Ser Phe Val Glu Lys
        195                 200                 205

aga gaa tct gct tct tta tgg aaa tta cgg gat gct ctt ctt cgt aga     672
Arg Glu Ser Ala Ser Leu Trp Lys Leu Arg Asp Ala Leu Leu Arg Arg
    210                 215                 220

gat ctc tca aca ggg caa agt ttg atg caa tct ctg gta tct gat ttg     720
Asp Leu Ser Thr Gly Gln Ser Leu Met Gln Ser Leu Val Ser Asp Leu
225                 230                 235                 240

gga gaa gat cct ctt gct att ctc aat ttt tta cgt agc caa tat tta     768
Gly Glu Asp Pro Leu Ala Ile Leu Asn Phe Leu Arg Ser Gln Tyr Leu
                245                 250                 255

tct ggg ctt cga gca ata gcg gaa cag tcg aaa gat cgc aaa aca caa     816
Ser Gly Leu Arg Ala Ile Ala Glu Gln Ser Lys Asp Arg Lys Thr Gln
            260                 265                 270

atc ttt ttc gca gca gga gag cag gct ctt ttg aat gga tta aac tta     864
Ile Phe Phe Ala Ala Gly Glu Gln Ala Leu Leu Asn Gly Leu Asn Leu
        275                 280                 285

ttt ttt cat gcg gag agt ttg att aag aat aac gtg caa gat ccc att     912
Phe Phe His Ala Glu Ser Leu Ile Lys Asn Asn Val Gln Asp Pro Ile
    290                 295                 300

ctt tct tta gaa acc atg att gca aga ctg taa                         945
Leu Ser Leu Glu Thr Met Ile Ala Arg Leu
305                 310                 315

<210> 24
<211> 314
<212> PRT
<213> Chlamydia muridarum

<400> 24
Met Glu Thr Cys Gln Asn Ser Val Arg Ile Glu Ser Ile Lys Asp Phe
1               5                   10                  15

Ile His His Phe Glu Asn Gln Arg Phe Lys Val Phe Ile Ile Gly Ser
            20                  25                  30

Ser Phe Pro Glu Asp Lys Asp Ile Phe Leu Glu Leu Tyr Val Ser Gly
        35                  40                  45

Arg Gly Arg Leu Phe Asp Gly Gln Arg Leu Val Lys Gln Glu Leu Leu
    50                  55                  60

Ser Trp Thr Asp Asn Phe Gly Leu Phe Thr Ser Lys Glu Thr Ile Gly
65                  70                  75                  80

Ile Tyr Gln Val Glu Lys Met Ser Ser Ser Leu Gln Glu Phe Ile Ile
                85                  90                  95

Ser Tyr Ala His His Pro Asn Pro His Leu Thr Leu Phe Leu Phe Thr
            100                 105                 110

Ser Lys Ala Glu Phe Phe Ser Ala Phe Ser Ser Lys Leu Pro Asn Ala
        115                 120                 125

Leu Cys Leu Ser Leu Phe Gly Glu Tyr Phe Ala Glu Arg Asp Ser Arg
    130                 135                 140

Ile Ala Gln Val Leu Ile Lys Arg Ser Met Asp Val Gln Leu Ser Cys
145                 150                 155                 160

Ser Leu Gly Val Ala Lys Leu Phe Val Ser Lys Phe Pro Gln Val Gly
                165                 170                 175

Val Phe Glu Ile Phe Ser Glu Phe Gln Lys Leu Ile Cys Gln Ile Gly
            180                 185                 190

Gly Lys Ser Cys Leu Glu Ala Ser Asp Val Gln Ser Phe Val Glu Lys
        195                 200                 205

Arg Glu Ser Ala Ser Leu Trp Lys Leu Arg Asp Ala Leu Leu Arg Arg
    210                 215                 220

Asp Leu Ser Thr Gly Gln Ser Leu Met Gln Ser Leu Val Ser Asp Leu
225                 230                 235                 240

Gly Glu Asp Pro Leu Ala Ile Leu Asn Phe Leu Arg Ser Gln Tyr Leu
                245                 250                 255

Ser Gly Leu Arg Ala Ile Ala Glu Gln Ser Lys Asp Arg Lys Thr Gln
            260                 265                 270

Ile Phe Phe Ala Ala Gly Glu Gln Ala Leu Leu Asn Gly Leu Asn Leu
        275                 280                 285

Phe Phe His Ala Glu Ser Leu Ile Lys Asn Asn Val Gln Asp Pro Ile
    290                 295                 300

Leu Ser Leu Glu Thr Met Ile Ala Arg Leu
305                 310

<210> 25
<211> 945
<212> DNA
<213> Chlamydia trachomatis

<400> 25
atgggaaaca gccagaatag tattcacata acgtctacta aagattttgt tcagtatatt 60

gaacgtgaac gatttagagt gattgtgatt ggctcttcat ctcttgagga caaagatatc 120

ttttcggaac tttatatttc tggaaggaag agcttttttg acggacagcg tttgttgcaa 180

caagagctct tatcttggac ggatcatttt ggtttgttcg cttcccaaga gactataggc 240

atttatcaag cagagaaaat gagctcttct ctacaagagt ttattataga ttacactcgg 300

catcctaacc cgaatcttac gttgttcctt ttcacaaata aagcagagct gttctcttct 360

ttatcttcga agctttctaa tgctctttgt ttatctttat ttggagaata ttttgctgag 420

cgagatgctc gcatagctca ggttttgata aaacgttcta aagagtcgca gctttcttgc 480

tctttaggag ttgcaaagat ttttgtgagt aagtttcctc agacaggttt atttgaaatt 540

cttagtgaat ttcaaaagtt aatttgtcag atgggagaca aggaatctat agaagcttcg 600

gatattcaat catttgtaga gaaaaaagag gcgatctctc tgtggaagtt gcgagatgct 660

cttcttcgta aagaccgtgt cgctgcacac agtctgatga gatctttggt gtctgatatg 720

ggagaggagc ctctggcgat tctcaacttt ttacgtagtc agtacctctt aggactgagg 780

gcagtggcag agcagtcgaa agagcgtaaa acacaaatat ttattgctgc aggagagccg 840

gctcttttaa atggtttgaa cctgtttttc catacggaaa gtttaattaa gaataacttc 900

caagatccca tgctttcatt agaaatgcta gtttcacgac tttag 945

<210> 26
<211> 945
<212> DNA
<213> Chlamydia trachomatis

<220>
<221> CDS
<222> (1)..(945)

<400> 26
atg gga aac agc cag aat agt att cac ata acg tct act aaa gat ttt 48
Met Gly Asn Ser Gln Asn Ser Ile His Ile Thr Ser Thr Lys Asp Phe
1               5                   10                  15

gtt cag tat att gaa cgt gaa cga ttt aga gtg att gtg att ggc tct 96
Val Gln Tyr Ile Glu Arg Glu Arg Phe Arg Val Ile Val Ile Gly Ser
            20                  25                  30

tca tct ctt gag gac aaa gat atc ttt tcg gaa ctt tat att tct gga 144
Ser Ser Leu Glu Asp Lys Asp Ile Phe Ser Glu Leu Tyr Ile Ser Gly
        35                  40                  45

agg aag agc ttt ttt gac gga cag cgt ttg ttg caa caa gag ctc tta 192
Arg Lys Ser Phe Phe Asp Gly Gln Arg Leu Leu Gln Gln Glu Leu Leu
    50                  55                  60

tct tgg acg gat cat ttt ggt ttg ttc gct tcc caa gag act ata ggc 240
Ser Trp Thr Asp His Phe Gly Leu Phe Ala Ser Gln Glu Thr Ile Gly
65                  70                  75                  80

att tat caa gca gag aaa atg agc tct tct cta caa gag ttt att ata 288
Ile Tyr Gln Ala Glu Lys Met Ser Ser Ser Leu Gln Glu Phe Ile Ile
                85                  90                  95

gat tac act cgg cat cct aac ccg aat ctt acg ttg ttc ctt ttc aca     336
Asp Tyr Thr Arg His Pro Asn Pro Asn Leu Thr Leu Phe Leu Phe Thr
            100                 105                 110

aat aaa gca gag ctg ttc tct tct tta tct tcg aag ctt tct aat gct     384
Asn Lys Ala Glu Leu Phe Ser Ser Leu Ser Ser Lys Leu Ser Asn Ala
        115                 120                 125

ctt tgt tta tct tta ttt gga gaa tat ttt gct gag cga gat gct cgc     432
Leu Cys Leu Ser Leu Phe Gly Glu Tyr Phe Ala Glu Arg Asp Ala Arg
    130                 135                 140

ata gct cag gtt ttg ata aaa cgt tct aaa gag tcg cag ctt tct tgc     480
Ile Ala Gln Val Leu Ile Lys Arg Ser Lys Glu Ser Gln Leu Ser Cys
145                 150                 155                 160

tct tta gga gtt gca aag att ttt gtg agt aag ttt cct cag aca ggt     528
Ser Leu Gly Val Ala Lys Ile Phe Val Ser Lys Phe Pro Gln Thr Gly
                165                 170                 175

tta ttt gaa att ctt agt gaa ttt caa aag tta att tgt cag atg gga     576
Leu Phe Glu Ile Leu Ser Glu Phe Gln Lys Leu Ile Cys Gln Met Gly
            180                 185                 190

gac aag gaa tct ata gaa gct tcg gat att caa tca ttt gta gag aaa     624
Asp Lys Glu Ser Ile Glu Ala Ser Asp Ile Gln Ser Phe Val Glu Lys
        195                 200                 205

aaa gag gcg atc tct ctg tgg aag ttg cga gat gct ctt ctt cgt aaa     672
Lys Glu Ala Ile Ser Leu Trp Lys Leu Arg Asp Ala Leu Leu Arg Lys
    210                 215                 220

gac cgt gtc gct gca cac agt ctg atg aga tct ttg gtg tct gat atg     720
Asp Arg Val Ala Ala His Ser Leu Met Arg Ser Leu Val Ser Asp Met
225                 230                 235                 240

gga gag gag cct ctg gcg att ctc aac ttt tta cgt agt cag tac ctc     768
Gly Glu Glu Pro Leu Ala Ile Leu Asn Phe Leu Arg Ser Gln Tyr Leu
                245                 250                 255

tta gga ctg agg gca gtg gca gag cag tcg aaa gag cgt aaa aca caa     816
Leu Gly Leu Arg Ala Val Ala Glu Gln Ser Lys Glu Arg Lys Thr Gln
            260                 265                 270

ata ttt att gct gca gga gag ccg gct ctt tta aat ggt ttg aac ctg     864
Ile Phe Ile Ala Ala Gly Glu Pro Ala Leu Leu Asn Gly Leu Asn Leu
        275                 280                 285

ttt ttc cat acg gaa agt tta att aag aat aac ttc caa gat ccc atg     912
Phe Phe His Thr Glu Ser Leu Ile Lys Asn Asn Phe Gln Asp Pro Met
    290                 295                 300

ctt tca tta gaa atg cta gtt tca cga ctt tag                         945
Leu Ser Leu Glu Met Leu Val Ser Arg Leu
305                 310                 315

<210> 27
<211> 314
<212> PRT
<213> Chlamydia trachomatis

<400> 27
Met Gly Asn Ser Gln Asn Ser Ile His Ile Thr Ser Thr Lys Asp Phe
  1               5                  10                  15

Val Gln Tyr Ile Glu Arg Glu Arg Phe Arg Val Ile Val Ile Gly Ser
             20                  25                  30

Ser Ser Leu Glu Asp Lys Asp Ile Phe Ser Glu Leu Tyr Ile Ser Gly
        35                  40                  45

Arg Lys Ser Phe Phe Asp Gly Gln Arg Leu Leu Gln Gln Glu Leu Leu
    50                  55                  60

Ser Trp Thr Asp His Phe Gly Leu Phe Ala Ser Gln Glu Thr Ile Gly
65                  70                  75                  80

Ile Tyr Gln Ala Glu Lys Met Ser Ser Ser Leu Gln Glu Phe Ile Ile
                85                  90                  95

Asp Tyr Thr Arg His Pro Asn Pro Asn Leu Thr Leu Phe Leu Phe Thr
            100                 105                 110

Asn Lys Ala Glu Leu Phe Ser Ser Leu Ser Ser Lys Leu Ser Asn Ala
        115                 120                 125

Leu Cys Leu Ser Leu Phe Gly Glu Tyr Phe Ala Glu Arg Asp Ala Arg
    130                 135                 140

Ile Ala Gln Val Leu Ile Lys Arg Ser Lys Glu Ser Gln Leu Ser Cys
145                 150                 155                 160

Ser Leu Gly Val Ala Lys Ile Phe Val Ser Lys Phe Pro Gln Thr Gly
                165                 170                 175

Leu Phe Glu Ile Leu Ser Glu Phe Gln Lys Leu Ile Cys Gln Met Gly
            180                 185                 190

Asp Lys Glu Ser Ile Glu Ala Ser Asp Ile Gln Ser Phe Val Glu Lys
        195                 200                 205

Lys Glu Ala Ile Ser Leu Trp Lys Leu Arg Asp Ala Leu Leu Arg Lys
    210                 215                 220

Asp Arg Val Ala Ala His Ser Leu Met Arg Ser Leu Val Ser Asp Met
225                 230                 235                 240

Gly Glu Glu Pro Leu Ala Ile Leu Asn Phe Leu Arg Ser Gln Tyr Leu
                245                 250                 255

Leu Gly Leu Arg Ala Val Ala Glu Gln Ser Lys Glu Arg Lys Thr Gln
            260                 265                 270

Ile Phe Ile Ala Ala Gly Glu Pro Ala Leu Leu Asn Gly Leu Asn Leu
        275                 280                 285

Phe Phe His Thr Glu Ser Leu Ile Lys Asn Asn Phe Gln Asp Pro Met
    290                 295                 300

Leu Ser Leu Glu Met Leu Val Ser Arg Leu
305                 310


<210> 28
<211> 948
<212> DNA
<213> Chlamydophila pneumonia

<400> 28
atgactctac ctatgcaaaa atccttaacg agttttgatg acttttccca ggcgtatgca 60

gagaaagtgc ccgctatagc tcttataggg agtgctttgg aagacgataa agatgcgctg 120

attgaattat tagtctctga gagcttcaaa gagctcggtg gtcagggact catgccagca 180

accctcatgt cttggaccga gacgtttgca ctctttcaag agcatgaaac tttggggatt 240
attcatgcag agaaattccc tctagcaact aaggaatttc taagccgcta tgctcggaat 300
cctcaacctc accttacgat tttgatcttc accacaaaac aagaatgctt tcgagaactg 360
tcaaaagcct tgccatcggc tctttctttg agtttatttg gtgagtggcc cgcagatcgt 420
cagaaaagga tcatacgcct cctgttgcaa agagctgagc gtgtggggat ttcttgctct 480
caatcattgg catctttgtt tttgcgtgca cttgcttcaa cctctcttcc tgatattctc 540
agtgaattcg ataagctact gtgctctgtt ggcaagaaaa cgtccttgga tcactctgat 600
attaaagagc tcgttgtcaa aaaagaaaag gcttccctat ggaaatttcg agactctcta 660
ttgaagaggg atccggtaga aggtcaccag cagttgcatt ttctactcga ggatggtgaa 720
gatcccttgg ggattattac tttccttcgt acccaatgtc tctatggttt acgtagtatt 780
gaagagggat cgaaagaaaa taaacaccga atgttcgtcc tttatggaaa ggagagacta 840
caccaagcct taaattcttt attttatgca gaaaccttaa ttaaaaataa tgtccaagat 900
cctatagtag ctgtggaaac tttagtgatt agaatggtta acctgtga 948

<210> 29
<211> 948
<212> DNA
<213> Chlamydophila pneumonia

<220>
<221> CDS
<222> (1)..(948)

<400> 29
atg act cta cct atg caa aaa tcc tta acg agt ttt gat gac ttt tcc 48
Met Thr Leu Pro Met Gln Lys Ser Leu Thr Ser Phe Asp Asp Phe Ser
1               5                   10                  15

cag gcg tat gca gag aaa gtg ccc gct ata gct ctt ata ggg agt gct 96
Gln Ala Tyr Ala Glu Lys Val Pro Ala Ile Ala Leu Ile Gly Ser Ala
            20                  25                  30

ttg gaa gac gat aaa gat gcg ctg att gaa tta tta gtc tct gag agc 144
Leu Glu Asp Asp Lys Asp Ala Leu Ile Glu Leu Leu Val Ser Glu Ser
        35                  40                  45

ttc aaa gag ctc ggt ggt cag gga ctc atg cca gca acc ctc atg tct 192
Phe Lys Glu Leu Gly Gly Gln Gly Leu Met Pro Ala Thr Leu Met Ser
    50                  55                  60

tgg acc gag acg ttt gca ctc ttt caa gag cat gaa act ttg ggg att 240
Trp Thr Glu Thr Phe Ala Leu Phe Gln Glu His Glu Thr Leu Gly Ile
65                  70                  75                  80

att cat gca gag aaa ttc cct cta gca act aag gaa ttt cta agc cgc 288
Ile His Ala Glu Lys Phe Pro Leu Ala Thr Lys Glu Phe Leu Ser Arg
                85                  90                  95

tat gct cgg aat cct caa cct cac ctt acg att ttg atc ttc acc aca 336
Tyr Ala Arg Asn Pro Gln Pro His Leu Thr Ile Leu Ile Phe Thr Thr
            100                 105                 110

aaa caa gaa tgc ttt cga gaa ctg tca aaa gcc ttg cca tcg gct ctt 384
Lys Gln Glu Cys Phe Arg Glu Leu Ser Lys Ala Leu Pro Ser Ala Leu
        115                 120                 125

tct ttg agt tta ttt ggt gag tgg ccc gca gat cgt cag aaa agg atc     432
Ser Leu Ser Leu Phe Gly Glu Trp Pro Ala Asp Arg Gln Lys Arg Ile
    130                 135                 140

ata cgc ctc ctg ttg caa aga gct gag cgt gtg ggg att tct tgc tct     480
Ile Arg Leu Leu Leu Gln Arg Ala Glu Arg Val Gly Ile Ser Cys Ser
145                 150                 155                 160

caa tca ttg gca tct ttg ttt ttg cgt gca ctt gct tca acc tct ctt     528
Gln Ser Leu Ala Ser Leu Phe Leu Arg Ala Leu Ala Ser Thr Ser Leu
                165                 170                 175

cct gat att ctc agt gaa ttc gat aag cta ctg tgc tct gtt ggc aag     576
Pro Asp Ile Leu Ser Glu Phe Asp Lys Leu Leu Cys Ser Val Gly Lys
            180                 185                 190

aaa acg tcc ttg gat cac tct gat att aaa gag ctc gtt gtc aaa aaa     624
Lys Thr Ser Leu Asp His Ser Asp Ile Lys Glu Leu Val Val Lys Lys
        195                 200                 205

gaa aag gct tcc cta tgg aaa ttt cga gac tct cta ttg aag agg gat     672
Glu Lys Ala Ser Leu Trp Lys Phe Arg Asp Ser Leu Leu Lys Arg Asp
    210                 215                 220

ccg gta gaa ggt cac cag cag ttg cat ttt cta ctc gag gat ggt gaa     720
Pro Val Glu Gly His Gln Gln Leu His Phe Leu Leu Glu Asp Gly Glu
225                 230                 235                 240

gat ccc ttg ggg att att act ttc ctt cgt acc caa tgt ctc tat ggt     768
Asp Pro Leu Gly Ile Ile Thr Phe Leu Arg Thr Gln Cys Leu Tyr Gly
                245                 250                 255

tta cgt agt att gaa gag gga tcg aaa gaa aat aaa cac cga atg ttc     816
Leu Arg Ser Ile Glu Glu Gly Ser Lys Glu Asn Lys His Arg Met Phe
            260                 265                 270

gtc ctt tat gga aag gag aga cta cac caa gcc tta aat tct tta ttt     864
Val Leu Tyr Gly Lys Glu Arg Leu His Gln Ala Leu Asn Ser Leu Phe
        275                 280                 285

tat gca gaa acc tta att aaa aat aat gtc caa gat cct ata gta gct     912
Tyr Ala Glu Thr Leu Ile Lys Asn Asn Val Gln Asp Pro Ile Val Ala
    290                 295                 300

gtg gaa act tta gtg att aga atg gtt aac ctg tga                     948
Val Glu Thr Leu Val Ile Arg Met Val Asn Leu
305                 310                 315

<210> 30
<211> 315
<212> PRT
<213> Chlamydophila pneumonia

<400> 30
Met Thr Leu Pro Met Gln Lys Ser Leu Thr Ser Phe Asp Asp Phe Ser
1               5                   10                  15

Gln Ala Tyr Ala Glu Lys Val Pro Ala Ile Ala Leu Ile Gly Ser Ala
            20                  25                  30

Leu Glu Asp Asp Lys Asp Ala Leu Ile Glu Leu Leu Val Ser Glu Ser
        35                  40                  45

Phe Lys Glu Leu Gly Gly Gln Gly Leu Met Pro Ala Thr Leu Met Ser
    50                  55                  60

Trp Thr Glu Thr Phe Ala Leu Phe Gln Glu His Glu Thr Leu Gly Ile
65                  70                  75                  80

Ile His Ala Glu Lys Phe Pro Leu Ala Thr Lys Glu Phe Leu Ser Arg
                85                  90                  95

Tyr Ala Arg Asn Pro Gln Pro His Leu Thr Ile Leu Ile Phe Thr Thr
            100                 105                 110

Lys Gln Glu Cys Phe Arg Glu Leu Ser Lys Ala Leu Pro Ser Ala Leu
        115                 120                 125

Ser Leu Ser Leu Phe Gly Glu Trp Pro Ala Asp Arg Gln Lys Arg Ile
    130                 135                 140

Ile Arg Leu Leu Leu Gln Arg Ala Glu Arg Val Gly Ile Ser Cys Ser
145                 150                 155                 160

Gln Ser Leu Ala Ser Leu Phe Leu Arg Ala Leu Ala Ser Thr Ser Leu
                165                 170                 175

Pro Asp Ile Leu Ser Glu Phe Asp Lys Leu Leu Cys Ser Val Gly Lys
            180                 185                 190

Lys Thr Ser Leu Asp His Ser Asp Ile Lys Glu Leu Val Val Lys Lys
        195                 200                 205

Glu Lys Ala Ser Leu Trp Lys Phe Arg Asp Ser Leu Leu Lys Arg Asp
    210                 215                 220

Pro Val Glu Gly His Gln Gln Leu His Phe Leu Leu Glu Asp Gly Glu
225                 230                 235                 240

Asp Pro Leu Gly Ile Ile Thr Phe Leu Arg Thr Gln Cys Leu Tyr Gly
                245                 250                 255

Leu Arg Ser Ile Glu Glu Gly Ser Lys Glu Asn Lys His Arg Met Phe
            260                 265                 270

Val Leu Tyr Gly Lys Glu Arg Leu His Gln Ala Leu Asn Ser Leu Phe
        275                 280                 285

Tyr Ala Glu Thr Leu Ile Lys Asn Asn Val Gln Asp Pro Ile Val Ala
    290                 295                 300

Val Glu Thr Leu Val Ile Arg Met Val Asn Leu
305                 310                 315

<210> 31
<211> 903
<212> DNA
<213> Deinococcus radiodurans

<400> 31
atgccagtcc tcgcctttac cggcaaccgt tttctggctg acgaaaccct gcgcgacacg 60

ctgagtgcgc gtgggctcaa cgcccgtgac ctgccgcgct tttccggcga ggacgtgagc 120

gccgagacgc tggggccgca cctcgcgccg agtctctttg gcgacggcgg cgtagtcgtc 180

gatttcgagg gcctcaagcc cgacaaggcc ctgctggagc tgctttcctc ggcgcccgtc 240

acggtggccg tgctcgacga ggcgccgccc gcaacccggc tcaagctcta tcagaaggcg 300

ggcgaggtta tcccctcggc ggccccgagt aagcccggcg acgtgaccgg ctgggtggtc 360

acgcgggcca aaaaaatggg cctgcggttg gagcgcgacg cggcgagcta tctggccgag 420

gtgttcggtg ccgacctggc gggcatcgcg ggcgagttga acaagctgga gctgctcggc 480

ggcgcgctga atcgagaacg ggtgcagggc atcgtgggcc gcgatccgcc cggcgactcg 540

tttgccatgc tcggcgcggc gacggcgggc cgccccggtg aagcggtgct gcaactgcgc 600

cgcctgctcg gctcgggcga agaccccttc cggctgctcg gcgcggtggt gtggcagtac 660

agcctgattg cccgcacggt cggcctgctc caggaggaag gccgcgtgaa tgacgcggtg 720

gccgctcagc gcctgggcgt caagcccttt ccggcgaaaa aggcgctgga agtcgcgcgg 780

cgcctgagtg aaagccggat tcgcagccac ctgggcgcca ttctcgatgc cgacctcgcc 840

atgaagcgcg ggctggaccc gcaagtcgcc cttgagcggc tgctggtcaa gctgagcgta 900

tag 903

<210> 32
<211> 903
<212> DNA
<213> Deinococcus radiodurans

<220>
<221> CDS
<222> (1)..(903)

<400> 32
atg cca gtc ctc gcc ttt acc ggc aac cgt ttt ctg gct gac gaa acc 48
Met Pro Val Leu Ala Phe Thr Gly Asn Arg Phe Leu Ala Asp Glu Thr
1               5                   10                  15

ctg cgc gac acg ctg agt gcg cgt ggg ctc aac gcc cgt gac ctg ccg 96
Leu Arg Asp Thr Leu Ser Ala Arg Gly Leu Asn Ala Arg Asp Leu Pro
            20                  25                  30

cgc ttt tcc ggc gag gac gtg agc gcc gag acg ctg ggg ccg cac ctc 144
Arg Phe Ser Gly Glu Asp Val Ser Ala Glu Thr Leu Gly Pro His Leu
        35                  40                  45

gcg ccg agt ctc ttt ggc gac ggc ggc gta gtc gtc gat ttc gag ggc 192
Ala Pro Ser Leu Phe Gly Asp Gly Gly Val Val Val Asp Phe Glu Gly
    50                  55                  60

ctc aag ccc gac aag gcc ctg ctg gag ctg ctt tcc tcg gcg ccc gtc 240
Leu Lys Pro Asp Lys Ala Leu Leu Glu Leu Leu Ser Ser Ala Pro Val
65                  70                  75                  80

acg gtg gcc gtg ctc gac gag gcg ccg ccc gca acc cgg ctc aag ctc 288
Thr Val Ala Val Leu Asp Glu Ala Pro Pro Ala Thr Arg Leu Lys Leu
                85                  90                  95

tat cag aag gcg ggc gag gtt atc ccc tcg gcg gcc ccg agt aag ccc 336
Tyr Gln Lys Ala Gly Glu Val Ile Pro Ser Ala Ala Pro Ser Lys Pro
            100                 105                 110

ggc gac gtg acc ggc tgg gtg gtc acg cgg gcc aaa aaa atg ggc ctg 384
Gly Asp Val Thr Gly Trp Val Val Thr Arg Ala Lys Lys Met Gly Leu
        115                 120                 125

cgg ttg gag cgc gac gcg gcg agc tat ctg gcc gag gtg ttc ggt gcc 432
Arg Leu Glu Arg Asp Ala Ala Ser Tyr Leu Ala Glu Val Phe Gly Ala
    130                 135                 140

gac ctg gcg ggc atc gcg ggc gag ttg aac aag ctg gag ctg ctc ggc 480
Asp Leu Ala Gly Ile Ala Gly Glu Leu Asn Lys Leu Glu Leu Leu Gly

145                 150                 155                 160

ggc gcg ctg aat cga gaa cgg gtg cag ggc atc gtg ggc cgc gat ccg     528
Gly Ala Leu Asn Arg Glu Arg Val Gln Gly Ile Val Gly Arg Asp Pro
                165                 170                 175

ccc ggc gac tcg ttt gcc atg ctc ggc gcg gcg acg gcg ggc cgc ccc     576
Pro Gly Asp Ser Phe Ala Met Leu Gly Ala Ala Thr Ala Gly Arg Pro
            180                 185                 190

ggt gaa gcg gtg ctg caa ctg cgc cgc ctg ctc ggc tcg ggc gaa gac     624
Gly Glu Ala Val Leu Gln Leu Arg Arg Leu Leu Gly Ser Gly Glu Asp
        195                 200                 205

ccc ttc cgg ctg ctc ggc gcg gtg gtg tgg cag tac agc ctg att gcc     672
Pro Phe Arg Leu Leu Gly Ala Val Val Trp Gln Tyr Ser Leu Ile Ala
    210                 215                 220

cgc acg gtc ggc ctg ctc cag gag gaa ggc cgc gtg aat gac gcg gtg     720
Arg Thr Val Gly Leu Leu Gln Glu Glu Gly Arg Val Asn Asp Ala Val
225                 230                 235                 240

gcc gct cag cgc ctg ggc gtc aag ccc ttt ccg gcg aaa aag gcg ctg     768
Ala Ala Gln Arg Leu Gly Val Lys Pro Phe Pro Ala Lys Lys Ala Leu
                245                 250                 255

gaa gtc gcg cgg cgc ctg agt gaa agc cgg att cgc agc cac ctg ggc     816
Glu Val Ala Arg Arg Leu Ser Glu Ser Arg Ile Arg Ser His Leu Gly
            260                 265                 270

gcc att ctc gat gcc gac ctc gcc atg aag cgc ggg ctg gac ccg caa     864
Ala Ile Leu Asp Ala Asp Leu Ala Met Lys Arg Gly Leu Asp Pro Gln
        275                 280                 285

gtc gcc ctt gag cgg ctg ctg gtc aag ctg agc gta tag                 903
Val Ala Leu Glu Arg Leu Leu Val Lys Leu Ser Val
    290                 295                 300

<210> 33
<211> 300
<212> PRT
<213> Deinococcus radiodurans

<400> 33
Met Pro Val Leu Ala Phe Thr Gly Asn Arg Phe Leu Ala Asp Glu Thr
1               5                   10                  15

Leu Arg Asp Thr Leu Ser Ala Arg Gly Leu Asn Ala Arg Asp Leu Pro
            20                  25                  30

Arg Phe Ser Gly Glu Asp Val Ser Ala Glu Thr Leu Gly Pro His Leu
        35                  40                  45

Ala Pro Ser Leu Phe Gly Asp Gly Gly Val Val Val Asp Phe Glu Gly
    50                  55                  60

Leu Lys Pro Asp Lys Ala Leu Leu Glu Leu Leu Ser Ser Ala Pro Val
65                  70                  75                  80

Thr Val Ala Val Leu Asp Glu Ala Pro Pro Ala Thr Arg Leu Lys Leu
                85                  90                  95

Tyr Gln Lys Ala Gly Glu Val Ile Pro Ser Ala Ala Pro Ser Lys Pro
            100                 105                 110

Gly Asp Val Thr Gly Trp Val Val Thr Arg Ala Lys Lys Met Gly Leu
        115                 120                 125

Arg Leu Glu Arg Asp Ala Ala Ser Tyr Leu Ala Glu Val Phe Gly Ala
    130                 135                 140

Asp Leu Ala Gly Ile Ala Gly Glu Leu Asn Lys Leu Glu Leu Leu Gly
145                 150                 155                 160

Gly Ala Leu Asn Arg Glu Arg Val Gln Gly Ile Val Gly Arg Asp Pro
                165                 170                 175

Pro Gly Asp Ser Phe Ala Met Leu Gly Ala Ala Thr Ala Gly Arg Pro
            180                 185                 190

Gly Glu Ala Val Leu Gln Leu Arg Arg Leu Leu Gly Ser Gly Glu Asp
        195                 200                 205

Pro Phe Arg Leu Leu Gly Ala Val Val Trp Gln Tyr Ser Leu Ile Ala
    210                 215                 220

Arg Thr Val Gly Leu Leu Gln Glu Glu Gly Arg Val Asn Asp Ala Val
225                 230                 235                 240

Ala Ala Gln Arg Leu Gly Val Lys Pro Phe Pro Ala Lys Lys Ala Leu
                245                 250                 255

Glu Val Ala Arg Arg Leu Ser Glu Ser Arg Ile Arg Ser His Leu Gly
            260                 265                 270

Ala Ile Leu Asp Ala Asp Leu Ala Met Lys Arg Gly Leu Asp Pro Gln
        275                 280                 285

Val Ala Leu Glu Arg Leu Leu Val Lys Leu Ser Val
    290                 295                 300

<210> 34
<211> 1035
<212> DNA
<213> Haemophilus influenzae

<400> 34
atgaaccgca tttttccaga gcaactaaat catcatcttg cgcaaggctt ggctagagtt 60

tatctcttgc aagggcaaga tcctttatta cttagtgaaa ctgaggatac tatttgtcaa 120

gtggcaaacc tgcaaggttt tgatgaaaaa aatactattc aggttgatag ccaaactgat 180

tgggcgcaac ttatagaatc ttgtcaatct ataggattgt tttttagtaa acaaatacta 240

agcttgaatt tacctgaaaa tttcaccgca cttttgcaga aaaatctgca agaacttata 300

tccgtattgc ataaagatgt cttgcttatt ttacaggtag caaaattggc taaagggatt 360

gaaaagcaaa cgtggtttat tacacttaat caatatgaac caaatacaat actgataaat 420

tgtcaaacac cgacggtaga aaacttacca cgttgggtaa aaaatcgtac caaagcaatg 480

ggattagacg ctgacaacga agcaatccaa caactttgtt atagttatga aaataacctg 540

ctcgcactca aacaagctct gcagctttta gatttgctct atcctgacca taaacttaac 600

tacaatcgtg ttattagcgt ggtggaacaa tcctcaattt ttacaccatt tcaatggatt 660

gatgcgttgc ttgttggaaa ggcaaatcgt gctaaacgta ttttaaaagg tttacaagct 720

gaagatgtgc aacctgttat tttattacgc acactacagc gagaattatt tacgttgctt 780

gaactcacaa aaccacagca acgtattgtg acaacggaaa aacttccaat tcaacaaatt 840

aagactgaat ttgaccgtct aaaaatctgg caaaatcgcc gaccgctctt tttaagtgcg 900

attcaacgtt taacttacca gactctctac gaaattatcc aagaactcgc taatattgaa 960

cgccttgcta aacaagaatt tagtgatgaa gtgtggatca aactcgctga tttatctgta 1020

aaaatttgtt tgtag 1035

<210> 35
<211> 1035
<212> DNA
<213> Haemophilus influenzae

<220>
<221> CDS
<222> (1)..(1035)

<400> 35
atg aac cgc att ttt cca gag caa cta aat cat cat ctt gcg caa ggc 48
Met Asn Arg Ile Phe Pro Glu Gln Leu Asn His His Leu Ala Gln Gly
1               5                   10                  15

ttg gct aga gtt tat ctc ttg caa ggg caa gat cct tta tta ctt agt 96
Leu Ala Arg Val Tyr Leu Leu Gln Gly Gln Asp Pro Leu Leu Leu Ser
            20                  25                  30

gaa act gag gat act att tgt caa gtg gca aac ctg caa ggt ttt gat 144
Glu Thr Glu Asp Thr Ile Cys Gln Val Ala Asn Leu Gln Gly Phe Asp
        35                  40                  45

gaa aaa aat act att cag gtt gat agc caa act gat tgg gcg caa ctt 192
Glu Lys Asn Thr Ile Gln Val Asp Ser Gln Thr Asp Trp Ala Gln Leu
    50                  55                  60

ata gaa tct tgt caa tct ata gga ttg ttt ttt agt aaa caa ata cta 240
Ile Glu Ser Cys Gln Ser Ile Gly Leu Phe Phe Ser Lys Gln Ile Leu
65                  70                  75                  80

agc ttg aat tta cct gaa aat ttc acc gca ctt ttg cag aaa aat ctg 288
Ser Leu Asn Leu Pro Glu Asn Phe Thr Ala Leu Leu Gln Lys Asn Leu
                85                  90                  95

caa gaa ctt ata tcc gta ttg cat aaa gat gtc ttg ctt att tta cag 336
Gln Glu Leu Ile Ser Val Leu His Lys Asp Val Leu Leu Ile Leu Gln
            100                 105                 110

gta gca aaa ttg gct aaa ggg att gaa aag caa acg tgg ttt att aca 384
Val Ala Lys Leu Ala Lys Gly Ile Glu Lys Gln Thr Trp Phe Ile Thr
        115                 120                 125

ctt aat caa tat gaa cca aat aca ata ctg ata aat tgt caa aca ccg 432
Leu Asn Gln Tyr Glu Pro Asn Thr Ile Leu Ile Asn Cys Gln Thr Pro
    130                 135                 140

acg gta gaa aac tta cca cgt tgg gta aaa aat cgt acc aaa gca atg 480
Thr Val Glu Asn Leu Pro Arg Trp Val Lys Asn Arg Thr Lys Ala Met
145                 150                 155                 160

gga tta gac gct gac aac gaa gca atc caa caa ctt tgt tat agt tat 528
Gly Leu Asp Ala Asp Asn Glu Ala Ile Gln Gln Leu Cys Tyr Ser Tyr
                165                 170                 175

gaa aat aac ctg ctc gca ctc aaa caa gct ctg cag ctt tta gat ttg 576
Glu Asn Asn Leu Leu Ala Leu Lys Gln Ala Leu Gln Leu Leu Asp Leu
            180                 185                 190

ctc tat cct gac cat aaa ctt aac tac aat cgt gtt att agc gtg gtg     624
Leu Tyr Pro Asp His Lys Leu Asn Tyr Asn Arg Val Ile Ser Val Val
        195                 200                 205

gaa caa tcc tca att ttt aca cca ttt caa tgg att gat gcg ttg ctt     672
Glu Gln Ser Ser Ile Phe Thr Pro Phe Gln Trp Ile Asp Ala Leu Leu
    210                 215                 220

gtt gga aag gca aat cgt gct aaa cgt att tta aaa ggt tta caa gct     720
Val Gly Lys Ala Asn Arg Ala Lys Arg Ile Leu Lys Gly Leu Gln Ala
225                 230                 235                 240

gaa gat gtg caa cct gtt att tta tta cgc aca cta cag cga gaa tta     768
Glu Asp Val Gln Pro Val Ile Leu Leu Arg Thr Leu Gln Arg Glu Leu
                245                 250                 255

ttt acg ttg ctt gaa ctc aca aaa cca cag caa cgt att gtg aca acg     816
Phe Thr Leu Leu Glu Leu Thr Lys Pro Gln Gln Arg Ile Val Thr Thr
            260                 265                 270

gaa aaa ctt cca att caa caa att aag act gaa ttt gac cgt cta aaa     864
Glu Lys Leu Pro Ile Gln Gln Ile Lys Thr Glu Phe Asp Arg Leu Lys
        275                 280                 285

atc tgg caa aat cgc cga ccg ctc ttt tta agt gcg att caa cgt tta     912
Ile Trp Gln Asn Arg Arg Pro Leu Phe Leu Ser Ala Ile Gln Arg Leu
    290                 295                 300

act tac cag act ctc tac gaa att atc caa gaa ctc gct aat att gaa     960
Thr Tyr Gln Thr Leu Tyr Glu Ile Ile Gln Glu Leu Ala Asn Ile Glu
305                 310                 315                 320

cgc ctt gct aaa caa gaa ttt agt gat gaa gtg tgg atc aaa ctc gct     1008
Arg Leu Ala Lys Gln Glu Phe Ser Asp Glu Val Trp Ile Lys Leu Ala
                325                 330                 335

gat tta tct gta aaa att tgt ttg tag                                 1035
Asp Leu Ser Val Lys Ile Cys Leu
            340                 345

<210> 36
<211> 344
<212> PRT
<213> Haemophilus influenzae

<400> 36
Met Asn Arg Ile Phe Pro Glu Gln Leu Asn His His Leu Ala Gln Gly
1               5                   10                  15

Leu Ala Arg Val Tyr Leu Leu Gln Gly Gln Asp Pro Leu Leu Leu Ser
            20                  25                  30

Glu Thr Glu Asp Thr Ile Cys Gln Val Ala Asn Leu Gln Gly Phe Asp
        35                  40                  45

Glu Lys Asn Thr Ile Gln Val Asp Ser Gln Thr Asp Trp Ala Gln Leu
    50                  55                  60

Ile Glu Ser Cys Gln Ser Ile Gly Leu Phe Phe Ser Lys Gln Ile Leu
65                  70                  75                  80

Ser Leu Asn Leu Pro Glu Asn Phe Thr Ala Leu Leu Gln Lys Asn Leu
                85                  90                  95

Gln Glu Leu Ile Ser Val Leu His Lys Asp Val Leu Leu Ile Leu Gln
            100                 105                 110

Val Ala Lys Leu Ala Lys Gly Ile Glu Lys Gln Thr Trp Phe Ile Thr
        115                 120                 125

Leu Asn Gln Tyr Glu Pro Asn Thr Ile Leu Ile Asn Cys Gln Thr Pro
    130                 135                 140

Thr Val Glu Asn Leu Pro Arg Trp Val Lys Asn Arg Thr Lys Ala Met
145                 150                 155                 160

Gly Leu Asp Ala Asp Asn Glu Ala Ile Gln Gln Leu Cys Tyr Ser Tyr
                165                 170                 175

Glu Asn Asn Leu Leu Ala Leu Lys Gln Ala Leu Gln Leu Leu Asp Leu
            180                 185                 190

Leu Tyr Pro Asp His Lys Leu Asn Tyr Asn Arg Val Ile Ser Val Val
        195                 200                 205

Glu Gln Ser Ser Ile Phe Thr Pro Phe Gln Trp Ile Asp Ala Leu Leu
    210                 215                 220

Val Gly Lys Ala Asn Arg Ala Lys Arg Ile Leu Lys Gly Leu Gln Ala
225                 230                 235                 240

Glu Asp Val Gln Pro Val Ile Leu Leu Arg Thr Leu Gln Arg Glu Leu
                245                 250                 255

Phe Thr Leu Leu Glu Leu Thr Lys Pro Gln Gln Arg Ile Val Thr Thr
            260                 265                 270

Glu Lys Leu Pro Ile Gln Gln Ile Lys Thr Glu Phe Asp Arg Leu Lys
        275                 280                 285

Ile Trp Gln Asn Arg Arg Pro Leu Phe Leu Ser Ala Ile Gln Arg Leu
    290                 295                 300

Thr Tyr Gln Thr Leu Tyr Glu Ile Ile Gln Glu Leu Ala Asn Ile Glu
305                 310                 315                 320

Arg Leu Ala Lys Gln Glu Phe Ser Asp Glu Val Trp Ile Lys Leu Ala
                325                 330                 335

Asp Leu Ser Val Lys Ile Cys Leu
            340

<210> 37
<211> 1023
<212> DNA
<213> Helicobacter pylori

<220>
<223> /note=Helicobacter pylori 26695

<400> 37
atgtatcgta aagatttgga ccattattta aaacaacgcc tccctaaagc ggtgtttttg 60

tatggggagt ttgatttttt tatccattat tatattcaaa cgattagcgc gctttttaaa 120

tgcgataacc ctgacataga aacttcgctt ttttatgcga gcgattatga aaaaagccag 180

attgcgaccc ttttagagca ggattcttta tttggaggga gcagtttagt cgttttaaaa 240

ctggattttg ccctgcataa gaaatttaag gaaaatgata tcaatctttt tttaaaagct 300

ttagagcggc ctagccataa caggcttatc atagggcttt ataatgctaa aagcgacacc 360

acaaaataca aatacactag cgatgctatc gttaaatttt tccaaaaaag ccccttgaaa 420

gatgaagcca tttgcgcgcg cttttttatc cctaaaactt gggagagttt gaaattcttg 480

caagaaaggg ctaatttttt gcatttagac atcagcggcc atcttttaaa cgctcttttt 540

gaaattaata acgaagattt aggcgtttcg tttaacgatt tagacaagct agcggtttta 600

aacgcaccca tcactttaga agacattcaa gaattaagct ccaatgcggg ggatatggat 660

ttgcaaaagc tcattttagg gcttttttta aaaaaaagtg cgcttgatat ttatgattat 720

ttgttaaaag agggcaaaaa agatgcggat attttaaggg ggttagagcg ctatttttac 780

caactttttt tatttttcgc tcatattaaa acgaccggtt taatggacgc taaagaggtt 840

ttaggctacg ctccccctaa agaaattgcc gaaaattacg ctaaaaacgc cttgcgtttg 900

aaagaagccg gctataagag ggtttttgaa atttttaggt tatggcacat tcaaagcatg 960

caagggcaaa aggaattggg ctttttgtat ttgacctcca ttcaaaaaat cattaacccc 1020

taa 1023

<210> 38
<211> 1023
<212> DNA
<213> Helicobacter pylori

<220>
<223> /note=Helicobacter pylori 26695

<220>
<221> CDS
<222> (1)..(1023)

<400> 38
atg tat cgt aaa gat ttg gac cat tat tta aaa caa cgc ctc cct aaa 48
Met Tyr Arg Lys Asp Leu Asp His Tyr Leu Lys Gln Arg Leu Pro Lys
1               5                   10                  15

gcg gtg ttt ttg tat ggg gag ttt gat ttt ttt atc cat tat tat att 96
Ala Val Phe Leu Tyr Gly Glu Phe Asp Phe Phe Ile His Tyr Tyr Ile
            20                  25                  30

caa acg att agc gcg ctt ttt aaa tgc gat aac cct gac ata gaa act 144
Gln Thr Ile Ser Ala Leu Phe Lys Cys Asp Asn Pro Asp Ile Glu Thr
        35                  40                  45

tcg ctt ttt tat gcg agc gat tat gaa aaa agc cag att gcg acc ctt 192
Ser Leu Phe Tyr Ala Ser Asp Tyr Glu Lys Ser Gln Ile Ala Thr Leu
    50                  55                  60

tta gag cag gat tct tta ttt gga ggg agc agt tta gtc gtt tta aaa 240
Leu Glu Gln Asp Ser Leu Phe Gly Gly Ser Ser Leu Val Val Leu Lys
65                  70                  75                  80

ctg gat ttt gcc ctg cat aag aaa ttt aag gaa aat gat atc aat ctt 288
Leu Asp Phe Ala Leu His Lys Lys Phe Lys Glu Asn Asp Ile Asn Leu
                85                  90                  95

ttt tta aaa gct tta gag cgg cct agc cat aac agg ctt atc ata ggg 336
Phe Leu Lys Ala Leu Glu Arg Pro Ser His Asn Arg Leu Ile Ile Gly
            100                 105                 110

ctt tat aat gct aaa agc gac acc aca aaa tac aaa tac act agc gat 384
Leu Tyr Asn Ala Lys Ser Asp Thr Thr Lys Tyr Lys Tyr Thr Ser Asp

115                 120                 125

gct atc gtt aaa ttt ttc caa aaa agc ccc ttg aaa gat gaa gcc att      432
Ala Ile Val Lys Phe Phe Gln Lys Ser Pro Leu Lys Asp Glu Ala Ile
    130                 135                 140

tgc gcg cgc ttt ttt atc cct aaa act tgg gag agt ttg aaa ttc ttg      480
Cys Ala Arg Phe Phe Ile Pro Lys Thr Trp Glu Ser Leu Lys Phe Leu
145                 150                 155                 160

caa gaa agg gct aat ttt ttg cat tta gac atc agc ggc cat ctt tta      528
Gln Glu Arg Ala Asn Phe Leu His Leu Asp Ile Ser Gly His Leu Leu
                165                 170                 175

aac gct ctt ttt gaa att aat aac gaa gat tta ggc gtt tcg ttt aac      576
Asn Ala Leu Phe Glu Ile Asn Asn Glu Asp Leu Gly Val Ser Phe Asn
            180                 185                 190

gat tta gac aag cta gcg gtt tta aac gca ccc atc act tta gaa gac      624
Asp Leu Asp Lys Leu Ala Val Leu Asn Ala Pro Ile Thr Leu Glu Asp
        195                 200                 205

att caa gaa tta agc tcc aat gcg ggg gat atg gat ttg caa aag ctc      672
Ile Gln Glu Leu Ser Ser Asn Ala Gly Asp Met Asp Leu Gln Lys Leu
    210                 215                 220

att tta ggg ctt ttt tta aaa aaa agt gcg ctt gat att tat gat tat      720
Ile Leu Gly Leu Phe Leu Lys Lys Ser Ala Leu Asp Ile Tyr Asp Tyr
225                 230                 235                 240

ttg tta aaa gag ggc aaa aaa gat gcg gat att tta agg ggg tta gag      768
Leu Leu Lys Glu Gly Lys Lys Asp Ala Asp Ile Leu Arg Gly Leu Glu
                245                 250                 255

cgc tat ttt tac caa ctt ttt tta ttt ttc gct cat att aaa acg acc      816
Arg Tyr Phe Tyr Gln Leu Phe Leu Phe Phe Ala His Ile Lys Thr Thr
            260                 265                 270

ggt tta atg gac gct aaa gag gtt tta ggc tac gct ccc cct aaa gaa      864
Gly Leu Met Asp Ala Lys Glu Val Leu Gly Tyr Ala Pro Pro Lys Glu
        275                 280                 285

att gcc gaa aat tac gct aaa aac gcc ttg cgt ttg aaa gaa gcc ggc      912
Ile Ala Glu Asn Tyr Ala Lys Asn Ala Leu Arg Leu Lys Glu Ala Gly
    290                 295                 300

tat aag agg gtt ttt gaa att ttt agg tta tgg cac att caa agc atg      960
Tyr Lys Arg Val Phe Glu Ile Phe Arg Leu Trp His Ile Gln Ser Met
305                 310                 315                 320

caa ggg caa aag gaa ttg ggc ttt ttg tat ttg acc tcc att caa aaa      1008
Gln Gly Gln Lys Glu Leu Gly Phe Leu Tyr Leu Thr Ser Ile Gln Lys
                325                 330                 335

atc att aac ccc taa                                                  1023
Ile Ile Asn Pro
            340

<210> 39
<211> 340
<212> PRT
<213> Helicobacter pylori

<220>
<223> /note=Helicobacter pylori 26695

<400> 39

Met Tyr Arg Lys Asp Leu Asp His Tyr Leu Lys Gln Arg Leu Pro Lys
1               5                   10                  15

Ala Val Phe Leu Tyr Gly Glu Phe Asp Phe Phe Ile His Tyr Tyr Ile
            20                  25                  30

Gln Thr Ile Ser Ala Leu Phe Lys Cys Asp Asn Pro Asp Ile Glu Thr
        35                  40                  45

Ser Leu Phe Tyr Ala Ser Asp Tyr Glu Lys Ser Gln Ile Ala Thr Leu
    50                  55                  60

Leu Glu Gln Asp Ser Leu Phe Gly Gly Ser Ser Leu Val Val Leu Lys
65                  70                  75                  80

Leu Asp Phe Ala Leu His Lys Lys Phe Lys Glu Asn Asp Ile Asn Leu
                85                  90                  95

Phe Leu Lys Ala Leu Glu Arg Pro Ser His Asn Arg Leu Ile Ile Gly
            100                 105                 110

Leu Tyr Asn Ala Lys Ser Asp Thr Thr Lys Tyr Lys Tyr Thr Ser Asp
        115                 120                 125

Ala Ile Val Lys Phe Phe Gln Lys Ser Pro Leu Lys Asp Glu Ala Ile
    130                 135                 140

Cys Ala Arg Phe Phe Ile Pro Lys Thr Trp Glu Ser Leu Lys Phe Leu
145                 150                 155                 160

Gln Glu Arg Ala Asn Phe Leu His Leu Asp Ile Ser Gly His Leu Leu
                165                 170                 175

Asn Ala Leu Phe Glu Ile Asn Asn Glu Asp Leu Gly Val Ser Phe Asn
            180                 185                 190

Asp Leu Asp Lys Leu Ala Val Leu Asn Ala Pro Ile Thr Leu Glu Asp
        195                 200                 205

Ile Gln Glu Leu Ser Ser Asn Ala Gly Asp Met Asp Leu Gln Lys Leu
    210                 215                 220

Ile Leu Gly Leu Phe Leu Lys Lys Ser Ala Leu Asp Ile Tyr Asp Tyr
225                 230                 235                 240

Leu Leu Lys Glu Gly Lys Lys Asp Ala Asp Ile Leu Arg Gly Leu Glu
                245                 250                 255

Arg Tyr Phe Tyr Gln Leu Phe Leu Phe Phe Ala His Ile Lys Thr Thr
            260                 265                 270

Gly Leu Met Asp Ala Lys Glu Val Leu Gly Tyr Ala Pro Pro Lys Glu
        275                 280                 285

Ile Ala Glu Asn Tyr Ala Lys Asn Ala Leu Arg Leu Lys Glu Ala Gly
    290                 295                 300

Tyr Lys Arg Val Phe Glu Ile Phe Arg Leu Trp His Ile Gln Ser Met
305                 310                 315                 320

Gln Gly Gln Lys Glu Leu Gly Phe Leu Tyr Leu Thr Ser Ile Gln Lys
                325                 330                 335

Ile Ile Asn Pro
            340

<210> 40

<211> 1023
<212> DNA
<213> Helicobacter pylori

<220>
<223> /note=Helicobacter pylori J99

<400> 40
atgtatcgta aagatttgga taattactta aaacagcgcc tccctaaagc ggtgtttttg 60

tatggggagt ttgatttttt catccattat tatattcaaa cgattagcgc gctttttaaa 120

ggcaataacc ctgacacaga aacttcgctt ttttatgcga gcgattatga aaaaagccag 180

attgcgaccc ttttagagca ggattcttta tttggaggga gcagtttagt tattttaaaa 240

ctggattttg cattgcataa gaaatttaag gaaaatgata tcaatccttt tttaaaagct 300

ttagagcggc ctagccataa taggcttatc atagggcttt ataatgctaa aagcgacacc 360

acaaaataca aatacactag cgaaattatc gttaaatttt tccaaaaaag ccccttgaaa 420

gatgaagcca tttgcgtgcg cttttttacc cctaaagcgt gggagagttt gaaattcttg 480

caagaaaggg ctaatttttt gcatttagac atcagcggcc atcttttaaa cgctcttttt 540

gaaattaata acgaagattt aagcgtttcg tttaacgatt tagacaagct agcggtttta 600

aacgcgccca tcactttaga agacattcaa gaattaagct ccaatgcggg ggatatggat 660

ttgcaaaagc tcattttagg gctttttttg aaaaaaagcg tccttgatat ttatgattat 720

ttgttaaaag agggcaaaaa ggatgcggat attttaaggg ggttagagcg ctatttttac 780

cagctttttt tatttttcgc ccacattaaa acgaccggtt taatggacgc taaagaggtc 840

ttaggctacg ctcctcctaa agagattgta gaaaattacg ctaaaaacgc cctgcgtttg 900

aaagaagccg gctataagag ggtttttgaa atttttaggt tatggcacct tcaaagcatg 960

caagggcaaa aggaattggg ctttttgtat ttgaccccca ttcaaaaaat cattaaccct 1020

tga 1023

<210> 41
<211> 1023
<212> DNA
<213> Helicobacter pylori J99

<220>
<223> /note=Helicobacter pylori J99

<220>
<221> CDS
<222> (1)..(1023)

<400> 41
atg tat cgt aaa gat ttg gat aat tac tta aaa cag cgc ctc cct aaa 48
Met Tyr Arg Lys Asp Leu Asp Asn Tyr Leu Lys Gln Arg Leu Pro Lys
1               5                   10                  15

gcg gtg ttt ttg tat ggg gag ttt gat ttt ttc atc cat tat tat att 96
Ala Val Phe Leu Tyr Gly Glu Phe Asp Phe Phe Ile His Tyr Tyr Ile
            20                  25                  30

caa acg att agc gcg ctt ttt aaa ggc aat aac cct gac aca gaa act 144
Gln Thr Ile Ser Ala Leu Phe Lys Gly Asn Asn Pro Asp Thr Glu Thr

        35                  40                  45

tcg ctt ttt tat gcg agc gat tat gaa aaa agc cag att gcg acc ctt 192
Ser Leu Phe Tyr Ala Ser Asp Tyr Glu Lys Ser Gln Ile Ala Thr Leu
    50                  55                  60

tta gag cag gat tct tta ttt gga ggg agc agt tta gtt att tta aaa 240
Leu Glu Gln Asp Ser Leu Phe Gly Gly Ser Ser Leu Val Ile Leu Lys
65                  70                  75                  80

ctg gat ttt gca ttg cat aag aaa ttt aag gaa aat gat atc aat cct 288
Leu Asp Phe Ala Leu His Lys Lys Phe Lys Glu Asn Asp Ile Asn Pro
                85                  90                  95

ttt tta aaa gct tta gag cgg cct agc cat aat agg ctt atc ata ggg 336
Phe Leu Lys Ala Leu Glu Arg Pro Ser His Asn Arg Leu Ile Ile Gly
            100                 105                 110

ctt tat aat gct aaa agc gac acc aca aaa tac aaa tac act agc gaa 384
Leu Tyr Asn Ala Lys Ser Asp Thr Thr Lys Tyr Lys Tyr Thr Ser Glu
        115                 120                 125

att atc gtt aaa ttt ttc caa aaa agc ccc ttg aaa gat gaa gcc att 432
Ile Ile Val Lys Phe Phe Gln Lys Ser Pro Leu Lys Asp Glu Ala Ile
    130                 135                 140

tgc gtg cgc ttt ttt acc cct aaa gcg tgg gag agt ttg aaa ttc ttg 480
Cys Val Arg Phe Phe Thr Pro Lys Ala Trp Glu Ser Leu Lys Phe Leu
145                 150                 155                 160

caa gaa agg gct aat ttt ttg cat tta gac atc agc ggc cat ctt tta 528
Gln Glu Arg Ala Asn Phe Leu His Leu Asp Ile Ser Gly His Leu Leu
                165                 170                 175

aac gct ctt ttt gaa att aat aac gaa gat tta agc gtt tcg ttt aac 576
Asn Ala Leu Phe Glu Ile Asn Asn Glu Asp Leu Ser Val Ser Phe Asn
            180                 185                 190

gat tta gac aag cta gcg gtt tta aac gcg ccc atc act tta gaa gac 624
Asp Leu Asp Lys Leu Ala Val Leu Asn Ala Pro Ile Thr Leu Glu Asp
        195                 200                 205

att caa gaa tta agc tcc aat gcg ggg gat atg gat ttg caa aag ctc 672
Ile Gln Glu Leu Ser Ser Asn Ala Gly Asp Met Asp Leu Gln Lys Leu
    210                 215                 220

att tta ggg ctt ttt ttg aaa aaa agc gtc ctt gat att tat gat tat 720
Ile Leu Gly Leu Phe Leu Lys Lys Ser Val Leu Asp Ile Tyr Asp Tyr
225                 230                 235                 240

ttg tta aaa gag ggc aaa aag gat gcg gat att tta agg ggg tta gag 768
Leu Leu Lys Glu Gly Lys Lys Asp Ala Asp Ile Leu Arg Gly Leu Glu
                245                 250                 255

cgc tat ttt tac cag ctt ttt tta ttt ttc gcc cac att aaa acg acc 816
Arg Tyr Phe Tyr Gln Leu Phe Leu Phe Phe Ala His Ile Lys Thr Thr
            260                 265                 270

ggt tta atg gac gct aaa gag gtc tta ggc tac gct cct cct aaa gag 864
Gly Leu Met Asp Ala Lys Glu Val Leu Gly Tyr Ala Pro Pro Lys Glu
        275                 280                 285

att gta gaa aat tac gct aaa aac gcc ctg cgt ttg aaa gaa gcc ggc 912
Ile Val Glu Asn Tyr Ala Lys Asn Ala Leu Arg Leu Lys Glu Ala Gly
    290                 295                 300

tat aag agg gtt ttt gaa att ttt agg tta tgg cac ctt caa agc atg 960
Tyr Lys Arg Val Phe Glu Ile Phe Arg Leu Trp His Leu Gln Ser Met

305                 310                 315                 320

caa ggg caa aag gaa ttg ggc ttt ttg tat ttg acc ccc att caa aaa      1008
Gln Gly Gln Lys Glu Leu Gly Phe Leu Tyr Leu Thr Pro Ile Gln Lys
                325                 330                 335

atc att aac cct tga                                                  1023
Ile Ile Asn Pro
            340

<210> 42
<211> 340
<212> PRT
<213> Helicobacter pylori J99

<220>
<223> /note=Helicobacter pylori J99

<400> 42
Met Tyr Arg Lys Asp Leu Asp Asn Tyr Leu Lys Gln Arg Leu Pro Lys
  1               5                  10                  15

Ala Val Phe Leu Tyr Gly Glu Phe Asp Phe Phe Ile His Tyr Tyr Ile
             20                  25                  30

Gln Thr Ile Ser Ala Leu Phe Lys Gly Asn Asn Pro Asp Thr Glu Thr
         35                  40                  45

Ser Leu Phe Tyr Ala Ser Asp Tyr Glu Lys Ser Gln Ile Ala Thr Leu
     50                  55                  60

Leu Glu Gln Asp Ser Leu Phe Gly Gly Ser Ser Leu Val Ile Leu Lys
 65                  70                  75                  80

Leu Asp Phe Ala Leu His Lys Lys Phe Lys Glu Asn Asp Ile Asn Pro
                 85                  90                  95

Phe Leu Lys Ala Leu Glu Arg Pro Ser His Asn Arg Leu Ile Ile Gly
            100                 105                 110

Leu Tyr Asn Ala Lys Ser Asp Thr Thr Lys Tyr Lys Tyr Thr Ser Glu
        115                 120                 125

Ile Ile Val Lys Phe Phe Gln Lys Ser Pro Leu Lys Asp Glu Ala Ile
    130                 135                 140

Cys Val Arg Phe Phe Thr Pro Lys Ala Trp Glu Ser Leu Lys Phe Leu
145                 150                 155                 160

Gln Glu Arg Ala Asn Phe Leu His Leu Asp Ile Ser Gly His Leu Leu
                165                 170                 175

Asn Ala Leu Phe Glu Ile Asn Asn Glu Asp Leu Ser Val Ser Phe Asn
            180                 185                 190

Asp Leu Asp Lys Leu Ala Val Leu Asn Ala Pro Ile Thr Leu Glu Asp
        195                 200                 205

Ile Gln Glu Leu Ser Ser Asn Ala Gly Asp Met Asp Leu Gln Lys Leu
    210                 215                 220

Ile Leu Gly Leu Phe Leu Lys Lys Ser Val Leu Asp Ile Tyr Asp Tyr
225                 230                 235                 240

Leu Leu Lys Glu Gly Lys Lys Asp Ala Asp Ile Leu Arg Gly Leu Glu
                245                 250                 255

Arg Tyr Phe Tyr Gln Leu Phe Leu Phe Phe Ala His Ile Lys Thr Thr
            260                 265                 270

Gly Leu Met Asp Ala Lys Glu Val Leu Gly Tyr Ala Pro Pro Lys Glu
        275                 280                 285

Ile Val Glu Asn Tyr Ala Lys Asn Ala Leu Arg Leu Lys Glu Ala Gly
    290                 295                 300

Tyr Lys Arg Val Phe Glu Ile Phe Arg Leu Trp His Leu Gln Ser Met
305                 310                 315                 320

Gln Gly Gln Lys Glu Leu Gly Phe Leu Tyr Leu Thr Pro Ile Gln Lys
                325                 330                 335

Ile Ile Asn Pro
            340

<210> 43
<211> 1005
<212> DNA
<213> Lactococcus lactis

<400> 43
atgacagtat ttgatgaatt taatgaaatc aaaagaaaag ggctccctca aattttagta 60

atttttggtg agtcaaggga actagttgga gaattgaaaa atcaactctt aacagaggtt 120

aatttcgagc caacggattt gagccaggct tattatgact taactcctag taatagcgat 180

ttagcccttg aagatttgga atctctccct tttttctcag attctcggtt ggtaatcctg 240

gaaaacttgg ttaatttaac aacggctaaa aaaagtgttt tagatgaaaa acagttgaag 300

cgctttgaaa attttttaga tgatccgccg gaaactacgc aacttgtttt aatcctttat 360

ggaaaattgg acagtcgtct taaagttgtt aaaaaattaa aagctaaagc aactttactt 420

gaagcacaag aattaaagag tcaagaatta attcaatatt ttagtcgaat aagtccttta 480

ccaaaggtag ttttgagttt aattgccgct aaatcaaatg ataatttttc agtaatgaag 540

caaaatattg acctcgttca aacttatgca ctagggcgag aagtgacact tgatgatgtg 600

gaaaaagtcg tcccaaaatc gctacaagat aatatttttg ccttaactga tttaattttt 660

aaaggaaaaa ttgatgaggc gcgtgattta gtccatgatt tgaccttgca aggggaagat 720

ttaattaaaa tattagctat tttgacaaat tcttatcgtt tgtatttaca agtcaaactt 780

ttccaagcca agggttggca agaaaatcaa caagttgcct ttttaaaaat gcacccttat 840

cctgtaaaat tggccaatca attggttaga aaattaaacg ttaaatcctt aaaaaatgga 900

ctttctgatt taattaaact tgattttgat ataaaaacaa gtgcagctga taaatcctat 960

ctttttgata tcactttaat caggttgacc ttgaaaaaaa actga 1005

<210> 44
<211> 1005
<212> DNA
<213> Lactococcus lactis

<220>
<221> CDS
<222> (1)..(1005)

<400> 44
atg aca gta ttt gat gaa ttt aat gaa atc aaa aga aaa ggg ctc cct    48
Met Thr Val Phe Asp Glu Phe Asn Glu Ile Lys Arg Lys Gly Leu Pro
1               5                   10                  15

caa att tta gta att ttt ggt gag tca agg gaa cta gtt gga gaa ttg    96
Gln Ile Leu Val Ile Phe Gly Glu Ser Arg Glu Leu Val Gly Glu Leu
            20                  25                  30

aaa aat caa ctc tta aca gag gtt aat ttc gag cca acg gat ttg agc    144
Lys Asn Gln Leu Leu Thr Glu Val Asn Phe Glu Pro Thr Asp Leu Ser
        35                  40                  45

cag gct tat tat gac tta act cct agt aat agc gat tta gcc ctt gaa    192
Gln Ala Tyr Tyr Asp Leu Thr Pro Ser Asn Ser Asp Leu Ala Leu Glu
    50                  55                  60

gat ttg gaa tct ctc cct ttt ttc tca gat tct cgg ttg gta atc ctg    240
Asp Leu Glu Ser Leu Pro Phe Phe Ser Asp Ser Arg Leu Val Ile Leu
65                  70                  75                  80

gaa aac ttg gtt aat tta aca acg gct aaa aaa agt gtt tta gat gaa    288
Glu Asn Leu Val Asn Leu Thr Thr Ala Lys Lys Ser Val Leu Asp Glu
                85                  90                  95

aaa cag ttg aag cgc ttt gaa aat ttt tta gat gat ccg ccg gaa act    336
Lys Gln Leu Lys Arg Phe Glu Asn Phe Leu Asp Asp Pro Pro Glu Thr
            100                 105                 110

acg caa ctt gtt tta atc ctt tat gga aaa ttg gac agt cgt ctt aaa    384
Thr Gln Leu Val Leu Ile Leu Tyr Gly Lys Leu Asp Ser Arg Leu Lys
        115                 120                 125

gtt gtt aaa aaa tta aaa gct aaa gca act tta ctt gaa gca caa gaa    432
Val Val Lys Lys Leu Lys Ala Lys Ala Thr Leu Leu Glu Ala Gln Glu
    130                 135                 140

tta aag agt caa gaa tta att caa tat ttt agt cga ata agt cct tta    480
Leu Lys Ser Gln Glu Leu Ile Gln Tyr Phe Ser Arg Ile Ser Pro Leu
145                 150                 155                 160

cca aag gta gtt ttg agt tta att gcc gct aaa tca aat gat aat ttt    528
Pro Lys Val Val Leu Ser Leu Ile Ala Ala Lys Ser Asn Asp Asn Phe
                165                 170                 175

tca gta atg aag caa aat att gac ctc gtt caa act tat gca cta ggg    576
Ser Val Met Lys Gln Asn Ile Asp Leu Val Gln Thr Tyr Ala Leu Gly
            180                 185                 190

cga gaa gtg aca ctt gat gat gtg gaa aaa gtc gtc cca aaa tcg cta    624
Arg Glu Val Thr Leu Asp Asp Val Glu Lys Val Val Pro Lys Ser Leu
        195                 200                 205

caa gat aat att ttt gcc tta act gat tta att ttt aaa gga aaa att    672
Gln Asp Asn Ile Phe Ala Leu Thr Asp Leu Ile Phe Lys Gly Lys Ile
    210                 215                 220

gat gag gcg cgt gat tta gtc cat gat ttg acc ttg caa ggg gaa gat    720
Asp Glu Ala Arg Asp Leu Val His Asp Leu Thr Leu Gln Gly Glu Asp
225                 230                 235                 240

tta att aaa ata tta gct att ttg aca aat tct tat cgt ttg tat tta    768
Leu Ile Lys Ile Leu Ala Ile Leu Thr Asn Ser Tyr Arg Leu Tyr Leu
                245                 250                 255

caa gtc aaa ctt ttc caa gcc aag ggt tgg caa gaa aat caa caa gtt    816
Gln Val Lys Leu Phe Gln Ala Lys Gly Trp Gln Glu Asn Gln Gln Val

                260                 265                 270

gcc ttt tta aaa atg cac cct tat cct gta aaa ttg gcc aat caa ttg     864
Ala Phe Leu Lys Met His Pro Tyr Pro Val Lys Leu Ala Asn Gln Leu
        275                 280                 285

gtt aga aaa tta aac gtt aaa tcc tta aaa aat gga ctt tct gat tta     912
Val Arg Lys Leu Asn Val Lys Ser Leu Lys Asn Gly Leu Ser Asp Leu
    290                 295                 300

att aaa ctt gat ttt gat ata aaa aca agt gca gct gat aaa tcc tat     960
Ile Lys Leu Asp Phe Asp Ile Lys Thr Ser Ala Ala Asp Lys Ser Tyr
305                 310                 315                 320

ctt ttt gat atc act tta atc agg ttg acc ttg aaa aaa aac tga         1005
Leu Phe Asp Ile Thr Leu Ile Arg Leu Thr Leu Lys Lys Asn
                325                 330                 335

<210> 45
<211> 334
<212> PRT
<213> Lactococcus lactis

<400> 45
Met Thr Val Phe Asp Glu Phe Asn Glu Ile Lys Arg Lys Gly Leu Pro
1               5                   10                  15

Gln Ile Leu Val Ile Phe Gly Glu Ser Arg Glu Leu Val Gly Glu Leu
            20                  25                  30

Lys Asn Gln Leu Leu Thr Glu Val Asn Phe Glu Pro Thr Asp Leu Ser
        35                  40                  45

Gln Ala Tyr Tyr Asp Leu Thr Pro Ser Asn Ser Asp Leu Ala Leu Glu
    50                  55                  60

Asp Leu Glu Ser Leu Pro Phe Phe Ser Asp Ser Arg Leu Val Ile Leu
65                  70                  75                  80

Glu Asn Leu Val Asn Leu Thr Thr Ala Lys Lys Ser Val Leu Asp Glu
                85                  90                  95

Lys Gln Leu Lys Arg Phe Glu Asn Phe Leu Asp Asp Pro Pro Glu Thr
            100                 105                 110

Thr Gln Leu Val Leu Ile Leu Tyr Gly Lys Leu Asp Ser Arg Leu Lys
        115                 120                 125

Val Val Lys Lys Leu Lys Ala Lys Ala Thr Leu Leu Glu Ala Gln Glu
    130                 135                 140

Leu Lys Ser Gln Glu Leu Ile Gln Tyr Phe Ser Arg Ile Ser Pro Leu
145                 150                 155                 160

Pro Lys Val Val Leu Ser Leu Ile Ala Ala Lys Ser Asn Asp Asn Phe
                165                 170                 175

Ser Val Met Lys Gln Asn Ile Asp Leu Val Gln Thr Tyr Ala Leu Gly
            180                 185                 190

Arg Glu Val Thr Leu Asp Asp Val Glu Lys Val Val Pro Lys Ser Leu
        195                 200                 205

Gln Asp Asn Ile Phe Ala Leu Thr Asp Leu Ile Phe Lys Gly Lys Ile
    210                 215                 220

Asp Glu Ala Arg Asp Leu Val His Asp Leu Thr Leu Gln Gly Glu Asp

225                 230                 235                 240

Leu Ile Lys Ile Leu Ala Ile Leu Thr Asn Ser Tyr Arg Leu Tyr Leu
                245                 250                 255

Gln Val Lys Leu Phe Gln Ala Lys Gly Trp Gln Glu Asn Gln Gln Val
            260                 265                 270

Ala Phe Leu Lys Met His Pro Tyr Pro Val Lys Leu Ala Asn Gln Leu
        275                 280                 285

Val Arg Lys Leu Asn Val Lys Ser Leu Lys Asn Gly Leu Ser Asp Leu
    290                 295                 300

Ile Lys Leu Asp Phe Asp Ile Lys Thr Ser Ala Ala Asp Lys Ser Tyr
305                 310                 315                 320

Leu Phe Asp Ile Thr Leu Ile Arg Leu Thr Leu Lys Lys Asn
                325                 330

<210> 46
<211> 1170
<212> DNA
<213> Mycobacterium leprae

<400> 46
gtgcctgcgg caatcgttcc cggtccggct agtgccctcg gcgtgctgct gctggtgttg 60

gtcctgtggc ggtgctttcg ggtggcgatg atttcagcac tgatggtcgc cgttgcgtgc 120

ttgcctgatt ggttgtcagg gttcctgacc ggcgggctta tcgcgggctc gtcggctcgt 180

cgtgccacca tctacggagt gagtaataag ttttcgtcgc tgcacttggt cctaggaaat 240

gaggaactgc tggttgagcg ggccgtgggg gaggtgttgc ggtcggcgcg gcagcgggca 300

ggcacccagg acgtccccgt tagccgaatg cgagccggtg acgtcggcac ctatgagctc 360

accgaactgc tgagcccgtc attgttcgcc gacgagcgga ttgtcgtgct agaggccgcc 420

gctgaagcgg gtaaggaggc cgctgcgctg atcgtgtcgg ctgccgctga cataccgcaa 480

ggcacggtgc tggtggtggt gcattcaggt ggtggtcgag ccaaagcgct ggctaacgag 540

ctgcagtcgt tgggtgctac ggtgcatccc tgcgcgcgga tcactaagct tagcgagcgt 600

actgattttg ttcgaaaaga attgcgttcg ttgcgggtca aggtcgacga gggggcagtg 660

accgccctgc tgaacgctgt tggttctgac gtgcgcgaac tcgcttcggc ctgttcgcaa 720

ttggtagccg acaccgccgg tgatgtcgac gccgacgcgg ttcagcgtta tcacagcggc 780

aaagcggaag tgaagggctt tgacattgcc gacaaggcgg tcggtggcga tgtctcagga 840

gcagtggagg cgttgcggtg ggcgatgatg cgcggcgagc cactggtggt gttggctgat 900

gcgctggccg aagcagtgca cacgatcggc cgggttgggc cgctgtccgg tgattcctac 960

cgcctggcgt cgcggttggg aatgccgccc tggcgggtgc agaaggcaca acaacaggct 1020

cggcgttggt cgcgtgacac agtggcagcc gcgatgagag tggtggctgc gctcaacgcc 1080

gacgtgaaag gggccgccgc ggacgcctac tacgctctgg aatccgcggt caggaaagtt 1140

gctgagctgg ccgccgatgg gagccgatga                                  1170

<210> 47
<211> 1170
<212> DNA
<213> Mycobacterium leprae

<220>
<221> CDS
<222> (1)..(1170)

<400> 47
gtg cct gcg gca atc gtt ccc ggt ccg gct agt gcc ctc ggc gtg ctg     48
Val Pro Ala Ala Ile Val Pro Gly Pro Ala Ser Ala Leu Gly Val Leu
1               5                   10                  15

ctg ctg gtg ttg gtc ctg tgg cgg tgc ttt cgg gtg gcg atg att tca     96
Leu Leu Val Leu Val Leu Trp Arg Cys Phe Arg Val Ala Met Ile Ser
            20                  25                  30

gca ctg atg gtc gcc gtt gcg tgc ttg cct gat tgg ttg tca ggg ttc     144
Ala Leu Met Val Ala Val Ala Cys Leu Pro Asp Trp Leu Ser Gly Phe
        35                  40                  45

ctg acc ggc ggg ctt atc gcg ggc tcg tcg gct cgt cgt gcc acc atc     192
Leu Thr Gly Gly Leu Ile Ala Gly Ser Ser Ala Arg Arg Ala Thr Ile
    50                  55                  60

tac gga gtg agt aat aag ttt tcg tcg ctg cac ttg gtc cta gga aat     240
Tyr Gly Val Ser Asn Lys Phe Ser Ser Leu His Leu Val Leu Gly Asn
65                  70                  75                  80

gag gaa ctg ctg gtt gag cgg gcc gtg ggg gag gtg ttg cgg tcg gcg     288
Glu Glu Leu Leu Val Glu Arg Ala Val Gly Glu Val Leu Arg Ser Ala
                85                  90                  95

cgg cag cgg gca ggc acc cag gac gtc ccc gtt agc cga atg cga gcc     336
Arg Gln Arg Ala Gly Thr Gln Asp Val Pro Val Ser Arg Met Arg Ala
            100                 105                 110

ggt gac gtc ggc acc tat gag ctc acc gaa ctg ctg agc ccg tca ttg     384
Gly Asp Val Gly Thr Tyr Glu Leu Thr Glu Leu Leu Ser Pro Ser Leu
        115                 120                 125

ttc gcc gac gag cgg att gtc gtg cta gag gcc gcc gct gaa gcg ggt     432
Phe Ala Asp Glu Arg Ile Val Val Leu Glu Ala Ala Ala Glu Ala Gly
    130                 135                 140

aag gag gcc gct gcg ctg atc gtg tcg gct gcc gct gac ata ccg caa     480
Lys Glu Ala Ala Ala Leu Ile Val Ser Ala Ala Ala Asp Ile Pro Gln
145                 150                 155                 160

ggc acg gtg ctg gtg gtg gtg cat tca ggt ggt ggt cga gcc aaa gcg     528
Gly Thr Val Leu Val Val Val His Ser Gly Gly Gly Arg Ala Lys Ala
                165                 170                 175

ctg gct aac gag ctg cag tcg ttg ggt gct acg gtg cat ccc tgc gcg     576
Leu Ala Asn Glu Leu Gln Ser Leu Gly Ala Thr Val His Pro Cys Ala
            180                 185                 190

cgg atc act aag ctt agc gag cgt act gat ttt gtt cga aaa gaa ttg     624
Arg Ile Thr Lys Leu Ser Glu Arg Thr Asp Phe Val Arg Lys Glu Leu
        195                 200                 205

cgt tcg ttg cgg gtc aag gtc gac gag ggg gca gtg acc gcc ctg ctg     672
Arg Ser Leu Arg Val Lys Val Asp Glu Gly Ala Val Thr Ala Leu Leu
    210                 215                 220

aac gct gtt ggt tct gac gtg cgc gaa ctc gct tcg gcc tgt tcg caa     720
Asn Ala Val Gly Ser Asp Val Arg Glu Leu Ala Ser Ala Cys Ser Gln

225                 230                 235                 240

ttg gta gcc gac acc gcc ggt gat gtc gac gcc gac gcg gtt cag cgt     768
Leu Val Ala Asp Thr Ala Gly Asp Val Asp Ala Asp Ala Val Gln Arg
                245                 250                 255

tat cac agc ggc aaa gcg gaa gtg aag ggc ttt gac att gcc gac aag     816
Tyr His Ser Gly Lys Ala Glu Val Lys Gly Phe Asp Ile Ala Asp Lys
            260                 265                 270

gcg gtc ggt ggc gat gtc tca gga gca gtg gag gcg ttg cgg tgg gcg     864
Ala Val Gly Gly Asp Val Ser Gly Ala Val Glu Ala Leu Arg Trp Ala
        275                 280                 285

atg atg cgc ggc gag cca ctg gtg gtg ttg gct gat gcg ctg gcc gaa     912
Met Met Arg Gly Glu Pro Leu Val Val Leu Ala Asp Ala Leu Ala Glu
    290                 295                 300

gca gtg cac acg atc ggc cgg gtt ggg ccg ctg tcc ggt gat tcc tac     960
Ala Val His Thr Ile Gly Arg Val Gly Pro Leu Ser Gly Asp Ser Tyr
305                 310                 315                 320

cgc ctg gcg tcg cgg ttg gga atg ccg ccc tgg cgg gtg cag aag gca     1008
Arg Leu Ala Ser Arg Leu Gly Met Pro Pro Trp Arg Val Gln Lys Ala
                325                 330                 335

caa caa cag gct cgg cgt tgg tcg cgt gac aca gtg gca gcc gcg atg     1056
Gln Gln Gln Ala Arg Arg Trp Ser Arg Asp Thr Val Ala Ala Ala Met
            340                 345                 350

aga gtg gtg gct gcg ctc aac gcc gac gtg aaa ggg gcc gcc gcg gac     1104
Arg Val Val Ala Ala Leu Asn Ala Asp Val Lys Gly Ala Ala Ala Asp
        355                 360                 365

gcc tac tac gct ctg gaa tcc gcg gtc agg aaa gtt gct gag ctg gcc     1152
Ala Tyr Tyr Ala Leu Glu Ser Ala Val Arg Lys Val Ala Glu Leu Ala
    370                 375                 380

gcc gat ggg agc cga tga                                             1170
Ala Asp Gly Ser Arg
385                 390

<210> 48
<211> 389
<212> PRT
<213> Mycobacterium leprae

<400> 48
Val Pro Ala Ala Ile Val Pro Gly Pro Ala Ser Ala Leu Gly Val Leu
1               5                   10                  15

Leu Leu Val Leu Val Leu Trp Arg Cys Phe Arg Val Ala Met Ile Ser
            20                  25                  30

Ala Leu Met Val Ala Val Ala Cys Leu Pro Asp Trp Leu Ser Gly Phe
        35                  40                  45

Leu Thr Gly Gly Leu Ile Ala Gly Ser Ser Ala Arg Arg Ala Thr Ile
    50                  55                  60

Tyr Gly Val Ser Asn Lys Phe Ser Ser Leu His Leu Val Leu Gly Asn
65                  70                  75                  80

Glu Glu Leu Leu Val Glu Arg Ala Val Gly Glu Val Leu Arg Ser Ala
                85                  90                  95

Arg Gln Arg Ala Gly Thr Gln Asp Val Pro Val Ser Arg Met Arg Ala

            100                 105                 110

Gly Asp Val Gly Thr Tyr Glu Leu Thr Glu Leu Leu Ser Pro Ser Leu
        115                 120                 125

Phe Ala Asp Glu Arg Ile Val Val Leu Glu Ala Ala Ala Glu Ala Gly
    130                 135                 140

Lys Glu Ala Ala Ala Leu Ile Val Ser Ala Ala Ala Asp Ile Pro Gln
145                 150                 155                 160

Gly Thr Val Leu Val Val Val His Ser Gly Gly Gly Arg Ala Lys Ala
                165                 170                 175

Leu Ala Asn Glu Leu Gln Ser Leu Gly Ala Thr Val His Pro Cys Ala
            180                 185                 190

Arg Ile Thr Lys Leu Ser Glu Arg Thr Asp Phe Val Arg Lys Glu Leu
        195                 200                 205

Arg Ser Leu Arg Val Lys Val Asp Glu Gly Ala Val Thr Ala Leu Leu
    210                 215                 220

Asn Ala Val Gly Ser Asp Val Arg Glu Leu Ala Ser Ala Cys Ser Gln
225                 230                 235                 240

Leu Val Ala Asp Thr Ala Gly Asp Val Asp Ala Asp Ala Val Gln Arg
                245                 250                 255

Tyr His Ser Gly Lys Ala Glu Val Lys Gly Phe Asp Ile Ala Asp Lys
            260                 265                 270

Ala Val Gly Gly Asp Val Ser Gly Ala Val Glu Ala Leu Arg Trp Ala
        275                 280                 285

Met Met Arg Gly Glu Pro Leu Val Val Leu Ala Asp Ala Leu Ala Glu
    290                 295                 300

Ala Val His Thr Ile Gly Arg Val Gly Pro Leu Ser Gly Asp Ser Tyr
305                 310                 315                 320

Arg Leu Ala Ser Arg Leu Gly Met Pro Pro Trp Arg Val Gln Lys Ala
                325                 330                 335

Gln Gln Gln Ala Arg Arg Trp Ser Arg Asp Thr Val Ala Ala Ala Met
            340                 345                 350

Arg Val Val Ala Ala Leu Asn Ala Asp Val Lys Gly Ala Ala Ala Asp
        355                 360                 365

Ala Tyr Tyr Ala Leu Glu Ser Ala Val Arg Lys Val Ala Glu Leu Ala
    370                 375                 380

Ala Asp Gly Ser Arg
385

<210> 49
<211> 969
<212> DNA
<213> Mycobacterium tuberculosis

<400> 49
gtgagcgagg ctaagccgtt gcacctggtc ctgggagacg aagaactgct ggtcgaaagg 60

gcggtggccg acgtgttgcg ctcggctcgg cagcgggcag gtacagccga cgtcccggtg 120

agccgaatgc gcgcgggtga cgtcggtgcc tatgagctcg ccgaactgct gagcccgtca 180

ctgttcgccg aggagcggat cgttgtgctg ggggccgctg cggaggcggg caaggacgct 240

gccgcggtaa tcgagtcggc cgccgccgat cttccggccg gcaccgtgct ggtagtggtc 300

cactcgggtg gcgggcgcgc caaatcgctg gccaaccagc tgcggtcgat gggtgcgcag 360

gttcatccgt gcgcgcggat caccaaggtc agtgagcgcg ccgacttcat ccgtagcgag 420

ttcgcgtcgc tgcgggtcaa ggtcgacgac gagaccgtga ccgccctgct ggacgccgtc 480

ggctccgacg tgcgcgaact cgcctcggcc tgttcacagc tggtcgccga taccggagga 540

gccgtcgacg ccgccgctgt acggcgctat cacagcggca aagccgaggt gaggggcttc 600

gacatcgccg acaaggcggt agccggcgac gtggcgggag ctgccgaagc gttgcggtgg 660

gcgatgatgc gcggtgagcc gctagtggtg ttggccgatg cgctcgccga agccgtgcac 720

accatcggcc gggtcggccc gcagtccggc gacccgtacc gcctggccgc acaactgggg 780

atgccgccct ggcgggtgca gaaagcccag aagcaggctc ggcggtggtc gcgtgacacg 840

gtggcgaccg cgatgaggtt ggtggccgaa ctcaatgcta acgtcaaggg cgccgtcgcg 900

gatgcggact acgcgctgga atccgcggtc cggcaggtcg ccgagttggt ggccgaccgc 960

ggccgatga 969

<210> 50
<211> 969
<212> DNA
<213> Mycobacterium tuberculosis

<220>
<221> CDS
<222> (1)..(969)

<400> 50
gtg agc gag gct aag ccg ttg cac ctg gtc ctg gga gac gaa gaa ctg 48
Val Ser Glu Ala Lys Pro Leu His Leu Val Leu Gly Asp Glu Glu Leu
1               5                   10                  15

ctg gtc gaa agg gcg gtg gcc gac gtg ttg cgc tcg gct cgg cag cgg 96
Leu Val Glu Arg Ala Val Ala Asp Val Leu Arg Ser Ala Arg Gln Arg
            20                  25                  30

gca ggt aca gcc gac gtc ccg gtg agc cga atg cgc gcg ggt gac gtc 144
Ala Gly Thr Ala Asp Val Pro Val Ser Arg Met Arg Ala Gly Asp Val
        35                  40                  45

ggt gcc tat gag ctc gcc gaa ctg ctg agc ccg tca ctg ttc gcc gag 192
Gly Ala Tyr Glu Leu Ala Glu Leu Leu Ser Pro Ser Leu Phe Ala Glu
    50                  55                  60

gag cgg atc gtt gtg ctg ggg gcc gct gcg gag gcg ggc aag gac gct 240
Glu Arg Ile Val Val Leu Gly Ala Ala Ala Glu Ala Gly Lys Asp Ala
65                  70                  75                  80

gcc gcg gta atc gag tcg gcc gcc gcc gat ctt ccg gcc ggc acc gtg 288
Ala Ala Val Ile Glu Ser Ala Ala Ala Asp Leu Pro Ala Gly Thr Val
                85                  90                  95

ctg gta gtg gtc cac tcg ggt ggc ggg cgc gcc aaa tcg ctg gcc aac 336
Leu Val Val Val His Ser Gly Gly Gly Arg Ala Lys Ser Leu Ala Asn
            100                 105                 110

cag ctg cgg tcg atg ggt gcg cag gtt cat ccg tgc gcg cgg atc acc 384
Gln Leu Arg Ser Met Gly Ala Gln Val His Pro Cys Ala Arg Ile Thr
        115                 120                 125

aag gtc agt gag cgc gcc gac ttc atc cgt agc gag ttc gcg tcg ctg 432
Lys Val Ser Glu Arg Ala Asp Phe Ile Arg Ser Glu Phe Ala Ser Leu
    130                 135                 140

cgg gtc aag gtc gac gac gag acc gtg acc gcc ctg ctg gac gcc gtc 480
Arg Val Lys Val Asp Asp Glu Thr Val Thr Ala Leu Leu Asp Ala Val
145                 150                 155                 160

ggc tcc gac gtg cgc gaa ctc gcc tcg gcc tgt tca cag ctg gtc gcc 528
Gly Ser Asp Val Arg Glu Leu Ala Ser Ala Cys Ser Gln Leu Val Ala
                165                 170                 175

gat acc gga gga gcc gtc gac gcc gcc gct gta cgg cgc tat cac agc 576
Asp Thr Gly Gly Ala Val Asp Ala Ala Ala Val Arg Arg Tyr His Ser
            180                 185                 190

ggc aaa gcc gag gtg agg ggc ttc gac atc gcc gac aag gcg gta gcc 624
Gly Lys Ala Glu Val Arg Gly Phe Asp Ile Ala Asp Lys Ala Val Ala
        195                 200                 205

ggc gac gtg gcg gga gct gcc gaa gcg ttg cgg tgg gcg atg atg cgc 672
Gly Asp Val Ala Gly Ala Ala Glu Ala Leu Arg Trp Ala Met Met Arg
    210                 215                 220

ggt gag ccg cta gtg gtg ttg gcc gat gcg ctc gcc gaa gcc gtg cac 720
Gly Glu Pro Leu Val Val Leu Ala Asp Ala Leu Ala Glu Ala Val His
225                 230                 235                 240

acc atc ggc cgg gtc ggg ccg cag tcc ggc gac ccg tac cgc ctg gcc 768
Thr Ile Gly Arg Val Gly Pro Gln Ser Gly Asp Pro Tyr Arg Leu Ala
                245                 250                 255

gca caa ctg ggg atg ccg ccc tgg cgg gtg cag aaa gcc cag aag cag 816
Ala Gln Leu Gly Met Pro Pro Trp Arg Val Gln Lys Ala Gln Lys Gln
            260                 265                 270

gct cgg cgg tgg tcg cgt gac acg gtg gcg acc gcg atg agg ttg gtg 864
Ala Arg Arg Trp Ser Arg Asp Thr Val Ala Thr Ala Met Arg Leu Val
        275                 280                 285

gcc gaa ctc aat gct aac gtc aag ggc gcc gtc gcg gat gcg gac tac 912
Ala Glu Leu Asn Ala Asn Val Lys Gly Ala Val Ala Asp Ala Asp Tyr
    290                 295                 300

gcg ctg gaa tcc gcg gtc cgg cag gtc gcc gag ttg gtg gcc gac cgc 960
Ala Leu Glu Ser Ala Val Arg Gln Val Ala Glu Leu Val Ala Asp Arg
305                 310                 315                 320

ggc cga tga 969
Gly Arg

<210> 51
<211> 322
<212> PRT
<213> Mycobacterium tuberculosis

<400> 51
Val Ser Glu Ala Lys Pro Leu His Leu Val Leu Gly Asp Glu Glu Leu
1               5                   10                  15

Leu Val Glu Arg Ala Val Ala Asp Val Leu Arg Ser Ala Arg Gln Arg
            20                  25                  30

Ala Gly Thr Ala Asp Val Pro Val Ser Arg Met Arg Ala Gly Asp Val
        35                  40                  45

Gly Ala Tyr Glu Leu Ala Glu Leu Leu Ser Pro Ser Leu Phe Ala Glu
    50                  55                  60

Glu Arg Ile Val Val Leu Gly Ala Ala Ala Glu Ala Gly Lys Asp Ala
65                  70                  75                  80

Ala Ala Val Ile Glu Ser Ala Ala Ala Asp Leu Pro Ala Gly Thr Val
                85                  90                  95

Leu Val Val Val His Ser Gly Gly Gly Arg Ala Lys Ser Leu Ala Asn
            100                 105                 110

Gln Leu Arg Ser Met Gly Ala Gln Val His Pro Cys Ala Arg Ile Thr
        115                 120                 125

Lys Val Ser Glu Arg Ala Asp Phe Ile Arg Ser Glu Phe Ala Ser Leu
    130                 135                 140

Arg Val Lys Val Asp Asp Glu Thr Val Thr Ala Leu Leu Asp Ala Val
145                 150                 155                 160

Gly Ser Asp Val Arg Glu Leu Ala Ser Ala Cys Ser Gln Leu Val Ala
                165                 170                 175

Asp Thr Gly Gly Ala Val Asp Ala Ala Ala Val Arg Arg Tyr His Ser
            180                 185                 190

Gly Lys Ala Glu Val Arg Gly Phe Asp Ile Ala Asp Lys Ala Val Ala
        195                 200                 205

Gly Asp Val Ala Gly Ala Ala Glu Ala Leu Arg Trp Ala Met Met Arg
    210                 215                 220

Gly Glu Pro Leu Val Val Leu Ala Asp Ala Leu Ala Glu Ala Val His
225                 230                 235                 240

Thr Ile Gly Arg Val Gly Pro Gln Ser Gly Asp Pro Tyr Arg Leu Ala
                245                 250                 255

Ala Gln Leu Gly Met Pro Pro Trp Arg Val Gln Lys Ala Gln Lys Gln
            260                 265                 270

Ala Arg Arg Trp Ser Arg Asp Thr Val Ala Thr Ala Met Arg Leu Val
        275                 280                 285

Ala Glu Leu Asn Ala Asn Val Lys Gly Ala Val Ala Asp Ala Asp Tyr
    290                 295                 300

Ala Leu Glu Ser Ala Val Arg Gln Val Ala Glu Leu Val Ala Asp Arg
305                 310                 315                 320

Gly Arg

<210> 52
<211> 1044
<212> DNA
<213> Mesorhizobium loti

<400> 52
atggcacaga agaaagcttt cgaggtcgat tcctggctcg cgcggcctga cgcgcgcatc 60

tccatcgtcc tgctctacgg ccccgatcgc ggcctcgtct ccgagcgtgc aaaggccttt 120

gccgaaaaga ccggcctgcc gctcgacgat ccgttttccg tggtccggct cgaggggtcg 180

gaggtcgacc gcgacgaagg ccggctgctc gacgaagccc gcaccgtgcc gatgttttcc 240

gaccgccgcc tgctctgggt gcgcaatgcc accggccaga aggcgctggc cgaggacgtc 300

aaggcgctga cagcggagcc cgcgcgcgat gccatcatcc tcatcgaggc cggcgacctc 360

aagaagggcg tcggcttgcg cgccgtggtc gagggcgccg acaatgccat ggcgctgcct 420

tgctacgccg acgaggcccg cgacatcgac acggtcatcg acgatgaatt gcgcaaggcc 480

ggcatgtcga tgacgctgga ggcgaggcag gcgctgcgcc gcaatctcgg cggcgacagg 540

ctcgcttcgc gcggcgagat cgaaaaactc atcctctacg cccacggcca gaaggagatc 600

ggcctggagg atgtcagggc gatgtcgggc gacgtgtccg gcacctcgtt cgacgacgcc 660

gtcgacgcgc tgctggaagg caaggtcggc gacttcgaca ccgccttcac ccgccactgc 720

cagtccggcg gcccaccctt cctggtgctg tcctcggcga tgcgccagct gcagcagatc 780

caaatcatgc gcggccagat ggactctggc gggcgcaatg ccgcgtcggt ggtcgccgcc 840

gcgcgcccgc cggttttctt ttcgcggcgc aagctggtgg agaaggcgct cgagcgctgg 900

agcagcgatg cgctgagccg cgcgctaacc cggctgcaga ccgcggtgct gcagacccgc 960

aaacggcccg atctgtcaga ggcgctggcg cgccaggcgc tgctcggcat tgcggtggag 1020

agcgcgaggc tggggcagcg ttag 1044

<210> 53
<211> 1044
<212> DNA
<213> Mesorhizobium loti

<220>
<221> CDS
<222> (1)..(1044)

<400> 53
atg gca cag aag aaa gct ttc gag gtc gat tcc tgg ctc gcg cgg cct 48
Met Ala Gln Lys Lys Ala Phe Glu Val Asp Ser Trp Leu Ala Arg Pro
1               5                   10                  15

gac gcg cgc atc tcc atc gtc ctg ctc tac ggc ccc gat cgc ggc ctc 96
Asp Ala Arg Ile Ser Ile Val Leu Leu Tyr Gly Pro Asp Arg Gly Leu
            20                  25                  30

gtc tcc gag cgt gca aag gcc ttt gcc gaa aag acc ggc ctg ccg ctc 144
Val Ser Glu Arg Ala Lys Ala Phe Ala Glu Lys Thr Gly Leu Pro Leu
        35                  40                  45

gac gat ccg ttt tcc gtg gtc cgg ctc gag ggg tcg gag gtc gac cgc 192
Asp Asp Pro Phe Ser Val Val Arg Leu Glu Gly Ser Glu Val Asp Arg
    50                  55                  60

gac gaa ggc cgg ctg ctc gac gaa gcc cgc acc gtg ccg atg ttt tcc 240
Asp Glu Gly Arg Leu Leu Asp Glu Ala Arg Thr Val Pro Met Phe Ser
65                  70                  75                  80

gac cgc cgc ctg ctc tgg gtg cgc aat gcc acc ggc cag aag gcg ctg 288
Asp Arg Arg Leu Leu Trp Val Arg Asn Ala Thr Gly Gln Lys Ala Leu
                85                  90                  95

gcc gag gac gtc aag gcg ctg aca gcg gag ccc gcg cgc gat gcc atc 336
Ala Glu Asp Val Lys Ala Leu Thr Ala Glu Pro Ala Arg Asp Ala Ile

                100                 105                 110

atc ctc atc gag gcc ggc gac ctc aag aag ggc gtc ggc ttg cgc gcc      384
Ile Leu Ile Glu Ala Gly Asp Leu Lys Lys Gly Val Gly Leu Arg Ala
        115                 120                 125

gtg gtc gag ggc gcc gac aat gcc atg gcg ctg cct tgc tac gcc gac      432
Val Val Glu Gly Ala Asp Asn Ala Met Ala Leu Pro Cys Tyr Ala Asp
    130                 135                 140

gag gcc cgc gac atc gac acg gtc atc gac gat gaa ttg cgc aag gcc      480
Glu Ala Arg Asp Ile Asp Thr Val Ile Asp Asp Glu Leu Arg Lys Ala
145                 150                 155                 160

ggc atg tcg atg acg ctg gag gcg agg cag gcg ctg cgc cgc aat ctc      528
Gly Met Ser Met Thr Leu Glu Ala Arg Gln Ala Leu Arg Arg Asn Leu
                165                 170                 175

ggc ggc gac agg ctc gct tcg cgc ggc gag atc gaa aaa ctc atc ctc      576
Gly Gly Asp Arg Leu Ala Ser Arg Gly Glu Ile Glu Lys Leu Ile Leu
            180                 185                 190

tac gcc cac ggc cag aag gag atc ggc ctg gag gat gtc agg gcg atg      624
Tyr Ala His Gly Gln Lys Glu Ile Gly Leu Glu Asp Val Arg Ala Met
        195                 200                 205

tcg ggc gac gtg tcc ggc acc tcg ttc gac gac gcc gtc gac gcg ctg      672
Ser Gly Asp Val Ser Gly Thr Ser Phe Asp Asp Ala Val Asp Ala Leu
    210                 215                 220

ctg gaa ggc aag gtc ggc gac ttc gac acc gcc ttc acc cgc cac tgc      720
Leu Glu Gly Lys Val Gly Asp Phe Asp Thr Ala Phe Thr Arg His Cys
225                 230                 235                 240

cag tcc ggc ggc cca ccc ttc ctg gtg ctg tcc tcg gcg atg cgc cag      768
Gln Ser Gly Gly Pro Pro Phe Leu Val Leu Ser Ser Ala Met Arg Gln
                245                 250                 255

ctg cag cag atc caa atc atg cgc ggc cag atg gac tct ggc ggg cgc      816
Leu Gln Gln Ile Gln Ile Met Arg Gly Gln Met Asp Ser Gly Gly Arg
            260                 265                 270

aat gcc gcg tcg gtg gtc gcc gcc gcg cgc ccg ccg gtt ttc ttt tcg      864
Asn Ala Ala Ser Val Val Ala Ala Ala Arg Pro Pro Val Phe Phe Ser
        275                 280                 285

cgg cgc aag ctg gtg gag aag gcg ctc gag cgc tgg agc agc gat gcg      912
Arg Arg Lys Leu Val Glu Lys Ala Leu Glu Arg Trp Ser Ser Asp Ala
    290                 295                 300

ctg agc cgc gcg cta acc cgg ctg cag acc gcg gtg ctg cag acc cgc      960
Leu Ser Arg Ala Leu Thr Arg Leu Gln Thr Ala Val Leu Gln Thr Arg
305                 310                 315                 320

aaa cgg ccc gat ctg tca gag gcg ctg gcg cgc cag gcg ctg ctc ggc      1008
Lys Arg Pro Asp Leu Ser Glu Ala Leu Ala Arg Gln Ala Leu Leu Gly
                325                 330                 335

att gcg gtg gag agc gcg agg ctg ggg cag cgt tag                      1044
Ile Ala Val Glu Ser Ala Arg Leu Gly Gln Arg
            340                 345

<210> 54
<211> 347
<212> PRT
<213> Mesorhizobium loti

<400> 54
Met Ala Gln Lys Lys Ala Phe Glu Val Asp Ser Trp Leu Ala Arg Pro
1               5                   10                  15

Asp Ala Arg Ile Ser Ile Val Leu Leu Tyr Gly Pro Asp Arg Gly Leu
            20                  25                  30

Val Ser Glu Arg Ala Lys Ala Phe Ala Glu Lys Thr Gly Leu Pro Leu
        35                  40                  45

Asp Asp Pro Phe Ser Val Val Arg Leu Glu Gly Ser Glu Val Asp Arg
    50                  55                  60

Asp Glu Gly Arg Leu Leu Asp Glu Ala Arg Thr Val Pro Met Phe Ser
65                  70                  75                  80

Asp Arg Arg Leu Leu Trp Val Arg Asn Ala Thr Gly Gln Lys Ala Leu
                85                  90                  95

Ala Glu Asp Val Lys Ala Leu Thr Ala Glu Pro Ala Arg Asp Ala Ile
            100                 105                 110

Ile Leu Ile Glu Ala Gly Asp Leu Lys Lys Gly Val Gly Leu Arg Ala
        115                 120                 125

Val Val Glu Gly Ala Asp Asn Ala Met Ala Leu Pro Cys Tyr Ala Asp
    130                 135                 140

Glu Ala Arg Asp Ile Asp Thr Val Ile Asp Asp Glu Leu Arg Lys Ala
145                 150                 155                 160

Gly Met Ser Met Thr Leu Glu Ala Arg Gln Ala Leu Arg Arg Asn Leu
                165                 170                 175

Gly Gly Asp Arg Leu Ala Ser Arg Gly Glu Ile Glu Lys Leu Ile Leu
            180                 185                 190

Tyr Ala His Gly Gln Lys Glu Ile Gly Leu Glu Asp Val Arg Ala Met
        195                 200                 205

Ser Gly Asp Val Ser Gly Thr Ser Phe Asp Asp Ala Val Asp Ala Leu
    210                 215                 220

Leu Glu Gly Lys Val Gly Asp Phe Asp Thr Ala Phe Thr Arg His Cys
225                 230                 235                 240

Gln Ser Gly Gly Pro Pro Phe Leu Val Leu Ser Ser Ala Met Arg Gln
                245                 250                 255

Leu Gln Gln Ile Gln Ile Met Arg Gly Gln Met Asp Ser Gly Gly Arg
            260                 265                 270

Asn Ala Ala Ser Val Val Ala Ala Ala Arg Pro Pro Val Phe Phe Ser
        275                 280                 285

Arg Arg Lys Leu Val Glu Lys Ala Leu Glu Arg Trp Ser Ser Asp Ala
    290                 295                 300

Leu Ser Arg Ala Leu Thr Arg Leu Gln Thr Ala Val Leu Gln Thr Arg
305                 310                 315                 320

Lys Arg Pro Asp Leu Ser Glu Ala Leu Ala Arg Gln Ala Leu Leu Gly
                325                 330                 335

Ile Ala Val Glu Ser Ala Arg Leu Gly Gln Arg
            340                 345

<210> 55
<211> 894
<212> DNA
<213> Mycoplasma genitalium

<400> 55
atgacaataa tttatggtca agatattggg ctaattcacc aaaaattaag tcaaatcaaa 60

agcaattctc catataaaac tatttggttt aaagatctta agcaacttta tgatctcttt 120

tcacaaccat tatttggttc taataatgaa aagtttattg tcaataattg cagttttttg 180

gaaaaaacaa gcttaactag acaagaaaac ttgtgtttag aaaaactaaa aacaactgat 240

gtagttttaa ctgtttatac tgacaatcct tttagtggga ttaaaaccat taaatcaatc 300

acaactgttt tttgtgataa attggattgg aaatcaatgc ataaagcaat tggtgaagtt 360

tgtagagaac ttaacttaaa acttgatttg gaaataattg attggttagc gaatgcttta 420

cctttaaata tgggggtaat ttaccaagaa attaacaaac ttagcttgtt aggtaaaaat 480

gaaatcaaag ataacaaatt agttgaaaca gttatttgtg attatcaacc tgttcaaatt 540

tacaaattaa ccaaagctct aacaaattca caaattgtaa aggcatttaa atatattgat 600

gaattggcaa gtacaaaacc aaattttgcc acccaatttt tagaattttt tagtggggag 660

ttgttattag cattgatggt taaaagttgt aatccaaagc aactaactaa cattaatcta 720

aatgttaatc agtttcgctt aattgctatt caaagtcagt accataactt tactagcaaa 780

gttttagtaa atataattaa tgcaattcaa aaattggata ttaaattaaa gcacaatgat 840

ggctttgcca ttccgttgtt aaaaaacttt gctttaagct tttttactaa ctaa 894

<210> 56
<211> 894
<212> DNA
<213> Mycoplasma genitalium

<220>
<221> CDS
<222> (1)..(894)

<400> 56
atg aca ata att tat ggt caa gat att ggg cta att cac caa aaa tta 48
Met Thr Ile Ile Tyr Gly Gln Asp Ile Gly Leu Ile His Gln Lys Leu
1               5                   10                  15

agt caa atc aaa agc aat tct cca tat aaa act att tgg ttt aaa gat 96
Ser Gln Ile Lys Ser Asn Ser Pro Tyr Lys Thr Ile Trp Phe Lys Asp
            20                  25                  30

ctt aag caa ctt tat gat ctc ttt tca caa cca tta ttt ggt tct aat 144
Leu Lys Gln Leu Tyr Asp Leu Phe Ser Gln Pro Leu Phe Gly Ser Asn
        35                  40                  45

aat gaa aag ttt att gtc aat aat tgc agt ttt ttg gaa aaa aca agc 192
Asn Glu Lys Phe Ile Val Asn Asn Cys Ser Phe Leu Glu Lys Thr Ser
    50                  55                  60

tta act aga caa gaa aac ttg tgt tta gaa aaa cta aaa aca act gat 240
Leu Thr Arg Gln Glu Asn Leu Cys Leu Glu Lys Leu Lys Thr Thr Asp
65                  70                  75                  80

gta gtt tta act gtt tat act gac aat cct ttt agt ggg att aaa acc 288

Val Val Leu Thr Val Tyr Thr Asp Asn Pro Phe Ser Gly Ile Lys Thr
                85                  90                  95

att aaa tca atc aca act gtt ttt tgt gat aaa ttg gat tgg aaa tca     336
Ile Lys Ser Ile Thr Thr Val Phe Cys Asp Lys Leu Asp Trp Lys Ser
            100                 105                 110

atg cat aaa gca att ggt gaa gtt tgt aga gaa ctt aac tta aaa ctt     384
Met His Lys Ala Ile Gly Glu Val Cys Arg Glu Leu Asn Leu Lys Leu
        115                 120                 125

gat ttg gaa ata att gat tgg tta gcg aat gct tta cct tta aat atg     432
Asp Leu Glu Ile Ile Asp Trp Leu Ala Asn Ala Leu Pro Leu Asn Met
    130                 135                 140

ggg gta att tac caa gaa att aac aaa ctt agc ttg tta ggt aaa aat     480
Gly Val Ile Tyr Gln Glu Ile Asn Lys Leu Ser Leu Leu Gly Lys Asn
145                 150                 155                 160

gaa atc aaa gat aac aaa tta gtt gaa aca gtt att tgt gat tat caa     528
Glu Ile Lys Asp Asn Lys Leu Val Glu Thr Val Ile Cys Asp Tyr Gln
                165                 170                 175

cct gtt caa att tac aaa tta acc aaa gct cta aca aat tca caa att     576
Pro Val Gln Ile Tyr Lys Leu Thr Lys Ala Leu Thr Asn Ser Gln Ile
            180                 185                 190

gta aag gca ttt aaa tat att gat gaa ttg gca agt aca aaa cca aat     624
Val Lys Ala Phe Lys Tyr Ile Asp Glu Leu Ala Ser Thr Lys Pro Asn
        195                 200                 205

ttt gcc acc caa ttt tta gaa ttt ttt agt ggg gag ttg tta tta gca     672
Phe Ala Thr Gln Phe Leu Glu Phe Phe Ser Gly Glu Leu Leu Leu Ala
    210                 215                 220

ttg atg gtt aaa agt tgt aat cca aag caa cta act aac att aat cta     720
Leu Met Val Lys Ser Cys Asn Pro Lys Gln Leu Thr Asn Ile Asn Leu
225                 230                 235                 240

aat gtt aat cag ttt cgc tta att gct att caa agt cag tac cat aac     768
Asn Val Asn Gln Phe Arg Leu Ile Ala Ile Gln Ser Gln Tyr His Asn
                245                 250                 255

ttt act agc aaa gtt tta gta aat ata att aat gca att caa aaa ttg     816
Phe Thr Ser Lys Val Leu Val Asn Ile Ile Asn Ala Ile Gln Lys Leu
            260                 265                 270

gat att aaa tta aag cac aat gat ggc ttt gcc att ccg ttg tta aaa     864
Asp Ile Lys Leu Lys His Asn Asp Gly Phe Ala Ile Pro Leu Leu Lys
        275                 280                 285

aac ttt gct tta agc ttt ttt act aac taa                             894
Asn Phe Ala Leu Ser Phe Phe Thr Asn
    290                 295

<210> 57
<211> 297
<212> PRT
<213> Mycoplasma genitalium

<400> 57
Met Thr Ile Ile Tyr Gly Gln Asp Ile Gly Leu Ile His Gln Lys Leu
1               5                   10                  15

Ser Gln Ile Lys Ser Asn Ser Pro Tyr Lys Thr Ile Trp Phe Lys Asp
            20                  25                  30

Leu Lys Gln Leu Tyr Asp Leu Phe Ser Gln Pro Leu Phe Gly Ser Asn
        35                  40                  45

Asn Glu Lys Phe Ile Val Asn Asn Cys Ser Phe Leu Glu Lys Thr Ser
    50                  55                  60

Leu Thr Arg Gln Glu Asn Leu Cys Leu Glu Lys Leu Lys Thr Thr Asp
65                  70                  75                  80

Val Val Leu Thr Val Tyr Thr Asp Asn Pro Phe Ser Gly Ile Lys Thr
                85                  90                  95

Ile Lys Ser Ile Thr Thr Val Phe Cys Asp Lys Leu Asp Trp Lys Ser
            100                 105                 110

Met His Lys Ala Ile Gly Glu Val Cys Arg Glu Leu Asn Leu Lys Leu
        115                 120                 125

Asp Leu Glu Ile Ile Asp Trp Leu Ala Asn Ala Leu Pro Leu Asn Met
    130                 135                 140

Gly Val Ile Tyr Gln Glu Ile Asn Lys Leu Ser Leu Leu Gly Lys Asn
145                 150                 155                 160

Glu Ile Lys Asp Asn Lys Leu Val Glu Thr Val Ile Cys Asp Tyr Gln
                165                 170                 175

Pro Val Gln Ile Tyr Lys Leu Thr Lys Ala Leu Thr Asn Ser Gln Ile
            180                 185                 190

Val Lys Ala Phe Lys Tyr Ile Asp Glu Leu Ala Ser Thr Lys Pro Asn
        195                 200                 205

Phe Ala Thr Gln Phe Leu Glu Phe Phe Ser Gly Glu Leu Leu Leu Ala
    210                 215                 220

Leu Met Val Lys Ser Cys Asn Pro Lys Gln Leu Thr Asn Ile Asn Leu
225                 230                 235                 240

Asn Val Asn Gln Phe Arg Leu Ile Ala Ile Gln Ser Gln Tyr His Asn
                245                 250                 255

Phe Thr Ser Lys Val Leu Val Asn Ile Ile Asn Ala Ile Gln Lys Leu
            260                 265                 270

Asp Ile Lys Leu Lys His Asn Asp Gly Phe Ala Ile Pro Leu Leu Lys
        275                 280                 285

Asn Phe Ala Leu Ser Phe Phe Thr Asn
    290                 295

<210> 58
<211> 945
<212> DNA
<213> Mycoplasma pneumoniae

<400> 58
atgggctgtg gggcttgttg tatactgtgg gtacagtctt ttactatgac cgtagtttat 60

ggtgccgata ttgggttaat tcaccagcag ttaaaccaac tgctcaaccc agccgcttgt 120

aaacaggtct ggtttcaaga tgttaataag ctgtatgatg tggtgttaaa ccagaatctg 180

tttgctgagg acactaaacc gatcctcatt cacaactgca gttttttgga aaagaacaac 240

ttaactaagg ctgaattaca ctgcttaaaa acacttaaag atactgatgt ggtggtcacc 300

atttacagcg acagtcccgc taatgccctc attaatgacc gggcaattac caagtatgct 360

tgtaaaccag tcacagctaa aaccatccac caggtgatca gtaaggccgc taagacactc 420

aaactcaact taaaccctga tttaattgac cacttagcca cgattctacc ctttaactta 480

ggggtcattg aacaggagtt gcgtaagtta actctactta gcccggcaga attacaggac 540

aaaaaaatgt tagaagccgt tttgtgtgat taccaaacca gtcaaatctt acaattaact 600

gacgccatgg tgcgtttgca aacagctaaa gccttaaagt tgattgaacg cttgttttta 660

ccaaagcagc tcacaccacc gcaattccta gaatttttag caaatgaact acttttggcc 720

ttgatggtca aaggcgttaa accacaagct gttttccaac tacagtggaa tgtcaatccc 780

tttcgtttaa aggcgattca aagccagtac cgcttttggt ccaccaacca attaacggcc 840

ttaattaatg cgatttggca gctggatatt aaaattaagc gcaatgatgg cttagcaatc 900

catttgctaa agcacttcgt gttgcgattc tttgcgcaaa agtaa 945

<210> 59
<211> 945
<212> DNA
<213> Mycoplasma pneumoniae

<220>
<221> CDS
<222> (1)..(945)

<400> 59
atg ggc tgt ggg gct tgt tgt ata ctg tgg gta cag tct ttt act atg 48
Met Gly Cys Gly Ala Cys Cys Ile Leu Trp Val Gln Ser Phe Thr Met
1               5                   10                  15

acc gta gtt tat ggt gcc gat att ggg tta att cac cag cag tta aac 96
Thr Val Val Tyr Gly Ala Asp Ile Gly Leu Ile His Gln Gln Leu Asn
            20                  25                  30

caa ctg ctc aac cca gcc gct tgt aaa cag gtc tgg ttt caa gat gtt 144
Gln Leu Leu Asn Pro Ala Ala Cys Lys Gln Val Trp Phe Gln Asp Val
        35                  40                  45

aat aag ctg tat gat gtg gtg tta aac cag aat ctg ttt gct gag gac 192
Asn Lys Leu Tyr Asp Val Val Leu Asn Gln Asn Leu Phe Ala Glu Asp
    50                  55                  60

act aaa ccg atc ctc att cac aac tgc agt ttt ttg gaa aag aac aac 240
Thr Lys Pro Ile Leu Ile His Asn Cys Ser Phe Leu Glu Lys Asn Asn
65                  70                  75                  80

tta act aag gct gaa tta cac tgc tta aaa aca ctt aaa gat act gat 288
Leu Thr Lys Ala Glu Leu His Cys Leu Lys Thr Leu Lys Asp Thr Asp
                85                  90                  95

gtg gtg gtc acc att tac agc gac agt ccc gct aat gcc ctc att aat 336
Val Val Val Thr Ile Tyr Ser Asp Ser Pro Ala Asn Ala Leu Ile Asn
            100                 105                 110

gac cgg gca att acc aag tat gct tgt aaa cca gtc aca gct aaa acc 384
Asp Arg Ala Ile Thr Lys Tyr Ala Cys Lys Pro Val Thr Ala Lys Thr
        115                 120                 125

atc cac cag gtg atc agt aag gcc gct aag aca ctc aaa ctc aac tta 432
Ile His Gln Val Ile Ser Lys Ala Ala Lys Thr Leu Lys Leu Asn Leu
    130                 135                 140

aac cct gat tta att gac cac tta gcc acg att cta ccc ttt aac tta     480
Asn Pro Asp Leu Ile Asp His Leu Ala Thr Ile Leu Pro Phe Asn Leu
145                 150                 155                 160

ggg gtc att gaa cag gag ttg cgt aag tta act cta ctt agc ccg gca     528
Gly Val Ile Glu Gln Glu Leu Arg Lys Leu Thr Leu Leu Ser Pro Ala
                165                 170                 175

gaa tta cag gac aaa aaa atg tta gaa gcc gtt ttg tgt gat tac caa     576
Glu Leu Gln Asp Lys Lys Met Leu Glu Ala Val Leu Cys Asp Tyr Gln
            180                 185                 190

acc agt caa atc tta caa tta act gac gcc atg gtg cgt ttg caa aca     624
Thr Ser Gln Ile Leu Gln Leu Thr Asp Ala Met Val Arg Leu Gln Thr
        195                 200                 205

gct aaa gcc tta aag ttg att gaa cgc ttg ttt tta cca aag cag ctc     672
Ala Lys Ala Leu Lys Leu Ile Glu Arg Leu Phe Leu Pro Lys Gln Leu
    210                 215                 220

aca cca ccg caa ttc cta gaa ttt tta gca aat gaa cta ctt ttg gcc     720
Thr Pro Pro Gln Phe Leu Glu Phe Leu Ala Asn Glu Leu Leu Leu Ala
225                 230                 235                 240

ttg atg gtc aaa ggc gtt aaa cca caa gct gtt ttc caa cta cag tgg     768
Leu Met Val Lys Gly Val Lys Pro Gln Ala Val Phe Gln Leu Gln Trp
                245                 250                 255

aat gtc aat ccc ttt cgt tta aag gcg att caa agc cag tac cgc ttt     816
Asn Val Asn Pro Phe Arg Leu Lys Ala Ile Gln Ser Gln Tyr Arg Phe
            260                 265                 270

tgg tcc acc aac caa tta acg gcc tta att aat gcg att tgg cag ctg     864
Trp Ser Thr Asn Gln Leu Thr Ala Leu Ile Asn Ala Ile Trp Gln Leu
        275                 280                 285

gat att aaa att aag cgc aat gat ggc tta gca atc cat ttg cta aag     912
Asp Ile Lys Ile Lys Arg Asn Asp Gly Leu Ala Ile His Leu Leu Lys
    290                 295                 300

cac ttc gtg ttg cga ttc ttt gcg caa aag taa                         945
His Phe Val Leu Arg Phe Phe Ala Gln Lys
305                 310                 315


<210> 60
<211> 314
<212> PRT
<213> Mycoplasma pneumoniae

<400> 60
Met Gly Cys Gly Ala Cys Cys Ile Leu Trp Val Gln Ser Phe Thr Met
1               5                   10                  15

Thr Val Val Tyr Gly Ala Asp Ile Gly Leu Ile His Gln Gln Leu Asn
            20                  25                  30

Gln Leu Leu Asn Pro Ala Ala Cys Lys Gln Val Trp Phe Gln Asp Val
        35                  40                  45

Asn Lys Leu Tyr Asp Val Val Leu Asn Gln Asn Leu Phe Ala Glu Asp
    50                  55                  60

Thr Lys Pro Ile Leu Ile His Asn Cys Ser Phe Leu Glu Lys Asn Asn
65                  70                  75                  80

Leu Thr Lys Ala Glu Leu His Cys Leu Lys Thr Leu Lys Asp Thr Asp

                85                  90                  95

Val Val Val Thr Ile Tyr Ser Asp Ser Pro Ala Asn Ala Leu Ile Asn
            100                 105                 110

Asp Arg Ala Ile Thr Lys Tyr Ala Cys Lys Pro Val Thr Ala Lys Thr
        115                 120                 125

Ile His Gln Val Ile Ser Lys Ala Ala Lys Thr Leu Lys Leu Asn Leu
    130                 135                 140

Asn Pro Asp Leu Ile Asp His Leu Ala Thr Ile Leu Pro Phe Asn Leu
145                 150                 155                 160

Gly Val Ile Glu Gln Glu Leu Arg Lys Leu Thr Leu Leu Ser Pro Ala
                165                 170                 175

Glu Leu Gln Asp Lys Lys Met Leu Glu Ala Val Leu Cys Asp Tyr Gln
            180                 185                 190

Thr Ser Gln Ile Leu Gln Leu Thr Asp Ala Met Val Arg Leu Gln Thr
        195                 200                 205

Ala Lys Ala Leu Lys Leu Ile Glu Arg Leu Phe Leu Pro Lys Gln Leu
    210                 215                 220

Thr Pro Pro Gln Phe Leu Glu Phe Leu Ala Asn Glu Leu Leu Leu Ala
225                 230                 235                 240

Leu Met Val Lys Gly Val Lys Pro Gln Ala Val Phe Gln Leu Gln Trp
                245                 250                 255

Asn Val Asn Pro Phe Arg Leu Lys Ala Ile Gln Ser Gln Tyr Arg Phe
            260                 265                 270

Trp Ser Thr Asn Gln Leu Thr Ala Leu Ile Asn Ala Ile Trp Gln Leu
        275                 280                 285

Asp Ile Lys Ile Lys Arg Asn Asp Gly Leu Ala Ile His Leu Leu Lys
    290                 295                 300

His Phe Val Leu Arg Phe Phe Ala Gln Lys
305                 310

<210> 61
<211> 999
<212> DNA
<213> Neisseria meningitidis

<220>
<223> /note=Neisseria meningitidis MC58

<400> 61
gtggcggcac atatcggacg cattgatacg gacgcgcctt tgaaacccct gtacgtcatc 60

cacggcgagg aagaactgtt gcgtatcgag gcattggacg cattgagggc ggcggcgaag 120

aaacaaggtt accttaatcg ggaagtttat acggcagaca atgccttcga ttggaacgag 180

ctgctgcaaa ccgcaggcag tgcgggtctg tttgccgatt tgaagctgtt ggaactgcat 240

atccctaacg gcaagcccgg caaaaccggc ggcgaggcgt tgcaggattt tgccgcccga 300

ttgccggaag atacggtaac gctggttttg ctgcccaaac tggagaaaac ccagctccag 360

tccaaatggt ttgccgcatt ggcggcaaag ggggaagtgt gggaagccaa accggtcggc 420

gcggcggctt tgccccaatg gatacgcgga cggctggaca aaatcggttt gggtatcgag 480

gcagacgcat tggcactgtt tgctgagcgc gtggaaggca atctgttggc ggcgcgtcag 540

gaaatcgaca agctcgggct gctgtatccg aaagggcata ccgtcaatat cgatgaggcg 600

caaaccgccg ttgccaacgt cgcccgcttc gacgcgttcc aactggcagg cgcgtggatg 660

aagggcgatg tcctgcgcgt atgcaggctt ttggacggat tgcgggaaga gggcgaagaa 720

ccggtgctgt tgctgtgggc ggttgccgaa gacgtgcgga cgctgatccg gcttgctgcc 780

gccctgaagc aggggcagag catccaatcc gtccgcaaca gcctcaggct ttggggcgac 840

aagcagacgc tcgcaccgct tgcggtcaag cggatttccg tcgtccgcct gcttgacgcg 900

ctcaaaacct gcgcccaaat cgaccgaatc atcaaaggtg cggaagacgg cgacgcatgg 960

acggtattca aacggcttgt cgtgtcgctg gcggaataa 999

<210> 62
<211> 999
<212> DNA
<213> Neisseria meningitidis

<220>
<223> /note=Neisseria meningitidis MC58

<220>
<221> CDS
<222> (1)..(999)

<400> 62
gtg gcg gca cat atc gga cgc att gat acg gac gcg cct ttg aaa ccc 48
Val Ala Ala His Ile Gly Arg Ile Asp Thr Asp Ala Pro Leu Lys Pro
1               5                   10                  15

ctg tac gtc atc cac ggc gag gaa gaa ctg ttg cgt atc gag gca ttg 96
Leu Tyr Val Ile His Gly Glu Glu Glu Leu Leu Arg Ile Glu Ala Leu
            20                  25                  30

gac gca ttg agg gcg gcg gcg aag aaa caa ggt tac ctt aat cgg gaa 144
Asp Ala Leu Arg Ala Ala Ala Lys Lys Gln Gly Tyr Leu Asn Arg Glu
        35                  40                  45

gtt tat acg gca gac aat gcc ttc gat tgg aac gag ctg ctg caa acc 192
Val Tyr Thr Ala Asp Asn Ala Phe Asp Trp Asn Glu Leu Leu Gln Thr
    50                  55                  60

gca ggc agt gcg ggt ctg ttt gcc gat ttg aag ctg ttg gaa ctg cat 240
Ala Gly Ser Ala Gly Leu Phe Ala Asp Leu Lys Leu Leu Glu Leu His
65                  70                  75                  80

atc cct aac ggc aag ccc ggc aaa acc ggc ggc gag gcg ttg cag gat 288
Ile Pro Asn Gly Lys Pro Gly Lys Thr Gly Gly Glu Ala Leu Gln Asp
                85                  90                  95

ttt gcc gcc cga ttg ccg gaa gat acg gta acg ctg gtt ttg ctg ccc 336
Phe Ala Ala Arg Leu Pro Glu Asp Thr Val Thr Leu Val Leu Leu Pro
            100                 105                 110

aaa ctg gag aaa acc cag ctc cag tcc aaa tgg ttt gcc gca ttg gcg 384
Lys Leu Glu Lys Thr Gln Leu Gln Ser Lys Trp Phe Ala Ala Leu Ala
        115                 120                 125

gca aag ggg gaa gtg tgg gaa gcc aaa ccg gtc ggc gcg gcg gct ttg 432
Ala Lys Gly Glu Val Trp Glu Ala Lys Pro Val Gly Ala Ala Ala Leu

130                    135                    140

ccc caa tgg ata cgc gga cgg ctg gac aaa atc ggt ttg ggt atc gag     480
Pro Gln Trp Ile Arg Gly Arg Leu Asp Lys Ile Gly Leu Gly Ile Glu
145                 150                 155                 160

gca gac gca ttg gca ctg ttt gct gag cgc gtg gaa ggc aat ctg ttg     528
Ala Asp Ala Leu Ala Leu Phe Ala Glu Arg Val Glu Gly Asn Leu Leu
                165                 170                 175

gcg gcg cgt cag gaa atc gac aag ctc ggg ctg ctg tat ccg aaa ggg     576
Ala Ala Arg Gln Glu Ile Asp Lys Leu Gly Leu Leu Tyr Pro Lys Gly
            180                 185                 190

cat acc gtc aat atc gat gag gcg caa acc gcc gtt gcc aac gtc gcc     624
His Thr Val Asn Ile Asp Glu Ala Gln Thr Ala Val Ala Asn Val Ala
        195                 200                 205

cgc ttc gac gcg ttc caa ctg gca ggc gcg tgg atg aag ggc gat gtc     672
Arg Phe Asp Ala Phe Gln Leu Ala Gly Ala Trp Met Lys Gly Asp Val
    210                 215                 220

ctg cgc gta tgc agg ctt ttg gac gga ttg cgg gaa gag ggc gaa gaa     720
Leu Arg Val Cys Arg Leu Leu Asp Gly Leu Arg Glu Glu Gly Glu Glu
225                 230                 235                 240

ccg gtg ctg ttg ctg tgg gcg gtt gcc gaa gac gtg cgg acg ctg atc     768
Pro Val Leu Leu Leu Trp Ala Val Ala Glu Asp Val Arg Thr Leu Ile
                245                 250                 255

cgg ctt gct gcc gcc ctg aag cag ggg cag agc atc caa tcc gtc cgc     816
Arg Leu Ala Ala Ala Leu Lys Gln Gly Gln Ser Ile Gln Ser Val Arg
            260                 265                 270

aac agc ctc agg ctt tgg ggc gac aag cag acg ctc gca ccg ctt gcg     864
Asn Ser Leu Arg Leu Trp Gly Asp Lys Gln Thr Leu Ala Pro Leu Ala
        275                 280                 285

gtc aag cgg att tcc gtc gtc cgc ctg ctt gac gcg ctc aaa acc tgc     912
Val Lys Arg Ile Ser Val Val Arg Leu Leu Asp Ala Leu Lys Thr Cys
    290                 295                 300

gcc caa atc gac cga atc atc aaa ggt gcg gaa gac ggc gac gca tgg     960
Ala Gln Ile Asp Arg Ile Ile Lys Gly Ala Glu Asp Gly Asp Ala Trp
305                 310                 315                 320

acg gta ttc aaa cgg ctt gtc gtg tcg ctg gcg gaa taa                 999
Thr Val Phe Lys Arg Leu Val Val Ser Leu Ala Glu
                325                 330

<210> 63
<211> 332
<212> PRT
<213> Neisseria meningitidis

<220>
<223> /note=Neisseria meningitidis MC58

<400> 63
Val Ala Ala His Ile Gly Arg Ile Asp Thr Asp Ala Pro Leu Lys Pro
1               5                   10                  15

Leu Tyr Val Ile His Gly Glu Glu Glu Leu Leu Arg Ile Glu Ala Leu
            20                  25                  30

Asp Ala Leu Arg Ala Ala Ala Lys Lys Gln Gly Tyr Leu Asn Arg Glu
        35                  40                  45

Val Tyr Thr Ala Asp Asn Ala Phe Asp Trp Asn Glu Leu Leu Gln Thr
    50                  55                  60

Ala Gly Ser Ala Gly Leu Phe Ala Asp Leu Lys Leu Leu Glu Leu His
65                  70                  75                  80

Ile Pro Asn Gly Lys Pro Gly Lys Thr Gly Gly Glu Ala Leu Gln Asp
                85                  90                  95

Phe Ala Ala Arg Leu Pro Glu Asp Thr Val Thr Leu Val Leu Leu Pro
            100                 105                 110

Lys Leu Glu Lys Thr Gln Leu Gln Ser Lys Trp Phe Ala Ala Leu Ala
        115                 120                 125

Ala Lys Gly Glu Val Trp Glu Ala Lys Pro Val Gly Ala Ala Ala Leu
    130                 135                 140

Pro Gln Trp Ile Arg Gly Arg Leu Asp Lys Ile Gly Leu Gly Ile Glu
145                 150                 155                 160

Ala Asp Ala Leu Ala Leu Phe Ala Glu Arg Val Glu Gly Asn Leu Leu
                165                 170                 175

Ala Ala Arg Gln Glu Ile Asp Lys Leu Gly Leu Leu Tyr Pro Lys Gly
            180                 185                 190

His Thr Val Asn Ile Asp Glu Ala Gln Thr Ala Val Ala Asn Val Ala
        195                 200                 205

Arg Phe Asp Ala Phe Gln Leu Ala Gly Ala Trp Met Lys Gly Asp Val
    210                 215                 220

Leu Arg Val Cys Arg Leu Leu Asp Gly Leu Arg Glu Glu Gly Glu Glu
225                 230                 235                 240

Pro Val Leu Leu Leu Trp Ala Val Ala Glu Asp Val Arg Thr Leu Ile
                245                 250                 255

Arg Leu Ala Ala Ala Leu Lys Gln Gly Gln Ser Ile Gln Ser Val Arg
            260                 265                 270

Asn Ser Leu Arg Leu Trp Gly Asp Lys Gln Thr Leu Ala Pro Leu Ala
        275                 280                 285

Val Lys Arg Ile Ser Val Val Arg Leu Leu Asp Ala Leu Lys Thr Cys
    290                 295                 300

Ala Gln Ile Asp Arg Ile Ile Lys Gly Ala Glu Asp Gly Asp Ala Trp
305                 310                 315                 320

Thr Val Phe Lys Arg Leu Val Val Ser Leu Ala Glu
                325                 330

<210> 64
<211> 999
<212> DNA
<213> Neisseria meningitidis

<220>
<223> /note=Neisseria meningitidis Z2491

<400> 64
gtggcggcac atatcggacg cattgatacg gacgcgcctt tgaaacctct gtacgtcatc 60

cacggcgagg aagaactgtt gcgtatcgag gcattggacg cattgagggc ggcggcgaag 120

aaacaaggtt accttaatcg ggaagtttat acggcagaca atgccttcga ttggaacgaa 180

ctgttgcaaa ccgcaggcag tgcgggtctg tttgccgatt tgaagctgtt ggaactgcat 240

atccccaacg gaaaacccgg caaaaccggc ggcgaggcgt tgcaggattt tgccgcccga 300

ttgccggaag atacggtaac gctggttttg ctgcccaaac tggagaaaac ccagctccag 360

tccaaatggt ttgccgcatt ggcggcaaag ggggaagtgt gggaagccaa accggtcggc 420

gcggcggctt tgccccaatg gatacgcgga cggctggaca aaatcggttt gggtatcgag 480

gcagacgcat tggcactgtt tgctgagcgc gtggaaggca atctgttggc ggcgcgtcag 540

gaaatcgaca agctcgggct gctgtatccg aaagggcata ccgtcaatat cgatgaggcg 600

caaaccgccg ttgccaacgt cgcccgcttc gacgcgttcc aactggcagg cgcgtggatg 660

aagggcgatg tcctgcgcgt atgcaggctt ttggacggat tgcgggaaga gggcgaagaa 720

ccggtgctgt tgctgtgggc ggttgccgaa gacgtgcgga cgctgatccg gcttgctgcc 780

gccctgaagc aggggcagag catccaatcc gtccgcaaca gcctcaggct ttggggcgac 840

aagcagacgc tcgcaccgct tgcggtcaag cggatttccg tcgtccgcct gcttgacgcg 900

ctcaaaacct gcgcccaaat cgaccgaatc atcaaaggcg cggaagaggg cgacgcatgg 960

acggtattca aacggcttgt cgtgtcgctg gcggaataa 999

<210> 65
<211> 999
<212> DNA
<213> Neisseria meningitidis

<220>
<223> /note=Neisseria meningitidis Z2491

<220>
<221> CDS
<222> (1)..(999)

<400> 65
gtg gcg gca cat atc gga cgc att gat acg gac gcg cct ttg aaa cct 48
Val Ala Ala His Ile Gly Arg Ile Asp Thr Asp Ala Pro Leu Lys Pro
1               5                   10                  15

ctg tac gtc atc cac ggc gag gaa gaa ctg ttg cgt atc gag gca ttg 96
Leu Tyr Val Ile His Gly Glu Glu Glu Leu Leu Arg Ile Glu Ala Leu
            20                  25                  30

gac gca ttg agg gcg gcg gcg aag aaa caa ggt tac ctt aat cgg gaa 144
Asp Ala Leu Arg Ala Ala Ala Lys Lys Gln Gly Tyr Leu Asn Arg Glu
        35                  40                  45

gtt tat acg gca gac aat gcc ttc gat tgg aac gaa ctg ttg caa acc 192
Val Tyr Thr Ala Asp Asn Ala Phe Asp Trp Asn Glu Leu Leu Gln Thr
    50                  55                  60

gca ggc agt gcg ggt ctg ttt gcc gat ttg aag ctg ttg gaa ctg cat 240
Ala Gly Ser Ala Gly Leu Phe Ala Asp Leu Lys Leu Leu Glu Leu His
65                  70                  75                  80

atc ccc aac gga aaa ccc ggc aaa acc ggc ggc gag gcg ttg cag gat 288
Ile Pro Asn Gly Lys Pro Gly Lys Thr Gly Gly Glu Ala Leu Gln Asp
                85                  90                  95

ttt gcc gcc cga ttg ccg gaa gat acg gta acg ctg gtt ttg ctg ccc 336
Phe Ala Ala Arg Leu Pro Glu Asp Thr Val Thr Leu Val Leu Leu Pro
            100                 105                 110

aaa ctg gag aaa acc cag ctc cag tcc aaa tgg ttt gcc gca ttg gcg 384
Lys Leu Glu Lys Thr Gln Leu Gln Ser Lys Trp Phe Ala Ala Leu Ala
        115                 120                 125

gca aag ggg gaa gtg tgg gaa gcc aaa ccg gtc ggc gcg gcg gct ttg 432
Ala Lys Gly Glu Val Trp Glu Ala Lys Pro Val Gly Ala Ala Ala Leu
    130                 135                 140

ccc caa tgg ata cgc gga cgg ctg gac aaa atc ggt ttg ggt atc gag 480
Pro Gln Trp Ile Arg Gly Arg Leu Asp Lys Ile Gly Leu Gly Ile Glu
145                 150                 155                 160

gca gac gca ttg gca ctg ttt gct gag cgc gtg gaa ggc aat ctg ttg 528
Ala Asp Ala Leu Ala Leu Phe Ala Glu Arg Val Glu Gly Asn Leu Leu
                165                 170                 175

gcg gcg cgt cag gaa atc gac aag ctc ggg ctg ctg tat ccg aaa ggg 576
Ala Ala Arg Gln Glu Ile Asp Lys Leu Gly Leu Leu Tyr Pro Lys Gly
            180                 185                 190

cat acc gtc aat atc gat gag gcg caa acc gcc gtt gcc aac gtc gcc 624
His Thr Val Asn Ile Asp Glu Ala Gln Thr Ala Val Ala Asn Val Ala
        195                 200                 205

cgc ttc gac gcg ttc caa ctg gca ggc gcg tgg atg aag ggc gat gtc 672
Arg Phe Asp Ala Phe Gln Leu Ala Gly Ala Trp Met Lys Gly Asp Val
    210                 215                 220

ctg cgc gta tgc agg ctt ttg gac gga ttg cgg gaa gag ggc gaa gaa 720
Leu Arg Val Cys Arg Leu Leu Asp Gly Leu Arg Glu Glu Gly Glu Glu
225                 230                 235                 240

ccg gtg ctg ttg ctg tgg gcg gtt gcc gaa gac gtg cgg acg ctg atc 768
Pro Val Leu Leu Leu Trp Ala Val Ala Glu Asp Val Arg Thr Leu Ile
                245                 250                 255

cgg ctt gct gcc gcc ctg aag cag ggg cag agc atc caa tcc gtc cgc 816
Arg Leu Ala Ala Ala Leu Lys Gln Gly Gln Ser Ile Gln Ser Val Arg
            260                 265                 270

aac agc ctc agg ctt tgg ggc gac aag cag acg ctc gca ccg ctt gcg 864
Asn Ser Leu Arg Leu Trp Gly Asp Lys Gln Thr Leu Ala Pro Leu Ala
        275                 280                 285

gtc aag cgg att tcc gtc gtc cgc ctg ctt gac gcg ctc aaa acc tgc 912
Val Lys Arg Ile Ser Val Val Arg Leu Leu Asp Ala Leu Lys Thr Cys
    290                 295                 300

gcc caa atc gac cga atc atc aaa ggc gcg gaa gag ggc gac gca tgg 960
Ala Gln Ile Asp Arg Ile Ile Lys Gly Ala Glu Glu Gly Asp Ala Trp
305                 310                 315                 320

acg gta ttc aaa cgg ctt gtc gtg tcg ctg gcg gaa taa 999
Thr Val Phe Lys Arg Leu Val Val Ser Leu Ala Glu
                325                 330

<210> 66
<211> 332
<212> PRT
<213> Neisseria meningitidis

<220>

<223> /note=Neisseria meningitidis Z2491

<400> 66
Val Ala Ala His Ile Gly Arg Ile Asp Thr Asp Ala Pro Leu Lys Pro
1               5                   10                  15

Leu Tyr Val Ile His Gly Glu Glu Glu Leu Leu Arg Ile Glu Ala Leu
            20                  25                  30

Asp Ala Leu Arg Ala Ala Ala Lys Lys Gln Gly Tyr Leu Asn Arg Glu
        35                  40                  45

Val Tyr Thr Ala Asp Asn Ala Phe Asp Trp Asn Glu Leu Leu Gln Thr
    50                  55                  60

Ala Gly Ser Ala Gly Leu Phe Ala Asp Leu Lys Leu Leu Glu Leu His
65                  70                  75                  80

Ile Pro Asn Gly Lys Pro Gly Lys Thr Gly Gly Glu Ala Leu Gln Asp
                85                  90                  95

Phe Ala Ala Arg Leu Pro Glu Asp Thr Val Thr Leu Val Leu Leu Pro
            100                 105                 110

Lys Leu Glu Lys Thr Gln Leu Gln Ser Lys Trp Phe Ala Ala Leu Ala
        115                 120                 125

Ala Lys Gly Glu Val Trp Glu Ala Lys Pro Val Gly Ala Ala Ala Leu
    130                 135                 140

Pro Gln Trp Ile Arg Gly Arg Leu Asp Lys Ile Gly Leu Gly Ile Glu
145                 150                 155                 160

Ala Asp Ala Leu Ala Leu Phe Ala Glu Arg Val Glu Gly Asn Leu Leu
                165                 170                 175

Ala Ala Arg Gln Glu Ile Asp Lys Leu Gly Leu Leu Tyr Pro Lys Gly
            180                 185                 190

His Thr Val Asn Ile Asp Glu Ala Gln Thr Ala Val Ala Asn Val Ala
        195                 200                 205

Arg Phe Asp Ala Phe Gln Leu Ala Gly Ala Trp Met Lys Gly Asp Val
    210                 215                 220

Leu Arg Val Cys Arg Leu Leu Asp Gly Leu Arg Glu Glu Gly Glu Glu
225                 230                 235                 240

Pro Val Leu Leu Leu Trp Ala Val Ala Glu Asp Val Arg Thr Leu Ile
                245                 250                 255

Arg Leu Ala Ala Ala Leu Lys Gln Gly Gln Ser Ile Gln Ser Val Arg
            260                 265                 270

Asn Ser Leu Arg Leu Trp Gly Asp Lys Gln Thr Leu Ala Pro Leu Ala
        275                 280                 285

Val Lys Arg Ile Ser Val Val Arg Leu Leu Asp Ala Leu Lys Thr Cys
    290                 295                 300

Ala Gln Ile Asp Arg Ile Ile Lys Gly Ala Glu Glu Gly Asp Ala Trp
305                 310                 315                 320

Thr Val Phe Lys Arg Leu Val Val Ser Leu Ala Glu
                325                 330

<210> 67

<211> 1080
<212> DNA
<213> Pasteurella multocida

<400> 67
atgcaacgga ttttcacaga acaattagga tctgctctac aaaacaaact agccaagctc 60

tattatttcg tagggcaaga tccgttactg ttaagtgaaa atcaagatcg cataaatcaa 120

tatgcattca agcaaggttt tgacgaaaaa aatgtcatca caattgatca gaatactaat 180

tggtctgatc tctttgaacg ctgccaatcc attggcttgt ttttcaataa acagattttg 240

tttcttaatt taccggaaaa cctcagtgca agtttacaaa ctcgtttaca agaactgatc 300

tcactcctca acgatgacat cctgttaata ctacaattgc ctaaactgaa caaagcgact 360

gaaaaacaaa catggtttat ccaagctaat cagtatgaac cggaagccgt actcgtgcat 420

tgccaaaccc caaccattga acaattgcct cgttggatcg cgttgcgtgc gaaaagtatg 480

ggactacaga ttgaagagca agcaatccaa cttctttgtt atagccatga aaataactta 540

ttagccttaa aacaaagttt gcagctcctt gctttactct atcctgacaa caaaatcagt 600

tatgcacgag tacaatccac tatcgctcag acctcagtgt tcacaccatt tcaatggctc 660

gatgcgctat tagaaggcaa aagtaaacgc gcccaacgta ttttagaagg attaaaagca 720

gaggaaatgg agcccattat tttattgcgg acgttacaac gagatttgct caccttatta 780

ggattggcga agccatcaca tcctgtcacc cttaccacgc ccctcccagc tcatcaactg 840

cgtgaacaat ttgatcaatt aaaagtgtgg caaaatcgac gccctttctt tactcaagcc 900

tttaaacgct tgaattacaa acagctttat ttgattattc aagcgctagc agagatcgaa 960

cgtttagcga aacacacatt tagccaagat atttgggatc gtttagccga actttctgct 1020

ttcatttgtt taccagatga tcccaatcac ctactatctc taccaacttt ctctcgttga 1080


<210> 68
<211> 1080
<212> DNA
<213> Pasteurella multocida

<220>
<221> CDS
<222> (1)..(1080)

<400> 68
atg caa cgg att ttc aca gaa caa tta gga tct gct cta caa aac aaa 48
Met Gln Arg Ile Phe Thr Glu Gln Leu Gly Ser Ala Leu Gln Asn Lys
1               5                   10                  15

cta gcc aag ctc tat tat ttc gta ggg caa gat ccg tta ctg tta agt 96
Leu Ala Lys Leu Tyr Tyr Phe Val Gly Gln Asp Pro Leu Leu Leu Ser
            20                  25                  30

gaa aat caa gat cgc ata aat caa tat gca ttc aag caa ggt ttt gac 144
Glu Asn Gln Asp Arg Ile Asn Gln Tyr Ala Phe Lys Gln Gly Phe Asp
        35                  40                  45

gaa aaa aat gtc atc aca att gat cag aat act aat tgg tct gat ctc 192
Glu Lys Asn Val Ile Thr Ile Asp Gln Asn Thr Asn Trp Ser Asp Leu
    50                  55                  60

ttt gaa cgc tgc caa tcc att ggc ttg ttt ttc aat aaa cag att ttg 240
Phe Glu Arg Cys Gln Ser Ile Gly Leu Phe Phe Asn Lys Gln Ile Leu
65                  70                  75                  80

ttt ctt aat tta ccg gaa aac ctc agt gca agt tta caa act cgt tta 288
Phe Leu Asn Leu Pro Glu Asn Leu Ser Ala Ser Leu Gln Thr Arg Leu
                85                  90                  95

caa gaa ctg atc tca ctc ctc aac gat gac atc ctg tta ata cta caa 336
Gln Glu Leu Ile Ser Leu Leu Asn Asp Asp Ile Leu Leu Ile Leu Gln
            100                 105                 110

ttg cct aaa ctg aac aaa gcg act gaa aaa caa aca tgg ttt atc caa 384
Leu Pro Lys Leu Asn Lys Ala Thr Glu Lys Gln Thr Trp Phe Ile Gln
        115                 120                 125

gct aat cag tat gaa ccg gaa gcc gta ctc gtg cat tgc caa acc cca 432
Ala Asn Gln Tyr Glu Pro Glu Ala Val Leu Val His Cys Gln Thr Pro
    130                 135                 140

acc att gaa caa ttg cct cgt tgg atc gcg ttg cgt gcg aaa agt atg 480
Thr Ile Glu Gln Leu Pro Arg Trp Ile Ala Leu Arg Ala Lys Ser Met
145                 150                 155                 160

gga cta cag att gaa gag caa gca atc caa ctt ctt tgt tat agc cat 528
Gly Leu Gln Ile Glu Glu Gln Ala Ile Gln Leu Leu Cys Tyr Ser His
                165                 170                 175

gaa aat aac tta tta gcc tta aaa caa agt ttg cag ctc ctt gct tta 576
Glu Asn Asn Leu Leu Ala Leu Lys Gln Ser Leu Gln Leu Leu Ala Leu
            180                 185                 190

ctc tat cct gac aac aaa atc agt tat gca cga gta caa tcc act atc 624
Leu Tyr Pro Asp Asn Lys Ile Ser Tyr Ala Arg Val Gln Ser Thr Ile
        195                 200                 205

gct cag acc tca gtg ttc aca cca ttt caa tgg ctc gat gcg cta tta 672
Ala Gln Thr Ser Val Phe Thr Pro Phe Gln Trp Leu Asp Ala Leu Leu
    210                 215                 220

gaa ggc aaa agt aaa cgc gcc caa cgt att tta gaa gga tta aaa gca 720
Glu Gly Lys Ser Lys Arg Ala Gln Arg Ile Leu Glu Gly Leu Lys Ala
225                 230                 235                 240

gag gaa atg gag ccc att att tta ttg cgg acg tta caa cga gat ttg 768
Glu Glu Met Glu Pro Ile Ile Leu Leu Arg Thr Leu Gln Arg Asp Leu
                245                 250                 255

ctc acc tta tta gga ttg gcg aag cca tca cat cct gtc acc ctt acc 816
Leu Thr Leu Leu Gly Leu Ala Lys Pro Ser His Pro Val Thr Leu Thr
            260                 265                 270

acg ccc ctc cca gct cat caa ctg cgt gaa caa ttt gat caa tta aaa 864
Thr Pro Leu Pro Ala His Gln Leu Arg Glu Gln Phe Asp Gln Leu Lys
        275                 280                 285

gtg tgg caa aat cga cgc cct ttc ttt act caa gcc ttt aaa cgc ttg 912
Val Trp Gln Asn Arg Arg Pro Phe Phe Thr Gln Ala Phe Lys Arg Leu
    290                 295                 300

aat tac aaa cag ctt tat ttg att att caa gcg cta gca gag atc gaa 960
Asn Tyr Lys Gln Leu Tyr Leu Ile Ile Gln Ala Leu Ala Glu Ile Glu
305                 310                 315                 320

cgt tta gcg aaa cac aca ttt agc caa gat att tgg gat cgt tta gcc 1008
Arg Leu Ala Lys His Thr Phe Ser Gln Asp Ile Trp Asp Arg Leu Ala
                325                 330                 335

gaa ctt tct gct ttc att tgt tta cca gat gat ccc aat cac cta cta     1056
Glu Leu Ser Ala Phe Ile Cys Leu Pro Asp Asp Pro Asn His Leu Leu
            340                 345                 350

tct cta cca act ttc tct cgt tga                                     1080
Ser Leu Pro Thr Phe Ser Arg
        355                 360

<210> 69
<211> 359
<212> PRT
<213> Pasteurella multocida

<400> 69
Met Gln Arg Ile Phe Thr Glu Gln Leu Gly Ser Ala Leu Gln Asn Lys
1               5                   10                  15

Leu Ala Lys Leu Tyr Tyr Phe Val Gly Gln Asp Pro Leu Leu Leu Ser
            20                  25                  30

Glu Asn Gln Asp Arg Ile Asn Gln Tyr Ala Phe Lys Gln Gly Phe Asp
        35                  40                  45

Glu Lys Asn Val Ile Thr Ile Asp Gln Asn Thr Asn Trp Ser Asp Leu
    50                  55                  60

Phe Glu Arg Cys Gln Ser Ile Gly Leu Phe Phe Asn Lys Gln Ile Leu
65                  70                  75                  80

Phe Leu Asn Leu Pro Glu Asn Leu Ser Ala Ser Leu Gln Thr Arg Leu
                85                  90                  95

Gln Glu Leu Ile Ser Leu Leu Asn Asp Asp Ile Leu Leu Ile Leu Gln
            100                 105                 110

Leu Pro Lys Leu Asn Lys Ala Thr Glu Lys Gln Thr Trp Phe Ile Gln
        115                 120                 125

Ala Asn Gln Tyr Glu Pro Glu Ala Val Leu Val His Cys Gln Thr Pro
    130                 135                 140

Thr Ile Glu Gln Leu Pro Arg Trp Ile Ala Leu Arg Ala Lys Ser Met
145                 150                 155                 160

Gly Leu Gln Ile Glu Glu Gln Ala Ile Gln Leu Leu Cys Tyr Ser His
                165                 170                 175

Glu Asn Asn Leu Leu Ala Leu Lys Gln Ser Leu Gln Leu Leu Ala Leu
            180                 185                 190

Leu Tyr Pro Asp Asn Lys Ile Ser Tyr Ala Arg Val Gln Ser Thr Ile
        195                 200                 205

Ala Gln Thr Ser Val Phe Thr Pro Phe Gln Trp Leu Asp Ala Leu Leu
    210                 215                 220

Glu Gly Lys Ser Lys Arg Ala Gln Arg Ile Leu Glu Gly Leu Lys Ala
225                 230                 235                 240

Glu Glu Met Glu Pro Ile Ile Leu Leu Arg Thr Leu Gln Arg Asp Leu
                245                 250                 255

Leu Thr Leu Leu Gly Leu Ala Lys Pro Ser His Pro Val Thr Leu Thr
            260                 265                 270

Thr Pro Leu Pro Ala His Gln Leu Arg Glu Gln Phe Asp Gln Leu Lys
        275                 280                 285

Val Trp Gln Asn Arg Arg Pro Phe Phe Thr Gln Ala Phe Lys Arg Leu
    290                 295                 300

Asn Tyr Lys Gln Leu Tyr Leu Ile Ile Gln Ala Leu Ala Glu Ile Glu
305                 310                 315                 320

Arg Leu Ala Lys His Thr Phe Ser Gln Asp Ile Trp Asp Arg Leu Ala
                325                 330                 335

Glu Leu Ser Ala Phe Ile Cys Leu Pro Asp Asp Pro Asn His Leu Leu
            340                 345                 350

Ser Leu Pro Thr Phe Ser Arg
        355

<210> 70
<211> 1038
<212> DNA
<213> Pseudomonas aeruginosa

<400> 70
   atgaagctga ctcccgcgca actcgccaag cacctccagg gccccctggc gcccgtctat 60
   gtcgtcagcg gcgatgaacc gctgctctgc caggaagcct gcgatgccat tcgccaggcc 120
   tgccgcgagc gcgacttcgg cgagcgccag gtattcaatg ccgaagccaa cttcgattgg 180
   ggcttgctgc tggaggccgg cgccagcctg tcgctgttcg ccgagaagcg cctgatcgaa 240
   ctgcgcctgc cctccggcaa gcccggcgac aaaggcgcgg cgattctcca ggaatacctg 300
   caacggccgc cggaagacac cgtgctcctg ctcggtctgc ccaagctcga tggcagcacg 360
   cagaagacca agtgggccaa ggccctgatc gatggcaacg ccgcgcagtt catccaggtc 420
   tggccggtcg acgtccacca gttgccgcag tggattcgcc agcgcctgtc ccaggctggc 480
   ctgtccgcca gccccgaagc cctggagctg atcgccgcgc gcgtggaagg caacctgctc 540
   gccgccgccc aggaaatcga aaagctcaag ctgctcgccg agggcaacca gatcgacgcc 600
   gccaccgtac aggccgcggt cgccgacagc gcacgcttcg atgtcttcgg cctgatcgac 660
   gctgcccttg gcggtgaagc ggcccacgcc ctgcgcatcc tggaaggact gcgcggcgaa 720
   ggcatcgagc cgccggtgat cctctggggc ctggcccgcg agatccgcct gctggctggc 780
   ctgtcccagc aatacggcca gggcatcccg ctggagaaag ccttcgccca ggcgcgtccg 840
   ccggtctggg acaagcgccg tccgctgctg acccgtgccc tgcaacgcca ctccagcagc 900
   cgctggaacc agatgctgag ggatgcccag ttgatcgacg cgcagatcaa gggacaggca 960
   cccggcagcc cctggagcgg tctctcgctt ctcgccctga gcctcgccgg ccagcgcctc 1020
   ggcctgccgg cagcctga                                                1038

<210> 71
<211> 1038
<212> DNA
<213> Pseudomonas aeruginosa

<220>
<221> CDS
<222> (1)..(1038)

<400> 71
atg aag ctg act ccc gcg caa ctc gcc aag cac ctc cag ggc ccc ctg     48
Met Lys Leu Thr Pro Ala Gln Leu Ala Lys His Leu Gln Gly Pro Leu
1               5                   10                  15

gcg ccc gtc tat gtc gtc agc ggc gat gaa ccg ctg ctc tgc cag gaa     96
Ala Pro Val Tyr Val Val Ser Gly Asp Glu Pro Leu Leu Cys Gln Glu
            20                  25                  30

gcc tgc gat gcc att cgc cag gcc tgc cgc gag cgc gac ttc ggc gag     144
Ala Cys Asp Ala Ile Arg Gln Ala Cys Arg Glu Arg Asp Phe Gly Glu
        35                  40                  45

cgc cag gta ttc aat gcc gaa gcc aac ttc gat tgg ggc ttg ctg ctg     192
Arg Gln Val Phe Asn Ala Glu Ala Asn Phe Asp Trp Gly Leu Leu Leu
    50                  55                  60

gag gcc ggc gcc agc ctg tcg ctg ttc gcc gag aag cgc ctg atc gaa     240
Glu Ala Gly Ala Ser Leu Ser Leu Phe Ala Glu Lys Arg Leu Ile Glu
65                  70                  75                  80

ctg cgc ctg ccc tcc ggc aag ccc ggc gac aaa ggc gcg gcg att ctc     288
Leu Arg Leu Pro Ser Gly Lys Pro Gly Asp Lys Gly Ala Ala Ile Leu
                85                  90                  95

cag gaa tac ctg caa cgg ccg ccg gaa gac acc gtg ctc ctg ctc ggt     336
Gln Glu Tyr Leu Gln Arg Pro Pro Glu Asp Thr Val Leu Leu Leu Gly
            100                 105                 110

ctg ccc aag ctc gat ggc agc acg cag aag acc aag tgg gcc aag gcc     384
Leu Pro Lys Leu Asp Gly Ser Thr Gln Lys Thr Lys Trp Ala Lys Ala
        115                 120                 125

ctg atc gat ggc aac gcc gcg cag ttc atc cag gtc tgg ccg gtc gac     432
Leu Ile Asp Gly Asn Ala Ala Gln Phe Ile Gln Val Trp Pro Val Asp
    130                 135                 140

gtc cac cag ttg ccg cag tgg att cgc cag cgc ctg tcc cag gct ggc     480
Val His Gln Leu Pro Gln Trp Ile Arg Gln Arg Leu Ser Gln Ala Gly
145                 150                 155                 160

ctg tcc gcc agc ccc gaa gcc ctg gag ctg atc gcc gcg cgc gtg gaa     528
Leu Ser Ala Ser Pro Glu Ala Leu Glu Leu Ile Ala Ala Arg Val Glu
                165                 170                 175

ggc aac ctg ctc gcc gcc gcc cag gaa atc gaa aag ctc aag ctg ctc     576
Gly Asn Leu Leu Ala Ala Ala Gln Glu Ile Glu Lys Leu Lys Leu Leu
            180                 185                 190

gcc gag ggc aac cag atc gac gcc gcc acc gta cag gcc gcg gtc gcc     624
Ala Glu Gly Asn Gln Ile Asp Ala Ala Thr Val Gln Ala Ala Val Ala
        195                 200                 205

gac agc gca cgc ttc gat gtc ttc ggc ctg atc gac gct gcc ctt ggc     672
Asp Ser Ala Arg Phe Asp Val Phe Gly Leu Ile Asp Ala Ala Leu Gly
    210                 215                 220

ggt gaa gcg gcc cac gcc ctg cgc atc ctg gaa gga ctg cgc ggc gaa     720
Gly Glu Ala Ala His Ala Leu Arg Ile Leu Glu Gly Leu Arg Gly Glu
225                 230                 235                 240

ggc atc gag ccg ccg gtg atc ctc tgg ggc ctg gcc cgc gag atc cgc     768
Gly Ile Glu Pro Pro Val Ile Leu Trp Gly Leu Ala Arg Glu Ile Arg
                245                 250                 255

ctg ctg gct ggc ctg tcc cag caa tac ggc cag ggc atc ccg ctg gag     816
Leu Leu Ala Gly Leu Ser Gln Gln Tyr Gly Gln Gly Ile Pro Leu Glu

260                 265                 270

aaa gcc ttc gcc cag gcg cgt ccg ccg gtc tgg gac aag cgc cgt ccg     864
Lys Ala Phe Ala Gln Ala Arg Pro Pro Val Trp Asp Lys Arg Arg Pro
        275                 280                 285

ctg ctg acc cgt gcc ctg caa cgc cac tcc agc agc cgc tgg aac cag     912
Leu Leu Thr Arg Ala Leu Gln Arg His Ser Ser Ser Arg Trp Asn Gln
    290                 295                 300

atg ctg agg gat gcc cag ttg atc gac gcg cag atc aag gga cag gca     960
Met Leu Arg Asp Ala Gln Leu Ile Asp Ala Gln Ile Lys Gly Gln Ala
305                 310                 315                 320

ccc ggc agc ccc tgg agc ggt ctc tcg ctt ctc gcc ctg agc ctc gcc     1008
Pro Gly Ser Pro Trp Ser Gly Leu Ser Leu Leu Ala Leu Ser Leu Ala
                325                 330                 335

ggc cag cgc ctc ggc ctg ccg gca gcc tga                             1038
Gly Gln Arg Leu Gly Leu Pro Ala Ala
            340                 345

<210> 72
<211> 345
<212> PRT
<213> Pseudomonas aeruginosa

<400> 72
Met Lys Leu Thr Pro Ala Gln Leu Ala Lys His Leu Gln Gly Pro Leu
1               5                   10                  15

Ala Pro Val Tyr Val Val Ser Gly Asp Glu Pro Leu Leu Cys Gln Glu
            20                  25                  30

Ala Cys Asp Ala Ile Arg Gln Ala Cys Arg Glu Arg Asp Phe Gly Glu
        35                  40                  45

Arg Gln Val Phe Asn Ala Glu Ala Asn Phe Asp Trp Gly Leu Leu Leu
    50                  55                  60

Glu Ala Gly Ala Ser Leu Ser Leu Phe Ala Glu Lys Arg Leu Ile Glu
65                  70                  75                  80

Leu Arg Leu Pro Ser Gly Lys Pro Gly Asp Lys Gly Ala Ala Ile Leu
                85                  90                  95

Gln Glu Tyr Leu Gln Arg Pro Pro Glu Asp Thr Val Leu Leu Leu Gly
            100                 105                 110

Leu Pro Lys Leu Asp Gly Ser Thr Gln Lys Thr Lys Trp Ala Lys Ala
        115                 120                 125

Leu Ile Asp Gly Asn Ala Ala Gln Phe Ile Gln Val Trp Pro Val Asp
    130                 135                 140

Val His Gln Leu Pro Gln Trp Ile Arg Gln Arg Leu Ser Gln Ala Gly
145                 150                 155                 160

Leu Ser Ala Ser Pro Glu Ala Leu Glu Leu Ile Ala Ala Arg Val Glu
                165                 170                 175

Gly Asn Leu Leu Ala Ala Ala Gln Glu Ile Glu Lys Leu Lys Leu Leu
            180                 185                 190

Ala Glu Gly Asn Gln Ile Asp Ala Ala Thr Val Gln Ala Ala Val Ala
        195                 200                 205

Asp Ser Ala Arg Phe Asp Val Phe Gly Leu Ile Asp Ala Ala Leu Gly
    210                 215                 220

Gly Glu Ala Ala His Ala Leu Arg Ile Leu Glu Gly Leu Arg Gly Glu
225                 230                 235                 240

Gly Ile Glu Pro Pro Val Ile Leu Trp Gly Leu Ala Arg Glu Ile Arg
                245                 250                 255

Leu Leu Ala Gly Leu Ser Gln Gln Tyr Gly Gln Gly Ile Pro Leu Glu
            260                 265                 270

Lys Ala Phe Ala Gln Ala Arg Pro Pro Val Trp Asp Lys Arg Arg Pro
        275                 280                 285

Leu Leu Thr Arg Ala Leu Gln Arg His Ser Ser Ser Arg Trp Asn Gln
    290                 295                 300

Met Leu Arg Asp Ala Gln Leu Ile Asp Ala Gln Ile Lys Gly Gln Ala
305                 310                 315                 320

Pro Gly Ser Pro Trp Ser Gly Leu Ser Leu Leu Ala Leu Ser Leu Ala
                325                 330                 335

Gly Gln Arg Leu Gly Leu Pro Ala Ala
            340                 345

<210> 73
<211> 966
<212> DNA
<213> Rickettsia prowazekii

<400> 73
atgcaatttt atttttcgca aataggtaga ttatttgaat taatagcagc cgggaagatt 60

agggctcttt tactatatgg tcctgataaa ggctatatag aaaagatttg tacatattta 120

atcaaaaact tgaatatgct acaatcttct atagaatatg aagatcttaa tattttatct 180

ttagatatat tacttaattc acctaatttt tttggtcaaa aagaattaat taaagtgaga 240

tcaattggaa attcactcga taagaattta aaaacaattc taagcagtga ttatataaat 300

ttccctgtat ttatagggga agatatgaat tctagtggga gtgttaaaaa gttttttgaa 360

acagaagaat atttagcggt agttgcttgt tatcatgacg atgaagcaaa aattgaacga 420

attattttag gtaaattagc aaaaactaat aaagttataa gtaaagaagc tatcacttac 480

ttaaaaactc atttaaaagg tgatcatgct ttaatttgta gcgaaatcaa taaattgata 540

ttttttgctc atgatgtaca tgaaattact ttaaaccatg ttttagaagt tataagtagt 600

gaaattacag caaatggtag taatttggca atatattttt caaaaaaaga ttatagtaat 660

tttctgcaag agcttgatat tttaaaaaaa caaaatatta atgaagtttt aattattaga 720

gctttaataa ggcattattt aaatttatat atagttttat cgaaagtaaa aaatggtgaa 780

tgtttggaaa ttgcaataaa atcattatca ccgccgattt tctatcataa tattaatgat 840

tttacaaaga tagtacatgc tctcagccta actgaatgta taaagacttt gaaactacta 900

caacaggcag aagtggatta taagttaaat cctgctagtt tcgatttatt tcaaagtata 960

ctctaa                                                            966

<210> 74
<211> 966
<212> DNA
<213> Rickettsia prowazekii

<220>
<221> CDS
<222> (1)..(966)

<400> 74
atg caa ttt tat ttt tcg caa ata ggt aga tta ttt gaa tta ata gca     48
Met Gln Phe Tyr Phe Ser Gln Ile Gly Arg Leu Phe Glu Leu Ile Ala
1               5                   10                  15

gcc ggg aag att agg gct ctt tta cta tat ggt cct gat aaa ggc tat     96
Ala Gly Lys Ile Arg Ala Leu Leu Leu Tyr Gly Pro Asp Lys Gly Tyr
            20                  25                  30

ata gaa aag att tgt aca tat tta atc aaa aac ttg aat atg cta caa     144
Ile Glu Lys Ile Cys Thr Tyr Leu Ile Lys Asn Leu Asn Met Leu Gln
        35                  40                  45

tct tct ata gaa tat gaa gat ctt aat att tta tct tta gat ata tta     192
Ser Ser Ile Glu Tyr Glu Asp Leu Asn Ile Leu Ser Leu Asp Ile Leu
    50                  55                  60

ctt aat tca cct aat ttt ttt ggt caa aaa gaa tta att aaa gtg aga     240
Leu Asn Ser Pro Asn Phe Phe Gly Gln Lys Glu Leu Ile Lys Val Arg
65                  70                  75                  80

tca att gga aat tca ctc gat aag aat tta aaa aca att cta agc agt     288
Ser Ile Gly Asn Ser Leu Asp Lys Asn Leu Lys Thr Ile Leu Ser Ser
                85                  90                  95

gat tat ata aat ttc cct gta ttt ata ggg gaa gat atg aat tct agt     336
Asp Tyr Ile Asn Phe Pro Val Phe Ile Gly Glu Asp Met Asn Ser Ser
            100                 105                 110

ggg agt gtt aaa aag ttt ttt gaa aca gaa gaa tat tta gcg gta gtt     384
Gly Ser Val Lys Lys Phe Phe Glu Thr Glu Glu Tyr Leu Ala Val Val
        115                 120                 125

gct tgt tat cat gac gat gaa gca aaa att gaa cga att att tta ggt     432
Ala Cys Tyr His Asp Asp Glu Ala Lys Ile Glu Arg Ile Ile Leu Gly
    130                 135                 140

aaa tta gca aaa act aat aaa gtt ata agt aaa gaa gct atc act tac     480
Lys Leu Ala Lys Thr Asn Lys Val Ile Ser Lys Glu Ala Ile Thr Tyr
145                 150                 155                 160

tta aaa act cat tta aaa ggt gat cat gct tta att tgt agc gaa atc     528
Leu Lys Thr His Leu Lys Gly Asp His Ala Leu Ile Cys Ser Glu Ile
                165                 170                 175

aat aaa ttg ata ttt ttt gct cat gat gta cat gaa att act tta aac     576
Asn Lys Leu Ile Phe Phe Ala His Asp Val His Glu Ile Thr Leu Asn
            180                 185                 190

cat gtt tta gaa gtt ata agt agt gaa att aca gca aat ggt agt aat     624
His Val Leu Glu Val Ile Ser Ser Glu Ile Thr Ala Asn Gly Ser Asn
        195                 200                 205

ttg gca ata tat ttt tca aaa aaa gat tat agt aat ttt ctg caa gag     672
Leu Ala Ile Tyr Phe Ser Lys Lys Asp Tyr Ser Asn Phe Leu Gln Glu
    210                 215                 220

ctt gat att tta aaa aaa caa aat att aat gaa gtt tta att att aga     720

Leu Asp Ile Leu Lys Lys Gln Asn Ile Asn Glu Val Leu Ile Ile Arg
225                 230                 235                 240

gct tta ata agg cat tat tta aat tta tat ata gtt tta tcg aaa gta     768
Ala Leu Ile Arg His Tyr Leu Asn Leu Tyr Ile Val Leu Ser Lys Val
                245                 250                 255

aaa aat ggt gaa tgt ttg gaa att gca ata aaa tca tta tca ccg ccg     816
Lys Asn Gly Glu Cys Leu Glu Ile Ala Ile Lys Ser Leu Ser Pro Pro
            260                 265                 270

att ttc tat cat aat att aat gat ttt aca aag ata gta cat gct ctc     864
Ile Phe Tyr His Asn Ile Asn Asp Phe Thr Lys Ile Val His Ala Leu
        275                 280                 285

agc cta act gaa tgt ata aag act ttg aaa cta cta caa cag gca gaa     912
Ser Leu Thr Glu Cys Ile Lys Thr Leu Lys Leu Leu Gln Gln Ala Glu
    290                 295                 300

gtg gat tat aag tta aat cct gct agt ttc gat tta ttt caa agt ata     960
Val Asp Tyr Lys Leu Asn Pro Ala Ser Phe Asp Leu Phe Gln Ser Ile
305                 310                 315                 320

ctc taa                                                             966
Leu


<210> 75
<211> 321
<212> PRT
<213> Rickettsia prowazekii

<400> 75
Met Gln Phe Tyr Phe Ser Gln Ile Gly Arg Leu Phe Glu Leu Ile Ala
1               5                   10                  15

Ala Gly Lys Ile Arg Ala Leu Leu Leu Tyr Gly Pro Asp Lys Gly Tyr
            20                  25                  30

Ile Glu Lys Ile Cys Thr Tyr Leu Ile Lys Asn Leu Asn Met Leu Gln
        35                  40                  45

Ser Ser Ile Glu Tyr Glu Asp Leu Asn Ile Leu Ser Leu Asp Ile Leu
    50                  55                  60

Leu Asn Ser Pro Asn Phe Phe Gly Gln Lys Glu Leu Ile Lys Val Arg
65                  70                  75                  80

Ser Ile Gly Asn Ser Leu Asp Lys Asn Leu Lys Thr Ile Leu Ser Ser
                85                  90                  95

Asp Tyr Ile Asn Phe Pro Val Phe Ile Gly Glu Asp Met Asn Ser Ser
            100                 105                 110

Gly Ser Val Lys Lys Phe Phe Glu Thr Glu Glu Tyr Leu Ala Val Val
        115                 120                 125

Ala Cys Tyr His Asp Asp Glu Ala Lys Ile Glu Arg Ile Ile Leu Gly
    130                 135                 140

Lys Leu Ala Lys Thr Asn Lys Val Ile Ser Lys Glu Ala Ile Thr Tyr
145                 150                 155                 160

Leu Lys Thr His Leu Lys Gly Asp His Ala Leu Ile Cys Ser Glu Ile
                165                 170                 175

Asn Lys Leu Ile Phe Phe Ala His Asp Val His Glu Ile Thr Leu Asn
            180                 185                 190

His Val Leu Glu Val Ile Ser Ser Glu Ile Thr Ala Asn Gly Ser Asn
        195                 200                 205

Leu Ala Ile Tyr Phe Ser Lys Lys Asp Tyr Ser Asn Phe Leu Gln Glu
    210                 215                 220

Leu Asp Ile Leu Lys Lys Gln Asn Ile Asn Glu Val Leu Ile Ile Arg
225                 230                 235                 240

Ala Leu Ile Arg His Tyr Leu Asn Leu Tyr Ile Val Leu Ser Lys Val
                245                 250                 255

Lys Asn Gly Glu Cys Leu Glu Ile Ala Ile Lys Ser Leu Ser Pro Pro
            260                 265                 270

Ile Phe Tyr His Asn Ile Asn Asp Phe Thr Lys Ile Val His Ala Leu
        275                 280                 285

Ser Leu Thr Glu Cys Ile Lys Thr Leu Lys Leu Leu Gln Gln Ala Glu
    290                 295                 300

Val Asp Tyr Lys Leu Asn Pro Ala Ser Phe Asp Leu Phe Gln Ser Ile
305                 310                 315                 320

Leu

<210> 76
<211> 1002
<212> DNA
<213> Synechocystis sp.

<400> 76
atgcccgtat atttctattg gggcgaagac caatttaccc tgcaccaagc agtcaaacaa 60

ctgcagaaac gttgtttaga tccccaatgg gaggccttta attttgaaaa aattccgggg 120

gaacaagctg atgctaccca acggggtctg gagcaagcct tgacaccccc ttttggtagt 180

ggcgatcgcc tggtgtgggt ggtggactcc accttaggac aaagttgcga cgatggtttg 240

ctggctaggt tgcaaaaaag tttgcccgcc attcccaccg actgccattt actattcaca 300

tccagtaaaa agctcgatcg ccgactgaaa tcgaccaaat atttggaagg taatgccact 360

atccgggaat ttgccctcat ttcaccctgg aatgtggacg ctctcatcca tcaaattcag 420

gcgatcgccc aggatctgca acttccctta gcaacggaaa cggaaggttt tttggcagaa 480

gcgttgggta atgacacgag gctaatttgg aatgagttgg gcaagctcaa actctatagc 540

gagagtcaaa cgggcccctt aacggtggcc caggtagaac agttggtcaa taccagcacc 600

caaaatagtt tgcaattagc ccaggcgatc cgggatggag aaacccccca ggcattgcaa 660

ttggtggcag acttattgct gacccacaac gaaccggctt taaaaatatt ggctaccctc 720

accggccagt ttcgtacttg gacattgatc aagcttgctt tggaggcagg ggaaaaagat 780

gacaaagcga tcgccgccca ggccgatctc agtaatccca aacgtttgta ttttttaaag 840

aaggaattgc ggggcattac cggcgggcaa ttgctcaaaa ccttacccct gttgttggaa 900

ttggaatatc aactaaagcg aggagcggac ccctgttcga gcctacaaac taaggtgatt 960

gaactatcat cattattcca gcccatacac cgtcgccgtt ga 1002

<210> 77
<211> 1002
<212> DNA
<213> Synechocystis sp.

<220>
<221> CDS
<222> (1)..(1002)

<400> 77
atg ccc gta tat ttc tat tgg ggc gaa gac caa ttt acc ctg cac caa 48
Met Pro Val Tyr Phe Tyr Trp Gly Glu Asp Gln Phe Thr Leu His Gln
1               5                   10                  15

gca gtc aaa caa ctg cag aaa cgt tgt tta gat ccc caa tgg gag gcc 96
Ala Val Lys Gln Leu Gln Lys Arg Cys Leu Asp Pro Gln Trp Glu Ala
            20                  25                  30

ttt aat ttt gaa aaa att ccg ggg gaa caa gct gat gct acc caa cgg 144
Phe Asn Phe Glu Lys Ile Pro Gly Glu Gln Ala Asp Ala Thr Gln Arg
        35                  40                  45

ggt ctg gag caa gcc ttg aca ccc cct ttt ggt agt ggc gat cgc ctg 192
Gly Leu Glu Gln Ala Leu Thr Pro Pro Phe Gly Ser Gly Asp Arg Leu
    50                  55                  60

gtg tgg gtg gtg gac tcc acc tta gga caa agt tgc gac gat ggt ttg 240
Val Trp Val Val Asp Ser Thr Leu Gly Gln Ser Cys Asp Asp Gly Leu
65                  70                  75                  80

ctg gct agg ttg caa aaa agt ttg ccc gcc att ccc acc gac tgc cat 288
Leu Ala Arg Leu Gln Lys Ser Leu Pro Ala Ile Pro Thr Asp Cys His
                85                  90                  95

tta cta ttc aca tcc agt aaa aag ctc gat cgc cga ctg aaa tcg acc 336
Leu Leu Phe Thr Ser Ser Lys Lys Leu Asp Arg Arg Leu Lys Ser Thr
            100                 105                 110

aaa tat ttg gaa ggt aat gcc act atc cgg gaa ttt gcc ctc att tca 384
Lys Tyr Leu Glu Gly Asn Ala Thr Ile Arg Glu Phe Ala Leu Ile Ser
        115                 120                 125

ccc tgg aat gtg gac gct ctc atc cat caa att cag gcg atc gcc cag 432
Pro Trp Asn Val Asp Ala Leu Ile His Gln Ile Gln Ala Ile Ala Gln
    130                 135                 140

gat ctg caa ctt ccc tta gca acg gaa acg gaa ggt ttt ttg gca gaa 480
Asp Leu Gln Leu Pro Leu Ala Thr Glu Thr Glu Gly Phe Leu Ala Glu
145                 150                 155                 160

gcg ttg ggt aat gac acg agg cta att tgg aat gag ttg ggc aag ctc 528
Ala Leu Gly Asn Asp Thr Arg Leu Ile Trp Asn Glu Leu Gly Lys Leu
                165                 170                 175

aaa ctc tat agc gag agt caa acg ggc ccc tta acg gtg gcc cag gta 576
Lys Leu Tyr Ser Glu Ser Gln Thr Gly Pro Leu Thr Val Ala Gln Val
            180                 185                 190

gaa cag ttg gtc aat acc agc acc caa aat agt ttg caa tta gcc cag 624
Glu Gln Leu Val Asn Thr Ser Thr Gln Asn Ser Leu Gln Leu Ala Gln
        195                 200                 205

gcg atc cgg gat gga gaa acc ccc cag gca ttg caa ttg gtg gca gac 672
Ala Ile Arg Asp Gly Glu Thr Pro Gln Ala Leu Gln Leu Val Ala Asp
    210                 215                 220

tta ttg ctg acc cac aac gaa ccg gct tta aaa ata ttg gct acc ctc 720

Leu Leu Leu Thr His Asn Glu Pro Ala Leu Lys Ile Leu Ala Thr Leu
225                 230                 235                 240

acc ggc cag ttt cgt act tgg aca ttg atc aag ctt gct ttg gag gca     768
Thr Gly Gln Phe Arg Thr Trp Thr Leu Ile Lys Leu Ala Leu Glu Ala
                245                 250                 255

ggg gaa aaa gat gac aaa gcg atc gcc gcc cag gcc gat ctc agt aat     816
Gly Glu Lys Asp Asp Lys Ala Ile Ala Ala Gln Ala Asp Leu Ser Asn
            260                 265                 270

ccc aaa cgt ttg tat ttt tta aag aag gaa ttg cgg ggc att acc ggc     864
Pro Lys Arg Leu Tyr Phe Leu Lys Lys Glu Leu Arg Gly Ile Thr Gly
        275                 280                 285

ggg caa ttg ctc aaa acc tta ccc ctg ttg ttg gaa ttg gaa tat caa     912
Gly Gln Leu Leu Lys Thr Leu Pro Leu Leu Leu Glu Leu Glu Tyr Gln
    290                 295                 300

cta aag cga gga gcg gac ccc tgt tcg agc cta caa act aag gtg att     960
Leu Lys Arg Gly Ala Asp Pro Cys Ser Ser Leu Gln Thr Lys Val Ile
305                 310                 315                 320

gaa cta tca tca tta ttc cag ccc ata cac cgt cgc cgt tga             1002
Glu Leu Ser Ser Leu Phe Gln Pro Ile His Arg Arg Arg
                325                 330

<210> 78
<211> 333
<212> PRT
<213> Synechocystis sp.

<400> 78
Met Pro Val Tyr Phe Tyr Trp Gly Glu Asp Gln Phe Thr Leu His Gln
  1               5                  10                  15

Ala Val Lys Gln Leu Gln Lys Arg Cys Leu Asp Pro Gln Trp Glu Ala
             20                  25                  30

Phe Asn Phe Glu Lys Ile Pro Gly Glu Gln Ala Asp Ala Thr Gln Arg
         35                  40                  45

Gly Leu Glu Gln Ala Leu Thr Pro Pro Phe Gly Ser Gly Asp Arg Leu
     50                  55                  60

Val Trp Val Val Asp Ser Thr Leu Gly Gln Ser Cys Asp Asp Gly Leu
 65                  70                  75                  80

Leu Ala Arg Leu Gln Lys Ser Leu Pro Ala Ile Pro Thr Asp Cys His
                 85                  90                  95

Leu Leu Phe Thr Ser Ser Lys Lys Leu Asp Arg Arg Leu Lys Ser Thr
            100                 105                 110

Lys Tyr Leu Glu Gly Asn Ala Thr Ile Arg Glu Phe Ala Leu Ile Ser
        115                 120                 125

Pro Trp Asn Val Asp Ala Leu Ile His Gln Ile Gln Ala Ile Ala Gln
    130                 135                 140

Asp Leu Gln Leu Pro Leu Ala Thr Glu Thr Glu Gly Phe Leu Ala Glu
145                 150                 155                 160

Ala Leu Gly Asn Asp Thr Arg Leu Ile Trp Asn Glu Leu Gly Lys Leu
                165                 170                 175

Lys Leu Tyr Ser Glu Ser Gln Thr Gly Pro Leu Thr Val Ala Gln Val

180                 185                 190

Glu Gln Leu Val Asn Thr Ser Thr Gln Asn Ser Leu Gln Leu Ala Gln
    195                 200                 205

Ala Ile Arg Asp Gly Glu Thr Pro Gln Ala Leu Gln Leu Val Ala Asp
    210                 215                 220

Leu Leu Leu Thr His Asn Glu Pro Ala Leu Lys Ile Leu Ala Thr Leu
225                 230                 235                 240

Thr Gly Gln Phe Arg Thr Trp Thr Leu Ile Lys Leu Ala Leu Glu Ala
                245                 250                 255

Gly Glu Lys Asp Asp Lys Ala Ile Ala Ala Gln Ala Asp Leu Ser Asn
            260                 265                 270

Pro Lys Arg Leu Tyr Phe Leu Lys Lys Glu Leu Arg Gly Ile Thr Gly
        275                 280                 285

Gly Gln Leu Leu Lys Thr Leu Pro Leu Leu Leu Glu Leu Glu Tyr Gln
    290                 295                 300

Leu Lys Arg Gly Ala Asp Pro Cys Ser Ser Leu Gln Thr Lys Val Ile
305                 310                 315                 320

Glu Leu Ser Ser Leu Phe Gln Pro Ile His Arg Arg Arg
                325                 330

<210> 79
<211> 990
<212> DNA
<213> Thermotoga maritima

<400> 79
atgagaggtg attgtatgcc agtcacgttt ctcacaggta ctgcagaaac tcagaaggaa 60

gaattgataa agaaactcct gaaggatggt aacgtggagt acataaggat ccatccggag 120

gatcccgaca agatcgattt cataaggtct ttactcagga caaagacgat cttttccaac 180

aagacgatca ttgacatcgt caatttcgat gagtggaaag cacaggagca gaagcgtctc 240

gttgaacttt tgaaaaacgt accggaagac gttcatatct tcatccgttc tcaaaaaaca 300

ggtggaaagg gagtagcgct ggagcttccg aagccatggg aaacggacaa gtggcttgag 360

tggatagaaa agcgcttcag ggagaatggt ttgctcatcg ataaagatgc ccttcagctg 420

tttttctcca aggttggaac gaacgacctg atcatagaaa gggagattga aaaactgaaa 480

gcttattccg aggacagaaa gataacggta gaagacgtgg aagaggtcgt ttttacctat 540

cagactccgg gatacgatga tttttgcttt gctgtttccg aaggaaaaag gaagctcgct 600

cactctcttc tgtcgcagct gtggaaaacc acagagtccg tggtgattgc cactgtcctt 660

gcgaatcact tcttggatct cttcaaaatc ctcgttcttg tgacaaagaa aagatactac 720

acctggcctg atgtgtccag ggtgtccaaa gagctgggaa ttcccgttcc tcgtgtggct 780

cgtttcctcg gtttctcctt taagacctgg aaattcaagg tgatgaacca cctcctctac 840

tacgatgtga agaaggttag aaagatactg agggatctct acgatctgga cagagccgtg 900

aaaagcgaag aagatccaaa accgttcttc cacgagttca tagaagaggt ggcactggat 960

gtatattctc ttcagagaga tgaagaataa                                    990

<210> 80
<211> 990
<212> DNA
<213> Thermotoga maritima

<220>
<221> CDS
<222> (1)..(990)

<400> 80
atg aga ggt gat tgt atg cca gtc acg ttt ctc aca ggt act gca gaa      48
Met Arg Gly Asp Cys Met Pro Val Thr Phe Leu Thr Gly Thr Ala Glu
1               5                   10                  15

act cag aag gaa gaa ttg ata aag aaa ctc ctg aag gat ggt aac gtg      96
Thr Gln Lys Glu Glu Leu Ile Lys Lys Leu Leu Lys Asp Gly Asn Val
            20                  25                  30

gag tac ata agg atc cat ccg gag gat ccc gac aag atc gat ttc ata     144
Glu Tyr Ile Arg Ile His Pro Glu Asp Pro Asp Lys Ile Asp Phe Ile
        35                  40                  45

agg tct tta ctc agg aca aag acg atc ttt tcc aac aag acg atc att     192
Arg Ser Leu Leu Arg Thr Lys Thr Ile Phe Ser Asn Lys Thr Ile Ile
    50                  55                  60

gac atc gtc aat ttc gat gag tgg aaa gca cag gag cag aag cgt ctc     240
Asp Ile Val Asn Phe Asp Glu Trp Lys Ala Gln Glu Gln Lys Arg Leu
65                  70                  75                  80

gtt gaa ctt ttg aaa aac gta ccg gaa gac gtt cat atc ttc atc cgt     288
Val Glu Leu Leu Lys Asn Val Pro Glu Asp Val His Ile Phe Ile Arg
                85                  90                  95

tct caa aaa aca ggt gga aag gga gta gcg ctg gag ctt ccg aag cca     336
Ser Gln Lys Thr Gly Gly Lys Gly Val Ala Leu Glu Leu Pro Lys Pro
            100                 105                 110

tgg gaa acg gac aag tgg ctt gag tgg ata gaa aag cgc ttc agg gag     384
Trp Glu Thr Asp Lys Trp Leu Glu Trp Ile Glu Lys Arg Phe Arg Glu
        115                 120                 125

aat ggt ttg ctc atc gat aaa gat gcc ctt cag ctg ttt ttc tcc aag     432
Asn Gly Leu Leu Ile Asp Lys Asp Ala Leu Gln Leu Phe Phe Ser Lys
    130                 135                 140

gtt gga acg aac gac ctg atc ata gaa agg gag att gaa aaa ctg aaa     480
Val Gly Thr Asn Asp Leu Ile Ile Glu Arg Glu Ile Glu Lys Leu Lys
145                 150                 155                 160

gct tat tcc gag gac aga aag ata acg gta gaa gac gtg gaa gag gtc     528
Ala Tyr Ser Glu Asp Arg Lys Ile Thr Val Glu Asp Val Glu Glu Val
                165                 170                 175

gtt ttt acc tat cag act ccg gga tac gat gat ttt tgc ttt gct gtt     576
Val Phe Thr Tyr Gln Thr Pro Gly Tyr Asp Asp Phe Cys Phe Ala Val
            180                 185                 190

tcc gaa gga aaa agg aag ctc gct cac tct ctt ctg tcg cag ctg tgg     624
Ser Glu Gly Lys Arg Lys Leu Ala His Ser Leu Leu Ser Gln Leu Trp
        195                 200                 205

aaa acc aca gag tcc gtg gtg att gcc act gtc ctt gcg aat cac ttc     672
Lys Thr Thr Glu Ser Val Val Ile Ala Thr Val Leu Ala Asn His Phe
    210                 215                 220

ttg gat ctc ttc aaa atc ctc gtt ctt gtg aca aag aaa aga tac tac     720
Leu Asp Leu Phe Lys Ile Leu Val Leu Val Thr Lys Lys Arg Tyr Tyr
225                 230                 235                 240

acc tgg cct gat gtg tcc agg gtg tcc aaa gag ctg gga att ccc gtt     768
Thr Trp Pro Asp Val Ser Arg Val Ser Lys Glu Leu Gly Ile Pro Val
                245                 250                 255

cct cgt gtg gct cgt ttc ctc ggt ttc tcc ttt aag acc tgg aaa ttc     816
Pro Arg Val Ala Arg Phe Leu Gly Phe Ser Phe Lys Thr Trp Lys Phe
            260                 265                 270

aag gtg atg aac cac ctc ctc tac tac gat gtg aag aag gtt aga aag     864
Lys Val Met Asn His Leu Leu Tyr Tyr Asp Val Lys Lys Val Arg Lys
        275                 280                 285

ata ctg agg gat ctc tac gat ctg gac aga gcc gtg aaa agc gaa gaa     912
Ile Leu Arg Asp Leu Tyr Asp Leu Asp Arg Ala Val Lys Ser Glu Glu
    290                 295                 300

gat cca aaa ccg ttc ttc cac gag ttc ata gaa gag gtg gca ctg gat     960
Asp Pro Lys Pro Phe Phe His Glu Phe Ile Glu Glu Val Ala Leu Asp
305                 310                 315                 320

gta tat tct ctt cag aga gat gaa gaa taa                             990
Val Tyr Ser Leu Gln Arg Asp Glu Glu
                325                 330

<210> 81
<211> 329
<212> PRT
<213> Thermotoga maritima

<400> 81
Met Arg Gly Asp Cys Met Pro Val Thr Phe Leu Thr Gly Thr Ala Glu
1               5                   10                  15

Thr Gln Lys Glu Glu Leu Ile Lys Lys Leu Leu Lys Asp Gly Asn Val
            20                  25                  30

Glu Tyr Ile Arg Ile His Pro Glu Asp Pro Asp Lys Ile Asp Phe Ile
        35                  40                  45

Arg Ser Leu Leu Arg Thr Lys Thr Ile Phe Ser Asn Lys Thr Ile Ile
    50                  55                  60

Asp Ile Val Asn Phe Asp Glu Trp Lys Ala Gln Glu Gln Lys Arg Leu
65                  70                  75                  80

Val Glu Leu Leu Lys Asn Val Pro Glu Asp Val His Ile Phe Ile Arg
                85                  90                  95

Ser Gln Lys Thr Gly Gly Lys Gly Val Ala Leu Glu Leu Pro Lys Pro
            100                 105                 110

Trp Glu Thr Asp Lys Trp Leu Glu Trp Ile Glu Lys Arg Phe Arg Glu
        115                 120                 125

Asn Gly Leu Leu Ile Asp Lys Asp Ala Leu Gln Leu Phe Phe Ser Lys
    130                 135                 140

Val Gly Thr Asn Asp Leu Ile Ile Glu Arg Glu Ile Glu Lys Leu Lys
145                 150                 155                 160

Ala Tyr Ser Glu Asp Arg Lys Ile Thr Val Glu Asp Val Glu Glu Val
                165                 170                 175

Val Phe Thr Tyr Gln Thr Pro Gly Tyr Asp Asp Phe Cys Phe Ala Val
            180                 185                 190

Ser Glu Gly Lys Arg Lys Leu Ala His Ser Leu Leu Ser Gln Leu Trp
        195                 200                 205

Lys Thr Thr Glu Ser Val Val Ile Ala Thr Val Leu Ala Asn His Phe
    210                 215                 220

Leu Asp Leu Phe Lys Ile Leu Val Leu Val Thr Lys Lys Arg Tyr Tyr
225                 230                 235                 240

Thr Trp Pro Asp Val Ser Arg Val Ser Lys Glu Leu Gly Ile Pro Val
                245                 250                 255

Pro Arg Val Ala Arg Phe Leu Gly Phe Ser Phe Lys Thr Trp Lys Phe
            260                 265                 270

Lys Val Met Asn His Leu Leu Tyr Tyr Asp Val Lys Lys Val Arg Lys
        275                 280                 285

Ile Leu Arg Asp Leu Tyr Asp Leu Asp Arg Ala Val Lys Ser Glu Glu
    290                 295                 300

Asp Pro Lys Pro Phe Phe His Glu Phe Ile Glu Glu Val Ala Leu Asp
305                 310                 315                 320

Val Tyr Ser Leu Gln Arg Asp Glu Glu
                325

<210> 82
<211> 765
<212> DNA
<213> Treponema pallidum

<400> 82
   atgtccgtct ggctttttac cggacctgaa ataggggagc gagatagtgc agttcaggag 60
   gtgtgcgcgc gtgcacaagc gcaagggacg gtggacgtac atcggctcta tgcacacgaa 120
   acgcctgtgg cagatcttgt agatctcctg agaactcgtg cgctgtttgc agatgcagtc 180
   tgcgtggtac tgtacaacgc agaggttatt aagaagtgtg acgaagtgca cgtgcttaca 240
   gaatggataa aggatggcgg ctcgcgcgct gacgtgtttt tggtactgat ttccgatagt 300
   gtcagtatac ataagcgtat agaacagaat atttctcccg tgcataagcg tgtttttggg 360
   gagttgtttg aaaataaaaa acacgcgtgg gtgcagcgat ttttttttca gcatgaaatg 420
   cgcattgaac aggaggctat cgaatctctt cttgagttgg tggagaacaa cactcgtgcg 480
   ctcaaaactg tttgtacgca gctttctctt ttttttgaaa aaggacgccg catcactgcg 540
   cacgacatta gttcgttgct ggtgcacaca aaagaagaga catcgtttac cttattcgat 600
   gcgctgtcca aacgagatct tgagcattca cttatgattc tcaacacgct gctttgttcg 660
   aaagacgttg cccctgtaca aatccttgca ggactgctta cgcgttccgt cgccttgcgc 720
   attggcacca tataccggag gacaaaacgc accctgcaac gctaa 765

<210> 83
<211> 765
<212> DNA

<213> Treponema pallidum

<220>
<221> CDS
<222> (1)..(765)

<400> 83
atg tcc gtc tgg ctt ttt acc gga cct gaa ata ggg gag cga gat agt     48
Met Ser Val Trp Leu Phe Thr Gly Pro Glu Ile Gly Glu Arg Asp Ser
1               5                   10                  15

gca gtt cag gag gtg tgc gcg cgt gca caa gcg caa ggg acg gtg gac     96
Ala Val Gln Glu Val Cys Ala Arg Ala Gln Ala Gln Gly Thr Val Asp
            20                  25                  30

gta cat cgg ctc tat gca cac gaa acg cct gtg gca gat ctt gta gat     144
Val His Arg Leu Tyr Ala His Glu Thr Pro Val Ala Asp Leu Val Asp
        35                  40                  45

ctc ctg aga act cgt gcg ctg ttt gca gat gca gtc tgc gtg gta ctg     192
Leu Leu Arg Thr Arg Ala Leu Phe Ala Asp Ala Val Cys Val Val Leu
    50                  55                  60

tac aac gca gag gtt att aag aag tgt gac gaa gtg cac gtg ctt aca     240
Tyr Asn Ala Glu Val Ile Lys Lys Cys Asp Glu Val His Val Leu Thr
65                  70                  75                  80

gaa tgg ata aag gat ggc ggc tcg cgc gct gac gtg ttt ttg gta ctg     288
Glu Trp Ile Lys Asp Gly Gly Ser Arg Ala Asp Val Phe Leu Val Leu
                85                  90                  95

att tcc gat agt gtc agt ata cat aag cgt ata gaa cag aat att tct     336
Ile Ser Asp Ser Val Ser Ile His Lys Arg Ile Glu Gln Asn Ile Ser
            100                 105                 110

ccc gtg cat aag cgt gtt ttt tgg gag ttg ttt gaa aat aaa aaa cac     384
Pro Val His Lys Arg Val Phe Trp Glu Leu Phe Glu Asn Lys Lys His
        115                 120                 125

gcg tgg gtg cag cga ttt ttt ttt cag cat gaa atg cgc att gaa cag     432
Ala Trp Val Gln Arg Phe Phe Phe Gln His Glu Met Arg Ile Glu Gln
    130                 135                 140

gag gct atc gaa tct ctt ctt gag ttg gtg gag aac aac act cgt gcg     480
Glu Ala Ile Glu Ser Leu Leu Glu Leu Val Glu Asn Asn Thr Arg Ala
145                 150                 155                 160

ctc aaa act gtt tgt acg cag ctt tct ctt ttt ttt gaa aaa gga cgc     528
Leu Lys Thr Val Cys Thr Gln Leu Ser Leu Phe Phe Glu Lys Gly Arg
                165                 170                 175

cgc atc act gcg cac gac att agt tcg ttg ctg gtg cac aca aaa gaa     576
Arg Ile Thr Ala His Asp Ile Ser Ser Leu Leu Val His Thr Lys Glu
            180                 185                 190

gag aca tcg ttt acc tta ttc gat gcg ctg tcc aaa cga gat ctt gag     624
Glu Thr Ser Phe Thr Leu Phe Asp Ala Leu Ser Lys Arg Asp Leu Glu
        195                 200                 205

cat tca ctt atg att ctc aac acg ctg ctt tgt tcg aaa gac gtt gcc     672
His Ser Leu Met Ile Leu Asn Thr Leu Leu Cys Ser Lys Asp Val Ala
    210                 215                 220

cct gta caa atc ctt gca gga ctg ctt acg cgt tcc gtc gcc ttg cgc     720
Pro Val Gln Ile Leu Ala Gly Leu Leu Thr Arg Ser Val Ala Leu Arg
225                 230                 235                 240

att ggc acc ata tac cgg agg aca aaa cgc acc ctg caa cgc taa         765

Ile Gly Thr Ile Tyr Arg Arg Thr Lys Arg Thr Leu Gln Arg
                245                 250                 255

<210> 84
<211> 254
<212> PRT
<213> Treponema pallidum

<400> 84
Met Ser Val Trp Leu Phe Thr Gly Pro Glu Ile Gly Glu Arg Asp Ser
1               5                   10                  15

Ala Val Gln Glu Val Cys Ala Arg Ala Gln Ala Gln Gly Thr Val Asp
            20                  25                  30

Val His Arg Leu Tyr Ala His Glu Thr Pro Val Ala Asp Leu Val Asp
        35                  40                  45

Leu Leu Arg Thr Arg Ala Leu Phe Ala Asp Ala Val Cys Val Val Leu
    50                  55                  60

Tyr Asn Ala Glu Val Ile Lys Lys Cys Asp Glu Val His Val Leu Thr
65                  70                  75                  80

Glu Trp Ile Lys Asp Gly Gly Ser Arg Ala Asp Val Phe Leu Val Leu
                85                  90                  95

Ile Ser Asp Ser Val Ser Ile His Lys Arg Ile Glu Gln Asn Ile Ser
            100                 105                 110

Pro Val His Lys Arg Val Phe Trp Glu Leu Phe Glu Asn Lys Lys His
        115                 120                 125

Ala Trp Val Gln Arg Phe Phe Phe Gln His Glu Met Arg Ile Glu Gln
    130                 135                 140

Glu Ala Ile Glu Ser Leu Leu Glu Leu Val Glu Asn Asn Thr Arg Ala
145                 150                 155                 160

Leu Lys Thr Val Cys Thr Gln Leu Ser Leu Phe Phe Glu Lys Gly Arg
                165                 170                 175

Arg Ile Thr Ala His Asp Ile Ser Ser Leu Leu Val His Thr Lys Glu
            180                 185                 190

Glu Thr Ser Phe Thr Leu Phe Asp Ala Leu Ser Lys Arg Asp Leu Glu
        195                 200                 205

His Ser Leu Met Ile Leu Asn Thr Leu Leu Cys Ser Lys Asp Val Ala
    210                 215                 220

Pro Val Gln Ile Leu Ala Gly Leu Leu Thr Arg Ser Val Ala Leu Arg
225                 230                 235                 240

Ile Gly Thr Ile Tyr Arg Arg Thr Lys Arg Thr Leu Gln Arg
                245                 250

<210> 85
<211> 945
<212> DNA
<213> Ureaplasma urealyticum

<400> 85
atgaacttaa taaataaaga actttttta attactggtg acaatcgttt aaaaatggat 60

caaaaaatta atgaaattgc gacctctttt gatgaattag ttaaaattaa tgatcaagaa 120

atttcattaa tttcttttaa aaatcttatt gaacaagatg atttattcaa tagtaataag 180

atctacttat ttaaaaacgt taactggttc gaaaatttag aacatttaaa aaatgttagt 240

gatttaattg attatttttt taataatcat gttgctatta ttattacaat tgaatctaca 300

aaaatttcta cagcaaaaaa aattcaagaa acaattgcaa aatttaatca tcaaattact 360

tttatgacat atacaaatga aaatgctatg gctttcttaa aagaagagtt aaatcagcgt 420

aatttatcat taaataaatc tataatgcaa acgattattc aaaaaacaaa ttttaatatt 480

aattttctta ataatgaatt agataaaata gaattaatta atgatttttt aaaaaatcat 540

ggtatgtttg atattaataa ttttatatgt gattatggtg aatatcaaat ttttagttta 600

ttaaatcttt tgtatcaaca taaaataaat gaatcaataa atttaattaa taaaatgctc 660

attgacaaaa ttgatgaact cacaataatt aatatgttag cgacaataat gagtacacat 720

tacttaatta aattattaca tgaaaaaaaa tataacaaaa acgttatatc tagctcttta 780

aatcaaaaac cttatattat tgatttaaat ttaaaattaa ttagaaatta ttcagctaaa 840

tttttactta aaaaaatgta tgatttatta caattagaaa ttaaagtcaa ggaaaataaa 900

attgataaat attttggatt aattaattgg gttttgaatt tttaa 945

<210> 86
<211> 945
<212> DNA
<213> Ureaplasma urealyticum

<220>
<221> CDS
<222> (1)..(945)

<400> 86
atg aac tta ata aat aaa gaa ctt ttt tta att act ggt gac aat cgt 48
Met Asn Leu Ile Asn Lys Glu Leu Phe Leu Ile Thr Gly Asp Asn Arg
1               5                   10                  15

tta aaa atg gat caa aaa att aat gaa att gcg acc tct ttt gat gaa 96
Leu Lys Met Asp Gln Lys Ile Asn Glu Ile Ala Thr Ser Phe Asp Glu
            20                  25                  30

tta gtt aaa att aat gat caa gaa att tca tta att tct ttt aaa aat 144
Leu Val Lys Ile Asn Asp Gln Glu Ile Ser Leu Ile Ser Phe Lys Asn
        35                  40                  45

ctt att gaa caa gat gat tta ttc aat agt aat aag atc tac tta ttt 192
Leu Ile Glu Gln Asp Asp Leu Phe Asn Ser Asn Lys Ile Tyr Leu Phe
    50                  55                  60

aaa aac gtt aac tgg ttc gaa aat tta gaa cat tta aaa aat gtt agt 240
Lys Asn Val Asn Trp Phe Glu Asn Leu Glu His Leu Lys Asn Val Ser
65                  70                  75                  80

gat tta att gat tat ttt ttt aat aat cat gtt gct att att att aca 288
Asp Leu Ile Asp Tyr Phe Phe Asn Asn His Val Ala Ile Ile Ile Thr
                85                  90                  95

att gaa tct aca aaa att tct aca gca aaa aaa att caa gaa aca att 336
Ile Glu Ser Thr Lys Ile Ser Thr Ala Lys Lys Ile Gln Glu Thr Ile
            100                 105                 110

gca aaa ttt aat cat caa att act ttt atg aca tat aca aat gaa aat     384
Ala Lys Phe Asn His Gln Ile Thr Phe Met Thr Tyr Thr Asn Glu Asn
        115                 120                 125

gct atg gct ttc tta aaa gaa gag tta aat cag cgt aat tta tca tta     432
Ala Met Ala Phe Leu Lys Glu Glu Leu Asn Gln Arg Asn Leu Ser Leu
    130                 135                 140

aat aaa tct ata atg caa acg att att caa aaa aca aat ttt aat att     480
Asn Lys Ser Ile Met Gln Thr Ile Ile Gln Lys Thr Asn Phe Asn Ile
145                 150                 155                 160

aat ttt ctt aat aat gaa tta gat aaa ata gaa tta att aat gat ttt     528
Asn Phe Leu Asn Asn Glu Leu Asp Lys Ile Glu Leu Ile Asn Asp Phe
                165                 170                 175

tta aaa aat cat ggt atg ttt gat att aat aat ttt ata tgt gat tat     576
Leu Lys Asn His Gly Met Phe Asp Ile Asn Asn Phe Ile Cys Asp Tyr
            180                 185                 190

ggt gaa tat caa att ttt agt tta tta aat ctt ttg tat caa cat aaa     624
Gly Glu Tyr Gln Ile Phe Ser Leu Leu Asn Leu Leu Tyr Gln His Lys
        195                 200                 205

ata aat gaa tca ata aat tta att aat aaa atg ctc att gac aaa att     672
Ile Asn Glu Ser Ile Asn Leu Ile Asn Lys Met Leu Ile Asp Lys Ile
    210                 215                 220

gat gaa ctc aca ata att aat atg tta gcg aca ata atg agt aca cat     720
Asp Glu Leu Thr Ile Ile Asn Met Leu Ala Thr Ile Met Ser Thr His
225                 230                 235                 240

tac tta att aaa tta tta cat gaa aaa aaa tat aac aaa aac gtt ata     768
Tyr Leu Ile Lys Leu Leu His Glu Lys Lys Tyr Asn Lys Asn Val Ile
                245                 250                 255

tct agc tct tta aat caa aaa cct tat att att gat tta aat tta aaa     816
Ser Ser Ser Leu Asn Gln Lys Pro Tyr Ile Ile Asp Leu Asn Leu Lys
            260                 265                 270

tta att aga aat tat tca gct aaa ttt tta ctt aaa aaa atg tat gat     864
Leu Ile Arg Asn Tyr Ser Ala Lys Phe Leu Leu Lys Lys Met Tyr Asp
        275                 280                 285

tta tta caa tta gaa att aaa gtc aag gaa aat aaa att gat aaa tat     912
Leu Leu Gln Leu Glu Ile Lys Val Lys Glu Asn Lys Ile Asp Lys Tyr
    290                 295                 300

ttt gga tta att aat tgg gtt ttg aat ttt taa                         945
Phe Gly Leu Ile Asn Trp Val Leu Asn Phe
305                 310                 315

<210> 87
<211> 314
<212> PRT
<213> Ureaplasma urealyticum

<400> 87
Met Asn Leu Ile Asn Lys Glu Leu Phe Leu Ile Thr Gly Asp Asn Arg
1               5                   10                  15

Leu Lys Met Asp Gln Lys Ile Asn Glu Ile Ala Thr Ser Phe Asp Glu
            20                  25                  30

Leu Val Lys Ile Asn Asp Gln Glu Ile Ser Leu Ile Ser Phe Lys Asn
        35                  40                  45

Leu Ile Glu Gln Asp Asp Leu Phe Asn Ser Asn Lys Ile Tyr Leu Phe
    50                  55                  60

Lys Asn Val Asn Trp Phe Glu Asn Leu Glu His Leu Lys Asn Val Ser
65                  70                  75                  80

Asp Leu Ile Asp Tyr Phe Phe Asn Asn His Val Ala Ile Ile Ile Thr
                85                  90                  95

Ile Glu Ser Thr Lys Ile Ser Thr Ala Lys Lys Ile Gln Glu Thr Ile
            100                 105                 110

Ala Lys Phe Asn His Gln Ile Thr Phe Met Thr Tyr Thr Asn Glu Asn
        115                 120                 125

Ala Met Ala Phe Leu Lys Glu Glu Leu Asn Gln Arg Asn Leu Ser Leu
    130                 135                 140

Asn Lys Ser Ile Met Gln Thr Ile Ile Gln Lys Thr Asn Phe Asn Ile
145                 150                 155                 160

Asn Phe Leu Asn Asn Glu Leu Asp Lys Ile Glu Leu Ile Asn Asp Phe
                165                 170                 175

Leu Lys Asn His Gly Met Phe Asp Ile Asn Asn Phe Ile Cys Asp Tyr
            180                 185                 190

Gly Glu Tyr Gln Ile Phe Ser Leu Leu Asn Leu Leu Tyr Gln His Lys
        195                 200                 205

Ile Asn Glu Ser Ile Asn Leu Ile Asn Lys Met Leu Ile Asp Lys Ile
    210                 215                 220

Asp Glu Leu Thr Ile Ile Asn Met Leu Ala Thr Ile Met Ser Thr His
225                 230                 235                 240

Tyr Leu Ile Lys Leu Leu His Glu Lys Lys Tyr Asn Lys Asn Val Ile
                245                 250                 255

Ser Ser Ser Leu Asn Gln Lys Pro Tyr Ile Ile Asp Leu Asn Leu Lys
            260                 265                 270

Leu Ile Arg Asn Tyr Ser Ala Lys Phe Leu Leu Lys Lys Met Tyr Asp
        275                 280                 285

Leu Leu Gln Leu Glu Ile Lys Val Lys Glu Asn Lys Ile Asp Lys Tyr
    290                 295                 300

Phe Gly Leu Ile Asn Trp Val Leu Asn Phe
305                 310

<210> 88
<211> 1029
<212> DNA
<213> Vibrio cholerae

<400> 88
atgcgcattt acgctgaaaa actggctgaa tcactgcaca aaacgctcta tccgatttac 60

ctcgtattcg gtaatgagcc actgctgctc caagaagcca aaaccgccat cgaaaaaacg 120

gcacaagcgc aaggtttttt ggaaaaacac cgatttagcg cggatgccgg gctcgattgg 180

aacgcggtgt atgactgttg ccaagcactg agtctgtttt catctcgcca acttatcgaa 240

attgagatcc cagaatctgg ggtcaatgct caaacggcaa aggaacttag cgcactggta 300

ggccaacttc accaagacat tctcttgctg gtgattggcc ccaaactgac taaggcgcaa 360

gagaacgcag cttggttcaa aacgcttgcg cagcaagcct gttgggttaa ctgcctcacg 420

ccagaattga gccgtttacc tcagtttgtt cagcagcgct gttttgccct cggtttaaaa 480

ccggatgccg aagcggtaca aatgttggcg cagtggcatg aaggtaatct ctttgcatta 540

gcccagagtc tcgaaaaatt agcgttactc taccccgatg gtctactgac tttagtgcgc 600

ttggaagagt cgctgagccg ccataaccat ttcacgcctt atcactggat ggatgcgcta 660

ttagaaggca aagccaaccg tgctcagcgt attttgcgcc agcttatgct cgaagagagt 720

gagccgatta ttctgatccg caccgcgcaa aaagagctga cccaactgct taaatggcaa 780

caagagcgcc aacagctggg taatttgggg agtctttttg atcgctatcg tgtctggcag 840

aatcgaagac cactctactc tgccgcttta cagcgcttac caagtcgcgc cctacttcgg 900

ttggttggaa tattaactca ggctgaatta ctggcaaaaa cgcaatacga acagccagta 960

tggccaatct tgcagcaact aagccttgaa tgctgcaatc ctcaggcaaa cctacctttg 1020

cctcactaa 1029

<210> 89
<211> 1029
<212> DNA
<213> Vibrio cholerae

<220>
<221> CDS
<222> (1)..(1029)

<400> 89
atg cgc att tac gct gaa aaa ctg gct gaa tca ctg cac aaa acg ctc 48
Met Arg Ile Tyr Ala Glu Lys Leu Ala Glu Ser Leu His Lys Thr Leu
1               5                   10                  15

tat ccg att tac ctc gta ttc ggt aat gag cca ctg ctg ctc caa gaa 96
Tyr Pro Ile Tyr Leu Val Phe Gly Asn Glu Pro Leu Leu Leu Gln Glu
            20                  25                  30

gcc aaa acc gcc atc gaa aaa acg gca caa gcg caa ggt ttt ttg gaa 144
Ala Lys Thr Ala Ile Glu Lys Thr Ala Gln Ala Gln Gly Phe Leu Glu
        35                  40                  45

aaa cac cga ttt agc gcg gat gcc ggg ctc gat tgg aac gcg gtg tat 192
Lys His Arg Phe Ser Ala Asp Ala Gly Leu Asp Trp Asn Ala Val Tyr
    50                  55                  60

gac tgt tgc caa gca ctg agt ctg ttt tca tct cgc caa ctt atc gaa 240
Asp Cys Cys Gln Ala Leu Ser Leu Phe Ser Ser Arg Gln Leu Ile Glu
65                  70                  75                  80

att gag atc cca gaa tct ggg gtc aat gct caa acg gca aag gaa ctt 288
Ile Glu Ile Pro Glu Ser Gly Val Asn Ala Gln Thr Ala Lys Glu Leu
                85                  90                  95

agc gca ctg gta ggc caa ctt cac caa gac att ctc ttg ctg gtg att 336
Ser Ala Leu Val Gly Gln Leu His Gln Asp Ile Leu Leu Leu Val Ile
            100                 105                 110

ggc ccc aaa ctg act aag gcg caa gag aac gca gct tgg ttc aaa acg 384
Gly Pro Lys Leu Thr Lys Ala Gln Glu Asn Ala Ala Trp Phe Lys Thr
        115                 120                 125

ctt gcg cag caa gcc tgt tgg gtt aac tgc ctc acg cca gaa ttg agc 432
Leu Ala Gln Gln Ala Cys Trp Val Asn Cys Leu Thr Pro Glu Leu Ser
    130                 135                 140

cgt tta cct cag ttt gtt cag cag cgc tgt ttt gcc ctc ggt tta aaa 480
Arg Leu Pro Gln Phe Val Gln Gln Arg Cys Phe Ala Leu Gly Leu Lys
145                 150                 155                 160

ccg gat gcc gaa gcg gta caa atg ttg gcg cag tgg cat gaa ggt aat 528
Pro Asp Ala Glu Ala Val Gln Met Leu Ala Gln Trp His Glu Gly Asn
                165                 170                 175

ctc ttt gca tta gcc cag agt ctc gaa aaa tta gcg tta ctc tac ccc 576
Leu Phe Ala Leu Ala Gln Ser Leu Glu Lys Leu Ala Leu Leu Tyr Pro
            180                 185                 190

gat ggt cta ctg act tta gtg cgc ttg gaa gag tcg ctg agc cgc cat 624
Asp Gly Leu Leu Thr Leu Val Arg Leu Glu Glu Ser Leu Ser Arg His
        195                 200                 205

aac cat ttc acg cct tat cac tgg atg gat gcg cta tta gaa ggc aaa 672
Asn His Phe Thr Pro Tyr His Trp Met Asp Ala Leu Leu Glu Gly Lys
    210                 215                 220

gcc aac cgt gct cag cgt att ttg cgc cag ctt atg ctc gaa gag agt 720
Ala Asn Arg Ala Gln Arg Ile Leu Arg Gln Leu Met Leu Glu Glu Ser
225                 230                 235                 240

gag ccg att att ctg atc cgc acc gcg caa aaa gag ctg acc caa ctg 768
Glu Pro Ile Ile Leu Ile Arg Thr Ala Gln Lys Glu Leu Thr Gln Leu
                245                 250                 255

ctt aaa tgg caa caa gag cgc caa cag ctg ggt aat ttg ggg agt ctt 816
Leu Lys Trp Gln Gln Glu Arg Gln Gln Leu Gly Asn Leu Gly Ser Leu
            260                 265                 270

ttt gat cgc tat cgt gtc tgg cag aat cga aga cca ctc tac tct gcc 864
Phe Asp Arg Tyr Arg Val Trp Gln Asn Arg Arg Pro Leu Tyr Ser Ala
        275                 280                 285

gct tta cag cgc tta cca agt cgc gcc cta ctt cgg ttg gtt gga ata 912
Ala Leu Gln Arg Leu Pro Ser Arg Ala Leu Leu Arg Leu Val Gly Ile
    290                 295                 300

tta act cag gct gaa tta ctg gca aaa acg caa tac gaa cag cca gta 960
Leu Thr Gln Ala Glu Leu Leu Ala Lys Thr Gln Tyr Glu Gln Pro Val
305                 310                 315                 320

tgg cca atc ttg cag caa cta agc ctt gaa tgc tgc aat cct cag gca 1008
Trp Pro Ile Leu Gln Gln Leu Ser Leu Glu Cys Cys Asn Pro Gln Ala
                325                 330                 335

aac cta cct ttg cct cac taa 1029
Asn Leu Pro Leu Pro His
            340

<210> 90
<211> 342
<212> PRT
<213> Vibrio cholerae

<400> 90
Met Arg Ile Tyr Ala Glu Lys Leu Ala Glu Ser Leu His Lys Thr Leu
1               5                   10                  15

Tyr Pro Ile Tyr Leu Val Phe Gly Asn Glu Pro Leu Leu Leu Gln Glu

20                  25                  30

Ala Lys Thr Ala Ile Glu Lys Thr Ala Gln Ala Gln Gly Phe Leu Glu
        35                  40                  45

Lys His Arg Phe Ser Ala Asp Ala Gly Leu Asp Trp Asn Ala Val Tyr
    50                  55                  60

Asp Cys Cys Gln Ala Leu Ser Leu Phe Ser Ser Arg Gln Leu Ile Glu
65                  70                  75                  80

Ile Glu Ile Pro Glu Ser Gly Val Asn Ala Gln Thr Ala Lys Glu Leu
                85                  90                  95

Ser Ala Leu Val Gly Gln Leu His Gln Asp Ile Leu Leu Leu Val Ile
            100                 105                 110

Gly Pro Lys Leu Thr Lys Ala Gln Glu Asn Ala Ala Trp Phe Lys Thr
        115                 120                 125

Leu Ala Gln Gln Ala Cys Trp Val Asn Cys Leu Thr Pro Glu Leu Ser
    130                 135                 140

Arg Leu Pro Gln Phe Val Gln Gln Arg Cys Phe Ala Leu Gly Leu Lys
145                 150                 155                 160

Pro Asp Ala Glu Ala Val Gln Met Leu Ala Gln Trp His Glu Gly Asn
                165                 170                 175

Leu Phe Ala Leu Ala Gln Ser Leu Glu Lys Leu Ala Leu Leu Tyr Pro
            180                 185                 190

Asp Gly Leu Leu Thr Leu Val Arg Leu Glu Glu Ser Leu Ser Arg His
        195                 200                 205

Asn His Phe Thr Pro Tyr His Trp Met Asp Ala Leu Leu Glu Gly Lys
    210                 215                 220

Ala Asn Arg Ala Gln Arg Ile Leu Arg Gln Leu Met Leu Glu Glu Ser
225                 230                 235                 240

Glu Pro Ile Ile Leu Ile Arg Thr Ala Gln Lys Glu Leu Thr Gln Leu
                245                 250                 255

Leu Lys Trp Gln Gln Glu Arg Gln Gln Leu Gly Asn Leu Gly Ser Leu
            260                 265                 270

Phe Asp Arg Tyr Arg Val Trp Gln Asn Arg Arg Pro Leu Tyr Ser Ala
        275                 280                 285

Ala Leu Gln Arg Leu Pro Ser Arg Ala Leu Leu Arg Leu Val Gly Ile
    290                 295                 300

Leu Thr Gln Ala Glu Leu Leu Ala Lys Thr Gln Tyr Glu Gln Pro Val
305                 310                 315                 320

Trp Pro Ile Leu Gln Gln Leu Ser Leu Glu Cys Cys Asn Pro Gln Ala
                325                 330                 335

Asn Leu Pro Leu Pro His
            340

<210> 91
<211> 1032
<212> DNA
<213> Xylella fastidiosa

<400> 91
atggaacttc gcccagagca actcgccaac ctgcctgcca atcaaccctt agagcctgcc 60

tacctcattg ccggccccga aaccctacgc ttagtcgaag ctgccgatgc gatacgtacc 120

catgctcgtg ccgacggcat caccgaacgc gaaatattcg acgtcgaaag ccgtgacttc 180

gactggcaac aacttgcctc cagcttcaac gcccccagcc tcttcagctc acgtcgctta 240

atcgaaatac gcctcccagg aggtaaacct ggtaaagaag gcagtgaagt catcacaagg 300

ttctgcgcca ccccctcatc agatgttatc ttgctgatca cctgcaacga atggagcaaa 360

gcacaccatg gcaagtggac cgatgccatc gctcgcatcg gtaaaatcgc aatcgcctgg 420

accatcaagc ctcacgaact acccgactgg attacacgcc gcctacatag caaaggctta 480

cgtgccaaca cagatgtgat acaccaatta agcacacgcc tagaaggcaa cctactcgcc 540

gccgcccaag aaattgacaa attggcgtta ttgaaccaag gccctctcct cgacctagag 600

accatggaat cctttgttgc cgacacagcg cgttacgacg tattccgact gaccgaagcg 660

atgctctccg gccaacccac cgcaacgata cgcatgctcg gcaaactgcg ggccgaaggt 720

gaagcagtgg cagcactgct gcccattctg accaaagaac tcatccgtac cacgaccctg 780

tcatgggtca aacatgacaa cggcaatcta gctgccgaaa tgaaagccca gggcatctgg 840

gaaacacgtc aagcaccgtt caagcgcgca ttacaacgtc atacctcacc gtacaaatgg 900

gaacacttcg ttgccgaagt cgggcgcata gaccgcatca ccaaaggacg caacgacggc 960

aatgcatggc tatcactcga acgcctcatg gtggcagtgg cagaagcaga tgcaacacgt 1020

ctgctagcat aa 1032

<210> 92
<211> 1032
<212> DNA
<213> Xylella fastidiosa

<220>
<221> CDS
<222> (1)..(1032)

<400> 92
atg gaa ctt cgc cca gag caa ctc gcc aac ctg cct gcc aat caa ccc 48
Met Glu Leu Arg Pro Glu Gln Leu Ala Asn Leu Pro Ala Asn Gln Pro
1               5                   10                  15

tta gag cct gcc tac ctc att gcc ggc ccc gaa acc cta cgc tta gtc 96
Leu Glu Pro Ala Tyr Leu Ile Ala Gly Pro Glu Thr Leu Arg Leu Val
            20                  25                  30

gaa gct gcc gat gcg ata cgt acc cat gct cgt gcc gac ggc atc acc 144
Glu Ala Ala Asp Ala Ile Arg Thr His Ala Arg Ala Asp Gly Ile Thr
        35                  40                  45

gaa cgc gaa ata ttc gac gtc gaa agc cgt gac ttc gac tgg caa caa 192
Glu Arg Glu Ile Phe Asp Val Glu Ser Arg Asp Phe Asp Trp Gln Gln
    50                  55                  60

ctt gcc tcc agc ttc aac gcc ccc agc ctc ttc agc tca cgt cgc tta 240
Leu Ala Ser Ser Phe Asn Ala Pro Ser Leu Phe Ser Ser Arg Arg Leu
65                  70                  75                  80

atc gaa ata cgc ctc cca gga ggt aaa cct ggt aaa gaa ggc agt gaa     288
Ile Glu Ile Arg Leu Pro Gly Gly Lys Pro Gly Lys Glu Gly Ser Glu
                85                  90                  95

gtc atc aca agg ttc tgc gcc acc ccc tca tca gat gtt atc ttg ctg     336
Val Ile Thr Arg Phe Cys Ala Thr Pro Ser Ser Asp Val Ile Leu Leu
            100                 105                 110

atc acc tgc aac gaa tgg agc aaa gca cac cat ggc aag tgg acc gat     384
Ile Thr Cys Asn Glu Trp Ser Lys Ala His His Gly Lys Trp Thr Asp
        115                 120                 125

gcc atc gct cgc atc ggt aaa atc gca atc gcc tgg acc atc aag cct     432
Ala Ile Ala Arg Ile Gly Lys Ile Ala Ile Ala Trp Thr Ile Lys Pro
    130                 135                 140

cac gaa cta ccc gac tgg att aca cgc cgc cta cat agc aaa ggc tta     480
His Glu Leu Pro Asp Trp Ile Thr Arg Arg Leu His Ser Lys Gly Leu
145                 150                 155                 160

cgt gcc aac aca gat gtg ata cac caa tta agc aca cgc cta gaa ggc     528
Arg Ala Asn Thr Asp Val Ile His Gln Leu Ser Thr Arg Leu Glu Gly
                165                 170                 175

aac cta ctc gcc gcc gcc caa gaa att gac aaa ttg gcg tta ttg aac     576
Asn Leu Leu Ala Ala Ala Gln Glu Ile Asp Lys Leu Ala Leu Leu Asn
            180                 185                 190

caa ggc cct ctc ctc gac cta gag acc atg gaa tcc ttt gtt gcc gac     624
Gln Gly Pro Leu Leu Asp Leu Glu Thr Met Glu Ser Phe Val Ala Asp
        195                 200                 205

aca gcg cgt tac gac gta ttc cga ctg acc gaa gcg atg ctc tcc ggc     672
Thr Ala Arg Tyr Asp Val Phe Arg Leu Thr Glu Ala Met Leu Ser Gly
    210                 215                 220

caa ccc acc gca acg ata cgc atg ctc ggc aaa ctg cgg gcc gaa ggt     720
Gln Pro Thr Ala Thr Ile Arg Met Leu Gly Lys Leu Arg Ala Glu Gly
225                 230                 235                 240

gaa gca gtg gca gca ctg ctg ccc att ctg acc aaa gaa ctc atc cgt     768
Glu Ala Val Ala Ala Leu Leu Pro Ile Leu Thr Lys Glu Leu Ile Arg
                245                 250                 255

acc acg acc ctg tca tgg gtc aaa cat gac aac ggc aat cta gct gcc     816
Thr Thr Thr Leu Ser Trp Val Lys His Asp Asn Gly Asn Leu Ala Ala
            260                 265                 270

gaa atg aaa gcc cag ggc atc tgg gaa aca cgt caa gca ccg ttc aag     864
Glu Met Lys Ala Gln Gly Ile Trp Glu Thr Arg Gln Ala Pro Phe Lys
        275                 280                 285

cgc gca tta caa cgt cat acc tca ccg tac aaa tgg gaa cac ttc gtt     912
Arg Ala Leu Gln Arg His Thr Ser Pro Tyr Lys Trp Glu His Phe Val
    290                 295                 300

gcc gaa gtc ggg cgc ata gac cgc atc acc aaa gga cgc aac gac ggc     960
Ala Glu Val Gly Arg Ile Asp Arg Ile Thr Lys Gly Arg Asn Asp Gly
305                 310                 315                 320

aat gca tgg cta tca ctc gaa cgc ctc atg gtg gca gtg gca gaa gca     1008
Asn Ala Trp Leu Ser Leu Glu Arg Leu Met Val Ala Val Ala Glu Ala
                325                 330                 335

gat gca aca cgt ctg cta gca taa                                     1032
Asp Ala Thr Arg Leu Leu Ala
            340

<210> 93
<211> 343
<212> PRT
<213> Xylella fastidiosa

<400> 93
Met Glu Leu Arg Pro Glu Gln Leu Ala Asn Leu Pro Ala Asn Gln Pro
1               5                   10                  15

Leu Glu Pro Ala Tyr Leu Ile Ala Gly Pro Glu Thr Leu Arg Leu Val
            20                  25                  30

Glu Ala Ala Asp Ala Ile Arg Thr His Ala Arg Ala Asp Gly Ile Thr
        35                  40                  45

Glu Arg Glu Ile Phe Asp Val Glu Ser Arg Asp Phe Asp Trp Gln Gln
    50                  55                  60

Leu Ala Ser Ser Phe Asn Ala Pro Ser Leu Phe Ser Ser Arg Arg Leu
65                  70                  75                  80

Ile Glu Ile Arg Leu Pro Gly Gly Lys Pro Gly Lys Glu Gly Ser Glu
                85                  90                  95

Val Ile Thr Arg Phe Cys Ala Thr Pro Ser Ser Asp Val Ile Leu Leu
            100                 105                 110

Ile Thr Cys Asn Glu Trp Ser Lys Ala His His Gly Lys Trp Thr Asp
        115                 120                 125

Ala Ile Ala Arg Ile Gly Lys Ile Ala Ile Ala Trp Thr Ile Lys Pro
    130                 135                 140

His Glu Leu Pro Asp Trp Ile Thr Arg Arg Leu His Ser Lys Gly Leu
145                 150                 155                 160

Arg Ala Asn Thr Asp Val Ile His Gln Leu Ser Thr Arg Leu Glu Gly
                165                 170                 175

Asn Leu Leu Ala Ala Ala Gln Glu Ile Asp Lys Leu Ala Leu Leu Asn
            180                 185                 190

Gln Gly Pro Leu Leu Asp Leu Glu Thr Met Glu Ser Phe Val Ala Asp
        195                 200                 205

Thr Ala Arg Tyr Asp Val Phe Arg Leu Thr Glu Ala Met Leu Ser Gly
    210                 215                 220

Gln Pro Thr Ala Thr Ile Arg Met Leu Gly Lys Leu Arg Ala Glu Gly
225                 230                 235                 240

Glu Ala Val Ala Ala Leu Leu Pro Ile Leu Thr Lys Glu Leu Ile Arg
                245                 250                 255

Thr Thr Thr Leu Ser Trp Val Lys His Asp Asn Gly Asn Leu Ala Ala
            260                 265                 270

Glu Met Lys Ala Gln Gly Ile Trp Glu Thr Arg Gln Ala Pro Phe Lys
        275                 280                 285

Arg Ala Leu Gln Arg His Thr Ser Pro Tyr Lys Trp Glu His Phe Val
    290                 295                 300

Ala Glu Val Gly Arg Ile Asp Arg Ile Thr Lys Gly Arg Asn Asp Gly
305                 310                 315                 320

Asn Ala Trp Leu Ser Leu Glu Arg Leu Met Val Ala Val Ala Glu Ala

325                 330                 335

Asp Ala Thr Arg Leu Leu Ala
            340


<210> 94
<211> 945
<212> DNA
<213> Mycoplasma pulmonis

<400> 94
   atgcatttaa tttatggaca agaagaattt ttaattgaat caaaaacaaa aaacataatt 60
   gagaatcaaa aaaaagattc gatagttttt acattttatg aaaactttga aattgatgat 120
   cttttaaatg atattgttca aaaagatctt tttagtccta ataaaataat tcatattaaa 180
   aactggaaat tacttgaaaa tccaagacct tcaaaagtaa ttttaaacaa actaaaattg 240
   cttagcgaaa taatagaaaa tgataaaagt ttaattttta ttttttctca cattggtgaa 300
   attgaaaaaa attttttaag tgatttttta ttaaaaaaag cttcagtaaa acactataaa 360
   aaacttgagt taaatgaaaa aattaaattc attgaaagct atgttactaa aaacaaaatt 420
   aaaatttcaa cagtgcttat ttcttattta ttaatcaatg tttcatcatc tatttcaatt 480
   gttataaatg aaattaataa actattttta gaaaaccaaa acatcactag agaaatgatt 540
   gaaaagtcag ttggttttta tactaaagaa cctaactttg aatttatcaa ttcaatcaaa 600
   tctagaaatt caaattcaat ttggaaaaca tttttagaac aaaaaaattc aggaattgaa 660
   cctttagatc ttttaaaaca aatttcatca gcttttattt taggaaataa aattaacaac 720
   atcatcaaat tagatcactc acttgatcaa gttgctaaaa ttttaaatgt gcacatttat 780
   agagcaaaag aaattttttc tttaattaaa ggttttagtc aaaactcaat taatgaaatt 840
   atcgaattat tagagttaac tgataaaaga atcaaaacta catatatgga tcctgagatg 900
   gctttagatt tttttattct aaaattaatt agacactata attaa                945


<210> 95
<211> 945
<212> DNA
<213> Mycoplasma pulmonis

<220>
<221> CDS
<222> (1)..(945)

<400> 95
atg cat tta att tat gga caa gaa gaa ttt tta att gaa tca aaa aca     48
Met His Leu Ile Tyr Gly Gln Glu Glu Phe Leu Ile Glu Ser Lys Thr
1               5                   10                  15

aaa aac ata att gag aat caa aaa aaa gat tcg ata gtt ttt aca ttt     96
Lys Asn Ile Ile Glu Asn Gln Lys Lys Asp Ser Ile Val Phe Thr Phe
            20                  25                  30

tat gaa aac ttt gaa att gat gat ctt tta aat gat att gtt caa aaa     144
Tyr Glu Asn Phe Glu Ile Asp Asp Leu Leu Asn Asp Ile Val Gln Lys
        35                  40                  45

gat ctt ttt agt cct aat aaa ata att cat att aaa aac tgg aaa tta     192

Asp Leu Phe Ser Pro Asn Lys Ile Ile His Ile Lys Asn Trp Lys Leu
    50                  55                  60

ctt gaa aat cca aga cct tca aaa gta att tta aac aaa cta aaa ttg 240
Leu Glu Asn Pro Arg Pro Ser Lys Val Ile Leu Asn Lys Leu Lys Leu
65                  70                  75                  80

ctt agc gaa ata ata gaa aat gat aaa agt tta att ttt att ttt tct 288
Leu Ser Glu Ile Ile Glu Asn Asp Lys Ser Leu Ile Phe Ile Phe Ser
                85                  90                  95

cac att ggt gaa att gaa aaa aat ttt tta agt gat ttt tta tta aaa 336
His Ile Gly Glu Ile Glu Lys Asn Phe Leu Ser Asp Phe Leu Leu Lys
            100                 105                 110

aaa gct tca gta aaa cac tat aaa aaa ctt gag tta aat gaa aaa att 384
Lys Ala Ser Val Lys His Tyr Lys Lys Leu Glu Leu Asn Glu Lys Ile
        115                 120                 125

aaa ttc att gaa agc tat gtt act aaa aac aaa att aaa att tca aca 432
Lys Phe Ile Glu Ser Tyr Val Thr Lys Asn Lys Ile Lys Ile Ser Thr
    130                 135                 140

gtg ctt att tct tat tta tta atc aat gtt tca tca tct att tca att 480
Val Leu Ile Ser Tyr Leu Leu Ile Asn Val Ser Ser Ser Ile Ser Ile
145                 150                 155                 160

gtt ata aat gaa att aat aaa cta ttt tta gaa aac caa aac atc act 528
Val Ile Asn Glu Ile Asn Lys Leu Phe Leu Glu Asn Gln Asn Ile Thr
                165                 170                 175

aga gaa atg att gaa aag tca gtt ggt ttt tat act aaa gaa cct aac 576
Arg Glu Met Ile Glu Lys Ser Val Gly Phe Tyr Thr Lys Glu Pro Asn
            180                 185                 190

ttt gaa ttt atc aat tca atc aaa tct aga aat tca aat tca att tgg 624
Phe Glu Phe Ile Asn Ser Ile Lys Ser Arg Asn Ser Asn Ser Ile Trp
        195                 200                 205

aaa aca ttt tta gaa caa aaa aat tca gga att gaa cct tta gat ctt 672
Lys Thr Phe Leu Glu Gln Lys Asn Ser Gly Ile Glu Pro Leu Asp Leu
    210                 215                 220

tta aaa caa att tca tca gct ttt att tta gga aat aaa att aac aac 720
Leu Lys Gln Ile Ser Ser Ala Phe Ile Leu Gly Asn Lys Ile Asn Asn
225                 230                 235                 240

atc atc aaa tta gat cac tca ctt gat caa gtt gct aaa att tta aat 768
Ile Ile Lys Leu Asp His Ser Leu Asp Gln Val Ala Lys Ile Leu Asn
                245                 250                 255

gtg cac att tat aga gca aaa gaa att ttt tct tta att aaa ggt ttt 816
Val His Ile Tyr Arg Ala Lys Glu Ile Phe Ser Leu Ile Lys Gly Phe
            260                 265                 270

agt caa aac tca att aat gaa att atc gaa tta tta gag tta act gat 864
Ser Gln Asn Ser Ile Asn Glu Ile Ile Glu Leu Leu Glu Leu Thr Asp
        275                 280                 285

aaa aga atc aaa act aca tat atg gat cct gag atg gct tta gat ttt 912
Lys Arg Ile Lys Thr Thr Tyr Met Asp Pro Glu Met Ala Leu Asp Phe
    290                 295                 300

ttt att cta aaa tta att aga cac tat aat taa 945
Phe Ile Leu Lys Leu Ile Arg His Tyr Asn
305 310 315

<210> 96
<211> 314
<212> PRT
<213> Mycoplasma pulmonis

<400> 96
Met His Leu Ile Tyr Gly Gln Glu Glu Phe Leu Ile Glu Ser Lys Thr
1               5                   10                  15

Lys Asn Ile Ile Glu Asn Gln Lys Lys Asp Ser Ile Val Phe Thr Phe
            20                  25                  30

Tyr Glu Asn Phe Glu Ile Asp Asp Leu Leu Asn Asp Ile Val Gln Lys
        35                  40                  45

Asp Leu Phe Ser Pro Asn Lys Ile Ile His Ile Lys Asn Trp Lys Leu
    50                  55                  60

Leu Glu Asn Pro Arg Pro Ser Lys Val Ile Leu Asn Lys Leu Lys Leu
65                  70                  75                  80

Leu Ser Glu Ile Ile Glu Asn Asp Lys Ser Leu Ile Phe Ile Phe Ser
                85                  90                  95

His Ile Gly Glu Ile Glu Lys Asn Phe Leu Ser Asp Phe Leu Leu Lys
            100                 105                 110

Lys Ala Ser Val Lys His Tyr Lys Lys Leu Glu Leu Asn Glu Lys Ile
        115                 120                 125

Lys Phe Ile Glu Ser Tyr Val Thr Lys Asn Lys Ile Lys Ile Ser Thr
    130                 135                 140

Val Leu Ile Ser Tyr Leu Leu Ile Asn Val Ser Ser Ser Ile Ser Ile
145                 150                 155                 160

Val Ile Asn Glu Ile Asn Lys Leu Phe Leu Glu Asn Gln Asn Ile Thr
                165                 170                 175

Arg Glu Met Ile Glu Lys Ser Val Gly Phe Tyr Thr Lys Glu Pro Asn
            180                 185                 190

Phe Glu Phe Ile Asn Ser Ile Lys Ser Arg Asn Ser Asn Ser Ile Trp
        195                 200                 205

Lys Thr Phe Leu Glu Gln Lys Asn Ser Gly Ile Glu Pro Leu Asp Leu
    210                 215                 220

Leu Lys Gln Ile Ser Ser Ala Phe Ile Leu Gly Asn Lys Ile Asn Asn
225                 230                 235                 240

Ile Ile Lys Leu Asp His Ser Leu Asp Gln Val Ala Lys Ile Leu Asn
                245                 250                 255

Val His Ile Tyr Arg Ala Lys Glu Ile Phe Ser Leu Ile Lys Gly Phe
            260                 265                 270

Ser Gln Asn Ser Ile Asn Glu Ile Ile Glu Leu Leu Glu Leu Thr Asp
        275                 280                 285

Lys Arg Ile Lys Thr Thr Tyr Met Asp Pro Glu Met Ala Leu Asp Phe
    290                 295                 300

Phe Ile Leu Lys Leu Ile Arg His Tyr Asn
305                 310

<210> 97

<211> 975
<212> DNA
<213> Staphylococcus aureus

<400> 97
atgagcgaca atattgtagc tatttatgga gatgtgcctg aattggttga aaaacaaagt 60

gcagaaatca tatcacaatt tttgaaaagt gatagagatg actttaactt tgtgaaatat 120

aatttatacg aaaaagagat tgcaccaatt gttgaagaaa cattaacatt gcctttcttt 180

tcagataaaa aagcaatttt ggttaaaaat gcatatatat ttacaggtga aaaagcgcca 240

aaagatatgg ctcataatgt agatcaatta atagaattta ttgaaaaata tgatggcgaa 300

aatttgattg tctttgagat atatcaaaat aaacttgatg aaagaaaaaa gttaactaaa 360

actctaaaaa agcatgcaag gcttaaaaaa atagagcaaa tgtctgaaga agaaataaaa 420

aaatggattc aaagtaaatt aaatgagaat ttcaaagata tcaaaagaga tgcattagat 480

ttatttattg agttgacagg tattaacttt aatattgtct cacaagagat agaaaagttg 540

attttatttt taggcgatag accaacaatt aataagcagg atgttaacca aattattaat 600

agaagtttag agcaaaatgt atttttactg actgaataca ttcagaaaag aaagaaagaa 660

caagcaattc atttagtaaa agatttaata actatgaaag aagaaccaat taaattactt 720

gcactaatta caagtaatta ccgattattt tatcaatgta agattctgag tcaaaaagga 780

tatagtggac agcaaattgc taaaacaata ggagtgcatc catacagagt aaaattagcg 840

ttaggacaag taagacatta tcaacttgat gaattattga atattgtaga tgcttgtgcg 900

gaaactgatt ataaacttaa atcatcatat atggataaac agttaatact ggaattattc 960

attctatctt tataa 975

<210> 98
<211> 975
<212> DNA
<213> Staphylococcus aureus

<220>
<221> CDS
<222> (1)..(975)

<400> 98
atg agc gac aat att gta gct att tat gga gat gtg cct gaa ttg gtt 48
Met Ser Asp Asn Ile Val Ala Ile Tyr Gly Asp Val Pro Glu Leu Val
1               5                   10                  15

gaa aaa caa agt gca gaa atc ata tca caa ttt ttg aaa agt gat aga 96
Glu Lys Gln Ser Ala Glu Ile Ile Ser Gln Phe Leu Lys Ser Asp Arg
            20                  25                  30

gat gac ttt aac ttt gtg aaa tat aat tta tac gaa aaa gag att gca 144
Asp Asp Phe Asn Phe Val Lys Tyr Asn Leu Tyr Glu Lys Glu Ile Ala
        35                  40                  45

cca att gtt gaa gaa aca tta aca ttg cct ttc ttt tca gat aaa aaa 192
Pro Ile Val Glu Glu Thr Leu Thr Leu Pro Phe Phe Ser Asp Lys Lys
    50                  55                  60

gca att ttg gtt aaa aat gca tat ata ttt aca ggt gaa aaa gcg cca 240
Ala Ile Leu Val Lys Asn Ala Tyr Ile Phe Thr Gly Glu Lys Ala Pro

65                  70                  75                  80

aaa gat atg gct cat aat gta gat caa tta ata gaa ttt att gaa aaa      288
Lys Asp Met Ala His Asn Val Asp Gln Leu Ile Glu Phe Ile Glu Lys
                85                  90                  95

tat gat ggc gaa aat ttg att gtc ttt gag ata tat caa aat aaa ctt      336
Tyr Asp Gly Glu Asn Leu Ile Val Phe Glu Ile Tyr Gln Asn Lys Leu
            100                 105                 110

gat gaa aga aaa aag tta act aaa act cta aaa aag cat gca agg ctt      384
Asp Glu Arg Lys Lys Leu Thr Lys Thr Leu Lys Lys His Ala Arg Leu
        115                 120                 125

aaa aaa ata gag caa atg tct gaa gaa gaa ata aaa aaa tgg att caa      432
Lys Lys Ile Glu Gln Met Ser Glu Glu Glu Ile Lys Lys Trp Ile Gln
    130                 135                 140

agt aaa tta aat gag aat ttc aaa gat atc aaa aga gat gca tta gat      480
Ser Lys Leu Asn Glu Asn Phe Lys Asp Ile Lys Arg Asp Ala Leu Asp
145                 150                 155                 160

tta ttt att gag ttg aca ggt att aac ttt aat att gtc tca caa gag      528
Leu Phe Ile Glu Leu Thr Gly Ile Asn Phe Asn Ile Val Ser Gln Glu
                165                 170                 175

ata gaa aag ttg att tta ttt tta ggc gat aga cca aca att aat aag      576
Ile Glu Lys Leu Ile Leu Phe Leu Gly Asp Arg Pro Thr Ile Asn Lys
            180                 185                 190

cag gat gtt aac caa att att aat aga agt tta gag caa aat gta ttt      624
Gln Asp Val Asn Gln Ile Ile Asn Arg Ser Leu Glu Gln Asn Val Phe
        195                 200                 205

tta ctg act gaa tac att cag aaa aga aag aaa gaa caa gca att cat      672
Leu Leu Thr Glu Tyr Ile Gln Lys Arg Lys Lys Glu Gln Ala Ile His
    210                 215                 220

tta gta aaa gat tta ata act atg aaa gaa gaa cca att aaa tta ctt      720
Leu Val Lys Asp Leu Ile Thr Met Lys Glu Glu Pro Ile Lys Leu Leu
225                 230                 235                 240

gca cta att aca agt aat tac cga tta ttt tat caa tgt aag att ctg      768
Ala Leu Ile Thr Ser Asn Tyr Arg Leu Phe Tyr Gln Cys Lys Ile Leu
                245                 250                 255

agt caa aaa gga tat agt gga cag caa att gct aaa aca ata gga gtg      816
Ser Gln Lys Gly Tyr Ser Gly Gln Gln Ile Ala Lys Thr Ile Gly Val
            260                 265                 270

cat cca tac aga gta aaa tta gcg tta gga caa gta aga cat tat caa      864
His Pro Tyr Arg Val Lys Leu Ala Leu Gly Gln Val Arg His Tyr Gln
        275                 280                 285

ctt gat gaa tta ttg aat att gta gat gct tgt gcg gaa act gat tat      912
Leu Asp Glu Leu Leu Asn Ile Val Asp Ala Cys Ala Glu Thr Asp Tyr
    290                 295                 300

aaa ctt aaa tca tca tat atg gat aaa cag tta ata ctg gaa tta ttc      960
Lys Leu Lys Ser Ser Tyr Met Asp Lys Gln Leu Ile Leu Glu Leu Phe
305                 310                 315                 320

att cta tct tta taa                                                  975
Ile Leu Ser Leu
                325

<210> 99

<211> 324
<212> PRT
<213> Staphylococcus aureus

<400> 99
Met Ser Asp Asn Ile Val Ala Ile Tyr Gly Asp Val Pro Glu Leu Val
1               5                   10                  15

Glu Lys Gln Ser Ala Glu Ile Ile Ser Gln Phe Leu Lys Ser Asp Arg
            20                  25                  30

Asp Asp Phe Asn Phe Val Lys Tyr Asn Leu Tyr Glu Lys Glu Ile Ala
        35                  40                  45

Pro Ile Val Glu Glu Thr Leu Thr Leu Pro Phe Phe Ser Asp Lys Lys
    50                  55                  60

Ala Ile Leu Val Lys Asn Ala Tyr Ile Phe Thr Gly Glu Lys Ala Pro
65                  70                  75                  80

Lys Asp Met Ala His Asn Val Asp Gln Leu Ile Glu Phe Ile Glu Lys
                85                  90                  95

Tyr Asp Gly Glu Asn Leu Ile Val Phe Glu Ile Tyr Gln Asn Lys Leu
            100                 105                 110

Asp Glu Arg Lys Lys Leu Thr Lys Thr Leu Lys Lys His Ala Arg Leu
        115                 120                 125

Lys Lys Ile Glu Gln Met Ser Glu Glu Glu Ile Lys Lys Trp Ile Gln
    130                 135                 140

Ser Lys Leu Asn Glu Asn Phe Lys Asp Ile Lys Arg Asp Ala Leu Asp
145                 150                 155                 160

Leu Phe Ile Glu Leu Thr Gly Ile Asn Phe Asn Ile Val Ser Gln Glu
                165                 170                 175

Ile Glu Lys Leu Ile Leu Phe Leu Gly Asp Arg Pro Thr Ile Asn Lys
            180                 185                 190

Gln Asp Val Asn Gln Ile Ile Asn Arg Ser Leu Glu Gln Asn Val Phe
        195                 200                 205

Leu Leu Thr Glu Tyr Ile Gln Lys Arg Lys Lys Glu Gln Ala Ile His
    210                 215                 220

Leu Val Lys Asp Leu Ile Thr Met Lys Glu Glu Pro Ile Lys Leu Leu
225                 230                 235                 240

Ala Leu Ile Thr Ser Asn Tyr Arg Leu Phe Tyr Gln Cys Lys Ile Leu
                245                 250                 255

Ser Gln Lys Gly Tyr Ser Gly Gln Gln Ile Ala Lys Thr Ile Gly Val
            260                 265                 270

His Pro Tyr Arg Val Lys Leu Ala Leu Gly Gln Val Arg His Tyr Gln
        275                 280                 285

Leu Asp Glu Leu Leu Asn Ile Val Asp Ala Cys Ala Glu Thr Asp Tyr
    290                 295                 300

Lys Leu Lys Ser Ser Tyr Met Asp Lys Gln Leu Ile Leu Glu Leu Phe
305                 310                 315                 320

Ile Leu Ser Leu

<210> 100
<211> 1011
<212> DNA
<213> Streptomyces coelicolor

<400> 100
gtgcgtgcga tgcttgtcgc gatggccagg aagactgcga acgacgaccc tctcgccccg 60

gtgacccttg ccgtgggcca ggaggacctc ctgctcgacc gtgccgtgca ggaggtggtg 120

gccgccgcga aggccgccga cgccgacacg gacgtacgcg acctcacccc cgaccagctg 180

cagcccggca ccctcgccga gctgaccagc ccctcgctct tcgccgagcg caaagtcgtg 240

gtcgtacgca atgcgcagga cctgtccgcc gacacggtca aggacgtcaa ggcctacctc 300

ggcgcccccg ccgaggagat caccctcgtc ctgctgcacg cgggcggcgc caagggcaag 360

ggcgtcctcg acgccggccg caaggcgggg gcgcgcgagg tggcctgccc gaagatgacc 420

aagcccgccg accggctggc cttcgtgcgc gcggagttcc gcacggccgg gcggtcggcc 480

acacccgagg cctgccaggc cctggtcgac gcgatcggca gcgacctcag ggagctggcc 540

tcggcggtgt cccagctgac cgccgacgtc gaggggaccg tcgacgaggc ggtcgtcggg 600

cggtactaca ccgggcgggc ggaggcctcc agcttcacgg tcgccgaccg ggcggtcgag 660

ggacgcgcgg cggaggcgct ggaggcgctg cgctggtcgc tggcgaccgg agtggcgccg 720

gtgctgatca ccagcgcgct cgcccagggc gtgcgggcca tcggcaagct gtcctccgcc 780

cgcggcggac gccccgccga cctcgcccgc gagctgggga tgccgccctg gaagatcgac 840

cgcgtccggc agcagatgcg cggctggacc ccggacggcg tctccgtggc cctgcgcgcg 900

gtcgccgagg ccgacgcggg ggtgaagggc ggcggggacg atcccgagta cgccctggag 960

aaggccgtgg tgatcatcgc gcgggcggcg cgttcacggg gccggacctg a 1011

<210> 101
<211> 1011
<212> DNA
<213> Streptomyces coelicolor

<220>
<221> CDS
<222> (1)..(1011)

<400> 101
gtg cgt gcg atg ctt gtc gcg atg gcc agg aag act gcg aac gac gac 48
Val Arg Ala Met Leu Val Ala Met Ala Arg Lys Thr Ala Asn Asp Asp
1               5                   10                  15

cct ctc gcc ccg gtg acc ctt gcc gtg ggc cag gag gac ctc ctg ctc 96
Pro Leu Ala Pro Val Thr Leu Ala Val Gly Gln Glu Asp Leu Leu Leu
            20                  25                  30

gac cgt gcc gtg cag gag gtg gtg gcc gcc gcg aag gcc gcc gac gcc 144
Asp Arg Ala Val Gln Glu Val Val Ala Ala Ala Lys Ala Ala Asp Ala
        35                  40                  45

gac acg gac gta cgc gac ctc acc ccc gac cag ctg cag ccc ggc acc 192
Asp Thr Asp Val Arg Asp Leu Thr Pro Asp Gln Leu Gln Pro Gly Thr
    50                  55                  60

ctc gcc gag ctg acc agc ccc tcg ctc ttc gcc gag cgc aaa gtc gtg 240

Leu Ala Glu Leu Thr Ser Pro Ser Leu Phe Ala Glu Arg Lys Val Val
65                  70                  75                  80

gtc gta cgc aat gcg cag gac ctg tcc gcc gac acg gtc aag gac gtc     288
Val Val Arg Asn Ala Gln Asp Leu Ser Ala Asp Thr Val Lys Asp Val
                85                  90                  95

aag gcc tac ctc ggc gcc ccc gcc gag gag atc acc ctc gtc ctg ctg     336
Lys Ala Tyr Leu Gly Ala Pro Ala Glu Glu Ile Thr Leu Val Leu Leu
            100                 105                 110

cac gcg ggc ggc gcc aag ggc aag ggc gtc ctc gac gcc ggc cgc aag     384
His Ala Gly Gly Ala Lys Gly Lys Gly Val Leu Asp Ala Gly Arg Lys
        115                 120                 125

gcg ggg gcg cgc gag gtg gcc tgc ccg aag atg acc aag ccc gcc gac     432
Ala Gly Ala Arg Glu Val Ala Cys Pro Lys Met Thr Lys Pro Ala Asp
    130                 135                 140

cgg ctg gcc ttc gtg cgc gcg gag ttc cgc acg gcc ggg cgg tcg gcc     480
Arg Leu Ala Phe Val Arg Ala Glu Phe Arg Thr Ala Gly Arg Ser Ala
145                 150                 155                 160

aca ccc gag gcc tgc cag gcc ctg gtc gac gcg atc ggc agc gac ctc     528
Thr Pro Glu Ala Cys Gln Ala Leu Val Asp Ala Ile Gly Ser Asp Leu
                165                 170                 175

agg gag ctg gcc tcg gcg gtg tcc cag ctg acc gcc gac gtc gag ggg     576
Arg Glu Leu Ala Ser Ala Val Ser Gln Leu Thr Ala Asp Val Glu Gly
            180                 185                 190

acc gtc gac gag gcg gtc gtc ggg cgg tac tac acc ggg cgg gcg gag     624
Thr Val Asp Glu Ala Val Val Gly Arg Tyr Tyr Thr Gly Arg Ala Glu
        195                 200                 205

gcc tcc agc ttc acg gtc gcc gac cgg gcg gtc gag gga cgc gcg gcg     672
Ala Ser Ser Phe Thr Val Ala Asp Arg Ala Val Glu Gly Arg Ala Ala
    210                 215                 220

gag gcg ctg gag gcg ctg cgc tgg tcg ctg gcg acc gga gtg gcg ccg     720
Glu Ala Leu Glu Ala Leu Arg Trp Ser Leu Ala Thr Gly Val Ala Pro
225                 230                 235                 240

gtg ctg atc acc agc gcg ctc gcc cag ggc gtg cgg gcc atc ggc aag     768
Val Leu Ile Thr Ser Ala Leu Ala Gln Gly Val Arg Ala Ile Gly Lys
                245                 250                 255

ctg tcc tcc gcc cgc ggc gga cgc ccc gcc gac ctc gcc cgc gag ctg     816
Leu Ser Ser Ala Arg Gly Gly Arg Pro Ala Asp Leu Ala Arg Glu Leu
            260                 265                 270

ggg atg ccg ccc tgg aag atc gac cgc gtc cgg cag cag atg cgc ggc     864
Gly Met Pro Pro Trp Lys Ile Asp Arg Val Arg Gln Gln Met Arg Gly
        275                 280                 285

tgg acc ccg gac ggc gtc tcc gtg gcc ctg cgc gcg gtc gcc gag gcc     912
Trp Thr Pro Asp Gly Val Ser Val Ala Leu Arg Ala Val Ala Glu Ala
    290                 295                 300

gac gcg ggg gtg aag ggc ggc ggg gac gat ccc gag tac gcc ctg gag     960
Asp Ala Gly Val Lys Gly Gly Gly Asp Asp Pro Glu Tyr Ala Leu Glu
305                 310                 315                 320

aag gcc gtg gtg atc atc gcg cgg gcg gcg cgt tca cgg ggc cgg acc     1008
Lys Ala Val Val Ile Ile Ala Arg Ala Ala Arg Ser Arg Gly Arg Thr
                325                 330                 335

tga                                                                 1011

<210> 102
<211> 336
<212> PRT
<213> Streptomyces coelicolor

<400> 102
Val Arg Ala Met Leu Val Ala Met Ala Arg Lys Thr Ala Asn Asp Asp
1               5                   10                  15

Pro Leu Ala Pro Val Thr Leu Ala Val Gly Gln Glu Asp Leu Leu Leu
            20                  25                  30

Asp Arg Ala Val Gln Glu Val Val Ala Ala Ala Lys Ala Ala Asp Ala
        35                  40                  45

Asp Thr Asp Val Arg Asp Leu Thr Pro Asp Gln Leu Gln Pro Gly Thr
    50                  55                  60

Leu Ala Glu Leu Thr Ser Pro Ser Leu Phe Ala Glu Arg Lys Val Val
65                  70                  75                  80

Val Val Arg Asn Ala Gln Asp Leu Ser Ala Asp Thr Val Lys Asp Val
                85                  90                  95

Lys Ala Tyr Leu Gly Ala Pro Ala Glu Glu Ile Thr Leu Val Leu Leu
            100                 105                 110

His Ala Gly Gly Ala Lys Gly Lys Gly Val Leu Asp Ala Gly Arg Lys
        115                 120                 125

Ala Gly Ala Arg Glu Val Ala Cys Pro Lys Met Thr Lys Pro Ala Asp
    130                 135                 140

Arg Leu Ala Phe Val Arg Ala Glu Phe Arg Thr Ala Gly Arg Ser Ala
145                 150                 155                 160

Thr Pro Glu Ala Cys Gln Ala Leu Val Asp Ala Ile Gly Ser Asp Leu
                165                 170                 175

Arg Glu Leu Ala Ser Ala Val Ser Gln Leu Thr Ala Asp Val Glu Gly
            180                 185                 190

Thr Val Asp Glu Ala Val Val Gly Arg Tyr Tyr Thr Gly Arg Ala Glu
        195                 200                 205

Ala Ser Ser Phe Thr Val Ala Asp Arg Ala Val Glu Gly Arg Ala Ala
    210                 215                 220

Glu Ala Leu Glu Ala Leu Arg Trp Ser Leu Ala Thr Gly Val Ala Pro
225                 230                 235                 240

Val Leu Ile Thr Ser Ala Leu Ala Gln Gly Val Arg Ala Ile Gly Lys
                245                 250                 255

Leu Ser Ser Ala Arg Gly Gly Arg Pro Ala Asp Leu Ala Arg Glu Leu
            260                 265                 270

Gly Met Pro Pro Trp Lys Ile Asp Arg Val Arg Gln Gln Met Arg Gly
        275                 280                 285

Trp Thr Pro Asp Gly Val Ser Val Ala Leu Arg Ala Val Ala Glu Ala
    290                 295                 300

Asp Ala Gly Val Lys Gly Gly Gly Asp Asp Pro Glu Tyr Ala Leu Glu
305                 310                 315                 320

Lys Ala Val Val Ile Ile Ala Arg Ala Ala Arg Ser Arg Gly Arg Thr
                325                 330                 335

<210> 103
<211> 1041
<212> DNA
<213> Streptococcus pyogenes

<400> 103
atgattgcga tagaaaagat tgaaaaactg agtaaagaaa atttgggtct tataaccctt 60

gtcacaggag atgacattgg tcagtatagc cagttgaaat cccgcttaat ggagcagatt 120

gcttttgata aggatgattt ggcctattct tactttgata tgtctgaggc cgcttatcag 180

gatgcagaaa tggatctagt gagcctaccc ttctttgctg agcagaaggt ggttattttt 240

gaccatttgt tagatatcac gaccaataaa aaaagtttct taaaagaaaa agacctaaag 300

gcctttgaag cctatttaga aaatccctta gagactactc gactaattat ctttgctcca 360

ggtaaattgg atagtaagag acggcttgtt aagcttttga aacgtgatgc ccttgtttta 420

gaagccaacc ctctgaaaga agcagagcta agaacttatt ttcaaaaata cagtcatcaa 480

ctgggtttag gtttcgagag tggtgccttt gaccaattac ttttgaaatc aaacgatgat 540

tttagtcaaa tcatgaaaaa catggccttt ttaaaagcct ataaaaaaac gggaaatatt 600

agcctaactg atattgagca agccattcct aaaagtttac aagataatat tttcgatctg 660

actagacttg tcctaggagg taaaattgat gcggctagag atttgattca tgatttacgg 720

ttatctggag aagatgacat taaattaatc gctatcatgc taggccaatt tcgcttattt 780

ttgcagctga ctattcttgc tagagatgta aaaaacgagc aacaactagt gattagttta 840

tcagatattc ttgggcggcg ggttaatcct taccaggtca agtatgcgtt aaaggattct 900

aggaccttat ctcttgcctt tctaacagga gcggtgaaaa ccttgattga gacagattac 960

cagataaaaa caggacttta tgagaagagt tatctagttg atattgctct cttaaaaatc 1020

atgactcact ctcaaaaata g                                           1041

<210> 104
<211> 1041
<212> DNA
<213> Streptococcus pyogenes

<220>
<221> CDS
<222> (1)..(1041)

<400> 104
atg att gcg ata gaa aag att gaa aaa ctg agt aaa gaa aat ttg ggt    48
Met Ile Ala Ile Glu Lys Ile Glu Lys Leu Ser Lys Glu Asn Leu Gly
1               5                   10                  15

ctt ata acc ctt gtc aca gga gat gac att ggt cag tat agc cag ttg    96
Leu Ile Thr Leu Val Thr Gly Asp Asp Ile Gly Gln Tyr Ser Gln Leu
            20                  25                  30

aaa tcc cgc tta atg gag cag att gct ttt gat aag gat gat ttg gcc    144
Lys Ser Arg Leu Met Glu Gln Ile Ala Phe Asp Lys Asp Asp Leu Ala

        35                  40                  45

tat tct tac ttt gat atg tct gag gcc gct tat cag gat gca gaa atg 192
Tyr Ser Tyr Phe Asp Met Ser Glu Ala Ala Tyr Gln Asp Ala Glu Met
    50                  55                  60

gat cta gtg agc cta ccc ttc ttt gct gag cag aag gtg gtt att ttt 240
Asp Leu Val Ser Leu Pro Phe Phe Ala Glu Gln Lys Val Val Ile Phe
65                  70                  75                  80

gac cat ttg tta gat atc acg acc aat aaa aaa agt ttc tta aaa gaa 288
Asp His Leu Leu Asp Ile Thr Thr Asn Lys Lys Ser Phe Leu Lys Glu
                85                  90                  95

aaa gac cta aag gcc ttt gaa gcc tat tta gaa aat ccc tta gag act 336
Lys Asp Leu Lys Ala Phe Glu Ala Tyr Leu Glu Asn Pro Leu Glu Thr
            100                 105                 110

act cga cta att atc ttt gct cca ggt aaa ttg gat agt aag aga cgg 384
Thr Arg Leu Ile Ile Phe Ala Pro Gly Lys Leu Asp Ser Lys Arg Arg
        115                 120                 125

ctt gtt aag ctt ttg aaa cgt gat gcc ctt gtt tta gaa gcc aac cct 432
Leu Val Lys Leu Leu Lys Arg Asp Ala Leu Val Leu Glu Ala Asn Pro
    130                 135                 140

ctg aaa gaa gca gag cta aga act tat ttt caa aaa tac agt cat caa 480
Leu Lys Glu Ala Glu Leu Arg Thr Tyr Phe Gln Lys Tyr Ser His Gln
145                 150                 155                 160

ctg ggt tta ggt ttc gag agt ggt gcc ttt gac caa tta ctt ttg aaa 528
Leu Gly Leu Gly Phe Glu Ser Gly Ala Phe Asp Gln Leu Leu Leu Lys
                165                 170                 175

tca aac gat gat ttt agt caa atc atg aaa aac atg gcc ttt tta aaa 576
Ser Asn Asp Asp Phe Ser Gln Ile Met Lys Asn Met Ala Phe Leu Lys
            180                 185                 190

gcc tat aaa aaa acg gga aat att agc cta act gat att gag caa gcc 624
Ala Tyr Lys Lys Thr Gly Asn Ile Ser Leu Thr Asp Ile Glu Gln Ala
        195                 200                 205

att cct aaa agt tta caa gat aat att ttc gat ctg act aga ctt gtc 672
Ile Pro Lys Ser Leu Gln Asp Asn Ile Phe Asp Leu Thr Arg Leu Val
    210                 215                 220

cta gga ggt aaa att gat gcg gct aga gat ttg att cat gat tta cgg 720
Leu Gly Gly Lys Ile Asp Ala Ala Arg Asp Leu Ile His Asp Leu Arg
225                 230                 235                 240

tta tct gga gaa gat gac att aaa tta atc gct atc atg cta ggc caa 768
Leu Ser Gly Glu Asp Asp Ile Lys Leu Ile Ala Ile Met Leu Gly Gln
                245                 250                 255

ttt cgc tta ttt ttg cag ctg act att ctt gct aga gat gta aaa aac 816
Phe Arg Leu Phe Leu Gln Leu Thr Ile Leu Ala Arg Asp Val Lys Asn
            260                 265                 270

gag caa caa cta gtg att agt tta tca gat att ctt ggg cgg cgg gtt 864
Glu Gln Gln Leu Val Ile Ser Leu Ser Asp Ile Leu Gly Arg Arg Val
        275                 280                 285

aat cct tac cag gtc aag tat gcg tta aag gat tct agg acc tta tct 912
Asn Pro Tyr Gln Val Lys Tyr Ala Leu Lys Asp Ser Arg Thr Leu Ser
    290                 295                 300

ctt gcc ttt cta aca gga gcg gtg aaa acc ttg att gag aca gat tac 960
Leu Ala Phe Leu Thr Gly Ala Val Lys Thr Leu Ile Glu Thr Asp Tyr

305                 310                 315                 320

cag ata aaa aca gga ctt tat gag aag agt tat cta gtt gat att gct     1008
Gln Ile Lys Thr Gly Leu Tyr Glu Lys Ser Tyr Leu Val Asp Ile Ala
                325                 330                 335

ctc tta aaa atc atg act cac tct caa aaa tag                         1041
Leu Leu Lys Ile Met Thr His Ser Gln Lys
            340                 345


<210> 105
<211> 346
<212> PRT
<213> Streptococcus pyogenes

<400> 105
Met Ile Ala Ile Glu Lys Ile Glu Lys Leu Ser Lys Glu Asn Leu Gly
1               5                   10                  15

Leu Ile Thr Leu Val Thr Gly Asp Asp Ile Gly Gln Tyr Ser Gln Leu
            20                  25                  30

Lys Ser Arg Leu Met Glu Gln Ile Ala Phe Asp Lys Asp Asp Leu Ala
        35                  40                  45

Tyr Ser Tyr Phe Asp Met Ser Glu Ala Ala Tyr Gln Asp Ala Glu Met
    50                  55                  60

Asp Leu Val Ser Leu Pro Phe Phe Ala Glu Gln Lys Val Val Ile Phe
65                  70                  75                  80

Asp His Leu Leu Asp Ile Thr Thr Asn Lys Lys Ser Phe Leu Lys Glu
                85                  90                  95

Lys Asp Leu Lys Ala Phe Glu Ala Tyr Leu Glu Asn Pro Leu Glu Thr
            100                 105                 110

Thr Arg Leu Ile Ile Phe Ala Pro Gly Lys Leu Asp Ser Lys Arg Arg
        115                 120                 125

Leu Val Lys Leu Leu Lys Arg Asp Ala Leu Val Leu Glu Ala Asn Pro
    130                 135                 140

Leu Lys Glu Ala Glu Leu Arg Thr Tyr Phe Gln Lys Tyr Ser His Gln
145                 150                 155                 160

Leu Gly Leu Gly Phe Glu Ser Gly Ala Phe Asp Gln Leu Leu Leu Lys
                165                 170                 175

Ser Asn Asp Asp Phe Ser Gln Ile Met Lys Asn Met Ala Phe Leu Lys
            180                 185                 190

Ala Tyr Lys Lys Thr Gly Asn Ile Ser Leu Thr Asp Ile Glu Gln Ala
        195                 200                 205

Ile Pro Lys Ser Leu Gln Asp Asn Ile Phe Asp Leu Thr Arg Leu Val
    210                 215                 220

Leu Gly Gly Lys Ile Asp Ala Ala Arg Asp Leu Ile His Asp Leu Arg
225                 230                 235                 240

Leu Ser Gly Glu Asp Asp Ile Lys Leu Ile Ala Ile Met Leu Gly Gln
                245                 250                 255

Phe Arg Leu Phe Leu Gln Leu Thr Ile Leu Ala Arg Asp Val Lys Asn
            260                 265                 270

Glu Gln Gln Leu Val Ile Ser Leu Ser Asp Ile Leu Gly Arg Arg Val
        275                 280                 285

Asn Pro Tyr Gln Val Lys Tyr Ala Leu Lys Asp Ser Arg Thr Leu Ser
    290                 295                 300

Leu Ala Phe Leu Thr Gly Ala Val Lys Thr Leu Ile Glu Thr Asp Tyr
305                 310                 315                 320

Gln Ile Lys Thr Gly Leu Tyr Glu Lys Ser Tyr Leu Val Asp Ile Ala
                325                 330                 335

Leu Leu Lys Ile Met Thr His Ser Gln Lys
            340                 345

<210> 106
<211> 879
<212> DNA
<213> Thermus thermophilus

<400> 106
atggtcatcg ccttcaccgg ggatcccttc ctggcgcggg aggccctctt agaggaggca 60

aggcttaggg gcctttcccg cttcaccgag cccaccccgg aggccctggc ccaggccctc 120

gccccggggc ttttcggggg cgggggggcg atgctggacc tgagggaggt gggggaggcg 180

gagtggaagg ccctaaagcc cctcctggaa agcgtgcccg agggcgtccc cgtcctcctc 240

ctggacccta agccaagccc ctcccgggcg gccttctacc ggaaccggga aaggcgggac 300

ttccccaccc ccaagggaaa ggacctggtg cggcacctgg aaaaccgggc caagcgcctg 360

gggctcaggc tcccgggcgg ggtggcccag tacctggcct ccctggaggg ggacctcgag 420

gccctggaac gggagctgga gaagcttgcc ctcctctccc ctcccctcac cctggagaag 480

gtggagaagg tggtggccct gaggcccccc ctcacgggct ttgacctggt gcgctccgtc 540

ctggagaagg accccaagga ggccctcctg cgcctcaggc gcctcaagga ggagggggag 600

gagcccctca ggctcctcgg ggccctctcc tggcagttcg ccctcctcgc ccgggccttc 660

ttcctcctcc gggaaaaccc caggcccaag gaggaggacc tcgcccgcct cgaggcccac 720

ccctacgccg ccaaaaaggc cctggaggcg gcgaggcgcc ttacggaaga agccctcaag 780

gaggccctgg acgccctcat ggaggcggaa aagagggcca agggggggaa agacccatgg 840

cttgccctgg aggcggcggt cctccgcctc gcccgttga                        879

<210> 107
<211> 879
<212> DNA
<213> Thermus thermophilus

<220>
<221> CDS
<222> (1)..(879)

<400> 107
atg gtc atc gcc ttc acc ggg gat ccc ttc ctg gcg cgg gag gcc ctc    48
Met Val Ile Ala Phe Thr Gly Asp Pro Phe Leu Ala Arg Glu Ala Leu
1               5                   10                  15

tta gag gag gca agg ctt agg ggc ctt tcc cgc ttc acc gag ccc acc    96

Leu Glu Glu Ala Arg Leu Arg Gly Leu Ser Arg Phe Thr Glu Pro Thr
            20                  25                  30

ccg gag gcc ctg gcc cag gcc ctc gcc ccg ggg ctt ttc ggg ggc ggg 144
Pro Glu Ala Leu Ala Gln Ala Leu Ala Pro Gly Leu Phe Gly Gly Gly
        35                  40                  45

ggg gcg atg ctg gac ctg agg gag gtg ggg gag gcg gag tgg aag gcc 192
Gly Ala Met Leu Asp Leu Arg Glu Val Gly Glu Ala Glu Trp Lys Ala
    50                  55                  60

cta aag ccc ctc ctg gaa agc gtg ccc gag ggc gtc ccc gtc ctc ctc 240
Leu Lys Pro Leu Leu Glu Ser Val Pro Glu Gly Val Pro Val Leu Leu
65                  70                  75                  80

ctg gac cct aag cca agc ccc tcc cgg gcg gcc ttc tac cgg aac cgg 288
Leu Asp Pro Lys Pro Ser Pro Ser Arg Ala Ala Phe Tyr Arg Asn Arg
                85                  90                  95

gaa agg cgg gac ttc ccc acc ccc aag gga aag gac ctg gtg cgg cac 336
Glu Arg Arg Asp Phe Pro Thr Pro Lys Gly Lys Asp Leu Val Arg His
            100                 105                 110

ctg gaa aac cgg gcc aag cgc ctg ggg ctc agg ctc ccg ggc ggg gtg 384
Leu Glu Asn Arg Ala Lys Arg Leu Gly Leu Arg Leu Pro Gly Gly Val
        115                 120                 125

gcc cag tac ctg gcc tcc ctg gag ggg gac ctc gag gcc ctg gaa cgg 432
Ala Gln Tyr Leu Ala Ser Leu Glu Gly Asp Leu Glu Ala Leu Glu Arg
    130                 135                 140

gag ctg gag aag ctt gcc ctc ctc tcc cct ccc ctc acc ctg gag aag 480
Glu Leu Glu Lys Leu Ala Leu Leu Ser Pro Pro Leu Thr Leu Glu Lys
145                 150                 155                 160

gtg gag aag gtg gtg gcc ctg agg ccc ccc ctc acg ggc ttt gac ctg 528
Val Glu Lys Val Val Ala Leu Arg Pro Pro Leu Thr Gly Phe Asp Leu
                165                 170                 175

gtg cgc tcc gtc ctg gag aag gac ccc aag gag gcc ctc ctg cgc ctc 576
Val Arg Ser Val Leu Glu Lys Asp Pro Lys Glu Ala Leu Leu Arg Leu
            180                 185                 190

agg cgc ctc aag gag gag ggg gag gag ccc ctc agg ctc ctc ggg gcc 624
Arg Arg Leu Lys Glu Glu Gly Glu Glu Pro Leu Arg Leu Leu Gly Ala
        195                 200                 205

ctc tcc tgg cag ttc gcc ctc ctc gcc cgg gcc ttc ttc ctc ctc cgg 672
Leu Ser Trp Gln Phe Ala Leu Leu Ala Arg Ala Phe Phe Leu Leu Arg
    210                 215                 220

gaa aac ccc agg ccc aag gag gag gac ctc gcc cgc ctc gag gcc cac 720
Glu Asn Pro Arg Pro Lys Glu Glu Asp Leu Ala Arg Leu Glu Ala His
225                 230                 235                 240

ccc tac gcc gcc aaa aag gcc ctg gag gcg gcg agg cgc ctt acg gaa 768
Pro Tyr Ala Ala Lys Lys Ala Leu Glu Ala Ala Arg Arg Leu Thr Glu
                245                 250                 255

gaa gcc ctc aag gag gcc ctg gac gcc ctc atg gag gcg gaa aag agg 816
Glu Ala Leu Lys Glu Ala Leu Asp Ala Leu Met Glu Ala Glu Lys Arg
            260                 265                 270

gcc aag ggg ggg aaa gac cca tgg ctt gcc ctg gag gcg gcg gtc ctc 864
Ala Lys Gly Gly Lys Asp Pro Trp Leu Ala Leu Glu Ala Ala Val Leu
        275                 280                 285

cgc ctc gcc cgt tga 879

Arg Leu Ala Arg
    290

<210> 108
<211> 292
<212> PRT
<213> Thermus thermophilus

<400> 108
Met Val Ile Ala Phe Thr Gly Asp Pro Phe Leu Ala Arg Glu Ala Leu
1               5                   10                  15

Leu Glu Glu Ala Arg Leu Arg Gly Leu Ser Arg Phe Thr Glu Pro Thr
            20                  25                  30

Pro Glu Ala Leu Ala Gln Ala Leu Ala Pro Gly Leu Phe Gly Gly Gly
        35                  40                  45

Gly Ala Met Leu Asp Leu Arg Glu Val Gly Glu Ala Glu Trp Lys Ala
    50                  55                  60

Leu Lys Pro Leu Leu Glu Ser Val Pro Glu Gly Val Pro Val Leu Leu
65                  70                  75                  80

Leu Asp Pro Lys Pro Ser Pro Ser Arg Ala Ala Phe Tyr Arg Asn Arg
                85                  90                  95

Glu Arg Arg Asp Phe Pro Thr Pro Lys Gly Lys Asp Leu Val Arg His
            100                 105                 110

Leu Glu Asn Arg Ala Lys Arg Leu Gly Leu Arg Leu Pro Gly Gly Val
        115                 120                 125

Ala Gln Tyr Leu Ala Ser Leu Glu Gly Asp Leu Glu Ala Leu Glu Arg
    130                 135                 140

Glu Leu Glu Lys Leu Ala Leu Leu Ser Pro Pro Leu Thr Leu Glu Lys
145                 150                 155                 160

Val Glu Lys Val Val Ala Leu Arg Pro Pro Leu Thr Gly Phe Asp Leu
                165                 170                 175

Val Arg Ser Val Leu Glu Lys Asp Pro Lys Glu Ala Leu Leu Arg Leu
            180                 185                 190

Arg Arg Leu Lys Glu Glu Gly Glu Glu Pro Leu Arg Leu Leu Gly Ala
        195                 200                 205

Leu Ser Trp Gln Phe Ala Leu Leu Ala Arg Ala Phe Phe Leu Leu Arg
    210                 215                 220

Glu Asn Pro Arg Pro Lys Glu Glu Asp Leu Ala Arg Leu Glu Ala His
225                 230                 235                 240

Pro Tyr Ala Ala Lys Lys Ala Leu Glu Ala Ala Arg Arg Leu Thr Glu
                245                 250                 255

Glu Ala Leu Lys Glu Ala Leu Asp Ala Leu Met Glu Ala Glu Lys Arg
            260                 265                 270

Ala Lys Gly Gly Lys Asp Pro Trp Leu Ala Leu Glu Ala Ala Val Leu
        275                 280                 285

Arg Leu Ala Arg
    290

<210> 109
<211> 1035
<212> DNA
<213> Yersinia pestis

<400> 109
atgatccgga tttaccctga acaacttgtc gcgcagctcc atgaggggct acgcgcttgt 60

tatctattgt gtggcaatga acccttgttg ctacaggaaa gtcaggacca cattcgtcgg 120

gtggcatcgc agcacgattt cactgagcat ttcagctttg cgttggacgc gcacaccgaa 180

tgggagcata tttttagcct ttgccaggca ctgagtttgt tcgccagccg ccagacactg 240

ctgctgagtt tcccagacag tgggctgacc gcgccaatta gcgagcagtt ggttaaatta 300

tctggcctgt tacacccaga cattttattg atattgcggg cgaataagtt gaccaaagcg 360

caagagaaca gcgcctggtt taaagctctc agtaaaaatg gggtatttgt cagttgccag 420

acaccagaac aggcccagct tccccgctgg gtgagcgctc gcgccaagag cctcaacttg 480

aacgtagacg atgccgccat tcaattactt tgctactgct atgaaggtaa cttgctggcc 540

ttatctcagg cgttagaacg gctctcgctg ctctaccccg acggtaagtt aaccctgcct 600

aaagtagaac aagccgtcaa cgacgctgcg catttcacac cataccattg gcttgatgcc 660

ttgttgatgg ggaagagcaa gcgggcttgg catattttgc agcaattaca gcaagaagac 720

agtgaacccg ttattttact gcgtaccgta caacgtgaac tgctcctgct gttagcgctc 780

aaacgtcaga tggaacaagt tccattgcgc gcattattcg atcaacacaa aatttggcag 840

aaccggcggc ccatgatgac tcaggcactg caacggctct cccttcagca actgcaacag 900

gcggttcatc tgctcacaca gatggaaatc cggctgaaac aggattatgg tcaatcaatc 960

tggcctgagt tagaaacgct atctatgctg atgtgtggaa aaacattacc tgagagcttt 1020

tttgatgccc attaa 1035

<210> 110
<211> 1035
<212> DNA
<213> Yersinia pestis

<220>
<221> CDS
<222> (1)..(1035)

<400> 110
atg atc cgg att tac cct gaa caa ctt gtc gcg cag ctc cat gag ggg 48
Met Ile Arg Ile Tyr Pro Glu Gln Leu Val Ala Gln Leu His Glu Gly
1               5                   10                  15

cta cgc gct tgt tat cta ttg tgt ggc aat gaa ccc ttg ttg cta cag 96
Leu Arg Ala Cys Tyr Leu Leu Cys Gly Asn Glu Pro Leu Leu Leu Gln
            20                  25                  30

gaa agt cag gac cac att cgt cgg gtg gca tcg cag cac gat ttc act 144
Glu Ser Gln Asp His Ile Arg Arg Val Ala Ser Gln His Asp Phe Thr
        35                  40                  45

gag cat ttc agc ttt gcg ttg gac gcg cac acc gaa tgg gag cat att 192
Glu His Phe Ser Phe Ala Leu Asp Ala His Thr Glu Trp Glu His Ile
    50                  55                  60

ttt agc ctt tgc cag gca ctg agt ttg ttc gcc agc cgc cag aca ctg 240
Phe Ser Leu Cys Gln Ala Leu Ser Leu Phe Ala Ser Arg Gln Thr Leu
65                  70                  75                  80

ctg ctg agt ttc cca gac agt ggg ctg acc gcg cca att agc gag cag 288
Leu Leu Ser Phe Pro Asp Ser Gly Leu Thr Ala Pro Ile Ser Glu Gln
                85                  90                  95

ttg gtt aaa tta tct ggc ctg tta cac cca gac att tta ttg ata ttg 336
Leu Val Lys Leu Ser Gly Leu Leu His Pro Asp Ile Leu Leu Ile Leu
            100                 105                 110

cgg gcg aat aag ttg acc aaa gcg caa gag aac agc gcc tgg ttt aaa 384
Arg Ala Asn Lys Leu Thr Lys Ala Gln Glu Asn Ser Ala Trp Phe Lys
        115                 120                 125

gct ctc agt aaa aat ggg gta ttt gtc agt tgc cag aca cca gaa cag 432
Ala Leu Ser Lys Asn Gly Val Phe Val Ser Cys Gln Thr Pro Glu Gln
    130                 135                 140

gcc cag ctt ccc cgc tgg gtg agc gct cgc gcc aag agc ctc aac ttg 480
Ala Gln Leu Pro Arg Trp Val Ser Ala Arg Ala Lys Ser Leu Asn Leu
145                 150                 155                 160

aac gta gac gat gcc gcc att caa tta ctt tgc tac tgc tat gaa ggt 528
Asn Val Asp Asp Ala Ala Ile Gln Leu Leu Cys Tyr Cys Tyr Glu Gly
                165                 170                 175

aac ttg ctg gcc tta tct cag gcg tta gaa cgg ctc tcg ctg ctc tac 576
Asn Leu Leu Ala Leu Ser Gln Ala Leu Glu Arg Leu Ser Leu Leu Tyr
            180                 185                 190

ccc gac ggt aag tta acc ctg cct aaa gta gaa caa gcc gtc aac gac 624
Pro Asp Gly Lys Leu Thr Leu Pro Lys Val Glu Gln Ala Val Asn Asp
        195                 200                 205

gct gcg cat ttc aca cca tac cat tgg ctt gat gcc ttg ttg atg ggg 672
Ala Ala His Phe Thr Pro Tyr His Trp Leu Asp Ala Leu Leu Met Gly
    210                 215                 220

aag agc aag cgg gct tgg cat att ttg cag caa tta cag caa gaa gac 720
Lys Ser Lys Arg Ala Trp His Ile Leu Gln Gln Leu Gln Gln Glu Asp
225                 230                 235                 240

agt gaa ccc gtt att tta ctg cgt acc gta caa cgt gaa ctg ctc ctg 768
Ser Glu Pro Val Ile Leu Leu Arg Thr Val Gln Arg Glu Leu Leu Leu
                245                 250                 255

ctg tta gcg ctc aaa cgt cag atg gaa caa gtt cca ttg cgc gca tta 816
Leu Leu Ala Leu Lys Arg Gln Met Glu Gln Val Pro Leu Arg Ala Leu
            260                 265                 270

ttc gat caa cac aaa att tgg cag aac cgg cgg ccc atg atg act cag 864
Phe Asp Gln His Lys Ile Trp Gln Asn Arg Arg Pro Met Met Thr Gln
        275                 280                 285

gca ctg caa cgg ctc tcc ctt cag caa ctg caa cag gcg gtt cat ctg 912
Ala Leu Gln Arg Leu Ser Leu Gln Gln Leu Gln Gln Ala Val His Leu
    290                 295                 300

ctc aca cag atg gaa atc cgg ctg aaa cag gat tat ggt caa tca atc 960
Leu Thr Gln Met Glu Ile Arg Leu Lys Gln Asp Tyr Gly Gln Ser Ile
305                 310                 315                 320

tgg cct gag tta gaa acg cta tct atg ctg atg tgt gga aaa aca tta 1008
Trp Pro Glu Leu Glu Thr Leu Ser Met Leu Met Cys Gly Lys Thr Leu
                325                 330                 335

cct gag agc ttt ttt gat gcc cat taa                                    1035
Pro Glu Ser Phe Phe Asp Ala His
            340                 345

<210> 111
<211> 344
<212> PRT
<213> Yersinia pestis

<400> 111
Met Ile Arg Ile Tyr Pro Glu Gln Leu Val Ala Gln Leu His Glu Gly
1               5                   10                  15

Leu Arg Ala Cys Tyr Leu Leu Cys Gly Asn Glu Pro Leu Leu Leu Gln
            20                  25                  30

Glu Ser Gln Asp His Ile Arg Arg Val Ala Ser Gln His Asp Phe Thr
        35                  40                  45

Glu His Phe Ser Phe Ala Leu Asp Ala His Thr Glu Trp Glu His Ile
    50                  55                  60

Phe Ser Leu Cys Gln Ala Leu Ser Leu Phe Ala Ser Arg Gln Thr Leu
65                  70                  75                  80

Leu Leu Ser Phe Pro Asp Ser Gly Leu Thr Ala Pro Ile Ser Glu Gln
                85                  90                  95

Leu Val Lys Leu Ser Gly Leu Leu His Pro Asp Ile Leu Leu Ile Leu
            100                 105                 110

Arg Ala Asn Lys Leu Thr Lys Ala Gln Glu Asn Ser Ala Trp Phe Lys
        115                 120                 125

Ala Leu Ser Lys Asn Gly Val Phe Val Ser Cys Gln Thr Pro Glu Gln
    130                 135                 140

Ala Gln Leu Pro Arg Trp Val Ser Ala Arg Ala Lys Ser Leu Asn Leu
145                 150                 155                 160

Asn Val Asp Asp Ala Ala Ile Gln Leu Leu Cys Tyr Cys Tyr Glu Gly
                165                 170                 175

Asn Leu Leu Ala Leu Ser Gln Ala Leu Glu Arg Leu Ser Leu Leu Tyr
            180                 185                 190

Pro Asp Gly Lys Leu Thr Leu Pro Lys Val Glu Gln Ala Val Asn Asp
        195                 200                 205

Ala Ala His Phe Thr Pro Tyr His Trp Leu Asp Ala Leu Leu Met Gly
    210                 215                 220

Lys Ser Lys Arg Ala Trp His Ile Leu Gln Gln Leu Gln Gln Glu Asp
225                 230                 235                 240

Ser Glu Pro Val Ile Leu Leu Arg Thr Val Gln Arg Glu Leu Leu Leu
                245                 250                 255

Leu Leu Ala Leu Lys Arg Gln Met Glu Gln Val Pro Leu Arg Ala Leu
            260                 265                 270

Phe Asp Gln His Lys Ile Trp Gln Asn Arg Arg Pro Met Met Thr Gln
        275                 280                 285

Ala Leu Gln Arg Leu Ser Leu Gln Gln Leu Gln Gln Ala Val His Leu
    290                 295                 300

Leu Thr Gln Met Glu Ile Arg Leu Lys Gln Asp Tyr Gly Gln Ser Ile
305                 310                 315                 320

Trp Pro Glu Leu Glu Thr Leu Ser Met Leu Met Cys Gly Lys Thr Leu
                325                 330                 335

Pro Glu Ser Phe Phe Asp Ala His
            340

<210> 112
<211> 1038
<212> DNA
<213> Streptococcus agalactiae

<400> 112
atgattgcga ttgaagaaat tgggagaatt accccagata atttagggtt agtaactgtt 60

ttagcaggag aagatttagg gcaatatgct cagatgaaag agaagctatt tcaggtaatt 120

ggattcaata aagacgattt agcctattct tattttgatt taagtgaaga agactatcaa 180

aatgcggaat tagacttaga aagtttacct tttttatcag actacaaggt tgttattttt 240

gatcagtttc aagatattac aactgataaa aaaacttatt tagatgaaca agcaatgaag 300

cgcttggaag cctacttgca gaatcctgta gatactacga gacttgttat ttgtgctcca 360

ggaaaacttg atggaaaacg tcgacttgta aagttattga aacgtgatgc tcgtgtttta 420

gaggctaata ctttaaaaga atcagattta aagacgtatt ttcaaaaata tgctcaccaa 480

gaaggattag tttttgaagc aggtgttttt gatgagttat tgattaagtc taactatgat 540

tttagtgata ctttaacgaa tatcgctttc ttaaagtcat ataaaacaga cggtcatatt 600

tcgtccaatg atgttagaga ggctatccct aaaagcctgc aagataatat atttgatttg 660

actcaagatg tcttacttgg caggatagat ttaccgcgtg atttagtaag agatttacgt 720

ctacaaggcg aagacgaaat caaattgatt gcaatcatgt taggacagtt tcggatgttt 780

ttgcaagtta aaatattggc tagtaaaggg aagtcagaga gtcaaattgt ttctgaacta 840

tcccattaca taggacgtaa aatcaaccct tatcaagtaa aatttgctgt cagagattct 900

agaaaccttc cccttgcttt tttaaaagaa gcgattagga ttttaatcga gactgactat 960

gctattaaaa gagggactta tgataaggat tatctctttg atttagcctt attaaaaatt 1020

gctcataaaa agccctaa                                                1038

<210> 113
<211> 1038
<212> DNA
<213> Streptococcus agalactiae

<220>
<221> CDS
<222> (1)..(1038)

<400> 113
atg att gcg att gaa gaa att ggg aga att acc cca gat aat tta ggg    48
Met Ile Ala Ile Glu Glu Ile Gly Arg Ile Thr Pro Asp Asn Leu Gly
1               5                   10                  15

tta gta act gtt tta gca gga gaa gat tta ggg caa tat gct cag atg 96
Leu Val Thr Val Leu Ala Gly Glu Asp Leu Gly Gln Tyr Ala Gln Met
            20                  25                  30

aaa gag aag cta ttt cag gta att gga ttc aat aaa gac gat tta gcc 144
Lys Glu Lys Leu Phe Gln Val Ile Gly Phe Asn Lys Asp Asp Leu Ala
        35                  40                  45

tat tct tat ttt gat tta agt gaa gaa gac tat caa aat gcg gaa tta 192
Tyr Ser Tyr Phe Asp Leu Ser Glu Glu Asp Tyr Gln Asn Ala Glu Leu
    50                  55                  60

gac tta gaa agt tta cct ttt tta tca gac tac aag gtt gtt att ttt 240
Asp Leu Glu Ser Leu Pro Phe Leu Ser Asp Tyr Lys Val Val Ile Phe
65                  70                  75                  80

gat cag ttt caa gat att aca act gat aaa aaa act tat tta gat gaa 288
Asp Gln Phe Gln Asp Ile Thr Thr Asp Lys Lys Thr Tyr Leu Asp Glu
                85                  90                  95

caa gca atg aag cgc ttg gaa gcc tac ttg cag aat cct gta gat act 336
Gln Ala Met Lys Arg Leu Glu Ala Tyr Leu Gln Asn Pro Val Asp Thr
            100                 105                 110

acg aga ctt gtt att tgt gct cca gga aaa ctt gat gga aaa cgt cga 384
Thr Arg Leu Val Ile Cys Ala Pro Gly Lys Leu Asp Gly Lys Arg Arg
        115                 120                 125

ctt gta aag tta ttg aaa cgt gat gct cgt gtt tta gag gct aat act 432
Leu Val Lys Leu Leu Lys Arg Asp Ala Arg Val Leu Glu Ala Asn Thr
    130                 135                 140

tta aaa gaa tca gat tta aag acg tat ttt caa aaa tat gct cac caa 480
Leu Lys Glu Ser Asp Leu Lys Thr Tyr Phe Gln Lys Tyr Ala His Gln
145                 150                 155                 160

gaa gga tta gtt ttt gaa gca ggt gtt ttt gat gag tta ttg att aag 528
Glu Gly Leu Val Phe Glu Ala Gly Val Phe Asp Glu Leu Leu Ile Lys
                165                 170                 175

tct aac tat gat ttt agt gat act tta acg aat atc gct ttc tta aag 576
Ser Asn Tyr Asp Phe Ser Asp Thr Leu Thr Asn Ile Ala Phe Leu Lys
            180                 185                 190

tca tat aaa aca gac ggt cat att tcg tcc aat gat gtt aga gag gct 624
Ser Tyr Lys Thr Asp Gly His Ile Ser Ser Asn Asp Val Arg Glu Ala
        195                 200                 205

atc cct aaa agc ctg caa gat aat ata ttt gat ttg act caa gat gtc 672
Ile Pro Lys Ser Leu Gln Asp Asn Ile Phe Asp Leu Thr Gln Asp Val
    210                 215                 220

tta ctt ggc agg ata gat tta ccg cgt gat tta gta aga gat tta cgt 720
Leu Leu Gly Arg Ile Asp Leu Pro Arg Asp Leu Val Arg Asp Leu Arg
225                 230                 235                 240

cta caa ggc gaa gac gaa atc aaa ttg att gca atc atg tta gga cag 768
Leu Gln Gly Glu Asp Glu Ile Lys Leu Ile Ala Ile Met Leu Gly Gln
                245                 250                 255

ttt cgg atg ttt ttg caa gtt aaa ata ttg gct agt aaa ggg aag tca 816
Phe Arg Met Phe Leu Gln Val Lys Ile Leu Ala Ser Lys Gly Lys Ser
            260                 265                 270

gag agt caa att gtt tct gaa cta tcc cat tac ata gga cgt aaa atc 864
Glu Ser Gln Ile Val Ser Glu Leu Ser His Tyr Ile Gly Arg Lys Ile
        275                 280                 285

aac cct tat caa gta aaa ttt gct gtc aga gat tct aga aac ctt ccc 912
Asn Pro Tyr Gln Val Lys Phe Ala Val Arg Asp Ser Arg Asn Leu Pro
    290                 295                 300

ctt gct ttt tta aaa gaa gcg att agg att tta atc gag act gac tat 960
Leu Ala Phe Leu Lys Glu Ala Ile Arg Ile Leu Ile Glu Thr Asp Tyr
305                 310                 315                 320

gct att aaa aga ggg act tat gat aag gat tat ctc ttt gat tta gcc 1008
Ala Ile Lys Arg Gly Thr Tyr Asp Lys Asp Tyr Leu Phe Asp Leu Ala
                325                 330                 335

tta tta aaa att gct cat aaa aag ccc taa 1038
Leu Leu Lys Ile Ala His Lys Lys Pro
            340                 345

<210> 114
<211> 345
<212> PRT
<213> Streptococcus agalactiae

<400> 114
Met Ile Ala Ile Glu Glu Ile Gly Arg Ile Thr Pro Asp Asn Leu Gly
1               5                   10                  15

Leu Val Thr Val Leu Ala Gly Glu Asp Leu Gly Gln Tyr Ala Gln Met
            20                  25                  30

Lys Glu Lys Leu Phe Gln Val Ile Gly Phe Asn Lys Asp Asp Leu Ala
        35                  40                  45

Tyr Ser Tyr Phe Asp Leu Ser Glu Glu Asp Tyr Gln Asn Ala Glu Leu
    50                  55                  60

Asp Leu Glu Ser Leu Pro Phe Leu Ser Asp Tyr Lys Val Val Ile Phe
65                  70                  75                  80

Asp Gln Phe Gln Asp Ile Thr Thr Asp Lys Lys Thr Tyr Leu Asp Glu
                85                  90                  95

Gln Ala Met Lys Arg Leu Glu Ala Tyr Leu Gln Asn Pro Val Asp Thr
            100                 105                 110

Thr Arg Leu Val Ile Cys Ala Pro Gly Lys Leu Asp Gly Lys Arg Arg
        115                 120                 125

Leu Val Lys Leu Leu Lys Arg Asp Ala Arg Val Leu Glu Ala Asn Thr
    130                 135                 140

Leu Lys Glu Ser Asp Leu Lys Thr Tyr Phe Gln Lys Tyr Ala His Gln
145                 150                 155                 160

Glu Gly Leu Val Phe Glu Ala Gly Val Phe Asp Glu Leu Leu Ile Lys
                165                 170                 175

Ser Asn Tyr Asp Phe Ser Asp Thr Leu Thr Asn Ile Ala Phe Leu Lys
            180                 185                 190

Ser Tyr Lys Thr Asp Gly His Ile Ser Ser Asn Asp Val Arg Glu Ala
        195                 200                 205

Ile Pro Lys Ser Leu Gln Asp Asn Ile Phe Asp Leu Thr Gln Asp Val
    210                 215                 220

Leu Leu Gly Arg Ile Asp Leu Pro Arg Asp Leu Val Arg Asp Leu Arg
225                 230                 235                 240

Leu Gln Gly Glu Asp Glu Ile Lys Leu Ile Ala Ile Met Leu Gly Gln
                245                 250                 255

Phe Arg Met Phe Leu Gln Val Lys Ile Leu Ala Ser Lys Gly Lys Ser
            260                 265                 270

Glu Ser Gln Ile Val Ser Glu Leu Ser His Tyr Ile Gly Arg Lys Ile
        275                 280                 285

Asn Pro Tyr Gln Val Lys Phe Ala Val Arg Asp Ser Arg Asn Leu Pro
    290                 295                 300

Leu Ala Phe Leu Lys Glu Ala Ile Arg Ile Leu Ile Glu Thr Asp Tyr
305                 310                 315                 320

Ala Ile Lys Arg Gly Thr Tyr Asp Lys Asp Tyr Leu Phe Asp Leu Ala
                325                 330                 335

Leu Leu Lys Ile Ala His Lys Lys Pro
            340                 345

<210> 115
<211> 1038
<212> DNA
<213> Streptococcus pneumonia

<400> 115
atgctagcca ttgaagaaag tcagaagttg actttatcaa atttaccgag cctgagccta 60

tttacaggga cagatcaggg tcagtttgaa gtgatgaaga gtcaaatgtt gaaacagatt 120

gggtatgatt ctgctgacct caactttgcc tactttgata tgaaagaagt agtttacaag 180

gatgtggaac tggagttggt cagccttcct ttctttgcgg atgagaaaat cgtgatatta 240

gattatttta tggatatcac gactgctaag aaacgctttt tgacagatga tgagcttaag 300

tcatttgagg aataccttga caatccttct ccaacaacca agttgataat ctttgcagaa 360

ggaaagctgg atagcaaaag acggttagtc aaattactta agcgtgatgc caaggccttc 420

gatgcagtag aagtaaaaga acaagaattg cgccagtact tccaaaagtg gagtcagaaa 480

caaggtctgc agtttaccaa tcattctttt gaaaatctcc tcatcaagtc ggggtttcaa 540

tttagcgaaa tccagaaaaa tcttctcttt ttacagtcct ataaggcgaa ttctgttatt 600

gaggaagagg atattgttaa cgcaattccc aagaccttgc aggacaatat ttttgattta 660

actcagttta ttctgactaa aaagatggat caggcgcgcg atttggtgag agacttgacc 720

ttgcaagggg aagatgaaat caaactgatt gcagtcatgc tgggacaatt tcggactttt 780

actcaggtga agattttggc ggagtctggc caaacagaat cgcagattgc aagtagttta 840

ggtagttatc tgggacgtaa cccaaatcct tatcaaatca agtttgcatt aagagattcg 900

agaggacttt ctttgagctt tttgaagcaa gctatttcct atttgattga gacagactat 960

cagattaaga caggtcttta tgaaaaaggt ttcctttttg aaaaggcact cttacagatt 1020

gctagtcagg tcaattga                                                1038

<210> 116
<211> 1038
<212> DNA

<213> Streptococcus pneumonia

<220>
<221> CDS
<222> (1)..(1038)

<400> 116
atg cta gcc att gaa gaa agt cag aag ttg act tta tca aat tta ccg     48
Met Leu Ala Ile Glu Glu Ser Gln Lys Leu Thr Leu Ser Asn Leu Pro
1               5                   10                  15

agc ctg agc cta ttt aca ggg aca gat cag ggt cag ttt gaa gtg atg     96
Ser Leu Ser Leu Phe Thr Gly Thr Asp Gln Gly Gln Phe Glu Val Met
            20                  25                  30

aag agt caa atg ttg aaa cag att ggg tat gat tct gct gac ctc aac     144
Lys Ser Gln Met Leu Lys Gln Ile Gly Tyr Asp Ser Ala Asp Leu Asn
        35                  40                  45

ttt gcc tac ttt gat atg aaa gaa gta gtt tac aag gat gtg gaa ctg     192
Phe Ala Tyr Phe Asp Met Lys Glu Val Val Tyr Lys Asp Val Glu Leu
    50                  55                  60

gag ttg gtc agc ctt cct ttc ttt gcg gat gag aaa atc gtg ata tta     240
Glu Leu Val Ser Leu Pro Phe Phe Ala Asp Glu Lys Ile Val Ile Leu
65                  70                  75                  80

gat tat ttt atg gat atc acg act gct aag aaa cgc ttt ttg aca gat     288
Asp Tyr Phe Met Asp Ile Thr Thr Ala Lys Lys Arg Phe Leu Thr Asp
                85                  90                  95

gat gag ctt aag tca ttt gag gaa tac ctt gac aat cct tct cca aca     336
Asp Glu Leu Lys Ser Phe Glu Glu Tyr Leu Asp Asn Pro Ser Pro Thr
            100                 105                 110

acc aag ttg ata atc ttt gca gaa gga aag ctg gat agc aaa aga cgg     384
Thr Lys Leu Ile Ile Phe Ala Glu Gly Lys Leu Asp Ser Lys Arg Arg
        115                 120                 125

tta gtc aaa tta ctt aag cgt gat gcc aag gcc ttc gat gca gta gaa     432
Leu Val Lys Leu Leu Lys Arg Asp Ala Lys Ala Phe Asp Ala Val Glu
    130                 135                 140

gta aaa gaa caa gaa ttg cgc cag tac ttc caa aag tgg agt cag aaa     480
Val Lys Glu Gln Glu Leu Arg Gln Tyr Phe Gln Lys Trp Ser Gln Lys
145                 150                 155                 160

caa ggt ctg cag ttt acc aat cat tct ttt gaa aat ctc ctc atc aag     528
Gln Gly Leu Gln Phe Thr Asn His Ser Phe Glu Asn Leu Leu Ile Lys
                165                 170                 175

tcg ggg ttt caa ttt agc gaa atc cag aaa aat ctt ctc ttt tta cag     576
Ser Gly Phe Gln Phe Ser Glu Ile Gln Lys Asn Leu Leu Phe Leu Gln
            180                 185                 190

tcc tat aag gcg aat tct gtt att gag gaa gag gat att gtt aac gca     624
Ser Tyr Lys Ala Asn Ser Val Ile Glu Glu Glu Asp Ile Val Asn Ala
        195                 200                 205

att ccc aag acc ttg cag gac aat att ttt gat tta act cag ttt att     672
Ile Pro Lys Thr Leu Gln Asp Asn Ile Phe Asp Leu Thr Gln Phe Ile
    210                 215                 220

ctg act aaa aag atg gat cag gcg cgc gat ttg gtg aga gac ttg acc     720
Leu Thr Lys Lys Met Asp Gln Ala Arg Asp Leu Val Arg Asp Leu Thr
225                 230                 235                 240

ttg caa ggg gaa gat gaa atc aaa ctg att gca gtc atg ctg gga caa     768

Leu Gln Gly Glu Asp Glu Ile Lys Leu Ile Ala Val Met Leu Gly Gln
                245                 250                 255

ttt cgg act ttt act cag gtg aag att ttg gcg gag tct ggc caa aca     816
Phe Arg Thr Phe Thr Gln Val Lys Ile Leu Ala Glu Ser Gly Gln Thr
            260                 265                 270

gaa tcg cag att gca agt agt tta ggt agt tat ctg gga cgt aac cca     864
Glu Ser Gln Ile Ala Ser Ser Leu Gly Ser Tyr Leu Gly Arg Asn Pro
        275                 280                 285

aat cct tat caa atc aag ttt gca tta aga gat tcg aga gga ctt tct     912
Asn Pro Tyr Gln Ile Lys Phe Ala Leu Arg Asp Ser Arg Gly Leu Ser
    290                 295                 300

ttg agc ttt ttg aag caa gct att tcc tat ttg att gag aca gac tat     960
Leu Ser Phe Leu Lys Gln Ala Ile Ser Tyr Leu Ile Glu Thr Asp Tyr
305                 310                 315                 320

cag att aag aca ggt ctt tat gaa aaa ggt ttc ctt ttt gaa aag gca    1008
Gln Ile Lys Thr Gly Leu Tyr Glu Lys Gly Phe Leu Phe Glu Lys Ala
                325                 330                 335

ctc tta cag att gct agt cag gtc aat tga                            1038
Leu Leu Gln Ile Ala Ser Gln Val Asn
            340                 345


<210> 117
<211> 345
<212> PRT
<213> Streptococcus pneumonia

<400> 117
Met Leu Ala Ile Glu Glu Ser Gln Lys Leu Thr Leu Ser Asn Leu Pro
1               5                   10                  15

Ser Leu Ser Leu Phe Thr Gly Thr Asp Gln Gly Gln Phe Glu Val Met
            20                  25                  30

Lys Ser Gln Met Leu Lys Gln Ile Gly Tyr Asp Ser Ala Asp Leu Asn
        35                  40                  45

Phe Ala Tyr Phe Asp Met Lys Glu Val Val Tyr Lys Asp Val Glu Leu
    50                  55                  60

Glu Leu Val Ser Leu Pro Phe Phe Ala Asp Glu Lys Ile Val Ile Leu
65                  70                  75                  80

Asp Tyr Phe Met Asp Ile Thr Thr Ala Lys Lys Arg Phe Leu Thr Asp
                85                  90                  95

Asp Glu Leu Lys Ser Phe Glu Glu Tyr Leu Asp Asn Pro Ser Pro Thr
            100                 105                 110

Thr Lys Leu Ile Ile Phe Ala Glu Gly Lys Leu Asp Ser Lys Arg Arg
        115                 120                 125

Leu Val Lys Leu Leu Lys Arg Asp Ala Lys Ala Phe Asp Ala Val Glu
    130                 135                 140

Val Lys Glu Gln Glu Leu Arg Gln Tyr Phe Gln Lys Trp Ser Gln Lys
145                 150                 155                 160

Gln Gly Leu Gln Phe Thr Asn His Ser Phe Glu Asn Leu Leu Ile Lys
                165                 170                 175

Ser Gly Phe Gln Phe Ser Glu Ile Gln Lys Asn Leu Leu Phe Leu Gln

180                 185                 190

Ser Tyr Lys Ala Asn Ser Val Ile Glu Glu Glu Asp Ile Val Asn Ala
        195                 200                 205

Ile Pro Lys Thr Leu Gln Asp Asn Ile Phe Asp Leu Thr Gln Phe Ile
    210                 215                 220

Leu Thr Lys Lys Met Asp Gln Ala Arg Asp Leu Val Arg Asp Leu Thr
225                 230                 235                 240

Leu Gln Gly Glu Asp Glu Ile Lys Leu Ile Ala Val Met Leu Gly Gln
                245                 250                 255

Phe Arg Thr Phe Thr Gln Val Lys Ile Leu Ala Glu Ser Gly Gln Thr
            260                 265                 270

Glu Ser Gln Ile Ala Ser Ser Leu Gly Ser Tyr Leu Gly Arg Asn Pro
        275                 280                 285

Asn Pro Tyr Gln Ile Lys Phe Ala Leu Arg Asp Ser Arg Gly Leu Ser
    290                 295                 300

Leu Ser Phe Leu Lys Gln Ala Ile Ser Tyr Leu Ile Glu Thr Asp Tyr
305                 310                 315                 320

Gln Ile Lys Thr Gly Leu Tyr Glu Lys Gly Phe Leu Phe Glu Lys Ala
                325                 330                 335

Leu Leu Gln Ile Ala Ser Gln Val Asn
            340                 345

<210> 118
<211> 1035
<212> DNA
<213> Actinobacillus actinomycetemcomitans

<400> 118
atgaatcggc tatttcctga acagcttgct tcgtcactgg aacggcattt agcccatgtg 60

tattttctgg tgggcgagga tccgttgttg ctcaatgaaa gcgccaatgg tatttaccaa 120

acggcgcttc agcggggatt cgatgaaaaa gttgaactgg acattaatgc ctccaccgac 180

tggaatgatt tattcgagcg ggtgcaatcc atgggcttat ttttcaataa acagctcatt 240

attttagatt tacctgaaaa tgcgaccgca cttttacaga aaaacttatc cgaattcatt 300

tcgttgcttc agcccgatgt gctaccgatt ttccgtttag ccaaactcac taaagccgct 360

gaaaagcagg cgtggtttat ggcggcgaat cagtatgaac cgcaggcggt attggtaaac 420

tgtcaaaccc ccaatgccga acaattgccc cgctgggtgg cgaatcgggc gaagatgttg 480

gggttaagca ttgaacaaga agcggtgcag ttgttgtgtt acagctacga aaacaacctg 540

ttggcactca agcaaacact gcaattactg gatttgcttt atcctgatcg taaactcact 600

tttgcccgcg tgaatagcgt ggtggaacag tcctcggtgt ttacgccgtt ccaatgggtt 660

gatgcgattt taggcggaaa agggaatcgc gcccgccgaa ttctcaccgg tttaaaagat 720

gaagatgttc agccgattat tctcctgcgc acgttgcagc gcgatttaat gactttactg 780

gagatcagta aaccggaaca accccaatcc cttgactcgc ctttacccac cgatcaactg 840

cgtgaacagt ttgatcgcct gaaagtatgg caaaaccgcc gttcattgtt tactcaagcg 900

gtgcaacgtt taacctaccg taaactctat cttttttttc agcaactcgc cgatgtggag 960

cgctgtgcga aacaggaatt tagcgatgat atttggcaac aactggaaga tttatcggta 1020

agatttgccg gttag 1035

<210> 119
<211> 1035
<212> DNA
<213> Actinobacillus actinomycetemcomitans

<220>
<221> CDS
<222> (1)..(1035)

<400> 119
atg aat cgg cta ttt cct gaa cag ctt gct tcg tca ctg gaa cgg cat 48
Met Asn Arg Leu Phe Pro Glu Gln Leu Ala Ser Ser Leu Glu Arg His
1               5                   10                  15

tta gcc cat gtg tat ttt ctg gtg ggc gag gat ccg ttg ttg ctc aat 96
Leu Ala His Val Tyr Phe Leu Val Gly Glu Asp Pro Leu Leu Leu Asn
            20                  25                  30

gaa agc gcc aat ggt att tac caa acg gcg ctt cag cgg gga ttc gat 144
Glu Ser Ala Asn Gly Ile Tyr Gln Thr Ala Leu Gln Arg Gly Phe Asp
        35                  40                  45

gaa aaa gtt gaa ctg gac att aat gcc tcc acc gac tgg aat gat tta 192
Glu Lys Val Glu Leu Asp Ile Asn Ala Ser Thr Asp Trp Asn Asp Leu
    50                  55                  60

ttc gag cgg gtg caa tcc atg ggc tta ttt ttc aat aaa cag ctc att 240
Phe Glu Arg Val Gln Ser Met Gly Leu Phe Phe Asn Lys Gln Leu Ile
65                  70                  75                  80

att tta gat tta cct gaa aat gcg acc gca ctt tta cag aaa aac tta 288
Ile Leu Asp Leu Pro Glu Asn Ala Thr Ala Leu Leu Gln Lys Asn Leu
                85                  90                  95

tcc gaa ttc att tcg ttg ctt cag ccc gat gtg cta ccg att ttc cgt 336
Ser Glu Phe Ile Ser Leu Leu Gln Pro Asp Val Leu Pro Ile Phe Arg
            100                 105                 110

tta gcc aaa ctc act aaa gcc gct gaa aag cag gcg tgg ttt atg gcg 384
Leu Ala Lys Leu Thr Lys Ala Ala Glu Lys Gln Ala Trp Phe Met Ala
        115                 120                 125

gcg aat cag tat gaa ccg cag gcg gta ttg gta aac tgt caa acc ccc 432
Ala Asn Gln Tyr Glu Pro Gln Ala Val Leu Val Asn Cys Gln Thr Pro
    130                 135                 140

aat gcc gaa caa ttg ccc cgc tgg gtg gcg aat cgg gcg aag atg ttg 480
Asn Ala Glu Gln Leu Pro Arg Trp Val Ala Asn Arg Ala Lys Met Leu
145                 150                 155                 160

ggg tta agc att gaa caa gaa gcg gtg cag ttg ttg tgt tac agc tac 528
Gly Leu Ser Ile Glu Gln Glu Ala Val Gln Leu Leu Cys Tyr Ser Tyr
                165                 170                 175

gaa aac aac ctg ttg gca ctc aag caa aca ctg caa tta ctg gat ttg 576
Glu Asn Asn Leu Leu Ala Leu Lys Gln Thr Leu Gln Leu Leu Asp Leu
            180                 185                 190

ctt tat cct gat cgt aaa ctc act ttt gcc cgc gtg aat agc gtg gtg 624
Leu Tyr Pro Asp Arg Lys Leu Thr Phe Ala Arg Val Asn Ser Val Val

195                    200                    205

gaa cag tcc tcg gtg ttt acg ccg ttc caa tgg gtt gat gcg att tta      672
Glu Gln Ser Ser Val Phe Thr Pro Phe Gln Trp Val Asp Ala Ile Leu
    210                 215                 220

ggc gga aaa ggg aat cgc gcc cgc cga att ctc acc ggt tta aaa gat      720
Gly Gly Lys Gly Asn Arg Ala Arg Arg Ile Leu Thr Gly Leu Lys Asp
225                 230                 235                 240

gaa gat gtt cag ccg att att ctc ctg cgc acg ttg cag cgc gat tta      768
Glu Asp Val Gln Pro Ile Ile Leu Leu Arg Thr Leu Gln Arg Asp Leu
                245                 250                 255

atg act tta ctg gag atc agt aaa ccg gaa caa ccc caa tcc ctt gac      816
Met Thr Leu Leu Glu Ile Ser Lys Pro Glu Gln Pro Gln Ser Leu Asp
            260                 265                 270

tcg cct tta ccc acc gat caa ctg cgt gaa cag ttt gat cgc ctg aaa      864
Ser Pro Leu Pro Thr Asp Gln Leu Arg Glu Gln Phe Asp Arg Leu Lys
        275                 280                 285

gta tgg caa aac cgc cgt tca ttg ttt act caa gcg gtg caa cgt tta      912
Val Trp Gln Asn Arg Arg Ser Leu Phe Thr Gln Ala Val Gln Arg Leu
    290                 295                 300

acc tac cgt aaa ctc tat ctt ttt ttt cag caa ctc gcc gat gtg gag      960
Thr Tyr Arg Lys Leu Tyr Leu Phe Phe Gln Gln Leu Ala Asp Val Glu
305                 310                 315                 320

cgc tgt gcg aaa cag gaa ttt agc gat gat att tgg caa caa ctg gaa      1008
Arg Cys Ala Lys Gln Glu Phe Ser Asp Asp Ile Trp Gln Gln Leu Glu
                325                 330                 335

gat tta tcg gta aga ttt gcc ggt tag                                  1035
Asp Leu Ser Val Arg Phe Ala Gly
            340                 345

<210> 120
<211> 344
<212> PRT
<213> Actinobacillus actinomycetemcomitans

<400> 120
Met Asn Arg Leu Phe Pro Glu Gln Leu Ala Ser Ser Leu Glu Arg His
1               5                   10                  15

Leu Ala His Val Tyr Phe Leu Val Gly Glu Asp Pro Leu Leu Leu Asn
            20                  25                  30

Glu Ser Ala Asn Gly Ile Tyr Gln Thr Ala Leu Gln Arg Gly Phe Asp
        35                  40                  45

Glu Lys Val Glu Leu Asp Ile Asn Ala Ser Thr Asp Trp Asn Asp Leu
    50                  55                  60

Phe Glu Arg Val Gln Ser Met Gly Leu Phe Phe Asn Lys Gln Leu Ile
65                  70                  75                  80

Ile Leu Asp Leu Pro Glu Asn Ala Thr Ala Leu Leu Gln Lys Asn Leu
                85                  90                  95

Ser Glu Phe Ile Ser Leu Leu Gln Pro Asp Val Leu Pro Ile Phe Arg
            100                 105                 110

Leu Ala Lys Leu Thr Lys Ala Ala Glu Lys Gln Ala Trp Phe Met Ala
        115                 120                 125

Ala Asn Gln Tyr Glu Pro Gln Ala Val Leu Val Asn Cys Gln Thr Pro
    130                 135                 140

Asn Ala Glu Gln Leu Pro Arg Trp Val Ala Asn Arg Ala Lys Met Leu
145                 150                 155                 160

Gly Leu Ser Ile Glu Gln Glu Ala Val Gln Leu Leu Cys Tyr Ser Tyr
                165                 170                 175

Glu Asn Asn Leu Leu Ala Leu Lys Gln Thr Leu Gln Leu Leu Asp Leu
            180                 185                 190

Leu Tyr Pro Asp Arg Lys Leu Thr Phe Ala Arg Val Asn Ser Val Val
        195                 200                 205

Glu Gln Ser Ser Val Phe Thr Pro Phe Gln Trp Val Asp Ala Ile Leu
    210                 215                 220

Gly Gly Lys Gly Asn Arg Ala Arg Arg Ile Leu Thr Gly Leu Lys Asp
225                 230                 235                 240

Glu Asp Val Gln Pro Ile Ile Leu Leu Arg Thr Leu Gln Arg Asp Leu
                245                 250                 255

Met Thr Leu Leu Glu Ile Ser Lys Pro Glu Gln Pro Gln Ser Leu Asp
            260                 265                 270

Ser Pro Leu Pro Thr Asp Gln Leu Arg Glu Gln Phe Asp Arg Leu Lys
        275                 280                 285

Val Trp Gln Asn Arg Arg Ser Leu Phe Thr Gln Ala Val Gln Arg Leu
    290                 295                 300

Thr Tyr Arg Lys Leu Tyr Leu Phe Phe Gln Gln Leu Ala Asp Val Glu
305                 310                 315                 320

Arg Cys Ala Lys Gln Glu Phe Ser Asp Asp Ile Trp Gln Gln Leu Glu
                325                 330                 335

Asp Leu Ser Val Arg Phe Ala Gly
            340

<210> 121
<211> 1056
<212> DNA
<213> Bordetella pertussis

<400> 121
   atggctgcgc ccctggacag cgacgccctg gccagccacc tcgcacgcgc ggggggccgg 60
   ctggcgccgc tgtacacggt atcgggcgac gagccgctgc tggtcaccga ggccgccgac 120
   gccatccgcg cggcggcgcg cgcggccggc tacaccgacc gcacctccat ggtcatggac 180
   gcgcgcagcg actggagcgc ggtggcggcc gccacgcaaa gcgtctcgct gttcggcgac 240
   cggcgcctgc tggaactgaa aatcccgacc ggcaagcccg gcaagagcgg cggcgagatg 300
   ctggcccggc tggccgacca ggcgcgcgac caggccgacg ccgacacgct cgtggtcgtg 360
   gccctgcccc gcctggacaa ggccacgcgc gaaagcaaat gggcccaggc gctggcgcgc 420
   ggcggcgtca tggccgacat cgccaacgtc gagcgcggcc gcctgccggc atggattggc 480
   atgcgcctgg ggcgccagaa ccagcgcgcc gacaccgcca cgctgcaatg gatggccgac 540
   aaggtcgagg gcaacctgct ggcggcccac caggaaatcc agaagctggg gctgctttat 600
   ccggaaggcc agctggccgc cgaggacgtc gagcgcgccg tgctcaacgt ggcgcgctac 660
   gacgtcttcg ggctgcgcga cgccatgctg gccggcgaca cggcgcgcac ggtgcgcatg 720
   ctcgacggct tgcgcgccga gggcgaagcc ctgccgctgg tgctgtgggc ggtcggcgag 780
   gaaatccgcc tgctggcgcg cgtagcgcag gcgcgccagc aaggccagga cgccggcgca 840
   ctgatgcgcc gtctgcgcat tttcggcgcc cacgaacggc ttgcgctgca agcgctgggc 900
   cgggtcgcac ccggcgcatg gccggcagcc gtgcagcacg cccatgaagt cgatcgcctg 960
   atcaaaggcc tgagcgtacc gggccggctg gccgatccct gggaagaaat gacccggctc 1020
   gcgctgcgcg tggccgcggc cggcgcccga ccgtga 1056

<210> 122
<211> 1056
<212> DNA
<213> Bordetella pertussis

<220>
<221> CDS
<222> (1)..(1056)

<400> 122
atg gct gcg ccc ctg gac agc gac gcc ctg gcc agc cac ctc gca cgc 48
Met Ala Ala Pro Leu Asp Ser Asp Ala Leu Ala Ser His Leu Ala Arg
1               5                   10                  15

gcg ggg ggc cgg ctg gcg ccg ctg tac acg gta tcg ggc gac gag ccg 96
Ala Gly Gly Arg Leu Ala Pro Leu Tyr Thr Val Ser Gly Asp Glu Pro
            20                  25                  30

ctg ctg gtc acc gag gcc gcc gac gcc atc cgc gcg gcg gcg cgc gcg 144
Leu Leu Val Thr Glu Ala Ala Asp Ala Ile Arg Ala Ala Ala Arg Ala
        35                  40                  45

gcc ggc tac acc gac cgc acc tcc atg gtc atg gac gcg cgc agc gac 192
Ala Gly Tyr Thr Asp Arg Thr Ser Met Val Met Asp Ala Arg Ser Asp
    50                  55                  60

tgg agc gcg gtg gcg gcc gcc acg caa agc gtc tcg ctg ttc ggc gac 240
Trp Ser Ala Val Ala Ala Ala Thr Gln Ser Val Ser Leu Phe Gly Asp
65                  70                  75                  80

cgg cgc ctg ctg gaa ctg aaa atc ccg acc ggc aag ccc ggc aag agc 288
Arg Arg Leu Leu Glu Leu Lys Ile Pro Thr Gly Lys Pro Gly Lys Ser
                85                  90                  95

ggc ggc gag atg ctg gcc cgg ctg gcc gac cag gcg cgc gac cag gcc 336
Gly Gly Glu Met Leu Ala Arg Leu Ala Asp Gln Ala Arg Asp Gln Ala
            100                 105                 110

gac gcc gac acg ctc gtg gtc gtg gcc ctg ccc cgc ctg gac aag gcc 384
Asp Ala Asp Thr Leu Val Val Val Ala Leu Pro Arg Leu Asp Lys Ala
        115                 120                 125

acg cgc gaa agc aaa tgg gcc cag gcg ctg gcg cgc ggc ggc gtc atg 432
Thr Arg Glu Ser Lys Trp Ala Gln Ala Leu Ala Arg Gly Gly Val Met
    130                 135                 140

gcc gac atc gcc aac gtc gag cgc ggc cgc ctg ccg gca tgg att ggc 480
Ala Asp Ile Ala Asn Val Glu Arg Gly Arg Leu Pro Ala Trp Ile Gly
145                 150                 155                 160

atg cgc ctg ggg cgc cag aac cag cgc gcc gac acc gcc acg ctg caa     528
Met Arg Leu Gly Arg Gln Asn Gln Arg Ala Asp Thr Ala Thr Leu Gln
                165                 170                 175

tgg atg gcc gac aag gtc gag ggc aac ctg ctg gcg gcc cac cag gaa     576
Trp Met Ala Asp Lys Val Glu Gly Asn Leu Leu Ala Ala His Gln Glu
            180                 185                 190

atc cag aag ctg ggg ctg ctt tat ccg gaa ggc cag ctg gcc gcc gag     624
Ile Gln Lys Leu Gly Leu Leu Tyr Pro Glu Gly Gln Leu Ala Ala Glu
        195                 200                 205

gac gtc gag cgc gcc gtg ctc aac gtg gcg cgc tac gac gtc ttc ggg     672
Asp Val Glu Arg Ala Val Leu Asn Val Ala Arg Tyr Asp Val Phe Gly
    210                 215                 220

ctg cgc gac gcc atg ctg gcc ggc gac acg gcg cgc acg gtg cgc atg     720
Leu Arg Asp Ala Met Leu Ala Gly Asp Thr Ala Arg Thr Val Arg Met
225                 230                 235                 240

ctc gac ggc ttg cgc gcc gag ggc gaa gcc ctg ccg ctg gtg ctg tgg     768
Leu Asp Gly Leu Arg Ala Glu Gly Glu Ala Leu Pro Leu Val Leu Trp
                245                 250                 255

gcg gtc ggc gag gaa atc cgc ctg ctg gcg cgc gta gcg cag gcg cgc     816
Ala Val Gly Glu Glu Ile Arg Leu Leu Ala Arg Val Ala Gln Ala Arg
            260                 265                 270

cag caa ggc cag gac gcc ggc gca ctg atg cgc cgt ctg cgc att ttc     864
Gln Gln Gly Gln Asp Ala Gly Ala Leu Met Arg Arg Leu Arg Ile Phe
        275                 280                 285

ggc gcc cac gaa cgg ctt gcg ctg caa gcg ctg ggc cgg gtc gca ccc     912
Gly Ala His Glu Arg Leu Ala Leu Gln Ala Leu Gly Arg Val Ala Pro
    290                 295                 300

ggc gca tgg ccg gca gcc gtg cag cac gcc cat gaa gtc gat cgc ctg     960
Gly Ala Trp Pro Ala Ala Val Gln His Ala His Glu Val Asp Arg Leu
305                 310                 315                 320

atc aaa ggc ctg agc gta ccg ggc cgg ctg gcc gat ccc tgg gaa gaa     1008
Ile Lys Gly Leu Ser Val Pro Gly Arg Leu Ala Asp Pro Trp Glu Glu
                325                 330                 335

atg acc cgg ctc gcg ctg cgc gtg gcc gcg gcc ggc gcc cga ccg tga     1056
Met Thr Arg Leu Ala Leu Arg Val Ala Ala Ala Gly Ala Arg Pro
            340                 345                 350

<210> 123
<211> 351
<212> PRT
<213> Bordetella pertussis

<400> 123
Met Ala Ala Pro Leu Asp Ser Asp Ala Leu Ala Ser His Leu Ala Arg
1               5                   10                  15

Ala Gly Gly Arg Leu Ala Pro Leu Tyr Thr Val Ser Gly Asp Glu Pro
            20                  25                  30

Leu Leu Val Thr Glu Ala Ala Asp Ala Ile Arg Ala Ala Ala Arg Ala
        35                  40                  45

Ala Gly Tyr Thr Asp Arg Thr Ser Met Val Met Asp Ala Arg Ser Asp
    50                  55                  60

Trp Ser Ala Val Ala Ala Ala Thr Gln Ser Val Ser Leu Phe Gly Asp
65                  70                  75                  80

Arg Arg Leu Leu Glu Leu Lys Ile Pro Thr Gly Lys Pro Gly Lys Ser
                85                  90                  95

Gly Gly Glu Met Leu Ala Arg Leu Ala Asp Gln Ala Arg Asp Gln Ala
            100                 105                 110

Asp Ala Asp Thr Leu Val Val Val Ala Leu Pro Arg Leu Asp Lys Ala
        115                 120                 125

Thr Arg Glu Ser Lys Trp Ala Gln Ala Leu Ala Arg Gly Gly Val Met
    130                 135                 140

Ala Asp Ile Ala Asn Val Glu Arg Gly Arg Leu Pro Ala Trp Ile Gly
145                 150                 155                 160

Met Arg Leu Gly Arg Gln Asn Gln Arg Ala Asp Thr Ala Thr Leu Gln
                165                 170                 175

Trp Met Ala Asp Lys Val Glu Gly Asn Leu Leu Ala Ala His Gln Glu
            180                 185                 190

Ile Gln Lys Leu Gly Leu Leu Tyr Pro Glu Gly Gln Leu Ala Ala Glu
        195                 200                 205

Asp Val Glu Arg Ala Val Leu Asn Val Ala Arg Tyr Asp Val Phe Gly
    210                 215                 220

Leu Arg Asp Ala Met Leu Ala Gly Asp Thr Ala Arg Thr Val Arg Met
225                 230                 235                 240

Leu Asp Gly Leu Arg Ala Glu Gly Glu Ala Leu Pro Leu Val Leu Trp
                245                 250                 255

Ala Val Gly Glu Glu Ile Arg Leu Leu Ala Arg Val Ala Gln Ala Arg
            260                 265                 270

Gln Gln Gly Gln Asp Ala Gly Ala Leu Met Arg Arg Leu Arg Ile Phe
        275                 280                 285

Gly Ala His Glu Arg Leu Ala Leu Gln Ala Leu Gly Arg Val Ala Pro
    290                 295                 300

Gly Ala Trp Pro Ala Ala Val Gln His Ala His Glu Val Asp Arg Leu
305                 310                 315                 320

Ile Lys Gly Leu Ser Val Pro Gly Arg Leu Ala Asp Pro Trp Glu Glu
                325                 330                 335

Met Thr Arg Leu Ala Leu Arg Val Ala Ala Ala Gly Ala Arg Pro
            340                 345                 350

<210> 124
<211> 1011
<212> DNA
<213> Bacillus anthracis

<400> 124
atgagtgata tacataaaaa gattaaaaaa aagcagtttg ctccgttgta tttactgtat 60

ggaacggaag cgttttttat aaatgaaacg ataaagctta ttacaacaga agcgcttgaa 120

gaggaagatc gcgagtttaa tgttgtgaca tatgatttag aagaagcgta tttagaagat 180

gtggttgagg atgcacgtac acttcctttt ttcggagaac gtaaagtgtt attaataaaa 240

tcaccattat ttttaacgtc acaaaaagaa aagttagaac aaaatataaa aattttagaa 300
gaatatatcg ggaaccttc tccattttct attcttgttt ttgttgcgcc ttacgaaaag 360
cttgatgaac gaaaaaaat tacaaaacta ttaaagaaaa cagcagatat agtagaagcg 420
aatgcgatgc aagtgcagga tgttcaaaag tggattgtag ctcgtgcaga ggaagggcat 480
gtacatattg ataatgcagc tgttagttta ttgttagagc ttgtgggaag taatgtgacg 540
atgttggcga aggaaatgga caagttaacg ttatacgtcg gtatgggcgg agagattacg 600
ccgaaacttg ttgcagagct tgtgccaaaa tcagttgaac aaaatgtatt tgctttaaca 660
gaaaaagtgg taaaaaaaga tatcgcaggt gcgatgcaaa ttttggatgg attatttacg 720
cagcaggagg aaccgattaa attgcttgcg ttgttagtaa gtcaattccg cttgctgcat 780
caagtaaaag agttacagca gcgtggttat ggacaaaatc aaattgcttc tcatattggt 840
gtacatccat atcgtgtaaa gttagcgatg aatcaaacga agtttttctc ttttgaagaa 900
ctaaaaaaag tgattataga attggcggaa gctgattata gtatgaagac tggaaaaatg 960
gataagaaac ttgtgcttga gtttttctta atgcgattaa atcatatgtg a 1011

<210> 125
<211> 1011
<212> DNA
<213> Bacillus anthracis

<220>
<221> CDS
<222> (1)..(1011)

<400> 125
atg agt gat ata cat aaa aag att aaa aaa aag cag ttt gct ccg ttg 48
Met Ser Asp Ile His Lys Lys Ile Lys Lys Lys Gln Phe Ala Pro Leu
1               5                   10                  15

tat tta ctg tat gga acg gaa gcg ttt ttt ata aat gaa acg ata aag 96
Tyr Leu Leu Tyr Gly Thr Glu Ala Phe Phe Ile Asn Glu Thr Ile Lys
            20                  25                  30

ctt att aca aca gaa gcg ctt gaa gag gaa gat cgc gag ttt aat gtt 144
Leu Ile Thr Thr Glu Ala Leu Glu Glu Glu Asp Arg Glu Phe Asn Val
        35                  40                  45

gtg aca tat gat tta gaa gaa gcg tat tta gaa gat gtg gtt gag gat 192
Val Thr Tyr Asp Leu Glu Glu Ala Tyr Leu Glu Asp Val Val Glu Asp
    50                  55                  60

gca cgt aca ctt cct ttt ttc gga gaa cgt aaa gtg tta tta ata aaa 240
Ala Arg Thr Leu Pro Phe Phe Gly Glu Arg Lys Val Leu Leu Ile Lys
65                  70                  75                  80

tca cca tta ttt tta acg tca caa aaa gaa aag tta gaa caa aat ata 288
Ser Pro Leu Phe Leu Thr Ser Gln Lys Glu Lys Leu Glu Gln Asn Ile
                85                  90                  95

aaa att tta gaa gaa tat atc ggg gaa cct tct cca ttt tct att ctt 336
Lys Ile Leu Glu Glu Tyr Ile Gly Glu Pro Ser Pro Phe Ser Ile Leu
            100                 105                 110

gtt ttt gtt gcg cct tac gaa aag ctt gat gaa cga aaa aaa att aca 384
Val Phe Val Ala Pro Tyr Glu Lys Leu Asp Glu Arg Lys Lys Ile Thr

115                 120                 125

aaa cta tta aag aaa aca gca gat ata gta gaa gcg aat gcg atg caa     432
Lys Leu Leu Lys Lys Thr Ala Asp Ile Val Glu Ala Asn Ala Met Gln
    130                 135                 140

gtg cag gat gtt caa aag tgg att gta gct cgt gca gag gaa ggg cat     480
Val Gln Asp Val Gln Lys Trp Ile Val Ala Arg Ala Glu Glu Gly His
145                 150                 155                 160

gta cat att gat aat gca gct gtt agt tta ttg tta gag ctt gtg gga     528
Val His Ile Asp Asn Ala Ala Val Ser Leu Leu Leu Glu Leu Val Gly
                165                 170                 175

agt aat gtg acg atg ttg gcg aag gaa atg gac aag tta acg tta tac     576
Ser Asn Val Thr Met Leu Ala Lys Glu Met Asp Lys Leu Thr Leu Tyr
            180                 185                 190

gtc ggt atg ggc gga gag att acg ccg aaa ctt gtt gca gag ctt gtg     624
Val Gly Met Gly Gly Glu Ile Thr Pro Lys Leu Val Ala Glu Leu Val
        195                 200                 205

cca aaa tca gtt gaa caa aat gta ttt gct tta aca gaa aaa gtg gta     672
Pro Lys Ser Val Glu Gln Asn Val Phe Ala Leu Thr Glu Lys Val Val
    210                 215                 220

aaa aaa gat atc gca ggt gcg atg caa att ttg gat gga tta ttt acg     720
Lys Lys Asp Ile Ala Gly Ala Met Gln Ile Leu Asp Gly Leu Phe Thr
225                 230                 235                 240

cag cag gag gaa ccg att aaa ttg ctt gcg ttg tta gta agt caa ttc     768
Gln Gln Glu Glu Pro Ile Lys Leu Leu Ala Leu Leu Val Ser Gln Phe
                245                 250                 255

cgc ttg ctg cat caa gta aaa gag tta cag cag cgt ggt tat gga caa     816
Arg Leu Leu His Gln Val Lys Glu Leu Gln Gln Arg Gly Tyr Gly Gln
            260                 265                 270

aat caa att gct tct cat att ggt gta cat cca tat cgt gta aag tta     864
Asn Gln Ile Ala Ser His Ile Gly Val His Pro Tyr Arg Val Lys Leu
        275                 280                 285

gcg atg aat caa acg aag ttt ttc tct ttt gaa gaa cta aaa aaa gtg     912
Ala Met Asn Gln Thr Lys Phe Phe Ser Phe Glu Glu Leu Lys Lys Val
    290                 295                 300

att ata gaa ttg gcg gaa gct gat tat agt atg aag act gga aaa atg     960
Ile Ile Glu Leu Ala Glu Ala Asp Tyr Ser Met Lys Thr Gly Lys Met
305                 310                 315                 320

gat aag aaa ctt gtg ctt gag ttt ttc tta atg cga tta aat cat atg     1008
Asp Lys Lys Leu Val Leu Glu Phe Phe Leu Met Arg Leu Asn His Met
                325                 330                 335

tga                                                                 1011


<210> 126
<211> 336
<212> PRT
<213> Bacillus anthracis

<400> 126
Met Ser Asp Ile His Lys Lys Ile Lys Lys Lys Gln Phe Ala Pro Leu
1               5                   10                  15

Tyr Leu Leu Tyr Gly Thr Glu Ala Phe Phe Ile Asn Glu Thr Ile Lys

20                  25                  30

Leu Ile Thr Thr Glu Ala Leu Glu Glu Glu Asp Arg Glu Phe Asn Val
            35                  40                  45

Val Thr Tyr Asp Leu Glu Glu Ala Tyr Leu Glu Asp Val Val Glu Asp
        50                  55                  60

Ala Arg Thr Leu Pro Phe Phe Gly Glu Arg Lys Val Leu Leu Ile Lys
65                  70                  75                  80

Ser Pro Leu Phe Leu Thr Ser Gln Lys Glu Lys Leu Glu Gln Asn Ile
                85                  90                  95

Lys Ile Leu Glu Glu Tyr Ile Gly Glu Pro Ser Pro Phe Ser Ile Leu
            100                 105                 110

Val Phe Val Ala Pro Tyr Glu Lys Leu Asp Glu Arg Lys Lys Ile Thr
        115                 120                 125

Lys Leu Leu Lys Lys Thr Ala Asp Ile Val Glu Ala Asn Ala Met Gln
    130                 135                 140

Val Gln Asp Val Gln Lys Trp Ile Val Ala Arg Ala Glu Glu Gly His
145                 150                 155                 160

Val His Ile Asp Asn Ala Ala Val Ser Leu Leu Leu Glu Leu Val Gly
                165                 170                 175

Ser Asn Val Thr Met Leu Ala Lys Glu Met Asp Lys Leu Thr Leu Tyr
            180                 185                 190

Val Gly Met Gly Gly Glu Ile Thr Pro Lys Leu Val Ala Glu Leu Val
        195                 200                 205

Pro Lys Ser Val Glu Gln Asn Val Phe Ala Leu Thr Glu Lys Val Val
    210                 215                 220

Lys Lys Asp Ile Ala Gly Ala Met Gln Ile Leu Asp Gly Leu Phe Thr
225                 230                 235                 240

Gln Gln Glu Glu Pro Ile Lys Leu Leu Ala Leu Leu Val Ser Gln Phe
                245                 250                 255

Arg Leu Leu His Gln Val Lys Glu Leu Gln Gln Arg Gly Tyr Gly Gln
            260                 265                 270

Asn Gln Ile Ala Ser His Ile Gly Val His Pro Tyr Arg Val Lys Leu
        275                 280                 285

Ala Met Asn Gln Thr Lys Phe Phe Ser Phe Glu Glu Leu Lys Lys Val
    290                 295                 300

Ile Ile Glu Leu Ala Glu Ala Asp Tyr Ser Met Lys Thr Gly Lys Met
305                 310                 315                 320

Asp Lys Lys Leu Val Leu Glu Phe Phe Leu Met Arg Leu Asn His Met
                325                 330                 335

<210> 127
<211> 1089
<212> DNA
<213> Burkholderia pseudomallei

<400> 127
atgcaactgc gacttgacgc gctggagccg catctctcca agggcctggc cggactgtac 60

gtcgtctacg gcgacgaacc gctgctcgcg caggaggcgt gcgacaagat ccgcgccgcc 120
gcgcgcgcgg cgggcttcac cgagcgttcg gtgcacaccg tcgagcgcgg cttcgactgg 180
agcacgctga tcggcgcgag ccaggcgatg tcgctgttcg gcgagcggca gctcgtcgag 240
ctgcggattc cgtccggcaa gcccggcaag gacggcgccg acgcgctgaa gacgctcgcc 300
ggcgcggcca acccggacgt gctgatgctc gtcacgctgc cgcggctcga tgcggcgacg 360
cagaagtccg cgtggttcac ggcgctcgcg aacggcggcg tcgcgctgaa gatcgacccc 420
gtcgagcgcg cgcagttgcc gaactggatc ggtcagcggc tcgcgctgca ggggcagcgg 480
gtcgccccgg gcgacgacgg acggcgcgcg ctcgcgttcg tcgccgagcg ggtcgaaggc 540
aatctgctcg ccgcgcatca ggaaatccag aagctcggcc tgctgtatcc ggccggcgcg 600
ctgacgttcg agcagatcca cgacgcggtg ctgaacgtcg cgcgctacga cgtgttcaag 660
ctcaacgagg cgatgctcgc gggcgacgcc gcgcggcttt cgcggatgat cgacggcctg 720
aagggcgagg gcgaggcgct cgtgctcgtg ctgtgggcgg tcgtcgagga gttgcgcacg 780
ctgttgcgga tcaagcgcgg cgtcgcggcc ggcaagccgc ttgccgtgct cgtgcgcgag 840
aaccgcgtgt gggggccgcg cgagcggctc gtcgggcccg cgctttcgcg cgtatcggaa 900
gcggcgctcg agcacgcgct cgcgttcgcc gcgcggctcg accggcaggt caaggggttg 960
tcggccgtgt cgcgcggcgc ggcgcgcggc gagccgccgc ccgatccgtg ggacggcctg 1020
ttccagctcg cgatgaccgt cgcgcgcgcg gccgggccag gcggcgacgc gccgcgccgg 1080
cccgcctga 1089

<210> 128
<211> 1089
<212> DNA
<213> Burkholderia pseudomallei

<220>
<221> CDS
<222> (1)..(1089)

<400> 128
atg caa ctg cga ctt gac gcg ctg gag ccg cat ctc tcc aag ggc ctg 48
Met Gln Leu Arg Leu Asp Ala Leu Glu Pro His Leu Ser Lys Gly Leu
1               5                   10                  15

gcc gga ctg tac gtc gtc tac ggc gac gaa ccg ctg ctc gcg cag gag 96
Ala Gly Leu Tyr Val Val Tyr Gly Asp Glu Pro Leu Leu Ala Gln Glu
            20                  25                  30

gcg tgc gac aag atc cgc gcc gcc gcg cgc gcg gcg ggc ttc acc gag 144
Ala Cys Asp Lys Ile Arg Ala Ala Ala Arg Ala Ala Gly Phe Thr Glu
        35                  40                  45

cgt tcg gtg cac acc gtc gag cgc ggc ttc gac tgg agc acg ctg atc 192
Arg Ser Val His Thr Val Glu Arg Gly Phe Asp Trp Ser Thr Leu Ile
    50                  55                  60

ggc gcg agc cag gcg atg tcg ctg ttc ggc gag cgg cag ctc gtc gag 240
Gly Ala Ser Gln Ala Met Ser Leu Phe Gly Glu Arg Gln Leu Val Glu
65                  70                  75                  80

ctg cgg att ccg tcc ggc aag ccc ggc aag gac ggc gcc gac gcg ctg 288

Leu Arg Ile Pro Ser Gly Lys Pro Gly Lys Asp Gly Ala Asp Ala Leu
                85                  90                  95

aag acg ctc gcc ggc gcg gcc aac ccg gac gtg ctg atg ctc gtc acg     336
Lys Thr Leu Ala Gly Ala Ala Asn Pro Asp Val Leu Met Leu Val Thr
            100                 105                 110

ctg ccg cgg ctc gat gcg gcg acg cag aag tcc gcg tgg ttc acg gcg     384
Leu Pro Arg Leu Asp Ala Ala Thr Gln Lys Ser Ala Trp Phe Thr Ala
        115                 120                 125

ctc gcg aac ggc ggc gtc gcg ctg aag atc gac ccc gtc gag cgc gcg     432
Leu Ala Asn Gly Gly Val Ala Leu Lys Ile Asp Pro Val Glu Arg Ala
    130                 135                 140

cag ttg ccg aac tgg atc ggt cag cgg ctc gcg ctg cag ggg cag cgg     480
Gln Leu Pro Asn Trp Ile Gly Gln Arg Leu Ala Leu Gln Gly Gln Arg
145                 150                 155                 160

gtc gcc ccg ggc gac gac gga cgg cgc gcg ctc gcg ttc gtc gcc gag     528
Val Ala Pro Gly Asp Asp Gly Arg Arg Ala Leu Ala Phe Val Ala Glu
                165                 170                 175

cgg gtc gaa ggc aat ctg ctc gcc gcg cat cag gaa atc cag aag ctc     576
Arg Val Glu Gly Asn Leu Leu Ala Ala His Gln Glu Ile Gln Lys Leu
            180                 185                 190

ggc ctg ctg tat ccg gcc ggc gcg ctg acg ttc gag cag atc cac gac     624
Gly Leu Leu Tyr Pro Ala Gly Ala Leu Thr Phe Glu Gln Ile His Asp
        195                 200                 205

gcg gtg ctg aac gtc gcg cgc tac gac gtg ttc aag ctc aac gag gcg     672
Ala Val Leu Asn Val Ala Arg Tyr Asp Val Phe Lys Leu Asn Glu Ala
    210                 215                 220

atg ctc gcg ggc gac gcc gcg cgg ctt tcg cgg atg atc gac ggc ctg     720
Met Leu Ala Gly Asp Ala Ala Arg Leu Ser Arg Met Ile Asp Gly Leu
225                 230                 235                 240

aag ggc gag ggc gag gcg ctc gtg ctc gtg ctg tgg gcg gtc gtc gag     768
Lys Gly Glu Gly Glu Ala Leu Val Leu Val Leu Trp Ala Val Val Glu
                245                 250                 255

gag ttg cgc acg ctg ttg cgg atc aag cgc ggc gtc gcg gcc ggc aag     816
Glu Leu Arg Thr Leu Leu Arg Ile Lys Arg Gly Val Ala Ala Gly Lys
            260                 265                 270

ccg ctt gcc gtg ctc gtg cgc gag aac cgc gtg tgg ggg ccg cgc gag     864
Pro Leu Ala Val Leu Val Arg Glu Asn Arg Val Trp Gly Pro Arg Glu
        275                 280                 285

cgg ctc gtc ggg ccc gcg ctt tcg cgc gta tcg gaa gcg gcg ctc gag     912
Arg Leu Val Gly Pro Ala Leu Ser Arg Val Ser Glu Ala Ala Leu Glu
    290                 295                 300

cac gcg ctc gcg ttc gcc gcg cgg ctc gac cgg cag gtc aag ggg ttg     960
His Ala Leu Ala Phe Ala Ala Arg Leu Asp Arg Gln Val Lys Gly Leu
305                 310                 315                 320

tcg gcc gtg tcg cgc ggc gcg gcg cgc ggc gag ccg ccg ccc gat ccg     1008
Ser Ala Val Ser Arg Gly Ala Ala Arg Gly Glu Pro Pro Pro Asp Pro
                325                 330                 335

tgg gac ggc ctg ttc cag ctc gcg atg acc gtc gcg cgc gcg gcc ggg     1056
Trp Asp Gly Leu Phe Gln Leu Ala Met Thr Val Ala Arg Ala Ala Gly
            340                 345                 350

cca ggc ggc gac gcg ccg cgc cgg ccc gcc tga                         1089

Pro Gly Gly Asp Ala Pro Arg Arg Pro Ala
        355                 360

<210> 129
<211> 362
<212> PRT
<213> Burkholderia pseudomallei

<400> 129
Met Gln Leu Arg Leu Asp Ala Leu Glu Pro His Leu Ser Lys Gly Leu
1               5                   10                  15

Ala Gly Leu Tyr Val Val Tyr Gly Asp Glu Pro Leu Leu Ala Gln Glu
            20                  25                  30

Ala Cys Asp Lys Ile Arg Ala Ala Ala Arg Ala Ala Gly Phe Thr Glu
        35                  40                  45

Arg Ser Val His Thr Val Glu Arg Gly Phe Asp Trp Ser Thr Leu Ile
    50                  55                  60

Gly Ala Ser Gln Ala Met Ser Leu Phe Gly Glu Arg Gln Leu Val Glu
65                  70                  75                  80

Leu Arg Ile Pro Ser Gly Lys Pro Gly Lys Asp Gly Ala Asp Ala Leu
                85                  90                  95

Lys Thr Leu Ala Gly Ala Ala Asn Pro Asp Val Leu Met Leu Val Thr
            100                 105                 110

Leu Pro Arg Leu Asp Ala Ala Thr Gln Lys Ser Ala Trp Phe Thr Ala
        115                 120                 125

Leu Ala Asn Gly Gly Val Ala Leu Lys Ile Asp Pro Val Glu Arg Ala
    130                 135                 140

Gln Leu Pro Asn Trp Ile Gly Gln Arg Leu Ala Leu Gln Gly Gln Arg
145                 150                 155                 160

Val Ala Pro Gly Asp Asp Gly Arg Arg Ala Leu Ala Phe Val Ala Glu
                165                 170                 175

Arg Val Glu Gly Asn Leu Leu Ala Ala His Gln Glu Ile Gln Lys Leu
            180                 185                 190

Gly Leu Leu Tyr Pro Ala Gly Ala Leu Thr Phe Glu Gln Ile His Asp
        195                 200                 205

Ala Val Leu Asn Val Ala Arg Tyr Asp Val Phe Lys Leu Asn Glu Ala
    210                 215                 220

Met Leu Ala Gly Asp Ala Ala Arg Leu Ser Arg Met Ile Asp Gly Leu
225                 230                 235                 240

Lys Gly Glu Gly Glu Ala Leu Val Leu Val Leu Trp Ala Val Val Glu
                245                 250                 255

Glu Leu Arg Thr Leu Leu Arg Ile Lys Arg Gly Val Ala Ala Gly Lys
            260                 265                 270

Pro Leu Ala Val Leu Val Arg Glu Asn Arg Val Trp Gly Pro Arg Glu
        275                 280                 285

Arg Leu Val Gly Pro Ala Leu Ser Arg Val Ser Glu Ala Ala Leu Glu
    290                 295                 300

His Ala Leu Ala Phe Ala Ala Arg Leu Asp Arg Gln Val Lys Gly Leu

305                 310                 315                 320

Ser Ala Val Ser Arg Gly Ala Ala Arg Gly Glu Pro Pro Pro Asp Pro
                325                 330                 335

Trp Asp Gly Leu Phe Gln Leu Ala Met Thr Val Ala Arg Ala Ala Gly
            340                 345                 350

Pro Gly Gly Asp Ala Pro Arg Arg Pro Ala
        355                 360

<210> 130
<211> 1041
<212> DNA
<213> Chlorobium tepidum

<400> 130
atggctgacc tgaaaaaaca gattgcctca ggcaaaatcg cccctgtcta tttttttcag 60

ggtccggaaa gctggctcaa ggaggagatt gaaacgcaac tgaaggcggc cattttccct 120

tcggaaaacg aggctgccct caacacgctt gtcctgtacg ggccagacct caccctcggc 180

cagatcgttt cagcggcctc agaatacccg atgttcaccg aaaagaagct ggtcgtcgtc 240

cgccagttcg acaagctgaa aaaggtgacc gacaaaacgg aaagccagcg gcaggagaag 300

tcgctcctga gctatctttc cgatccgccg tcatttaccg tgctggtgct cgatgcagac 360

gagatcgaaa aaacggagct tgaaaaagcc ccgtacaaac atctcaaagc gtttcgccat 420

gacttcgggg cgatcaaaaa tgcggagatc ttcgccgctg aaagagcccg ggaagcggga 480

tgggagttcg agccggaagc cctgaaaaca ttctcgggat acatcgagcc atcatcgagg 540

ctgatctgcc aggagatcga gaagctcacg ctctacgcct cgaacaaacg ccccgaacgg 600

cgcatcaccc tcgacgacgt ctgcgactgc gtcggcatct ctcgaaaata taatgtcttc 660

gagcttgaaa aagcactggt cagcaagaac ctccggcagt gtagcggcat cgctttgatg 720

atcatggagc aggaggggca gaaagagggg ctcatgaaca tcgtgcgcta cctgaccacc 780

tttttcctgc ggatatggaa gctccagaca ccgggcgcac agcaactctc ccttcaggaa 840

acagcagcga tgctcggcat gtacggacgc caggagtatt tcgccaaaaa cttcatcggc 900

tacgcccgcc agttcagcgc cgccgaaacc gaaaatgcca ttctcgccct gcgtgacgcc 960

gatgccgccc tcaaaggcat tattccctcg cccgacgaca gattcaccct gctcaaactg 1020

atgcagcaac tcttcaattg a                                           1041

<210> 131
<211> 1041
<212> DNA
<213> Chlorobium tepidum

<220>
<221> CDS
<222> (1)..(1041)

<400> 131
atg gct gac ctg aaa aaa cag att gcc tca ggc aaa atc gcc cct gtc    48
Met Ala Asp Leu Lys Lys Gln Ile Ala Ser Gly Lys Ile Ala Pro Val
1               5                   10                  15

tat ttt ttt cag ggt ccg gaa agc tgg ctc aag gag gag att gaa acg 96
Tyr Phe Phe Gln Gly Pro Glu Ser Trp Leu Lys Glu Glu Ile Glu Thr
            20                  25                  30

caa ctg aag gcg gcc att ttc cct tcg gaa aac gag gct gcc ctc aac 144
Gln Leu Lys Ala Ala Ile Phe Pro Ser Glu Asn Glu Ala Ala Leu Asn
        35                  40                  45

acg ctt gtc ctg tac ggg cca gac ctc acc ctc ggc cag atc gtt tca 192
Thr Leu Val Leu Tyr Gly Pro Asp Leu Thr Leu Gly Gln Ile Val Ser
    50                  55                  60

gcg gcc tca gaa tac ccg atg ttc acc gaa aag aag ctg gtc gtc gtc 240
Ala Ala Ser Glu Tyr Pro Met Phe Thr Glu Lys Lys Leu Val Val Val
65                  70                  75                  80

cgc cag ttc gac aag ctg aaa aag gtg acc gac aaa acg gaa agc cag 288
Arg Gln Phe Asp Lys Leu Lys Lys Val Thr Asp Lys Thr Glu Ser Gln
                85                  90                  95

cgg cag gag aag tcg ctc ctg agc tat ctt tcc gat ccg ccg tca ttt 336
Arg Gln Glu Lys Ser Leu Leu Ser Tyr Leu Ser Asp Pro Pro Ser Phe
            100                 105                 110

acc gtg ctg gtg ctc gat gca gac gag atc gaa aaa acg gag ctt gaa 384
Thr Val Leu Val Leu Asp Ala Asp Glu Ile Glu Lys Thr Glu Leu Glu
        115                 120                 125

aaa gcc ccg tac aaa cat ctc aaa gcg ttt cgc cat gac ttc ggg gcg 432
Lys Ala Pro Tyr Lys His Leu Lys Ala Phe Arg His Asp Phe Gly Ala
    130                 135                 140

atc aaa aat gcg gag atc ttc gcc gct gaa aga gcc cgg gaa gcg gga 480
Ile Lys Asn Ala Glu Ile Phe Ala Ala Glu Arg Ala Arg Glu Ala Gly
145                 150                 155                 160

tgg gag ttc gag ccg gaa gcc ctg aaa aca ttc tcg gga tac atc gag 528
Trp Glu Phe Glu Pro Glu Ala Leu Lys Thr Phe Ser Gly Tyr Ile Glu
                165                 170                 175

cca tca tcg agg ctg atc tgc cag gag atc gag aag ctc acg ctc tac 576
Pro Ser Ser Arg Leu Ile Cys Gln Glu Ile Glu Lys Leu Thr Leu Tyr
            180                 185                 190

gcc tcg aac aaa cgc ccc gaa cgg cgc atc acc ctc gac gac gtc tgc 624
Ala Ser Asn Lys Arg Pro Glu Arg Arg Ile Thr Leu Asp Asp Val Cys
        195                 200                 205

gac tgc gtc ggc atc tct cga aaa tat aat gtc ttc gag ctt gaa aaa 672
Asp Cys Val Gly Ile Ser Arg Lys Tyr Asn Val Phe Glu Leu Glu Lys
    210                 215                 220

gca ctg gtc agc aag aac ctc cgg cag tgt agc ggc atc gct ttg atg 720
Ala Leu Val Ser Lys Asn Leu Arg Gln Cys Ser Gly Ile Ala Leu Met
225                 230                 235                 240

atc atg gag cag gag ggg cag aaa gag ggg ctc atg aac atc gtg cgc 768
Ile Met Glu Gln Glu Gly Gln Lys Glu Gly Leu Met Asn Ile Val Arg
                245                 250                 255

tac ctg acc acc ttt ttc ctg cgg ata tgg aag ctc cag aca ccg ggc 816
Tyr Leu Thr Thr Phe Phe Leu Arg Ile Trp Lys Leu Gln Thr Pro Gly
            260                 265                 270

gca cag caa ctc tcc ctt cag gaa aca gca gcg atg ctc ggc atg tac 864
Ala Gln Gln Leu Ser Leu Gln Glu Thr Ala Ala Met Leu Gly Met Tyr
        275                 280                 285

gga cgc cag gag tat ttc gcc aaa aac ttc atc ggc tac gcc cgc cag     912
Gly Arg Gln Glu Tyr Phe Ala Lys Asn Phe Ile Gly Tyr Ala Arg Gln
    290                 295                 300

ttc agc gcc gcc gaa acc gaa aat gcc att ctc gcc ctg cgt gac gcc     960
Phe Ser Ala Ala Glu Thr Glu Asn Ala Ile Leu Ala Leu Arg Asp Ala
305                 310                 315                 320

gat gcc gcc ctc aaa ggc att att ccc tcg ccc gac gac aga ttc acc     1008
Asp Ala Ala Leu Lys Gly Ile Ile Pro Ser Pro Asp Asp Arg Phe Thr
                325                 330                 335

ctg ctc aaa ctg atg cag caa ctc ttc aat tga                         1041
Leu Leu Lys Leu Met Gln Gln Leu Phe Asn
            340                 345

<210> 132
<211> 346
<212> PRT
<213> Chlorobium tepidum

<400> 132
Met Ala Asp Leu Lys Lys Gln Ile Ala Ser Gly Lys Ile Ala Pro Val
1               5                   10                  15

Tyr Phe Phe Gln Gly Pro Glu Ser Trp Leu Lys Glu Glu Ile Glu Thr
            20                  25                  30

Gln Leu Lys Ala Ala Ile Phe Pro Ser Glu Asn Glu Ala Ala Leu Asn
        35                  40                  45

Thr Leu Val Leu Tyr Gly Pro Asp Leu Thr Leu Gly Gln Ile Val Ser
    50                  55                  60

Ala Ala Ser Glu Tyr Pro Met Phe Thr Glu Lys Lys Leu Val Val Val
65                  70                  75                  80

Arg Gln Phe Asp Lys Leu Lys Lys Val Thr Asp Lys Thr Glu Ser Gln
                85                  90                  95

Arg Gln Glu Lys Ser Leu Leu Ser Tyr Leu Ser Asp Pro Pro Ser Phe
            100                 105                 110

Thr Val Leu Val Leu Asp Ala Asp Glu Ile Glu Lys Thr Glu Leu Glu
        115                 120                 125

Lys Ala Pro Tyr Lys His Leu Lys Ala Phe Arg His Asp Phe Gly Ala
    130                 135                 140

Ile Lys Asn Ala Glu Ile Phe Ala Ala Glu Arg Ala Arg Glu Ala Gly
145                 150                 155                 160

Trp Glu Phe Glu Pro Glu Ala Leu Lys Thr Phe Ser Gly Tyr Ile Glu
                165                 170                 175

Pro Ser Ser Arg Leu Ile Cys Gln Glu Ile Glu Lys Leu Thr Leu Tyr
            180                 185                 190

Ala Ser Asn Lys Arg Pro Glu Arg Arg Ile Thr Leu Asp Asp Val Cys
        195                 200                 205

Asp Cys Val Gly Ile Ser Arg Lys Tyr Asn Val Phe Glu Leu Glu Lys
    210                 215                 220

Ala Leu Val Ser Lys Asn Leu Arg Gln Cys Ser Gly Ile Ala Leu Met
225                 230                 235                 240

Ile Met Glu Gln Glu Gly Gln Lys Glu Gly Leu Met Asn Ile Val Arg
                245                 250                 255

Tyr Leu Thr Thr Phe Phe Leu Arg Ile Trp Lys Leu Gln Thr Pro Gly
            260                 265                 270

Ala Gln Gln Leu Ser Leu Gln Glu Thr Ala Ala Met Leu Gly Met Tyr
        275                 280                 285

Gly Arg Gln Glu Tyr Phe Ala Lys Asn Phe Ile Gly Tyr Ala Arg Gln
    290                 295                 300

Phe Ser Ala Ala Glu Thr Glu Asn Ala Ile Leu Ala Leu Arg Asp Ala
305                 310                 315                 320

Asp Ala Ala Leu Lys Gly Ile Ile Pro Ser Pro Asp Asp Arg Phe Thr
                325                 330                 335

Leu Leu Lys Leu Met Gln Gln Leu Phe Asn
            340                 345

<210> 133
<211> 840
<212> DNA
<213> Chloroflexus aurantiacus

<400> 133
caactggttg cggcatgtga ggcacacccg tttcttgccg agcggcgact ggtgatcgtc 60

tacgatgcgt tgaagcatag taaggcaggg aaggggcgcg aagaattacg tagttattgc 120

gagcgtgtgc cggcaacctg tgatctggtg tttgtggagc aagaagaggt tgatcgccga 180

catctgcttt tcacatacct cagcaagcat ggtgtcgttc gtgaattccc gttgctccag 240

ggagcagagt tactccgctg gattgaacag cgggcgaaga tgctgcatgc gacgattgaa 300

tctgccgccg cccaacgtct ggtcgaactg gcagggaatg atagccggtt actggctaac 360

gagctggcca aactggccag ttatgtgggt cgcaatggga agatcgatct gcgggctgta 420

gatcgcctgg ttaccgatca acaagagcaa aatctgtttg cctttctcga tgaattaagt 480

tcgcgacggt tagggccggc attacgcggc gcacgagcac tggttgaaga tggccaggca 540

ccgccctacg tgctctttat gctggctcgt cagatcagaa ttctcttgca ggtcaggcaa 600

ctgctcagtc aacggcgtcg cgctgatgag attgctgccg agctgaacct gaaaccattc 660

gtggcccgaa aggcaactgc acaggcgcgt aacttcagcc ttcccgaact gatacacgcc 720

catgatcgtc tgctcgaact tgatcacgcg atcaagaccg gacgtatcca ggccgaaaca 780

gcacttgaac tatttgttgt ggaagtgtgt cagatgccgc aagaggtggg gaagcgttag 840

<210> 134
<211> 840
<212> DNA
<213> Chloroflexus aurantiacus

<220>
<221> CDS
<222> (1)..(840)

<400> 134

caa ctg gtt gcg gca tgt gag gca cac ccg ttt ctt gcc gag cgg cga 48
Gln Leu Val Ala Ala Cys Glu Ala His Pro Phe Leu Ala Glu Arg Arg
1               5                   10                  15

ctg gtg atc gtc tac gat gcg ttg aag cat agt aag gca ggg aag ggg 96
Leu Val Ile Val Tyr Asp Ala Leu Lys His Ser Lys Ala Gly Lys Gly
            20                  25                  30

cgc gaa gaa tta cgt agt tat tgc gag cgt gtg ccg gca acc tgt gat 144
Arg Glu Glu Leu Arg Ser Tyr Cys Glu Arg Val Pro Ala Thr Cys Asp
        35                  40                  45

ctg gtg ttt gtg gag caa gaa gag gtt gat cgc cga cat ctg ctt ttc 192
Leu Val Phe Val Glu Gln Glu Glu Val Asp Arg Arg His Leu Leu Phe
    50                  55                  60

aca tac ctc agc aag cat ggt gtc gtt cgt gaa ttc ccg ttg ctc cag 240
Thr Tyr Leu Ser Lys His Gly Val Val Arg Glu Phe Pro Leu Leu Gln
65                  70                  75                  80

gga gca gag tta ctc cgc tgg att gaa cag cgg gcg aag atg ctg cat 288
Gly Ala Glu Leu Leu Arg Trp Ile Glu Gln Arg Ala Lys Met Leu His
                85                  90                  95

gcg acg att gaa tct gcc gcc gcc caa cgt ctg gtc gaa ctg gca ggg 336
Ala Thr Ile Glu Ser Ala Ala Ala Gln Arg Leu Val Glu Leu Ala Gly
            100                 105                 110

aat gat agc cgg tta ctg gct aac gag ctg gcc aaa ctg gcc agt tat 384
Asn Asp Ser Arg Leu Leu Ala Asn Glu Leu Ala Lys Leu Ala Ser Tyr
        115                 120                 125

gtg ggt cgc aat ggg aag atc gat ctg cgg gct gta gat cgc ctg gtt 432
Val Gly Arg Asn Gly Lys Ile Asp Leu Arg Ala Val Asp Arg Leu Val
    130                 135                 140

acc gat caa caa gag caa aat ctg ttt gcc ttt ctc gat gaa tta agt 480
Thr Asp Gln Gln Glu Gln Asn Leu Phe Ala Phe Leu Asp Glu Leu Ser
145                 150                 155                 160

tcg cga cgg tta ggg ccg gca tta cgc ggc gca cga gca ctg gtt gaa 528
Ser Arg Arg Leu Gly Pro Ala Leu Arg Gly Ala Arg Ala Leu Val Glu
                165                 170                 175

gat ggc cag gca ccg ccc tac gtg ctc ttt atg ctg gct cgt cag atc 576
Asp Gly Gln Ala Pro Pro Tyr Val Leu Phe Met Leu Ala Arg Gln Ile
            180                 185                 190

aga att ctc ttg cag gtc agg caa ctg ctc agt caa cgg cgt cgc gct 624
Arg Ile Leu Leu Gln Val Arg Gln Leu Leu Ser Gln Arg Arg Arg Ala
        195                 200                 205

gat gag att gct gcc gag ctg aac ctg aaa cca ttc gtg gcc cga aag 672
Asp Glu Ile Ala Ala Glu Leu Asn Leu Lys Pro Phe Val Ala Arg Lys
    210                 215                 220

gca act gca cag gcg cgt aac ttc agc ctt ccc gaa ctg ata cac gcc 720
Ala Thr Ala Gln Ala Arg Asn Phe Ser Leu Pro Glu Leu Ile His Ala
225                 230                 235                 240

cat gat cgt ctg ctc gaa ctt gat cac gcg atc aag acc gga cgt atc 768
His Asp Arg Leu Leu Glu Leu Asp His Ala Ile Lys Thr Gly Arg Ile
                245                 250                 255

cag gcc gaa aca gca ctt gaa cta ttt gtt gtg gaa gtg tgt cag atg 816
Gln Ala Glu Thr Ala Leu Glu Leu Phe Val Val Glu Val Cys Gln Met
            260                 265                 270

ccg caa gag gtg ggg aag cgt tag                                    840
Pro Gln Glu Val Gly Lys Arg
        275                 280

<210> 135
<211> 279
<212> PRT
<213> Chloroflexus aurantiacus

<400> 135
Gln Leu Val Ala Ala Cys Glu Ala His Pro Phe Leu Ala Glu Arg Arg
1               5                   10                  15

Leu Val Ile Val Tyr Asp Ala Leu Lys His Ser Lys Ala Gly Lys Gly
            20                  25                  30

Arg Glu Glu Leu Arg Ser Tyr Cys Glu Arg Val Pro Ala Thr Cys Asp
        35                  40                  45

Leu Val Phe Val Glu Gln Glu Glu Val Asp Arg Arg His Leu Leu Phe
    50                  55                  60

Thr Tyr Leu Ser Lys His Gly Val Val Arg Glu Phe Pro Leu Leu Gln
65                  70                  75                  80

Gly Ala Glu Leu Leu Arg Trp Ile Glu Gln Arg Ala Lys Met Leu His
                85                  90                  95

Ala Thr Ile Glu Ser Ala Ala Ala Gln Arg Leu Val Glu Leu Ala Gly
            100                 105                 110

Asn Asp Ser Arg Leu Leu Ala Asn Glu Leu Ala Lys Leu Ala Ser Tyr
        115                 120                 125

Val Gly Arg Asn Gly Lys Ile Asp Leu Arg Ala Val Asp Arg Leu Val
    130                 135                 140

Thr Asp Gln Gln Glu Gln Asn Leu Phe Ala Phe Leu Asp Glu Leu Ser
145                 150                 155                 160

Ser Arg Arg Leu Gly Pro Ala Leu Arg Gly Ala Arg Ala Leu Val Glu
                165                 170                 175

Asp Gly Gln Ala Pro Pro Tyr Val Leu Phe Met Leu Ala Arg Gln Ile
            180                 185                 190

Arg Ile Leu Leu Gln Val Arg Gln Leu Leu Ser Gln Arg Arg Arg Ala
        195                 200                 205

Asp Glu Ile Ala Ala Glu Leu Asn Leu Lys Pro Phe Val Ala Arg Lys
    210                 215                 220

Ala Thr Ala Gln Ala Arg Asn Phe Ser Leu Pro Glu Leu Ile His Ala
225                 230                 235                 240

His Asp Arg Leu Leu Glu Leu Asp His Ala Ile Lys Thr Gly Arg Ile
                245                 250                 255

Gln Ala Glu Thr Ala Leu Glu Leu Phe Val Val Glu Val Cys Gln Met
            260                 265                 270

Pro Gln Glu Val Gly Lys Arg
        275

<210> 136
<211> 1044

<212> DNA
<213> Clostridium difficile

<400> 136
atgaatcaca aggatattat taggaatgta aaagagaaaa attttgaaaa agtgtatctt 60

ttttatggaa gagagtatta tttaattgag aatgcaataa aagcatttaa agatagtcta 120

aacgagggaa tgcttgattt taatttagat ataatagatg ggaaggaaat agtcttaaat 180

cagcttataa gctctataga aacacttcca tttatggatg ataggaagat agtaataata 240

aaagactttg aactattaaa aggaaagaaa aagaatttta cagatagtga tgagaaatac 300

ttaatagagc atttagacaa tattccagac accacaacta tagtttttgt tgtatatgga 360

gatgtagaca aaagaaaatc attagttaag aaaataggaa ataatggaat tgtttttgat 420

tgtgataagc tttctgatat ggatttgttt aaatggataa aaaaatcttt tgctttaaat 480

gatgttataa tagataattc tcaaatcatg tattttattg aacaagaagg atacagagat 540

aagagtagtg aaaagacatt atctgattta gaaaatgaaa taaataaaat aagttcattt 600

gttggtaaag ggaataatgt aaccaatgat gtaattgata aattatctca aaaaaaagtt 660

gagaatgata tattcaaatt aatcgattat attggagaac aaaatgcttc taatgcaatg 720

aaaatattaa atgatatgat acaagaaggc gaatctgtat taggtatatt ttctatgata 780

gcaagacaat ttaaaataat tatgcaagta agacagttac aacttgatgg atattcaaca 840

aaacttatag ctgataaatt aaaaatgcat caatttgttg taggaaaagc attaaagcaa 900

actaaaaatt tttctgatga tataattgtg gaaatattaa actatatatt agaaagtgat 960

tataaaataa agacaggtct tataagagat actttagcag ttgaaatgtt ggttagtaga 1020

tactgtaaaa gagaagcaat ttaa 1044

<210> 137
<211> 1044
<212> DNA
<213> Clostridium difficile

<220>
<221> CDS
<222> (1)..(1044)

<400> 137
atg aat cac aag gat att att agg aat gta aaa gag aaa aat ttt gaa 48
Met Asn His Lys Asp Ile Ile Arg Asn Val Lys Glu Lys Asn Phe Glu
1               5                   10                  15

aaa gtg tat ctt ttt tat gga aga gag tat tat tta att gag aat gca 96
Lys Val Tyr Leu Phe Tyr Gly Arg Glu Tyr Tyr Leu Ile Glu Asn Ala
            20                  25                  30

ata aaa gca ttt aaa gat agt cta aac gag gga atg ctt gat ttt aat 144
Ile Lys Ala Phe Lys Asp Ser Leu Asn Glu Gly Met Leu Asp Phe Asn
        35                  40                  45

tta gat ata ata gat ggg aag gaa ata gtc tta aat cag ctt ata agc 192
Leu Asp Ile Ile Asp Gly Lys Glu Ile Val Leu Asn Gln Leu Ile Ser
    50                  55                  60

tct ata gaa aca ctt cca ttt atg gat gat agg aag ata gta ata ata 240

Ser Ile Glu Thr Leu Pro Phe Met Asp Asp Arg Lys Ile Val Ile Ile
65                  70                  75                  80

aaa gac ttt gaa cta tta aaa gga aag aaa aag aat ttt aca gat agt     288
Lys Asp Phe Glu Leu Leu Lys Gly Lys Lys Lys Asn Phe Thr Asp Ser
                85                  90                  95

gat gag aaa tac tta ata gag cat tta gac aat att cca gac acc aca     336
Asp Glu Lys Tyr Leu Ile Glu His Leu Asp Asn Ile Pro Asp Thr Thr
            100                 105                 110

act ata gtt ttt gtt gta tat gga gat gta gac aaa aga aaa tca tta     384
Thr Ile Val Phe Val Val Tyr Gly Asp Val Asp Lys Arg Lys Ser Leu
        115                 120                 125

gtt aag aaa ata gga aat aat gga att gtt ttt gat tgt gat aag ctt     432
Val Lys Lys Ile Gly Asn Asn Gly Ile Val Phe Asp Cys Asp Lys Leu
    130                 135                 140

tct gat atg gat ttg ttt aaa tgg ata aaa aaa tct ttt gct tta aat     480
Ser Asp Met Asp Leu Phe Lys Trp Ile Lys Lys Ser Phe Ala Leu Asn
145                 150                 155                 160

gat gtt ata ata gat aat tct caa atc atg tat ttt att gaa caa gaa     528
Asp Val Ile Ile Asp Asn Ser Gln Ile Met Tyr Phe Ile Glu Gln Glu
                165                 170                 175

gga tac aga gat aag agt agt gaa aag aca tta tct gat tta gaa aat     576
Gly Tyr Arg Asp Lys Ser Ser Glu Lys Thr Leu Ser Asp Leu Glu Asn
            180                 185                 190

gaa ata aat aaa ata agt tca ttt gtt ggt aaa ggg aat aat gta acc     624
Glu Ile Asn Lys Ile Ser Ser Phe Val Gly Lys Gly Asn Asn Val Thr
        195                 200                 205

aat gat gta att gat aaa tta tct caa aaa aaa gtt gag aat gat ata     672
Asn Asp Val Ile Asp Lys Leu Ser Gln Lys Lys Val Glu Asn Asp Ile
    210                 215                 220

ttc aaa tta atc gat tat att gga gaa caa aat gct tct aat gca atg     720
Phe Lys Leu Ile Asp Tyr Ile Gly Glu Gln Asn Ala Ser Asn Ala Met
225                 230                 235                 240

aaa ata tta aat gat atg ata caa gaa ggc gaa tct gta tta ggt ata     768
Lys Ile Leu Asn Asp Met Ile Gln Glu Gly Glu Ser Val Leu Gly Ile
                245                 250                 255

ttt tct atg ata gca aga caa ttt aaa ata att atg caa gta aga cag     816
Phe Ser Met Ile Ala Arg Gln Phe Lys Ile Ile Met Gln Val Arg Gln
            260                 265                 270

tta caa ctt gat gga tat tca aca aaa ctt ata gct gat aaa tta aaa     864
Leu Gln Leu Asp Gly Tyr Ser Thr Lys Leu Ile Ala Asp Lys Leu Lys
        275                 280                 285

atg cat caa ttt gtt gta gga aaa gca tta aag caa act aaa aat ttt     912
Met His Gln Phe Val Val Gly Lys Ala Leu Lys Gln Thr Lys Asn Phe
    290                 295                 300

tct gat gat ata att gtg gaa ata tta aac tat ata tta gaa agt gat     960
Ser Asp Asp Ile Ile Val Glu Ile Leu Asn Tyr Ile Leu Glu Ser Asp
305                 310                 315                 320

tat aaa ata aag aca ggt ctt ata aga gat act tta gca gtt gaa atg     1008
Tyr Lys Ile Lys Thr Gly Leu Ile Arg Asp Thr Leu Ala Val Glu Met
                325                 330                 335

ttg gtt agt aga tac tgt aaa aga gaa gca att taa                     1044

Leu Val Ser Arg Tyr Cys Lys Arg Glu Ala Ile
            340                 345

<210> 138
<211> 347
<212> PRT
<213> Clostridium difficile

<400> 138
Met Asn His Lys Asp Ile Ile Arg Asn Val Lys Glu Lys Asn Phe Glu
1               5                   10                  15

Lys Val Tyr Leu Phe Tyr Gly Arg Glu Tyr Tyr Leu Ile Glu Asn Ala
            20                  25                  30

Ile Lys Ala Phe Lys Asp Ser Leu Asn Glu Gly Met Leu Asp Phe Asn
        35                  40                  45

Leu Asp Ile Ile Asp Gly Lys Glu Ile Val Leu Asn Gln Leu Ile Ser
    50                  55                  60

Ser Ile Glu Thr Leu Pro Phe Met Asp Asp Arg Lys Ile Val Ile Ile
65                  70                  75                  80

Lys Asp Phe Glu Leu Leu Lys Gly Lys Lys Lys Asn Phe Thr Asp Ser
                85                  90                  95

Asp Glu Lys Tyr Leu Ile Glu His Leu Asp Asn Ile Pro Asp Thr Thr
            100                 105                 110

Thr Ile Val Phe Val Val Tyr Gly Asp Val Asp Lys Arg Lys Ser Leu
        115                 120                 125

Val Lys Lys Ile Gly Asn Asn Gly Ile Val Phe Asp Cys Asp Lys Leu
    130                 135                 140

Ser Asp Met Asp Leu Phe Lys Trp Ile Lys Lys Ser Phe Ala Leu Asn
145                 150                 155                 160

Asp Val Ile Ile Asp Asn Ser Gln Ile Met Tyr Phe Ile Glu Gln Glu
                165                 170                 175

Gly Tyr Arg Asp Lys Ser Ser Glu Lys Thr Leu Ser Asp Leu Glu Asn
            180                 185                 190

Glu Ile Asn Lys Ile Ser Ser Phe Val Gly Lys Gly Asn Asn Val Thr
        195                 200                 205

Asn Asp Val Ile Asp Lys Leu Ser Gln Lys Lys Val Glu Asn Asp Ile
    210                 215                 220

Phe Lys Leu Ile Asp Tyr Ile Gly Glu Gln Asn Ala Ser Asn Ala Met
225                 230                 235                 240

Lys Ile Leu Asn Asp Met Ile Gln Glu Gly Glu Ser Val Leu Gly Ile
                245                 250                 255

Phe Ser Met Ile Ala Arg Gln Phe Lys Ile Ile Met Gln Val Arg Gln
            260                 265                 270

Leu Gln Leu Asp Gly Tyr Ser Thr Lys Leu Ile Ala Asp Lys Leu Lys
        275                 280                 285

Met His Gln Phe Val Val Gly Lys Ala Leu Lys Gln Thr Lys Asn Phe
    290                 295                 300

Ser Asp Asp Ile Ile Val Glu Ile Leu Asn Tyr Ile Leu Glu Ser Asp

305                 310                 315                 320

Tyr Lys Ile Lys Thr Gly Leu Ile Arg Asp Thr Leu Ala Val Glu Met
                325                 330                 335

Leu Val Ser Arg Tyr Cys Lys Arg Glu Ala Ile
            340                 345

<210> 139
<211> 975
<212> DNA
<213> Corynebacterium diphtheriae

<400> 139
atgggtcagc aacttctacc agtgcacctc gtagttggcg acgatgaatt cctagccgaa 60

cgcgcacgac acagcatcat cgcagccatc aacaaaacca ttggcagcga cgtccccgtg 120

accacccttc gggcaggcga agtcaacgcc tccgaactca tccaactcac cagcccgtcg 180

ctcttcggcg aagaccgcat cattgtgctg accaacatgg aagatgctgg caaagaaccc 240

gcggaactcg tcctcaacgt tgccgtcgac ccagcaccag gaatctacct catcatcgtg 300

cactccggcg gcggacggca aaaagcacta gtaccaaaac tcagcaaaat cagccaagtc 360

cacgaagcca acaaactcaa acctaaagac cgcgtgggct gggtcaccaa cgaattcaaa 420

cgccacggag tacgccccac cccagacgtg gtccacgccc tgctcgaagg cgtaggatcc 480

gacctccgcg aactcgcctc cgccatcggc cagcttgttg ctgacaacaa tggcgacgtc 540

accctcgcca ccgtccgcga ctactacgtg ggagtagcag aagtctccgg tttcgacgtg 600

gcagatcttg cctgctcagg acaaacccaa cgtgccgtcg ccagcgcgcg ccgggcactg 660

caactaggag tctcaccggt ggcacttgcc gccgcattga gcatgaaagt tagcgctatc 720

gcgcggttgt attccacacg agggcgtatt gactcccgta aactcgcagg agagctgggc 780

atgcacccct tcgttgttga aaaaaccgcc aaagttgcgc gcatgtggac gggtaatgct 840

gtgagccaag cggtgatctt gatggcggat gtggattcca gtcttaaggg ccacggcctc 900

gatggcgaac tcggcggtga ccccgactac gaaatcgagg atgctatccg caggatctct 960

gagctcgcag gctaa                                                  975

<210> 140
<211> 975
<212> DNA
<213> Corynebacterium diphtheriae

<220>
<221> CDS
<222> (1)..(975)

<400> 140
atg ggt cag caa ctt cta cca gtg cac ctc gta gtt ggc gac gat gaa      48
Met Gly Gln Gln Leu Leu Pro Val His Leu Val Val Gly Asp Asp Glu
1               5                   10                  15

ttc cta gcc gaa cgc gca cga cac agc atc atc gca gcc atc aac aaa      96
Phe Leu Ala Glu Arg Ala Arg His Ser Ile Ile Ala Ala Ile Asn Lys
            20                  25                  30

acc att ggc agc gac gtc ccc gtg acc acc ctt cgg gca ggc gaa gtc 144
Thr Ile Gly Ser Asp Val Pro Val Thr Thr Leu Arg Ala Gly Glu Val
        35                  40                  45

aac gcc tcc gaa ctc atc caa ctc acc agc ccg tcg ctc ttc ggc gaa 192
Asn Ala Ser Glu Leu Ile Gln Leu Thr Ser Pro Ser Leu Phe Gly Glu
    50                  55                  60

gac cgc atc att gtg ctg acc aac atg gaa gat gct ggc aaa gaa ccc 240
Asp Arg Ile Ile Val Leu Thr Asn Met Glu Asp Ala Gly Lys Glu Pro
65                  70                  75                  80

gcg gaa ctc gtc ctc aac gtt gcc gtc gac cca gca cca gga atc tac 288
Ala Glu Leu Val Leu Asn Val Ala Val Asp Pro Ala Pro Gly Ile Tyr
                85                  90                  95

ctc atc atc gtg cac tcc ggc ggc gga cgg caa aaa gca cta gta cca 336
Leu Ile Ile Val His Ser Gly Gly Gly Arg Gln Lys Ala Leu Val Pro
            100                 105                 110

aaa ctc agc aaa atc agc caa gtc cac gaa gcc aac aaa ctc aaa cct 384
Lys Leu Ser Lys Ile Ser Gln Val His Glu Ala Asn Lys Leu Lys Pro
        115                 120                 125

aaa gac cgc gtg ggc tgg gtc acc aac gaa ttc aaa cgc cac gga gta 432
Lys Asp Arg Val Gly Trp Val Thr Asn Glu Phe Lys Arg His Gly Val
    130                 135                 140

cgc ccc acc cca gac gtg gtc cac gcc ctg ctc gaa ggc gta gga tcc 480
Arg Pro Thr Pro Asp Val Val His Ala Leu Leu Glu Gly Val Gly Ser
145                 150                 155                 160

gac ctc cgc gaa ctc gcc tcc gcc atc ggc cag ctt gtt gct gac aac 528
Asp Leu Arg Glu Leu Ala Ser Ala Ile Gly Gln Leu Val Ala Asp Asn
                165                 170                 175

aat ggc gac gtc acc ctc gcc acc gtc cgc gac tac tac gtg gga gta 576
Asn Gly Asp Val Thr Leu Ala Thr Val Arg Asp Tyr Tyr Val Gly Val
            180                 185                 190

gca gaa gtc tcc ggt ttc gac gtg gca gat ctt gcc tgc tca gga caa 624
Ala Glu Val Ser Gly Phe Asp Val Ala Asp Leu Ala Cys Ser Gly Gln
        195                 200                 205

acc caa cgt gcc gtc gcc agc gcg cgc cgg gca ctg caa cta gga gtc 672
Thr Gln Arg Ala Val Ala Ser Ala Arg Arg Ala Leu Gln Leu Gly Val
    210                 215                 220

tca ccg gtg gca ctt gcc gcc gca ttg agc atg aaa gtt agc gct atc 720
Ser Pro Val Ala Leu Ala Ala Ala Leu Ser Met Lys Val Ser Ala Ile
225                 230                 235                 240

gcg cgg ttg tat tcc aca cga ggg cgt att gac tcc cgt aaa ctc gca 768
Ala Arg Leu Tyr Ser Thr Arg Gly Arg Ile Asp Ser Arg Lys Leu Ala
                245                 250                 255

gga gag ctg ggc atg cac ccc ttc gtt gtt gaa aaa acc gcc aaa gtt 816
Gly Glu Leu Gly Met His Pro Phe Val Val Glu Lys Thr Ala Lys Val
            260                 265                 270

gcg cgc atg tgg acg ggt aat gct gtg agc caa gcg gtg atc ttg atg 864
Ala Arg Met Trp Thr Gly Asn Ala Val Ser Gln Ala Val Ile Leu Met
        275                 280                 285

gcg gat gtg gat tcc agt ctt aag ggc cac ggc ctc gat ggc gaa ctc 912
Ala Asp Val Asp Ser Ser Leu Lys Gly His Gly Leu Asp Gly Glu Leu
    290                 295                 300

ggc ggt gac ccc gac tac gaa atc gag gat gct atc cgc agg atc tct     960
Gly Gly Asp Pro Asp Tyr Glu Ile Glu Asp Ala Ile Arg Arg Ile Ser
305                 310                 315                 320

gag ctc gca ggc taa                                                 975
Glu Leu Ala Gly
                325

<210> 141
<211> 324
<212> PRT
<213> Corynebacterium diphtheriae

<400> 141
Met Gly Gln Gln Leu Leu Pro Val His Leu Val Val Gly Asp Asp Glu
1               5                   10                  15

Phe Leu Ala Glu Arg Ala Arg His Ser Ile Ile Ala Ala Ile Asn Lys
            20                  25                  30

Thr Ile Gly Ser Asp Val Pro Val Thr Thr Leu Arg Ala Gly Glu Val
        35                  40                  45

Asn Ala Ser Glu Leu Ile Gln Leu Thr Ser Pro Ser Leu Phe Gly Glu
    50                  55                  60

Asp Arg Ile Ile Val Leu Thr Asn Met Glu Asp Ala Gly Lys Glu Pro
65                  70                  75                  80

Ala Glu Leu Val Leu Asn Val Ala Val Asp Pro Ala Pro Gly Ile Tyr
                85                  90                  95

Leu Ile Ile Val His Ser Gly Gly Gly Arg Gln Lys Ala Leu Val Pro
            100                 105                 110

Lys Leu Ser Lys Ile Ser Gln Val His Glu Ala Asn Lys Leu Lys Pro
        115                 120                 125

Lys Asp Arg Val Gly Trp Val Thr Asn Glu Phe Lys Arg His Gly Val
    130                 135                 140

Arg Pro Thr Pro Asp Val Val His Ala Leu Leu Glu Gly Val Gly Ser
145                 150                 155                 160

Asp Leu Arg Glu Leu Ala Ser Ala Ile Gly Gln Leu Val Ala Asp Asn
                165                 170                 175

Asn Gly Asp Val Thr Leu Ala Thr Val Arg Asp Tyr Tyr Val Gly Val
            180                 185                 190

Ala Glu Val Ser Gly Phe Asp Val Ala Asp Leu Ala Cys Ser Gly Gln
        195                 200                 205

Thr Gln Arg Ala Val Ala Ser Ala Arg Arg Ala Leu Gln Leu Gly Val
    210                 215                 220

Ser Pro Val Ala Leu Ala Ala Ala Leu Ser Met Lys Val Ser Ala Ile
225                 230                 235                 240

Ala Arg Leu Tyr Ser Thr Arg Gly Arg Ile Asp Ser Arg Lys Leu Ala
                245                 250                 255

Gly Glu Leu Gly Met His Pro Phe Val Val Glu Lys Thr Ala Lys Val
            260                 265                 270

Ala Arg Met Trp Thr Gly Asn Ala Val Ser Gln Ala Val Ile Leu Met
        275                 280                 285

Ala Asp Val Asp Ser Ser Leu Lys Gly His Gly Leu Asp Gly Glu Leu
    290                 295                 300

Gly Gly Asp Pro Asp Tyr Glu Ile Glu Asp Ala Ile Arg Arg Ile Ser
305                 310                 315                 320

Glu Leu Ala Gly


<210> 142
<211> 1035
<212> DNA
<213> Cytophaga hutchinsonii

<400> 142
atggctgaaa ccagttctgc tccggataaa gtattaaaag accttaagga aggtaaatac 60

gctcctgtat attttttgca gggagaggaa ccctatttta tagatctaat atccagttat 120

atagaaaata actgtttgcc ggaatcggag agaggattta atcagacgat cctgtatggc 180

aaagatacca atgtttctac cattcttcag aatgcccgca agtatccgat gttttcggaa 240

aggcaggtag taatggttaa ggaagcacag gatgtttcgg atctgaccaa agattcaaca 300

gagaaatttt taatggcata tctcgaaaat ccattgccta caaccattct ggtattttgc 360

cacaagtata aaaaactgga tgcccgtaaa aaaataacca agcacctgca gaagtattcg 420

gtgtttgttg aatctgccaa actttacgat aataaattgc ctgaatggat taatggctat 480

ttcagggaaa agaattacag gattgatccg aaggcggtcg ttctattggc cgaatctatt 540

ggtacggatc tttcccgaat ggcaaatgaa attgataagc tgctcatcaa cgttaaagat 600

cctgctataa ccatcaccga agatttaatc gaaaaatatg ttggcgtgag caaagaatac 660

aatgtctttg aattacaaaa agctatcggt gtacgggata taataaaagc gaataaaatc 720

gttaatcatt ttgaatccaa tccaaaagcg aatccgattg taatggtgct gtcaagtttg 780

tttacttact atcttaaaat actggcgatt catgccgcca cagataagag tgaagcaagc 840

ctggcaaagg ttgttggtgt acatccattc tttgtaaaag aatacatcaa tgcgtcgcgc 900

atcaatgata ttaataaatg catctcttgt gtgcgcgcca ttcgtgaagc ggacaaaatg 960

gtgaaagggt atacggttgt taatgttacc gatggcttta ttttaaaaga attattgttt 1020

aaattattac attga                                                  1035


<210> 143
<211> 1035
<212> DNA
<213> Cytophaga hutchinsonii

<220>
<221> CDS
<222> (1)..(1035)

<400> 143
atg gct gaa acc agt tct gct ccg gat aaa gta tta aaa gac ctt aag    48
Met Ala Glu Thr Ser Ser Ala Pro Asp Lys Val Leu Lys Asp Leu Lys
1               5                   10                  15

gaa ggt aaa tac gct cct gta tat ttt ttg cag gga gag gaa ccc tat    96

Glu Gly Lys Tyr Ala Pro Val Tyr Phe Leu Gln Gly Glu Glu Pro Tyr
            20                  25                  30

ttt ata gat cta ata tcc agt tat ata gaa aat aac tgt ttg ccg gaa 144
Phe Ile Asp Leu Ile Ser Ser Tyr Ile Glu Asn Asn Cys Leu Pro Glu
        35                  40                  45

tcg gag aga gga ttt aat cag acg atc ctg tat ggc aaa gat acc aat 192
Ser Glu Arg Gly Phe Asn Gln Thr Ile Leu Tyr Gly Lys Asp Thr Asn
    50                  55                  60

gtt tct acc att ctt cag aat gcc cgc aag tat ccg atg ttt tcg gaa 240
Val Ser Thr Ile Leu Gln Asn Ala Arg Lys Tyr Pro Met Phe Ser Glu
65                  70                  75                  80

agg cag gta gta atg gtt aag gaa gca cag gat gtt tcg gat ctg acc 288
Arg Gln Val Val Met Val Lys Glu Ala Gln Asp Val Ser Asp Leu Thr
                85                  90                  95

aaa gat tca aca gag aaa ttt tta atg gca tat ctc gaa aat cca ttg 336
Lys Asp Ser Thr Glu Lys Phe Leu Met Ala Tyr Leu Glu Asn Pro Leu
            100                 105                 110

cct aca acc att ctg gta ttt tgc cac aag tat aaa aaa ctg gat gcc 384
Pro Thr Thr Ile Leu Val Phe Cys His Lys Tyr Lys Lys Leu Asp Ala
        115                 120                 125

cgt aaa aaa ata acc aag cac ctg cag aag tat tcg gtg ttt gtt gaa 432
Arg Lys Lys Ile Thr Lys His Leu Gln Lys Tyr Ser Val Phe Val Glu
    130                 135                 140

tct gcc aaa ctt tac gat aat aaa ttg cct gaa tgg att aat ggc tat 480
Ser Ala Lys Leu Tyr Asp Asn Lys Leu Pro Glu Trp Ile Asn Gly Tyr
145                 150                 155                 160

ttc agg gaa aag aat tac agg att gat ccg aag gcg gtc gtt cta ttg 528
Phe Arg Glu Lys Asn Tyr Arg Ile Asp Pro Lys Ala Val Val Leu Leu
                165                 170                 175

gcc gaa tct att ggt acg gat ctt tcc cga atg gca aat gaa att gat 576
Ala Glu Ser Ile Gly Thr Asp Leu Ser Arg Met Ala Asn Glu Ile Asp
            180                 185                 190

aag ctg ctc atc aac gtt aaa gat cct gct ata acc atc acc gaa gat 624
Lys Leu Leu Ile Asn Val Lys Asp Pro Ala Ile Thr Ile Thr Glu Asp
        195                 200                 205

tta atc gaa aaa tat gtt ggc gtg agc aaa gaa tac aat gtc ttt gaa 672
Leu Ile Glu Lys Tyr Val Gly Val Ser Lys Glu Tyr Asn Val Phe Glu
    210                 215                 220

tta caa aaa gct atc ggt gta cgg gat ata ata aaa gcg aat aaa atc 720
Leu Gln Lys Ala Ile Gly Val Arg Asp Ile Ile Lys Ala Asn Lys Ile
225                 230                 235                 240

gtt aat cat ttt gaa tcc aat cca aaa gcg aat ccg att gta atg gtg 768
Val Asn His Phe Glu Ser Asn Pro Lys Ala Asn Pro Ile Val Met Val
                245                 250                 255

ctg tca agt ttg ttt act tac tat ctt aaa ata ctg gcg att cat gcc 816
Leu Ser Ser Leu Phe Thr Tyr Tyr Leu Lys Ile Leu Ala Ile His Ala
            260                 265                 270

gcc aca gat aag agt gaa gca agc ctg gca aag gtt gtt ggt gta cat 864
Ala Thr Asp Lys Ser Glu Ala Ser Leu Ala Lys Val Val Gly Val His
        275                 280                 285

cca ttc ttt gta aaa gaa tac atc aat gcg tcg cgc atc aat gat att 912

Pro Phe Phe Val Lys Glu Tyr Ile Asn Ala Ser Arg Ile Asn Asp Ile
    290                 295                 300

aat aaa tgc atc tct tgt gtg cgc gcc att cgt gaa gcg gac aaa atg     960
Asn Lys Cys Ile Ser Cys Val Arg Ala Ile Arg Glu Ala Asp Lys Met
305                 310                 315                 320

gtg aaa ggg tat acg gtt gtt aat gtt acc gat ggc ttt att tta aaa     1008
Val Lys Gly Tyr Thr Val Val Asn Val Thr Asp Gly Phe Ile Leu Lys
                325                 330                 335

gaa tta ttg ttt aaa tta tta cat tga                                 1035
Glu Leu Leu Phe Lys Leu Leu His
            340                 345


<210> 144
<211> 344
<212> PRT
<213> Cytophaga hutchinsonii

<400> 144
Met Ala Glu Thr Ser Ser Ala Pro Asp Lys Val Leu Lys Asp Leu Lys
  1               5                  10                  15

Glu Gly Lys Tyr Ala Pro Val Tyr Phe Leu Gln Gly Glu Glu Pro Tyr
             20                  25                  30

Phe Ile Asp Leu Ile Ser Ser Tyr Ile Glu Asn Asn Cys Leu Pro Glu
         35                  40                  45

Ser Glu Arg Gly Phe Asn Gln Thr Ile Leu Tyr Gly Lys Asp Thr Asn
     50                  55                  60

Val Ser Thr Ile Leu Gln Asn Ala Arg Lys Tyr Pro Met Phe Ser Glu
 65                  70                  75                  80

Arg Gln Val Val Met Val Lys Glu Ala Gln Asp Val Ser Asp Leu Thr
                 85                  90                  95

Lys Asp Ser Thr Glu Lys Phe Leu Met Ala Tyr Leu Glu Asn Pro Leu
            100                 105                 110

Pro Thr Thr Ile Leu Val Phe Cys His Lys Tyr Lys Lys Leu Asp Ala
        115                 120                 125

Arg Lys Lys Ile Thr Lys His Leu Gln Lys Tyr Ser Val Phe Val Glu
    130                 135                 140

Ser Ala Lys Leu Tyr Asp Asn Lys Leu Pro Glu Trp Ile Asn Gly Tyr
145                 150                 155                 160

Phe Arg Glu Lys Asn Tyr Arg Ile Asp Pro Lys Ala Val Val Leu Leu
                165                 170                 175

Ala Glu Ser Ile Gly Thr Asp Leu Ser Arg Met Ala Asn Glu Ile Asp
            180                 185                 190

Lys Leu Leu Ile Asn Val Lys Asp Pro Ala Ile Thr Ile Thr Glu Asp
        195                 200                 205

Leu Ile Glu Lys Tyr Val Gly Val Ser Lys Glu Tyr Asn Val Phe Glu
    210                 215                 220

Leu Gln Lys Ala Ile Gly Val Arg Asp Ile Ile Lys Ala Asn Lys Ile
225                 230                 235                 240

Val Asn His Phe Glu Ser Asn Pro Lys Ala Asn Pro Ile Val Met Val

245                 250                 255

Leu Ser Ser Leu Phe Thr Tyr Tyr Leu Lys Ile Leu Ala Ile His Ala
            260                 265                 270

Ala Thr Asp Lys Ser Glu Ala Ser Leu Ala Lys Val Val Gly Val His
        275                 280                 285

Pro Phe Phe Val Lys Glu Tyr Ile Asn Ala Ser Arg Ile Asn Asp Ile
    290                 295                 300

Asn Lys Cys Ile Ser Cys Val Arg Ala Ile Arg Glu Ala Asp Lys Met
305                 310                 315                 320

Val Lys Gly Tyr Thr Val Val Asn Val Thr Asp Gly Phe Ile Leu Lys
                325                 330                 335

Glu Leu Leu Phe Lys Leu Leu His
            340

<210> 145
<211> 1200
<212> DNA
<213> Dehalococcoides ethenogenes

<400> 145
atgtttgcgg agttcgtagg gcattacaac ttcttcgggc attgtttcaa atcctccaag 60

tgcagcttac gggcagattg taacatactg ccaggcattt ttgcactaaa cttcaaaggt 120

tacgcgcccg tttcgttatg ctacaatatc agccgaaggt ggctgttaaa tttgctgtat 180

atttttaccg gcagtgatga tttttccctg aaagaacgcc tgaagcagat aaaactggct 240

ttgggtgatg agtctttgtc ctctgccaat accaccatgc tggagggcaa aaacctgacg 300

gctgccgaac ttcaaaacta tgtccagact attccctttt tagcccctgc ccggctggtt 360

atggtaaacg gcctgctgga gaggtttgaa ggctcaaaag gtaaagccgc cgccaaagat 420

aaggcagaca aaaacagccc ggatgaattt gcccgggcgc tgtctaatct gcctcccaca 480

accctggcgg tgcttctgga ctataccacc gctaaagacg gtgattaccg tgaaaaagaa 540

aaagtatccg ctttggcctc aaaccccctt tttaagctgg taagccccgg tgctgaggta 600

aagcactttg cacccctttc tgcccagaac ctgcccggct gggtaatgca gcgtgccaaa 660

cagtcttcta tccagatgtc tgttccggca gcccgtctgc tggcccgttt tgtgggggcg 720

gacttgtggg cgctttcctc cgagattgag aaactgggct tgtatgccca ggggcgggcg 780

gtggaggaag ccgatgttga aaatatggtg ggttattccc aggaagtaaa tatattcggt 840

ttggtggatg ccgtactgga ccgcaaggtt aaagacgccc agcagtctct ggcggctttt 900

wctgycggcg gcgggtyacc cggttttttg ctgtttatgc tggtacgcca gctgcggctg 960

atggtgcgtt ataccgccct taaaaagcgg gggctgaaag aggccgagat ttttaaagct 1020

atggkcggct ctgagtacgc ccttaggaaa acagcctctc aggcggcctg ccagaacatg 1080

gacagcctta aagctattta ttccaaactg ctggaaacag attttaatat aaaaaccggc 1140

cgctttgaac cggagctggc catgggtttg ctggttgccg aactttgcgg gagaaaatag 1200

<210> 146
<211> 1200
<212> DNA
<213> Dehalococcoides ethenogenes

<220>
<221> CDS
<222> (1)..(1200)

<400> 146
atg ttt gcg gag ttc gta ggg cat tac aac ttc ttc ggg cat tgt ttc     48
Met Phe Ala Glu Phe Val Gly His Tyr Asn Phe Phe Gly His Cys Phe
1               5                   10                  15

aaa tcc tcc aag tgc agc tta cgg gca gat tgt aac ata ctg cca ggc     96
Lys Ser Ser Lys Cys Ser Leu Arg Ala Asp Cys Asn Ile Leu Pro Gly
            20                  25                  30

att ttt gca cta aac ttc aaa ggt tac gcg ccc gtt tcg tta tgc tac     144
Ile Phe Ala Leu Asn Phe Lys Gly Tyr Ala Pro Val Ser Leu Cys Tyr
        35                  40                  45

aat atc agc cga agg tgg ctg tta aat ttg ctg tat att ttt acc ggc     192
Asn Ile Ser Arg Arg Trp Leu Leu Asn Leu Leu Tyr Ile Phe Thr Gly
    50                  55                  60

agt gat gat ttt tcc ctg aaa gaa cgc ctg aag cag ata aaa ctg gct     240
Ser Asp Asp Phe Ser Leu Lys Glu Arg Leu Lys Gln Ile Lys Leu Ala
65                  70                  75                  80

ttg ggt gat gag tct ttg tcc tct gcc aat acc acc atg ctg gag ggc     288
Leu Gly Asp Glu Ser Leu Ser Ser Ala Asn Thr Thr Met Leu Glu Gly
                85                  90                  95

aaa aac ctg acg gct gcc gaa ctt caa aac tat gtc cag act att ccc     336
Lys Asn Leu Thr Ala Ala Glu Leu Gln Asn Tyr Val Gln Thr Ile Pro
            100                 105                 110

ttt tta gcc cct gcc cgg ctg gtt atg gta aac ggc ctg ctg gag agg     384
Phe Leu Ala Pro Ala Arg Leu Val Met Val Asn Gly Leu Leu Glu Arg
        115                 120                 125

ttt gaa ggc tca aaa ggt aaa gcc gcc gcc aaa gat aag gca gac aaa     432
Phe Glu Gly Ser Lys Gly Lys Ala Ala Ala Lys Asp Lys Ala Asp Lys
    130                 135                 140

aac agc ccg gat gaa ttt gcc cgg gcg ctg tct aat ctg cct ccc aca     480
Asn Ser Pro Asp Glu Phe Ala Arg Ala Leu Ser Asn Leu Pro Pro Thr
145                 150                 155                 160

acc ctg gcg gtg ctt ctg gac tat acc acc gct aaa gac ggt gat tac     528
Thr Leu Ala Val Leu Leu Asp Tyr Thr Thr Ala Lys Asp Gly Asp Tyr
                165                 170                 175

cgt gaa aaa gaa aaa gta tcc gct ttg gcc tca aac ccc ctt ttt aag     576
Arg Glu Lys Glu Lys Val Ser Ala Leu Ala Ser Asn Pro Leu Phe Lys
            180                 185                 190

ctg gta agc ccc ggt gct gag gta aag cac ttt gca ccc ctt tct gcc     624
Leu Val Ser Pro Gly Ala Glu Val Lys His Phe Ala Pro Leu Ser Ala
        195                 200                 205

cag aac ctg ccc ggc tgg gta atg cag cgt gcc aaa cag tct tct atc     672
Gln Asn Leu Pro Gly Trp Val Met Gln Arg Ala Lys Gln Ser Ser Ile
    210                 215                 220

cag atg tct gtt ccg gca gcc cgt ctg ctg gcc cgt ttt gtg ggg gcg     720
Gln Met Ser Val Pro Ala Ala Arg Leu Leu Ala Arg Phe Val Gly Ala

225                     230                     235                     240

gac ttg tgg gcg ctt tcc tcc gag att gag aaa ctg ggc ttg tat gcc     768
Asp Leu Trp Ala Leu Ser Ser Glu Ile Glu Lys Leu Gly Leu Tyr Ala
                245                     250                     255

cag ggg cgg gcg gtg gag gaa gcc gat gtt gaa aat atg gtg ggt tat     816
Gln Gly Arg Ala Val Glu Glu Ala Asp Val Glu Asn Met Val Gly Tyr
            260                     265                     270

tcc cag gaa gta aat ata ttc ggt ttg gtg gat gcc gta ctg gac cgc     864
Ser Gln Glu Val Asn Ile Phe Gly Leu Val Asp Ala Val Leu Asp Arg
        275                     280                     285

aag gtt aaa gac gcc cag cag tct ctg gcg gct ttt wct gyc ggc ggc     912
Lys Val Lys Asp Ala Gln Gln Ser Leu Ala Ala Phe Xaa Xaa Gly Gly
    290                     295                     300

ggg tya ccc ggt ttt ttg ctg ttt atg ctg gta cgc cag ctg cgg ctg     960
Gly Xaa Pro Gly Phe Leu Leu Phe Met Leu Val Arg Gln Leu Arg Leu
305                     310                     315                     320

atg gtg cgt tat acc gcc ctt aaa aag cgg ggg ctg aaa gag gcc gag     1008
Met Val Arg Tyr Thr Ala Leu Lys Lys Arg Gly Leu Lys Glu Ala Glu
                325                     330                     335

att ttt aaa gct atg gkc ggc tct gag tac gcc ctt agg aaa aca gcc     1056
Ile Phe Lys Ala Met Xaa Gly Ser Glu Tyr Ala Leu Arg Lys Thr Ala
            340                     345                     350

tct cag gcg gcc tgc cag aac atg gac agc ctt aaa gct att tat tcc     1104
Ser Gln Ala Ala Cys Gln Asn Met Asp Ser Leu Lys Ala Ile Tyr Ser
        355                     360                     365

aaa ctg ctg gaa aca gat ttt aat ata aaa acc ggc cgc ttt gaa ccg     1152
Lys Leu Leu Glu Thr Asp Phe Asn Ile Lys Thr Gly Arg Phe Glu Pro
    370                     375                     380

gag ctg gcc atg ggt ttg ctg gtt gcc gaa ctt tgc ggg aga aaa tag     1200
Glu Leu Ala Met Gly Leu Leu Val Ala Glu Leu Cys Gly Arg Lys
385                     390                     395                     400

<210> 147
<211> 399
<212> PRT
<213> Dehalococcoides ethenogenes

<400> 147
Met Phe Ala Glu Phe Val Gly His Tyr Asn Phe Phe Gly His Cys Phe
1               5                   10                  15

Lys Ser Ser Lys Cys Ser Leu Arg Ala Asp Cys Asn Ile Leu Pro Gly
            20                  25                  30

Ile Phe Ala Leu Asn Phe Lys Gly Tyr Ala Pro Val Ser Leu Cys Tyr
        35                  40                  45

Asn Ile Ser Arg Arg Trp Leu Leu Asn Leu Leu Tyr Ile Phe Thr Gly
    50                  55                  60

Ser Asp Asp Phe Ser Leu Lys Glu Arg Leu Lys Gln Ile Lys Leu Ala
65                  70                  75                  80

Leu Gly Asp Glu Ser Leu Ser Ser Ala Asn Thr Thr Met Leu Glu Gly
                85                  90                  95

Lys Asn Leu Thr Ala Ala Glu Leu Gln Asn Tyr Val Gln Thr Ile Pro

                100                 105                 110

Phe Leu Ala Pro Ala Arg Leu Val Met Val Asn Gly Leu Leu Glu Arg
        115                 120                 125

Phe Glu Gly Ser Lys Gly Lys Ala Ala Ala Lys Asp Lys Ala Asp Lys
    130                 135                 140

Asn Ser Pro Asp Glu Phe Ala Arg Ala Leu Ser Asn Leu Pro Pro Thr
145                 150                 155                 160

Thr Leu Ala Val Leu Leu Asp Tyr Thr Thr Ala Lys Asp Gly Asp Tyr
                165                 170                 175

Arg Glu Lys Glu Lys Val Ser Ala Leu Ala Ser Asn Pro Leu Phe Lys
            180                 185                 190

Leu Val Ser Pro Gly Ala Glu Val Lys His Phe Ala Pro Leu Ser Ala
        195                 200                 205

Gln Asn Leu Pro Gly Trp Val Met Gln Arg Ala Lys Gln Ser Ser Ile
    210                 215                 220

Gln Met Ser Val Pro Ala Ala Arg Leu Leu Ala Arg Phe Val Gly Ala
225                 230                 235                 240

Asp Leu Trp Ala Leu Ser Ser Glu Ile Glu Lys Leu Gly Leu Tyr Ala
                245                 250                 255

Gln Gly Arg Ala Val Glu Glu Ala Asp Val Glu Asn Met Val Gly Tyr
            260                 265                 270

Ser Gln Glu Val Asn Ile Phe Gly Leu Val Asp Ala Val Leu Asp Arg
        275                 280                 285

Lys Val Lys Asp Ala Gln Gln Ser Leu Ala Ala Phe Xaa Xaa Gly Gly
    290                 295                 300

Gly Xaa Pro Gly Phe Leu Leu Phe Met Leu Val Arg Gln Leu Arg Leu
305                 310                 315                 320

Met Val Arg Tyr Thr Ala Leu Lys Lys Arg Gly Leu Lys Glu Ala Glu
                325                 330                 335

Ile Phe Lys Ala Met Xaa Gly Ser Glu Tyr Ala Leu Arg Lys Thr Ala
            340                 345                 350

Ser Gln Ala Ala Cys Gln Asn Met Asp Ser Leu Lys Ala Ile Tyr Ser
        355                 360                 365

Lys Leu Leu Glu Thr Asp Phe Asn Ile Lys Thr Gly Arg Phe Glu Pro
    370                 375                 380

Glu Leu Ala Met Gly Leu Leu Val Ala Glu Leu Cys Gly Arg Lys
385                 390                 395

<210> 148
<211> 1008
<212> DNA
<213> Porphyromonas gingivalis

<400> 148
atggcctcct attccgatat tgtcgattct gtacgtaagt gccaattctc gccactctat 60

ctgctggccg gcgaagaacc cttctatata gacgagttgg ccactctgct ggagacgcac 120

gtggtgccgg tagatgaatg ggacttcaac agggttatcc tctacggcga caaaacctcc 180

gtagccgata tagcgaacga agcgcgccgt ttcccgatga tgggccggag gcagttgatc 240

gtcgttcggg aggcgcagtt agtggacaat atcgacttgc tcgaagcaca ttatggcact 300

ttccccgata ctacgattct ggtcattgcc tataagaaga aacccgataa gcgaaaggct 360

ttctatacga aggctgaaaa gttcgggaaa gtattcgtgt ccgagaccat acccgactat 420

aagatgccgg acttcatcct gtcggctgct gccggcaaga aactgagcgt atcgcccgaa 480

gtggcatata tgctggccga ttacctcggc aatgatctgg agaagttgat gaatgagctg 540

gacaagctca ttctcataac acaagatagt aggggagtgg tcacgtccga gatcgtggag 600

cagcatatcg gagtaagcaa atccttcaat aatttcgaac tccttcgtgc catagtcaat 660

cgcgaaacgg gcaaaacctt tcgcatcgct cactactttg ccagaaatga gaaggagcac 720

cccatacagg ccactctacc cgtactcttc aactatttca gcaacctgat gatcgtttgc 780

tacttgccac agaaaaaacc cgatgctatc atgaaagcat tgtcgatacg caatttccaa 840

gtgcgcgact atatgacagg gctgaagatg tattcgacga ggaaggtttt cgacattata 900

cacgagatcc ggatgacgga tgcccgcagt aagggggtgg atacgaccgg cacatttgcc 960

ggcagtgggg atttgcttcg ggagctactc cattttatat tccattga 1008

<210> 149
<211> 1008
<212> DNA
<213> Porphyromonas gingivalis

<220>
<221> CDS
<222> (1)..(1008)

<400> 149
atg gcc tcc tat tcc gat att gtc gat tct gta cgt aag tgc caa ttc 48
Met Ala Ser Tyr Ser Asp Ile Val Asp Ser Val Arg Lys Cys Gln Phe
1               5                   10                  15

tcg cca ctc tat ctg ctg gcc ggc gaa gaa ccc ttc tat ata gac gag 96
Ser Pro Leu Tyr Leu Leu Ala Gly Glu Glu Pro Phe Tyr Ile Asp Glu
            20                  25                  30

ttg gcc act ctg ctg gag acg cac gtg gtg ccg gta gat gaa tgg gac 144
Leu Ala Thr Leu Leu Glu Thr His Val Val Pro Val Asp Glu Trp Asp
        35                  40                  45

ttc aac agg gtt atc ctc tac ggc gac aaa acc tcc gta gcc gat ata 192
Phe Asn Arg Val Ile Leu Tyr Gly Asp Lys Thr Ser Val Ala Asp Ile
    50                  55                  60

gcg aac gaa gcg cgc cgt ttc ccg atg atg ggc cgg agg cag ttg atc 240
Ala Asn Glu Ala Arg Arg Phe Pro Met Met Gly Arg Arg Gln Leu Ile
65                  70                  75                  80

gtc gtt cgg gag gcg cag tta gtg gac aat atc gac ttg ctc gaa gca 288
Val Val Arg Glu Ala Gln Leu Val Asp Asn Ile Asp Leu Leu Glu Ala
                85                  90                  95

cat tat ggc act ttc ccc gat act acg att ctg gtc att gcc tat aag 336
His Tyr Gly Thr Phe Pro Asp Thr Thr Ile Leu Val Ile Ala Tyr Lys
            100                 105                 110

aag aaa ccc gat aag cga aag gct ttc tat acg aag gct gaa aag ttc     384
Lys Lys Pro Asp Lys Arg Lys Ala Phe Tyr Thr Lys Ala Glu Lys Phe
        115                 120                 125

ggg aaa gta ttc gtg tcc gag acc ata ccc gac tat aag atg ccg gac     432
Gly Lys Val Phe Val Ser Glu Thr Ile Pro Asp Tyr Lys Met Pro Asp
    130                 135                 140

ttc atc ctg tcg gct gct gcc ggc aag aaa ctg agc gta tcg ccc gaa     480
Phe Ile Leu Ser Ala Ala Ala Gly Lys Lys Leu Ser Val Ser Pro Glu
145                 150                 155                 160

gtg gca tat atg ctg gcc gat tac ctc ggc aat gat ctg gag aag ttg     528
Val Ala Tyr Met Leu Ala Asp Tyr Leu Gly Asn Asp Leu Glu Lys Leu
                165                 170                 175

atg aat gag ctg gac aag ctc att ctc ata aca caa gat agt agg gga     576
Met Asn Glu Leu Asp Lys Leu Ile Leu Ile Thr Gln Asp Ser Arg Gly
            180                 185                 190

gtg gtc acg tcc gag atc gtg gag cag cat atc gga gta agc aaa tcc     624
Val Val Thr Ser Glu Ile Val Glu Gln His Ile Gly Val Ser Lys Ser
        195                 200                 205

ttc aat aat ttc gaa ctc ctt cgt gcc ata gtc aat cgc gaa acg ggc     672
Phe Asn Asn Phe Glu Leu Leu Arg Ala Ile Val Asn Arg Glu Thr Gly
    210                 215                 220

aaa acc ttt cgc atc gct cac tac ttt gcc aga aat gag aag gag cac     720
Lys Thr Phe Arg Ile Ala His Tyr Phe Ala Arg Asn Glu Lys Glu His
225                 230                 235                 240

ccc ata cag gcc act cta ccc gta ctc ttc aac tat ttc agc aac ctg     768
Pro Ile Gln Ala Thr Leu Pro Val Leu Phe Asn Tyr Phe Ser Asn Leu
                245                 250                 255

atg atc gtt tgc tac ttg cca cag aaa aaa ccc gat gct atc atg aaa     816
Met Ile Val Cys Tyr Leu Pro Gln Lys Lys Pro Asp Ala Ile Met Lys
            260                 265                 270

gca ttg tcg ata cgc aat ttc caa gtg cgc gac tat atg aca ggg ctg     864
Ala Leu Ser Ile Arg Asn Phe Gln Val Arg Asp Tyr Met Thr Gly Leu
        275                 280                 285

aag atg tat tcg acg agg aag gtt ttc gac att ata cac gag atc cgg     912
Lys Met Tyr Ser Thr Arg Lys Val Phe Asp Ile Ile His Glu Ile Arg
    290                 295                 300

atg acg gat gcc cgc agt aag ggg gtg gat acg acc ggc aca ttt gcc     960
Met Thr Asp Ala Arg Ser Lys Gly Val Asp Thr Thr Gly Thr Phe Ala
305                 310                 315                 320

ggc agt ggg gat ttg ctt cgg gag cta ctc cat ttt ata ttc cat tga     1008
Gly Ser Gly Asp Leu Leu Arg Glu Leu Leu His Phe Ile Phe His
                325                 330                 335

<210> 150
<211> 335
<212> PRT
<213> Porphyromonas gingivalis

<400> 150
Met Ala Ser Tyr Ser Asp Ile Val Asp Ser Val Arg Lys Cys Gln Phe
1               5                   10                  15

Ser Pro Leu Tyr Leu Leu Ala Gly Glu Glu Pro Phe Tyr Ile Asp Glu
            20                  25                  30

Leu Ala Thr Leu Leu Glu Thr His Val Val Pro Val Asp Glu Trp Asp
            35                  40                  45

Phe Asn Arg Val Ile Leu Tyr Gly Asp Lys Thr Ser Val Ala Asp Ile
    50                  55                  60

Ala Asn Glu Ala Arg Arg Phe Pro Met Met Gly Arg Arg Gln Leu Ile
65                  70                  75                  80

Val Val Arg Glu Ala Gln Leu Val Asp Asn Ile Asp Leu Leu Glu Ala
                85                  90                  95

His Tyr Gly Thr Phe Pro Asp Thr Thr Ile Leu Val Ile Ala Tyr Lys
            100                 105                 110

Lys Lys Pro Asp Lys Arg Lys Ala Phe Tyr Thr Lys Ala Glu Lys Phe
            115                 120                 125

Gly Lys Val Phe Val Ser Glu Thr Ile Pro Asp Tyr Lys Met Pro Asp
    130                 135                 140

Phe Ile Leu Ser Ala Ala Ala Gly Lys Lys Leu Ser Val Ser Pro Glu
145                 150                 155                 160

Val Ala Tyr Met Leu Ala Asp Tyr Leu Gly Asn Asp Leu Glu Lys Leu
                165                 170                 175

Met Asn Glu Leu Asp Lys Leu Ile Leu Ile Thr Gln Asp Ser Arg Gly
            180                 185                 190

Val Val Thr Ser Glu Ile Val Glu Gln His Ile Gly Val Ser Lys Ser
            195                 200                 205

Phe Asn Asn Phe Glu Leu Leu Arg Ala Ile Val Asn Arg Glu Thr Gly
    210                 215                 220

Lys Thr Phe Arg Ile Ala His Tyr Phe Ala Arg Asn Glu Lys Glu His
225                 230                 235                 240

Pro Ile Gln Ala Thr Leu Pro Val Leu Phe Asn Tyr Phe Ser Asn Leu
                245                 250                 255

Met Ile Val Cys Tyr Leu Pro Gln Lys Lys Pro Asp Ala Ile Met Lys
            260                 265                 270

Ala Leu Ser Ile Arg Asn Phe Gln Val Arg Asp Tyr Met Thr Gly Leu
            275                 280                 285

Lys Met Tyr Ser Thr Arg Lys Val Phe Asp Ile Ile His Glu Ile Arg
    290                 295                 300

Met Thr Asp Ala Arg Ser Lys Gly Val Asp Thr Thr Gly Thr Phe Ala
305                 310                 315                 320

Gly Ser Gly Asp Leu Leu Arg Glu Leu Leu His Phe Ile Phe His
                325                 330                 335

<210> 151
<211> 1005
<212> DNA
<213> Prochlorococcus marinus

<400> 151
atgccaatac aaataatatg gggaaatgat ttaaatgcaa gtaataagtt tataaaacaa 60

ataattgatg aaaaagtatc caaaacatgg ggagaaataa atattagtta cttaaatggt 120

gatgatgata atcaaataaa acaagcattt gatgaaatac taacacctcc tttgggggat 180

ggatctcgag tagttgtatt aaaaaataac ccattattca caaataaaaa tgaggaaata 240

aggctcaaat ttgaaaaaat ttatcaaaac attccaagta acacttactt tcttttacaa 300

aatacaaaaa aaccagattc aagactaaag agtacaaaat ttattcaaaa tttaataaaa 360

aaagatttag caattgaaac ctcattttca ttgcccgata tctgggattt tgaaggccag 420

aaaagatact tagaattcac agcaaattcg atgaatattc atttaggtaa agggaccgct 480

gatttaatta ttgattcagt aggaaatgat agttttaact tagctagcga attatctaaa 540

gcaaagatat atctatctgc aataaataat aacgaaaaca aaggacttat tctagaaagt 600

gatgttgtaa aaaaaatatt caatgatcaa cagtcaaata tctttaaaat aatcgatcat 660

ctattacaaa atagtattag tgatggtctt atagaaatct attatttatt aaaaaaaggg 720

gaacctgctt taaggctcaa tgcgggatta ataagtcaaa tcagaatgca taccatagta 780

aaattacttc ataaatctgg tgagcaagat ttatcaaaaa tatgcaaact tgcaaatata 840

tctaacccaa aaagaatttt ttttatccgt aaaaaagtaa acaatatatc ctcagatttc 900

ttaattaaat tgatgagtaa tttattagat attgaatcat cactaaaaaa aggcaataat 960

cctattaatg tatttgcgga aaatctcgtt aacttaagtt aataa 1005

<210> 152
<211> 1005
<212> DNA
<213> Prochlorococcus marinus

<220>
<221> CDS
<222> (1)..(1005)

<400> 152
atg cca ata caa ata ata tgg gga aat gat tta aat gca agt aat aag 48
Met Pro Ile Gln Ile Ile Trp Gly Asn Asp Leu Asn Ala Ser Asn Lys
1               5                   10                  15

ttt ata aaa caa ata att gat gaa aaa gta tcc aaa aca tgg gga gaa 96
Phe Ile Lys Gln Ile Ile Asp Glu Lys Val Ser Lys Thr Trp Gly Glu
            20                  25                  30

ata aat att agt tac tta aat ggt gat gat gat aat caa ata aaa caa 144
Ile Asn Ile Ser Tyr Leu Asn Gly Asp Asp Asp Asn Gln Ile Lys Gln
        35                  40                  45

gca ttt gat gaa ata cta aca cct cct ttg ggg gat gga tct cga gta 192
Ala Phe Asp Glu Ile Leu Thr Pro Pro Leu Gly Asp Gly Ser Arg Val
    50                  55                  60

gtt gta tta aaa aat aac cca tta ttc aca aat aaa aat gag gaa ata 240
Val Val Leu Lys Asn Asn Pro Leu Phe Thr Asn Lys Asn Glu Glu Ile
65                  70                  75                  80

agg ctc aaa ttt gaa aaa att tat caa aac att cca agt aac act tac 288
Arg Leu Lys Phe Glu Lys Ile Tyr Gln Asn Ile Pro Ser Asn Thr Tyr
                85                  90                  95

ttt ctt tta caa aat aca aaa aaa cca gat tca aga cta aag agt aca 336
Phe Leu Leu Gln Asn Thr Lys Lys Pro Asp Ser Arg Leu Lys Ser Thr

                100                 105                 110

aaa ttt att caa aat tta ata aaa aaa gat tta gca att gaa acc tca     384
Lys Phe Ile Gln Asn Leu Ile Lys Lys Asp Leu Ala Ile Glu Thr Ser
        115                 120                 125

ttt tca ttg ccc gat atc tgg gat ttt gaa ggc cag aaa aga tac tta     432
Phe Ser Leu Pro Asp Ile Trp Asp Phe Glu Gly Gln Lys Arg Tyr Leu
    130                 135                 140

gaa ttc aca gca aat tcg atg aat att cat tta ggt aaa ggg acc gct     480
Glu Phe Thr Ala Asn Ser Met Asn Ile His Leu Gly Lys Gly Thr Ala
145                 150                 155                 160

gat tta att att gat tca gta gga aat gat agt ttt aac tta gct agc     528
Asp Leu Ile Ile Asp Ser Val Gly Asn Asp Ser Phe Asn Leu Ala Ser
                165                 170                 175

gaa tta tct aaa gca aag ata tat cta tct gca ata aat aat aac gaa     576
Glu Leu Ser Lys Ala Lys Ile Tyr Leu Ser Ala Ile Asn Asn Asn Glu
            180                 185                 190

aac aaa gga ctt att cta gaa agt gat gtt gta aaa aaa ata ttc aat     624
Asn Lys Gly Leu Ile Leu Glu Ser Asp Val Val Lys Lys Ile Phe Asn
        195                 200                 205

gat caa cag tca aat atc ttt aaa ata atc gat cat cta tta caa aat     672
Asp Gln Gln Ser Asn Ile Phe Lys Ile Ile Asp His Leu Leu Gln Asn
    210                 215                 220

agt att agt gat ggt ctt ata gaa atc tat tat tta tta aaa aaa ggg     720
Ser Ile Ser Asp Gly Leu Ile Glu Ile Tyr Tyr Leu Leu Lys Lys Gly
225                 230                 235                 240

gaa cct gct tta agg ctc aat gcg gga tta ata agt caa atc aga atg     768
Glu Pro Ala Leu Arg Leu Asn Ala Gly Leu Ile Ser Gln Ile Arg Met
                245                 250                 255

cat acc ata gta aaa tta ctt cat aaa tct ggt gag caa gat tta tca     816
His Thr Ile Val Lys Leu Leu His Lys Ser Gly Glu Gln Asp Leu Ser
            260                 265                 270

aaa ata tgc aaa ctt gca aat ata tct aac cca aaa aga att ttt ttt     864
Lys Ile Cys Lys Leu Ala Asn Ile Ser Asn Pro Lys Arg Ile Phe Phe
        275                 280                 285

atc cgt aaa aaa gta aac aat ata tcc tca gat ttc tta att aaa ttg     912
Ile Arg Lys Lys Val Asn Asn Ile Ser Ser Asp Phe Leu Ile Lys Leu
    290                 295                 300

atg agt aat tta tta gat att gaa tca tca cta aaa aaa ggc aat aat     960
Met Ser Asn Leu Leu Asp Ile Glu Ser Ser Leu Lys Lys Gly Asn Asn
305                 310                 315                 320

cct att aat gta ttt gcg gaa aat ctc gtt aac tta agt taa taa        1005
Pro Ile Asn Val Phe Ala Glu Asn Leu Val Asn Leu Ser
                325                 330                 335

<210> 153
<211> 333
<212> PRT
<213> Prochlorococcus marinus

<400> 153
Met Pro Ile Gln Ile Ile Trp Gly Asn Asp Leu Asn Ala Ser Asn Lys
1               5                   10                  15

Phe Ile Lys Gln Ile Ile Asp Glu Lys Val Ser Lys Thr Trp Gly Glu
            20                  25                  30

Ile Asn Ile Ser Tyr Leu Asn Gly Asp Asp Asp Asn Gln Ile Lys Gln
        35                  40                  45

Ala Phe Asp Glu Ile Leu Thr Pro Pro Leu Gly Asp Gly Ser Arg Val
    50                  55                  60

Val Val Leu Lys Asn Asn Pro Leu Phe Thr Asn Lys Asn Glu Glu Ile
65                  70                  75                  80

Arg Leu Lys Phe Glu Lys Ile Tyr Gln Asn Ile Pro Ser Asn Thr Tyr
                85                  90                  95

Phe Leu Leu Gln Asn Thr Lys Lys Pro Asp Ser Arg Leu Lys Ser Thr
            100                 105                 110

Lys Phe Ile Gln Asn Leu Ile Lys Lys Asp Leu Ala Ile Glu Thr Ser
        115                 120                 125

Phe Ser Leu Pro Asp Ile Trp Asp Phe Glu Gly Gln Lys Arg Tyr Leu
    130                 135                 140

Glu Phe Thr Ala Asn Ser Met Asn Ile His Leu Gly Lys Gly Thr Ala
145                 150                 155                 160

Asp Leu Ile Ile Asp Ser Val Gly Asn Asp Ser Phe Asn Leu Ala Ser
                165                 170                 175

Glu Leu Ser Lys Ala Lys Ile Tyr Leu Ser Ala Ile Asn Asn Asn Glu
            180                 185                 190

Asn Lys Gly Leu Ile Leu Glu Ser Asp Val Val Lys Lys Ile Phe Asn
        195                 200                 205

Asp Gln Gln Ser Asn Ile Phe Lys Ile Ile Asp His Leu Leu Gln Asn
    210                 215                 220

Ser Ile Ser Asp Gly Leu Ile Glu Ile Tyr Tyr Leu Leu Lys Lys Gly
225                 230                 235                 240

Glu Pro Ala Leu Arg Leu Asn Ala Gly Leu Ile Ser Gln Ile Arg Met
                245                 250                 255

His Thr Ile Val Lys Leu Leu His Lys Ser Gly Glu Gln Asp Leu Ser
            260                 265                 270

Lys Ile Cys Lys Leu Ala Asn Ile Ser Asn Pro Lys Arg Ile Phe Phe
        275                 280                 285

Ile Arg Lys Lys Val Asn Asn Ile Ser Ser Asp Phe Leu Ile Lys Leu
    290                 295                 300

Met Ser Asn Leu Leu Asp Ile Glu Ser Ser Leu Lys Lys Gly Asn Asn
305                 310                 315                 320

Pro Ile Asn Val Phe Ala Glu Asn Leu Val Asn Leu Ser
                325                 330

<210> 154
<211> 1032
<212> DNA
<213> Salmonella typhi

<400> 154
atgatcaggt tgtatcctga acaactccgc gcgcagctca acgaagggct gcgtgcggca 60

tatctattat tgggcaacga tccgctgctg ctgcaggaaa gtcaggacgc tattcgtctg 120

gcggctgcgt cgcaaggctt cgaagagcac cacgctttta ctctcgatcc cagtaccgac 180

tggggatcgc ttttctcact ctgccaggcg atgagcctgt tcgccagccg ccaaacgctg 240

gtgctgcaac tgccggaaaa cgggcctaac gcggcaatga atgagcaact cgccaccctg 300

agcgagctac tgcatgacga cctgttatta atagtacgcg gaaacaagct caccaaagcg 360

caggaaaacg ccgcctggta taccgcgctg gcagatcgta gcgttcaggt cagttgtcag 420

acgccggagc aggcgcagct tccccgctgg gtggccgcca gagcgaaagc acagaatttg 480

caactggatg acgccgcgaa ccaactgctg tgctattgct atgagggcaa tctgttggcg 540

ctggcgcagg cgctggaacg gctgtcgctg ctttggcctg acggtaagtt aacgcttcct 600

cgcgtggagc aggccgtcaa tgacgccgcc cactttaccc ctttccactg ggtggatgcg 660

ttactgatgg ggaaaagcaa acgcgcgctg catatcctgc aacagctacg ccttgaaggc 720

agcgaaccgg ttattctgct gcggaccctc cagcgagagc tgctgctact ggtaaatctc 780

aaacgtcagt ccgcgcatac gccgttacgc gcgctgtttg ataaacaccg ggtctggcaa 840

aaccgtcgcc ccatgatcgg cgacgcgttg cagcgtctcc atcctgcaca gcttcgccag 900

gccgtgcagt tactgacgcg tacagaaatt acgctcaaac aggattatgg tcagtcggta 960

tgggcagatc ttgaagggct gtcgctcctg ctctgccata aggcgctggc agacgtattt 1020

attgatgggt ga 1032

<210> 155
<211> 1032
<212> DNA
<213> Salmonella typhi

<220>
<221> CDS
<222> (1)..(1032)

<400> 155
atg atc agg ttg tat cct gaa caa ctc cgc gcg cag ctc aac gaa ggg 48
Met Ile Arg Leu Tyr Pro Glu Gln Leu Arg Ala Gln Leu Asn Glu Gly
1               5                   10                  15

ctg cgt gcg gca tat cta tta ttg ggc aac gat ccg ctg ctg ctg cag 96
Leu Arg Ala Ala Tyr Leu Leu Leu Gly Asn Asp Pro Leu Leu Leu Gln
            20                  25                  30

gaa agt cag gac gct att cgt ctg gcg gct gcg tcg caa ggc ttc gaa 144
Glu Ser Gln Asp Ala Ile Arg Leu Ala Ala Ala Ser Gln Gly Phe Glu
        35                  40                  45

gag cac cac gct ttt act ctc gat ccc agt acc gac tgg gga tcg ctt 192
Glu His His Ala Phe Thr Leu Asp Pro Ser Thr Asp Trp Gly Ser Leu
    50                  55                  60

ttc tca ctc tgc cag gcg atg agc ctg ttc gcc agc cgc caa acg ctg 240
Phe Ser Leu Cys Gln Ala Met Ser Leu Phe Ala Ser Arg Gln Thr Leu
65                  70                  75                  80

gtg ctg caa ctg ccg gaa aac ggg cct aac gcg gca atg aat gag caa 288
Val Leu Gln Leu Pro Glu Asn Gly Pro Asn Ala Ala Met Asn Glu Gln

                85                  90                  95

ctc gcc acc ctg agc gag cta ctg cat gac gac ctg tta tta ata gta 336
Leu Ala Thr Leu Ser Glu Leu Leu His Asp Asp Leu Leu Leu Ile Val
            100                 105                 110

cgc gga aac aag ctc acc aaa gcg cag gaa aac gcc gcc tgg tat acc 384
Arg Gly Asn Lys Leu Thr Lys Ala Gln Glu Asn Ala Ala Trp Tyr Thr
        115                 120                 125

gcg ctg gca gat cgt agc gtt cag gtc agt tgt cag acg ccg gag cag 432
Ala Leu Ala Asp Arg Ser Val Gln Val Ser Cys Gln Thr Pro Glu Gln
    130                 135                 140

gcg cag ctt ccc cgc tgg gtg gcc gcc aga gcg aaa gca cag aat ttg 480
Ala Gln Leu Pro Arg Trp Val Ala Ala Arg Ala Lys Ala Gln Asn Leu
145                 150                 155                 160

caa ctg gat gac gcc gcg aac caa ctg ctg tgc tat tgc tat gag ggc 528
Gln Leu Asp Asp Ala Ala Asn Gln Leu Leu Cys Tyr Cys Tyr Glu Gly
                165                 170                 175

aat ctg ttg gcg ctg gcg cag gcg ctg gaa cgg ctg tcg ctg ctt tgg 576
Asn Leu Leu Ala Leu Ala Gln Ala Leu Glu Arg Leu Ser Leu Leu Trp
            180                 185                 190

cct gac ggt aag tta acg ctt cct cgc gtg gag cag gcc gtc aat gac 624
Pro Asp Gly Lys Leu Thr Leu Pro Arg Val Glu Gln Ala Val Asn Asp
        195                 200                 205

gcc gcc cac ttt acc cct ttc cac tgg gtg gat gcg tta ctg atg ggg 672
Ala Ala His Phe Thr Pro Phe His Trp Val Asp Ala Leu Leu Met Gly
    210                 215                 220

aaa agc aaa cgc gcg ctg cat atc ctg caa cag cta cgc ctt gaa ggc 720
Lys Ser Lys Arg Ala Leu His Ile Leu Gln Gln Leu Arg Leu Glu Gly
225                 230                 235                 240

agc gaa ccg gtt att ctg ctg cgg acc ctc cag cga gag ctg ctg cta 768
Ser Glu Pro Val Ile Leu Leu Arg Thr Leu Gln Arg Glu Leu Leu Leu
                245                 250                 255

ctg gta aat ctc aaa cgt cag tcc gcg cat acg ccg tta cgc gcg ctg 816
Leu Val Asn Leu Lys Arg Gln Ser Ala His Thr Pro Leu Arg Ala Leu
            260                 265                 270

ttt gat aaa cac cgg gtc tgg caa aac cgt cgc ccc atg atc ggc gac 864
Phe Asp Lys His Arg Val Trp Gln Asn Arg Arg Pro Met Ile Gly Asp
        275                 280                 285

gcg ttg cag cgt ctc cat cct gca cag ctt cgc cag gcc gtg cag tta 912
Ala Leu Gln Arg Leu His Pro Ala Gln Leu Arg Gln Ala Val Gln Leu
    290                 295                 300

ctg acg cgt aca gaa att acg ctc aaa cag gat tat ggt cag tcg gta 960
Leu Thr Arg Thr Glu Ile Thr Leu Lys Gln Asp Tyr Gly Gln Ser Val
305                 310                 315                 320

tgg gca gat ctt gaa ggg ctg tcg ctc ctg ctc tgc cat aag gcg ctg 1008
Trp Ala Asp Leu Glu Gly Leu Ser Leu Leu Leu Cys His Lys Ala Leu
                325                 330                 335

gca gac gta ttt att gat ggg tga 1032
Ala Asp Val Phe Ile Asp Gly
            340

<210> 156

<211> 343
<212> PRT
<213> Salmonella typhi

<400> 156
Met Ile Arg Leu Tyr Pro Glu Gln Leu Arg Ala Gln Leu Asn Glu Gly
1               5                   10                  15

Leu Arg Ala Ala Tyr Leu Leu Leu Gly Asn Asp Pro Leu Leu Leu Gln
            20                  25                  30

Glu Ser Gln Asp Ala Ile Arg Leu Ala Ala Ala Ser Gln Gly Phe Glu
        35                  40                  45

Glu His His Ala Phe Thr Leu Asp Pro Ser Thr Asp Trp Gly Ser Leu
    50                  55                  60

Phe Ser Leu Cys Gln Ala Met Ser Leu Phe Ala Ser Arg Gln Thr Leu
65                  70                  75                  80

Val Leu Gln Leu Pro Glu Asn Gly Pro Asn Ala Ala Met Asn Glu Gln
                85                  90                  95

Leu Ala Thr Leu Ser Glu Leu Leu His Asp Asp Leu Leu Leu Ile Val
            100                 105                 110

Arg Gly Asn Lys Leu Thr Lys Ala Gln Glu Asn Ala Ala Trp Tyr Thr
        115                 120                 125

Ala Leu Ala Asp Arg Ser Val Gln Val Ser Cys Gln Thr Pro Glu Gln
    130                 135                 140

Ala Gln Leu Pro Arg Trp Val Ala Ala Arg Ala Lys Ala Gln Asn Leu
145                 150                 155                 160

Gln Leu Asp Asp Ala Ala Asn Gln Leu Leu Cys Tyr Cys Tyr Glu Gly
                165                 170                 175

Asn Leu Leu Ala Leu Ala Gln Ala Leu Glu Arg Leu Ser Leu Leu Trp
            180                 185                 190

Pro Asp Gly Lys Leu Thr Leu Pro Arg Val Glu Gln Ala Val Asn Asp
        195                 200                 205

Ala Ala His Phe Thr Pro Phe His Trp Val Asp Ala Leu Leu Met Gly
    210                 215                 220

Lys Ser Lys Arg Ala Leu His Ile Leu Gln Gln Leu Arg Leu Glu Gly
225                 230                 235                 240

Ser Glu Pro Val Ile Leu Leu Arg Thr Leu Gln Arg Glu Leu Leu Leu
                245                 250                 255

Leu Val Asn Leu Lys Arg Gln Ser Ala His Thr Pro Leu Arg Ala Leu
            260                 265                 270

Phe Asp Lys His Arg Val Trp Gln Asn Arg Arg Pro Met Ile Gly Asp
        275                 280                 285

Ala Leu Gln Arg Leu His Pro Ala Gln Leu Arg Gln Ala Val Gln Leu
    290                 295                 300

Leu Thr Arg Thr Glu Ile Thr Leu Lys Gln Asp Tyr Gly Gln Ser Val
305                 310                 315                 320

Trp Ala Asp Leu Glu Gly Leu Ser Leu Leu Leu Cys His Lys Ala Leu
                325                 330                 335

Ala Asp Val Phe Ile Asp Gly
            340

<210> 157
<211> 32
<212> DNA
<213> Artificial Sequence

<220>
<223> Description of Artificial Sequence: Primer

<400> 157
gatcctgcag gaaaccacaa tattccagtt cc                                32

<210> 158
<211> 40
<212> DNA
<213> Artificial Sequence

<220>
<223> Description of Artificial Sequence: Primer

<400> 158
gatcaagctt ttattatcca ccatgagaag tatttttcac                        40

<210> 159
<211> 32
<212> DNA
<213> Artificial Sequence

<220>
<223> Description of Artificial Sequence: Primer

<400> 159
gatccatatg gaaaccacaa tattccagtt cc                                32

<210> 160
<211> 40
<212> DNA
<213> Artificial Sequence

<220>
<223> Description of Artificial Sequence: Primer

<400> 160
gatcggtacc ttattatcca ccatgagaag tatttttcac                        40

<210> 161
<211> 32
<212> DNA
<213> Artificial Sequence

<220>
<223> Description of Artificial Sequence: Primer

<400> 161
gatcggtacc cagaggtgat tgtatgccag tc                                32

<210> 162
<211> 34
<212> DNA
<213> Artificial Sequence

<220>
<223> Description of Artificial Sequence: Primer

<400> 162
gatcactagt ttattattct tcatctctct gaag                                34


<210> 163
<211> 29
<212> DNA
<213> Artificial Sequence

<220>
<223> Description of Artificial Sequence: Primer

<400> 163
gatccatatg agaggtgatt gtatgccag                                      29


<210> 164
<211> 35
<212> DNA
<213> Artificial Sequence

<220>
<223> Description of Artificial Sequence: Primer

<400> 164
gatcctgcag gaaaaagttt ttttggaaaa actcc                               35


<210> 165
<211> 36
<212> DNA
<213> Artificial Sequence

<220>
<223> Description of Artificial Sequence: Primer

<400> 165
gatcggtacc ttattaatcc gcctgaacgg ctaacg                              36


<210> 166
<211> 35
<212> DNA
<213> Artificial Sequence

<220>
<223> Description of Artificial Sequence: Primer

<400> 166
gatccatatg gaaaaagttt ttttggaaaa actcc                               35


<210> 167
<211> 31
<212> DNA
<213> Artificial Sequence

<220>
<223> Description of Artificial Sequence: Primer

<400> 167
gatcctgcag agtatttcag aaaaaccctt g                                   31

<210> 168
<211> 35
<212> DNA
<213> Artificial Sequence

<220>
<223> Description of Artificial Sequence: Primer

<400> 168
gatcggtacc taatcaaacg ctgaactttt cttcg                                35


<210> 169
<211> 27
<212> DNA
<213> Artificial Sequence

<220>
<223> Description of Artificial Sequence: Primer

<400> 169
gatctcatga gtatttcaga aaaaccc                                         27


<210> 170
<211> 33
<212> DNA
<213> Artificial Sequence

<220>
<223> Description of Artificial Sequence: Primer

<400> 170
gatcctgcag aacgatttga tcagaaagta cgc                                  33


<210> 171
<211> 34
<212> DNA
<213> Artificial Sequence

<220>
<223> Description of Artificial Sequence: Primer

<400> 171
gatcggtacc ttatcagctc caagcgttga cacc                                 34


<210> 172
<211> 33
<212> DNA
<213> Artificial Sequence

<220>
<223> Description of Artificial Sequence: Primer

<400> 172
gatccatatg aacgatttga tcagaaagta cgc                                  33


<210> 173
<211> 1161
<212> PRT
<213> Aquifex aeolicus

<400> 173
Met Ser Lys Asp Phe Val His Leu His Leu His Thr Gln Phe Ser Leu
1               5                   10                  15

Leu Asp Gly Ala Ile Lys Ile Asp Glu Leu Val Lys Lys Ala Lys Glu
            20                  25                  30

Tyr Gly Tyr Lys Ala Val Gly Met Ser Asp His Gly Asn Leu Phe Gly
        35                  40                  45

Ser Tyr Lys Phe Tyr Lys Ala Leu Lys Ala Glu Gly Ile Lys Pro Ile
    50                  55                  60

Ile Gly Met Glu Ala Tyr Phe Thr Thr Gly Ser Arg Phe Asp Arg Lys
65                  70                  75                  80

Thr Lys Thr Ser Glu Asp Asn Ile Thr Asp Lys Tyr Asn His His Leu
                85                  90                  95

Ile Leu Ile Ala Lys Asp Asp Lys Gly Leu Lys Asn Leu Met Lys Leu
            100                 105                 110

Ser Thr Leu Ala Tyr Lys Glu Gly Phe Tyr Tyr Lys Pro Arg Ile Asp
        115                 120                 125

Tyr Glu Leu Leu Glu Lys Tyr Gly Glu Gly Leu Ile Ala Leu Thr Ala
    130                 135                 140

Cys Leu Lys Gly Val Pro Thr Tyr Tyr Ala Ser Ile Asn Glu Val Lys
145                 150                 155                 160

Lys Ala Glu Glu Trp Val Lys Lys Phe Lys Asp Ile Phe Gly Asp Asp
                165                 170                 175

Leu Tyr Leu Glu Leu Gln Ala Asn Asn Ile Pro Glu Gln Glu Val Ala
            180                 185                 190

Asn Arg Asn Leu Ile Glu Ile Ala Lys Lys Tyr Asp Val Lys Leu Ile
        195                 200                 205

Ala Thr Gln Asp Ala His Tyr Leu Asn Pro Glu Asp Arg Tyr Ala His
    210                 215                 220

Thr Val Leu Met Ala Leu Gln Met Lys Lys Thr Ile His Glu Leu Ser
225                 230                 235                 240

Ser Gly Asn Phe Lys Cys Ser Asn Glu Asp Leu His Phe Ala Pro Pro
                245                 250                 255

Glu Tyr Met Trp Lys Lys Phe Glu Gly Lys Phe Glu Gly Trp Glu Lys
            260                 265                 270

Ala Leu Leu Asn Thr Leu Glu Val Met Glu Lys Thr Ala Asp Ser Phe
        275                 280                 285

Glu Ile Phe Glu Asn Ser Thr Tyr Leu Leu Pro Lys Tyr Asp Val Pro
    290                 295                 300

Pro Asp Lys Thr Leu Glu Glu Tyr Leu Arg Glu Leu Ala Tyr Lys Gly
305                 310                 315                 320

Leu Arg Gln Arg Ile Glu Arg Gly Gln Ala Lys Asp Thr Lys Glu Tyr
                325                 330                 335

Trp Glu Arg Leu Glu Tyr Glu Leu Glu Val Ile Asn Lys Met Gly Phe
            340                 345                 350

Ala Gly Tyr Phe Leu Ile Val Gln Asp Phe Ile Asn Trp Ala Lys Lys
        355                 360                 365

Asn Asp Ile Pro Val Gly Pro Gly Arg Gly Ser Ala Gly Gly Ser Leu

370                 375                 380

Val Ala Tyr Ala Ile Gly Ile Thr Asp Val Asp Pro Ile Lys His Gly
385                 390                 395                 400

Phe Leu Phe Glu Arg Phe Leu Asn Pro Glu Arg Val Ser Met Pro Asp
                405                 410                 415

Ile Asp Val Asp Phe Cys Gln Asp Asn Arg Glu Lys Val Ile Glu Tyr
            420                 425                 430

Val Arg Asn Lys Tyr Gly His Asp Asn Val Ala Gln Ile Ile Thr Tyr
        435                 440                 445

Asn Val Met Lys Ala Lys Gln Thr Leu Arg Asp Val Ala Arg Ala Met
    450                 455                 460

Gly Leu Pro Tyr Ser Thr Ala Asp Lys Leu Ala Lys Leu Ile Pro Gln
465                 470                 475                 480

Gly Asp Val Gln Gly Thr Trp Leu Ser Leu Glu Glu Met Tyr Lys Thr
                485                 490                 495

Pro Val Glu Glu Leu Leu Gln Lys Tyr Gly Glu His Arg Thr Asp Ile
            500                 505                 510

Glu Asp Asn Val Lys Lys Phe Arg Gln Ile Cys Glu Glu Ser Pro Glu
        515                 520                 525

Ile Lys Gln Leu Val Glu Thr Ala Leu Lys Leu Glu Gly Leu Thr Arg
    530                 535                 540

His Thr Ser Leu His Ala Ala Gly Val Val Ile Ala Pro Lys Pro Leu
545                 550                 555                 560

Ser Glu Leu Val Pro Leu Tyr Tyr Asp Lys Glu Gly Glu Val Ala Thr
                565                 570                 575

Gln Tyr Asp Met Val Gln Leu Glu Glu Leu Gly Leu Leu Lys Met Asp
            580                 585                 590

Phe Leu Gly Leu Lys Thr Leu Thr Glu Leu Lys Leu Met Lys Glu Leu
        595                 600                 605

Ile Lys Glu Arg His Gly Val Asp Ile Asn Phe Leu Glu Leu Pro Leu
    610                 615                 620

Asp Asp Pro Lys Val Tyr Lys Leu Leu Gln Glu Gly Lys Thr Thr Gly
625                 630                 635                 640

Val Phe Gln Leu Glu Ser Arg Gly Met Lys Glu Leu Leu Lys Lys Leu
                645                 650                 655

Lys Pro Asp Ser Phe Asp Asp Ile Val Ala Val Leu Ala Leu Tyr Arg
            660                 665                 670

Pro Gly Pro Leu Lys Ser Gly Leu Val Asp Thr Tyr Ile Lys Arg Lys
        675                 680                 685

His Gly Lys Glu Pro Val Glu Tyr Pro Phe Pro Glu Leu Glu Pro Val
    690                 695                 700

Leu Lys Glu Thr Tyr Gly Val Ile Val Tyr Gln Glu Gln Val Met Lys
705                 710                 715                 720

Met Ser Gln Ile Leu Ser Gly Phe Thr Pro Gly Glu Ala Asp Thr Leu
                725                 730                 735

Arg Lys Ala Ile Gly Lys Lys Lys Ala Asp Leu Met Ala Gln Met Lys
            740                 745                 750

Asp Lys Phe Ile Gln Gly Ala Val Glu Arg Gly Tyr Pro Glu Glu Lys
        755                 760                 765

Ile Arg Lys Leu Trp Glu Asp Ile Glu Lys Phe Ala Ser Tyr Ser Phe
    770                 775                 780

Asn Lys Ser His Ser Val Ala Tyr Gly Tyr Ile Ser Tyr Trp Thr Ala
785                 790                 795                 800

Tyr Val Lys Ala His Tyr Pro Ala Glu Phe Phe Ala Val Lys Leu Thr
                805                 810                 815

Thr Glu Lys Asn Asp Asn Lys Phe Leu Asn Leu Ile Lys Asp Ala Lys
            820                 825                 830

Leu Phe Gly Phe Glu Ile Leu Pro Pro Asp Ile Asn Lys Ser Asp Val
        835                 840                 845

Gly Phe Thr Ile Glu Gly Glu Asn Arg Ile Arg Phe Gly Leu Ala Arg
    850                 855                 860

Ile Lys Gly Val Gly Glu Glu Thr Ala Lys Ile Ile Val Glu Ala Arg
865                 870                 875                 880

Lys Lys Tyr Lys Gln Phe Lys Gly Leu Ala Asp Phe Ile Asn Lys Thr
                885                 890                 895

Lys Asn Arg Lys Ile Asn Lys Lys Val Val Glu Ala Leu Val Lys Ala
            900                 905                 910

Gly Ala Phe Asp Phe Thr Lys Lys Lys Arg Lys Glu Leu Leu Ala Lys
        915                 920                 925

Val Ala Asn Ser Glu Lys Ala Leu Met Ala Thr Gln Asn Ser Leu Phe
    930                 935                 940

Gly Ala Pro Lys Glu Glu Val Glu Glu Leu Asp Pro Leu Lys Leu Glu
945                 950                 955                 960

Lys Glu Val Leu Gly Phe Tyr Ile Ser Gly His Pro Leu Asp Asn Tyr
                965                 970                 975

Glu Lys Leu Leu Lys Asn Arg Tyr Thr Pro Ile Glu Asp Leu Glu Glu
            980                 985                 990

Trp Asp Lys Glu Ser Glu Ala Val Leu Thr Gly Val Ile Thr Glu Leu
        995                 1000                1005

Lys Val Lys Lys Thr Lys Asn Gly Asp Tyr Met Ala Val Phe Asn Leu
    1010                1015                1020

Val Asp Lys Thr Gly Leu Ile Glu Cys Val Val Phe Pro Gly Val Tyr
1025                1030                1035                1040

Glu Glu Ala Lys Glu Leu Ile Glu Glu Asp Arg Val Val Val Val Lys
                1045                1050                1055

Gly Phe Leu Asp Glu Asp Leu Glu Thr Glu Asn Val Lys Phe Val Val
            1060                1065                1070

Lys Glu Val Phe Ser Pro Glu Glu Phe Ala Lys Glu Met Arg Asn Thr
        1075                1080                1085

Leu Tyr Ile Phe Leu Lys Arg Glu Gln Ala Leu Asn Gly Val Ala Glu
    1090                1095                1100

Lys Leu Lys Gly Ile Ile Glu Asn Asn Arg Thr Glu Asp Gly Tyr Asn
1105                1110                1115                1120

Leu Val Leu Thr Val Asp Leu Gly Asp Tyr Phe Val Asp Leu Ala Leu
                1125                1130                1135

Pro Gln Asp Met Lys Leu Lys Ala Asp Arg Lys Val Val Glu Glu Ile
            1140                1145                1150

Glu Lys Leu Gly Val Lys Val Ile Ile
        1155                1160

<210> 174
<211> 363
<212> PRT
<213> Aquifex aeolicus

<400> 174
Met Arg Val Lys Val Asp Arg Glu Glu Leu Glu Glu Val Leu Lys Lys
1               5                   10                  15

Ala Arg Glu Ser Thr Glu Lys Lys Ala Ala Leu Pro Ile Leu Ala Asn
            20                  25                  30

Phe Leu Leu Ser Ala Lys Glu Glu Asn Leu Ile Val Arg Ala Thr Asp
        35                  40                  45

Leu Glu Asn Tyr Leu Val Val Ser Val Lys Gly Glu Val Glu Glu Glu
    50                  55                  60

Gly Glu Val Cys Val His Ser Gln Lys Leu Tyr Asp Ile Val Lys Asn
65                  70                  75                  80

Leu Asn Ser Ala Tyr Val Tyr Leu His Thr Glu Gly Glu Lys Leu Val
                85                  90                  95

Ile Thr Gly Gly Lys Ser Thr Tyr Lys Leu Pro Thr Ala Pro Ala Glu
            100                 105                 110

Asp Phe Pro Glu Phe Pro Glu Ile Val Glu Gly Gly Glu Thr Leu Ser
        115                 120                 125

Gly Asn Leu Leu Val Asn Gly Ile Glu Lys Val Glu Tyr Ala Ile Ala
    130                 135                 140

Lys Glu Glu Ala Asn Ile Ala Leu Gln Gly Met Tyr Leu Arg Gly Tyr
145                 150                 155                 160

Glu Asp Arg Ile His Phe Val Gly Ser Asp Gly His Arg Leu Ala Leu
                165                 170                 175

Tyr Glu Pro Leu Gly Glu Phe Ser Lys Glu Leu Leu Ile Pro Arg Lys
            180                 185                 190

Ser Leu Lys Val Leu Lys Lys Leu Ile Thr Gly Ile Glu Asp Val Asn
        195                 200                 205

Ile Glu Lys Ser Glu Asp Glu Ser Phe Ala Tyr Phe Ser Thr Pro Glu
    210                 215                 220

Trp Lys Leu Ala Val Arg Leu Leu Glu Gly Glu Phe Pro Asp Tyr Met
225                 230                 235                 240

Ser Val Ile Pro Glu Glu Phe Ser Ala Glu Val Leu Phe Glu Thr Glu
                245                 250                 255

Glu Val Leu Lys Val Leu Lys Arg Leu Lys Ala Leu Ser Glu Gly Lys
            260                 265                 270

Val Phe Pro Val Lys Ile Thr Leu Ser Glu Asn Leu Ala Ile Phe Glu
        275                 280                 285

Phe Ala Asp Pro Glu Phe Gly Glu Ala Arg Glu Glu Ile Glu Val Glu
    290                 295                 300

Tyr Thr Gly Glu Pro Phe Glu Ile Gly Phe Asn Gly Lys Tyr Leu Met
305                 310                 315                 320

Glu Ala Leu Asp Ala Tyr Asp Ser Glu Arg Val Trp Phe Lys Phe Thr
                325                 330                 335

Thr Pro Asp Thr Ala Thr Leu Leu Glu Ala Glu Asp Tyr Glu Lys Glu
            340                 345                 350

Pro Tyr Lys Cys Ile Ile Met Pro Met Arg Val
        355                 360

<210> 175
<211> 202
<212> PRT
<213> Aquifex aeolicus

<400> 175
Met Lys Ser Ser Pro Lys Ser Leu Lys Met Arg Asp Asn Leu Leu Asp
1               5                   10                  15

Gly Thr Phe Val Val Ile Asp Leu Glu Ala Thr Gly Phe Asp Val Glu
            20                  25                  30

Lys Ser Glu Val Ile Asp Leu Ala Ala Val Arg Val Glu Gly Gly Ile
        35                  40                  45

Ile Thr Glu Lys Phe Ser Thr Leu Val Tyr Pro Gly Tyr Phe Ile Pro
    50                  55                  60

Glu Arg Ile Lys Lys Leu Thr Gly Ile Thr Asn Ala Met Leu Val Gly
65                  70                  75                  80

Gln Pro Thr Ile Glu Glu Val Leu Pro Glu Phe Leu Glu Phe Val Gly
                85                  90                  95

Asp Asn Ile Val Val Gly His Phe Val Glu Gln Asp Ile Lys Phe Ile
            100                 105                 110

Asn Lys Tyr Thr Lys Gln Tyr Arg Gly Lys Lys Phe Arg Asn Pro Ser
        115                 120                 125

Leu Cys Thr Leu Lys Leu Ala Arg Lys Val Phe Pro Gly Leu Lys Lys
    130                 135                 140

Tyr Ser Leu Lys Glu Ile Ala Glu Asn Phe Gly Phe Glu Thr Asn Gly
145                 150                 155                 160

Val His Arg Ala Leu Lys Asp Ala Thr Leu Thr Ala Glu Ile Phe Ile
                165                 170                 175

Lys Ile Leu Glu Glu Leu Trp Phe Lys Tyr Gly Ile Gly Asp Tyr Tyr
            180                 185                 190

Ser Leu Lys Arg Leu Glu Lys Gly Lys Phe
        195                 200

<210> 176
<211> 473
<212> PRT
<213> Aquifex aeolicus

<400> 176
Met Asn Tyr Val Pro Phe Ala Arg Lys Tyr Arg Pro Lys Phe Phe Arg
1               5                   10                  15

Glu Val Ile Gly Gln Glu Ala Pro Val Arg Ile Leu Lys Asn Ala Ile
            20                  25                  30

Lys Asn Asp Arg Val Ala His Ala Tyr Leu Phe Ala Gly Pro Arg Gly
        35                  40                  45

Val Gly Lys Thr Thr Ile Ala Arg Ile Leu Ala Lys Ala Leu Asn Cys
    50                  55                  60

Lys Asn Pro Ser Lys Gly Glu Pro Cys Gly Glu Cys Glu Asn Cys Arg
65                  70                  75                  80

Glu Ile Asp Arg Gly Val Phe Pro Asp Leu Ile Glu Met Asp Ala Ala
                85                  90                  95

Ser Asn Arg Gly Ile Asp Asp Val Arg Ala Leu Lys Glu Ala Val Asn
            100                 105                 110

Tyr Lys Pro Ile Lys Gly Lys Tyr Lys Val Tyr Ile Ile Asp Glu Ala
        115                 120                 125

His Met Leu Thr Lys Glu Ala Phe Asn Ala Leu Leu Lys Thr Leu Glu
    130                 135                 140

Glu Pro Pro Pro Arg Thr Val Phe Val Leu Cys Thr Thr Glu Tyr Asp
145                 150                 155                 160

Lys Ile Leu Pro Thr Ile Leu Ser Arg Cys Gln Arg Ile Ile Phe Ser
                165                 170                 175

Lys Val Arg Lys Glu Lys Val Ile Glu Tyr Leu Lys Lys Ile Cys Glu
            180                 185                 190

Lys Glu Gly Ile Glu Cys Glu Glu Gly Ala Leu Glu Val Leu Ala His
        195                 200                 205

Ala Ser Glu Gly Cys Met Arg Asp Ala Ala Ser Leu Leu Asp Gln Ala
    210                 215                 220

Ser Val Tyr Gly Glu Gly Arg Val Thr Lys Glu Val Val Glu Asn Phe
225                 230                 235                 240

Leu Gly Ile Leu Ser Gln Glu Ser Val Arg Ser Phe Leu Lys Leu Leu
                245                 250                 255

Leu Asn Ser Glu Val Asp Glu Ala Ile Lys Phe Leu Arg Glu Leu Ser
            260                 265                 270

Glu Lys Gly Tyr Asn Leu Thr Lys Phe Trp Glu Met Leu Glu Glu Glu
        275                 280                 285

Val Arg Asn Ala Ile Leu Val Lys Ser Leu Lys Asn Pro Glu Ser Val
    290                 295                 300

Val Gln Asn Trp Gln Asp Tyr Glu Asp Phe Lys Asp Tyr Pro Leu Glu
305                 310                 315                 320

Ala Leu Leu Tyr Val Glu Asn Leu Ile Asn Arg Gly Lys Val Glu Ala
                325                 330                 335

Arg Thr Arg Glu Pro Leu Arg Ala Phe Glu Leu Ala Val Ile Lys Ser
            340                 345                 350

Leu Ile Val Lys Asp Ile Ile Pro Val Ser Gln Leu Gly Ser Val Val
        355                 360                 365

Lys Glu Thr Lys Lys Glu Glu Lys Lys Val Glu Val Lys Glu Glu Pro
    370                 375                 380

Lys Val Lys Glu Glu Lys Pro Lys Glu Gln Glu Glu Asp Arg Phe Gln
385                 390                 395                 400

Lys Val Leu Asn Ala Val Asp Gly Lys Ile Leu Lys Arg Ile Leu Glu
                405                 410                 415

Gly Ala Lys Arg Glu Glu Arg Asp Gly Lys Ile Val Leu Lys Ile Glu
            420                 425                 430

Ala Ser Tyr Leu Arg Thr Met Lys Lys Glu Phe Asp Ser Leu Lys Glu
        435                 440                 445

Thr Phe Pro Phe Leu Glu Phe Glu Pro Val Glu Asp Lys Lys Lys Pro
    450                 455                 460

Gln Lys Ser Ser Gly Thr Arg Leu Phe
465                 470


<210> 177
<211> 147
<212> PRT
<213> Aquifex aeolicus

<400> 177
Met Leu Asn Lys Val Phe Ile Ile Gly Arg Leu Thr Gly Asp Pro Val
1               5                   10                  15

Ile Thr Tyr Leu Pro Ser Gly Thr Pro Val Val Glu Phe Thr Leu Ala
            20                  25                  30

Tyr Asn Arg Arg Tyr Lys Asn Gln Asn Gly Glu Phe Gln Glu Glu Ser
        35                  40                  45

His Phe Phe Asp Val Lys Ala Tyr Gly Lys Met Ala Glu Asp Trp Ala
    50                  55                  60

Thr Arg Phe Ser Lys Gly Tyr Leu Val Leu Val Glu Gly Arg Leu Ser
65                  70                  75                  80

Gln Glu Lys Trp Glu Lys Glu Gly Lys Lys Phe Ser Lys Val Arg Ile
                85                  90                  95

Ile Ala Glu Asn Val Arg Leu Ile Asn Arg Pro Lys Gly Ala Glu Leu
            100                 105                 110

Gln Ala Glu Glu Glu Glu Glu Val Pro Pro Ile Glu Glu Glu Ile Glu
        115                 120                 125

Lys Leu Gly Lys Glu Glu Glu Lys Pro Phe Thr Asp Glu Glu Asp Glu
    130                 135                 140

Ile Pro Phe
145


<210> 178
<211> 478

<212> PRT
<213> Thermotoga maritima

<400> 178
Met Glu Val Leu Tyr Arg Lys Tyr Arg Pro Lys Thr Phe Ser Glu Val
1               5                   10                  15

Val Asn Gln Asp His Val Lys Lys Ala Ile Ile Gly Ala Ile Gln Lys
            20                  25                  30

Asn Ser Val Ala His Gly Tyr Ile Phe Ala Gly Pro Arg Gly Thr Gly
        35                  40                  45

Lys Thr Thr Leu Ala Arg Ile Leu Ala Lys Ser Leu Asn Cys Glu Asn
    50                  55                  60

Arg Lys Gly Val Glu Pro Cys Asn Ser Cys Arg Ala Cys Arg Glu Ile
65                  70                  75                  80

Asp Glu Gly Thr Phe Met Asp Val Ile Glu Leu Asp Ala Ala Ser Asn
                85                  90                  95

Arg Gly Ile Asp Glu Ile Arg Arg Ile Arg Asp Ala Val Gly Tyr Arg
            100                 105                 110

Pro Met Glu Gly Lys Tyr Lys Val Tyr Ile Ile Asp Glu Val His Met
        115                 120                 125

Leu Thr Lys Glu Ala Phe Asn Ala Leu Leu Lys Thr Leu Glu Glu Pro
    130                 135                 140

Pro Ser His Val Val Phe Val Leu Ala Thr Thr Asn Leu Glu Lys Val
145                 150                 155                 160

Pro Pro Thr Ile Ile Ser Arg Cys Gln Val Phe Glu Phe Arg Asn Ile
                165                 170                 175

Pro Asp Glu Leu Ile Glu Lys Arg Leu Gln Glu Val Ala Glu Ala Glu
            180                 185                 190

Gly Ile Glu Ile Asp Arg Glu Ala Leu Ser Phe Ile Ala Lys Arg Ala
        195                 200                 205

Ser Gly Gly Leu Arg Asp Ala Leu Thr Met Leu Glu Gln Val Trp Lys
    210                 215                 220

Phe Ser Glu Gly Lys Ile Asp Leu Glu Thr Val His Arg Ala Leu Gly
225                 230                 235                 240

Leu Ile Pro Ile Gln Val Val Arg Asp Tyr Val Asn Ala Ile Phe Ser
                245                 250                 255

Gly Asp Val Lys Arg Val Phe Thr Val Leu Asp Asp Val Tyr Tyr Ser
            260                 265                 270

Gly Lys Asp Tyr Glu Val Leu Ile Gln Glu Ala Val Glu Asp Leu Val
        275                 280                 285

Glu Asp Leu Glu Arg Glu Arg Gly Val Tyr Gln Val Ser Ala Asn Asp
    290                 295                 300

Ile Val Gln Val Ser Arg Gln Leu Leu Asn Leu Leu Arg Glu Ile Lys
305                 310                 315                 320

Phe Ala Glu Glu Lys Arg Leu Val Cys Lys Val Gly Ser Ala Tyr Ile
                325                 330                 335

Ala Thr Arg Phe Ser Thr Thr Asn Val Gln Glu Asn Asp Val Arg Glu

340                 345                 350

Lys Asn Asp Asn Ser Asn Val Gln Gln Lys Glu Glu Lys Lys Glu Thr
        355                 360                 365

Val Lys Ala Lys Glu Glu Lys Gln Glu Asp Ser Glu Phe Glu Lys Arg
    370                 375                 380

Phe Lys Glu Leu Met Glu Glu Leu Lys Glu Lys Gly Asp Leu Ser Ile
385                 390                 395                 400

Phe Val Ala Leu Ser Leu Ser Glu Val Gln Phe Asp Gly Glu Lys Val
                405                 410                 415

Ile Ile Ser Phe Asp Ser Ser Lys Ala Met His Tyr Glu Leu Met Lys
            420                 425                 430

Lys Lys Leu Pro Glu Leu Glu Asn Ile Phe Ser Arg Lys Leu Gly Lys
        435                 440                 445

Lys Val Glu Val Glu Leu Arg Leu Met Gly Lys Glu Glu Thr Ile Glu
    450                 455                 460

Lys Val Ser Gln Lys Ile Leu Arg Leu Phe Glu Gln Glu Gly
465                 470                 475


<210> 179
<211> 366
<212> PRT
<213> Thermotoga maritima

<400> 179
Met Lys Val Thr Val Thr Thr Leu Glu Leu Lys Asp Lys Ile Thr Ile
1               5                   10                  15

Ala Ser Lys Ala Leu Ala Lys Lys Ser Val Lys Pro Ile Leu Ala Gly
            20                  25                  30

Phe Leu Phe Glu Val Lys Asp Gly Asn Phe Tyr Ile Cys Ala Thr Asp
        35                  40                  45

Leu Glu Thr Gly Val Lys Ala Thr Val Asn Ala Ala Glu Ile Ser Gly
    50                  55                  60

Glu Ala Arg Phe Val Val Pro Gly Asp Val Ile Gln Lys Met Val Lys
65                  70                  75                  80

Val Leu Pro Asp Glu Ile Thr Glu Leu Ser Leu Glu Gly Asp Ala Leu
                85                  90                  95

Val Ile Ser Ser Gly Ser Thr Val Phe Arg Ile Thr Thr Met Pro Ala
            100                 105                 110

Asp Glu Phe Pro Glu Ile Thr Pro Ala Glu Ser Gly Ile Thr Phe Glu
        115                 120                 125

Val Asp Thr Ser Leu Leu Glu Glu Met Val Glu Lys Val Ile Phe Ala
    130                 135                 140

Ala Ala Lys Asp Glu Phe Met Arg Asn Leu Asn Gly Val Phe Trp Glu
145                 150                 155                 160

Leu His Lys Asn Leu Leu Arg Leu Val Ala Ser Asp Gly Phe Arg Leu
                165                 170                 175

Ala Leu Ala Glu Glu Gln Ile Glu Asn Glu Glu Glu Ala Ser Phe Leu
            180                 185                 190

Leu Ser Leu Lys Ser Met Lys Glu Val Gln Asn Val Leu Asp Asn Thr
        195                 200                 205

Thr Glu Pro Thr Ile Thr Val Arg Tyr Asp Gly Arg Arg Val Ser Leu
    210                 215                 220

Ser Thr Asn Asp Val Glu Thr Val Met Arg Val Val Asp Ala Glu Phe
225                 230                 235                 240

Pro Asp Tyr Lys Arg Val Ile Pro Glu Thr Phe Lys Thr Lys Val Val
                245                 250                 255

Val Ser Arg Lys Glu Leu Arg Glu Ser Leu Lys Arg Val Met Val Ile
            260                 265                 270

Ala Ser Lys Gly Ser Glu Ser Val Lys Phe Glu Ile Glu Glu Asn Val
        275                 280                 285

Met Arg Leu Val Ser Lys Ser Pro Asp Tyr Gly Glu Val Val Asp Glu
    290                 295                 300

Val Glu Val Gln Lys Glu Gly Glu Asp Leu Val Ile Ala Phe Asn Pro
305                 310                 315                 320

Lys Phe Ile Glu Asp Val Leu Lys His Ile Glu Thr Glu Glu Ile Glu
                325                 330                 335

Met Asn Phe Val Asp Ser Thr Ser Pro Cys Gln Ile Asn Pro Leu Asp
            340                 345                 350

Ile Ser Gly Tyr Leu Tyr Ile Val Met Pro Ile Arg Leu Ala
        355                 360                 365

<210> 180
<211> 189
<212> PRT
<213> Thermotoga maritima

<400> 180
Met Leu Ala Met Ile Trp Asn Asp Thr Val Phe Cys Val Val Asp Thr
1               5                   10                  15

Glu Thr Thr Gly Thr Asp Pro Phe Ala Gly Asp Arg Ile Val Glu Ile
            20                  25                  30

Ala Ala Val Pro Val Phe Lys Gly Lys Ile Tyr Arg Asn Lys Ala Phe
        35                  40                  45

His Ser Leu Val Asn Pro Arg Ile Arg Ile Pro Ala Leu Ile Gln Lys
    50                  55                  60

Val His Gly Ile Ser Asn Met Asp Ile Val Glu Ala Pro Asp Met Asp
65                  70                  75                  80

Thr Val Tyr Asp Leu Phe Arg Asp Tyr Val Lys Gly Thr Val Leu Val
                85                  90                  95

Phe His Asn Ala Asn Phe Asp Leu Thr Phe Leu Asp Met Met Ala Lys
            100                 105                 110

Glu Thr Gly Asn Phe Pro Ile Thr Asn Pro Tyr Ile Asp Thr Leu Asp
        115                 120                 125

Leu Ser Glu Glu Ile Phe Gly Arg Pro His Ser Leu Lys Trp Leu Ser
    130                 135                 140

Glu Arg Leu Gly Ile Lys Thr Thr Ile Arg His Arg Ala Leu Pro Asp
145                 150                 155                 160

Ala Leu Val Thr Ala Arg Val Phe Val Lys Leu Val Glu Phe Leu Gly
                165                 170                 175

Glu Asn Arg Val Asn Glu Phe Ile Arg Gly Lys Arg Gly
            180                 185


<210> 181
<211> 1367
<212> PRT
<213> Thermotoga maritima

<400> 181
Met Lys Lys Ile Glu Asn Leu Lys Trp Lys Asn Val Ser Phe Lys Ser
  1               5                  10                  15

Leu Glu Ile Asp Pro Asp Ala Gly Val Val Leu Val Ser Val Glu Lys
             20                  25                  30

Phe Ser Glu Glu Ile Glu Asp Leu Val Arg Leu Leu Glu Lys Lys Thr
         35                  40                  45

Arg Phe Arg Val Ile Val Asn Gly Val Gln Lys Ser Asn Gly Asp Leu
     50                  55                  60

Arg Gly Lys Ile Leu Ser Leu Leu Asn Gly Asn Val Pro Tyr Ile Lys
 65                  70                  75                  80

Asp Val Val Phe Glu Gly Asn Arg Leu Ile Leu Lys Val Leu Gly Asp
                 85                  90                  95

Phe Ala Arg Asp Arg Ile Ala Ser Lys Leu Arg Ser Thr Lys Lys Gln
            100                 105                 110

Leu Asp Glu Leu Leu Pro Pro Gly Thr Glu Ile Met Leu Glu Val Val
        115                 120                 125

Glu Pro Pro Glu Asp Leu Leu Lys Lys Glu Val Pro Gln Pro Glu Lys
    130                 135                 140

Arg Glu Glu Pro Lys Gly Glu Glu Leu Lys Ile Glu Asp Glu Asn His
145                 150                 155                 160

Ile Phe Gly Gln Lys Pro Arg Lys Ile Val Phe Thr Pro Ser Lys Ile
                165                 170                 175

Phe Glu Tyr Asn Lys Lys Thr Ser Val Lys Gly Lys Ile Phe Lys Ile
            180                 185                 190

Glu Lys Ile Glu Gly Lys Arg Thr Val Leu Leu Ile Tyr Leu Thr Asp
        195                 200                 205

Gly Glu Asp Ser Leu Ile Cys Lys Val Phe Asn Asp Val Glu Lys Val
    210                 215                 220

Glu Gly Lys Val Ser Val Gly Asp Val Ile Val Ala Thr Gly Asp Leu
225                 230                 235                 240

Leu Leu Glu Asn Gly Glu Pro Thr Leu Tyr Val Lys Gly Ile Thr Lys
                245                 250                 255

Leu Pro Glu Ala Lys Arg Met Asp Lys Ser Pro Val Lys Arg Val Glu
            260                 265                 270

Leu His Ala His Thr Lys Phe Ser Asp Gln Asp Ala Ile Thr Asp Val

        275                 280                 285

Asn Glu Tyr Val Lys Arg Ala Lys Glu Trp Gly Phe Pro Ala Ile Ala
    290                 295                 300

Leu Thr Asp His Gly Asn Val Gln Ala Ile Pro Tyr Phe Tyr Asp Ala
305                 310                 315                 320

Ala Lys Glu Ala Gly Ile Lys Pro Ile Phe Gly Ile Glu Ala Tyr Leu
                325                 330                 335

Val Ser Asp Val Glu Pro Val Ile Arg Asn Leu Ser Asp Asp Ser Thr
            340                 345                 350

Phe Gly Asp Ala Thr Phe Val Val Leu Asp Phe Glu Thr Thr Gly Leu
        355                 360                 365

Asp Pro Gln Val Asp Glu Ile Ile Glu Ile Gly Ala Val Lys Ile Gln
    370                 375                 380

Gly Gly Gln Ile Val Asp Glu Tyr His Thr Leu Ile Lys Pro Ser Arg
385                 390                 395                 400

Glu Ile Ser Arg Lys Ser Ser Glu Ile Thr Gly Ile Thr Gln Glu Met
                405                 410                 415

Leu Glu Asn Lys Arg Ser Ile Glu Glu Val Leu Pro Glu Phe Leu Gly
            420                 425                 430

Phe Leu Glu Asp Ser Ile Ile Val Ala His Asn Ala Asn Phe Asp Tyr
        435                 440                 445

Arg Phe Leu Arg Leu Trp Ile Lys Lys Val Met Gly Leu Asp Trp Glu
    450                 455                 460

Arg Pro Tyr Ile Asp Thr Leu Ala Leu Ala Lys Ser Leu Leu Lys Leu
465                 470                 475                 480

Arg Ser Tyr Ser Leu Asp Ser Val Val Glu Lys Leu Gly Leu Gly Pro
                485                 490                 495

Phe Arg His His Arg Ala Leu Asp Asp Ala Arg Val Thr Ala Gln Val
            500                 505                 510

Phe Leu Arg Phe Val Glu Met Met Lys Lys Ile Gly Ile Thr Lys Leu
        515                 520                 525

Ser Glu Met Glu Lys Leu Lys Asp Thr Ile Asp Tyr Thr Ala Leu Lys
    530                 535                 540

Pro Phe His Cys Thr Ile Leu Val Gln Asn Lys Lys Gly Leu Lys Asn
545                 550                 555                 560

Leu Tyr Lys Leu Val Ser Asp Ser Tyr Ile Lys Tyr Phe Tyr Gly Val
                565                 570                 575

Pro Arg Ile Leu Lys Ser Glu Leu Ile Glu Asn Arg Glu Gly Leu Leu
            580                 585                 590

Val Gly Ser Ala Cys Ile Ser Gly Glu Leu Gly Arg Ala Ala Leu Glu
        595                 600                 605

Gly Ala Ser Asp Ser Glu Leu Glu Glu Ile Ala Lys Phe Tyr Asp Tyr
    610                 615                 620

Ile Glu Val Met Pro Leu Asp Val Ile Ala Glu Asp Glu Glu Asp Leu
625                 630                 635                 640

Asp Arg Glu Arg Leu Lys Glu Val Tyr Arg Lys Leu Tyr Arg Ile Ala
                645                 650                 655

Lys Lys Leu Asn Lys Phe Val Val Met Thr Gly Asp Val His Phe Leu
            660                 665                 670

Asp Pro Glu Asp Ala Arg Gly Arg Ala Ala Leu Leu Ala Pro Gln Gly
        675                 680                 685

Asn Arg Asn Phe Glu Asn Gln Pro Ala Leu Tyr Leu Arg Thr Thr Glu
    690                 695                 700

Glu Met Leu Glu Lys Ala Ile Glu Ile Phe Glu Asp Glu Glu Ile Ala
705                 710                 715                 720

Arg Glu Val Val Ile Glu Asn Pro Asn Arg Ile Ala Asp Met Ile Glu
                725                 730                 735

Glu Val Gln Pro Leu Glu Lys Lys Leu His Pro Pro Ile Ile Glu Asn
            740                 745                 750

Ala Asp Glu Ile Val Arg Asn Leu Thr Met Lys Arg Ala Tyr Glu Ile
        755                 760                 765

Tyr Gly Asp Pro Leu Pro Glu Ile Val Gln Lys Arg Val Glu Lys Glu
    770                 775                 780

Leu Asn Ala Ile Ile Asn His Gly Tyr Ala Val Leu Tyr Leu Ile Ala
785                 790                 795                 800

Gln Glu Leu Val Gln Lys Ser Met Ser Asp Gly Tyr Val Val Gly Ser
                805                 810                 815

Arg Gly Ser Val Gly Ser Ser Leu Val Ala Asn Leu Leu Gly Ile Thr
            820                 825                 830

Glu Val Asn Pro Leu Pro Pro His Tyr Arg Cys Pro Glu Cys Lys Tyr
        835                 840                 845

Phe Glu Val Val Glu Asp Asp Arg Tyr Gly Ala Gly Tyr Asp Leu Pro
    850                 855                 860

Asn Lys Asn Cys Pro Arg Cys Gly Ala Pro Leu Arg Lys Asp Gly His
865                 870                 875                 880

Gly Ile Pro Phe Glu Thr Phe Met Gly Phe Glu Gly Asp Lys Val Pro
                885                 890                 895

Asp Ile Asp Leu Asn Phe Ser Gly Glu Tyr Gln Glu Arg Ala His Arg
            900                 905                 910

Phe Val Glu Glu Leu Phe Gly Lys Asp His Val Tyr Arg Ala Gly Thr
        915                 920                 925

Ile Asn Thr Ile Ala Glu Arg Ser Ala Val Gly Tyr Val Arg Ser Tyr
    930                 935                 940

Glu Glu Lys Thr Gly Lys Lys Leu Arg Lys Ala Glu Met Glu Arg Leu
945                 950                 955                 960

Val Ser Met Ile Thr Gly Val Lys Arg Thr Thr Gly Gln His Pro Gly
                965                 970                 975

Gly Leu Met Ile Ile Pro Lys Asp Lys Glu Val Tyr Asp Phe Thr Pro
            980                 985                 990

Ile Gln Tyr Pro Ala Asn Asp Arg Asn Ala Gly Val Phe Thr Thr His
        995                 1000                1005

Phe Ala Tyr Glu Thr Ile His Asp Asp Leu Val Lys Ile Asp Ala Leu
    1010                1015                1020

Gly His Asp Asp Pro Thr Phe Ile Lys Met Leu Lys Asp Leu Thr Gly
1025                1030                1035                1040

Ile Asp Pro Met Thr Ile Pro Met Asp Asp Pro Asp Thr Leu Ala Ile
                1045                1050                1055

Phe Ser Ser Val Lys Pro Leu Gly Val Asp Pro Val Glu Leu Glu Ser
            1060                1065                1070

Asp Val Gly Thr Tyr Gly Ile Pro Glu Phe Gly Thr Glu Phe Val Arg
        1075                1080                1085

Gly Met Leu Val Glu Thr Arg Pro Lys Ser Phe Ala Glu Leu Val Arg
    1090                1095                1100

Ile Ser Gly Leu Ser His Gly Thr Asp Val Trp Leu Asn Asn Ala Arg
1105                1110                1115                1120

Asp Trp Ile Asn Leu Gly Tyr Ala Lys Leu Ser Glu Val Ile Ser Cys
                1125                1130                1135

Arg Asp Asp Ile Met Asn Phe Leu Ile His Lys Gly Met Glu Pro Ser
            1140                1145                1150

Leu Ala Phe Lys Ile Met Glu Asn Val Arg Lys Gly Lys Gly Ile Thr
        1155                1160                1165

Glu Glu Met Glu Ser Glu Met Arg Arg Leu Lys Val Pro Glu Trp Phe
    1170                1175                1180

Ile Glu Ser Cys Lys Arg Ile Lys Tyr Leu Phe Pro Lys Ala His Ala
1185                1190                1195                1200

Val Ala Tyr Val Ser Met Ala Phe Arg Ile Ala Tyr Phe Lys Val His
                1205                1210                1215

Tyr Pro Leu Gln Phe Tyr Ala Ala Tyr Phe Thr Ile Lys Gly Asp Gln
            1220                1225                1230

Phe Asp Pro Val Leu Val Leu Arg Gly Lys Glu Ala Ile Lys Arg Arg
        1235                1240                1245

Leu Arg Glu Leu Lys Ala Met Pro Ala Lys Asp Ala Gln Lys Lys Asn
    1250                1255                1260

Glu Val Ser Val Leu Glu Val Ala Leu Glu Met Ile Leu Arg Gly Phe
1265                1270                1275                1280

Ser Phe Leu Pro Pro Asp Ile Phe Lys Ser Asp Ala Lys Lys Phe Leu
                1285                1290                1295

Ile Glu Gly Asn Ser Leu Arg Ile Pro Phe Asn Lys Leu Pro Gly Leu
            1300                1305                1310

Gly Asp Ser Val Ala Glu Ser Ile Ile Arg Ala Arg Glu Glu Lys Pro
        1315                1320                1325

Phe Thr Ser Val Glu Asp Leu Met Lys Arg Thr Lys Val Asn Lys Asn
    1330                1335                1340

His Ile Glu Leu Met Lys Ser Leu Gly Val Leu Gly Asp Leu Pro Glu
1345                1350                1355                1360

Thr Glu Gln Phe Thr Leu Phe

                1365

<210> 182
<211> 842
<212> PRT
<213> Thermotoga maritima

<400> 182
Met Ile Pro Trp Val Ile Ser Pro Tyr Ser Phe Asp Gly Ser Val Val
1               5                   10                  15

Arg Phe Glu Lys Leu Ala Leu Leu Leu Lys Arg Lys Gly Leu Lys Ser
            20                  25                  30

Val Ile Leu Ala Asp Arg Asn Phe His Ala Ala Val Lys Phe Asn Thr
        35                  40                  45

Ile Met Arg Lys His Gly Leu Ile Pro Val His Gly Leu Trp Lys Asp
    50                  55                  60

Gly Arg Ile Phe Val Ala Arg Asn Arg Glu Glu Phe Asp Ser Leu Val
65                  70                  75                  80

Arg Tyr Tyr Asn Gly Glu Thr His Glu Ile Glu Asp Ile Pro Val Phe
                85                  90                  95

Gln Glu Ser Glu Leu Thr Pro Val Arg Tyr Leu Asp Ala Ser Glu Lys
            100                 105                 110

Lys Ala Ser Ile Phe Met Arg Lys Ile Phe Gly Leu Asp Glu Asp Val
        115                 120                 125

Gln Gly Phe Pro Glu Lys Cys Glu Asp Val Ala Asp Ile Leu Asn Ala
    130                 135                 140

Glu Ala Tyr Asp Leu Arg Val Asn His Arg Phe Pro Thr Pro Pro Lys
145                 150                 155                 160

Asn Trp Asn Glu Leu Leu Ile Lys Lys Ala Glu Pro Leu Gly Glu Glu
                165                 170                 175

Tyr Ile Ser Arg Leu Lys Arg Glu Leu Glu Val Ile Lys Arg Lys Gly
            180                 185                 190

Phe Thr Pro Tyr Ile Tyr Thr Val Glu Lys Val Val Glu Ile Ala Lys
        195                 200                 205

Lys Met Gly Ile Lys Val Gly Pro Gly Arg Gly Ser Ala Val Gly Ser
    210                 215                 220

Leu Val Ala Tyr Leu Cys Gly Ile Thr Glu Val Asp Pro Ile Lys Tyr
225                 230                 235                 240

Asp Leu Leu Phe Glu Arg Phe Leu Asn Glu Glu Arg Gln Glu Pro Pro
                245                 250                 255

Asp Ile Asp Val Asp Val Glu Asp Arg Arg Arg Lys Asp Leu Ile Lys
            260                 265                 270

Glu Leu Ser Lys Ser Phe Gln Val Tyr Gln Val Ser Thr Phe Gly Asn
        275                 280                 285

Leu Thr Glu Lys Ser Leu Lys Asn Leu Ile Asn Ser Val Leu Pro Asp
    290                 295                 300

Ala Ser Leu Glu Glu Lys Asn Glu Ile Tyr Lys Thr Val Tyr Gly Leu
305                 310                 315                 320

Pro His His Pro Ser Val His Ala Ala Gly Val Val Ile Ser Glu Asn
                325                 330                 335

Pro Leu Pro Leu Pro Thr Arg Thr Glu Glu Asp Ile Pro Ile Thr Asp
            340                 345                 350

Tyr Asp Met Tyr Asp Leu Gln Glu Ile Gly Val Val Lys Ile Asp Ile
        355                 360                 365

Leu Gly Leu Lys Thr Leu Ser Phe Ile Lys Asp Phe Lys Lys Glu Ile
    370                 375                 380

Phe Asp Tyr Ser Asp Glu Lys Thr Tyr His Leu Ile Ser Lys Gly Lys
385                 390                 395                 400

Thr Leu Gly Val Phe Gln Leu Glu Gly Leu Gln Ala Arg Lys Leu Cys
                405                 410                 415

Arg Arg Ile Ser Pro Arg Asn Met Asp Glu Leu Ser Ile Leu Leu Ala
            420                 425                 430

Leu Asn Arg Pro Gly Pro Leu Arg Ser Gly Leu Asp Val Met Phe Ser
        435                 440                 445

Asn Ser Lys Asn Val Pro Ala Phe Phe Arg Lys Met Phe Pro Glu Thr
    450                 455                 460

Arg Gly Val Leu Ile Tyr Gln Glu Gln Ile Met Arg Leu Ala Met Phe
465                 470                 475                 480

Ala Gly Leu Ser Gly Thr Glu Ala Asp Ile Leu Arg Arg Ala Ile Ala
                485                 490                 495

Lys Lys Glu Arg Glu Lys Met Glu Pro Leu Leu Glu Lys Met Lys Lys
            500                 505                 510

Gly Leu Leu Glu Lys Gly Met Glu Asn Ala Glu Gln Ile Leu Glu Ile
        515                 520                 525

Leu Leu Asn Phe Ser Ser Tyr Ala Phe Asn Lys Ser His Ser Val Ala
    530                 535                 540

Tyr Ala His Ile Thr Tyr Gln Thr Ala Tyr Leu Lys Ala His His Leu
545                 550                 555                 560

Glu Glu Phe Phe Lys Leu Tyr Phe Ala Tyr Asn Ser Ser Asp Ala Gly
                565                 570                 575

Lys Ile Phe Leu Ala Val Gln Glu Leu Arg Asn Glu Gly Tyr Arg Val
            580                 585                 590

His Pro Pro Asp Ile Asn Ile Ser Gly Lys Asp Leu Val Phe His Gly
        595                 600                 605

Lys Asp Val Tyr Leu Pro Leu Thr Val Val Lys Gly Val Gly Val Thr
    610                 615                 620

Leu Val Glu Gln Ile Glu Lys Ile Arg Pro Val Ser Ser Val Arg Glu
625                 630                 635                 640

Leu Gln Glu Arg Val Thr Gly Val Pro Arg Asn Val Val Glu Ser Leu
                645                 650                 655

Ile Thr Ala Gly Ala Phe Asp Lys Leu Tyr Glu Asn Arg Lys Leu Ala
            660                 665                 670

Leu Glu Glu Leu Asn Lys Arg Val Glu Lys Asp Ile Leu Glu Ile Arg

        675                 680                 685

Ser Leu Phe Gly Glu Lys Val Glu Gln Glu Ser Ser Asn Ile Lys Ile
    690                 695                 700

Gly Asp Ile Thr Glu Leu Glu Glu Lys Ser Met Gly Phe Pro Leu Thr
705                 710                 715                 720

Pro Val His Glu Val Pro Thr Gly Leu Phe Ala Arg Ile Asp Asp Val
                725                 730                 735

Phe Thr Tyr Gly Arg Ile Leu Pro Val Leu Val Lys Arg Val Ser Arg
            740                 745                 750

Asn Ile Val Thr Asp Gly Leu Ser Val Cys Arg Val Arg Thr Asp Val
        755                 760                 765

Pro Asp Gly Val His Leu Val Leu Leu Ser Pro Leu Gln Lys Ile Ile
    770                 775                 780

Lys Ile Trp Pro Phe Asn Glu Asn Thr Arg Phe Val Tyr Arg Val Asp
785                 790                 795                 800

Phe Thr Ala Thr Leu Glu Lys Ala Gly Gln Asn Glu Ile Thr Glu Val
                805                 810                 815

Leu Lys Asn Gly Ala Val Val Arg Tyr Glu Gly Tyr Arg Pro Leu Thr
            820                 825                 830

Asp Glu Tyr Arg Tyr Arg Val Val Pro Arg
        835                 840

<210> 183
<211> 141
<212> PRT
<213> Thermotoga maritima

<400> 183
Met Ser Phe Phe Asn Lys Ile Ile Leu Ile Gly Arg Leu Val Arg Asp
1               5                   10                  15

Pro Glu Glu Arg Tyr Thr Leu Ser Gly Thr Pro Val Thr Thr Phe Thr
            20                  25                  30

Ile Ala Val Asp Arg Val Pro Arg Lys Asn Ala Pro Asp Asp Ala Gln
        35                  40                  45

Thr Thr Asp Phe Phe Arg Ile Val Thr Phe Gly Arg Leu Ala Glu Phe
    50                  55                  60

Ala Arg Thr Tyr Leu Thr Lys Gly Arg Leu Val Leu Val Glu Gly Glu
65                  70                  75                  80

Met Arg Met Arg Arg Trp Glu Thr Pro Thr Gly Glu Lys Arg Val Ser
                85                  90                  95

Pro Glu Val Val Ala Asn Val Val Arg Phe Met Asp Arg Lys Pro Ala
            100                 105                 110

Glu Thr Val Ser Glu Thr Glu Glu Glu Leu Glu Ile Pro Glu Glu Asp
        115                 120                 125

Phe Ser Ser Asp Thr Phe Ser Glu Asp Glu Pro Pro Phe
    130                 135                 140

<210> 184

<211> 250
<212> DNA
<213> Artificial Sequence

<220>
<223> Description of Artificial Sequence: PCR product

<400> 184
gaattcggat cccgggtcta gaggaggtta attaacatat gaaaaaaaaa accaggttgc 60

tagcggtacc actagtggtg gtggtggtct ggttccgcgt ggttcccacc accaccacct 120

gcaccacatc ctggacgctc agaaaatggt ttggaaccac tgccgttgat gagcagttag 180

agaaagaacc catagtagga gcagaaactt tctaggtcga cagcttggct gttttggcgg 240

atgagagaag 250


<210> 185
<211> 250
<212> DNA
<213> Artificial Sequence

<220>
<223> Description of Artificial Sequence: PCR product

<220>
<221> misc_feature
<222> ((1)..(250))
<223> /note=complement

<400> 185
cttctctcat ccgccaaaac agccaagctg tcgacctaga aagtttctgc tcctactatg 60

ggttctttct ctaactgctc atcaacggca gtggttccaa accattttct gagcgtccag 120

gatgtggtgc aggtggtggt ggtgggaacc acgcggaacc agaccaccac caccactagt 180

ggtaccgcta gcaacctggt tttttttttc atatgttaat taacctcctc tagacccggg 240

atccgaattc 250


<210> 186
<211> 200
<212> DNA
<213> Artificial Sequence

<220>
<223> Description of Artificial Sequence: PCR product

<400> 186
gaattcggat cccgggtcta gaggaggtaa taacatatgg ctggttgcct gaacgacatc 60

ttcgaagctc agaaaatcga atggcaccat caccatcacc atctggttcc gcgtggttcc 120

ggcggcggcg gcctgcaggt acccaaaaat gcatgagctc gctagcaagc ttaaaaaaaa 180

aactagtgtc gacagcttgg 200


<210> 187
<211> 200
<212> DNA
<213> Artificial Sequence

<220>

<223> Description of Artificial Sequence: PCR product

<220>
<221> misc_feature
<222> ((1)..(200))
<223> /note=complement

<400> 187
ccaagctgtc gacactagtt ttttttttaa gcttgctagc gagctcatgc atttttgggt 60

acctgcaggc cgccgccgcc ggaaccacgc ggaaccagat ggtgatggtg atggtgccat 120

tcgattttct gagcttcgaa gatgtcgttc aggcaaccag ccatatgtta ttacctcctc 180

tagacccggg atccgaattc 200

<210> 188
<211> 200
<212> DNA
<213> Artificial Sequence

<220>
<223> Description of Artificial Sequence: PCR product

<400> 188
gaattcggat cccgggtcta gaggaggtaa taacatatgg ctggttgcct gaacgacatc 60

ttcgaagctc agaaaatcga atggcaccat caccatcacc atctggttcc gcgtggttcc 120

ggcggcggcg gcctgcagaa cctaggaaaa aaaaaggtac caaaaaaaaa ggccggccac 180

tagtgtcgac agcttggctg 200

<210> 189
<211> 200
<212> DNA
<213> Artificial Sequence

<220>
<223> Description of Artificial Sequence: PCR product

<220>
<221> misc_feature
<222> ((1)..(200))
<223> /note=complement

<400> 189
cagccaagct gtcgacacta gtggccggcc tttttttttg gtaccttttt ttttcctagg 60

ttctgcaggc cgccgccgcc ggaaccacgc ggaaccagat ggtgatggtg atggtgccat 120

tcgattttct gagcttcgaa gatgtcgttc aggcaaccag ccatatgtta ttacctcctc 180

tagacccggg atccgaattc 200

<210> 190
<211> 250
<212> DNA
<213> Artificial Sequence

<220>
<223> Description of Artificial Sequence: PCR product

<400> 190
acacagaatt cggatcccgg gtctagagga ggttaattaa ccatggaaaa aaaaaggtac 60

caaaaaaaaa ggccggccac tagtggtggt ggtggtctgg ttccgcgtgg ttcccaccac 120

caccacctgc accacatcct ggacgctcag aaaatggttt ggaaccactg ccgttgatga 180

gcagttagag aaagaaccca tagtaggagc agaaactttc taggtcgaca gcttggctgt 240

tttggcggat 250

<210> 191
<211> 250
<212> DNA
<213> Artificial Sequence

<220>
<223> Description of Artificial Sequence: PCR product

<220>
<221> misc_feature
<222> ((1)..(250))
<223> /note=complement

<400> 191
atccgccaaa acagccaagc tgtcgaccta gaaagtttct gctcctacta tgggttcttt 60

ctctaactgc tcatcaacgg cagtggttcc aaaccatttt ctgagcgtcc aggatgtggt 120

gcaggtggtg gtggtgggaa ccacgcggaa ccagaccacc accaccacta gtggccggcc 180

tttttttttg gtaccttttt ttttccatgg ttaattaacc tcctctagac ccgggatccg 240

aattctgtgt 250

<210> 192
<211> 150
<212> DNA
<213> Artificial Sequence

<220>
<223> Description of Artificial Sequence: PCR product

<400> 192
acacagaatt cggatcccgg gtctagagga ggttaattaa ccatgagtat ttcagaaaaa 60

cccttgagtg aactgctcag gccgaaagac ttcgaggatt tcgtcggtca ggatcatata 120

ttcggtaata aagggattct ccggcgaact 150

<210> 193
<211> 150
<212> DNA
<213> Artificial Sequence

<220>
<223> Description of Artificial Sequence: PCR product

<220>
<221> misc_feature
<222> ((1)..(150))
<223> /note=complement

<400> 193
agttcgccgg agaatccctt tattaccgaa tatatgatcc tgaccgacga aatcctcgaa 60

gtctttcggc ctgagcagtt cactcaaggg tttttctgaa atactcatgg ttaattaacc 120

tcctctagac ccgggatccg aattctgtgt                                    150

<210> 194
<211> 22
<212> PRT
<213> Artificial Sequence

<220>
<223> Description of Artificial Sequence: Consensus
      sequence

<220>
<221> UNSURE
<222> (1)
<223> Xaa at position 1 is Leu, Ile or Val

<220>
<221> UNSURE
<222> (2)..(3)
<223> Xaa at position 2-3 is any amino acid

<220>
<221> UNSURE
<222> (4)
<223> Xaa at position 4 is Leu, Ile or Val

<220>
<221> UNSURE
<222> (6)
<223> Xaa at position 6 is Leu, Ile or Val

<220>
<221> UNSURE
<222> (7)
<223> Xaa at position 7 is Leu, Ile or Val

<220>
<221> UNSURE
<222> (8)
<223> Xaa at position 8 is any amino acid

<220>
<221> UNSURE
<222> (10)
<223> Xaa at position 10 is any amino acid

<220>
<221> UNSURE
<222> (12)..(13)
<223> Xaa at position 12-13 is any amino acid

<220>
<221> UNSURE
<222> (14)
<223> Xaa at position 14 is Leu, Ile or Val

<220>
<221> UNSURE
<222> (15)
<223> Xaa at position 15 is Leu, Ile or Val

<220>
<221> UNSURE
<222> (16)..(21)
<223> Xaa at position 16-21 is any amino acid

<220>
<221> UNSURE
<222> (22)
<223> Xaa at position 22 is Leu, Ile or Val

<220>
<221> UNSURE
<222> (11)
<223> Xaa at position 11 is Asp or Glu

<400> 194
Xaa Xaa Xaa Xaa Tyr Xaa Xaa Xaa Gly Xaa Xaa Xaa Xaa Xaa Xaa Xaa
1               5                   10                  15

Xaa Xaa Xaa Xaa Xaa Xaa
            20


<210> 195
<211> 19
<212> PRT
<213> Artificial Sequence

<220>
<223> Description of Artificial Sequence: Consensus
      sequence

<220>
<221> UNSURE
<222> (1)
<223> Xaa at position 1 is Leu, Ile or Val

<220>
<221> UNSURE
<222> (2)
<223> Xaa at position 2 is Leu, Ile or Val

<220>
<221> UNSURE
<222> (3)..(6)
<223> Xaa at position 3-6 is any amino acid

<220>
<221> UNSURE
<222> (8)..(9)
<223> Xaa at position 8-9 is any amino acid

<220>
<221> UNSURE
<222> (10)
<223> Xaa at position 10 is Leu, Ile or Val

<220>
<221> UNSURE
<222> (13)
<223> Xaa at position 13 is any amino acid

<220>
<221> UNSURE
<222> (15)
<223> Xaa at position 15 is any amino acid

<220>
<221> UNSURE
<222> (16)..(19)
<223> Xaa at position 16-19 is Leu, Ile or Val

<220>

<221> UNSURE
<222> (7)
<223> Xaa at position 7 is Ala or Ser

<220>
<221> UNSURE
<222> (12)
<223> Xaa at position 12 is Ala or Ser

<220>
<221> UNSURE
<222> (14)
<223> Xaa at position 14 is Lys or Arg

<400> 195
Xaa Xaa Xaa Xaa Xaa Xaa Xaa Xaa Xaa Xaa Phe Xaa Xaa Xaa Xaa Xaa
1               5                   10                  15

Xaa Xaa Xaa



<210> 196
<211> 21
<212> PRT
<213> Artificial Sequence

<220>
<223> Description of Artificial Sequence: Consensus
      sequence

<220>
<221> UNSURE
<222> (1)
<223> Xaa at position 1 is Leu, Ile or Val

<220>
<221> UNSURE
<222> (2)..(3)
<223> Xaa at position 2-3 is any amino acid

<220>
<221> UNSURE
<222> (4)..(5)
<223> Xaa at position 4-5 is Leu, Ile or Val

<220>
<221> UNSURE
<222> (6)..(10)
<223> Xaa at position 6-10 is any amino acid

<220>
<221> UNSURE
<222> (12)
<223> Xaa at position 12 is any amino acid

<220>
<221> UNSURE
<222> (13)..(16)
<223> Xaa at position 13-16 is Leu, Ile or Val

<220>
<221> UNSURE
<222> (17)..(19)
<223> Xaa at position 17-19 is any amino acid

<220>
<221> UNSURE

<222> (21)
<223> Xaa at position 21 is Leu, Ile or Val

<220>
<221> UNSURE
<222> (11)
<223> Xaa at position 11 is Asp or Glu

<400> 196
Xaa Xaa Xaa Xaa Xaa Xaa Xaa Xaa Xaa Xaa Xaa Xaa Xaa Xaa Xaa Xaa
1               5                   10                  15

Xaa Xaa Xaa Lys Xaa
            20


<210> 197
<211> 38
<212> PRT
<213> Artificial Sequence

<220>
<223> Description of Artificial Sequence: Consensus
      sequence

<220>
<221> UNSURE
<222> (1)
<223> Xaa at position 1 is Arg or Lys

<220>
<221> UNSURE
<222> (2)..(4)
<223> Xaa at position 2-4 is any amino acid

<220>
<221> UNSURE
<222> (34)
<223> Xaa at position 34 is Arg or Lys

<220>
<221> UNSURE
<222> (5)
<223> Xaa at position 5 is Leu, Ile or Val

<220>
<221> UNSURE
<222> (6)
<223> Xaa at position 6 is any amino acid

<220>
<221> UNSURE
<222> (7)
<223> Xaa at position 7 is Leu, Ile or Val

<220>
<221> UNSURE
<222> (8)
<223> Xaa at position 8 is any amino acid

<220>
<221> UNSURE
<222> (9)
<223> Xaa at position 9 is Leu, Ile or Val

<220>
<221> UNSURE
<222> (11)

<223> Xaa at position 11 is any amino acid

<220>
<221> UNSURE
<222> (13)
<223> Xaa at position 13 is any amino acid

<220>
<221> UNSURE
<222> (14)
<223> Xaa at position 14 is Leu, Ile or Val

<220>
<221> UNSURE
<222> (15)..(16)
<223> Xaa at position 15-16 is any amino acid

<220>
<221> UNSURE
<222> (17)
<223> Xaa at position 17 is Leu, Ile or Val

<220>
<221> UNSURE
<222> (18)..(23)
<223> Xaa at position 18-23 is any amino acid

<220>
<221> UNSURE
<222> (25)
<223> Xaa at position 25 is Leu, Ile or Val

<220>
<221> UNSURE
<222> (26)..(27)
<223> Xaa at position 26-27 is any amino acid

<220>
<221> UNSURE
<222> (28)
<223> Xaa at position 28 Leu, Ile or Val

<220>
<221> UNSURE
<222> (29)..(30)
<223> Xaa at position 29-30 is any amino acid

<220>
<221> UNSURE
<222> (32)
<223> Xaa at position 32 is Leu, Ile or Val

<220>
<221> UNSURE
<222> (33)
<223> Xaa at position 33 is any amino acid

<220>
<221> UNSURE
<222> (35)
<223> Xaa at position 35 is Leu, Ile or Val

<220>
<221> UNSURE
<222> (36)
<223> Xaa at position 36 is any amino acid

<220>

<221> UNSURE
<222> (37)..(38)
<223> Xaa at position 37-38 is Leu, Ile or Val

<220>
<221> UNSURE
<222> (10)
<223> Xaa at position 10 is Asp or Glu

<220>
<221> UNSURE
<222> (12)
<223> Xaa at position 12 is Asp or Glu

<220>
<221> UNSURE
<222> (31)
<223> Xaa at position 31 is Asp or Glu

<400> 197
Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Asn Xaa Xaa Xaa Xaa Xaa Xaa Xaa Xaa
            20                  25                  30

Xaa Xaa Xaa Xaa Xaa Xaa
        35


<210> 198
<211> 46
<212> PRT
<213> Artificial Sequence

<220>
<223> Description of Artificial Sequence: Consensus
      sequence

<220>
<221> UNSURE
<222> (2)
<223> Xaa at position 2 is any amino acid

<220>
<221> UNSURE
<222> (3)
<223> Xaa at position 3 is Leu, Ile or Val

<220>
<221> UNSURE
<222> (4)
<223> Xaa at position 4 is any amino acid

<220>
<221> UNSURE
<222> (7)..(8)
<223> Xaa at position 7-8 is Leu, Ile or Val

<220>
<221> UNSURE
<222> (9)
<223> Xaa at position 9 is any amino acid

<220>
<221> UNSURE
<222> (12)..(14)
<223> Xaa at position 12-14 is any amino acid

<220>
<221> UNSURE
<222> (16)
<223> Xaa at position 16 is Leu, Ile or Val

<220>
<221> UNSURE
<222> (17)
<223> Xaa at position 17 is any amino acid

<220>
<221> UNSURE
<222> (18)..(19)
<223> Xaa at position 18-19 is Leu, Ile or Val

<220>
<221> UNSURE
<222> (20)..(21)
<223> Xaa at position 20-21 is any amino acid

<220>
<221> UNSURE
<222> (22)
<223> Xaa at position 22 is Leu, Ile or Val

<220>
<221> UNSURE
<222> (23)..(25)
<223> Xaa at position 23-25 is any amino acid

<220>
<221> UNSURE
<222> (27)
<223> Xaa at position 27 is any amino acid

<220>
<221> UNSURE
<222> (30)
<223> Xaa at position 30 is Leu, Ile or Val

<220>
<221> UNSURE
<222> (31)
<223> Xaa at position 31 is any amino acid

<220>
<221> UNSURE
<222> (32)..(33)
<223> Xaa at position 32-33 is Leu, Ile or Val

<220>
<221> UNSURE
<222> (34)..(35)
<223> Xaa at position 34-35 is any amino acid

<220>
<221> UNSURE
<222> (36)
<223> Xaa at position 36 is Leu, Ile or Val

<220>
<221> UNSURE
<222> (37)..(39)
<223> Xaa at position 37-39 is any amino acid

<220>
<221> UNSURE

<222> (40)
<223> Xaa at position 40 is Leu, Ile or Val

<220>
<221> UNSURE
<222> (41)..(42)
<223> Xaa at position 41-42 is any amino acid

<220>
<221> UNSURE
<222> (43)
<223> Xaa at position 43 is Leu, Ile or Val

<220>
<221> UNSURE
<222> (44)..(45)
<223> Xaa at position 44-45 is any amino acid

<220>
<221> UNSURE
<222> (46)
<223> Xaa at position 46 is Leu, Ile or Val

<220>
<221> UNSURE
<222> (5)
<223> Xaa at position 5 is Asp or Glu

<220>
<221> UNSURE
<222> (28)
<223> Xaa at position 28 is Asp or Glu

<220>
<221> UNSURE
<222> (6)
<223> Xaa at position 6 is Ala or Ser

<220>
<221> UNSURE
<222> (15)
<223> Xaa at position 15 is Ala or Ser

<220>
<221> UNSURE
<222> (11)
<223> Xaa at position 11 is Arg or Lys

<400> 198
Phe Xaa Xaa Xaa Xaa Xaa Xaa Xaa Xaa Gly Xaa Xaa Xaa Xaa Xaa Xaa
  1               5                  10                  15

Xaa Xaa Xaa Xaa Xaa Xaa Xaa Xaa Xaa Gly Xaa Xaa Pro Xaa Xaa Xaa
            20                  25                  30

Xaa Xaa Xaa Xaa Xaa Xaa Xaa Xaa Xaa Xaa Xaa Xaa Xaa Xaa
        35                  40                  45


<210> 199
<211> 34
<212> PRT
<213> Artificial Sequence

<220>
<223> Description of Artificial Sequence: Consensus
      sequence

<220>
<221> UNSURE
<222> (1)
<223> Xaa at position 1 is Leu, Ile or Val

<220>
<221> UNSURE
<222> (2)..(4)
<223> Xaa at position 2-4 is any amino acid

<220>
<221> UNSURE
<222> (5)
<223> Xaa at position 5 is Leu, Ile or Val

<220>
<221> UNSURE
<222> (6)..(7)
<223> Xaa at position 6-7 is any amino acid

<220>
<221> UNSURE
<222> (8)
<223> Xaa at position 8 is Leu, Ile or Val

<220>
<221> UNSURE
<222> (9)..(11)
<223> Xaa at position 9-11 is any amino acid

<220>
<221> UNSURE
<222> (13)..(14)
<223> Xaa at position 13-14 is any amino acid

<220>
<221> UNSURE
<222> (15)
<223> Xaa at position 15 is Leu, Ile or Val

<220>
<221> UNSURE
<222> (17)..(21)
<223> Xaa at position 17-21 is any amino acid

<220>
<221> UNSURE
<222> (23)..(26)
<223> Xaa at position 23-26 is any amino acid

<220>
<221> UNSURE
<222> (27)
<223> Xaa at position 27 is Leu, Ile or Val

<220>
<221> UNSURE
<222> (29)..(30)
<223> Xaa at position 29-30 is any amino acid

<220>
<221> UNSURE
<222> (31)..(32)
<223> Xaa at position 31- 32 is Leu, Ile or Val

<220>
<221> UNSURE
<222> (33)

<223> Xaa at position 33 is any amino acid

<220>
<221> UNSURE
<222> (34)
<223> Xaa at position 34 is Leu, Ile or Val

<220>
<221> UNSURE
<222> (12)
<223> Xaa at position 12 is Asp or Glu

<220>
<221> UNSURE
<222> (22)
<223> Xaa at position 22 is Asp or Glu

<220>
<221> UNSURE
<222> (28)
<223> Xaa at position 28 is Asp or Glu

<220>
<221> UNSURE
<222> (16)
<223> Xaa at position 16 is Arg or Lys

<400> 199
Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Xaa Xaa Xaa Xaa Xaa Xaa Xaa Xaa
            20                  25                  30

Xaa Xaa



<210> 200
<211> 643
<212> PRT
<213> Escherichia coli

<400> 200
Met Ser Tyr Gln Val Leu Ala Arg Lys Trp Arg Pro Gln Thr Phe Ala
1               5                   10                  15

Asp Val Val Gly Gln Glu His Val Leu Thr Ala Leu Ala Asn Gly Leu
            20                  25                  30

Ser Leu Gly Arg Ile His His Ala Tyr Leu Phe Ser Gly Thr Arg Gly
        35                  40                  45

Val Gly Lys Thr Ser Ile Ala Arg Leu Leu Ala Lys Gly Leu Asn Cys
    50                  55                  60

Glu Thr Gly Ile Thr Ala Thr Pro Cys Gly Val Cys Asp Asn Cys Arg
65                  70                  75                  80

Glu Ile Glu Gln Gly Arg Phe Val Asp Leu Ile Glu Ile Asp Ala Ala
                85                  90                  95

Ser Arg Thr Lys Val Glu Asp Thr Arg Asp Leu Leu Asp Asn Val Gln
            100                 105                 110

Tyr Ala Pro Ala Arg Gly Arg Phe Lys Val Tyr Leu Ile Asp Glu Val
        115                 120                 125

His Met Leu Ser Arg His Ser Phe Asn Ala Leu Leu Lys Thr Leu Glu
    130                 135                 140

Glu Pro Pro Glu His Val Lys Phe Leu Leu Ala Thr Thr Asp Pro Gln
145                 150                 155                 160

Lys Leu Pro Val Thr Ile Leu Ser Arg Cys Leu Gln Phe His Leu Lys
                165                 170                 175

Ala Leu Asp Val Glu Gln Ile Arg His Gln Leu Glu His Ile Leu Asn
            180                 185                 190

Glu Glu His Ile Ala His Glu Pro Arg Ala Leu Gln Leu Leu Ala Arg
        195                 200                 205

Ala Ala Glu Gly Ser Leu Arg Asp Ala Leu Ser Leu Thr Asp Gln Ala
    210                 215                 220

Ile Ala Ser Gly Asp Gly Gln Val Ser Thr Gln Ala Val Ser Ala Met
225                 230                 235                 240

Leu Gly Thr Leu Asp Asp Asp Gln Ala Leu Ser Leu Val Glu Ala Met
                245                 250                 255

Val Glu Ala Asn Gly Glu Arg Val Met Ala Leu Ile Asn Glu Ala Ala
            260                 265                 270

Ala Arg Gly Ile Glu Trp Glu Ala Leu Leu Val Glu Met Leu Gly Leu
        275                 280                 285

Leu His Arg Ile Ala Met Val Gln Leu Ser Pro Ala Ala Leu Gly Asn
    290                 295                 300

Asp Met Ala Ala Ile Glu Leu Arg Met Arg Glu Leu Ala Arg Thr Ile
305                 310                 315                 320

Pro Pro Thr Asp Ile Gln Leu Tyr Tyr Gln Thr Leu Leu Ile Gly Arg
                325                 330                 335

Lys Glu Leu Pro Tyr Ala Pro Asp Arg Arg Met Gly Val Glu Met Thr
            340                 345                 350

Leu Leu Arg Ala Leu Ala Phe His Pro Arg Met Pro Leu Pro Glu Pro
        355                 360                 365

Glu Val Pro Arg Gln Ser Phe Ala Pro Val Ala Pro Thr Ala Val Met
    370                 375                 380

Thr Pro Thr Gln Val Pro Pro Gln Pro Gln Ser Ala Pro Gln Gln Ala
385                 390                 395                 400

Pro Thr Val Pro Leu Pro Glu Thr Thr Ser Gln Val Leu Ala Ala Arg
                405                 410                 415

Gln Gln Leu Gln Arg Val Gln Gly Ala Thr Lys Ala Lys Lys Ser Glu
            420                 425                 430

Pro Ala Ala Ala Thr Arg Ala Arg Pro Val Asn Asn Ala Ala Leu Glu
        435                 440                 445

Arg Leu Ala Ser Val Thr Asp Arg Val Gln Ala Arg Pro Val Pro Ser
    450                 455                 460

Ala Leu Glu Lys Ala Pro Ala Lys Lys Glu Ala Tyr Arg Trp Lys Ala
465                 470                 475                 480

Thr Thr Pro Val Met Gln Gln Lys Glu Val Val Ala Thr Pro Lys Ala
                485                 490                 495

Leu Lys Lys Ala Leu Glu His Glu Lys Thr Pro Glu Leu Ala Ala Lys
            500                 505                 510

Leu Ala Ala Glu Ala Ile Glu Arg Asp Pro Trp Ala Ala Gln Val Ser
        515                 520                 525

Gln Leu Ser Leu Pro Lys Leu Val Glu Gln Val Ala Leu Asn Ala Trp
    530                 535                 540

Lys Glu Glu Ser Asp Asn Ala Val Cys Leu His Leu Arg Ser Ser Gln
545                 550                 555                 560

Arg His Leu Asn Asn Arg Gly Ala Gln Gln Lys Leu Ala Glu Ala Leu
                565                 570                 575

Ser Met Leu Lys Gly Ser Thr Val Glu Leu Thr Ile Val Glu Asp Asp
            580                 585                 590

Asn Pro Ala Val Arg Thr Pro Leu Glu Trp Arg Gln Ala Ile Tyr Glu
        595                 600                 605

Glu Lys Leu Ala Gln Ala Arg Glu Ser Ile Ile Ala Asp Asn Asn Ile
    610                 615                 620

Gln Thr Leu Arg Arg Phe Phe Asp Ala Glu Leu Asp Glu Glu Ser Ile
625                 630                 635                 640

Arg Pro Ile

<210> 201
<211> 15
<212> PRT
<213> Artificial Sequence

<220>
<223> Description of Artificial Sequence: Consensus
      sequence

<220>
<221> UNSURE
<222> (2)..(5)
<223> Xaa at position 2-5 is any amino acid

<220>
<221> UNSURE
<222> (8)
<223> Xaa at position 8 is Thr or Ser

<220>
<221> UNSURE
<222> (9)..(14)
<223> Xaa at position 9-14 is any amino acid

<220>
<221> UNSURE
<222> (15)
<223> Xaa at position 15 is Ile or Val

<400> 201
Gly Xaa Xaa Xaa Xaa Gly Lys Xaa Xaa Xaa Xaa Xaa Xaa Xaa Xaa
  1               5                  10                  15

<210> 202
<211> 14

<212> PRT
<213> Artificial Sequence

<220>
<223> Description of Artificial Sequence: Consensus
      sequence

<220>
<221> UNSURE
<222> (1)
<223> Xaa at position 1 is Arg or Lys

<220>
<221> UNSURE
<222> (2)..(4)
<223> Xaa at position 2-4 is any amino acid

<220>
<221> UNSURE
<222> (6)..(8)
<223> Xaa at position 6-8 is any amino acid

<220>
<221> UNSURE
<222> (13)
<223> Xaa at position 13 is Arg or Lys

<220>
<221> UNSURE
<222> (10)..(13)
<223> Xaa at position 10-13 is hydrophobic residue

<400> 202
Xaa Xaa Xaa Xaa Gly Xaa Xaa Xaa Leu Xaa Xaa Xaa Xaa Asp
  1               5                   10


<210> 203
<211> 14
<212> PRT
<213> Artificial Sequence

<220>
<223> Description of Artificial Sequence: Consensus
      sequence

<220>
<221> UNSURE
<222> (14)
<223> Xaa at position 14 is Thr or Ser

<220>
<221> UNSURE
<222> (8)..(9)
<223> Xaa at position 8-9 is any amino acid

<400> 203
His Ala Tyr Leu Phe Ser Gly Xaa Xaa Gly Xaa Gly Lys Xaa
  1               5                   10


<210> 204
<211> 10
<212> PRT
<213> Artificial Sequence

<220>
<223> Description of Artificial Sequence: Consensus

sequence

<220>
<221> UNSURE
<222> (1)
<223> Xaa at position 1 is Leu, Ile or Val

<220>
<221> UNSURE
<222> (3)..(4)
<223> Xaa at position 3-4 is Leu, Ile or Val

<220>
<221> UNSURE
<222> (6)
<223> Xaa at position 6 is Leu, Ile or Val

<220>
<221> UNSURE
<222> (8)
<223> Xaa at position 8 is Ala or Gly

<400> 204
Xaa Asp Xaa Xaa Glu Xaa Asp Xaa Ala Ser
1               5                   10


<210> 205
<211> 12
<212> PRT
<213> Artificial Sequence

<220>
<223> Description of Artificial Sequence: Consensus
      sequence

<220>
<221> UNSURE
<222> (1)
<223> Xaa at position 1 is Lys or Arg

<220>
<221> UNSURE
<222> (2)
<223> Xaa at position 2 is Leu, Ile or Val

<220>
<221> UNSURE
<222> (4)..(5)
<223> Xaa at position 4-5 is Leu, Ile or Val

<220>
<221> UNSURE
<222> (11)
<223> Xaa at position 11 is Leu, Ile or Val

<220>
<221> UNSURE
<222> (12)
<223> Xaa at position 12 is Ser or Thr

<220>
<221> UNSURE
<222> (3)
<223> Xaa at position 3 is Tyr or Phe

<220>
<221> UNSURE

<222> (8)
<223> Xaa at position 8 is any amino acid

<400> 205
Xaa Xaa Xaa Xaa Xaa Asp Glu Xaa His Met Xaa Xaa
1               5                   10


<210> 206
<211> 334
<212> PRT
<213> Artificial Sequence

<220>
<223> Description of Artificial Sequence: Consensus
      sequence

<400> 206
Met Arg Trp Tyr Pro Trp Leu Arg Pro Asp Phe Glu Lys Leu Val Ala
1               5                   10                  15

Ser Tyr Gln Ala Gly Arg Gly His His Ala Leu Leu Ile Gln Ala Leu
            20                  25                  30

Pro Gly Met Gly Asp Asp Ala Leu Ile Tyr Ala Leu Ser Arg Tyr Leu
        35                  40                  45

Leu Cys Gln Gln Pro Gln Gly His Lys Ser Cys Gly His Cys Arg Gly
    50                  55                  60

Cys Gln Leu Met Gln Ala Gly Thr His Pro Asp Tyr Tyr Thr Leu Ala
65                  70                  75                  80

Pro Glu Lys Gly Lys Asn Thr Leu Gly Val Asp Ala Val Arg Glu Val
                85                  90                  95

Thr Glu Lys Leu Asn Glu His Ala Arg Leu Gly Gly Ala Lys Val Val
            100                 105                 110

Trp Val Thr Asp Ala Ala Leu Leu Thr Asp Ala Ala Ala Asn Ala Leu
        115                 120                 125

Leu Lys Thr Leu Glu Glu Pro Pro Ala Glu Thr Trp Phe Phe Leu Ala
    130                 135                 140

Thr Arg Glu Pro Glu Arg Leu Leu Ala Thr Leu Arg Ser Arg Cys Arg
145                 150                 155                 160

Leu His Tyr Leu Ala Pro Pro Pro Glu Gln Tyr Ala Val Thr Trp Leu
                165                 170                 175

Ser Arg Glu Val Thr Met Ser Gln Asp Ala Leu Leu Ala Ala Leu Arg
            180                 185                 190

Leu Ser Ala Gly Ser Pro Gly Ala Ala Leu Ala Leu Phe Gln Gly Asp
        195                 200                 205

Asn Trp Gln Ala Arg Glu Thr Leu Cys Gln Ala Leu Ala Tyr Ser Val
    210                 215                 220

Pro Ser Gly Asp Trp Tyr Ser Leu Leu Ala Ala Leu Asn His Glu Gln
225                 230                 235                 240

Ala Pro Ala Arg Leu His Trp Leu Ala Thr Leu Leu Met Asp Ala Leu
                245                 250                 255

Lys Arg His His Gly Ala Ala Gln Val Thr Asn Val Asp Val Pro Gly
            260                 265                 270

Leu Val Ala Glu Leu Ala Asn His Leu Ser Pro Ser Arg Leu Gln Ala
        275                 280                 285

Ile Leu Gly Asp Val Cys His Ile Arg Glu Gln Leu Met Ser Val Thr
    290                 295                 300

Gly Ile Asn Arg Glu Leu Leu Ile Thr Asp Leu Leu Leu Arg Ile Glu
305                 310                 315                 320

His Tyr Leu Gln Pro Gly Val Val Leu Pro Val Pro His Leu
                325                 330

<210> 207
<211> 13
<212> PRT
<213> Artificial Sequence

<220>
<223> Description of Artificial Sequence: Consensus
      sequence

<220>
<221> UNSURE
<222> (1)
<223> Xaa at position 1 is Ala or Ser

<220>
<221> UNSURE
<222> (4)
<223> Xaa at position 4 is Ala or Ser

<220>
<221> UNSURE
<222> (5)..(6)
<223> Xaa at position 5-6 is Leu, Ile or Val

<220>
<221> UNSURE
<222> (9)
<223> Xaa at position 9 is Leu, Ile or Val

<220>
<221> UNSURE
<222> (7)
<223> Xaa at position 7 is Lys or Arg

<220>
<221> UNSURE
<222> (8)
<223> Xaa at position 8 is any amino acid

<220>
<221> UNSURE
<222> (11)
<223> Xaa at position 11 is Glu or Asp

<400> 207
Xaa Ala Asn Xaa Xaa Xaa Xaa Xaa Xaa Glu Xaa Pro Pro
  1               5                  10

<210> 208
<211> 8
<212> PRT
<213> Artificial Sequence

<220>
<223> Description of Artificial Sequence: Consensus
      sequence

<220>
<221> UNSURE
<222> (1)..(2)
<223> Xaa at position 1-2 is Leu, Ile or Val

<220>
<221> UNSURE
<222> (5)
<223> Xaa at position 5 is Leu, Ile or Val

<220>
<221> UNSURE
<222> (3)
<223> Xaa at position 3 is any amino acid

<220>
<221> UNSURE
<222> (6)
<223> Xaa at position 6 is any amino acid

<400> 208
Xaa Xaa Xaa Thr Xaa Xaa Ser Arg
  1               5


<210> 209
<211> 343
<212> PRT
<213> Artificial Sequence

<220>
<223> Description of Artificial Sequence: Consensus
      sequence

<400> 209
   Met Ile Arg Leu Tyr Pro Glu Gln Leu Arg Ala Gln Leu Asn Glu Gly
    1               5                  10                  15
   Leu Arg Ala Ala Tyr Leu Leu Leu Gly Asn Asp Pro Leu Leu Leu Gln
               20                  25                  30
   Glu Ser Gln Asp Ala Val Arg Gln Val Ala Ala Ala Gln Gly Phe Glu
           35                  40                  45
   Glu His His Thr Phe Ser Ile Asp Pro Asn Thr Asp Trp Asn Ala Ile
       50                  55                  60
   Phe Ser Leu Cys Gln Ala Met Ser Leu Phe Ala Ser Arg Gln Thr Leu
   65                  70                  75                  80
   Leu Leu Leu Leu Pro Glu Asn Gly Pro Asn Ala Ala Ile Asn Glu Gln
                   85                  90                  95
   Leu Leu Thr Leu Thr Gly Leu Leu His Asp Asp Leu Leu Leu Ile Val
               100                 105                 110
   Arg Gly Asn Lys Leu Ser Lys Ala Gln Glu Asn Ala Ala Trp Phe Thr
           115                 120                 125
   Ala Leu Ala Asn Arg Ser Val Gln Val Thr Cys Gln Thr Pro Glu Gln
       130                 135                 140
   Ala Gln Leu Pro Arg Trp Val Ala Ala Arg Ala Lys Gln Leu Asn Leu
   145                 150                 155                 160
   Glu Leu Asp Asp Ala Ala Asn Gln Val Leu Cys Tyr Cys Tyr Glu Gly
                   165                 170                 175
   Asn Leu Leu Ala Leu Ala Gln Ala Leu Glu Arg Leu Ser Leu Leu Trp
               180                 185                 190
   Pro Asp Gly Lys Leu Thr Leu Pro Arg Val Glu Gln Ala Val Asn Asp
           195                 200                 205
   Ala Ala His Phe Thr Pro Phe His Trp Val Asp Ala Leu Leu Met Gly
       210                 215                 220
   Lys Ser Lys Arg Ala Leu His Ile Leu Gln Gln Leu Arg Leu Glu Gly
   225                 230                 235                 240
   Ser Glu Pro Val Ile Leu Leu Arg Thr Leu Gln Arg Glu Leu Leu Leu
                   245                 250                 255
   Leu Val Asn Leu Lys Arg Gln Ser Ala His Thr Pro Leu Arg Ala Leu
               260                 265                 270
   Phe Asp Lys His Arg Val Trp Gln Asn Arg Arg Gly Met Met Gly Glu
           275                 280                 285
   Ala Leu Asn Arg Leu Ser Gln Thr Gln Leu Arg Gln Ala Val Gln Leu
       290                 295                 300
   Leu Thr Arg Thr Glu Leu Thr Leu Lys Gln Asp Tyr Gly Gln Ser Val
   305                 310                 315                 320
   Trp Ala Glu Leu Glu Gly Leu Ser Leu Leu Leu Cys His Lys Pro Leu
                   325                 330                 335
   Ala Asp Val Phe Ile Asp Gly
               340


<210> 210
<211> 324
<212> PRT
<213> Thermotoga maritima

<400> 210
Met Pro Val Thr Phe Leu Thr Gly Thr Ala Glu Thr Gln Lys Glu Glu
  1               5                  10                  15

Leu Ile Lys Lys Leu Leu Lys Asp Gly Asn Val Glu Tyr Ile Arg Ile
             20                  25                  30

His Pro Glu Asp Pro Asp Lys Ile Asp Phe Ile Arg Ser Leu Leu Arg
         35                  40                  45

Thr Lys Thr Ile Phe Ser Asn Lys Thr Ile Ile Asp Ile Val Asn Phe
     50                  55                  60

Asp Glu Trp Lys Ala Gln Glu Gln Lys Arg Leu Val Glu Leu Leu Lys
 65                  70                  75                  80

Asn Val Pro Glu Asp Val His Ile Phe Ile Arg Ser Gln Lys Thr Gly
                 85                  90                  95

Gly Lys Gly Val Ala Leu Glu Leu Pro Lys Pro Trp Glu Thr Asp Lys
            100                 105                 110

Trp Leu Glu Trp Ile Glu Lys Arg Phe Arg Glu Asn Gly Leu Leu Ile
        115                 120                 125

Asp Lys Asp Ala Leu Gln Leu Phe Phe Ser Lys Val Gly Thr Asn Asp
    130                 135                 140

Leu Ile Ile Glu Arg Glu Ile Glu Lys Leu Lys Ala Tyr Ser Glu Asp
145                 150                 155                 160

Arg Lys Ile Thr Val Glu Asp Val Glu Glu Val Val Phe Thr Tyr Gln
                165                 170                 175

Thr Pro Gly Tyr Asp Asp Phe Cys Phe Ala Val Ser Glu Gly Lys Arg
            180                 185                 190

Lys Leu Ala His Ser Leu Leu Ser Gln Leu Trp Lys Thr Thr Glu Ser
        195                 200                 205

Val Val Ile Ala Thr Val Leu Ala Asn His Phe Leu Asp Leu Phe Lys
    210                 215                 220

Ile Leu Val Leu Val Thr Lys Lys Arg Tyr Tyr Thr Trp Pro Asp Val
225                 230                 235                 240

Ser Arg Val Ser Lys Glu Leu Gly Ile Pro Val Pro Arg Val Ala Arg
                245                 250                 255

Phe Leu Gly Phe Ser Phe Lys Thr Trp Lys Phe Lys Val Met Asn His
            260                 265                 270

Leu Leu Tyr Tyr Asp Val Lys Lys Val Arg Lys Ile Leu Arg Asp Leu
        275                 280                 285

Tyr Asp Leu Asp Arg Ala Val Lys Ser Glu Glu Asp Pro Lys Pro Phe
    290                 295                 300

Phe His Glu Phe Ile Glu Glu Val Ala Leu Asp Val Tyr Ser Leu Gln
305                 310                 315                 320

Arg Asp Glu Glu


<210> 211
<211> 1115
<212> DNA
<213> Thermotoga maritima

<400> 211
cactttgaaa ttgtttctca aagcgtgtta aaatcaccat gagaggtgat tgtatgccag 60

tcacgtttct cacaggtact gcagaaactc agaaggaaga attgataaag aaactcctga 120

aggatggtaa cgtggagtac ataaggatcc atccggagga tcccgacaag atcgatttca 180

taaggtcttt actcaggaca aagacgatct tttccaacaa gacgatcatt gacatcgtca 240

atttcgatga gtggaaagca caggagcaga agcgtctcgt tgaacttttg aaaaacgtac 300

cggaagacgt tcatatcttc atccgttctc aaaaaacagg tggaaaggga gtagcgctgg 360

agcttccgaa gccatgggaa acggacaagt ggcttgagtg gatagaaaag cgcttcaggg 420

agaatggttt gctcatcgat aaagatgccc ttcagctgtt tttctccaag gttggaacga 480

acgacctgat catagaaagg gagattgaaa aactgaaagc ttattccgag gacagaaaga 540

taacggtaga agacgtggaa gaggtcgttt ttacctatca gactccggga tacgatgatt 600

tttgctttgc tgtttccgaa ggaaaaagga agctcgctca ctctcttctg tcgcagctgt 660

ggaaaaccac agagtccgtg gtgattgcca ctgtccttgc gaatcacttc ttggatctct 720

tcaaaatcct cgttcttgtg acaaagaaaa gatactacac ctggcctgat gtgtccaggg 780

tgtccaaaga gctgggaatt cccgttcctc gtgtggctcg tttcctcggt ttctccttta 840

agacctggaa attcaaggtg atgaaccacc tcctctacta cgatgtgaag aaggttagaa 900

agatactgag ggatctctac gatctggaca gagccgtgaa aagcgaagaa gatccaaaac 960

cgttcttcca cgagttcata gaagaggtgg cactggatgt atattctctt cagagagatg 1020

aagaataact ggtacagcct cgcggccctt ctctcaacca tatacagcag gcacctcgat 1080

gtggaagcaa gacctgtgaa gtttgaggaa ataaa 1115

<210> 212
<211> 1200
<212> DNA
<213> Aquifex aeolicus

<400> 212
tggcttttac ctctttgtag agttaagtat taaatttaat ttaactgtgg aaaccacaat 60

attccagttc cagaaaactt ttttcacaaa acctccgaag gagagggtct tcgtccttca 120

tggagaagag cagtatctca taagaacctt tttgtctaag ctgaaggaaa agtacgggga 180

gaattacacg gttctgtggg gggatgagat aagcgaggag gaattctaca ctgccctttc 240

cgagaccagt atattcggcg gttcaaagga aaaagcggtg gtcatttaca acttcgggga 300

tttcctgaag aagctcggaa ggaagaaaaa ggaaaaagaa aggcttataa aagtcctcag 360

aaacgtaaag agtaactacg tatttatagt gtacgatgcg aaactccaga aacaggaact 420

ttcttcggaa cctctgaaat ccgtagcgtc tttcggcggt atagtggtag caaacaggct 480

gagcaaggag aggataaaac agctcgtcct taagaagttc aaagaaaaag ggataaacgt 540

agaaaacgat gcccttgaat accttctcca gctcacgggt tacaacttga tggagctcaa 600

acttgaggtt gaaaaactga tagattacgc aagtgaaaag aaaattttaa cactcgatga 660

ggtaaagaga gtagccttct cagtctcaga aaacgtaaac gtatttgagt tcgttgattt 720

actcctctta aaagattacg aaaaggctct taaagttttg gactccctca tttccttcgg 780

aatacacccc ctccagatta tgaaaatcct gtcctcctat gctctaaaac tttacaccct 840

caagaggctt gaagagaagg gagaggacct gaataaggcg atggaaagcg tgggaataaa 900

gaacaacttt ctcaagatga agttcaaatc ttacttaaag gcaaactcta aagaggactt 960

gaagaaccta atcctctccc tccagaggat agacgctttt tctaaacttt actttcagga 1020

cacagtgcag ttgctgaggg atttcttgac ctcaagactg gagagggaag ttgtgaaaaa 1080

tacttctcat ggtggataat cttttttatg aagtttgcgg tttgcgtttt tcccggttct 1140

aactgcgatt acgacaccta ctacgttata agggacattc ttgaaaagga cgtggaattc 1200

<210> 213
<211> 350
<212> PRT
<213> Aquifex aeolicus

<400> 213
Met Glu Thr Thr Ile Phe Gln Phe Gln Lys Thr Phe Phe Thr Lys Pro
1               5                   10                  15

Pro Lys Glu Arg Val Phe Val Leu His Gly Glu Glu Gln Tyr Leu Ile
            20                  25                  30

Arg Thr Phe Leu Ser Lys Leu Lys Glu Lys Tyr Gly Glu Asn Tyr Thr
        35                  40                  45

Val Leu Trp Gly Asp Glu Ile Ser Glu Glu Glu Phe Tyr Thr Ala Leu
    50                  55                  60

Ser Glu Thr Ser Ile Phe Gly Gly Ser Lys Glu Lys Ala Val Val Ile
65                  70                  75                  80

Tyr Asn Phe Gly Asp Phe Leu Lys Lys Leu Gly Arg Lys Lys Lys Glu
                85                  90                  95

Lys Glu Arg Leu Ile Lys Val Leu Arg Asn Val Lys Ser Asn Tyr Val
            100                 105                 110

Phe Ile Val Tyr Asp Ala Lys Leu Gln Lys Gln Glu Leu Ser Ser Glu
        115                 120                 125

Pro Leu Lys Ser Val Ala Ser Phe Gly Gly Ile Val Val Ala Asn Arg
    130                 135                 140

Leu Ser Lys Glu Arg Ile Lys Gln Leu Val Leu Lys Lys Phe Lys Glu
145                 150                 155                 160

Lys Gly Ile Asn Val Glu Asn Asp Ala Leu Glu Tyr Leu Leu Gln Leu
                165                 170                 175

Thr Gly Tyr Asn Leu Met Glu Leu Lys Leu Glu Val Glu Lys Leu Ile
            180                 185                 190

Asp Tyr Ala Ser Glu Lys Lys Ile Leu Thr Leu Asp Glu Val Lys Arg
        195                 200                 205

Val Ala Phe Ser Val Ser Glu Asn Val Asn Val Phe Glu Phe Val Asp
    210                 215                 220

Leu Leu Leu Leu Lys Asp Tyr Glu Lys Ala Leu Lys Val Leu Asp Ser
225                 230                 235                 240

Leu Ile Ser Phe Gly Ile His Pro Leu Gln Ile Met Lys Ile Leu Ser
                245                 250                 255

Ser Tyr Ala Leu Lys Leu Tyr Thr Leu Lys Arg Leu Glu Glu Lys Gly
            260                 265                 270

Glu Asp Leu Asn Lys Ala Met Glu Ser Val Gly Ile Lys Asn Asn Phe
        275                 280                 285

Leu Lys Met Lys Phe Lys Ser Tyr Leu Lys Ala Asn Ser Lys Glu Asp
    290                 295                 300

Leu Lys Asn Leu Ile Leu Ser Leu Gln Arg Ile Asp Ala Phe Ser Lys
305                 310                 315                 320

Leu Tyr Phe Gln Asp Thr Val Gln Leu Leu Arg Asp Phe Leu Thr Ser
                325                 330                 335

Arg Leu Glu Arg Glu Val Val Lys Asn Thr Ser His Gly Gly
            340                 345                 350

<210> 214
<211> 32
<212> PRT
<213> Escherichia coli

<400> 214
Met Ala Gly Cys Leu Asn Asp Ile Phe Glu Ala Gln Lys Ile Glu Trp
1               5                   10                  15

His His His His His His Leu Val Pro Arg Gly Ser Gly Gly Gly Gly
            20                  25                  30

<210> 215
<211> 305
<212> PRT
<213> Aquifex aeolicus

<400> 215
Met Glu Lys Val Phe Leu Glu Lys Leu Gln Lys Thr Leu His Ile Pro
1               5                   10                  15

Gly Gly Leu Leu Phe Tyr Gly Lys Glu Gly Ser Gly Lys Thr Lys Thr
            20                  25                  30

Ala Phe Glu Phe Ala Lys Gly Ile Leu Cys Lys Glu Asn Val Pro Trp
        35                  40                  45

Gly Cys Gly Ser Cys Pro Ser Cys Lys His Val Asn Glu Leu Glu Glu
    50                  55                  60

Ala Phe Phe Lys Gly Glu Ile Glu Asp Phe Lys Val Tyr Lys Asp Lys
65                  70                  75                  80

Asp Gly Lys Lys His Phe Val Tyr Leu Met Gly Glu His Pro Asp Phe
                85                  90                  95

Val Val Ile Ile Pro Ser Gly His Tyr Ile Lys Ile Glu Gln Ile Arg
            100                 105                 110

Glu Val Lys Asn Phe Ala Tyr Val Lys Pro Ala Leu Ser Arg Arg Lys
        115                 120                 125

Val Ile Ile Ile Asp Asp Ala His Ala Met Thr Ser Gln Ala Ala Asn
    130                 135                 140

Ala Leu Leu Lys Val Leu Glu Glu Pro Pro Ala Asp Thr Thr Phe Ile
145                 150                 155                 160

Leu Thr Thr Asn Arg Arg Ser Ala Ile Leu Pro Thr Ile Leu Ser Arg
                165                 170                 175

Thr Phe Gln Val Glu Phe Lys Gly Phe Ser Val Lys Glu Val Met Glu
            180                 185                 190

Ile Ala Lys Val Asp Glu Glu Ile Ala Lys Leu Ser Gly Gly Ser Leu
        195                 200                 205

Lys Arg Ala Ile Leu Leu Lys Glu Asn Lys Asp Ile Leu Asn Lys Val
    210                 215                 220

Lys Glu Phe Leu Glu Asn Glu Pro Leu Lys Val Tyr Lys Leu Ala Ser
225                 230                 235                 240

Glu Phe Glu Lys Trp Glu Pro Glu Lys Gln Lys Leu Phe Leu Glu Ile
                245                 250                 255

Met Glu Glu Leu Val Ser Gln Lys Leu Thr Glu Glu Lys Lys Asp Asn
            260                 265                 270

Tyr Thr Tyr Leu Leu Asp Thr Ile Arg Leu Phe Lys Asp Gly Leu Ala
        275                 280                 285

Arg Gly Val Asn Glu Pro Leu Trp Leu Phe Thr Leu Ala Val Gln Ala
    290                 295                 300

Asp
305

<210> 216
<211> 1540
<212> DNA
<213> Aquifex aeolicus

<400> 216
actctctttc agacttaaag aaaagatacg ggaaattctc ggcgagaaat ttgagcattt 60

aataaaagtt ttaggggata taatttccga taatacactt gaaacacctt taataacact 120

cggtgaaggt aaaaacctca agaaaaagtt taacgaggaa ttcatagaaa cccttgagaa 180

tgcttcctat tcaaacgagg aggccggaaa attaagggaa tttttaaaga cactttacgc 240

tttggcactc tgtaatagta gggaagaaat cactctggac ctttgtgtaa tattactcca 300

ctccgaacag cacgcaactt tagaaaaaca actcctttct ctcgtacaac cgctactaaa 360

aattgataaa aaaccgaaga aagaactgaa cctatagtcc gcgaagttgc aaaggttata 420

aacgaaacct gtttaaatta aatttgtatg gaaaaagttt ttttggaaaa actccagaaa 480

accttgcaca tacccggagg actccttttt tacggcaaag aaggaagcgg aaagacgaaa 540

acagcttttg aatttgcaaa aggtatttta tgtaaggaaa acgtaccgtg gggatgcgga 600

agttgtccct cctgcaaaca cgtaaacgag ctggaggaag ccttctttaa aggagaaata 660

gaagacttta aagtttataa agacaaggac ggtaaaaagc acttcgttta ccttatgggc 720

gaacatcccg actttgtggt aataatcccg agcggacatt acataaagat agaacagata 780

agggaagtta agaactttgc ctatgtgaag cccgcactaa gcaggagaaa agtaattata 840

atagacgacg cccacgcgat gacctctcag gcggcaaacg ctcttttaaa ggtattggaa 900

gagccacctg cggacaccac ctttatcttg accacgaaca ggcgttctgc aatcctgccg 960

actatcctct ccagaacttt tcaagtggag ttcaagggct tttcagtaaa agaggttatg 1020

gaaatagcga aagtagacga ggaaatagcg aaactctctg gaggcagtct aaaaagggct 1080

atcttactaa aggaaaacaa agatatccta aacaaagtaa aggaattctt ggaaaacgag 1140

ccgttaaaag tttacaagct tgcaagtgaa ttcgaaaagt gggaacctga aaagcaaaaa 1200

ctcttccttg aaattatgga agaattggta tctcaaaaat tgaccgaaga gaaaaaagac 1260

aattacacct accttcttga tacgatcaga ctctttaaag acggactcgc aaggggtgta 1320

aacgaacctc tgtggctgtt tacgttagcc gttcaggcgg attaataaac cgttattgat 1380

tccgtaacat ttaaacctta atctaaatta tgagagcctt tgaaggaggt ctggtatgga 1440

aaatttgaag attagatata tagatacgag gaagatagga accgtgagcg gtgtaaaagt 1500

tccggtaaaa aagggctaca aaatagtcgt ggaagacgag 1540

<210> 217
<211> 599
<212> PRT
<213> Thermotoga maritima

<400> 217
Met Ser Ile Ser Glu Lys Pro Leu Ser Glu Leu Leu Arg Pro Lys Asp
1               5                   10                  15

Phe Glu Asp Phe Val Gly Gln Asp His Ile Phe Gly Asn Lys Gly Ile
            20                  25                  30

Leu Arg Arg Thr Leu Lys Thr Gly Asn Met Phe Ser Ser Ile Leu Tyr
        35                  40                  45

Gly Pro Pro Gly Ser Gly Lys Thr Ser Val Phe Ser Leu Leu Lys Arg
    50                  55                  60

Tyr Phe Asn Gly Glu Val Val Tyr Leu Ser Ser Thr Val His Gly Val
65                  70                  75                  80

Ser Glu Ile Lys Asn Val Leu Lys Arg Gly Glu Gln Leu Arg Lys Tyr
                85                  90                  95

Gly Lys Lys Leu Leu Leu Phe Leu Asp Glu Ile His Arg Leu Asn Lys
            100                 105                 110

Asn Gln Gln Met Val Leu Val Ser His Val Glu Arg Gly Asp Ile Val
        115                 120                 125

Leu Val Ala Thr Thr Thr Glu Asn Pro Ser Phe Val Ile Val Pro Ala
    130                 135                 140

Leu Leu Ser Arg Cys Arg Ile Leu Tyr Phe Lys Lys Leu Ser Asp Glu
145                 150                 155                 160

Asp Leu Met Lys Ile Leu Lys Lys Ala Thr Glu Val Leu Asn Ile Asp
                165                 170                 175

Leu Glu Glu Ser Val Glu Lys Ala Ile Val Arg His Ser Glu Gly Asp
            180                 185                 190

Ala Arg Lys Leu Leu Asn Thr Leu Glu Ile Val His Gln Ala Phe Lys
        195                 200                 205

Asn Lys Arg Val Thr Leu Glu Asp Leu Glu Thr Leu Leu Gly Asn Val
    210                 215                 220

Ser Gly Tyr Thr Lys Glu Ser His Tyr Asp Phe Ala Ser Ala Phe Ile
225                 230                 235                 240

Lys Ser Met Arg Gly Ser Asp Pro Asn Ala Ala Val Tyr Tyr Leu Val
                245                 250                 255

Lys Met Ile Glu Met Gly Glu Asp Pro Arg Phe Ile Ala Arg Arg Met
            260                 265                 270

Ile Ile Phe Ala Ser Glu Asp Val Gly Leu Ala Asp Pro Asn Ala Leu
        275                 280                 285

His Ile Ala Val Ser Thr Ser Ile Ala Val Glu His Val Gly Leu Pro

290                 295                 300

Glu Cys Leu Met Asn Leu Val Glu Cys Ala Val Tyr Leu Ser Leu Ala
305                 310                 315                 320

Pro Lys Ser Asn Ser Val Tyr Leu Ala Met Lys Lys Val Gln Glu Leu
                325                 330                 335

Pro Val Glu Asp Val Pro Leu Phe Leu Arg Asn Pro Val Thr Glu Glu
            340                 345                 350

Met Lys Lys Arg Gly Tyr Gly Glu Gly Tyr Leu Tyr Pro His Asp Phe
        355                 360                 365

Gly Gly Phe Val Lys Thr Asn Tyr Leu Pro Glu Lys Leu Lys Asn Glu
    370                 375                 380

Val Ile Phe Gln Pro Lys Arg Val Gly Phe Glu Glu Glu Leu Phe Glu
385                 390                 395                 400

Arg Leu Arg Lys Leu Trp Pro Glu Lys Tyr Gly Gly Glu Ser Met Ala
                405                 410                 415

Glu Val Arg Lys Glu Leu Glu Tyr Lys Gly Lys Lys Ile Arg Ile Val
            420                 425                 430

Lys Gly Asp Ile Thr Arg Glu Glu Val Asp Ala Ile Val Asn Ala Ala
        435                 440                 445

Asn Glu Tyr Leu Lys His Gly Gly Gly Val Ala Gly Ala Ile Val Arg
    450                 455                 460

Ala Gly Gly Ser Val Ile Gln Glu Glu Ser Asp Arg Ile Val Gln Glu
465                 470                 475                 480

Arg Gly Arg Val Pro Thr Gly Glu Ala Val Val Thr Ser Ala Gly Lys
                485                 490                 495

Leu Lys Ala Lys Tyr Val Ile His Thr Val Gly Pro Val Trp Arg Gly
            500                 505                 510

Gly Ser His Gly Glu Asp Glu Leu Leu Tyr Lys Ala Val Tyr Asn Ala
        515                 520                 525

Leu Leu Arg Ala His Glu Leu Lys Leu Lys Ser Ile Ser Met Pro Ala
    530                 535                 540

Ile Ser Thr Gly Ile Phe Gly Phe Pro Lys Glu Arg Ala Val Gly Ile
545                 550                 555                 560

Phe Ser Lys Ala Ile Arg Asp Phe Ile Asp Gln His Pro Asp Thr Thr
                565                 570                 575

Leu Glu Glu Ile Arg Ile Cys Asn Ile Asp Glu Glu Thr Thr Lys Ile
            580                 585                 590

Phe Glu Glu Lys Phe Ser Val
        595

<210> 218
<211> 2640
<212> DNA
<213> Thermotoga maritima

<400> 218
caagacagga tccgtacggt gaagcgggcg tggtggccat cttcactgaa aggatgctca 60

ggggagagga agttcacata ttcggtgatg gagagtacgt cagagactac gtttacgttg 120
atgacgtggt cagagcgaat cttctcgcca tggagaaagg cgacaacgaa gtcttcaaca 180
taggaacggg aaggggcaca accgtgaacc agctcttcaa actgctgaaa gagatcactg 240
gctacgataa agagcctgtc tataaaccac cgcgtaaggg cgacgtgaga aagagcattc 300
tcgattacac gaaggcgaag gaaaagctcg gctgggaacc caaagtttcc cttgaggagg 360
gattgaagct caccgttgag tatttcagaa aaacccttga gtgaactgct caggccgaaa 420
gacttcgagg atttcgtcgg tcaggatcat atattcggta ataaagggat tctccggcga 480
actttaaaga caggcaacat gttctcttct atcctctatg gaccaccggg gtctggtaag 540
acctcggttt tttcactgct gaaaaggtat ttcaacggcg aggtagttta tctgagctcc 600
accgttcacg gggtttctga aataaagaac gttctcaaaa gaggagaaca gttgagaaaa 660
tatggaaaaa aattgcttct ctttctcgat gaaatacacc gcctgaacaa aaaccagcag 720
atggttctgg tttcccatgt tgaacgagga gacatcgtac tggttgcgac aacaactgaa 780
aatccgagtt ttgtcatcgt acctgcactt ctctcaaggt gcaggattct ttatttcaaa 840
aaactctctg acgaggacct gatgaagatc ttgaaaaaag caacagaggt tctcaacatc 900
gatctggaag aatcggtaga gaaagccata gtaaggcatt ccgaaggaga cgccagaaaa 960
ctcttgaaca ccctggagat cgtccaccag gcgttcaaaa acaagagagt aactcttgag 1020
gacctggaaa ctctgctggg aaacgtgagc gggtacacga aggaatcaca ctacgatttc 1080
gcttcggcct tcataaagag tatgagaggt agcgatccaa acgccgctgt ttactacctc 1140
gtcaaaatga tagagatggg agaagacccg cgattcatag cacgaagaat gatcatattc 1200
gccagcgaag acgttggact cgccgaccca aacgctctac atatcgccgt ttcgacctcc 1260
atcgctgtcg aacacgtggg acttcccgaa tgtttgatga accttgtaga gtgtgccgtc 1320
tatctttccc tcgctcccaa gagcaactct gtttatctcg caatgaaaaa agtccaggaa 1380
cttcctgtgg aggacgtacc gctttttctg agaaatcccg tcaccgaaga gatgaaaaag 1440
cgtggatacg gagaagggta tctttatccc catgatttcg gtggtttcgt gaaaacgaac 1500
tatcttccag aaaagttgaa aaatgaggtc atcttccagc caaagagagt aggttttgag 1560
gaagaactct ttgaaaggct cagaaaactc tggcccgaga agtacggggg tgaaagtatg 1620
gccgaagtga gaaaagaact ggaatacaaa gggaaaaaga tcagaatcgt aaagggagac 1680
atcacaagag aagaagtgga cgccatagtg aacgcggcga acgaatatct gaaacacgga 1740
ggaggagtag cgggagcgat cgtgagagca ggtggaagcg ttatccagga agaaagcgac 1800
aggatcgttc aagaacgagg aagagtcccg acaggtgaag cggtggtaac cagcgctgga 1860
aagctgaagg caaaatacgt gatccacacc gtggggcccg tttggagagg aggcagtcat 1920
ggagaggacg aacttctcta caaagcagtt tacaacgctc ttcttcgagc tcacgaactg 1980
aaattgaaga gcatctcaat gcctgctatc agtacaggaa tattcggatt tccgaaagaa 2040
agagcggtgg gaatcttttc aaaagcaata agagatttca tcgatcaaca tcctgatacc 2100

actctggaag agatccgcat atgcaacata gatgaggaaa caacgaagat tttcgaagaa 2160
aagttcagcg tttgatgaaa ccaaaagaag gggcaaacgc cccttctttt ttaagacctc 2220
tgaggaagta ttgaatcaac tcaaacgagt tcttggacct cttccgcaac agatttccat 2280
acctgaaagg aactattgat attttcatca taccacaaaa actaaaaaag taaagggggc 2340
caagggcccc ctttatttcc atcagaattc ttcaggcatg ctcggtgttt ctttcttctc 2400
ctcgggtttt tcaacgatga gcacttccgt tgtcaggagc attccagcga tggatgcagc 2460
attctgcagg gcgctccttg taacctttgc tgggtcaatg attcctcttt caaacatgtt 2520
gcagtattct cctctgagtg cgtcgaatcc ataggctgga tcgtcattcg aaagtatctt 2580
ctcaatgatc accgctccgt cgtatcccgc gttttcagca atctgcttaa taggggctga 2640

<210> 219
<211> 312
<212> PRT
<213> Thermotoga maritima

<400> 219
Met Asn Asp Leu Ile Arg Lys Tyr Ala Lys Asp Gln Leu Glu Thr Leu
1               5                   10                  15

Lys Arg Ile Ile Glu Lys Ser Glu Gly Ile Ser Ile Leu Ile Asn Gly
            20                  25                  30

Glu Asp Leu Ser Tyr Pro Arg Glu Val Ser Leu Glu Leu Pro Glu Tyr
        35                  40                  45

Val Glu Lys Phe Pro Pro Lys Ala Ser Asp Val Leu Glu Ile Asp Pro
    50                  55                  60

Glu Gly Glu Asn Ile Gly Ile Asp Asp Ile Arg Thr Ile Lys Asp Phe
65                  70                  75                  80

Leu Asn Tyr Ser Pro Glu Leu Tyr Thr Arg Lys Tyr Val Ile Val His
                85                  90                  95

Asp Cys Glu Arg Met Thr Gln Gln Ala Ala Asn Ala Phe Leu Lys Ala
            100                 105                 110

Leu Glu Glu Pro Pro Glu Tyr Ala Val Ile Val Leu Asn Thr Arg Arg
        115                 120                 125

Trp His Tyr Leu Leu Pro Thr Ile Lys Ser Arg Val Phe Arg Val Val
    130                 135                 140

Val Asn Val Pro Lys Glu Phe Arg Asp Leu Val Lys Glu Lys Ile Gly
145                 150                 155                 160

Asp Leu Trp Glu Glu Leu Pro Leu Leu Glu Arg Asp Phe Lys Thr Ala
                165                 170                 175

Leu Glu Ala Tyr Lys Leu Gly Ala Glu Lys Leu Ser Gly Leu Met Glu
            180                 185                 190

Ser Leu Lys Val Leu Glu Thr Glu Lys Leu Leu Lys Lys Val Leu Ser
        195                 200                 205

Lys Gly Leu Glu Gly Tyr Leu Ala Cys Arg Glu Leu Leu Glu Arg Phe
    210                 215                 220

Ser Lys Val Glu Ser Lys Glu Phe Phe Ala Leu Phe Asp Gln Val Thr

225                 230                 235                 240

Asn Thr Ile Thr Gly Lys Asp Ala Phe Leu Leu Ile Gln Arg Leu Thr
                245                 250                 255

Arg Ile Ile Leu His Glu Asn Thr Trp Glu Ser Val Glu Asp Gln Lys
            260                 265                 270

Ser Val Ser Phe Leu Asp Ser Ile Leu Arg Val Lys Ile Ala Asn Leu
        275                 280                 285

Asn Asn Lys Leu Thr Leu Met Asn Ile Leu Ala Ile His Arg Glu Arg
    290                 295                 300

Lys Arg Gly Val Asn Ala Trp Ser
305                 310

<210> 220
<211> 1980
<212> DNA
<213> Thermotoga maritima

<400> 220
ggaagatagc tccgctccgc ccaaacgtcc ggttgctcgt tcagaatgtg gtacagtatc 60

tcaagaccgt ggtttgaagt acctacctcg taagtgtcgg gaaaaacgag ggctatccgg 120

agggaaattt cctctggatc tttcacaacg ctgttgattt ctcttcctat gtatctactt 180

ggttttctaa cccagggaag tgtttcattc aagaatttta gaatcatcct ttcacctcag 240

ttctgatttt atcattggtt gttataatta gaaggcgagg tgagatcatg aacgatttga 300

tcagaaagta cgctaaagat caactggaaa ctttgaaaag gatcatagaa aagtctgaag 360

gaatatccat cctcataaat ggagaagatc tctcgtatcc gagagaagta tcccttgaac 420

ttcccgagta cgtggagaaa tttcccccga aggcctcgga tgttctggag atagatcccg 480

agggggagaa cataggcata gacgacatca gaacgataaa ggacttcctg aactacagcc 540

ccgagctcta cacgagaaag tacgtgatag tccacgactg tgaaagaatg acccagcagg 600

cggcgaacgc gtttctgaag gcccttgaag aaccaccaga atacgctgtg atcgttctga 660

acactcgccg ctggcattat ctactgccga cgataaagag ccgagtgttc agagtggttg 720

tgaacgttcc aaaggagttc agagatctcg tgaaagagaa aataggagat ctctgggagg 780

aacttccact tcttgagaga gacttcaaaa cggctctcga agcctacaaa cttggtgcgg 840

aaaaactttc tggattgatg gaaagtctca aagttttgga gacggaaaaa ctcttgaaaa 900

aggtcctttc aaaaggcctc gaaggttatc tcgcatgtag ggagctcctg gagagatttt 960

caaaggtgga atcgaaggaa ttctttgcgc tttttgatca ggtgactaac acgataacag 1020

gaaaagacgc gtttcttttg atccagagac tgacaagaat cattctccac gaaaacacat 1080

gggaaagcgt tgaagatcaa aaaagcgtgt ctttcctcga ttcaattctc agggtgaaga 1140

tagcgaatct gaacaacaaa ctcactctga tgaacatcct cgcgatacac agagagagaa 1200

agagaggtgt caacgcttgg agctgaaggc aagggcagtg ggagtggaga taataccaaa 1260

gggtaagata atttactact ctgtacccaa cggtgatgag tacgagagag gagacctcgt 1320

tctggtactg ggagattttg gacttgaagt gggaaaggtt ctgatacctc ccagagaggt 1380

ggtcatagac gaggtggggt acgaactcaa ggccgtgatc agaaagttga cggatgaaga 1440

tctggaacaa tacaggaaga atgtagagga tgcgttgaag gccttccaga tatgtaagca 1500

gaaaataaaa gaacacggtc ttcccatgaa gcttctctac gccaagtaca ccttcgatag 1560

aactaggctc atctttttct tcagtgcgga aggaagggtt gatttcagag agcttgtaag 1620

agacctcgca aagatcttca aaacaaggat agagttgagg caagttggtg tgagagatga 1680

gatgaagttc tttggaggcc ttggattgtg cggacttccg acatgctgtt ccacatttct 1740

cagagagttc accagcgtca cactgaagca cgcaaagaag cagcagatga tgatcaatcc 1800

agcgaagata tccggaccat gcagaaggct tctgtgctgt cttacctacg aatacgattt 1860

ctatgaaaag gagctggagg gaattcctga cgaagggagc acgatcacct acgaaggaaa 1920

gcgctacaaa gtcgtgaacg tgaacgtgtt tctcagaacg gtgactctct tctcggaaca 1980

<210> 221
<211> 6
<212> PRT
<213> Artificial Sequence

<220>
<223> Description of Artificial Sequence: Consensus
      sequence

<220>
<221> UNSURE
<222> (6)
<223> Xaa at position 6 is Thr or Ser

<220>
<221> UNSURE
<222> (2)
<223> Xaa at position 2 is any amino acid

<400> 221
Gly Xaa Gly Lys Thr Xaa
  1               5

<210> 222
<211> 11
<212> PRT
<213> Artificial Sequence

<220>
<223> Description of Artificial Sequence: Consensus
      sequence

<400> 222
Asn Ala Leu Leu Lys Thr Leu Glu Glu Pro Pro
  1               5                  10

<210> 223
<211> 996
<212> DNA
<213> Mycobacterium tuberculosis

<400> 223
gtgagcgagg ctaagccgtt gcacctggtg agcgaggcta agccgttgca cctggtcctg 60

ggagacgaag aactgctggt cgaaagggcg gtggccgacg tgttgcgctc ggctcggcag 120

cgggcaggta cagccgacgt cccggtgagc cgaatgcgcg cgggtgacgt cggtgcctat 180

gagctcgccg aactgctgag cccgtcactg ttcgccgagg agcggatcgt tgtgctgggg 240

gccgctgcgg aggcgggcaa ggacgctgcc gcggtaatcg agtcggccgc cgccgatctt 300

ccggccggca ccgtgctggt agtggtccac tcgggtggcg ggcgcgccaa atcgctggcc 360

aaccagctgc ggtcgatggg tgcgcaggtt catccgtgcg cgcggatcac caaggtcagt 420

gagcgcgccg acttcatccg tagcgagttc gcgtcgctgc gggtcaaggt cgacgacgag 480

accgtgaccg ccctgctgga cgccgtcggc tccgacgtgc gcgaactcgc ctcggcctgt 540

tcacagctgg tcgccgatac cggaggagcc gtcgacgccg ccgctgtacg gcgctatcac 600

agcggcaaag ccgaggtgag gggcttcgac atcgccgaca aggcggtagc cggcgacgtg 660

gcgggagctg ccgaagcgtt gcggtgggcg atgatgcgcg gtgagccgct agtggtgttg 720

gccgatgcgc tcgccgaagc cgtgcacacc atcggccggg tcgggccgca gtccggcgac 780

ccgtaccgcc tggccgcaca actggggatg ccgccctggc gggtgcagaa agcccagaag 840

caggctcggc ggtggtcgcg tgacacggtg gcgaccgcga tgaggttggt ggccgaactc 900

aatgctaacg tcaagggcgc cgtcgcggat gcggactacg cgctggaatc cgcggtccgg 960

caggtcgccg agttggtggc cgaccgcggc cgatga 996

<210> 224
<211> 996
<212> DNA
<213> Mycobacterium tuberculosis

<220>
<221> CDS
<222> (1)..(996)

<400> 224
gtg agc gag gct aag ccg ttg cac ctg gtg agc gag gct aag ccg ttg 48
Val Ser Glu Ala Lys Pro Leu His Leu Val Ser Glu Ala Lys Pro Leu
1               5                   10                  15

cac ctg gtc ctg gga gac gaa gaa ctg ctg gtc gaa agg gcg gtg gcc 96
His Leu Val Leu Gly Asp Glu Glu Leu Leu Val Glu Arg Ala Val Ala
            20                  25                  30

gac gtg ttg cgc tcg gct cgg cag cgg gca ggt aca gcc gac gtc ccg 144
Asp Val Leu Arg Ser Ala Arg Gln Arg Ala Gly Thr Ala Asp Val Pro
        35                  40                  45

gtg agc cga atg cgc gcg ggt gac gtc ggt gcc tat gag ctc gcc gaa 192
Val Ser Arg Met Arg Ala Gly Asp Val Gly Ala Tyr Glu Leu Ala Glu
    50                  55                  60

ctg ctg agc ccg tca ctg ttc gcc gag gag cgg atc gtt gtg ctg ggg 240
Leu Leu Ser Pro Ser Leu Phe Ala Glu Glu Arg Ile Val Val Leu Gly
65                  70                  75                  80

gcc gct gcg gag gcg ggc aag gac gct gcc gcg gta atc gag tcg gcc 288
Ala Ala Ala Glu Ala Gly Lys Asp Ala Ala Ala Val Ile Glu Ser Ala
                85                  90                  95

gcc gcc gat ctt ccg gcc ggc acc gtg ctg gta gtg gtc cac tcg ggt     336
Ala Ala Asp Leu Pro Ala Gly Thr Val Leu Val Val Val His Ser Gly
            100                 105                 110

ggc ggg cgc gcc aaa tcg ctg gcc aac cag ctg cgg tcg atg ggt gcg     384
Gly Gly Arg Ala Lys Ser Leu Ala Asn Gln Leu Arg Ser Met Gly Ala
        115                 120                 125

cag gtt cat ccg tgc gcg cgg atc acc aag gtc agt gag cgc gcc gac     432
Gln Val His Pro Cys Ala Arg Ile Thr Lys Val Ser Glu Arg Ala Asp
    130                 135                 140

ttc atc cgt agc gag ttc gcg tcg ctg cgg gtc aag gtc gac gac gag     480
Phe Ile Arg Ser Glu Phe Ala Ser Leu Arg Val Lys Val Asp Asp Glu
145                 150                 155                 160

acc gtg acc gcc ctg ctg gac gcc gtc ggc tcc gac gtg cgc gaa ctc     528
Thr Val Thr Ala Leu Leu Asp Ala Val Gly Ser Asp Val Arg Glu Leu
                165                 170                 175

gcc tcg gcc tgt tca cag ctg gtc gcc gat acc gga gga gcc gtc gac     576
Ala Ser Ala Cys Ser Gln Leu Val Ala Asp Thr Gly Gly Ala Val Asp
            180                 185                 190

gcc gcc gct gta cgg cgc tat cac agc ggc aaa gcc gag gtg agg ggc     624
Ala Ala Ala Val Arg Arg Tyr His Ser Gly Lys Ala Glu Val Arg Gly
        195                 200                 205

ttc gac atc gcc gac aag gcg gta gcc ggc gac gtg gcg gga gct gcc     672
Phe Asp Ile Ala Asp Lys Ala Val Ala Gly Asp Val Ala Gly Ala Ala
    210                 215                 220

gaa gcg ttg cgg tgg gcg atg atg cgc ggt gag ccg cta gtg gtg ttg     720
Glu Ala Leu Arg Trp Ala Met Met Arg Gly Glu Pro Leu Val Val Leu
225                 230                 235                 240

gcc gat gcg ctc gcc gaa gcc gtg cac acc atc ggc cgg gtc ggg ccg     768
Ala Asp Ala Leu Ala Glu Ala Val His Thr Ile Gly Arg Val Gly Pro
                245                 250                 255

cag tcc ggc gac ccg tac cgc ctg gcc gca caa ctg ggg atg ccg ccc     816
Gln Ser Gly Asp Pro Tyr Arg Leu Ala Ala Gln Leu Gly Met Pro Pro
            260                 265                 270

tgg cgg gtg cag aaa gcc cag aag cag gct cgg cgg tgg tcg cgt gac     864
Trp Arg Val Gln Lys Ala Gln Lys Gln Ala Arg Arg Trp Ser Arg Asp
        275                 280                 285

acg gtg gcg acc gcg atg agg ttg gtg gcc gaa ctc aat gct aac gtc     912
Thr Val Ala Thr Ala Met Arg Leu Val Ala Glu Leu Asn Ala Asn Val
    290                 295                 300

aag ggc gcc gtc gcg gat gcg gac tac gcg ctg gaa tcc gcg gtc cgg     960
Lys Gly Ala Val Ala Asp Ala Asp Tyr Ala Leu Glu Ser Ala Val Arg
305                 310                 315                 320

cag gtc gcc gag ttg gtg gcc gac cgc ggc cga tga                     996
Gln Val Ala Glu Leu Val Ala Asp Arg Gly Arg
                325                 330

<210> 225
<211> 331
<212> PRT
<213> Mycobacterium tuberculosis

<400> 225
Val Ser Glu Ala Lys Pro Leu His Leu Val Ser Glu Ala Lys Pro Leu

1                   5                   10                  15

His Leu Val Leu Gly Asp Glu Glu Leu Leu Val Glu Arg Ala Val Ala
                20                  25                  30

Asp Val Leu Arg Ser Ala Arg Gln Arg Ala Gly Thr Ala Asp Val Pro
            35                  40                  45

Val Ser Arg Met Arg Ala Gly Asp Val Gly Ala Tyr Glu Leu Ala Glu
    50                  55                  60

Leu Leu Ser Pro Ser Leu Phe Ala Glu Glu Arg Ile Val Val Leu Gly
65                  70                  75                  80

Ala Ala Ala Glu Ala Gly Lys Asp Ala Ala Ala Val Ile Glu Ser Ala
                85                  90                  95

Ala Ala Asp Leu Pro Ala Gly Thr Val Leu Val Val Val His Ser Gly
            100                 105                 110

Gly Gly Arg Ala Lys Ser Leu Ala Asn Gln Leu Arg Ser Met Gly Ala
        115                 120                 125

Gln Val His Pro Cys Ala Arg Ile Thr Lys Val Ser Glu Arg Ala Asp
    130                 135                 140

Phe Ile Arg Ser Glu Phe Ala Ser Leu Arg Val Lys Val Asp Asp Glu
145                 150                 155                 160

Thr Val Thr Ala Leu Leu Asp Ala Val Gly Ser Asp Val Arg Glu Leu
                165                 170                 175

Ala Ser Ala Cys Ser Gln Leu Val Ala Asp Thr Gly Gly Ala Val Asp
            180                 185                 190

Ala Ala Ala Val Arg Arg Tyr His Ser Gly Lys Ala Glu Val Arg Gly
        195                 200                 205

Phe Asp Ile Ala Asp Lys Ala Val Ala Gly Asp Val Ala Gly Ala Ala
    210                 215                 220

Glu Ala Leu Arg Trp Ala Met Met Arg Gly Glu Pro Leu Val Val Leu
225                 230                 235                 240

Ala Asp Ala Leu Ala Glu Ala Val His Thr Ile Gly Arg Val Gly Pro
                245                 250                 255

Gln Ser Gly Asp Pro Tyr Arg Leu Ala Ala Gln Leu Gly Met Pro Pro
            260                 265                 270

Trp Arg Val Gln Lys Ala Gln Lys Gln Ala Arg Arg Trp Ser Arg Asp
        275                 280                 285

Thr Val Ala Thr Ala Met Arg Leu Val Ala Glu Leu Asn Ala Asn Val
    290                 295                 300

Lys Gly Ala Val Ala Asp Ala Asp Tyr Ala Leu Glu Ser Ala Val Arg
305                 310                 315                 320

Gln Val Ala Glu Leu Val Ala Asp Arg Gly Arg
                325                 330

<210> 226
<211> 350
<212> PRT
<213> Aquifex aeolicus

<400> 226
Met Glu Thr Thr Ile Phe Gln Phe Gln Lys Thr Phe Phe Thr Lys Pro
1               5                   10                  15

Pro Lys Glu Arg Val Phe Val Leu His Gly Glu Glu Gln Tyr Leu Ile
            20                  25                  30

Arg Thr Phe Leu Ser Lys Leu Lys Glu Lys Tyr Gly Glu Asn Tyr Thr
        35                  40                  45

Val Leu Trp Gly Asp Glu Ile Ser Glu Glu Glu Phe Tyr Thr Ala Leu
    50                  55                  60

Ser Glu Thr Ser Ile Phe Gly Gly Ser Lys Glu Lys Ala Val Val Ile
65                  70                  75                  80

Tyr Asn Phe Gly Asp Phe Leu Lys Lys Leu Gly Arg Lys Lys Lys Glu
                85                  90                  95

Lys Glu Arg Leu Ile Lys Val Leu Arg Asn Val Lys Ser Asn Tyr Val
            100                 105                 110

Phe Ile Val Tyr Asp Ala Lys Leu Gln Lys Gln Glu Leu Ser Ser Glu
        115                 120                 125

Pro Leu Lys Ser Val Ala Ser Phe Gly Gly Ile Val Val Ala Asn Arg
    130                 135                 140

Leu Ser Lys Glu Arg Ile Lys Gln Leu Val Leu Lys Lys Phe Lys Glu
145                 150                 155                 160

Lys Gly Ile Asn Val Glu Asn Asp Ala Leu Glu Tyr Leu Leu Gln Leu
                165                 170                 175

Thr Gly Tyr Asn Leu Met Glu Leu Lys Leu Glu Val Glu Lys Leu Ile
            180                 185                 190

Asp Tyr Ala Ser Glu Lys Lys Ile Leu Thr Leu Asp Glu Val Lys Arg
        195                 200                 205

Val Ala Phe Ser Val Ser Glu Asn Val Asn Val Phe Glu Phe Val Asp
    210                 215                 220

Leu Leu Leu Leu Lys Asp Tyr Glu Lys Ala Leu Lys Val Leu Asp Ser
225                 230                 235                 240

Leu Ile Ser Phe Gly Ile His Pro Leu Gln Ile Met Lys Ile Leu Ser
                245                 250                 255

Ser Tyr Ala Leu Lys Leu Tyr Thr Leu Lys Arg Leu Glu Glu Lys Gly
            260                 265                 270

Glu Asp Leu Asn Lys Ala Met Glu Ser Val Gly Ile Lys Asn Asn Phe
        275                 280                 285

Leu Lys Met Lys Phe Lys Ser Tyr Leu Lys Ala Asn Ser Lys Glu Asp
    290                 295                 300

Leu Lys Asn Leu Ile Leu Ser Leu Gln Arg Ile Asp Ala Phe Ser Lys
305                 310                 315                 320

Leu Tyr Phe Gln Asp Thr Val Gln Leu Leu Arg Asp Phe Leu Thr Ser
                325                 330                 335

Arg Leu Glu Arg Glu Val Val Lys Asn Thr Ser His Gly Gly
            340                 345                 350

<210> 227
<211> 316
<212> PRT
<213> Mycobacterium tuberculosis

<400> 227
Met His Leu Val Leu Gly Asp Glu Glu Leu Leu Val Glu Arg Ala Val
1               5                   10                  15

Ala Asp Val Leu Arg Ser Ala Arg Gln Arg Ala Gly Thr Ala Asp Val
            20                  25                  30

Pro Val Ser Arg Met Arg Ala Gly Asp Val Gly Ala Tyr Glu Leu Ala
        35                  40                  45

Glu Leu Leu Ser Pro Ser Leu Phe Ala Glu Glu Arg Ile Val Val Leu
    50                  55                  60

Gly Ala Ala Ala Glu Ala Gly Lys Asp Ala Ala Ala Val Ile Glu Ser
65                  70                  75                  80

Ala Ala Ala Asp Leu Pro Ala Gly Thr Val Leu Val Val Val His Ser
                85                  90                  95

Gly Gly Gly Arg Ala Lys Ser Leu Ala Asn Gln Leu Arg Ser Met Gly
            100                 105                 110

Ala Gln Val His Pro Cys Ala Arg Ile Thr Lys Val Ser Glu Arg Ala
        115                 120                 125

Asp Phe Ile Arg Ser Glu Phe Ala Ser Leu Arg Val Lys Val Asp Asp
    130                 135                 140

Glu Thr Val Thr Ala Leu Leu Asp Ala Val Gly Ser Asp Val Arg Glu
145                 150                 155                 160

Leu Ala Ser Ala Cys Ser Gln Leu Val Ala Asp Thr Gly Gly Ala Val
                165                 170                 175

Asp Ala Ala Ala Val Arg Arg Tyr His Ser Gly Lys Ala Glu Val Arg
            180                 185                 190

Gly Phe Asp Ile Ala Asp Lys Ala Val Ala Gly Asp Val Ala Gly Ala
        195                 200                 205

Ala Glu Ala Leu Arg Trp Ala Met Met Arg Gly Glu Pro Leu Val Val
    210                 215                 220

Leu Ala Asp Ala Leu Ala Glu Ala Val His Thr Ile Gly Arg Val Gly
225                 230                 235                 240

Pro Gln Ser Gly Asp Pro Tyr Arg Leu Ala Ala Gln Leu Gly Met Pro
                245                 250                 255

Pro Trp Arg Val Gln Lys Ala Gln Lys Gln Ala Arg Arg Trp Ser Arg
            260                 265                 270

Asp Thr Val Ala Thr Ala Met Arg Leu Val Ala Glu Leu Asn Ala Asn
        275                 280                 285

Val Lys Gly Ala Val Ala Asp Ala Asp Tyr Ala Leu Glu Ser Ala Val
    290                 295                 300

Arg Gln Val Ala Glu Leu Val Ala Asp Arg Gly Arg
305                 310                 315

<210> 228

<211> 322
<212> PRT
<213> Mycobacterium tuberculosis

<400> 228
Met Ser Glu Ala Lys Pro Leu His Leu Val Leu Gly Asp Glu Glu Leu
1               5                   10                  15

Leu Val Glu Arg Ala Val Ala Asp Val Leu Arg Ser Ala Arg Gln Arg
            20                  25                  30

Ala Gly Thr Ala Asp Val Pro Val Ser Arg Met Arg Ala Gly Asp Val
        35                  40                  45

Gly Ala Tyr Glu Leu Ala Glu Leu Leu Ser Pro Ser Leu Phe Ala Glu
    50                  55                  60

Glu Arg Ile Val Val Leu Gly Ala Ala Ala Glu Ala Gly Lys Asp Ala
65                  70                  75                  80

Ala Ala Val Ile Glu Ser Ala Ala Ala Asp Leu Pro Ala Gly Thr Val
                85                  90                  95

Leu Val Val Val His Ser Gly Gly Gly Arg Ala Lys Ser Leu Ala Asn
            100                 105                 110

Gln Leu Arg Ser Met Gly Ala Gln Val His Pro Cys Ala Arg Ile Thr
        115                 120                 125

Lys Val Ser Glu Arg Ala Asp Phe Ile Arg Ser Glu Phe Ala Ser Leu
    130                 135                 140

Arg Val Lys Val Asp Asp Glu Thr Val Thr Ala Leu Leu Asp Ala Val
145                 150                 155                 160

Gly Ser Asp Val Arg Glu Leu Ala Ser Ala Cys Ser Gln Leu Val Ala
                165                 170                 175

Asp Thr Gly Gly Ala Val Asp Ala Ala Ala Val Arg Arg Tyr His Ser
            180                 185                 190

Gly Lys Ala Glu Val Arg Gly Phe Asp Ile Ala Asp Lys Ala Val Ala
        195                 200                 205

Gly Asp Val Ala Gly Ala Ala Glu Ala Leu Arg Trp Ala Met Met Arg
    210                 215                 220

Gly Glu Pro Leu Val Val Leu Ala Asp Ala Leu Ala Glu Ala Val His
225                 230                 235                 240

Thr Ile Gly Arg Val Gly Pro Gln Ser Gly Asp Pro Tyr Arg Leu Ala
                245                 250                 255

Ala Gln Leu Gly Met Pro Pro Trp Arg Val Gln Lys Ala Gln Lys Gln
            260                 265                 270

Ala Arg Arg Trp Ser Arg Asp Thr Val Ala Thr Ala Met Arg Leu Val
        275                 280                 285

Ala Glu Leu Asn Ala Asn Val Lys Gly Ala Val Ala Asp Ala Asp Tyr
    290                 295                 300

Ala Leu Glu Ser Ala Val Arg Gln Val Ala Glu Leu Val Ala Asp Arg
305                 310                 315                 320

Gly Arg

<210> 229
<211> 336
<212> PRT
<213> Streptomyces coelicolor

<400> 229
Met Arg Ala Met Leu Val Ala Met Ala Arg Lys Thr Ala Asn Asp Asp
1               5                   10                  15

Pro Leu Ala Pro Val Thr Leu Ala Val Gly Gln Glu Asp Leu Leu Leu
            20                  25                  30

Asp Arg Ala Val Gln Glu Val Val Ala Ala Ala Lys Ala Ala Asp Ala
        35                  40                  45

Asp Thr Asp Val Arg Asp Leu Thr Pro Asp Gln Leu Gln Pro Gly Thr
    50                  55                  60

Leu Ala Glu Leu Thr Ser Pro Ser Leu Phe Ala Glu Arg Lys Val Val
65                  70                  75                  80

Val Val Arg Asn Ala Gln Asp Leu Ser Ala Asp Thr Val Lys Asp Val
                85                  90                  95

Lys Ala Tyr Leu Gly Ala Pro Ala Glu Glu Ile Thr Leu Val Leu Leu
            100                 105                 110

His Ala Gly Gly Ala Lys Gly Lys Gly Val Leu Asp Ala Gly Arg Lys
        115                 120                 125

Ala Gly Ala Arg Glu Val Ala Cys Pro Lys Met Thr Lys Pro Ala Asp
    130                 135                 140

Arg Leu Ala Phe Val Arg Ala Glu Phe Arg Thr Ala Gly Arg Ser Ala
145                 150                 155                 160

Thr Pro Glu Ala Cys Gln Ala Leu Val Asp Ala Ile Gly Ser Asp Leu
                165                 170                 175

Arg Glu Leu Ala Ser Ala Val Ser Gln Leu Thr Ala Asp Val Glu Gly
            180                 185                 190

Thr Val Asp Glu Ala Val Val Gly Arg Tyr Tyr Thr Gly Arg Ala Glu
        195                 200                 205

Ala Ser Ser Phe Thr Val Ala Asp Arg Ala Val Glu Gly Arg Ala Ala
    210                 215                 220

Glu Ala Leu Glu Ala Leu Arg Trp Ser Leu Ala Thr Gly Val Ala Pro
225                 230                 235                 240

Val Leu Ile Thr Ser Ala Leu Ala Gln Gly Val Arg Ala Ile Gly Lys
                245                 250                 255

Leu Ser Ser Ala Arg Gly Gly Arg Pro Ala Asp Leu Ala Arg Glu Leu
            260                 265                 270

Gly Met Pro Pro Trp Lys Ile Asp Arg Val Arg Gln Gln Met Arg Gly
        275                 280                 285

Trp Thr Pro Asp Gly Val Ser Val Ala Leu Arg Ala Val Ala Glu Ala
    290                 295                 300

Asp Ala Gly Val Lys Gly Gly Gly Asp Asp Pro Glu Tyr Ala Leu Glu
305                 310                 315                 320

Lys Ala Val Val Ile Ile Ala Arg Ala Ala Arg Ser Arg Gly Arg Thr

325                 330                 335

<210> 230
<211> 478
<212> PRT
<213> Thermotoga maritima

<400> 230
Met Glu Val Leu Tyr Arg Lys Tyr Arg Pro Lys Thr Phe Ser Glu Val
1               5                   10                  15

Val Asn Gln Asp His Val Lys Lys Ala Ile Ile Gly Ala Ile Gln Lys
            20                  25                  30

Asn Ser Val Ala His Gly Tyr Ile Phe Ala Gly Pro Arg Gly Thr Gly
        35                  40                  45

Lys Thr Thr Leu Ala Arg Ile Leu Ala Lys Ser Leu Asn Cys Glu Asn
    50                  55                  60

Arg Lys Gly Val Glu Pro Cys Asn Ser Cys Arg Ala Cys Arg Glu Ile
65                  70                  75                  80

Asp Glu Gly Thr Phe Met Asp Val Ile Glu Leu Asp Ala Ala Ser Asn
                85                  90                  95

Arg Gly Ile Asp Glu Ile Arg Arg Ile Arg Asp Ala Val Gly Tyr Arg
            100                 105                 110

Pro Met Glu Gly Lys Tyr Lys Val Tyr Ile Ile Asp Glu Val His Met
        115                 120                 125

Leu Thr Lys Glu Ala Phe Asn Ala Leu Leu Lys Thr Leu Glu Glu Pro
    130                 135                 140

Pro Ser His Val Val Phe Val Leu Ala Thr Thr Asn Leu Glu Lys Val
145                 150                 155                 160

Pro Pro Thr Ile Ile Ser Arg Cys Gln Val Phe Glu Phe Arg Asn Ile
                165                 170                 175

Pro Asp Glu Leu Ile Glu Lys Arg Leu Gln Glu Val Ala Glu Ala Glu
            180                 185                 190

Gly Ile Glu Ile Asp Arg Glu Ala Leu Ser Phe Ile Ala Lys Arg Ala
        195                 200                 205

Ser Gly Gly Leu Arg Asp Ala Leu Thr Met Leu Glu Gln Val Trp Lys
    210                 215                 220

Phe Ser Glu Gly Lys Ile Asp Leu Glu Thr Val His Arg Ala Leu Gly
225                 230                 235                 240

Leu Ile Pro Ile Gln Val Val Arg Asp Tyr Val Asn Ala Ile Phe Ser
                245                 250                 255

Gly Asp Val Lys Arg Val Phe Thr Val Leu Asp Asp Val Tyr Tyr Ser
            260                 265                 270

Gly Lys Asp Tyr Glu Val Leu Ile Gln Glu Ala Val Glu Asp Leu Val
        275                 280                 285

Glu Asp Leu Glu Arg Glu Arg Gly Val Tyr Gln Val Ser Ala Asn Asp
    290                 295                 300

Ile Val Gln Val Ser Arg Gln Leu Leu Asn Leu Leu Arg Glu Ile Lys
305                 310                 315                 320

Phe Ala Glu Glu Lys Arg Leu Val Cys Lys Val Gly Ser Ala Tyr Ile
                325                 330                 335

Ala Thr Arg Phe Ser Thr Thr Asn Val Gln Glu Asn Asp Val Arg Glu
            340                 345                 350

Lys Asn Asp Asn Ser Asn Val Gln Gln Lys Glu Glu Lys Lys Glu Thr
        355                 360                 365

Val Lys Ala Lys Glu Glu Lys Gln Glu Asp Ser Glu Phe Glu Lys Arg
    370                 375                 380

Phe Lys Glu Leu Met Glu Glu Leu Lys Glu Lys Gly Asp Leu Ser Ile
385                 390                 395                 400

Phe Val Ala Leu Ser Leu Ser Glu Val Gln Phe Asp Gly Glu Lys Val
                405                 410                 415

Ile Ile Ser Phe Asp Ser Ser Lys Ala Met His Tyr Glu Leu Met Lys
            420                 425                 430

Lys Lys Leu Pro Glu Leu Glu Asn Ile Phe Ser Arg Lys Leu Gly Lys
        435                 440                 445

Lys Val Glu Val Glu Leu Arg Leu Met Gly Lys Glu Glu Thr Ile Glu
    450                 455                 460

Lys Val Ser Gln Lys Ile Leu Arg Leu Phe Glu Gln Glu Gly
465                 470                 475

## Description

### FIELD OF THE INVENTION

The present invention relates to gene and amino acid sequences encoding DNA polymerase III holoenzyme δ subunits and structural genes from bacteria. The present invention also provides antibodies and other reagents useful to identify DNA polymerase III δ subunit molecules. The present invention also provides methods for various uses of δ subunit molecules, including their use in DNA replication and in the identification of modulators of DNA replication. The present invention also provides δ proteins of thermophilic organisms for use in for DNA synthesis and amplification.

### BACKGROUND OF THE INVENTION

Like many other complex mechanisms of macromolecular synthesis, the fundamental mechanisms of DNA replication have been conserved throughout biology. The chemistry and direction of synthesis, the requirement for RNA primers, the mechanisms of semi-discontinuous replication with Okazaki fragments on the lagging strand and the need for well-defined origins are shared (Kornberg, A. and Baker, T. A. *DNA Replication,* WH Freeman and Company, New York (1992)). The basic features of the replicative apparatus are also shared. All replicases consist of a replicative polymerase that is distinguished from others primarily by its capability of participating in specific protein-protein interactions with the rest of the replicative apparatus. All prokaryotic cells have at least three polymerases; eukaryotes have at least five. Yet, only a subset of these polymerases can function as the replicase catalytic subunit (Table 1). In eukaryotes, it has been proposed that the δ polymerase is the leading strand polymerase and ∈ is the lagging (Burgers, P.M.J., J. Biol. Chem. 266:22698-22706 (1991); Nethanel, T. and Kaufmann, G., J. Virol. 64:5912-5918 (1990)), whereas, in *E. coli,* the a subunit of DNA polymerase III serves as the sole polymerization subunit. Another key replicase component is the so-called β sliding clamp that confers high processivity on the replicase. Processivity is defined as the number of nucleotides inserted per template association-catalysis-dissociation event. β consists of a bracelet-shaped molecule that clamps around DNA, permitting it to rapidly slide down DNA, but not to dissociate. The clamp contacts the polymerase by protein-protein interactions and prevents it from falling off of the template, ensuring high processivity. Two representative prokaryotic and eukaryotic sliding clamps are β and PCNA, respectively. The crystal structures of yeast PCNA and *E*. *coli β* are nearly superimposable (Kong, X.P., et al., Cell 79:1233-1243 (1994); Krishna, T.S., et al., Cell 79:1233-1243 (1994)). In both bacteria and eukaryotes, a 5-protein complex is responsible for transferring the sliding clamp onto a primer-terminus in an ATP-dependent reaction (Lee, S.H., et al., J. Biol. Chem. 266:594-602 (1991); Bunz, F., et al., Proc. Natl. Acad. Sci. U.S.A. 90:11014-11018 (1993)). Some of the subunits exhibit recognizable sequence homology between eukaryotes and both gram-negative and positive prokaryotes, and would be expected to act by a similar mechanism (Carter, J.R., et al., J. Bacteriol. 175:3812-3822 (1993); O'Donnell, M., et al., Nucleic Acids Res. 21:1-3 (1993)).

The DNA polymerase III holoenzyme is the replicative polymerase of E. coli, responsible for synthesis of the majority of the chromosome (for a review, see Kelman. Z. and ODonnell, M., Annu. Rev. Biochem. 64:171-200 (1995)). The replicative role of the enzyme has been established both by biochemical and genetic criteria. Holoenzyme was biochemically defined and purified using natural chromosomal assays. Only the holoenzyme form of DNA polymerase III efficiently replicates single-stranded bacteriophages in vitro in the presence of other known replicative proteins (Wickner, W. and Kornberg, A., Proc. Natl. Acad Sci. U.S.A. 70:3679-3683 (1973); Hurwitz, J. and Wickner, S., Proc. Natl. Acad Sci. U.S.A. 71:6-10 (1974); McHenry, C.S. and Kornberg, A., J. Biol. Chem. 252:6478-6484 (1977)). Only the holoenzyme functions in the replication of bacteriophage λ plasmids and molecules containing the *E*. *coli* replicative origin, *oriC.* The holoenzyme contains 10 subunits: α, τ, γ, β, δ, δ', ∈, ψ, χ and θ of 129,900, 71,000,47,400,40,600, 38,700, 36,900, 26,900, 16,600,15,000 and 8,800 daltons, respectively.

**Table 1 Relationships of the Components of Replicative Enzymes**

| | | | |
|---|---|---|---|
| **Component/Function** | ***E. coli*** | **Phage T4** | **Eukaryotes** |
| DNA polymerase | α | Gene 43 | polymerase δ (and ∈?) |
| Sliding Clamp | β | Gene 45 protein | PCNA |
| Clamp loading complex | DnaX, δ, δ', χ ψ | Gene 44/62 complex | Activator 1 (RFC) |
| Single-stranded binding protein | DNA SSB | Gene 32 protein | RFA |
| Primer generation | DnaG primase | Gene 61 protein | primase-α polymerase complex |

Genetic studies also support assignment of the major replicative role to the pol III holoenzyme. Temperature-sensitive mutations in *dna*E, the structural gene for the a catalytic subunit, are conditionally lethal (Gefter, M.L., et al., Proc. Natl Acad. Sci. U.S.A. 68:3150-3153 (1971)). Similarly, temperature-sensitive, conditionally lethal mutations have been isolated for the *dna*N, *dna*X and dnaQ genes that encode the β processivity factor, the DnaX protein and the ∈ proofreading exonuclease, respectively (Sakakibara, Y. and Mizukami, T., Mol. Gen. Genet. 178:541-553 (1980); Chu, H., et al., J. Bacteriol. 132:151-158 (1997); Henson, J.M., et al., Genetics 92:1041-1059 (1979); Horiuchi, T., et al., Mol. Gen. Genet. 163:277-283 (1978)). The structural genes for the final five holoenzyme subunits were identified by a reverse genetics approach (Carter, J.R, et al., (1993), ibid; Carter, J.R., et al., J. Bacteriol. 174:7013-7025 (1992); Carter, J.R., et al., Mol. Gen. Genet. 241:399-408 (1993); Carter, J.R., et al., Nucleic Acids Res. 21:3281-3286 (1993); Carter. J.R, et al., J. Bacteriol. 175:5604-5610 (1993); Dong, Z., et aL, J. Biol. Chem. 268:11758-11765 (1993); Xiao, H., et al., J. Biol. Chem. 268:11773-11778 (1993)). These were named these *hol*A, *hol*B, *hol*C*, hol*D, and *hol*E for the δ, δ', χ ψ and θ genes, respectively.

There are at least three distinct polymerases in E. coli, yet only the pol III holoenzyme appears to play a major replicative role. What are the special features of the pol III holoenzyme that confer its unique role in replication? Work to date suggests that the rapid elongation rate, high processivity, ability to utilize a long single-stranded template coated with the single-stranded DNA binding protein, resistance to physiological levels of salt and ability to interact with other proteins of the replicative apparatus are all critical to its unique functions. In contrast, other non-replicative polymerases such as DNA polymerase I cannot substitute effectively for replicases either in vitro or in vivo (Kornberg, and Baker, *ibid.).* This is because of their low processivity, their corresponding slow reaction rate, and their inability to interact with other replication proteins to enable catalysis of a coordinated reaction at the replication fork.

*Multiple DNA polymerase III forms-The* DNA polymerase III holoenzyme can be biochemically resolved into a series of successively simpler forms. DNA polymerase III core contains the α catalytic subunit complexed tightly to ∈ (proofreading subunit) and θ. DNA polymerase III' contains core + τ (DnaX). The DnaX protein is the designation used for the dnaX gene product in this document. The DnaX protein appears in *E. coli* as two related proteins *γ* (47.4 kDa) and τ (71kDa) (Tsuchihashi, Z. and Kornberg, A., J. Biol. Chem. 264:17790-17795 (1989)). γ represents the amino-terminal 5/7 of τ. The two proteins are nearly functionally interchangable for most purposes and are not distinguished for most purposes in this document. DNA polymerase III* contains pol III' + γ, δ-δ', χ-ψ. Holoenzyme is composed of pol III* +β.

*Processivity-Studies* of the processivities of the multiple polymerase **III** forms have revealed individual contributions of subunits (Fay, P.J., et al., .J. BioL Chem. 256:976-983 (1981); Fay, P. J., et al., J Biol. Chem. 257:5692-5699 (1982)). The multiple forms of DNA polymerase III exhibit strikingly different processivities. The core pol III has a low processivity *(ca.* 10 bases) in low ionic strength that decreases to being completely distributive (processivity = 1) under more physiological conditions. Processivity is enhanced by addition of the τ DnaX subunit to form pol III'. Pol III' achieves maximum processivity in the presence of physiological concentrations of spermidine, an agent that inhibits the core pol III. Addition of the γ, δδ' and χψ to pol III' to form pol III* further increases processivity in the presence of single-stranded DNA binding protein (SSB). The holoenzyme exhibits a processivity orders of magnitude greater than any of its subassemblies. In carefully . controlled experiments with single-stranded phage, the entire template (up to 8000 nucleotides) is synthesized in a single processive event in under 15 s at 30°C (Johanson, K.O. and McHenry, C.S., (1982) *ibid.)* (1 min at 22°C (Fay, P.J., *et al.,* (1981), *ibid.).* For coupled replication fork systems where the holoenzyme acts with primosomal components, processivities of 150-500 kb have been directly observed (Wu, C.A., et al., J. Biol. Chem. 267:4064-4073 (1992)). These products are synthesized at rates of 500-700 nt/s, permitting synthesis of 100,000 bases in ca 3 min. Thus, a progression in processivities that parallels the structural complexity of the corresponding enzyme form is observed. For comparative purposes, the processivities of "repair-type" polymerases of the bacterial DNA polymerase I class are typically 15-50 bases (Bambara, R.A., et al., J. Biol. Chem. 253:413-423 (1978)).

*Initiation Complex Formation-To* achieve high processivity, the holoenzyme requires ATP (or dATP) and primed DNA to form a stable initiation complex (Fay, P.J., *et al.,* (1981) *ibid.*). Initiation complexes can be isolated by gel filtration and, upon addition of dNTPs, form a complete RFII in 10-15 seconds without ever dissociating (Wickner, W. and Kornberg, A., *ibid.;* Hurwitz, J. and Wickner, S., *ibid.;* Johanson, K.O. and McHenry, C.S., J. Biol. Chem. 255:10984-10990 (1980)). Initiation complex formation can be monitored experimentally as a conversion of replicative activity to anti-β IgG resistance (Johanson, K.O. and McHenry, C.S., (1982) *ibid.;* Johanson, K.O. and McHenry, C.S., (1980) *ibid).* β participates in elongation; antibody resistance arises from β's immersion in the complex, sterically precluding antibody attachment. Consistent with this observation, M. ODonnell's lab has found that a kinase recognition peptide fused to the carboxyl-terminus of β can be readily phosphorylated in solution but not in initiation complexes (Stukenberg, P.T., et al., Cell 78:877-887 (1994)).

*Structure of the β Sliding Clamp-The* X-ray crystallographic structure of *β,* solved by Kuriyan, O'Donnell and coworkers (Kong, X.P. *et al.,* (1994) *ibid.),* provides a simple and elegant explanation for its function. The β dimer forms a bracelet-like structure, presumably with DNA passing through the central hole, permitting it to slide down DNA rapidly but preventing it from readily dissociating. Protein-protein contacts between β and other components of the replicative complex tether the polymerase to the DNA, increasing its processivity. A tightly clasped bracelet would not be expected to readily associate with DNA. This explains the need for an energy-dependent clamp-setting complex, the DnaX-complex, to recognize the primer terminus and open and close the β-bracelet around DNA.

*DnaX Complex: The Apparatus that Sets the* β *Sliding Clamp onto Primed DNA-*The DnaX protein contains a consensus ATP binding site near its amino-terminus (Yin, K.C., et al., Nucleic Acids Res. 14:6541-6549 (1986)) that is used to bind and hydrolyze ATP, in concert with δ-δ' and χ-ψ, setting the β processivity clamp on the primer-terminus. DnaX binds ATP with a dissociation constant *of ca.* 2 *µ*M (Tsuchihashi, Z. and Kornberg, A. (1989) *ibid.)* and is a DNA-dependent ATPase (*ibid.*; Lee, S.H. and Walker, J.R, Proc. Natl. Acad. Sci. U.S.A. 84:2713-2717 (1987)). The primer-terminus appears to be the most active effector of the ATPase (Onrust, R., et al., J. Biol. Chem. 266:21681-21686 (1991)). DnaX (τ) binds the a subunit DNA polymerase III core and causes it to dimerize, forming the dimeric scaffold upon which other auxiliary proteins can assemble to form a dimeric replicative complex. *In vitro,* the τ DnaX subunit can readily form a "τ-complex" (*τ-ψ-χ*-δ-δ') that functions to load β onto primed DNA (Dallmann, H.G. and McHenry, C.S., J. Biol. Chem. 270:29563-29569 (1995); Onrust, R., et al., J. Biol. Chem. 270:13348-13357 (1995); Dallmann, H.G., et al., J. Biol. Chem. 270:29555-29562 (1995)). The stoichiometry of the DnaX complexes and has been found to have 3 copies of the DnaX protein and 1 each of the ancillary subunits (DnaX₃δ₁δ'₁χ₁ψ₁) (Pritchard, A., Dallmann, G., Glover, B. and McHenry, C. (2000) EMBO J. 19:6536-6545).

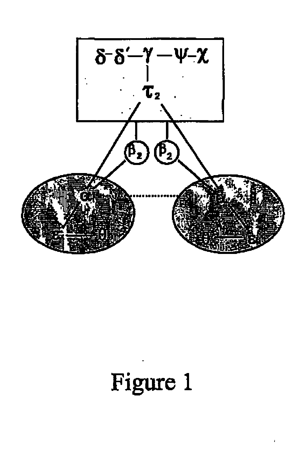

*Structure of the DNA polymerase III holoenzyme-* Figure 1 illustrates the current working hypothesis for holoenzyme subunit-subunit interactions. a and ∈ form an isolable complex upon mixing (Maki, H. and Kornberg, A., Proc. Natl. Acad. Sci. U.S.A. 84:4389-4392 (1987)). Mutations in the structural gene for ∈ have also been found that suppress *dna*E (α) mutations (Maurer, R, et al., Genetics 108:25-38 (1984)). Suppressor mutations most likely arise through modification of a subunit that interacts directly with the suppressed mutant gene product. Pol III core (*α∈θ*) is isolable (McHenry, C.S. and Crow, W., J. Biol. Chem. 254:1748-1753 (1979)). DnaX (τ can be isolated in a complex with pol III core (McHenry, C.S., J. Biol. Chem. 257:2657-2663 (1982)). Suppressor data suggest an interaction between DnaX and β (Engstrom, J., et al., Genetics 113:499-516 (1986)), although a direct interaction has not yet been demonstrated biochemically. DnaX in the presence of δ and δ' can transfer β to primed DNA to form a preinitiation complex (O'Donnell, M. and Studwell, P.S. (1990) J. Biol. Chem. 265:1179-1187). Suppressor data indicate an interaction between β and a (Kuwabara, N. and Uchida, H., Proc. Natl. Acad. Sci. U.S.A. 78:5764-5767 (1981)), a notion supported by the ability of β to interact with and increase the processivity of core pol III (Laduca, R.J., et al., J. Biol. Chem. 261:7550-7557 (1986)) and the observation of an interaction between β and the carboxyl-terminal domain of the a catalytic subunit (Kim, D. R. and McHenry, C.S., J. Biol. Chem. 271:20699-20704 (1996)). Genetic evidence for α-α interaction and for the dimeric nature of pol III holoenzyme was obtained through interallelic complementation between *dna*E₁₀₂₆ and *dna*E₄₈₆ or *dna* E₅₁₁ (Bryan, S., et al., "DNA Polymerase III is Required for Mutagenesis," in DNA Replication and Mutagenesis, Moses, R. & Summers, W., eds., American Society for Microbiology, Washington, DC) - if this interaction occurs, it must be weak and require the presence of other subunits, since no α-α interaction is seen *in vitro.* χ and ψ have been isolated in a complex with DnaX (Olson, M.W., et al., J. Biol. Chem. 270:29570-29577 (1995); O'Donnell, M. and Studwell, P.S., J. Biol. Chem. 255:1179-1187 (1990)). δ and δ' can interact weakly by themselves in solution and together can interact with DnaX (Dallmann, H.G. and McHenry, C.S., J. Biol. Chem. 270:29563-29569 (1995); Olson, M.W., *et al*., *ibid*.; Onrust, R and O'Donnell, M., J. Biol. Chem. 268:11766-11772 (1993)). ψ and δ' are apparently the subunits that interact directly with DnaX (Onrust, R., et al., J. Biol. Chem. 270:13348-13357 (1995)). A direct δ-β interaction has been detected (Naktinis, V., et al., J. Biol. Chem. 270:13358-13365 (1995)). There are three copies of DnaX protein in the holoenzyme (Pritchard, A., *et al*., (2000) *ibid.*).

*The Polymerase Chain Reaction* -The polymerase chain reaction (PCR) has made enormous contributions to molecular biology. It is useful for direct DNA amplification from complex genome preparations, for direct sequence analysis, for detection or diagnosis of mutations, for pathogen diagnosis and forensic analysis, and for a variety of manipulations used in molecular biology research (Arnheim, N. and Erlich, H., Annu. Rev. Biochem. 61:131-156 (1992)). In its simplest form, the reaction involves three steps: (1) Target DNA is thermally denatured to make it single-stranded. *(2)* Oligonucleotides flanking the sequence to be amplified are annealed. Sequences of *ca.* 20 nucleotides normally provide the required specificity for amplification of a specific gene. If the genome sequence is known, sequences can be selected to increase specificity and avoid partial regions of complementarity elsewhere on the genome. Solution conditions can also be altered to influence stringency. *(3)* A thermophilic DNA polymerase extends the primers until replication occurs past the second primer binding site. The three steps are repeated until the product is sufficiently abundant to permit isolation and analysis, typically 25-30 cycles.

A major limitation of the method is the requirement that replication must occur over the entire length of the DNA separating the primers. This introduces serious limitations with natural sequence DNA in the range over 10 kb and, for practical purposes, often over shorter regions. Innovations have enabled PCR amplification to 30-40 kb by well-set up labs. Although a large number of solution conditions must be optimized for each sequence amplified, the major contribution comes from inclusion of a proofreading polymerase in the thermophilic polymerase mixture (Barnes, W.M., Proc. Natl. Acad. Sci. U.S.A. 91:2216-2220 (1994); Cheng, S., et al., Proc. Natl. Acad. Sci. U.S.A. 91:5695-5599 (1994)). Presnm,ably, the 3'→5' proofreading exonuclease removes misincorporated bases that are not extended by most DNA polymerases. Many labs have found this method difficult to adapt to practice (Hengen, P.N., Trends Biochem. Sci. 19:341-342 (1994)) and even in expert labs, not all sequences over 20 kb are amplifiable (Cheng, S., et al., Proc. Natl. Acad. Sci. U.S.A. 91:5695-5699 (1994)). The thermophilic eubacterial DNA polymerases used in these systems are of the DNA polymerase I class (Lawyer, F.C., et al., J. Biol. Chem. 264:6427-6437 (1989)) that would be expected to have limited processivity and limited efficiency for amplification of very long sequences. Archaebacterial DNA polymerases are less defined. However, the polymerases used are the most abundant in cells and by analogy to both eukaryotic and prokaryotic systems, would be expected to be abundant repair class polymerases of limited processivity. A robust processive thermophilic DNA polymerase III holoenzyme should enable one to develop practical applications for amplification of high molecular weight **DNA**

### DNA polymerase III holoenzyme from thermophilic organisms

A subassembly of a themaophilic DNA polymerase III holoenzyme has been isolated, indicating that a thermophilic replicase, which had long-eluded previous isolation attempts, was generally used for bacterial DNA replication and was not limited to mesophilic organisms (McHenry, C.S., et al., J. Mol. Biol. 272:178-189 (1997)). Using protein sequence obtained from the isolated replicase, the identity of γ and *τ-*like proteins in *T. thermophilus* were directly demonstrated (McHenry, C.S., *et al.,* (1997) *ibid).* In a parallel approach, the O'Donnell laboratory directly isolated a *dna*X gene from *T. thermophilus* chromosomal DNA using PCR and oligonucleotides corresponding to conserved regions (Yurieva, O., et al., J. Biol. Chem. 272:27131-27139 (1997)). Expression of *T. thermophilus dnaX in E. coli leads* to synthesis of γ- and τ-like proteins (Yurieva, O., et *al., ibid.;* PCT WO99/13060; PCT WO9953074), apparently by a transcriptional slippage mechanism (Larsen, B., et al., Proc. Natl. Acad. Sci. U.S.A. 97:1683-1688 (2000)). Partial protein sequence from the isolated *T*. *thermophilus* α subunit has led to the identification of the structural gene (PCT W099/13060; PCT WO9953074) and expression of active native and tagged *T*. *thermophilus* α subunit (PCT WO991/13060). Taking advantage of the close genetic linkage between the chromosomal initiation protein *dna*A and the structural gene for the sliding clamp processivity factor β (*dna*N), the *T*. *thermophilus dna*N gene was isolated, sequenced and the information used to direct the synthesis and subsequent purification of active *T*. *thermophilus* β (PCT WO99/13060; PCT WO9953074). The *Thermus thermophilus* DnaX complex subunits δ and δ' have also been expressed in active form and used to reconstitute an active *T*. *thermophilus* DNA polymerwe III holoenzyme (U.S. Utility Patent Application No. 09/818,780).

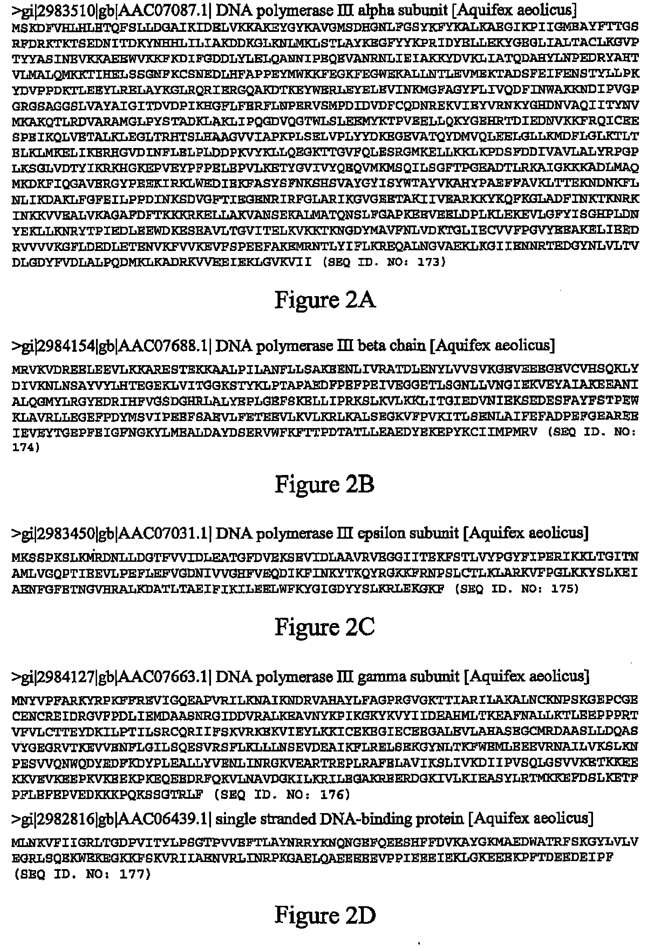

From the sequence of the *Aquifex aeolicus* and *Thermotoga maritima* genomes, it is apparent that a DNA polymerase III holoenzyme exists in these organisms too (Deckert, G., et al., Nature 392:353-358 (1998); Huang, Y.P. and Ito, J., Nucleic Acids Res. 26:5300-5309 (1998); Nelson, K.E., et al., Nature 399:323-329 (1999)). The sequence of *Aquifex aeolicus* was annotated, at the time of publication, to reveal homologs of the a (*dna*E) and ∈ (*dna*Q) components of the DNA polymerase III core, a β sliding clamp processivity factor (*dna*N), a single stranded DNA binding protein *(ssb)* and the central DnaX protein of the DnaX complex (Deckert, G., *et al.,* (1998) *ibid*.) (Figure 2A-D). Missing however, was the analog of the δ', δ, χ and ψ DNA polymerase III holoenzyme subunits.

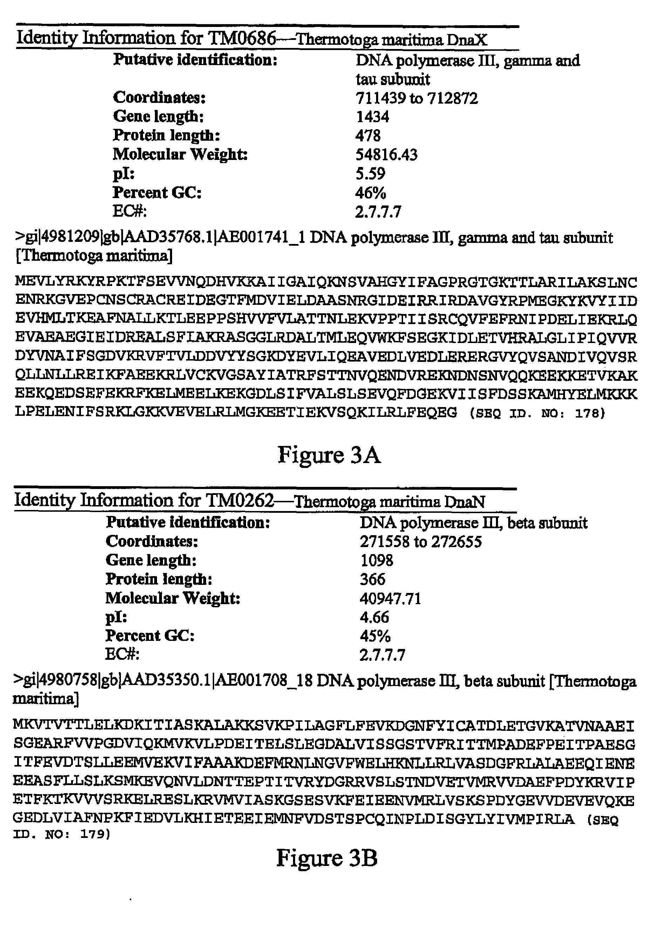

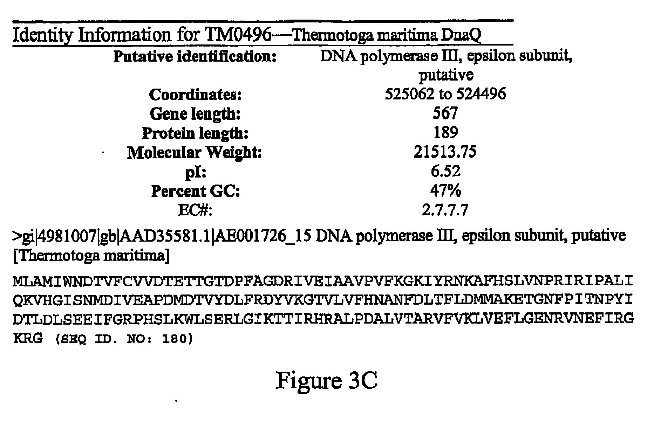

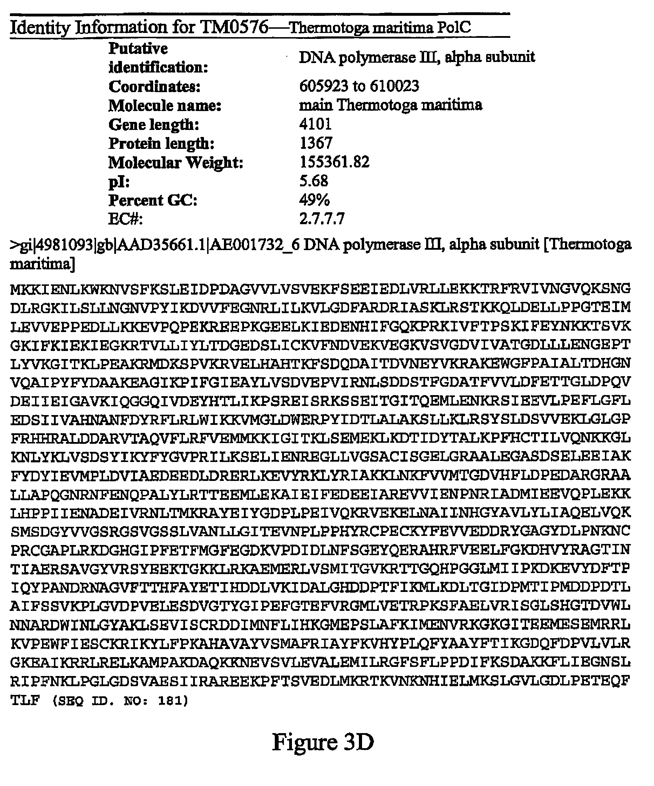

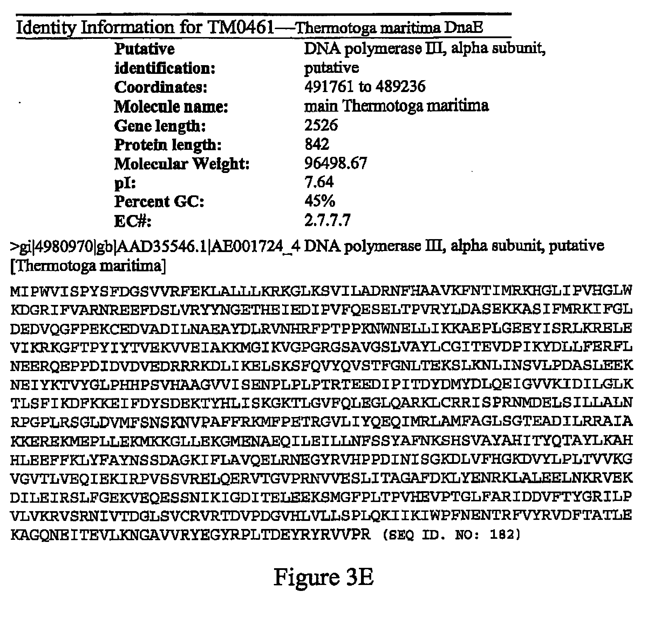

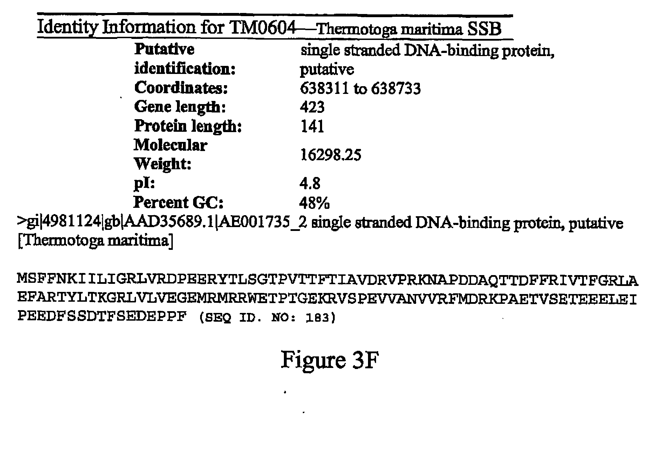

In *Thermotoga maritima* the sequence of two distinct DNA polymerase III-like catalytic subunits was published (Huang, Y.P. and Ito, J., (1998) ibid), a situation in parallel with gram-positive (firmicute phylum) mesophilic bacteria (Koonin, E.V., and Bork, P., Trends Biochem. Sci. 21:128-129 (1996)). One DNA polymerase III contained the 3'→5' exonuclease as part of the same polypeptide while a second α subunit and ∈ subunit are apparently encoded by an *E. coli-like dnaE* and *dnaQ* gene respectively. The homologs for *dnaN,* ssb, dnaX are annotated (Fig. 3A-F). An additional "γ-like" gene (TM0771) is annotated that is likely holB; however, because of poor sequence conservation, the structural genes for the homologs of the DNA polymerase III holoenzyme δ, χ and ψ subunits are not apparent. The lack of a structural gene for a δ subunit is particularly problematic because of its expected essentiality for reconstitution of a processive DNA polymerase III holoenzyme from *Aquifex aeolicus* and *Thermotoga maritima.*

### Replication in Gram-Positive (Firmicute Phylum) Organisms

DNA replication has not been as extensively studied in the prototype for gram-positive (firmicute phylum) organisms, *B. subtilis,* as in *E. coli.* Of the elongation components, only the basic DNA polymerase III catalytic subunit has been purified (Low RL., Rashbaum S.A., and Cozzarelli N.R J. Biol. Chem. 251:1311-1325 (1976); Hammond, R.A., Barnes, M.H., Mack, S.L.,Mitchener. J.A., Brown, N.C., Gene 98:29-36 (1991)). This enzyme shows sequence similarity to the *E. coli* DNA polymerase III, but differs in that the 3'→5' proofreading activity is contained within the same polypeptide chain. Examination of the sequence *of B. subtilis* pol III reveals sequence with close similarity with the *E. coli* ε pmofreading subunit inserted near the amino-terminus (Barmes, M., Hammond, R, Kennedy, C., Mack, S. and Brown, N.C., Gene 111:43-49 (1992)). With the completion of several genomic sequences from gram-positive organisms, it became apparent that a second DNA polymerase III α subunit exists with the sequence more closely resembling the E. *coli*-like enzyme (Koonin, B.V., and Bork, P., Trends Biochem. Sci. 21:128-129 (1996)). Recently, a similar situation has been observed in the more distant *Thermotoga maritima* (Huang, Y.P. & Ito, J. (1998) Nucleic Acids Res. 26:5300-5309 (1998)). Pritchard (Pritchard, A. & McHenry, C.S., J. Mol. Biol. 286:1067-1080 (1999)) and Ito (Huang, Y.P., and Ito, J. (1998) *ibid.)* have referred to the prototypical *E*. *coli-like* and *B. subtilis*-like polymerases type I and II, respectively, a convention followed in this application. A type II pol III, where it exists, always is present as the second pol III-like enzyme. This raises important questions regarding the mechanistic contributions of each polymerase; an issue that these proposed studies should resolve. In *B*. *subtilis*, the prototypical gram-positive type II pol III is clearly essential for cell viability. Hydroxyphenylazopyrimidines inhibit this enzyme and drug-resistance maps to the structural gene for the type II enzyme (Love, B., Dambrosio, J., Brown, N., and Dubnau, D., Mol. Gen. Genet. 144:313-321 (1976)). However, D. Ehrlich and colleagues have recently shown that the type I pol III is also required for cell viability (personal communication). In the closely related organism *S*. *pyogenes,* the minimal components for the elongation apparatus (type II pol III, β, τ, δ and δ') have recently been expressed and used to reconstitute a minimal replicase (Bruck, I. and O'Donnell, M., J. Biol. Chem. 275:28971-28983 (2000); Klemperer, N, Zhang, D, Skasgalis, M and O'Donnell, M. J. Biol. Chem. 275:26136-26143 (2000); PCT WO 01/09164).

**Table 2 E. coli and Corresponding B. subtilis Replication Proteins**

| ***E coli* gene** | **subunit/fonction** | ***B. subtilis* gene** | **homology with *E. coli*** |
|---|---|---|---|
| **Elongation Components** | | | |
| *dna*E | α/replicative polymerase (pol III type I) | *dna*E | strong |
| | α + ε / replicative polymerase (pol III type II) | *polC* | moderate |
| *dna*Q | ε / 3'→5' proofreading exonuclease | *not known* | ND |
| *hol*E | θ / no known function | not known | ND^{b} |
| *dna*X | γ, τ / ATPase that loads β₂ onto DNA | *dna*X | strong |
| *holA* | δ / binds β, essential part of DnaX complex | *yqe*N | weak |
| *hol*IB | δ' / essential part of DnaX complex | *hol*B | strong |
| *hol*C | χ / nonessential-interacts with SSB | not known | ND |
| *hol*D | ψ / nonessential-increases the affinity of DnaX for δ' | not known | ND |
| *dna*N | β / processivity factor | *dna*N | strong |
| *ssb* | SSB / single stranded DNA binding protein | *ssb* | strong |
| *dna*G | primase / RNA priming | *dna*G | strong |

| | | | |
|---|---|---|---|
| ^{b}No homology to *E*. *coli* replication proteins detectable. | | | |

The genomic sequences of *B. subtilis* and other related organisms reveal the likely structural genes for the DNA polymerase III holoenzyme auxiliary subunits with the exception of θ, χ and ψ which are not highly conserved, even among very closely related bacteria. *B. subtilis* homologs of *dna*X*, hol*B (δ'), *dna*N (β) and *ssb* are also apparent and documented within the *B. subtilis* genome project web site (available at the SubtiList website of the Pasteur Institute, Paris). The primase and SSB proteins are apparent from sequence examination and has been documented in the genomes ofB. *subtilis* and other organisms.

*Cytology of DNA Replication Apparatus-*Recently developed improvements in technology has enabled visualization of the replication apparatus in bacteria (Lemon, K. and Grossman, A. (1998) Science 282:1516-1519; Gordon, G.S., Sitnikov, D., Webb, C.D., Teleman, A., Straight, A., Losick, R, Murray A.W., Wright, A., Cell 90:1113-1121 (1997); Newman, G. and Crooke, E., J. Bacteriol. 182:2604-2610 (2000)). Using C-terminally tagged *B*. *subtilis* Pol C (DNA polymerase III type II), DnaX (τ) and HolB (δ'), Grossman and colleagues have demonstrated that the replication apparatus is fixed with a central part of the cell and that the chromosome passes through it during replication (Lemon, K. and Grossman, A., Science 282:1516-1519 (1998); Lemon, K. and Grossman, A., Molecular Cell 6:1321-1330 (2000)). However, the origin appears to progress to the cell poles as it is replicated. Two gene products that appear to be associated with the initiation of chromosomal replication *(B. subtilis* DnaB and DnaI proteins) have been visualized to co-localize as foci predominantly associated with the edges of the nucleoid during the initiation of replication (Imai Y, Ogasawara N, Ishigo-Oka D, Kadoya R, Daito T, Moriya S., Mol. Microbiol. 36:1037-48 (2000)}.

**Bacteria and Antibacterial Drugs.** Bacterial infections continue to represent a major worldwide health hazard (Overcoming Antimicrobial Resistance. World Health Organization, Report on Infectious Diseases (WHO/CDS/2000.2) (2000)). Infections range from the relatively innocuous, such as skin rashes and common ear infections in infants, to the very serious and potentially lethal infections in immune-compromised patients. Drug-resistant hospital-acquired and community infections are increasing. With the recent emergence of numerous, clinically important, drug-14resistant bacteria including *Staphylococcus aureus*, *Streptococcus pneumoniae*, *Enterococcus faecalis*, *Mycobacterium tuberculosis,* and *Pseudomonas aeruginosa*, an emergency is becoming apparent

**Classes of Antibacterials and Mechanism of Resistance.** Antibacterials kill bacteria by interfering with major processes of cellular function that are essential for survival. The β-lactams (penicillins and cephalosporins) and the glycopeptides (vancomycin and teicoplanin) inhibit synthesis of the cell wall. Macrolides (erythromycin, clarithromycin, and azithromycin), clindamycin, chloramphenicol, aminoglycosides (streptomycin, gentamicin, and amikacin) and the tetracyclines inhibit protein synthesis. Also inhibiting protein synthesis is the newest class of antibacterials to be approved (linezolid) that are synthetic oxazolidinones. Rifampin inhibits RNA synthesis, the fluoroquinolones (such as ciprofloxacin) inhibit DNA synthesis *indirectly* by inhibiting the enzymes that maintain the topological state of DNA. Trimethoprim and the sulfonamides inhibit folate biosynthesis directly and DNA synthesis indirectly by depleting the pools of one of the required nucleotides (Chambers, H.F. and Sande, M.A., Antimicrobial Agents. Goodman & Gilman's The Pharmacological Basis of Therapeutics, McGraw-Hill, New York (1996)).

Resistance to antibacterials can occur when the target of a drug mutates so that it can still function, but is no longer blocked by the drug, or by mutation of efflux pumps or modification enzymes to broaden their specificity to a new drug. Because of the growth advantage this gives to the resistant cell and its progeny in the presence of the antibacterial, the resistant organisms quickly take over a population of bacteria. Resistance developed in one cell can be transferred to other bacteria in the population since bacteria have mechanisms for directly exchanging genetic material. In a recent congressional report, the General Accounting Office (GAO) has summarized the current and future public health burden resulting from drug-resistant bacteria (Antimicrobial Resistance. General Accounting Office, GAO/RCED-99-132 (1999)). According to this report, the number of patients treated in a hospital setting for an infection with drug-resistant bacteria has doubled from 1994 to 1996 and again almost doubled from 1996 to 1997. Resistant strains can spread easily in environments such as hospitals or tertiary care facilities that have a sizeable population of immunosuppressed patients. The same GAO report also provides clear evidence that previously susceptible bacteria are increasingly becoming resistant and spreading around the world. Furthermore, the proportion of resistant bacteria within bacterial populations is on the rise. An especially frightening development is the appearance of bacterial strains that are resistant to *all* approved antibacterials. Recognizing the dramatic increase of drug-resistant bacteria, the Food and Drug Administration has recently issued a recommendation urging physicians to use antibacterials more judiciously and only when clinically necessary (FDA Advisory. Federal Register 65 (182), 56511-56518 (2000)).

Until recently, drug developers tended to respond to emerging, drug-resistant bacterial strains by simply modifying existing antibacterials. Indeed, most of the antibacterials in late-stage development are analogs of drugs already in clinical use. This strategy is becoming less effective since the basic mechanism for resistance to the antibacterial class is already widespread in nature. Further mutation of the resistant target to confer resistance on a new antibacterial of the same class can often occur by a single base change in the gene for the target. Thus, the utility of antibacterials presently in the development pipeline is expected to be finite and the health care industry is currently in severe need of *novel antibacterials that are mechanistically distinct from existing drugs.* Accordingly, there remains a need for a chromosomal replication system to discover novel therapeutic agents that inhibit the central DNA replication apparatus of bacteria in order to combat bacterial health hazards.

### SUMMARY OF THE INVENTION

The present invention relates to gene and amino acid sequences encoding DNA polymerase III holoenzyme δ subunits from bacterial genes and nucleic acid molecules, including those that encode such proteins and to antibodies raised against such proteins. The present invention also includes methods to obtain such proteins, nucleic acid molecules and antibodies.

The present invention provides methods for identifying a bacterial DNA polymerase holoenzyme δ subunit protein from at least one completely sequenced genome comprising providing a database of sequence information for a plurality of different organisms, setting an inclusion threshold to a level sufficient to minimize the number of required iterations, selecting the organisms to be searched wherein the selected organisms are candidate organisms, providing the amino acid sequence of the δ subunit protein from a reference organism, comparing the sequences of the candidate organisms and the reference organism, excluding known DnaX proteins and known δ' proteins from further steps, selecting proteins comprising between 300 and 400 amino acids, selecting a delta candidate from a candidate organism comprising selecting sequence with the lowest E score from the candidate organism and optionally repeating steps the preceding three steps wherein the sequences of the candidate organisms are sequences with the lowest E score from the candidate organism, wherein a bacterial DNA polymerase holoenzyme δ subunit protein may be identified.

The present invention provides bacterial DNA polymerase III holoenzyme δ subunit proteins identified by the method. The method can be modified to obtain δ subunit proteins from organisms with only partially sequenced genomes.

The present invention also provides methods for identifying a bacterial DNA polymerase holoenzyme δ protein from at least one completely sequenced genome comprising providing a database of sequence information for a plurality of different organisms, setting the inclusion threshold to a level sufficient to minimize the number of required iterations, selecting the organisms to be searched, wherein the selected organisms are candidate organisms, providing the amino acid sequence of the δ subunit protein from a reference organism, comparing the sequences of the candidate organisms and the reference organism, excluding known DnaX proteins and known δ' proteins from further steps, selecting proteins comprising at least three of the following six characteristics:
a. having at least five out of ten conserved residues from the conserved region 17-(L/I/V)XX(L/I/V)Y(L/I/V)(L/I/V)XGX(D/E)XX(L/I/V)(L/I/V)XXXXXX(L/I/V)-38;
b. having at least five out of eleven conserved residues from the conserved region 64-(L/I/V)(L/I/V)XXXX(A/S)XX(L/I/V)F(A/S)X(K/R)X(L/I/V)(L/I/V)(L/I/V)(L/I/V)-82;
*c*. having at least five out of ten conserved residues from the conserved region 97-(L/I/V)XX(L/I/V)(L/I/V)XXXXX(D/E)X(L/I/V)(L/I/V)(L/I/V)(L/I/V)XXXK(L/I/V)-117;
d. having at least nine out of eighteen conserved residues from the conserved region 154-(R/K)XXX(L/I/V)X(L/I/V)X(L/I/V)(D/E)X(D/E)(A/S)(L/I/V)XX(L/I/V)XXXXXXN(L/I/V) XX(L/I/V)XX(D/E)(L/I/V)X(R/K)(L/I/V)X(L/I/V)(L/I/V)-191;
e. having at least eleven out of twenty-three conserved residues from the conserved region 215-FX(L/I/V)X(D/E)(A/S)(L/I/V)(L/I/V)XG(R/K)XXX(A/S)(L/I/V)X(L/I/V)(L/I/V)XX(L/I/V) XXXGX(D/E)P(L/I/V)X(L/I/V)(L/I/V)XX(L/I/V)XXX(L/I/V)XX(L/I/V)XX(L/I/V)-260; and
*f*. having at least six out of twelve conserved residues from the conserved region 298-(L/I/V)XXX(L/I/V)XX(L/I/V)XXX(D/E)XX(L/I/V)(R/K)XXXXX(D/E)XXXX(L/I/V)(D/E) XX(L/I/V)(L/I/V)X(L/I/V)-331; and
retaining the sequences above the threshold, wherein the retained sequences are delta candidate sequences; and optionally repeating steps the preceding three steps wherein the sequences of the candidate organisms are delta candidate sequences, whereby a δ protein may be identified.

The present invention provides bacterial DNA polymerase III holoenzyme δ subunit proteins identified by the method. The method can be modified to obtain δ subunit proteins from organisms with only partially sequenced genomes.

The present invention also provides methods for identifying a δ protein from at least one target organism comprising identifying a δ protein from an evolutionarily related organism, and performing a search for a similar sequence in the sequence of the target organism.

The present invention further provides isolated bacterial DNA polymerase δ subunit proteins, wherein the δ subunit protein is not a member of the group consisting *of an E. coli* δ subunit protein, a *Haemophilis influenzae* **δ** subunit protein, and a *Neisseria meningitides* δ subunit protein.

The present invention further provides isolated bacterial DNA polymerase 6 subunit proteins, wherein the δ subunit protein is not a member of the group consisting of a gamma division of proteobacteria δ subunit protein and a beta division of proteobacteria δ subunit protein.

The present invention further provides isolated bacterial DNA polymerase δ subunit proteins, wherein the δ subunit protein is a gram-positive (firmicute phylum) bacteria δ subunit protein, including *S. pyogenes,* δ subunit proteins, *S. aureus δ* subunit proteins, and *S. pneumoniae* δ subunit proteins.

The present invention further provides isolated bacterial DNA polymerase δ subunit proteins derived from thermophilic organisms, wherein the δ subunit protein is selected from the group consisting of *Thermus Thermophilus, Aquifex aeolicus,* and *Thermatoga maritima δ* subunit protein.

The present invention provides an antibody to a δ subunit protein of the present invention, which may be a polyclonal, monoclonal, chimeric, single chain, F_{ab} fragments, or F_{ab} expression library, and a method for producing anti-DNA polymerase III δ subunit antibodies comprising exposing an animal having immunocompetent cells to an immunogen comprising at least an antigentic portion of DNA polymerase III δ subunit.

The present invention provides a method for detecting DNA polymerase III δ subunit protein comprising, providing in any order, a sample suspected of containing DNA polymerase III, an antibody capable of specifically binding to at least a portion of the DNA polymerase III δ subunit protein, mixing the sample and the antibody under conditions wherein the antibody can bind to the DNA polymerase III; and detecting the binding.

The present invention also provides isolated nucleic acid molecules which encode a δ subunit protein of the present invention and their complements.

The present invention also provides arecombinant molecules and recombinant cells comprising at least a portion of a bacterial DNA polymerase III nucleic acid molecule according to the present invention.

Furthermore, the present invention provides a method for detection of nucleic acid molecules encoding at least a portion of DNA polymerase III δ subunit in a biological sample comprising the steps of hybridizing at least a portion of a *hol*A nucleic acid molecule to nucleic acid material of a biological sample, thereby forming a hybridization complex, and detecting the hybridization complex, wherein the presence of the complex correlates with the presence of a polynucleotide encoding at least a portion of DNA polymerase III δ subunit in the biological sample.

The present invention also provides a method for detecting DNA polymerase III δ subunit expression, including expression of modified or mutated DNA polymerase III δ subunit proteins or gene sequences comprising the steps of providing a test sample suspected of containing DNA polymerase III δ subunit protein, as appropriate; and comparing test DNA polymerase III δ subunit with quantitated DNA polymerase III holoenzyme or holoenzyme subunit in a control to determine the relative concentration of the test DNA polymerase III δ subunit in the sample.

In addition, the present invention provides a method of screening for a compound that modulates the activity of a DNA polymerase III replicase comprising contacting an isolated replicase with at least one test compound under conditions permissive for replicase activity, assessing the activity of the replicase in the presence of the test compound; and comparing the activity of the replicase in the presence of the test compound with the activity of the replicase in the absence of the test compound, wherein a change in the activity of the replicase in the presence of the test compound is indicative of a compound that modulates the activity of the replicase and wherein said replicase comprises an isolated DNA polymerase III δ subunit protein.

The present invention describes a method of identifying compounds which modulate the activity of a DNA polymerase III replicase. This method is carried out by forming a reaction mixture that includes a primed DNA molecule, a DNA polymerase III replicase, a candidate compound, a dNTP or up to 4 different dNTPs, ATP, and optionally either a β subunit, a DnaX complex, or both the β subunit and the DnaX complex; subjecting the reaction mixture to conditions effective to achieve nucleic acid polymerization in the absence of the candidate compound; analyzing the reaction mixture for the presence or absence of nucleic acid polymerization extension products and comparing the activity of the replicase in the presence of the test compound with the activity of the replicase in the absence of the test compound. A change in the activity of the replicase in the presence of the test compound is indicative of a compound that modulates the activity of the replicase. The DnaX complex comprises a δ subunit protein of the present invention.

The present invention describes a method to identify candidate pharmaceuticals that modulate the activity of a DnaX complex and a β subunit in stimulating the DNA polymerase. The method includes contacting a primed DNA (which may be coated with SSB) with a DNA polymerase, a β subunit, and a DnaX complex (or subunit or subassembly of the DnaX complex) in the presence of the candidate pharmaceutical, and dNTPs (or modified dNTPs) to form a reaction mixture. The reaction mixture is subjected to conditions which, in the absence of the candidate pharmaceutical, would effect nucleic acid polymerization and the presence or absence of the extension product in the reaction mixture is analyzed. The nucleic acid polymerization in the presence of the test compound is compared with the nucleic acid polymerization in the absence of the test compound. A change in the nucleic acid polymerization in the presence of the test compound is indicative of a compound that modulates the activity of a DnaX complex and a β subunit. The DnaX complex comprises a δ subunit of the present invention.

The present invention describes a method to identify chemicals that modulate the ability of a β subunit and a DnaX complex (or a subunit or subassembly of the DnaX complex) to interact. This method includes contacting the β subunit with the DnaX complex (or subunit or subassembly of the DnaX complex) in the presence of ATP (or a suitable ATP analog, such as ATPγS, a non-hydrolyzable analog of ATP, that enables interaction of β with the DnaX complex (or subunit or subassembly of the DnaX complex) the candidate pharmaceutical to form a reaction mixture. The reaction mixture is subjected to conditions under which the DnaX complex (or the subunit or subassembly of the DnaX complex) and the β subunit would interact in the absence of the candidate pharmaceutical. The reaction mixture is then analyzed for interaction between the β subunit and the DnaX complex (or the subunit or subassembly of the DnaX complex). The extent of interaction in the presence of the test compound is compared with the extent of interaction in the absence of the test compound. A change in the interaction between the β subunit and the DnaX complex (or the subunit or subassembly of the DnaX complex) is indicitive of a compound that modulates the interaction The DnaX complex comprises a δ protein of the present invention.

The present invention describes a method to identify chemicals that modulate the ability of a δ subunit and the δ' and/or DnaX subunit to interact. This method includes contacting the δ subunit with the δ' and/or δ' plus DnaX subunit in the presence of the candidate pharmaceutical to form a reaction mixture. The reaction mixture is subjected to conditions under which the δ subunit and the δ' and/or δ' plus DnaX subunit would interact in the absence of the candidate pharmaceutical. The reaction mixture is then analyzed for interaction between the δ subunit and the δ' and/or DnaX subunit The extent of interaction in the presence of the test compound is compared with the extent of interaction in the absence of the test compound. A change in the interaction between the δ subunit and the δ' and/or DnaX subunit is indicitive of a compound that modulates the interaction.

The present invention describes a method to identify chemicals that modulate the ability of a DnaX complex (or a subassembly of the DnaX complex) to assemble a β subunit onto a DNA molecule. This method involves contacting a circular primed DNA molecule (which may be coated with SSB) with the DnaX complex (or the subassembly thereof) and the β subunit in the presence of the candidate pharmaceutical, and ATP or dATP to form a reaction mixture. The reaction mixture is subjected to conditions under which the DnaX complex (or subassembly) assembles the β subunit on the DNA molecule absent the candidate pharmaceutical. The presence or absence of the β subunit on the DNA molecule in the reaction mixture is analyzed. The extent of assembly in the presence of the test compound is compared with the extent of assmbly in the absence of the test compound. A change in the amount of β subunit on the DNA molecule is indicative of a compound that modulates the ability of a DnaX complex (or a subassembly of the DnaX complex) to assemble a β subunit onto a DNA molecule. The DnaX complex comprises a delta protein of the present invention.

The present invention describes a method to identify chemicals that modulate the ability of a DnaX complex (or a subunit (s) of the DnaX complex) to disassemble a β subunit from a DNA molecule. This method comprises contacting a DNA molecule onto which the β subunit has been assembled in the presence of the candidate pharmaceutical, to form a reaction mixture. The reaction mixture is subjected to conditions under which the DnaX complex (or a subunit (s) or subassembly of the DnaX complex) disassembles the β subunit from the DNA molecule absent the candidate pharmaceutical. The presence or absence of the β subunit on the DNA molecule in the reaction mixture is analyzed. The extent of assembly in the presence of the test compound is compared with the extent of assembly in the absence of the test compound. A change in the amount of β subunit on the DNA molecule is indicative of a compound that modulates the ability of a DnaX complex (or a subassembly of the DnaX complex) to disassemble a β subunit onto a DNA molecule. The DnaX complex comprises a δ subunit of the present invention

The present invention describes a method to identify chemicals that modulate the dATP/ATP binding activity of a DnaX complex or a DnaX complex subunit (e. g. DnaX subunit). This method includes contacting the DnaX complex (or the DnaX complex subunit) with dATP/ATP either in the presence or absence of a DNA molecule and/or the β subunit in the presence of the candidate pharmaceutical to form a reaction. The reaction mixture is subjected to conditions in which the DnaX complex (or the subunit of DnaX complex) interacts with dATP/ATP in the absence of the candidate pharmaceutical. The reaction is analyzed to determine if dATP/ATP is bound to the DnaX complex (or the subunit of DnaX complex) in the presence of the candidate pharmaceutical. The extent of binding in the presence of the test compound is compared with the extent of binding in the absence of the test compound. A change in the dATP/ATP binding is indicative of a compound that modulates the dATP/ATP binding activity of a DnaX complex or a DnaX complex subunit (e.g. DnaX subunit). The DnaX complex comprises a δ subunit of the present invention.

The present invention describes a method to identify chemicals that modulate the dATP/ATPase activity of a DnaX complex or a DnaX complex subunit (e.g., the DnaX subunit). This method involves contacting the DnaX complex (or the DnaX complex subunit) with dATP/ATP either in the presence or absence of a DNA molecule and/or a β subunit in the presence of the candidate pharmaceutical to form a reaction mixture. The reaction mixture is subjected to conditions in which the DnaX subunit (or complex) hydrolyzes dATP/ATP in the absence of the candidate pharmaceutical The reaction is analyzed to determine if dATP/ATP was hydrolyzed. The extent of hyrdolysis in the presence of the test compound is compared with the extent of hydrolysis in the absence of the test compound. A change in the amount of dATP/ATP hydrolyzed is indicative of a compound that modulates the dATP/ATPase activity of a DnaX complex or a DnaX complex subunit (e. g., the DnaX subunit). The DnaX complex comprises a δ protein of the present invention.

The invention provides a further method for identifying chemicals that modulate the activity of a DNA polymerase comprising contacting a circular primed DNA molecule (may be coated with SSB) with a DnaX complex, a β subunit and an a subunit in the presence of the candidate pharmaceutical, and dNTPs (or modified dNTPs) to form a reaction mixture. The reaction mixture is subjected to conditions, which in the absence of the candidate pharmaceutical, affect nucleic acid polymerization, and the presence or absence of the extension product in the reaction mixture is analyzed. The activity of the replicase in the presence of the test compound is compared with the activity of the replicase in the absence of the test compound. A change in the activity of the replicase in the presence of the test compound is indicative of a compound that modulates the activity of the replicase. The DnaX complex comprises a δ protein of the present invention.

The present invention also includes a test kit to identify a compound capable of modulating DNA polymerase III replicase activity. Such a test kit includes an isolated δ subunit protein and a means for determining the extent of modulation of DNA replication activity in the presence of (i.e., effected by) a putative inhibitory compound.

The present invention also includes modulators and inhibitors isolated by such a method, and/or test kit, and their use to inhibit any replicase comprising a δ subunit that is susceptible to such an inhibitor. Modulators of bacterial DNA replication can also be used directly as to treat animals, plants and humans suspected of having a bacterial infection as long as such compounds are not harmful to the species being treated. Similarly, molecules that bind to any replicase comprising a δ subunit, incuding but not limited to antibodies and small molecules, can also be used to target cytotoxic, therapeutic or imaging entities to a site of infection.
The present invention also includes a method of synthesizing a DNA molecule comprising hybridizing a primer to a first DNA molecule; and incubating said DNA molecule in the presence of a thermostable DNA polymerase replicase and one or more dNTPs under conditions sufficient to synthesize a second DNA molecule complementary to all or a portion of said first DNA molecule, wherein said thermostable DNA polymerase replicase comprises an δ subunit protein selected from the group consisting of an *Aquifex, Thermus* and *Thermatoga* δ subunit protein.

The present invention also provides a method of amplifying a double-stranded DNA molecule comprising providing a first and second primer, wherein said first primer is complementary to a sequence at or near the 3'-termini of the first strand of said DNA molecule and said second primer is complementary to a sequence at or near the 3'-termini of the second strand of said DNA molecule, hybridizing said primer to said first strand and said second primer to said second strand in the presence of a thermostable DNA polymerase replicase, under conditions such that a third DNA molecule complementary to said first strand and a fourth DNA molecule complementary to said second strand are synthesized, denaturing said first and third strand, and said second and fourth strands; and repeating the steps one or more times; wherein said thermostable DNA polymerase is selected from the group consisting of an *Aquifex, Thermus* and *Thermatoga* δ subunit protein.

The present invention also provides method of amplifying nucleic acid sequences, the method comprising mixing a rolling circle replication primer with one or more amplification target circles, to produce a primer-amplification target circle mixture, and incubating the primer-amplification target circle mixture under conditions that promote hybridization between the amplification target circles and the rolling circle replication primer in the primer-amplification target circle mixture, wherein the amplification target circles each comprise a single-stranded, circular DNA molecule comprising a primer complement portion, and wherein the primer complement portion is complementary to the rolling circle replication primer; and mixing a thermostable DNA polymerase replicase with the primer-amplification target circle mixture, to produce a replicase-amplification target circle mixture, and incubating the replicase-amplification target circle mixture under conditions that promote replication of the amplification target circles, wherein replication of the amplification target circles results in the formation of tandem sequence DNA, and wherein said thermostable DNA polymerase replicase comprises an δ subunit protein selected from the group consisting of an *Aquifex, Thermus* and *Thermatoga* δ subunit protein.

The present invention further provides a method of synthesizing DNA which comprises utilizing one or more polypeptides, said one or more polypeptides comprising an amino acid sequence having at least 95% sequence identity to an δ subunit protein of th present invention.

### BRIEF DESCRIPTION OF THE FIGURES

Fig. 1 shows structural features of the DNA polymerase III holoenzyme.
Fig. 2A shows the annotated DNA polymerase III holoenzyme alpha subunit gene from *Aquifex aeolicus.*
Fig. 2B shows the annotated DNA polymerase III holoenzyme β subunit gene from *Aquifex aeolicus.*
Fig. 2C shows the annotated DNA polymerase III holoenzyme epsilon subunit gene from *Aquifex aeolicus.*
Fig. 2D shows the annotated DNA polymerase III holoenzyme gamma (DnaX) subunit gene from *Aquifex aeolicus.*
Fig. 2E shows the annotated DNA polymerase III holoenzyme SSB gene from *Aquifex aeolicus.*
Fig. 3A shows the annotated DNA polymerase III holoenzyme gamma/τ (DnaX) gene from *Thermotoga maritima.*
Fig. 3B shows the annotated DNA polymerase III holoenzyme β gene from *Thermotoga maritima.*
Fig. 3C shows the annotated DNA polymerase III holoenzyme epsilon gene from *Thermotoga maritima.*
Fig. 3D shows the annotated DNA polymerase III holoenzyme alpha gene for DNA polymerase III, type II from *Thermotoga maritima.*
Fig. 3E shows the annotated DNA polymerase III holoenzyme alpha gene for DNA polymerase III, type I from *Thermotoga maritima.*
Fig. 3F shows the annotated DNA polymerase III SSB gene from *Thermotoga maritima.*
Figure 4A-HHH shows delta sequences discovered from organisms with completely sequenced genomes and the DNA sequence of their structural gene *(hol*A*).*
Figure 5A-G shows delta protein and DNA sequences for S. *pyogenes, S. pneumoniae, S. aureus.*
Figure 6 shows the amino acid alignment of the newly identified 6 proteins shown in Figures 4 and 5 with the established δ protein sequences *of E. coli* and *T. thermophilus* with the alignment performed using Clusta1W 1.8.
Figure 7A-F shows δ protein and gene sequences identified from partially sequenced genomes.
Figure 8 shows a listing of all of the recognized bacterial phyla and the first level of subclassifications of each pyla along with a listing of bacteria from which delta sequences have been identified.
Figure 9 shows a phylogenic tree constructed by relating organisms by their evolutionary relationship determined by their rRNA sequences that allows evolutionarily close organisms to be identified for use in searching for new δ proteins.
Figure 10A-BB shows the additional δ proteins and their structural *(hol*A*)* genes that were idendfied using phylogenetic considerations.
Figure 11 shows a graphical depiction of the PCR product "PCR Aquifex NB delta" which will be used to express the tagged *Aquifex aeolicus holA* gene.
Figure 12 shows the region of pA1-NB-KpnI that the PCR product ""PCR Aquifex NB delta" will be inserted into after digestion with appropriate restriction enzymes.
Figure 13 shows a graphical depiction of the PCR product "PCR Aquifex native delta" which will be used to express the native *Aquifex aeolicus hol*A gene.
Figure 14 shows the reigion of pAl-NB-KpnI that the PCR product "PCR Aquifex native delta" will be inserted into after digestion with appropriate restriction enzymes.
Figure 15 shows a graphical depiction of the PCR product "PCR NB Thermotoga D" which which will be used to express the tagged *Thermotoga maritima holA* gene.
Figure 16 shows a graphical depiction of the PCR product "PCR native Thermatoga D" which which will be used to express the native *Thermotoga maritima hol*A gene.
Figure 17 shows the three *dna*X like *Thermotoga maritima* as aligned using ClustalW.
Figure 18 shows a graphical depiction of the PCR product "PCR AQ NB delta'" which will be used to express the tagged *Aquifex hol*B gene (AQ_1526).
Figure 19 shows the region of pA1-NB-Avr2 that PCR product "PCR AQ NB delta"' will be inserted into following digestion with appropriate restriction enzymes.
Figure 20 shows a graphical depiction of the PCR product "PCR of native Aquifex D"' which will be used to express *Aquifex δ*' subunit in an untagged form.
Figure 21 shows a graphical depiction of the PCR product "PCR TM NB D'(0508)" which will be used to express the tagged *T. maritima* TM0508 gene.
Figure 22 shows a graphical depiction of the PCR product "PCR TM native D'(0508)" which will be used to express the native *T. maritima* TM0508 gene.
Figure 23 shows the region of pA1-CB-NcoI that "PCR TM native D'(0508)" will be inserted into following digestion with appropriate restriction enzymes.
Figure 24 shows the region of pA1-TM[0508] containing the site in which the RcaI digested restriction site was spliced into the NcoI restriction site of pA1-CB-NcoI.
Figure 25 shows a graphical description of the PCR product "PCR TM NB D'0771," which will be used for the expression of tagged *T. maritima* protein TM0771.
Figure 26 shows a graphical description of the PCR product "PCR TM native D'(0771)" which will be used for the expression of native *T. maritima* protein TM0771.

### DETAILED DESCRIPTION OF THE PREFERRED EMBODIMENTS

The present invention relates to structural gene and amino acid sequences encoding δ subunits of DNA polymerase III holoenzyme from a variety of bacteria. As used herein, the term "gene" refers to a nucleic acid (*e*.*g*., DNA) sequence that comprises coding sequences necessary for the production of a polypeptide or precursor (*e*.*g*., DNA polymerase III holoenzyme or holoenzyme subunit, as appropriate). The polypeptide can be encoded by a full length coding sequence or by any portion of the coding sequence so long as the desired activity or functional properties (*e*.*g*., enzymatic activity, ligand binding, binding of another DNA polymerase III holoenzyme subunit, processivity, etc.) of the full-length or fragment are retained. The term also encompasses the coding region of a structural gene and the including sequences located adjacent to the coding region on both the 5' and 3' ends for a distance of about 1 kb on either end such that the gene corresponds to the length of the full-length mRNA. The term "gene" encompasses both cDNA and genomic forms of a gene. A genomic form or clone of a gene contains the coding region interrupted with non-coding sequences termed "intervening regions" or "intervening sequences." The mRNA functions during translation to specify the sequence or order of amino acids in a nascent polypeptide.

As used herein, the term "DNA polymerase III holoenzyme" refers to the entire DNA polymerase III entity (i.e., all of the polymerase subunits, as well as the other associated accessory proteins required for processive replication of a chromosome or genome), while "DNA polymerase III" is just the core [*α, ∈, θ*] for type I DNA Polymerase IIIs (encoded by *dna*E gene) or just an a catalytic subunit for type II DNA Polymerase IIIs (encoded by *pol*C gene) that includes and epsilon-like sequence within the same polypeptide chain. "DNA polymerase III holoenzyme subunit" is used in reference to any of the subunit entities that comprise the DNA polymerase III holoenzyme. Thus, the term "DNA polymerase III" encompasses "DNA polymerase III holoenzyme subunits" and "DNA polymerase III subunits." Subunits include, but are not limited to, DnaX or τ (and y from some organisms, including, but not limited to, many proteobacteria and *T*. *thermophilus*), HolA or δ, HolB or δ' proteins, DnaN or β proteins, DnaE or a subunit of DNA polymerase III-type I, PolC or α subunit ofDNA polymerase III-type II and DnaQ or s proteins. As used herein, "replicase" means an enzyme that duplicates a DNA polynucleotide sequence.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited proteins. It is to be noted that the term "a" or "an" entity refers to one or more of that entity; for example, a protein refers to one or more proteins or at least one protein. As such, the terms "a" (or "an"), "one or more" and "at least one" can be used interchangeably herein. It is also to be noted that the terms "comprising", "including", and "having" can be used interchangeably. Furthermore, a compound "selected from the group consisting of' refers to one or more of the compounds in the list that follows, including mixtures (i.e., combinations) of two or more of the compounds.

### Methods of identifying δ subunit proteins and nucleic acid molecules of the present invention

One embodiment of the present invention is an isolated DNA polymerase III holoenzyme subunit δ protein. According to the present invention, an isolated, or biologically pure, protein, is a protein that has been removed from its natural environment. As such, "isolated" and "biologically pure" do not necessarily reflect the extent to which the protein has bean purified. An isolated δ subunit protein of the present invention can be obtained from its natural source, can be produced using recombinant DNA technology or can be produced by chemical synthesis. As used herein, an isolated δ subunit protein can be a full-length protein or any homologue of such a protein.

The minimal size of a protein homolog of the present invention is a size sufficient to be encoded by a nucleic acid molecule capable of forming a stable hybrid with the complementary sequence of a nucleic acid molecule encoding the corresponding natural protein. As such, the size of the nucleic acid molecule encoding such a protein homolog is dependent on nucleic acid composition and percent homology between the nucleic acid molecule and complementary sequence as well as upon hybridization conditions per se (e.g., temperature, salt concentration, and formamide concentration). The minimal size of such nucleic acid molecules is typically at least about 12 to about 15 nucleotides in length if the nucleic acid molecules are GC-rich and at least about 15 to about 17 bases in length if they are AT-rich. As such, the minimal size of a nucleic acid molecule used to encode a protein homolog of the present invention is from about 12 to about 18 nucleotides in length. There is no limit on the maximal size of such a nucleic acid molecule in that the nucleic acid molecule can include a portion of a gene, an entire gene, or multiple genes, or portions thereof. Similarly, the minimal size of a DNA polymerase III subunit protein homolog of the present invention is from about 4 to about 6 amino acids in length, with preferred sizes depending on whether a full-length, multivalent (i.e., fusion protein having more than one domain each of which has a function), or functional portions of such proteins are desired. Polymerase protein homologs of the present invention preferably have activity corresponding to the natural subunit, such as being able to perform the activity of the subunit in a binding assay or DNA replication assay.

DNA polymerase III holoenzyme subunit δ protein homologs can be the result of natural allelic variation or natural mutation. The protein homologs of the present invention can also be produced using techniques known in the art including, but not limited to, direct modifications to the protein or modifications to the gene encoding the protein using, for example, classic or recombinant DNA techniques to effect random or targeted mutagenesis.

According the methods of the present invention, δ subunit proteins may be identified from any bacteria. Such identification of δ subunit proteins has previously not been possible due to the limited sequence relatedness of δ proteins. Prior to the present invention, δ proteins were known only for *E. coli,* and two related proteobacterial deltas *-Haemophilis influenzae* and *Neisseria meningitides.* A simple BLAST search of NCBI databases for δ proteins based on the known *E. coli* sequence returns results for only a small number of closely related organisms: *Vibrio cholerae, Pasteurella multocida, Haemophilus influenzae, Buchnera sp. APS, Pseudomonas aeruginosa, Xylella fastidiosa,* from the gamma proteobacteria division, and *Neisseria meningitides* from the beta division of proteobacteria. No sequences from the alpha, delta or epsilon divisions of proteobacteria are retrieved. The present invention provides methods, detailed below and and in the Examples section, that allow for the identification of δ protein subunits from bacteria from all known phyla.

The present invention also provides δ subunit proteins identified by the methods. In one embodiment, the present invention provides a method for identifying a bacterial δ protein from a sequenced genome.

In a preferred embodiment, the method for identifying a bacterial DNA polymerase holoenzyme δ subunit protein from at least one completely sequenced genome comprises providing a database of sequence information for a plurality of different organisms, setting an inclusion threshold to a level sufficient to minimize the number of required iterations, selecting the organisms to be searched, providing the amino acid sequence of the δ subunit protein from a reference organism, comparing the sequences of the selected organisms and the reference organism, excluding known DnaX proteins and known δ' proteins from further steps, and repeating the cycle to permit identification of additional δs using the pattern of similarity of identified δ sequences in subsequent searches, selecting proteins comprising between about 300 and about 400 amino acids, wherein a bacterial DNA polymerase III holoenzyme δ subunit protein may be identified.

As used herein, setting the inclusion threshold to a level sufficient to minimize the number of iterations required refers to a threshold that permits a search to be found with a reasonable time and effort. An iteration is a repetition of the steps of excluding known DnaX proteins and known δ' proteins from further steps, and selecting proteins comprising between about 300 and about 400 amino acids. A reasonable number of iterations will depend on the complexity of the database searched, the sequence relaledness of the δ sequences from the targeted organisms, among other factors. Under the conditions used in the present invention, less than about 20 iterations is a reasonable number. In preferred embodiments, less than about 15 iterations is a reasonable number. In more preferred embodiments, less than about 10 iterations is a reasonable number. In other preferred embodiments, less than about 5 iteration is a reasonable number.

As used herein, the inclusion threshold is also referred to as the E or "Expect" value limit. Potential 'hits' that have an E value higher than the inclusion threshold are excluded. In one preferred embodiment, the inclusion threshold is 0.002. In another preferred embodiment, the threshold is 0.001. (With the threshold set to 0.001, there is a 1 in 1000 chance for an inappropriate hit, based on the referenced stochastic model.)

Conserved regions are based on alignments of identified deltas from a number of organisms. After finding a number of candidate delta using this method, they were aligned using Clustal W (Thompson, J.D., Higgins, D.G., and Gibson, T.J., Nucleic Acids Res. 22:4673-4680 (1994)), and conserved regions were identified using methods known to those skilled in the art. Conserved amino acids within the conserved regions are shown in boldface letters.

These conserved regions are:
1.) 17-(L/IV)XX(L/I/V)Y(L/I/V)(L/I/V)XGX(D/E)XX(L/I/V)(L/I/V)XXXXX X(L/I/V)-38 (SEQ ID NO:194)
2.) 64-(L/I/V)(L/I/V)XXXX(A/S)XX(L/I/V)F(A/S)X(K/R)X(L/I/V)(L/I/V) (L/I/V)(L/I/V)-82 (SEQ ID NO:195)
3.) 97-(L/IV)XX(L/I/V)(L/I/V)XXXXX(D/E)X(L/I/V)(L/I/V)(L/I/V)(L/I/V)X XXK(L/I/V)-117 (SEQ ID NO:196)
4.) 154-(R/K)XXX(L/I/V)X(L/I/V)X(L/I/V)(D/E)X(D/E)(A/S)(L/I/V)XX(L/I/V )XXXXXXN(L/I/V)XX(L/I/V)XX(D/E)(L/I/V)X(R/K) (L/I/V)X(L/I/V)(L/I/V)-191 (SEQ ID NO:197)
5.) 215-FX(L/I/V)X(D/E)(A/S)(L/I/V)(L/I/V)XG(R/K)XXX(A/S) (L/I/V)X(L/I/V) (L/I/V)XX(L/I/V)XXXGX(D/E)P(L/I/V)X(L/I/V) (L/I/V)XX(L/I/V)XXX(L/I/V)XX(L/I/V)XX(L/I/V)-260 (SEQ ID NO: 198)
6.) 298-**(L/I/V)XXX(L/I/V)XX(L/I/V)XXX(D/E)XX(L/I/V)(R/K)XXXXX(D/E) XXXX(L/I/V)(D/E)XX(L/I/V)(L/I/V)X(L/I/V)-**331 (SEQ ID NO:198)

The present invention also provides a method for identifying a δ protein from at least one partially sequenced genome. The step of selecting organisms comprises selecting organisms in the database which are only partially sequenced.

The present invention also provides a method for identifying a δ protein from at least one target organism comprising using the method to identify a δ protein from an evolutionarily related organism, and performing a search for a similar sequence in the sequence of the target organism.

In another embodiment, the method comprises providing a database of sequence information of different organisms, setting the inclusion threshold to a level sufficient to minimize the number of required iterations, selecting the organism or organisms to be searched, entering the amino acid sequence of the δ subunit protein from a reference organism for comparison, comparing the sequences of the selected organisms and the reference organism, excluding known DnaX proteins and known δ' proteins from further steps, selecting proteins comprising i) comprising at least three of the follwing six characterisics:
a. having at least five out of ten conserved residues fom the conserved region 17-(L/I/V)XX(L/I/V)Y(L/I/V)(L/I/V)XGX(D/E)XX(L/I/V)(L/I/V)XXXXXX(L/I/V)-38; (SEQ ID NO:194)
b. having at least five out of eleven conserved residues fom the conserved region 64-(L/I/V)(L/I/V)XXXX(A/S)XX(L/I/V)F(A/S)X(K/R)X(L/I/V)(L/I/V)(L/I/V)(L/I/V)-82; (SBQ ID NO: 195)
c. having at least five out of ten conserved residues fom the conserved region 97-(L/I/V)XX(L/I/V)(L/I/V)XXXXX(D/E)X(L/I/V)(L/I/V)(L/IV)(L/I/V)XXXK(L/I/V)-117; (SEQ ID NO:196)
d. having at least nine out of eighteen conserved residues fom the conserved region 154-(R/K)XXX(L/I/V)X(L/I/V)X(L/I/V) (D/E)X(D/E)(A/S)(L/I/V)XX(L/I/V)XXXXXXN(L/I/V)XX(L/I/V)XX(D/E)(L/I/V)X(R/K) (L/I/V)X(L/I/V)(L/I/V)-191; (SEQ ID NO:197)
e. having at least eleven out of twenty-three conserved residues fom the conserved region 215-FX(L/I/V)X(D/E)(A/S)(L/I/V)(L/I/V) XG(R/K)XXX(A/S)(L/I/V)X(L/I/V)(L/I/V)XX(L/I/V)XXXGX(D/E)P(L/I/V)X(L/I/V)(L/I /V)XX(L/I/V)XXX(L/I/V)XX(L/I/V)XX(L/I/V)-260 (SEQ ID NO:198); and
f. having at least six out of twelve conserved residues fom the conserved region 298-(L/I/V)XXX(L/I/V)XX(L/I/V)XXX(D/E)XX(L/I/V)(R/K)XXXXX(D/E)XXXX(L/I/V)(D/ E)XX(L/I/V)(L/I/V)X(L/I/V)-331 (SEQ ID NO:199); and retaining the sequences above the threshold. The retained sequences are identified as δ subunit proteins.

An iteration is a repetition of the steps of excluding known DnaX proteins and known δ' proteins from further steps, and selecting proteins having three of the six conserved region characteristic.

The present invention also provides methods to identify δ subunit proteins from genomes that are not completely sequenced, but are partially sequenced. This is accomplished, in the above method, by replacing the step of selecting the organism or organisms to be searched with the selection of organisms in the database which are partially sequenced. In databases that do not allow a search of only the partially sequenced genomes, it is permissible to search all bacteria which includes partially and completely sequenced organisms, or other suitable subset of organisms. Sequences already identified from completely sequenced organisms could be specifically excluded or, alternatively, one can just pick out the new δ sequences identified.

The present invention also provides methods for identifying δ proteins not already identified by the above methods. These methods are useful for identifying δ from already sequenced or partially sequenced genomes, but for which a δ protein has not been found, and for identifying δ from organisms as the sequences of these organisms become available. The method is based on phylogenetic relationships among bacteria. To identify δ from a bacterial species for which a δ protein has not been found, the search comprises searching the sequences for the new organism against the 6 amino acid sequences from organisms that are evolutionarily close (i.e., members of the same class/phylum) to the target organism. That is, the 6 protein from an evolutionarily close organism is used as the reference or query sequence. The reference δ sequence can be obtained by any of the methods of the present invention. If the initial search produces no hits, then searches should continue using δ amino acid sequences as query sequences that are gradually more evolutionarily distant from the target organism until δ has been identified, or until δ sequences from all known phyla have been exhausted. Illustrative examples of the use of these methods are described in the examples section.

### δ subunit proteins

One embodiment of the present invention includes an isolated *Aquifex, Bacillus, Buchnera, Borrelia, Campylobacter, Caulobacter, Chlamydia, Chlamydophila, Deinococcus, Haemophilus, Helicobacter, Lactococcus, Mycobacterium, Mesorhizobium Mycoplasma, Neisseria, Pasteurella, Pseudomonas, Rickettsia, Synechocystis, Thermatoga, Treponema, Ureaplasma, Vibrio, Xylella, Mycoplasma, Staphylococcus, Streptococcus, Streptomyces. Streptococcus, Streptococcus, Thermus, Yersisna, Actinobacillus, Bordetealla, Bacillus, Burkholderia, Chlorobium, Chloroflexus, Clostridium, Corynebacterium, Cytopahaga, Dehalococcoides, Porphyromonas, Prochlorococcus,* and *Salmonella DNA polymerase III holoenzyme δ* subunit protein.

One embodiment of the present invention includes an isolated *Aquifex aeolicus, Bacillus halodurans, Bacillus subtilis, Buchnera, Borrelia burgdorferi, Campylobacter jejuni, Caulobacter crescentus*, *Chlamydia muridarum, Chlamydia trachomatis, Chlamydophila pneumonia, Deinococcus radiodurans, Haemophilus influenzae, Helicobacter pylori, Helicobacter pylori* 26695, *Helicobacter pylori* J99, *Lactococcus lactis, Mycobacterium laprae, Mycobacterium tuberculosis, Mesorhizobium loti, Mycoplasma genitalium, Mycoplasma pneunoniae, Neisseria meningitidis* MC58, *Neisseria meningitidis* Z2491, *Pasteurella multocida, Pseudomonas aeruginosa, Rickettsia prowazekii, Synechocystis, Thermatoga maritima, Treponema pallidum, Ureaplasma urealyticum, Vibrio cholerae, Xylella fastidiosa, Mycoplasma pulmonis, Staphylococcus aureus, Streptococcu*s *agalactiae, Streptomyces coelicolor, Streptococcus pneumoniae, Streptococcus pyogenes, Thermus thermophilus, Yersisna pestis, Actinobacillus actinomycetemcomitans, Bordetealla pertussis, Bacillus anthracis, Burkholderia pseudomallei, Chlorobium tepidum, Chloroflexus aurantiacus, Clostridium difficile, Corynebacterium diphtheriae, Cytopahaga hutchinsonii, Dehalococcoides ethenogenes, Porphyromonas gingivalis, Prochlorococcus marinus,* and *Salmonella typhi* DNA polymerase III holoenzyme δ subunit protein.

Also preferred is a delta protein comprising an amino acid sequence that is at least about 75%, more preferably at least about 80%, more preferably at least about 85%, more preferably at least about 90%, more preferably at least about 95%, and more preferably at least about 98% identical to amino acid sequence SEQ ID NO:3, SEQ ID NO:226, SEQ ID NO:6, SEQ ID NO:9, SEQ ID NO:12, SEQ ID NO:15, SEQ ID NO:18, SEQ ID NO:21, SEQ ID NO:24, SEQ ID NO:27, SEQ ID NO:30, SEQ ID NO:33, SEQ ID NO:36, SEQ ID NO:39, SEQ ID NO:42, SEQ ID NO:45, SEQ ID NO:48, SEQ ID NO:51, SEQ ID NO:54, SEQ ID NO:57, SEQ ID NO:60, SEQ ID NO:63, SEQ ID NO:66, SEQ ID NO:69, SEQ ID NO:72, SEQ ID NO:75, SEQ ID NO:78, SEQ ID NO:81, SEQ ID NO:84, SEQ ID NO:87, SEQ ID NO:90, SEQ ID NO:93, SEQ ID NO:96, SEQ ID NO:99, SEQ ID NO:102, SEQ ID NO:229, SEQ ID NO:105, SEQ ID NO:108, SEQ ID NO:111, SEQ ID NO:114, SEQ ID NO:117, SEQ ID NO:120, SEQ ID NO:123, SEQ ID NO:126, SEQ ID NO:129, SEQ ID NO:132, SEQ ID NO:135, SEQ ID NO:138, SEQ ID NO:141, SEQ ID NO:144, SEQ ID NO:147, SEQ ID NO:150, SEQ ID NO:153, SEQ ID NO:156, or a protein encoded by an allelic variant of a nucleic acid molecule encoding a protein comprising any of these sequences. Methods to determine percent identities between amino acid sequences and between nucleic acid sequences are known to those skilled in the art. Preferred methods to determine percent identities between sequences include computer programs such as GCG program (available from Genetics Computer Group, Madison, Wisc.), the MacVectors program (available from the Eastman Kodak Company, New Haven, Conn.), the DNAsis™ program (available from Hitachi Software, San Bruno, Calif.) the Vector NTI Suite of Programs (available from Informax, Inc., North Bethesda, MD) or the BLAST software available on the NCBI website.

In one embodiment, a preferred δ subunit protein comprises an amino acid sequence of at least about 5 amino acids, preferably at least about 50 amino acids, more preferably at least about 100 amino acids, more preferably at least about 200 amino acids, more preferably at least about 250 amino acids, more preferably at least about 275 amino acids, more preferably at least about 300 amino acids, more preferably at least about 350 amino acids, more preferably at least about 375 amino acids or the full-length δ protein, whichever is shorter. In another embodiment, preferred δ subunit proteins comprise full-length proteins, i.e., proteins encoded by full-length coding regions, or post-translationally modified proteins thereof, such as mature proteins from which initiating methionine and/or signal sequences or "pro" sequences have been removed.

A fragment of a δ subunit protein of the present invention preferably comprises at least about 5 amino acids, more preferably at least about 10 amino acids, more preferably at least about 15 amino acids, more preferably at least about 20 amino acids, more preferably at least about 25 amino acids, more preferably at least about 30 amino acids, more preferably at least about 35 amino acids, more preferably at least about 40 amino acids, more preferably at least about 45 amino acids, more preferably. at least about 50 amino acids, more preferably at least about 55 amino acids, more preferably at least about 60 amino acids, more preferably at least about 65 amino acids, more preferably at least about 70 amino acids, more preferably at least about 75 amino acids, more preferably at least about 80 amino acids, more preferably at least about 85 amino acids, more preferably at least about 90 amino acids, more preferably at least about 95 amino acids, and even more preferably at least about 100 amino acids in length.

In one embodiment, a preferred isolated δ subunit protein of the present invention is a protein encoded by a nucleic acid molecule having the nucleic acid sequence SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO:112, SEQ ID NO:115, SEQ ID NO:118, SEQ ID NO:121. SEQ ID NO:124, SEQ ID NO:127, SEQ ID NO:130, SEQ ID NO: 133, SEQ ID NO: 136, SEQ ID NO: 139, SEQ ID NO: 142, SEQ ID NO: 145, SEQ ID NO: 148, SEQ ID NO:151, SEQ ID NO:154; or by an allelic variant of a nucleic acid molecule having any of these sequences.

One embodiment of the present invention includes a non-native δ subunit protein encoded by a nucleic acid molecule that hybridizes under stringent hybridization conditions with a δ subunit gene, or *hol*A gene. As used herein, stringent hybridization conditions refer to standard hybridization conditions under which nucleic acid molecules, including oligonucleotides, are used to identify molecules having similar nucleic acid sequences. Such standard conditions are disclosed, for example, in Sambrook et al., Molecular Cloning: A Laboratory Manual, Cold Spring Harbor Labs Press (1989). Sambrook et al., ibid., is incorporated by reference herein in its entirety. Stringent hybridization conditions typically permit isolation of nucleic acid molecules having at least about 70% nucleic acid sequence identity with the nucleic acid molecule being used to probe in the hybridization reaction. Formulae to calculate the appropriate hybridization and wash conditions to achieve hybridization permitting 30% or less mismatch of nucleotides are disclosed, for example, in Meinkoth, J. et al., Anal. Biochem. 138:267-284 (1984); Meinkoth, J. et al., *ibid*., is incorporated by reference herein in its entirety. In preferred embodiments, hybridization conditions will permit isolation of nucleic acid molecules having at least about 80% nucleic acid sequence identity with the nucleic acid molecule being used to probe. In more preferred embodiments, hybridization conditions will permit isolation of nucleic acid molecules having at least about 90% nucleic acid sequence identity with the nucleic acid molecule being used to probe. In more preferred embodiments, hybridization conditions will permit isolation of nucleic acid molecules having at least about 95% nucleic acid sequence identity with the nucleic acid molecule being used to probe.

A preferred δ subunit protein includes a protein encoded by a nucleic acid molecule which is at least about 50 nucleotides and which hybridizes under conditions which preferably allow about 20% base pair mismatch, more preferably under conditions which allow about 15% base pair mismatch, more preferably under conditions which allow about 10% base pair mismatch, more preferably under conditions which allow about 5% base pair mismatch, and even more preferably under conditions which allow about 2% base pair mismatch with a nucleic acid molecule selected from the group consisting of SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ D7 NO:103, SEQ ID NO:106, SEQ ID NO:109, SEQ ID NO:112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO:148, SEQ ID NO:151, SEQ ID NO:154, or a complement of any of these nucleic acid molecules.

As used herein, a *holA* gene includes all nucleic acid sequences related to a natural *holA* gene such as regulatory regions that control production of the δ subunit protein encoded by that gene (such as, but not limited to, transcription, translation or post-translation control regions) as well as the coding region itself. In one embodiment, a *hol*A gene includes the nucleic acid sequence SEQ ID NO: 1. Nucleic acid sequence SEQ ID NO:1 represents the deduced sequence of a nucleic acid molecule denoted herein as nAeod, the deduction of which is disclosed in the Examples. It should be noted that since nucleic acid sequencing technology is not entirely error-free, SEQ ID NO:1 (as well as other sequences presented herein), at best, represents an apparent nucleic acid sequence of the nucleic acid molecule encoding a *Aquifex aeolicus* δ subunit protein of the present invention.

In another embodiment, a *hol*A gene can be an allelic variant that includes a similar but not identical sequence to SEQ ID NO:1. An allelic variant of a *hol*A gene including SEQ ID NO:1 is a gene that occurs at essentially the same locus (or loci) in the genome as the gene including SEQ ID NO:1, but which, due to natural variations caused by, for example, mutation or recombination, has a similar but not identical sequence. Allelic variants typically encode proteins having similar activity to that of the protein encoded by the gene to which they are being compared. Allelic variants can also comprise alterations in the 5' or 3' untranslated regions of the gene (e.g., in regulatory control regions). Allelic variants are well known to those skilled in the art and would be expected to be found within a given bacterium since the genome is diploid and/or among a population comprising two or more bacteria. For example, it is believed that *H. pylori* 26695 δ subunit nucleic acid molecule nHpy12d, having nucleic acid sequence SEQ ID NO:37 and to be described in more detail below, represents an allelic variant *of H. pylori* J99 δ subunit nucleic acid molecule nHpylJd, having nucleic acid sequence SEQ II3 NO:40.

Similarly, a *Streptococcus pyogenes holA* gene includes all nucleic acid sequences related to a natural *Streptococcus pyogenes holA* gene such as regulatory regions that control production of the *Streptococcus pyogenes δ* subunit protein encoded by that gene as well as the coding region itself. In one embodiment, an *Streptococcus pyogenes hol*A gene includes the nucleic acid sequence SEQ ID NO:103. Nucleic acid sequence SEQ ID NO:103 represents the deduced sequence of a DNA nucleic acid molecule denoted herein as nSpyod, the production of which is disclosed in the Examples. In another embodiment, an *Streptococcus pyogenes holA* gene can be an allelic variant that includes a similar but not identical sequence to SEQ ID NO: 103.

Similarly, all other *holA* genes described herein include all nucleic acid sequences related to the natural *holA* gene such as regulatory regions that control production of the δ subunit protein encoded by that gene as well as the coding region itself.

An isolated δ subunit protein of the present invention can be obtained from its natural source, can be produced using recombinant DNA technology or can be produced by chemical synthesis. As used herein, an isolated δ subunit protein can be a full-length protein or any homologue of such a protein. Examples of δ subunit homologues include δ proteins in which amino acids have been deleted (e.g., a truncated version of the protein, such as a peptide or by a protein splicing reaction when an intein has been removed or two exteins are joined), inserted, inverted, substituted and/or derivatized (e.g., by glycosylation, phosphorylation, acetylation, methylation, myristylation, prenylation, palmitoylation, amidation and/or addition of glycerophosphatidyl inositol) such that the homologue includes at least one epitope capable of eliciting an immune response against a δ protein. That is, when the homologue is administered to an animal as an immunogen, using techniques known to those skilled in the art, the animal will produce a humoral and/or cellular immune response against at least one epitope of a δ protein. δ homologues can also be selected by their ability to selectively bind to immune serum. Methods to measure such activities are disclosed herein. δ homologues also include those proteins that are capable of performing the function of the native δ proteins in a functional assay.

A δ subunit protein of the present invention may be identified by its ability to perform the function of a δ subunit in a functional assay. The phrase "capable of performing the function of that subunit in a functional assay" means that the protein has at least about 10% of the activity of the natural protein subunit in the functional assay. In other preferred embodiments, has at least about 20% of the activity of the natural protein subunit in the functional assay. In other preferred embodiments, has at least about 30% of the activity of the natural protein subunit in the functional assay. In other preferred embodiments, has at least about 40% of the activity of the natural protein subunit in the functional assay. In other preferred embodiments, has at least about 50% of the activity of the natural protein subunit in the functional assay. In other preferred embodiments, the protein has at least about 60% of the activity of the natural protein subunit in the functional assay. In more preferred embodiments, the protein has at least about 70% of the activity of the natural protein subunit in the functional assay. In more preferred embodiments, the protein has at least about 80% of the activity of the natural protein subunit in the functional assay. In more preferred embodiments, the protein has at least about 90% of the activity of the natural protein subunit in the functional assay.

Examples of functional assays are described in detail below in the section entitled "Use of the proteins of the present invention" and include, but are not limited to, DNA replication assays, β clamp-loading or -unloading assays, ATP binding and hydrolysis assays, protein binding assays and the like.

An isolated protein of the present invention can be produced in a variety of ways, including recovering such a protein from a bacterium and producing such a protein recombinantly. One embodiment of the present invention is a method to produce an isolated protein of the present invention using recombinant DNA technology. Such a method includes the steps of (a) culturing a recombinant cell comprising a nucleic acid molecule encoding a protein of the present invention to produce the protein and (b) recovering the protein therefrom. Details on producing recombinant cells and culturing thereof are presented below. The phrase "recovering the protein" refers simply to collecting the whole fermentation medium containing the protein and need not imply additional steps of separation or purification. Proteins of the present invention can be purified using a variety of standard protein purification techniques.

Isolated proteins of the present invention are preferably retrieved in "substantially pure" form. As used herein, "substantially pure" refers to a purity that allows for the effective use of the protein in a DNA replication assay.

### δ Subunit Nucleic Acid Molecules or holA genes

Another embodiment of the present invention is an isolated nucleic acid molecule capable of hybridizing under stringent conditions with a gene encoding a δ subunit protein. Such a nucleic acid molecule is also referred to herein as a δ subunit nucleic acid molecule or *a hol*A nucleic acid molecule. Particularly preferred is an isolated nucleic acid molecule that hybridizes under stringent conditions with a *holA* gene. The characteristics of such genes are disclosed herein. In accordance with the present invention, an isolated nucleic acid molecule is a nucleic acid molecule that has been removed from its natural milieu (i.e., that has been subject to human manipulation). As such, "isolated" does not reflect the extent to which the nucleic acid molecule has been purified. An isolated nucleic acid molecule can include DNA, RNA, or derivatives of either DNA or RNA.

As stated above, a *holA* gene includes all nucleic acid sequences related to a natural *hol*A gene such as regulatory regions that control production of a δ subunit protein encoded by that gene (such as, but not limited to, transcriptional, translational or post-translational control regions) as well as the coding region itself. A nucleic acid molecule of the present invention can be an isolated *holA* nucleic acid molecule or a homologue thereof. A nucleic acid molecule of the present invention can include one or more regulatory regions, full-length or partial coding regions, or combinations thereof. The minimal size of a *hol*A nucleic acid molecule of the present invention is the minimal size capable of forming a stable hybrid under stringent hybridization conditions with a corresponding natural gene. *hol*A nucleic acid molecules can also include a nucleic acid molecule encoding a hybrid protein, a fusion protein, a multivalent protein or a truncation fragment.

An isolated nucleic acid molecule of the present invention can be obtained from its natural source either as an entire (i.e., complete) gene or a portion thereof capable of forming a stable hybrid with that gene. As used herein, the phrase "at least a portion of' an entity refers to an amount of the entity that is at least sufficient to have the functional aspects of that entity. For example, at least a portion of a nucleic acid sequence, as used herein, is an amount of a nucleic acid sequence capable of forming a stable hybrid with the corresponding gene under stringent hybridization conditions.

An isolated nucleic acid molecule of the present invention can also be produced using recombinant DNA technology (e.g., polymerase chain reaction (PCR) amplification, cloning, etc.) or chemical synthesis. Isolated *holA* nucleic acid molecules include natural nucleic acid molecules and homologues thereof, including, but not limited to, natural allelic variants and modified nucleic acid molecules in which nucleotides have been inserted, deleted, substituted, and/or inverted in such a manner that such modifications do not substantially interfere with the ability of the nucleic acid molecule to encode a δ subunit protein of the present invention or to form stable hybrids under stringent conditions with natural nucleic acid molecule isolates.

A *hol*A nucleic acid molecule homologue can be produced using a number of methods known to those skilled in the art (see, for example, Sambrook et al., ibid.). For example, nucleic acid molecules can be modified using a variety of techniques including, but not limited to, classic mutagenesis techniques and recombinant DNA techniques, such as site-directed mutagenesis, chemical treatment of a nucleic acid molecule to induce mutations, restriction enzyme cleavage of a nucleic acid fragment, ligation of nucleic acid fragments, polymerase chain reaction (PCR) amplification and/or mutagenesis of selected regions of a nucleic acid sequence, synthesis of oligonucleotide mixtures and ligation of mixture groups to "build" a mixture of nucleic acid molecules and combinations thereof. Nucleic acid molecule homologues can be selected from a mixture of modified nucleic acids by screening for the function of the protein encoded by the nucleic acid (e.g., the ability of a homologue to elicit an immune response against a 6 subuit protein and/or to function in a DNA replication assay, or other functional assay, and/or by hybridization with isolated δ subunit-encoding nucleic acids under stringent conditions.

An isolated δ subunit nucleic acid molecule of the present invention can include a nucleic acid sequence that encodes at least one δ subunit protein of the present invention, examples of such proteins being disclosed herein. Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding a δ subunit protein.

One embodiment of the present invention is a *holA* nucleic acid molecule of the present invention that is capable of hybridizing under stringent conditions to a nucleic acid strand that encodes at least a portion of a δ subunit or a homologue thereof or to the complement of such a nucleic acid strand. A nucleic acid sequence complement of any nucleic acid sequence of the present invention refers to the nucleic acid sequence of the nucleic acid strand that is complementary to (i.e., can form a complete double helix with) the strand for which the sequence is cited. It is to be noted that a double-stranded nucleic acid molecule of the present invention for which a nucleic acid sequence has been determined for one strand, that is represented by a SEQ ID NO, also comprises a complementary strand having a sequence that is a complement of that SEQ ID NO. As such, nucleic acid molecules of the present invention, which can be either double-stranded or single-stranded, include those nucleic acid molecules that form stable hybrids under stringent hybridization conditions with either a given SEQ ID NO denoted herein and/or with the complement of that SEQ ID NO, which may or may not be denoted herein. Methods to deduce a complementary sequence are known to those skilled in the art. Preferred is a *hol*A nucleic acid molecule that includes a nucleic acid sequence having at least about 65 percent, preferably at least about 70 percent, more preferably at least about 75 percent, more preferably at least about 80 percent, more preferably at least about 85 percent, more preferably at least about 90 percent and even more preferably at least about 95 percent homology with the corresponding region(s) of the nucleic acid sequence encoding at least a portion of a δ subunit protein. Particularly preferred is a δ subunit nucleic acid molecule capable of encoding at least a portion of a δ subunit protein that naturally is present in bacteria.

**Preferred *holA* nucleic acid molecules include at least one of the following sequences:** SEQ ID NO:1, SEQ ID NO:4, SEQ ID NO:7, SEQ ID NO:10, SEQ ID NO: 13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO: 112, SEQ ID NO: 115, SEQ ID NO: 118, SEQ ID NO: 121, SEQ ID NO: 124, SEQ ID NO:127, SEQ ID NO:130, SEQ ID NO:133, SEQ ID NO:136, SEQ ID NO:139, SEQ ID NO:142, SEQ ID NO:145. SEQ ID NO: 148. SEQ ID NO:151. SEQ ID NO:154. Also preferred are allelic variants of such nucleic acid molecules.

Knowing a nucleic acid molecule of a δ subunit protein of the present invention allows one skilled in the art to make copies of that nucleic acid molecule as well as to obtain a nucleic acid molecule including additional portions of δ subunit protein-encoding genes (e.g., nucleic acid molecules that include the translation start site and/or transcription and/or translation control regions), and/or δ subunit nucleic acid molecule homologues. Knowing a portion of an amino acid sequence of a δ subunit protein of the present invention allows one skilled in the art to clone nucleic acid sequences encoding such a δ subunit protein. In addition, a desired δ subunit nucleic acid molecule can be obtained in a variety of ways including screening appropriate expression libraries with antibodies which bind to δ subunit proteins of the present invention; traditional cloning techniques using oligonucleotide probes of the present invention to screen appropriate libraries or DNA; and PCR amplification of appropriate libraries, or RNA or DNA using oligonucleotide primers of the present invention (genomic and/or cDNA libraries can be used). Illustrative examples are given in the Examples section.

The present invention also includes nucleic acid molecules that are oligonucleotides capable of hybridizing, under stringent conditions, with complementary regions of other, preferably longer, nucleic acid molecules of the present invention that encode at least a portion of a δ subunit protein. Oligonucleotides of the present invention can be RNA, DNA, or derivatives of either. The minimal size of such oligonucleotides is the size required to form a stable hybrid between a given oligonucleotide and the complementary sequence on another nucleic acid molecule of the present invention. Minimal size characteristics are disclosed herein. The size of the oligonucleotide must also be sufficient for the use of the oligonucleotide in accordance with the present invention. Oligonucleotides of the present invention can be used in a variety of applications including, but not limited to, as probes to identify additional nucleic acid molecules, as primers to amplify or extend nucleic acid molecules or in therapeutic applications to inhibit δ subunit production. Such therapeutic applications include the use of such oligonucleotides in, for example, antisense-, triplex formation-, ribozyme- and/or RNA drug-based technologies. The present invention, therefore, includes such oligonucleotides and methods to interfere with the production of δ subunit proteins by use of one or more of such technologies.

### Natural, wild-type bacterial cells and Recombinant Molecules and Cells

The present invention also includes a recombinant vector, which includes a *holA* nucleic acid molecule of the present invention inserted into any vector capable of delivering the nucleic acid molecule into a host cell. Such a vector contains heterologous nucleic acid sequences, that is nucleic acid sequences that are not naturally found adjacent to *holA* nucleic acid molecules of the present invention. The vector can be either RNA or DNA, either prokaryotic or eukaryotic, and typically is a virus or a plasmid. Recombinant vectors can be used in the cloning, sequencing, and/or otherwise manipulating of *hol*A nucleic acid molecules of the present invention. One type of recombinant vector, herein referred to as a recombinant molecule and described in more detail below, can be used in the expression of nucleic acid molecules of the present invention. Preferred recombinant vectors are capable of replicating in the transformed cell. Preferred nucleic acid molecules to include in recombinant vectors of the present invention are disclosed herein.

As heretofore disclosed, one embodiment of the present invention is a method to produce a δ subunit protein of the present invention by culturing a cell capable of expressing the protein under conditions effective to produce the protein, and recovering the protein.

In a preferred embodiment, the cell to culture is a natural bacterial cell, and delta is isolated from these cells by resolution and reconstitution approaches where all of the required components of the DNA polymerase III holoenzyme are resolved and used to make an assay for one protein. That one protein, if present in limiting quantity, in the presence of saturating levels of the other proteins, permits DNA replication on long circlular DNA templates containing single primers to proceed at a level proportional to the amount of the limiting protein added. Such assays can be used to locate the protein during purification procedures and to assess it purity (ratio of activity/protein= specific activity). Examples of resolution and reconstitution are known in the art and are given in: Schekman, R., Weiner, A., and Kornberg, A., Science 186:987-993 (1974); and McHenry, C. S. and Kornberg, A., J. Biol. Chem. 252:6478-6484 (1977).

In another embodiment, a preferred cell to culture is a recombinant cell that is capable of expressing the δ subunit protein, the recombinant cell being produced by transforming a host cell with one or more nucleic acid molecules of the present invention. Transformation of a nucleic acid molecule into a cell can be accomplished by any method by which a nucleic acid molecule can be inserted into the cell. Transformation techniques include, but are not limited to, transfection, electraporation, microinjection, lipofection, adsorption, and protoplast fusion. A recombinant cell may remain unicellular or may grow into a tissue, organ or a multicellular organism. Transformed nucleic acid molecules of the present invention can remain extrachromosomal or can integrate into one or more sites within a chromosome of the transformed (i.e., recombinant) cell in such a manner that their ability to be expressed is retained. Preferred nucleic acid molecules with which to transform a host cell are disclosed herein.

Suitable host cells to transform include any cell that can be transformed and that can express the introduced δ subunit protein. Such cells are, therefore, capable of producing δ subunit proteins of the present invention after being transformed with at least one nucleic acid molecule of the present invention. Host cells can be either untransformed cells or cells that are already transformed with at least one nucleic acid molecule. Suitable host cells of the present invention can include bacterial, fungal (including yeast), insect, animal and plant cells. Preferred host cells include bacterial cells, with *E. coli* and *B. subtilis* cells being particularly preferred. Alternative preferred host cells are untransformed (wild-type) bacterial cells producing cognate δ subunit proteins, including attenuated strains with reduced pathogenicity, as appropriate.

A recombinant cell is preferably produced by transforming a host cell with one or more recombinant molecules, each comprising one or more nucleic acid molecules of the present invention operatively linked to an expression vector containing one or more transcription control sequences. The phrase operatively linked refers to insertion of a nucleic acid molecule into an expression vector in a manner such that the molecule is able to be expressed when transformed into a host cell. As used herein, an expression vector is a DNA or RNA vector that is capable of transforming a host cell and of effecting expression of a specified nucleic acid molecule. Preferably, the expression vector is also capable of replicating within the host cell. Expression vectors can be either prokaryotic or eukaryotic, and are typically viruses or plasmids. Expression vectors of the present invention include any vectors that function (i.e., direct gene expression) in recombinant cells of the present invention, including in bacterial, fungal, insect, animal, and/or plant cells. As such, nucleic acid molecules of the present invention can be operatively linked to expression vectors containing regulatory sequences such as promoters, operators, repressors, enhancers, termination sequences, origins of replication, and other regulatory sequences that are compatible with the recombinant cell and that control the expression of nucleic acid molecules of the present invention. As used herein, a transcription control sequence includes a sequence which is capable of controlling the initiation, elongation, and termination of transcription. Particularly important transcription control sequences are those which control transcription initiation, such as promoter, enhancer, operator and repressor sequences. Suitable transcription control sequences include any transcription control sequence that can function in at least one of the recombinant cells of the present invention. A variety of such transcription control sequences are known to the art. Preferred transcription control sequences include those which function in bacterial, yeast, insect and mammalian cells, such as, but not limited to, tac, lac, pA1 (Kim, D.R. and McHenry, C.S., *J*. *Biol*. *Chem.* 271:20G90-20698 (1996)) tzp, trc, oxy-pro, omp/lpp, rmB, bacteriophage lambda (.lambda.) (such as .lambda.p.sub.L and .lambda.p.sub.R and fusions that include such promoters), bacteriophage T7, T7lac, bacteriophage T3, bacteriophage SP6, bacteriophage SP01, naetallothionein, alpha mating factor, Pichia alcohol oxidase, alphavirus subgenomic promoters (such as Sindbis virus subgenomic promoters), baculovirus, Heliothis zea insect virus, vaccinia virus, herpesvirus, poxvirus, adenovirus, simian virus 40, retrovirus actin, retroviral long terminal repeat, Rous sarcoma virus, heat shock, phosphate and nitrate transcription control sequences as well as other sequences capable of controlling gene expression in prokaryotic or eukaryotic cells. Additional suitable transcription control sequences include tissue-specific promoters and enhancers as well as lymphokine-inducible promoters (e.g., promoters inducible by interferons or interleukins). Transcription control sequences of the present invention can also include naturally occurring transcription control sequences naturally associated with a DNA sequence encoding a δ subunit protein.

Expression vectors of the present invention may also contain secretory signals (i.e., signal segment nucleic acid sequences) to enable an expressed δ subunit protein to be secreted from the cell that produces the protein. Suitable signal segments include a δ subunit protein signal segment or any heterologous signal segment capable of directing the secretion of a δ subunit protein, including fusion proteins, of the present invention. Preferred signal segments include, but are not limited to, δ subunit, tissue plasminogen activator (t-PA), interferon, interleukin, growth hormone, histocompatibility and viral envelope glycoprotein signal segments.

Expression vectors of the present invention may also contain fusion sequences which lead to the expression of inserted nucleic acid molecules of the present invention as fusion proteins. Inclusion of a fusion sequence as part *of a hol*A nucleic acid molecule of the present invention can enhance the stability during production, storage and/or use of the protein encoded by the nucleic acid molecule. Furthermore, a fusion segment can function as a tool to simplify purification of a δ subunit protein, such as to enable purification of the resultant fusion protein using affinity chromatography. A suitable fusion segment can be a domain of any size that has the desired function (e.g., increased stability and/or purification tool). It is within the scope of the present invention to use one or more fusion segments. Fusion segments can be joined to amino and/or carboxyl termini of a δ subunit protein. Linkages between fusion segments and 6 subunit proteins can be constructed to be susceptible to cleavage to enable straightforward recovery of the δ subunit proteins. Fusion proteins are preferably produced by culturing a recombinant cell transformed with a fusion nucleic acid sequence that encodes a protein including the fusion segment attached to either the carboxyl and/or amino terminal end of a δ subunit protein.

A recombinant molecule of the present invention is a molecule that can include at least one of any nucleic acid molecule heretofore described operatively linked to at least one of any transcription control sequence capable of effectively regulating expression of the nucleic acid molecules in the cell to be transformed. A preferred recombinant molecule includes one or more nucleic acid molecules of the present invention, with those that encode one or more δ subunit proteins being particularly preferred. Preferred recombinant molecules of the present invention and their production are described in the Examples section. Similarly, a preferred recombinant cell includes one or more nucleic acid molecules of the present invention, with those that encode one or more δ subunit proteins. Preferred recombinant cells of the present invention include those disclosed in the Examples section.

It may be appreciated by one skilled in the art that use of recombinant DNA technologies can improve expression of transformed nucleic acid molecules by manipulating, for example, the number of copies of the nucleic acid molecules within a host cell, the efficiency with which those nucleic acid molecules are transcribed, the efficiency with which the resultant transcripts are translated, and the efficiency of post-translational modifications. Recombinant techniques useful for increasing the expression of nucleic acid molecules of the present invention include, but are not limited to, operatively linking nucleic acid molecules to high-copy number plasmids, integration of the nucleic acid molecules into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Dalgarno sequences), modification of nucleic acid molecules of the present invention to correspond to the codon usage of the host cell, deletion of sequences that destabilize transcripts, and use of control signals that temporally separate recombinant cell growth from recombinant protein production during fermentation. The activity of an expressed recombinant protein of the present invention may be improved by fragmenting, modifying, or derivatizing the resultant protein.

In accordance with the present invention, recombinant cells can be used to produce δ subunit proteins of the present invention by culturing such cells under conditions effective to produce such a protein, and recovering the protein. Effective conditions to produce a protein include, but are not limited to, appropriate media, bioreactor, temperature, pH and oxygen conditions that permit protein production. An appropriate, or effective, medium refers to any medium in which a cell of the present invention, when cultured, is capable of producing a δ subunit protein. Such a medium is typically an aqueous medium comprising assimilable carbohydrate, nitrogen and phosphate sources, as well as appropriate salts, minerals, metals and other nutrients, such as vitamins. The medium may comprise complex, nutrients or may be a defined minimal medium.

Cells of the present invention can be cultured in conventional fermentation bioreactors, which include, but are not limited to, batch, fed-batch, cell recycle, and continuous fermentors. Culturing can also be conducted in shake flasks, test tubes, microtiter dishes, and petri plates. Culturing is carried out at a temperature, pH and oxygen content appropriate for the recombinant cell. Such culturing conditions are well within the expertise of one of ordinary skill in the art.

Depending on the vector and host system used for production, resultant δ subunit proteins may either remain within the recombinant cell; be secreted into the fermentation medium; be secreted into a space between two cellular membranes, such as the periplasmic space in *E. coli;* or be retained on the outer surface of a cell or viral membrane. Methods to purify such proteins are disclosed in the Examples section.

### Antibodies

The present invention also includes isolated anti-δ subunit antibodies and their use. An anti-δ subunit antibody is an antibody capable of selectively binding to a δ subunit Isolated antibodies are antibodies that have been removed from their natural milieu. The term "isolated" does not refer to the state of purity of such antibodies. As such, isolated antibodies can include atrti-sera containing such antibodies, or antibodies that have been purified to varying degrees. As used herein, the term "selectively binds to" refers to the ability of such antibodies to preferentially bind to the protease against which the antibody was raised (i.e., to be able to distinguish that protease from unrelated components in a mixture). Binding affinities, commonly expressed as equilibrium association constants, typically range from about 10³ M⁻¹ to about 10¹² M⁻¹. Binding can be measured using a variety ofmethods known to those skilled in the art including immunoblot assays, immunoprecipitation assays, radioimmunoassays, enzyme immunoassays (e.g., ELISA), immunofluorescent antibody assays and immunoelectron microscopy, see, for example, Sambrook et al., ibid.

Antibodies of the present invention can be either polyclonal or monoclonal antibodies. Antibodies of the present invention include functional equivalents such as antibody fragments and genetically-engineered antibodies, including single chain antibodies, that are capable of selectively binding to at least one of the epitopes of the protein used to obtain the antibodies. Antibodies of the present invention also include chimeric antibodies that can bind to more than one epitope. Preferred antibodies are raised in response to proteins that are encoded, at least in part, by a δ subunit nucleic acid molecule of the present invention.

Anti-δ subunit antibodies of the present invention include antibodies raised in an animal administered a δ subunit. Anti-δ subunit antibodies of the present invention also include antibodies raised in an animal against one or more δ subunit proteins of the present invention that are then recovered from the cell using techniques known to those skilled in the art. Yet additional antibodies of the present invention are produced recombinantly using techniques as heretofore disclosed for δ subunit proteins of the present invention. Antibodies produced against defined proteins can be advantageous because such antibodies are not substantially contaminated with antibodies against other substances that might otherwise cause interference in a diagnostic assay or side effects if used in a therapeutic composition.

Anti-δ subunit antibodies of the present invention have a variety of uses that are within the scope of thus present invention. For example, such antibodies can be used in a composition of the present invention to passively immunize an animal in order to protect the animal from bacterial infection. Anti-δ subunit antibodies can also be used as tools to screen expression libraries and/or to recover desired proteins of the present invention from a mixture of proteins and other contaminants. Furthermore, antibodies of the present invention can be used to target cytotoxic, therapeutic or imaging agents to bacteria or bacteria-infected organs or tissues in order to kill bacteria, deliver therapeutic agents or localize imaging agents to bacteria-infected organs or tissues. Targeting can be accomplished by conjugating (i.e., stably joining) such antibodies to the cytotoxic, therapeutic or imaging agents using techniques known to those skilled in the art.

A preferred anti-δ subunit antibody of the present invention can selectively bind to, and preferentially reduce the DNA replication activity of a replicase comprising a δ subunit protein.

### Use of the proteins of the present invention

The present invention provides methods for the use of δ subunit proteins of the present invention in a reconstitution assay of DNA polymerase III holoenzyme (comprising α, β, DnaX, δ, and δ' subunits). Such methods are described in detail in United States Provisional Patent application Serial Nos. 60/244,023, filed October 27, 2000 and 60/290,725, filed May 14, 2001, incorporated by reference herein in their entirety.

The present invention also provides methods for the use of δ subunit proteins of the present invention derived from thermophilic bacteria for amplification of DNA, inlucluding polymerase chain reaction (PCR) and isothermal amplification, including, but not limited to, rolling circle amplification, for research (cloning and analytical) and for diagnostic purposes. Such methods are described in detail in WO 99/13060. Such δ subunit proteins include, but are not limited to those dervived from *Aquifex*, *Thermus, and Thermatoga* genuses. Specific examples are δ subunit protein from *Aquifex aeolicus* (SEQ ID NO:3, SEQ ID NO:226); *Thermus thermophilus* (SEQ ID NO:108), and *Thermatoga* maratima (SEQ ID NO:81).

In one embodiment, a thermostable polymerase is one that retains at least 50% of its activity upon incubation at 70°C for 5 minutes in a solution containing 50 mM Tris-HCl (pH 7.5), 100 mM potassium glutamate, 20% glycerol and 1 mM dithiothreitol. In another embodiment, a thermostable polymerase is one that retains at least 50% of its activity upon incubation at 60°C for 5 minutes in a solution containing 50 mM Tris-HCl (pH 7.5), 100 mM potassium glutamate, 20% glycerol and 1 mM dithiothreitol In preferred embodiments, a thermostable polymerase is one that retains at least 50% of its activity upon incubation at 60°C for 10 minutes in a solution containing 50 mM Tris-HCl (pH 7.5), 100 mM potassium glutamate, 20% glycerol and 1 mM dithiothreitol.

The present invention also includes the use of modulators of bacterial DNA replication that reduce the replication activity of a DNA polymerase III replicase comprising a δ subunit to reduce bacterial infection of animals, plants, humans and the surrounding environment. As used herein, modulators of bacterial DNA replication are compounds that interact with the replicase thereby altering the ability of replicase to replicate DNA. A preferred modulator inhibits replication.

The minimal assembly of the essential subunits of a bacterial replicase should permit rapid and processive synthesis of long stretches of DNA. In all bacterial replicases studied so far, the minimal functional holoenzyme consists of three components: *1*) the polymerase core, 2) the β processivity factor-loading (or clamp-loading) complex and 3) the sliding clamp processivity factor, β. In *E*. *coli,* the minimal holoenzyme consists of the α subunit, DnaX-δδ' clamp loading complex and the β subunit. The same components are minimally required for functional DNA Pol III holoenzyme from Gram-positive *S. pyogenes* and *T. thermophilus.* Given the generality of this requirement among distantly related organisms, it is expected that α, β, DnaX, δ, and δ' will be sufficient for the assembly of a replicase for most organisms. As used herein, DnaX complex is the term used to describe an assembly of DnaX, delta and delta'.

Target indications may include arresting cell growth or causing cell death resulting in recovery from the bacterial infection in animal studies. A wide variety of assays for activity and binding agents are provided, including DNA synthesis, ATPase, clamp loading onto DNA, protein-protein binding assays, immunoassays, cell based assays, etc. The replication protein compositions, used to identify pharmacological agents, are in isolated, partially pure or pure form and are typically, but not necessarily, recombinantly produced. The replication protein may be part of a fusion product with another peptide or polypeptide (e. g., a polypeptide that is capable of providing or enhancing protein-protein binding, stability under assay conditions (e. g., a tag for detection or anchoring), etc.). The assay mixtures comprise a natural intracellular replication protein-binding target such as DNA, another protein, NTP, or dNTP. For binding assays, while native binding targets may be used, it is frequently preferred to use portions (e. g., peptides, nucleic acid fragments) thereof so long as the portion provides binding affinity and avidity to the subject replication protein conveniently measurable in the assay. The assay mixture also comprises a candidate pharmacological agent. Generally, a plurality of assay mixtures are run in parallel with different agent concentrations to obtain a differential response to the various concentrations. Typically, one of these concentrations serves as a negative control (i.e., at zero concentration or below the limits of assay detection). Additional controls are often present such as a positive control, a dose response curve, use of known inhibitors, use of control heterologous proteins, etc. Candidate agents encompass numerous chemical classes, though typically they are organic compounds; preferably they are small organic compounds and are obtained from a wide variety of sources, including libraries of synthetic or natural compounds. A variety of other reagents may also be included in the mixture. These include reagents like salts, buffers, neutral proteins (e.g., albumin, detergents, etc.) which maybe used to facilitate optimal binding and/or reduce nonspecific or background intersactions, etc. Also, reagents that otherwise improve the efficiency of the assay (e.g., protease inhibitors, nuclease inhibitors, antimicrobial agents, etc.) may be used.

The invention provides replication protein specific assays and the binding agents including natural intracellular binding targets such as other replication proteins, etc., and methods of identifying and making such agents, and their use in a a variety of diagnostic and therapeutic applications, especially where disease is associated with excessive cell growth. Novel replication protein-specific antibodies and other natural intracellular binding agents identified with assays such as one- and two-hybrid screens, non-natural intracellular binding agents identified in screens of chemical libraries, etc.

Generally, replication protein-specificity of the binding agent is shown by equilibrium constants or values that approximate equilibrium constants such as the concentration that inhibits a reaction 50% (IC₅₀). Such agents are capable of selectively binding a replication protein (i.e., with an equilibrium association constant at least about 10⁷ M⁻¹, preferably, at least about 10⁸ M⁻¹, more preferably, at least about 10⁹ M⁻¹). A wide variety of cell based and cell-free assays may be used to demonstrate replication protein-specific activity, binding, gel shift assays, immunoassays, etc.

The resultant mixture is incubated under conditions whereby, but for the presence of the candidate pharmacological agent, the replication protein specifically binds the cellular binding target, portion, or analog. The mixture of components can be added in any order that provides for the requisite bindings.

Incubations may be performed at any temperature which facilitates optimal binding, typically between 4°C and 44°C, more commonly between 15°C and 37°C. Incubation periods are likewise selected for optimal binding but also minimized to facilitate rapid, high-throughput screening, and are typically between 0.1 and 10 hours, preferably less than 5 hours, more preferably less than 1 hour.

After incubation, the presence or absence of activity or specific binding between the replication protein and one or more binding targets is detected by any convenient way. For cell-free activity and binding type assays, a separation step may be used to separate the activity product or the bound from unbound components.

Separation may be effected by precipitation (e. g., immunoprecipitation or acid precipitation of a nucleic acid product), immobilization (e. g., on a solid substrate such as a microtiter plate), etc., followed by washing. Many assays that do not require separation are also possible such as use of europium conjugation in proximity assays, fluorescence increases upon double stranded DNA synthesis in the presence of dyes that increase fluorescence upon intercalating in double-stranded DNA, scintillation proximity assays or a detection system that is dependent on a reaction product or loss of substrate.

Detection may be effected in any convenient way. For cell-free activity and binding assays, one of the components usually comprises or is coupled to a label.

A wide variety of labels may be employed-essentially any label that provides for detection of DNA product, loss of DNA substrate, conversion of a nucleotide substrate, or bound protein is useful. The label may provide for direct detection such as radioactivity, fluorescence, luminescence, optical, or electron density, etc. or indirect detection such as an epitope tag, an enzyme, etc. The label may be appended to the protein (e.g., a phosphate group comprising a radioactive isotope of phosphorous), or incorporated into the DNA substrate or the protein structure (e.g., a methionine residue comprising a radioactive isotope of sulfur). A variety of methods may be used to detect the label depending on the nature of the label and other assay components. For example, the label may be detected bound to the solid substrate, or a portion of the bound complex containing the label may be separated from the solid substrate, and thereafter the label detected. Labels may be directly detected through optical or electron density, radioactive emissions, nonradiative energy transfer, fluorescence emission, etc. or indirectly detected with antibody conjugates, etc. For example, in the case of radioactive labels, emissions maybe detected directly (e.g., with particle counters) or indirectly (e.g., with scintillation cocktails and counters).

The present invention provides methods by which replication proteins from bacteria are used to discover new pharmaceutical agents. The function of replication proteins is quantified in the presence of different chemical test compounds. A chemical compound that inhibits the function is a candidate antibiotic.

In some embodiments it is prefereable to use replication protein subunits derived from a single organism. In other embodiments, the various subunits may be derived from one or more organisms. Replication proteins from Gram positive bacteria and from Gram negative bacteria can be interchanged for one another in certain embodiments. Hence, they can function as mixtures. Suitable *E. coli* replication proteins are the subunits of its Pol III holoenzyme which are described in U. S. Patent Nos. 5,583,026 and 5,668,004 to O'Donnell, which are hereby incorporated by reference.

Some other replication subunit proteins useful in the present invention are detailed in Table 3; "DnaX proteins identified from PSI-BLAST search of completely sequenced genomes;" Table 4, "δ' proteins identified in PSI-BLAST searches of all finished genomes at NCBI;" Table 5, "DnaX proteins from *S. pyogenes, S. pneumoniae* and *S. aureus;"* Table 6, "δ' proteins from *S. pyogenes, S. pneumoniae* and *S*. *aureus;"* Table 7, "δ proteins identified in PSI-BLAST searches of unfinished bacterial genomes at NCBI;" Table 8, "DnaX proteins corresponding to δ proteins identified from PSI-BLAST search of unfinished genomes at NCBI;" Table 9, "δ' proteins corresponding to δ proteins identified from PSI-BLAST search of unfinished genomes at NCBI;" Table 10, "DnaX proteins corresponding to δ proteins identified in BLAST searches of phylogenically related organisms;" Table 11, "δ' proteins corresponding to δ proteins identified in BLAST searches of phylogenically related organisms;" Table 12, "DnaE Proteins corresponding to δ proteins identified in PSI-BLAST searches of finished genomes at NCBI;" Table 13, "DnaE and PolC Proteins from *S. pyogenes, S. pneumoniae* and *S*. *aureus;"* Table 14, "DnaE and Pol C Proteins Corresponding to Additional δ Proteins Identified from PSI-BLAST search of Partially Sequenced Genomes," Table 15, "DnaE and PolC proteins corresponding to δ proteins identified in BLAST searches of phylogenically related organisms;" Table 16, "β Proteins corresponding to δ identified from PSI-BLAST search of completely sequenced genomes;" Table 17, "β Proteins from *S. pyogenes, S. pneumoniae* and *S. aureus;"* and Table 18, "β Proteins Corresponding to δ Proteins Identified in BLAST Searches of Phylogenically Related Organisms." Table 21, "β Proteins Corresponding to Additional δ Proteins Identified from PSI-BLAST Search of Partially Sequenced Genomes." For those organisms not represented in the aforementioned Tables, the likely reason is that the relevant region of the genome has not yet been sequenced. Once sequenced, the proteins can be found using methods outlined herein. In each of the aforementioned Tables, NCBI refers to the NCBI nr database, a compilation of non-redundant sequences. The accession numbers reported are not NCBI database accession numbers per se, but reference the database that compiled the sequences.

Some of the information detailed in Tables 3-18 and 21 is known in the art. Some of this information was obtained using the methods detailed herein in the Examples section, particulary Examples 1-3 and 5. Other subunit proteins useful in the present invention are detailed in Figures. Still other subunit proteins are well known in the art.

The present invention, in one embodiment provides a method of screening for a compound or chemical that modulates the activity of a DNA polymerase III replicase comprising, a) contacting an isolated replicase with at least one test compound under conditions permissive for replicase activity, b) assessing the activity of the replicase in the presence of the test compound, and c) comparing the activity of the replicase in the presence of the test compound with the activity of the replicase in the absence of the test compound, wherein a change in the activity of the replicase in the presence of the test compound is indicative of a compound that modulates the activity of the replicase, wherein said replicase comprises an isolated DNA polymerase III δ subunit protein of the present invention.

The present invention describes a method of identifying compounds which modulate the activity of a DNA polymerase III replicase. This method is carried out by forming a reaction mixture that includes a primed DNA molecule, a DNA polymerase III replicase, a candidate compound, a dNTP or up to 4 different dNTPs, ATP, and optionally either aβ subunit, a DnaX complex, or both the β subunit and the DnaX complex; subjecting the reaction mixture to conditions effective to achieve nucleic acid polymerization in the absence of the candidate compound; analyzing the reaction mixture for the presence or absence of nucleic acid polymerization extension products and comparing the activity of the replicase in the presence of the test compound with the activity of the replicase in the absence of the test compound. A change in the activity of the replicase in the presence of the test compound is indicative of a compound that modulates the activity of the replicase. The DnaX complex comprises a δ subunit protein of the present invention.

The present invention describes a method to identify candidate pharmaceuticals that modulate the activity of a DnaX complex and a β subunit in stimulating the DNA polymerase. The method includes contacting a primed DNA (which may be coated with SSB) with a DNA polymerase, a β subunit, and a DnaX complex (or subunit or subassembly of the DnaX complex) in the presence of the candidate pharmaceutical, and dNTPs (or modified dNTPs) to form a reaction mixture. The reaction mixture is subjected to conditions which, in the absence of the candidate pharmaceutical, would effect nucleic acid polymerization and the presence or absence of the extension product in the reaction mixture is analyzed. The nucleic acid polymerization in the presence of the test compound is compared with the nucleic acid polymerization in the absence of the test compound. A change in the nucleic acid polymerization in the presence of the test compound is indicative of a compound that modulates the activity of a DnaX complex and a β subunit. The DnaX complex comprises a δ subunit of the present invention.

The present invention describes a method to identify chemicals that modulate the ability of a β subunit and a DnaX complex (or a subunit or subassembly of the DnaX complex) to interact. This method includes contacting the β subunit with the DnaX complex (or subunit or subassembly of the DnaX complex) in the presence of ATP (or a suitable ATP analog, such as ATPγS, a non-hydrolyzable analog of ATP, that enables interaction of β with the DnaX complex (or subunit or subassembly of the DnaX complex) the candidate pharmaceutical to form a reaction mixture. The reaction mixture is subjected to conditions under which the DnaX complex (or the subunit or subassembly of the DnaX complex) and the β subunit would interact in the absence of the candidate pharmaceutical The reaction mixture is then analyzed for interaction between the β subunit and the DnaX complex (or the subunit or subassembly of the DnaX complex). The extent of interaction in the presence of the test compound is compared with the extent of interaction in the absence of the test compound. A change in the interaction between the β subunit and the DnaX complex (or the subunit or subassembly of the DnaX complex) is indicitive of a compound that modulates the interaction. The DnaX complex comprises a δ protein of the present invention.

The present invention describes a method to identify chemicals that modulate the ability of a δ subunit and the δ' and/or DnaX subunit to interact. This method includes contacting the δ subunit with the δ' and/or δ' plus DnaX subunit in the presence of the candidate pharmaceutical to form a reaction mixture. The reaction mixture is subjected to conditions under which the δ subunit and the δ' and/or δ' plus DnaX subunit would interact in the absence of the candidate pharmaceutical. The reaction mixture is then analyzed for interaction between the δ subunit and the δ' and/or DnaX subunit. The extent of interaction in the presence of the test compound is compared with the extent of interaction in the absence of the test compound. A change in the interaction between the δ subunit and the δ' and/or DnaX subunit is indicitive of a compound that modulates the interaction.

The present invention describes a method to identify chemicals that modulate the ability of a DnaX complex (or a subassembly of the DnaX complex) to assemble a β subunit onto a DNA molecule. This method involves contacting a circular primed DNA molecule (which may be coated with SSB) with the DnaX complex (or the subassembly thereof and the β subunit in the presence of the candidate pharmaceutical, and ATP or dATP to form a reaction mixture. The reaction mixture is subj ected to conditions under which the DnaX complex (or subassembly) assembles the β subunit on the DNA molecule absent the candidate pharmaceutical. The presence or absence of the β subunit on the DNA molecule in the reaction mixture is analyzed. The extent of assembly in the presence of the test compound is compared with the extent of assmbly in the absence of the test compound. A change in the amount of β subunit on the DNA molecule is indicative of a compound that modulates the ability of a DnaX complex (or a subassembly of the DnaX complex) to assemble a β subunit onto a DNA molecule. The DnaX complex comprises a delta protein of the present invention.

The present invention describes a method to identify chemicals that modulate the ability of a DnaX complex (or a subunit (s) of the DnaX complex) to disassemble a β subunit from a DNA molecule. This method comprises contacting a DNA molecule onto which the β subunit has been assembled in the presence of the candidate pharmaceutical, to form a reaction mixture. The reaction mixture is subjected to conditions under which the DnaX complex (or a subunit (s) or subassembly of the DnaX complex) disassembles the β subunit from the DNA molecule absent the candidate pharmaceutical. The presence or absence of the β subunit on the DNA molecule in the reaction mixture is analyzed. The extent of assembly in the presence of the test compound is compared with the extent of assembly in the absence of the test compound. A change in the amount of β subunit on the DNA molecule is indicative of a compound that modulates the ability of a DnaX complex (or a subassembly of the DnaX complex) to disassemble a β subunit onto a DNA molecule. The DnaX complex comprises a δ subunit of the present invention.

The present invention describes a method to identify chemicals that modulate the dATP/ATP binding activity of a DnaX complex or a DnaX complex subunit (e. g. DnaX subunit). This method includes contacting the DnaX complex (or the DnaX complex subunit) with dATP/ATP either in the presence or absence of a DNA molecule and/or the β subunit in the presence of the candidate pharmaceutical to form a reaction. The reaction mixture is subjected to conditions in which the DnaX complex (or the subunit of DnaX complex) interacts with dATP/ATP in the absence of the candidate pharmaceutical. The reaction is analyzed to determine if dATP/ATP is bound to the DnaX complex (or the subunit of DnaX complex) in the presence of the candidate pharmaceutical. The extent of binding in the presence of the test compound is compared with the extent of binding in the absence of the test compound. A change in the dATP/ATP binding is indicative of a compound that modulates the dATP/ATP binding activity of a DnaX complex or a DnaX complex subunit (e.g. DnaX subunit). The DnaX complex comprises a δ subunit of the present invention.

The present invention describes a method to identify chemicals that modulate the dATP/ATPase activity of a DnaX complex or a DnaX complex subunit (e.g., the DnaX subunit). This method involves contacting the DnaX complex (or the DnaX complex subunit) with dATP/ATP either in the presence or absence of a DNA molecule and/or a β subunit in the presence of the candidate pharmaceutical to form a reaction mixture. The reaction mixture is subjected to conditions in which the DnaX subunit (or complex) hydrolyzes dATP/ATP in the absence of the candidate pharmaceutical. The reaction is analyzed to determine if dATP/ATP was hydrolyzed. The extent of hyrdolysis in the presence of the test compound is compared with the extent of hydrolysis in the absence of the test compound. A change in the amount of dATP/ATP hydrolyzed is indicative of a compound that modulates the dATP/ATPase activity of a DnaX complex or a DnaX complex subunit (e. g., the DnaX subunit). The DnaX complex comprises a δ protein of the present invention.

The invention provides a further method for identifying chemicals that modulate the activity of a DNA polymerase comprising contacting a circular primed DNA molecule (may be coated with SSB) with a DnaX complex, a β subunit and an a subunit in the presence of the candidate pharmaceutical, and dNTPs (or modified dNTFs) to form a reaction mixture. The reaction mixture is subjected to conditions, which in the absence of the candidate pharmaceutical, affect nucleic acid polymerization, and the presence or absence of the extension product in the reaction mixture is analyzed. The activity of the replicase in the presence of the test compound is compared with the activity of the replicase in the absence of the test compound. A change in the activity of the replicase in the presence of the test compound is indicative of a compound that modulates the activity of the replicase. The DnaX complex comprises a δ protein of the present invention.

The present invention also includes a test kit to identify a compound capable of modulating DNA polymerase III replicase activity. Such a test kit includes an isolated δ subunit protein and a means for determining the extent of modulation of DNA replication activity in the presence of (i.e., effected by) a putative inhibitory compound.

The present invention also includes modulators and inhibitors isolated by such a method, and/or test kit, and their use to inhibit any replicase comprising a δ subunit that is susceptible to such an inhibitor. Modulators of bacterial DNA replication can also be used directly as to treat animals, plants and humans suspected of having a bacterial infection as long as such compounds are not harmful to the species being treated. Similarly, molecules that bind to any replicase comprising a δ subunit, incuding but not limited to antibodies and small molecules, can also be used to target cytotoxic, therapeutic or imaging entities to a site of infection.

With the availability of the essential DNA polymerase III holoenzyme subunits purified by a reconstitution approach, replication methods of the invention are amenable to a high-throughput screen against the new target provided. This can be done by titrating each component until it is barely limiting so that modulation will be observed upon interaction with active compounds within small molecule libraries. Buffer conditions will be optimized to ensure stability of the enzyme mix, stored in a separate chilled container, during the multiplate screening process. The time and temperature of the assay will also be reoptimized to ensure a stable linear response. Similarly, concentrations of all the relevant components of the assay will be optimized to ensure high sensitivity of the assay to perturbation by test compounds.

The methods are amenable to automated, cost-effective high-throughput screening of chemical libraries for lead compounds. Identified reagents find use in the pharmaceutical industries for animal and human trials as well as agricultural industries for plant pathogens; for example, the reagents may be derivatized and rescreened in in *vitro* and in *vivo* assays to optimize activity and minimize toxicity for pharmaceutical, animal care or agricultural product development.

### EXAMPLES

### Example 1. General Strategy

Identification of members of a related family of bacterial DnaX complex subunits (DnaX, δ' and δ of DNA polymerase III holoenzymes from organisms whose genomes have been completely sequenced and deposited at the National Center for Biotechnology Information (NCBI) is possible using the computer program PSI-BLAST (Position-Specific Iterated BLAST) (Altschul, et al., Nucleic Acids Res. 25:3389-402 (1997)). PSI-BLAST (or ψ-BLAST) is a program in which a profile (or position specific scoring matrix, PSSM) is constructed from a multiple alignment of the highest scoring hits in an initial BLAST (Basic Local Alignment Search Tool) search (Altschul, et al., J. Mol. Biol. 215:403-410 (1990)). BLAST is a set of similarity search programs designed to explore all of the available sequence databases regardless of whether the query is protein or DNA.

The ψ-BLAST profile contruct from BLAST searches is used to perform a second (and successive) search and the results of each "iteration" used to refine the profile. This iterative searching strategy results in increased sensitivity. BLAST and PSI-BLAST can be accessed at the NCBI web site accessible by Microsoft Explorer, Netscape or other suitable web browser. Rules or criteria are presented to allow for the identification of bacterial DnaX, δ' and δ.

The DnaX proteins from targeted organisms are first identified. Next, the δ' subunits from those organisms are identified. δ' subunits exhibit sequence similarity with DnaX, but lack certain conserved motifs found only in DnaX proteins. Finally, the δ subunits from the organisms are identified. δ subunits are less conserved but two procedures have been developed that permit their unambiguous identification.

### Example 2. Identification of DnaX Sequences from Completely Sequenced Genomes

Identification of DnaX proteins: The sensitivity of the initial BLAST search and ensuing iterations may be manipulated by settings found in the "options" and "format" portions of the search window. The settings are described in the steps listed below and were used in searches for DnaX, δ', and δ.

Step 1: Search criteria should be set in "format" to these settings:
- Set inclusion threshold (E or Expect value) to 0.001 or 0.002
   Search criteria should be set in "option" to these settings:
- Set matrix to Blosum62
- Set gap cost to Existence: 11 Extension: 1
- Set expect to 10
Information regarding the input parameters is available from the BLAST web page available throught NCBI. The statistical significance threshold for including a sequence in the model used by PSI-BLAST to create the PSSM on the next iteration.

For example, different substitution matrices are tailored to detecting similarities among sequences that are diverged by differing degrees. A single matrix may nevertheless be reasonably efficient over a relatively broad range of evolutionary change. Experimentation has shown that the BLOSUM-62 matrix is among the best for detecting most weak protein similarities. A detailed statistical theory for gapped alignments has not been developed, and the best gap costs to use with a given substitution matrix are determined empirically; however, it is suggested that an initial search start with Existence: 10, Extension: 1.

"Expect", also referred to as an 'E value' or 'E' in this application is the statistical significance threshold for reporting matches against database sequences. When Expect is set to 10, 10 matches are expected to be found merely by chance, according to the stochastic model of Karlin and Altschul (1990). With Expect set to 0.001, there is a 1 in 1000 chance for an inappropriate hit, based on the referenced stochastic modeL If the statistical significance ascribed to a match is greater than the Expect threshold, the match will not be reported. Lower Expect thresholds are more stringent, leading to fewer chance matches being reported. Increasing the threshold shows less stringent matches. Fractional values are acceptable.

Step 2: There are also settings in the "option" portion of the search window to limit the search to the genomes of specific organisms. In the window labeled "Limit by entrez query" the names of the organisms to which you are limiting your search must be typed or pasted. Entrez is a retrieval system for searching several linked databases, by entering specific organism here the search is limited to searching for protein sequences similar to the query sequence only from the specified organisms. The search was limited to only those organisms that have the genomic DNA completely sequenced and available at National Center for Biotechnology Information. The listing for these organisms can be found at the NCBI web site. The search for proteins in the examples described in this application was limited to the 34 organisms listed below.

*Aquifex* aeolicus[Organism] OR *Bacillus halodurans*[Organism] OR *Bacillus* sublilis[Orgamsm] OR Buchnera[Organism] OR *Borrelia burgdorferi*[Organism] OR *Campylobacterjejuni*[Organism] OR *Caulobacter crescentus*[Organism] OR *Chlamydia pneumoniae*[Organism] OR *Chlamydia muridarum*[Organism] OR *Chlamydia trachomatis*[Organism] OR *Deinococcus radiodurans*[Organism] OR *Escherichia coli*[Organism] OR *Escherichia coli K12*[Organism] OR *Escherichia coli 0157*[Organism] OR *Haemophilus influenzae*[Organism] OR *Helicobacter pylori 26695*[Organism] OR *Helicobacter pylori J99*[Organism] OR *Lactococcus lactis*[Organism] OR *Mesorhizobium loti*[Organism] OR *Mycobacterium leprae*[Organism] OR *Mycobacterium tuberculosis*[Organism] OR *Mycoplasma genitalium*[Organism] OR *Mycoplasma pneumoniae*[Organism] OR *Neisseria meningitidis MC58*[Organism] OR *Neisseria meningitidis Z249.1* [Organism] OR *Pasteurella multocida*[Organism] OR *Pseudomonas aeruginosa[Organism]* OR *Rickettsia prowazekii*[Organism] OR *Synechocystis*[Organism] OR *Thermotoga* maritima[Organism] OR *Treponema pallidum*[Organism] OR *Ureaplasma urealyticum*[Organism] OR *Vibrio cholerae*[Organism] OR *Xylella fastidiosa* [Organism]

The format for entering organisms must be as shown. The entire name of the organism must be spelled out. Using the abbreviated form of a name (i.e. *E. coli)* is not permitted. The name can be followed by a strain designation (i.e. *Neisseria meningitidis* Z2491), but not by a designation for an entire species (i.e. *Buchnera* but not *Buchnera sp.).* Also, the name must be followed by a classification enclosed by brackets (i.e. [Organism] or [orgn]) with no spaces separating the name from the bracket.

Step 3: The amino acid sequence of a query protein must be entered into the search box. The initial BLAST search was against this initial amino acid query sequence. There is a window (search) within the initial search window to specify the query sequence, In this search, the amino acid sequence for *E. coli* DnaX (SEQ ID. NO.200) was used (shown below).
MSYQVLARKWRPQTFADVVGQEHVLTALANGLSLGRIHHAYLFSGTRGVGKTSIAR LLAKGLNCETGITATPCGVCDNCREIEQGRFVDLIEIDAASRTKVEDTRDLLDNVQYA PARGREKXVYLIDEVHMLSRHSFNALLKTLEEPPEHVKFLLATTDPQKLPVTILSRCLQ FHLKALDVEQIRHQLEHILNEEHIAHEPRALQLLARAAEGSLTDALSLTDQAIASGDG QVSTQAVSAMLGTLDDDQALSLVEAMVEANGERVMALINEAAARGIEWEALLVEM LGLLHRIAMVQLSPAALGNDMAAIELRMRELARTIPPTDIQLYYQTLLIGRKELPYAP DRRMGVEMPTLLRALAFHPRMPLPEPEVPRQSFAVAPTAVMTPTQVPPQPQSAPQQ APTVPLPETTSQVLAARQQLQRVQGATKAKKSEPAAATRARPVNNAALERLASVTD RVQARPVPSALEKAPAKKEAYRWKATTPVMQQKEVVATPKALKKALEHEKTPELA AKLAAEAIERDPWAAQVSQLSLPKLVEQVALNAWKEESDNAVCLHLRSSQRHLNNR GAQQKLAEALSMLKGSTVELTIVEDDNPAVRTPLEWRQAIYEEKLAQARESITADNNI QTLRRFFDAELDEESIRPI
The PSI-BLAST search returned 143 hits. Hits are shown in descending similarity to the *E. coli* query sequence, and typically proteins similar to the *E*. *coli* query protein are among the first hits.

### Step 4: Criteria used to select hits that are DnaX proteins.

ATP binding proteins share local similarities required for nucleotide binding and hydrolysis called the Walker A and Walker B motifs. The Walker A nucleotide binding fold consensus sequence is G(X)₄GK(T/S)(X)₆(I/V) (SEQ ID NO:201 (where X is any amino acid) and the Walker B binding fold consensus sequence is (R/K)(X)₃G(X)₃L(hydrophobic)₄D (SEQ ID NO:202) as described by Walker, J.E. et al., EMBO J. 1:945-951 (1982). When two amino acids are indicated in parentheses separated by a / mark, that indicates either amino acid may appear at that position. DnaX proteins as part of this group of ATP binding proteins contain the defined conserved residues of the Walker A motif. However, there are additional conserved residues that are used for verification of DnaX that are expanded for DnaX beyond the Walker A motif and this entire region will be referred to as the 'DnaX Walker A' sequence. Conserved residues in DnaX proteins that encompass the Walker B motif have also been expanded for identification and verification purposed and will be referred to as the 'DnaX Walker B'. Conservation of these residues is criteria used for selection of DnaX proteins.
*i.* Select proteins that contain the required strictly conserved residues indicated for DnaX from the parameters listed below. Possible DnaX, δ' and δ must be aligned with the corresponding *E*. *coli* protein (in a pairwise alignment) using CLUSTAL W (Thompson, J.D. et aL, Nucleic Acids Res. 22:4673-4680 (1994)). In these searches CLUSTAL W was used as part of Vector NTI v. 5.2.1.3. The "slow pairwise alignment algorithm" was used with the residue substitution matrix set to "Blosum", the gap opening penalty set to 10, and the gap extension penalty set to 0.1. Numbers indicate the N-terminal and C-terminal residues in *E. coli* numbering. When there is more than one residue in parenthesis and separated by a slash, any of these residues may correspond to a conserved residue. To be scored, a residue must align with the conserved *E. coli* residue at the position strictly according to *E*. *coli* numbering even though gaps may be inserted between defined positions for optimal alignment (in either the *E*. *coli* sequence or target sequence) by the CLUSTAL W program. Residues designated as X can be any amino acid and the number of X's indicate the number of amino acids between defined conserved residues (in the *E. coli* numbering system).
   a. Conserved residues (indicated with boldface letters) encompassing the DnaX Walker A motif. Six of the eleven conserved residues must be present. These six conserved residues must include the K(T/S) residues at position 51 and 52:39-**HAYLFSGXXGXGK(T/S)-52** SEQ ID NO:203)
   b. Conserved residues between the DnaX Walker A and B motifs. Seven of the ten conserved residues must be present: 88-(L/I/V)D(LI/I/V)(L/I/V)E(L/I/V)D(A/G)AS-97 (SEQ ID NO:204)
   c. Conserved residues encompassing the DnaX Walker B motif. Nine of the eleven conserved residues must be present. These nine conserved residues must include the D residue at position 126: 121-(K/R)(L/I/V)(Y/F)(M/V)(L/I/V)DEXHM(L/I/V)(S/T)-132 (SEQ ID NO:205)
Some of these conserved DnaX residues have been reported in the scientific literature (Walker, J.R., et al., J. Bacteriol. 182:6106-6113 (2000); Neuwald, A.F., et al., Genome Res. 9:27-43 (1999))

The initial search (iteration) gave 143 total hits with 90 above threshold and 53 below threshold. DnaX from all genomes searched were contained within the first 40 hits. The remaining hits in the top forty contained duplicate sequences. The accession numbers and GI numbers for the sequences of the DnaX proteins identified are shown in Table 3.

### Step 5. Perform additional iterations if DnaX from all target organisms were not identified.

i. Limit the hits for further searches (iterations) to sequences with E-values greater than threshold and sequences selected as DnaX proteins in step 4. DnaX proteins identified in additional iterations must meet criteria outlined in 4i.

### Example 3. Identification of δ' Proteins From Completely Sequenced Genomes

The search for δ' was preformed using PSI-BLAST as in the search for DnaX proteins. DnaX and δ' have regions of similarity; therefore, the DnaX proteins identified in the DnaX search were excluded.

**Step 1**. Set search criteria in PSI-BLAST as defined in step 1 of the DnaX search (Example 2).

**Step 2.** Limit the search by Entrez query to only those organisms that have the genomic DNA completely sequenced and available at National Center for Biotechnology Information. To exclude DnaX proteins as possible hits they must be exclude in the window labeled "Limit by entrez query" by listing the GenBank identification (GI) numbers following the list of organism the search is limited to as shown below. All duplicated proteins with different GI numbers shown in PSI-BLAST alignments must also be excluded. The search for proteins was limited to the organisms listed below, with DnaX proteins excluded by GI number as shown below.

*Aguifex* aeolicus[Organism] OR *Bacillus halodurans*[Organism] *OR Bacillus subtilis*[Organism] OR Buchnera[Organism] OR *Borrelia burgdorferi*[Organism] OR *Campylobacterjejuni*[Organism] OR *Caulobacter crescentus*[Organism] OR *Chlamydia pneumoniae*[Organism] OR *Chlamydia muridarum*[Organism] OR *Chlamydia trachomatis*[Organism] OR *Deinococcus radiodurans*[Organism] OR *Escherichia coli*[Organism] OR *Escherichia coli K12*[Organism] OR *Escherichia coli O157*[Organism] OR *Haemophilus influenzae*[Organism] OR *Helicobacter pylori 26695*[Organism] OR *Helicobacter pylori J99*[Organism] OR *Lactococcus lactis*[Organism] OR *Mesorhizobium loti*[Organism] OR *Mycobacterium* leprae[Organism] OR *Mycobacterium tuberculosis*[Organism] OR *Mycoplasma genitalium*[Organism] OR *Mycoplasma pneumoniae[Orgaxnem]* OR *Neisseria meningitidis MC58*[Organism] OR *Neisseria meningitidis Z2491* [Organism] OR *Pasteurella* multocida[Organism] OR *Pseudomonas aeruginosa*[Organism] OR *Rickettsia prowazekii*[Organism] OR *Synechocystis*[Organism] OR *Thermotoga* maritima[Organism] OR *Treponema pallidum*[Organism] OR *Ureaplasma urealyticum[Organism]* OR *Vibrio cholerae[Organism] OR Xylella fastidiosa*[Organism] NOT (7514776[gi] OR 98292[gi] OR 7463153[gi] OR 11346858[gi] OR 7468911 [gi] OR 7471826[gi] OR 1786676[gi] OR 1075209[gi] OR 7464043[gi] OR 3150103[gi] OR 1293572[gi] OR 7477980[gi] OR 1361493[gi] OR 2146109[gi] OR 11261620[gi] OR 7467603[gi] OR 11348451[gi] OR OR 7469304[gi] OR7462368[gi] OR 2583049[gi] OR 7521020[gi] OR 11356842[gi] OR 11261617[gi] OR 11261615[gi] OR 12720609[gi] OR 12513340[gi] OR 118808[gi] OR 1169397[gi] OR 10039144[gi] OR 10172646[gi] OR 118807[gi] OR 9789547[gi] OR 7468224[gi] OR 8978415[gi] OR 12725258[gi] OR 13357643[gi] OR 6014997[gi] OR 2494198[gi] OR 12045280[gi] OR 581255[gi] OR 13474589[gi] OR 13421402[gi] OR 7464040[gi] OR 11360902[gi] OR 13508357[gi] OR 13359980[gi] OR 67058[gi] OR 41291[gi] OR 43320[gi] OR 145297[gi] OR 1773152[gi] OR 1574159[gi] OR 9655520[gi] OR 9947490[gi] OR 9106885[gi] OR 7380298[gi] OR 580855[gi] OR 467409[gi] OR 580914[gi] OR 2632286[gi] OR 6968590[gi] OR 2984127[gi] OR 4981209[gi] OR 4155207[gi] OR 2313841 [gi] OR 4376294[gi] OR 7189650[gi] OR 3328753[gi] OR 7190650[gi] OR 13093951[gi] OR 3323329[gi] OR 2688379[gi] OR 3861390[gi] OR 6899039[gi] OR 6460225[gi] OR 2960145[gi] OR 1673890[gi] OR 1352305[gi] OR 3845015[gi] OR 1652627[gi])

The format for listing organisms is as described for DnaX proteins. Proteins to be excluded are listed by GI numbers followed without spaces by brackets enclosing the term "gi".

**Step 3.** The amino acid sequence of a query protein must be entered into the search box. In this search, the amino acid sequence for *E. coli* δ' was used (shown below).. MRWYPWLRPDFEKLVASYQAGRGHHALLIQALPGMGDDALIYALSRYLLCQQPQG HKSCGHCRGCQLMQAGTHPDYYTLAPBKGKNTLGVDAVRBVTBKLNBHARLGGAK VVWVTDAALLTDAAANALLKTLEEPPAETWFFLATREPERLLATLRSRCRLHYLAPP PEQYAVTWLSREVTMSQDALLAALRLSAGSPGAALALFQGDNWQARETLCQALAY SVPSGDWYSLLAALNHEQAPARLHWLATLLMDALKRHHGAAQVTNVDVPGLVAE LANHLSPSRLQAILGDVCHIREQLMSVTGINRELLITDLLLRIEHYLQPGVVLPVPHL

The Psi-Blast search returned 42 hits listed in decreasing similarity, with 32 hits having E-values greater than threshold.

### Step 4. Criteria used to select hits that are δ'.

i. Select only proteins that contain conserved residues for δ' from the parameters listed in a. and b. below. Numbers indicate the N-terminal and C-terminal residues in *E. coli* δ' numbering. To be scored, a residue must align with the conserved *E*. *coli* residue (in a pairwise alignment) at the position strictly according to *E. coli* numbering even though gaps may be inserted between defined positions for optimal alignment (in either the *E. coli* sequence or target sequence) by the CLUSTAL W program. Residues designated as X can be any amino acid.
   a. δ' molecules must contain eight of the twelve conserved residues in the conserved region 124-(A/S)AN(A/S)(L/I/V)(L/I/V)(K/R)X(L/I/V)E(E/D)PP-136. (SEQ ID NO:207)
   b. δ' molecules must contain four of the six conserved residues in the conserved region 151-(L/I/V)(L/I/V)XT(L/I/V)XSR-158 (SEQ ID NO:208).
*ii.* Exclude proteins containing parameters outlined in 4ib in the search for DnaX proteins above (Example 2).

In the initial search (iteration) δ' was identified for 29 of the organisms searched (1 hit was a duplicate). The δ'-subunits for four remaining organisms were noted as having E-values less than threshold (giving a total of 33 δ' identified).
Some of these concerved residues have been reported (Neuwald, A.F., et al., Genome Res. 9:27-43 (1999))

Step 5. Perform additional iterations if δ' from all target organisms were not identified.
i. Limit the hits for further searches (iterations) to sequences with E-values greater than threshold and sequences selected as δ' proteins in step 4.
*ii*. δ' proteins identified in additional iterations must meet criteria outlined in step 4i for identification of δ'.
iii. Repeat step 5 until δ' from all specified organisms have been identified.

The second iteration gave 317 total hits and indicated that the δ'-subunits from the four organisms with E-values below threshold now contained E-values greater than threshold. The δ' from the final (34th) species was also identified above threshold. The accession numbers and GI numbers for these sequences are provided in Table 4.

### Example 4. Identification of δ Proteins From Completely Sequenced Genomes

As indicated above, δ proteins are not well conserved, and thus are not searchable through standard techniques. For example, a simple BLAST search of NCBI databases for δ proteins based on the known *E. coli* sequence returns results for only a small number of organisms: *Vibrio cholerae, Pasteurella multocida, Haemophilus influenzae, Buchnera sp. APS, Pseudomonas aeruginosa, Xylella fastidiosa,* from the gamma proteobacter division, and *Neisseria meningitides Z2491* from the beta division of proteobacteria. No sequences from the alpha or epsilon divisions of proteobacteria are retrieved.

Two related methods were used to identify delta sequences using Psi-Blast. The first (method A) as described in our provisional application filed July 14, 2000, uses ψ-Blast starting with the *E*. *coli* δ sequence (accession number P28630; GI number GI123452). Candidate identified sequences are restricted by elimination of DnaX sequences and δ' sequences, by the requirement that candidate δ sequences be between 300 and 400 amino acids long and that they be the 'best' hit (lowest E score) from an organism after elimination of DnaX and δ' sequences and proteins less than 300 amino acids or greater than 400. In the second method (method B), the sequences identified by method A were used to perform an alignment enabling identification of certain conserved residues present in all δ sequences. These conserved residues were included in the δ identification criteria as described below. This permitted elimination of the length criteria, permitting δ sequences to be identified that fell ourside of the 300-400 amino acid length or proteins resulting from genes that had errors in their reported sequences, resulting in a deduced protein sequence incorrectly documented as being shorter than 300 amino acids or longer than 400 amino acids.

### Method A.

**Step 1.** Perform a ψ-blast search using the NCBI website and the NCBI nr database with the 'E value' threshold set to 0.001. The delta sequences from *Vibrio cholerae, Pasteurella multocida, Haemophilus influenzae, Buchnera sp. APS, Pseudomonas aeruginosa, Xylella fastidiosa,* and *Neisseria meningitides* are identified and with E values lower than the threshold value. If the E value was set at 0.002 instead of 0.001, deltas subunit proteins from the same seven organisms were still identified.

**Step 2.** Hits are examined and those for DnaX proteins (generally annotated as DnaX protein, τ and/or γ protein, etc. in their respective databases and found to give a better match (lower E score when searched against *E. coli* DnaX sequences than with δ sequence)) and δ' (generally annotated as *hol*B gene product, delta' or DnaX-like protein, τ/γ-like protein, etc. in their respective databases and found to give a better match (lower E score when searched against *E. coli* δ' sequences than with δ sequence)) were eliminated. Since these methods address bacterial δ sequences and their cognate replicases, sequences from organisms other than bacteria were ignored.

**Step 3.** The length of the encoded candidate δ proteins were examined and only those between 300 and 400 amino acids were further considered.

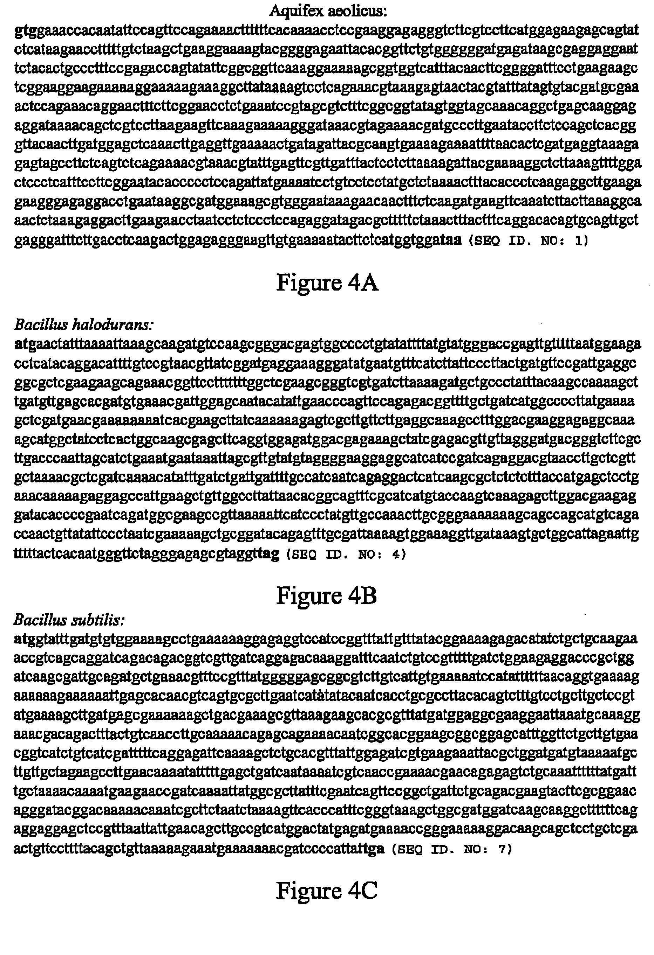

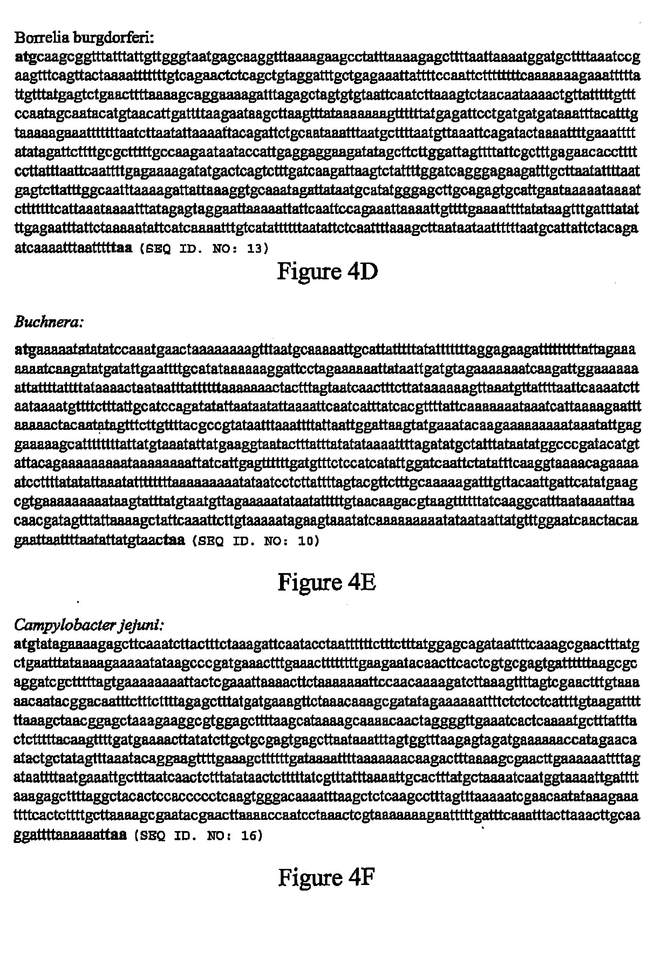

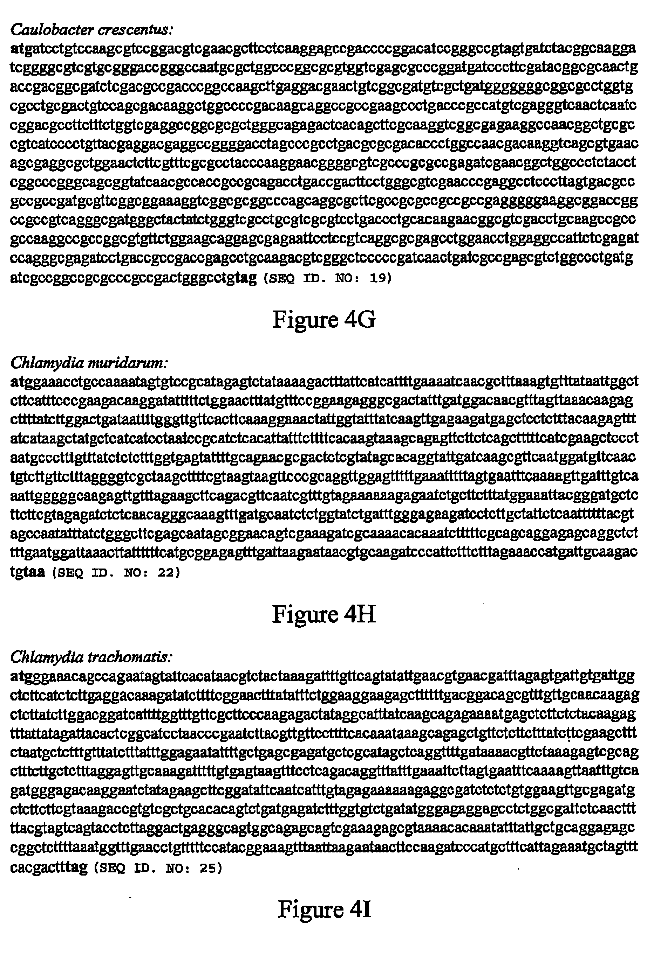

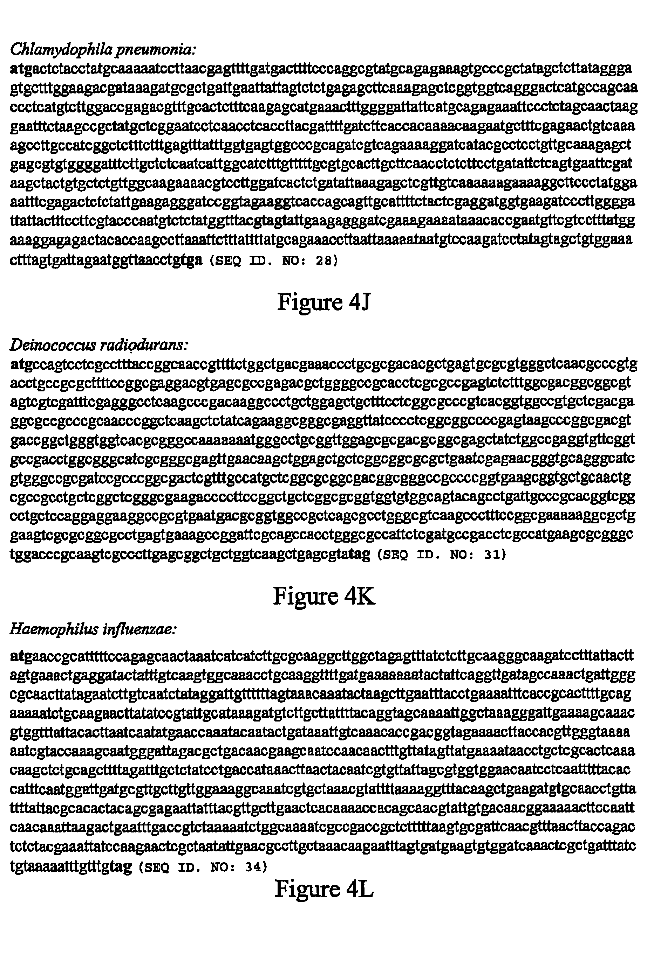

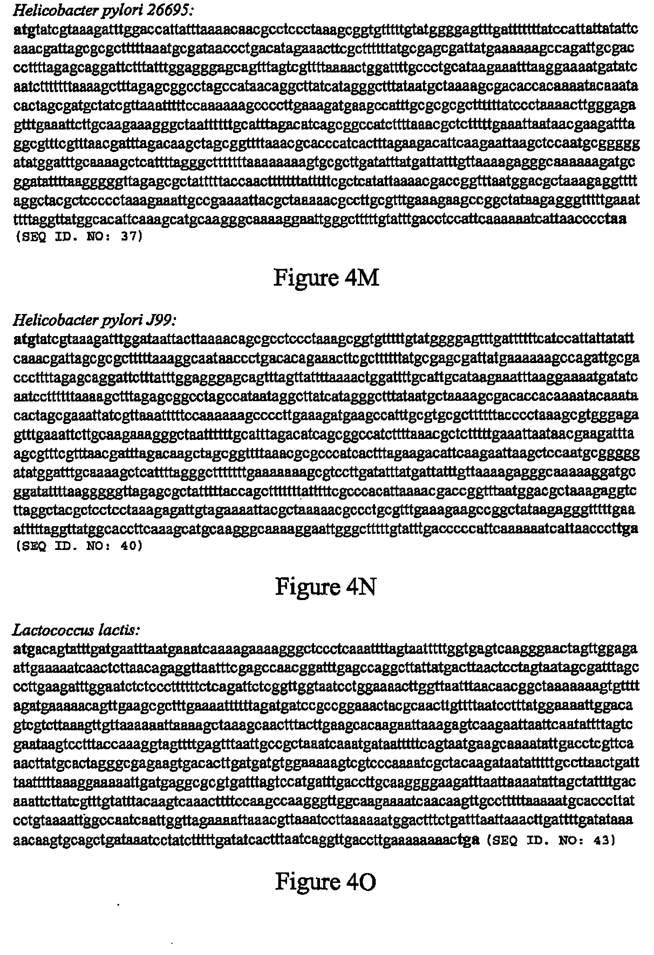

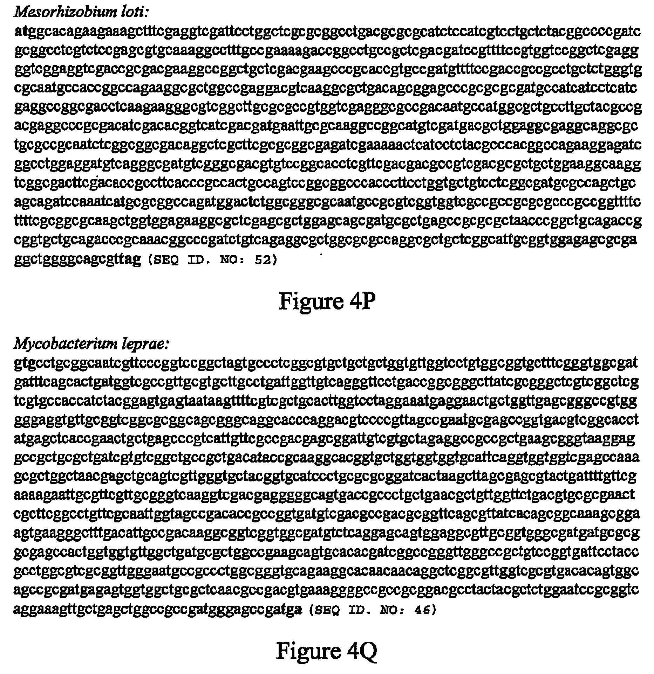

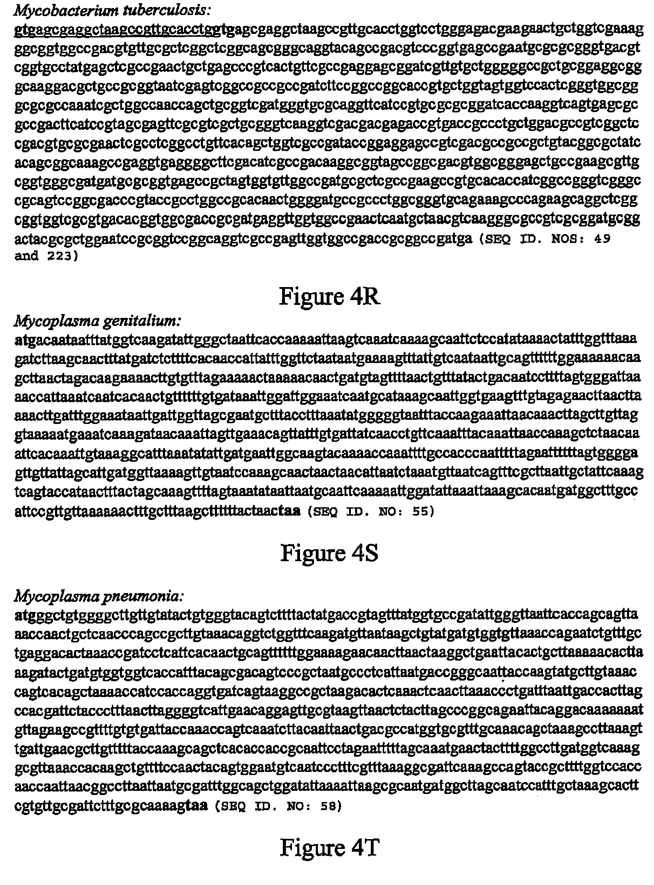

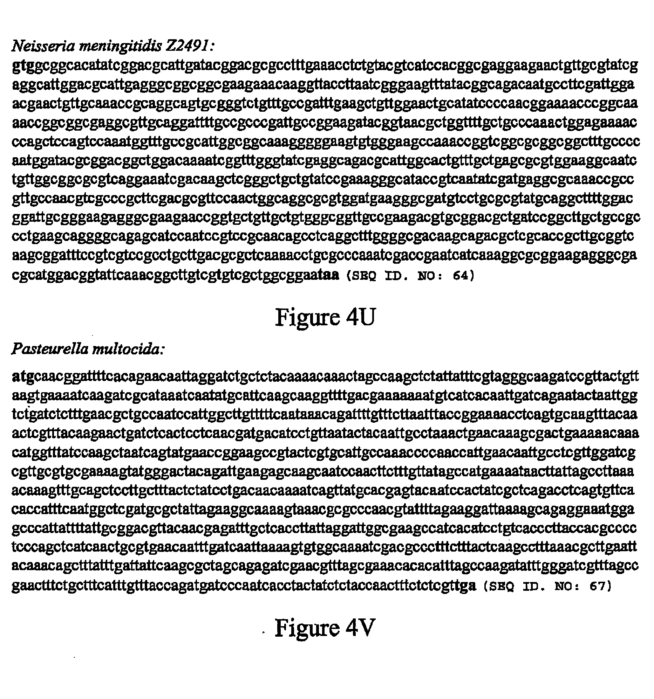

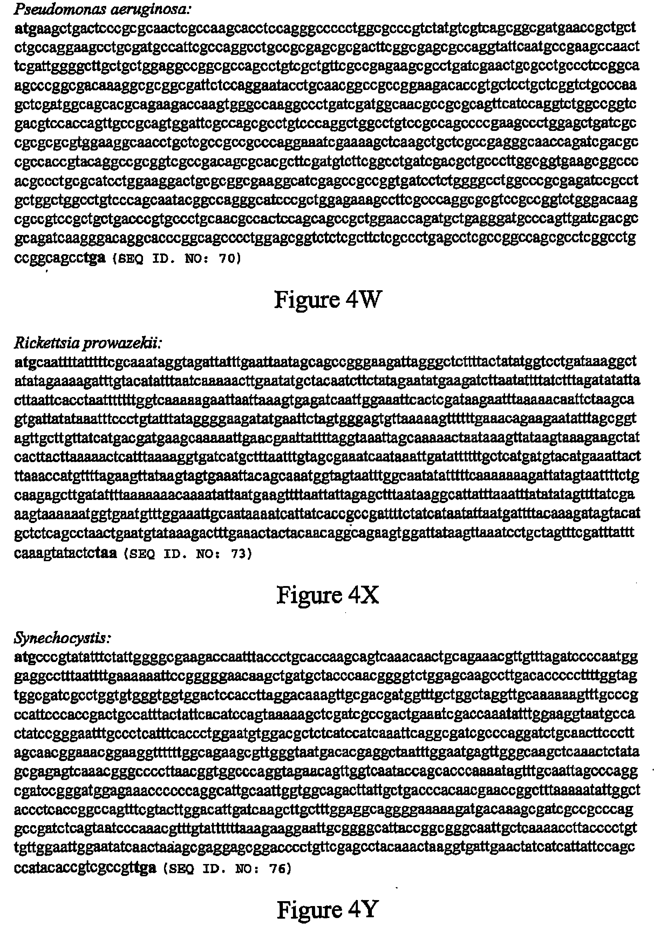

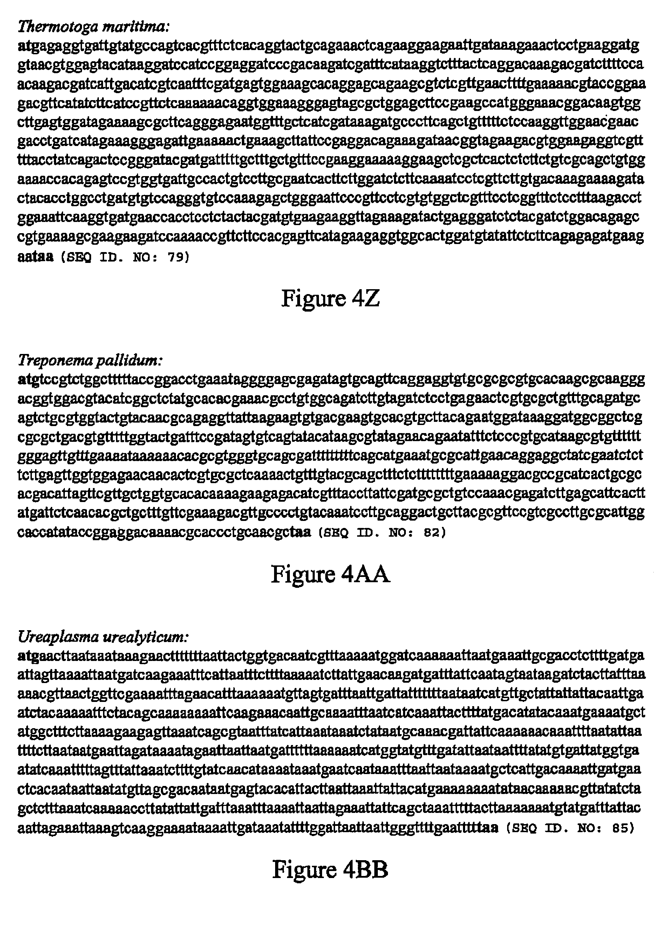

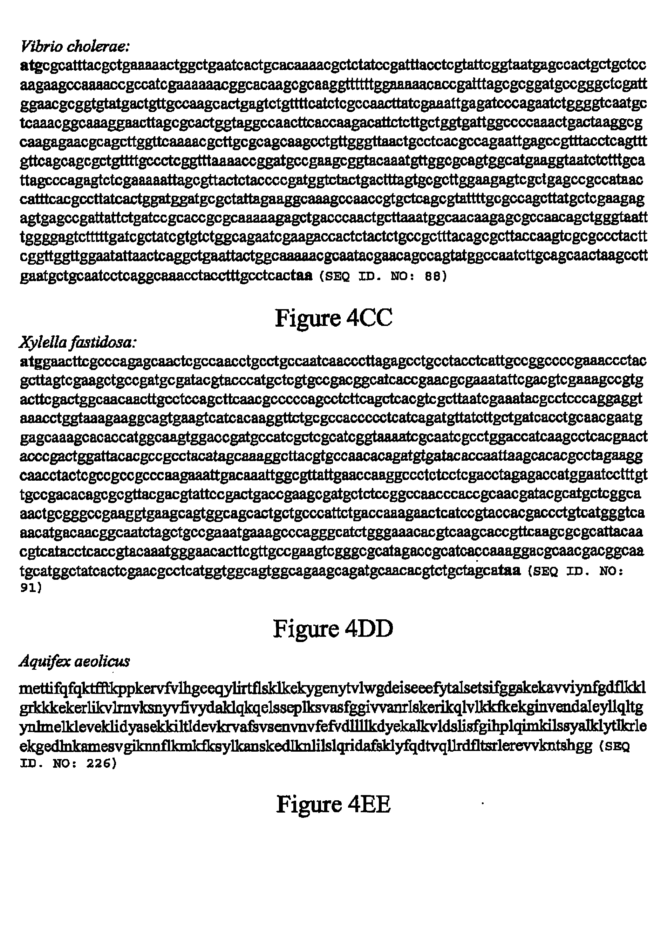

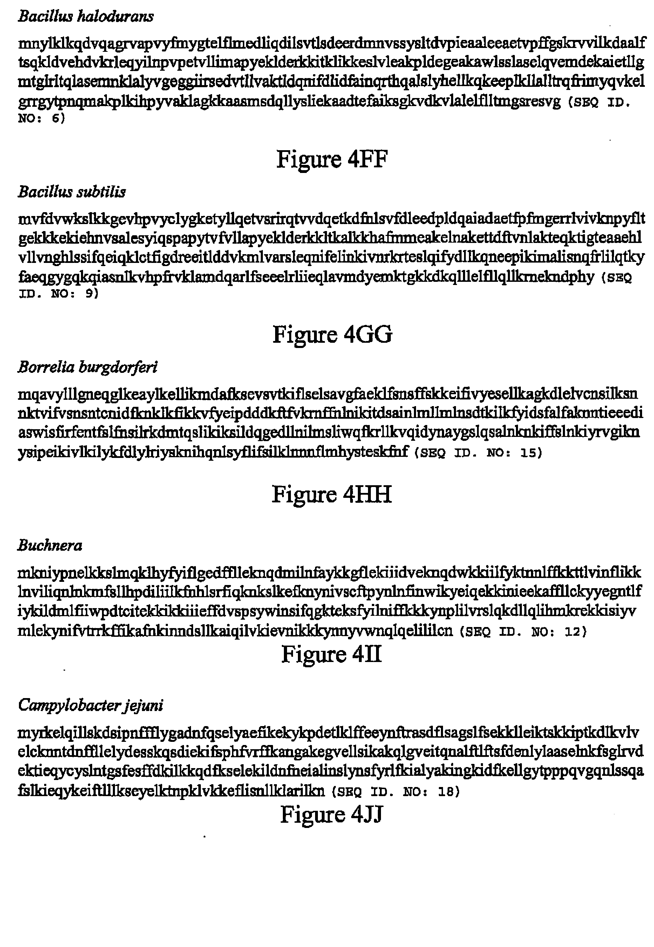

**Step 4.** Only the best hit from a given candidate organism was included (having excluded any additional DnaX and HoIB sequences). From those organisms that have had their genomes completely sequences, the list of delta sequences shown in Figure 4 was discovered.

Steps 2-4 can be repeated with additional iterations with the same setting until delta from the target organism(s) are identified.

**Step 5**. Although the identification of the δ sequences from the genomes that have presently been completely sequenced and are present in the NCBI nr database are unambiguous, additional criteria (outlined in the July 14, 2000 provisional application) could be applied to confirm δ sequences if ambiguities exist. These include *i*) the ability to bind δ' encoded by the *hol*B gene of the same organism, ii) the ability to bind P encoded by the *dna*N gene from the same organism and iii) the ability to reconstitute DNA polymerase III holoenzyme function when combined with the a subunit(s) of DNA polymerase III, the β processivity factor and the DnaX and δ' components of the DnaX complex expressed from the corresponding structural genes for these proteins from the same organism. Also, candidate δ sequences can be aligned with other identified δ sequences using the Clustal W program, as performed in the July 14, 2000 application, with visual inspection to insure that the overall pattern of conserved residues is mostly correct.

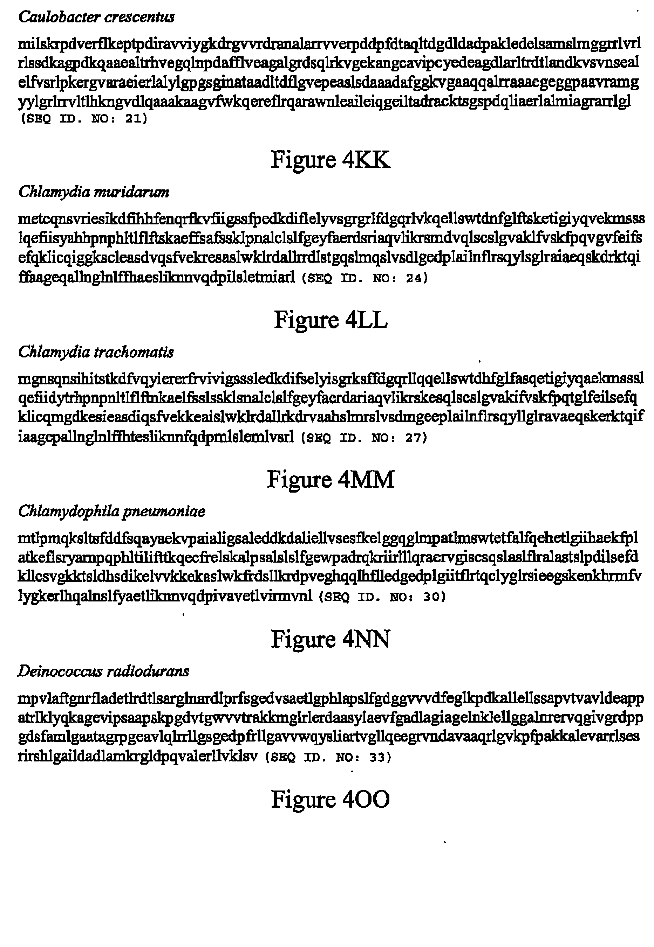

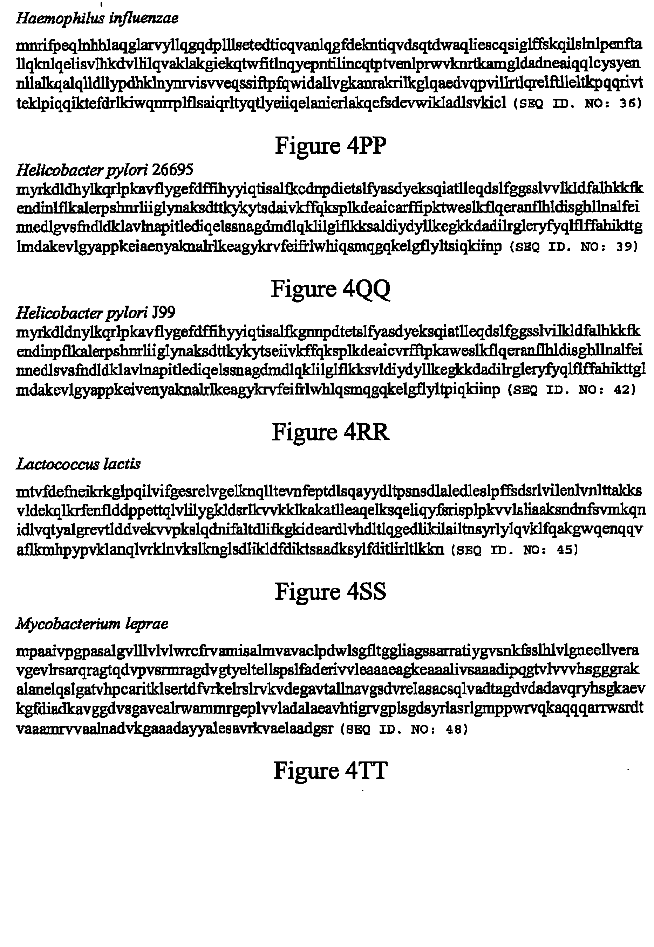

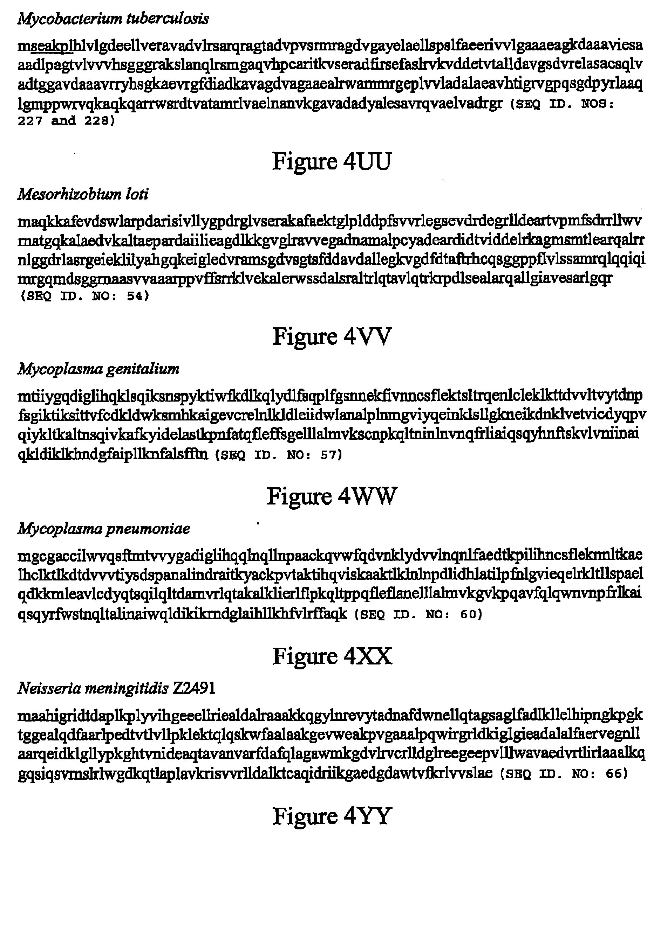

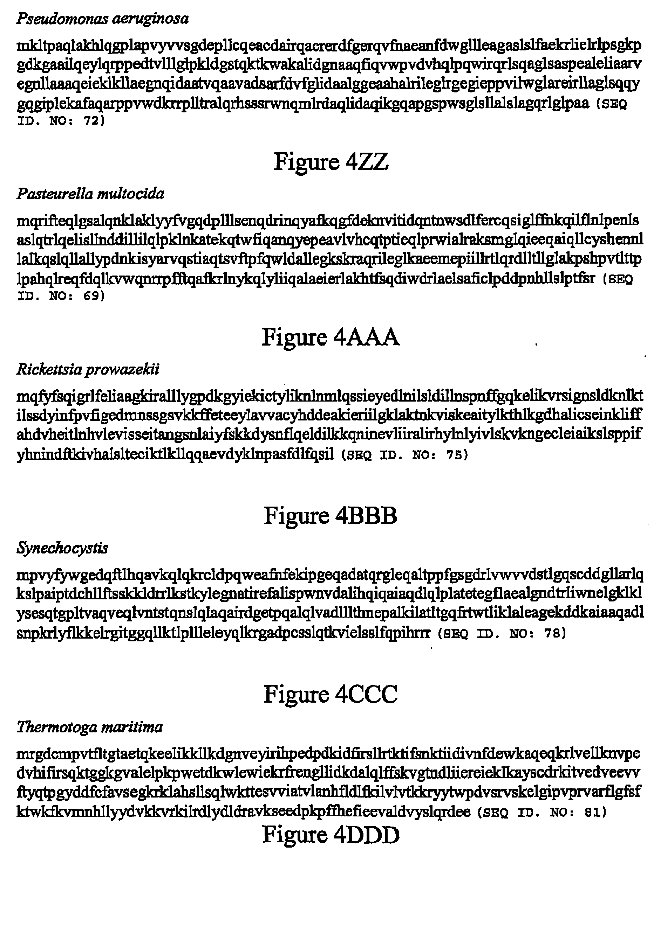

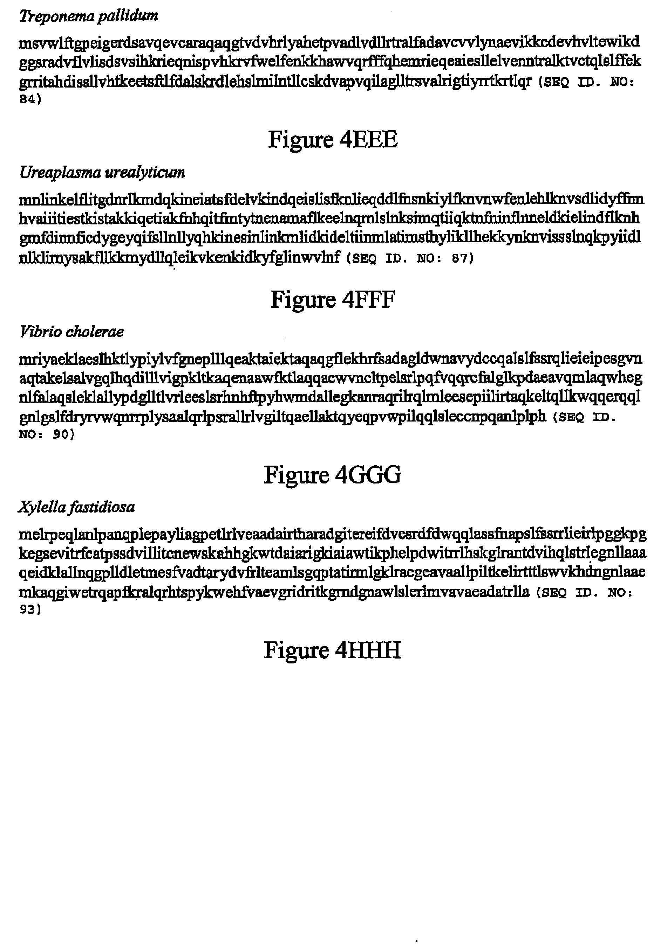

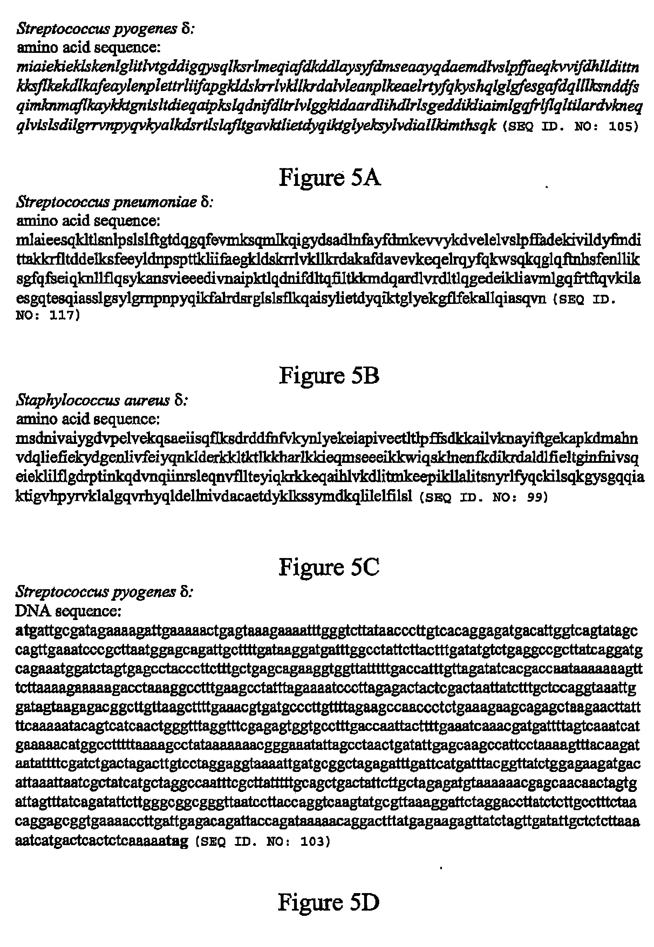

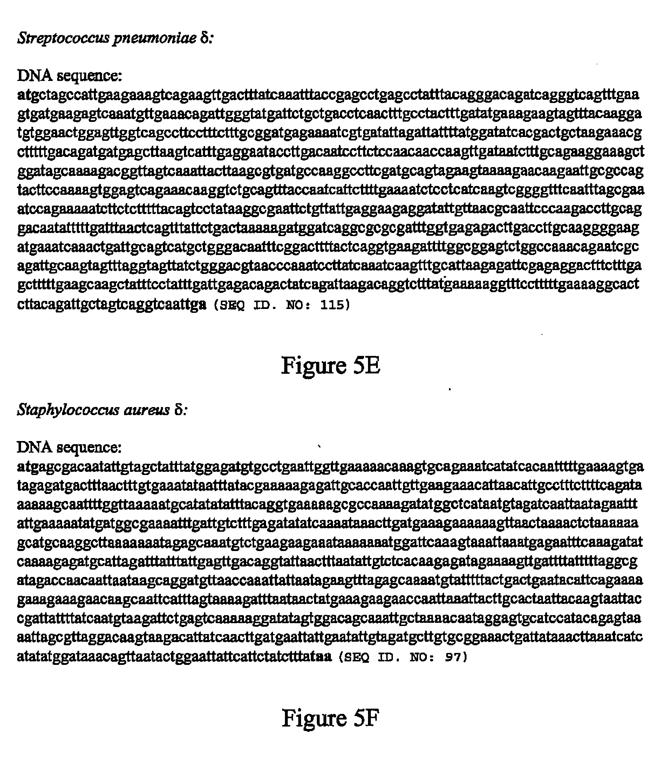

Having this more diverse group of δ sequences permitted the identification of sequences from additional organisms not accessible by Psi-Blast or the NCBI database (but could have been accessible using the same methods on locally run Psi-Blast searching local databases). Simple BLAST searches of reported 'in progress' or other remote databases were performed using tBLASTn and identified coding sequences for δ genes. The settings used in these tblastn BLAST (Gish, W. & States, D.J., Nature Genet. 3:266-272 (1993)) searches were as described below. This program compares a protein query sequence against a nucleotide database that has been translated in all six reading frames. Default parameters were used in each case. For example, searching the The University of Oklahoma Advanced Center for Genome Technology database of *S. pyogenes* sequences against the *Bacillus subtilis* δ protein sequence permitted identification of the sequences endoding *S*. *pyogenes δ* (Figure 5). Search of the TIGR, the Institute for Genomic Research database against the *Bacillus subtilis* δ protein sequence permitted identification of the sequences of Streptococcus pneumoniae δ (Figure 5). Search of the Sanger Centre database against the *Bacillus subtilis* δ protein sequence permitted identification of the sequences of Staphylococcus aureus δ (Figure 5). The corresponding genes for DnaX and delta' proteins from *S. pyogenes, S. pneumoniae* and *S*. *aureus* are shown in Table 6.

**Method B.** The search for δ was preformed using PSI-BLAST as in the search for DnaX and δ'. DnaX and δ' may have regions of similarity with δ therefore the DnaX and δ' proteins identified in the DnaX and δ' search were excluded as possible hits.

**Step 1.** Set search criteria in PSI-BLAST as defined in step 1 of the DnaX search (Example 2).

**Step 2.** Limit the search by Entrez query to only those organisms that have the genomic DNA completely sequenced and available at NCBL To exclude DnaX and δ' proteins as possible hits they must be exclude in the window labeled "Limit by entrez query" by listing the GI numbers following the list of organism the search is limited to as shown below. All duplicated DnaX and δ' proteins with different GI numbers shown in PSI-BLAST alignments must also be excluded. The search for proteins was limited to the organisms listed below, with DnaX and δ' proteins excluded by GI number as shown below.

*Aquifex aeolicus*[Organism] OR *Bacillus halodurans[Organism]* OR *Bacillus* subtilis[Organism] OR *Buchnera*[Organism] OR *Borrelia burgdorferi* [Organism] OR *Campylobacter jejuni*[Organism] OR *Caulobacter crescentus*[Organism] OR *Chlamydia* pneumoniae[Organism] OR *Chlamydia muridarum[Organism]* OR *Chlamydia trachomatis[Organism]* OR *Deinococcus radiodurans[Organism]* OR *Escherichia coli*[Organism] OR *Escherichia coli K12*[Organism] OR *Escherichia coli O157*[Organism] OR *Haemophilus* influenzae[Organism] OR *Helicobacter pylori 26695[Organism]* OR *Helicobacter pylori J99*[Organism] ORLactococcus lactis[Organism] OR *Mesorhizobium loti*[Organism] OR *Mycobacterium* leprae[Organism] OR *Mycobacterium tuberculosis[Organism]* OR *Mycoplasma genitalium*[Organism] OR *Mycoplasma pneumoniae*[Organism] OR *Neisseria meningitidis MC58*[Organism] OR *Neisseria meningitidis Z2491* [Organism] OR *Pasteurella* multocida[Organism] OR *Pseudomonas aeruginosa*[Organism] OR *Rickettsia prowazekii[Organism]* OR *Synechocystis*[Organism] OR *Thermotoga* maritima[Organism] OR *Treponema pallidum*[Organism] OR *Ureaplasma urealyticum*[Organism] OR *Vibrio cholerae*[Organism] OR *Xylella fastidaosa*[Organism] NOT (7514776[gi] OR 98292[gi] OR 7463153[gi] OR 11346858[gi] OR 7468911[gi] OR 7471826[gi] OR 1786676[gi] OR 1075209[gi] OR 7464043 [gi] OR 3150103[gi] OR 1293572[gi] OR 7477980[gi] OR 1361493[gi] OR 2146109[gi] OR 11261620[gi] OR 7467603[gi] OR 11348451[gi] OR 7469304[gi] OR 7462368[gi] OR 2583049[gi] OR 7521020[gi] OR 11356842[gi] OR 11261617[gi] OR 11261615[gi] OR 12720609[gi] OR 12513340[gi] OR 118808[gi] OR 1169397[gi] OR 10039144[gi] OR 10172646[gi] OR 118807[gi] OR 9789547[gi] OR 7468224[gi] OR 8978415[gi] OR 12725258[gi] OR 13357643[gi] OR 6014997[gi] OR 2494198[gi] OR 12045280[gi] OR 581255[gi] OR 13474589[gi] OR 13421402[gi] OR 7464040[gi] OR 11360902[gi] OR 13508357[gi] OR 13359980[gi] OR 67058[gi] OR 41291[gi] OR 43320[gi] OR 145297[gi] OR 1773152[gi] OR 1574159[gi] OR 9655520[gi] OR 9947490[gi] OR 9106885[gi] OR 7380298[gi] OR 580855[gi] OR 467409[gi] OR 580914[gi] OR 2632286[gi] OR 6968590[gi] OR 2984127[gi] OR 4981209[gi] OR 4155207[gi] OR 2313841 [gi] OR 4376294[gi] OR 7189650[gi] OR 3328753[gi] OR 7190650[gi] OR 13093951[gi] OR 3323329[gi] OR 2688379[gi] OR 3861390[gi] OR 6899039[gi] OR 6460225[gi] OR 2960145[gi] OR 1673890[gi] OR 1352305[gi] OR 3845015[gi] OR 1652627[gi] OR 2506368[gi] OR 479843[gi] OR 3212391[gi] OR 145783[gi] OR 1651540[gi] OR 1787341[gi] OR 1074027[gi] OR 2506369[gi] OR 1573429[gi] OR 12722081[gi] OR 11280161[gi] OR 9656559[gi] OR 12644665[gi] OR 11348448[gi] OR 9949059[gi] OR 11280162[gi] OR 972778[gi] OR 11353892[gi] OR 7379683[gi] OR 11353126[gi] OR 7226000[gi] OR 11360901[gi] OR 9105556[gi] OR 7477702[gi] OR 2105050[gi] OR 3097239[gi] OR 13092554[gi] OR 12723272[gi] OR 10172656[gi] OR 13423258[gi] OR 6318311 [gi] OR 13470653[gi] OR 586870[gi] OR2126931[gi] OR 467421[gi] OR 2632298[gi] OR 7473469[gil OR 6460143[gi] OR 7514459[gi] OR 2983903[gi] OR 7467604[gi] OR 3860738[gi] OR 7462369[gi] OR 4981299[gi] OR 11360903[gi] OR 7190502[gi] OR 7468912[gi] OR 3328592[gi] OR 7469488[gi] OR 1653573[gi] OR 13507746[gi] OR 2496262[gi] OR 2146108[gi] OR 1673807[gi] OR 7468225[gi] OR 4376545[gi] OR 7189405[gi] OR 8978646[gi] OR 7463400[gi] OR 2688709[gi] OR 13357575[gi] OR 11356841[gi] OR 7465204[gi] OR 4155746[gi] OR 7521018[gi] OR 3322812[gi] OR 7464041 [gi] OR 2314393[gi] OR 11346661[gi] OR6968051[gi])

**Step 3.** Search designated genomes against *E. coli* δ amino acid sequence (shown below). This amino acid sequence of *E. coli* δ (SEQ ID NO:209 must be entered into the search box of the initial search window. MIRLYPEQLRAQLNEGLRAAYLLLGNDPLLLQESQDAVRQVAAAQGFEEHHTFSIDP NTDWNAIFSLCQAMSLFASRQTLLLLLPENGPNAAINEQLLTLTGLLHDDLLLIVRGN KLSKAQENAAWFTALANRSVQVTCQTPEQAQLPRWVAARAKQLNLELDDAANQV LCYCYEGNLLALAQALERISLLWPDGKLTLPRVEQAVNDAAHFTPFHWVDALLMG KSKRALHILQQLRLEGSBPVILLRTLQRELLLLVNLKRQSAHTPLRALFDKHRVWQN RRGMMGEALNRLSQTQLRQAVQLLTRTELTLKQDYGQSVWAELEGLSLLLCHKPL ADVFIDG

The PSI-BLAST search returned 13 hits listed in decreasing similarity, with 10 hits containing E-values greater than threshold and 3 hits with E-values less than threshold.

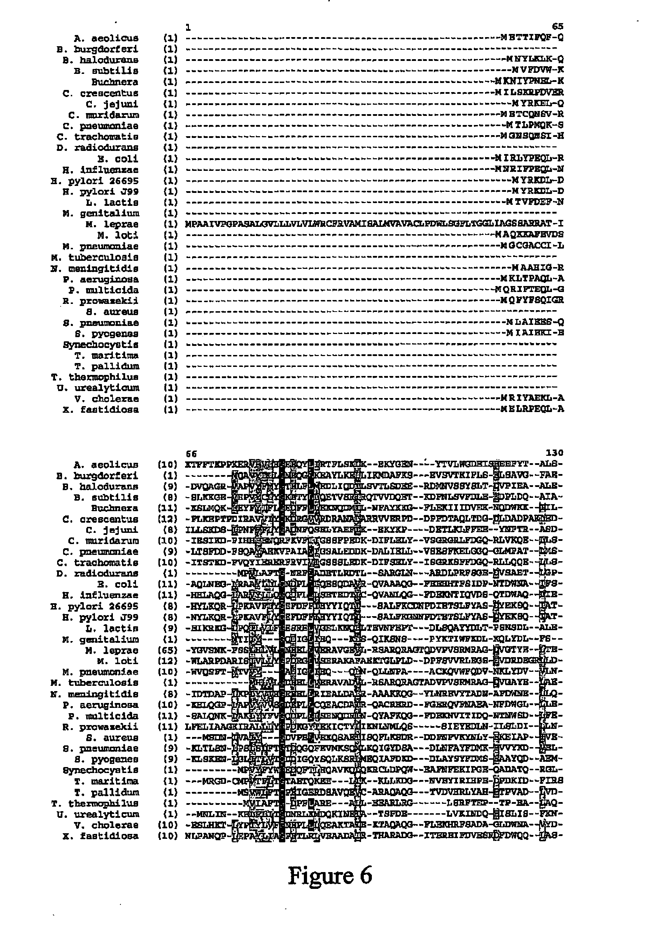

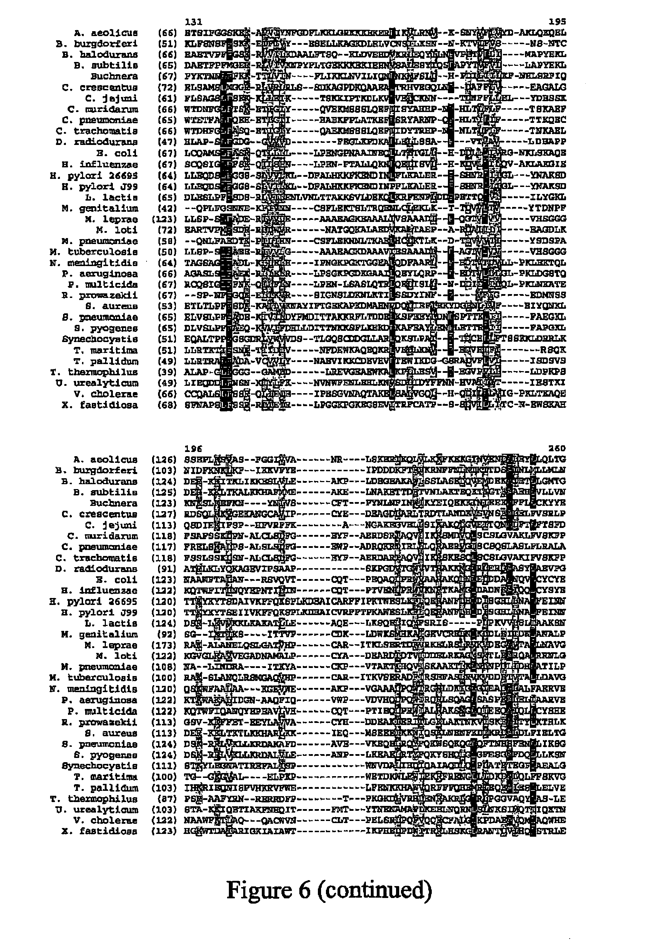

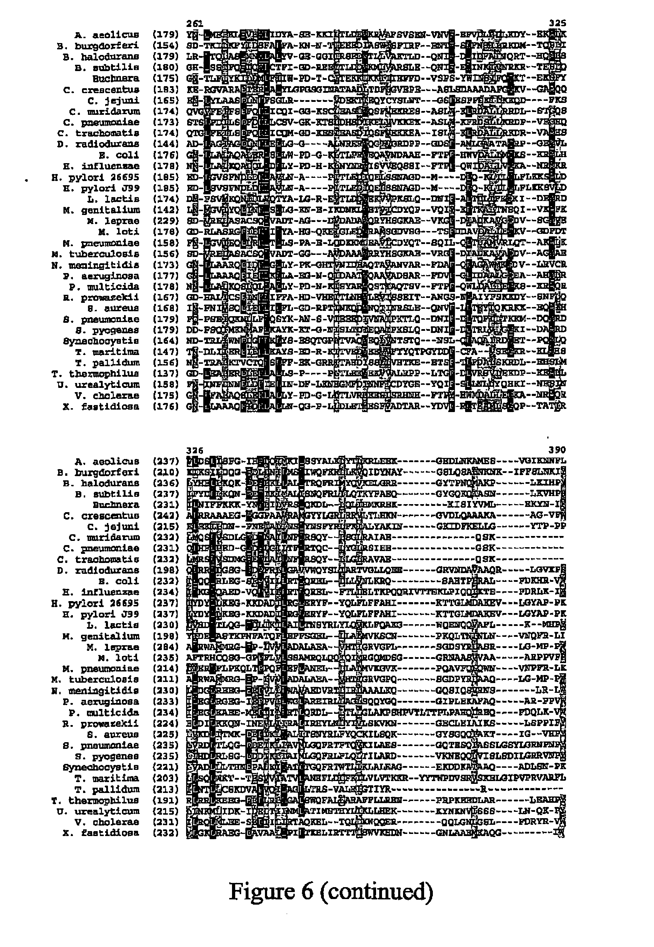

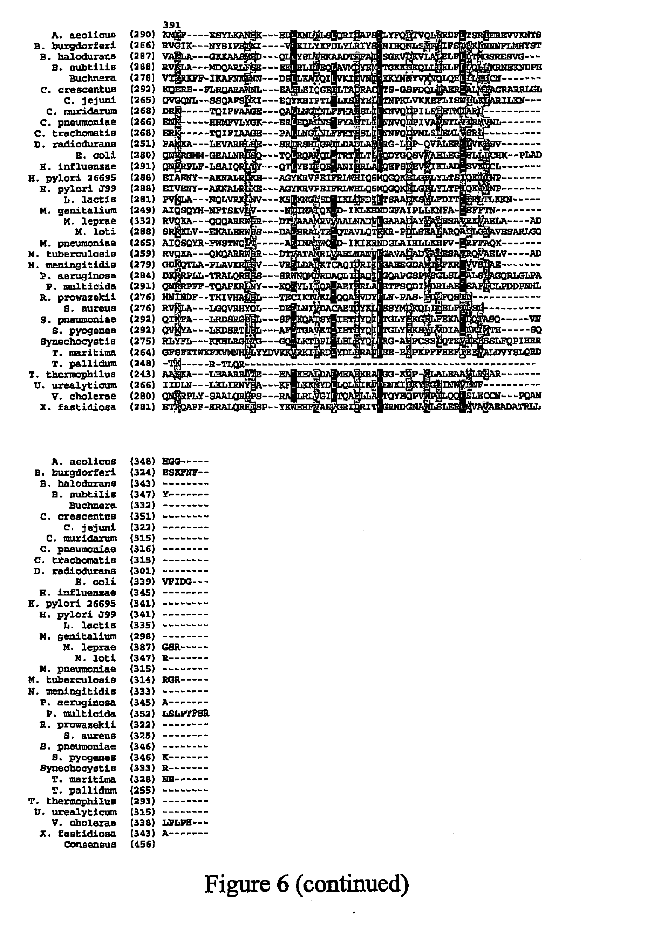

**Step 4.** Criteria to select hits that are δ proteins. Using the δ proteins identified in method A, the δ sequence from *Thermus thermophilus* (patent application No. 09/818,780) and δ subunit sequences from *Streptococcus pyogenes, Streptococcus pneumoniae* and *Staphylococcus aureus* (Figure 5), alignments using Clustal W indicated six areas containing highly conserved amino acids. Conserved residues in these six regions provide additional sequence-based criteria for confirmation of δ proteins. The sequences selected represented the most conserved stretches greater than 17 amino acids in aligned deltas (Figure 6)
*i.* Exclude DnaX and δ' sequences that were identified in the DnaX and δ' searches.
ii Identified δ subunits must meet the following criteria in at least 3 of the 6 conserved regions shown below. In conserved regions #1-6 the indicated number of conserved residues must be observed: #1, 5 out of possible 10, #2, 5 out of possible 11, #3, 5 out of possible 10, #4 9 out of possible 18, #5 11 out of possible 23, #6 6 out of possible 12 conserved residues. Pairwise alignments and selection must be performed as described in step 4i in Example 2.
   1.) 17-(L/I/V)XX(L/I/V)Y(L/I/V)(L/I/V)XGX(D/E)XX(L/I/V)(L/I/V)XXXXXX (L/I/V)-38 (SEQ ID NO:194)
   2.) 64-(L/I/V)(L/I/V)XXXX(A/S)XX(M/V)F(A/S)X(K/R)X(L/I/V)(L/I/V)(L/I/ V)(L/I/V)-82 (SEQ ID NO:195)
   3.) 97-(L/I/V)XX(L/I/V)(L/I/V)XXXXX(D/E)X(L/I/V)(L/I/V)(L/I/V)(L/I/V)XX XK(L/I/V)-117 (SEQ ID NO:196)
   4.) 154-(R/K)XXX(L/I/V)X(L/I/V)X(L/I/V)(D/E)X(D/E)(A/S)(L/I/V)XX(L/I/V) XXXXXXN(L/I/V)XX(L/I/V)XX(D/E)(L/I/V)X(R/K)(L/I/V)X(L/I/V)(L/I /V)-191 (SEQ ID N0:197)
   5.) 215-FX(L/I/V)X(D/E)(A/S)(L/I/V)(L/I/V)XG(R/K)XXX(A/S) (L/I/V)X(L/I/V)(L/I/V)XX(L/I/V)XXXGX(D/E)P(L/I/V)X(L/I/V)(L/I/V) XX(L/I/V)XXX(L/I/V)XX(L/I/V)XX(L/I/V)-260 (SEQ ID NO:198)
   6.) 298-(L/IV)XXX(L/I/V)XX(L/I/V)XXX(D/E)XX(L/I/V)(R/K)XXXXX(D/E)X XXX(L/I/V)(D/E)XX(L/I/V)(L/I/V)X(L/I/V)-331 (SEQ ID NO:199)
All of the hits (10) with E-values greater than threshold contained sequences that were likely δ proteins. These hits were marked for the second iteration.

**Step 5.** Additional iterations if δ from all target organism were not identified; that is, Step 4 is repeated until δ from all target organisms are identified.
*i.* Limit the hits for further searches (iterations) by sequences with E-values greater than threshold and sequences selected as δ in step 4.
*ii.* Possible δ proteins identified in additional iterations must meet criteria outlined in the δ search step 4 shown above.
iii. Repeat step 5 until δ from all specified organisms have been identified.

The second iteration returned 33 hits with 20 hits having E-values greater than threshold. Nine new hits were identified as δ subunits. These hits were selected as described above and marked for the third iteration. The third iteration returned 50 hits, containing δ from all except one of the target organisms. The fourth iteration contained 64 hits including the final δ. Conserved residues in the conserved regions for each δ is shown in Table 19.

### Example 5. Identification of DnaX. δ' and δ From Partially Sequenced Genomes Using PSI-BLAST

Some protein sequences that are not from totally sequenced genomes but were identified as a protein from partially sequenced genomes have also been filed with NCBI. DnaX, δ' and δ proteins from these partially sequenced genomes can be identified if they have been deposited with NCBI by using the criteria described above for identifying DnaX, δ' and δ from fully sequenced genomes. The only exception with the search methods is that the search will not be limited by entrez query to only completely sequenced genomes, but will instead be limited by entrez query to all bacteria.

To search for DnaX proteins the term bacteria should be entered into the window labeled "Limit by entrez query" in the initial search window followed by exclusion of the DnaX and δ' proteins identified in the search for DnaX and δ' proteins from fully sequenced genomes as shown below.
bacteria [Organism] NOT (7514776[gi] OR 98292[gi] OR 7463153[gi] OR 11346858[gi] OR 7468911 [gi] OR 7471826[gi] OR 1786676[gi] OR 1075209[gi] OR 7464043[gi] OR 3150103[gi] OR 1293572[gi] OR 7477980[gi] OR 1361493[gi] OR 2146109[gi] OR 11261620[gi] OR 7467603[gi] OR 11348451[gi] OR 7469304[gi] OR 7462368[gi] OR 2583049[gi] OR 7521020[gi] OR 11356842[gi] OR 11261617[gi] OR 11261615[gi] OR 12720609[gi] OR 12513340[gi] OR 118808[gi] OR 1169397[gi] OR 10039144[gi] OR 10172646[gi] OR 118807[gi] OR 9789547[gi] OR 7468224[gi] OR 8978415[gi] OR 12725258[gi] OR 13357643[gi] OR 6014997[gi] OR 2494198[gi] OR 12045280[gi] OR 581255[gi] OR 13474589[gi] OR 13421402[gi] OR 7464040[gi] OR 11360902[gi] OR 13508357[gi] OR 13359980[gi] OR 67058[gi] OR 41291[gi] OR 43320[gi] OR 145297[gi] OR 1773152[gi] OR 1574159[gi] OR 9655520[gi] OR 9947490[gi] OR 9106885[gi] OR 7380298[gi] OR 580855[gi] OR 467409[gi] OR 580914[gi] OR 2632286[gi] OR 6968590[gi] OR 2984127[gi] OR 4981209[gi] OR 4155207[gi] OR 2313841[gi] OR 4376294[gi] OR 7189650[gi] OR 3328753 [gi] OR 7190650[gi] OR 13093951[gi] OR 3323329[gi] OR 2688379[gi] OR 3861390[gi] OR 6899039[gi] OR 6460225[gi] OR 2960145[gi] OR 1673890[gi] OR 1352305[gi] OR 3845015[gil OR 1652627[gi] OR 2506368[gi] OR 479843[gi] OR 3212391 [gi] OR 145783[gi] OR 1651540[gi] OR 1787341 [gi] OR 1074027[gi] OR 2506369[gi] OR 1573429[gi] OR 12722081 [gi] OR 11280161[gi] OR 9656559[gi] OR 12644665[gi] OR 11348448[gi] OR 9949059[gi] OR 11280162[gi] OR 972778[gi] OR 11353892[gi] OR 7379683[gi] OR 11353126[gi] OR 7226000[gi] OR 11360901 [gi] OR 9105556[gi] OR 7477702[gi] OR 2105050[gi] OR 3097239[gi] OR 13092554[gi] OR 12723272[gi] OR 10172656[gi] OR 13423258[gi] OR 6318311[gi] OR 13470653 [gi] OR 586870[gi] OR 2126931 [gi] OR 467421[gi] OR 2632298[gi] OR 7473469[gi] OR 6460143[gi] OR 7514459[gi] OR 2983903[gi] OR 7467604[gi] OR 3860738[gi] OR 7462369[gi] OR 4981299[gi] OR 11360903[gi] OR 7190502[gi] OR 7468912[gi] OR 3328592[gi] OR 7469488[gi] OR 1653573[gi] OR 13507746[gi] OR 2496262[gi] OR 2145108[gi] OR 1673807[gi] OR 7468225[gi] OR 4376545[gi] OR 7189405[gi] OR 8978646[gi] OR 7463400[gi] OR 2688709[gi] OR 13357575[gi] OR 11356841[gi] OR 7465204[gi] OR 4155746[gi] OR 75210 18[gi] OR 3322812[gi] OR 7464041[gi] OR 2314393[gi] OR 11346661[gi] OR 6968051[gi])
To be distinguished as DnaX, a protein must meet the criteria outlined above in step 4 of the search for DnaX from fully sequenced genomes (Example 2). Three additional DnaX proteins were identified after one iteration. The organisms from which the DnaX proteins were identified and that correspond to delta subunit proteins identified in an analogous manner (see below) were *Mycoplasma pulmonis* and *Streptomyces coelicolor* (Table 8).

This same procedure is used to identify δ' proteins deposited with NCBI from partially sequenced genomes. To search for δ' proteins the term bacteria should be entered into the window labeled "Limit by entrez query" in the initial search window followed by exclusion of the DnaX and δ' proteins identified in the search for DnaX proteins and the search for δ' proteins. δ' proteins identified in the searches from both completely sequenced genomes and from genomes that have not been fully sequenced should be entered as shown below.
bacteria [Organism] NOT (7514776[gi] OR 98292[gi] OR 7463153[gi] OR 11346858[gi] OR 7468911 [gi] OR 7471826[gi] OR 1786676[gi] OR 1075209[gi] OR 7464043[gi] OR 3150103[gi] OR 1293572[gi] OR 7477980[gi] OR 1361493[gi] OR 2146109[gi] OR 11261620[gi] OR 7467603[gi] OR 11348451[gi] OR7469304[gi] OR 7462368[gi] OR 2583049[gi] OR 7521020[gi] OR 11356842[gi] OR 11261617[gi] OR 11261615[gi] OR 12720609[gi] OR 12513340[gi] OR 118808[gi] OR 1169397[gi] OR 10039144[gi] OR 10172646[gi] OR 118807[gi] OR 9789547[gi] OR 7468224[gi] OR 8978415[gi] OR 12725258[gi] OR 13357643[gi] OR 6014997[gi] OR 2494198[gi] OR 12045280[gi] OR 581255[gi] OR 13474589[gi] OR 13421402[gi] OR 7464040[gi] OR 11360902[gi] OR 13508357[gi] OR 13359980[gi] OR 67058[gi] OR 41291[gi] OR 43320[gi] OR 145297[gi] OR 1773152[gi] OR 1574159[gi] OR 9655520[gi] OR 9947490[gi] OR 9106885[gi] OR 7380298[gi] OR 580855[gi] OR 467409[gi] OR 580914[gi] OR 2632286[gi] OR 6968590[gi] OR 2984127[gi] OR 4981209[gi] OR 4155207[gi] OR 2313841[gi] OR 4376294[gi] OR 7189650[gi] OR 3328753[gi] OR 7190650[gi] OR 13093951[gi] OR 3323329[gi] OR 2688379[gi] OR 3861390[gi] OR 6899039[gi] OR 6460225[gi] OR 2960145[gi] OR 1673890[gi] OR 1352305[gi] OR 3845015[gi] OR 1652627[gi] OR 2506368[gi] OR 479843[gi] OR 3212391[gi] OR 145783[gi] OR 1651540[gi] OR 1787341[gi] OR 1074027[gi] OR 2506369[gi] OR 1573429[gi] OR 12722081[gi] OR 11280161[gi] OR 9656559[gi] OR 12644665[gi] OR 11348448[gi] OR 9949059[gi] OR 11280162[gi] OR 972778[gi] OR 11353892[gi] OR 7379683[gi] OR 11353126[gi] OR 7226000[gi] OR 11360901[gi] OR 9105556[gi] OR 7477702[gi] OR 2105050[gi] OR 3097239[gi] OR 13092554[gi] OR 12723272[gi] OR 10172656[gi] OR 13423258[gi] OR 6318311[gi] OR 13470653[gi] OR 586870[gi] OR 2126931[gi] OR 467421[gi] OR 2632298[gi] OR 7473469[gi] OR 6460143[gi] OR 7514459[gi] OR 2983903[gi] OR 7467604[gi] OR 3860738[gi] OR 7462369[gi] OR 4981299[gi] OR 11360903[gi] OR 7190502[gi] OR 7468912[gi] OR 3328592[gi] OR 7469488[gi] OR 1653573[gi] OR 13507746[gi] OR 2496262[gi] OR 2146108[gi] OR 1673807[gi] OR 7468225[gi] OR 4376545[gi] OR 7189405[gi] OR 8978646[gi] OR 7463400[gi] OR 2688709[gi] OR 13357575[gi] OR 11356841[gi] OR 7465204[gi] OR 4155746[gi] OR 7521018[gi] OR 3322812[gi] OR 7464041[gi] OR2314393[gi] OR 11346661[gi] OR 6968051[gi] OR 1527142[gi] OR 13700368[gi] OR 5918469[gi] OR 14089462[gi])

To be distinguished as δ', a protein must meet the criteria outlined above in step 4 of the search for δ' from fully sequenced genomes.

After three iterations five additional δ' proteins were identified from: *Mycoplasma fermentans, Mycoplasma pulmonis, Plectonema boryanum, Streptomyces coelicolor.*

This same procedure is used to identify δ proteins deposited with NCBI from partially sequenced genomes. To search for δ proteins the term bacteria should be entered into the window labeled "Limit by entrez query" in the initial search window followed by exclusion of the DnaX and δ' proteins identified in the search for DnaX proteins and the search for δ' proteins. δ' proteins identified in the searches from both completely sequenced genomes and from genomes that have not been fully sequenced should be entered as shown below.
bacteria[Organism] NOT (7514776[gi] OR 98292[gi] OR 7463153[gi] OR 11346858[gi] OR 7468911[gi] OR 7471826[gi] OR 1786676[gi] OR 1075209[gi] OR 7464043[gi] OR 3150103[gi] OR 1293572[gi] OR 7477980[gi] OR 1361493[gi] OR 2146109[gi] OR 11261620[gi] OR 7467603[gi] OR 11348451[gi] OR 5918469[gi] OR 7469304[gi] OR 7462368[gi] OR 2583049[gi] OR 7521020[gi] OR 11356842[gi] OR 11261617[gi] OR 11261615[gi] OR 12720609[gi] OR 12513340[gi] OR 118808[gi] OR 1169397[gi] OR 10039144[gi] OR 10172646[gi] OR 118807[gi] OR 9789547[gi] OR 7468224[gi] OR 8978415[gi] OR 12725258[gi] OR 13357643[gi] OR 6014997[gi] OR 2494198[gi] OR 12045280[gi] OR 581255[gi] OR 13474589[gi] OR 13421402[gi] OR 7464040[gi] OR 11360902[gi] OR 13508357[gi] OR 13359980[gi] OR 67058[gi] OR 41291[gi] OR 43320[gi] OR 145297[gi] OR 1773152[gi] OR 1574159[gi] OR 9655520[gi] OR 9947490[gi] OR 9106885[gi] OR 7380298[gi] OR 580855[gi] OR 467409[gi] OR 580914[gi] OR 2632286[gi] OR 6968590[gi] OR 2984127[gi] OR 4981209[gi] OR 4155207[gi] OR 2313841[gi] OR 4376294[gi] OR 7189650[gi] OR 3328753[gi] OR 7190650[gi] OR 13093951[gi] OR 3323329[gi] OR 2688379[gi] OR 3861390[gi] OR 6899039[gi] OR 6460225[gi] OR 2960145[gi] OR 1673890[gi] OR 1352305[gi] OR 3845015[gi] OR 1652627[gi] OR 2506368[gi] OR 479843[gi] OR 3212391[gi] OR 145783[gi] OR 1651540[gi] OR 1787341[gi] OR 1074027[gi] OR 2506369[gi] OR 1573429[gi] OR 12722081[gi] OR 11280161[gi] OR 9656559[gi] OR 12644665[gi] OR 11348448[gi] OR 9949059[gi] OR 11280162[gi] OR 972778[gi] OR 11353892[gil OR 7379683[gi] OR 11353126[gi] OR 7226000[gi] OR 11360901[gi] OR 9105556[gi] OR 7477702[gi] OR 2105050[gi] OR 3097239[gi] OR 13092554[gi] OR 12723272[gi] OR 10172656[gi] OR 13423258[gi] OR 6318311[gi] OR 13470653[gi] OR 586870[gi] OR 2126931 [gi] OR 467421[gi] OR 2632298[gi] OR 7473469[gi] OR 6460143 [gi] OR 7514459[gi] OR 2983903[gi] OR 7467604[gi] OR 3860738[gi] OR 7462369[gi] OR 4981299[gi] OR 11360903[gi] OR 7190502[gi] OR 7468912[gi] OR 3328592[gi] OR 7469488[gi] OR 1653573[gi] OR 13507746[gi] OR 2496262[gi] OR 2146108[gi] OR 1673807[gi] OR 7468225[gi] OR 4376545[gi] OR 7189405[gi] OR 8978646[gi] OR 7463400[gi] OR 2688709[gi] OR 13357575[gi] OR 11356841[gi] OR 7465204[gi] OR 4155746[gi] OR 7521018[gi] OR 3322812[gi] OR 7464041[gi] OR 2314393[gi] OR 11346661[gi] OR 6968051[gi] OR 1527142[gi] OR 13700368[gi] OR 5918469[gi] OR 14089462[gi] OR 4587466[gi] OR 14089466[gi] OR 1732451[gi] OR 13700374[gi] OR 7480627[gi])

To be distinguished as δ, a protein must meet the criteria outlined above in step 4 of the search for δ from fully sequenced genomes.

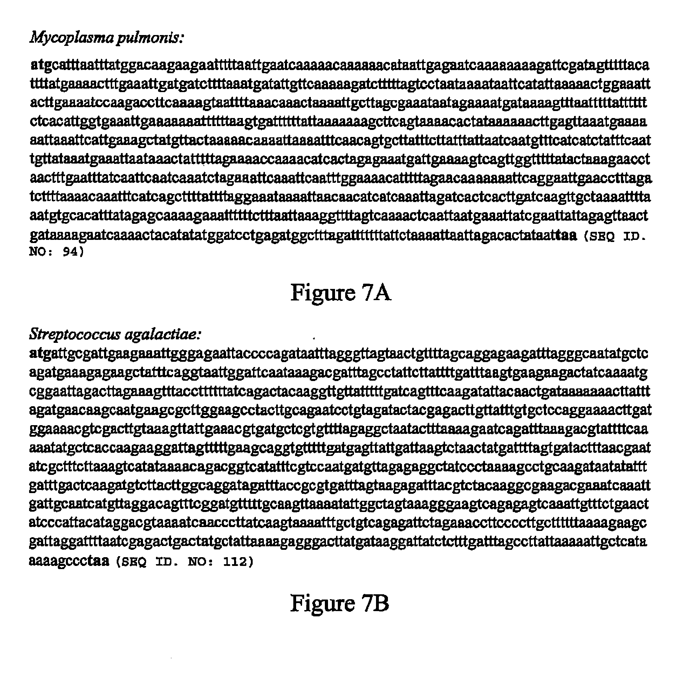

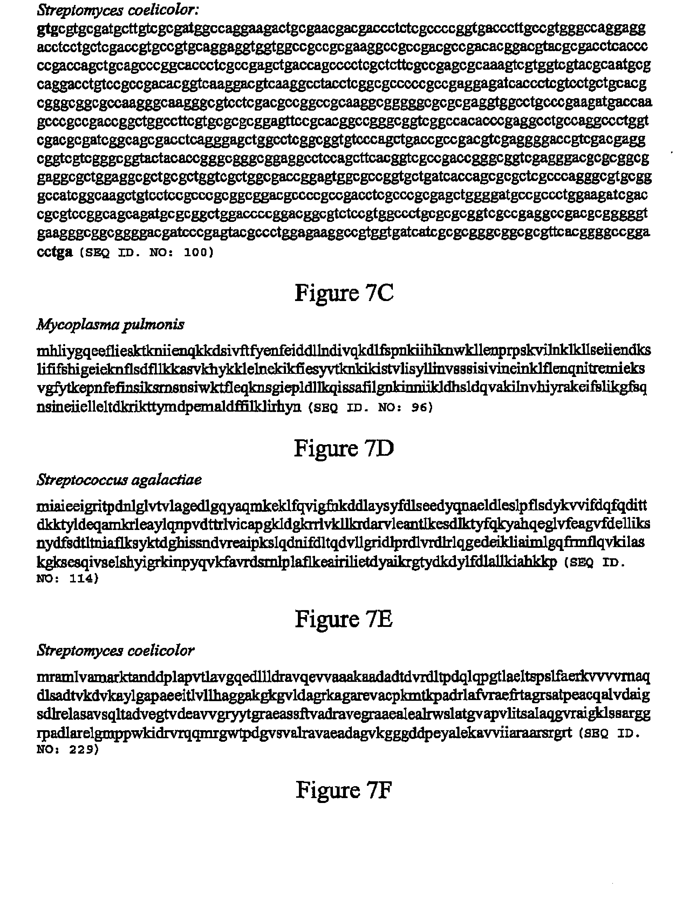

After four iterations four additional δ proteins were identified from: *Mycoplasma pulmonis, Streptococcus agalactiae and Streptomyces coelicolor.* The sequences of these δ proteins are shown in Figure 7. The locations of the corresponding DnaX and δ' genes from *Mycoplasma pulmonis* and *Streptomyces coelicolor* are shown in Tables 8 and 9, respectively.

### Example 6. Identification of additional δ-encoding sequences using BLAST searches initiated with delta sequences from phylogenically related organisms

In this example, the broad group of delta sequences generated in the preceding sections (Examples 4 and 5) to enables, using phylogenic relationships, the identification of delta sequences from any organism, using the standard 'BLAST' program. This method would not have been possible in the beginning using only the few known delta sequences since delta sequences are not well-conserved. But, now that representatives in all major branches of the bacterial phylogenic tree have been identified, deltas can be identified from newly sequenced organisms by searching with a delta sequence from a closely related organism.

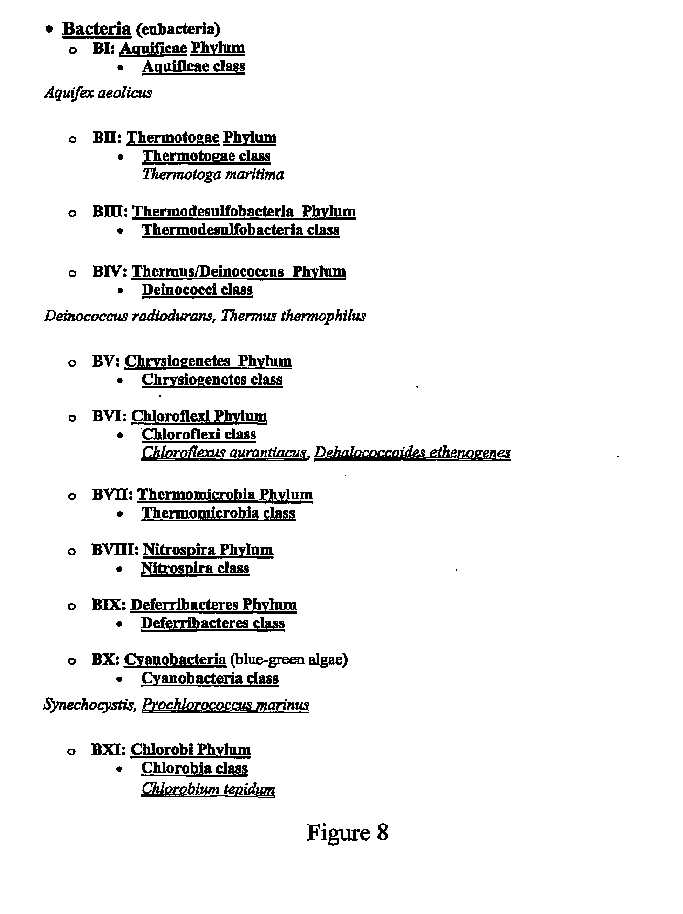

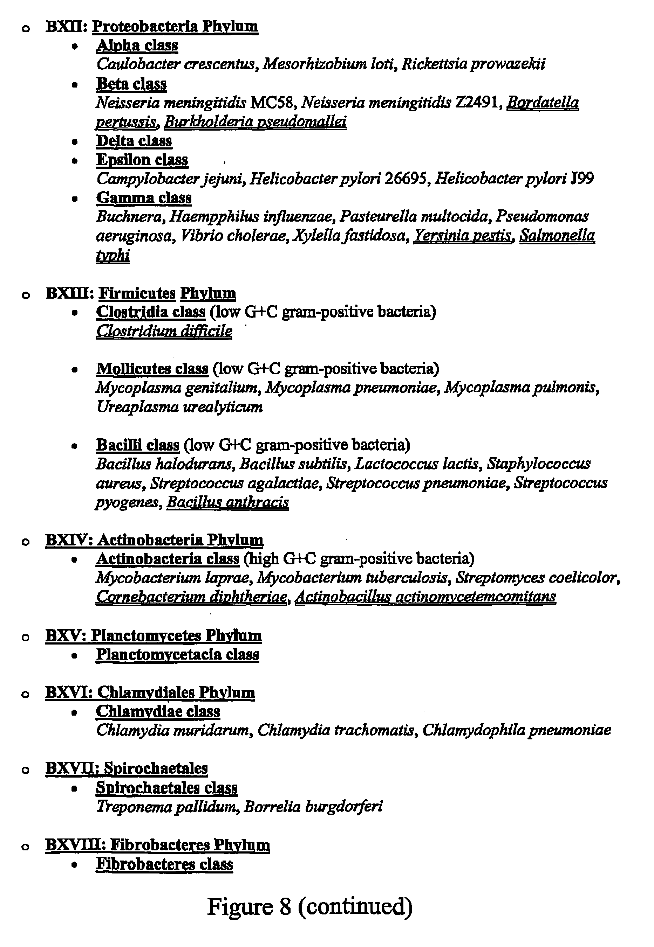

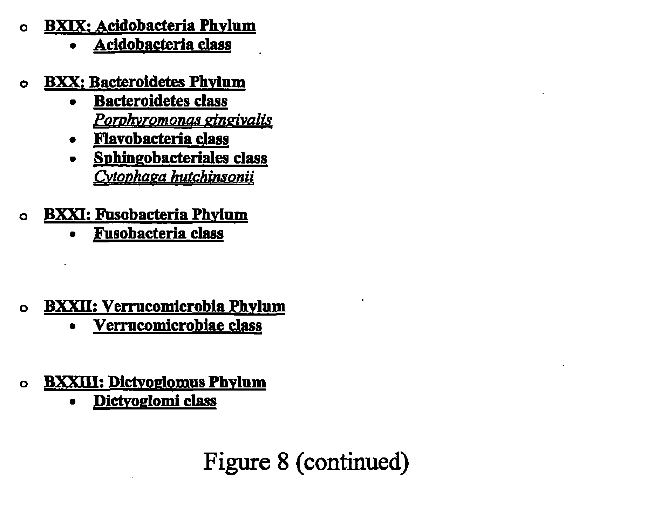

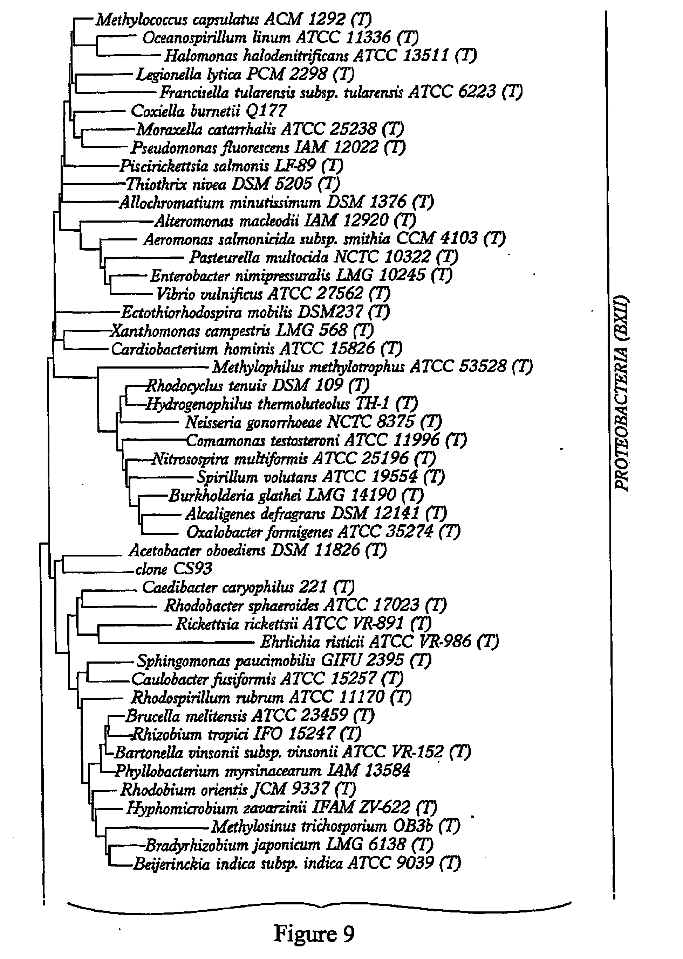

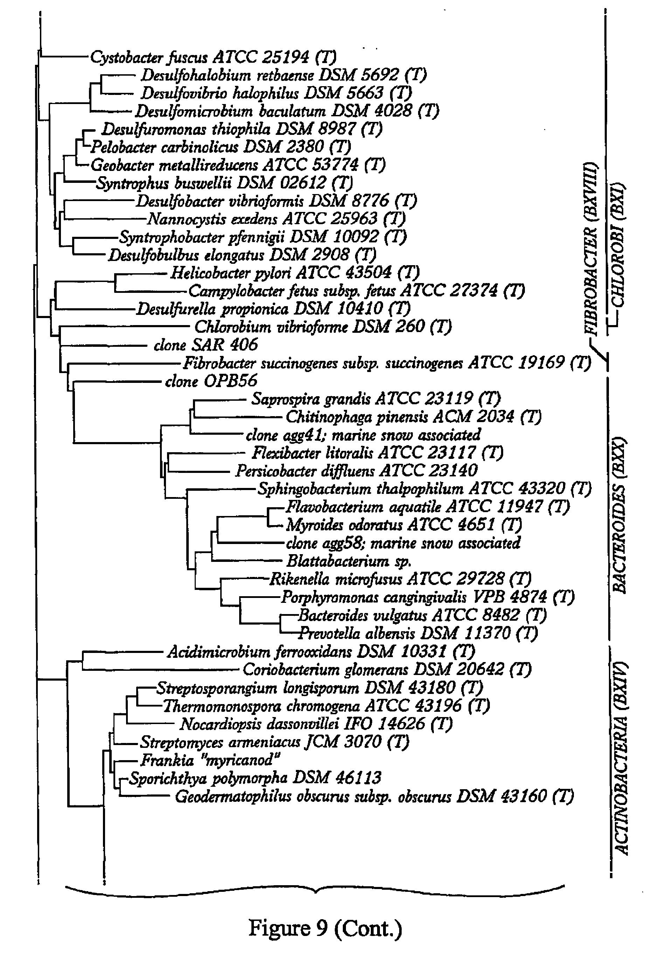

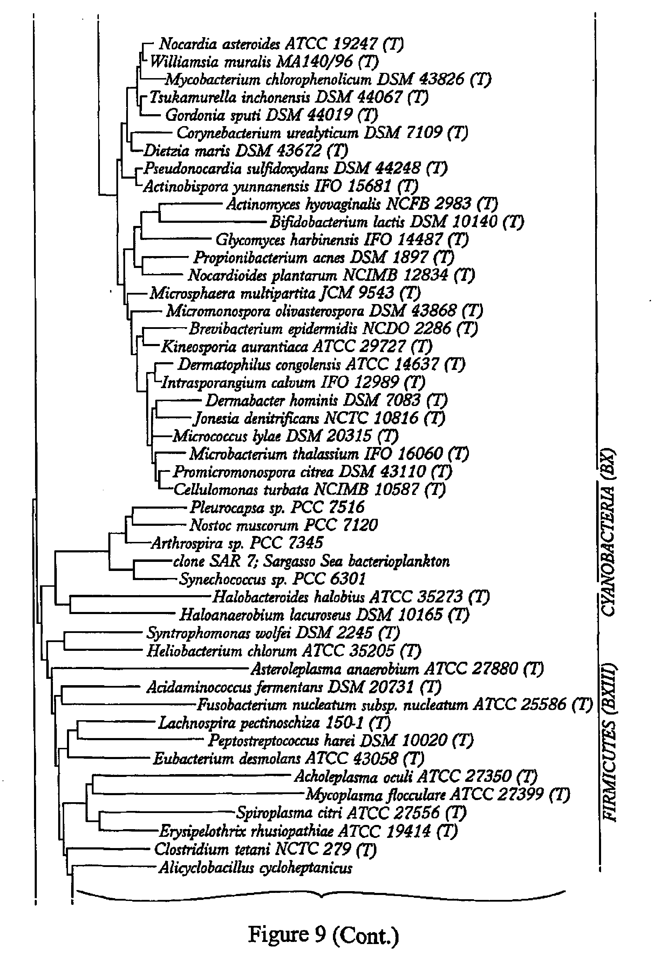

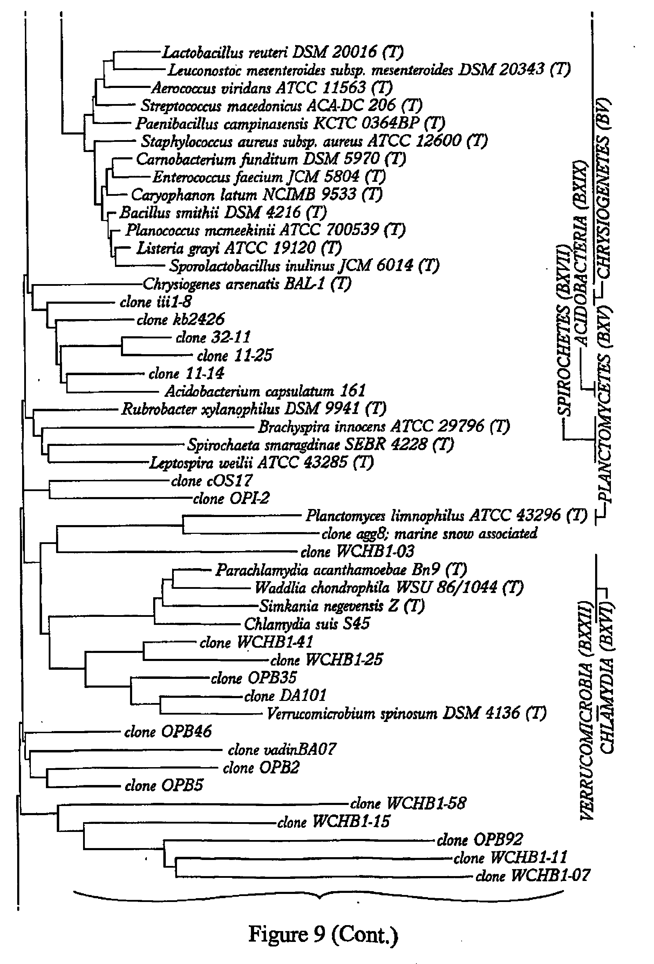

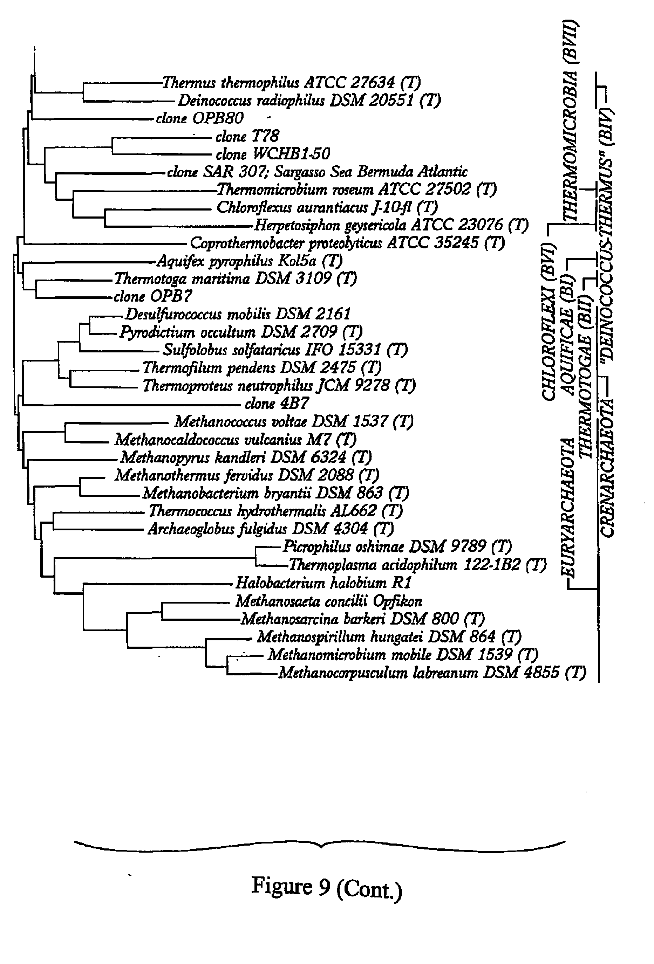

A phylogenic model exists relating organisms by their evolutionary relationship determined by their rRNA sequences ("Taxonomic Outline of the Procaryotic Genera", Bergey's Manual® of Systemic Bacteriology, Second edition, Release 1.0, April 2001) and is shown in Figure 8. This phylogenic model shows 23 different phyla and the classes within these phyla based on data available at the time of the release. A phylum is the first taxonomic level into which classes (the second taxonomic level) of bacteria are assigned by the relationship of their rRNA sequences. The phylogenic model was used to determine closely related organisms so that new delta sequences could be identified by blast searches. After use of psi-blast in examples 4 and 5, delta sequences remained unidentified in several phyla because there were no representatives of those phyla in the NCBI nr database used for Psi Blast searches. To identify δ proteins from organism representative of other phyla of the phylogenic model additional searches are required. To aid in identifying other organisms the 2000 Poster Backbone Tree supplement for ASM 2000 poster presented by Michigan State University and The Ribosome Database Project was also used. This figure has been adapted and appears here as Figure 9. This phylogenic tree allows evolutionarily close organisms to be identified for use in searching for new δ proteins.

Databases were searched from sequencing projects underway at: The Sanger Centre, TIGR, the Institute for Genomic Research, The University of Oklahoma Advanced Center for Genome Technology and DOE-Joint Genome Institute. The searches were conducted using the tblastn BLAST search. The settings used in these tblastn BLAST (Gish, W. and States, D.J., Nature Genet. 3:266-272 (1993)) searches were as described below. This program compares a protein query sequence against a nucleotide database that has been translated in all six reading frames. The search parameters should be set at default, in these searches default parameter options were:
- Expect set at 10
- Matrix set at BLOSUM 62
- HSP score set at "sump"
- Filter type set at " seg"
- Filter low complexity regions
- Sort results by pvalue
- Genetic code set at standard

To be designated as a δ, the protein (hits) must meet the requirements of step 4 of the PSI-BLAST δ search described above in Example 4, method B. To identify δ from new bacterial species the search should initially begin by searching against δ amino acid sequences from organisms that are evolutionarily close (i.e., members of the same class/phylum) to the target organism. If the initial search produces no hits, then searches should continue using δ amino acid sequences as query sequences that are gradually more evolutionarily distant from the target organism using Figure 9 as a guide (picking an organism that is linked by the fewest branches to the target organism) until δ has been identified, or until δ sequences from all known phyla have been exhausted. The organisms whose δ amino acid sequences were used to search target databases to identify new δ proteins are shown in Table 20.

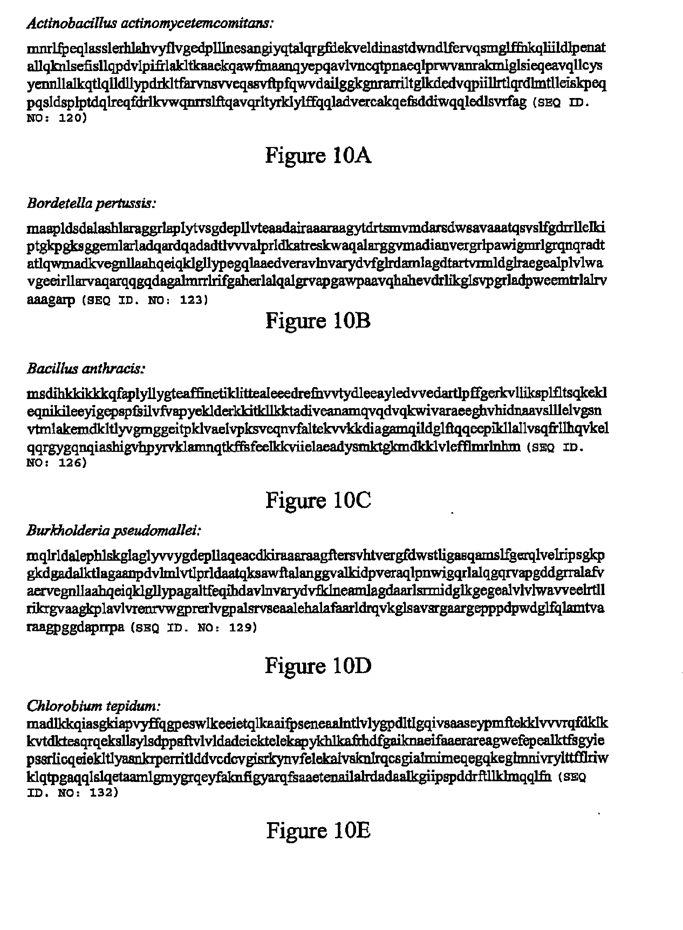

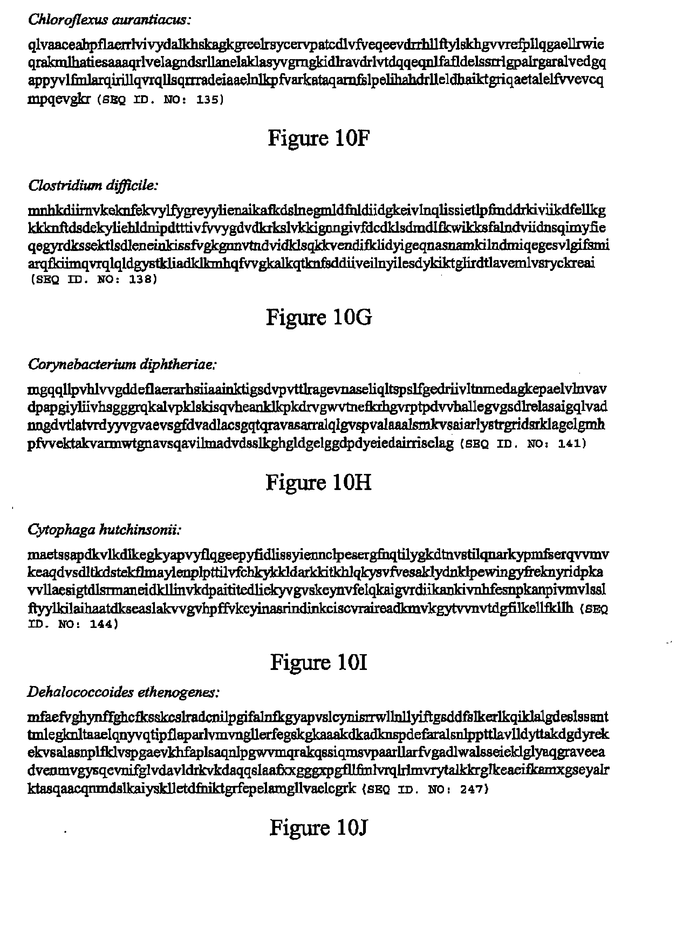

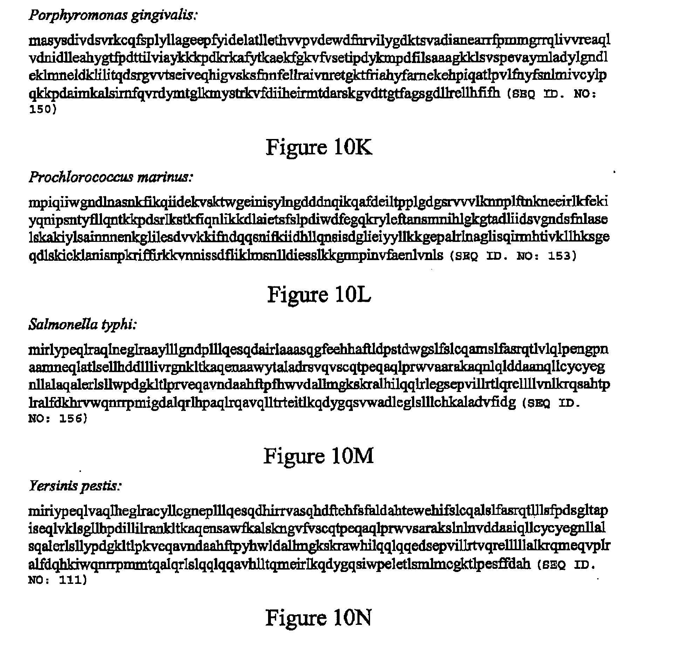

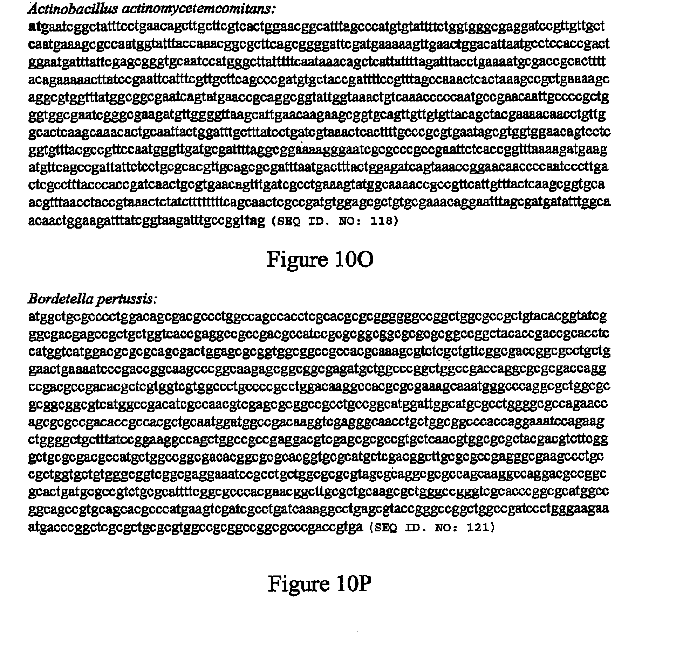

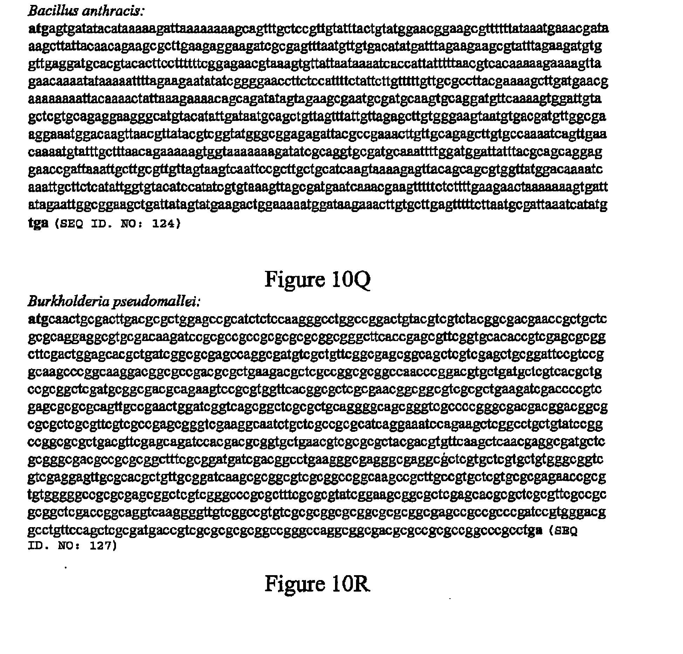

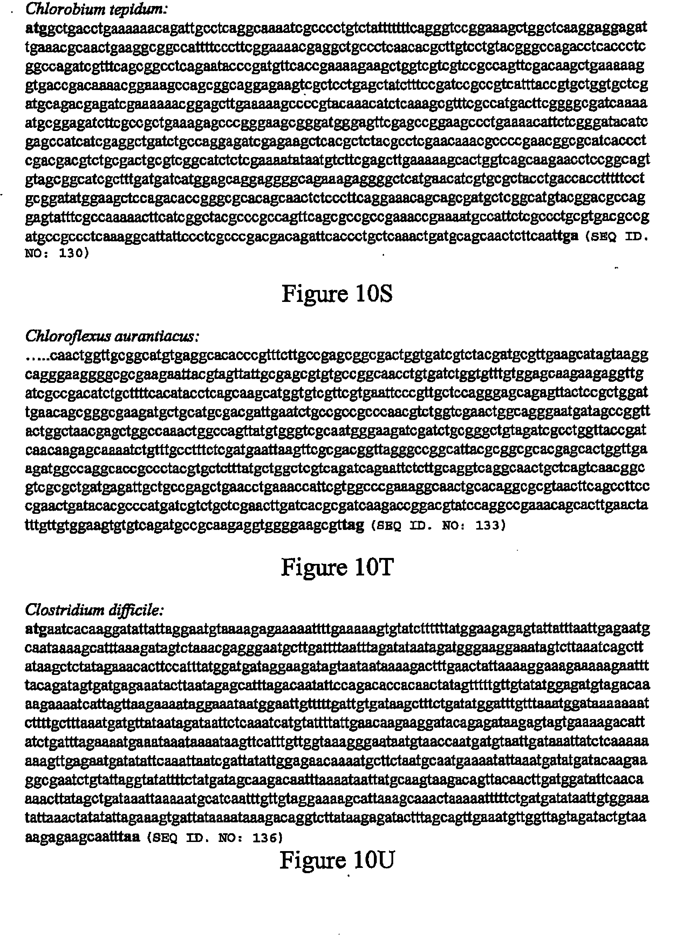

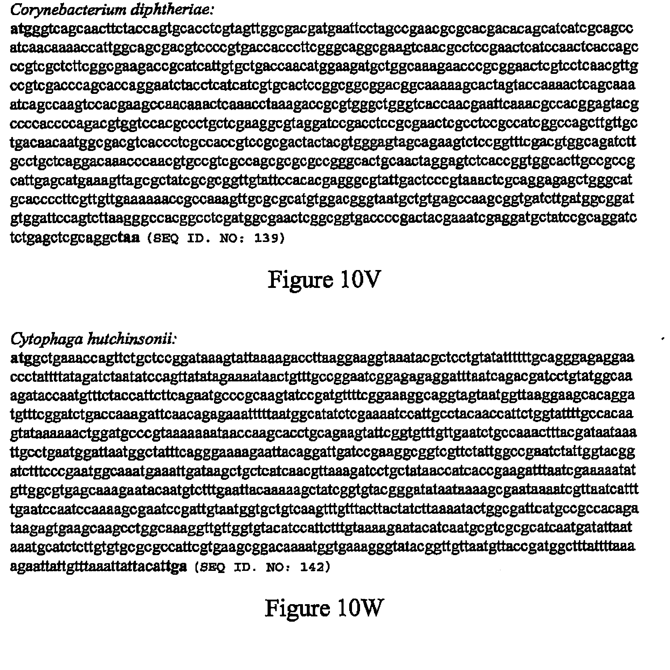

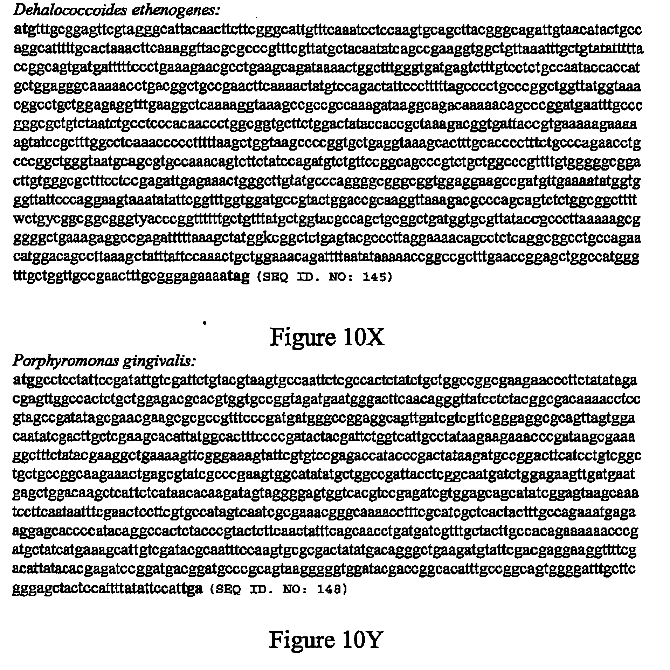

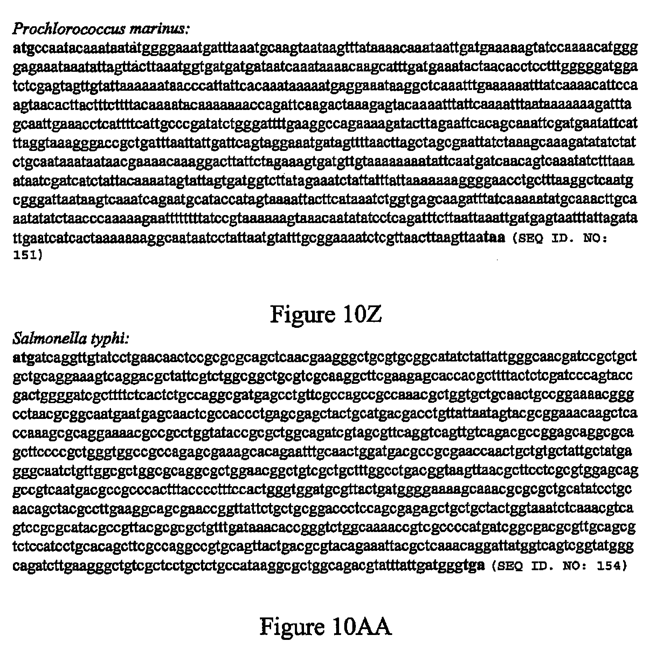

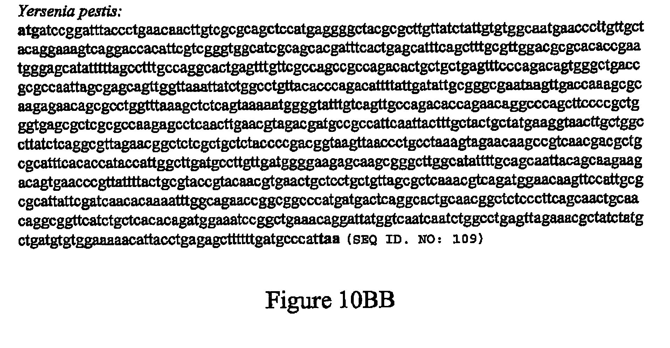

Fourteen additional δ proteins (Figure 10) were identified representing organisms from various phyla. *Bacillus anthracis, Bordatella pertussis, Burkholderia pseudomallei, Chlorobium tepidurn, Clostridum difficile, Corynebacterium diphtheriae, Cytophaga hutchinsonii, Dehalococcoides ethenogenes, Prochlorococcus marinus, Porphyromonas gingivalis, Actinobacillus actinomycetemcomitans, Yersenia pestis* and *Salmonella typhi.* A search of the database of *Chloroflexus aurantiacus* resulted in a protein meeting the requirements of step 4 of the PSI-BI,AST δ search described above, however the DNA encoding this protein was located at the 5' end of a contig and therefore as many as 60 amino acids at the N-terminus of this δ sequence may be missing. These organisms containing the 14 additional δ proteins identified have been added to the phylogenic classification shown as double underlined organisms in Figure 8. This model may be used as a guide in identification of new δ proteins.

These results indicate that using the above outlined procedures, δ can be identified from any organism in which the region of the genome encompassing the gene encoding δ has been sequenced.

The databases containing genomic DNA sequences from the same fourteen organisms from which δ was identified was next searched against evolutionarily close DnaX and δ' proteins using tblastn as described above for tblastn searches to identify δ. The DnaX and δ' gene location or protein annotations are shown in (Table 10) and (Table 11).

As an alternative to performing the described searches in Examples 1-6 using the internet, sequence databases may be installed locally and searches may be conducted using Psi-Blast and Blast. BLAST and PSI-BLAST can be run locally and used to perform searches against local databases. BLAST binaries are provided for Macintosh, Win32 (PC), LINUX, Solaris, SGI, and HP UX systems. Standalone BLAST executables may be found on the NCBI anonymous FTP server under. Read the README file in this directory for more information. Information on setting up Standalone BLAST can also be found at the National Human Genome Research Institute web site.

### Example 7. Identification of the structural gene for the δ subunit of DNA polymerase III holoenzyme (holA) from sequenced thermophilic organisms. Thermotoga maritima and Aquifex aeolicus.

Using the described ψ-BLAST procedure, starting with a 0.001 expectation value for inclusion in the first and subsequent iterations, only *hol*A genes closely related to *E. coli* were identified *(Haemophilis influenzae or Neisseria meningitidis).* These three sequences were included in a second iteration, permitting a search by only elements common between the family of three δs. This resulted in identification of a candidate from *Aquifex aeolicus* (hypothetical protein AQ_1104; length=350 aa; score e=9x10⁻⁷) and candidates from *B*. *subtilis* and *Deinococcus radiodurans.* This procedure was repeated through three iterations until a *Thermotoga maritima* candidate was identified (TM0181; length= 324 amino acids; score e=2x10⁻⁷).

From the The Institute for Genomic Research (TIGR) web site the following sequence for *Thermotoga maritima* protein TM0181 was downloaded: 
mpvtfltgtaetqkeelikkllkdgnveyirihpedpdkidfirsllrtktifsnktiidivnfdewkaqeqkrlvellknvpedvhifir
sqktggkgvalelpkpwetdkwlewiekrfrengllidkdalqlffskvgtndliiereieklkaysedrkitvedveevvftyqtpg
yddfcfavsegkrklahsllsqlwkttesvviatvlanhfldlfkilvlvtkkryytwpdvsrvskelgipvprvarflgfsfktwkfk
vmnhllyydvkkvrkilrdlydldravkseedpkpffhefieevaldvyslqrdee (SEQ ID NO:210)
 The gene sequenced, obtained from the same website was:

cactttgaaattgtttctcaaagcgtgttaaaatcaccATGagaggtgattgtatgccagtcacgtttctcacaggtactgcagaaactc
agaaggaagaattgataaagaaactcctgaaggatggtaacgtggagtacataaggatccatccggaggatcccgacaagatcgattt
cataaggtctttactcaggacaaagacgatcttttccaacaagacgatcattgacatcgtcaatttcgatgagtggaaagcacaggagca
gaagcgtctcgttgaacttttgaaaaacgtaccggaagacgttcatatcttcatccgttctcaaaaaacaggtggaaagggagtagcgct
ggagcttccgaagccatgggaaacggacaagtggcttgagtggatagaaaagcgcttcagggagaatggtttgctcatcgataaaga
tgcccttcagctgtttttctccaaggttggaacgaacgacctgatcatagaaagggagattgaaaaactgaaagcttattccgaggacag
aaagataacggtagaagacgtggaagaggtcgtttttacctatcagactccgggatacgatgatttttgctttgctgtttccgaaggaaaa
aggaagctcgctcactctcttctgtcgcagctgtggaaaaccacagagtccgtggtgattgccactgtccttgcgaatcacttcttggatc
tcttcaaaatcctcgttcttgtgacaaagaaaagatactacacctggcctgatgtgtccagggtgtccaaagagctgggaattcccgttcc
tcgtgtggctcgtttcctcggtttctcctttaagacctggaaattcaaggtgatgaaccacctcctctactacgatgtgaagaaggttagaa
agatactgagggatctctacgatctggacagagccgtgaaaagcgaagaagatccaaaaccgttcttccacgagttcatagaagaggt
ggcactggatgtatattctcttcagagagatgaagaataactggtacagcctcgcggcccttctctcaaccatatacagcaggcacctc
gatgtggaagcaagacctgtgaagtttgaggaaataaa SEQ ID NO:211)

The stop codon (taa) and start (atg) specified in the sequence database are in lower case and underlined.

Subjecting the identified gene and flanking sequences to open reading frame analysis using the NCBI orf finder program (NCBI web site) revealed another in-frame possible start five codons upstream (ATG) in capitals and underlined in the sequence listed above. This alternative start would encode a protein with the penta-peptide sequence MRGDC on the amino-terminal end of the database listing from *Thermotoga maritima* protein TMO181. Other orf finder programs are widely available and well known in the art can be used to identity open reading frames, i.e., Vector NTI V.5.2.1.0, Informax, Inc. (Dec. 9,1998).

From the NCBI Entrez website, the structural gene for hypothetical protein AQ_1104 (SEQ ID NO:) was downloaded and the corresponding protein sequence (SEQ ID NO:) was verified: 
tggcttttacctctttgtagagttaagtattaaatttaatttaactgtggaaaccacaatattccagttccagaaaactttttcacaa
aacctccgaaggagagggtcttcgtccttcatggagaagagcagtatctcataagaacctttttgtctaagctgaaggaaaagtacggg
gagaattacacggttctgtgggggatgagataagcgaggaggaattctacactgccctttccgagaccagtatattcggcggttcaaa
ggaaaaagcggtggtcatttacaacttcggggatttcctgaagaagctcggaaggaagaaaaaggaaaaagaaaggcttataaaagt
cctcagaaacgtaaagagtaactacgtatttatagtgtacgatgcgaaactccagaaacaggaactttcttcggaacctctgaaatccgt
agcgtctttcggcggtatagtggtagcaaacaggctgagcaaggagaggataaaacagctcgtccttaagaagttcaaagaaaaagg
gataaacgtagaaaacgatgcccttgaatacctttctccagctcacgggttacaacttgatggagctcaaacttgaggttgaaaaactgat
agattacgcaagtgaaaagaaaattttaacactcgatgaggtaaagagagtagccttctcagtctcagaaaacgtaaacgtatttgagtt
cgttgatttactcctcttaaaagattacgaaaaggctcttaaagttttggactccctcatttccttcggaatacacccccctccagattatgaaa
atcctgtcctcctatgctctaaaactttacaccctcaagaggcttgaagagaagggagaggacctgaataaggcgatggaaagcgtgg
gaataaagaacaactttctcaagatgaagttcaaatcttacttaaaggcaaactctaaagaggacttgaagaacctaatcctctccctcca
gaggatagacgctttttctaaactttactttcaggacacagtgcagttgctgagggatttcttgacctcaagactggagagggaagttgtg
aaaaatacttctcatggtggataatctttttatgaagtttgcggtttgcgtttttcccggttctaactgcgattacgacacctactacgttataa
gggacattcttgaaaaggacgtggaattc (SEQ ID NO:212)
 The gtg start codon and the taa stop codon are shown as bold underlined text.

mettifqfqktfftkppkervfvlhgeeqylirtflsklkekygenytvlwgdeiseeefytalsetsifggskekavviynfgdflkkl
grkkkekerlikvlrnvksnyvfivydaklqkqelsseplksvasfggivvanrlskerikqlvlkkfkekginvendaleyllqltg
ynlmelkleveklidyasekkiltldevkrvafsvsenvnvfefvdllllkdyekalkvldslisfgihplqimkilssyalklytlkrle
ekgedlnkamesvgiknnflkmkfksylkanskedlknlilslqridafsklyfqdtvqllrdfltsrlerevvkntshgg (SEQ
ID NO:213)

Alignment of the two newly identified δ's with the established δ sequences of *E*. *coli* and *T. thermophilus* and the candidate *B. subtilis* candidate (hypothetical protein YQEN) using ClustalW 1.8 at The Baylor College of Medicine Search Launcher web site (Aiyar, A., et al., Methods Mol. BioL 132:221-241 (2000); Thompson, J. D., et al., Nucleic Acids Res. 25(24):4876-4882 (1997)) resulted in the the alignment shown in Figure 6.

The Baylor College of Medicine Search Launcher is an on-going project to organize molecular biology-related search and analysis services available on the WWW by function by providing a single point-of-entry for related searches (e.g., a single page for launching protein sequence searches using standard parameters). It can be noted that the two newly identified *Aquifex aeolicus* and *Thermotoga maritima* candidate δs share the same pattern of similar residues as the other δ sequences. The ambiguity in the proper start for the *Thermotoga maritima hol*A gene was not resolved by the alignment.

### Example 8. Expression of Aquifex aeolicus holA.

*Aquifex aeolicus hol*A will be expressed to produce a native *Aquifex aeolicus δ* protein with anATG start codon replacing the native gtg start and the natural termination codon and as a protein with an amino-terminal extension with a peptide sequence that is biotinylated in *E*. *coli* and a hexaHis sequence. This peptide (MAGCLNDIFEAQKIEWHHHHHHLVPRGSGGGG .... ) (SEQ ID NO:214) and vectors used for expressing proteins fused to it are described (Kim, D.R. and McHenry,C.S., *ibid.;* PCT WO99/13060). The tagged protein will first be expressed with the amino-terminal Biotin-hexaHis extension because of (*i*) the ease of monitoring and quantifying expression of tagged δ using conjugated streptavidin reagents in biotin blots (Kim, D.R. and McHenry,C.S., *ibid.)* and (*ii*) for ease in subsequent purifications aided by affinity chromatography on Ni⁺⁺-NTA and monomeric avidin columns. Purified tagged protein will be used for initial attempts to form reconstitution assays (see Example 16) and for an antigen for production of rabbit polyclonal antibodies that can be used to monitor the purification of native *Aquifex aeolicus δ* in the absence of a functional assay, if necessary. Polyclonal and monoclonal antibodies will be made by methods old and well known in the art Total protein levels will be determined by the Bradford procedure (Bradford, M.M., Anal. Biochem. 72:248-254 (1976)) or other suitable method in this and other examples.

To express tagged *Aquifex aeolicus* δ, the sequence encoding the biotin/hexaHis tag will be fused with the second codon *of Aquifex aeolicus hol*A. This will be accomplished by PCR, which will be used to amplify the *Aquifex hol*A gene (AQ_1104). The primers will be designed so that *a Pst*I site will be added to the 5' end of the gene and a *Hind*III site to the 3' end of the gene.
The forward primer will be:
5'-gatc*ctgcag*GAAACCACAATATTCCAGTTCC-3' (SEQ ID NO:157)

The complementary portion of this primer is shown as upper case and begins at codon #2 (the ATG start codon of the Aquifex *holA* gene will be excluded). The *Pst*I site is shown in italics and underlined. This will place the open reading frame (ORF) of the *Aquifex* delta gene inframe with the N-terminal fusion peptide. There is a 4 base extension on the 5' end of the primer to allow for efficient digestion by *Pst*I.

The reverse primer will be:
5'-gatc*aagctt*ttaTTATCCACCATGAGAAGTATTTTTCAC-3' (SEQ ID NO:158)

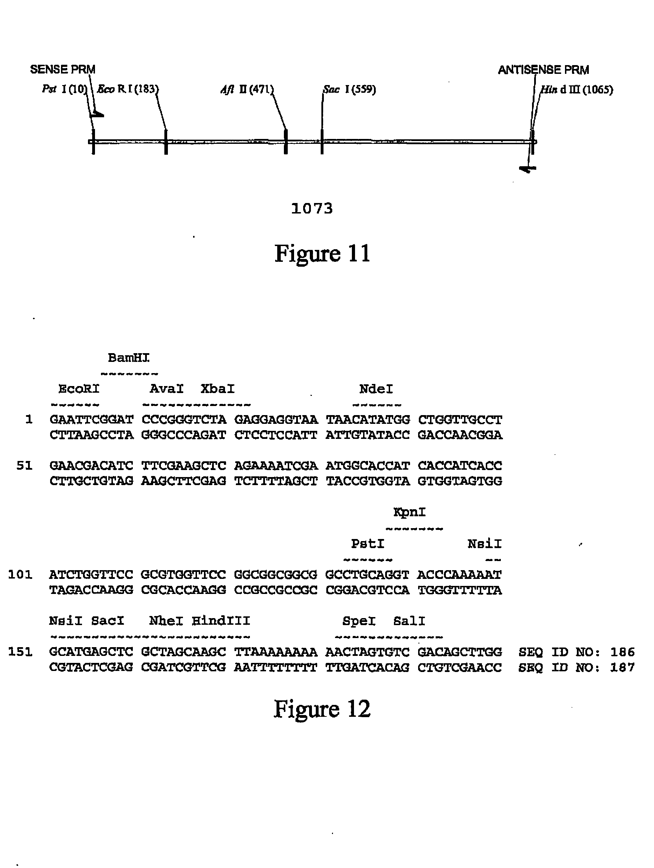

The complementary portion of the primer is shown as upper case. The *Hind*III restriction site is in italics and underlined and there is a 4 base extension to allow for efficient digestion by *Hind*III. The non-complementary portion contains an additional stop codon (tta) to enhance termination of translation. A graphical depiction of the PCR product is shown in Figure 11. The PCR product will be digested with *PstI* and *Hind*III and inserted into the vector pAl-NB-KpnI digested with the same restriction enzymes. The region of pAl-NB-KpnI that the PCR product will be inserted into is shown in Figure 12. This resulting plasmid (pA1-NB-AQD) will be transformed into *E. coli* DH5α. Positive isolates will be selected by ampicillin resistance and plasmids isolated from the positive isolates will be sequenced across the region contain the insert. Verification of expression will be accomplished by cell growth to OD 0.5 and induction by addition of 1 mM IPTG for three hours. Extracts of cells will be subjected to electrophoresis on SDS gels, transferred to membranes and detected with streptavidin conjugated reagents as taught in PCT WO 99/13060 and United States Provisional Patent Application No. 60/192,736, filed July 14, 2000, the disclosure of which are hereby incorporated by reference.

In the above procedures and in the following examples, the *following general molecular cloning procedures* will be used: All intermediate plasmids will be transformed into *E*. *coli* DH5α Amp^{R} colonies will be selected and the plasmids will be screened for gain or loss of the appropriate restriction sites. The sequences of inserted DNA will be confirmed by DNA sequencing. Ligations will be performed in the presence of T4 DNA ligase and ATP. Expression of proteins will be confirmed by transforming the plasmids into *E*. *coli* MGC1030 (mcrA, mcrB, lamBDA(-), (RRND-RRNE)1, lexA3) and AP1.L1 *E. coli* bacteria. The parent to the AP1.L1 bacterial strain was BLR bacterial strain [F-, ompT hsdSB(rB-mB-) gal dcm δ(sr1-recA)306::Tn10 (Novagen). A T1 phage-resistant version of this BLR strain, is desginated AP1.L1. Single colonies (3 colonies from each transformation) of transformed cells selected for by ampicillin-resistance will be inoculated into 2 ml of 2xYT culture media containing 100 *µ*g/ml ampicillin and grown overnight at 37 °C in a shaking incubator. In the morning, 0.5 ml of the turbid culture from the overnight growth will be inoculated into 1.5 ml of fresh 2xYT culture media. The cultures will be grown for 1 hour at 37 °C with shaking and expression will be induced by addition of isopropyl-β-D-thiogalactopyranoside (IPTG) to a final concentration of 1 mM. The cells will be harvested by centrifugation 3 hours post-induction. The cell pellets will be immediately resuspended in 1/10 culture volume of 2x Laemelli sample buffer (2x solution: 125 mM Tris-HCl (pH 6.8), 20% glycerol, 4% sodium dodecyl sulfate (SDS), 5% β-mercaptoethanol, and 0.005% bromophenol blue w/v), and sonicated to complete lysis of cells and to shear the DNA. The samples will be heated for 10 minutes at 90-100°C, and centrifuged to remove insoluble debris. An aliquot (3 µl) of each supernatant will be subjected to electrophoresis in a 4-20% SDS-polyacrylamide mini-gel (Novex, EC60255; 1 mm thick, with 15 wells/gel) in 25 mM in Tris base, 192 mM glycine, and 0.1 % SDS. The resulting gels will be stained with Coomassie Brilliant Blue to visualize protein bands.

Proteins expressed as tagged proteins will receive d-biotin at the time of induction to a final concentration of 10 *µ*M. The control culture received *d*-biotin only and will not be induced with IPTG. The expressed proteins will be analyzed using biotin blots. The total protein in each lysate will be transferred (blotted) from polyacrylamide gel to nitrocellulose membrane using a Novex transfer apparatus at 30 V constant voltage in 12 mM Tris base, 96 mM glycine, 0.01% SDS (w/v), and 20% methanol (v/v) for 60 minutes at room temperature. The membrane will be blocked in 0.2% Tween 20 (v/v)-TBS (TBST) (tris-buffered saline; 8 g/L NaCl, 0.2 g/L KCl, 3 g/L Tris-HCl (pH 7.4)) containing 5% non-fat dry milk (w/v) for 1 hour at room temperature. The blotted nitrocellulose will next be rinsed using TBST, and then incubated in 2 µg/ml alkaline phosphatase-conjugated streptavidin (Pierce Chemical Co.) in TBST for 1 hour at room temperature. Following extensive washing with TBST, the blot will be developed with BCIP/NBT (KPL #50-81-07; one component system). The endogenous *E. coli* biotin-carboxyl carrier protein (biotin-CCP, ca. 20 kDa) can be detectable in both induced and non-induced samples. The target protein expressed as an N-terminal tagged protein will be visualized in the induced samples only.

*Aquifex aeolicus* δ will be expressed as a native protein. The gene (AQ_1104) will be amplified from *Aquifex* genomic DNA using primers designed to allow insertion of the gene into a vector in which the gene can be expressed in a native form.

The forward primer will be designed so that the start "ATG" codon will incorporated into an *Nde*I restriction site. The forward primer is shown below.
5'-gatc*catATG*GAAACCACAATATTCCAGTTCC-3' (SEQ ID NO:159)

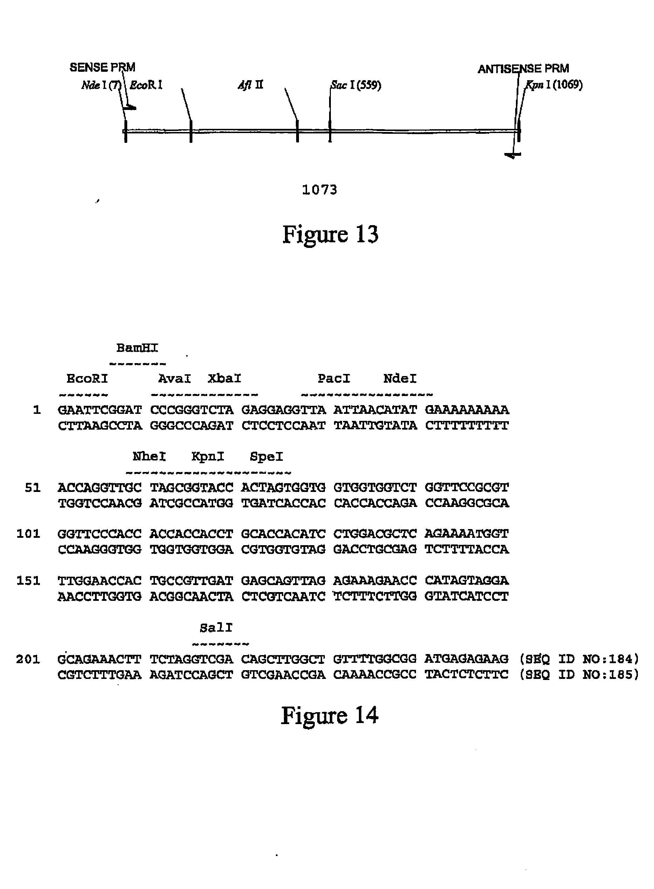

The complementary portion of this primer is shown as upper case, the *Nde*I site is in italics and underlined. There is a 4 base extension on the 5' end of the primer to allow for efficient digestion by *Nde*I.
The reverse primer will contain a *Kpn*I site and is shown below.
gatc*ggtacc*ttaTTATCCACCATGAGAAGTATTTTTCAC-3' (SEQ ID NO:160)
The complementary portion of the primer is shown as upper case. The *Kpn*I restriction site is in italics and underlined and there is a 4 base extension to allow for efficient digestion by *Kpn*I. The non-complementary portion also contains an additional stop codon (tta) to enhance termination of translation. A graphical depiction of the PCR product is shown in Figure 13.

The PCR product will be digested with *NdeI* and *KpnI* and inserted into the vector pA1-CB-NdeI (74) digested with the same restriction enzymes. The region of pAl-CB NdeI that the PCR product will be inserted into is shown in Figure 14. There is a sequence encoding a C-terminal biotin/hexaHis fusion peptide located downstream of the *KpnI* site and thus downstream of the stop codon of the inserted *Aquifex* holA gene (AQ_1104) and will not be expressed. The sequence of these plasmids will verified as described above and expressed in *E*. *coli* MGC1030 and AP1.L1.

Verification of expression will be accomplished by examination of Coomassic-stained SDS gels from extracts of candidate cells for IPTG-expression of a band of the expected molecular weight. If that procedure fails, polyclonal antibodies will be raised against purified tagged *Aquifex aeolicus δ* and expressed proteins detected by Western Blots. Expression will also be verified and quantified by employing functional assays as described under Example 16.

### Example 9. Purification of Aquifex aeolicus δ

Tagged *Aquifex aeolicus* δ will be purified by ammonium sulfate fractionation, Ni⁺⁺-NTA chromatography, and, if needed, monomeric avidin chromatography. Extracts will be prepared by formation of spheroplasts and a gently heat-induced lysis in the presence of spermidine (PCT W099/13060). Ammonium sulfate fractions (30, 40, 50, 60 70% saturation) will be made and the amount of tagged δ precipitated relative to total protein determined by biotin blots in the linear response range. The concentration of ammonium sulfate that results in precipitation of 80% of the tagged δ will be used in subsequent purifications. An ammonium sulfate pellet resulting from an optimized fractionation procedure will be dissolved in a suitable buffer and chromatographed on Ni⁺⁺-NTA columns by procedures similar to those described (PCT WO99/13060). The elution position of tagged δ will be determined from stained SDS gels and Biotin-blots. If protein is pure, it will be dialyzed and used to support antibody production and preliminary functional assays. If needed, tagged δ first will be further purified by monomeric avidin chromatography (PCT WO99/13060).

Native *Aquifex aeolicus* δ will be purified by ammonium sulfate fractionation and standard chromatographic procedures. Purification will await the assembly of a reconstituted reaction employing purified tagged *Aquifex aeolicus* pol III holoenzyme subunits α, β, δ, δ', ∈ and DnaX (see Example 16). A functional assay is important for optimal production of fully active subunits free of tags that might partially impair reaction efficiency or weaken protein-protein interactions. Only with a functional assay is it apparent when a procedure leads to protein inactivation. A functional assay is also useful in monitoring the presence of the desired protein in column fractions in the presence of contaminants. Activity is quantified before and after each chromatographic step and only those procedures that result in retention of significant (generally > 50%) activity with the loss of contaminating proteins are included in the final purification procedures.

Purification development is an empirical process. Optimizing an ammonium sulfate fractionation from lysates will be the first step, much as described for the tagged protein above, except that δ will be quantified by a functional assay. Then, a survey a variety of cation exchange, anion exchange, hydroxyapatite and hydrophobic interaction chromatographic resins will be used to determine buffer conditions required for binding of δ activity and elution. For those resins that give the highest yields, gradient elution conditions will be optimized to give the optimal separation of δ from contaminants consistent with high recovery. Successive chromatographic procedures will be developed until δ is nearly homogeneous. The final purification step will likely be gel filtration since it permits removal of any aggregated protein and provides an efficient method to place the purified protein in an optimal storage buffer. Final recovered protein will be aliquoted and stored under optimal conditions, likely rapid freezing by plunging into liquid N₂ and storage at - 80 °C until needed.

### Example 10. Expression of Thermotoga maritima holA

TM0181 will be expressed as a fusion protein, tagged on its N-terminus, much like described for *Aquifex aeolicus holA* under Example 8. Since there is ambiguity in the start site, the more upstream site, will be used to ensure all coding sequences are included. Specifically, PCR will be used to amplify the *T. maritima* holA gene (TM0181). The primers will be designed so that a *KpnI* site will be added to the 5' end of the gene and a *Spe*I site to the 3' end of the gene. The gene encoding the *T. maritima* holA gene contains an internal *PstI* site, which is normally used for inserting genes in frame with an N-terminal biotin/hexaHis fusion peptide. To overcome this problem the expression vector pA1-NB-KpnI contains a *KpnI* restriction site that partially overlaps the *PstI* site. Use of this restriction sites will result in an additional two amino acids (Val and Pro) being added in the final protein between the N-terminal biotin/hexaHis fusion peptide and the *T. maritima* holA gene.
The forward primer will be:
5-'gatc*ggtaccc*AGAGGTGATTGTATGCCAGTC-3' (SEQ ID NO:161)
The complementary portion of this primer is shown as upper case and begins at codon #2 (the ATG start codon of the *T. maritima holA* gene will be excluded), the *KpnI* site is in italics and underlined. There is a 4 base extension on the 5' end of the primer to allow for efficient digestion by *Kpn*I. There is an additional nucleotide "c" in the non-complementary region of the primer to maintain an ORF between the biotin/hexaHis fusion peptide and the *T. maritima* holA gene.

The reverse primer is shown below.
*5'-gatcactagttta* TTATTCTTCATCTCTCTGAAG-3' (SEQ ID NO:162)

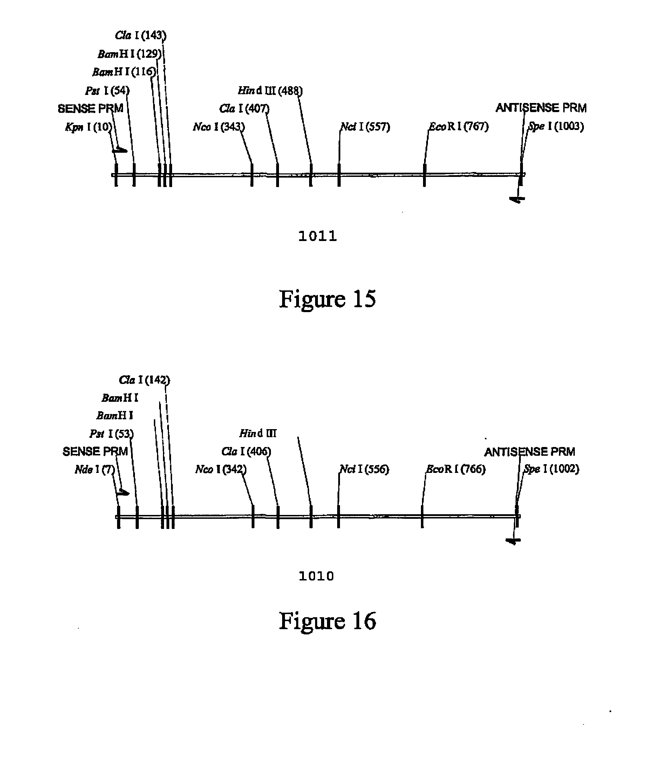

The complementary portion of the primer is shown as upper case. The SpeI restriction site is in italics and underlined and there is a 4 base extension to allow for efficient digestion by *SpeI.* The non-complementary portion also contains an additional stop codon (tta) to enhance termination of translation. A graphical depiction of the PCR product is shown in Figure 15.

The PCR product will be digested with *KpnI* and *Spe*I and inserted into the vector pA1 NB-KpnI digested with the same restriction enzymes. The region of pA1-NB-KpnI that the PCR product will be inserted into is shown in Figure 12. These plasmids (pA1-NB-TMD) will be transformed into DH5αbacteria and screened for ampicillin resistance, isolated from positive isolates and the sequence of the isolated plasmids will verified be as described above and expressed in *E*. *coli* MGC1030 and AP1.L1. Expressing cells will be verified as described under Example 8.

Antibody against purified tagged *Thermotoga maritima* δ (obtained from the procedures described under Example 8) will be used to immunoprecipitate δ from *Thermotoga maritima* extracts. The precipitated protein will be further purified by SDS polyacrylamide gel electrophoresis, transferred to a membrane and sequenced at its amino-terminus to determine which of the two alternative start sites are used. If this procedure fails, both possible 6 proteins will be expressed to determine which works best in reconstituting a *Thermotoga maritima* DNA polymerase III holoenzyme.

Native (untagged) *Thermotoga maritima* δ will be expressed by amplification of the gene (TM0181) from *T. maritima* genomic DNA using primers designed to allow insertion of the gene into a vector in which the gene can be expressed in a native form.

The forward primer will be designed so that the start "ATG" codon will be incorporated into an *Nde*I restriction site. The forward primer is shown below.
5'-gatc*catATG*AGAGGTGATTGTATGCCAG-3' (SEQ ID NO:163)
The complementary portion of the primer is shown as upper case. The *Nde*I restriction site is in italics and underlined and there is a 4 base extension to allow for efficient digestion by *Nde*I.

The reverse primer is the same reverse primer used in construction of pA1-NB-TMD (SEQ ID NO:) and contains a *Spe*I restriction site and an addition stop codon adjacent to the native stop codon.
A graphical depiction of the PCR product is shown in Figure 16. The PCR product will be digested with *Nde*I and *Spe*I and inserted into the vector pA1-CB-NdeI digested with the same restriction enzymes. The region of pA1-CB-NdeI that the PCR product will be inserted into is shown in Figure 14. These plasmids (pA1-TMD) will be transformed into DH5α screened for ampicillin resistance, isolated from positive isolates and the sequence of the isolated plasmids will verified as described above and expressed in *E*. *coli* MGC1030 and AP1.L1. Expression will be verified as described under Example 8 for *Aquifex aeolicus δ*. The shorter version of this gene beginning at the second possible start site can also be inserted into expression vectors. This will be accomplished using the same strategy used to clone both tagged and untagged forms of the gene encoding the longer version of the *Thermotoga maritima* δ gene.

### Example 11. Purification of Thermotoga maritima δ

Tagged and native *Thermotoga maritima* δ will be purified by strategies similar to those described under Example 9. Antibodies against tagged protein will be prepared as described in Example 8.

### Example 12. Identification of the Structural Gene for the δ' Subunit of DNA Polymerase III Holoenzyme (holB) from Sequenced Thermophilic Organisms, Thermotoga maritima and Aquifex aeolicus

All sequenced bacteria contain two genes that show significant homology to bacterial *dna*X. The shorter of the two proteins that shows lesser homology is δ' in *E*. *coli* (Carter, J.R, *et al.,* (1993) *ibid. )* and *T. thermophilus.* This principal was exploited to detect the structural genes for δ' (*hol*B) in *Thermotoga maritima* and *Aquifex aeolicus.* A blast search was conducted using *E*. *coli dna*X and the default parameters of BLAST except the option of showing up to 250 matches was indicated. The *Aquifex aeolicus dnaX* gene was located (e=1 x 10⁻⁶⁵) and a second gene (aq_1526; 312 amino acids; e=5 x 10⁻¹⁴) was located, providing a candidate *Aquifex hol*B*.* The following protein sequence of *Aquifex aeolicus* hypothetical protein aq_1526 (SEQ ID NO: 215) was downloaded from the NCBI Entrez website: >gi|2983903|gb|AAC07454.1] hypothetical protein [Aquifex aeolicus]

MEKVFLEKLQKTLHIPGGLLFYGKEGSGKTKTAFEFAKGILCKENVPWGCGSCPSCK
HVNELEEAFFKGEIEDFKVYKDKDGKKHFVYLMGEHPDFVVIIPSGHYIKIEQIREVK
NFAYVKPALSRRKVIIIDDAHAMTSQAANALLKVLEEPPADTTFILTTNRRSAILPTILS
RTFQVEFKGFSVKEVMEIAKVDEEIAKLSGGSLKRAILLKENKDILNKVKEFLENEPL
KVYKLASEFEKWEPEKQKLFLEIMEELVSQKLTEEKKDNYTYLLDTIRLFKDGLARG
VNEPLWLFTLAVQAD (SEQ ID NO:215)

The following DNA sequence encoding aq_1526 (SEQ ID NO:216) was downloaded and verified using the NCBI orf finder program. (The start and stop codons are underlined):

actctctttcagacttaaagaaaagatacgggaaattctcggcgagaaatttgagcatttaataaagttttaggggatataatttccgataatacacttg
aaacacctttaataacactcggtgaaggtaaaaacctcaagaaaaagtttaacgaggaattcatagaaacccttgagaatgcttcctattcaaacgag
gaggccggaaaattaagggaatttttaaagacactttacgctttggcactctgtaatagtagggaagaaatcactctggacctttgtgtaatattactcc
actccgaacagcacgcaactttagaaaaacaactcctttctctcgtacaaccgctactaaaaattgataaaaaaccgaagaaagaactgaacctata
gtccgcgaagttgcaaaggttataaacgaaacctgtttaaattaaatttgtATGgaaaaagtttttttggaaaaactccagaaaaccttgcacatacc
cggaggactcctttttacggcaaagaaggaagcggaaagacgaaaacagcttttgaatttgcaaaaggtatttatgtaaggaaaacgtaccgtgg
ggatgcggaagttgtccctcctgcaaacacgtaaacgagctggaggaagccttctttaaaggagaaatagaagactttaaagtttataaagacaag
gacggtaaaaagcacttcgtttaccttatgggcgaacatcccgactttgtggtaataatcccgagcggacattacataaagatagaacagataaggg
aagttaagaactttgcctatgtgaagcccgcactaagcaggagaaaagtaattataatagacgacgcccacgcgatgacctctcaggcggcaaac
gctctttttaaaggtattggaagagccacctgcggacaccacctttatcttgaccacgaacaggcgttctgcaatcctgccgactatcctctccagaac
ttttcaagtggagttcaagggcttttcagtaaaagaggttatggaaatagcgaaagtagacgaggaaatagcgaaactctctggaggcagtctaaaa
agggctatcttactaaaggaaaacaaagatatcctaaacaaagtaaaggaattcttggaaaacgagccgttaaaagtttacaagcttgcaagtgaatt
cgaaaagtgggaacctgaaaagcaaaaactcttccttgaaattatggaagaattggtatctcaaaaattgaccgaagagaaaaaagacaattacac
ctaccttcttgatacgatcagactctttaaagacggactcgcaaggggtgtaaacgaacctctgtggctgtttacgttagccgttcaggcggatTA

Ataaaccgttattgattccgtaacatttaaaccttaatctaaattatgagagcctttgaaggaggtctggtatggaaaatttgaagattagatatatagat
acgaggaagataggaaccgtgagcggtgtaaaagttccggtaaaaaagggctacaaaatagtcgtggaagacgag (SEQ ID NO:
216)

For *Thermotoga maritima,* the primary homology was again found to the annotated *dna*X (TM0686; 478 amino acids; e=2x10⁻⁶⁴); however, two protein of secondary homology were identified: TM0508 (599 residues; e=1x10⁻⁴) and TM0771 (documented as γ-subunit related in the *Thermotoga maritima* TIGR database) (312 residues; e=6x10⁻⁴). The finding of three dnaX-like genes in one bacterium is unprecedented. Documented δ' sequences fall in the 300-400 amino acid range, suggesting that TM0771 was the most likely *Thermotoga maritima hol*B candidate. The following protein and DNA sequences were downloaded from the NCBI website; the start and stop codons (underlined below) of each orfwas verified and the corresponding start and stop sites underlined within the DNA sequence:

TM0508: (SEQ ID NO:217)
MSISEKPLSELLRPKDFEDFVGQDHIFGNKGILRRTLKTGNMFSSILYGPPGSGKTSVF
SLLKRYFNGEVVYLSSTVHGVSEIKNVLKRGEQLRKYGKKLLLFLDEIHRLNKNQQ
MVLVSHVERGDIVLVATTTENPSFVIVPALLSRCRILYFKKLSDEDLMKILKKATEVL
NIDLEESVEKAIVRHSEGDARKLLNTLEIVHQAFKNKRVTLEDLETLLGNVSGYTKES
HYDFASAFIKSMRGSDPNAAVYYLVKMIEMGEDPRFIARRMIIFASEDVGLADPNAL
HIAVSTSIAVEHVGLPECLMNLVECAVYLSLAPKSNSVYLAMKKVQELPVEDVPLFL
RNPVTEEMKKRGYGEGYLYPHDFGGFVKTNYLPEKLKNEVIFQPKRVGFEEELFERL
RKLWPEKYGGESMAEVRKELEYKGKKIRIVKGDITREEVDAIVNAANEYLKHGGGV
AGAIVRAGGSVIQEESDRIVQERGRVPTGEAVVTSAGKLKAKYVIHTVGPVWRGGS
HGEDELLYKAVYNALLRAHELKLKSISMPAISTGIFGFPKERAVGIFSKAIRDFIDQHP
DTTLEEIRICNIDEETTKIFEEKFSV

(SEQ ID NO:218):
caagacaggatccgtacggtgaagcgggcgtggtggccatcttcactgaaaggatgctcaggggagaggaagttcacatattcggtgatggaga
gtacgtcagagactacgtttacgttgatgacgtggtcagagcgaatcttctcgccatggagaaaggcgacaacgaagtcttcaacataggaacgg
gaaggggcacaaccgtgaaccagctcttcaaactgctgaaagagatcactggctacgataaagagcctgtctataaaccaccgcgtaagggcga
cgtgagaaagagcattctcgattacacgaaggcgaaggaaaagctcggctgggaacccaaagtttcccttgaggagggattgaagctcaccgTT
TGagtatttcagaaaaacccttgagtgaactgctcaggccgaaagacttcgaggatttcgtcggtcaggatcatatattcggtaataaagggattct
ccggcgaactttaaagacaggcaacatgttctcttctatcctctatggaccaccggggtctggtaagacctcggtttttcactgctgaaaaggtatttc
aacggcgaggtagtttatctgagctccaccgttcacggggtttctgaaataaagaacgttctcaaaagaggagaacagttgagaaaatatggaaaa
aaattgcttctctttctcgatgaaatacaccgcctgaacaaaaaccagcagatggttctggtttcccatgttgaacgaggagacatcgtactggttgcg
acaacaactgaaaatccgagttttgtcatcgtacctgcacttctctcaaggtgcaggattctttatttcaaaaaactctctgacgaggacctgatgaaga
tcttgaaaaaagcaacagaggttctcaacatcgatctggaagaatcggtagagaaagccatagtaaggcattccgaaggagacgccagaaaactc
ttgaacaccctggagatcgtccaccaggcgttcaaaaacaagagagtaactcttgaggacctggaaactctgctgggaaacgtgagcgggtacac
gaaggaatcacactacgatttcgcttcggccttcataaagagtatgagaggtagcgatccaaacgccgctgtttactacctcgtcaaaatgatagag
atgggagaagacccgcgattcatagcacgaagaatgatcatattcgccagcgaagacgttggactcgccgacccaaacgctctacatatcgccgt
ttcgacctccatcgctgtcgaacacgtgggacttcccgaatgtttgatgaaccttgtagagtgtgccgtctatctttccctcgctcccaagagcaactct
gtttatctcgcaatgaaaaaagtccaggaacttcctgtggaggacgtaccgctttttctgagaaatcccgtcaccgaagagatgaaaaagcgtggat
acggagaagggtatctttatccccatgatttcggtggtttcgtgaaaacgaactatcttccagaaaagttgaaaaatgaggtcatcttccagccaaag
agagtaggttttgaggaagaactctttgaaaggctcagaaaactctggcccgagaagtacgggggtgaaagtatggccgaagtgagaaaagaac
tggaatacaaagggaaaaagatcagaatcgtaaagggagacatcacaagagaagaagtggacgccatagtgaacgcggcgaacgaatatctga
aacacggaggaggagtagcgggagcgatcgtgagagcaggtggaagcgttatccaggaagaaagcgacaggatcgttcaagaacgaggaag
agtcccgacaggtgaagcggtggtaaccagcgctggaaagctgaaggcaaaatacgtgatccacaccgtggggcccgtttggagaggaggca
gtcatggagaggacgaacttctctacaaagcagtttacaacgctcttcttcgagctcacgaactgaaattgaagagcatctcaatgcctgctatcagt
acaggaatattcggatttccgaaagaaagagcggtgggaatctttttcaaaagcaataagagatttcatcgatcaacatcctgataccactctggaag
agatccgcatatgcaacatagatgaggaaacaacgaagattttcgaagaaaagttcagcgttTGAtgaaaccaaaagaaggggcaaacgccc
cttctttttaagacctctgaggaagtattgaatcaactcaaacgagttcttggacctcttccgcaacagatttccatacctgaaaggaactattgatattt

catcataccacaaaaactaaaaaagtaaagggggccaagggcccccttatttccatcagaattcttcaggcatgctcggtgtttcttcttctcctcgg
gtttttcaacgatgagcacttccgttgtcaggagcattccagcgatggatgcagcattctgcagggcgctccttgtaacctttgctgggtcaatgattc
ctctttcaaacatgttgcagtattctcctctgagtgcgtcgaatccataggctggatcgtcattcgaaagtatcttctcaatgatcaccgctccgtcgtat
cccgcgttttcagcaatctgcttaataggggctga

TM0771: (SEQ ID NO:219)
mndlirkyakdqletlkriieksegisilingedlsyprevslelpeyvekfppkasdvleidpegenigiddirtikdflnyspelytr
kyvivhdcermtqqaanaflkaleeppeyavivlntrrwhyllptiksrvfrvvvnvpkefrdlvkekigdlweelpllerdfktale
ayklgaeklsglmeslkvletekllkkvlskglegylacrellerfskveskeffalfdqvtntitgkdaflliqrltriilhentwesved
qksvsfldsilrvkianlnnkltlmnilaihrerkrgvnaws

(SEQ ID NO:220):
ggaagatagctccgctccgcccaaacgtccggttgctcgttcagaatgtggtacagtatctcaagaccgtggtttgaagtacctacctcgtaagtgtc
gggaaaaacgagggctatccggagggaaatttcctctggatctttcacaacgctgttgatttctcttcctatgtatctacttggtttctaacccagggaa
gtgtttcattcaagaattttagaatcatcctttcacctcagttctgattttatcattggttgttataattagaaggcgaggtgagatcATGaacgatttgat
cagaaagtacgctaaagatcaactggaaactttgaaaaggatcatagaaaagtctgaaggaatatccatcctcataaatggagaagatctctcgtat
ccgagagaagtatcccttgaacttcccgagtacgtggagaaatttcccccgaaggcctcggatgttctggagatagatcccgagggggagaacat
aggcatagacgacatcagaacgataaaggacttcctgaactacagccccgagctctacacgagaaagtacgtgatagtccacgactgtgaaaga
atgacccagcaggcggcgaacgcgtttctgaaggcccttgaagaaccaccagaatacgctgtgatcgttctgaacactcgccgctggcattatcta
ctgccgacgataaagagccgagtgttcagagtggttgtgaacgttccaaaggagttcagagatctcgtgaaagagaaaataggagatctctggga
ggaacttccacttcttgagagagacttcaaaacggctctcgaagcctacaaacttggtgcggaaaaactttctggattgatggaaagtctcaaagtttt
ggagacggaaaaactcttgaaaaaggtcctttcaaaaggcctcgaaggttatctcgcatgtagggagctcctggagagatttcaaaggtggaatc
gaaggaattctttgcgctttttgatcaggtgactaacacgataacaggaaaagacgcgtttcttttgatccagagactgacaagaatcattctccacga
aaacacatgggaaagcgttgaagatcaaaaaagcgtgtctttcctcgattcaattctcagggtgaagatagcgaatctgaacaacaaactcactctg
atgaacatcctcgcgatacacagagagagaaagagaggtgtcaacgcttggagcTGAaggcaagggcagtgggagtggagataataccaa
agggtaagataatttactactctgtacccaacggtgatgagtacgagagaggagacctcgttctggtactgggagatttggacttgaagtgggaaa
ggttctgatacctcccagagaggtggtcatagacgaggtggggtacgaactcaaggccgtgatcagaaagttgacggatgaagatctggaacaat
acaggaagaatgtagaggatgcgttgaaggccttccagatatgtaagcagaaaataaaagaacacggtcttcccatgaagcttctctacgccaagt
acaccttcgatagaactaggctcatctttttcttcagtgcggaaggaagggttgatttcagagagcttgtaagagacctcgcaaagatcttcaaaaca
aggatagagttgaggcaagttggtgtgagagatgagatgaagttctttggaggccttggattgtgcggacttccgacatgctgttccacatttctcag
agagttcaccagcgtcacactgaagcacgcaaagaagcagcagatgatgatcaatccagcgaagatatccggaccatgcagaaggcttctgtgc
tgtcttacctacgaatacgatttctatgaaaaggagctggagggaattcctgacgaagggagcacgatcacctacgaaggaaagcgctacaaagt
cgtgaacgtgaacgtgtttctcagaacggtgactctcttctcggaaca

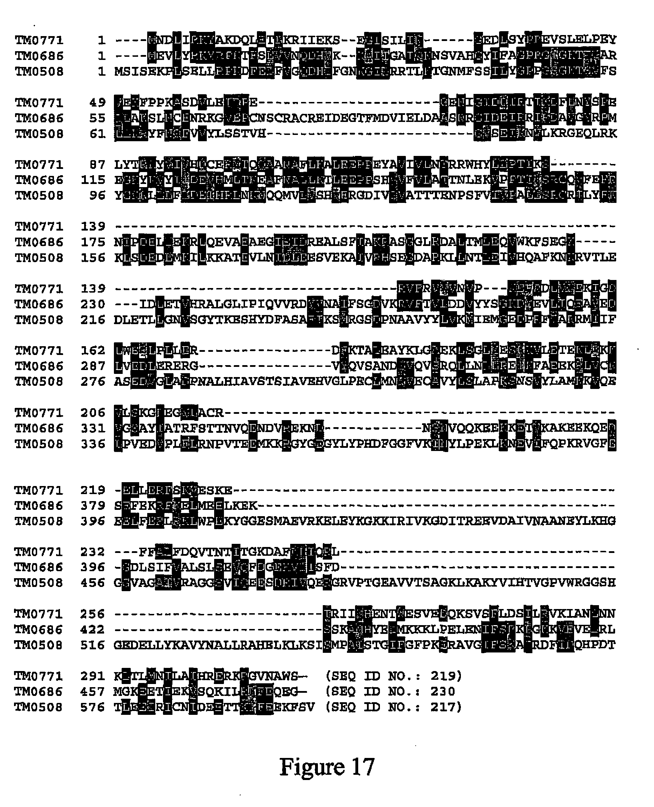

Rather than just relying on the homology score, the three *dna*X like *Thermotoga maritima* proteins were aligned using Clustal so that their similarities and differences could be more closely scrutinized as shown in Figure 17.

Both TM0686 and TM0508 appear, by sequence, to be functional ATPases. Both have perfect agreement with the Walker A motif (GXGKT(T/S) (SEQ ID NO:221) found in all functional DnaX molecules, but lacking from δ's. Both have functional "DEAD" boxes of the type found in functional DnaX (DEhH, where h is a bulky hydrophobic residue), and both have an SRC sequence. TM0771 lacks a functional ATPase, and a reduced "DEAD-box" homology, a property shared with δ' sequences. TM0771 and TM0686 share significant similarity through an 11-residue stretch of unknown function that is shared by other DnaX and δ' proteins. TM0686 exhibits a perfect match to the NALLKTLEEPP (SEQ ID NO:222) sequence found in both *E. coli* DnaX and δ'. SRV is found in TM0771 in place of the conserved SRC found in TM0686 and TM0508. These data suggest strongly that these three *Thermotoga maritima* proteins are part of a β processivity factor assembly apparatus and that TM0771 is the best *hol*B candidate. TM0686 (annotated in the TIGR database as the *Thermotoga maritima* γ and τ subunits) and TM0508 (annotated as a hypothetical protein) are both likely functional ATPases in the DnaX complex. Perhaps TM0686 encodes the γ-homologue and the longer TM0508 the τ-homologue in *Thermotoga maritima.* Alternatively, with two distinct DNA polymerase III molecules in *Thermotoga maritima,* TM0508 and TM0686 may serve different polymerase IIIs. All candidates will be expressed and employ them to reconstitute a *Thermotoga maritima* processive DNA polymerase III holoenzyme.

### Example 13. Expression of Aquifex aeolicus holB

PCR will be used to amplify the *Aquifex hol*B gene (AQ_1526). The primers will be designed so that *a Pst*I site will be added to the 5' end of the gene and a *KpnI* site to the 3' end of the gene.

The forward primer will be :
5'-gatc*ctgcag*GAAAAAGTTTTTTTGGAAAAACTCC-3' (SEQ ID NO: 164)

The complementary portion of this primer is shown as upper case and begins at codon #2 (the ATG start codon of the Aquifex holB gene will be excluded). The *Pst*I site is shown in italics and underlined. This will place the open reading frame (ORF) of the *Aquifex* holB gene in frame with the N-terminal fusion peptide. There is a 4 base extension on the 5' end of the primer to allow for efficient digestion by *Pst*I. The reverse primer will be:
5'-gatc*ggtacc*ttaTTAATCCGCCTGAACGGCTAACG-3' (SEQ ID NO: 165)

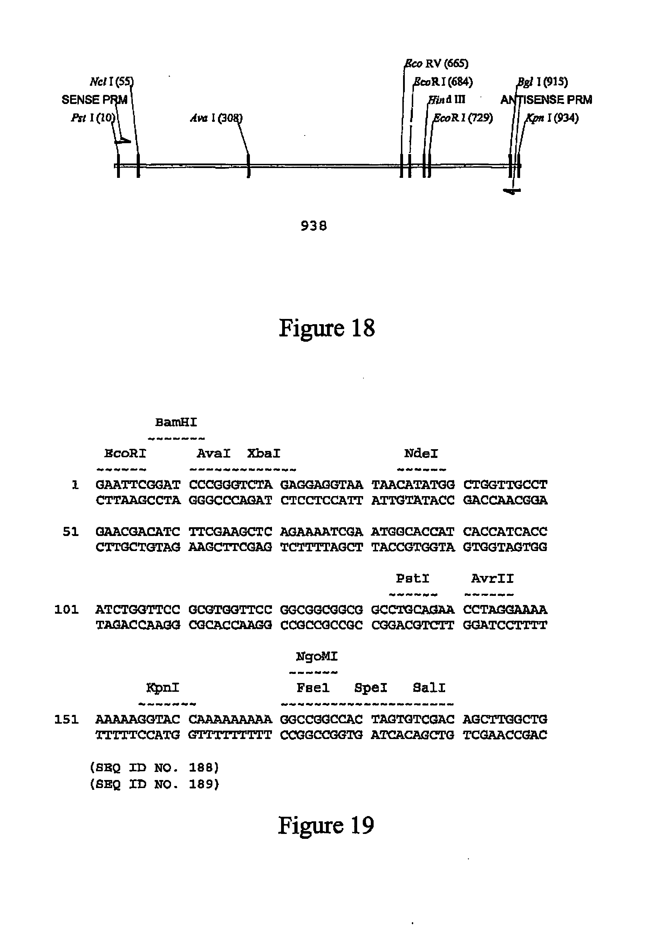

The complementary portion of the primer is shown as upper case. The *Kpn*I restriction site is in italics and underlined and there is a 4 base extension to allow for efficient digestion by *Kpn*I. The non-complementary portion also contains an additional stop codon (tta) to enhance termination of translation. A graphical depiction of the PCR product is shown in Figure 18.

The PCR product will be digested with *Pst*I and *Kpn*I and inserted into the vector pA1-NB-Avr2 (74) digested with the same restriction enzymes. The region of pA1-NB-Avr2 that the PCR product will be inserted into is shown in Figure 19.

These plasmids (pA1-NB-AQD') will be transformed into DH5αbacteria and screened for ampicillin resistance, isolated from positive isolates and the sequence of the isolated plasmids will verified as described above and expressed *in E. coli* MGC1030 and AP1.L1.

To express the *Aquifex* δ' subunit in an untagged form, the gene (AQ_1526) will be amplified from *Aquifex* genomic DNA using primers designed to allow insertion of the gene into a vector in which the gene can be expressed in a native form.

The forward primer will be designed so that the start "ATG" codon will incorporated into an *Nde*I restriction site. The forward primer is shown below.
5'.gatc*catATG*GAAAAAGTITTTITGGAAAAACTCC-3' (SEQ ID NO:166)

The complementary portion of the primer is shown as upper case. The *NdeI* restriction site is in italics and underlined and there is a 4 base extension to allow for efficient digestion by *Nde*I*.*

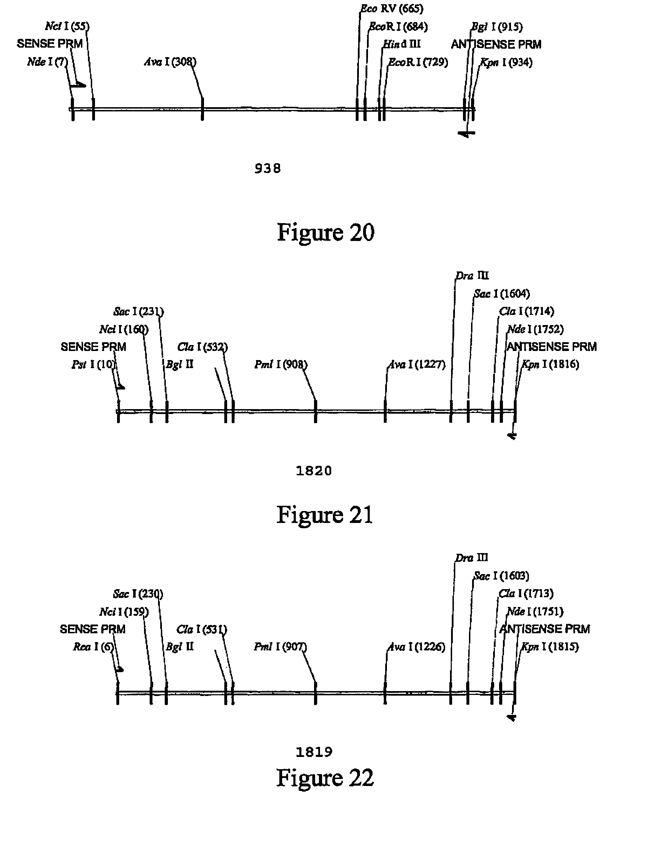

The reverse primer is the same reverse primer used in construction of pA1-NB-AQD' (SEQ ID NO: I65) and contains a *Kpn*I restriction site and an addition stop codon adjacent to the native stop codon. A graphical depiction of the PCR product is shown in Figure 20.

The PCR product will be digested with *NdeI* and *KpnI* and inserted into the vector pA1-CB-NdeI digested with the same restriction enzymes. The region ofpAl-CB-NdeI that the PCR product will be inserted into is shown in Figure 14.These plasmids (pA1-AQD') will be transformed into DH5α screened screened for ampicillin resistance, isolated from positive isolates and the sequence of the isolated plasmids will verified as described above and expressed *in E. coli* MGC1030 and AP1.L1.

### Example 14. Purification of Aquifex aeolicus δ'

*Aquifex aeolicus δ',* both in tagged and native (untagged) form will be purified using the approaches and strategies outlined in Example 9.

### Example 15. Expression and Purification of Aquifex aeolicus Replication Proteins: α, ∈, β, DnaX and SSB

Native and tagged *Aquifex aeolicus* α, ∈, β, DnaX and SSB will be expressed and purified using the general strategies described in the preceding examples and in references (Kim, D.R. and McHenry, C.S., *ibid.;* Dallmann, H.G. and McHenry, C.S., J Biol Chem 270:29563-29569 (1995); Dallmann, H. G., et al., J Biol Chem 270:29555-29562 (1995); PCT WO99/13060). α will be purified using a gap-filling assay on nuclease-activated DNA to monitor its presence, yield and specific activity. ∈ will be purified using its intrinsic nuclease activity in the presence and absence of a to monitor its purification (Maki and Kornberg, (1987) *ibid.;* Griep, M., et al., Biochemistry 29:9006-9014 (1990); Reems, J., et al., J. Biol. Chem. 266:4878-4882 (1991)). β and DnaX will initially be purified as tagged proteins and once a reconstitution assay is developed, they will be purified in native form. SSB will be expressed in native form and purified by established procedures (Griep, M. and McHenry, C., J. Biol Chem. 264:11294-11301 (1989); Lohman,T M., et al., Biochemistry 25:21 (1986)).

### Example 16. Development of Reconstitution Assays for Subunits of the Aquifex aeolicus DNA Polymerase III Holoenzyme

For both *E. coli* and *T. thermophilus,* a combination of the α, DnaX, β, δ and δ' subunits results in reconstitution of a minimally processive replicase capable of replicating a long single-stranded template, such as primed M13Gori DNA (Kornberg, and Baker, *ibid.*; PCT WO99/13060). The ∈ subunit of the *E. coli* DNA polymerase III core stabilizes and stimulates the α subunits polymerization activity; thus, it too will be included in the anticipated replication reaction, as convenient. Once the *Aquifex aeolicus* replication subunits are available in tagged form, they will be mixed together with primed M13Gori DNA primed with an annealed DNA oligonucleotide primer or primed in a separate reaction by the action *of E. coli* SSB and *E. coli* DnaG primase. dNTPs, ATP and Mg⁺⁺ will be included at the experimentally determined optimal concentrations (initially 1 mM ATP, 10 mM Mg⁺⁺, 150 *µ*M dCTP, dATP, dGTP and 50 *µ*M [³H] TTP (ca. 400 cpm/pmol)). Reactions will be conducted at the experimentally-determined optimum temperature (starting at 65 °C) and performed for a time when the amount of DNA synthesis is proportional to time (initially 5 min.). Reactions will be compared with *Tth* proteins as a control. If roughly the same level of synthesis is observed with the *Aquifex aeolicus* proteins as the tagged *Tth* control proteins, optimization of the reaction will follow.

If poor synthesis is observed with approximately the same level of tagged Aquifex *aeolicus* proteins as used in the *Tth* control reaction, levels of individual tagged proteins (or groups of proteins) will be increased in an attempt to optimize synthesis. Higher levels of a protein or proteins may be required if missing proteins are required for optimal function of the complex or if tags interfere with critical protein-protein interactions. If increasing levels of tagged proteins does not result in reconstitution of an efficient reaction, ammonium sulfate cuts for individual subunits (or groups of subunits) for the corresponding tagged proteins will be substituted to determine which protein(s) are defective in tagged form. The ammonium sulfate concentration required to yield a precipitate optimized for protein recovery without inclusion of unnecessary contaminants will be determined using antibodies for quantitative Western Blots in advance of the availability of a functional assay. If an ammonium sulfate fraction of an untagged protein is found to be more active than a tagged purified protein, it will be substituted for the tagged protein in the reconstituted reaction. If problems remain, individual *Aquifex aeolicus* proteins will be expressed at decreased (15°C) or increased (43°C) temperatures for varying times or in the presence of co-overprodued chaperones (GroEL.GroES) and/or (DnaK/DnaJ/GrpE) to try to increase the relative levels of properly folded active proteins.

Once an optimal reaction is reconstituted, in the presence of individual limiting subunits, the level of the other subunits required to give an optimal reaction with minimal background will be determined. Whether the synthesis obtained in individual reactions is proportional to the level of the limiting protein added will then be determined. This linear response is necessary to use the assay as a tool for quantifying the level of the limiting protein.

Once the *Aquifex aeolicus* reconstitution reaction is established as a quantitative tool, it can be used to monitor and quantify the purification of individual native untagged *Aquifex aeolicus* replication proteins. Varying quantities of protein fractions containing unknown levels of a specific *Aquifex aeolicus* subunit will be added to reactions containing optimal levels of the other subunits. Values obtained where at least two levels of protein give proportional responses will be used for quantitation. The level of a given protein required to give one pmole of DNA synthesis (total nucleotide incorporated) in one minute under optimal reaction conditions as one unit will be expressed. As individual untagged proteins become available in pure form, they will be substituted for the untagged protein in the reconstitution assay. This will require reoptimization of the assay in terms of levels of the added untagged protein. If the untagged protein binds more tightly to the other components than its tagged counterpart, lower levels might be required to achieve optimal synthesis.

### Example 17. Tests of Interactions of Aquifex aeolicus δ and δ' subunits

In addition to the requirement for candidate *Aquifex aeolicus δ'* and δ to reconstitute the *Aquifex aeolicus* replicase in the presence of the required components, being able to demonstrate function and implied utility in reconstituted replication reactions by showing these protein interact with each other and other components of the *Aquifex aeolicus* replicase is anticipated.

It is anticipated that expressed, purified *Aquifex aeolicus δ* and δ' will interact with each other. Demonstration of an interaction will demonstrate that each participate in the same functional unit, providing evidence that each was appropriately identified and that a functional protein is expressed. Levels of δ and δ' sufficient for detection upon a ten-fold dilution will be mixed and subjected to gel filtration on a Sepharose 12 column. Protein will be detected by either Coomassie-staining of SDS polyacrylamide gels of individual eluted fractions or by Western-blots of gels blotted to membranes. Individual δ and δ' will be subjected to gel filtration under identical conditions. An interaction is indicated by a change in elution position of both δ and δ' to a position to a higher molecular weight with both proteins present in roughly stoichiometric ratios in the eluted fractions. If an interaction is not observed, the concentration of δ and δ' will be increased up to 10 *µ*M.

If an interaction is not observed, it may be due to a requirement for DnaX for a tight complex to form. If an interaction is not observed between δ and δ' alone, DnaX will be included in the mixture subjected to gel filtration and gels analyzed to see if δ and δ' shift to a higher molecular weight and co-elute with DnaX.

It is also expected that *Aquifex aeolicus* δ will interact with *Aquifex aeolicus β.* δ and β will be mixed and subjected to gel filtration as described above in this section for δ and δ'. If an interaction is not observed, *Aquifex aeolicus* β will be mixed with δ-δ' and DnaX in the presence of ATP to test an interaction. This is the complex that is expected to be functional in the assembly of *β* onto primed DNA (Naktinis, V*., et al., ibid*)*.*

### Example 18. Reconstitution of Aquifex aeolicus DNA Polymerase III Holoenzyme

Once all *Aquifex aeolicus* replication proteins are available in purified form they will be combined and used for purposes of (i) synthesis of DNA at high temperatures (temperatures at or above 60 °C), (ii) synthesis of high molecular weight DNA in applications requiring either a single high temperature or thermal cycling, (iii) processive synthesis of high molecular weight DNA (processive indicates incorporation of greater than 1000 nucleotides per association-elongation-polymerase dissociation event), (iv) rapid synthesis of DNA at rates greater than 200 nucleotides/second, (v) highly accurate DNA synthesis resulting from inclusion of the ∈ subunit, and (vi) for processive reactions in the presence of expressed *Aquifex aeolicus* SSB or *E. coli* SSB.

### Example 19. Expression of Thermotoga maritima Protein TM0508 (Hypothetical Second DnaX protein)

PCR will be used to amplify the *T. maritima* gene (TM0508). The primers will be designed so that a *Pst*I site will be added to the 5' end of the gene and *a Kpn*I site to the 3' end of the gene.

The forward primer will be :
5'-gatc*ctgcag*AGTATTTCAGAAAAACCCTTG-3' (SEQ ID NO: 167)

The complementary portion of this primer is shown as upper case and begins at codon #2 (the ATG (TTG) start codon of the T. maritima gene (TM0508) will be excluded). The *PstI* site is shown in italics and underlined. This will place the open reading frame of the *T. maritima* gene (TM0508) inframe with the N-terminal fusion peptide. There is a 4 base extension on the 5' end of the primer to allow for efficient digestion by *Pst*I.

The reverse primer will be:
5'-gatc*ggtacc*taaTCAAACGCTGAACTTTTCTTCG-3' (SEQ ID NO: 168)

The complementary portion of the primer is shown as upper case. The *Kpn*I restriction site is in italics and underlined and there is a 4 base extension to allow for efficient digestion by *Kpn*I*.* The non-complementary portion also contains an additional stop codon (tta) to enhance termination of translation. A graphical depiction of the PCR product is shown in Figure 21.

The PCR product will be digested with *Pst*I and *Kpn*I and inserted into the vector pA1-NB-Avr2 (74) digested with the same restriction enzymes. The region of pA1-NB-Avr2 that the PCR product will be inserted into is shown in Figure 19. These plasmids (pA1-NB-TM[0508]) will be transformed into DH5α bacteria and screened for ampicillin resistance, isolated from positive isolates and the sequence of the isolated plasmids will verified as described above and express in *E*. *coli* MGC1030 and AP1.L1.

To express the *T*. *maritima* subunit [TM0508] in an untagged form, the gene (TM0508) will be amplified from *T*. *maritima* genomic DNA using primers designed to allow insertion of the gene into a vector in which the gene can be expressed in a native form. The TM0508 gene contains internal *NdeI* sites which will not allow the use of the pA1-CB-NdeI expression vector. This problem was circumvented by using an expression vector (pA1-CB-NcoI) which contained an *Nco*I restriction site in place of the *Nde*I restriction site. The *Nde*I restriction site contained the "ATG" used as the start codon at the end of the restriction site (catatg). The *Nco*I restriction site however, contains the "ATG" (c/catgg) internally. The first two codons of the TM0508 gene are "atgagt", which code for Met and Ser. Thus, this restriction site could not be use as part of the forward primer and allow both insertion into the *Nco*I restriction site of pA1-GB-NcoI and retain both codons encoding Met and Ser. This problem was overcome by adding the *Rca*I restriction site (t/catga) to the forward primer. This restriction site allows the PCR product cleaved with *Rca*I to be spliced into pA1-CB-NcoI cleaved with *Nco*I. This will destroy both restriction sites but will allow the TM0508 gene to be inserted into the pA1-CB-NcoI expression vector. The forward primer is shown below.
5'-gatc*tcATGA*GTATTTCAGAAAAACCC-3' (SEQ ID NO: 169)

The complementary portion of the primer is shown as upper case. The *Rca*I restriction site is in italics and underlined and there is a 4 base extension to allow for efficient digestion by *Rca*I.

The reverse primer is the same reverse primer used in construction of pA1-NB-TM[0508] (SEQ ID NO:168) and contains a *Kpn*I restriction site and an addition stop codon adj scent to the native stop codon. A graphical depiction of the PCR product is shown in Figure 22.

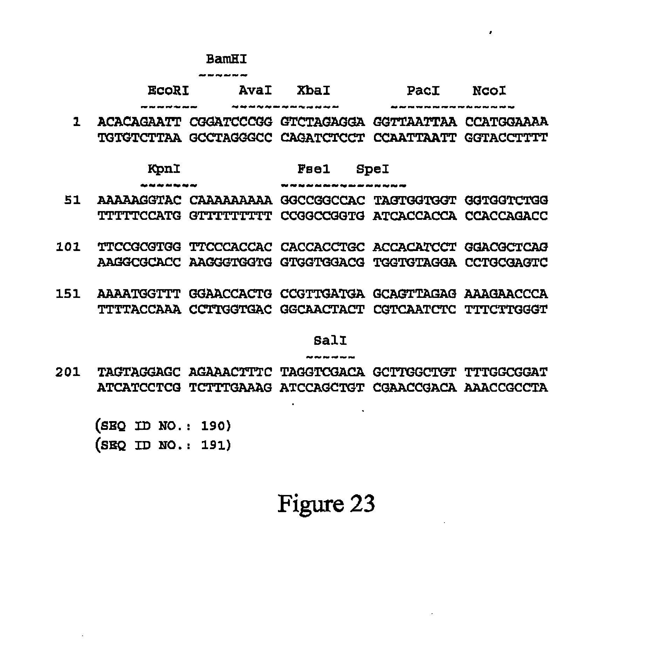

The PCR product will be digested with *Rca*I and *Kpn*I and inserted into the vector pA1-CB-NcoI digested with the same restriction enzymes. The region of pA1-CB-NcoI that the PCR product will be inserted into is shown in Figure 23.

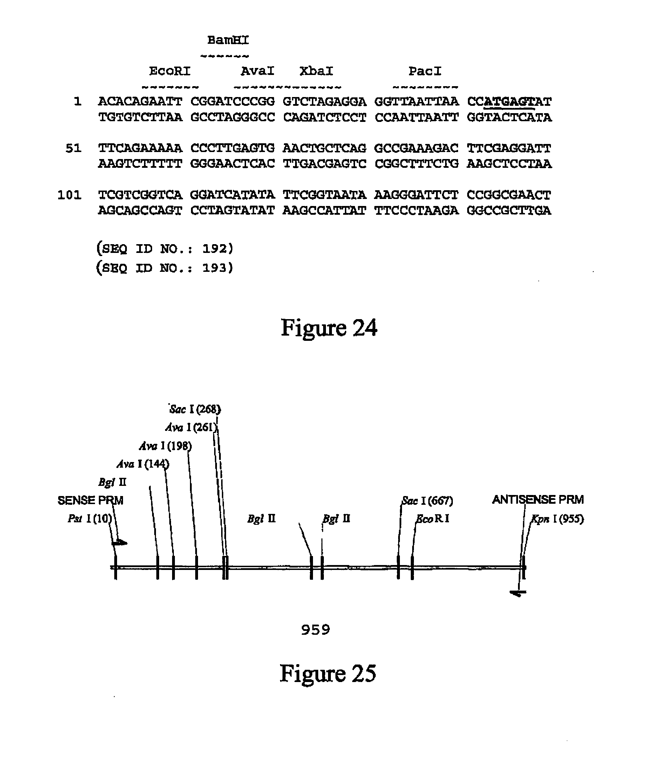

As described in the preceeding paragraphs, insertion of the RcaI/KpnI digested PCR product into the NcoI/KpnI digested pAl-CB-NcoI (74) resulted in the plasmid pAl-TM[0508]. The region of pAl-TM[0508] containing the site in which the RcaI digested restriction site was spliced into the NcoI restriction site of pA1-CB-NcoI is shown in Figure 24.

The first two codons of the TM0508 gene in pA1-TM[0508] are shown in bold underlined text in Figure 24. Both the *Nco*I and the *Rca*I restrictions no longer exist in this plasmid. These plasmids (pA1-TM[0508]) will be transformed into DH5α bacteria and screened for ampicillin resistance, isolated from positive isolates and the sequence of the isolated plasmids will verified as described above and express in *E*. *coli* MGC1030 and AP1.Ll.
Note: This 'second' DnaX-like protein from Thermotoga maritima does not meet the full consensus sequence criteria for a DnaX protein as specified in this application, but is sufficiently DnaX-like that it will be expressed and tested in reconstituted Thermotoga DNA polymerase III holoenzyme reactions as described to test whether it constitutes an additional required or adjunct protein not found in other bacterial replicases to date.

### Example 20. Expression of Thermotoga maritima protein TM0771 (Hypothetical HoIB (δ'))

Primers will be designed so that *a Pst*I site will be added to the 5' end of the gene and a *Kpn*I site to the 3' end of the gene.
The forward primer will be :
5'-gat*cctgcag*AACGATTTGATCAGAAAGTACGC-3' (SEQ ID NO: 170)

The complementary portion of this primer is shown as upper case and begins at codon #2 (the ATG start codon of the T. maritima δ' gene (TM0771)will be excluded). The *Pst*I site is shown in italics and underlined. This will place the open reading frame of the *T. maritima* δ' gene (TM0771) in frame with the N-terminal fusion peptide. There is a 4 base extension on the 5' end of the primer to allow for efficient digestion by *PstI.*

The reverse primer will be:
5'-gatc*ggtacc*ttaTCAGCTCCAAGCGTTGACACC-3' (SEQ ID NO:171)

The complementary portion of the primer is shown as upper case. The *KpnI* restriction site is in italics and underlined and there is a 4 base extension to allow for efficient digestion *by Kpn*I*.* The non-complementary portion also contains an additional stop codon (tta) to enhance termination of translation. A graphical depiction of the PCR product is shown in Figure 25.

The PCR product will be digested with *Pst*I and *Kpn*I and inserted into the vector pA1-NB-Avr2 digested with the same restriction enzymes. The region of pA1-NB-Avr2 that the PCR product will be inserted into is shown in figure 19. These plasmids (pA1-NB-TMD'[0771]) will be transformed into DH5αbacteria and screened for ampicillin resistance, isolated from positive isolates and the sequence of the isolated plasmids will verified as described above and expressed in *E*. *coli* MGC1030 and AP1.L1.

To express the *T. maritima* δ' subunit [0771] in an untagged form, the gene (TM0771) will be amplified from *T. maritima* genomic DNA using primers designed to allow insertion of the gene into a vector in which the gene can be expressed in a native form.

The forward primer will be designed so that the start "ATG" codon will incorporated into an *NdeI* restriction site. The forward primer is shown below.
5'-gatccatATGAACGATTTGATCAGAAAGTACGC-3' (SEQ ID NO: 172)

The complementary portion of the primer is shown as upper case. The *NdeI* restriction site is in italics and underlined and there is a 4 base extension to allow for efficient digestion by *NdeI.*

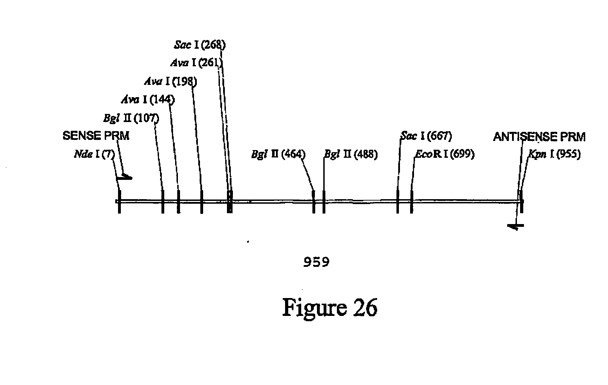

The reverse primer is the same reverse primer used in construction of pA1-NB-TMD'[0771] (SEQ ID NO:171) and contains a *Kpn*I restriction site and an addition stop codon adjacent to the native stop codon. A graphical depiction of the PCR product is shown in Figure 26.

The PCR product will be digested with *Nde*I and *Kpn*I and inserted into the vector pA1-CB-NdeI digested with the same restriction enzymes. The region of pA1-CB-NdeI that the PCR product will be inserted into is shown in figure 14. These plasmids (pA1-TMD'[0771]) will be transformed into DH5α screened screened for ampicillin resistance, isolated from positive isolates and the sequence of the isolated plasmids will verified as described above and expressed in *E. coli* MGC1030 and AP1.L1.

### Example 21. Purification of Thermotoga maritima protein TM0508 (Hypothetical Second DnaX Protein)

*Thermotoga maritima* TM0508, both in tagged and native (untagged) form will be purified using the approaches and strategies outlined in Example 9. Being able to monitor the purification of the native protein will be dependent on its requirement for reconstitution of a *Thermotoga maritima* replicase.

### Example 22. Purification of Thermotoga maritima protein TM0771 (Hypothetical HolB (δ'))

*Thermotoga maritima* δ', both in tagged and native (untagged) form will be purified using the approaches and strategies outlined in *Example3* except the reconstitution assays described under Example 18 will be employed.

### Example 23. Expression of Components of the Thermotoga maritima DNA Polymerase III Holoenzyme: α Subunit of DNA polymerase III Type L α Subunit of DNA Polymerase III Type II, ∈, β, DnaX and SSB.

Native and tagged *Thermotoga maritima* α both type I and type II, TM 0461 and TM 0576, respectively), ∈ (TM0496), β (TM0262), DnaX (TM 0686) and SSB (TM 0604) will be expressed and purified using the general strategies described in the preceding examples and in references (Kim, D.R. and McHenry, C.S., ibld); Dallmann, H.G. and McHenry, C.S., J Biol Chem 270:29563-29569 (1995); Dallmann, H. G., et al., JBiol Chem 270:29555-29562 (1995); PCT WO99/13060; PCT WO9953074). Both a subunits (from type I and II DNA polymerase IIIs) will be purified using a gap-filling assay on nuclease-activated DNA to monitor its presence, yield and specific activity. ∈ will be purified using its intrinsic nuclease activity in the presence and absence of αfrom type I DNA polymerase III) to monitor its purification (Maki and Komberg (1987) *ibid.;* Griep, M., *et al.,* (1990) *ibid.;* Reems, J., *et al., ibid.).* β and DnaX will initially be purified as tagged proteins and once a reconstitution assay is developed, they will be purified in native form. SSB will be expressed in native form and purified by established procedures (general SSB refs).

### Example 24. Development of Reconstitution Assays for Subunits of the Thermotoga maritima DNA Polymerase III Holoenzyme

Reconstitution assays for components of the *Thermotoga maritima* replicase will be developed using the general procedures and strategies described for *Aquifex aeolicus* in Example 16 except all tests will be done with either the type I a subunit (plus ∈ when convenient) or type II a or both a subunits in combination. Experiments will also be performed with the annotated *Thermotoga maritima* DnaX (TM0686) or putative second DnaX (TM 0508) alone or in combination.

### Example 25. Tests of Interactions of Thermotoga maritima δ and δ' Subunits

Experiments to test the identity and function of purified *Thermotoga maritima δ* and δ' will be as described in Example 17 except for those experiment involving DnaX, either TM0686 or the second putative DnaX (TM 0508) will be included alone or in combination.

### Example 26. Reconstitution of Thermotoga maritima DNA Polymerase III Holoenzyme

The *Thermotoga maritima* DNA polymerase III holoenzyme will be reconstituted using the components identified in the preceding examples as being necessary or stimulatory for the purposed described under Example 18 for the *Aquifex aeolicus* DNA polymerase III holoenzyme.

### Example 27. Extension of the Procedures Reported in Examples 1-26 to the Detection of Subunits of the DNA Polymerase III Holoenzyme From all other Bacteria

The general procedures described in the introduction and the preceding examples should be generally applicable to any bacteria that is sequenced. Particularly, our procedures for finding the δ component, δ' components and additional DnaX-like components should permit these components to be identified and expressed. These components, together with the more easily-identified α, ε β and primary DnaX proteins should reconstitute DNA polymerase III holoenzyme for use in synthesis of DNA as described under Example 18 plus any additional applications that involves synthesis of DNA or discovering inhibitors of that process.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifiations of the described modes for carrying out the invention which are obvious to those skilled in the relevant arts are intended to be within the scope of the following claims.

### Example 28. Expression of DNA Polymerase III Holoenzyme Subunits from Bacteria from Any Phyla, Purification,4 Reconstitution of Holoenzyme, and Test of Function

The subunits of DNA polymerase III holoenzyme identified in this application or holoenzyme subunits identified from bacteria sequenced in the future using approaches described in this application will be expressed, purified and used to reconstitute active DNA polymerase III holoenzyme by methods and using assays described for *Thermotoga maritima* and *Aquifex aeolicus* in this application and for *Yersinia pestis, Pseudomonas aeruginosa, Streptococcus pyogenes, Thermus thermophilus* and other organisms in the referenced patents and patent applications. The authenticity of the identified δ subunits in this application can be further verified by their requirment to reconstitute DNA polymerase III holoenzyme activity and/or by their interaction with β or δ' subunits from the cognate organism or other bacteria. DNA polymerase III holoenzyme reconstitutions assays can take place using subunits from the cognate organism or different organisms.

**Table 3. DnaX proteins identified from PSI-BLAST search of completely sequenced genomes**

| **Organism** | **Accession number** | **GenBank gi number** | **Database** |
|---|---|---|---|
| *Aquifex aeolicus* | A70460, AAC07663.1 | 7514776, 2984127 | NCBI +¹ |
| *Bacillus halodurans* | BAB03753.1 1 | 10172646 | NCBI + |
| *Bacillus subtilis* | S13786, BAA05255.1, CAA34877.1, CAB11795.1, P09122 | 98292, 467409, 580914,2632286, 118807 | NCBI+ |
| *Buchnera* | BAB13178.1 | 10039144 | NCBI+ |
| *Borrelia burgdorferi* | D70157, AAC66831.1 | 7463153, 2688379 | NCBI + |
| *Campylobacter jejuni* | G81320, CAB73411.1 | 11346858, 6968590 | NCBI + |
| *Caulobacter crescentus* | AAK22254.1, AAB61695.1 | 13421402, 1293572 | NCBI + |
| *Chlamydia muridarum* | D81682, AAF39442.1 | 11360902, 7190650 | NCBI+ |
| *Chlamydia trachomatis* | A71528, AAC67929.1 | 7468911, 3328753 | NCBI + |
| *Chlamydophila pneumonia* | C72127, AAD18193.1, AAF38540.1, BAA98252.1 | 7468224, 4376294, 7189650, 8978415 | NCBI + |
| *Deinococcus radiodurans* | C75278, AAFI1953.1 | 7471826,6460225 | NCBI + |
| *Haemophilus influenzae* | P43746, F6411, AAC22882.1 | 1169397,1075209, 1574159 | NCBI + |
| *Helicobacter pylori J99* | A71906, AAD06231.1 | 7464040, 4155207 | NCBI + |
| *Helicobacter pylori 26695* | E64609, AAD07767.1 | 7464043, 2313841 | NCBI + |
| *Lactococcus lactis* | AAK06292.1 | 12725258 | NCBI + |
| *Mesorhizobium loti* | NP_106158.1 | 13474589 | NCBI + |
| *Mycobacterium leprae* | CAA19155.1, CAC31851.1 | 3150103,13093951 | NCBI + |
| *Mycobacterium tuberculosis* | 069688, B70796, CAA18043.1 | 6014997, 7477980, 2960145 | NCBI + |
| *Mycoplasma genitalium* | NP_073091.1, P47659, D64246, AAC71649.1 | 12045280, 1352305,1361493, 3845015 | NCBI + |
| *Mycoplasma pneumoniae* | NP_110307.1, P75177, 573550, AAB95872.1 | 13508357, 2494198, 2146109, 1673890 | NCBI + |
| *Neisseria meningitidis* Z2491 | D81860, CAB84884.1, | 11261620,7380298 | NCBI + |
| *Pasteurella multocida* | AAK02448 | 12720609 | NCBI + |
| *Pseudomonas aeruginosa* | A83455, AE004581 | 11348451, 9947490 | NCBI+ |
| *Rickettsia prowazekii* | A71649, CAA15289.1 | 7467603, 3861390 | NCBI+ |
| *Synechocystis* | S77213, BAA17547.1 | 7469304,1652627 | NCBI +² |
| *Thermotoga maritima* | D72344, AAD35768.1 | 7462368, 4981209 | NCBI+ |
| *Treponema pallidum* | G71254, AAC65953.1 | 7521020, 3323329 | NCBI + |
| *Ureaplasma urealyticum* | NP_077917.1, F82935, AAF30492.1 | 13357643, 11356842,6899039 | NCBI + |
| *Vibrio cholerae* | H82246, AAF94213.1 | 11261617, 9655520 | NCBI+ |
| *Xylella fastidiosa* | B82635, AAF84614.1 | 11261615, 9106885 | NCBI + |

| | | | |
|---|---|---|---|
| ¹ A plus symbol next to the database name indicates that the protein is annotated as a DnaX or tau/gamma protein. A minus symbol next to the database indicates that the protein is not annotated. ² The mature form of DnaX from *Synechocystis* is 685 amino acids in length, but the native protein before the intein is removed is 1116 amino acids in length. The entries in NCBI indicate the longer unprocessed form of *Synechocystis* DnaX. | | | |

**Table 4. δ' proteins identified in PSI-BLAST searches of all finished genomes at NCBI**

| **Organism** | **Original accession number³** | **GenBank gi number⁴** | **Database** |
|---|---|---|---|
| *Aquifex aeolicus* | D70432 | 7514459 | NCBI⁵-⁶ |
| *Bacillus halodurans* | BAB03763 | 10172656 | NCBI + |
| *Bacillus subtilis* | S66061 | 2126931 | NCBI + |
| *Buchnera* | P57435 | 11132951 | NCBI + |
| *Borrelia burgdorferi* | AAC67118 | 2688709 | NCBI - |
| *Campylobacter jejuni* | CAB75220 | 6968051 | NCBI - |
| *Caulobacter crescentus* | AAK23798 | 13423258 | NCBI + |
| *Chlamydia muridarum* | F81700 | 11360903 | NCBI - |
| *Chlamydia trachomatis* | D71546 | 7468912 | NCBI - |
| *Chlamydophila pneumonia* | E72098 | 7468225 | NCBI - |
| *Deinococcus radiodurans* | F75287 | 7473469 | NCBI - |
| *Haemophilus influenzae* | P43748 | 2506369 | NCBI + |
| *Helicobacter pylori J99* | G64673 | 7464041 | NCBI + |
| *Helicobacter pylori 26695* | AAD06725 | 4155746 | NCBI + |
| *Lactococcus lactis* | AAK04497 | 12723272 | NCBI + |
| *Mesorhizobium loti* | NP_102222 | 13470653 | NCBI + |
| *Mycobacterium leprae* | CAA18816 | 3097239 | NCBI - |
| *Mycobacterium tuberculosis* | E70563 | 7477702 | NCBI - |
| *Mycoplasma genitalium* | NP_072667 | 12044857 | NCBI - |
| *Mycoplasma pneumoniae* | P75105 | 1673807 | NCBI + |
| *Neisseria meningitidis Z2491* | C81945 | 11353892 | NCBI + |
| *Pasteurella multocida* | AAK0375.8 | 12722081 | NCBI + |
| *Pseudomonas aeruginosa* | D83275 | 11348448 | NCBI + |
| *Rickettsia prowazekii* | H71727 | 7467604 | NCBI + |
| *Synechocystis* | S76227 | 7469488 | NCBI - |
| *Thermotoga maritima* | A72337 | 7462369 | NCBI - |
| *Treponema pallidum* | AAC65508 | 3322812 | NCBI - |
| *Ureaplasma urealyticum* | G82944 | 11356841 | NCBI +⁷ |
| *Vibrio cholerae* | H82127 | 11280161 | NCBI + |
| *Xylella fastidiosa* | C82777 | 11360901 | NCBI + |

| | | | |
|---|---|---|---|
| ³ An accession number applies to the complete record and is usually a combination of letter(s) and numbers. Accession numbers do not change, even if information in the record is changed at the author's request Records from different databases of reference sequences have different accession number formats. ⁴ GenInfo Identifier (GI) sequence identification numbers are assigned consecutively by NCBI to each sequence it processes for nucleotide and amino acid sequences. If a sequence changes in any way, a new GI number will be assigned. A separate GI number is also assigned to each protein translation within a nucleotide sequence record, and a new GI is assigned if the protein translation changes in any way. Unlike accession numbers, GI numbers allow all sequences to be maintained and therefore appears to be a better recording method. ⁵ GenBank is the NIH genetic sequence database, an annotated collection of all publicly available DNA sequences. GenBank (at NCBI), together with the DNA DataBank of Japan (DDBJ) and the European Molecular Biology Laboratory (EMBL) comprise the International Nucleotide Sequence Database Collaboration. These three organizations exchange data on a daily basis. ⁶ A plus symbol next to the database name indicates that the protein is annotated as a δ' or probable δ' protein. A minus symbol next to the database indicates that the protein is not annotated as a δ' protein or is annotated as a tau/gama like protein. The nucleotide and amino acid sequences of proteins with a minus symbol are shown below the table. ⁷ *Ureaplasma urealyticum* and *Xylella fastidiosa* are annotated as δ instead of δ'. | | | |

**Table 5. DnaX Proteins from S. pyogenes, S. pneumoniae and S. aureus**

| **Organism** | **Accession number/gene location** | **GenBank gi number/reading frame** | **Database** |
|---|---|---|---|
| *Streptococcus pyogenes⁸* | AAF98348 contig1, position 1130862-1132529 at Univ. of Okla. | 9789547 | NCBI+ |
| *Streptococcus pneumoniae* | contig 3836, position 705181-706833 | +1 | TIGR - |
| *Staphylococcus aureus* | BAB41666 | 13700368 | NCBI + |
| ⁸ The protein sequenc of *Streptococcus pyogenes* DnaX protein was initially submitted to GenBank by Bruck,I. and O'Donnell,M. and Rockefeller University/HHMI, 1230 York Ave, New York, NY 10021, USA an June 21,2000 and published August 10,2000. | | | |

**Table 6. δ' Proteins from S. pyogenes, S. pneumoniae and S. aureus**

| **Organism** | **Accession number/gene location** | **GenBank gi number/reading frame** | **Database** |
|---|---|---|---|
| *Streptococcus pyogenes⁹* | AAF98347 | 11992993 | NCBI + |
| *Streptococcus pneumoniae* | contig 3836, position 781821-782675 | +3 | TIGR - |
| *Staphylococcus aureus* | BAB41672 | 13700374 | NCBI + |

| | | | |
|---|---|---|---|
| ⁹ The protein sequenc *of Streptococcus pyogenes* δ' protein was initially submitted to GenBank by Bruck,I. and O'Donnell,M. and Rockefeller University/HHMI, 1230 York Ave, New York, NY 10021, USA on June 21,2000 and published August 10, 2000. This was the wrong sequence. The incorrect sequence was replaced by the correct sequence on December 27, 2000. The corrected sequence did not contain the C-terminal Glu residue. However, in the PCT #WO 01/09164 A2 the correct sequence is shown in its entirety. Another *Streptococcus pyogenes* δ' protein from the *Streptococcus pyogenes* M1 GAS strain was submitted to GenBank on April 10, 2001 and published on June 1, 2001 by *Streptoccus pyogenes* Genome Sequencing Project at the University of Oklahoma. | | | |

**Table 7. δ proteins identified in PSI-BLAST searches of unfinished bacterial genomes at NCBI**

| **Organism** | **Accession number** | **GenBank gi number** | **Database** |
|---|---|---|---|
| *Mycoplasma pulmonis* | CAC13625 | 14089876 | NCBI |
| *Streptococcus agalactiae* | CAA72898 | 2765186 | NCBI |
| *Streptomyces coelicolor* | CAB66242 | 6714670 | NCBI |

**Table 8. DnaX proteins corresponding to δ proteins identified from PSI-BLAST search of unfinished genomes at NCBI**

| **Organism** | **Accession number** | **GenBank gi number** | **Database** |
|---|---|---|---|
| *Mycoplasma pulmonis* | CAC13222 | 14089462 | NCBI+ |
| *Streptococcus agalactiae* | Not found | | NCBI |
| *Streptomyces coelicolor* | CAB56347 | 5918469 | NCBI + |

**Table 9. δ' Proteins corresponding to δ proteins identified from PSI-BLAST search of unfinished genomes at NCBI**

| **Organism** | **Accession number** | **GenBank gi number** | **Database** |
|---|---|---|---|
| *Mycoplasma pulmonas* | CAC13226 | 14089466 | NCBI - |
| *Streptococcus agalactiae* | Not found | | NCBI |
| *Streptomyces coelicolor* | T36661 | 7480627 | NCBI + |

**Table 10. DnaX proteins corresponding to δ proteins identified in BLAST searches of phylogenically related organisms**

| **Organism** | **Gene Location or Protein** | **Reading Frame** | **Database** |
|---|---|---|---|
| *Actinobacillus actinomycetemcomitans* | Contig 106, positions 9219-11261 | +3 | Univ. of Oklahoma - |
| *Bacillus anthracis* | Contig 4047, positions 9897-11585 | +3 | TIGR |
| *Bordatella pertussis* | Genomic positions 1938958-1940010 | +1 | Sanger - |
| *Burkholderia pseudomallei* | Genomic positions 113078-114112 | +2 | Sanger - |
| *Chlorobium tepidum* | contig 3499, position 798393-800252 | -2 | TIGR - |
| *Chloroflexus aurantiacus* | Contig 1016, positions 682-2133 | -3 | DOE-Joint Genome |
| | | | Institute - |
| *Clostridum difficile* | Contig 907 positions 3472-4407 | +1 | Sanger- |
| *Corynebacterium diphtheriae* | Genomic positions 221399-223522 | +2 | Sanger - |
| *Cytophaga hutchinsonii* | Contig 123, positions 1345-3077 | -3 | DOE-Joint Genome |
| *Dehalococcoides ethenogenes* | Contig 6145, positions 43320-44988 | +3 | Institute TIGR |
| *Prochlorococcus marinus* | Contig 26, positions 834903-836642 | -1 | DOE-Joint Genome Institute |
| *Porphyramonas gingivalis* | Genomic positions 1503207-1505012 | TIGR locus PG1418 | TIGR |
| *Salmonella typhi* | genomic position 533757-535685 | +3 | Sanger - |
| *Yerisenia pestis* | genomic position 3478956-3480929 | -1 | Sanger- |

**Table 11. δ' proteins corresponding to δ proteins identified in BLAST searches of phylogenically related organisms**

| **Organism** | **Gene Location or Protein** | **Reading Frame** | **Database** |
|---|---|---|---|
| *Actinobacillus actinomycetemcomitans* | Contig 73, positions 16499-17482 | +2 | Univ. of Oklahoma - |
| *Bacillus anthracis* | Contig 3937, positions 5108-6091 | +2 | TIGR |
| *Bordatella pertussis* | Genomic positions 1938958-1940010 | +1 | Sanger - |
| *Burkholderia pseudomallei* | Genomic positions 113078-114112 | +2 | Sanger - |
| *Chlorobium tepidum* | Contig 3499, positions 1065254-1066426 | +2 | TIGR |
| *Chloroflexus aurantiacus* | Contig 1088, positions 13027-14049 | -1 | DOE-Joint Genome |
| *Clostridum difficile* | Contig 907 positions 3472-4407 | +1 | Institute Sanger- |
| *Corynebacterium diphtheriae* | Genomic positions 304135-305310 | +1 | Sanger- |
| *Cytophaga hutchinsonii* | Contig 154, positions 10459-11586 | +1 | DOE-Joint Genome |
| *Dehalococcoides ethenogenes* | Contig 6152, positions 30780-31799 | +2 | Institute TIGR |
| *Prochlorococcus marinus* | Contig 26, positions 641321-642280 | +2 | DOE-Joint Genome |
| *Porphyromonas gingivalis* | Genomic positions 988645-989892 | TIGR locus | Institute TIGR |
| *Salmonella typhi* | Genomic positions 1193926-1194930 | PG0932 +1 | Sanger |
| *Yersinia pestis* | Genomic position 1829514-1830536 | +3 | Sanger |

**Table 12. DnaE and Pol C Proteins corresponding to δ proteins identified in PSI-BLAST searches of finished genomes at NCBI**

| **Organism** | **Protein** | **Accession number** | **GenBank gi number** | **Database** |
|---|---|---|---|---|
| *Aquifex aeolicus* | DnaE | 067125 | 6014991 | NCBI-¹⁰ |
| *Bacillus halodurans* | DnaE | Q9K838 | 14194680 | NCBI + |
| *Bacillus subtilis* | DnaE | 034623 | 6166145 | NCBI + |
| *Buchnera* | DnaE | P57332 | 11132275 | NCBI + |
| *Borrelia burgdorferi* | DnaE | B70172 | 7434830 | NCBI - |
| *Campylobacterjejuni* | DnaE | Q9PPI9 | 14194692 | NCBI - |
| *Caulobacter crescentus* | DnaE | AAK23901 | 13423381 | NCBI + |
| *Chlamydia muridarum* | DnaE | Q9PJJ7 | 14194688 | NCBI - |
| *Chlamydia trachomatis* | DnaE | 084549 | 14194664 | NCBI - |
| *Chlamydophila pneumonia* | DnaE | Q9Z7N8 | 14194705 | NCBI - |
| *Detnococcus radiodurans* | DnaE | Q9RX08 | 14194695 | NCBI - |
| *Haemophilus influenzae* | DnaE | P43743 | 1169392 | NCBI + |
| *Helicobacter pylori J99* | DnaE | Q9ZJF9 | 11132624 | NCBI + |
| *Helicobacter pylori 26695* | DnaE | P56157 | 2494191 | NCBI + |
| *Lactococcus lactis* | DnaE | Q9CI70 | 14194667 | NCBI + |
| *Mesorhizobium loti* | DnaE | NP_102580 | 13471011 | NCBI + |
| *Mycobacterium leprae* | DnaE | Q9X7F0 | 14194699 | NCBI - |
| *Mycobacterium tuberculosis* | DnaE | Q10779 | 1706493 | NCBI - |
| *Mycoplasma genitalium* | DnaE | NP_072927 | 12045116 | NCBI- |
| *Mycoplasma pneumoniae* | DnaE | NP_110066 | 13508117 | NCBI + |
| *Neisseria meningitidis* | DnaE | Q9JXZ2 | 14194679 | NCBI + |
| *MC58* | | | | |
| *Neisseria meningitidis* | DnaE | Q9JVX8, | 14194676, | NCBI + |
| *Z2491* | | Q9JVX8 | 14194676 | |
| *Pasteurella multocida* | DnaE | Q9CPK3 | 14194670 | NCBI + |
| *Pseudomonas aeruginosa* | DnaE | Q9HXZ1 | 14194674 | NCBI + |
| *Rickettsia prowazekii* | DnaE | 005974 | 6226600 | NCBI + . |
| *Synechocystis* | DnaE | A59016 | 7434836 | NCBI - |
| *Thermotoga maritima* | DnaE | Q9ZHG4 | 7227889 | NCBI - |
| *Treponema pallidum* | DnaE | 083675 | 6014994 | NCBI- |
| *Ureaplasma urealyticum* | DnaE | NP_078251 | 13357977 | NCBI + |
| *Vibrio cholerae* | DnaE | G82100, P52022 | 11261593, 14195659 | NCBI + |
| *Xylella fastidiosa* | DnaE | C82834, Q9PGU4 | 11261591, 14194684 | NCBI + |
| *Bacillus subtilis* | PolC | AAA22666 | 143342 | NCBI- |
| *Bacillus halodurans* | PolC | Q9KA,72 | 13959348 | NCBI + |
| *Lactococcus lactis* | PolC | AAK06222 | 12725180 | NCBI + |
| *Mycoplasma genitalium* | PolC | NP_072691 | 12044881 | NCBI + |
| *Mycoplasma pneumoniae* | PolC | NP_109722 | 13507773 | NCBI - |
| *Thermotoga maritima* | PolC | Q9ZHF6 | 6225287 | NCBI - |
| *Ureaplasma urealyticum* | PolC | NP 07821 | 13357937 | NCBI + |

| | | | | |
|---|---|---|---|---|
| ¹⁰ A plus symbol next to the database name indicates that the protein is annotated asa DnaE/PolC protein. A minus symbol next to the database indicates that the protein is not annotated | | | | |

**Table 13. DnaE and PoIC proteins from S. pyogenes, S. pneumoniae and S. aureus**

| **Organism** | **protein** | **Accession number/gene location** | **GenBank gi number/reading frame** | **Datab ase** |
|---|---|---|---|---|
| *Staphylococcus aureus* | DnaE | BAB42792 | 13701498 | NCBI +¹¹ |
| *Streptococcus pyogenes* | DnaE | AAF98354 | 9789551 | NCBI+ |
| *Streptococcus pneumoniae* | DnaE | Contig 3836, positions 740775-743876 | +3 | TIGR- |
| *Staphylococcus aureus* | PolC | Q53665 | 13959683 | NCBI + |
| *Streptococcus pyogenes* | PolC | AAF98345 | 9789541 | NCBI + |
| *Streptococcus pneumoniae* | PolC | Contig 3836, positions 144313-148704 | +1 | TIGR - |

| | | | | |
|---|---|---|---|---|
| ¹¹ A plus symbol next to the database name indicates that the protein is annotated as a DnaE/PoIC protein. A minus symbol next to the database indicates that the protein is not annotated | | | | |

**Table 14. DnaE and Pol C Proteins Corresponding to Additional δ Proteins Identified from PSI-BLAST search of Partially Sequenced Genomes**

| **Organism** | **protein** | **Accession number** | **GenBank gi number** | **Database** |
|---|---|---|---|---|
| *Mycoplasma pulmonis* | Dna E | CAC13892 | 14090134 | NGBI +¹² |
| *Streptococcus agalactiae* | Dna E | Not found | | |
| *Streptomyces coelicolor* | Dna E | Q53665, CAC36747 | 14194704, 13620707 | NCBI+ |
| *Mycoplasma pulmonis* | PolC | CAC13848 | 14090090 | NCBI + |
| *Streptococcus agalactiae* | PoIC | Not found | | |
| *Streptomyces coelicolor* | Po1C | Not found | | |

| | | | | |
|---|---|---|---|---|
| ¹² A plus symbol next to the database name indicates that the protein is annotated as a DnaE/PolC protein. A minus symbol next to the database indicates that the protein is not annotated | | | | |

**Table 15. DnaE and PolC proteins corresponding to δ proteins identified in BLAST searches of phylogenically related organisms**

| **Organism** | *Protein* | *Gene Location or Protein* | **Reading Frame** | *Database* |
|---|---|---|---|---|
| *Actinobacillus actinomycetemcomitans* | DnaE | Contig 264, position 9232-12705 | -1 | Univ. of Oklahoma -¹³ |
| *Bacillus anthracis* | DnaE | Contig 3830, positions 2 46303-49629 | | TIGR |
| *Bordatella pertussis* | DnaE | Genomic position 2465147-2468635 | -3 | Sanger- |
| *Burkholderia pseudomallei* | DnaE | Contig 181 positions 61605-65129 | -1 | Sanger- |
| *Chlorobium tepidum* | DnaE | Contig 3499, positions 354713-358282 | +1 | TIGR - |
| *Chloroflexus aurantiacus* | DnaE | Partial sequence corresponding to different regions of DnaE found in Contigs 918, 349, 261, 1137 | | DOE-Joint Genome Institute - |
| *Clostridum difficile* | DnaE | Contig 4 positions 65634-69203 | -3 | Sanger- |
| *Corynebaderium diphtheriae* | DnaE | Genomic positions 1609896-1606336 | -1 | Sanger - |
| *Cytophaga hutchinsonii* | DnaE | Contig 152 positions 55726-59314; Gene 48 | -1 | DOE-Joint Genome Institute + |
| *Dehalococcoides ethenogenes* | DnaE | Contig 6150, positions 75823-79336 | +1 | TIGR - |
| *Prochlorococcus marinus* | DnaE | Contig 24, positions 147546-151043; gene105 | +3 | DOE-Joint Genome Institute + |
| *Porphyromonas gingivalis* | DnaE | TIGR gene PGO035 | | TIGR + |
| *Salmonella typhi* | DnaE | Genomic positions 266430-269909 | +3 | Sanger - |
| *Yersinia pestis* | DnaE | Genomic positions 1200879-1204361 | +1 | Sanger - |
| *Bacillus anthracis* | PolC | Contig 3835, positions 27724-32019 | -1 | TIGR |
| *Clostridum difficile* | PolC | Contig 792, positions 15028-19326 | +2 | Sanger - |

| | | | | |
|---|---|---|---|---|
| ¹³ A plus symbol next to the database name indicates that the protein is annotated as a DnaE/PolC protein. A minus symbol next to the database indicates that the protein is not annotated. | | | | |

**Table 16. β Proteins corresponding to δ identified from PSI-BLAST search of completely sequenced genomes**

| **Organism** | **Accession number** | **GenBank gi number** | **Database** |
|---|---|---|---|
| *Aquifex aeolicus* | 067725, C70462, AAC07688.1 | 3913514, 7434840, 2984154 | NCBI +¹⁴ |
| *Bacillus halodurans* | Q9RCA1, BAA82686.1, BAB03721.1 | 13959350, 5672648, 10172614 | NCBI + |
| *Bacillus subtilis* | P05649, B22930, CAA26218.1, BAA05238.1, CAB11778.1 | 118797, 80346, 40015,467392, 2632269 | NCBI + |
| *Buchnera* | P57127, BAB12739.1, A.AG33956.1, P29439, JC1159, AAA73150.1, AAC38107.1, AAG33955.1, AAK21713.1, AAK21711.1,AAK21716.1, AAG33944.1, AAK21701.1, AAK21702.1, AAK21703.1, AAK21704.1, AAK21705.1, AAK21706.1, AAK21707.1, AAK21709.1, AAK21710.1, AAK21712.1, AAK21714.1, AAK21715.1, AAK21717.1, AAK21718.1, AAK21720.1, AAK21721.1, AAK21719.1, AAG33948.1, AAG33945.1, AAG33947.1, AAG33949.1, AAG33943.1, AAG33951.1, AAG33954.1, AAG33942.1, AAF19207.1, AAF19206.1, AAF19208.1, AAG33950.1, AAG33953.1, AAG33952.1, AAG33946.1, AAF19209.1 | 11132241,10038704, 11320921,232008, 94507, 144150, 2827015,11320919, 13398088, 13398084, 1339809513398078, 11320897, 13398063, 13398065, 13398067, 13398069, 13398072, 13398074, 13398076, 13398080, 13398082, 13398086,13398090, 13398093,13398097, 13398099,13398103, 13398105, 13398101, 11320905,11320899, 11320903, 11320907, 11320895,11320911, 11320917,11320893. 6606556,6606554, 6606558,11320909, 11320915,11320913, 11320901,6606560 | NCBI+ |
| *Borrelia burgdorferi* | E70154, AAB91514.1, P33761, S34948, AAA58942.1 | 7463155, 2688357, 461953, 479619, 454040 | NCBI + |
| *Campylobacterjejuni* | E81415, CAB72495.1 | 11346582,6967507 | NCBI + |
| *Caulobacter crescentus* | P48198, AAB51448.1, AAK22143.1 | 1352300, 1049324, 13421269 | NCBI + |
| *Chlamydia muridarum* | Q9PKW4, C81713, AAF39208.1 | 14194b91, 11261632, 7190388 | NCBI + |
| *Chlamydia trachomatis* | O84078, E71559, AAC67666.1 | 14194661, 7468908, 3328470 | NCBI + |
| *Chlamydophila pneumonia* | Q9Z8K0, BAA98549.1, H72090, AAD18487.1, F81578, AAF38262.1 | 14194707, 8978712, 7434845, 4376617, 11261630,7189343 | NCBI + |
| *Deinococcus radiodurans* | D75571, AAF09595.1 | 7471825, 6457660' | NCBI + |
| *Haemophilus influenzae* | P43744, A64107, AAC22654.1 | 1169393, 1074026, 1574022 | NCBI + |
| *Helicobacter pylori J99* | Q9ZLX4, A71931, AAD06030.1 | 11132657, 7464044, 4154988 | NCBI + |
| *Helicobacterpylori 26695* | 025242, D64582, AAD07565.1 | 3913505, 7464039, 2313610 | NCBI+ |
| *Lactococcus lactis* | AAK04100, 054376, T30306, AAC12964.1 | 12722837,3913511, 7474141,2909714 | NCBI + |
| *Mesorhizobium loti* | NP_106216, BAB52002.1 | 13474647,14025402 | NCBI + |
| *Mycobacterium leprae* | P46387, T10002, AAB53142.1, CAA94709.1, CAC29510.1 | 1169395,7434842, 886326, 1262353, 13092414 | NCBI + |
| *Mycobacterium tuberculosis* | Q50790, F70850, CAA16239.1, AAK44225.1, AAGO0841.1 | 3913519, 7434846, 3261513, 13879043, 9845532 | NCBI + |
| *Mycoplasma genitalium* | NP_072661.1, P47247, AAC71217.1 | 12044851, 6226601, 3844620 | NCBI + |
| *Mycoplasma pneumoniae* | NP_109689, Q50313, S62836, S73479, AAG34739.1 | 13507740, 2494195, 2127610, 2146107, 11379480 | NCBI+ |
| *Neisseria meningitidis* MD58 | C81030, AAF42232.1 | 11353125, 7227158 | NCBI + |
| *Neisseria meningitidis* Z2491 | A81974, A81974, CAB83846.1 | 11353891, 11353891, 7379292 | NCBI + |
| *Pasteurella multocida* | AAK03244.1 | 12721508 | NCBI + |
| *Pseudomonas aeruginosa* | Q917C4, F83644, AAG03392.1 | 13959347,11348446, 9945820 | NCBI + |
| *Rickettsia prowazekii* | Q9ZDB3, B71700, CAA14876.1 | 6225282, 7434844, 3860976 | NCBI + |
| *Synechocystis* | P72856, S74720, BAA16871.1 | 2494196, 7469303, 1651945 | NCBI + |
| *Thermotoga maritima* | E72400, AAD35350.1 | 7434843, 4980758 | NCBI + |
| *Treponema pallidum* | 083048, A71378, AAC65000.1 | 4033379, 7434841, 3322257 | NCBI + |
| *Ureaplasma urealyticum* | NP_077909, C82937, AAF30484.1 | 13357635, 11356839, 6899030 | NCBI + |
| *Vibrio cholerae* | Q9KVX5, F82376, AAF93191.1 | 14547987,11354872, 9654404 | NCBI + |
| *Xylella fastidiosa* | H82859, AAF82815.1 | 11360900, 9104762 | NCBI + |

| | | | |
|---|---|---|---|
| ¹⁴ A plus symbol next to the database name indicates that the protein is annotated as β protein. A minus symbol next to the database indicates that the protein is not annotated. | | | |

**Table 17 β Proteins from S. pyogenes, S. pneumoniae and S. aureus**

| **Organism** | **Accession number/gene location** | **GenBankgi number/reading frame** | **Database** |
|---|---|---|---|
| Streptococcus *pyogenes* | AAK33147 | 13621327 | NCIBI +¹⁵ |
| *Streptococcus pneumoniae* | 006672 | 3913504 | NCBI + |
| *Staphylococcus aureus* | S54708 | 1084187 | NCBI + |

| | | | |
|---|---|---|---|
| ¹⁵ A plus symbol next to the database name indicates that the protein is annotated as β protein. A minus symbol next to the database indicates that the protein is not annotated. | | | |

**Table 18. β Proteins Corresponding to δ Proteins Identified in BLAST Searches of Phylogenically Related Organisms**

| **Organism** | ***Gene Location or Protein*** | **Reading Frame** | ***Database*** |
|---|---|---|---|
| *Actinobacillus actinomycetemcomitans* | Contig 213, position 10095-11192 | +3 | Univ. of Oklahoma -¹⁶ |
| *Bacillus anthracis* | Contig 3902, positions 32884-34024 | +1 | TIGR - |
| *Bordatella pertussis* | Contig 142 positions 503449-504558 | -1 | Sanger- |
| *Burkholderia pseudomallei* | Contig 298 positions 65824-66927 | -1 | Sanger- |
| *Chlorobium tepidum* | Contig 3499, positions 1708292-1707174 | -2 | TIGR- |
| *Chloroflexus aurantiacus* | Contig 1129, positions 766-1900 | +1 | DOE-Joint Genome Institute - |
| *Clostridum difficile* | Contig 871, positions 1457-2665 | +2 | Sanger - |
| *Corynebacterium diphtheriae* | Genomic positions 2316-3503 | +3 | Sanger - |
| *Cytophaga hutchinsonii* | Contig 132 positions 47216-48340; Gene 21 | -2 | DOE-Joint Genome Institute + |
| *Dehalococcoides ethenogenes* | Contig 6139, positions 20994-22129 | -1 | TIGR- |
| *Prochlorococcus marinus* | Contig 26, positions 767092-768248; gene 877 | -3 | DOE-Joint Genome Institute + |
| *Porphyromonas gingivalis* | Contig GPG.con, positions 1953200-1952090; TIGR gene PG1853 , | -1 | TIGR- |
| *Salmonella typhi* | Genomic positions 3806530-3807632 | +1 | Sanger - |
| *Yersinia pestis* | Genomic positions 4617236-4618336 | -2 | Sanger- |

| | | | |
|---|---|---|---|
| ¹⁶ A plus symbol next to the database name indicates that the protein is annotated as β protein. A minus symbol next to the database indicates that the protein is not annotated. | | | |

**Table 19**

| **conserved regions** | **17-38** | **64-B2** | **97-117** | **154-191** | **215-260** | **298-331** | **regions meeting criteria** |
|---|---|---|---|---|---|---|---|
| **number of conserved as per region** | **10** | **11** | **10** | **18** | **23** | **12** | |
| **Organism** | | | | | | | |
| *Aquifex aeolicus* | 7 | 5 | 7 | 15 | 16 | 7 | 6 |
| *Bacillus halodurans* | 7 | 7 | 8 | 12 | 15 | 10 | 6 |
| *Bacillus subtilis* | 9 | 6 | 7 | 10 | 16 | 11 | 6 |
| *Buchnera sp.* | 8 | 8 | 7 | 11 | 14 | 10 | 6 |
| *Borrelia burgdorferi* | 8 | 7 | 5 | 8 | 17 | 10 | 5 |
| *Campylobacter jejuni* | 5 | 8 | 5 | 13 | 11 | 7 | 6 |
| *Caulobacter crescentus* | 6 | 7 | 6 | 10 | 9 | 7 | 5 |
| *Chlamydophila pneumoniae* | 2 | 7 | 4 | 9 | 14 | 6 | 4 |
| *Chlamydia muridarum* | 2 | 5 | 5 | 10 | 13 | 6 | 5 |
| *Chlamydia trachomatis* | 4 | 7 | 4 | 9 | 15 | 6 | 4 |
| *Deinococcus radiodurans* | 4 | 7 | 5 | 16 | 15 | 11 | 5 |
| *Escherichia coli* | 9 | 9 | 9 | 15 | 22 | 10 | 6 |
| *Haemophilus influenzae* | 10 | 9 | 10 | 14 | 19 | 9 | 6 |
| *Helicobacter pylori 26695* | 5 | 7 | 4 | 9 | 12 | 3 | 4 |
| *Helicobacter pylori J99* | 5 | 7 | 4 | 9 | 11 | 3 | 4 |
| *Lactocaccus lactis* | 8 | 8 | 7 | 5 | 17 | 11 | 5 |
| *Mesorhizobium loti* | 7 | 7 | 8 | 8 | 12 | 7 | 5 |
| *Mycobacterium leprae* | 8 | 9 | 4 | 10 | 14 | 11 | 5 |
| *Mycobacterium tuberculosis* | 7 | 9 | 4 | 10 | 13 | 9 | 5 |
| *Mycoplasma genitalium* | 7 | 2 | 5 | 15 | 9 | 8 | 4 |
| *Mycoplasma pneumoniae* | 7 | 6 | 6 | 15 | 14 | 9 | 6 |
| *Neisseria meningitidis Z2491* | 9 | 9 | 7 | 16 | 17 | 9 | 6 |
| *Pasteurella multocida* | 8 | 8 | 10 | 14 | 20 | 8 | 6 |
| *Pseudomonas aeruginosa* | 9 | 10 | 9 | 14 | 20 | 7 | 6 |
| *Rickettsia* prowazekii | 6 | 7 | 6 | 11 | 10 | 5 | 4 |
| *Streptococcus pneumoniae* | 5 | 8 | 7 | 4 | 15 | 10 | 5 |
| *Streptococcus pyogenes* | 7 | 6 | 7 | 6 | 16 | 12 | 5 |
| *Staphylococcus aureus* | 5 | 3 | 3 | 12 | 16 | 5 | 3 |
| *Streptomyces coelicolor* | 8 | 10 | 6 | 7 | 17 | 12 | 5 |
| Synechocystis | 6 | 5 | 7 | 11 | 14 | 11 | 6 |
| *Thermotoga maritima* | 4 | 8 | 6 | 13 | 14 | 9 | 6 |
| Thermus *thermophilus* | 5 | 5 | 6 | 13 | 17 | 11 | 6 |
| *Treponema pallidum* | 5 | 8 | 5 | 10 | 18 | - | 6 |
| *Ureaplasma urealyticum* | 5 | 4 | 6 | 12 | 15 | 9 | 5 |
| *Vibrio cholerae* | 10 | 9 | 10 | 16 | 17 | 7 | 6 |
| *Xylella fastidiosa* | 8 | 9 | 6 | 13 | 15 | 8 | 6 |

**Table 20**

| **Organism** | **Sequencing Center** | **δ sequences producing hits^{a}** | **δ sequences producing no hits** |
|---|---|---|---|
| *Actinobacillus actinomycetemco mitans* | The University of Oklahoma Advanced Center for Genome Technology | *Escherichia coli*, *Neisseria meningitidis* | *Dehalococcoides ethenogenes, Campylobacter jejuni*, *Caulobacter crescentus, Ureaplasma urealyticum, Chlamydia trachomatis, Synechocystis, Escherichia coli* |
| *Bacillus anthracis* | TIGR, the Institute for Genomic Research | *Bacillus subtilis, Lactococcus lactis* | *Escherichia coli* |
| *Bordatella pertussis Burkholderia pseudomallei* | The Sanger Centre The Sanger Centre | *Neisseria meningitidis, Escherichia coli Neisseria meningitialis, Escherichia coli, Bordatella pertussis* | |
| *Chlorobium tepidum* | TIGR, the Institute for Genomic Research | *Cytophaga hutchinsonii, Porphyromonas gingivalis* | *Chlamydia muridarum, Chlamydia trachomatis, Chlamydophila pneumonia. Synechocystis, Ureaplasma urealyticum, Mycobacterium leprae, Escherichia coli* |
| *Chloroflexus anrantiacus* | DOE-Joint Genome Institute | *Dehalococcoides ethenogenes*, *Escherichia coli* | *Treponema pallidum, Helicobacter pylori* |
| *Clostridum difficile* | The Sanger Centre | *Lactococcus lactis, Streptococcus pyogenes* | *Escherichia coli* |
| *Corynebacterium diphtheriae* | The Sanger Centre | *Mycobacterium leprae, Streptomyces coelicolor* | *Escherichia coli* |
| *Cytophaga hutchinsonii Dehalococcoides* | DOE-Joint Genome Institute TIGR, the | *Neisseria meningitidis, Bordatella pertussis Thermus thermophilus,* | *Escherichia coli* |
| *ethenogenes* | Institute for Genomic Research | *Deinococcus radiodurans, Escherichia coli* | |
| *Prochlorococcus marinus* | DOE-Joint Genome Institute | *Synechocystis* | *Escherichia coli* |
| *Porphyromonas gingivalis* | TIGR, the Institute for Genomic Research | *Cytophaga hutchinsonii,* | *Escherichia coli, Neisseria meningitidis* |
| *Yersinia pestis* | The Sanger Centre | *Escherichia coli* | |
| *Salmonella thphi* | The Sanger Centre | *Escherichia coli* | |

| | | | |
|---|---|---|---|
| **^{a} A hit is defined as a sequence identified as delta using the criteria described in step 4 of the 'psi-blast' delta search** | | | |

**Table 21. β Proteins Corresponding to Additional δ Proteins Identified from PSI-BLAST Search of Partially Sequenced Genomes**

| **Organism** | **Accession number** | **GenBank gi number** | **Database** |
|---|---|---|---|
| *Mycoplasma pulmonis* | CAC13175 | 14089415 | NCBI+¹⁷ |
| *Streptococcus agalactiae* | Not found | | |
| *Streptomyces coelicolor.* | P27903 | 118804 | NCBI + |

| | | | |
|---|---|---|---|
| ¹⁷ A plus symbol next to the database name indicates that the protein is annotated as β protein. A minus symbol next to the database indicates that the protein is not annotated. | | | |

## Claims

**1.** A method of screening for a compound that modulates the activity of a DNA polymerase III replicase, said method comprising:
a) contacting an isolated replicase with at least one test compound under conditions permissive for replicase activity;
b) assessing the activity of the replicase in the presence of the test compound; and
c) comparing the activity of the replicase in the presence of the test compound with the activity of the replicase in the absence of the test compound, wherein a change in the activity of the replicase in the presence of the test compound is indicative of a compound that modulates the activity of the replicase,
wherein said replicase comprises an isolated DNA polymerase III δ subunit protein.

**2.** A method of claim 1, wherein the isolated DNA polymerase III δ subunit protein is an *Yersinia, Pseudomonas, Streptococcus, Aquifex, Bacillus, Buchnera, Borrelia, Campylobacter, Caulobacter, Chlamydia, Chlamydia, Chlamydophila, Deinococcus, Helicobacter, Lactococcus, Mycobacterium, Mesorhizobium, Mycoplasma,* Pasteurella, *Rickettsia, Synechocystis, Thermatoga, Treponema, Ureaplasma, Vibrio, Xylella, Mycoplasma, Staphylococcus, Streptomyces, Streptococcus, Streptococcus, Thermus, Actinobacillus, Bordetealla, Bacillus, Burkholderia, Chlorobium, Chloroflexus, Clostridium, Corynebacterium, Cytopahaga, Dehalococcoides, Porphyromonas, Prochlorococcus* or Salmonella DNA polymerase III δ subunit protein.

**3.** A method of claim 1, wherein the isolated DNA polymerase III δ subunit protein is an *Yersinia pestis, Pseudomonas aeruginosa, Streptococcus pyogenes, Aquifex aeolicus, Bacillus* halodurans, *Bacillus subtilis, Buchnera*, *Borrelia burgdorferi, Campylobacter jejuni,* Caulobacter *crescentus, Chlamydia muridarum, Chlamydia trachomatis, Chlamydophila pneumonia, Deinococcus radiodurans, Helicobacter pylori* 26695, *Helicobacter pylori* J99, *Lactococcus lactis, Mycobacterium laprae, Mesorhizobium loti, Mycoplasma genitalium, Mycoplasma pneumoniae, Pasteurella multocida, Pseudomonas aeruginosa, Rickettsia prowazekii*, *Synechocystis, Thermatoga maritima, Treponema pallidum, Ureaplasma urealyticum, Vibrio cholerae, Xylella fastidiosa, Mycoplasma pulmonis, Staphylococcus aureus, Streptococcus agalactiae, Streptomyces coelicolor, Streptococcus pneumoniae, Streptococcus pyogenes, Thermus thermophilus, Yersinia pestis, Actinobacillus actinomycetemcomitans, Bordetealla pertussis*, *Bacillus* anthracis, *Burkholderia pseudomallei, Chlorobium tepidum,* Chloroflexus aurantiacus, Clostridium *difficile, Corynebacterium* diphtheriae, *Cytopahaga hutchinsonii*, *Dehalococcoides ethenogenes, Porphyromonas gingivalis, Prochlorococcus marinus* or *Salmonella typhi* DNA polymerase III δ subunit protein.

**4.** A method of claim 1, wherein the isolated DNA polymerase III δ subunit protein is encoded by a nucleic acid molecule selected from the group of SEQ ID NO:109, SEQ ID NO:70 or SEQ ID NO:103.

**5.** A method for identifying a bacterial DNA polymerase holoenzyme δ subunit protein from at least one completely sequenced genome comprising:
a) providing a database of sequence information for a plurality of different organisms;
b) setting an inclusion threshold to a level sufficient to minimize the number of required iterations;
c) selecting the organisms to be searched wherein the selected organisms are candidate organisms;
d) providing the amino acid sequence of the δ subunit protein from a reference organism;
e) comparing the sequences of the candidate organisms and the reference organism;
f) excluding known DnaX proteins and known δ' proteins from further steps;
g) selecting proteins comprising between 300 and 400 amino acids;
h) selecting a delta candidate from a candidate organism comprising selecting sequence with the lowest E score from the candidate organism; and
i) optionally repeating steps e) - g) wherein the sequences of the candidate organisms are sequences selected in step h,
wherein a bacterial DNA polymerase holoenzyme δ subunit protein may be identified.

**6.** The method of Claim 5, wherein the inclusion threshold is 0.001.

**7.** The method of Claim 5, wherein the inclusion threshold is 0.002.

**8.** A bacterial DNA polymerase III holoenzyme δ subunit protein identified by the method of Claim 5.

**9.** A method for identifying a δ protein from at least one partially sequenced genome comprising identifying a δ protein according the method of Claim 5, wherein the selecting of step c) comprises selecting all organisms in the database for search which are not completely sequenced.

**10.** A method for identifying a bacterial DNA polymerase holoenzyme δ protein from at least one completely sequenced genome comprising:
a) providing a database of sequence information for a plurality of different organisms;
b) setting the inclusion threshold to a level sufficient to minimize the number of required iterations;
c) selecting the organisms to be searched, wherein the selected organisms are candidate organisms;
d) providing the amino acid sequence of the δ subunit protein from a reference organism;
e) comparing the sequences of the candidate organisms and the reference organism;
f) excluding known DnaX proteins and known δ' proteins from further steps;
g) selecting proteins comprising at least three of the following six characteristics:
*a*. having at least five out of ten conserved residues from the conserved region 17-(L/I/V)XX(L/I/V)Y(L/I/V)(L/I/V)XGX(D/E)XX (UI/V)(L/I/V)XXXXXX(L/I/V)-38;
*b*. having at least five out of eleven conserved residues from the conserved region 64-(L/I/V)(L/I/V)XXXX(A/S)XX(L/I/V)F(A/S)X (K7R)X(L/I/V)(L/I/V)(L/I/V)(L/I/V)-82;
*c*. having at least five out of ten conserved residues from the conserved region 97--(L/I/V)XX(L/I/V)(L/I/V))XXXXX(D/E)X(L/I/V) (L/I/V)(L/I/V)(L/I/V)XXXK(L/I/V)-117;
d having at least nine out of eighteen conserved residues from the conserved region 154-(R/K)XXX(L/I/V)X(L/I/V)X(L/I/V) (D/E)X(D/F-)(A/S)(L/I/V)XX(L/I/V)XXXXXXN(L/I/V)XX(L/I/V)XX(D/E)(L/I/V)X(R/ K)(L/I/V)X(L/I/V)(L/I/V)-191;
*e*. having at least eleven out of twenty-three conserved residues from the conserved region 215-FX(LXV)X(D/E)(A/S)(L/I/V)(L/I/V) XG(R/K)XXX(A/S)(L/I/V)X(L/I/V)(L/I/V)XX(L/I/V)XXXGX(D/E)P(L/I/V)X(L/I/V)(L /I/V)XX(L/I/V)XXX(L/I/V)XX(L/I/V)XX(L/I/V)-260; and
*f*. having at least six out of twelve conserved residues from the conserved region 298-(L/I/V)XXX(L/I/V)XX(L/I/V)XXX(D/E)XX (L/I/V)(R/K)XXXXX(D/E)XXXX(L/I/V)(D/E)XX(L/I/V)(L/I/V)X(L/I/V)-331 and
h) retaining the sequences above the threshold, wherein the retained sequences are delta candidate sequences; and
j) optionally repeating steps e) -g) wherein the sequences of the candidate organisms are delta candidate sequences,
whereby a δ protein may be identified.

**11.** The method of Claim B, wherein the plurality of different organisms comprises a plurality of evolutionarily distant organisms.

**12.** The method of Claim 10, wherein the inclusion threshold is 0.001.

**13.** The method of Claim 10, wherein the inclusion threshold is 0.002.

**14.** A bacterial DNA polymerase III holoenzyme δ subunit protein identified by the method of Claim 10.

**15.** A method for identifying a δ protein from at least one partially sequenced genome comprising identifying a δ protein according the method of Claim 10, wherein the selecting of step c) comprises selecting organisms in the database which are not completely sequenced.

**16.** A method for identifying a δ protein from at least one target organism comprising identifying a δ protein from an evolutionarily related organism, and performing a search for a similar sequence in the sequence of the target organism.

**17.** An isolated bacterial DNA polymerase δ subunit protein, wherein the δ subunit protein is not a member of the group consisting of an *E. coli δ* subunit protein, a *Haemophilis influenzae δ* subunit protein, and a *Neisseria meningitides δ* subunit protein.

**18.** An isolated bacterial DNA polymerase δ subunit protein, wherein the δ subunit protein is not a member of the group consisting of a gamma division of proteobacteria δ subunit protein and a beta division of proteobacteria δ subunit protein.

**19.** An isolated bacterial DNA polymerase δ subunit protein, wherein the δ subunit protein is a gram-positive bacteria δ subunit protein.

**20.** An isolated bacterial DNA polymerase δ subunit protein of Claim 19,
wherein the δ subunit protein is a member of the group consisting of an *S*. *pyogenes*, *δ* subunit protein, an *S. aureus δ* subunit protein, and an *S. pneumoniae δ* subunit protein.

**21.** An isolated bacterial DNA polymerase δ subunit protein, wherein the δ subunit protein is selected from the group consisting of *Bacillus subtilis*, *Aquifex aeolicus,* and *Thermatoga maritima δ* subunit protein.

**22.** The isolated bacterial DNA polymerase δ subunit protein of Claim 21, wherein the protein comprises an amino acid sequence selected from the group consisting of
a) an amino acid sequence selected from the group consisting of SEQ ID NO:3, SEQ ID NO:226, SEQ ID NO:6, SEQ ID NO:9, SEQ ID NO:12, SEQ ID NO:15, SEQ ID NO:18, SEQ ID NO:21, SEQ ID NO:24, SEQ ID NO:27, SEQ ID NO:30, SEQ ID NO:33, SEQ ID NO:36, SEQ ID NO:39, SEQ ID NO:42, SEQ ID NO:45, SEQ ID NO:48, SEQ ID NO:51, SEQ ID NO:227, SEQ ID NO:228, SEQ ID NO:54, SEQ ID NO:57, SEQ ID NO:60, SEQ ID NO:63, SEQ ID NO:66, SEQ ID NO:69, SEQ ID NO:72, SEQ ID NO:75, SEQ ID NO:78, SEQ ID NO:81, SEQ ID NO:84, SEQ ID NO:87, SEQ ID NO:90, SEQ ID NO:93, SEQ ID NO:96, SEQ ID NO:99, SEQ ID NO:102, SEQ ID NO:229, SEQ ID NO:105, SEQ ID NO: 108, SEQ ID NO:111, SEQ ID NO:114, SEQ ID NO:117, SEQ ID NO:120, SEQ ID NO: 123, SEQ ID NO: 126, SEQ ID NO: 129, SEQ ID NO: 132, SEQ ID NO: 135, SEQ ID NO:138, SEQ ID NO:141, SEQ ID NO:144, SEQ ID NO: 147, SEQ ID NO:150. SEQ ID NO: 153, and SEQ ID NO:156; and
b) an amino acid sequence selected from the group consisting of an amino acid sequence having at least 95% sequence identity to an amino acid sequence of a).

**23.** An antibody, wherein the antibody is capable of specifically binding to at least one antigenic determinant on the protein encoded by an amino acid sequence according to Claim 20.

**24.** The antibody of Claim 23 wherein the antibody type is selected from the group consisting of polyclonal, monoclonal, chimeric, single chain, F_{ab} fragments, and an F_{ab} expression library.

**25.** A method for producing anti-DNA polymerase III δ subunit antibodies comprising exposing an animal having immunocompetent cells to an immunogen comprising at least an antigentic portion of DNA polymerase III δ subunit.

**26.** The method of Claim 25, further comprising the step of harvesting the antibodies.

**27.** The method of Claim 25, further comprising fusing the immunocompetent cells with an immortal cell line under conditions such that a hybridoma is produced.

**28.** A method for detecting DNA polymerase III δ subunit protein comprising,
a) providing in any order, a sample suspected of containing DNA polymerase III, an antibody capable of specifically binding to at least a portion of the DNA polymerase III δ subunit protein;
b) mixing the sample and the antibody under conditions wherein the antibody can bind to the DNA polymerase III; and
c) detecting the binding.

**29.** An isolated nucleic acid molecule selected from the group consisting of:
a) an isolated bacterial nucleic acid molecule comprising a nucleic acid sequence encoding a protein selected from the group consisting of:
i) a protein having an amino acid sequence selected from the group consisting of SEQ ID NO:3, SEQ ID NO:226, SEQ ID NO:6, SEQ ID NO:9, SEQ ID NO:12, SEQ ID NO:15, SEQ ID NO:18, SEQ ID NO:21, SEQ ID NO:24, SEQ ID NO:27, SEQ ID NO:30, SEQ ID NO:33, SEQ ID NO:36, SEQ ID NO:39, SEQ ID NO:42, SEQ ID NO:45, SEQ ID NO:48, SEQ ID NO:51, SEQ ID NO:227, SEQ ID NO:228, SEQ ID NO:54, SEQ ID NO:57, SEQ ID NO:60, SEQ ID NO:63, SEQ ID NO:66, SEQ ID NO:69, SEQ ID NO:72, SEQ ID NO:75, SEQ ID NO:78, SEQ ID NO:81, SEQ ID NO:84, SEQ ID NO:87, SEQ ID NO:90, SEQ ID NO:93, SEQ ID NO:96, SEQ ID NO:99, SEQ ID NO:102, SEQ ID NO:229, SEQ ID NO:105, SEQ ID NO: 108, SEQ ID NO:111, SEQ ID NO:114, SEQ ID NO:117, SEQ ID NO:120, SEQ ID NO:123, SEQ ID NO:126, SEQ ID NO:129, SEQ ID NO: 132, SEQ ID NO: 135, SEQ ID NO:138, SEQ ID NO:141, SEQ ID NO:144, SEQ ID NO:147, SEQ ID NO:150, SEQ ID NO: 153, and SEQ ID NO:156; and
ii) a protein selected from the group consisting of a protein having at least 95% sequence identity to an amino acid sequence of i); and
b) an isolated bacterial nucleic acid molecule which is fully complementary to any said nucleic acid molecule recited in a).

**30.** The nucleic acid molecule of claim 29 wherein said nucleic acid molecule is selected from the group consisting of *Aquifex*, *Bacillus*, *Buchnera*, *Borrelia*, *Campylobacter, Caulobacter*; *Chlamydia*, *Chlamydia*, *Chlamydophila*, *Deinococcus*, *Haemphilus*, *Helicobacter*, *Lactococcus, Mycobacterium*, *Mesorhizobium*, *Mycoplasma*, *Neisseria*, *Pasteurella*, *Pseudomonas*, *Rickettsia*, *Synechocystis*, *Thermatoga, Treponema, Ureaplasma*, *Vibrio*, *Xylella, Mycoplasma*, *Staphylococcus*, *Streptococcus, Streptomyces, Streptococcus*, *Streptococcus, Thermus*, *Yersinia*, *Actinobacillus*, *Bordetealla, Bacillus, Burkholderia, Chlorobium, Chloroflexus, Clostridium, Corynebacterium, Cytopahaga, Dehalococcoides, Porphyromonas, Prochlorococcus,* and *Salmonella* nucleic acid molecules.

**31.** The nucleic acid molecule of claim 29, wherein said nucleic acid molecule is selected from the group consisting *of Aquifex aeolicus, Bacillus halodurans, Bacillus subtilis, Buchnera, Borrelia burgdorferi, Campylobacter jejuni, Caulobacter crescentus*, *Chlamydia muridarum, Chlamydia trachomatis, Chlamydophila pneumonia, Deinococcus radiodurans, Haempphilus influenzae, Helicobacter pylori* 26695, *Helicobacter pylori* J99, *Lactococcus lactis, Mycobacterium laprae, Mesorhizobium loti, Mycoplasma genitalium, Mycoplasma pneumoniae, Pasteurella multocida, Pseudomonas aeruginosa, Rickettsia prowazekii Synechocystis, Thermatoga maritima, Treponema pallidum, Ureaplasma urealyticum, Vibrio cholerae, Xylella fastidiosa, Mycoplasma pulmonis, Staphylococcus aureus, Streptococcus agalactiae, Streptomyces coelicolor, Streptococcus pneumoniae, Streptococcus pyogenes, Thermus thermophilus, Yersinia pestis, Actinobacillus actinomycetemcomitans, Bordetealla pertussis; Bacillus anthracis, Burkholderia pseudomallei, Chlorobium tepidum, Chloroflexus aurantiacus, Clostridium difficile, Corynebacterium diphtheriae, Cytopahaga hutchinsonii; Dehalococcoides ethenogenes, Porphyromonas gingivalis, Prochlorococcus marinus,* and *Salmonella typhi* nucleic acid molecules.

**32.** The nucleic acid molecule of claim 29, wherein said nucleic acid molecule comprises a nucleic acid molecule selected from the group consisting of SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO: 109, SEQ ID NO:112, SEQ ID NO:115, SEQ ID NO:118, SEQ ID NO:121, SEQ ID NO: 124, SEQ ID NO: 127, SEQ ID NO: 130, SEQ ID NO: 133, SEQ ID NO:136, SEQ ID NO: 139, SEQ ID NO: 142, SEQ ID NO: 145, SEQ ID NO: 148, SEQ ID NO:151, and SEQ ID NO:154.

**33.** A recombinant molecule comprising at least a portion of a bacterial DNA polymerase III *hol*A nucleic acid molecule according to Claim 29, 30, 31, or 32.

**34.** A recombinant cells comprising at least a portion of a bacterial DNA polymerase III *hol*A nucleic acid molecule according to Claim, 29, 30, 31 or 32.

**35.** A method for detection of nucleic acid molecules encoding at least a portion of DNA polymerase III δ subunit in a biological sample comprising the steps of:
a) hybridizing at least a portion of a *hol*A nucleic acid molecule to nucleic acid material of a biological sample, thereby forming a hybridization complex, and
b) detecting the hybridization complex, wherein the presence of the complex correlates with the presence of a polynucleotide encoding at least a portion of DNA polymerase III δ subunit in the biological sample.

**36.** The method of Claim 35, wherein the *hol*A nucleic acid molecule is a *hol*A nucleic acid molecule according to Claim 29, 30, 31, or 32.

**37.** A method for detecting DNA polymerase III δ subunit expression, including expression of modified or mutated DNA polymerase III δ subunit proteins or gene sequences comprising the steps of
a) providing a test sample suspected of containing DNA polymerase III δ subunit protein, as appropriate; and
b) comparing test DNA polymerase III δ subunit with quantitated DNA polymerase III holoenzyme or holoenzyme subunit in a control to determine the relative concentration of the test DNA polymerase III δ subunit in the sample.

**38.** A method of screening for a compound that modulates the activity of a DNA polymerase III replicase, said method comprising:
a) contacting an isolated replicase with at least one test compound under conditions permissive for replicase activity;
b) assessing the activity of the replicase in the presence of the test compound; and
c) comparing the activity of the replicase in the presence of the test compound with the activity of the replicase in the absence of the test compound, wherein a change in the activity of the replicase in the presence of the test compound is indicative of a compound that modulates the activity of the replicase,
wherein said replicase comprises an isolated DNA polymerase III δ subunit protein.

**39.** The method of claim 38, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO: 13, SEQ ID NO:16, SEQ ID NO: 19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID N0:58, SEQ m N0:61, SEQ m NO:64, SEQ ID NO:67, SEQ m NO:70, SEQ m NO:73, SEQ m NO:76, SEQ ID NO:79, SEQ m NO:82, SEQ m NO:85, SEQ m NO: 88, SEQ ID NO:91, SEQ ID NO:94, SEQ m NO:97, SEQ ID NO:100, SEQ m NO:103, SEQ ID N0:106, SEQ ID NO:109, SEQ ID NO:112, SEQ ID NO:115, SEQ ID NO:118, SEQ ID NO: 12 1, SEQ ID NO: 124, SEQ ID NO: 127, SEQ ID NO: 130, SEQ ID NO: 133, SEQ ID NO: 136, SEQ ID NO:139, SEQ ID NO:142, SEQ ID NO:145, SEQ ID NO: 148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**40.** A compound that modulates the activity of a DNA polymerase III replicase identified by the method of Claim 38 or 39.

**41.** A method of identifying compounds which modulate the activity of a DNA polymerase III replicase comprising
a) forming a reaction mixture that includes a primed DNA molecule, a DNA polymerase α subunit, a candidate compound, a dNTP, and optionally, a member of the group consisting of a β subunit, a τ complex, and both the β subunit and the τ complex to form a replicase;
b) subjecting the reaction mixture to conditions effective to achieve nucleic acid polymerization in the absence of the candidate compound: and
c) comparing the activity of the replicase in the presence of the test compound with the activity of the replicase in the absence of the test compound, wherein a change in the activity of the replicase in the presence of the test compound is indicative of a compound that modulates the activity of the replicase,
wherein said replicase comprises a DNA polymerase III δ subunit protein.

**42.** The method of claim 41, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO: 13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO: 100, SEQ ID NO:103, SEQ ID NO: 106, SEQ ID NO:109, SEQ ID NO: 112, SEQ ID NO:115, SEQ ID NO: 118, SEQ ID NO:121, SEQ ID NO: 124, SEQ ID NO: 127, SEQ ID NO: 130, SEQ ID NO:133, SEQ ID NO:136, SEQ ID NO: 139, SEQ ID NO: 142, SEQ ID NO: 145, SEQ ID NO:148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**43.** A compound that modulates the activity of a DNA polymerase III replicase identified by the method of Claim 41 or 42.

**44.** A method of identifying compounds that modulate the activity of a DnaX complex and a β subunit in stimulating a DNA polymerase replicase comprising
a) contacting a primed DNA (which may be coated with SSB) with a DNA polymerase replicase, a β subunit, and a τ complex (or subunit or subassembly of the DnaX complex) in the presence of the candidate pharmaceutical, and dNTPs (or modified dNTPs) to form a reaction mixture
b) subjecting the reaction mixture to conditions effective to achieve nucleic acid polymerization in the absence of the candidate compound; and
c) comparing the nucleic acid polymerization in the presence of the test compound with the nucleic acid polymerization in the absence of the test compound,
wherein a change in the nucleic acid polymerization in the presence of the test compound is indicative of a compound that modulates the activity of a DnaX complex and a β subunit,
wherein said τ complex (or subunit or subassembly of the τ complex) comprises a DNA polymerase III δ subunit protein.

**45.** The method of claim 44, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO:112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO:148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**46.** A compound that modulates the activity of a DNA polymerase III replicase identified by the method of Claim 44 or 45.

**47.** A method to identify compounds that modulate the ability of a β subunit and a DnaX complex (or a subunit or subassembly of the DnaX complex) to interact comprising
a) contacting the β subunit with the DnaX complex (or subunit or subassembly of the DnaX complex) in the presence of the compounds to form a reaction mixture
b) subjecting the reaction mixture to conditions under which the DnaX complex (or the subunit or subassembly of the DnaX complex) and the β subunit would interact in the absence of the compound; and
c) comparing the extent of interaction in the presence of the test compound with the extent of interaction in the absence of the test compound, wherein a change in the interaction between the β subunit and the DnaX complex (or the subunit or subassembly of the DnaX complex) is indicative of a compound that modulates the interaction,
wherein said DnaX complex (or subunit or subassembly of the DnaX complex) comprises a DNA polymerase III δ subunit protein.

**48.** The method of claim 47, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO:112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO:148, SEQ ID NO:151, and SEQ ID NO: 154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**49.** A compound that modulates the activity of a DNA polymerase III replicase identified by the method of Claim 47 or 48.

**50.** A method to identify compounds that modulate the ability of a DnaX complex (or a subassembly of the DnaX complex) to assemble a β subunit onto a DNA molecule comprising
a) contacting a circular primed DNA molecule (which may be coated with SSB) with the DnaX complex (or the subassembly thereof) and the β subunit in the presence of the compound, and ATP or dATP to form a reaction mixture
b) subjecting the reaction mixture to conditions under which the DnaX complex (or subassembly) assembles the β subunit on the DNA molecule absent the compound; and
c) comparing extent of assembly in the presence of the test with the extent of assembly in the absence of the test compound, wherein a change in the amount of β subunit on the DNA molecule is indicative of a compound that modulates the ability of a DnaX complex (or a subassembly of the DnaX complex) to assemble a β subunit onto a DNA molecule,
wherein the DnaX complex (or a subassembly of the DnaX complex) comprises a DNA polymerase III δ subunit protein.

**51.** The method of claim 50, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ m NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID N0:91, SEQ ID NO:94, SEQ ID NO:97, SEQ ID NO:100, SEQ ID NO:103, SEQ ID NO:106, SEQ ID NO:109, SEQ ID NO:112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO: 145, SEQ ID NO: 148, SEQ ID NO:151, and SEQ ID NO:1S4; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**52.** A compound that modulates the activity of a DNA polymerase III replicase identified by the method of Claim 50 or 51.

**53.** A method to identify compounds that modulate the ability of a DnaX complex (or a subunit (s) of the DnaX complex) to disassemble a β subunit from a DNA molecule comprising
a) contacting a DNA molecule onto which the β subunit has been assembled in the presence of the compound, to form a reaction mixture;
b) subjecting the reaction mixture to conditions under which the DnaX complex (or a subunit (s) or subassembly of the DnaX complex) disassembles the β subunit from the DNA molecule absent the compound; and
c) comparing the extent of assembly in the presence of the test compound with the extent of assembly in the absence of the test compound, wherein a change in the amount of β subunit on the DNA molecule is indicative of a compound that modulates the ability of a DnaX complex (or a subassembly of the DnaX complex) to disassemble a β subunit onto a DNA molecule,
wherein the DnaX complex (or a subassembly of the DnaX complex) comprises a DNA polymerase III δ subunit protein.

**54.** The method of claim 53, wherein said DNA polymerase III δ subunit protein is encoded by a nucleic acid molecule selected from the group consisting of
a) SEQ ID NO:1, SEQ ID NO:4, SEQ ID NO:7, SEQ ID NO: 10, SEQ ID NO: 13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO:112, SEQ ID NO:115, SEQ ID NO: 118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO:148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**55.** A compound that modulates the activity of a DNA polymerase III replicase identified by the method of Claim 53 or 54.

**56.** A method to identify compounds that modulate the dATP/ATP binding activity of a DnaX complex or a DnaX complex subunit (e. g. subunit) comprising
a) contacting the DnaX complex (or the DnaX complex subunit) with dATP/ATP either in the presence or absence of a DNA molecule and/or the β subunit in the presence of the compound to form a reaction mixture;
b) subjecting the reaction mixture to conditions in which the DnaX complex (or the subunit of DnaX complex) interacts with dATP/ATP in the absence of the compound; and
c) comparing the extent of binding in the presence of the test compound with the extent of binding in the absence of the test compound, wherein a change in the dATP/ATP binding is indicative of a compound that modulates the dATP/ATP binding activity of a DnaX complex or a DnaX complex subunit (e. g. τ subunit),
wherein the DnaX complex (or the subunit of DnaX complex) comprises a DNA polymerase III δ subunit protein.

**57.** The method of claim 56, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO:112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO:148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**58.** An compound that modulates the activity of a DNA polymerase III replicase identified by the method of Claim 56 or 57.

**59.** A method to identify compound that modulate the dATP/ATPase activity of a DnaX complex or a DnaX complex subunit (e. g., the τ subunit)comprising
a) contacting the DnaX complex (or the DnaX complex subunit) with dATP/ATP either in the presence or absence of a DNA molecule and/or a β subunit in the presence of the compound to form a reaction mixture;
b) subjecting the reaction mixture to conditions in which the DnaX subunit (or complex) hydrolyzes dATP/ATP in the absence of the compound; and
c) comparing the extent of hydrolysis in the presence of the test compound with the extent of hydrolysis in the absence of the test compound, wherein a change in the amount of dATP/ATP hydrolyzed is indicative of a compound that modulates the dATP/ATPase activity of a DnaX complex or a DnaX complex subunit (e. g., the τ subunit)
wherein the DnaX complex (or subunit) comprises a DNA polymerase III δ subunit protein.

**60.** The method of claim 59, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO: 100, SEQ ID NO:103, SEQ ID NO:106, SEQ ID NO:109, SEQ ID NO:112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO: 148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**61.** An compound that modulates the activity of a DNA polymerase III replicase identified by the method of Claim 59 or 60.

**62.** A method for identifying compound that modulate the activity of a DNA polymerase replicase comprising
a) contacting a circular primed DNA molecule, optionally coated with SSB, with a DnaX complex, a β subunit and an α subunit in the presence of the compound, and dNTPs (or modified dNTPs) to form a reaction mixture;
b) subjecting the reaction mixture to conditions, which in the absence of the compound, affect nucleic acid polymerization; and
c) comparing the nucleic acid polymerization in the presence of the test compound with the nucleic acid polymerization in the absence of the test compound, wherein a change in the activity of the replicase in the presence of the test compound is indicative of a compound that modulates the activity of the replicase,
wherein the DnaX complex comprises a DNA polymerase III δ subunit protein.

**63.** The method of claim 62, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO: 112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO:148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**64.** A compound that modulates the activity of a DNA polymerase III replicase identified by the method of Claim 62 or 63.

**65.** A method to identify compound that modulate the ability of a δ subunit and the δ' and/or DnaX subunit to interact comprising
a) contacting the δ subunit with the δ' and/or δ' plus DnaX subunit in the presence of the compound to form a reaction mixture
b) subjecting the reaction mixture to conditions under which the δ subunit and the δ' and/or δ' plus DnaX subunit would interact in the absence of the compound
c) comparing the extent of interaction in the presence of the test compound with the extent of interaction in the absence of the test compound, wherein a change in the interaction between the δ subunit and the δ' and/or DnaX subunit is indicative of a compound that modulates the interaction,
wherein the DnaX complex comprises a DNA polymerase III δ subunit protein.

**66.** The method of claim 65, wherein said DNA polymerase III δ subunit protein is encoded by a nucleic acid molecule selected from the group consisting of
a) SEQ ID NO:1, SEQ ID NO:4, SEQ ID NO:7, SEQ ID NO:10, SEQ ID NO:13, SEQ ID NO:16, SEQ ID NO:19, SEQ ID NO:22, SEQ ID NO:25, SEQ ID NO:28, SEQ ID NO:31, SEQ ID NO:34, SEQ ID NO:37, SEQ ID NO:40, SEQ ID NO:43, SEQ ID NO:46, SEQ ID NO:49, SEQ ID NO:223, SEQ ID NO:52, SEQ ID NO:55, SEQ ID NO:58, SEQ ID NO:61, SEQ ID NO:64, SEQ ID NO:67, SEQ ID NO:70, SEQ ID NO:73, SEQ ID NO:76, SEQ ID NO:79, SEQ ID NO:82, SEQ ID NO:85, SEQ ID NO:88, SEQ ID NO:91, SEQ ID NO:94, SEQ ID NO:97, SEQ ID NO:100, SEQ ID NO:103, SEQ ID NO:106, SEQ ID NO:109, SEQ ID NO:112, SEQ ID NO:115, SEQ ID NO:118, SEQ ID NO:121, SEQ ID NO:124, SEQ ID NO:127, SEQ ID NO:130, SEQ ID NO:133, SEQ ID NO:136, SEQ ID NO:139, SEQ ID NO:142, SEQ ID NO:145, SEQ ID NO:148, SEQ ID NO:151, and SEQ ID NO:154; and
b) a protein comprising a homologue of a protein of a), wherein said homologue encodes a protein containing one or more amino acid deletions, substitutions, or insertions, and wherein said protein performs the function of a natural δ subunit protein in a bacterial replication assay; and
c) an isolated bacterial nucleic acid molecule which is fully complementary to any said nucleic acid molecule recited in a).

**67.** A compound that modulates the activity of a DNA polymerase III replicase identified by the method of Claim 65 or 66.

**68.** A method of synthesizing a DNA molecule comprising
a) hybridizing a primer to a first DNA molecule; and
b) incubating said DNA molecule in the presence of a thermostable DNA polymerase replicase and one or more dNTPs under conditions sufficient to synthesize a second DNA molecule complementary to all or a portion of said first DNA molecule;
wherein said thermostable DNA polymerase replicase comprises an δ subunit protein selected from the group consisting of an *Aquifex, Thermus* and *Thermatoga* δ subunit protein.

**69.** The method of Claim 70, wherein said thermothermostable DNA polymerase replicase comprises an δ subunit protein selected from the group consisting of an *Aquifex aeolicus, Thermus thermophilus,* and *Thermatoga maratima* δ subunit protein.

**71.** The method of Claim 69, wherein said thermostable DNA polymerase replicase comprises a DNA polymerase δ subunit protein selected from the group consisting of
i) a protein selected from the group consisting of SEQ ID NO:3, SEQ ID NO:226, SEQ ID NO:81, and SEQ ID NO:108; and;
ii) and a protein selected from the group consisting of a protein having 95% sequence identity to an amino acid sequence of i).

**72.** A method of amplifying a double-stranded DNA molecule comprising:
a) providing a first and second primer, wherein said first primer is complementary to a sequence at or near the 3'-termini of the first strand of said DNA molecule and said second primer is complementary to a sequence at or near the 3'-termini of the second strand of said DNA molecule;
b) hybridizing said primer to said first strand and said second primer to said second strand in the presence of a thermostable DNA polymerase replicase, under conditions such that a third DNA molecule complementary to said first strand and a fourth DNA molecule complementary to said second strand are synthesized;
c) denaturing said first and third strand, and said second and fourth strands; and
d) repeating steps a) to c) one or more times; wherein:
said thermostable DNA polymerase is selected from the group consisting of an *Aquifex*, *Thermus* and *Thermatoga δ* subunit protein.

**71.** The method of Claim X, wherein said thermostable DNA polymerase replicase comprises an δ subunit protein selected from the group consisting of an *Aquife*x *aeolicus, Thermus thermophilus,* and *Thermatoga maratima δ* subunit protein.

**73.** The method of Claim 72, wherein said thermostable DNA polymerase replicase comprises a DNA polymerase δ subunit protein selected from the group consisting of
i) a protein selected from the group consisting of SEQ ID N0:3, SEQ ID NO:226, SEQ ID NO:81, and SEQ ID NO: 108; and;
ii) and a protein selected from the group consisting of a protein having 95% sequence identity to an amino acid sequence of i).

**74.** A method of amplifying nucleic acid sequences, the method comprising,
a) mixing a rolling circle replication primer with one or more amplification target circles, to produce a primer-amplification target circle mixture, and incubating the primer-amplification target circle mixture under conditions that promote hybridization between the amplification target circles and the rolling circle replication primer in the primer-amplification target circle mixture,
wherein the amplification target circles each comprise a single-stranded, circular DNA molecule comprising a primer complement portion, and wherein the primer complement portion is complementary to the rolling circle replication primer, and
b) mixing a thermostable DNA polymerase replicase with the primer-amplification target circle mixture, to produce a replicase-amplification target circle mixture, and incubating the replicase-amplification target circle mixture under conditions that promote replication of the amplification target circles,
wherein replication of the amplification target circles results in the formation of tandem sequence DNA,
and wherein said thermostable DNA polymerase replicase comprises an δ subunit protein selected from the group consisting of an *Aquifex*, *Thermus* and *Thermatoga 8* subunit protein.

**75.** The method of Claim 74, wherein said thermostable DNA polymerase replicase comprises an δ subunit protein selected from the group consisting of an *Aquifex aeolicus, Thermus thermophilus,* and *Thermatoga maratima δ* subunit protein.

**76.** The method of Claim 75, wherein said thermostable DNA polymerase replicase comprises a DNA polymerase δ subunit protein selected from the group consisting of
i) a protein selected from the group consisting of SEQ ID NO:3, SEQ ID NO:226, SEQ ID NO:8 1, and SEQ ID NO: 108; and;
ii) and a protein selected from the group consisting of a protein having 95% sequence identity to an amino acid sequence of i).

**77.** The method of Claim 74, wherein said DNA polymerase replicase comprises a homolog of *E*. *coli* DnaB helicase.

**78.** A method of synthesizing DNA which comprises utilizing one or more polypeptides, said one or more polypeptides comprising an amino acid sequence having at least 95% sequence identity to an amino acid sequence from the group consisting of SEQ ID NO:3, SEQ ID NO:226, SEQ ID NO:6, SEQ ID NO:9, SEQ ID NO:12, SEQ ID NO:15, SEQ ID NO:18, SEQ ID NO:21, SEQ ID NO:24, SEQ ID NO:27, SEQ ID NO:30, SEQ ID NO:33, SEQ ID NO:36, SEQ ID NO:39, SEQ ID NO:42, SEQ ID NO:45, SEQ ID NO:48, SEQ ID NO:51, SEQ ID NO:227, SEQ ID NO:228, SEQ ID NO:54, SEQ ID NO:57, SEQ ID NO:60, SEQ ID NO:63, SEQ ID NO:66, SEQ ID NO:69, SEQ ID NO:72, SEQ ID NO:75, SEQ ID NO:78, SEQ ID NO:81, SEQ ID NO:84, SEQ ID NO:87, SEQ ID NO:90, SEQ ID NO:93, SEQ ID NO:96, SEQ ID NO:99, SEQ ID NO:102, SEQ ID NO:229, SEQ ID NO:105, SEQ ID NO:108, SEQ ID NO:111, SEQ ID NO:114, SEQ ID NO:117, SEQ ID NO:120, SEQ ID NO:123, SEQ ID NO:126, SEQ ID NO:129, SEQ ID NO:132, SEQ ID NO:135, SEQ ID NO:138, SEQ ID NO:141, SEQ ID NO:144, SEQ ID NO:147, SEQ ID NO:150, SEQ ID NO:153, and SEQ ID NO:156.

**79.** The method of claim 78 further comprising providing in any order: a reaction mixture comprising components comprising template, and nucleotides, and incubating said reaction mixture for a length of time and at a temperature sufficient to obtain DNA synthesis.

**80.** An isolated bacterial DNA polymerase δ subunit protein, wherein the δ subunit protein is a phylum Firmicutes δ subunit protein.
